# EUROPÄISCHE PATENTSCHRIFT

(11) **EP 3 086 814 B1**
(45) Veröffentlichungstag und Bekanntmachung des Hinweises auf die Patenterteilung: **24.06.2020**
(21) Anmeldenummer: 14833342.0
(22) Anmeldetag: 10.12.2014
(51) Int. Cl.: A61K 47/22, C07D 233/64, C07D 207/335, A61K 35/00, A61K 47/68, A61K 31/40, A61K 31/415, A61P 35/00

(54) **BINDER-KONJUGATE (ADCS) MIT KSP-INHIBITOREN**
DRUG CONJUGATES (ADCS) WITH KINESIN SPINDEL PROTEIN (KSP)
CONJUGUÉS DE LIANTS (CONJUGUÉ ANTICORPS-MÉDICAMENT) COMPRENANT DES INHIBITEURS DE LA PROTÉINE KINÉSINE DU FUSEAU

(30) Priorität: 23.12.2013 EP 13199358; 30.05.2014 EP 14170585
(43) Veröffentlichungstag der Anmeldung: 02.11.2016
(73) Patentinhaber: Bayer Pharma Aktiengesellschaft, 13353 Berlin (DE)
(72) Erfinder: LERCHEN, Hans-Georg, 51375 Leverkusen (DE); WITTROCK, Sven, 10117 Berlin (DE); GRIEBENOW, Nils, 41541 Dormagen (DE); STELTE-LUDWIG, Beatrix, 42489 Wülfrath (DE); SOMMER, Anette, 10405 Berlin (DE); BERNDT, Sandra, 16540 Hohen Neuendorf (DE); MAHLERT, Christoph, 42111 Wuppertal (DE); LOBELL, Mario, 42113 Wuppertal (DE); TERJUNG, Carsten, 44799 Bochum (DE); GREVEN, Simone, 41541 Dormagen (DE); CANCHO GRANDE, Yolanda, 51373 Leverkusen (DE)
(74) Vertreter: BIP Patents
(86) Internationale Anmeldenummer: PCT/EP2014/077144
(87) Internationale Veröffentlichungsnummer: WO 2015/096982

(56) Entgegenhaltungen:
- WO-A1-2006/002236
- WO-A1-2007/021794
- WO-A2-2004/100873
- WO-A2-2008/086122
- US-B1- 7 662 581

## Beschreibung

### Einleitung und Stand der Technik

Die Erfindung betrifft Binder-Wirkstoff-Konjugate (ADCs) von Kinesin Spindel Protein-Inhibitoren, wirksamer Metabolite dieser ADCs, Verfahren zur Herstellung dieser ADCs, die Verwendung dieser ADCs zur Behandlung und/oder Prävention von Krankheiten sowie die Verwendung dieser ADCs zur Herstellung von Arzneimitteln zur Behandlung und/oder Prävention von Krankheiten, insbesondere von hyperproliferativen und/oder angiogenen Erkrankungen wie beispielsweise Krebserkrankungen. Solche Behandlungen können als Monotherapie oder auch in Kombination mit anderen Arzneimitteln oder weiteren therapeutischen Maßnahmen erfolgen.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Tumore früher Stadien lassen sich gegebenenfalls durch chirurgische und radiotherapeutische Maßnahmen entfernen. Metastasierte Tumore können im Regelfall durch Chemotherapeutika nur palliativ therapiert werden. Ziel hierbei ist, die optimale Kombination aus einer Verbesserung der Lebensqualität und der Verlängerung der Lebenszeit zu erreichen.

Konjugate von Binderproteinen mit einem oder mehreren Wirkstoffmolekülen sind bekannt, insbesondere in Form sogannter "antibody drug conjugates" (ADCs), in welchen ein internalisierender, gegen ein Tumor-assoziiertes Antigen gerichteter Antikörper auf kovalente Weise über eine Verknüpfungseinheit ("linker") mit einem cytotoxisch wirkenden Agens verbunden ist. Nach Einschleusung des ADCs in die Tumorzelle und anschließender Spaltung des Konjugats wird dann innerhalb der Tumorzelle entweder das cytotoxische Agens selbst oder ein anderer daraus gebildeter cytotoxisch wirksamer Metabolit freigesetzt, und kann dort seine Wirkung direkt und selektiv entfalten. Auf diesem Wege könnte die Schädigung von normalem Gewebe im Vergleich zu einer konventionellen Chemotherapie der Krebserkrankung in signifikant engeren Grenzen gehalten werden [siehe z.B. J. M. Lambert, Curr. Opin. Pharmacol. 5, 543-549 (2005); A. M. Wu und P. D. Senter, Nat. Biotechnol. 23, 1137-1146 (2005); P. D. Senter, Curr. Opin. Chem. Biol. 13, 235-244 (2009); L. Ducry und B. Stump, Bioconjugate Chem. 21, 5-13 (2010)]. So beschreibt WO2012/171020 ADCs, bei denen mehrere Toxophormoleküle über einen polymeren Linker an einen Antikörper verknüpft sind. Als mögliche Toxophore werden in WO2012/171020 unter anderem die Substanzen SB 743921, SB 715992 (Ispinesib), MK-0371, AZD8477, AZ3146 und ARRY-520 erwähnt.

Die zuletzt genannten Substanzen sind sogenannte Kinesin Spindel Protein-Inhibitoren. Kinesin Spindel Protein (KSP, auch bekannt als Eg5, HsEg5, KNSL1 oder KIF11) ist ein Kinesin-ähnliches Motorprotein, welches für die Funktion der bipolaren mitotischen Spindel essentiell ist. Die Inhibierung von KSP führt zum mitotischen Stillstand und über einen längeren Zeitraum zur Apoptose (Tao et al., Cancer Cell 2005 Jul 8(1), 39-59). Nach dem Auffinden des ersten zellgängigen KSP-Inhibitors, Monastrol, haben sich KSP-Inhibitoren als eine Klasse neuer Chemotherapeutika etabliert (Mayer et al., Science 286: 971-974, 1999) und sind Gegenstand einer Reihe von Patentanmeldungen (z.B. WO2006/044825; WO2006/002236; WO2005/051922; WO2006/060737; WO03/060064; WO03/040979; und WO03/049527). Da KSP jedoch nur in einem kurzen Zeitraum der Mitosephase seine Wirkung entfaltet, müssen KSP-Inhibitoren in ausreichend hoher Konzentration während dieser ersten Phasen vorliegen.

### Zusammenfassung der Erfindung

Vor diesem Hintergrund ist es die Aufgabe der vorliegenden Erfindung, Substanzen bereitzustellen, die nach Verabreichung in relativ geringer Konzentration eine apoptotische Wirkung zeigen und damit für die Krebstherapie nützlich sein können.

Zur Lösung dieser Aufgabe stellt die Erfindung Konjugate eines Binders oder Derivats hiervon mit einem oder mehreren Wirkstoffmolekülen bereit, wobei das Wirkstoffmolekül ein Kinesin Spindel Protein-Inhibitor (KSP-Inhibitor) ist, das mit dem Binder über einen Linker L verbunden ist. Der Binder ist vorzugsweise ein Binderprotein oder -peptid, besonders bevorzugt ein humaner, humanisierter oder chimärer monoklonaler Antikörper oder ein Antigen-bindendes Fragment hiervon ist, insbesondere ein anti-TWEAKR-Antikörper oder ein Antigen-bindendes Fragment hiervon oder ein anti-EGFR-Antikörper oder ein Antigen-bindendes Fragment hiervon. Besonders bevorzugt sind ein anti-TWEAKR-Antikörper, der spezifisch an die Aminosäure D in Position 47 (D47) von TWEAKR (SEQ ID NO:169) bindet, insbesondere der anti-TWEAKR-Antikörper TPP-2090, oder die anti-EGFR-Antikörper Cetuximab oder Nimotuzumab.

Die Erfinder haben insbesondere eine Reihe von Möglichkeiten gefunden, wie der Binder an den KSP-Inhibitor gebunden werden kann, um die oben genannte Aufgabe zu lösen.

Erfindungsgemäß können die Kinesin Spindel Protein-Inhibitoren die folgende Substruktur I(sub) aufweisen: 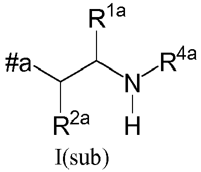 wobei
#a eine Bindung zum Restmolekül darstellt;
R^{1a} H oder -(CH₂)₀₋₃Z darstellt, wobei Z -H, Halogen, -OY³, -SY³, -NHY³, -CO-NY¹Y², oder-CO-OY³ darstellt,
wobei Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, SO₃H, NH₂, COOH, -NH-CO-CH₂-CH₂-CH(NH₂)COOH oder -(CO-NH-CHY⁴)₁₋₃COOH darstellt, wobei W H oder OH darstellt, wobei Y⁴ unabhängig voneinander lineares oder verzweigtes, gegebenenfalls mit -NHCONH₂ substituiertes, C₁₋₆Alkyl oder gegebenenfalls mit -NH₂ substituiertes Aryl oder Benzyl darstellt;
R^{2a} und R^{4a} unabhängig voneinander H, -CO-CHY⁴-NHY⁵ oder -(CH₂)₀₋₃Z darstellen, oder R^{2a} und R^{4a} gemeinsam (unter Bildung eines Pyrrolidinringes) -CH₂-CHR¹⁰- oder -CHR¹⁰-CH₂-darstellen, wobei R¹⁰ H, NH₂, COOH, SO₃H, SH, oder OH darstellt, und wobei Z -H, -OY³,-SY³, -NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt.

Erfindungsgemäß kann der Kinesin Spindel Protein-Inhibitor durch Substitution eines H-Atoms an R^{1a}, R^{2a}, R^{4a} oder R¹⁰ über einen Linker mit dem Binder verbunden werden.

Der KSP-Inhibitor, der an diesen Binder gebunden ist, (bzw. die KSP-Inhibitoren, da regelmäßig mehr als ein KSP-Inhibitor an den Binder gebunden wird), ist vorzugsweise eine Verbindung der folgenden Formel (Ia) oder (IIa):
Formel (Ia): 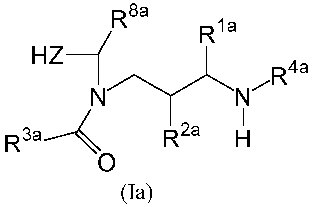 wobei
R^{1a} H, -MOD oder -(CH₂)₀₋₃Z darstellt, wobei Z -H, Halogen, -OY³, -SY³, -NHY³, -CO-NY¹Y², oder -CO-OY³ darstellt,
mit Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, SO₃H, NH₂, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R^{2a} und R^{4a} unabhängig voneinander H, -CO-CHY⁴-NHY⁵ oder -(CH₂)₀₋₃Z darstellen, oder R^{2a} und R^{4a} gemeinsam (unter Bildung eines Pyrrolidinringes) -CH₂-CHR¹⁰- oder -CHR¹⁰-CH₂-darstellen, wobei R¹⁰ H, SO₃H, NH₂, COOH, SH, oder OH darstellt;
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
R^{3a} -MOD oder eine gegebenenfalls substituierte Alkyl-, Cycloalkyl-, Aryl-, Heteroaryl-Heteroalkyl- oder Heterocycloalkyl- Gruppe darstellt,
bevorzugt eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³,-NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder - (CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet);
R^{8a} C₁₋₁₀-Alkyl oder -(CH₂)₀₋₂-(HZ²) darstellt, wobei HZ² ein 4 bis 7-gliedriger Heterocyclus mit bis zu zwei Heteroatomen ausgewählt aus N, O und S darstellt;
HZ ein mono- oder bicyclischer Heterozyklus ist, der mit einem oder mehreren Substituenten ausgewählt aus Halogen, C₁₋₁₀-Alkylgruppen, C₆₋₁₀-Arylgruppen oder C₆₋₁₀-Aralkylgruppen, die ggf. mit Halogen substituiert sein können, substituiert sein kann;
wobei -MOD -(NR¹⁰)ₙ-(G1)ₒ-G2-H darstellt, wobei
   R¹⁰ H oder C₁-C₃-Alkyl darstellt;
   G1 -NHCO- , -CONH- oder 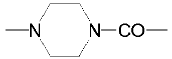 darstellt (wobei wenn G1 -NHCO- oder 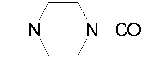 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NR^{y}-, -NR^{y}CO-, CONR^{y}-, -NR^{y}NR^{y}-, -SO2NR^{y}NR^{y}-, -CONRYNRY-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
wobei der Kinesin Spindel Protein-Inhibitor durch Substitution eines H-Atoms an R^{1a}, R^{2a}, R^{3a}, R^{4a}, R^{8a} oder R¹⁰ oder ggf. über einen der Substituenten von HZ, insbesondere über R^{1a}, R^{2a}, R^{3a}, R^{4a} oder R¹⁰, mit dem Linker verbunden ist,
sowie ihre Salze, Solvate und Salze der Solvate.

Formel (IIa): 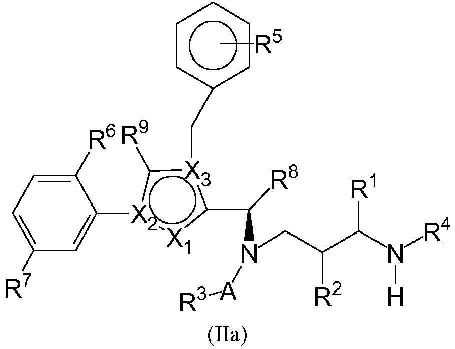 wobei
X₁ N, X₂ N und X₃ C darstellt; oder
X₁ CH oder CF, X₂ C und X₃ N darstellt; oder
X₁ NH, X₂ C und X₃ C darstellt; oder
X₁ CH, X₂ N und X₃ C darstellt;
(wobei X₁ CH, X₂ C und X₃ N bevorzugt ist);
R¹ H, -L-#1, -MOD oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² -L-#1, H, -MOD, -CO-CHY⁴-NHY⁵ oder -(CH₂)₀₋₃Z darstellt, oder R² und R⁴ gemeinsam (unter Bildung eines Pyrrolidinringes) -CH₂-CHR¹⁰- oder -CHR¹⁰-CH₂- darstellen, wobei R¹⁰ L-#1, H, NH₂, SO₃H, COOH, SH, oder OH darstellt;
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
R⁴ -L-#1, H, -CO-CHY⁴-NHY⁵ oder -(CH₂)₀₋₃Z darstellt,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
oder R² und R⁴ gemeinsam (unter Bildung eines Pyrrolidinringes) -CH₂-CHR¹⁰- oder -CHR¹⁰-CH₂- darstellen, wobei R¹⁰ L-#1, H, NH₂, SO₃H, COOH, SH, oder OH darstellt;
A CO, SO, SO₂, SO₂NH oder CNNH darstellt;
R³ -L-#1, -MOD, oder eine gegebenenfalls substituierte Alkyl-, Cycloalkyl-, Aryl-, Heteroaryl-Heteroalkyl-, Heterocycloalkyl- Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3-SO₂-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder-CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet);
R⁵ -L-#1, H, NH₂, NO₂, Halogen (insbesondere F, Cl, Br), -CN, CF₃, -OCF₃, -CH₂F, -CH₂F, SH oder -(CH₂)₀₋₃Z darstellt, wobei Z -H, -OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
darstellt,
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, NO₂, NH₂, COOH oder Halogen (insbesondere F, Cl, Br) darstellen,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, (ggf. fluoriertes) C₄₋₁₀-Cycloalkyl oder-(CH₂)₀₋₂-(HZ²) darstellt, wobei HZ² ein 4 bis 7-gliedriger Heterocyclus mit bis zu zwei Heteroatomen ausgewählt aus N, O und S darstellt, wobei jede dieser Gruppen mit -OH, CO₂H or NH₂ oder -L-#1 substituiert sein kann;
wobei einer oder keiner der Substituenten R¹, R², R³, R⁴ R⁵, R⁸ und R¹⁰ -L-#1 darstellt bzw. (im Falle von R⁸) aufweist,
L für den Linker steht und #1 für die Bindung zum Binder bzw. Derivat hiervon steht, R⁹ H, F, CH₃, CF₃, CH₂F oder CHF₂ darstellt;
wobei -MOD -(NR¹⁰)ₙ-(G1)ₒ-G2-H darstellt, wobei
   R¹⁰ H oder C₁-C₃-Alkyl darstellt;
   G1 -NHCO- , -CONH- oder 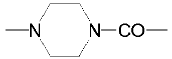 darstellt (wobei wenn G1 -NHCO- oder 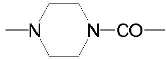 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NRY-, -NRYCO-, CONRY-, -NRYNRY-, -SO2NR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
sowie ihre Salze, Solvate und Salze der Solvate.

Die erfindungsgemäßen Konjugate können chemisch labile Linker, enzymatisch labile Linker oder stabile Linker aufweisen. Besonders bevorzugt sind stabile Linker und durch Cathepsin spaltbare Linker. Die Erfindung stellt ferner bereit Verfahren zur Herstellung der erfindungsgemäßen Konjugate sowie Vorstufen und Intermediate zur Herstellung.

Die Herstellung der erfindungsgemäßen Konjugate umfasst regelmäßig die folgenden Schritte:
(i) Herstellen einer ggf. Schutzgruppen tragenden Linkervorstufe mit einer reaktiven Gruppe, die an den Binder koppeln kann;
(ii) Konjugieren der Linkervorstufe an das ggf. Schutzgruppen tragende Derivat eines niedermolekularen KSP-Inhibitor (vorzugsweise ein KSP-Inhibitor mit der Substruktur I(sub), besonders bevorzugt mit Formel (Ia)und insbesondere mit Formel (IIa), wobei in diesen Formeln eine Bindung an einen Linker noch nicht vorliegt), wobei ein ggf. Schutzgruppen tragendes reaktives KSP-Inhibitor-Linker-Konjugat erhalten wird;
(iii) Abspaltung der ggf. vorhandenen Schutzgruppen im KSP-Inhibitor-Linker-Konjugat und
(iv) Konjugieren des Binders an das KSP-Inhibitor-Linker-Konjugat, wobei das erfindungsgemäße Binder-KSP-Inhibitor-Konjugat erhalten wird.

Die Anknüpfung der reaktiven Gruppe kann auch nach Aufbau einer ggf. geschützt vorliegenden KSP-Inhibitor-Linkervorstufe Konjugats erfolgen.

In Abhängigkeit vom Linker können Succinimid-verknüfte ADCs nach der Konjugation nach Schema 26 in die offenkettigenen Bernsteinsäureamide überführt werden, die ein vorteilhaftes Stabilitätsprofil aufweisen.

Wie oben erläutert kann das Konjugieren der Linkervorstufe an einen niedermolekularen KSP-Inhibitor durch Substitution eines Wasserstoffatoms an R^{1a}, R^{2a}, R^{4a} oder R¹⁰ in Substruktur I(sub), R^{1a}, R^{2a}, R^{3a}, R^{4a}, R^{8a} oder R¹⁰ in Formel (Ia), oder R¹, R², R³, R⁴, R⁵ , R⁸ oder R¹⁰ in Formel (IIa) durch den Linker erfolgen. In den Syntheseschritten vor der Konjugation können ggf. vorhandene funktionelle Gruppen auch in geschützter Form vorliegen. Vor dem Konjugationsschritt werden diese Schutzgruppen nach bekannten Methoden der Peptidchemie entfernt. Die Konjugation kann chemisch auf verschiedenen Wegen erfolgen, wie in den Schemata 7 bis 31in den Beispielen exemplarisch dargelegt. Es ist insbesondere möglich, den niedermolekularen KSP-Inhibitor für die Konjugation an den Linker ggf. zu modifizieren, z.B. durch Einführen von Schutzgruppen oder Abgangsgruppen zur einfacheren Substituierung.

Insbesondere stellt die Erfindung neue niedermolekulare KSP-Inhibitoren bereit, die ggf. an einen Binder konjugiert werden können. Diese KSP-Inhibitoren bzw. ihre Binder-Konjugate weisen die folgende allgemeine Formel (IIIa) auf: 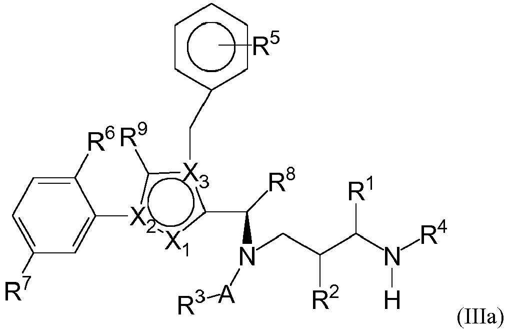 wobei
X₁ N, X₂ N und X₃ C darstellt; oder
X₁ CH oder CF, X₂ C und X₃ N darstellt; oder
X₁ NH, X₂ C und X₃ C darstellt; oder
X₁ CH, X₂ N und X₃ C darstellt;
(wobei X₁ CH, X₂ C und X₃ N bevorzugt ist);
R¹ H, -L-BINDER, -MOD oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² -L-BINDER, H, -MOD, -CO-CHY⁴-NHY⁵ oder -(CH₂)₀₋₃Z darstellt, oder R² und R⁴ gemeinsam (unter Bildung eines Pyrrolidinringes) -CH₂-CHR¹⁰- oder -CHR¹⁰-CH₂- darstellen, wobei R¹⁰ L-#1, H, NH₂, SO₃H, COOH, SH, oder OH darstellt;
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
R⁴ -L-BINDER, H, -CO-CHY⁴-NHY⁵ oder -(CH₂)₀₋₃Z darstellt,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
oder R² und R⁴ gemeinsam (unter Bildung eines Pyrrolidinringes) -CH₂-CHR¹⁰- oder -CHR¹⁰-CH₂- darstellen, wobei R¹⁰ -L-BINDER, H, NH₂, SO₃H, COOH, SH, oder OH darstellt;
A CO, SO, SO₂, SO₂NH oder CNNH darstellt;
R³ -L-BINDER, -MOD, oder eine gegebenenfalls substituierte Alkyl-, Cycloalkyl-, Aryl-, Heteroaryl- Heteroalkyl-, Heterocycloalkyl- Gruppe darstellt, bevorzugt -L-BINDER oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 -N(Alkyl)₂ Gruppen, 1-3-NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet);
R⁵ -L-BINDER, H, NH₂, NO₂, Halogen (insbesondere F, Cl, Br), -CN, CF₃, -OCF₃, -CH₂F,-CH₂F, SH oder -(CH₂)₀₋₃Z darstellt, wobei Z -H, -OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
darstellt,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, (ggf. fluoriertes) C₄₋₁₀-Cycloalkyl oder -(CH₂)₀₋₂-(HZ²) darstellt, wobei HZ² ein 4 bis 7-gliedriger Heterocyclus mit bis zu zwei Heteroatomen ausgewählt aus N, O und S darstellt (vorzugsweise Oxetan), wobei jede dieser Gruppen mit -OH, CO₂H or NH₂ oder -L-BINDER substituiert sein kann;
wobei L für einen Linker steht und BINDER für Binder bzw. Derivat hiervon steht, wobei der Binder ggf. an mehrere Wirkstoffmoleküle gebunden sein kann,
wobei maximal ein Vertreter von R¹, R², R³ R⁴, R⁵, R⁸ und R¹⁰ -L-Binder darstellt;
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, NO₂, NH₂, COOH oder Halogen (insbesondere F, Cl, Br) darstellen,
R⁹ H, F, CH₃, CF₃, CH₂F oder CHF₂ darstellt;
wobei -MOD -(NR¹⁰)ₙ-(G1)ₒ-G2-H darstellt, wobei
   R¹⁰ H oder C₁-C₃-Alkyl darstellt;
   G1 -NHCO- , -CONH- oder 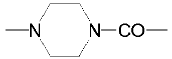 darstellt (wobei wenn G1 -NHCO- oder 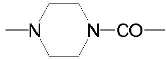 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NRY-, -NRYCO-, CONRY-, -NRYNRY-, -SO2NR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
sowie ihre Salze, Solvate und Salze der Solvate.

### Beschreibung der Figuren

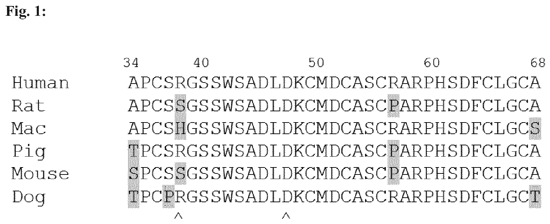

**Figur 1****:** Alignment der TWEAKR-cysteinreichen Domäne (Aminosäure 34 bis 68) von verschienden Spezies. (Die Zahlen zeigen die Aminosäureposition in Vollängenkonstrukten inklusive der Signalsequenzen; SEQ ID NO: 169).
**Figur 2****:**
   A -Schematisches Diagram der Struktur von TWEAKR (SEQ ID NO: 169). Das Diagramm zeigt die extrazelluläre Domäne (Aminosäuren 28-80) (SEQ ID NO: 168) einschließlich der cysteinreichen Domäne (36-67), der Transmembrandomäne - TM (81-101) und der intrazellulären Domäne (102-129). TPP-2202 - die vollständige Ektodomäne (28-80), an die Fc-Domäne von hIgG1 fusioniert. TPP-2203 - extrazelluläre Domäne mit N- und C-terminaler Verkürzung (34-68), fusioniert an die Fc-Domäne von hIgG1. Disulfidbrücken Cys36-Cys49, Cys52-Cys67 und Cys55-Cys64 sind durch schwarze Balken angezeigt. N-terminal erhält TPP-2203 zwei Aminosäuren und C-terminal eine Aminosäure mehr verglichen mit der reinen cysteinreichen Domäne, um eine anständige Faltung zu gewährleisten. TPP-1984 - extrazelluläre Domäne mit C-terminaler Verkürzung (28-68), an einen HIS6-Tag fusioniert. Alle drei Konstrukte zeigen eine vergleichbare Bindung an die erfindungsgemäßen Antikörper und PDL-192 (TPP-1104). P4A8 (TPP-1324) bindet nur an die Vollängen-extrazelluläre Domäne (TPP-2202).
   B -Aminosäuresequenz der extrazellulären Domäne: Es ist veröffentlicht worden, dass die Aminosäure 64 essenziell für die TWEAK-Ligandenbindung ist; und die Aminosäure 47 ist essentiell für die Bindung der erfindungsgemäßen Antikörper, wie hier bestimmt wurde.

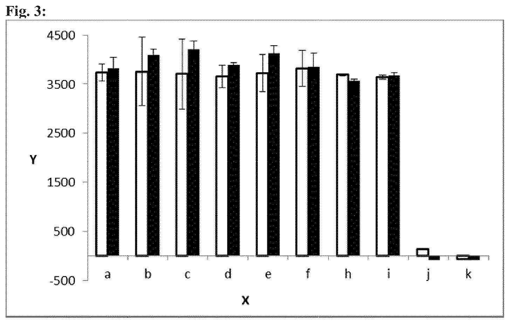

**Figur 3****:** Interaktion der TWEAKR-Ektodomäne mit Antikörpern und Referenzantikörpern. Gezeigt ist das Ergebnis eines ELISA mit TWEAKR-Fc-Fusionsproteinbeschichtung (TPP-2202, 1µg/ml) und mit 0,08 µg/ml (offene Balken) und 0,03 µ/ml (ausgefüllte Balken) biotinyliertem IgG als löslichen Bindungspartner. Der Nachweis wurde mit Streptavidin-HRP und Amplex-Red-Substrat vorgenommen. Y ist die "ELISA-Signalintensität [Rfu]"; X sind die "getesteten Antikörperkonstrukte": a ist "TPP-2090"; b ist "TPP-2084"; c ist "PDL-192(TPP-1104)"; d ist "P4A8(TPP-1324)"; e ist "P3G5(TPP-2195)"; f ist "136.1(TPP-2194)"; h ist "ITEM1"; i ist "ITEM4"; j ist eine Mausisotypkontrolle; k ist eine humane Isotypkontrolle. Alle untersuchten Antikörper zeigen eine gesättigte Bindung mit einer Konzentration von 80ng/ml.

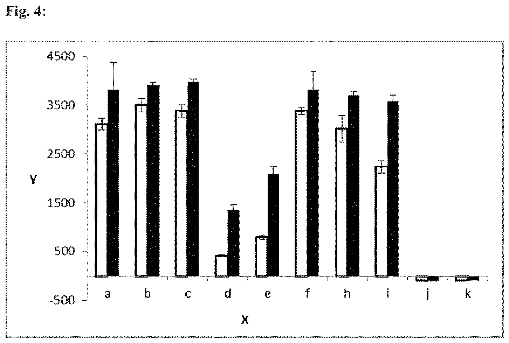

**Figur 4****:** Interaktion der cysteinreichen Domäne von TWEAKR mit erfindungsgemäßen Antikörpern und Referenzantikörpern. Gezeigt ist das Ergebnis eines ELISA mit TWEAKR (34-68)-Fc-Fusionsproteinbeschichtung (TPP-2203, 1µg/ml) und 0.08 µg/ml (offene Balken) bzw. 0,3 µ/ml (ausgefüllte Balken) biotilyniertem IgG als löslichen Bindungspartner. Der Nachweis wurde mit Streptavidin-HRP und Amplex-Red-Substrat vorgenommen. X sind die "getesteten Antikörperkonstrukte": a ist "TPP-2090"; b ist "TPP-2084"; c ist "PDL-192(TPP-1104)"; d ist "P4A8(TPP-1324)"; e ist "P3G5(TPP-2195)"; f ist "136.1(TPP-2194)"; h ist "ITEM1"; i ist "ITEM4"; j ist eine Mausisotypkontrolle; k ist eine human Isotypkontrolle. Alle untersuchten Antikörper zeigen eine gesättigte Bindung mit einer Konzentration von 80ng/ml.

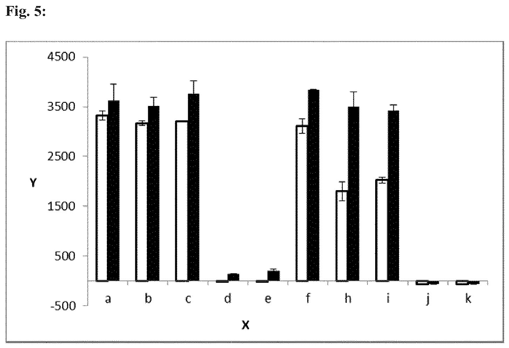

**Figur 5****:** Interaktion von TWEAKR (28-68) mit erfindungsgemäßen Antikörpern und Referenzantikörpern. Gezeigt ist das Ergebnis eines ELISA mit TWEAKR (28-68)-HIS-Beschichtung (TTP-1984, 1µg/ml) und 0.08 µg/ml (offene Balken) bzw. 0,3 µ/ml (ausgefüllte Balken) biotilyniertem IgG als löslichen Bindungspartner. Der Nachweis wurde mit Streptavidin-HRP und Amplex-Red-Substrat vorgenommen. X sind die "getesteten Antikörperkonstrukte": a ist "TPP-2090"; b ist "TPP-2084"; c ist "PDL-192(TPP-1104)"; d ist "P4A8(TPP-1324)"; e ist "P3G5(TPP-2195)"; f ist "136.1(TPP-2194)"; h ist "ITEM1"; i ist "ITEM4"; j ist eine Mausisotypkontrolle; k ist eine humane Isotypkontrolle. Alle untersuchten Antikörper zeigen eine gesättigte Bindung mit einer Konzentration von 80ng/ml.

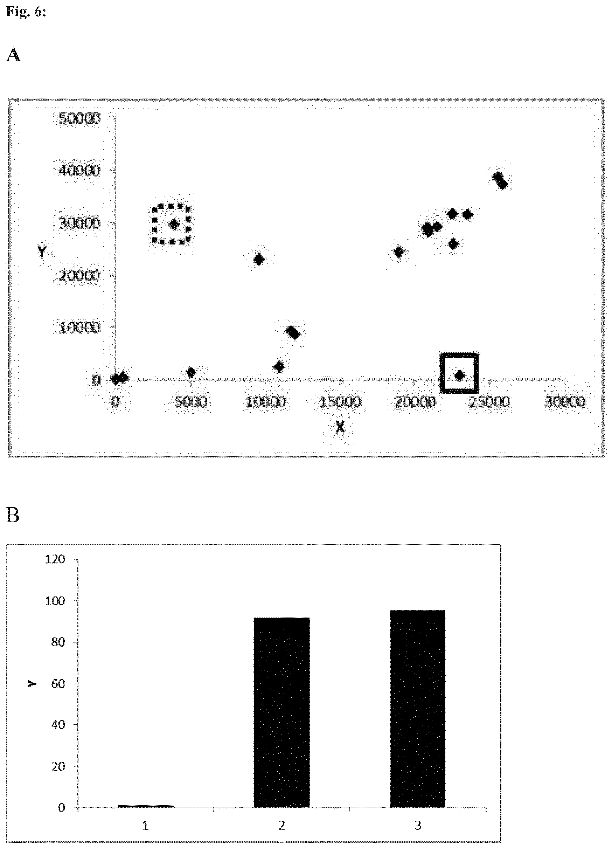

**Figur 6****:** A - Alaninscan der cysteinreichen Domäne. Muteine von TWEAKR(34-68)-Fc wurden auf PDL-192(TPP-1104) (X)- und TPP-2090 (Y)-Bindung analysiert. S37A-, R38A-, S40A-, W42A-, S43A-, D45A-, D47A-, K48A-, D51A-, S54A-, R56A-, R58A-, P59A-, H60A-, S61A-, D62A-, F63A- und L65A-Muteine wurden in HEK293-Zellen exprimiert (schwarze Rauten). PFL192(TPP-1104) und TPP-2090 wurden beschichtet (1µg/ml) und ein 8-facher verdünnter Überstand der HEK293-Fermentationsbrühe wurde für die TWEAKR-Proteinbindung hinzugegeben. X ist die "ELISA-Intensität der PDL-192/TTP-1104)-Interaktion [Rfu]", Y ist die "ELISA-Intensität der TPP-2090-Interaktion [Rfu]". TPP-2090 (Y) zeigt verschlechterte Bindung für das D74A-TWEAKR-Mutein (geschlossene Box), und PDL-192(TPP-1104) (X) zeigt eine verschlechterte Bindung an R56A (gepunktete Box).
B - Y ist die "%-Bindung normalisiert durch das Wildtypbindungssignal [%]", 1 ist "TPP-2090"; 2, ist "PDL-192(TPP-1104); 3 ist "P4A8(TPP-1324)". (1µg/ml), die TWEAKR-Variante wurde bei 250ng/ml hinzugegeben, der Nachweis fand über Anti-HIS HRP statt. TTP-2090 zeigt weniger als 5%-Bindung im Vergleich zum Wildtypkonstrukt.

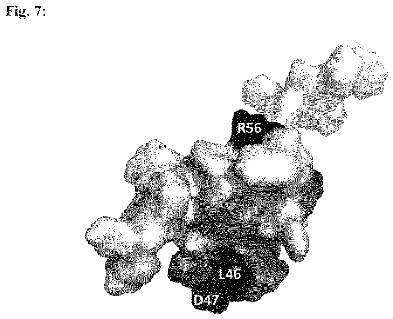

**Figur 7****:** NMR-Struktur der TWEAKR-Ektodomäne, wie von Pellegrini et al. publiziert (FEBS 280:1818-1829). Die TWEAK-Bindung hängt von L46 (Pellegrini et al.) ab, die TTP-2090- Binung hängt von D47 ab, und PDL-192 bindet an R56. PDL-192 bindet gegenüber der TWEAK-Ligandenbindungsstelle, TPP-2090 bindet direkt an die TWEAK-Ligandenstelle.

### Detaillierte Beschreibung der Erfindung

Die Erfindung stellt Konjugate eines Binders oder Derivats hiervon mit einem oder mehreren Wirkstoffmolekülen bereit, wobei das Wirkstoffmolekül ein Kinesin Spindel Protein-Inhibitor (KSP-Inhibitor) ist, das mit dem Binder über einen Linker L verbunden ist.

Das erfindungsgemäße Konjugat kann durch die allgemeine Formel 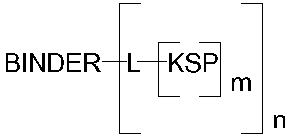 dargestellt werden, wobei BINDER für den Binder, vorzugsweise einen Antikörper, L für den Linker, KSP für den KSP-Inhibitor, m für eine Zahl von 1 bis 2, vorzugsweise 1, und n für eine Zahl von 1 bis 50, vorzugsweise 1,2 bis 20 und besonders bevorzugt 2 bis 8 steht. Hierbei ist m die Zahl der KSP-Inhibitoren pro Linker und n ein Mittelwert der Anzahl an KSP-Inhibitor-Linker-Konjugate pro BINDER. Die Summe aller KSP, die im Konjugat vorliegen, ist also das Produkt von m und n. KSP-L weist vorzugsweise die oben aufgeführte Formel (I) oder (II) auf. Vorzugsweise ist der Binder ein Binderpeptid oder -protein wie z.B. ein Antikörper. Weiterhin ist es bevorzugt, dass der Linker an verschiedene Aminosäuren des Binderpeptids oder -proteins bzw. Derivats hiervon gebunden ist. Besonders bevorzugt ist eine Bindung an unterschiedliche Cysteinreste des Binders.

Im Folgenden werden erfindungsgemäß verwendbare Binder, erfindungsgemäß verwendbare KSP-Inhibitoren sowie erfindungsgemäß verwendbare Linker beschrieben, die ohne Einschränkung in Kombination verwendet werden können. Insbesondere können die jeweils als bevorzugt bzw. besonders bevorzugt dargestellten Binder in Kombination mit den jeweils als bevorzugt bzw. besonders bevorzugt dargestellten KSP-Inhibitoren, ggf. in Kombination mit den jeweils als bevorzugt bzw. besonders bevorzugt dargestellten Linkern verwendet werden.

### KSP-Inhibitoren und deren Binderkonjugate

Niedermolekulare KSP-Inhibitoren sind z.B. aus WO2006/044825; WO2006/002236; WO2005/051922; WO2006/060737; WO03/060064; WO03/040979; und WO03/049527 bekannt.

KSP-Inhibitoren weisen regelmäßig die folgende Substruktur I(sub) auf: 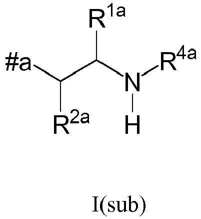 wobei
#a eine Bindung zum Restmolekül darstellt;
R^{1a} H oder -(CH₂)₀₋₃Z darstellt, wobei Z -H, Halogen, NHY³, -CO-NY¹Y², oder -CO-OY³ darstellt,
mit Y¹ und Y² unabhängig voneinander H, NH₂, -(CH₂CH₂O)₀₋₃-(CH₂)₀₋₃Z' (z.B. -(CH₂)₀₋₃Z'), oder -CH(CH₂W)Z' darstellen, und Y³ H oder -(CH₂)₀₋₃Z' darstellt, wobei Z' H, SO₃H, NH₂ COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R^{2a} und R^{4a} unabhängig voneinander H, -CO-CHY⁴-NHY⁵ oder -(CH₂)₀₋₃Z darstellen, oderR^{2a} und R^{4a} gemeinsam (unter Bildung eines Pyrrolidinringes) -CH₂-CHR¹⁰- oder -CHR¹⁰-CH₂-darstellen, wobei R¹⁰ H, NH₂, COOH, SO₃H, SH, oder OH darstellt;
wobei Z -H, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt.

Besonders häufig findet man die folgende Substruktur II(sub) 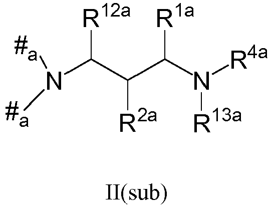
wobei #a, R^{1a}, R^{2a}, R^{4a} die selbe Bedeutung haben wie in I(sub) und R^{12a} und R^{13a} H oder R^{12a} und R^{13a} gemeinsam (unter Bildung eines Piperidinringes) -CH₂-CHR¹⁰- oder -CHR¹⁰-CH₂-darstellen, wobei R¹⁰ H, NH₂, COOH, SO₃H, SH, oder OH darstellt;
wobei Z -H, -OY³, -SY³, NHY³, -CO-NY¹Y², oder -CO-OY³ darstellt,
wobei Y¹ und Y² unabhängig voneinander H, NH₂, oder -(CH₂CH₂O)₀₋₃-(CH₂)₀₋₃Z' (z.B.-(CH₂)₀₋₃Z') darstellen, und Y³ H oder -(CH₂)₀₋₃Z' darstellt, wobei Z' H, SO₃H, NH₂, COOH oder -(CO-NH-CHY⁴)₁₋₃COOH darstellt; wobei Y⁴ unabhängig voneinander lineares oder verzweigtes, gegebenenfalls mit-NHCONH₂ substituiertes, C₁₋₆Alkyl oder gegebenenfalls mit - NH₂ substituiertes Aryl oder Benzyl darstellt.

Insbesondere weisen eine Reihe von KSP-Inhibitoren die Substruktur II(sub) auf, wobei R^{1a}, R^{2a}, R^{4a}, R^{12a} und R^{13a} H darstellen.

Erfindungsgemäß können KSP-Inhibitoren mit der Substruktur I(sub) oder der Substruktur II(sub) verwendet werden. Zu den erfindungsgemäß verwendeten KSP-Inhibitoren gehören auch z.B. Ispinesib (Cytokinetics/GSK), MK-0731 (Merck), AZD4877 (AstraZeneca), ARRY-520 (Array BioPharma) und ARQ 621 (ArQule).

Erfindungsgemäß bevorzugte KSP-Inhibitoren weisen die folgende Grundstruktur auf:
Formel (I): 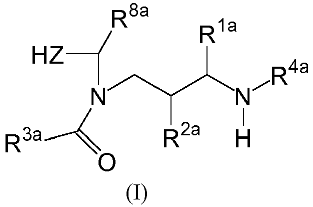 wobei
R^{1a} H, -MOD oder -(CH₂)₀₋₃Z darstellt, wobei Z -H, Halogen, -OY³, -SY³, -NHY³, -CO-NY¹Y², oder -CO-OY³ darstellt,
mit Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, SO₃H, NH₂, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R^{2a} und R^{4a} unabhängig voneinander H, -CO-CHY⁴-NHY⁵ oder -(CH₂)₀₋₃Z darstellen, oder R^{2a} und R^{4a} gemeinsam (unter Bildung eines Pyrrolidinringes) -CH₂-CHR¹⁰- oder -CHR¹⁰-CH₂-darstellen, wobei R¹⁰ H, SO₃H, NH₂, COOH, SH, oder OH darstellt;
wobei Z -H, Halogen, -OY³, -SY³, NHY³, -CO-NY¹Y², oder -CO-OY³ darstellt,
wobei Y₁ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt.
R^{3a} -MOD oder eine gegebenenfalls substituierte Alkyl-, Cycloalkyl-, Aryl-, Heteroaryl-Heteroalkyl- oder Heterocycloalkyl- Gruppe darstellt,
bevorzugt eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³,-NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder-(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet);
R^{8a} C₁₋₁₀-Alkyl darstellt;
HZ ein mono- oder bicyclischer Heterozyklus ist, der mit einem oder mehreren Substituenten ausgewählt aus Halogen, C₁₋₁₀-Alkylgruppen, C₆₋₁₀-Arylgruppen oder C₆₋₁₀-Aralkylgruppen, die ggf. mit Halogen substituiert sein können, substituiert sein kann;
wobei -MOD -(NR¹⁰)ₙ-(G1)ₒ-G2-H darstellt, wobei
   R¹⁰ H oder C₁-C₃-Alkyl darstellt;
   G1 -NHCO- , -CONH- oder 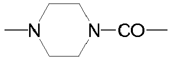 darstellt (wobei wenn G1 -NHCO- oder 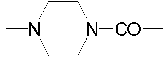 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NRY-, -NRYCO-, CONRY-, -NRYNRY-, -SO2NR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
   sowie ihre Salze, Solvate und Salze der Solvate

Erfindungsgemäß kann ein solcher Kinesin Spindel Protein-Inhibitor durch Substitution eines H-Atoms an R^{1a}, R^{2a}, R^{3a}, R^{4a}, R^{8a} oder R¹⁰ oder ggf. über einen der Substituenten von HZ, insbesondere über R^{1a}, R^{2a}, R^{3a}, R^{4a} oder R¹⁰, mit dem Linker verbunden sein.

Die Substituenten der Formel (I) haben vorzugsweise die folgenden Bedeutungen, wobei diese bevorzugten Bedeutungen vorzugsweise miteinander kombiniert werden:
R^{1a} stellt vorzugsweise H oder -(CH₂)₀₋₃Z dar, wobei Z -H, Halogen, -OY³, -SY³, NHY³, -CO-NY¹Y², oder -CO-OY³ darstellt,
mit Y¹ und Y² unabhängig voneinander H, NH₂, -(CH₂CH₂O)₀₋₃-(CH₂)₀₋₃Z' (z.B. -(CH₂)₀₋₃Z'), oder -CH(CH₂W)Z' darstellen, und Y³ H oder -(CH₂)₀₋₃Z' darstellt, wobei Z' H, SO₃H, NH₂, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt.
R^{2a} und R^{4a} stellen vorzugsweise unabhängig voneinander H, -COCHY⁴-NHY⁵ oder -(CH₂)₀₋₃Z dar, oder R^{2a} und R^{4a} gemeinsam (unter Bildung eines Pyrrolidinringes) -CH₂-CHR¹⁰- oder-CHR¹⁰-CH₂- darstellen, wobei R¹⁰ H, SO₃H, NH₂, COOH, SH, oder OH darstellt;
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt; wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt.
R^{3a} stellt vorzugsweise eine gegebenenfalls substituierte Alkyl-, Aryl-, Heteroaryl- Heteroalkyl-, Heterocycloalkyl- Gruppe dar, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet).
R^{8a} stellt vorzugsweise C₁₋₁₀-Alkyl dar.
HZ ist vorzugsweise ein mono- oder bicyclischer Heterozyklus, der mit einem oder mehreren Substituenten ausgewählt aus Halogen, C₁₋₁₀-Alkylgruppen, C₆₋₁₀-Arylgruppen oder C₆₋₁₀Aralkylgruppen, die ggf. mit Halogen substituiert sein können, substituiert sein kann.

C₁₋₁₀-Alkyl steht im Rahmen der Erfindung (d.h. in der obigen Formel wie auch in den folgenden Formeln) für einen linearen oder verzweigten Alkylrest mit 1 bis 10 Kohlenstoffatomen. Beispielhaft und vorzugsweise seien genannt: Methyl, Ethyl, *n*-Propyl, Isopropyl, *n*-Butyl, Isobutyl, 1-Methylpropyl und *tert*.-Butyl.

C₆₋₁₀-Aryl- steht im Rahmen der Erfindung für einen mono-oder bicyclischen aromatischen Homocyclus, beispielhaft Phenyl und Naphthyl.

C₆₋₁₀-Aralkyl-Gruppe steht im Rahmen der Erfindung für einen monocyclischen aromatischen Homocyclus, bespielhaft Phenyl, an den eine C1-C4-Alkylgruppe gebunden ist. Eine beispielhafte C₆₋₁₀-Aralkyl-Gruppe ist Benzyl.

C₅₋₁₀-Heteroaryl steht im Rahmen der Erfindung für einen mono-oder bicyclischen, aromatischen Heterocyclus mit insgesamt 6 bis 10 Ring-Kohlenstoffatome, wobei der bzw. die Ringe ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt Pyridyl, Furanyl, Pyrimidyl, Imidazolyl, Thienyl, Thiophenyl, Isoxazoyl, Isothiazoyl, 1,2,3-Oxadiazoyl, Furazanyl, 1,2,3-Triazoyl, 1,2,4-Triazoyl, Pyridazyl, Pyrrolyl, Triazinyl, Indolyl, Chinolinyl, Chinazolinyl, 1,3-Benzodioxol, Isoindolyl, Indazolyl, 1H-Pyrazolo[3,4-d]pyrimidyl, Benzotriazolyl, Isochinolinyl, Sinolinyl, Phthalazinyl, Pteredinyl, Naphthyridinyl, Benzimidazolinyl, Benzothiazolinyl, Benzoxazolinyl, 3,4-Methylenedioxyphenyl und Benzo[6]furanyl.

Mono- oder bicyclischer Heterozyklus steht im Rahmen der Erfindung für einen mono-oder bicyclischen Heterocyclus mit insgesamt 5 bis 10 Ring-Kohlenstoffatome, wobei der bzw. die Ringe ein bis drei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt Piperidyl, Pyrrolinyl, Morpholinyl, 3,4-Methylenedioxyphenyl und Tetrahydrofuranyl.

Halogenatom steht im Rahmen der Erfindung für F, Cl, Br, oder I.

Durch Substitution eines H-Atoms an R^{1a}, R^{2a}, R^{4a} oder R¹⁰ in Substruktur I(sub) oder Substruktur II(sub), oder R^{1a}, R^{2a}, R^{3a}, R^{4a}, R^{8a} oder R¹⁰ an HZ in Formel (I) kann in einer dem Durchschnittsfachmann bekannten Weise die Verbindung der Formel (I) mit einem Linker verbunden werden. Besonders bevorzugt erfolgt die Substitution des H-Atoms an R^{1a}, R^{2a}, R^{3a}, R^{4a}, oder an dem durch R^{2a} und R^{4a} gebildeten Pyrrolidinring. Diese Konjugation kann chemisch auf verschiedenen Wegen erfolgen, wie in den Schemata 7 bis 31 in den Beispielen exemplarisch dargelegt. Es ist insbesondere möglich, den niedermolekularen KSP-Inhibitor für die Konjugation an den Linker ggf. zu modifizieren, z.B. durch Einführen von Schutzgruppen oder Abgangsgruppen zur einfacheren Substituierung (so dass in der Reaktion diese Abgangsgruppe durch den Linker substituiert wird, und nicht ein H-Atom). Das so erhaltene KSP-Inhibitor - Linker - Molekül (wobei der Linker eine reaktive Gruppe zur Ankopplung an den Binder aufweist) kann dann mit dem Binder umgesetzt werden, um ein erfindungsgemäßes Binderkonjugat zu erhalten. Diese Vorgehensweise ist im experimentellen Teil anhand einer Vielzahl von Beispielen exemplarisch veranschaulicht.

Als R^{1a} ist bevorzugt H, -COOH, -CONHNH₂, -(CH₂)₁₋₃NH₂, -CONZ"(CH₂)₁₋₃ NH₂ und-CONZ"CH₂COOH, wobei Z" H oder NH₂ darstellt.

Als R^{2a} und R^{4a} sind bevorzugt H, oder R^{2a} und R^{4a} stellen gemeinsam (unter Bildung eines Pyrrolidinringes) CH₂-CHR¹⁰- oder -CHR¹⁰-CH₂- dar, wobei R¹⁰ H darstellt.

Als R^{3a} ist bevorzugt eine C₁₋₁₀-Alkyl-, die mit -OH, O-Alkyl, SH, S-Alkyl, O-CO-Alkyl, O-CO-NH-Alkyl, NH-CO-Alkyl, NH-CO-NH-Alkyl, S(O)ₙ-Alkyl, SO₂-NH-Alkyl, NH-Alkyl, N(Alkyl)₂, oder NH2 substituiert sein kann (wobei Alkyl vorzugsweise C1-3 Alkyl bedeutet).

Als R^{8a} ist bevorzugt eine verzweigte C₁₋₅-Alkyl-Gruppe, vorzugsweise Methyl, Ethyl, *n*-Propyl, Isopropyl, n-Butyl, Isobutyl, 1-Methylpropyl und *tert*.-Butyl*.*

Als HZ ist bevorzugt ein mono- oder bicyclischer Heterozyklus ist, der mit einem oder mehreren Substituenten ausgewählt aus Halogen, C₁₋₁₀-Alkylgruppen, C₆₋₁₀-Arylgruppen oder C₆₋₁₀-Aralkylgruppen, die ggf. mit Halogen substituiert sein können, substituiert sein kann.

Besonders bevorzugt ist HZ ein substituiertes Pyrol oder Pyrazol das in ortho-Stellung (in Bezug auf den Substituenten mit R1a etc) mit einer ggf. substituierten Benzylgruppe substituiert ist. Das substituierte Pyrol oder Pyrazol kann weiterhin vorzugsweise mit Oxo (im Falle von Dihydrochinazolin) oder einer mit 1 bis 2 Halogenatomen substituierten Phenylgruppe substituiert sein. Besonders bevorzugt ist HZ ein substituiertes Pyrrol.

Ein bevorzugt verwendeter KSP-Inhibitor ist Ispinesib. Ein weiterer bevorzugter KSP-Inhibitor ist Arry-520.

Andere besonders bevorzugte Verbindungen weisen die folgende Formel (IIa) oder (II) auf:
Formel (IIa): 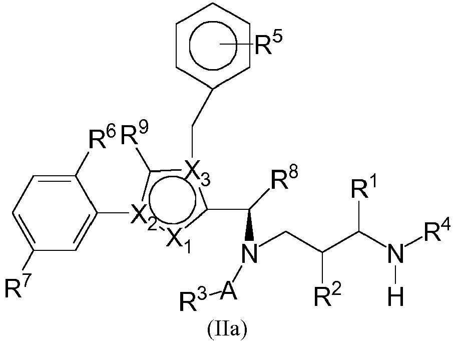 wobei
X₁ N, X₂ N und X₃ C darstellt; oder
X₁ CH oder CF, X₂ C und X₃ N darstellt; oder
X₁ NH, X₂ C und X₃ C darstellt; oder
X₁ CH oder CF, X₂ N und X₃ C darstellt;
(wobei X₁ CH, X₂ C und X₃ N bevorzugt ist);
R¹ H, -L-#1, -MOD oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² -L-#1, H, -MOD, -CO-CHY⁴-NHY⁵ oder -(CH₂)₀₋₃Z darstellt,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
R⁴ -L-#1, H, -CO-CHY⁴-NHY⁵ oder -(CH₂)₀₋₃Z darstellt,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
oder R² und R⁴ gemeinsam (unter Bildung eines Pyrrolidinringes) -CH₂-CHR¹⁰- oder -CHR¹⁰-CH₂- darstellen, wobei R¹⁰ L-#1, H, NH₂, SO₃H, COOH, SH, oder OH darstellt;
A CO, SO, SO₂, SO₂NH oder CNNH darstellt;
R³ -L-#1, -MOD oder eine gegebenenfalls substituierte Alkyl-, Cycloalkyl-, Aryl-, Heteroaryl-, Heteroalkyl-, Heterocycloalkyl- Gruppe darstellt, bevorzugt eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 -N(Alkyl)₂ Gruppen, 1-3 -NH((CH₂CH₂O)₁₋₂₀H)-Gruppen, 1-3 -NH₂ Gruppen oder 1-3 -(CH₂)₀₋₃Z Gruppen, wobei Z -H, Halogen, -OY³, -SY³,-NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder-(CH₂)₀₋₃Z' darstellt, wobei Z' H, SO₃H, NH₂ oder COOH darstellt, substituiert sein kann (wobei "Alkyl" bevorzugt C₁₋₁₀-Alkyl bedeutet);
R⁵ -L-#1, H, -MOD, NH₂, NO₂, Halogen (insbesondere F, Cl, Br), -CN, CF₃, -OCF₃, -CH₂F,-CH₂F, SH oder -(CH₂)₀₋₃Z darstellt, wobei Z -H, -OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, NO₂, NH₂, COOH oder Halogen (insbesondere F, Cl, Br) darstellen,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, (ggf. fluoriertes) C₄₋₁₀-Cycloalkyl oder -(CH₂)₀₋₂-(HZ²) darstellt, wobei HZ² ein 4 bis 7-gliedriger Heterocyclus mit bis zu zwei Heteroatomen ausgewählt aus N, O und S darstellt (vorzugsweise Oxetan), wobei jede dieser Gruppen mit -OH, CO₂H or NH₂ oder -L-#1 substituiert sein kann;
wobei einer oder keiner der Substituenten R¹, R², R³, R⁴ R⁵, R⁸ und R¹⁰ -L-#1 darstellt (bzw. im Falle von R⁸ aufweist),
L für den Linker steht und #1 für die Bindung zum Binder bzw. Derivat hiervon steht,
R⁹ H, F, CH₃, CF₃, CH₂F oder CHF₂ darstellt;
wobei -MOD -(NR¹⁰)ₙ-(G1)ₒ-G2-H darstellt, wobei
   R¹⁰ H oder C₁-C₃-Alkyl darstellt;
   G1 -NHCO- , -CONH- oder 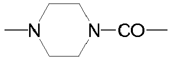 darstellt (wobei wenn G1 -NHCO- oder 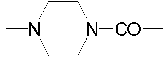 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NRY-, -NRYCO-, CONRY-, -NRYNRY-, -SO2NR^{y}NR^{y}-, -CONRYNRY-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
sowie ihre Salze, Solvate und Salze der Solvate.
   Formel (II): 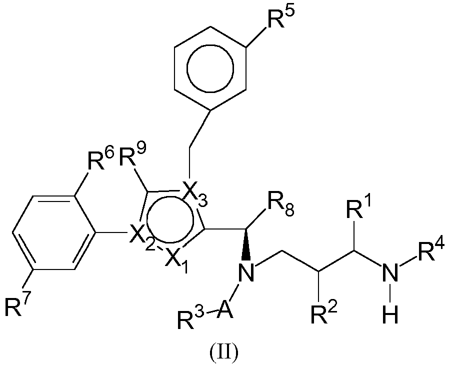 wobei
   X₁ N, X₂ N und X₃ C darstellt; oder
   X₁ CH oder CF, X₂ C und X₃ N darstellt; oder
   X₁ NH, X₂ C und X₃ C darstellt; oder
   X₁ CH, X₂ N und X₃ C darstellt;
   R¹ H, -L-#1, oder -(CH₂)₀₋₃Z darstellt, wobei Z -H, -NHY³, -OY³, -SY³, Halogen, -CO-NY¹Y², oder -CO-OY³ darstellt,
   wobei Y¹ und Y² unabhängig voneinander H, NH₂, -(CH₂CH₂O)₀₋₃-(CH₂)₀₋₃Z' (z.B. -(CH₂)₀₋₃Z'), oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt;
   wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt.
   R² und R⁴ unabhängig voneinander H, -L-#1, -CO-CHY⁴-NHY⁵ oder -(CH₂)₀₋₃Z darstellen, oder R² und R⁴ gemeinsam (unter Bildung eines Pyrrolidinringes) -CH₂-CHR¹⁰- oder -CHR¹⁰-CH₂-darstellen, wobei R¹⁰ H, -L-#1, NH₂, SO₃H, COOH, SH, oder OH darstellt;
   wobei Z -H, Halogen, -OY³, -SY³, NHY³, -CO-NY¹Y², oder -CO-OY³ darstellt,
   wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH2 oder COOH darstellt;
   wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt, wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
   A CO, SO, SO₂, SO₂NH oder CNNH darstellt;
   R³ eine gegebenenfalls substituierte Alkyl-, Aryl-, Heteroaryl- Heteroalkyl-, Heterocycloalkyl-Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen)" 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen,-OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
   substituiert sein kann (wobei "Alkyl" bevorzugt C₁₋₁₀-Alkyl bedeutet);
   R⁵ H, F, NH₂, NO₂, Halogen, SH oder -(CH₂)₀₋₃Z darstellt, wobei Z -H, Halogen, -OY³, -SY³, NHY³, -CO-NY¹Y², oder -CO-OY³ darstellt,
   wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
   R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy oder Halogen darstellen,
   R⁸ (ggf. fluoriertes) C₁₋₁₀-Alkyl, (ggf. fluoriertes) C₄₋₁₀-Cycloalkyl oder optional substituiertes Oxetan darstellt; und
   R⁹ H, F, CH₃, CF₃, CH₂F oder CHF₂ darstellt;
   sowie ihre Salze, Solvate und Salze der Solvate.

Durch Substitution eines H-Atoms an R¹, R², R³, R⁴, R⁵ oder R⁸ oder an dem durch R² und R⁴ gebildeten Pyrrolidinring (R¹⁰) kann in einer dem Durchschnittsfachmann bekannten Weise die Verbindung der Formel (IIa) oder (II), in der keiner der Substituenten R¹, R², R³, R⁴ R⁵ , R⁸ und R¹⁰ -L-#1 darstellt, mit einem Linker verbunden werden. Hierdurch werden Konjugate der Formel (IIa) oder (II) erhalten, wobei einer der Substituenten R¹, R², R³, R⁴, R⁵,R⁸ oder R¹⁰ -L-#1 darstellt, L für den Linker steht und #1 für die Bindung zum Binder bzw. Derivat hiervon steht. Wenn der KSP-Inhibitor nach Formel (IIa) oder (II) mit einem Binder konjugiert ist, stellt einer der Substituenten R¹, R², R³, R⁴ , R⁵ , R⁸ oder R¹⁰ also -L-#1 dar, wobei L für den Linker steht und #1 für die Bindung zum Binder bzw. Derivat hiervon steht. D.h. im Falle der Konjugate stellt einer der Substituenten R¹, R², R³, R⁴, R⁵, 'R⁸ und R¹⁰ -L-#1 dar, wobei-L-#1 an den Binder wie z.B. einen Antikörper darstellt. Besonders bevorzugt ist es, wenn einer der Substituenten R¹ und R³ -L-#1 darstellt. Der Binder ist vorzugsweise ein humaner, humanisierter oder chimärer monoklonaler Antikörper oder ein Antigen-bindendes Fragment hiervon ist, insbesondere ein anti-TWEAKR-Antikörper oder ein Antigen-bindendes Fragment hiervon oder ein anti-EGFR-Antikörper oder ein Antigen-bindendes Fragment hiervon. Besonders bevorzugt sind ein anti-TWEAKR-Antikörper, der spezifisch an die Aminosäure D in Position 47 (D47) von TWEAKR (SEQ ID NO:169) bindet, insbesondere der anti-TWEAKR-Antikörper TPP-2090, oder die anti-EGFR-Antikörper Cetuximab oder Nimotuzumab.

Anstelle von -L-#1 kann auch die Gruppe -L-#3 in der Verbindung vorhanden sein, wobei L für den Linker steht und #3 für die reaktive Gruppe zur Bindung an den Binder bzw. Derivat hiervon steht. Verbindungen mit -L-#3 sind reaktive Verbindungen, die mit dem Binder bzw. Derivat hiervon reagieren. #3 ist vorzugsweise eine Gruppe, die mit einer Amino- oder Thiolgruppe unter Bildung einer kovalenten Bindung reagiert, vorzugsweise mit dem Cysteinrest in einem Protein. Der Cysteinrest in einem Protein kann natürlich im Protein vorliegen, durch biochemische Methoden eingebracht werden oder vorzugsweise durch vorherige Reduktion von Disulfiden des Binders generiert werden kann.

Die Verbindungen der Formel (IIa) oder (II), in der einer der Substituenten R¹, R², R³, R⁴, R⁵ und R¹⁰-L-#1 darstellt, und in denen
X₁ N, X₂ N und X₃ C darstellt;
X₁ CH oder CF, X₂ C und X₃ N darstellt;
X₁ NH, X₂ C und X₃ C darstellt; oder
X₁ CH, X₂ N und X₃ C darstellt;
sind besonders bevorzugt,
insbesondere solche, in denen
X₁ N, X₂ N und X₃ C darstellt; oder X₁ CH, X₂ C und X₃ N darstellt. Insbesondere sind Verbindungen bevorzugt, in denen X₁ CH, X₂ C und X₃ N darstellt.

Als A ist CO (carbonyl) bevorzugt.

Als R¹ ist bevorzugt -L-#1, H, -COOH, -CONHNH₂, -(CH₂)₁₋₃NH₂, -CONZ"(CH₂)₁₋₃ NH₂ und-CONZ"CH₂COOH, wobei Z" H oder NH₂.

Als R² und R⁴ sind bevorzugt H, -L-#1 oder R² und R⁴ stellen gemeinsam (unter Bildung eines Pyrrolidinringes) -CH2-CHR¹⁰- oder -CHR¹⁰-CH₂- darstellen, wobei R¹⁰ H oder -L-#1, darstellt.

Als R³ ist bevorzugt -L-#1 oder eine C₁₋₁₀-Alkyl-, die ggf. mit -OH, O-Alkyl, SH, S-Alkyl, O-CO-Alkyl, O-CO-NH-Alkyl, NH-CO-Alkyl, NH-CO-NH-Alkyl, S(O)ₙ-Alkyl, SO₂-NH-Alkyl, NH-Alkyl, N(Alkyl)₂, oder NH₂ substituiert sein kann (wobei Alkyl vorzugsweise C₁₋₃ Alkyl bedeutet).

Als R⁵ ist bevorzugt -L-#1, H oder F.

Als R⁶ und R⁷ sind unabhängig voneinander bevorzugt H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen.

Als R⁸ ist bevorzugt eine verzweigte C₁₋₅-Alkyl-Gruppe, insbesondere eine Gruppe der Formel-C(CH₃)₂-(CH₂)₀₋₂ -R_{y}, wobei R_{y} -H, -OH, CO₂H, NH₂ oder -L-#1. Besonders bevorzugt ist Gruppe der Formel -C(CH₃)₂-(CH₂) -R_{y}, wobei R_{y} -H oder -L-#1. ist
Als R⁹ ist bevorzugt H oder F.

Insbesondere bevorzugt sind Verbindungen der Formel (IIa) oder (II), in der keiner oder einer der Substituenten R¹, R², R³, R⁴, R⁵, R⁸ und R¹⁰ -L-#1 darstellt, und
in denen
X₁ N, X₂ N und X₃ C darstellt;
X₁ CH oder CF, X₂ C und X₃ N darstellt;
X₁ NH, X₂ C und X₃ C darstellt; oder
X₁ CH, X₂ N und X₃ C darstellt;
A CO (carbonyl) ist;
R¹ H, -COOH, -CONHNH₂, -(CH₂)₁₋₃NH₂, -CONZ"(CH₂)₁₋₃ NH₂ und -CONZ"CH₂COOH ist, wobei Z" H oder NH₂;
R² und R⁴ H ist, oder R² und R⁴ gemeinsam (unter Bildung eines Pyrrolidinringes) -CH₂-CHR¹⁰- oder-CHR¹⁰-CH2- darstellen, wobei R¹⁰ H oder -L-#1, darstellt;
R³ eine Phenylgruppe, die ein oder mehrfach mit Halogen (insbesondere F) oder ggf. fluoriertes C₁₋₃ Alkyl substituiert sein kann, oder eine ggf. fluorierte C₁₋₁₀-Alkylgruppe darstellt, die ggf. mit -OY⁴,-SY⁴, -O-CO-Y⁴, -O-CO-NH-Y⁴, NH-CO-Y⁴, -NH-CO-NH-Y⁴, S(O)ₙ-Y⁴(wobei n 0, 1 oder 2 darstellt), -SO₂-NH-Y⁴, NH-Y⁴, oder N(Y⁴)₂, substituiert sein kann, wobei Y4 H, Phenyl (ggf. ein oder mehrfach mit Halogen (insbesondere F) oder ggf. fluoriertes C₁₋₃ Alkyl substituiert), oder Alkyl (wobei die Alkylgruppe mit -OH, -COOH, und/oder -NHCO-C₁₋₃-Alkyl substituiert sein kann, und wobei Alkyl vorzugsweise C₁₋₃ Alkyl bedeutet) darstellt;
wobei iinsbesondere bevorzugt R³mit -OH, O-Alkyl, SH, S-Alkyl, O-CO-Alkyl, O-CO-NH-Alkyl, NH-CO-Alkyl, NH-CO-NH-Alkyl, S(O)ₙ-Alkyl, SO₂-NH-Alkyl, NH-Alkyl, N(Alkyl)₂, oder NH₂ substituiert sein kann (wobei Alkyl vorzugsweise C₁₋₃ Alkyl bedeutet)
R⁵ H oder F ist;
R⁶ und R⁷ unabhängig voneinander H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist; und
R⁹ H oder F ist.

Weiterhin sind bevorzugt (allein oder in Kombination), wenn
- R¹ -L-#1, COOH oder H darstellt,
- R² und R⁴ unabhängig voneinander -L-#1 oder H oder R² und R⁴ gemeinsam (unter Bildung eines Pyrrolidinringes) -CH₂-CHR¹⁰- oder -CHR¹⁰-CH₂- darstellen, wobei R¹⁰ H oder -L-#1, darstellt,
- A CO darstellt,
- R³ -(CH₂)OH, -CH(CH₃)OH, -CH₂SCH₂CH(COOH)NHCOCH₃, -CH(CH₃)OCH₃, eine Phenylgruppe, die mit 1-3 Halogenatomen, 1-3 Aminogruppen oder 1-3 Alkylgruppen (die ggf. halogeniert sein können, substituiert sein kann, oder -L-#1 darstellt,
- R⁵ -L-#1 oder H darstellt,
- R⁶ und R⁷ unabhängig voneinander H, C₁₋₃-Alkyl oder Halogen darstellen, insbesondere dass R⁶ und R⁷ F darstellen;
- R⁸ C₁₋₄-Alkyl (vorzugsweise tert-butyl) darstellt; und/ oder
- R⁹ H darstellt,
- wobei einer der Substituenten R¹, R², R³, R⁴, R⁵ und R¹⁰ -L-#1 darstellt.

Zudem ist es erfindungsgemäß bevorzugt, wenn
- R¹ -L-#1, COOH oder H darstellt,
- R² und R⁴ unabhängig voneinander -L-#1 oder H oder R² und R⁴ gemeinsam (unter Bildung eines Pyrrolidinringes) -CH₂-CHR¹⁰- oder -CHR¹⁰-CH₂- darstellen, wobei R¹⁰ H oder -L-#1, darstellt,
- A CO darstellt,
- R³ -(CH₂)OH, -CH(CH₃)OH, -CH₂SCH₂CH(COOH)NHCOCH₃, -CH(CH₃)OCH₃, eine Phenylgruppe, die mit 1-3 Halogenatomen, 1-3 Aminogruppen oder 1-3 Alkylgruppen (die ggf. halogeniert sein können, substituiert sein kann, oder -L-#1 darstellt,
- R⁵ -L-#1 oder H darstellt,
- R⁶ und R⁷ unabhängig voneinander H, C₁₋₃-Alkyl oder Halogen darstellen, insbesondere dass R⁶ und R⁷ F darstellen;
- R⁸ C₁₋₄-Alkyl (vorzugsweise tert-butyl) darstellt; und
- R⁹ H darstellt,
- wobei einer der Substituenten R¹, R², R³, R⁴, R⁵ und R¹⁰ -L-#1 darstellt.

Andere besonders bevorzugte Verbindungen weisen die folgende Formel (IIIa) oder (III) auf:
Formel (IIIa): 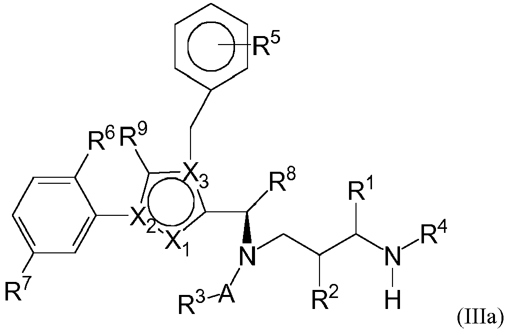 wobei
X₁ N, X₂ N und X₃ C darstellt; oder
X₁ CH oder CF, X₂ C und X₃ N darstellt; oder
X₁ NH, X₂ C und X₃ C darstellt; oder
X₁ CH, X₂ N und X₃ C darstellt;
(wobei X₁ CH, X₂ C und X₃ N bevorzugt ist);
R¹ H, -L-Binder, -MOD oder -(CH₂)₀₋₃Z darstellt, wobei Z -H, -NHY³, -OY³, -SY³, Halogen,-CO-NY¹Y², oder -CO-OY³ darstellt,
wobei Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, NH₂, SO₃H,-COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² H, -L-Binder, -MOD, -CO-CHY⁴-NHY⁵ oder -(CH₂)₀₋₃Z darstellt, wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆Alkyl darstellt,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
R⁴ H, -L-Binder, -CO-CHY⁴-NHY⁵ oder -(CH₂)₀₋₃Z darstellt,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
oder R² und R⁴ gemeinsam (unter Bildung eines Pyrrolidinringes) -CH₂-CHR¹⁰- oder -CHR¹⁰-CH₂- darstellen, wobei R¹⁰ -L-Binder, H, NH₂, SO₃H, COOH, SH, oder OH darstellt;
A CO, SO, SO₂, SO₂NH oder CNNH darstellt;
R³ -L-Binder, -MOD, oder eine gegebenenfalls substituierte Alkyl-, Cycloalkyl-, Aryl-, Heteroaryl- Heteroalkyl-, Heterocycloalkyl- Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3-SO₂-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder-CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet);
R⁵ -L-Binder, H, NH₂, NO₂, Halogen (insbesondere F, Cl, Br), -CN, CF₃, -OCF₃, -CH₂F, -CH₂F, SH oder -(CH₂)₀₋₃Z darstellt, wobei Z -H, -OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
darstellt,
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, NO₂, NH₂, COOH oder Halogen (insbesondere F, Cl, Br) darstellen,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, (ggf. fluoriertes) C₄₋₁₀-Cycloalkyl oder-(CH₂)₀₋₂-(HZ²) darstellt, wobei HZ² ein 4 bis 7-gliedriger Heterocyclus mit bis zu zwei Heteroatomen ausgewählt aus N, O und S darstellt, wobei jede dieser Gruppen mit -OH, CO₂H or NH₂ oder -L-BINDER substituiert sein kann;
R⁹ H, F, CH₃, CF₃, CH₂F oder CHF₂ darstellt;
wobei -MOD -(NR¹⁰)ₙ-(G1)ₒ-G2-H darstellt, wobei
   R¹⁰ H oder C₁-C₃-Alkyl darstellt;
   G1 -NHCO- , -CONH- oder 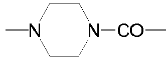 darstellt (wobei wenn G1 -NHCO- oder 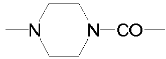 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NRY-, -NRYCO-, CONRY-, -NRYNRY-, -SO2NR^{y}NR^{y}-, -CONRYNRY-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
sowie ihre Salze, Solvate und Salze der Solvate.

Im Falle von Binderkonjugaten der KSP-Inhibitoren der Formel (IIIa) kann maximal ein Vertreter von R¹, R², R³ R⁴, R⁵, R⁸ und R¹⁰ (alternativ zu einer der oben angegebenen Bedeutungen) -L-Binder darstellen, wobei L für einen Linker steht und BINDER für Binder bzw. Derivat hiervon steht, wobei der Binder ggf. an mehrere Wirkstoffmoleküle gebunden sein kann.
Formel (III): 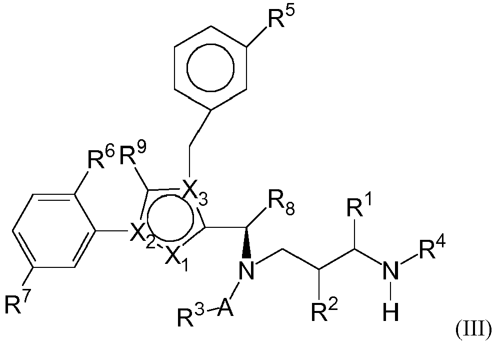 wobei
X₁ N, X₂ N und X₃ C darstellt, oder X₁ CH, X₂ C und X₃ N darstellt oder X₁ NH, X₂ C und X₃ C darstellt, oder X₁ CH, X₂ N und X₃ C darstellt;
R1 -L-BINDER, H, oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander H, NH2, -(CH₂CH₂O)₀₋₃-(CH₂)₀₋₃Z' oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² und R⁴ unabhängig voneinander -L-BINDER, H, -CO-CHY⁴-NHY⁵ oder -(CH₂)₀₋₃Z darstellen, oder R² und R⁴ gemeinsam (unter Bildung eines Pyrrolidinringes) -CH₂-CHR¹⁰- oder -CHR¹⁰-CH₂-darstellen, wobei R¹⁰ L-#1, H, NH₂, SO₃H, COOH, SH, oder OH darstellt;
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder -(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
A CO, SO, SO₂, SO₂NH oder CNNH darstellt;
R³ -L-BINDER oder eine gegebenenfalls substituierte Alkyl-, Aryl-, Heteroaryl- Heteroalkyl-, Heterocycloalkyl- Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen)" 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 - N(Alkyl)₂ Gruppen, 1-3-NH₂ Gruppen oder 1-3-(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder - (CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt, substituiert sein kann (wobei "Alkyl" bevorzugt C₁₋₁₀-Alkyl bedeutet);
R⁵ -L-BINDER, H, F, NH₂, NO₂, Halogen, SH oder -(CH₂)₀₋₃Z darstellt, wobei Z -H, Halogen, -OY³, - SY³, -NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder -(CH₂)₀₋₃Z' darstellt, wobei Z' H, SO₃H, NH₂ oder COOH darstellt;
wobei L für einen Linker steht und BINDER für Binder bzw. Derivat hiervon steht, wobei der Binder ggf. an mehrere Wirkstoffmoleküle gebunden sein kann,
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy oder Halogen darstellen,
R⁸ (ggf. fluoriertes) C₁₋₁₀-Alkyl, (ggf. fluoriertes) C₄₋₁₀-Cycloalkyl oder optional substituiertes Oxetan darstellt; und
R⁹ H, F, CH₃, CF₃, CH₂F oder CHF₂ darstellt;
sowie ihre Salze, Solvate und Salze der Solvate.

Weiterhin sind erfindungsgemäß bevorzugt folgende KSP-Inhibitoren bzw. deren Binderkonjugate:
Formel (IIIb): 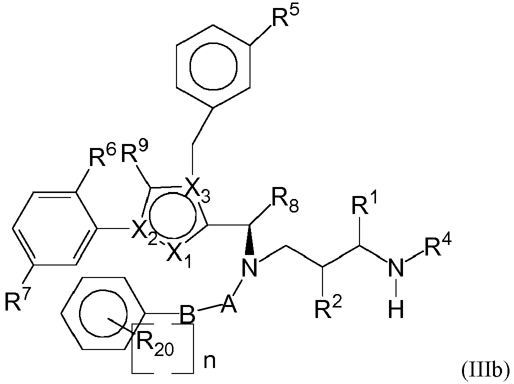 wobei X₁, X₂, X₃ die gleiche Bedeutung wie in Formel (IIIa) oder (III) aufweisen (vorzugsweise mit X₁ CH, X₂ C und X₃ N), R¹, R², R⁴, R⁵, R⁶, R⁷, R⁸ und R⁹ die gleiche Bedeutung wie in Formel (IIIa) oder (III) aufweisen, A CO darstellt, B eine Einfachbindung, -O-CH₂- oder -CH₂-O- darstellt, und R²⁰ NH₂, F, CF3, oder CH₃, und n 0, 1, oder 2 darstellt.

Formel (IIIc): 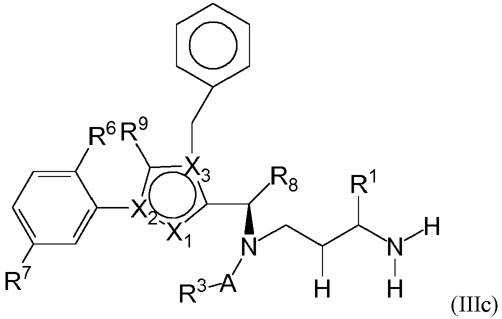 wobei X₁, X₂, X₃ die gleiche Bedeutung wie in Formel (IIIa) oder (III) aufweisen (vorzugsweise mit X₁ CH, X₂ C und X₃ N), A, R¹, R³, R⁶, R⁷, R⁸, und R⁹ die gleiche Bedeutung wie in Formel (IIIa) oder (III) aufweisen, wobei A vorzugsweise CO und R³ -CH₂OH, -CH₂OCH₃, CH(CH3)OH oder CH(CH₃)OCH₃ darstellt.

Formel (IIId): 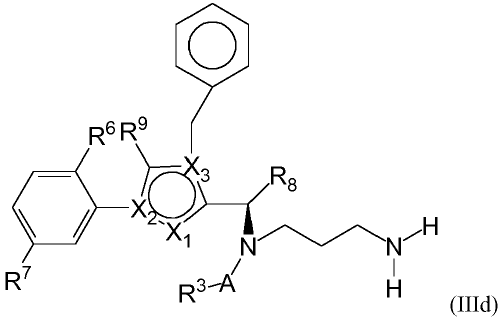 wobei X₁, X₂, X₃ die gleiche Bedeutung wie in Formel (IIIa) oder (III) aufweisen (vorzugsweise mit X₁ CH, X₂ C und X₃ N), A, R³, R⁶, R⁷, R⁸ und R⁹ die gleiche Bedeutung wie in Formel (IIIa) oder (III) aufweisen, wobei A vorzugsweise CO und R³ -CH₂-Sₓ-(CH₂)₀₋₄-CHY⁵-COOH auf, wobei x 0 oder 1 ist, und Y⁵ H oder NHY⁶ darstellt, wobei Y⁶ H oder -COCH₃ darstellt.

Formel (IIIe): 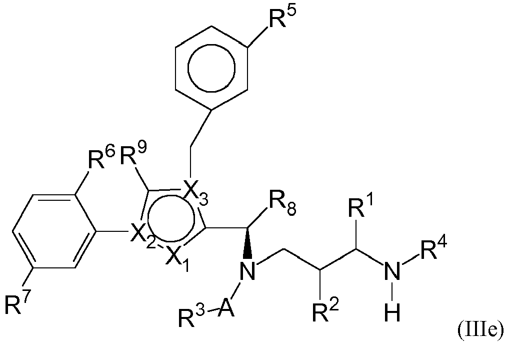 wobei X₁ CH, X₂ C und X₃ N, A, R³, R⁶, R⁷, R⁸ und R⁹ die gleiche Bedeutung wie in Formel (IIIa) oder (III) aufweisen, und R¹ -L-BINDER darstellt.

Weiterhin sind bevorzugt in den Verbindungen der Formeln (III), (IIIa), (IIIb), (IIIc), (IIId) und (IIIe) (allein oder in Kombination), wenn:
- Z Cl oder Br darstellt;
- R¹ -(CH₂)₀₋₃Z darstellt, wobei Z -CO-NY¹Y² darstellt, wobei Y²-(CH₂CH₂O)₀₋₃-(CH₂)₀₋₃Z' und Y¹ H, NH2, oder -(CH₂CH₂O)₀₋₃-(CH₂)₀₋₃Z' darstellt;
- Y¹ H darstellt, Y²-(CH₂CH₂O)₃-CH₂CH₂Z' darstellt und Z'-COOH darstellt;
- Y¹ H darstellt, Y²-CH₂CH₂Z' darstellt und Z'-(CONHCHY⁴)₂COOH stellt;
- Y¹ H darstellt, Y² -CH₂CH₂Z' darstellt, Z'-(CONHCHY⁴)₂COOH stellt und eine Y⁴ *i*-propyl darstellt und der andere ein -(CH₂)₃-NHCONH₂ darstellt;
- Y¹ H darstellt, Y² -CH₂CH₂Z' darstellt, Z'-(CONHCHY⁴)₂COOH stellt und eine Y⁴-CH₃ darstellt und der andere ein -(CH₂)₃-NHCONH₂ darstellt;
- Y⁴ lineares oder verzweigtes, gegebenenfalls mit -NHCONH₂ substituiertes, C₁₋₆ Alkyl darstellt;
- mindestens ein Vertreter von Y⁴ ausgewählt wird aus *i*-propyl oder -CH₃.
- Y¹ H darstellt, Y² -CH₂CH₂Z' darstellt, Z'-CONHCHY⁴COOH darstellt und Y⁴ gegebenenfalls mit -NH₂ substituiertes Aryl oder Benzyl darstellt;
- Y⁴ Aminobenzyl darstellt;
- R²-(CH₂)₀₋₃Z darstellt und Z-SY³ darstellt;
- R⁴-CO-CHY⁴-NHY⁵ darstellt und Y⁵ H darstellt;
- R⁴-CO-CHY⁴-NHY⁵ darstellt und Y⁵-CO-CHY⁶-NH₂ darstellt;
- Y⁴ lineares oder verzweigtes, gegebenenfalls substituiertes mit -NHCONH₂ C₁₋₆ Alkyl darstellt.

Weiterhin ist es bevorzugt, wenn R¹, R² oder R³ in Formel (IIa) oder (IIIa) -MOD darstellt, insbesondere wenn R⁴-L-#1 bzw. -L-BINDER darstellt (insbesondere wenn -L ein spaltbarer Linker ist, der direkt an -N-R⁴ bzw. -N-L-#1 bzw. -L-BINDER spaltet, so dass R⁴ bzw. L durch H ersetzt wird).

Besonders bevorzugt stellt R³ -MOD und R¹ oder R⁴-L-#1 bzw. -L-BINDER dar,
wobei -MOD -(NR¹⁰)ₙ-(G1)ₒ-G2-H darstellt, wobei
R¹⁰ H oder C₁-C₃-Alkyl darstellt;
G1 -NHCO- , -CONH- oder 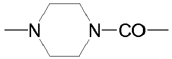 darstellt (wobei wenn G1 -NHCO- oder 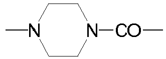 darstellt, R¹⁰ nicht NH₂ ist);
n 0 oder 1 ist;
o 0 oder 1 ist; und
G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NRY-, -NRYCO-, CONRY-, -NRYNRY-, -SO2NR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;

Besonders bevorzugt weist die Gruppe -MOD eine (vorzugsweise terminale) -COOH-Gruppe auf, z.B. in einer Betaingruppe. Vorzugsweise weist die Gruppe -MOD die Formel -CH₂-Sₓ-(CH₂)₀₋₄-CHY⁵-COOH auf, wobei x 0 oder 1 ist, und Y⁵ H oder NHY⁶ darstellt, wobei Y6 H oder -COCH₃ darstellt.

Weiterhin sind bevorzugt (allein oder in Kombination), wenn in Formel (IIa), (II), (III), (IIIa), (IIIb), (IIIc), (IIId) oder (IIIe):
- Z Cl oder Br darstellt;
- R¹ -(CH₂)₀₋₃Z darstellt, wobei Z-CO-NY¹Y² darstellt, wobei Y²-(CH₂CH₂O)₀₋₃-(CH₂)₀₋₃Z' und Y¹ H, NH2, oder -(CH₂CH₂O)₀₋₃-(CH₂)₀₋₃Z' darstellt;
- Y¹ H darstellt, Y²-(CH₂CH₂O)₃-CH₂CH₂Z' darstellt und Z'-COOH darstellt;
- Y¹ H darstellt, Y² -CH₂CH₂Z' darstellt und Z'-(CONHCHY⁴)₂COOH stellt;
- Y¹ H darstellt, Y²-CH₂CH₂Z' darstellt, Z'-(CONHCHY⁴)₂COOH stellt und ein Vertreter von Y⁴ *i*-propyl darstellt und der andere -(CH₂)₃-NHCONH₂ darstellt;
- Y¹ H darstellt, Y² -CH₂CH₂Z' darstellt, Z'-(CONHCHY⁴)₂COOH stellt und ein Vertreter von Y⁴ -CH₃ darstellt und der andere -(CH₂)₃-NHCONH₂ darstellt;
- Y⁴ lineares oder verzweigtes, gegebenenfalls mit -NHCONH₂ substituiertes, C₁₋₆ Alkyl darstellt;
- mindestens ein Vertreter von Y⁴ ausgewählt wird aus *i*-propyl or -CH₃.
- Y¹ H darstellt, Y²-CH₂CH₂Z' darstellt, Z'-CONHCHY⁴COOH darstellt und Y⁴ gegebenenfalls mit -NH₂ substituiertes Aryl oder Benzyl darstellt;
- Y⁴ eine Aminobenzyl darstellt;
- R²-(CH₂)₀₋₃Z darstellt und Z-SY³ darstellt;
- R⁴-CO-CHY⁴-NHY⁵ darstellt und Y⁵ H darstellt;
- R⁴-CO-CHY⁴-NHY⁵ darstellt und Y⁵-CO-CHY⁶-NH₂ darstellt;
- Y⁴ lineares oder verzweigtes gegebenenfalls substituiertes mit -NHCONH₂ C₁₋₆ Alkyl darstellt.

Weiterhin sind bevorzugt Verbindungen der Formel (IIa), (II), (III) oder (IIIa),
wobei
X₁ N, X₂ N und X₃ C darstellt; oder
X₁ CH oder CF, X₂ C und X₃ N darstellt; oder
X₁ NH, X₂ C und X₃ C darstellt; oder
X₁ CH oder CF, X₂ N und X₃ C darstellt;
(wobei X₁ CH, X₂ C und X₃ N bevorzugt ist);
R¹ H, -L-#1 bzw. -L-BINDER, -MOD oder -(CH₂)₀₋₃Z darstellt, wobei Z -H, -NHY³, -OY³, - SY³, Halogen, -CO-NY¹Y², oder -CO-OY³ darstellt,
wobei Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² H, -CO-CHY⁴-NHY⁵ oder -(CH₂)₀₋₃Z darstellt,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder -
(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
R⁴ H darstellt;
A CO, SO, SO₂, SO₂NH oder CNNH darstellt;
R³ -L-#1 bzw. -L-BINDER, -MOD oder eine gegebenenfalls substituierte Alkyl-, Cycloalkyl-, Aryl-, Heteroaryl-, Heteroalkyl-, Heterocycloalkyl- Gruppe darstellt, bevorzugt eine C₁₋₁₀-Alkyl-, C₆₋₁₀-Aryl- oder C₆₋₁₀-Aralkyl-, C₅₋₁₀-Heteroalkyl-, C₁₋₁₀-Alk-yl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 - SO₂-NH-Alkyl Gruppen, 1-3 -NH-Alkyl Gruppen, 1-3 -N(Alkyl)₂ Gruppen, 1-3 - NH((CH₂CH₂O)₁₋₂₀H)-Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt, substituiert sein kann (wobei "Alkyl" bevorzugt C₁₋₁₀-Alkyl bedeutet);
R⁵ H, -MOD, NH₂, NO₂, Halogen (insbesondere F, Cl, Br), -CN, CF₃, -OCF₃, -CH₂F, -CH₂F, SH oder -(CH₂)₀₋₃Z darstellt, wobei Z -H, -OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt;
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, NO₂, NH₂, COOH oder Halogen (insbesondere F, Cl, Br) darstellen,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, oder (ggf. fluoriertes) C₄₋₁₀-Cycloalkyl;
wobei einer oder keiner der Substituenten R¹ und R³-L-#1 -L-#1 bzw. -L-BINDER darstellt,
L für den Linker steht und #1 für die Bindung zum Binder bzw. Derivat hiervon und BINDER für den Binder steht,
R⁹ H, F, CH₃, CF₃, CH₂F oder CHF₂ darstellt;
wobei -MOD -(NR¹⁰)ₙ-(G1)ₒ-G2-H darstellt, wobei
   R¹⁰ H oder C₁-C₃-Alkyl darstellt;
   G1-NHCO- , -CONH- oder 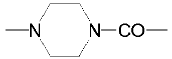 darstellt (wobei wenn G1 -NHCO- oder 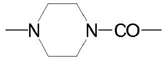 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NR^{y}-, -NR^{y}CO-, CONR^{y}-, -NR^{y}NR^{y}-, -SO2NR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
sowie ihre Salze, Solvate und Salze der Solvate.

Weiterhin sind bevorzugt Verbindungen der Formel Formel (IIa), (II), (III) oder (IIIa), in denen
X₁ N, X₂ N und X₃ C darstellt; oder
X₁ CH oder CF, X₂ C und X₃ N darstellt; oder
X₁ NH, X₂ C und X₃ C darstellt; oder
X₁ CH oder CF, X₂ N und X₃ C darstellt;
(wobei X₁ CH, X₂ C und X₃ N bevorzugt ist);
R¹ H, -L-#1 bzw. -L-BINDER, -MOD oder -(CH₂)₀₋₃Z darstellt, wobei Z -H, -NHY³, -OY³, - SY³, Halogen, -CO-NY¹Y², oder -CO-OY³ darstellt,
wobei Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² H, -CO-CHY⁴-NHY⁵ oder -(CH₂)₀₋₃Z darstellt,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder -
(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
R⁴ H darstellt,
A CO, SO, SO₂, SO₂NH oder CNNH darstellt;
R³ -L-#1 bzw. -L-BINDER, -MOD oder eine gegebenenfalls substituierte Alkyl-, Cycloalkyl-, Aryl-, Heteroaryl-, Heteroalkyl-, Heterocycloalkyl- Gruppe darstellt, bevorzugt eine C₁₋₁₀-Alkyl-, C₆₋₁₀-Aryl- oder C₆₋₁₀-Aralkyl-, C₅₋₁₀-Heteroalkyl-, C₁₋₁₀-Alk-yl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 - SO₂-NH-Alkyl Gruppen, 1-3 -NH-Alkyl Gruppen, 1-3 -N(Alkyl)₂ Gruppen, 1-3 - NH((CH₂CH₂O)₁₋₂₀H)-Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z'-(CH₂)₀₋₃-CH(NH₂)Z', oder -(CH₂)₀₋₃Z' darstellt, wobei Z' H, SO₃H, NH₂ oder COOH darstellt, substituiert sein kann (wobei "Alkyl" bevorzugt C₁₋₁₀-Alkyl bedeutet);
R⁵ H, -MOD, NH₂, NO₂, Halogen (insbesondere F, Cl, Br), -CN, CF₃, -OCF₃, -CH₂F, -CH₂F, SH oder -(CH₂)₀₋₃Z darstellt, wobei Z -H, -OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt;
R⁶ und R⁷ unabhängig voneinander H oder Halogen (insbesondere F, Cl, Br) darstellen,
R⁸ (ggf. fluoriertes) C₁₋₁₀-Alkyl darstellt;
wobei einer oder keiner der Substituenten R¹ und R³-L-#1 bzw. -L-BINDER darstellt,
L für den Linker steht und #1 für die Bindung zum Binder bzw. Derivat hiervon und BINDER für den Binder steht,
R⁹ H, F, CH₃, CF₃, CH₂F oder CHF₂ darstellt;
wobei -MOD -CH₂-Sₓ-(CH₂)₀₋₄-CHY⁵-COOH auf, wobei x 0 oder 1 ist, und Y⁵ H oder NHY⁶ darstellt, wobei Y6 H oder -COCH₃ darstellt,
sowie ihre Salze, Solvate und Salze der Solvate.

Weiterhin sind die folgende Verbindungen bevorzugt, die ggf. gemeinsam mit einer Säure wie z.B. Trifluoressigsäure vorliegen können. Diese Verbindungen können über die Positionen entsprechend den Positionen R¹, R², R³ R⁴, R⁵, R⁸ und R¹⁰:, insbesondere R¹ und R³, über einen Linker an einen Binder gebunden werden (wobei ein Wasserstoffatom durch den Linker substituiert wird):
N-(3-Aminopropyl)-N-{(1R)-1-[4-benzyl-1-(2,5-difluorphenyl)-1H-pyrazol-3-yl]-2,2-dimethylpropyl}-2-hydroxyacetamid;
N-(3-Aminopropyl)-N-{(1R)-1-[1-benzyl-4-(2,5-difluorphenyl)-1H-pyrrol-2-yl]-2,2-dimethylpropyl}-2-hydroxyacetamid;
(2S)-2-amino-4-[{(1R)-1-[1-benzyl-4-(2,5-difluorphenyl)-1H-pyrrol-2-yl]-2,2-dimethylpropyl}(glycoloyl)amino]-N-methylbutanamid(1:1);
N-(3-Aminopropyl)-N-{(1S)-1-[1-benzyl-4-(2,5-difluorphenyl)-1H-pyrrol-2yl]-2,2-dimethylpropyl} acetamid.

N-(3-Aminopropyl)-N-{(1S)-1-[1-benzyl-4-(2,5-difluorphenyl)-1H-pyrrol-2-yl]-2,2-dimethylpropyl}-2-hydroxyacetamid;
S-[1-(2-{[2-({(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}amino)ethyl]amino}-2-oxoethyl)-2,5-dioxopyrrolidin-3-yl]-L-cystein:
N-{(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}-beta-alanyl-L-alanyl-N-[4-(3-{[(2R)-2-amino-2-carboxyethyl]sulfanyl}-2,5-dioxopyrrolidin-1-yl)phenyl]-N⁵-carbamoyl-L-ornithinamid;
S-(1-{2-[(N-{(2S)-2-Amino-4-[-{(1R)-1-[1-benzyl-4-(2,5-difluorphenyl)-1H-pyrrol-2-yl]-2,2 dimethylpropyl}(glycoloyl)amino]butanoyl}-beta-alanyl)amino]ethyl}-2,5-dioxopyrrolidin-3-yl)-L-cystein;
S-[1-(2-{[2-({(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}amino)ethyl]amino}-2-oxoethyl)-2,5-dioxopyrrolidin-3-yl]-L-cystein;
N-{(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}-beta-alanyl-L-alanyl-N-[4-(3-{[(2R)-2-amino-2-carboxyethyl]sulfanyl}-2,5-dioxopyrrolidin-1-yl)phenyl]-N⁵-carbamoyl-L-ornithinamid;
S-(1-{2-[(N-{(2S)-2-Amino-4-[-{(1R)-1-[1-benzyl-4-(2,5-difluorphenyl)-1H-pyrrol-2-yl]-2,2-dimethylpropyl}(glycoloyl)amino]butanoyl}-beta-alanyl)amino]ethyl}-2,5-dioxopyrrolidin-3-yl)-L-cystein;
N-[6-(3-{[(2R)-2-Amino-2-carboxyethyl]sulfanyl}-2,5-dioxopyrrolidin-1-yl)hexanoyl]-L-valyl-N⁵-carbamoyl-L-ornithyl-N⁶-{(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}-L-lysin;
S-[1-(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)-2,5-dioxopyrrolidin-3-yl]-L-cystein;
S-(2-{[2-({(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}amino)ethyl]amino}-2-oxoethyl)-L-cystein;
S-{1-[6-(2-{(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}hydrazino)-6-oxohexyl]-2,5-dioxopyrrolidin-3-yl}-L-cystein;
N-[19-(3(R/S)-{[(2R)-2-Amino-2-carboxyethyl]sulfanyl}-2,5-dioxopyrrolidin-1-yl)-17-oxo-4,7,10,13-tetraoxa-16-azanonadecan-1-oyl]-R/S-{2-[(3-aminopropyl){(1R)-1-[1-benzyl-4-(2,5-difluorphenyl)-1H-pyrrol-2-yl]-2,2-dimethylpropyl}amino]-2-oxoethyl}homocystein;
S-{(3R/S)-1-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-2,5-dioxopyrrolidin-3-yl}-L-cystein;
N-[19-(3(R/S)-{[(2R)-2-Amino-2-carboxyethyl]sulfanyl}-2,5-dioxopyrrolidin-1-yl)-17-oxo-4,7,10,13-tetraoxa-16-azanonadecan-1-oyl]-R/S-{2-[(3-aminopropyl){(1R)-1-[1-benzyl-4-(2,5-difluorphenyl)-1H-imidazol-2-yl]-2,2-dimethylpropyl}amino]-2-oxoethyl}homocystein;
S-[(3R/S)-1-(2-{[6-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)hexanoyl]amino}ethyl)-2,5-dioxopyrrolidin-3-yl]-L-cystein;
S-{1-[2-({[(1R,3S)-3-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)cyclopentyl]carbonyl}amino)ethyl]-2,5-dioxopyrrolidin-3-yl} -L-cystein;
S-(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)-L-cystein;
N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-N²-{N-[6-(3-{[(2R)-2-amino-2-carboxyethyl]sulfanyl}-2,5-dioxopyrrolidin-1-yl)hexanoyl]-L-valyl-L-alanyl}-L-lysin;
N-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-L-glutamin;
N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-L-lysin;
N-(3-Aminopropyl)-N-{(1R)-1-[4-benzyl-1-(2,5-difluorphenyl)-1H-pyrazol-3-yl]-2,2-dimethylpropyl}acetamid;
N-(3-Aminopropyl)-N-{(1R)-1-[4-benzyl-1-(2,5-difluorphenyl)-1H-pyrazol-3-yl]-2,2-dimethylpropyl}-2-methoxyacetamid;
N-(3-Aminopropyl)-N-{(1R)-1-[4-benzyl-1-(2,5-difluorphenyl)-1H-pyrazol-3-yl]-2,2-dimethylpropyl}-2,4-difluorobenzamid;
N-(3-Aminopropyl)-N-{(1R)-1-[4-benzyl-1-(2,5-difluorphenyl)-1H-pyrazol-3-yl]-2,2-dimethylpropyl}-4-methylbenzamid;
N-(3-Aminopropyl)-N-{(1R)-1-[4-benzyl-1-(2,5-difluorphenyl)-1H-pyrazol-3-yl]-2,2-dimethylpropyl}-2-ethoxyacetamid;
N-(3-Aminopropyl)-N-{(1R)-1-[1-benzyl-4-(2,5-difluorphenyl)-1H-pyrrol-2-yl]-2,2-dimethylpropyl}-3,3,3 -trifluorpropanamid;
N-(3-Aminopropyl)-N-{(1R)-1-[1-benzyl-4-(2,5-difluorphenyl)-1H-pyrrol-2-yl]-2,2-dimethylpropyl}-4-fluorbenzamid;
N-(3-Aminopropyl)-N-{(1R)-1-[1-benzyl-4-(2,5-difluorphenyl)-1H-pyrrol-2-yl]-2,2-dimethylpropyl}acetamid;
N-(3-Aminopropyl)-N- {(1R)-1-[1-benzyl-4-(2,5-difluorphenyl)-1H-pyrrol-2-yl]-2,2-dimethylpropyl}-4-(trifluoromethyl)benzamid;
N-(3-Aminopropyl)-N-{(1R)-1-[4-benzyl-1-(2,5-difluorphenyl)-1H-pyrazol-3-yl]-2,2-dimethylpropyl}-2-ethoxyacetamid;
N-(3-Aminopropyl)-N-{(1R)-1-[4-benzyl-1-(2,5-difluorphenyl)-1H-pyrazol-3-yl]-2,2-dimethylpropyl}-2-ethoxyacetamid
(2S)-2-Amino-4-[{(1R)-1-[1-benzyl-4-(2,5-difluorphenyl)-1H-pyrrol-2-yl]-2,2-dimethylpropyl} (glycoloyl)amino]butansäure;
(2S)-2-amino-N-(2-aminoethyl)-4-[{(1R)-1-[1-benzyl-4-(2,5-difluorphenyl)-1H-pyrrol-2-yl]-2,2-dimethylpropyl}(glycoloyl)amino]butanamid;
4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure;
4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-2-{[(2R)-2-amino-2-carboxyethyl]sulfanyl}-4-oxobutansäure;
N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanin;
N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-L-serin;
N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-L-alanin;
N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}glycin;
N-(3-aminopropyl)-N-{(1R)-1-[1-benzyl-4-(2,5-difluorphenyl)-1H-pyrrol-2-yl]-2,2-dimethylpropyl}-4-methylbenzamid;
N-(3-aminopropyl)-N-{(1R)-1-[1-benzyl-4-(2,5-difluorphenyl)-1H-pyrrol-2-yl]-2,2-dimethylpropyl}-4-(methylsulfanyl)benzamid;
(2S)-N-(3-aminopropyl)-N-{(1R)-1-[1-benzyl-4-(2,5-difluorphenyl)-1H-pyrrol-2-yl]-2,2-dimethylpropyl} -2-hydroxypropanamid;
N-(3-aminopropyl)-N-{(1R)-1-[1-benzyl-4-(2,5-difluorphenyl)-1H-pyrrol-2-yl]-2,2-dimethylpropyl}-2-(methylsulfanyl) acetamid;
(2S)-N-(3-aminopropyl)-N-{(1R)-1-[4-benzyl-1-(2,5-difluorphenyl)-1H-pyrazol-3-yl]-2,2-dimethylpropyl} -2-hydroxypropanamid;
Methyl-4-[(3-aminopropyl){(1R)-1-[1-benzyl-4-(2,5-difluorphenyl)-1H-pyrrol-2-yl]-2,2-dimethylpropyl}amino]-4-oxobutanoat;
4-[(3-Aminopropyl){(1R)-1-[1-benzyl-4-(2,5-difluorphenyl)-1H-pyrrol-2-yl]-2,2-dimethylpropyl}amino]-4-oxobutansäure;
(2R)-22-[(3R/S)-3-{[(2R)-2-Amino-2-carboxyethyl]sulfanyl}-2,5-dioxopyrrolidin-1-yl]-2-[({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)methyl]-4,20-dioxo-7,10,13,16-tetraoxa-3,19-diazadocosan-1-säure;
4-amino-N-(3-aminopropyl)-N-{(1R)-1-[4-benzyl-1-(2,5-difluorphenyl)-1H-pyrazol-3-yl]-2,2-dimethylpropyl}benzamid;
N-Acetyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein;
N-Acetyl-S-[2-([3-(L-alanylamino)propyl]{(1R)-1-[1-benzyl-4-(2,5-difluorphenyl)-1H-pyrrol-2-yl]-2,2-dimethylpropyl}amino)-2-oxoethyl]-L-cystein;
(2S)-N-(3-aminopropyl)-N-{(1R)-1-[1-benzyl-4-(2,5-difluorphenyl)-1H-pyrrol-2-yl]-2,2-dimethylpropyl}tetrahydrofuran-2-carboxamid;
3-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)propansäure;
S-{2-[(3-Aminopropyl){(1R)-1-[1-benzyl-4-(2,5-difluorphenyl)-1H-pyrrol-2-yl]-2,2-dimethylpropyl}amino]-2-oxoethyl}homocystein;
4-amino-N-(3-aminopropyl)-N-{(1R)-1-[1-benzyl-4-(2,5-difluorphenyl)-1H-pyrrol-2-yl]-2,2-dimethylpropyl}benzamid;
4-[(2-{[(2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-2-carboxyethyl]amino}-2-oxoethyl)amino]-3-{[(2R)-2-amino-2-carboxyethyl]sulfanyl}-4-oxobutansäure;
   4-[(2-{[(2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-2-carboxyethyl]amino}-2-oxoethyl)amino]-2-{[(2R)-2-amino-2-carboxyethyl]sulfanyl}-4-oxobutansäure;
Erfindungsgemäß besonders bevorzugt sind die folgenden Verbindungen der Formeln V, VI oder VII, wobei R¹, R², R³, R⁴ und R⁵ die oben genannten Bedeutungen (wie z.B. für Formel (IIa) oder (IIIa) angegeben) haben:

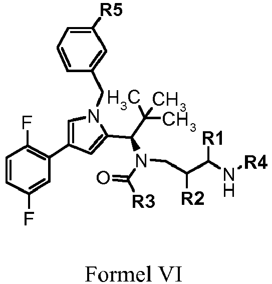

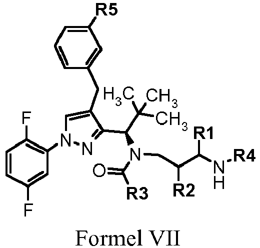

Besonders bevorzugt sind die Verbindungen der Formeln VI, VII, wobei R¹, und R⁵ H oder -L-#1 darstellen; R² und R⁴ unabhängig voneinander -L-#1 oder H oder R² und R⁴ gemeinsam (unter Bildung eines Pyrrolidinringes) -CH₂-CHR¹⁰- oder -CHR¹⁰-CH₂- darstellen, wobei R¹⁰ H oder -L-#1, darstellt; und R³ CH₂OH, CH(CH₃)OH oder -L-#1 darstellt, wobei einer der Substituenten R¹, R², R³, R⁴, R⁵ und R¹⁰ -L-#1 darstellt. Insbesondere bevorzugt sind entsprechende Verbindungen der Formel VI.

### Linker

Aus der Literatur sind verschiedene Möglichkeiten der kovalenten Kopplung (Konjugation) von organischen Molekülen an Binder wie z.B. Antikörper bekannt (siehe z.B. K. Lang and J. W. Chin. Chem. Rev. 2014, 114, 4764-4806, M. Rashidian et al. Bioconjugate Chem. 2013, 24, 1277-1294). Erfindungsgemäß bevorzugt ist die Konjugation der KSP-Inhibitoren an einen Antikörper über ein oder mehrere Schwefelatome von Cystein-Resten des Antikörpers, die entweder als freie Thiole bereits vorliegen oder durch Reduktion von Disulfidbrücken generiert werden, und/oder über eine oder mehrere NH-Gruppen von Lysin-Resten des Antikörpers. Es ist jedoch auch möglich, den KSP-Inhibitor an den Antikörper über Tyrosinreste, über Glutaminreste, über Reste unnatürlicher Aminosäuren, über freie Carboxylgruppen oder über Zuckerreste des Antikörpers zu binden. Zur Kopplung werden sogenannte Linker verwendet. Linker lassen sich in die Gruppe der *in vivo* spaltbaren Linker und in die Gruppe der *in vivo* stabilen Linker unterteilen (siehe L. Ducry und B. Stump, Bioconjugate Chem. 21, 5-13 (2010)). Die *in vivo* spaltbaren Linker weisen eine *in vivo* spaltbare Gruppe auf, wobei wiederum zwischen chemisch *in vivo* spaltbaren und enzymatisch *in vivo* spaltbaren Gruppen unterschieden werden kann. "Chemisch *in vivo* spaltbar" bzw. "enzymatisch *in vivo* spaltbar" bedeutet, dass die Linker bzw. Gruppen im Blutkreislauf stabil sind und erst an bzw. in der Zielzelle durch die dort veränderte chemische bzw. enzymatische Umgebung (niedrigerer pH-Wert; erhöhte Glutathion-Konzentration; Vorliegen lysosomaler Enzyme wie Cathepsin oder Plasmin, oder Glyosidasen wie beispielsweise β-Glucuronidasen) sich spalten, um so den niedermolekularen KSP-Inhibitor oder ein Derivat davon freizusetzen. Die chemisch *in vivo* spaltbaren Gruppen sind insbesondere Disulfid, Hydrazon, Acetal und Aminal; die enzymatisch *in vivo* spaltbaren Gruppen sind insbesondere 2-8-Oligopeptidgruppe, insbesondere eine Dipeptidgruppe oder Glycoside. Peptidspaltstellen sind in Bioconjugate Chem. 2002, 13, 855-869, sowie Bioorganic & Medicinal Chemistry Letters 8 (1998) 3341-3346 *sowie* Bioconjugate Chem. 1998, 9, 618-626 offenbart. Hierzu gehören z.B. Valin-Alanin, Valin-Lysin, Valin-Citrullin, Alanin-Lysin und Phenylalanin-Lysin (ggf. mit zusätzlicher Amidgruppe).

Die *in vivo* stabilen Linker zeichnen sich durch eine hohe Stabilität aus (weniger als 5 % Metabolite nach 24 Stunden in Plasma) und weisen die oben genannten chemisch oder enzymatisch *in vivo* spaltbaren Gruppen nicht auf.

Der Linker -L- weist vorzugsweise eine der folgenden Grundstrukturen (i) bis (iv) auf:
(i) -(CO)ₘ₋SG1-L1-L2-
(ii) -(CO)ₘ-L1-SG-L1-L2-
(iii) -(CO)ₘ-L1-L2-
(iv) -(CO)ₘ-L1-SG-L2
wobei m 0 oder 1 ist; SG eine (chemisch oder enzymatisch) *in vivo* spaltbare Gruppe (insbesondere Disulfid, Hydrazon, Acetal und Aminal; oder eine mit Cathepsin oder Plasmin spaltbare 2-8-Oligopeptidgruppe) ist, SG1 eine Oligopeptidgruppe oder vorzugsweise eine Dipeptidgruppe ist, L1 unabhängig voneinander für *in vivo* stabile organische Gruppen stehen, und L2 für eine Kopplungsgruppe an den Binder oder eine Einfachbindung steht. Die Kopplung erfolgt hierbei vorzugsweise an einen Cysteinrest oder einen Lysinrest des Binders. Alternativ kann die Kopplung an einen Tyrosinrest, Glutaminrest oder an eine unnatürliche Aminosäure des Binders erfolgen. Die unnatürlichen Aminosäuren können beispielsweise Aldehyd- oder Ketogruppen (wie z.B. Formylglycin) oder Azid- oder Alkingruppen enthalten (siehe hierzu Lan & Chin, Cellular Incorporation of Unnatural Amino Acids and Bioorthogonal Labeling of Proteins, Chem.Rev. 2014, 114, 4764-4806).

Erfindungsgemäß bevorzugt ist insbesondere die Linker-Grundstruktur (iii), insbesondere wenn der Binder ein anti-TWEAKR-Antikörper oder ein anti-EGFR-Antikörper. Die Verabreichung eines erfindungsgemäßen Konjugats mit einer Linker-Grundstruktur (iii) und Kopplung des Linkers an einen Cystein- oder Lysinrest des Binderproteins oder -peptids führt durch Metabolisierung zu Cystein- bzw. Lysinderivaten der folgenden Formeln: 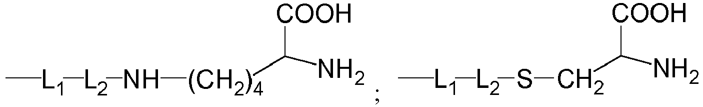 wobei L1 jeweils an den niedermolekularen KSP-Inhibitor gebunden ist, z.B. einer Verbindung der Formel (I), (IIa), (II), (III), (IIIa), (IIIb), (IIIc), (IIId), (IIIe), oder (IV).

Erfindungsgemäß bevorzugt sind auch die Linker-Grundstrukturen (ii) and (iv), insbesondere bei Anbindung an die Position (iii), insbesondere wenn die Gruppe L1 eine der folgenden Struktur aufweist:
(a) -NH-(CH₂)₀₋₄-(CHCH₃)₀₋₄-CHY⁵-CO-Y⁷, wobei Y⁵ H oder NHY⁶ darstellt, wobei Y⁶ H oder-COCH₃ darstellt, und Y⁷ eine Einfachbindung oder -NH-(CH₂)₀₋₄ -CHNH₂-CO- darstellt, so dass nach der Spaltung die entsprechende Struktur -NH-(CH₂)₀₋₄-(CHCH₃)₀₋₄-CHY⁵-COOH bzw. -NH-(CH₂)₀₋₄-(CHCH₃)₀₋₄-CHY⁵-CO-NH-(CH₂)₀₋₄-CHNH₂-COOH erhalten wird.
(b) -CH₂-Sₓ-(CH₂)₀₋₄-CHY⁵-CO- auf, wobei x 0 oder 1 ist, und Y⁵ H oder NHY⁶ darstellt, wobei Y6 H oder -COCH₃ darstellt, so dass nach der Spaltung die entsprechende Struktur -CH₂-Sₓ-(CH₂)₀₋₄-CHY⁵-COOH erhalten wird.

Diese Ausführungsform ist bevorzugt, wenn L1 jeweils an den niedermolekularen KSP-Inhibitor gebunden ist, z.B. einer Verbindung der Formel (I), (IIa), (II), (III), (IIIa), (IIIb), (IIIc), (IIId), (IIIe), oder (IV), insbesondere an der Position R₄, Der Binder ist vorzugsweise ein anti-TWEAKR-Antikörper oder ein anti-EGFR-Antikörper.

Wenn der Linker an eine Cystein-Seitenkette bzw. einen Cysteinrest gebunden ist, leitet sich L2 vorzugsweise von einer mit der Sulfhydryl-Gruppe des Cysteins reaktiven Gruppe ab. Hierzu zählen Haloacetyle, Maleimide, Aziridines, Acryloyle, Aryliende Verbindungen, Vinylsulfone, Pyridyldisulfide, TNB-thiole and Disulfid-reduzierende Mittel. Diese Gruppen reagieren in der Regel elektrophil mit der Sulfhydryl-Bindung unter Bildung einer Sulfid (z.B. Thioether)- oder DisulfidBrücke. Bevorzugt sind stabile Sulfidbrücken. L2 ist vorzugsweise 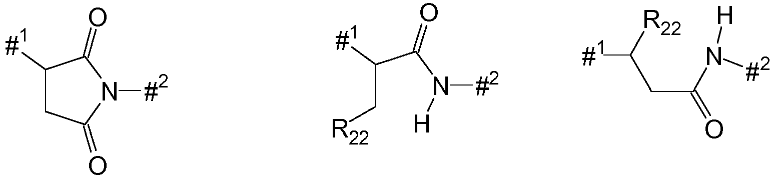 #¹-S-#² 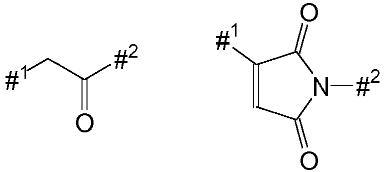 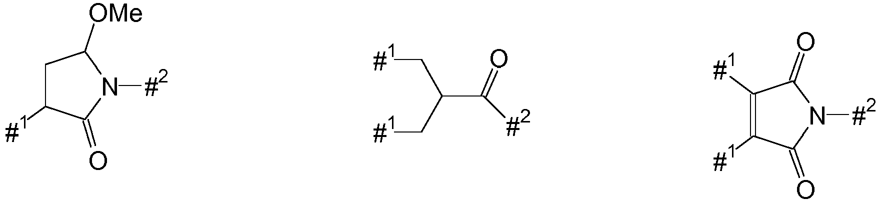 wobei
- #¹: die Verknüpfungsstelle mit dem Schwefelatom des Binders kennzeichnet,
- #²: die Verknüpfungsstelle mit der Gruppe L¹ kennzeichnet, und

R₂₂ COOH, COOR, COR, CONHR, CONR₂ (mit R jeweils C1-3-Alkyl), CONH2, vorzugsweise COOH darstellt.

Besonders bevorzugt als L2 ist: 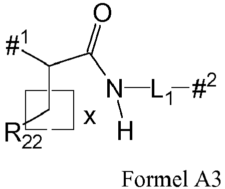 bzw. 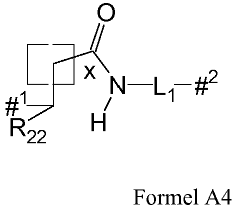 wobei #¹ die Verknüpfungsstelle mit dem Schwefelatom des Binders kennzeichnet, #² die Verknüpfungsstelle mit der dem Wirkstoff kennzeichnet, x 1 oder 2 darstellt, und R²² COOH, COOR, COR, CONHR (mit R jeweils C1-3-Alkyl), CONH2, vorzugsweise COOH darstellt. Bevorzugt ist es, wenn x=1 und R²² COOH darstellt.

In einem erfindungsgemäßen Konjugat bzw. in einem Gemisch der erfindungsgemäßen Konjugate liegen die Bindungen an einen Cysteinrest des Binders vorzugsweise zu über 80 %, besonders bevorzugt zu über 90 % (jeweils bezogen auf die Gesamtanzahl von Bindungen des Linkers an den Binder), besonders bevorzugt in einer der beiden Strukturen der Formel A3 oder A4 vor. Hierbei liegen die Strukturen der Formel A3 oder A4 in der Regel gemeinsam vor, vorzugsweise in einem Verhältnis von 60:40 bis 40:60 bezogen auf die Anzahl der Bindungen an den Binder. Die restlichen Bindungen liegen dann in der Struktur 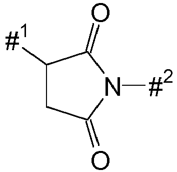 vor.
Erfindungsgemäß wird L1 vorzugsweise durch die Formel

#¹-(NR¹⁰)ₙ-(G1)ₒ-G2-#²

dargestellt, wobei
R¹⁰ H, NH₂ oder C₁-C₃-Alkyl darstellt;
G1-NHCO- , -CONH- oder 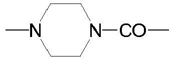 darstellt; (R¹⁰ ist vorzugsweie nicht NH₂, wenn G1 NHCO oder 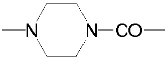 ).
n 0 oder 1 ist;
o 0 oder 1 ist; und
G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO₂, -NRY-, -NRYCO-, - C(NH)NR^{y}-, -CONR^{y}-, -NRYNRY-, -SO2NR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, - NH₂, NH-CNNH₂, Sulfonamid, Sulfon, Sulfoxid, oder Sulfonsäure substituiert sein können), -CO-, - CR^{x}=N-O- (wobei Rx H, C₁-C₃-Alkyl oder Phenyl darstellt) und/oder einen 3 bis 10-gliedriger, aromatischen oder nicht aromatischen Heterozyklus mit bis zu 4 Heteroatomen ausgewählt aus N, O und S, -SO- oder -SO2- unterbrochen sein kann (vorzugsweise 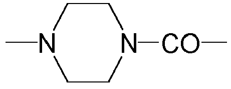 ), wobei die Kohlenwasserstoffkette einschließlich der Seitenketten, sofern vorhanden, mit -NHCONH₂, -COOH, - OH, -NH₂, NH-CNNH₂, Sulfonamid, Sulfon, Sulfoxid, oder Sulfonsäure substituiert sein können.

G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO₂, -NH-, -CO-, - NHCO-, -CONH-, -NMe-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und
S, oder -SO- unterbrochen sein kann (vorzugsweise 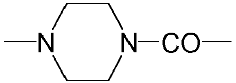 ), wobei die Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein können.

G2 ist vorzugsweise eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO₂, -NH-, -CO-, -NHCO-, -CONH-, -NMe-, -NHNH-, -SO2NHNH-, -CONHNH-, -CR^{x}=N-O-(wobei Rx H, C₁-C₃-Alkyl oder Phenyl darstellt) und einen 3 bis 10-gliedriger, z.B. 5 bis 10gliedriger aromatischen oder nicht aromatischen Heterozyklus mit bis zu 4 Heteroatomen ausgewählt aus N, O und S, -SO- oder -SO2- unterbrochen sein kann (vorzugsweise 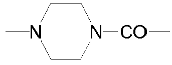 ), wobei Kohlenwasserstoffkette einschließlich der Seitenketten, sofern vorhanden, mit -NHCONH₂, -COOH, - OH, -NH₂, NH-CNNH₂, Sulfonamid, Sulfon, Sulfoxid, oder Sulfonsäure substituiert sein können.

Weitere unterbrechende Gruppen in G2 sind vorzugsweise 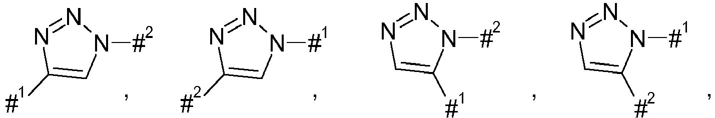 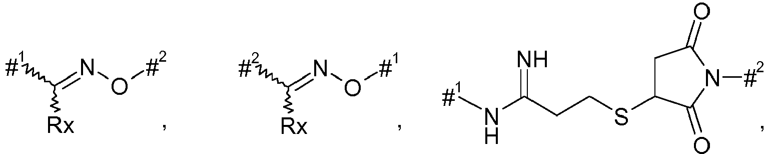 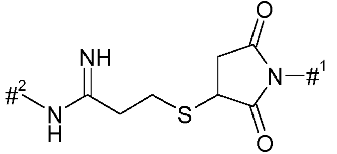 wobei Rx H, C₁-C₃-Alkyl oder Phenyl darstellt.

Hierbei ist #¹ die Bindung zum KSP-Inhibitor und #² die Bindung zur Kopplungsgruppe zum Binder (z.B. L2).

Eine geradkettige oder verzweigte Kohlenwasserstoffkette aus Arylengruppen und/oder geradkettigen und/oder verzweigten und/oder cyclischen Alkylengruppen umfasst in der Regel einen α,ω-divalenten Alkylrest mit der jeweils angegebenen Zahl von Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen), Pentan-1,5-diyl (1,5-Pentylen), Hexan-1,6-diyl (1,6-Hexylen), Heptan-1,7-diyl (1,7-Hexylen), Octan-1,8-diyl (1,8-Octylen), Nonan-1,9-diyl (1,9-Nonylen), Decan-1,10-diyl (1,10-Decylen). Die Alkylengruppen in der Kohlenwasserstoffkette können jedoch auch verzweigt sein, d.h. ein oder mehrere H-Atome der oben genannten linearen Alkylengruppen können ggf. durch C1-10-Alkylgruppen substituiert sein und so Seitenketten bilden. Die Kohlenwasserstoffkette kann weiterhin cyclische Alkylengruppen (Cycloalkandiyl) enthalten, z.B. 1,4-Cyclohexandiyl oder 1,3-Cyclopentandiyl. Diese cyclischen Gruppen können ungesättigt sein. Insbesondere können in der Kohlenwasserstoffgruppe aromatische Gruppen (Arylengruppen) vorliegen, z.B. Phenylen. Auch in den cyclischen Alkylengruppen und den Arylengruppen können wiederum in oder mehrere H-Atome ggf. durch C1-10-Alkylgruppen substituiert sein. Es wird so eine Kohlenwasserstoffkette gebildet, die ggf. verzweigt ist. Diese Kohlenwasserstoffkette weist insgesamt 0 bis 100 Kohlenstoffatomen, vorzugsweise 1 bis 50, besonders bevorzugt 2 bis 25 Kohlenstoffatome auf.

Die Seitenketten können, sofern vorhanden, mit -NHCONH₂, -COOH, -OH, -NH₂, NH-CNNH₂, Sulfonamid, Sulfon, Sulfoxid, oder Sulfonsäure substituiert sein.

Die Kohlenwasserstoffkette kann einfach oder mehrfach durch eine oder mehrere der Gruppen -O-, -S-, -SO-, SO₂, -NH-, -CO-, -NHCO-, -CONH-, -NMe-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und S, -SO- oder -SO2- unterbrochen sein (vorzugsweise 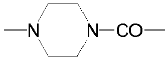 ). Weitere unterbrechende Gruppen in G2 sind vorzugsweise 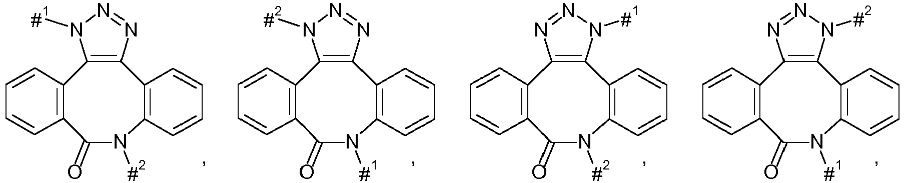 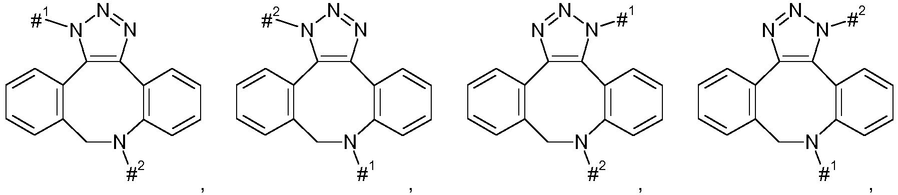 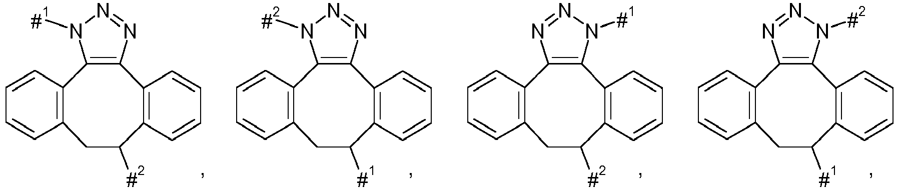 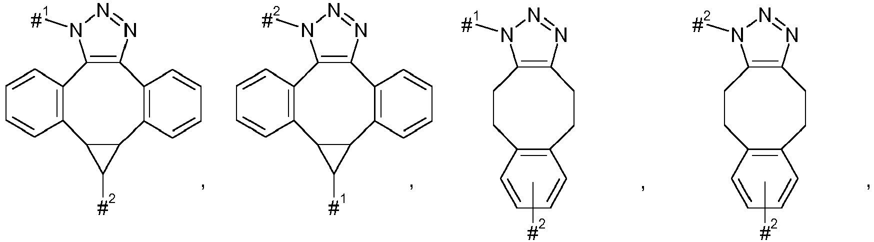 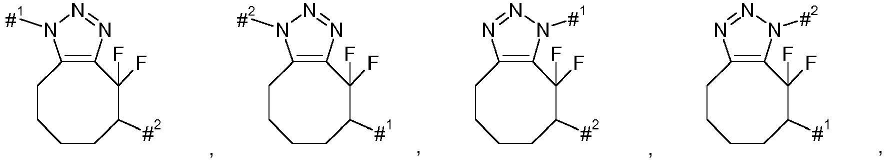 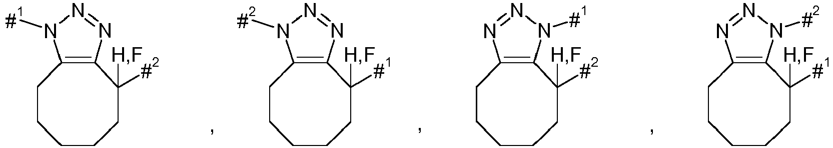 Vorzugsweise entspricht der Linker der folgenden Formel:

§-(CO)m-L1-L2-§§

wobei
m 0 oder 1 ist;
§ die Bindung an das Wirkstoffmolekül darstellt und
§ § die Bindung an das Binderpeptid oder -protein darstellt, und
L1 und L2 die oben genannten Bedeutungen aufweisen.

Besonders bevorzugt weist L1 die Formel -NR¹¹B- auf, wobei
R¹¹ H oder NH₂ darstellt;
B-[(CH₂)ₓ-(X⁴)_{y}]_{W}-(CH₂)_{z}- darstellt,
w = 0 bis 20 ist;
x = 0 bis 5 ist;
x = 0 bis 5 ist;
y = 0 oder 1 ist;
z = 0 bis 5 ist; und
X⁴ -O-, -CONH-,-NHCO- oder 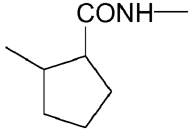 darstellt.

Erfindungsgemäß bevorzugte Linker L weisen die folgende Formel auf: 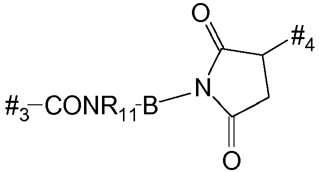 wobei
#3 die Bindung an das Wirkstoffmolekül darstellt,
#4 die Bindung an das Binderpeptid oder -protein darstellt,
R¹¹ H oder NH2 darstellt;
B-[(CH₂)ₓ-(X⁴)_{y}]_{W}-(CH₂)_{z}- darstellt,
w = 0 bis 20 ist;
x = 0 bis 5 ist;
y = 0 oder 1 ist;
z = 1 bis 5 ist; und
X⁴ -O-, -CONH-, -NHCO- oder 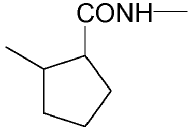 darstellt.

Die oben genannten Linker werden insbesondere bevorzugt in Konjugaten der Formel (I) oder (II), in denen der Linker durch Substitution eines H-Atoms an R1 oder in Verbindung mit einem spaltbaren Linker SG1 an R4 ankoppelt, d.h. R1-L-#1 darstellt oder R4 -SG1-L-#1, wobei #1 die Bindung an den Binder darstellt.

Weiterhin sind die folgenden Linker erfindungsgemäß bevorzugt: In einem erfindungsgemäßen Konjugat bzw. in einem Gemisch der erfindungsgemäßen Konjugate liegen die Bindungen an einen Cysteinrest des Binders vorzugsweise zu über 80 %, besonders bevorzugt zu über 90 % (jeweils bezogen auf die Gesamtanzahl von Bindungen des Linkers an den Binder), besonders bevorzugt in einer der beiden Strukturen der Formel A5 oder A6 vor: 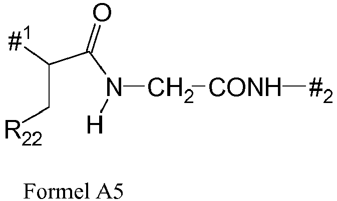 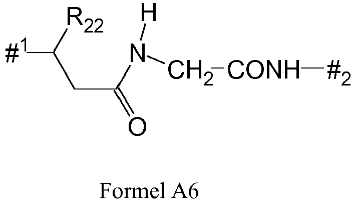 wobei
#¹ die Verknüpfungsstelle mit dem Schwefelatom des Binders kennzeichnet,
#² die Verknüpfungsstelle mit der Gruppe L¹ kennzeichnet, und
R²² COOH, COOR, COR, CONHR (mit R jeweils C1-3-Alkyl), CONH₂, vorzugsweise COOH darstellt.

Hierbei liegen die Strukturen der Formel A5 oder A6 in der Regel gemeinsam vor, vorzugsweise in einem Verhältnis von 60:40 bis 40:60 bezogen auf die Anzahl der Bindungen an den Binder. Die restlichen Bindungen liegen dann in der Struktur 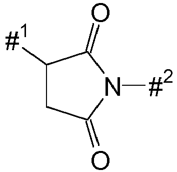 vor.

Andere Linker -L-, die an eine Cystein-Seitenkette bzw. Cysteinrest gebunden ist, weisen die folgende Formel auf: 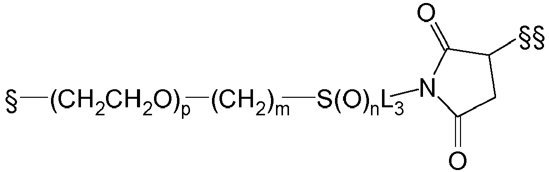 wobei
§ die Bindung an das Wirkstoffmolekül darstellt und
§§ die Bindung an das Binderpeptid oder -protein darstellt,
m 0, 1, 2, oder 3 ist;
n 0, 1 oder 2 ist;
p 0 bis 20 beträgt; und
L3 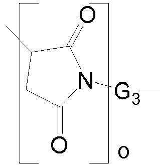 darstellt;
   wobei
   o 0 oder 1 ist;
   und
   G₃ eine geradkettige oder verzweigte Kohlenwasserstoffkette mit 1 bis 100 Kohlenstoffatomen aus Arylengruppen und/oder geradkettigen und/oder cyclischen Alkylengruppen ist, die einfach oder mehrfach durch eine oder mehrere der Gruppen -O-, -S-, -SO-, SO2, -NH-, -CO-, -NHCO-, -CONH-, - NMe-, -NHNH-, -SO2NHNH-, -CONHNH- und einen 3 bis 10gliedriger (vorzugsweise 5 bis10gliedriger), aromatischen oder nicht aromatischen Heterozyklus mit bis zu 4 Heteroatomen ausgewählt aus N, O und S, -SO- oder -SO2- unterbrochen sein kann (vorzugsweise 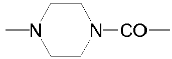 ), wobei die Seitenketten, sofern vorhanden, mit -NHCONH₂, -COOH, -OH, -NH₂, NH-CNNH₂, Sulfonamid, Sulfon, Sulfoxid oder Sulfonsäure substituiert sein können.
Bevorzugt ist in der obigen Formel

m 1 ist;
p 0 ist;
n 0 ist;
und L3 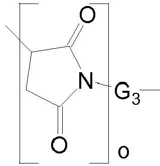 darstellt; wobei
o 0 oder 1 ist; und
G₃ -(CH₂CH₂O)ₛ(CH₂)ₜ(CONH)ᵤCH₂CH₂O)ᵥ(CH₂)_{w}- darstellt, wobei
s, t, v und w jeweils unabhängig voneinander 0 bis 20 betragen, und u 0 oder 1 beträgt.

Bevorzugte Gruppen L1 in der obigen Formel §-(CO)m-L1-L2-§§ sind die folgenden, wobei r jeweils unabhängig voneinander eine Zahl von 0 bis 20, vorzugsweise von 0 bis 15, besonders bevorzugt von 1 bis 20, insbesondere bevorzugt von 2 bis 10 aufweist:

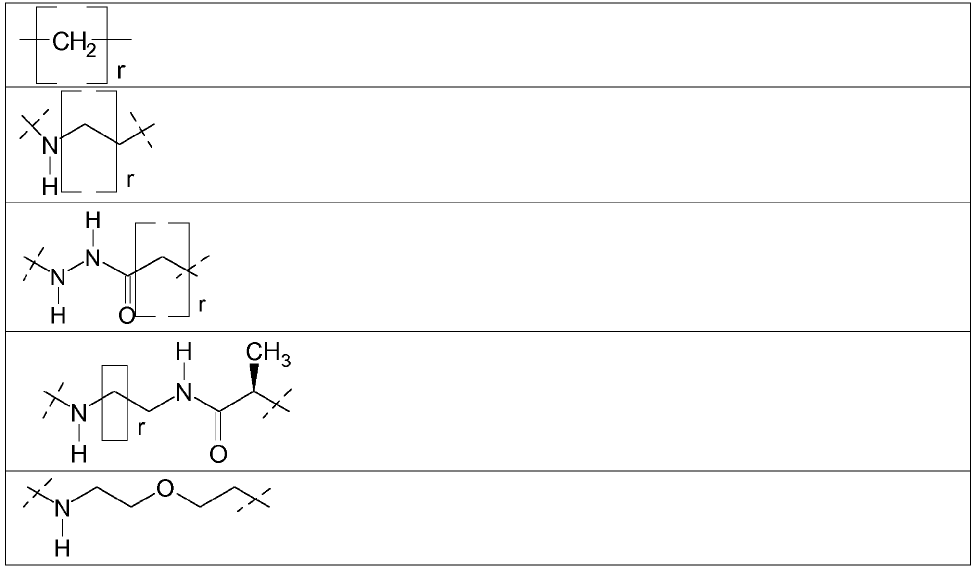

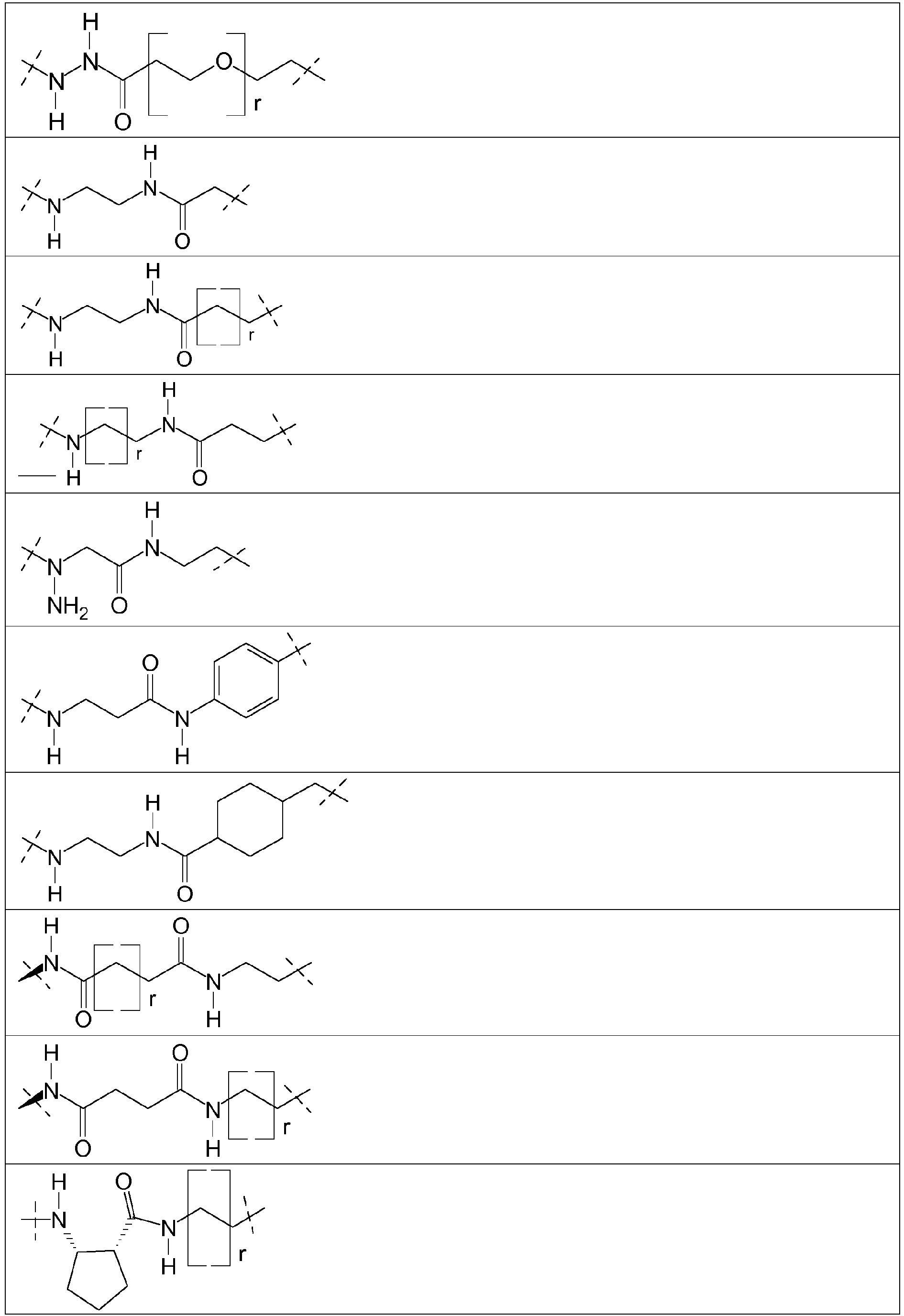

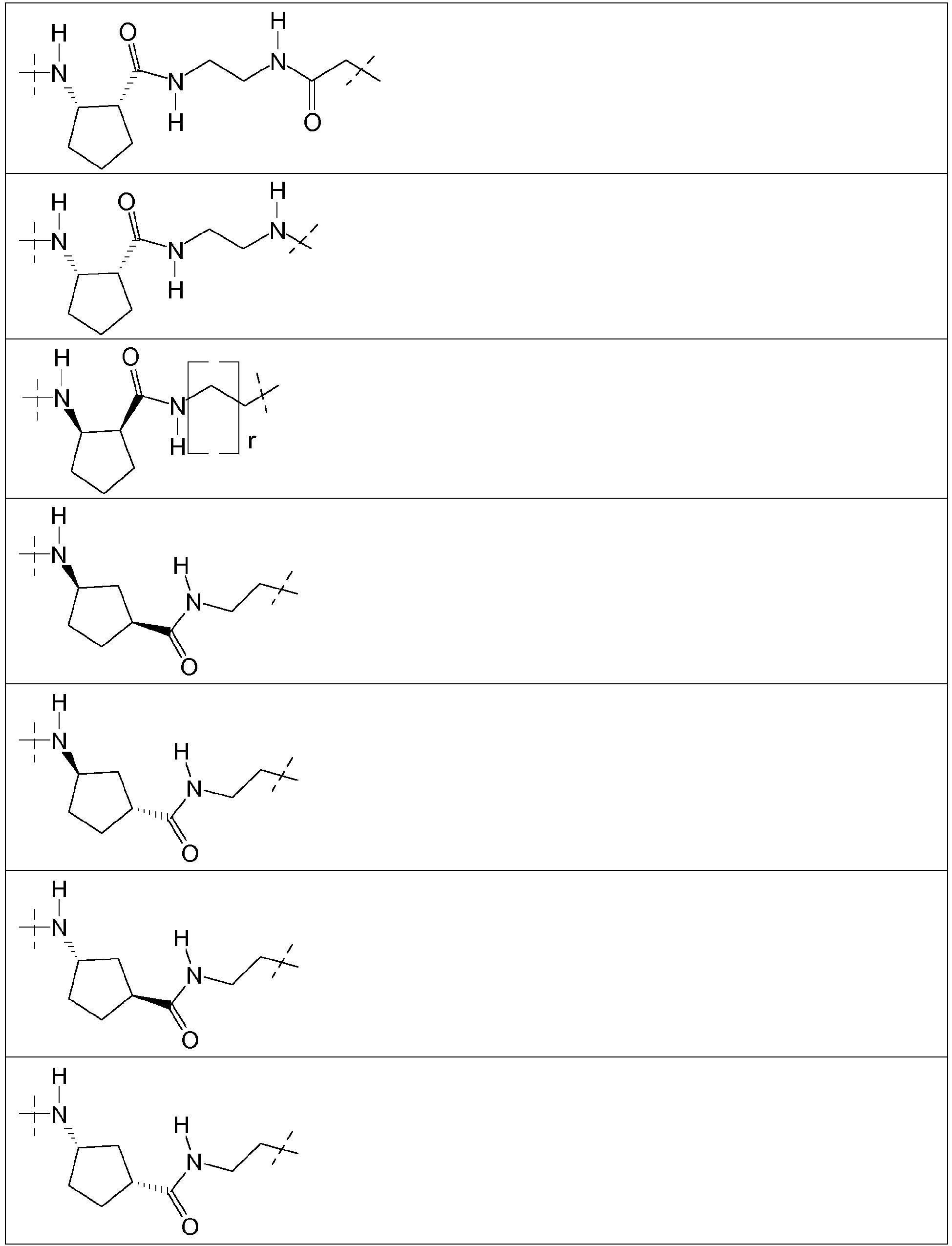

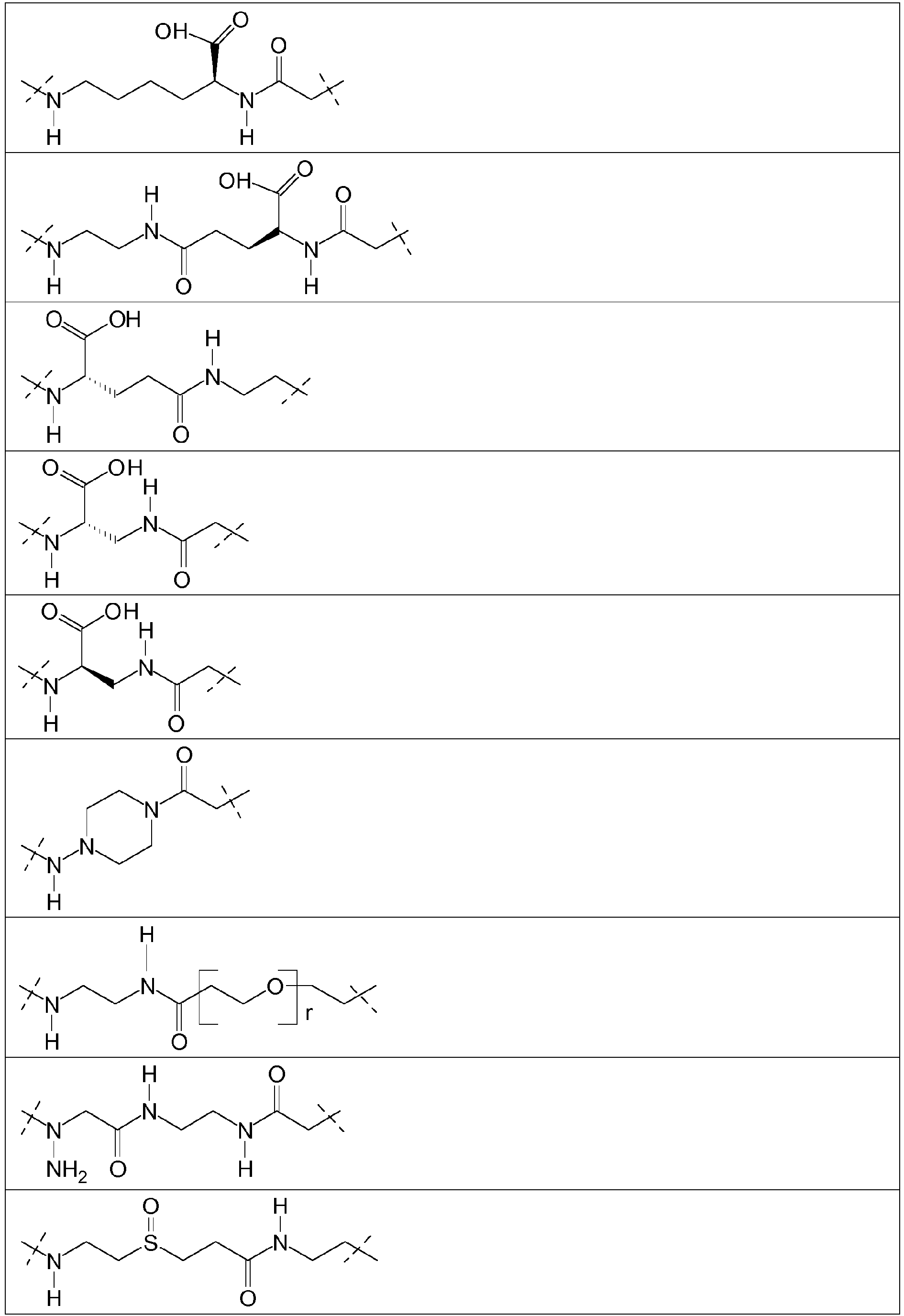

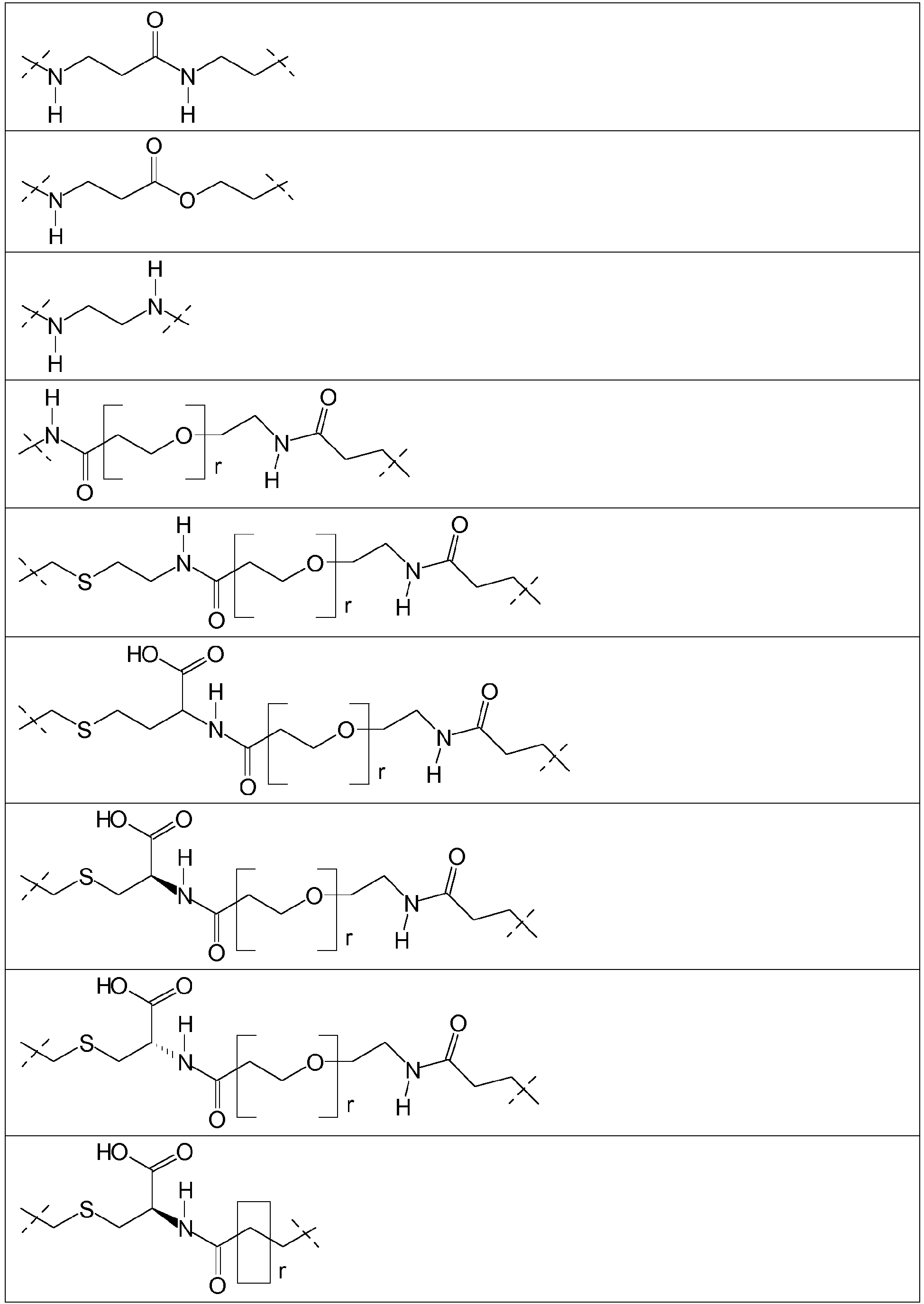

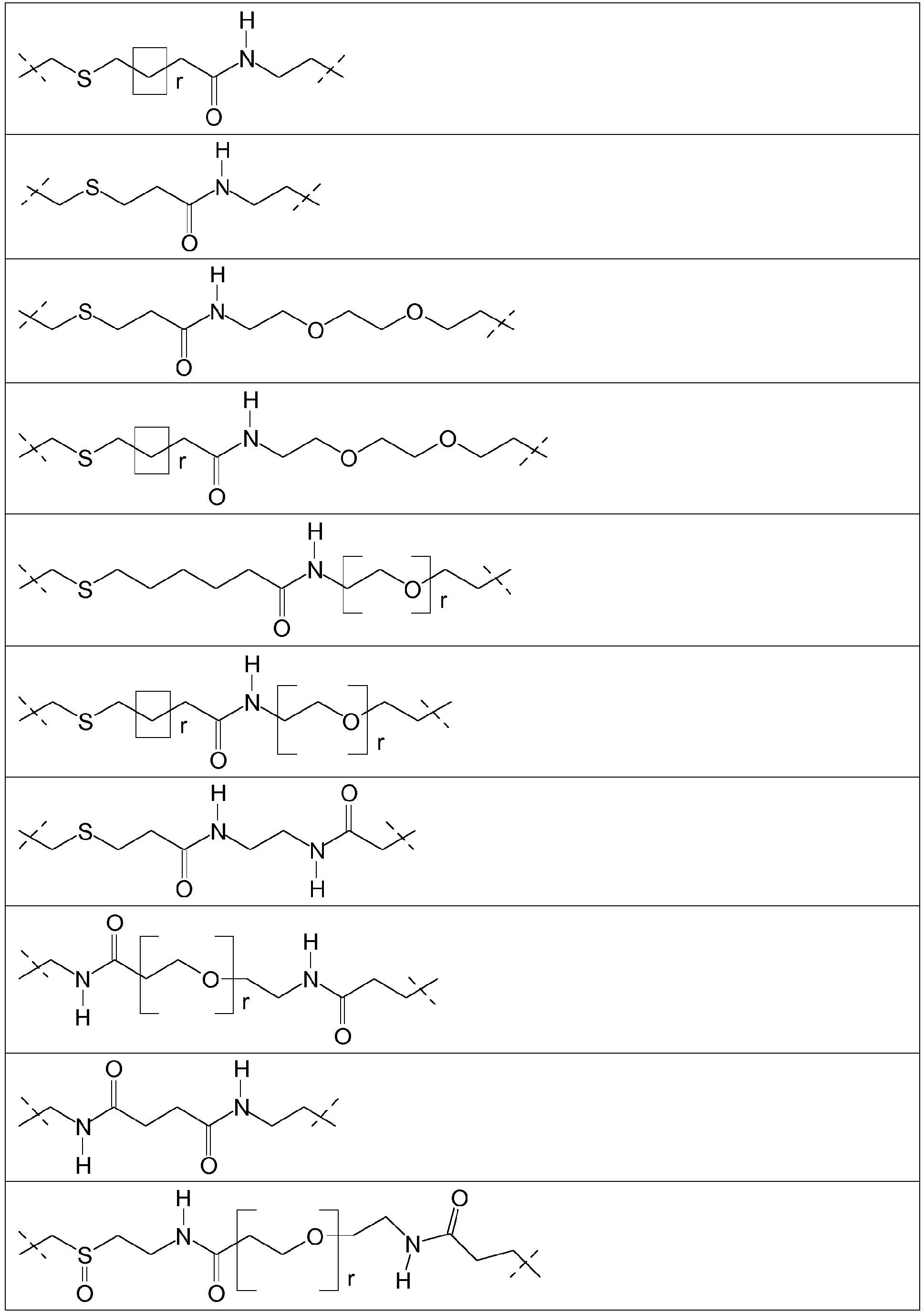

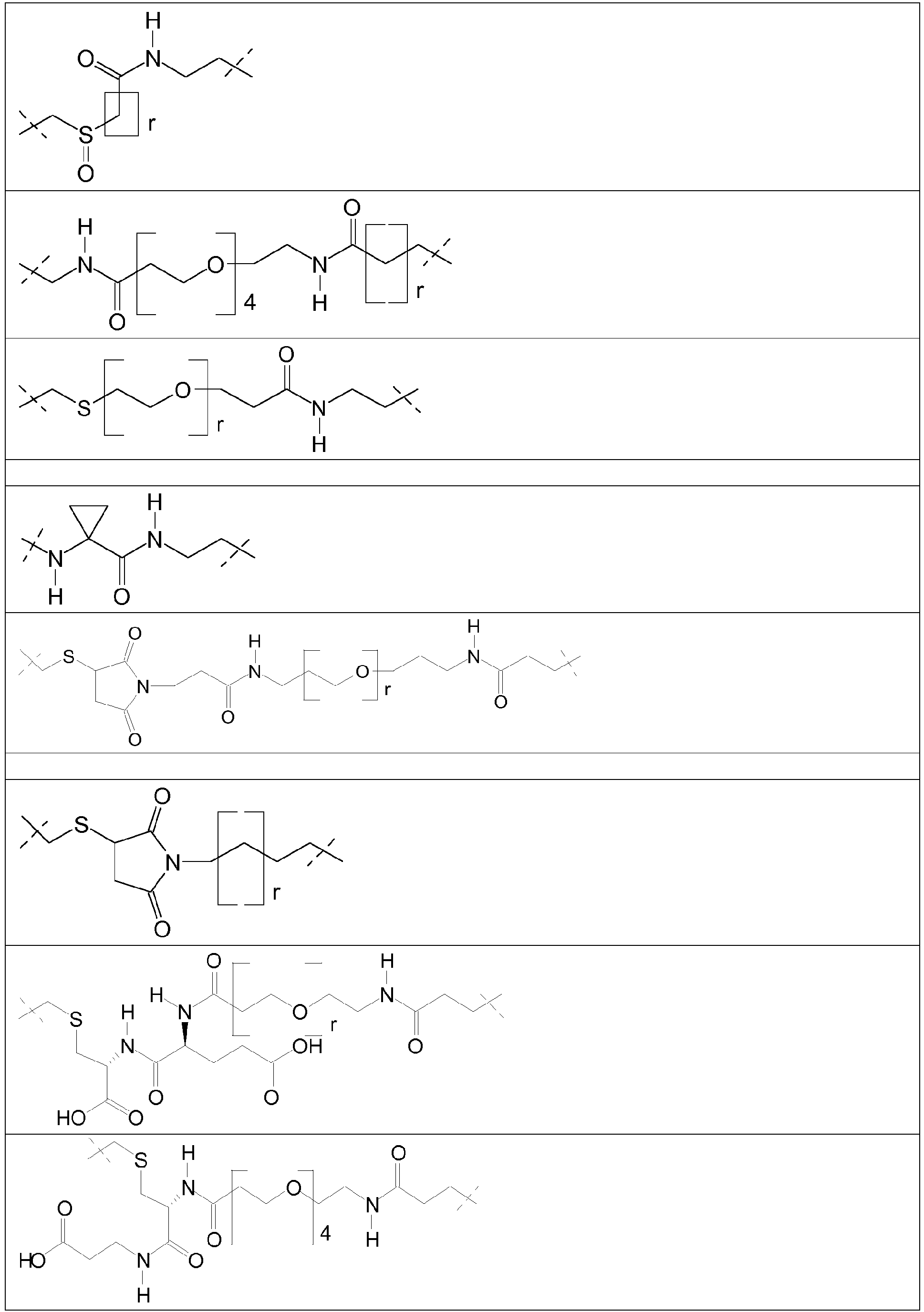

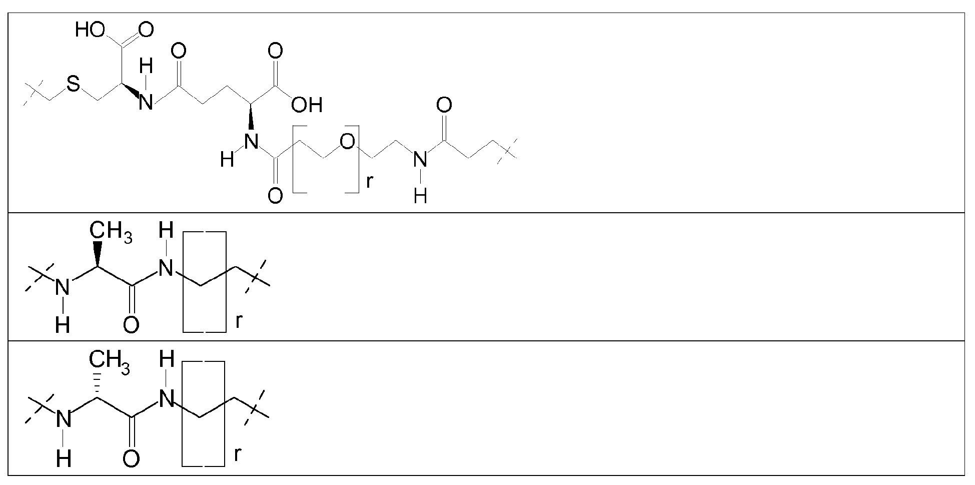

Weitere Beispiele für L1 sind in Tabelle C angegeben, in denen diese Gruppe in einem Kasten hervorgehoben ist.

In den folgenden Tabellen A und A' sind Beispiele für einen Linkerteil L1 angegeben. In der Tabelle ist weiterhin angegeben, mit welcher Gruppe L2 diese Beispiele für L1 vorzugsweise kombiniert werden, sowie die bevorzugte Ankopplungsstelle (R¹-R⁵) sowie der bevorzugte Wert für m, also ob vor L1 eine Carbonylgruppe vorliegt oder nicht (vgl. §-(CO)m-L1-L2-§§). Diese Linker werden vorzugsweise an einen Cysteinrest gekoppelt. In der ersten Spalte sind weiterhin die Beispielnummern für die Cetuximab ADCs angegeben, in denen entsprechende Linker Anwendung finden, die jedoch gleichermaßen in jeder Zeile für ADCs mit anderen Antikörpern gelten. Wenn L2 ein Bernsteinsäureimid ist bzw. sich hiervon ableitet, kann dieses Imid ganz oder teilweise auch in Form des hydrolysierten offenkettigen Bernsteinsäureamids vorliegen, wie oben beschrieben. In Abhängigkeit von L1 kann diese Hydrolyse zu offenkettigen Bernsteinsäureamiden mehr oder weniger stark oder gar nicht ausgeprägt sein.

**Tabelle A**

| Beisp. | Subst. | m | L1 | L2 |
|---|---|---|---|---|
| 2 | R¹ | 1 | 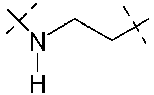 | 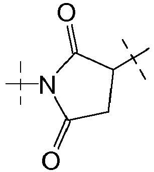 |
| 3 | R¹ | 1 | 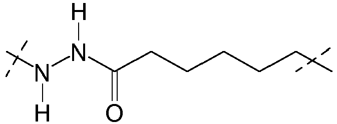 | 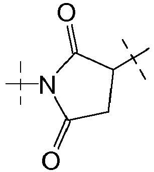 |
| 4 | R¹ | 1 | 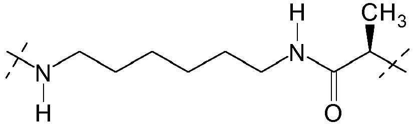 | 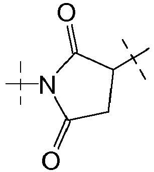 |
| 5 | R¹ | 1 | 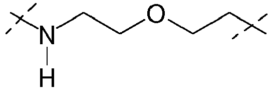 | 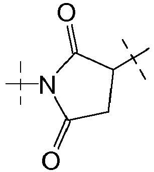 |
| 6 | R¹ | 1 | 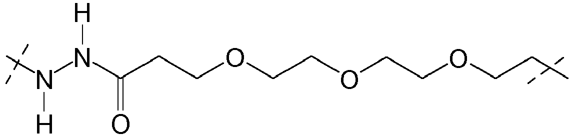 | 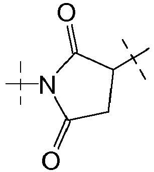 |
| 7 | R¹ | 1 | 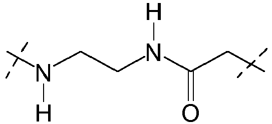 | 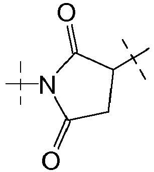 Siehe Anmerkung ** |
| 9 | R¹ | 1 | 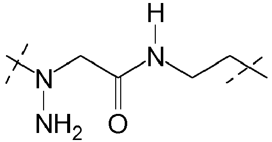 | 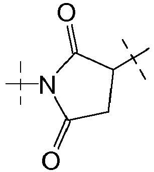 |
| 10 | R¹ | 1 | 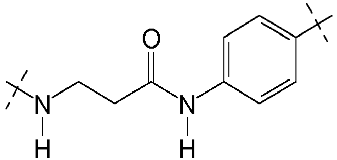 | 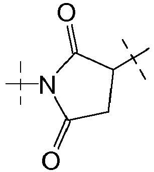 |
| 11 | R¹ | 1 | 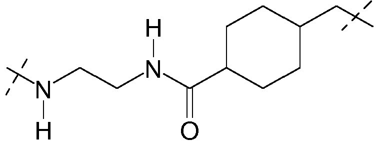 | 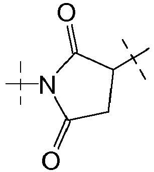 |
| 12 | R¹ | 1 | 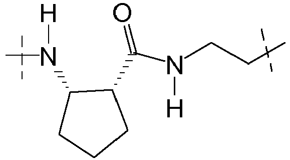 | 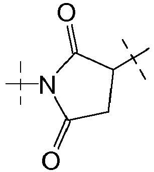 |
| 13 | R¹ | 1 | 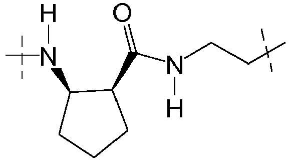 | 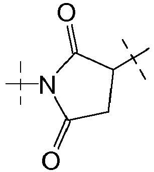 |
| 19 | R¹ | 1 | 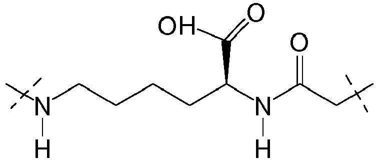 | 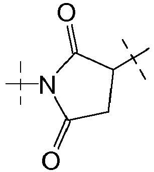 Siehe Anmerkung ** |
| 20 | R¹ | 1 | 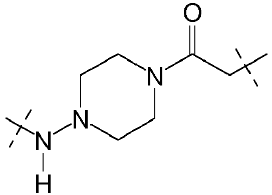 | 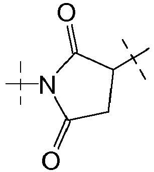 Siehe Anmerkung ** |
| 21/176 | R¹ | 1 | 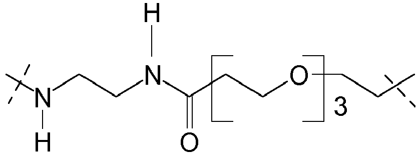 | 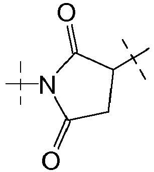 |
| 22 | R¹ | 1 | 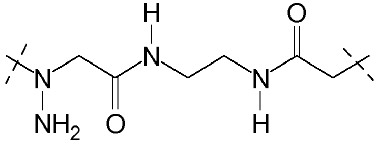 | 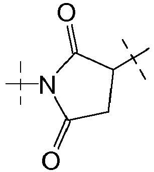 Siehe Anmerkung ** |
| 30 | R¹ | 1 | 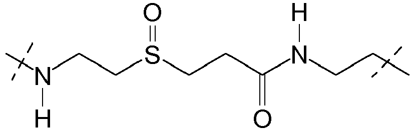 | 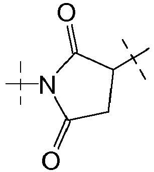 |
| 32 | R¹ | 1 | 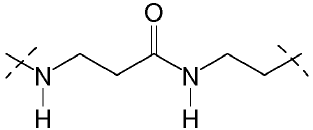 | 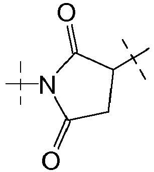 |
| 33 | R¹ | 1 | 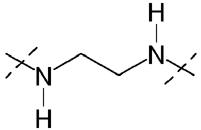 | 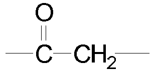 |
| 37 | R²-R⁴* | 0 | 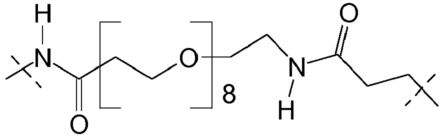 | 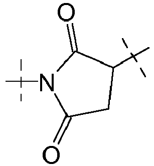 |
| 38 | R³ | 0 | 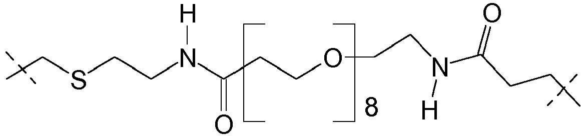 | 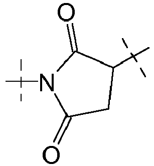 |
| 40 | R²-R⁴* | 0 | 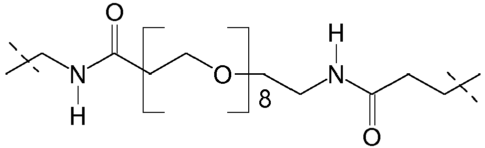 | 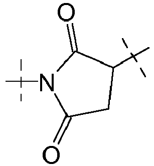 |
| 41 | R³ | 0 | 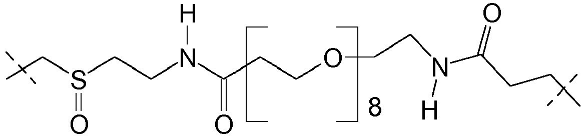 | 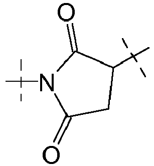 |
| 42 | R²-R⁴* | 1 | 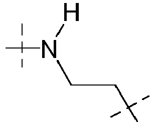 | 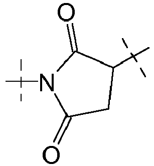 |
| 44 | R²-R⁴* | 0 | 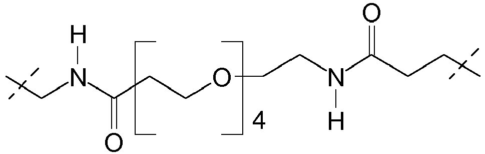 | 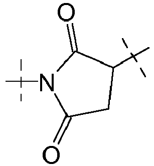 |
| 48 | R³ | 0 | 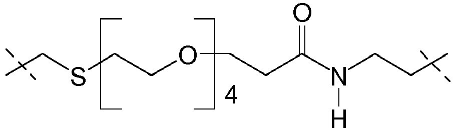 | 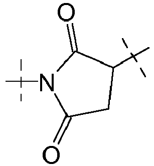 |
| 49 | R³ | 0 | 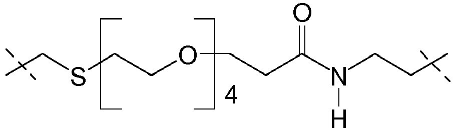 | 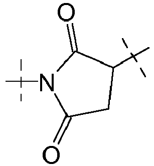 |
| 50 | R¹ | 1 | 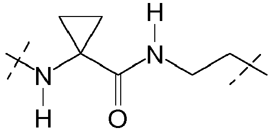 | 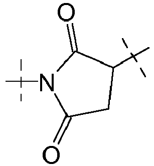 |
| 51 | R² | 0 | 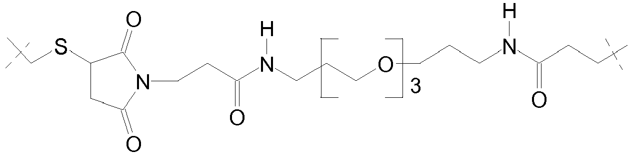 | 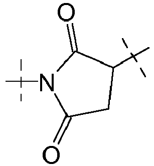 |
| 52 | R² | 0 | 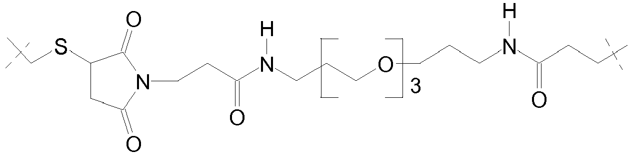 | 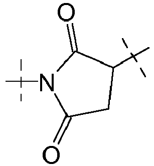 |
| 53 | R² | 0 | 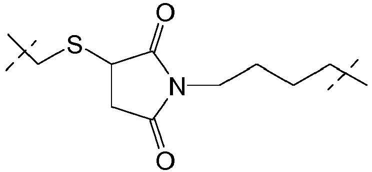 | 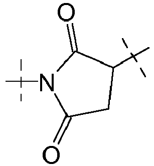 |
| 54 | R² | 0 | 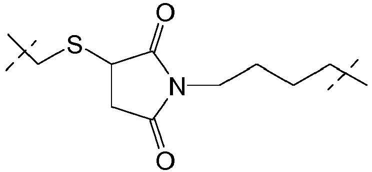 | 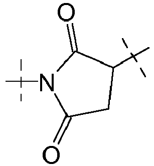 |
| 56 | R¹ | 1 | 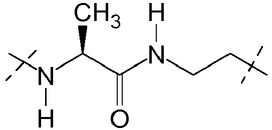 | 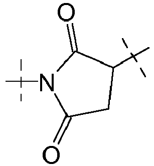 |
| 57 | R¹ | 1 | 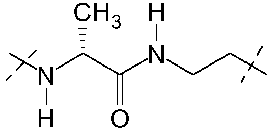 | 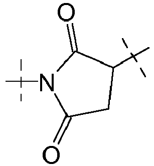 |

| | | | | |
|---|---|---|---|---|
| *R₂ und R⁴ bilden einen Pyrrolidinring, der mit dem Linker substituiert ist. **Besonders bevorzugt werden die in diesen Zeilen angegebenen Linker L1 mit einem Linker L2 verknüpft, der ausgewählt ist aus: | | | | |

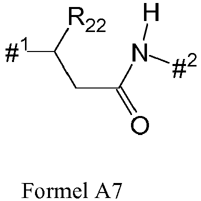

und/oder 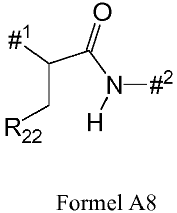 vor, wobei #¹ die Verknüpfungsstelle mit dem Schwefelatom des Binders kennzeichnet, #² die Verknüpfungsstelle mit der Gruppe L¹ kennzeichnet, R²² vorzugsweise COOH darstellt. In einem erfindungsgemäßen Konjugat bzw. in einem Gemisch der erfindungsgemäßen Konjugate liegen die Bindungen an einen Cysteinrest des Binders vorzugsweise zu über 80 %, besonders bevorzugt zu über 90 % (jeweils bezogen auf die Gesamtanzahl von Bindungen des Linkers an den Binder), besonders bevorzugt in einer der beiden Strukturen der Formel A7 oder A8 vor. Hierbei liegen die Strukturen der Formel A7 oder A8 in der Regel gemeinsam vor, vorzugsweise in einem Verhältnis von 60:40 bis 40:60 bezogen auf die Anzahl der Bindungen an den Binder. Die restlichen Bindungen liegen dann in der Struktur 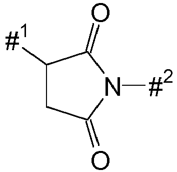 vor.

**Tabelle A'**

| Beisp. | Subst. | m | L1 | L2 |
|---|---|---|---|---|
| 105 | R¹ | 1 | 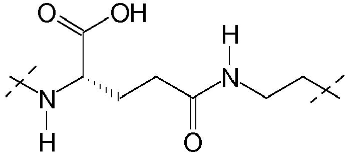 | 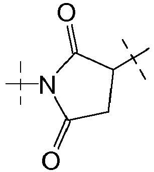 |
| 111 | R¹ | 1 | 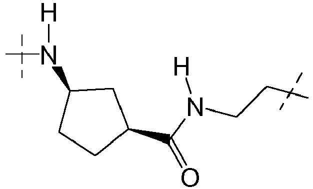 | 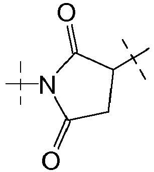 |
| 114 | R¹ | 1 | 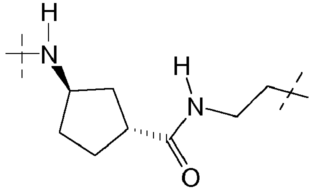 | 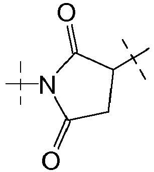 |
| 119/158 | R¹ | 1 | 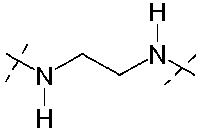 | 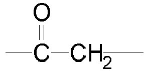 |
| 120 | R¹ | 1 | 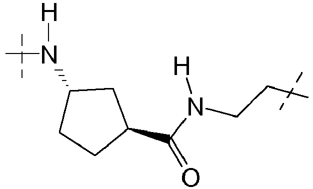 | 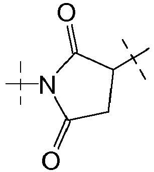 |
| 123/157/125 | R¹ | 1 | 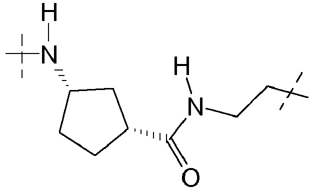 | 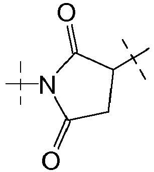 |
| 138/142 | R³ | 0 | 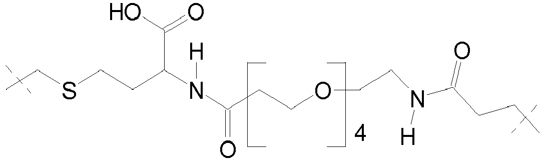 | 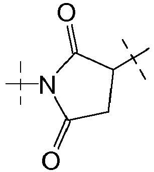 |
| 141/143 | R³ | 0 | 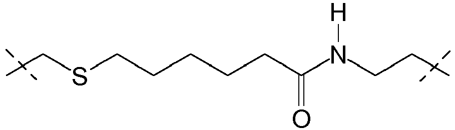 | 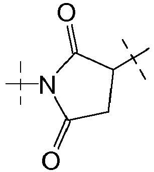 |
| 144 | R³ | 0 | 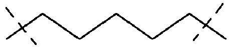 | 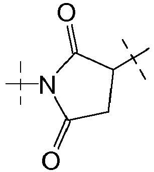 |
| 147 | R³ | 0 | 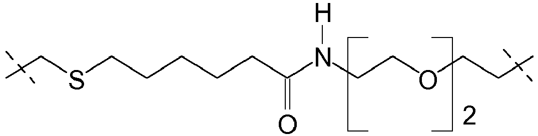 | 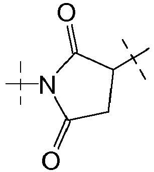 |
| 148 | R³ | 0 | 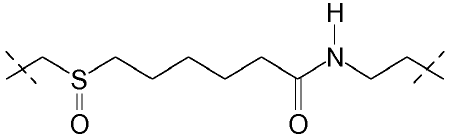 | 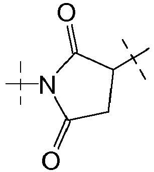 |
| 175 | R¹ | 1 | 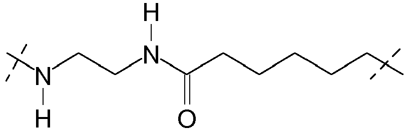 | 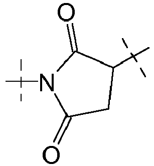 |
| 177 | R¹ | 1 | 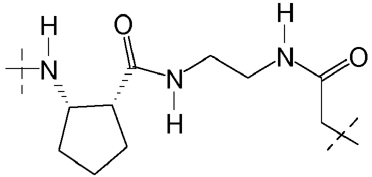 | 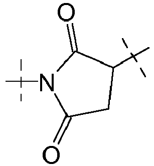 Siehe Anmerkung ** |
| 178 | R¹ | 1 | 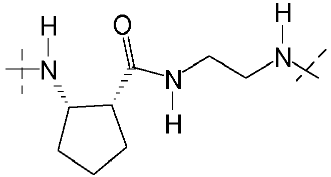 | 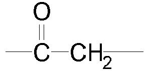 |
| 179 | R¹ | 1 | 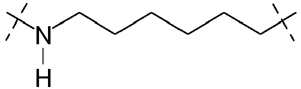 | 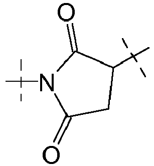 |
| 180 | R¹ | 1 | 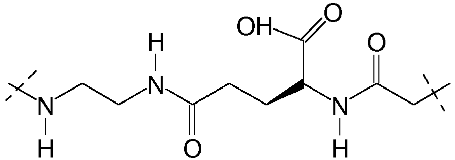 | 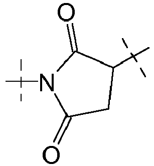 Siehe Anmerkung ** |
| 192 | R¹ | 1 | 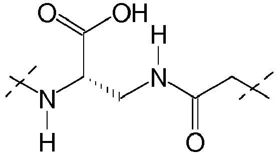 | 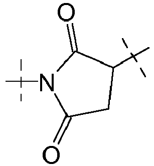 Siehe Anmerkung ** |
| 193 | R¹ | 1 | 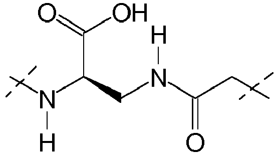 | 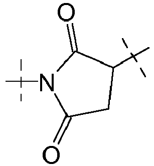 Siehe Anmerkung ** |
| 195 | R¹ | 0 | 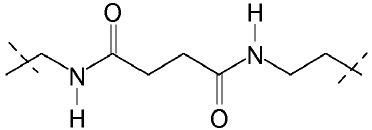 | 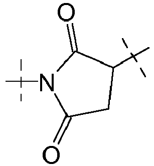 |
| 196 | R¹ | 1 | 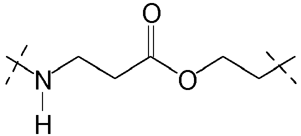 | 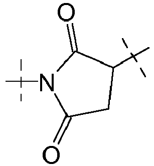 |
| 208/199 | R¹ | 1 | 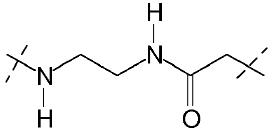 | 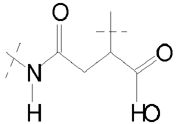 und |
| | | | | 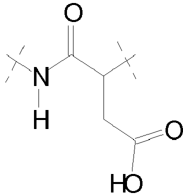 Siehe Anmerkung *** |
| 208/ 199 | R¹ | 1 | 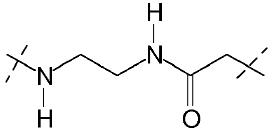 | 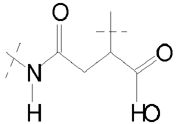 |
| 208/ 199 | R¹ | 1 | 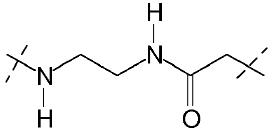 | 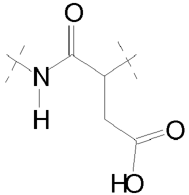 |
| 204 | R¹ | 1 | 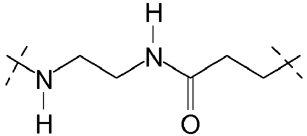 | 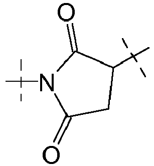 |
| 209/ 226 | R³ | 0 | 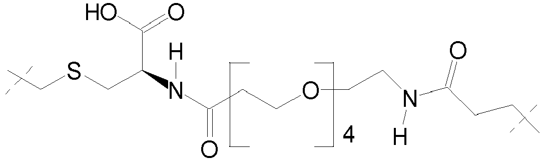 | 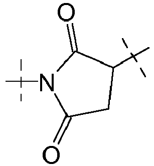 |
| 210 | R³ | 0 | 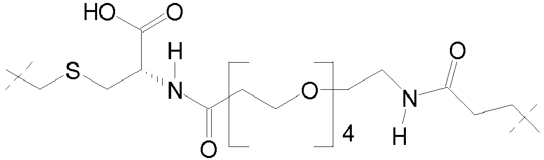 | 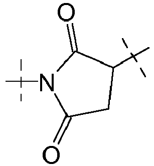 |
| 211 | R³ | 0 | 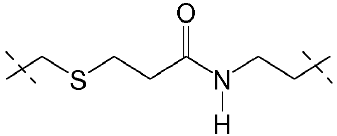 | 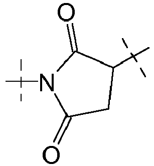 |
| 212 | R³ | 0 | 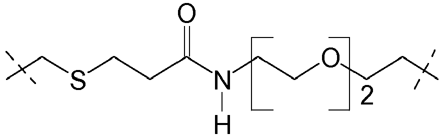 | 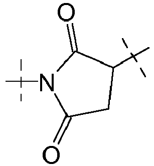 |
| 213 | R³ | 0 | 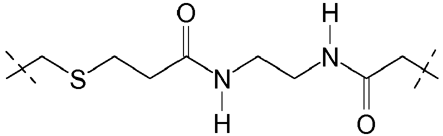 | 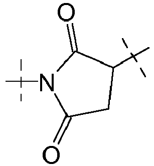 Siehe Anmerkung ** |
| 214 | R³ | 0 | 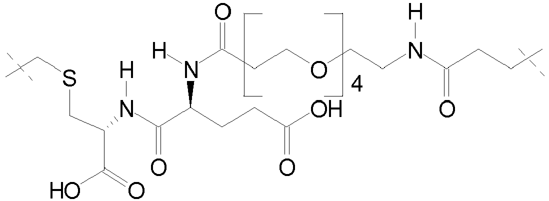 | 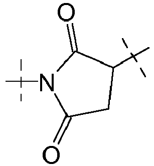 |
| 216 | R³ | 0 | 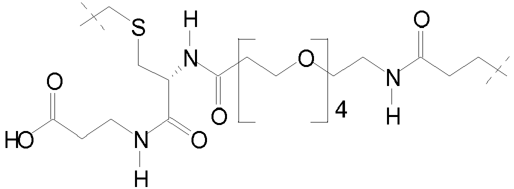 | 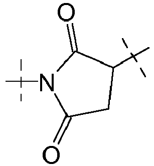 |
| 217 | R³ | 0 | 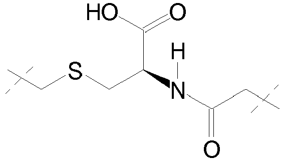 | 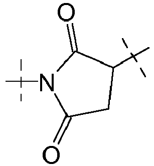 Siehe Anmerkung ** |
| 218 | R³ | 0 | 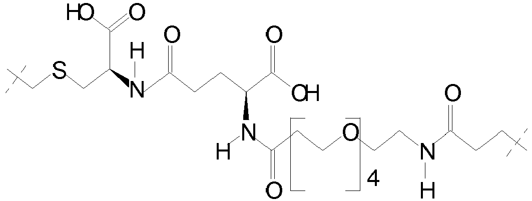 | 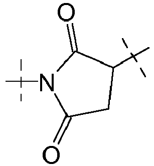 |
| 238 | R² | 0 | 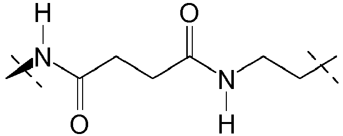 | 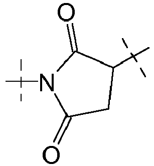 |
| | R¹ | 1 | 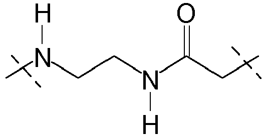 | 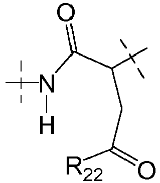 Mit R₂₂ = -OH oder _NH₂ |
| | R¹ | 1 | 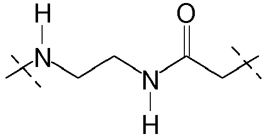 | 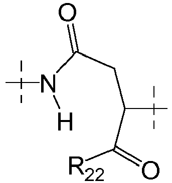 Mit R₂₂ = -OH oder _NH₂ |
| 234 | R¹ | 1 | 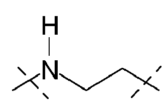 | 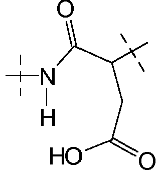 und 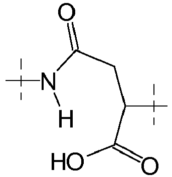 Siehe Anmerkung *** |
| 234 | R¹ | 1 | 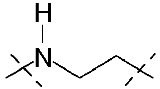 | 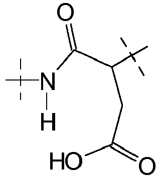 |
| 234 | R¹ | 1 | 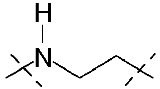 | 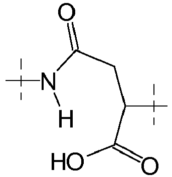 |
| 236 | R¹ | 1 | 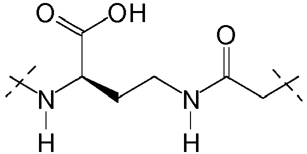 | 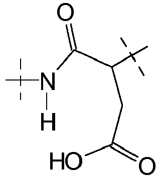 und 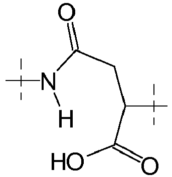 Siehe Anmerkung *** |
| 236 | R¹ | 1 | 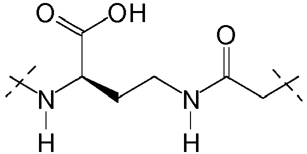 | 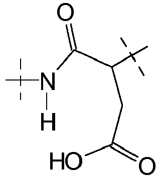 |
| 236 | R¹ | 1 | 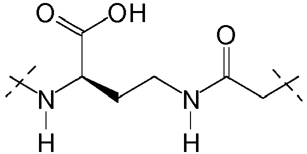 | 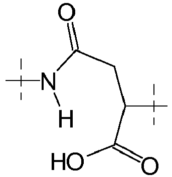 |
| 237 | R³ | 0 | 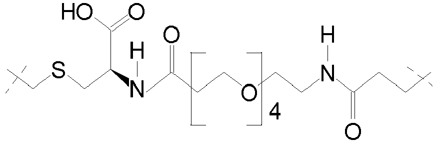 | 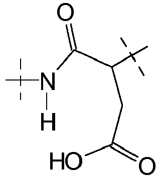 und |
| | | | | 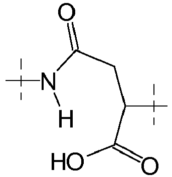 Siehe Anmerkung *** |
| 237 | R³ | 0 | 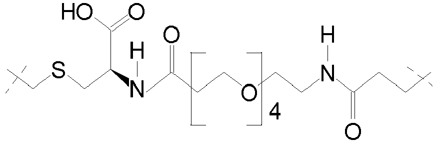 | 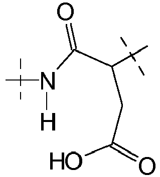 |
| 237 | R³ | 0 | 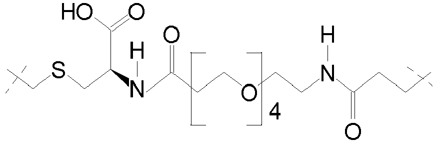 | 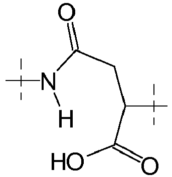 |
| 239 | R³ | 0 | 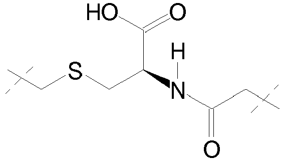 | 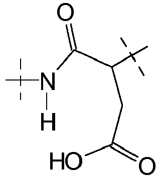 und 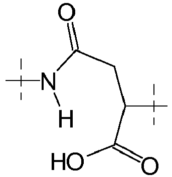 Siehe Anmerkung *** |
| 239 | R³ | 0 | 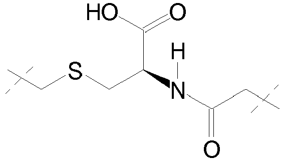 | 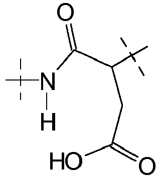 |
| 239 | R³ | 0 | 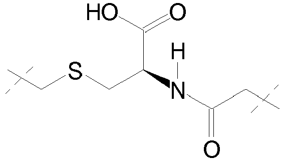 | 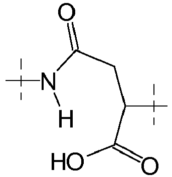 |
| 240 | R³ | 0 | 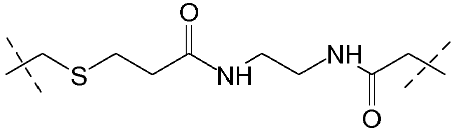 | 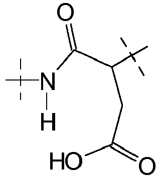 und 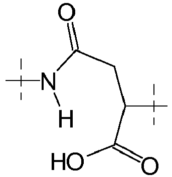 Siehe Anmerkung *** |
| 240 | R³ | 0 | 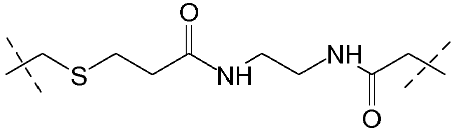 | 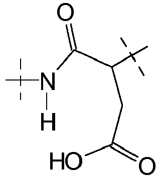 |
| 240 | R³ | 0 | 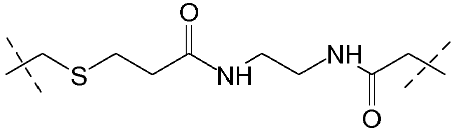 | 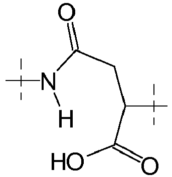 |
| 241 | R¹ | 1 | 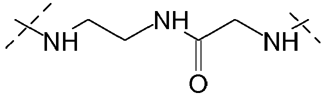 | 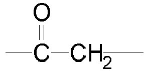 |
| 242 | R¹ | 1 | 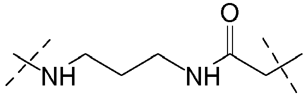 | 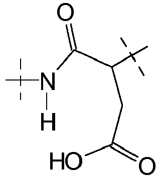 und 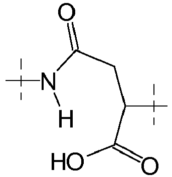 Siehe Anmerkung *** |
| 242 | R¹ | 1 | 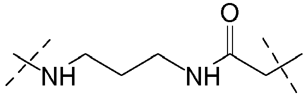 | 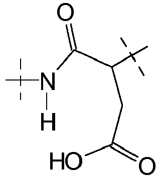 |
| 242 | R¹ | 1 | 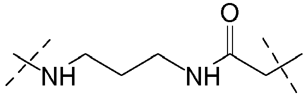 | 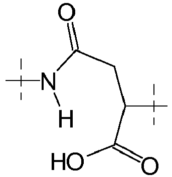 |
| 243 | R¹ | 1 | 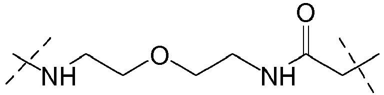 | 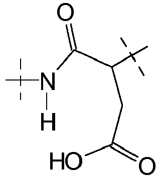 und 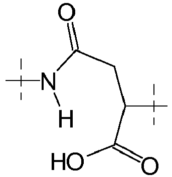 Siehe Anmerkung *** |
| 243 | R¹ | 1 | 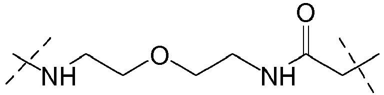 | 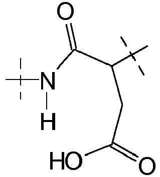 |
| 243 | R¹ | 1 | 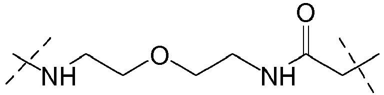 | 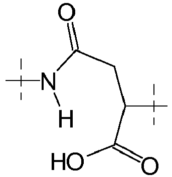 |
| 244 | R3 | 0 | 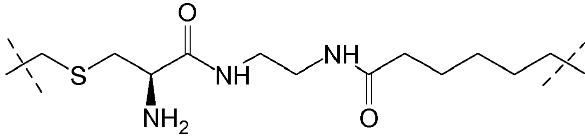 | 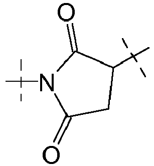 |
| 245 | R1 | 0 | 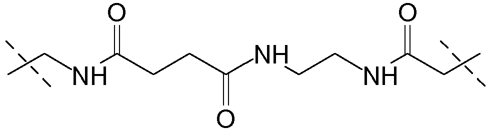 | 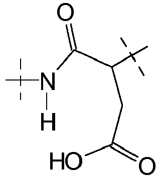 und 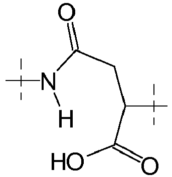 Siehe Anmerkung *** |
| 245 | R1 | 0 | 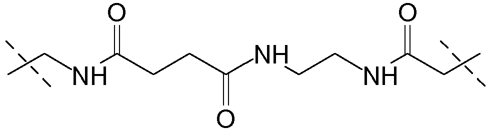 | 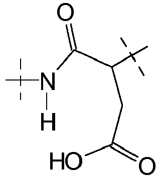 |
| 245 | R1 | 0 | 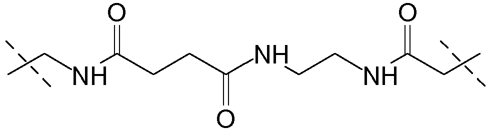 | 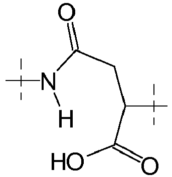 |
| 247 | R1 | 1 | 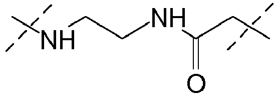 | 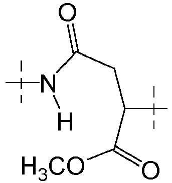 |
| 248 | R1 | 1 | 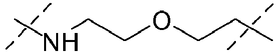 | 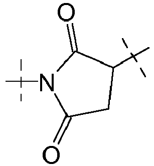 |

| | | | | |
|---|---|---|---|---|
| **: Siehe Anmerkung ** zu Tabelle A. ***: Bei Vorhandensein dieser Struktur L2 kann zugleich eine Struktur L2 der folgenden Formel vorliegen: 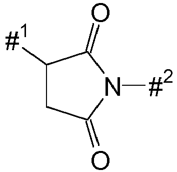 | | | | |

Beispiele für Konjugate mit entsprechenden Linkern weisen folgende Strukturen auf, wobei X1, X2, X3, und L1 die oben angegebenen Bedeutungen aufweisen, L2 und L3 dieselbe Bedeutung wie L1 hat, AK1 für einen Antikörper steht, der über einen Cysteinrest gebunden ist, und n eine Zahl von 1 bis 10 ist. Besonders bevorzugt ist AK1 ein humaner, humanisierter oder chimärer monoklonaler Antikörper oder ein Antigen-bindendes Fragment hiervon ist, insbesondere ein anti-TWEAKR-Antikörper oder ein Antigen-bindendes Fragment hiervon oder ein anti-EGFR-Antikörper oder ein Antigen-bindendes Fragment hiervon. Besonders bevorzugt sind ein anti-TWEAKR-Antikörper, der spezifisch an die Aminosäure D in Position 47 (D47) von TWEAKR (SEQ ID NO:169) bindet, insbesondere der anti-TWEAKR-Antikörper TPP-2090, oder die anti-EGFR-Antikörper Cetuximab oder Nimotuzumab.

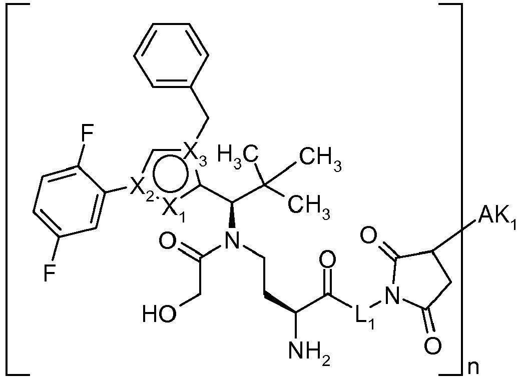

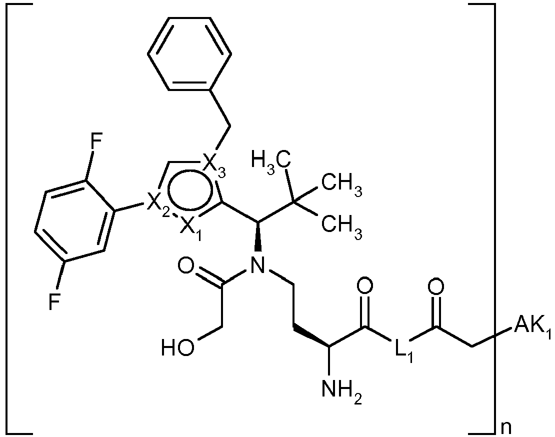

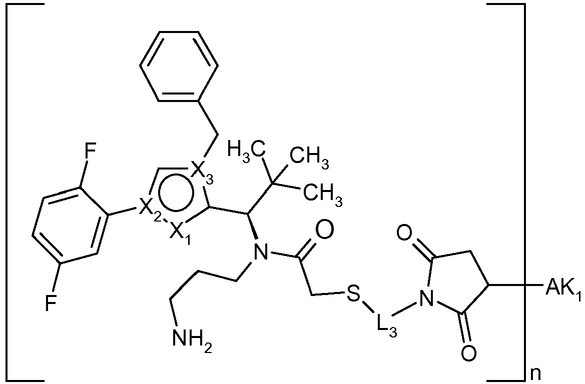

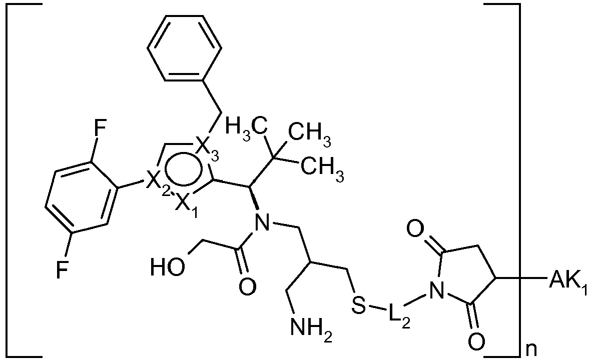

Wenn der Linker an eine Lysin-Seitenkette bzw. einen Lysinrest gebunden ist, weist er vorzugsweise die folgende Formel aufweist:

-§-(SG)ₓ-L4-CO-§§,

wobei
§ die Bindung an das Wirkstoffmolekül darstellt und
§ § die Bindung an das Binderpeptid oder -protein darstellt,
x 0 oder 1 darstellt,
SG eine spaltbare Gruppe, vorzugsweise ein 2-8 Oligopeptid, besonders, bevorzugt ein Dipeptid darstellt,
und
L4 eine Einfachbindung oder eine Gruppe -(CO)_{y}-G4- darstellt, wobei y 0 oder 1 darstellt, und G4 eine geradkettige oder verzweigte Kohlenwasserstoffkette mit 1 bis 100 Kohlenstoffatomen aus Arylengruppen und/oder geradkettigen und/oder verzweigte und/oder cyclischen Alkylengruppen ist, die einfach oder mehrfach durch eine oder mehrere der Gruppen -O-, -S-, -SO-, SO₂, -NH-, -CO-, -NHCO-, -CONH-, -NMe-, -NHNH-, -SO2NHNH-, -CONHNH- und einen 5 bis 10-gliedriger, aromatischen oder nicht aromatischen Heterozyklus mit bis zu 4 Heteroatomen ausgewählt aus N, O und S, -SO- oder - SO2- unterbrochen sein kann (vorzugsweise 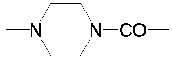 ), wobei die Seitenketten, sofern vorhanden, mit -NHCONH₂, -COOH, -OH, -NH₂, NH-CNNH₂, Sulfonamid, Sulfon, Sulfoxid oder Sulfonsäure substituiert sein können.

In der folgenden Tabelle B sind Beispiele für Linker an einen Lysinrest angegeben. In der Tabelle ist weiterhin die bevorzugte Ankopplungsstelle (R¹-R⁵) angegeben. In der ersten Spalte sind weiterhin die Beispielnummern angegeben, in denen entsprechende Linker Anwendung finden.

**Tabelle B: Lysin-Linker**

| -§-(SG)ₓ-L4-CO-§§, | | |
|---|---|---|
| Beisp. | Subst. | (SG)ₓ-L4 |
| 1A | R¹ | Einfachbindung |
| 58/194 | R⁴ | 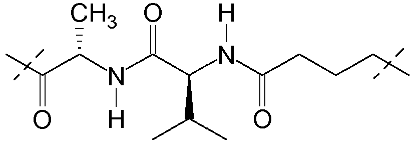 |

Beispiele für Konjugate mit entsprechenden Linkern weisen folgende Strukturen auf, wobei X1, X2, X3, und L4 die oben angegebenen Bedeutungen aufweisen, AK2 für einen Antikörper steht, der über einen Lysinrest gebunden ist, und n eine Zahl von 1 bis 10 ist. Besonders bevorzugt ist AK2 ein humaner, humanisierter oder chimärer monoklonaler Antikörper oder ein Antigen-bindendes Fragment hiervon ist, insbesondere ein anti-TWEAKR-Antikörper oder ein Antigen-bindendes Fragment hiervon oder ein anti-EGFR-Antikörper oder ein Antigen-bindendes Fragment hiervon. Besonders bevorzugt sind ein anti-TWEAKR-Antikörper, der spezifisch an die Aminosäure D in Position 47 (D47) von TWEAKR (SEQ ID NO:169) bindet, insbesondere der anti-TWEAKR-Antikörper TPP-2090, oder die anti-EGFR-Antikörper Cetuximab oder Nimotuzumab.

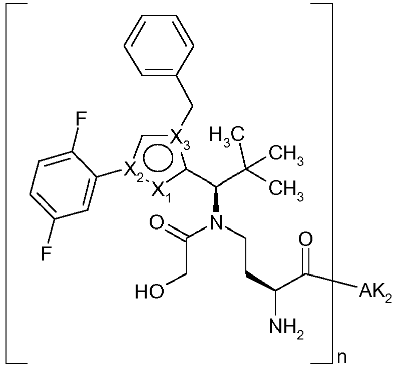

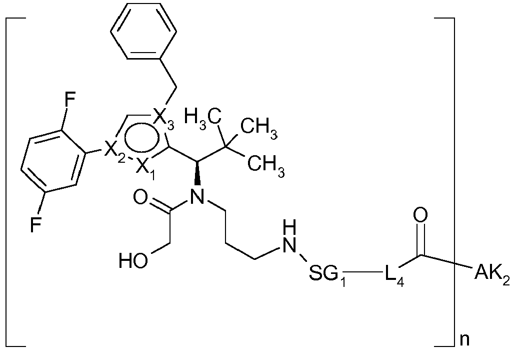

Weiterhin ist erfindungsgemäß bevorzugt die Grundstruktur (i), (ii), oder (iv), wobei SG1 bzw. SG eine durch Cathepsin spaltbare Gruppe darstellt, und L1 und L2 die oben aufgeführten Bedeutungen aufweisen. Besonders bevorzugt sind die folgenden Gruppen:
- -Val-Ala-CONH- (hierdurch Spaltung der Amid-Bindung am C-terminalen Amid von Alanin)
- -NH-Val-Lys-CONH- (Spaltung der Amid-Bindung am C-terminalen Amid von Lysin)
- -NH-Val-Cit-CONH- (Spaltung der Amid-Bindung am C-terminalen Amid von Citrullin)
- -NH-Phe-Lys-CONH (Spaltung der Amid-Bindung am C-terminalen Amid von Lysin)
- -NH-Ala-Lys-CONH- (Spaltung der Amid-Bindung am C-terminalen Amid von Lysin)
- -NH-Ala-Cit-CONH- (Spaltung der Amid-Bindung am C-terminalen Amid von Citrullin) SG1 bzw. SG ist besonders bevorzugt 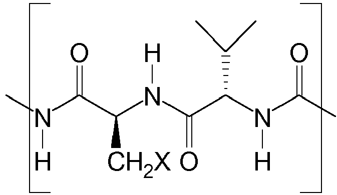 oder 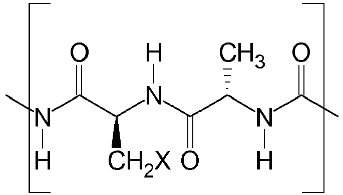 bzw. 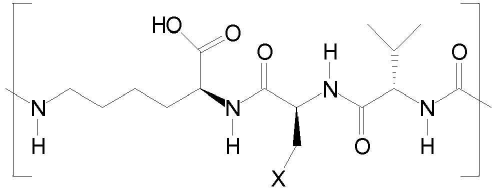 wobei X H, eine C₁₋₁₀-Alkylgruppe ist, die gegebenenfalls mit -NHCONH₂, -COOH, -OH, NH₂, -NH-CNNH₂, oder Sulfonsäure substituiert sein kann.

In der folgenden Tabelle C sind Beispiele für einen Linkerteil -SG1-L1- bzw. -L1-SG-L1- angegeben, wobei SG1 bzw. SG eine durch Cathepsin spaltbare Gruppe ist. In der Tabelle C ist weiterhin angegeben, mit welcher Gruppe L2 diese Beispiele für -SG1-L1- bzw. -L1-SG-L1- vorzugsweise kombiniert werden, sowie die bevorzugte Ankopplungsstelle (R¹-R⁵) sowie der bevorzugte Wert für m, also ob vor L1 eine Carbonylgruppe vorliegt oder nicht (vgl. §-(CO)m-L1-L2-§§). Diese Linker werden vorzugsweise an einen Cysteinrest gekoppelt. In der ersten Spalte sind weiterhin beispielhaft für die Cetuximab ADCs die Beispielnummern angegeben, in denen entsprechende Linker Anwendung finden. Sie gelten in gleicher Weise für die entsprechenden ADCs mit anderen Antikörpern. Die L1-Gruppe ist in einem Kasten hervorgehoben. Diese Gruppen L1 können jedoch durch eine der Gruppe L1, die für die obige Formel §-(CO)m-L1-L2-§§ angegeben ist, ausgetauscht werden. Wenn L2 ein Bernsteinsäureamid ist bzw. sich hiervon ableitet, kann dieses Amid ganz oder teilweise auch in Form des hydrolysierten offenkettigen Bernsteinsäureamids vorliegen, wie oben beschrieben.

**Tabelle C**

| Beisp. | Subst. | m | -SG1-L1- bzw. -L1-SG-L1- | L2 |
|---|---|---|---|---|
| 8A/163A | R¹ | 1 | 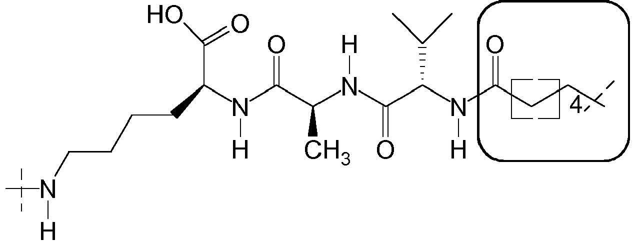 | 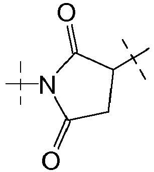 |
| 14A | R¹ | 1 | 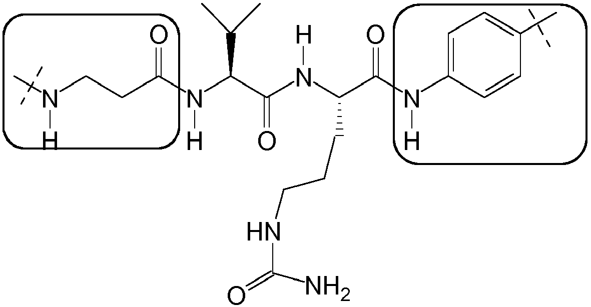 | 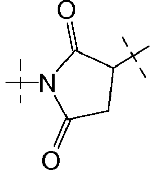 |
| 15A | R¹ | 1 | 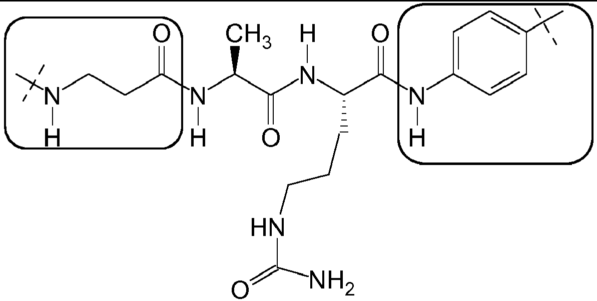 | 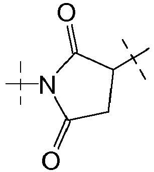 |
| 16A | R¹ | 1 | 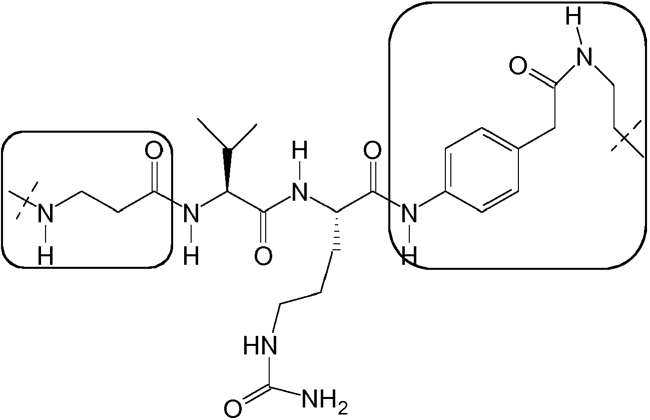 | 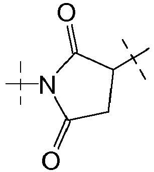 |
| 17A/168A | R¹ | 1 | 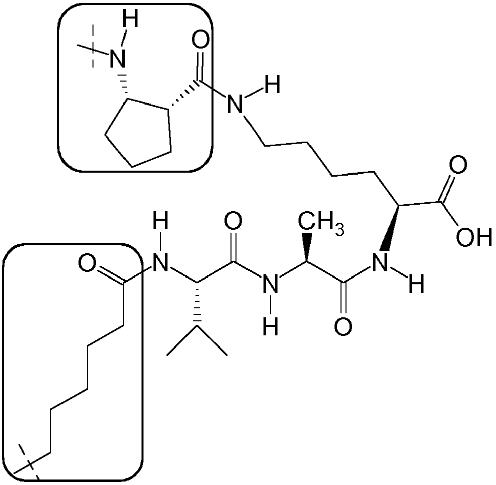 | 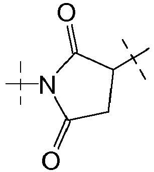 |
| 18A | R¹ | 1 | 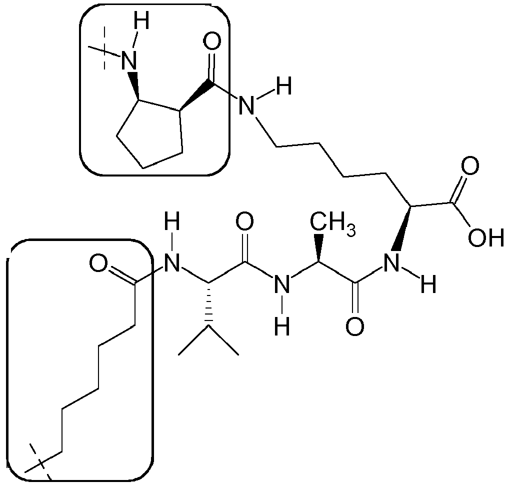 | 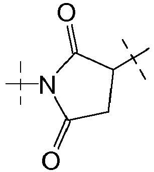 |
| 23A/164A | R¹ | 1 | 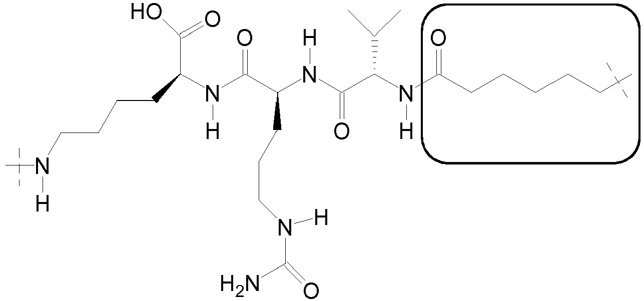 | 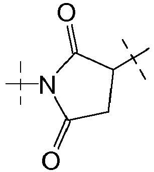 |
| 24A | R¹ | 1 | 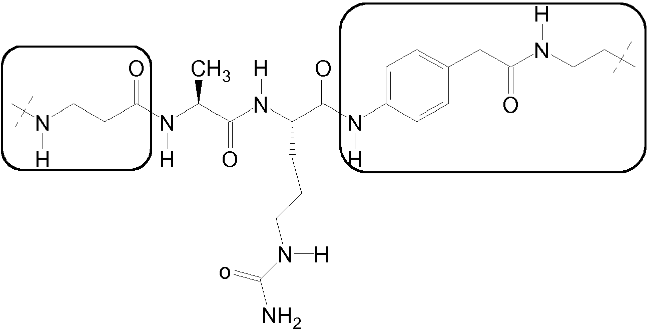 | 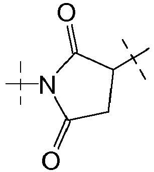 |
| 25A | R¹ | 1 | 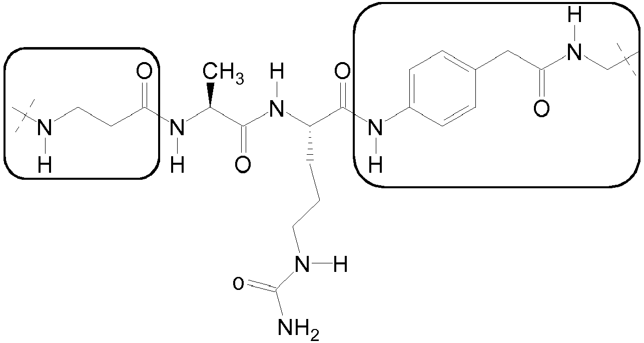 | 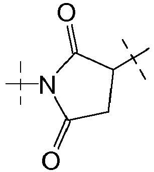 |
| 26A | R¹ | 1 | 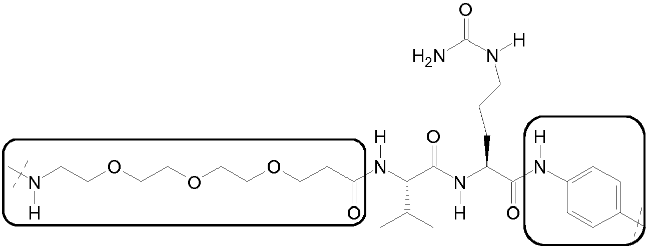 | 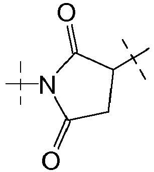 |
| 27A | R¹ | 1 | 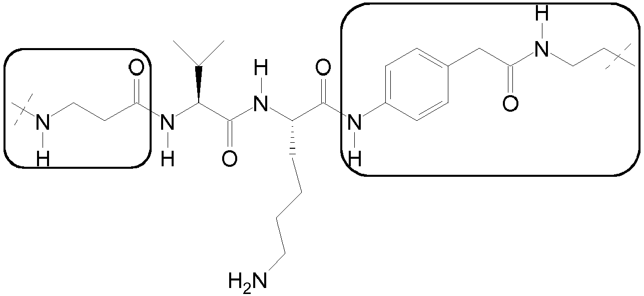 | 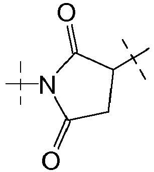 |
| 28A | R¹ | 1 | 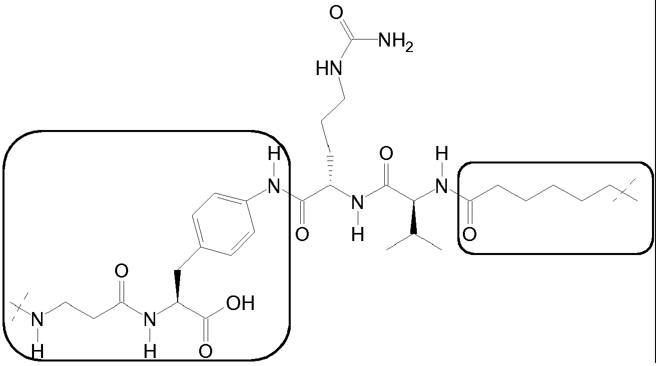 | 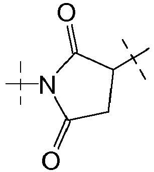 |
| 29A | R¹ | 1 | 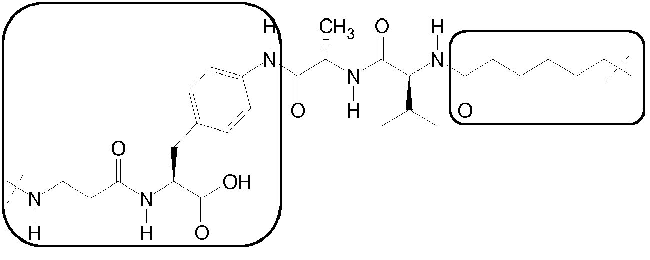 | 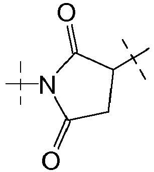 |
| 31A | R⁵ (m) | 0 | 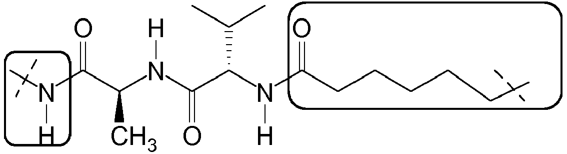 | 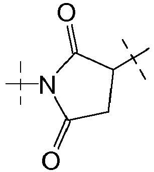 |
| 34A | R¹ | 1 | 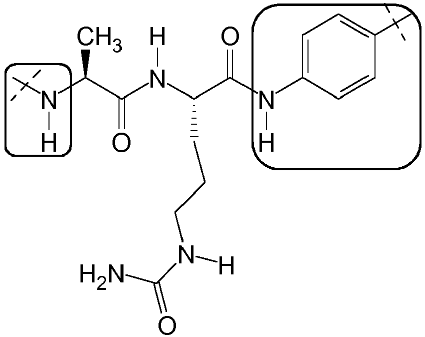 | 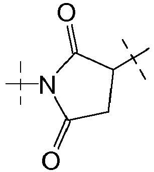 |
| 35A | R⁴ | 0 | 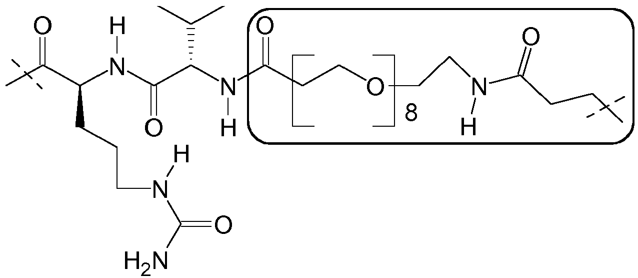 | 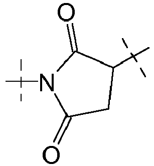 |
| 36A | R⁴ | 0 | 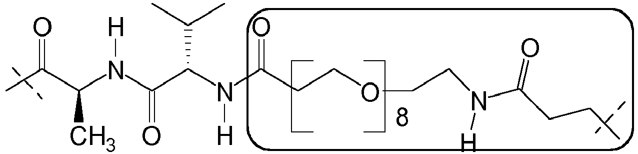 | 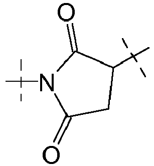 |
| 39A/215A | R⁴ | 0 | 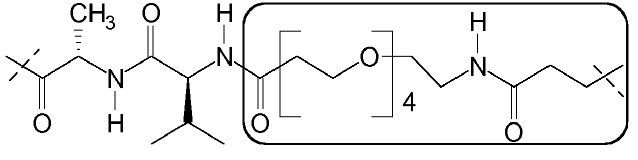 | 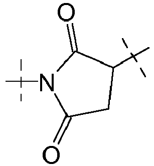 |
| 43A | R⁴ | 0 | 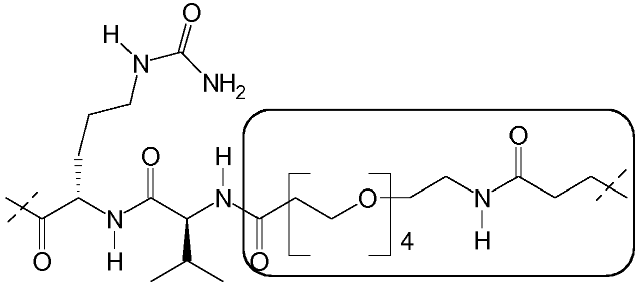 | 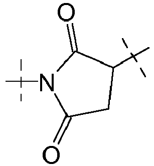 |
| 45A | R⁴ | 0 | 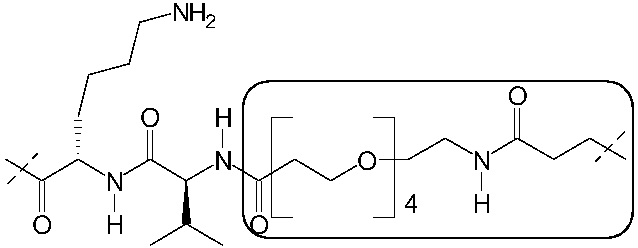 | 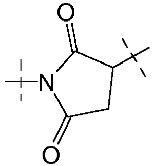 |
| 46A | R⁴ | 0 | 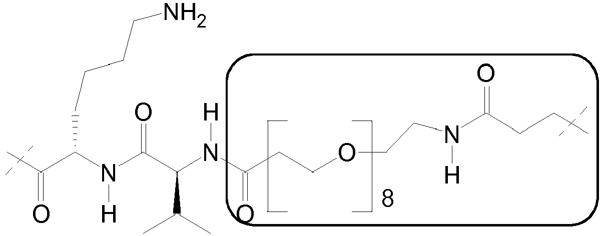 | 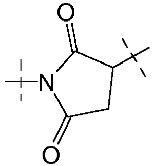 |
| 47A | R⁴ | 0 | 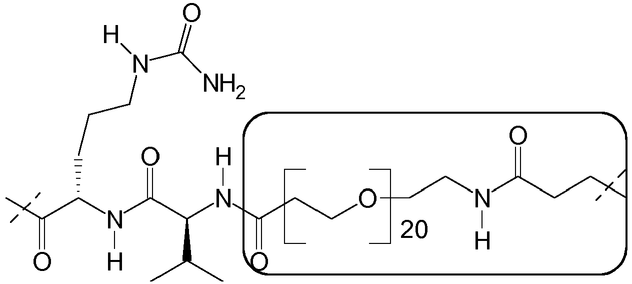 | 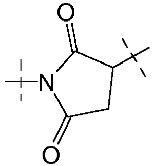 |
| 55A | R³ | 0 | 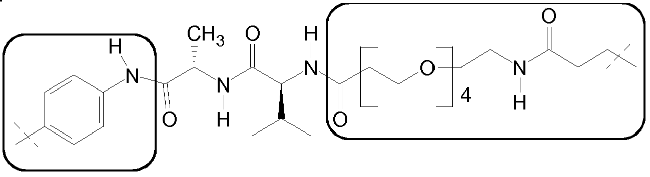 | 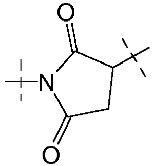 |
| 55B | R³ | 0 | 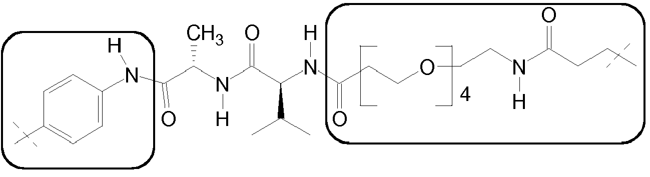 | 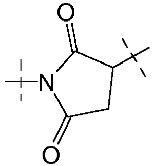 |
| 110A/156A | R¹ | 1 | 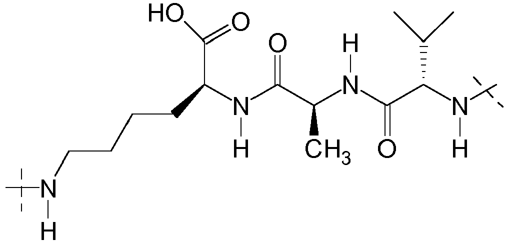 | 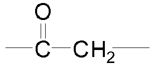 |
| 128A | R¹ | 1 | 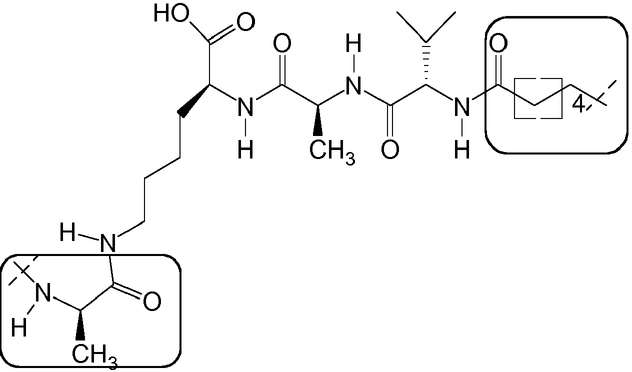 | 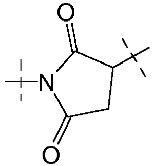 |
| 129A/167A | R¹ | 1 | 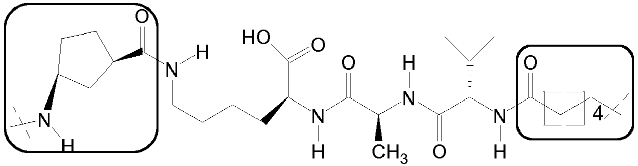 | 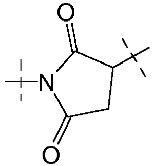 |
| 150A | R³ | 0 | 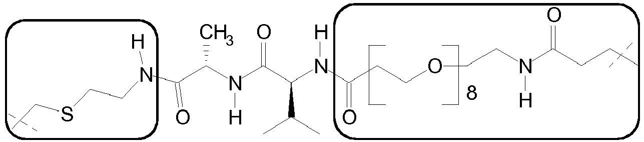 | 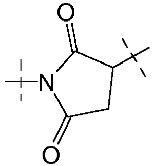 |
| 154A/155A/160 | R¹ | 1 | 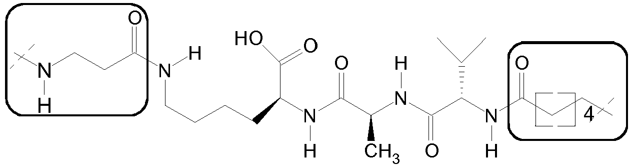 | 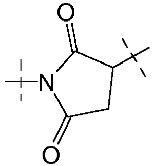 |
| 165A | R¹ | 1 | 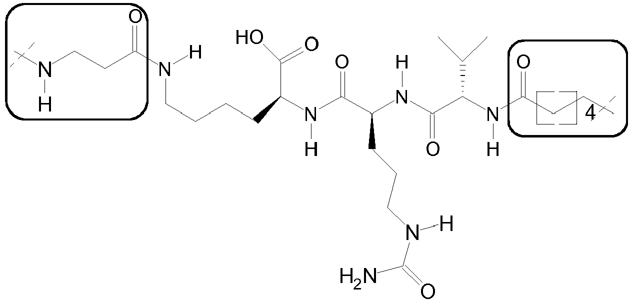 | 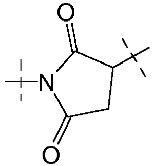 |
| 166A | R¹ | 1 | 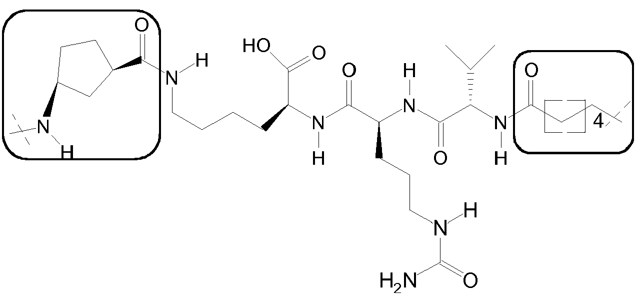 | 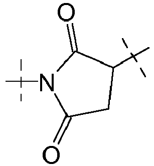 |
| 169A | R¹ | 1 | 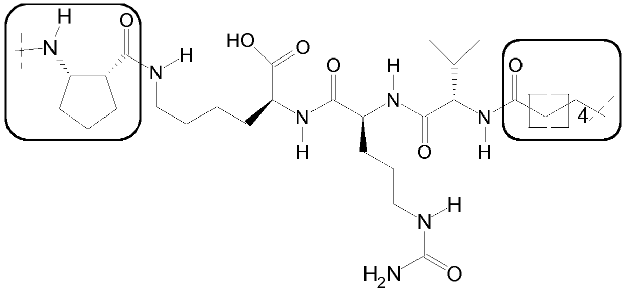 | 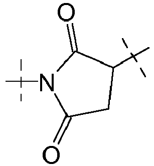 |
| 170A | R¹ | 1 | 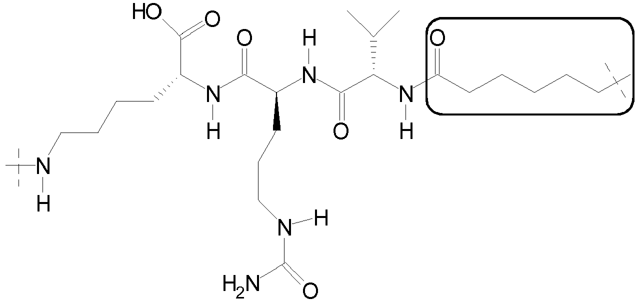 | 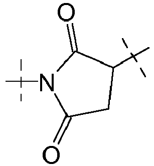 |
| 171A | R¹ | 1 | 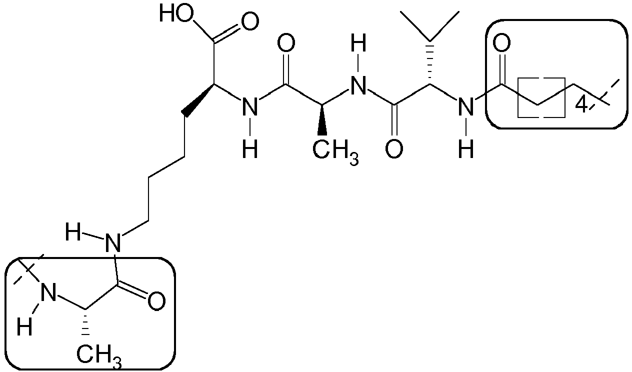 | 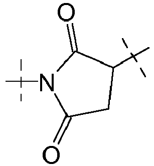 |
| 172A/173A/174A | R¹ | 1 | 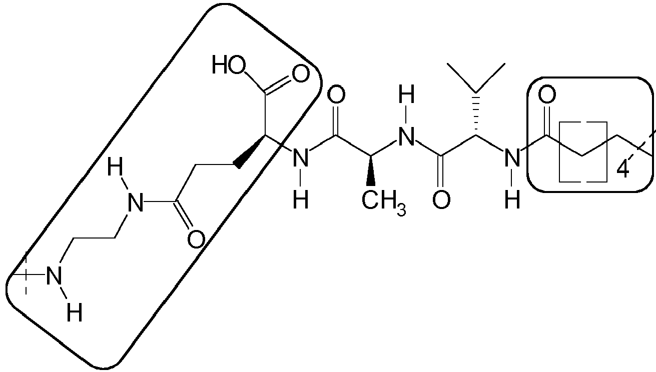 | 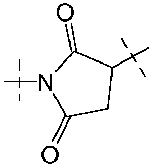 |
| 205A | R¹ | 1 | 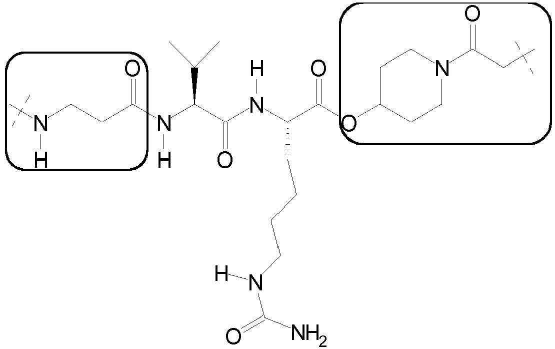 | 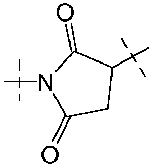 |
| 206A | R¹ | 1 | 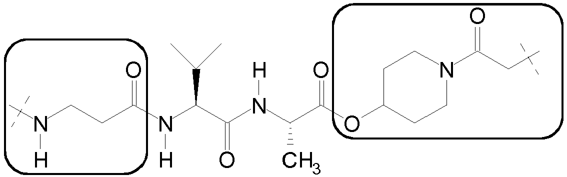 | 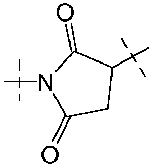 |
| 207A | R¹ | 1 | 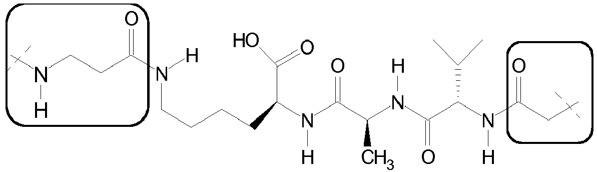 | 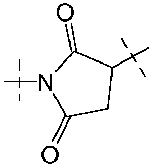 |
| 235A | R³ | 0 | 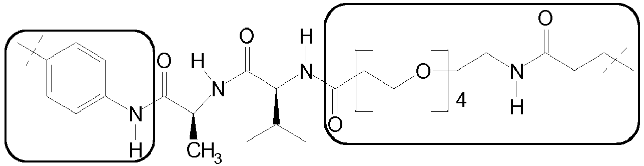 | 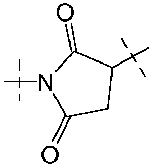 |

Beispiele für Konjugate mit der Grundstruktur (i) weisen die folgende Struktur auf, wobei X1, X2, und X3 die oben angegebenen Bedeutungen aufweisen, L4 dieselbe Bedeutung wie L1 hat, AK1 für einen Antikörper steht, der über einen Cysteinrest gebunden ist, und n eine Zahl von 1 bis 10 ist. Besonders bevorzugt ist AK1 ein anti-TWEAKR-Antikörper, insbesondere einer der spezifisch an die Aminosäure D in Position 47 (D47) von TWEAKR (SEQ ID NO:169) bindet, insbesondere der anti-TWEAKR-Antikörper TPP-2090.

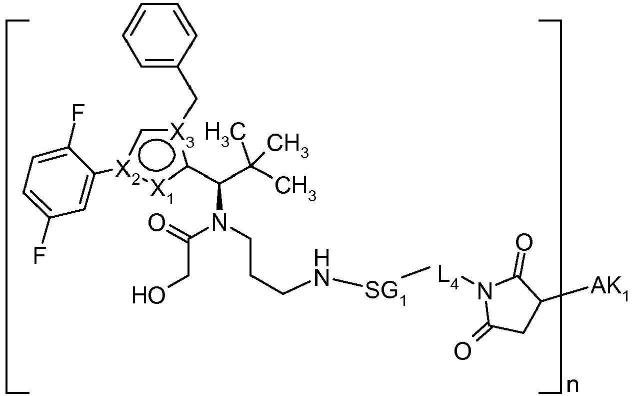

### KSP-Inhibitor - Linker-Intermediate und Herstellung der Konjugate

Die erfindungsgemäßen Konjugate werden hergestellt, in dem zunächst der niedermolekulare KSP-Inhibitor mit einem Linker versehen wird. Da so erhaltene Intermediat wird dann mit dem Binder (vorzugsweise Antikörper) umgesetzt.

Für die Kopplung an einen Cysteinrest wird vorzugsweise eine der folgenden Verbindungen mit dem Cystein-haltigen Binder wie z.B. einem Antikörper, der dazu ggf. partiell reduziert wurde, umgesetzt: 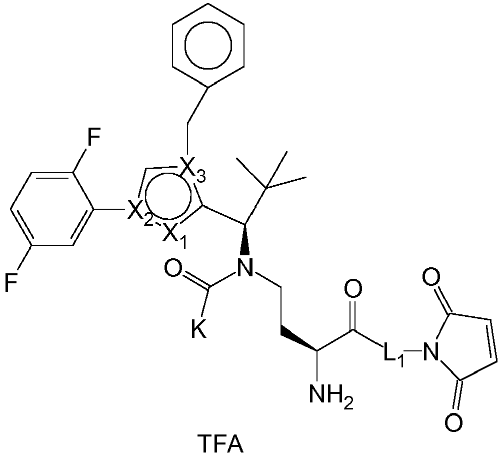 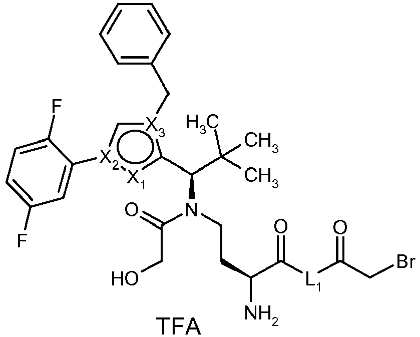 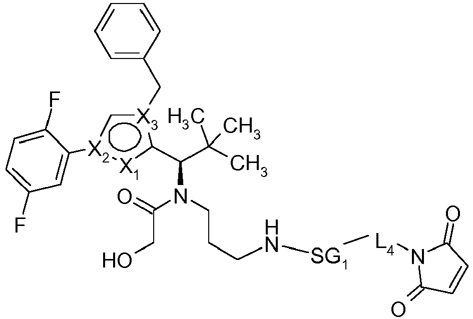 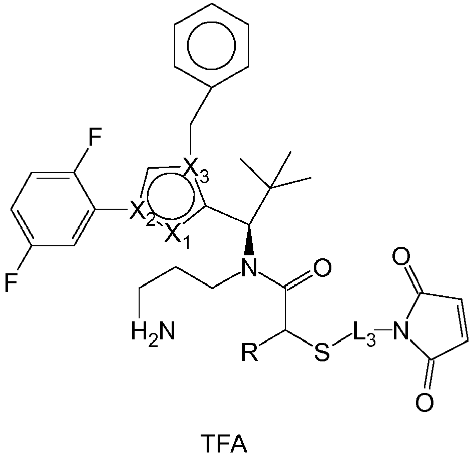 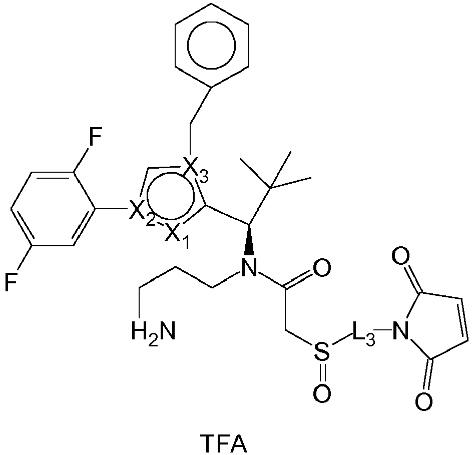 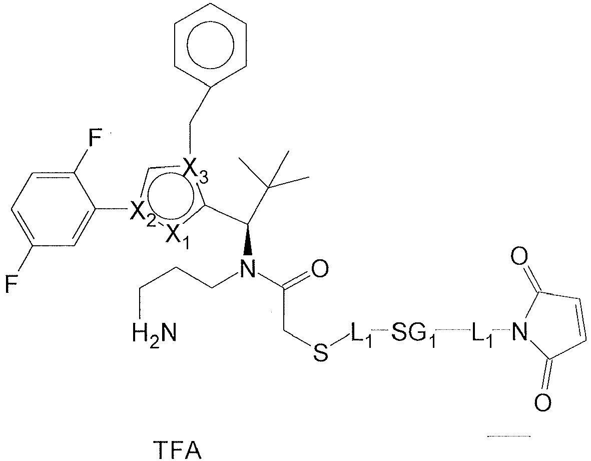 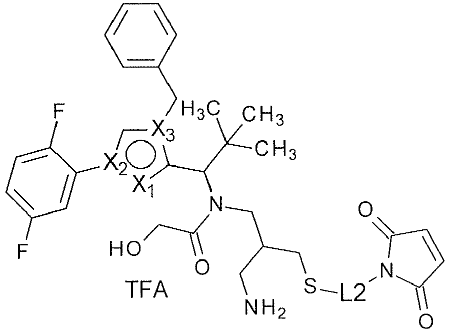 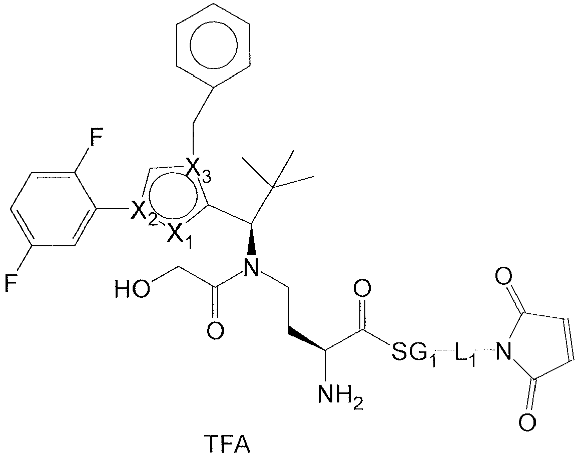 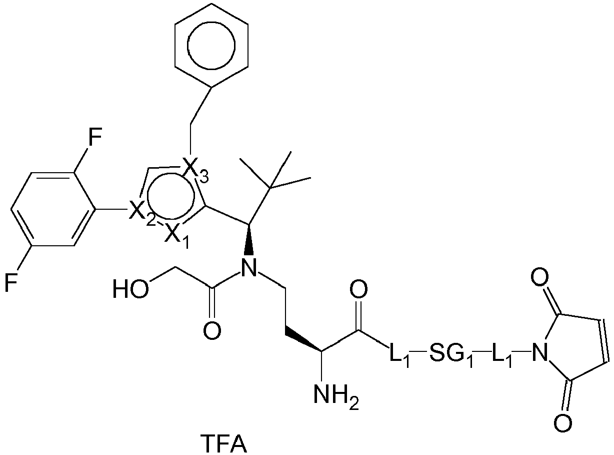 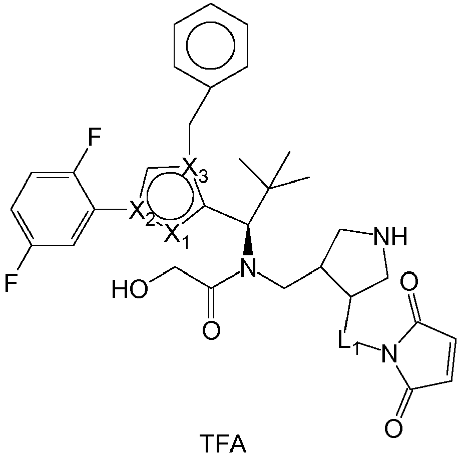 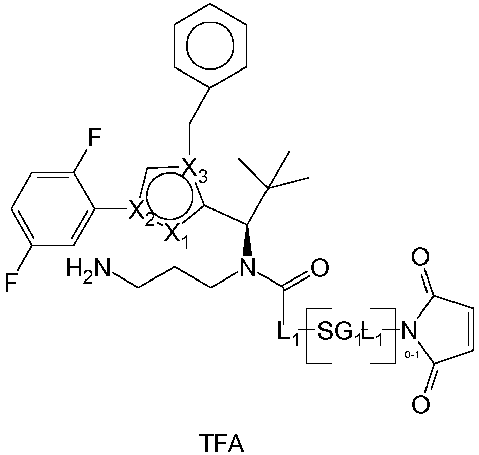 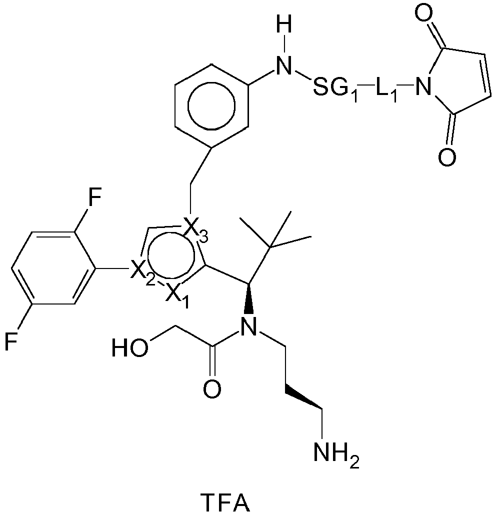
wobei R -H oder -COOH darstellt,
wobei K lineares oder verzweigtes, gegebenenfalls mit C₁-C₆ Alkoxy oder -OH substituiertes C₁-C₆ Alkyl darstellt, und
wobei X₁, X₂, X₃, SG1, L1, L2, L3 und L4 die gleiche Bedeutung haben wie oben beschrieben.

Die Verbindung kann z.B in Form ihres Trifluoressigsäuresalzes verwendet werden. Zur Reaktion mit dem Binder wie z.B. dem Antikörper wird die Verbindung vorzugsweise in 2 bis 12 fachem molaren Überschuss gegenüber dem Binder verwendet.

Für die Kopplung an einen Lysinrest wird vorzugsweise eine der folgenden Verbindungen mit dem Lysin-haltigen Binder wie z.B. einem Antikörper umgesetzt: 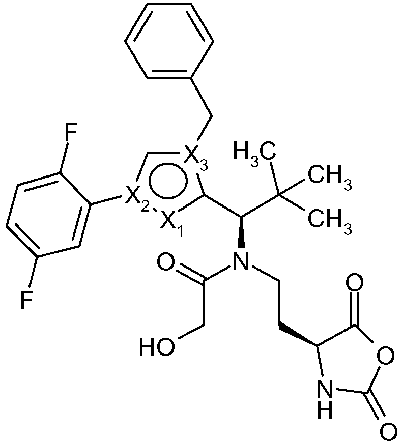 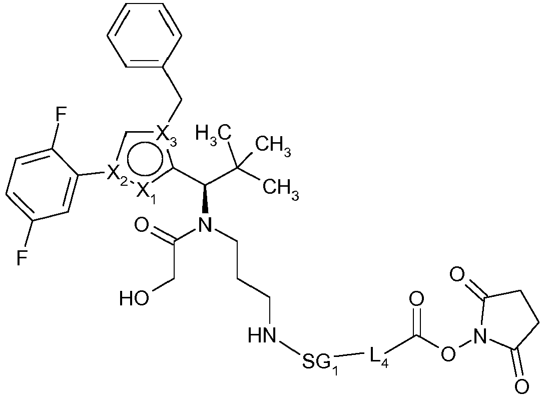 wobei X₁, X₂, X₃ die gleiche Bedeutung haben wie in Formel (II), und L4 die gleiche Bedeutung wie L1 hat, und L1 die gleiche Bedeutung hat wie oben beschrieben,

Für ein an einen Cysteinrest ankoppelndes Intermediat können die Reaktionen wie folgt veranschaulicht werden:

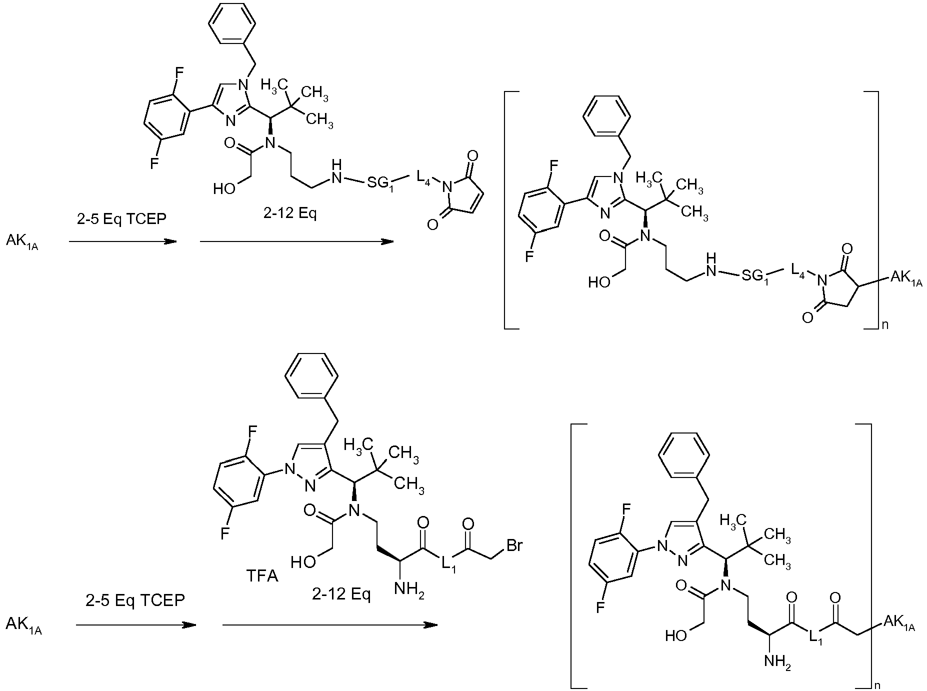

Die anderen Intermediate und andere Antikörper können entsprechend umgesetzt werden.

Für ein an einen Lysinrest ankoppelndes Intermediat kann die Reaktion wie folgt veranschaulicht werden:

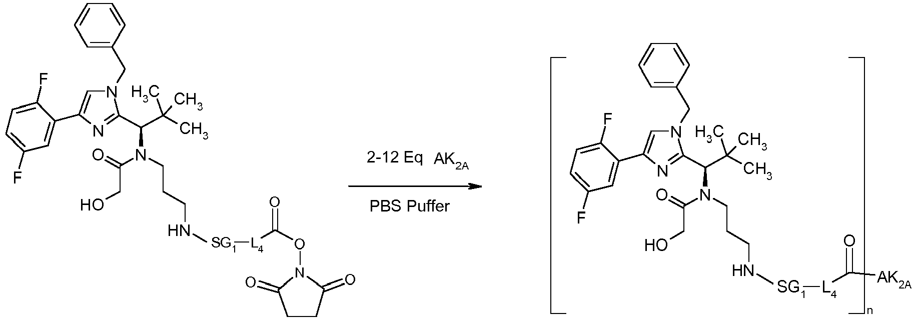

Erfindungsgemäß werden so die folgenden Konjugate erhalten:

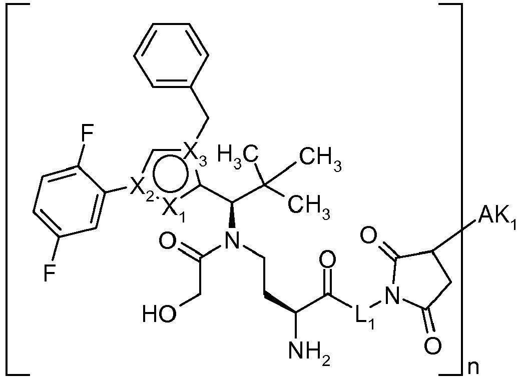

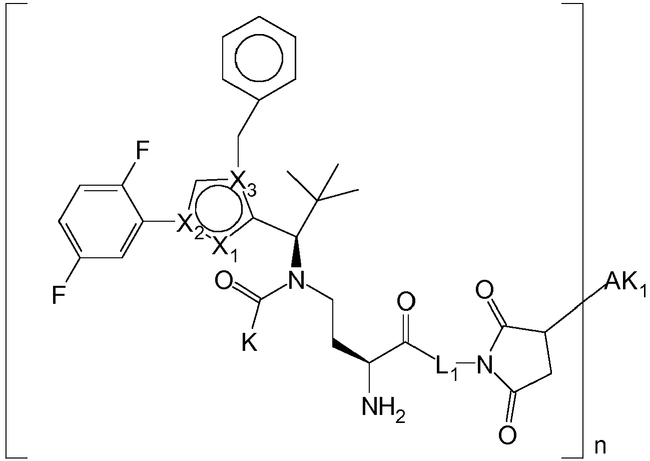

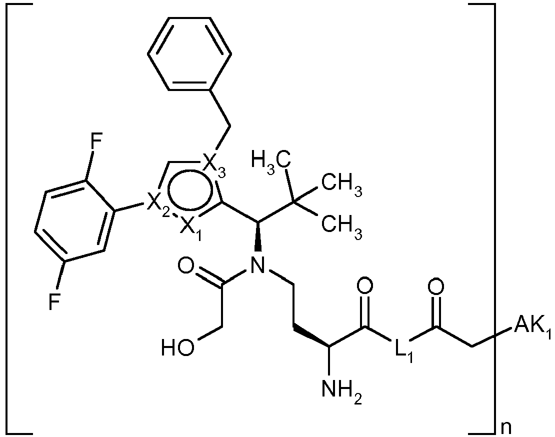

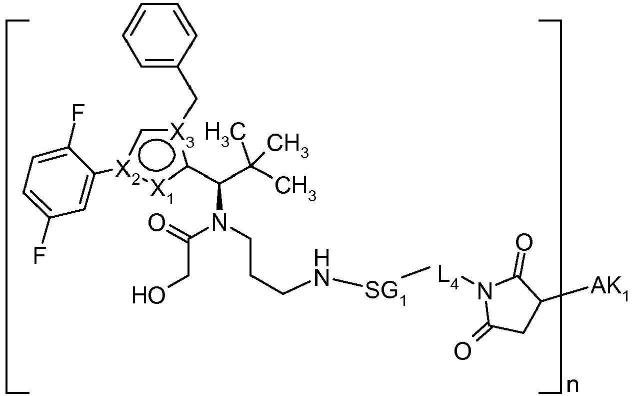

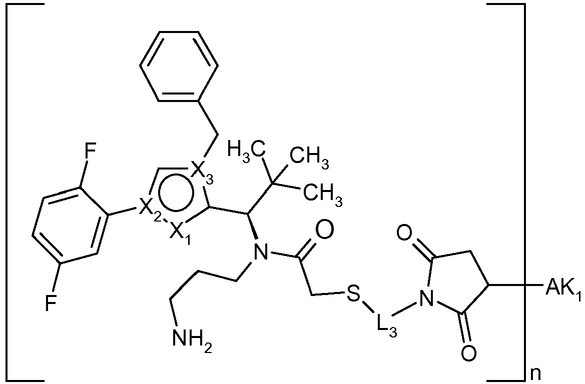

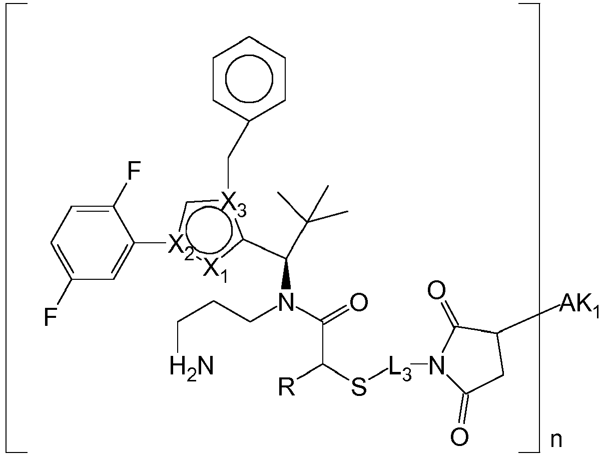

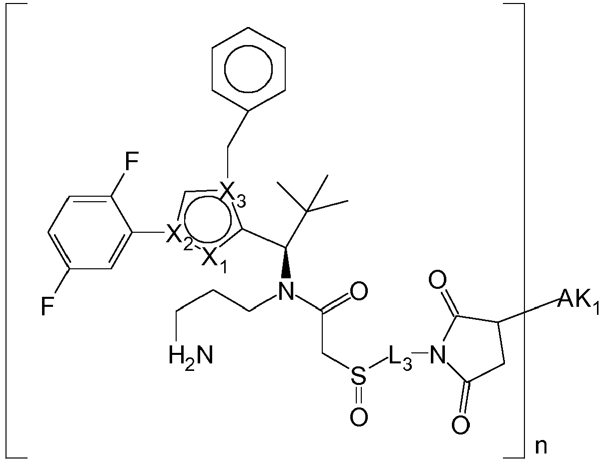

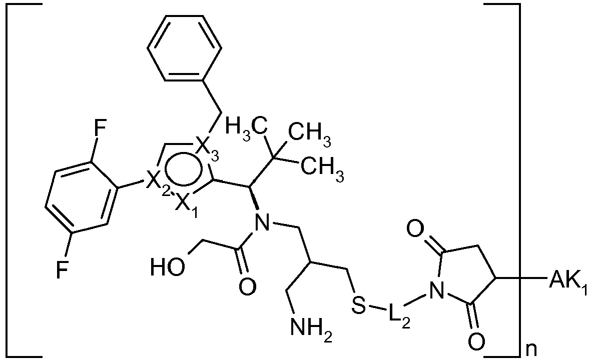

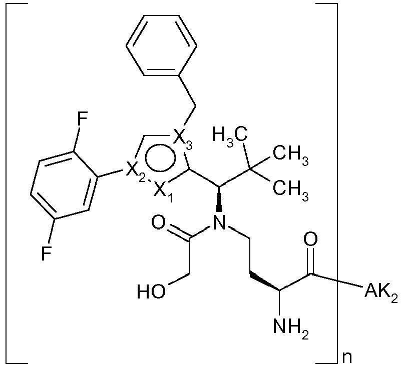

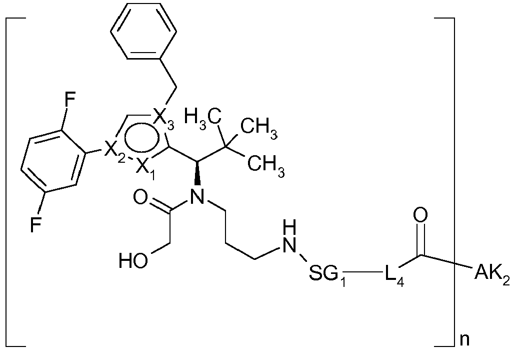

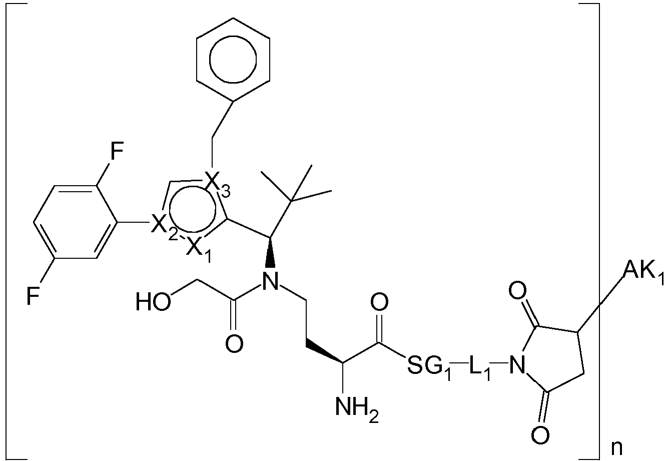

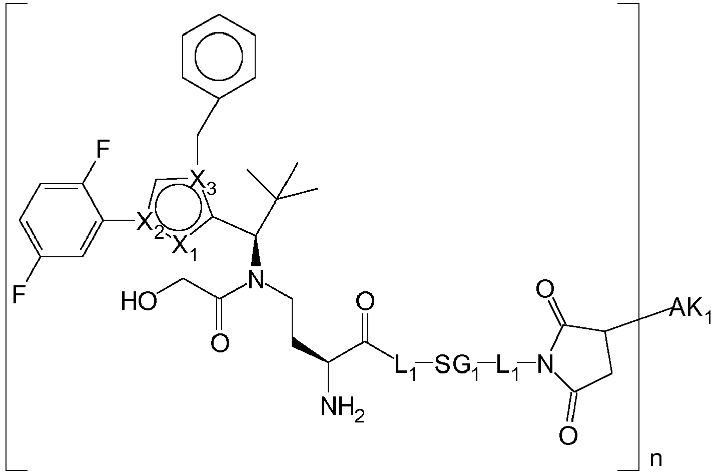

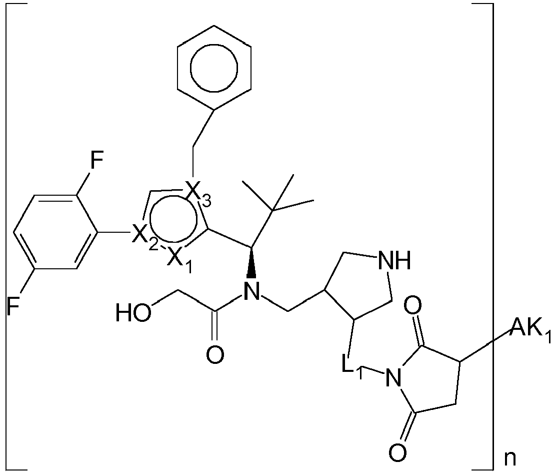

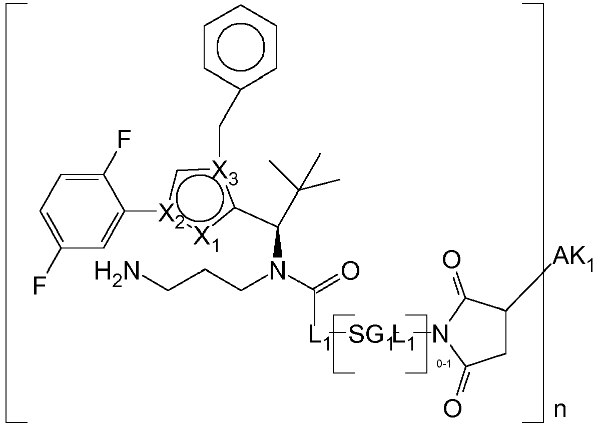

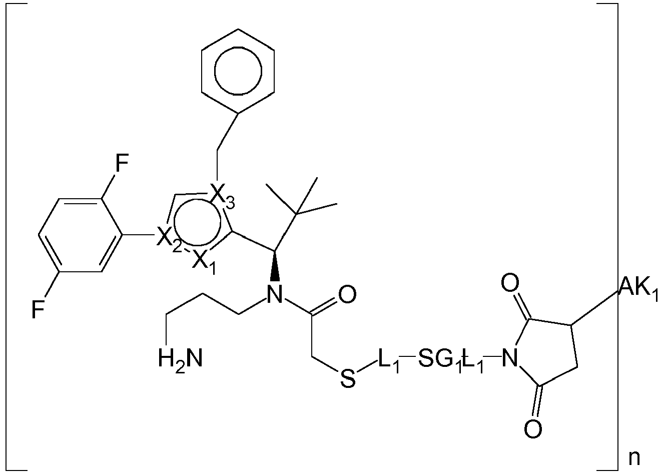

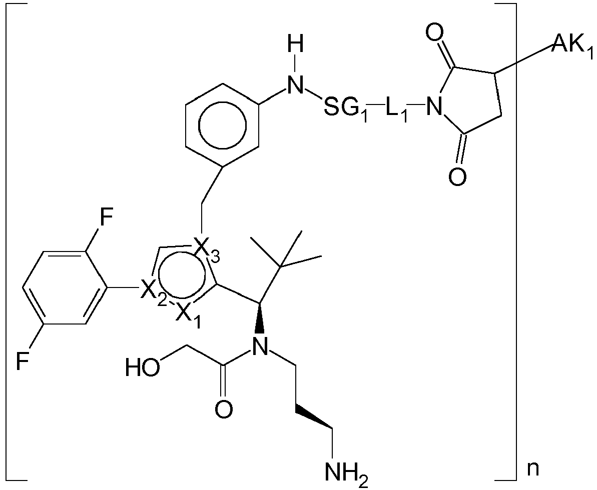

In Abhängigkeit vom Linker können Succinimid-verknüfte ADCs nach der Konjugation in die offenkettigenen Bernsteinsäureamide überführt werden, die ein vorteilhaftes Stabilitätsprofil aufweisen. 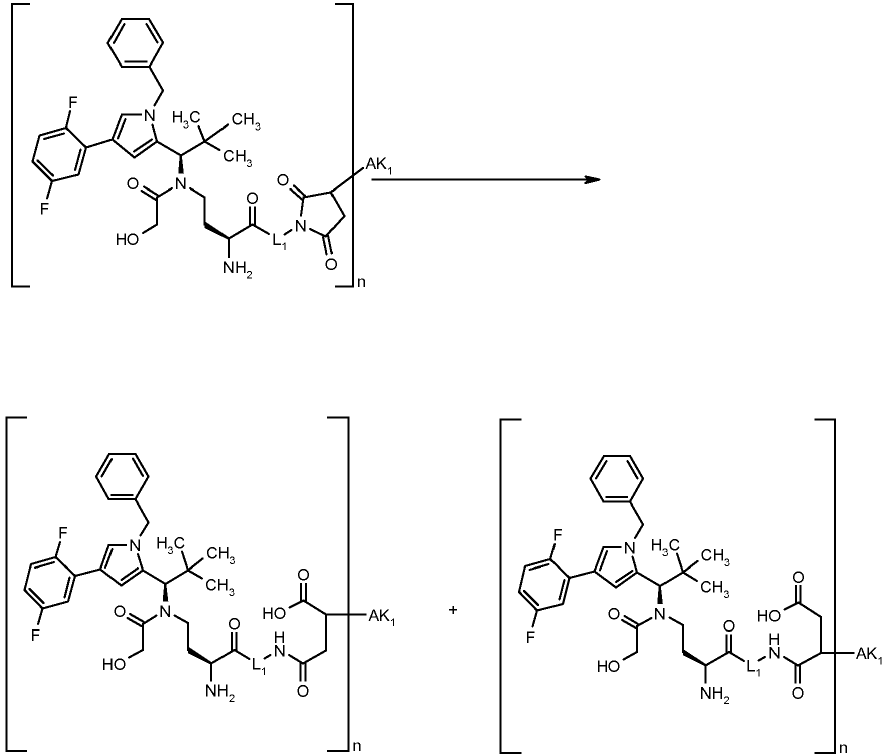 Diese Umsetzung (Ringöffnung) kann bei pH 7.5 bis 9, vorzugsweise bei pH 8, bei einer Temperatur von 25 ° C von 37 °C erfolgen, z.B. durch Rühren. Die bevorzugte Rührzeit beträgt 8 bis 30 Stunden.

In den obigen Formeln haben X₁, X₂, X₃ die gleiche Bedeutung wie in Formel (II), SG1 und L1 die gleiche Bedeutung haben wie oben beschrieben, und L2, L3 und L4 die gleiche Bedeutung wie L1; R und K die gleiche Bedeutung haben wie oben beschrieben. AK1 ist ein über einen Cysteinrest gekoppelter Antikörper, und AK2 ist ein über ein Lysinrest gekoppelter Antikörper. Besonders bevorzugt ist AK1 und AK2 ein anti-TWEAKR-Antikörper, insbesondere einer der spezifisch an die Aminosäure D in Position 47 (D47) von TWEAKR (SEQ ID NO:169) bindet, insbesondere der anti-TWEAKR-Antikörper TPP-2090.

### Binder

Der Begriff "Binder" wird im breitesten Sinne als ein Molekül verstanden, welches an ein Zielmolekül, das auf einer bestimmten, mit dem Binder-Wirkstoffkonjugat zu adressierenden Zielzellen-Population vorhanden ist, bindet. Der Begriff Binder ist in seiner breitesten Auslegung zu verstehen und umfasst z.B. auch Lektine, Proteine die bestimmte Zuckerketten binden können, oder Phospholipid-bindende Proteine. Solche Binder umfassen beispielsweise hochmolekulare Proteine (Bindeproteine), Polypeptide oder Peptide (Bindepeptide), nicht-peptidische (z.B. Aptamere (US5,270,163) Übersichtsartikel von Keefe AD., et al., Nat. Rev. Drug Discov. 2010; 9:537-550), oder Vitamine) und alle anderen zellbindenden Moleküle oder Substanzen. Bindeproteine sind z.B. Antikörper und Antikörperfragmente oder Antikörpermimetika wie z.B. Affibodies, Adnectins, Anticalins, DARPins, Avimers, Nanobodies (Übersichtsartikel von Gebauer M. et al., Curr. Opinion in Chem. Biol. 2009; 13:245-255; Nuttall S.D. et al., Curr. Opinion in Pharmacology 2008; 8:608-617). Bindepeptide sind z.B. Liganden eines Liganden-Rezeptorspaares, wie z.B. VEGF des Liganden-Rezeptorpaares VEGF/KDR, wie Transferrin des Liganden-Rezeptorpaares Transferrin/Transferrin-Rezeptor oder Cytokine/Cytokin-Rezeptor, wie TNFalpha des Liganden-Rezeptorpaares TNFalpha/TNFalpha Rezeptor.

Aus der Literatur sind verschiedene Möglichkeiten der kovalenten Kopplung (Konjugation) von organischen Molekülen an Antikörper bekannt. Erfindungsgemäß bevorzugt ist die Konjugation der Toxophore an den Antikörper über ein oder mehrere Schwefelatome von Cystein-Resten des Antikörpers und/oder über ein oder mehrere NH-Gruppen von Lysin-Resten des Antikörpers. Es ist jedoch auch möglich, das Toxophor an den Antikörper über freie Carboxylgruppen oder über Zuckerreste des Antikörpers zu binden.

Ein "Zielmolekül" wird im breitesten Sinne als ein Molekül verstanden, welches in der Zielzellenpopulation vorhanden ist, und kann ein Protein (z.B. ein Rezeptor eines Wachstumsfaktors) oder ein nicht-peptidisches Molekül sein (z.B. ein Zucker oder Phospholipid). Bevorzugt ist es ein Rezeptor oder ein Antigen.

Der Begriff "extrazelluläres" Zielmolekül beschreibt ein an die Zelle gebundenes Zielmolekül, welches sich auf der Außenseite einer Zelle befindet oder den Teil eines Zielmoleküls, welcher sich auf der Außenseite einer Zelle befindet, d.h. ein Binder kann an einer intakten Zelle an sein extrazelluläres Zielmolekül binden. Ein extrazelluläres Zielmolekül kann in der Zellmembran verankert sein oder Bestandteil der Zellmembran sein. Der Fachmann kennt Verfahren, um extrazelluläre Zielmoleküle zu identifizieren. Für Proteine kann dies über eine Bestimmung der Transmembrandomäne(n) und die Orientierung des Proteins in der Membran geschehen. Diese Angaben sind in der Regel in Proteindatenbanken (z.B. SwissProt) hinterlegt.

Der Begriff "Krebs-Zielmolekül" beschreibt ein Zielmolekül, welches auf einer oder mehreren Krebszellarten im Vergleich zu nicht-Krebszellen des gleichen Gewebetypus vermehrt vorhanden ist. Bevorzugt ist das Krebs-Zielmolekül auf einer oder mehreren Krebszellarten im Vergleich zu nicht-Krebszellen des gleichen Gewebetypus selektiv vorhanden, wobei selektiv eine mindestens zweifache Anreicherung auf Krebszellen im Vergleich zu nicht-Krebszellen des gleichen Gewebetypus beschreibt (ein "selektives Krebs-Zielmolekül"). Die Verwendung von Krebs-Zielmolekülen erlaubt die selektive Therapie von Krebszellen mit den erfindungsgemäßen Konjugaten.

Der Binder kann über eine Bindung mit dem Linker verknüpft werden. Die Verknüpfung des Binders kann mittels eines Heteroatoms des Binders erfolgen. Erfindungsgemäße Heteroatome des Binders, die zur Verknüpfung verwendet werden können, sind Schwefel (in einer Ausführungsform via einer Sulfhydrylgruppe des Binders), Sauerstoff (erfindungsgemäß mittels einer Carboxyl oder Hydroxylgruppe des Binders) und Stickstoff (in einer Ausführungsform via einer primären oder sekundären Amingruppe oder Amidgruppe des Binders). Diese Heteroatome können im natürlichen Binder vorhanden sein oder durch chemische oder molekularbiologische Methoden eingeführt werden. Erfindungsgemäß hat die Verknüpfung des Binders mit dem Toxophor nur einen geringen Einfluß auf die Bindeaktivität des Binders zum Zielmolekül. In einer bevorzugten Ausführungsform hat die Verknüpfung keinen Einfluß auf die Bindeaktivität des Binders zum Zielmolekül.

Der Begriff "Antikörper" wird gemäß der vorliegenden Erfindung in seinem breitesten Sinne verstanden und umfasst Immunglobulinmoleküle, beispielsweise intakte oder modifizierte monoklonale Antikörper, polyklonale Antikörper oder multispezifische Antikörper (z.B. bispezifische Antikörper). Ein Immunglobulinmolekül umfasst bevorzugt ein Molekül mit vier Polypeptidketten, zwei schwere Ketten (H Ketten) und zwei leichte Ketten (L Ketten), welche typischerweise durch Disulfidbrücken verknüpft sind. Jede schwere Kette umfasst eine variable Domäne der schweren Kette (abgekürzt VH) und konstante Domäne der schweren Kette. Die konstante Domäne der schweren Kette kann beispielsweise drei Domänen CH1, CH2 und CH3 umfassen. Jede leichte Kette umfasst eine variable Domäne (abgekürzt VL) und eine konstante Domäne. Die konstante Domäne der leichten Kette umfasst eine Domäne (abgekürzt CL). Die VH und VL Domänen können weiter unterteilt werden in Regionen mit Hypervariabilität, auch Komplementaritäts-bestimmende Regionen genannt ("complementarity determining region", abgekürzt CDR) und Regionen mit geringerer Sequenzvariabilität ("framework region", abgekürzt FR). Jede VH und VL Region setzt sich typischerweise aus drei CDRs und bis zu vier FRs zusammen. Beispielsweise vom Aminoterminus zum Carboxyterminus in der folgenden Reihenfolge FR1, CDR1, FR2, CDR2, FR3, CDR3, FR4. Ein Antikörper kann aus jeder dafür geeeigneten Spezies erhalten werden z.B. Kaninchen, Lama, Kamel, Maus, oder Ratte. In einer Ausführungsform ist der Antikörper humanen oder murinen Ursprungs. Ein Antikörper kann z.B. human, humanisiert oder chimär sein.

Der Begriff "monoklonaler" Antikörper bezeichnet Antikörper, die aus einer Population substantiell homogener Antikörper erhalten wurde, d.h. individuelle Antikörper der Population sind bis auf natürlich auftretende Mutationen, welche in geringfügiger Anzahl auftreten können, identisch. Monoklonale Antikörper erkennen mit hoher Spezifität eine einzige antigene Bindestelle. Der Begriff monoklonaler Antikörper bezieht sich nicht auf ein bestimmtes Herstellungsverfahren.

Der Begriff "intakter" Antikörper bezieht sich auf Antikörper, die sowohl eine Antigen-bindende Domäne als auch die konstante Domäne der leichten und schweren Kette umfassen. Die konstante Domäne kann eine natürlich vorkommende Domäne sein, oder eine Variante davon, bei der mehrere Aminosäurepositionen verändert wurden.

Der Begriff "modifizierter intakter" Antikörper bezieht sich auf intakte Antikörper, die mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert wurden. Des Weiteren können Antikörper dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug;26(8):925-32).

Der Begriff "humaner" Antikörper bezeichnet Antikörper, die aus einem Menschen erhalten werden können oder synthetische humane Antikörper sind. Ein "synthetischer" humaner Antikörper ist ein Antikörper, der in Teilen oder schweren als ganzes von synthetischen Sequenzen in silico erhältlich ist, die auf der Analyse humaner Antikörpersequenzen basieren. Ein humaner Antikörper kann z.B. durch eine Nukleinsäure kodiert sein, die aus einer Bibliothek von Antikörpersequenzen, die humanen Ursprungs sind, isoliert wurde. Ein Beispiel solcher Antikörper ist in Söderlind et al., Nature Biotech. 2000, 18:853-856 zu finden.

Der Begriff "humanisierter" oder "chimärer" Antikörper beschreibt Antikörper, die aus einem nicht-humanen und einem humanen Sequenzanteil bestehen. Bei diesen Antikörpern wird ein Teil der Sequenzen des humanen Immunoglobulins (Rezipient) durch Sequenzanteile eines nicht-humanen Immunoglobulins (Donor) ersetzt. Der Donor ist in vielen Fällen ein murines Immunoglobulin. Bei humanisierten Antikörpern werden Aminosäuren der CDR des Rezipienten durch Aminosäuren des Donors ersetzt. Manchmal werden auch noch Aminosäuren des Frameworks durch korrespondierende Aminosäuren des Donors ersetzt. In machen Fällen enthält der humanisierte Antikörper Aminosäuren, die weder im Rezipient noch im Donor enthalten waren und die während der Optimierung des Antikörpers eingefügt wurden. Bei chimären Antikörpern werden die variablen Domänen des Donor-Immunoglobulins mit den konstanten Regionen eines humanen Antikörpers fusioniert.

Der Begriff Komplementaritäts-bestimmende Region (CDR) wie er hier verwendet wird, bezieht sich auf diejenigen Aminosäuren einer variablen Antikörperdomäne, die für die Bindung an das Antigen notwendig sind. Jede variable Region hat typischerweise drei CDR Regionen, welche als CDR1, CDR2 und CDR3 bezeichnet werden. Jede CDR Region kann Aminosäuren nach der Definition von Kabat und/oder Aminosäuren eines Hypervariablen Loops definiert nach Chotia umfassen. Die Definition nach Kabat umfasst zum Beispiel die Region von ungefähr Aminosäureposition 24 - 34 (CDR1), 50 - 56 (CDR2) und 89 - 97 (CDR3) der variablen leichten Kette und 31 - 35 (CDR1), 50 - 65 (CDR2) und 95 - 102 (CDR3) der variablen schweren Kette (Kabat et al., Sequences of Proteins of Immunological Interest, 5th Ed. Public Health Service, National Institutes of Health, Bethesda, MD. (1991)). Die Definition nach Chotia umfasst zum Beispiel die Region von ungefähr Aminosäureposition 26 - 32 (CDR1), 50 - 52 (CDR2) und 91 -96 (CDR3) der variablen leichetn Kette und 26 - 32 (CDR1), 53 - 55 (CDR2) und 96 - 101 (CDR3) der variablen schweren Kette Chothia and Lesk; J Mol Biol 196: 901-917 (1987)). In manchen Fällen kann eine CDR Aminosäuren aus einer CDR Region definiert nach Kabat und Chotia umfassen.

Abhängig von der Aminosäure-Sequenz der konstanten Domäne der schweren Kette können Antikörper in verschiedene Klassen eingeteilt werden. Es gibt fünf Hauptklassen von intakten Antikörpern: IgA, IgD, IgE, IgG und IgM, wobei mehrere davon in weitere Unterklassen aufgegliedert werden können. (Isotypen), z.B. IgG1, IgG2, IgG3, IgG4, IgA1 und IgA2. Die konstante Domäne der schweren Kette, die zu den unterschiedlichen Klassen korrespondieren, werden als [alpha/a], [delta/δ], [epsilon/ε], [gamma/γ] und [my/µ] bezeichnet. Sowohl die dreidimensionale Struktur als auch die Untereinheitenstruktur von Antikörpern sind bekannt.

Der Begriff "funktionales Fragment" oder "antigen-bindendes Antikörperfragment" eines Antikörpers/Immunoglobulins ist definiert als ein Fragment eines Antikörpers/Immunoglobulins (z.B. die variable Domänen eines IgG), welches die Antigen-Bindedomänen des Antikörpers/Immunoglobulins noch umfasst. Die "Antigen-Bindedomäne" eines Antikörpers umfasst typischerweise eine oder mehrere Hypervariable Regionen eines Antikörpers, z.B. die CDR, CDR2 und/oder CDR3 Region. Allerdings kann auch die "Framework" oder die "Gerüst" Region eines Antikörpers zur Bindung des Antikörpers an das Antigen eine Rolle spielen. Die Framework Region bildet das Gerüst für die CDRs. Vorzugsweise umfasst die Antigen-Bindedomäne mindestens die Aminosäuren 4 bis 103 der variablen leichten Kette und Aminosäure 5 bis 109 der variablen schweren Kette, bevorzugter die Aminosäure 3 bis 107 der variablen leichten Kette und 4 bis 111 der variablen schweren Kette, besonders bevorzugt sind die kompletten variablen leichten und schweren Ketten , also Aminosäure 1 - 109 der VL und 1 bis 113 der VH (Nummerierung nach WO97/08320).

"Funktionale Fragmente" oder "antigen-bindende Antikörperfragmente" der Erfindung umfassen nicht abschliessend Fab, Fab', F(ab')₂ und Fv Fragmente, Diabodies, Single Domain Antibodies (DAbs), linerae Antikörper, Einzelketten Antikörper (single-chain Fv, abgekürzt scFv); und multispezifische, wie z.B. bi- und tri-spezifische, Antikörper, gebildet aus Antikörperfragmenten C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). Andere Antikörper als "multi-spezifische" oder "multi-funktionale" sind solche mit identischen Bindestellen. Multispezifische Antikörper können spezifisch für unterschiedliche Epitope eines Antigens sein oder spezifisch für Epitope von mehr als einem Antigen sein (siehe z.B. WO 93/17715; WO 92/08802; WO 91/00360; WO 92/05793; Tutt, et al., 1991, J. Immunol. 147:60 69; U. S. Pat. Nos. 4,474,893; 4,7 14,68 1 ; 4,925,648; 5,573,920; 5,601,8 19; oder Kostelny et al., 1992, J. Immunol. 148: 1547 1553). Ein F(ab')₂ oder Fab Molekül kann so konstruiert werden, dass die Zahl der intermolekularen Disulfidinteraktionen, die zwischen den Ch1 und den CL Domänen stattfindet, reduziert oder oder komplett verhindert werden kann.

"Epitope" bezeichnen Proteindeterminanten, die eine spezifische Bindung mit einem Immunglobulin oder T-Zell-Rezeptoren eingehen können. Epitopische Determinanten bestehen normalerweise aus chemisch aktiven Oberflächengruppen von Molekülen wie Aminosäuren oder Zuckerseitenketten, oder Kombinationen hiervon, und weisen normalerweise spezifische 3-dimensionale Struktureigenschaften wie auch spezifische Ladungseigenschaften auf.

"Funktionale Fragmente" oder "antigen-bindende Antikörperfragmente" können mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert werden. Des Weiteren können Antikörper und antigen-bindende Fragmente dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug; 26(8):925-32).

Polyklonale Antikörper können durch für den Durchschnittsfachmann bekannte Methoden hergestellt werden. Monoklonale Antiköper können durch für den Durchschnittsfachmann bekannte Methoden hergestellt werden (Köhler und Milstein, Nature, 256, 495-497, 1975). Humane bzw. humanisierte monoklonale Antiköper können durch für den Durchschnittsfachmann bekannte Methoden hergestellt werden (Olsson et al., Meth Enzymol. 92, 3-16 bzw. Cabilly et al US 4,816,567 oder Boss et al US 4,816,397).

Der Durchschnittsfachmann kennt vielfältige Methoden, um humane Antikörper und deren Fragmente herzustellen, wie beispielsweise mittels transgener Mäuse (N Lonberg und D Huszar, Int Rev Immunol. 1995; 13(1):65-93) oder Phage Display Technologien (Clackson et al., Nature. 1991 Aug 15;352(6336):624-8). Antikörper der Erfindung können aus rekombinanten Antikörperbibliotheken erhalten werden, die z.B. auf den Aminosäuresequenzen einer Vielzahl von Antikörpern besteht, die aus einer großen Anzahl gesunder Freiwilliger erstellt wurden. Antikörper können auch mittels bekannter rekombinanter DNS-Technologien hergestellt werden. Die Nukleinsäuresequenz eines Antikörpers kann durch Routinesequenzierung erhalten werden, oder ist aus öffentlich zugänglichen Datenbanken erhältlich.

Ein "isolierter" Antikörper oder Binder wurde von anderen Bestandteilen der Zelle gereinigt. Kontaminierende Bestandteile einer Zelle, welche mit einer diagnostischen oder therapeutischen Verwendung interferieren können, sind z.B. Enzyme, Hormone, oder andere peptidische- oder nicht-peptidische Bestandteile einer Zelle. Bevorzugt ist ein Antikörper oder Binder, der zu mehr als 95 Gew-% bezogen auf den Antikörper bzw. Binder aufgereinigt wurde (bestimmt durch z.B. Lowry Verfahren, UV-Vis Spektroskopie oder durch SDS-Kapillargelelektrophorese). Ausserdem ein Antikörper, der soweit aufgereinigt wurde, dass mindestens 15 Aminosäuren des Aminoterminus oder einer internen Aminosäuresequenz bestimmt werden können, oder zur Homogenität aufgereinigt wurde, wobei die Homogenität bestimmt wird durch SDS-PAGE unter reduzierenden oder nicht-reduzierenden Bedingungen (die Detektion kann mittels Coomassie Blau Anfärbung oder bevorzugt durch Silberfärbung bestimmt werden). Jedoch wird ein Antikörper normalerweise durch einen oder mehrere Reinigungsschritte hergestellt.

Der Begriff "spezifische Bindung" oder "bindet spezifisch" bezieht sich auf einen Antikörper oder Binder, der an ein vorbestimmtes Antigen/Zielmolekül bindet. Spezifische Bindung eines Antikörpers oder Binders beschreibt typischerweise einen Antikörper bzw. Binder mit einer Affinität von mindestens 10⁻⁷ M (als Kd-Wert; also vorzugsweise solche mit kleineren Kd-Werten als 10⁻⁷ M), wobei der Antikörper bzw. Binder eine mindestens zweifach höhere Affinität zum vorbestimmten Antigen/Zielmolekül als zu einem nicht-spezifischen Antigen/Zielmolekül hat (z.B. Rinder Serumalbumin, oder Casein), welches nicht das vorbestimmte Antigen/Zielmolekül oder ein eng verwandtes Antigen/Zielmolekül ist. Die Antikörper weisen vorzugsweise eine Affinität von mindestens 10⁻⁷ M (als Kd-Wert; also vorzugsweise solche mit kleineren Kd-Werten als 10⁻⁷ M), vorzugsweise von mindestens 10⁻⁸ M, besonders bevorzugt in dem Bereich von 10⁻⁹ M bis 10⁻¹¹ M auf. Die Kd-Werte können durch z.B. Oberflächenplasmonresonanzspektroskopie bestimmt werden.

Die erfindungsgemäßen Antikörper-Wirkstoff-Konjugate weisen ebenfalls Affinitäten in diesen Bereichen auf. Durch die Konjugation der Wirkstoffe wird die Affinität vorzugsweise nicht wesentlich beeinflusst (in der Regel wird die Affinität weniger als eine Größenordnung verringert, also z.B. maximal von 10⁻⁸ M auf 10⁻⁷ M).

Die erfindungsgemäßen verwendeten Antikörper zeichnen sich weiterhin vorzugsweise durch eine hohe Selektivität aus. Eine hohe Selektivität liegt vor, wenn der erfindungsgemäße Antikörper eine mindestens um den Faktor 2, bevorzugt Faktor 5 oder insbesondere bevorzugt Faktor 10 bessere Affinität am Zielprotein aufweisst als an einem unabhängigen anderen Antigen, z.B. humanem Serumalbumin (die Affinität kann z.B. durch Oberflächenplasmonenresonanzspektroskopie bestimmt werden).

Zudem sind die erfindungsgemäßen verwendeten Antikörper vorzugsweise kreuzreaktiv. Um präklinische Studien, z.B. toxikologische oder Wirksamkeitsstudien (z.B. in Xenograft-Mäusen), zu erleichtern und besser interpretieren zu können, ist es von Vorteil, wenn der erfindungsgemäß verwendete Antikörper nicht nur das humane Zielprotein bindet, sondern auch in der für die Studien verwendeten Spezies das Spezies-Zielprotein bindet. In einer Ausführungsform ist der erfindungsgemäß verwendete Antikörper zusätzlich zum humanen Zielprotein kreuzreaktiv zum Zielprotein mindestens einer weiteren Spezies. Für toxikologische und Wirksamkeitsstudien werden bevorzugt Spezien der Familien Nager, Hunde und nicht-humane Primaten, verwendet. Bevorzugte Nager Spezien sind Maus und Ratte. Bevorzugte nicht-humane Primaten sind Rhesusaffen, Schimpansen und Langschwanzmakaken.

In einer Ausführungsform ist der erfindungsgemäß verwendete Antikörper zusätzlich zum humanen Zielprotein kreuzreaktiv zum Zielprotein mindestens einer weiteren Spezies ausgewählt aus der Gruppe von Spezien bestehend aus Maus, Ratte und Langschwanzmakak (Macaca fascicularis). Insbesondere bevorzugt sind erfindungsgemäß verwendete Antikörper, die zusätzlich zum humanen Zielprotein mindestens kreuzreaktiv zum Maus-Zielprotein sind. Bevorzugt sind kreuzreaktive Antikörper, deren Affinität zum Zielprotein der weiteren nicht-humanen Spezies sich nicht um mehr als den Faktor 50, insbesondere nicht mehr als den Faktor zehn von der Affinität zum humanen Zielprotein unterscheidet.

### Gegen ein Krebs-Zielmolekül gerichtete Antikörper

Bevorzugt ist das Zielmolekül, gegen das der Binder, z.B. ein Antikörper oder ein Antigen bindendes Fragment davon, gerichtet ist, ein Krebs-Zielmolekül. Der Begriff "Krebs-Zielmolekül" beschreibt ein Zielmolekül, welches auf einer oder mehreren Krebszellarten im Vergleich zu nicht-Krebszellen des gleichen Gewebetypus vermehrt vorhanden ist. Bevorzugt ist das Krebs-Zielmolekül auf einer oder mehreren Krebszellarten im Vergleich zu nicht-Krebszellen des gleichen Gewebetypus selektiv vorhanden, wobei selektiv eine mindestens zweifache Anreicherung auf Krebszellen im Vergleich zu nicht-Krebszellen des gleichen Gewebetypus beschreibt (ein "selektives Krebs-Zielmolekül"). Die Verwendung von Krebs-Zielmolekülen erlaubt die selektive Therapie von Krebszellen mit den erfindungsgemäßen Konjugaten.

Antikörper, welche spezifisch gegen ein Antigen, wie z.B. ein Krebszell-Antigen, gerichtet sind, können vom Durchschnittsfachmann mittels ihm bekannter Verfahren hergestellt werden (wie z.B. rekombinante Expression) oder kommerziell erworben werden (wie z.B. von Merck KGaA, Deustchland). Beispiele bekannter kommerziell erhältlicher Antikörper in der Krebstherapie sind Erbitux® (Cetuximab, Merck KGaA), Avastin® (Bevacizumab, Roche) und Herceptin® (Trastuzumab, Genentech). Trastuzumab ist ein rekombinanter humanisierter monokloaler Antikörper vom IgG1 kappa Typ, welcher mit hoher Affinität in einem Zell-basierten Assay (Kd = 5 nM) die extrazelluläre Domäne des humanen epidermalen Wachstumsrezeptors bindet. Der Antikörper wird rekombinant in CHO-Zellen hergestellt.

In einer bevorzugten Ausführungsform ist das Zielmolekül ein selektives Krebs-Zielmolekül.

In einer besonders bevorzugten Ausführungsform ist das Zielmolekül ein Protein.

In einer Ausführungsform ist das Zielmolekül ein extrazelluläres Zielmolekül. In einer bevorzugten Ausführungsform ist das extrazelluläre Zielmolekül ein Protein.

Krebs-Zielmoleküle sind dem Fachmann bekannt. Beispiele hierfür sind im Folgenden aufgeführt.

Beispiele für Krebs-Zielmoleküle sind:
(1) EGF-Rezeptor (NCBI-Referenzsequenz NP_005219.2), SEQ ID NO: 213(1210 Aminosäuren):

Die extrazelluläre Domäne ist durch Unterstreichung gekennzeichnet.
(2) Mesothelin (SwissProt-Referenz Q13421-3), SEQ ID NO: 214 (622 Aminosäuren): Wobei Mesothelin von den Aminosäuren 296-598 kodiert wird. Aminosäuren 37-286 kodieren für "megakaryocyte-potentiating factor". Mesothelin ist durch einen GPI-Anker in der Zellmembran verankert und ist extrazellulär lokalisiert.
(3) Carboanhydrase IX (SwissProt-Referenz Q16790), SEQ ID NO: 215 (459 Aminosäuren):

Die extrazelluläre Domäne ist durch Unterstreichung gekennzeichnet.
(4) C4.4a (NCBI -Referenzsequenz NP_055215.2; Synonym LYPD3), SEQ ID NO: 216 (346 Aminosäuren):

Die maturierte, extrazelluläre Domäne ist durch Unterstreichung gekennzeichnet.
(5) CD52 (NCBI-Referenzsequenz NP_001794.2), SEQ ID NO: 217 >gi|68342030|refNP_001794.2| CAMPATH-1 antigen-Vorläufer [Homo sapiens] MKRFLFLLLTISLLVMVQIQTGLSGQNDTSQTSSPSASSNISGGIFLFFVANAIIHLFCFS
(6) Her2 (NCBI-Referenzsequenz NP_004439.2), SEQ ID NO: 218
(7) CD20 (NCBI-Referenzsequenz NP_068769.2), SEQ ID NO: 219
(8) das Lymphozyten aktivierungs Antigen CD30 (SwissProt ID P28908), SEQ ID NO: 220
(9) das Lymphozyten Adhesionsmolekül CD22 (SwissProt ID P20273), SEQ ID NO: 221
(10) das Myloidzellen Oberflächenantigen CD33 (SwissProt ID **P20138**), SEQ ID NO: 222
(11) das Transmembran Glykoprotein NMB (SwissProt ID **Q14956**), SEQ ID NO: 223
(12) das Adhesionsmolekül CD56 (SwissProt ID P13591), SEQ ID NO: 224
(13) das Oberflächenmolekül CD70 (SwissProt ID P32970), SEQ ID NO: 225
(14) das Oberflächenmolekül CD74 (SwissProt ID P04233), SEQ ID NO: 226
(15) das B-Lymphozyten Antigen CD19 (SwissProt ID P15391), SEQ ID NO: 227
(16) das Oberflächenprotein Mucin-1 (SwissProt ID P15941), SEQ ID NO: 228
(17) das Oberflächenprotein CD138 (SwissProt ID P18827), SEQ ID NO: 229
(18) das Integrin alphaV (Genbank Accession No.: NP_002201.1), SEQ ID NO: 230
(19) das teratocarcinoma-derived growth factor 1 Protein TDGF1 (Genbank Accession No.: NP_003203.1), SEQ ID NO: 231
(20) das Prostata spezifische Membranantigen PSMA (Swiss Prot ID: Q04609), SEQ ID NO: 232
(21) die Tyrosin-Proteinkinase EPHA2 (Swiss Prot ID: P29317), SEQ ID NO: 233
(22) das Oberflächenprotein SLC44A4 (Genbank Accession No: NP_001171515), SEQ ID NO: 234
(23) das Oberflächenprotein BMPR1B (SwissProt: 000238)
(24) das Transportprotein SLC7A5 (SwissProt: Q01650)
(25) das epitheliale Psostataantigen STEAP1 (SwissProt: Q9UHE8)
(26) das Ovarkarzinomantigen MUC16 (SwissProt: Q8WXI7)
(27) das Transportprotein SLC34A2 (SwissProt: O95436)
(28) das Oberflächenprotein SEMA5b (SwissProt: Q9P283)
(29) das Oberflächenprotein LYPD1 (SwissProt: Q8N2G4)
(30) der Endothelin Rezeptor Typ B EDNRB (SwissProt: P24530)
(31) das Ringfingerprotein RNF43 (SwissProt: Q68DV7)
(32) das Prostatakarzinom-assozierte Protein STEAP2 (SwissProt: Q8NFT2)
(33) der Kationenkanal TRPM4 (SwissProt: Q8TD43)
(34) der Komplementrezeptor CD21 (SwissProt: P20023)
(35) das B-Zell Antigen Rezeptorkomplex-assozierte Protein CD79b (SwissProt: P40259)
(36) das Zelladhäsionsantigen CEACAM6 (SwissProt: P40199)
(37) die Dipeptidase DPEP1 (SwissProt: P16444)
(38) der Interleukinrezeptor IL20Ralpha (SwissProt: Q9UHF4)
(39) das Proteoglykan BCAN (SwissProt: Q96GW7)
(40) der Ephrin Rezeptor EPHB2 (SwissProt: P29323)
(41) das Prostatastammzellen-assozierte Protein PSCA (Genbank Accession No: NP_005663.2)
(42) das Oberflächenprotein LHFPL3 (SwissProt: Q86UP9)
(43) das Rezeptorprotein TNFRSF13C (SwissProt: Q96RJ3)
(44) das B-Zell Antigen Rezeptorkomplex-assozierte Protein CD79a (SwissProt: P11912)
(45) das Rezeptorprotein CXCR5 (SwissProt: P32302)
(46) der Ionenkanal P2X5 (SwissProt: Q93086)
(47) das Lymphozytenantigen CD180 (SwissProt: Q99467)
(48) das Rezeptorprotein FCRL1 (SwissProt: Q96LA6)
(49) das Rezeptorprotein FCRL5 (SwissProt: Q96RD9)
(50) das MHC Klasse II Molekül Ia Antigen HLA-DOB (Genbank Accession No: NP_002111.1)
(51) das T-Zell Protein VTCN1 (SwissProt: Q7Z7D3)
(52) TWEAKR (SEQ ID NO:169 (Protein); SEQ ID NO:170 (DNA).
(53) das Lymphozytenantigen CD37 (Swiss Prot: P11049)
(54) Der FGF Rezeptor 2; FGFR2 (Gene ID: 2263; Official Symbol: FGFR2). Der FGFR2 Rezeptor kommt in verschiedenen Spleißvarianten vor (alpha, beta, IIIb, IIIc). Alle Spleißevarianten können als Zielmolekül fungieren.
(55) das transmembrane Glycoprotein B7H3 (CD276; Gene ID: 80381)
(56) der B Zell Rezeptor BAFFR (CD268; Gene ID: 115650)
(57) das Rezeptorprotein ROR 1 (Gene ID: 4919)
(58) der Oberflächenrezeptor IL3RA (CD123; Gene ID: 3561)
(59) der CXC Chemokinrezeptor CXCR5 (CD185; Gene ID 643)
(60) das Rezeptorprotein Syncytin (Gene ID 30816)

In einem bevorzugten Gegenstand der Erfindung ist das Krebs-Zielmolekül ausgewählt aus der Gruppe bestehend aus den Krebs-Zielmolekülen (1) - (60), insbesondere (1), (6) und (52).

In einem weiteren besonders bevorzugten Gegenstand der Erfindung bindet der Binder an ein extrazelluläres Krebs-Zielmolekül, welches ausgewählt wird aus der Gruppe bestehend aus aus den Krebs-Zielmolekülen (1) - (60), insbesondere (1), (6) und (52).

In einem weiteren besonders bevorzugten Gegenstand der Erfindung bindet der Binder spezifisch an ein extrazelluläres Krebs-Zielmolekül, welches ausgewählt wird aus der Gruppe bestehend aus aus den Krebs-Zielmolekülen (1) - (60), insbesondere (1), (6) und (52).In einer bevorzugten Ausführungsform wird der Binder nach Bindung an sein extrazelluläres Zielmolekül auf der Zielzelle durch die Bindung von der Zielzelle internalisiert. Dies bewirkt, dass das Binder-Wirkstoffkonjugat, welches ein Immunokonjugat oder ein ADC sein kann, von der Zielzelle aufgenommen wird. Anschliessend wird der Binder vorzugsweise intrazellulär, bevorzugt lysosomal, prozessiert.

In einer Ausführungsform ist der Binder ein Bindeprotein. In einer bevorzugten Ausführungsform ist der Binder ein Antikörper, ein antigen-bindendes Antikörperfragment, ein multispezifischer Antikörper oder ein Antikörpermimetikum.

Bevorzugte Antikörpermimetika sind Affibodies, Adnectins, Anticalins, DARPins, Avimers, oder Nanobodies. Bevorzugte multispezifischer Antikörper sind bispezifische und trispezifische Antikörper.

In einer bevorzugten Ausführungsform ist der Binder ein Antikörper oder ein antigen-bindendes Antikörperfragment, weiter bevorzugt ist ein isolierter Antikörper oder ein isoliertes antigen-bindendes Antikörperfragment.

Bevorzugte antigen-bindende Antikörperfragmente sind Fab, Fab', F(ab')₂ und Fv Fragmente, Diabodies, DAbs, lineare Antikörper und scFv. Besonders bevorzugt sind Fab, Diabodies und scFv.

In einer besonders bevorzugten Ausführungsform ist der Binder ein Antikörper. Besonders bevorzugt sind monoklonale Antikörper oder antigen-bindende Antikörperfragmente davon. Weiter besonders bevorzugt sind humane, humanisierte oder chimäre Antikörper oder antigen-bindende Antikörperfragmente davon.

Antikörper oder antigen-bindende Antikörperfragmente, die Krebs-Zielmoleküle binden, können vom Durchschnittsfachmann mit bekannten Verfahren hergestellt werden, wie z.B. chemische Synthese oder rekombinante Expression. Binder für Krebs-Zielmoleküle können kommerziell erworben werden oder können durch den Durchschnittsfachmann mit bekannten Verfahren hergestellt werden, wie z.B. chemische Synthese oder rekombinante Expression. Weitere Verfahren zur Herstellung von Antikörpern oder antigen-bindenden Antikörperfragmenten sind in WO 2007/070538 beschrieben (siehe Seite 22 "Antibodies"). Der Fachmann kennt Verfahren wie sogenannte Phage-Display Bibliotheken (z.B. Morphosys HuCAL Gold) erstellt und zur Auffindung von Antikörpern oder antigen-bindenden Antikörperfragmenten verwendet werden können (siehe WO 2007/070538, Seite 24 ff und AK-Beispiel 1 auf Seite 70, AK-Beispiel 2 auf Seite 72). Weitere Verfahren zur Herstellung von Antikörper, die DNA Bibliotheken aus B-Zellen verwenden, sind zum Beispiel auf Seite 26 (WO 2007/070538) beschrieben. Verfahren zur Humanisierung von Antikörpern sind auf Seite 30-32 von WO2007070538 und im Detail in Queen, et al.,.Pros. Natl. Acad. Sci. USA 86:10029-10033,1989 oder in WO 90/0786 beschrieben. Des Weiteren sind dem Fachmann Verfahren zur rekombinanten Expression von Proteinen im allgemeinen und im speziellen von Antikörpern bekannt (siehe z.B. in Berger and Kimrnel (Guide to Molecular Cloning Techniques, Methods in Enzymology, Vol. 152, Academic Press, Inc.); Sambrook, et al., (Molecular Cloning: A Laboratory Manual, (Second Edition, Cold Spring Harbor Laboratory Press; Cold Spring Harbor, N.Y.; 1989) Vol. 1-3); Current Protocols in Molecular Biolony, (F. M. Ausabel et al. [Eds.], Current Protocols, Green Publishing Associates, Inc. / John Wiley & Sons, Inc.); Harlow et al., (Monoclonal Antibodies: A Laboratory Manual, Cold Spring Harbor Laboratory Press (19881, Paul [Ed.]); Fundamental Immunology, (Lippincott Williams & Wilkins (1998)); and Harlow, et al., (Using Antibodies: A Laboratory Manual, Cold Spring Harbor Laboratory Press (1998)). Der Fachmann kennt die entsprechenden Vektoren, Promotoren und Signalpeptide die zur Expression eines Proteins/Antikörpers notwendig sind. Gebräuchliche Verfahren sind auch in WO 2007/070538 auf den Seiten 41 - 45 beschrieben. Verfahren zur Herstellung eines IgG1-Antikörpers sind z.B. in WO 2007/070538 in Beispiel 6 auf Seite 74 ff beschrieben. Verfahren, mit denen die Internalisierung eines Antikörpers nach Bindung an sein Antigen bestimmt werden kann, sind dem Fachmann bekannt und sind z.B. in WO 2007/070538 auf Seite 80 beschrieben. Der Fachmann kann die in WO 2007/070538 beschriebenen Verfahren, die zur Herstellung von Carboanhydrase IX (Mn)-Antikörpern verwendet wurden, anlog zur Herstellung für Antikörper mit anderer Zielmolekülspezifität verwenden.

### anti-EGFR-Antikörper

Beispiele für Antikörper, die das Krebs-Zielmoleküle EGFR binden, sind Cetuximab (INN Nummer 7906), Panitumumab (INN Nummer 8499) und Nimotuzumab (INN Nummer 8545). Cetuximab (Drug Bank Accession Number DB00002) ist ein chimärer anti-EGFRl-Antikörper, der in SP2/0 Maus-Myelom-Zellen produziert und von ImClone Systems Inc/Merck KgaA/Bristol-Myers Squibb Co vertrieben wird. Cetuximab ist indiziert zur Behandlung des metastasierenden, EGFR exprimierenden Kolorektalkarzinoms mit Wildtyp-K-Ras Gen. Er hat eine Affinität von 10⁻¹⁰M.

### Sequenz:

Cetuximab Leichte Kette (kappa), SEQ ID NO: 235:
Cetuximab Schwere Kette, SEQ ID NO: 236:

Panitumumab (INN Nummer 8499) (Drug Bank Accession Number DB01269) ist ein rekombinanter monoklonaler humaner IgG2 Antikörper, der spezifisch an den humanen EGF-Rezeptor 1 bindet und von Abgenix / Amgen vertrieben wird. Panitumumab stammt aus der Immunisierung von transgenen Mäusen (XenoMouse). Diese Mäuse sind in der Lage humane Immunglobuline (leichte und schwere Ketten) zu produzieren. Es wurde ein spezieller B-Zell-Klon ausgewählt, der Antikörper gegen EGFR produziert, und dieser mit CHO-Zellen (Chinese hamster ovary cells) immortalisiert. Diese Zellen werden jetzt für die Produktion eines zu 100 % humanen Antikörpers verwendet. Panitumumab ist indiziert zur Behandlung des EGFR-exprimierenden, metastasierenden Kolorektalkarzinoms, das refraktär gegenüber einer chemotherapeutischen Behandlung mit Fluropyrimidin, Oxaliplatin und Irinotecan ist. Er hat eine Affinität von 10⁻¹¹M.

### Sequenz:

**Panitumumab** Leichte Kette (kappa), SEQ ID NO: 237:
**Panitumumab** Schwere Kette, SEQ ID NO: 238:

Bei Nimotuzumab (INN Nummer 8545) (EP 00586002, EP 00712863) handelt es sich um einen humanisierten monoklonalen IgG1 Antikörper, der spezifisch an den humanen EGF-Rezeptor 1 bindet und von YM BioScienecs Inc. (Mississauga Canada) vertrieben wird. Er wird in nicht-sekretierenden NSO-Zellen (Säugerzelllinie) produziert. Nimotuzumab ist zugelassen zur Behandlung von Kopf- und Halstumoren, hoch-malignem Astrocytoma und Glioblastoma multiforme (nicht in EU und US) und Pankreaskarzinom (Orphan drug, EMA). Er hat eine Affinität von 10⁻⁸ M.
**Nimotuzumab** Leichte Kette, SEQ ID NO: 239:
**Nimotuzumab** Schwere Kette, SEQ ID NO: 240:

Weitere Ausführungsformen für EGFR-Antikörper sind:
- Zalutumumab / 2F8 / HuMax-EGFR, Firma Genmab A/S (WO 02/100348, WO 2004/056847, INN-Nummer 8605)
- Necitumumab / 11F8, ImClone / IMC-11F8, Firma ImClone Systems Inc [Eli Lilly & Co] (WO 2005/090407 (EP 01735348-A1, US 2007/0264253-A1, US 7,598,350, WO 2005/090407-A1), INN- Nummer 9083)
- Matuzumab / anti-EGFR MAb, Merck KGaA / anti-EGFR MAb, Takeda / EMD 72000 / EMD-6200 / EMD-72000 und EMD-55900 / MAb 425 / monoclonal antibody 425, Firma Merck KGaA / Takeda (WO 92/15683, INN-Nummer 8103 (Matuzumab))
- RG-7160 / GA-201 / GA201 / R-7160 / R7160 / RG7160 / RO-4858696 / RO-5083945 / RO4858696 / RO5083945, Firma Glycart Biotechnology AG (Roche Holding AG) (WO 2010/112413-A1, WO 2010/115554)
- GT-MAB 5.2-GEX / CetuGEX, Firma Glycotope GmbH (WO 2008/028686-A2 (EP 01900750-A1, EP 01911766-A1, EP 02073842-A2, US 2010/0028947-A1)
- ISU-101, Firma Isu Abxis Inc (ISU Chemical Co Ltd) / Scancell (WO 2008/004834-A1)
- ABT-806 / mAb-806 / ch-806 / anti-EGFR monoclonal antibody 806, Firma Ludwig Institute for Cancer Research / Abbott / Life Science Pharmaceuticals (WO 02/092771, WO 2005/081854 und WO 2009/023265)
- SYM-004 (consists of two chimeric IgG1 antibodies (992 and 1024)), Firma Symphogen A/S (WO 2010/022736-A2)
- MR1-1 /MR1-1KDEL, Firma IVAX Corp (Teva Pharmaceutical Industries Ltd) (Duke University), (Patent: WO2001/062931-A2)
- Antikörper gegen die Deletionsmutante, EGFRvIII, Firma Amgen/Abgenix (WO 2005/010151, US 7,628,986)
- SC-100, Firma Scancell Ltd (WO 01/088138-A1)
- MDX-447 / EMD 82633 / BAB-447 / H 447 / MAb, EGFR, Medarex/Merck KgaA, Firma Bristol-Myers Squibb (US) / Merck KGaA (DE) / Takeda (JP), (WO 91/05871, WO 92/15683)
- anti-EGFR-Mab, Firma Xencor (WO 2005/056606)
- DXL-1218 / anti-EGFR monoclonal antibody (cancer), InNexus, Firma InNexus Biotechnology Inc, Pharmaprojects PH048638

In einer bevorzugten Ausführungsform werden die anti-EGFR Antikörper ausgewählt aus der Gruppe bestehend aus Cetuximab, Panitumumab, Nimotuzumab, Zalutumumab, Necitumumab, Matuzumab, RG-716, GT-MAB 5.2-GEX, ISU-101, ABT-806, SYM-004, MR1-1, SC-100, MDX-447, und DXL-1218.

In einer besonders bevorzugten Ausführungsform werden die anti-EGFR Antikörper ausgewählt aus der Gruppe bestehend aus Cetuximab, Panitumumab, Nimotuzumab, Zalutumumab, Necitumumab und Matuzumab.

Der Fachmann kennt Verfahren, mit denen aus den CDR Regionen der obengenannten Antikörper durch Sequenzvariationen weitere Antikörper hergestellt werden können, die eine ähnliche oder besser Affinität und/oder Spezifität zum Zielmolekül haben.

In einer weiteren Ausführungsform werden die anti-EGFR Antikörper oder antigen-bindenden Antikörperfragmente ausgewählt aus der Gruppe bestehend aus

Antikörper oder antigenbindende Antikörperfragmente, die drei CDR Regionen der leichten Kette und die drei CDR Regionen der schweren Kette eines der folgenden Antikörper umfasst: Cetuximab, Panitumumab, Nimotuzumab, Zalutumumab, Necitumumab, Matuzumab, RG-716, GT-MAB 5.2-GEX, ISU-101, ABT-806, SYM-004, MR1-1, SC-100, MDX-447, und DXL-1218.

In einer weiteren Ausführungsform werden die anti-EGFR Antikörper oder antigen-bindenden Antikörperfragmente ausgewählt aus der Gruppe bestehend aus

Antikörper oder antigenbindende Antikörperfragmente, die drei CDR Regionen der leichten Kette und die drei CDR Regionen der schweren Kette eines der folgenden Antikörper umfasst: Cetuximab, Panitumumab, Nimotuzumab, Zalutumumab, Necitumumab, Matuzumab. Diese Antikörper und antigenbindende Fragmente davon sind hierin durch Verweis aufgenommen und können im Rahmen dieser Erfindung verwendet werden.

### anti-Carboanhydrase IX Antikörper

Beispiele für Antikörper, die das Krebs-Zielmoleküle Carboanhydrase IX binden, sind in WO 2007/070538-A2 (z.B. Anspüche 1 - 16) beschrieben.

In einer bevorzugten Ausführungsform werden die anti-Carboanhydrase IX Antikörper oder antigen-bindenden Antikörperfragmente ausgewählt aus der Gruppe bestehend aus anti-Carboanhydrase IX Antikörper oder antigen-bindenden Antikörperfragmente 3ee9 (Anspruch 4 (a) in WO 2007/070538-A2), 3ef2 (Anspruch 4 (b) in WO 2007/070538-A2), 1e4 (Anspruch 4 (c) in WO 2007/070538-A2), 3a4 (Anspruch 4 (d) in WO 2007/070538-A2), 3ab4 (Anspruch 4 (e) in WO 2007/070538-A2), 3ah10 (Anspruch 4 (f) in WO 2007/070538-A2), 3bb2 (Anspruch 4 (g) in WO 2007/070538-A2), 1aa1 (Anspruch 4 (h) in WO 2007/070538-A2), 5a6 (Anspruch 4 (i) in WO 2007/070538-A2) und 5aa3 (Anspruch 4 (j) in WO 2007/070538-A2).

### anti-C4.4a Antikörper:

Erfindungsgemäß können C4.4a Antikörper verwendet werden.

Beispiele für C4.4a Antikörper und antigen-bindende Fragmente sind in WO 2012/143499 A2 beschrieben. Alle Antikörper der WO 2012/143499 A2 sind hiermit in die Beschreibung der vorliegenden Erfindung durch Verweis aufgenommen und können in der vorliegenden Erfindung verwendet werden. Die Sequenzen der Antikörper sind in Tabelle 1 der WO 2012/143499 A2 angegeben, wobei jede Zeile die jeweiligen CDR Aminosäuresequenzen der variablen leichten Kette bzw. der variablen Schweren Kette des in Spalte 1 aufgeführten Antikörpers wiedergibt.

In einer Ausführungsform werden die anti-C4.4a Antikörper oder antigen-bindenden Antikörperfragmente davon nach Bindung an eine C4.4a exprimierende Zelle von der Zelle internalisiert.

In einer weiteren Ausführungsform umfassen die anti-C4.4a Antikörper oder antigen-bindenden Antikörperfragmente mindestens eine, zwei oder drei CDR Aminosäuresequenzen eines in Tabelle 1 von WO 2012/143499 A2 oder Tabelle 2 von WO 2012/143499 A2 angeführten Antikörpers. Bevorzugte Ausführungsformen solcher Antikörper sind ebenfalls in WO 2012/143499 A2 aufgeführt und hierin durch Verweis aufgenommen.

### anti-HER2-Antikörper:

Ein Beispiel für einen Antikörper, der das Krebs-Zielmoleküle Her2 bindet, ist Trastuzumab (Genentech). Trastuzumab ist ein humanisierter Antikörper, der zur unter anderem Behandlung von Brustkrebs eingesetzt wird.

Weitere Beispiele für Antikörper, die an HER2 binden, sind neben Trastuzumab (INN 7637, CAS NR: RN: 180288-69-1) und Pertuzumab (Cas NR: 380610-27-5), auch Antikörper, wie offenbart in WO 2009/123894-A2, WO 200/8140603-A2, oder in WO 2011/044368-A2. Beispiel für ein anti-HER2 Konjugat ist Trastuzumab-Emtansine (INN-Nr. 9295). Diese Antikörper und antigenbindende Fragmente davon sind hierin durch Verweis aufgenommen und können im Rahmen dieser Erfindung verwendet werden.

### anti-CD20-Antikörper:

Ein Beispiel für einen Antikörper, der das Krebs-Zielmoleküle CD20 bindet, ist Rituximab (Genentech). Rituximab (CAS-Nummer: 174722-31-7) ist ein chimärer Antikörper, der zur Behandlung von Non-Hodgkin-Lymphom verwendet wird. Diese Antikörper und antigenbindende Fragmente davon sind hierin durch Verweis aufgenommen und können im Rahmen dieser Erfindung verwendet werden.

### anti-CD52-Antikörper:

Ein Beispiel für einen Antikörper, der das Krebs-Zielmoleküle CD52 bindet, ist Alemtuzumab (Genzyme). Alemtuzumab (CAS-Nummer: 216503-57-0) ist ein humanisierter Antikörper, der zur Behandlung von chronischer lymphatischer Leukämie eingesetzt wird. Diese Antikörper und antigenbindende Fragmente davon sind hierin durch Verweis aufgenommen und können im Rahmen dieser Erfindung verwendet werden.

### anti-Mesothelin-Antikörper:

Beispiele für anti-Mesothelin -Antikörper sind z.B. WO 2009/068204 beschrieben. Alle in WO 2009/068204 beschriebenen Antikörper sind in diese Beschreibung hiermit durch Verweis aufgenommen, so dass diese Antikörper im Rahmen der hierin offenbarten Erfindung verwendet werden können.

Die erfindungsgemäßen verwendeten anti-Mesothelin-Antikörper zeichnen sich weiterhin vorzugsweise durch eine invariante Bindung an Mesothelin aus. Invariante Bindung zeichnet sich zum Beispiel dadurch aus, dass der erfindungsgemäßen verwendeten Antikörper an ein Epitop von Mesothelin bindet, welches nicht durch ein weiteres extrazelluläres Protein maskiert werden kann. Solch ein weiteres extrazelluläres Protein ist z.B. das Protein Ovarian Cancer Antigen 125 (CA125). Vorzugsweise verwendete Antikörper zeichnen sich dadurch aus, dass deren Bindung an Mesothelin nicht durch CA125 blockiert wird.

### anti-CD30-Antikörper

Beispiele für Antikörper, die das Krebs-Zielmolekül CD30 binden und zur Behandlung von Krebs z.B. Hodgkin-Lymphoma verwendet werden können, sind Brentuximab, Iratumumab und Antikörper, wie in WO 2008/092117, WO 2008/036688 oder WO 2006/089232 offenbart. Beispiele für ein anti- CD30 Konjugat ist Brentuximab Vedotin (INN-Nr. 9144). Diese Antikörper und antigenbindende Fragmente davon sind hierin durch Verweis aufgenommen und können im Rahmen dieser Erfindung verwendet werden.

### anti-CD22-Antikörper

Beispiele für Antikörper, die das Krebs-Zielmolekül CD22 binden und zur Behandlung von Krebs z.B. Lymphoma verwendet werden können, sind Inotuzumab oder Epratuzumab. Beispiele für anti- CD22 Konjugate sind Inotuzumab Ozagamycin (INN-Nr. 8574), oder anti-CD22-MMAE und anti-CD22-MC-MMAE (CAS RN: 139504-50-0 bzw. 474645-27-7). Diese Antikörper und antigenbindende Fragmente davon sind hierin durch Verweis aufgenommen und können im Rahmen dieser Erfindung verwendet werden.

### anti-CD33-Antikörper

Beispiele für Antikörper, die das Krebs-Zielmolekül CD33 binden und zur Behandlung von Krebs z.B. Leukämie verwendet werden können, sind Gemtuzumab oder Lintuzumab (INN 7580). Ein Beispiel für ein anti-CD33 Konjugat ist Gemtuzumab-Ozagamycin. Diese Antikörper und antigenbindende Fragmente davon sind hierin durch Verweis aufgenommen und können im Rahmen dieser Erfindung verwendet werden.

### anti-NMB-Antikörper

Ein Beispiel für einen Antikörper, der das Krebs-Zielmolekül NMB bindet und zur Behandlung von Krebs z.B. Melanom oder Brustkrebs verwendet werden kann, ist Glembatumumab (INN 9199). Ein Beispiel für ein anti-NMB Konjugat ist Glembatumumab Vedotin (CAS RN: 474645-27-7). Diese Antikörper und antigenbindende Fragmente davon sind hierin durch Verweis aufgenommen und können im Rahmen dieser Erfindung verwendet werden.

### Anti-CD56-Antikörper

Ein Beispiel für einen Antikörper, der das Krebs-Zielmolekül CD56 bindet und zur Behandlung von Krebs z.B. Multiples Myelom, Kleinzelliges Lungenkarzinom, MCC oder Ovarialkarzinom verwendet werden kann, ist Lorvotuzumab. Ein Beispiel für ein anti-CD56 Konjugat ist Lorvotuzumab Mertansine (CAS RN: 139504-50-0). Diese Antikörper und antigenbindende Fragmente davon sind hierin durch Verweis aufgenommen und können im Rahmen dieser Erfindung verwendet werden.

### anti-CD70-Antikörper

Beispiele für Antikörper, die das Krebs-Zielmolekül CD70 binden und zur Behandlung von Krebs z.B. Non-Hodgkin-Lymphom oder Nierenzellkrebs verwendet werden können, sind in WO 2007/038637-A2 oder WO 2008/070593-A2 offenbart. Ein Beispiel für ein anti-CD70 Konjugat ist SGN-75 (CD70 MMAF). Diese Antikörper und antigenbindende Fragmente davon sind hierin durch Verweis aufgenommen und können im Rahmen dieser Erfindung verwendet werden.

### anti-CD74-Antikörper

Ein Beispiel für einen Antikörper, der das Krebs-Zielmolekül CD74 bindet und zur Behandlung von Krebs z.B. Multiples Myelom verwendet werden kann, ist Milatuzumab. Ein Beispiel für ein anti-CD74 Konjugat ist Milatuzumab-Doxorubicin (CAS RN: 23214-92-8). Diese Antikörper und antigenbindende Fragmente davon sind hierin durch Verweis aufgenommen und können im Rahmen dieser Erfindung verwendet werden.

### anti-CD19-Antikörper

Ein Beispiel für einen Antikörper, der das Krebs-Zielmolekül CD19 bindet und zur Behandlung von Krebs z.B. Non-Hodgkin-Lymphom verwendet werden kann, ist in WO 2008/031056-A2 offenbart. Weitere Antikörper und Beispiele für ein anti-CD19 Konjugat (SAR3419) sind in WO 2008/047242-A2 offenbart. Diese Antikörper und antigenbindende Fragmente davon sind hierin durch Verweis aufgenommen und können im Rahmen dieser Erfindung verwendet werden.

### anti-Mucin-Antikörper

Beispiele für Antikörper, die das Krebs-Zielmolekül Mucin-1 binden und zur Behandlung von Krebs z.B. Non-Hodkin-Lymphom verwendet werden können, sind Clivatuzumab oder die in WO 2003/106495-A2, WO 2008/028686-A2 offenbarten Antikörper. Beispiele für anti-Mucin Konjugate sind in WO 2005/009369-A2 offenbart. Diese Antikörper und antigenbindende Fragmente davon sind hierin durch Verweis aufgenommen und können im Rahmen dieser Erfindung verwendet werden.

### anti-CD138-Antikörper

Beispiele für Antikörper, die das Krebs-Zielmolekül CD138 binden und Konjugate davon, die zur Behandlung von Krebs z.B. Multiples Myelom verwendet werden können, sind WO 2009/080829-A1, WO 2009/080830-A1 offenbart. Diese Antikörper und antigenbindende Fragmente davon sind hierin durch Verweis aufgenommen und können im Rahmen dieser Erfindung verwendet werden.

### anti-Integrin-alphaV-Antikörper

Beispiele für Antikörper, die das Krebs-Zielmolekül Integrin alphaV binden und zur Behandlung von Krebs z.B. Melanoma, Sarcoma oder Carcinoma verwendet werden können, sind Intetumumab (Cas RN: 725735-28-4), Abciximab (Cas-RN: 143653-53-6), Etaracizumab (Cas-RN: 892553-42-3) oder die in US 7,465,449, EP 719859-A1, WO 2002/012501-A1 oder WO2006/062779-A2 offenbarten Antikörper. Beispiele für anti-Integrin AlphaV Konjugate sind Intetumumab-DM4 und weitere in WO 2007/024536-A2 offenbarte ADCs. Diese Antikörper und antigenbindende Fragmente davon sind hierin durch Verweis aufgenommen und können im Rahmen dieser Erfindung verwendet werden.

### anti-TDGF1-Antikörper

Beispiele für Antikörper, die das Krebs-Zielmolekül TDGF1 binden und zur Behandlung von Krebs verwendet werden können, sind die in WO 02/077033-A1, US 7,318,924, WO 2003/083041-A2 und WO 2002/088170-A2 offenbarten Antikörper. Beispiele für anti-TDGF1 Konjugate sind in WO 2002/088170-A2 offenbart. Diese Antikörper und antigenbindende Fragmente davon sind hierin durch Verweis aufgenommen und können im Rahmen dieser Erfindung verwendet werden.

### anti-PSMA-Antikörper

Beispiele für Antikörper, die das Krebs-Zielmolekül PSMA binden und zur Behandlung von Krebs z.B. Prostatakarzinom verwendet werden können, sind die in WO 97/35616-A1, WO 99/47554-A1, WO 01/009192-A1 und WO2003/034903 offenbarten Antikörper. Beispiele für anti-PSMA Konjugate sind in WO 2009/026274-A1 und WO 2007/002222 offenbart. Diese Antikörper und antigenbindende Fragmente davon sind hierin durch Verweis aufgenommen und können im Rahmen dieser Erfindung verwendet werden.

### anti-EPHA2-Antikörper

Beispiele für Antikörper, die das Krebs-Zielmolekül EPHA2 binden, zur Herstellung eines Konjugats und zur Behandlung von Krebs verwendet werden können, sind in WO 2004/091375-A2 offenbart. Diese Antikörper und antigenbindende Fragmente davon sind hierin durch Verweis aufgenommen und können im Rahmen dieser Erfindung verwendet werden.

### anti- SLC44A4-Antikörper

Beispiele für Antikörper, die das Krebs-Zielmolekül SLC44A4 binden, zur Herstellung eines Konjugats und zur Behandlung von Krebs, z.B. Pankreas- oder Prostatakarzinom verwendet werden können, sind in WO2009/033094-A2 und US2009/0175796-A1 offenbart. Diese Antikörper und antigenbindende Fragmente davon sind hierin durch Verweis aufgenommen und können im Rahmen dieser Erfindung verwendet werden.

### anti-HLA-DOB-Antikörper

Ein Beispiel für einen Antikörper, der das Krebs-Zielmolekül HLA-DOB bindet, ist der Antikörper Lym-1 (Cas-RN: 301344-99-0), der zur Behandlung von Krebs, z.B. Non-Hodgkin-Lymphom verwendet werden kann. Beispiele für anti-HLA-DOB Konjugate sind z.B. in WO 2005/081711-A2 offenbart. Diese Antikörper und antigenbindende Fragmente davon sind hierin durch Verweis aufgenommen und können im Rahmen dieser Erfindung verwendet werden.

### anti- VTCN1-Antikörper

Beispiele für Antikörper, die das Krebs-Zielmolekül VTCN1 binden, zur Herstellung eines Konjugats und zur Behandlung von Krebs, z.B. Ovarialkarzinom, Pankreas-, Lungen-, oder Brustkrebs verwendet werden können, sind in WO 2006/074418-A2 offenbart. Diese Antikörper und antigenbindende Fragmente davon sind hierin durch Verweis aufgenommen und können im Rahmen dieser Erfindung verwendet werden.

### anti--FGFR2-Antikörper

Erfindungsgemäß können anti-FGFR2 Antikörper verwendet werden.

Beispiele für anti-FGFR2 Antikörper und antigen-bindende Fragmente sind in WO2013076186 beschrieben. Alle Antikörper der WO2013076186 sind hiermit in die Beschreibung der vorliegenden Erfindung durch Verweis aufgenommen und können in der vorliegenden Erfindung verwendet werden. Die Sequenzen der Antikörper sind in Tabelle 9 und Tabelle 10 der WO2013076186 angegeben. Bevorzugt sind Antikörper, antigen-bindende Fragmente und Varianten der Antikörper, die sich von den als M048-D01 und M047-D08 bezeichneten Antikörpern ableiten. Bevorzugte anti-FGFR2 binden an die verschiedenen von FGFR2 bekannten Spleißvarianten.

In einer Ausführungsform werden die anti- FGFR2 Antikörper oder antigen-bindenden Antikörperfragmente davon nach Bindung an eine FGFR2 exprimierende Zelle von der Zelle internalisiert.

In einer weiteren Ausführungsform umfassen die anti- FGFR2Antikörper oder antigen-bindenden Antikörperfragmente mindestens eine, zwei oder drei CDR Aminosäuresequenzen eines in Tabelle 9 oder Tabelle 10 von WO2013076186 angeführten Antikörpers. Bevorzugte Ausführungsformen solcher Antikörper sind ebenfalls in WO2013076186 aufgeführt und hierin durch Verweis aufgenommen.

### Anti-TWEAKR-Antikörper

In einer bevorzugten Ausführungsform wird, wenn in den Verfahren gemäß der vorliegenden Erfindung ein anti-TWEAKR-Antikörper oder ein antigenbindendes Fragment davon verwendet wird, dieser/dieses unter den nachfolgend beschriebenen ausgewählt. Darüber hinaus sind dem Fachmann Antikörper, die an TWEAKR binden bekannt, siehe z.B. WO2009/020933(A2) oder WO2009140177 (A2).

Die Erfindung betrifft insbesondere Konjugate mit Antikörpern oder antigenbindende Antikörperfragmente davon oder Varianten davon, die zu einer starken Aktivierung des TWEAKR (SEQ ID NO:169 (Protein); SEQ ID NO:170 (DNA)) führen, was zu einer starken Induktion von Apoptose in verschiedenen Krebszellen, die eine Überexpression des TWEAKR zeigen, führt.

Die agonistische Aktivität von TWEAKR in Bezug auf die Induktion von Apoptose und Inhibition der Proliferation der früher beschriebenen anti-TWEAKR-Antikörper (z.B. PDL-192) ist beschränkt und erreicht nicht die Wirksamkeit des endogenen Liganden TWEAK. Dieser Mangel an agonistischer Aktivität beruht nicht auf einer verminderten Affinität, da diese Antikörper an den TWEAKR mit Affinitäten binden, die verglichen mit dem endogenen Liganden TWEAK in einem ähnlichen Bereich liegen (Michaelson JS et al, MAbs. 2011 Jul-Aug;3(4):362-75; Culp PA et al, Clin Cancer Res. 2010 Jan 15;16(2):497-508), und auch Antikörper mit höherer Bindeaffinität nicht notwendigerweise eine wirksamere Signalgebungsaktivität zeigen (Culp PA, et al, Clin Cancer Res. 2010 Jan 15;16(2):497-508). Zusätzlich wurde gezeigt, dass die Anti-Tumoraktivität der früher beschriebenen Antikörper abhängig von der Fc-Effektorfunktion ist, und es wurde gezeigt, dass ADCC eine bedeutsame Rolle für die in-vivo-Wirksamkeit in Mausmodellen spielt.

### Erzeugung der anti-TWEAKR-Antikörper

Eine vollständig humane Antikörper-Phagen-Bibliothek (Hoet RM et al, Nat Biotechnol 2005;23(3):344-8) wurde verwendet, um TWEAKR-spezifische, humane, monoklonale Antikörper der vorliegenden Erfindung durch Protein-Panning (Hoogenboom H.R., Nat Biotechnol 2005;23(3):1105-16) mit dimeren, Fc-fusionierten extrazellulären Domänen von humanem und Maus-TWEAKR als immobilisiertes Target zu isolieren. 11 unterschiedliche Fab-Phagen wurden identifiziert, und die entsprechenden Antikörper wurden in einen Säuger-EgG-Expressionsvektor umkloniert, der die im löslichen FAb fehlenden CH2-CH3 Domänen bereitstellt. Nach der Identifizierung bevorzugter Antikörper, wurden diese als Volllängen-IgGs exprimiert. Diese Konstrukte wurden zum Beispiel transient in Säugerzellen exprimiert, wie von Tom et al., Kapitel 12 in Methods Express: Expression Systems herausgegeben von Micheal R. Dyson and Yves Durocher, Scion Publishing Ltd, 2007 (siehe AK-Beispiel 1) beschrieben. Die Antikörper wurden durch Protein-A-Chomatographie gereinigt und weiter über ihre Bindungsaffinität an löslichen monomeren TWEAKR mittels ELISA- und BIAcore-Analyse charakterisiert, wie in AK-Beispiel 2 beschrieben. Um die Zellbindungscharakteristika der anti-TWEAKR Antikörper zu bestimmen, wurde die Bindung mittels Druchflusszytometrie an einer Reihe von Zelllinien (HT29, HS68, HS578) getestet. NFκB -Reporter-Gen-Assays wurden durchgeführt, um die agonistische Aktivität aller 11 identifizierten Antikörper zu untersuchen (humanes IgG1). Der Antikörper mit der stärksten in-vitro-Wirksamkeit (TPP-883) wurde für weitere Wirksamkeits- und Affinitätsreifung ausgewählt (siehe AK-Beispiel 1 für Details). Eine Einzelsubstitutionsvariante mit verbesserter agonistischer Aktivität wurde nachgewiesen: G102T von CDR-H3. Letztlich wurden 7 Varianten basierend auf der erhöhten Affinität, verglichen mit der besten Einzelsubstitutionsvariante G102T, ausgewählt. Die entsprechende DNA davon wurde in einen Säuger-IgG-Expressionsvektor umkloniert und auf funktionale Aktivität im zuvor erwähnten NF-kappaB-Reporter-Gen-Assay untersucht. Letztlich wurden die erhaltenen Sequenzen mit humanen Keimbahnsequenzen verglichen und Abweichungen ohne signifikanten Einfluss auf die Affinität und die Wirksamkeit wurden angepasst. Die folgenden Antikörper wurden durch Antikörper-Bibliotheksscreening und durch Affinitäts- und/oder Wirksamkeitsreifung erhalten: "TPP-2090", "TPP-2149", "TPP-2093", "TPP-2148", "TPP-2084", "TPP-2077", "TPP-1538", "TPP-883", "TPP-1854", "TPP-1853", "TPP-1857", und "TPP-1858".

Antikörper der Erfindung können weiter durch fachbekannte Verfahren wie Antikörper-Phagen-Display-Screening (z.B. siehe Hoet RM et al, Nat Biotechnol 2005;23(3):344-8), der gut etablierten Hybridomtechnologie (z.B. siehe Köhler and Milstein Nature. 1975 Aug 7;256(5517):495-7), oder Immunisierung von Mäusen, unter anderem Immunisierung von hMAb Mäusen (z.B. VelocImmune mouse®) gewonnen werden.

### Besondere Ausführungsformen von anti-TWEAKR-Antikörpern

Eine Ausführungsform der Erfindung ist die Bereitstellung von Antikörpern oder antigenbindenden Antikörperfragmenten davon oder Varianten davon, die eine starke Induktion von Caspase 3/7 in einer oder mehreren TWEAKR-exprimierenden Zelllinien zeigen. In einer bevorzugten Ausführungsform ist die eine oder mehrere TWEAKR-exprimierende Zellline in der Gruppe bestehend aus WiDr, A253, NCI-H322, HT29 und 786-O enthalten. "Induktion von Caspase 3/7" kann durch übliche fachbekannte Verfahren gemessen werden, einschließlich der hierin beschriebenen. In einer Ausführungsform wird die "Induktion von Caspase 3/7" gemäß dieser Erfindung unter Verwendung der Aktivitätsbestimmung mit Caspase 3/7-Lösung (Promega, #G8093) und Auslesen der Lumineszenz auf einem VICTOR V (Perkin Elmer) bestimmt. Am Ende der Inkubationszeit wurde die Caspase 3/7-Aktivität bestimmt und der Faktor der Induktion von Caspase 3/7 wurde im Vergleich zu unbehundelten Zellen bestimmt. Es wird gesagt, dass ein Antikörper "starke Induktion" von Caspase 3/7 aufweist, wenn der Faktor der Induktion größer als 1,2, bevorzugt größer als 1,5, noch bevorzugter größer als 1,8, noch bevorzugter größer als 2,1, noch bevorzugter größer als 2,5 ist. Es werden anti-TWEAKR-Antikörper bereitgestellt, die zu einer stärkeren Induktion von Caspase 3/7 in HT29-Zellen führen, verglichen mit früher beschriebenen agonistischen Antikörpern [z.B. PDL-192(TPP-1104), P4A8(TPP-1324), 136.1(TPP-2194)] und auch verglichen mit 300 ng/ml rekombinantem humanen TWEAK. Diese starke Wirksamkeit, Caspase 3/7 in Krebszellen zu induzieren, wurde auch in WiDr-, A253-, NIC-H322- und 786-O-Zellen beobachtet, wo die untersuchten Antikörper der Erfindung in den meisten Experimenten höhere Faktoren der Veränderung verglichen mit den Referenzantikörpern [PDL-192(TPP-1104), P4A8(TPP-1324)] und mit 300 ng/ml TWEAK induzierten. Einige Antikörper der Erfindung binden an den TWEAKR mit lediglich moderater Affinität (>10nM), die klar niedriger ist, als die Affinität des endogenen Liganden TWEAK, als auch niedriger verglichen mit anderen bekannten agonitischen Antikörpern. Diese Eigenschaft bietet weitere mögliche Vorteile wie z.B. potentiell tieferes Eindringen in den Tumor.

Diesbezüglich ist eine Ausführungsform der Erfindung die Bereitstellung von Antikörpern oder antigenbindenden Antikörperfragmenten davon, die spezifisch an einen TWEAKR an einem neuen Epitop binden, das durch das selektive Binden an Aspartat (D) an der Position 47 (D47) von TWEAKR (SEQ ID NO:169; siehe auch Figur 1) ausgezeichnet ist. Die identifizierten Abhängigkeiten von bestimmten TWEAKR-Aminosäuren für die Antikörperinteraktion korrelieren mit der agonistischen Aktivität, die für diese Antikörper bestimmt worden ist. Der native Ligand TWEAK zeigt eine wirksame Aktivierung des TWEAKR und bindet abhängig von Leucin 46 in der cysteinreichen Domäne von TWEAKR (Pellegrini et al, FEBS 280:1818-1829). P4A8 zeigt eine sehr niedrige agonistische Aktivität und interagiert mindestens teilweise mit Domänen außerhalb der cysteinreichen Domäne von TWEAKR. PDL-192 zeigt eine moderate agonistische Aktivität und bindet abhängig von R56 an die cysteinreiche Domäne, aber gegenüber der TWEAK-Ligandenstelle. Antikörper dieser Erfindung (z.B. TPP-2090) binden abhängig von D47, und TWEAK bindet abhängig von L46. Somit bindet TWEAK an eine ähnliche aber unterscheidbare Bindestelle (Figur 7). Deshalb binden die Antikörper dieser Erfindung, die eine starke agonistische Aktivität zeigen, an ein neues Epitop (D47-abhängig) für Antikörper, das mit sehr starker agonistischer Aktivität Zusammenhang steht.

Die Aminosäure an der Position 47 (D47) von TWEAKR (SEQ ID NO:169) wird als kritisch für das Binden der erfindungsgemäßen Antikörper angesehen, was bedeutet, dass der Antikörper spezifisch an das D an der Position 47 (D47) von TWEAKR (SEQ ID NO:169) bindet, wenn der Antikörper mehr als 20%, alternativ mehr als 30%, alternativ mehr als 40%, alternativ mehr als 50%, alternativ mehr als 60%, alternativ mehr als 70%, alternativ mehr als 80%, alternativ mehr als 90%, alternativ 100% seines ELISA-Signals durch Änderung dieses Rests in Alanin, wie in AK-Beispiel 2 und Figur 6 beschrieben, verliert. Alternativ bindet ein Antikörper spezifisch an das D an der Position 47 (D47) von TWEAKR (SEQ ID NO:169), wenn der Antikörper mehr als 20%, alternativ mehr als 30%, alternativ mehr als 40%, alternativ mehr als 50%, alternativ mehr als 60%, alternativ mehr als 70%, alternativ mehr als 80%, alternativ mehr als 90%, alternativ 100% seines ELISA-Signals auf TPP-2614 verglichen mit TPP-2203 verliert. Bevorzugt bindet ein Antikörper spezifisch an das D an der Position 47 (D47) von TWEAKR (SEQ ID NO:169), wenn der Antikörper mehr als 80% seines ELISA-Signals auf TPP-2614 verglichen mit TPP-2203 verliert.

In dieser Anmeldung wird auf die folgenden bevorzugten Antikörper der Erfindung Bezug genommen, wie in der nachstehenden Tabelle gezeigt: "TPP-2090", "TPP-2149", "TPP-2093", "TPP-2148", "TPP-2084", "TPP-2077", "TPP-1538", "TPP-883", "TPP-1854", "TPP-1853", "TPP-1857", "TPP-1858".

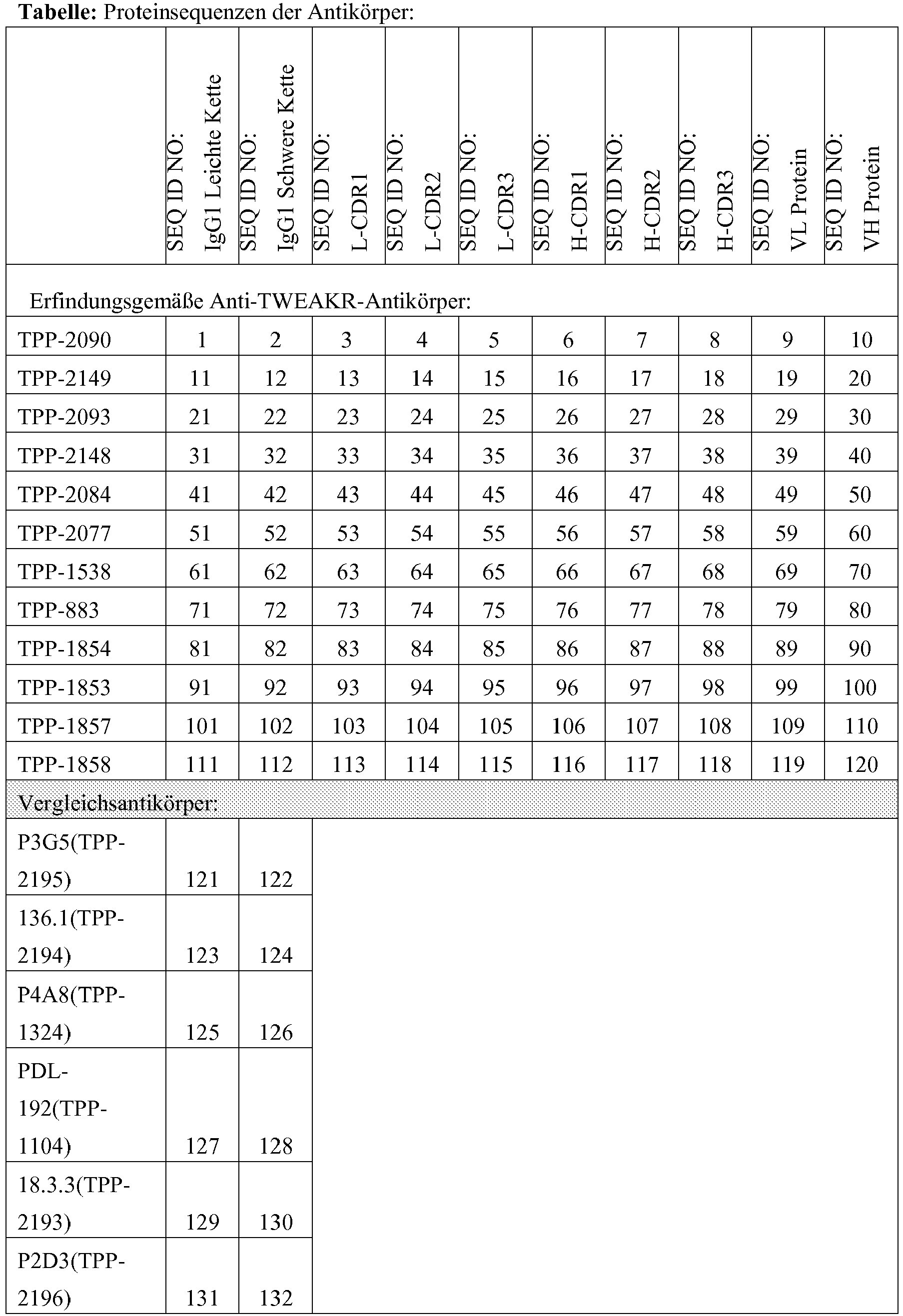

TPP-2090 ist: ein Antikörper umfassend eine Region der schweren Kette entsprechend SEQ ID NO: 2 sowie eine Region der leichten Kette entsprechend SEQ ID NO: 1.

TPP-2149 ist: ein Antikörper umfassend eine Region der schweren Kette entsprechend SEQ ID NO: 12 sowie eine Region der leichten Kette entsprechend SEQ ID NO: 11.

TPP-2093 ist: ein Antikörper umfassend eine Region der schweren Kette entsprechend SEQ ID NO: 22 sowie eine Region der leichten Kette entsprechend SEQ ID NO: 21.

TPP-2148 ist: ein Antikörper umfassend eine Region der schweren Kette entsprechend SEQ ID NO: 32 sowie eine Region der leichten Kette entsprechend SEQ ID NO: 31.

TPP-2084 ist: ein Antikörper umfassend eine Region der schweren Kette entsprechend SEQ ID NO: 42 sowie eine Region der leichten Kette entsprechend SEQ ID NO: 41.

TPP-2077 ist: ein Antikörper umfassend eine Region der schweren Kette entsprechend SEQ ID NO: 52 sowie eine Region der leichten Kette entsprechend SEQ ID NO: 51.

TPP-1538 ist: ein Antikörper umfassend eine Region der schweren Kette entsprechend SEQ ID NO: 62 sowie eine Region der leichten Kette entsprechend SEQ ID NO: 61.

TPP-883 ist: ein Antikörper umfassend eine Region der schweren Kette entsprechend SEQ ID NO: 72 sowie eine Region der leichten Kette entsprechend SEQ ID NO: 71.

TPP-1854 ist: ein Antikörper umfassend eine Region der schweren Kette entsprechend SEQ ID NO: 82 sowie eine Region der leichten Kette entsprechend SEQ ID NO: 81.

TPP-1853 ist: ein Antikörper umfassend eine Region der schweren Kette entsprechend SEQ ID NO: 92 sowie eine Region der leichten Kette entsprechend SEQ ID NO: 91.

TPP-1857 ist: ein Antikörper umfassend eine Region der schweren Kette entsprechend SEQ ID NO: 102 sowie eine Region der leichten Kette entsprechend SEQ ID NO: 101.

TPP-1858 ist: ein Antikörper umfassend eine Region der schweren Kette entsprechend SEQ ID NO: 112 sowie eine Region der leichten Kette entsprechend SEQ ID NO: 111.

TPP-2090 ist: ein Antikörper umfassend eine variable Region der schweren Kette entsprechend SEQ ID NO: 10 sowie eine variable Region der leichten Kette entsprechend SEQ ID NO: 9.

TPP-2149 ist: ein Antikörper umfassend eine variable Region der schweren Kette entsprechend SEQ ID NO: 20 sowie eine variable Region der leichten Kette entsprechend SEQ ID NO: 19.

TPP-2093 ist: ein Antikörper umfassend eine variable Region der schweren Kette entsprechend SEQ ID NO: 30 sowie eine variable Region der leichten Kette entsprechend SEQ ID NO: 29.

TPP-2148 ist: ein Antikörper umfassend eine variable Region der schweren Kette entsprechend SEQ ID NO: 40 sowie eine variable Region der leichten Kette entsprechend SEQ ID NO: 39.

TPP-2084 ist: ein Antikörper umfassend eine variable Region der schweren Kette entsprechend SEQ ID NO: 50 sowie eine variable Region der leichten Kette entsprechend SEQ ID NO: 49.

TPP-2077 ist: ein Antikörper umfassend eine variable Region der schweren Kette entsprechend SEQ ID NO: 60 sowie eine variable Region der leichten Kette entsprechend SEQ ID NO: 59.

TPP-1538 ist: ein Antikörper umfassend eine variable Region der schweren Kette entsprechend SEQ ID NO: 70 sowie eine variable Region der leichten Kette entsprechend SEQ ID NO: 69.

TPP-883 ist: ein Antikörper umfassend eine variable Region der schweren Kette entsprechend SEQ ID NO: 80 sowie eine variable Region der leichten Kette entsprechend SEQ ID NO: 79.

TPP-1854 ist: ein Antikörper umfassend eine variable Region der schweren Kette entsprechend SEQ ID NO: 90 sowie eine variable Region der leichten Kette entsprechend SEQ ID NO: 89.

TPP-1853 ist: ein Antikörper umfassend a eine variable Region der schweren Kette entsprechend SEQ ID NO: 100 sowie a eine variable Region der leichten Kette entsprechend SEQ ID NO: 99.

TPP-1857 ist: ein Antikörper umfassend eine variable Region der schweren Kette entsprechend SEQ ID NO: 110 sowie eine variable Region der leichten Kette entsprechend SEQ ID NO: 109.

TPP-1858 ist: ein Antikörper umfassend eine variable Region der schweren Kette entsprechend SEQ ID NO: 120 sowie eine variable Region der leichten Kette entsprechend SEQ ID NO: 119.

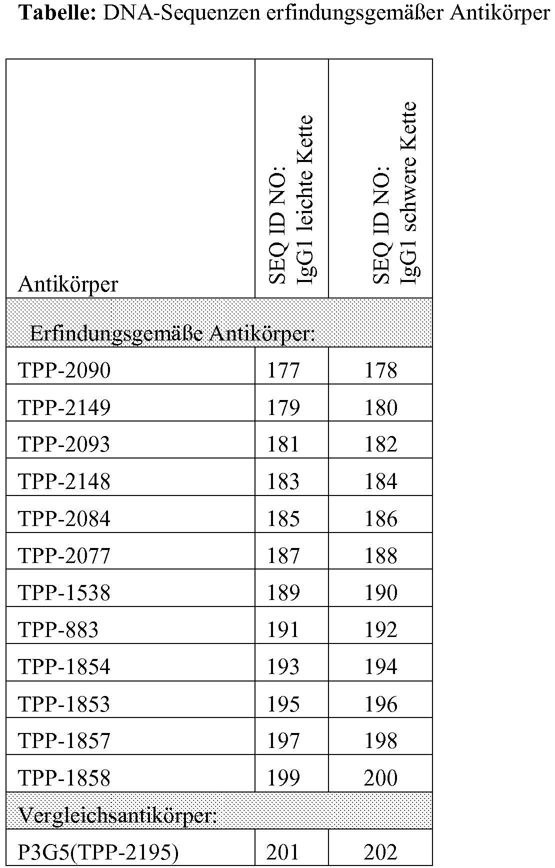

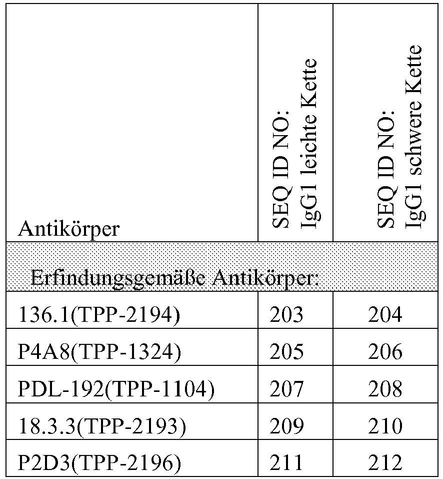

Bevorzugte Ausführungsformen des anti-TWEAKR-Antikörpers sind die folgenden:
1. Ein Anti-TWEAKR-Antikörper oder ein antigenbindendes Fragment davon, der/das spezifisch an das D an der Position 47 (D47) des TWEAKR (SEQ ID NO:169) bindet.
2. Der Antikörper oder ein antigenbindendes Fragment davon gemäß Ausführungsform 1, wobei der Antikörper ein agonistischer Antikörper ist.
3. Der Antikörper oder ein antigenbindendes Fragment davon gemäß den Ausführungsformen 1 oder 2, der/das umfasst:
   eine variable schwere Kette umfassend:
      (a) eine CDR1 der schweren Kette, die von einer Aminosäuresequenz umfassend die Formel PYPMX (SEQ ID NO: 171) kodiert wird, wobei X I oder M ist;
      (b) eine CDR2 der schweren Kette, die von einer Aminosäuresequenz umfassend die Formel YISPSGGXTHYADSVKG (SEQ ID NO: 172) kodiert wird, wobei X S oder K ist; und
      (c) eine CDR3 der schweren Kette, die von einer Aminosäuresequenz umfassend die Formel GGDTYFDYFDY (SEQ ID NO: 173) kodiert wird;
      und eine variable leichte Kette umfassend:
      (a) eine CDR1 der leichten Kette, die von einer Aminosäuresequenz umfassend die Formel RASQSISXYLN (SEQ ID NO: 174) kodiert wird, wobei X G oder S ist;
      (b) eine CDR2 der leichten Kette, die von einer Aminosäuresequenz umfassend die Formel XASSLQS (SEQ ID NO: 175) kodiert wird, wobei X Q , A oder N ist; und
      (c) eine CDR3 der leichten Kette, die von einer Aminosäuresequenz umfassend die Formel QQSYXXPXIT (SEQ ID NO: 176) kodiert wird, wobei X an der Position 5 T oder S ist, X an der Position 6 T oder S ist und X an der Position 8 G oder F ist.
4. Der Antikörper oder ein antigenbindendes Fragment davon gemäß einer der vorhergehenden Ausführungsformen umfassend:
   a. eine variable schwere Kette umfassend die variable CDR1-Sequenz der schweren Kette, wie dargestellt durch SEQ ID NO: 6, die variable CDR2-Sequenz der schweren Kette, wie dargestellt durch SEQ ID NO: 7 und die variable CDR3-Sequenz der schweren Kette, wie dargestellt durch SEQ ID NO: 8, sowie
      eine variable leichte Kette umfassend die variable CDR1-Sequenz der leichten Kette dargestellt durch SEQ ID NO: 3, die variable CDR2-Sequenz der leichten Kette dargestellt durch SEQ ID NO: 4 und die variable CDR3-Sequenz der leichten Kette dargestellt durch SEQ ID NO: 5 oder
   b. eine variable schwere Kette umfassend die variable CDR1-Sequenz der schweren Kette, wie dargestellt durch SEQ ID NO: 16, die variable CDR2-Sequenz der schweren Kette, wie dargestellt durch SEQ ID NO: 17, die variable CDR3-Sequenz der schweren Kette, wie dargestellt durch SEQ ID NO:18, sowie
      eine variable leichte Kette umfassend die variable CDR1-Sequenz der leichten Kette dargestellt durch SEQ ID NO: 13, die variable CDR2-Sequenz der leichten Kette dargestellt durch SEQ ID NO: 14 und die variable CDR3-Sequenz der leichten Kette dargestellt durch SEQ ID NO:15 oder
   c. eine variable schwere Kette umfassend die variable CDR1-Sequenz der schweren Kette, wie dargestellt durch SEQ ID NO: 26, die variable CDR2-Sequenz der schweren Kette, wie dargestellt durch SEQ ID NO: 27, die variable CDR3-Sequenz der schweren Kette, wie dargestellt durch SEQ ID NO:28, sowie
      eine variable leichte Kette umfassend die variable CDR1-Sequenz der leichten Kette dargestellt durch SEQ ID NO: 23, die variable CDR2-Sequenz der leichten Kette dargestellt durch SEQ ID NO: 24 und die variable CDR3-Sequenz der leichten Kette dargestellt durch SEQ ID NO:25 oder
   d. eine variable schwere Kette umfassend die variable CDR1-Sequenz der schweren Kette, wie dargestellt durch SEQ ID NO: 36, die variable CDR2-Sequenz der schweren Kette, wie dargestellt durch SEQ ID NO: 37, die variable CDR3-Sequenz der schweren Kette, wie dargestellt durch SEQ ID NO:38, sowie
      eine variable leichte Kette umfassend die variable CDR1-Sequenz der leichten Kette dargestellt durch SEQ ID NO: 33, die variable CDR2-Sequenz der leichten Kette dargestellt durch SEQ ID NO: 34 und die variable CDR3-Sequenz der leichten Kette dargestellt durch SEQ ID NO:35 oder
   e. eine variable schwere Kette umfassend die variable CDR1-Sequenz der schweren Kette, wie dargestellt durch SEQ ID NO: 46, die variable CDR2-Sequenz der schweren Kette, wie dargestellt durch SEQ ID NO: 47, die variable CDR3-Sequenz der schweren Kette, wie dargestellt durch SEQ ID NO:48, sowie
      eine variable leichte Kette umfassend die variable CDR1-Sequenz der leichten Kette dargestellt durch SEQ ID NO: 43, die variable CDR2-Sequenz der leichten Kette dargestellt durch SEQ ID NO: 44 und die variable CDR3-Sequenz der leichten Kette dargestellt durch SEQ ID NO:45 oder
   f. eine variable schwere Kette umfassend die variable CDR1-Sequenz der schweren Kette, wie dargestellt durch SEQ ID NO: 56, die variable CDR2-Sequenz der schweren Kette, wie dargestellt durch SEQ ID NO: 57, die variable CDR3-Sequenz der schweren Kette, wie dargestellt durch SEQ ID NO:58, sowie
      eine variable leichte Kette umfassend die variable CDR1-Sequenz der leichten Kette dargestellt durch SEQ ID NO: 53, die variable CDR2-Sequenz der leichten Kette dargestellt durch SEQ ID NO: 54 und die variable CDR3-Sequenz der leichten Kette dargestellt durch SEQ ID NO:55 oder
   g. eine variable schwere Kette umfassend die variable CDR1-Sequenz der schweren Kette, wie dargestellt durch SEQ ID NO: 66, die variable CDR2-Sequenz der schweren Kette, wie dargestellt durch SEQ ID NO: 67, die variable CDR3-Sequenz der schweren Kette, wie dargestellt durch SEQ ID NO:68, sowie
      eine variable leichte Kette umfassend die variable CDR1-Sequenz der leichten Kette dargestellt durch SEQ ID NO: 63, die variable CDR2-Sequenz der leichten Kette dargestellt durch SEQ ID NO: 64 und die variable CDR3-Sequenz der leichten Kette dargestellt durch SEQ ID NO:65oder
   h. eine variable schwere Kette umfassend die variable CDR1-Sequenz der schweren Kette, wie dargestellt durch SEQ ID NO: 76, die variable CDR2-Sequenz der schweren Kette, wie dargestellt durch SEQ ID NO: 77, die variable CDR3-Sequenz der schweren Kette, wie dargestellt durch SEQ ID NO:78, sowie
      eine variable leichte Kette umfassend die variable CDR1-Sequenz der leichten Kette dargestellt durch SEQ ID NO: 73, die variable CDR2-Sequenz der leichten Kette dargestellt durch SEQ ID NO: 74 und die variable CDR3-Sequenz der leichten Kette dargestellt durch SEQ ID NO:75 oder
   i. eine variable schwere Kette umfassend die variable CDR1-Sequenz der schweren Kette, wie dargestellt durch SEQ ID NO: 86, die variable CDR2-Sequenz der schweren Kette, wie dargestellt durch SEQ ID NO: 87, die variable CDR3-Sequenz der schweren Kette, wie dargestellt durch SEQ ID NO:88, sowie
      eine variable leichte Kette umfassend die variable CDR1-Sequenz der leichten Kette dargestellt durch SEQ ID NO: 83, die variable CDR2-Sequenz der leichten Kette dargestellt durch SEQ ID NO: 84 und die variable CDR3-Sequenz der leichten Kette dargestellt durch SEQ ID NO:85 oder
   j. eine variable schwere Kette umfassend die variable CDR1-Sequenz der schweren Kette, wie dargestellt durch SEQ ID NO: 96, die variable CDR2-Sequenz der schweren Kette, wie dargestellt durch SEQ ID NO: 97, die variable CDR3-Sequenz der schweren Kette, wie dargestellt durch SEQ ID NO:98, sowie
      eine variable leichte Kette umfassend die variable CDR1-Sequenz der leichten Kette dargestellt durch SEQ ID NO: 93, die variable CDR2-Sequenz der leichten Kette dargestellt durch SEQ ID NO: 94 und die variable CDR3-Sequenz der leichten Kette dargestellt durch SEQ ID NO:95 oder
   k. eine variable schwere Kette umfassend die variable CDR1-Sequenz der schweren Kette, wie dargestellt durch SEQ ID NO: 106, die variable CDR2-Sequenz der schweren Kette, wie dargestellt durch SEQ ID NO: 107, die variable CDR3-Sequenz der schweren Kette, wie dargestellt durch SEQ ID NO:108, sowie
      eine variable leichte Kette umfassend die variable CDR1-Sequenz der leichten Kette dargestellt durch SEQ ID NO: 103, die variable CDR2-Sequenz der leichten Kette dargestellt durch SEQ ID NO: 104 und die variable CDR3-Sequenz der leichten Kette dargestellt durch SEQ ID NO:105 oder
   l. eine variable schwere Kette umfassend die variable CDR1-Sequenz der schweren Kette, wie dargestellt durch SEQ ID NO: 116, die variable CDR2-Sequenz der schweren Kette, wie dargestellt durch SEQ ID NO: 117, die variable CDR3-Sequenz der schweren Kette, wie dargestellt durch SEQ ID NO:118, sowie
      eine variable leichte Kette umfassend die variable CDR1-Sequenz der leichten Kette dargestellt durch SEQ ID NO: 113, die variable CDR2-Sequenz der leichten Kette dargestellt durch SEQ ID NO: 114 und die variable CDR3-Sequenz der leichten Kette dargestellt durch SEQ ID NO:115.
5. Der Antikörper oder das antigenbindende Fragment davon gemäß gemäß einer der vorhergehenden Ausführungsformen, umfassend:
   a. eine variable Sequenz der schweren Kette, wie dargestellt durch SEQ ID NO:10, sowie eine variable Sequenz der leichten Kette, wie dargestellt durch SEQ ID NO:9 oder
   b. eine variable Sequenz der schweren Kette, wie dargestellt durch SEQ ID NO:20, sowie eine variable Sequenz der leichten Kette, wie dargestellt durch SEQ ID NO:19 oder
   c. eine variable Sequenz der schweren Kette, wie dargestellt durch SEQ ID NO:30, sowie eine variable Sequenz der leichten Kette, wie dargestellt durch SEQ ID NO:29 oder
   d. eine variable Sequenz der schweren Kette, wie dargestellt durch SEQ ID NO:40, sowie eine variable Sequenz der leichten Kette, wie dargestellt durch SEQ ID NO:39 oder
   e. eine variable Sequenz der schweren Kette, wie dargestellt durch SEQ ID NO:50, sowie eine variable Sequenz der leichten Kette, wie dargestellt durch SEQ ID NO:49 oder
   f. eine variable Sequenz der schweren Kette, wie dargestellt durch SEQ ID NO:60, sowie eine variable Sequenz der leichten Kette, wie dargestellt durch SEQ ID NO:59 oder
   g. eine variable Sequenz der schweren Kette, wie dargestellt durch SEQ ID NO:70, sowie eine variable Sequenz der leichten Kette, wie dargestellt durch SEQ ID NO:69 oder
   h. eine variable Sequenz der schweren Kette, wie dargestellt durch SEQ ID NO:80, sowie eine variable Sequenz der leichten Kette, wie dargestellt durch SEQ ID NO:79 oder
   i. eine variable Sequenz der schweren Kette, wie dargestellt durch SEQ ID NO:90, sowie eine variable Sequenz der leichten Kette, wie dargestellt durch SEQ ID NO:89 oder
   j. eine variable Sequenz der schweren Kette, wie dargestellt durch SEQ ID NO:100, sowie eine variable Sequenz der leichten Kette, wie dargestellt durch SEQ ID NO:99 oder
   k. eine variable Sequenz der schweren Kette, wie dargestellt durch SEQ ID NO:110, sowie eine variable Sequenz der leichten Kette, wie dargestellt durch SEQ ID NO:109 oder
   l. eine variable Sequenz der schweren Kette, wie dargestellt durch SEQ ID NO:120, sowie eine variable Sequenz der leichten Kette, wie dargestellt durch SEQ ID NO:119.
6. Der Antikörper gemäß einer der vorhergehenden Ausführungsformen, der ein IgG Antikörper ist.
7. Der Antikörper gemäß einer der vorhergehenden Ausführungsformen, umfassend:
   a. eine Sequenz der schweren Kette, wie dargestellt durch SEQ ID NO:2, sowie eine Sequenz der leichten Kette, wie dargestellt durch SEQ ID NO:1 oder
   b. eine Sequenz der schweren Kette, wie dargestellt durch SEQ ID NO:12, sowie eine Sequenz der leichten Kette, wie dargestellt durch SEQ ID NO:11 oder
   c. eine Sequenz der schweren Kette, wie dargestellt durch SEQ ID NO:22, sowie eine Sequenz der leichten Kette, wie dargestellt durch SEQ ID NO:21 oder
   d. eine Sequenz der schweren Kette, wie dargestellt durch SEQ ID NO:32, sowie eine Sequenz der leichten Kette, wie dargestellt durch SEQ ID NO:31 oder
   e. eine Sequenz der schweren Kette, wie dargestellt durch SEQ ID NO:42, sowie eine Sequenz der leichten Kette, wie dargestellt durch SEQ ID NO:41 oder
   f. eine Sequenz der schweren Kette, wie dargestellt durch SEQ ID NO:52, sowie eine Sequenz der leichten Kette, wie dargestellt durch SEQ ID NO:51 oder
   g. eine Sequenz der schweren Kette, wie dargestellt durch SEQ ID NO:62, sowie eine Sequenz der leichten Kette, wie dargestellt durch SEQ ID NO:61 oder
   h. eine Sequenz der schweren Kette, wie dargestellt durch SEQ ID NO:72, sowie eine Sequenz der leichten Kette, wie dargestellt durch SEQ ID NO:71 oder
   i. eine Sequenz der schweren Kette, wie dargestellt durch SEQ ID NO:82, sowie eine Sequenz der leichten Kette, wie dargestellt durch SEQ ID NO:81 oder
   j. eine Sequenz der schweren Kette, wie dargestellt durch SEQ ID NO:92, sowie eine Sequenz der leichten Kette, wie dargestellt durch SEQ ID NO:91 oder
   k. eine Sequenz der schweren Kette, wie dargestellt durch SEQ ID NO:102, sowie eine Sequenz der leichten Kette, wie dargestellt durch SEQ ID NO:101 oder
   l. eine Sequenz der schweren Kette, wie dargestellt durch SEQ ID NO:112, sowie eine Sequenz der leichten Kette, wie dargestellt durch SEQ ID NO:111.
8. Das antigenbindende Fragment gemäß einer der vorhergehenden Ausführungsformen oder ein antigenbindendes Fragment eines Antikörpers gemäß einer der vorhergehenden Ausführungsformen, das ein scFv-, Fab-, Fab' -Fragment oder ein F(ab')₂-Fragment ist.
9. Der Antikörper oder das antigenbindende Fragment gemäß gemäß einer der vorhergehenden Ausführungsformen, der ein monoklonaler Antikörper oder ein antigenbindendes Fragment davon ist.
10. Der Antikörper oder das antigenbindende Fragment gemäß einem der vorhergehenden Ansprüche, der ein humaner, humanisierter oder chimärer Antikörper oder ein antigenbindendes Fragment ist.

Besonders bevorzugt ist der anti-TWEAKR-Antikörper TPP-2090 .

### Isotope, Salze, Solvate, isotopische Varianten

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C , ¹⁵N , ¹⁷O, ¹⁸O, ³²P, ³³P, ³³S, ³⁴S, ³⁵S, ³⁶S, ¹⁸F , ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagenzien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

### Besondere Ausführungsformen

Die folgenden Ausführungsformen sind besonders bevorzugt:

### Ausführungsform A:

Ein ADC der Formel 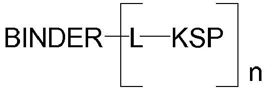 wobei KSP-L- eine Verbindung der folgenden Formel (II), (IIa), (IIb), (IIc), (IId), (IIe) oder der folgenden Formel (IIf)ist, der Binder ein anti-TWEAKR-Antikörper (besonders bevorzugt ein anti-TWEAKR-Antikörper, der spezifisch an die Aminosäure D in Position 47 (D47) von TWEAKR (SEQ ID NO:169) bindet, insbesondere der anti-TWEAK R-Antikörper TPP-2090), und n eine Zahl von 1 bis 10 ist:
Formel (IIf): 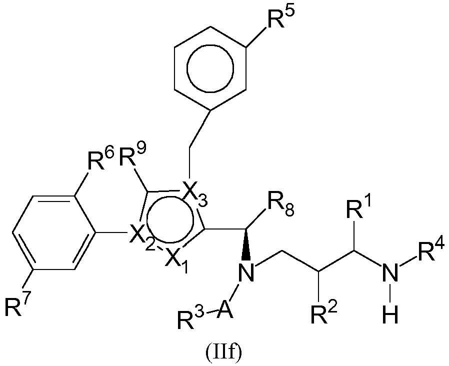 wobei
X₁ N, X₂ N und X₃ C darstellt;
X₁ CH, X₂ C und X₃ N darstellt;
X₁ NH, X₂ C und X₃ C darstellt; oder
X₁ CH, X₂ N und X₃ C darstellt;
A CO (carbonyl) ist;
R¹ -L-#1, H, -COOH, -CONHNH₂, -(CH₂)₁₋₃NH₂, -CONZ"(CH₂)₁₋₃ NH₂ und -CONZ"CH₂COOH ist, wobei Z" H oder NH₂;
R² und R⁴ H oder -L-#1ist, oder R² und R⁴ gemeinsam (unter Bildung eines Pyrrolidinringes) -CH₂-CHR¹⁰- oder -CHR¹⁰-CH₂- darstellen, wobei R¹⁰ H oder -L-#1, darstellt;
R³ -L-#1 oder eine C1-10-Alkyl-, die ggf. mit -OH, O-Alkyl, SH, S-Alkyl, O-CO-Alkyl, O-CO-NH-Alkyl, NH-CO-Alkyl, NH-CO-NH-Alkyl, S(O)ₙ-Alkyl, SO₂-NH-Alkyl, NH-Alkyl, N(Alkyl)2, oder NH₂ substituiert sein kann (wobei Alkyl vorzugsweise C₁₋₃ Alkyl bedeutet) ist;
R⁵ -L-#1, H oder F ist;
R⁶ und R⁷ unabhängig voneinander H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist, die mit -L-#1 substituiert sein kann; und
R⁹ H oder F ist,
wobei einer der Substituenten R¹, R², R³, R⁴, R⁵, R⁸ und R¹⁰ -L-#1 darstellt, und -L-den Linker und #1 die Bindung zum Antikörper darstellt,
sowie Salze, Solvate und Salze der Solvate des ADCs.

Der Linker ist vorzugsweise ein Linker

§-(CO)m-L1-L2-§§

wobei
m 0 oder 1 ist;
§ die Bindung an KSP darstellt und
§ § die Bindung an den Antikörper darstellt, und
L2 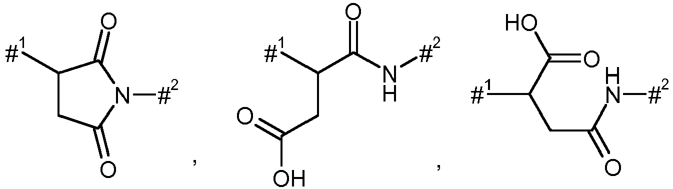 oder 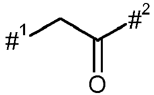 darstellt, wobei
   #¹ die Verknüpfungsstelle mit dem Schwefelatom des Antikörpers kennzeichnet,
   #² die Verknüpfungsstelle mit der Gruppe L¹ kennzeichnet,
   und L1 durch die Formel

      #¹-(NR¹⁰)ₙ₋(G0)ₒ-G2-#²
   dargestellt wird,
   wobei
   R¹⁰ H, NH₂ oder C1-C3-Alkyl darstellt;
   G1 -NHCO- oder 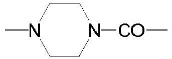 darstellt;
   n 0 oder 1 ist;
   o 0 oder 1 ist; und
   G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO2, -NH-, -CO-, - NHCO-, -CONH-, -NMe-, -NHNH-, -SO2NHNH-, -CONHNH- und einen 3 bis 10gliedriger, aromatischen oder nicht aromatischen Heterozyklus mit bis zu 4 Heteroatomen ausgewählt aus N, O und S, oder -SO- unterbrochen sein kann (vorzugsweise 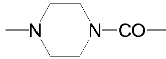 ), wobei die Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein können.

Hierbei ist #¹ die Bindung zum KSP-Inhibitor und #² die Bindung zur Kopplungsgruppe zum Binder (z.B. L2).

### Ausführungsform B:

Ein ADC der Formel 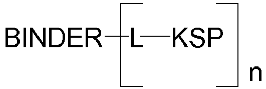 wobei KSP-L- eine Verbindung der folgenden Formel (II), (IIa), (IIb), (IIc), (IId), (IIe), (IIf) oder der folgenden Formel (IIg)ist, der Binder ein Antikörper ist, und n eine Zahl von 1 bis 10 ist:
Formel (IIg): 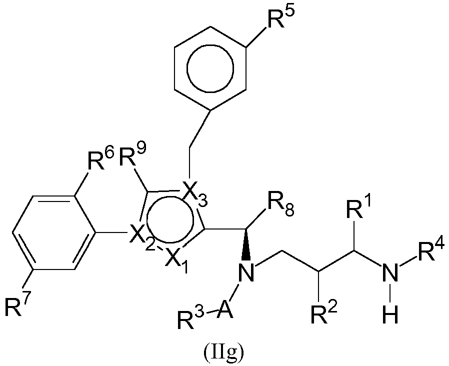 wobei
X₁ N, X₂ N und X₃ C darstellt;
X₁ CH, X₂ C und X₃ N darstellt;
X₁ NH, X₂ C und X₃ C darstellt; oder
X₁ CH, X₂ N und X₃ C darstellt;
A CO (carbonyl) ist;
R¹ -L-#1, H, -COOH, -CONHNH₂, -(CH₂)₁₋₃NH₂, -CONZ"(CH₂)₁₋₃ NH₂ und -CONZ"CH₂COOH ist, wobei Z" H oder NH₂;
R² und R⁴ H oder -L-#1 ist, oder R² und R⁴ gemeinsam (unter Bildung eines Pyrrolidinringes) -CH₂-CHR¹⁰- oder -CHR¹⁰-CH₂- darstellen, wobei R¹⁰ H oder -L-#1, darstellt;
R³ -L-#1 oder eine C₁₋₁₀-Alkyl-, die ggf. mit -OH, O-Alkyl, SH, S-Alkyl, O-CO-Alkyl, O-CO-NH-Alkyl, NH-CO-Alkyl, NH-CO-NH-Alkyl, S(O)ₙ-Alkyl, SO₂-NH-Alkyl, NH-Alkyl, N(Alkyl)₂, oder NH₂ substituiert sein kann (wobei Alkyl vorzugsweise C₁₋₃ Alkyl bedeutet) ist;
R⁵ -L-#1, H oder F ist;
R⁶ und R⁷ unabhängig voneinander H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist; und
R⁹ H oder F ist,
wobei einer der Substituenten R¹, R², R³, R⁴, R⁵ und R¹⁰ -L-#1 darstellt, und -L-den Linker und #1 die Bindung zum Antikörper darstellt,
wobei -L- dargestellt wird durch

   §-(CO)m-L1-L2-§§

   wobei
   m 0 oder 1 ist;
   § die Bindung an KSP darstellt und
   § § die Bindung an den Antikörper darstellt, und
   L2 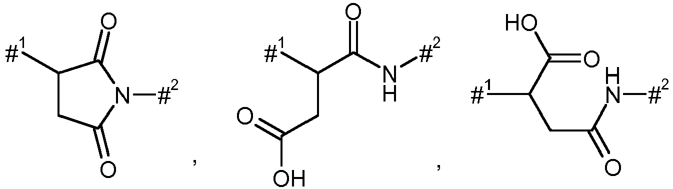 oder 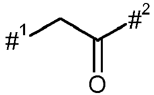
   darstellt, wobei
   - #¹: die Verknüpfungsstelle mit dem Schwefelatom des Antikörpers kennzeichnet,
   - #²: die Verknüpfungsstelle mit der Gruppe L¹ kennzeichnet,
   und L1 durch die Formel

   #¹-(NR¹⁰)ₙ₋(G0)ₒ-G2-#²

   dargestellt wird,
   wobei
   R¹⁰ H, NH₂ oder C1-C3-Alkyl darstellt;
   G1 -NHCO- oder 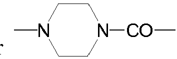 darstellt;
   n 0 oder 1 ist;
   o 0 oder 1 ist; und
   G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO2, -NH-, -CO-, - NHCO-, -CONH-, -NMe-, -NHNH-, -SO2NHNH-, -CONHNH- und einen 3 bis 10gliedriger, aromatischen oder nicht aromatischen Heterozyklus mit bis zu 4 Heteroatomen ausgewählt aus N, O und S, oder -SO- unterbrochen sein kann (vorzugsweise 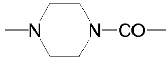 ), wobei die Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein können,
   #¹ die Bindung zum KSP-Inhibitor und #² die Bindung zur Kopplungsgruppe zum Antikörper ist (z.B. L2),
   sowie Salze, Solvate und Salze der Solvate des ADCs.

### Ausführungsform C:

Ein ADC der Formel 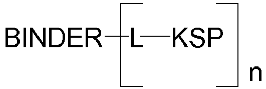 wobei KSP-L- eine Verbindung mit der folgenden Substruktur I(sub) ist, der Binder ein anti-TWEAKR-Antikörper (besonders bevorzugt ein anti-TWEAKR-Antikörper, der spezifisch an die Aminosäure D in Position 47 (D47) von TWEAKR (SEQ ID NO:169) bindet, insbesondere der anti-TWEAK R-Antikörper TPP-2090), anti-HER2-Antikörper oder anti-EGRF-Antikörper (bevorzugt Nimotuzumab) ist, und n eine Zahl von 1 bis 10 ist: 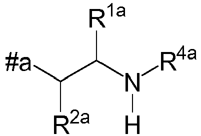 wobei
#a eine Bindung zum Restmolekül darstellt;
R^{1a} -L-#1, H oder -(CH₂)₀₋₃Z darstellt, wobei Z -H, Halogen, -NHY³, -CO-NY¹Y², oder -CO-OY³ darstellt, mit Y¹ und Y² unabhängig voneinander H, NH2, oder -(CH₂CH₂O)₀₋₃-(CH₂)₀₋₃Z' darstellen, und Y³ H oder -(CH₂)₀₋₃Z' darstellt, wobei Z' H, SO₃H, NH₂, COOH oder -(CO-NH-CHY⁴)_{1- 3}COOH darstellt; wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R^{2a} und R^{4a} unabhängig voneinander H, -L-#1, -CO-CHY⁴-NHY⁵ oder -(CH₂)₀₋₃Z darstellen, oder R^{2a} und R^{4a} gemeinsam (unter Bildung eines Pyrrolidinringes) -CH₂-CHR¹⁰- oder -CHR¹⁰-CH₂- darstellen, wobei R¹⁰ -L-#1, H, NH₂, COOH, SO₃H, SH, oder OH darstellt, und wobei Z - H, -CO-NY¹Y², oder -CO-OY³ darstellt,
wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt,
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
wobei einer der Substituenten R^{1a}, R^{2a}, R^{4a} oder R¹⁰ -L-#1 darstellt,
-L-den Linker und #1 die Bindung zum Antikörper darstellt,
wobei -L- dargestellt wird durch

   §-(CO)m-L1-L2-§§

   wobei
   m 0 oder 1 ist;
   § die Bindung an KSP darstellt und
   § § die Bindung an den Antikörper darstellt, und
   L2 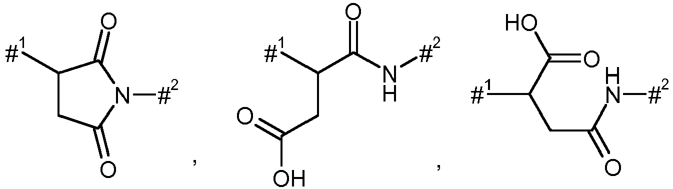 oder 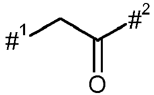
   darstellt, wobei
   - #¹: die Verknüpfungsstelle mit dem Schwefelatom des Antikörpers kennzeichnet,
   - #²: die Verknüpfungsstelle mit der Gruppe L¹ kennzeichnet,
   und L1 durch die Formel

   #¹-(NR¹⁰)ₙ₋(G1)ₒ-G2-#²

   dargestellt wird,
   wobei
   R¹⁰ H, NH2 oder C1-C3-Alkyl darstellt;
   G1 -NHCO- oder 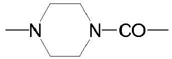 darstellt;
   n 0 oder 1 ist;
   o 0 oder 1 ist; und
   G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO₂, -NH-, -CO-, - NHCO-, -CONH-, -NMe-, -NHNH-, -SO₂NHNH-, -CONHNH- und einen 3 bis 10gliedriger, aromatischen oder nicht aromatischen Heterozyklus mit bis zu 4 Heteroatomen ausgewählt aus N, O und
   S, oder -SO- unterbrochen sein kann (vorzugsweise 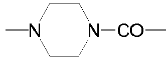 ), wobei die Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein können,
   #¹ die Bindung zum KSP-Inhibitor und #² die Bindung zur Kopplungsgruppe zum Antikörper ist (z.B. L2),
   sowie Salze, Solvate und Salze der Solvate des ADCs.

### Ausführungsform D:

Ein ADC der Formel 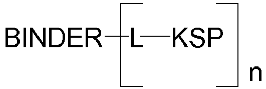 wobei KSP-L- eine Verbindung der folgenden Formel (II), (IIa), (IIb), (IIc), (IId), (IIe), (IIf), (IIg) oder der folgenden Formel (IIh)ist, der Binder ein Antikörper, und n eine Zahl von 1 bis 10 ist:
Formel (IIh): 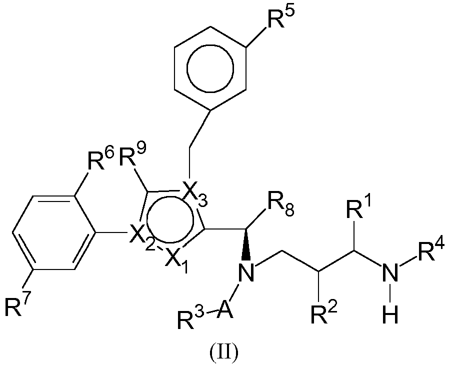 wobei
X₁ N, X₂ N und X₃ C darstellt;
X₁ CH, X₂ C und X₃ N darstellt;
X₁ NH, X₂ C und X₃ C darstellt; oder
X₁ CH, X₂ N und X₃ C darstellt;
A CO (carbonyl) ist;
R¹ -L-#1 ist,;
R² und R⁴ H ist, oder R² und R⁴ gemeinsam (unter Bildung eines Pyrrolidinringes) -CH₂-CHR¹⁰- oder - CHR¹⁰-CH₂- darstellen, wobei R¹⁰ H darstellt;
   R³ eine C₁₋₁₀-Alkyl-, die ggf. mit -OH, O-Alkyl, SH, S-Alkyl, O-CO-Alkyl, O-CO-NH-Alkyl, NH-CO-Alkyl, NH-CO-NH-Alkyl, S(O)ₙ-Alkyl, SO₂-NH-Alkyl, NH-Alkyl, N(Alkyl)2, oder NH₂ substituiert sein kann (wobei Alkyl vorzugsweise C₁₋₃ Alkyl bedeutet), oder -MOD ist;
   wobei -MOD -(NR¹⁰)ₙ-(G1)ₒ-G2-H darstellt, wobei
   R¹⁰ H oder C₁-C₃-Alkyl darstellt;
   G1 -NHCO- , -CONH- oder 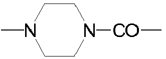 darstellt (wobei wenn G1 -NHCO- oder 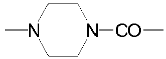 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NRY-, -NRYCO-, CONRY-, -NRYNRY-, - SO2NR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, - NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
R⁵ H oder F ist;
R⁶ und R⁷ unabhängig voneinander H, (ggf. fluoriertes) C₁₋₃-Alkyl, (ggf. fluoriertes) C₂₋₄-Alkenyl, (ggf. fluoriertes) C₂₋₄-Alkinyl, Hydroxy oder Halogen darstellen;
R⁸ eine verzweigte C₁₋₅-Alkyl-Gruppe ist; und
R⁹ H oder F ist,
wobei -L-den Linker und #1 die Bindung zum Antikörper darstellt,
wobei -L- dargestellt wird durch

   §-(CO)m-L1-L2-§§

   wobei
   m 0 oder 1 ist;
   § die Bindung an KSP darstellt und
   § § die Bindung an den Antikörper darstellt, und
   L2 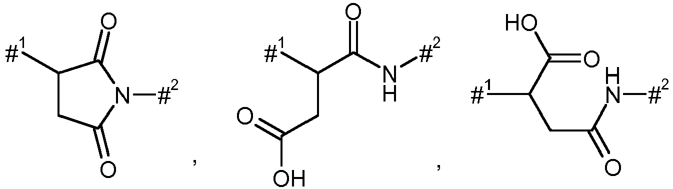 oder 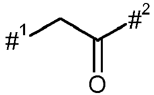 darstellt, wobei
      - #¹: die Verknüpfungsstelle mit dem Schwefelatom des Antikörpers kennzeichnet,
      - #²: die Verknüpfungsstelle mit der Gruppe L¹ kennzeichnet,
      und L1 durch die Formel

      #¹-(NR¹⁰)ₙ₋(G0)ₒ-G2-#²
   dargestellt wird,
   wobei
   R¹⁰ H, NH₂ oder C1-C3-Alkyl darstellt;
   G1 -NHCO- oder 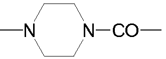 darstellt;
   n 0 oder 1 ist;
   o 0 oder 1 ist; und
   G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO₂, -NH-, -CO-, - NHCO-, -CONH-, -NMe-, -NHNH-, -SO2NHNH-, -CONHNH-, -CR^{x}=N-O- (wobei Rx H, C₁-C₃-Alkyl oder Phenyl darstellt) und einen 3 bis 10-gliedriger, aromatischen oder nicht aromatischen Heterozyklus mit bis zu 4 Heteroatomen ausgewählt aus N, O und S, -SO- oder -SO2- unterbrochen sein kann (vorzugsweise 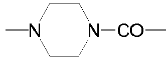 ), wobei Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein können,
   #¹ die Bindung zum KSP-Inhibitor und #² die Bindung zur Kopplungsgruppe zum Antikörper ist (z.B. L2),
   sowie Salze, Solvate und Salze der Solvate des ADCs.

### Ausführungsform E:

Ein ADC der Formel 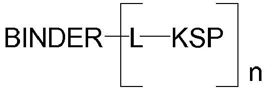 wobei KSP-L- eine Verbindung mit der folgenden Substruktur I(sub) ist, der Binder Nimutuzumab ist, und n eine Zahl von 1 bis 10 ist: 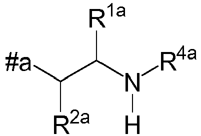 wobei
#a eine Bindung zum Restmolekül darstellt;
R^{1a} -L-#1, H oder -(CH₂)₀₋₃Z darstellt, wobei Z -H, Halogen, -NHY³, -CO-NY¹Y², oder -CO-OY³ darstellt,
mit Y¹ und Y² unabhängig voneinander H, NH2, oder -(CH₂CH₂O)₀₋₃-(CH₂)₀₋₃Z' darstellen, und Y³ H oder -(CH₂)₀₋₃Z' darstellt, wobei Z' H, SO₃H, NH₂, COOH oder -(CO-NH-CHY⁴)₁₋₃COOH darstellt; wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
R^{2a} und R^{4a} unabhängig voneinander H, -L-#1, -CO-CHY⁴-NHY⁵ oder -(CH₂)₀₋₃Z darstellen, oder R^{2a} und R^{4a} gemeinsam (unter Bildung eines Pyrrolidinringes) -CH₂-CHR¹⁰- oder -CHR¹⁰-CH₂- darstellen, wobei R¹⁰ -L-#1, H, NH₂, COOH, SO₃H, SH, oder OH darstellt, und wobei Z - H, -CO-NY¹Y², oder -CO-OY³ darstellt,
wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt,
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
wobei einer der Substituenten R^{1a}, R^{2a}, R^{4a} oder R¹⁰ -L-#1 darstellt,
-L-den Linker und #1 die Bindung zum Antikörper darstellt,
wobei -L- dargestellt wird durch

   §-(CO)m-L1-L2-§§

   wobei
   m 0 oder 1 ist;
   § die Bindung an KSP darstellt und
   § § die Bindung an den Antikörper darstellt, und
   L2 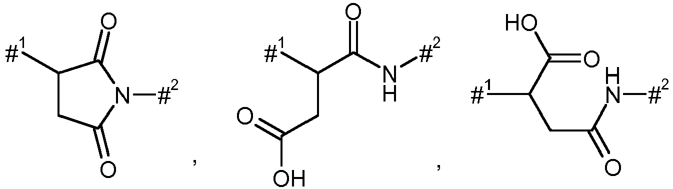 oder 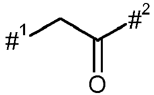
   darstellt, wobei
   #¹ die Verknüpfungsstelle mit dem Schwefelatom des Antikörpers kennzeichnet,
   #² die Verknüpfungsstelle mit der Gruppe L¹ kennzeichnet,
   und L1 durch die Formel

      #¹-(NR¹⁰)ₙ₋(G0)ₒ-G2-#²

      dargestellt wird,
      wobei
      R¹⁰ H, NH2 oder C1-C3-Alkyl darstellt;
      G1 -NHCO- oder 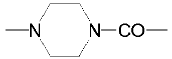 darstellt;
      n 0 oder 1 ist;
      o 0 oder 1 ist; und
      G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO₂, -NH-, -CO-, - NHCO-, -CONH-, -NMe-, -NHNH-, -SO₂NHNH-, -CONHNH- und einen 3 bis 10gliedriger, aromatischen oder nicht aromatischen Heterozyklus mit bis zu 4 Heteroatomen ausgewählt aus N, O und S, oder -SO- unterbrochen sein kann (vorzugsweise 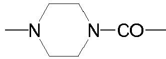 ), wobei die Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein können,
      #¹ die Bindung zum KSP-Inhibitor und #² die Bindung zur Kopplungsgruppe zum Antikörper ist (z.B. L2),
      sowie Salze, Solvate und Salze der Solvate des ADCs.

Besonders bevorzugt sind in dieser Ausführungsform, wenn KSP-L- die folgende Formel (IIi) aufweist:
Formel (IIi): 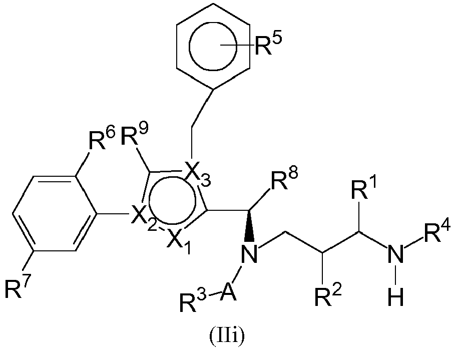 wobei
X₁ N, X₂ N und X₃ C darstellt; oder
X₁ CH oder CF, X₂ C und X₃ N darstellt; oder
X₁ NH, X₂ C und X₃ C darstellt; oder
X₁ CH, X₂ N und X₃ C darstellt;
(wobei X₁ CH, X₂ C und X₃ N bevorzugt ist);
R¹ H,-MOD oder -(CH₂)₀₋₃Z darstellt, wobei Z -H, -NHY³, -OY³, -SY³, Halogen, -CO-NY¹Y², oder -CO-OY³ darstellt, wobei Y¹ und Y² unabhängig voneinander H, NH₂, -(CH₂CH₂O)₀₋₃-(CH₂)₀₋₃Z'(z.B. -(CH₂)₀₋₃Z'), oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² H, -MOD, -CO-CHY⁴-NHY⁵ oder -(CH₂)₀₋₃Z darstellt, oder R² und R⁴ gemeinsam (unter Bildung eines Pyrrolidinringes) -CH₂-CHR¹⁰- oder -CHR¹⁰-CH₂- darstellen, wobei R¹⁰ H, NH₂, SO₃H, COOH, SH, oder OH darstellt;
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder -
(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
R⁴ H, -CO-CHY⁴-NHY⁵ oder -(CH₂)₀₋₃Z darstellt, vorzugsweise H,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder -
(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
oder R² und R⁴ gemeinsam (unter Bildung eines Pyrrolidinringes) -CH₂-CHR¹⁰- oder -CHR¹⁰-CH₂- darstellen, wobei R¹⁰ H, NH₂, SO₃H, COOH, SH, oder OH darstellt;
A CO, SO, SO₂, SO₂NH oder CNNH darstellt;
R³ -L-#1 darstellt,
R⁵ H, NH₂, NO₂, Halogen (insbesondere F, Cl, Br), -CN, CF₃, -OCF₃, -CH₂F, -CH₂F, SH oder - (CH₂)₀₋₃Z darstellt, wobei Z -H, -OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt; darstellt,
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, NO₂, NH₂, COOH oder Halogen (insbesondere F, Cl, Br) darstellen,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, (ggf. fluoriertes) C₄₋₁₀-Cycloalkyl oder-(CH₂)₀₋₂-(HZ²) darstellt, wobei HZ² ein 4 bis 7-gliedriger Heterocyclus mit bis zu zwei Heteroatomen ausgewählt aus N, O und S darstellt, wobei jede dieser Gruppen mit -OH, CO₂H oder NH₂ substituiert sein kann;
R⁹ H, F, CH₃, CF₃, CH₂F oder CHF₂ darstellt;
L für den Linker steht und #1 für die Bindung zum Binder bzw. Derivat hiervon steht,
wobei -MOD -(NR¹⁰)ₙ-(G1)ₒ-G2-H darstellt, wobei
   R¹⁰ H oder C₁-C₃-Alkyl darstellt;
   G1 -NHCO- , -CONH- oder 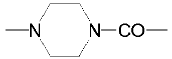 darstellt (wobei wenn G1 -NHCO- oder 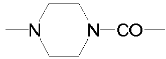 darstellt, R¹⁰ nicht NH₂ ist);
   n 0 oder 1 ist;
   o 0 oder 1 ist; und
   G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NRY-, -NRYCO-, CONRY-, -NRYNRY-, -SO2NR^{y}NR^{y}-, -CONRYNRY-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
   sowie ihre Salze, Solvate und Salze der Solvate.

### Ausführungsform F:

Verbindungen der allgemeinen Formel: 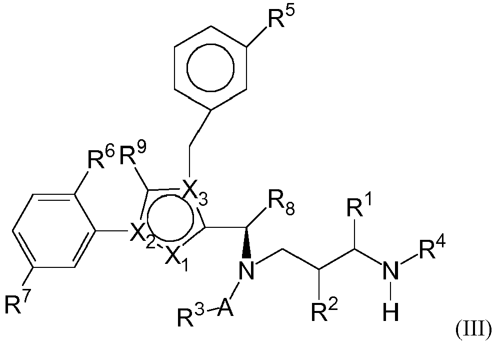 wobei
X₁ N, X₂ N und X₃ C darstellt, oder X₁ CH, X₂ C und X₃ N darstellt;
R¹ H, oder -(CH₂)₀₋₃Z darstellt, wobei Z -H, -NHY³, -OY³, -SY³, Halogen, -CO-NY¹Y², oder-CO-OY³ darstellt,
wobei Y¹ und Y² unabhängig voneinander H, NH2, -(CH₂CH₂O)₀₋₃-(CH₂)₀₋₃Z', oder-CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt, wobei W H oder OH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² und R⁴ unabhängig voneinander H, -CO-CHY⁴-NHY⁵ oder -(CH₂)₀₋₃Z darstellen, oder R² und R⁴ gemeinsam (unter Bildung eines Pyrrolidinringes) -CH₂-CHR¹⁰- oder -CHR¹⁰-CH₂- darstellen, wobei R¹⁰ H, NH₂, SO₃H, COOH, SH, oder OH darstellt;
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
A CO, SO, SO₂, SO₂NH oder CNNH darstellt;
R³ oder eine gegebenenfalls substituierte Alkyl-, Aryl-, Heteroaryl- Heteroalkyl-, Heterocycloalkyl- Gruppe darstellt, bevorzugt eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3-OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 - NH-CO-NH-Alkyl Gruppen, 1-3 -S(O)ₙ-Alkyl Gruppen, 1-3 -SO₂-NH-Alkyl Gruppen, 1-3 - NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet);
R⁵ H, F, NH₂, NO₂, Halogen, SH oder -(CH₂)₀₋₃Z darstellt, wobei Z -H, Halogen, -OY³, -SY³, - NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
darstellt,
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy oder Halogen darstellen,
R⁸ (ggf. fluoriertes) C₁₋₁₀-Alkyl, (ggf. fluoriertes) C₄₋₁₀-Cycloalkyl oder optional substituiertes Oxetan darstellt; und
R⁹ H, F, CH₃, CF₃, CH₂F oder CHF₂ darstellt;
sowie ihre Salze, Solvate und Salze der Solvate.

### Ausführungsform G:

Die Erfindung stellt auch Binder-Wirkstoff-der folgenden allgemeinen Formel bereit: 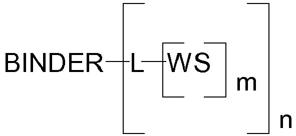 wobei BINDER für den Binder (bevorzugt: Antikörper) bzw. ein Derivat hiervon (bevorzugt: Cysteinrest),, vorzugsweise einen Antikörper, L für den Linker, WS für den Wirkstoff, vorzugsweise einen KSP-Inhibitor, wie z.B. ein erfindungsgemäßer KSP-Inhibitor einer der Formeln (II), (IIa), (IIb), (IIc), (IId), (IIe), (IIf), (IIg) oder (IIh) ist, m für eine Zahl von 1 bis 2, vorzugsweise 1, und n für eine Zahl von 1 bis 50, vorzugsweise 1,2 bis 20 und besonders bevorzugt 2 bis 8 steht, wobei L eine der folgenden Strukturen aufweist. Hierbei ist m die Zahl der Wirkstoffmoleküle pro Linker und n ein Mittelwert der Anzahl an Wirkstoff-Linker-Konjugaten pro BINDER. Die Summe aller WS, die in einem Konjugatmolekül vorliegen, ist also das Produkt von m und n.

WS ist ein Wirkstoff, der in Tieren, bevorzugt Menschen, eine lokale oder systemische Wirkung therapeutische Wirkung zeigt. Diese Wirkstoffe haben in der Regel ein Molekulargewicht von unter 5 kDa, bevorzugt unter 1.5 kDa. Bevorzugte Wirkstoffe sind antiproliferative Substanzen, z.B. zytotoxische oder zytostatische Substanzen. Bevorzugte Wirkstoffe sind zytotoxische Substanzen, Angiogenese Inhibitoren, Zellzyklusinhibitoren, PI3-Kinase oder m-TOR Inhibitoren, MAPK Signalkaskadeweg Inhibitoren, HDAC Inhibitoren, Proteasom-Inhibitoren, PARP Inhibitoren, Wnt/Hedgehog Signalkaskadeweg-Inhibitoren und RNA Polymeraseinhibitoren. Zytotoxische Substanzen sind unter anderem DNA-bindende oder interkalierende Substanzen, DNA alkylierende Substanzen, Mikrotubulin stabilisierende oder destabilisierende Substanzen, Platin-Verbindungen, und Topoisomerase I-Inhibitoren. Bespielhafte DNA-bindende sind z.B. Anthracycline, wie Doxorubicin oder Daunorubicin). Bespielhafte DNA-alkylierende Substanzen sind z.B. Calichemicine, Temozolomid oder Cyclophosphamid und Derivate. Bespielhafte Mikrotubulin stabilisierende oder destabilisierende Substanzen sind z.B. Taxane, wie z.B. Paclitaxel, Docetaxel, Maytansinoide, und Auristatine, Tubulysine, Vincaalkaloide, Epothilone und Derivate davon. Beispiele für Maytansinoide sind Maytansin, Maytansinol, DM-1 und DM-4 (siehe z.B. US Patent US5208020). Beispiele für Auristatine sind Auristatin E, Monomethylauristatin E (MMAE), Auristatin F, Monomethylauristatin F (MMAF) und Dolastin (siehe unter anderem WO 09/117531, WO 2005/081711, WO 04/010957; WO002/088172 oder WO01/24763). Beispiele für Vincaalkaloide sind Vincristin oder Vinblastin. Beispiele für Epothilone sind Epothilon A, B, C, D, E oder F (siehe unter anderem WO 98/13375; WO004/005269; WO 2008/138561; WO 2009/002993; WO 2009/055562; WO 2009/012958; WO02009/026177; WO 2009/134279; WO 2010/033733; WO2010/034724; WO 2011/017249; WO2011/057805). Beispiele für Platin Verbindungen sind Cis-Platin, oder Carboplatin. Beispiele für Topoisomerase I-Inhibitoren sind Camptothecin und Derivate. Beispiele für Angiogenese Inhibitoren sind MetAP2 Inhibitoren, wie z.B. Fumagillol. Beispiele für Zellzyklusinhibitoren sind CDK Inhibitoren (z.B. BMS-387032 oder PD0332991), Rho-Kinase Inhibitoren wie z.B. GSK429286, PLK Inhibitoren wie z.B. Volasertib, Aurora-Kinase Inhibitoren wie z.B. AZD1152 oder MLN805Z. Beispiele für MAPK Signalkaskadeweg Inhibitoren sind unter anderem MEK-Inhibitoren (z.B. PD0325901), Ras-Inhibitoren, JNK-Inhibitoren, B-Raf-Inhibitoren (z.B. SB590885) oder p38 MAPK-Inhibitoren(z.B. SB202190). Beispiele für HDAC-Inhibitoren sind Belinostat oder Givinostat. Beispiele für PARP-Inhibitoren sind Iniparib oder Olaparib. Beispiele für RNA Polymerase-Inhibitoren sind Amatoxine, wie z.B. alpha-Amantin, Amanin oder Amanullin. Besonders bevorzugte Wirkstoffe sind Vinca-Alkaloide, Auristatine, Maytansinoide, Tubulysine, Duocarmycine, Kinase-Inhibitoren, MEK-Inhibitoren und KSP-Inhibitoren.

Hierbei steht L für eine der folgenden Formeln A3 oder A4 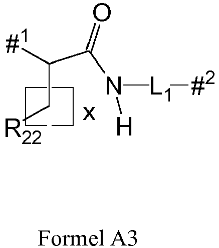 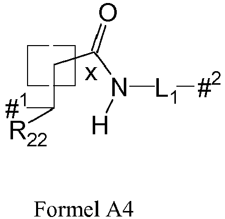 wobei #¹ die Verknüpfungsstelle mit dem Schwefelatom des Binders kennzeichnet, #² die Verknüpfungsstelle mit der dem Wirkstoff kennzeichnet, x 1 oder 2 darstellt, und R²² COOH, COOR, COR (mit R jeweils Cl-3-Alkyl), CONH2, Br, vorzugsweise COOH darstellt,.

L1 weist dieselbe Bedeutung wie oben auf. Vorzugsweise wird -L1-#2 durch die folgende Formel dargestellt:

#³-(NR¹⁰)ₙ-(G1)ₒ-G2-#²

dargestellt, wobei
#3 die Verknüpfungsstelle mit dem Stickstoffatom kennzeichnet
R¹⁰ H, NH₂ oder C₁-C₃-Alkyl darstellt;
G1 -NHCO- , -CONH- oder 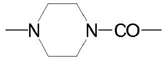 darstellt; (wobei wenn G1 NHCO oder 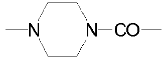 darstellt, R10 nicht NH2 ist),
n 0 oder 1 ist;
o 0 oder 1 ist; und
G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO₂, -NRY-, -NRYCO-,-C(NH)NR^{y}-, -CONR^{y}-, -NR^{y}NR^{y}-, -SO2NR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, - NH₂, NH-CNNH₂, Sulfonamid, Sulfon, Sulfoxid, oder Sulfonsäure substituiert sein können), -CO-, - CR^{x}=N-O- (wobei Rx H, C₁-C₃-Alkyl oder Phenyl darstellt) und/oder einen 3 bis 10-gliedriger, aromatischen oder nicht aromatischen Heterozyklus mit bis zu 4 Heteroatomen ausgewählt aus N, O und S, -SO- oder -SO2- unterbrochen sein kann (vorzugsweise 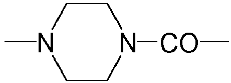 ), wobei die Kohlenwasserstoffkette einschließlich der Seitenketten, sofern vorhanden, mit -NHCONH₂, -COOH,-OH, -NH₂, NH-CNNH₂, Sulfonamid, Sulfon, Sulfoxid, oder Sulfonsäure substituiert sein können.

Weitere unterbrechende Gruppen in G2 sind vorzugsweise 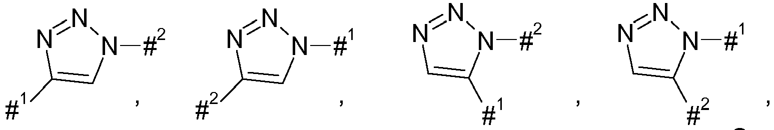 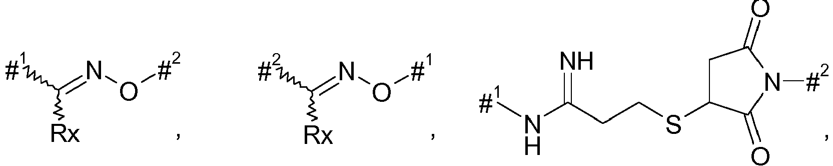 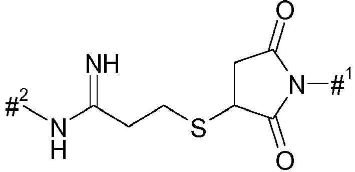 wobei Rx H, C₁-C₃-Alkyl oder Phenyl darstellt.

In dem erfindungsgemäßen Konjugat bzw. in einem Gemisch der erfindungsgemäßen Konjugate liegen die Bindungen an einen Cysteinrest des Binders vorzugsweise zu über 80 %, besonders bevorzugt zu über 90 %, besonders bevorzugt (bezogen auf die Gesamtanzahl von Bindungen des Linkers an den Binder) in einer der beiden Strukturen der Formel A3 oder A4 vor.

Die Konjugate mit den Linkern der Formel A3 bzw. A4 können durch Kupplung der Binder an die entsprechenden Bromderivate der folgenden Formeln A3' bzw. A4' erhalten werden:

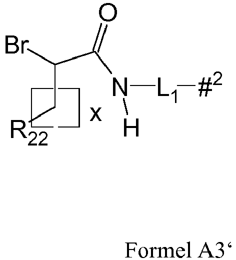

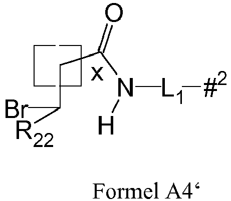

Diese Bromderivate der Formel A3' bzw. A4' können durch Reaktion von HOOCCH₂CHBrCOOR₂₂ bzw. HOOCCHBrCH₂COOR₂₂ mit einer Amingruppe des Binders erhalten werden, wie in den folgenden Schemata 30 bis 32 beispielhaft veranschaulicht. 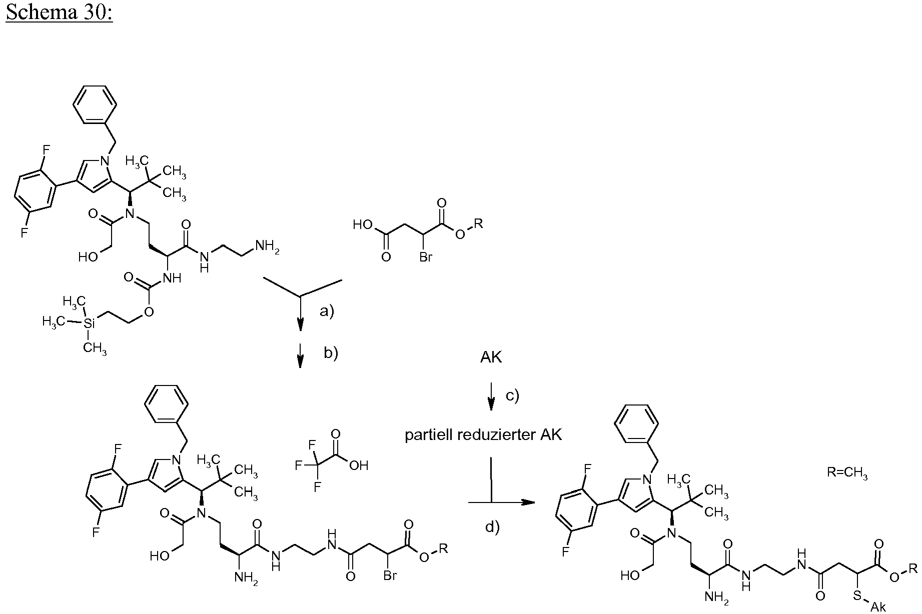 [a): 2-Brom-1-ethylpyridinium-Tetrafluoroborat (BEP), DCM, Pyridin, RT; b) Zinkchlorid, Trifluorethanol, 50°C, EDTA; c) 3-4 Äquivalente TCEP, PBS Puffer; d) PBS Puffer, 20h RT.] 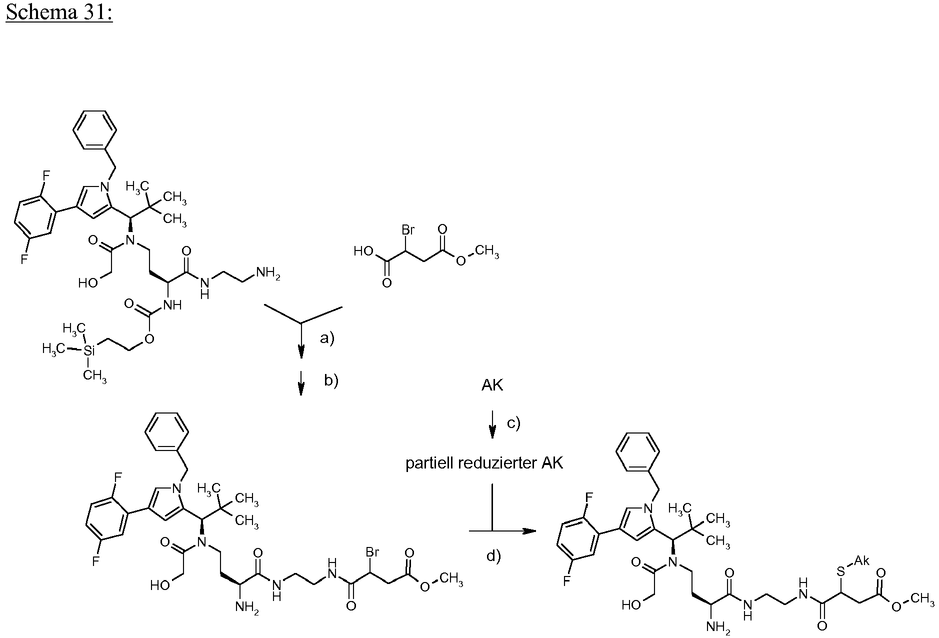 [a): 2-Brom-1-ethylpyridinium-Tetrafluoroborat (BEP), DCM, Pyridin, RT; b) Zinkchlorid, Trifluorethanol, 50°C, EDTA; c) 3-4 Äquivalente TCEP, PBS Puffer; d) PBS Puffer, 20h RT.]

### Ausführungsform H:

Die Erfindung stellt auch Binder-Wirkstoff der folgenden allgemeinen Formel bereit: 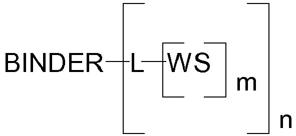 wobei BINDER für den Binder (bevorzugt: Antikörper) bzw. ein Derivat hiervon (bevorzugt: Cysteinrest), vorzugsweise einen Antikörper, L für den Linker, WS für den Wirkstoff, vorzugsweise einen KSP-Inhibitor, wie z.B. ein erfindungsgemäßer KSP-Inhibitor einer der Formeln (II), (IIa), (III) oder (IIIa), m für eine Zahl von 1 bis 2, vorzugsweise 1, und n für eine Zahl von 1 bis 50, vorzugsweise 1,2 bis 20 und besonders bevorzugt 2 bis 8 steht, wobei L eine der folgenden Strukturen aufweist. Hierbei ist m die Zahl der Wirkstoffmoleküle pro Linker und n ein Mittelwert der Anzahl an Wirkstoff-Linker-Konjugaten pro BINDER. Die Summe aller WS, die in einem Konjugatmolekül vorliegen, ist also das Produkt von m und n.

Hierbei steht L für: 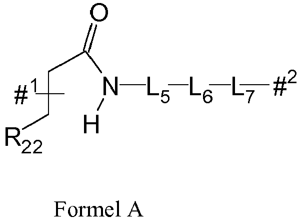 wobei #¹ die Verknüpfungsstelle mit dem Schwefelatom des Binders kennzeichnet, #² die Verknüpfungsstelle mit der dem Wirkstoff kennzeichnet, und R²² COOH, COOR, COR (mit R jeweils C1-3-Alkyl), CONH2, Br, vorzugsweise COOH darstellt. darstellt. Die Verknüpfung mit dem Schwefelatom des Binders kann somit eine der folgenden Strukturen aufweisen:

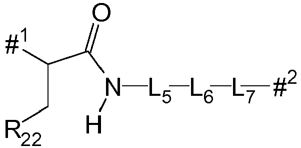

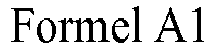

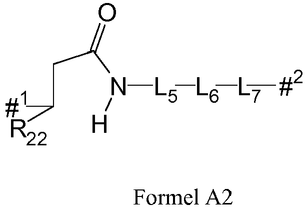

Bei Wirkstoff-Binder-Konjugaten, die mehr als ein Wirkstoffmolekül WS pro Wirkstoff-Binder-Konjugat enthalten, können beide Strukturen gemäß den Formeln A1 und/oder A2 in einem Wirkstoff-Binder-Konjugat vorliegen. Da es sich bei den erfindungsgemäßen Wirkstoff-Binder-Konjugaten um eine Mischung unterschiedlicher Wirkstoff-Binder-Konjugate handeln kann, ist es auch möglich, dass in dieser Mischung Wirkstoff-Binder-Konjugate sowohl mit der Formel A1 oder Formel A2 als auch mit der Formel A1 und A2 enthalten sind.

L₅ ist eine Gruppe ausgewählt aus -(CH₂)ₘ-(CHR^{S})ₙ-(OCH₂CH₂)ₒ-(X)ₚ-(CH₂)_{q-}, wobei m, n, o, p und q unabhängig voneinander die folgenden Werte aufweisen: m=0-10; n=0 oder 1; o=0-10; p=0 oder 1; und q=0-10, wobei m+n+o=1-15, vorzugsweise 1-6 ist. X stellt einen 5- oder 6-gliedrigen, aromatischen oder nicht-aromatischen Hetero- oder Homocyclus dar, vorzugsweise -C₆H₄- oder-C₆H₁₀-. R^{S} stellt eine Säuregruppe, vorzugsweise -COOH oder SO₃H dar.

L₆ ist eine Gruppe ausgewählt aus -CONH-, -OCONH-, -NHCO-, -NHCOO-, 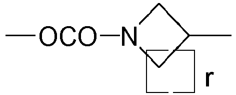 oder 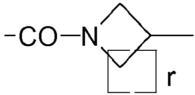 wobei r 1, 2 oder 3 ist.

L₇ ist eine Einfachbindung oder eine Gruppe ausgewählt aus einer geradkettige oder verzweigten Kohlenwasserstoffkette mit 1 bis 100 (vorzugsweise 1 bis 10) Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, -SO-, SO₂, -NRY-, -NR^{y}CO-, -C(NH)NR^{y}-,-CONR^{y}-, -NR^{y}NR^{y}-, -SONR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) und/oder einen 3 bis 10-gliedriger, vorzugsweise 5 bis 10-gliedriger, aromatischen oder nicht aromatischen Heterozyklus mit bis zu 4 Heteroatomen ausgewählt aus N, O und S, -SO- oder -SO2- unterbrochen sein kann (vorzugsweise 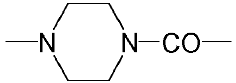 ), wobei die Kohlenwasserstoffkette einschließlich der Seitenketten, sofern vorhanden, mit -NHCONH₂,-COOH, -OH, -NH₂, NH-CNNH₂, Sulfonamid, Sulfon, Sulfoxid, oder Sulfonsäure substituiert sein können.

L₅ ist bevorzugt eine Gruppe -(CH₂)ₘ-(CHR^{S})ₙ-(OCH₂CH₂)ₒ-(X)ₚ-(CH₂)_{q}-, wobei m=1-3, n=0, o=0-7, p=0, und q=0 oder 1 darstellt. Besonders bevorzugt ist eine Gruppe -(CH₂)ₘ-(CHR^{S})ₙ-(OCH₂CH₂)ₒ-(X)ₚ-(CH₂)_{q}-, wobei m=1 oder 2, n=0, o=0 oder 1, p=0, und q=0 oder 1 darstellt.

L₆ ist bevorzugt eine Gruppe ausgewählt aus -CONH- und -NHCO-.

L₇ ist bevorzugt eine Einfachbindung oder -[(CH₂)ₓ-(X⁴)_{y}]_{W}-(CH₂)_{z}-,
wobei
w = 0 bis 20 ist;
x = 0 bis 5 ist;
y = 0 oder 1 ist;
z = 1 bis 5 ist; und
X⁴ -O-, -CONH-, -NHCO- oder 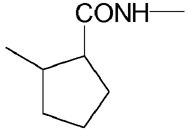 darstellt.

Besonders bevorzugt ist L₇ eine Einfachbindung oder eine Gruppe -[(CH₂)ₓ-NHCO-)], wobei x = 1 bis 5 ist.

Besonders bevorzugt stellt -L₅-L₆-L₇- -(CH₂)ₘ-(CHR^{S})ₙ-(OCH₂CH₂)ₒ-(X)ₚ-(CH₂)_{q}--NHCO-[(CH₂)ₓ-NHCO-)] dar, wobei m=1 oder 2, n=0, o=0 oder 1, p=0, und q=0 oder 1, und x=1-5 darstellt.

Es ist jedoch auch möglich, dass diese beiden Strukturen gemeinsam in dem erfindungsgemäßen Konjugat vorliegen.

Diese Binder-Wirkstoff-Konjugate können erfindungsgemäß aus den Verbindungen der Formel 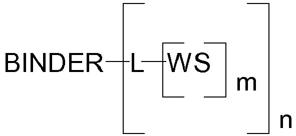 wobei L die folgende Formel A' aufweist, hergestellt werden:

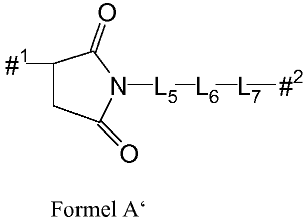

Vorzugsweise erfolgt die Umsetzung von A' zu A durch Rühren in einem pH-Puffer mit einem pH-Wert von 7.5 bis 8.5, vorzugsweise 8, bei einer Temperatur unterhalb 37°C, vorzugsweise 10 bis 25 °C, über einen Zeitraum von bis zu 40 Stunden, vorzugsweise 1 bis 15 Stunden.

### Ausführungsform I:

Ein Antikörper-Konjugat der Formel 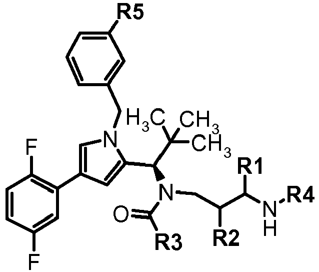 wobei
R2, R4 und R5H darstellen;
R3-CH2OH darstellt;
R1-L1-L2-BINDER darstellt, wobei
L1 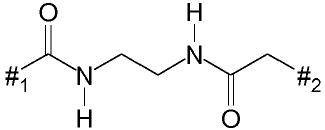 darstellt, wobei #2 die Verknüfung mit der L2 darstellt, und #1 die Verknüfung mit die andere Verknüpfung darstellt;
und L2 eine oder beide der folgenden Strukturen der Formeln A5 und A6 darstellt: 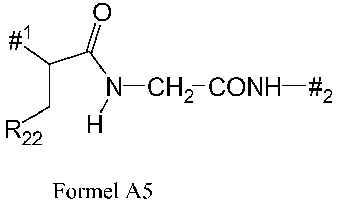 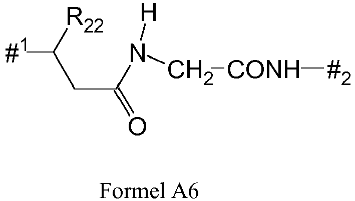 wobei
   - #¹: die Verknüpfungsstelle mit dem Schwefelatom des Binders kennzeichnet,
   - #²: die Verknüpfungsstelle mit der Gruppe L¹ kennzeichnet, und
   R²² COOH, COOR, COR, CONHR (mit R jeweils C1-3-Alkyl), CONH₂, vorzugsweise COOH darstellt.

In einem erfindungsgemäßen Konjugat bzw. in einem Gemisch der erfindungsgemäßen Konjugate liegen die Bindungen an einen Cysteinrest des Binders vorzugsweise zu über 80 %, besonders bevorzugt zu über 90 % (jeweils bezogen auf die Gesamtanzahl von Bindungen des Linkers an den Binder), besonders bevorzugt in einer der beiden Strukturen der Formel A5 oder A6 vor:

Hierbei liegen die Strukturen der Formel A5 oder A6 in der Regel gemeinsam vor, vorzugsweise in einem Verhältnis von 60:40 bis 40:60 bezogen auf die Anzahl der Bindungen an den Binder. Die restlichen Bindungen liegen dann in der Struktur 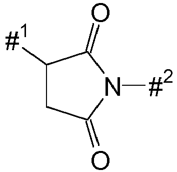 vor.

Der Binder ist vorzugsweise ein Binderprotein oder -peptid, besonders bevorzugt ein humaner, humanisierter oder chimärer monoklonaler Antikörper oder ein Antigen-bindendes Fragment hiervon ist, insbesondere ein anti-TWEAKR-Antikörper oder ein Antigen-bindendes Fragment hiervon oder ein anti-EGFR-Antikörper oder ein Antigen-bindendes Fragment hiervon. Besonders bevorzugt sind ein anti-TWEAKR-Antikörper, der spezifisch an die Aminosäure D in Position 47 (D47) von TWEAKR (SEQ ID NO:169) bindet, insbesondere der anti-TWEAKR-Antikörper TPP-2090, oder die anti-EGFR-Antikörper Cetuximab oder Nimotuzumab. Alternativ zum Binder kann auch ein Cysteinrest vorliegen.

### Therapeutische Verwendung

Zu den hyperproliferativen Erkrankungen, zu deren Behandlung die erfindungsgemäßen Verbindungen eingesetzt werden können, zählt insbesondere die Gruppe der Krebs- und Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (Mammakarzinome einschließlich ductaler und lobulärer Formen, auch *in situ*), Atemwegstumore (kleinzelliges und nicht-kleinzelliges Karzinom, Bronchialkarzinome), Hirntumore (z.B. des Hirnstamms und des Hypothalamus, Astrocytoma, Ependymoma, Glioblastoma, Gliome, Medulloblastoma, Meningiome sowie neuro-ektodermale und pineale Tumore), Tumore der Verdauungsorgane (Speiseröhren-, Magen-, Gallenblasen-, Dünndarm-, Dickdarm-, Rektum- und Analkarzinome), Lebertumore (u.a. hepatozelluläres Karzinom, Cholangiokarzinom und gemischt-hepatozelluläres Cholangiokarzinom), Tumore des Kopf- und Halsbereiches (Larynx-, Hypopharynx-, Nasopharynx-, Oropharynx-, Lippen- und Mundhöhlenkarzinome, orale Melanome), Hauttumore (Basaliome, Spinaliome, Plattenepithelkarzinome, Kaposi-Sarkom, maligne Melanome, nicht-melanomartiger Hautkrebs, Merkelzell-Hautkrebs, Mastzelltumore), Tumore des Stütz- und Bindegewebes (u.a. Weichteilsarkome, Osteosarkome, maligne fibröse Histiozytome, Chondrosarkome, Fibrosarkome, Hämangiosarkome, Leiomyosarkome, Liposarkome, Lymphosarkome und Rhabdomyosarkome), Tumore der Augen (u.a. intraokuläres Melanom und Retinoblastom), Tumore der endokrinen und exokrinen Drüsen (z.B. der thyroiden und parathyroiden Drüsen, Bauchspeicheldrüsen- und Speicheldrüsenkarzinome, Adenokarzinome), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Erkrankungen des Blutes, des Lymphsystems und des Rückenmarks, in solider Form und als zirkulierende Zellen, wie Leukämien, Lymphome und myeloproliferative Erkrankungen, z.B. akute myeloide, akute lymphoblastische, chronisch-lymphozytische, chronisch-myelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem. Diese gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Die Behandlung der zuvor genannten Krebserkrankungen mittels der erfindungsgemäßen Verbindungen umfasst sowohl eine Behandlung der soliden Tumore als auch eine Behandlung metastasierter oder zirkulierender Formen hiervon.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prävention von Erkrankungen, insbesondere der zuvor genannten Erkrankungen.

Weiterer Gegenstand der vorliegenden Erfindung sind Verbindungen zur Verwendung in einem Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten anti-hyperproliferativen, zytostatischen oder zytotoxischen Substanzen zur Behandlung von Krebserkrankungen kombiniert werden. Als geeignete Kombinationswirkstoffe seien beispielhaft genannt:
1311-chTNT, Abarelix, Abirateron, Aclarubicin, Afatinib, Aflibercept, Aldesleukin, Alemtuzumab, Alisertib, Alitretinoin, Alpharadin (Radium-223-chlorid), Altretamin, Aminoglutethimid, AMP-514, Amrubicin, Amsacrin, Anastrozol, Arglabin, Arsentrioxid, Asparaginase, AT9283, Axitinib, Azacitidin, Basiliximab, Belotecan, Bendamustin, Bevacizumab, Bexaroten, Bicalutamid, Bisantren, Bleomycin, BMS-936559, Bosutinib, Bortezomib, Brentuximab vedotin, Buserelin, Busulfan, Cabazitaxel, Cabozantinib, Calciumfolinat, Calciumlevofolinat, Capecitabin, Carboplatin, Carfilzomib (proteasome inhibitor), Carmofur, Carmustin, Catumaxomab, Celecoxib, Celmoleukin, Cetuximab, Chlorambucil, Chlormadinon, Chlormethin, Cisplatin, Cladribin, Clodronsäure, Clofarabin, Copanlisib , Crisantaspase, Crizotinib, Cyclophosphamid, CYC116, Cyproteron, Cytarabin, Dacarbazin, Dactinomycin, Darbepoetin-alfa, Dabrafenib, Danusertib, Dasatinib, Daunorubicin, Decitabin, Degarelix, Denileukin-Diftitox, Denosumab, Deslorelin, Dibrospidiumchlorid, Docetaxel, Doxifluridin, Doxorubicin, Doxorubicin + Estron, Eculizumab, Edrecolomab, Elliptiniumacetat, Eltrombopag, Endostatin, ENMD-2076, Enocitabin, Epirubicin, Epitiostanol, Epoetin-alfa, Epoetin-beta, Eptaplatin, Eribulin, Erlotinib, Estradiol, Estramustin, Etoposid, Everolimus, Exemestan, Fadrozol, Filgrastim, Fludarabin, Fluoruracil, Flutamid, Formestan, Fotemustin, Fulvestrant, Galliumnitrat, Ganirelix, Gefitinib, Gemcitabin, Gemtuzumab, Glutoxim, Goserelin, Histamindihydrochlorid, Histrelin, Hydroxycarbamid, I-125-Seeds, Ibandronsäure, Ibritumomab-Tiuxetan, Ibrutinib, Idarubicin, Ifosfamid, Imatinib, Imiquimod, INCB24360, Improsulfan, Interferon-alfa, Interferon-beta, Interferon-gamma, Ipilimumab, Irinotecan, Ixabepilon, Lambrolizumab, Lanreotid, Lapatinib, Lenalidomid, Lenograstim, Lentinan, Letrozol, Leuprorelin, Levamisol, Lisurid, Lobaplatin, Lomustin, Lonidamin, Masoprocol, Medroxyprogesteron, Megestrol, Melphalan, Mepitiostan, Mercaptopurin, Methotrexat, Methoxsalen, Methylaminolevulinat, Methyltestosteron, Mifamurtid, Miltefosin, Miriplatin, Mitobronitol, Mitoguazon, Mitolactol, Mitomycin, Mitotan, Mitoxantron, MLN-8054, Mpsl-Inhibitoren (offenbart in WO2013/087579, insbesondere Beispiel 01.01, WO02014/131739, insbesondere Beispiel 2), Nedaplatin, Nelarabin, Nemorubicin, Nilotinib, Nilutamid, Nimotuzumab, Nimustin, Nitracrin, Nivolumab, NMS-P715, NMS-P937, Ofatumumab, Omeprazol, Oprelvekin, Oxaliplatin, p53-Gentherapie, Paclitaxel, Palbociclib, Palifermin, Palladium-103-Seed, Pamidronsäure, Panitumumab, Pazopanib, Pegaspargase, PEG-epoetin-beta (Methoxy-PEG-epoetin-beta), Pegfilgrastim, Peg-interferon-alfa-2b, Pemetrexed, Pentazocin, Pentostatin, Peplomycin, Perfosfamid, Picibanil, Pirarubicin, Plerixafor, Plicamycin, Poliglusam, Polyestradiolphosphat, Polysaccharid-K, Ponatinib, Porfimer-Natrium, Pralatrexat, Prednimustin, Procarbazin, Quinagolid, , R763, Raloxifen, Raltitrexed, Ranimustin, Razoxan, Refametinib, Regorafenib, Risedronsäure, Rituximab, Romidepsin, Romiplostim, Roninciclib, Ruxolitinib, Sargramostim, Sipuleucel-T, Sizofiran, Sobuzoxan, Natriumglycididazol, SNS-314, Sorafenib, Streptozocin, Sunitinib, Talaporfin, Tamibaroten, Tamoxifen, Tasonermin, Teceleukin, Tegafur, Tegafur + Gimeracil + Oteracil, Temoporfin, Temozolomid, Temsirolimus, Teniposid, Testosteron, Tetrofosmin, Thalidomid, Thiotepa, Thymalfasin, TKM-PLK1, Tioguanin, Tocilizumab, Topotecan, Toremifen, Tositumomab, Tozasertib, Trabectedin, Trametinib, Trastuzumab, Trastuzumab emtansine, Treosulfan, Tretinoin, Trilostan, Triptorelin, Trofosfamid, Tryptophan, Ubenimex, Valrubicin, Vandetanib, Vapreotid, Vemurafenib, Vinblastin, Vincristin, Vindesin, Vinflunin, Vinorelbin, Volasertib, Vorinostat, Vorozol, XL228, Yttrium-90-Glasmikrokugeln, Zinostatin, Zinostatin-Stimalamer, Zoledronsäure, Zorubicin.

Darüber hinaus können die Verbindungen der vorliegenden Erfindung beispielsweise mit Bindern kombiniert werden, die beispielhaft an die folgenden Targets binden können: OX-40, CD137/4-1BB, DR3, IDO1/IDO2, LAG-3, CD40.

Darüber hinaus können die erfindungsgemäßen Verbindungen auch in Kombination mit einer Strahlentherapie und/oder einer chirurgischen Intervention eingesetzt werden.

Generell können mit der Kombination von Verbindungen der vorliegenden Erfindung mit anderen, zytostatisch oder zytotoxisch wirksamen Agenzien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Kombination mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie beispielsweise, parenteral, möglicherweise inhalativ oder als Implantat bzw. Stent.

Für diese Applikationswege können die erfindungsgemäßen Verbindungen in geeigneten Applikationsformen verabreicht werden.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B.. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen oder Lyophilisaten. Bevorzugt ist die parenterale Applikation, insbesondere die intravenöse Applikation.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Beispiele

Die nachfolgenden Beispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Synthesewege:

Exemplarisch für die Ausführungsbeispiele sind in folgenden Schemata beispielhafte Synthesewege zu den Ausführungsbeispielen dargestellt: 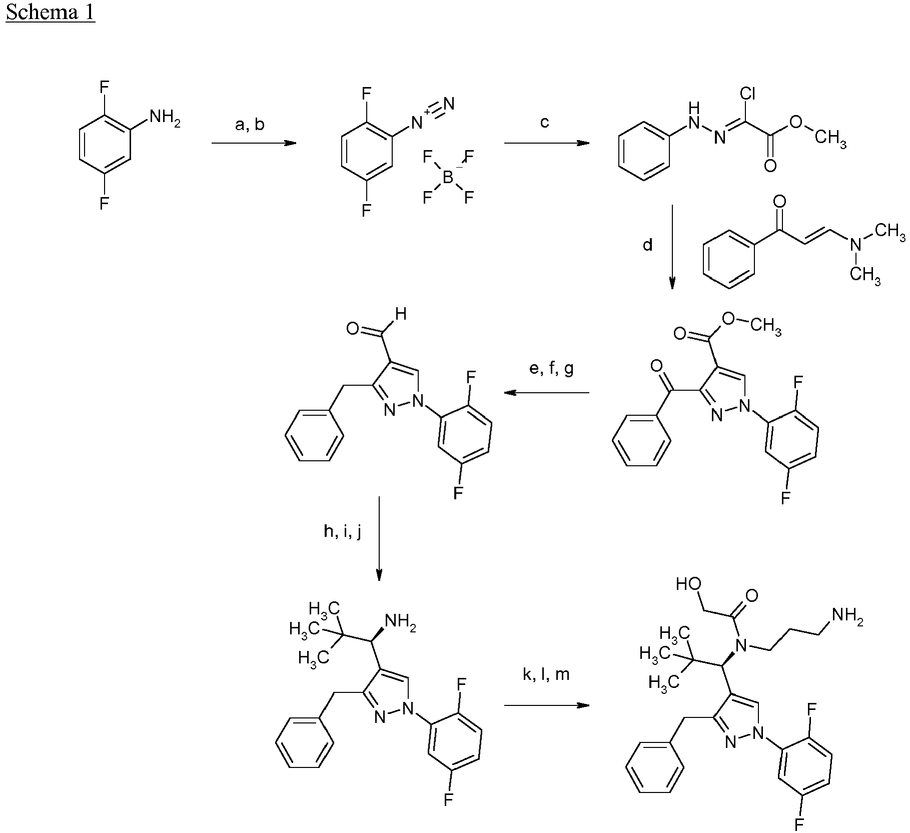 [a): beispielsweise BF₃OEt₂, THF, 0°C; b): beispielsweise Isoamylnitrit, -10°C, 0.5 h; c): beispielsweise Methyl-2-chlor-3-oxobutanoat, Pyridin, Wasser, -5°C; d): beispielsweise NEt₃, Toluol, RT; e): beispielsweise Et₃SiH, TFA, RT; f): beispielsweise LiBH₄, THF, 60°C; g): beispielsweise Dess-Martin-Periodinan, DCM, RT; h): beispielsweise (R)-(+)-Methyl-2-propansulfinsäureamid, Titan(IV)isopropylat, THF, RT; i): beispielsweise tert-BuLi, Pentan, THF, -78°C; j): beispielsweise HCl in Dioxan, THF, MeOH, RT; k): beispielsweise 3-(1,3-Dioxo-1,3-dihydro-2H-isoindol-2-yl)propanal, NaB(OAc)₃H, AcOH, DCM, RT; 1): beispielsweise 2-Chlor-2-oxoethylacetat, NEt₃, DCM, RT; m): beispielsweise Methylamin, Wasser, EtOH, 50°C] 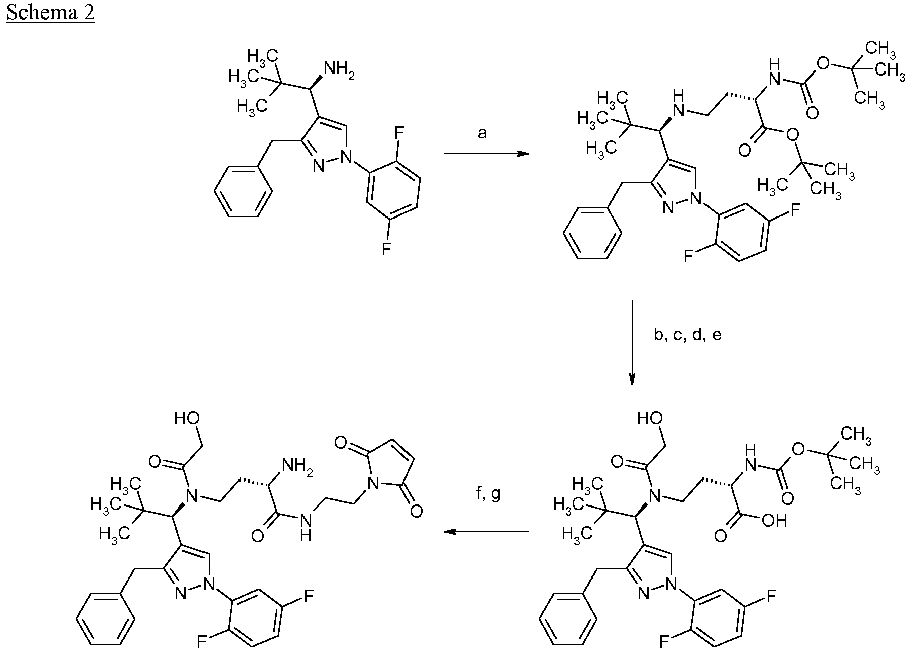 [a): beispielsweise tert-Butyl-N-(tert-butoxycarbonyl)-5-oxo-L-norvalinat, NaB(OAc)₃H, AcOH, DCM, RT; b): beispielsweise 2-Chlor-2-oxoethylacetat, NEt₃, DCM, RT; c): beispielsweise Methylamin, Wasser, EtOH, 60°C; d): beispielsweise THF, DCM, 50°C; e): beispielsweise Boc₂O, NEt₃, DCM, RT; f): beispielsweise Trifluoressigsäure-1-(2-aminoethyl)-1H-pyrrol-2,5-dion(1:1), HATU, Diisopropylethylamin, DMF, RT; f): beispielsweise TFA, DCM, RT] 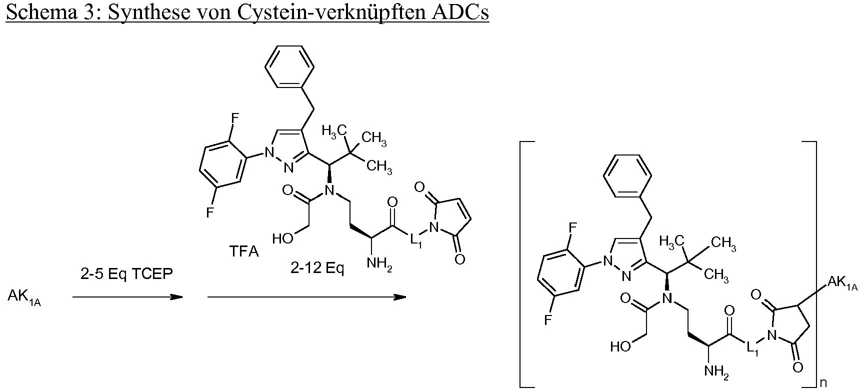 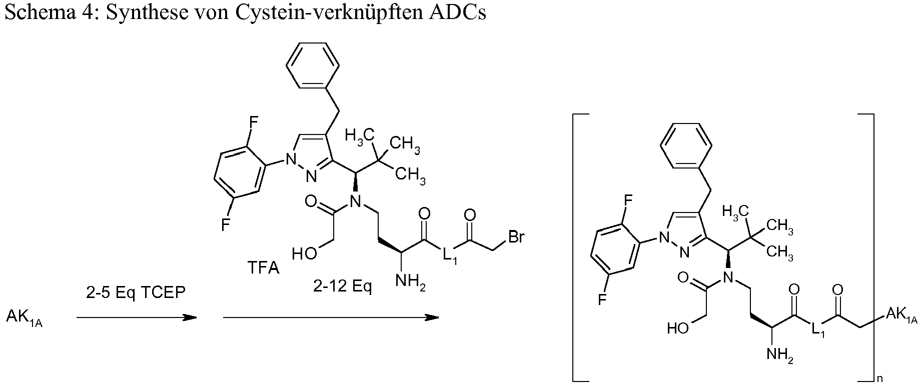 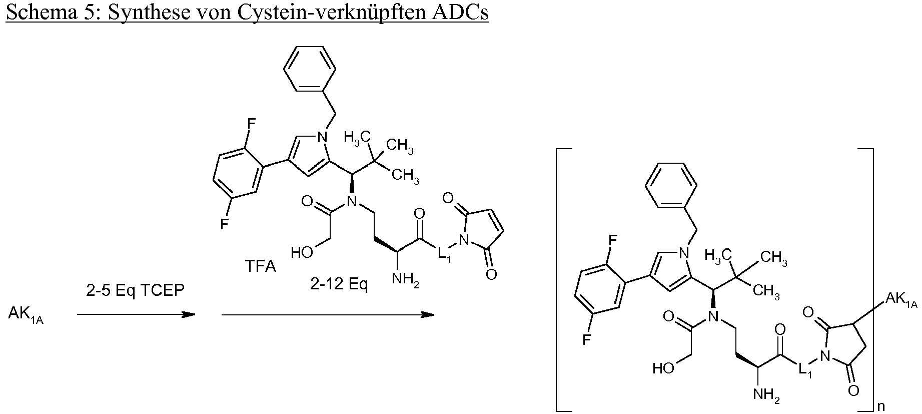 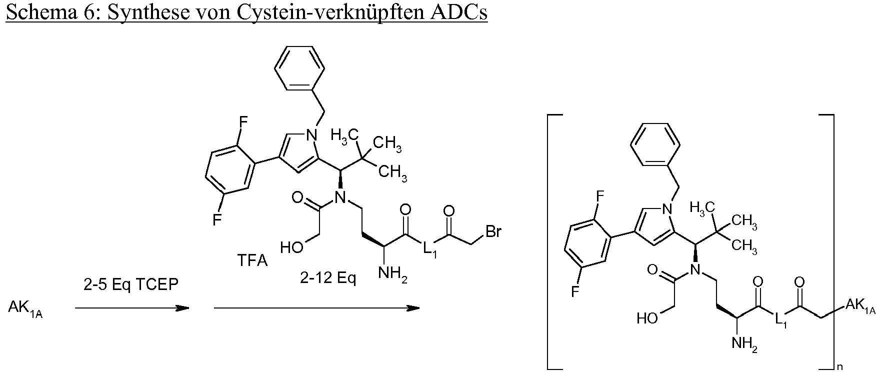 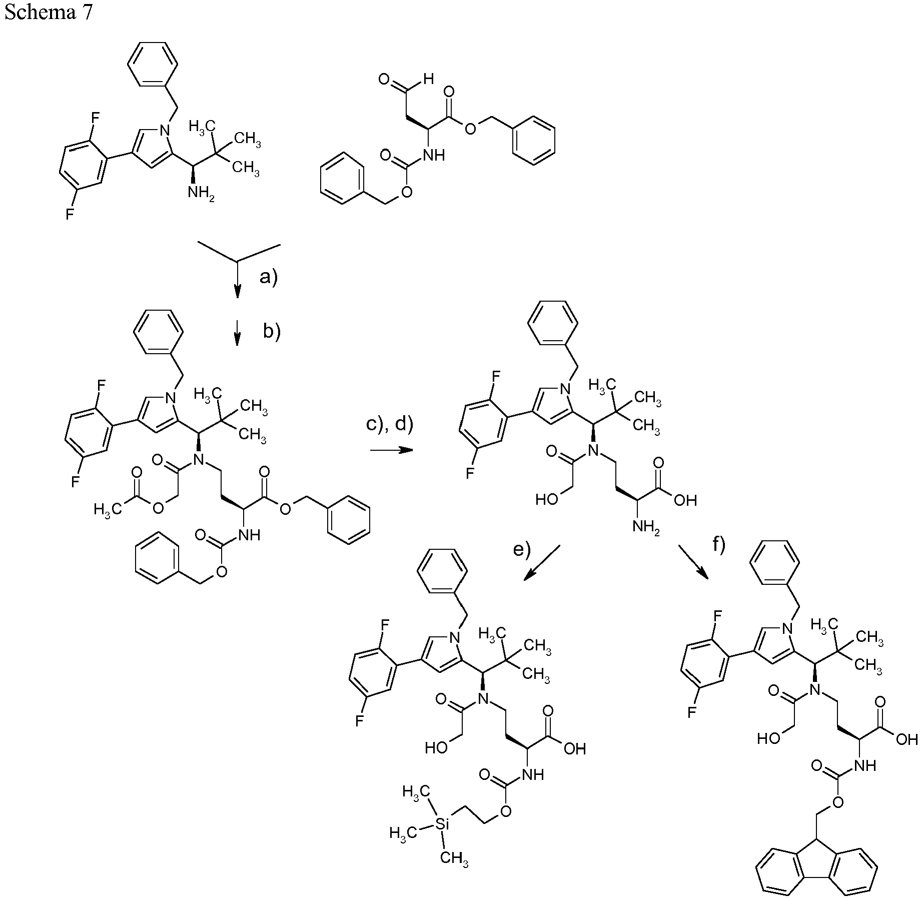 [a): beispielsweise Natriumtriacetoxyborhydrid, Essigsäure, DCM, RT; b) beispielsweise Acetoxyacetylchlorid, NEt3, DCM, RT; c) beispielsweise LiOH, THF/Wasser, RT; d) beispielsweise H₂, Pd-C, EtOH, RT; e) beispielsweise Teoc-OSu, NEt3, Dioxan, RT; f) beispielsweise Fmoc-Cl, Diisopropylethylamin, Dioxan/Wasser 2:1, RT] 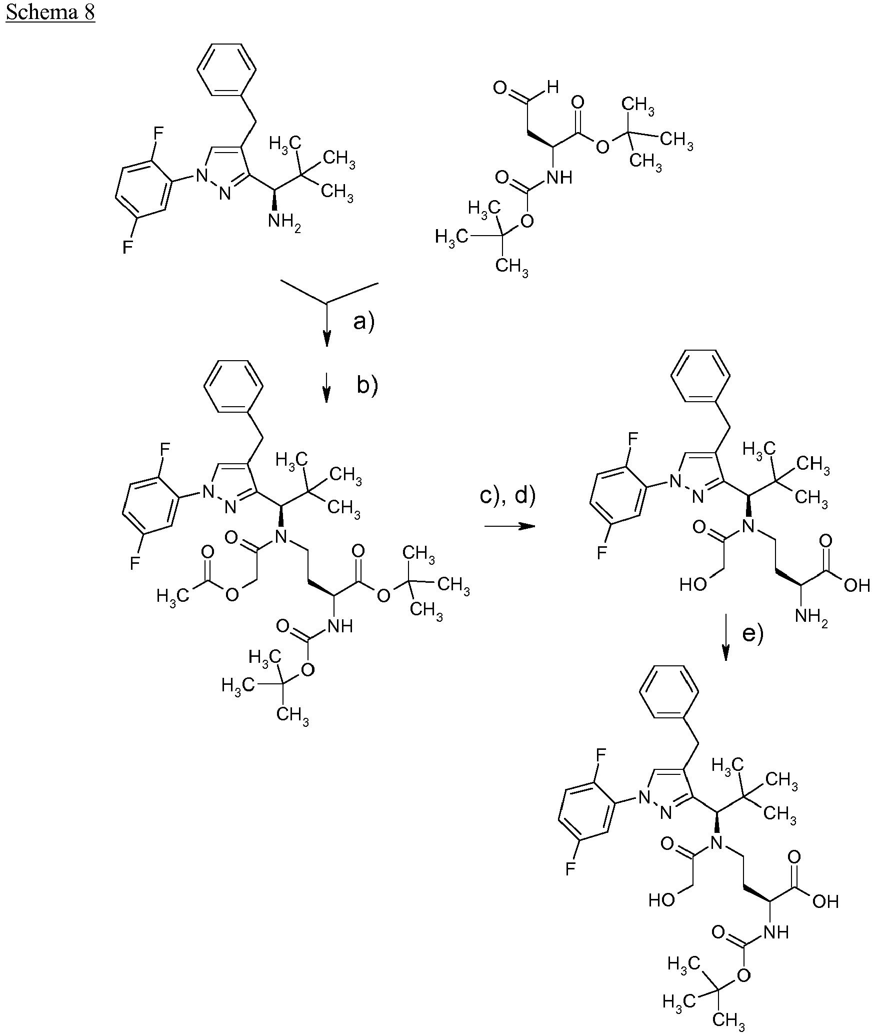 [a): beispielsweise Natriumtriacetoxyborhydrid, Essigsäure, DCM, RT; b) beispielsweise Acetoxyacetylchlorid, NEt3, DCM, RT;c) beispielsweise LiOH, Methanol, RT; d) beispielsweise TFA, DCM, RT; e) beispielsweise Boc₂O, Diisopropylethylamin, DCM, RT] 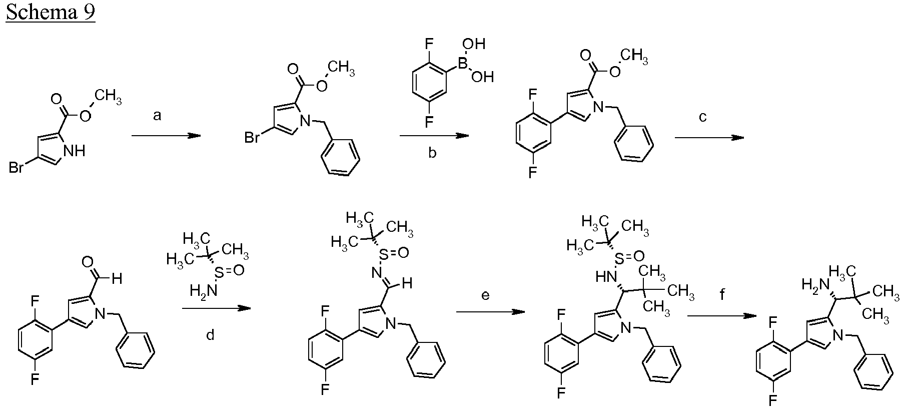 [a): beispielsweise Benzylbromid, Cs₂CO₃, DMF, RT; b) beispielsweise Pd(dppf)₂Cl₂, DMF, Na₂CO₃, 85 °C; c) beispielsweise LiAlH₄, THF, 0 °C; MnO₂, DCM, RT; d) beispielsweise Ti(iOPr)₄, THF, RT; e) beispielsweise tBuLi, THF, -78 °C; MeOH, NH₄Cl; f) beispielsweise HCl/1,4-Dioxan] 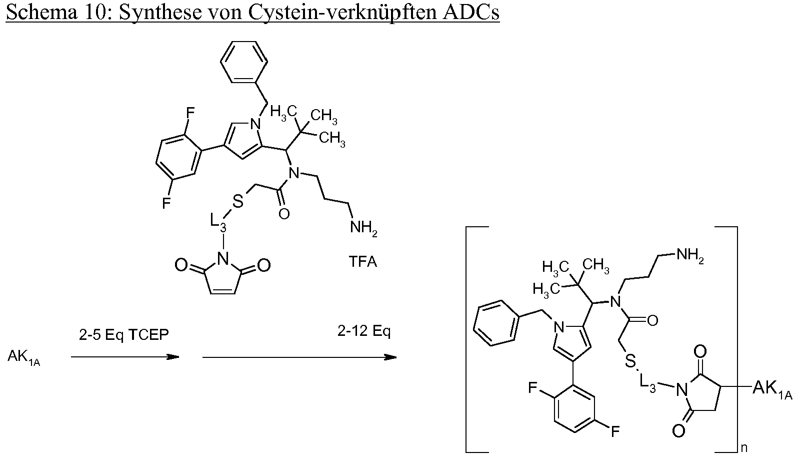 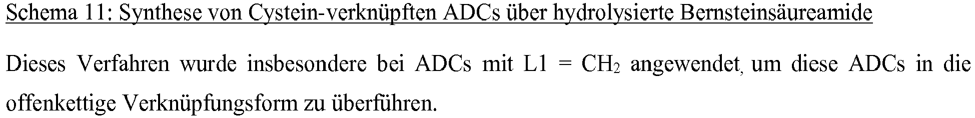 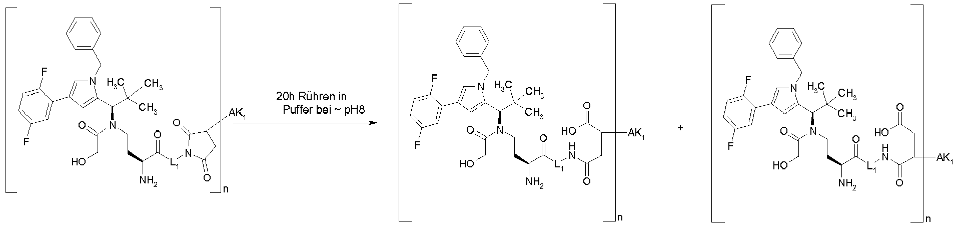 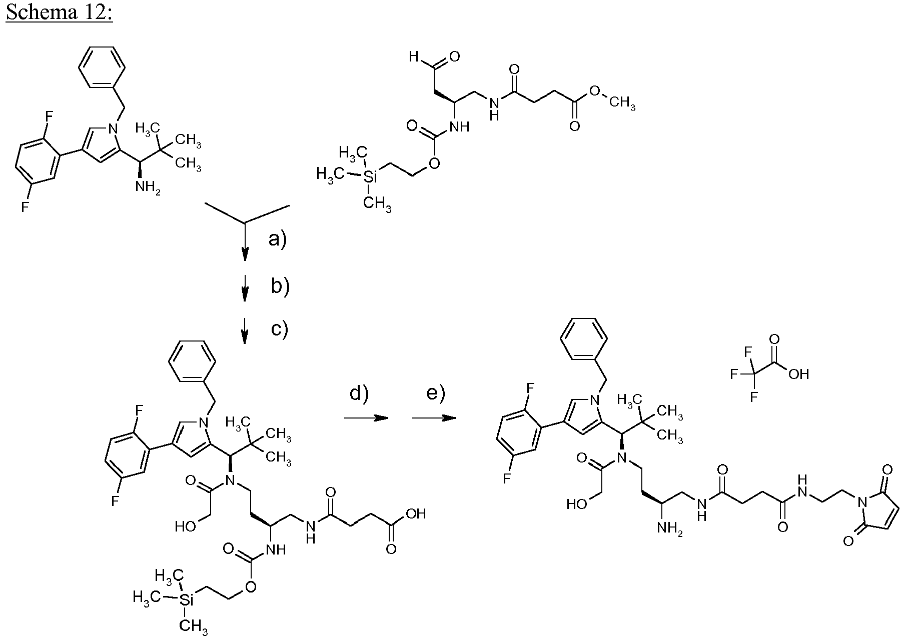 [a): Natriumtriacetoxyborhydrid, Essigsäure, DCM, RT; b) Acetoxyacetylchlorid, Diisopropylethylamin, DCM, RT; c) LiOH, MeOH, RT; d) Trifluoressigsäure--1-(2-aminoethyl)-1H-pyrrol-2,5-dion (1:1) HATU, DMF, Diisopropylethylamin, RT; e) Zinkchlorid, Trifluorethanol, 50°C, EDTA.] 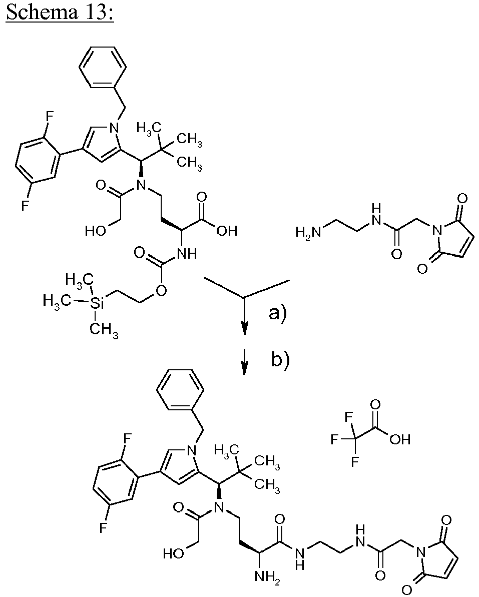 [a): HATU, DMF, Diisopropylethylamin, RT; b) Zinkchlorid, Trifluorethanol, 50°C, EDTA.] 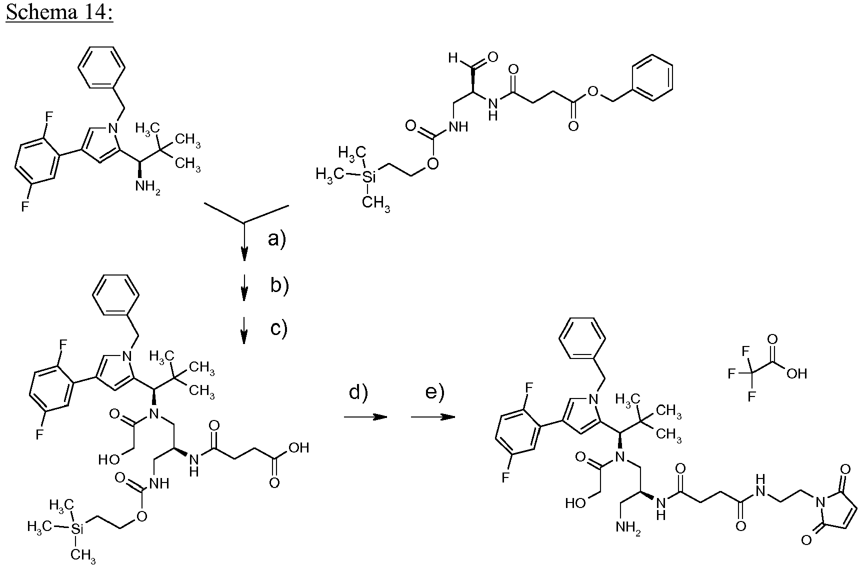 [a): Natriumtriacetoxyborhydrid, Essigsäure, DCM, RT; b) Acetoxyacetylchlorid, Triethylamin, DCM, RT; c) LiOH, MeOH, RT; d) Trifluoressigsäure--1-(2-aminoethyl)-1H-pyrrol-2,5-dion (1:1) HATU, DMF, Diisopropylethylamin, RT; e) Zinkchlorid, Trifluorethanol, 50°C, EDTA.] 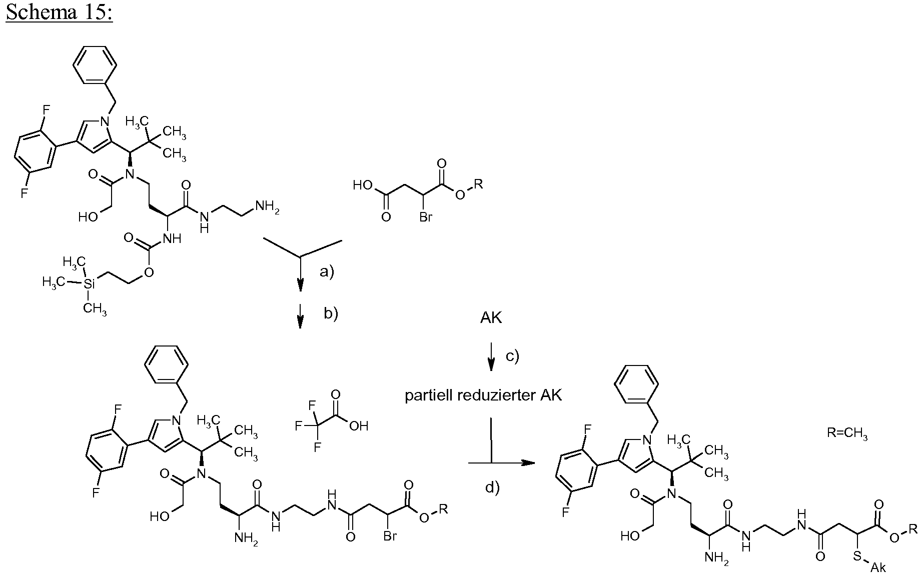 [a): 2-Brom-1-ethylpyridinium-Tetrafluoroborat (BEP), DCM, Pyridin, RT; b) Zinkchlorid, Trifluorethanol, 50°C, EDTA; c) 3-4 Äquivalente TCEP, PBS Puffer; d) PBS Puffer, 20h RT.] 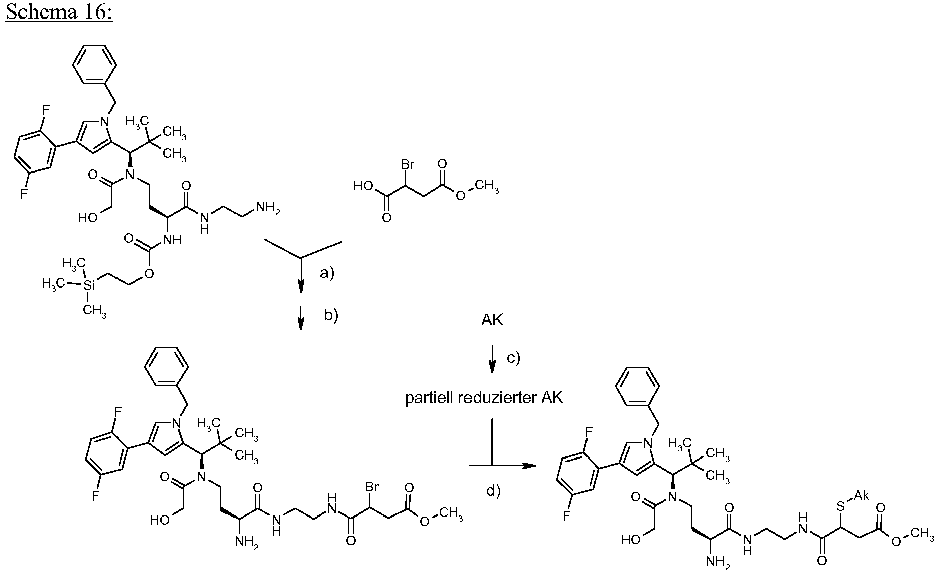 [a): 2-Brom-1-ethylpyridinium-Tetrafluoroborat (BEP), DCM, Pyridin, RT; b) Zinkchlorid, Trifluorethanol, 50°C, EDTA; c) 3-4 Äquivalente TCEP, PBS Puffer; d) PBS Puffer, 20h RT.]

### A. Beispiele

### Abkürzungen und Akronyme:

- A431NS: humane Tumorzelllinie
- A549: humane Tumorzelllinie
- ABCB1: ATP-binding cassette sub-family B member 1 (Synonym für P-gp und MDR1)
- abs.: Absolut
- Ac: Acetyl
- ACN: Acetonitril
- aq.: wässrig, wässrige Lösung
- ATP: Adenosintriphosphat
- BCRP: Brustkrebs-Resistenz-Protein, ein Efflux-Transporter
- BEP: 2-Brom-1-ethylpyridinium-Tetrafluoroborat
- Boc: tert.-Butoxycarbonyl
- br.: breit (bei NMR)
- Bsp.: Beispiel
- *ca.*: *circa,* ungefähr
- CI: chemische Ionisation (bei MS)
- d: Dublett (bei NMR)
- d: Tag(e)
- DC: Dünnschichtchromatographie
- DCI: direkte chemische Ionisation (bei MS)
- dd: Dublett von Dublett (bei NMR)
- DMAP: 4-*N,N*-Dimethylaminopyridin
- DME: 1,2-Dimethoxyethan
- DMEM: Dulbecco's Modified Eagle Medium (standardisiertes Nährmedium für die Zellkultur)
- DMF: *N,N*-Dimethylformamid
- DMSO: Dimethylsulfoxid
- DPBS,: D-PBS, PBS Dulbecco's Phosphat-gepufferte Salz-Lösung
PBS = DPBS = D-PBS, pH7,4, Fa. Sigma, No D8537
Zusammensetzung:
0,2 g KCl
0,2 g KH₂PO₄ (anhyd)
8,0 g NaCl
1,15 g Na₂HPO₄ (anhyd)
ad 11 mit H₂O auffüllen
- dt: Dublett von Triplett (bei NMR)
- DTT: DL-Dithiothreitol
- d.: Th. der Theorie (bei chemischer Ausbeute)
- EDC: *N*'-(3-Dimethylaminopropyl)-*N*-ethylcarbodiimid-Hydrochlorid
- EGFR: Epidermal growth factor receptor = Epidermaler Wachstumsfaktor Rezeptor
- EI: Elektronenstoß-Ionisation (bei MS)
- ELISA: Enzyme-linked Immunosorbent Assay
- eq.: Äquivalent(e)
- ESI: Elektrospray-Ionisation (bei MS)
- ESI-MicroTofq: ESI- MicroTofq (Name des Massenspektrometer mit Tof = Time Of Flight und q = Quadrupol)
- FCS: fötales Kälberserum
- Fmoc: (9*H*-Fluoren-9-ylmethoxy)carbonyl
- ges.: gesättigt
- GTP: Guanosin-5'-triphosphat
- h: Stunde(n)
- HATU: *O*-(7-Azabenzotriazol-1-yl)-*N,N,N,N'*-tetramethyluronium-hexafluorophosphat
- HCT-116: HumaneTumorzelllinie
- HEPES: 4-(2-Hydroxyethyl)piperazin-1-ethansulfonsäure
- HOAc: Essigsäure
- HOAt: 1-Hydroxy-7-azabenzotriazol
- HOBt: 1-Hydroxy-1*H*-benzotriazol-Hydrat
- HOSu: *N*-Hydroxysuccinimid
- HPLC: Hochdruck-, Hochleistungsflüssigchromatographie
- HT29: humane Tumorzelllinie
- IC₅₀: halbmaximale Inhibitionskonzentration
- i.m.: intramuskulär, Applikation in den Muskel
- i.v.: intravenös, Applikation in die Vene
- konz.: konzentriert
- LC-MS: Flüssigchromatographie-gekoppelte Massenspektrometrie
- LLC-PK1: -Zellen Lewis lung carcinoma pork kidney cell line
- L-MDR: Human MDR1 transfizierte LLC-PK1 Zellen
- m: Multiplett (bei NMR)
- MDR1: Multidrug resistence protein 1
- MeCN: Acetonitril
- min: Minute(n)
- MS: Massenspektrometrie
- MTT: 3-(4,5-Dimethylthiazol-2-yl)-2,5-diphenyl-2*H*-tetrazoliumbromid
- NCI-H292: humane Tumorzelllinie
- NCI-H520: humane Tumorzelllinie
- NMM: *N*-Methylmorpholin
- NMP: *N*-Methyl-2-pyrrolidinon
- NMR: Kernresonanzspektrometrie
- NMRI: Mausstamm, Herkunft aus dem Naval Medical Research Institute (NMRI)
- Nude: Mäuse Nacktmäuse (Versuchstiere)
- NSCLC: Non small cell lung cancer (Nicht-kleinzelliges Bronchialkarzinom)
- PBS: Phosphat-gepufferte Salz-Lösung
- Pd/C: Palladium auf Aktivkohle
- P-gp: P-Glycoprotein, ein Transporterprotein
- PNGaseF: Enzym zur Zuckerabspaltung
- quant.: quantitativ (bei Ausbeute)
- quart: Quartett (bei NMR)
- quint: Quintett (bei NMR)
- R_{f}: Retentionsindex (bei DC)
- RT: Raumtemperatur
- Rₜ: Retentionszeit (bei HPLC)
- s: Singulett (bei NMR)
- s.c.: subcutan, Applikation unter die Haut
- SCC-4: Humane Tumorzelllinie
- SCC-9: Humane Tumorzelllinie
- SCID: Mäuse Versuchsmäuse mit einem schweren kombinierten Immundefekt (severe combined immunodeficiency)
- t: Triplett (bei NMR)
- TBAF: Tetra-n-butylammoniumfluorid
- TEMPO: (2,2,6,6-Tetramethyl-piperidin-1-yl)oxyl
- *tert.*: tertiär
- TFA: Trifluoressigsäure
- THF: Tetrahydrofuran
- T3P®: 2,4,6-Tripropyl-1,3,5,2,4,6-trioxatriphosphinan-2,4,6-trioxid
- UV: Ultraviolett-Spektrometrie
- v/v: Volumen zu Volumen-Verhältnis (einer Lösung)
- Z: Benzyloxycarbonyl

Wenn bei einer Reaktionsbeschreibung im Rahmen dieser Offenbarung keine Temperatur angegeben ist, ist immer von Raumtemperatur auszugehen.

### HPLC- und LC-MS-Methoden:

### Methode 1 (LC-MS):

Instrument: Waters ACQUITY SQD UPLC System; Säule: Waters Acquity UPLC HSS T3 1.8 µ 50 x 1 mm; Eluent A: 1 1 Wasser + 0.25 mL 99%ige Ameisensäure , Eluent B: 1 1 Acetonitril + 0.25 mL 99%ige Ameisensäure; Gradient: 0.0 min 90% A → 1.2 min 5% A → 2.0 min 5% A Ofen: 50°C; Fluss: 0.40 mL/min; UV-Detektion: 208 - 400 nm.

### Methode 2 (LC-MS):

Gerätetyp MS: Waters Synapt G2S; Gerätetyp UPLC: Waters Acquity I-CLASS; Säule: Waters, BEH300, 2.1 x 150 mm, C18 1.7 µm; Eluent A: 1 1 Wasser + 0.01% Ameisensäure; Eluent B: 1 1 Acetonitril + 0.01% Ameisensäure; Gradient: 0.0 min 2% B → 1.5 min 2% B → 8.5 min 95% B → 10.0 min 95% B; Ofen: 50°C; Fluss: 0.50 mL/min; UV-Detektion: 220 nm

### Methode 3 (LC-MS):

Instrument MS: Waters (Micromass) QM; Instrument HPLC: Agilent 1100 Serie; Säule : Agient ZORBAX Extend-C18 3.0x50mm 3.5-Micron; Eluent A: 1 1 Wasser + 0.01 mol Ammoniumcarbonat, Eluent B: 1 1 Acetonitril; Gradient: 0.0 min 98% A → 0.2min 98% A → 3.0 min 5% A → 4.5 min 5% A ; Ofen: 40°C; Fluss: 1.75 mL/min; UV-Detektion: 210 nm

### Methode 4 (LC-MS):

Gerätetyp MS: Waters Synapt G2S; Gerätetyp UPLC: Waters Acquity I-CLASS; Säule: Waters, HSST3, 2.1 x 50 mm, C18 1.8 µm; Eluent A: 1 1 Wasser + 0.01% Ameisensäure; Eluent B: 1 1 Acetonitril + 0.01% Ameisensäure; Gradient: 0.0 min 10% B → 0.3 min 10% B → 1.7 min 95% B → 2.5 min 95% B; Ofen: 50°C; Fluss: 1.20 mL/min; UV-Detektion: 210 nm

### Methode 5 (LC-MS):

Instrument: Waters ACQUITY SQD UPLC System; Säule: Waters Acquity UPLC HSS T3 1.8 µ 50 x 1 mm; Eluent A: 1 1 Wasser + 0.25 mL 99%ige Ameisensäure , Eluent B: 1 1 Acetonitril + 0.25 mL 99%ige Ameisensäure; Gradient: 0.0 min 95% A → 6.0 min 5% A → 7.5 min 5% A Ofen: 50°C; Fluss: 0.35 mL/min; UV-Detektion: 210 - 400 nm.

### Methode 6 (LC-MS):

Instrument: Micromass Quattro Premier mit Waters UPLC Acquity; Säule: Thermo Hypersil GOLD 1.9 µ 50 x 1 mm; Eluent A: 1 1 Wasser + 0.5 mL 50%ige Ameisensäure, Eluent B: 1 1 Acetonitril + 0.5 mL 50%ige Ameisensäure; Gradient: 0.0 min 97% A → 0.5 min 97% A → 3.2 min 5% A → 4.0 min 5% A Ofen: 50°C; Fluss: 0.3 mL/min; UV-Detektion: 210 nm.

### Methode 7 (LC-MS):

Instrument: Agilent MS Quad 6150;HPLC: Agilent 1290; Säule: Waters Acquity UPLC HSS T3 1.8 µ 50 x 2.1 mm; Eluent A: 1 1 Wasser + 0.25 mL 99%ige Ameisensäure , Eluent B: 1 1 Acetonitril + 0.25 mL 99%ige Ameisensäure; Gradient: 0.0 min 90% A → 0.3 min 90% A → 1.7 min 5% A → 3.0 min 5% A Ofen: 50°C; Fluss: 1,20 mL/min; UV-Detektion: 205 - 305 nm.

### Methode 8 (LC-MS):

Gerätetyp MS: Waters Synapt G2S; Gerätetyp UPLC: Waters Acquity I-CLASS; Säule: Waters, HSST3, 2.1 x 50 mm, C18 1.8 µm; Eluent A: 1 1 Wasser + 0.01% Ameisensäure; Eluent B: 1 1 Acetonitril + 0.01% Ameisensäure; Gradient: 0.0 min 2% B → 2.0 min 2% B → 13.0 min 90% B → 15.0 min 90% B; Ofen: 50°C; Fluss: 1.20 ml/min; UV-Detektion: 210 nm

### Methode 9: LC-MS-Prep Reinigungsmethode für die Beispiele 181-191 (Methode LIND-LC-MS-Prep)

Instrument MS: Waters, Instrument HPLC: Waters (Säule Waters X-Bridge C18, 19 mm x 50 mm, 5 µm, Eluent A: Wasser + 0.05% Ammoniak, Eluent B: Acetonitril (ULC) mit Gradient; Fluss: 40 ml/min; UV-Detektion: DAD; 210 - 400 nm).

### bzw.:

Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäure, Eluent B: Acetonitril (ULC) mit Gradient; Fluss: 40 ml/min; UV-Detektion: DAD; 210 - 400 nm).

### Methode 10: LC-MS-Analytik-Methode für die Beispiele 181-191 (LIND_SQD_SB_AQ)

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 11 (HPLC):

| | |
|---|---|
| Gerät: | HP1100 Serie |
| Säule: | Merck Chromolith SpeedROD RP-18e, 50-4,6mm, Best.- |
| | Nr.1.51450.0001, Vorsäule Chromolith Guard Cartridge Kit, RP-18e, |
| | 5-4,6mm, Best.-Nr. 1.51470.0001 |
| Gradient: | Flow 5mL/ Min |
| | Injection Volume 5µl |
| | Solvent A: HCLO4 (70%ig) in Wasser (4mL/ 1) |
| | Solvent B: Acetonitril |
| | Start 20% B |
| | 0.50 Min 20% B |
| | 3.00 Min 90% B |
| | 3.50 Min 90% B |
| | 3.51 Min 20% B |
| | 4.00 Min 20% B |
| | Säulentemperatur: 40°C |
| Wellenlänge: | 210nm |

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Intermediat C11

### R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-homocystein-trifluoressigsäure(1:1)

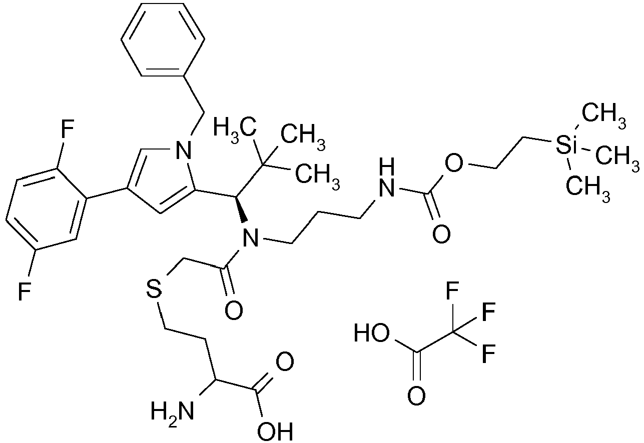

990.0 mg (2.79 mmol) (1R)-1-[1-Benzyl-4-(2,5-difluorphenyl)-1H-pyrrol-2-yl]-2,2-dimethylpropan-1-amin wurden in 15.0 mL Dichlormethan vorgelegt und mit 828.8 mg (3.91 mmol) Natriumtriacetoxyborhydrid und 129.9 mg (3.21 mmol) Essigsäure versetzt und 5 min bei RT grührt. Es wurden 698.1 mg (3.21 mmol) 2-(Trimethylsilyl)ethyl-(3-oxopropyl)carbamat (Intermediat L58) gelöst in 15.0 mL Dichlormethan zugegeben und die Reaktionsmischung über Nacht bei RT gerührt. Die Reaktionsmischung wurde mit Ethylacetat verdünnt und die organische Phase je zweimal mit ges. Natriumcarbonat-Lösung und ges. NaCl-Lösung gewaschen. Die organische Phase wurde über Magnesiumsulfat getrocknet und das Lösemittel im Vakuum verdampft. Der Rückstand wurde mittels Kieselgel (Laufmittel: Dichlormethan/Methanol 100:2) gereinigt. Die Lösemittel wurden im Vakuum verdampft und der Rückstand im Hochvakuum getrocknet. Man erhielt 1.25 g (73 % d. Th.) der Verbindung 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat.
LC-MS (Methode 1): Rₜ = 1.09 min; MS (ESIpos): m/z = 556 (M+H)⁺.

400.0 mg (0.65 mmol) 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat wurden mit 151.4 mg (1.5 mmol) Triethylamin und mit 161.6 mg (1.43 mmol) Chloracetylchlorid versetzt. Die Reaktionsmischung wurde über Nacht bei RT gerührt. Die Reaktionsmischung wurde mit Ethylacetat versetzt und die organische Phase dreimal mit Wasser und einmal mit ges. NaCl-Lösung gewaschen. Es wurde über Magnesiumsulfat getrocknet und das Lösemittel im Vakuum verdampft. Der Rückstand wurde mittels Kieselgel (Laufmittel: Cyclohexan/Ethylacetat 3:1) gereinigt. Die Lösemittel wurden im Vakuum verdampft und der Rückstand im Hochvakuum getrocknet. Man erhielt 254.4 mg (57 % d. Th.) der Verbindung 2-(Trimethylsilyl)ethyl-{3-[{(1R)-1-[1-benzyl-4-(2,5-difluorphenyl)-1H-pyrrol-2-yl]-2,2-dimethylpropyl} (chloracetyl)amino]propyl} carbamat.
LC-MS (Methode 1): Rₜ = 1.49 min; MS (ESIneg): m/z = 676 (M+HCOO⁻)⁻.

117.4 mg (0.19 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat wurde in 10.0 mL Isopropanol gelöst und mit 928.4 µL 1M NaOH und 50.2 mg (0.37 mmol) DL-Homocystein versetzt. Die Reaktionsmischung wurde 4.5 h bei 50 °C gerührt. Die Reaktionsmischung wurde mit Ethylacetat versetzt und die organische Phase mit ges. Natriumhydrogencarbonat-Lösung und ges. NaCl-Lösung gewaschen. Die organische Phase wurde über Magnesiumsulfat getrocknet und das Lösemittel im Vakuum verdampft. Der Rückstand wurde mittles präp. RP-HPLC gereinigt (Säule: Reprosil 250x40; 10µ, Fluss: 50 mL/min, MeCN/Wasser, 0.1 % TFA). Die Lösemittel wurden im Vakuum verdampft und der Rückstand im Hochvakuum getrocknet. Man erhielt 75.3 mg (48 % d. Th.) der Titelverbindung.
LC-MS (Methode 1): Rₜ = 1.24 min; MS (ESIpos): m/z = 731 (M+H)⁺.
¹H-NMR (400 MHz, DMSO-d₆): δ [ppm] = 0.03 (s, 9H), 0.40 (m, 1H), 0.75-0.91 (m, 11H), 1.30 (m, 1H), 1.99-2.23 (m, 2H), 2.63-2.88 (m, 4H), 3.18-3.61 (m, 5H), 3.79-4.10 (m, 3H), 4.89 (d, 1H), 4.89 (d, 1H), 5.16 (d, 1H), 5.56 (s, 1H), 6.82 (m, 1H), 6.91 (s, 1H), 6.97 (m, 1H), 7.13-7.38 (m, 6H), 7.49 (s, 1H), 7.63 (m, 1H), 8.26 (s, 3H).

### Intermediat C12

### R/S-[(8S)-11-{(1R)-1-[1-Benzyl-4-(2,5-difluorphenyl)-1H-pyrrol-2-yl]-2,2-dimethylpropyl}-8-carboxy-2,2-dimethyl-6,12-dioxo-5-oxa-7,11-diaza-2-silatridecan-13-yl]homocystein

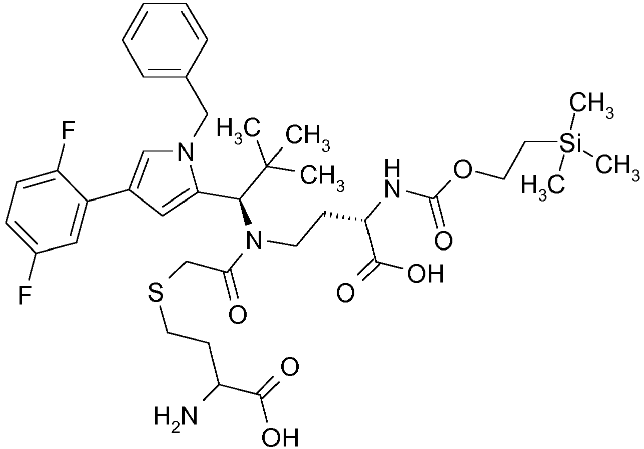

Die Synthese erfolgte in Analogie zur Synthese von Intermediat C11 mit Methyl-(2S)-4-oxo-2-({[2-(trimethylsilyl)ethoxy]carbonyl}amino)butanoat (Intermediat L57) und Intermediat C52 als Edukten.
LC-MS (Methode 1): Rₜ = 1.18 min; MS (ESIpos): m/z = 775 (M+H)⁺.

### Intermediat C42

### 2,5-Difluorbenzoldiazoniumtetrafluoroborat

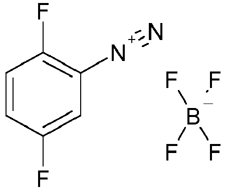

Es wurden 3.00 g (21.16 mmol, 2.68 ml) Bortrifluorid-Diethylether-Komplex vorgelegt und 1.37 g (10.58 mmol) 2,5-Difluoranilin gelöst in 27 ml absolutem THF bei 0°C langsam zu getropft. Bei --10°C wurde eine Lösung von 1.61 g (13.75 mmol, 1.85 ml) Isoamylnitrit gelöst in 3 ml absolutem THF zu getropft und 30 Min. bei gleicher Temperatur weiter gerührt. Es wurden 15 ml Diethylether addiert, das ausgefallene Diazoniumsalz abfiltriert, mit etwas Diethylether gewaschen und im Hochvakuum getrocknet. Es wurden 2.27 g der Zielverbindung (94% d.Th.) erhalten.
LC-MS (Methode 6): Rₜ = 0.24 min; MS (ESIpos): m/z = 141 [M]⁺.
¹H-NMR (400 MHz, DMSO-d₆): δ [ppm] = 8.11-8.17 (m, 1H), 8.36-8.43 (m, 1H), 8.69-8.73 (m, 1H).

### Intermediat C43

### Methyl-chlor[2-(2,5-difluorphenyl)hydrazinyliden]acetat

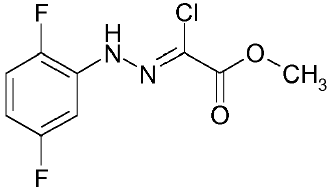

Unter Argonatmospähre wurden 3.63 g (24.13 mmol) Methyl-2-chlor-3-oxobutanoat in 100 ml Wasser vorgelegt und bei -5°C mit 48.90 g (618.19 mmol, 50.00 ml) Pyridin versetzt und 10 Min. bei dieser Temperatur gerührt. Anschließend wurden bei -5°C 5.00 g (21.94 mmol) 2,5-Difluorbenzoldiazoniumtetrafluoroborat zugegeben, wobei sich eine orangefarbene Suspension bildete. Es wurde 30 Min. bei dieser Temperatur gerührt, der Ansatz mit Wasser verdünnt und dreimal mit Dichlormethan extrahiert. Die vereinigten organischen Phasen wurden mit gesättigter Natriumchlorid-Lösung gewaschen, über Natriumsulfat getrocknet, am Rotationsverdampfer eingeengt und im Hochvakuum getrocknet. Es wurden 5.52 g der Zielverbindung (97% d. Th., LC/MS-Reinheit = 96%) erhalten.
LC-MS (Methode 1): Rₜ = 1.03 min; MS (ESIpos): m/z = 249 [M+H]⁺.
¹H-NMR (400 MHz, DMSO-d₆): δ [ppm] = 3.85 (s, 3H), 6.88-6.94 (m, 1H), 7.16-7.21 (m, 1H), 7.31-7.37 (m, 1H), 10.00 (s, 1H).

### Intermediat C44

### Methyl-4-benzoyl-1-(2,5-difluorphenyl)-1H-pyrazol-3-carboxylat

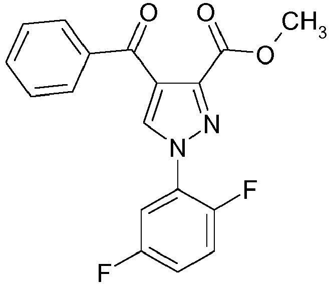

3.50 g (13.52 mmol) Methyl-chlor[2-(2,5-difluorphenyl)hydrazinyliden]acetat (LC/MS-Reinheit 96%) wurden in 9 ml absolutem Toluol gelöst, mit 2.61 g (14.87 mmol) (2E)-3-(Dimethylamino)-1-phenylprop-2-en-1-on sowie 3.01 g (29.73 mmol), 4.14 ml) Triethylamin versetzt und 16 h bei Raumtemperatur gerührt. Das Reaktionsgemisch wurde am Rotationsverdampfer eingeengt und der Rückstand mittels präparativer HPLC (Eluent: ACN/Wasser mit 0.1% Ameisensäure, Gradient) getrennt. Es wurden 1.79 g (39% d. Th.) der Zielverbindung erhalten.
LC-MS (Methode 1): Rₜ = 1.07 min; MS (ESIpos): m/z = 343 [M+H]⁺.
¹H-NMR (400 MHz, DMSO-d₆): δ [ppm] = 3.86 (s, 3H), 7.44-7.50 (m, 1H), 7.55-7.72 (m, 4H), 7.81-7.87 (m, 3H), 8.80 (d, 1H).

### Intermediat C45

### [4-Benzyl-1-(2,5-difluorphenyl)-1H-pyrazol-3-yl]methanol

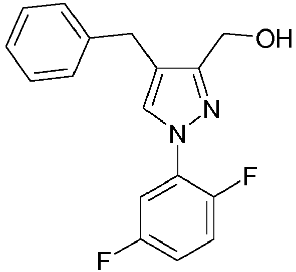

3.18 g (8.92 mmol) Methyl-4-benzoyl-1-(2,5-difluorphenyl)-1H-pyrazol-3-carboxylat (LC/MS-Reinheit = 96%) wurden in 50 ml Trifluoressigsäure vorgelegt, mit 8.74 g (75.13 mmol, 12 ml) Triethylsilan tropfenweise versetzt und 1 h bei Raumtemperatur gerührt. Das Reaktionsgemisch wurde am Rotationsverdampfer eingeengt und im Hochvakuum getrocknet. Der erhaltene Rückstand wurde in 120 ml absolutem THF aufgenommen und bei 0°C mit 2.89 g (33.63 mmol, 33.63 ml) Boran-Tetrahydrofuran-Komplex tropfenweise versetzt. Es wurde über Nacht gerührt. Aufgrund des geringen Umsatzes wurden noch 12.33 ml (12.33 mmol) einer 1M Lithiumborhydrid-Lösung in THF zugegeben. Es wurde 1 h bei Raumtemperatur 30 Min. bei 60°C und 2 h bei 80°C gerührt. Bei 0°C wurde vorsichtig mit 60 ml gesättigter Natriumhydrogencarbonat-Lösung gequencht. Das Gemisch wurde zweimal mit je 100 ml Essigsäureethylester extrahiert, die vereinigten organischen Phasen über Natriumsulfat getrocknet, am Rotationsverdampfer eingeengt und der Rückstand im Hochvakuum getrocknet. Es wurden 2.67 g (76% d. Th., Reinheit = 96%) der Zielverbindung erhalten.
LC-MS (Methode 3): Rₜ = 2.79 min; MS (ESIpos): m/z = 329 [M+H]⁺.
¹H-NMR (400 MHz, DMSO-d₆): δ [ppm] = 3.91 (s, 2H), 4.45 (d, 2H), 6.51 (s, 1H), 7.18-7.23 (m, 2H), 7.27-7.32 (m, 4H), 7.46-7.53 (m, 1H), 7.60-7.65 (m, 1H), 7.95 (d, 1H).

### Intermediat C46

### 4-Benzyl-1-(2,5-difluorphenyl)-1H-pyrazol-3-carbaldehyd

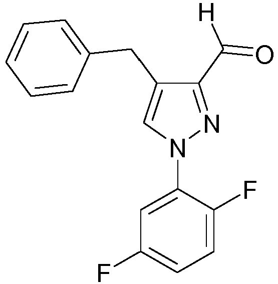

2.66 g (8.50 mmol) [4-Benzyl-1-(2,5-difluorphenyl)-1H-pyrazol-3-yl]methanol (Reinheit 96%) wurden in 150 ml Dichlormethan gelöst und mit 4.33 g (10.20 mmol) Dess-Martin-Periodinan portionsweise versetzt. Das Gemisch wurde 2 h bei Raumtemperatur gerührt und anschließend mit 100 ml einer halbkonzentrierten Natriumhydrogencarbonat-Lösung sowie mit 100 ml einer 10%-igen Natriumthiosulfat-Lösung versetzt, und für 20 Min. gerührt. Die organische Phase wurde abgetrennt, über Natriumsulfat getrocknet und im Hochvakuum eingeengt. Es wurden 2.35 g (88% d. Th., Reinheit = 95%) der Zielverbindung erhalten.

LC-MS (Methode 7): Rₜ = 1.49 min; MS (ESIpos): m/z = 299 [M+H]⁺.
¹H-NMR (400 MHz, DMSO-d₆): δ [ppm] = 4.12 (s, 2H), 7.17-7.21 (m, 1H), 7.27-7.31 (m, 4H), 7.37-7.42 (m, 1H), 7.57-7.62 (m, 1H), 7.75-7.78 (m, 1H), 8.22 (d, 1H), 10.06 (s, 1H).

### Intermediat C47

### (1R)-1-[4-benzyl-1-(2,5-difluorophenyl)-1H-pyrazol-3-yl]-2,2-dimethylpropan-1-amine

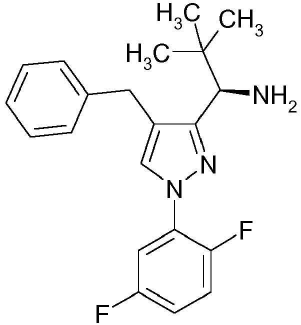

2.35 g (7.56 mmol) 4-Benzyl-1-(2,5-difluorphenyl)-1H-pyrazol-3-carbaldehyd wurden in 25 ml absolutem THF gelöst und mit 1.10 g (9.08 mmol) (R)-(+)-2-Methyl-2-propansulfinsäureamid sowie mit 4.73 g (16.64 mmol) Titan(IV)isopropylat versetzt. Das Reaktionsgemisch wurde bei Raumtemperatur für 16h gerührt, anschließend mit 20 ml einer gesättigten Natriumchlorid-Lösung und 30 ml Essigsäureethylester versetzt. Danach gab man ca. 3 g Kieselgur zu und kochte 1 h unter Rückfluss. Es wurde filtriert und vom Filtrat die organische Phase abgetrennt. Die wässrige Phase wurde mit Essigsäureethylester extrahiert, die vereinigten organischen Phasen mit gesättigter Natriumchlorid-Lösung gewaschen, über Natriumsulfat getrocknet, am Rotationsverdampfer eingeengt und im Hochvakuum getrocknet. Der Rückstand wurde ohne weitere Reinigung weiter eingesetzt.

Unter Argonatmospähre wurde der Rückstand in 60 ml absolutem THF gelöst und auf -78°C gekühlt und 14.5 ml (23.24 mmol) einer Lösung von tert.-Butyllithium in Pentan (c = 1.6 mol/l) zugetropft. Es wurde 3 h bei -78°C gerührt und anschließend mit 5 ml Methanol und 15 ml einer gesättigten Ammoniumchlorid-Lösung gequencht. Unter Rühren ließ man das Reaktionsgemisch auf Raumtemperatur kommen (ca. 30 Min.). Es wurde mit Essigsäureethylester extrahiert, die organische Phase mit gesättigter Natriumchlorid-Lösung extrahiert, am Rotationsverdampfer eingeengt und im Hochvakuum getrocknet. Der Rückstand wurde ohne weitere Reinigung weiter eingesetzt.

Der Rückstand wurde in 30 ml THF und 6 ml Methanol aufgenommen, mit 6 ml (24.00 mmol) einer 4N Chlorwasserstoff-Lösung in Dioxan versetzt und 1 h bei Raumtemperatur gerührt. Anschließend gab man 15 ml gesättigte Natriumcarbonat-Lösung zu und extrahierte mit Essigsäureethylester. Die organische Phase wurde abgetrennt, am Rotationsverdampfer eingeengt und im Hochvakuum getrocknet. Der Rückstand wurde mittels präparativer HPLC (Eluent: ACN/Wasser, Gradient) getrennt. Es wurden zwei Fraktionen der Zielverbindung erhalten. Die erste Fraktion ergab 1.31 g (72 % d. Th., LC/MS Reinheit = 97%) und die zweite 0.37 g (17% d. Th., LC/MS Reinheit = 83%) Produkt.
LC-MS (Methode 1): Rₜ = 0.88 min; MS (ESIpos): m/z = 356 [M+H]⁺.
¹H-NMR (400 MHz, DMSO-d₆): δ [ppm] = 0.91 (s, 9H), 1.71 (s, 2H), 3.59 (s, 1H), 3.87 (s, 2H), 7.17-7.32 (m, 6H), 7.45-7.51 (m, 1H), 7.61-7.65 (m, 1H), 7.84(s br, 1H).

### Intermediat C48

### Tert-butyl-(2S)-4-({(1R)-1-[4-benzyl-1-(2,5-difluorphenyl)-1H-pyrazol-3-yl]-2,2-dimethylpropyl}amino)-2-[(tert-butoxycarbonyl)amino]butanoat

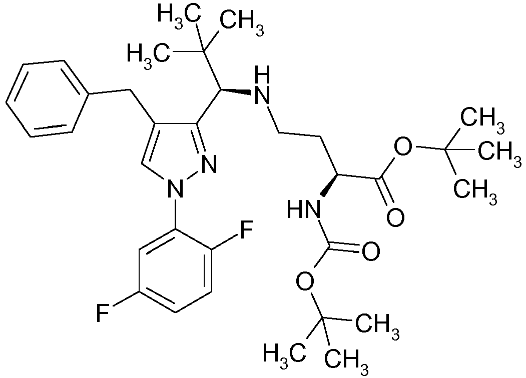

1.28 g (3.35 mmol, LC/MS Reinheit 93%) (1R)-1-[4-benzyl-1-(2,5-difluorophenyl)-1H-pyrazol-3-yl]-2,2-dimethylpropan-1-amine wurden in 100 ml absolutem Dichlormethan gelöst und bei Raumtemperatur mit 261 mg (4.35 mmol, 250 µl) Essigsäure sowie 1.14 g (4.34 mmol) Natriumtriacetoxyborhydrid versetzt und nach 5 Min rühren addierte man 1.19 g (4.35 mmol) tert-Butyl-(2S)-2-[(tert-butoxycarbonyl)amino]-4-oxobutanoat. Es wurde 15 Min. bei Raumtemperatur gerührt, am Rotationsverdampfer eingeengt in Acetonitril und Wasser aufgenommen und mittels präparativer HPLC (Eluent: ACN/Wasser + 0.1% TFA, Gradient) gereinigt. Es wurden 1.64 g (80% d. Th.) der Zielverbindung erhalten.
LC-MS (Methode 1): Rₜ = 1.10 min; MS (ESIpos): m/z = 613 [M+H]⁺.
¹H-NMR (400 MHz, DMSO-d₆): δ [ppm] = 1.01 (s, 9H), 1.32 (s, 9H), 1.35 (s, 9H), 1.80-1.89 (m, 1H), 2.01-2.11 (m, 1H), 2.54-2.71 (m, 2H), 3.75-3.81 (m, 1H), 3.90 (s, 2H), 4.18 (d, 1H), 7.13 (d, 1H), 7.20-7.24 (m, 1H), 7.28-7.34 (m, 5H), 7.52-7.58 (m, 1H), 7.76-7.80 (m, 1H), 8.10 (s br, 1H), 8.23 (s br, 1H).

### Intermediat C49

### (2S)-4-[{(1R)-1-[4-Benzyl-1-(2,5-difluorphenyl)-1H-pyrazol-3-yl]-2,2-dimethylpropyl}(glycoloyl)amino]-2-[(tert-butoxycarbonyl)amino]butansäure

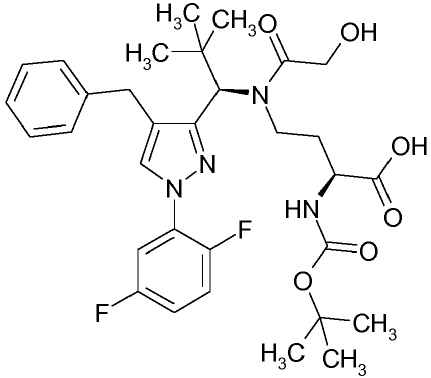

225 mg (0.37 mmol) Tert-butyl-(2S)-4-({(1R)-1-[4-benzyl-1-(2,5-difluorphenyl)-1H-pyrazol-3-yl]-2,2-dimethylpropyl}amino)-2-[(tert-butoxycarbonyl)amino]butanoat wurden in 10 ml absolutem Dichlormethan gelöst, mit 156 mg (1.54 mmol) Triethylamin versetzt. Bei 0°C wurden 125 mg (0.92 mmol) Acetoxyacetylchlorid zugegeben und bei RT für 16 h gerührt. Es wurden erneut 251 mg (1.84 mmol) Acetoxyacetylchlorid und 186 mg (1.84 mmol) Triethylamin addiert und 3 h bei RT gerührt. Es wurde etwas Dichlormethan zugegeben, mit gesättigter Natriumhydrogencarbonat-Lösung und gesättigter Natriumchlorid-Lösung gewaschen. Die organische Phase wurde über Natriumsulfat getrocknet, am Rotationsverdampfer eingeengt und im Hochvakuum getrocknet. Der Rückstand wurde in 10 ml Ethanol aufgenommen und mit einer 0.91 ml (12.67 mmol) einer 40%-igen wässrigen Methylamin-Lösung versetzt und bei 50°C 3 h gerührt. Das Gemisch wurde am Rotationsverdampfer eingeengt, der Rückstand in Dichlormethan aufgenommen und die organische Phase zweimal mit Wasser gewaschen. Die organische Phase wurde über Natriumsulfat getrocknet, am Rotationsverdampfer eingeengt und im Hochvakuum getrocknet. Der Rückstand wurde in 2 ml Dichlormethan aufgenommen mit 2 ml (25.96 mmol) Trifluoressigsäure versetzt und bei 50°C 4 h gerührt. Das Gemisch wurde am Rotationsverdampfer eingeengt und der Rückstand im Hochvakuum getrocknet. Der Rückstand wurde in 10 ml absolutem Dichlormethan aufgenommen, mit 298 mg (2.95 mmol) Triethylamin sowie 429 mg (1.97 mmol) Di-tert-butyldicarbonat versetzt und 1 h bei RT gerührt. Das Gemisch wurde am Rotationsverdampfer eingeengt und der Rückstand mittels präparativer HPLC (Eluent: ACN/Wasser, Gradient) gereinigt. Es wurden 62 mg (27% d. Th.) der Zielverbindung erhalten.
LC-MS (Methode 1): Rₜ = 1.32 min; MS (ESIpos): m/z = 615 [M+H]⁺.
¹H-NMR (400 MHz, DMSO-d₆): δ [ppm] = 0.91 (s, 9H), 1.32 (s, 9H), 2.64-2.72 (m, 4H), 3.50-3.58 (m, 1H), 3.72 (dd, 2H), 4.07-4.22 (m, 2H), 4.47-4.54 (m, 1H), 5.75 (s, 1H), 6.84-6.89 (m, 1H), 7.15-7.30 (m, 6H), 7.47-7.53 (m, 1H), 7.70-7.75 (m, 1H), 8.09-8.13 (m, 1H), 11.66 (s br, 1H).

### Intermediat C50

### tert-Butyl-[(2S)-4-[{(1R)-1-[4-benzyl-1-(2,5-difluorphenyl)-1H-pyrazol-3-yl]-2,2-dimethylpropyl}(glycoloyl)amino]-1-{[2-(2,5-dioxo-2,5-dihydro-1H-pyrrol-1-yl)ethyl]amino}-1-oxobutan-2-yl]carbamat

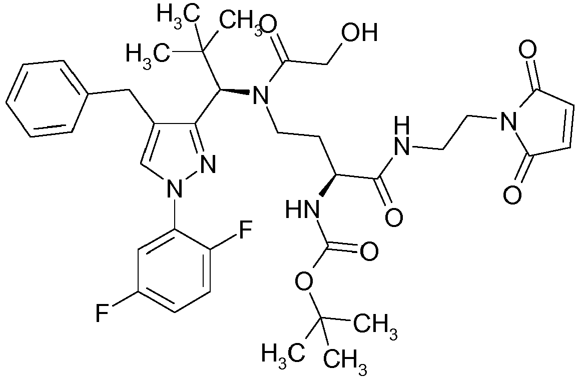

60 mg (0.1 mmol) (2S)-4-[{(1R)-1-[4-Benzyl-1-(2,5-difluorphenyl)-1H-pyrazol-3-yl]-2,2-dimethylpropyl}(glycoloyl)amino]-2-[(tert-butoxycarbonyl)amino]butansäure wurden in 10 ml absolutem DMF gelöst und mit 74 mg (0.20 mmol) HATU versetzt. 74 mg (0.29 mmol) Trifluoressigsäure-1-(2-aminoethyl)-1H-pyrrol-2,5-dion(1:1) wurden separat in 2 ml absolutem DMF gelöst und mit 38 mg (0.29 mmol) N,N-Diisopropylethylamin versetzt und tropfenweise zum Reaktionsgemisch gegeben. Es wurde 3 d bei RT gerührt. Das Gemisch wurde direkt mittels präparativer HPLC (Eluent: ACN/Wasser + 0.1% TFA, Gradient) gereinigt. Es wurden 9.3 mg (13% d. Th.) der Zielverbindung erhalten.

LC-MS (Methode 1): Rₜ = 1.34 min; MS (ESIpos): m/z = 737 [M+H]⁺.

### Intermediat C51

### N-{(2S)-4-[{(1R)-1-[4-Benzyl-1-(2,5-difluorphenyl)-1H-pyrazol-3-yl]-2,2-dimethylpropyl}(glycoloyl)amino]-2-[(tert-butoxycarbonyl)amino]butanoyl}-beta-alanin

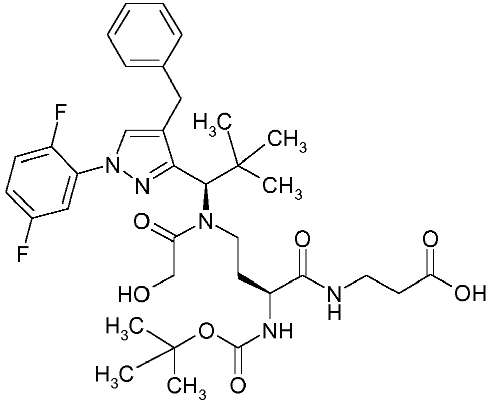

Zunächst wurde Intermediat C47 mit Benzyl-N-{(2S)-2-[(tert-butoxycarbonyl)amino]-4-oxobutanoyl}-beta-alaninat in Analogie zu Intermediat C2 reduktiv alkyliert. Anschließend wurde die sekundäre Aminogruppe mit 2-Chlor-2-oxoethylacetat wie in Intermediat C27 beschrieben acyliert und schließlich mit 2M Lithiumhydroxidlösung in Methanol die Hydrolyse der beiden Estergruppen durchgeführt. Es wurden 23 mg der Titelverbindung erhalten.
LC-MS (Methode 1): Rₜ = 1.24 min; MS (ESIpos): m/z = 686 (M+H)⁺.

### Intermediat C52

### (1R)-1-[1-Benzyl-4-(2,5-difluorphenyl)-1H-pyrrol-2-yl]-2,2-dimethylpropan-1-amin

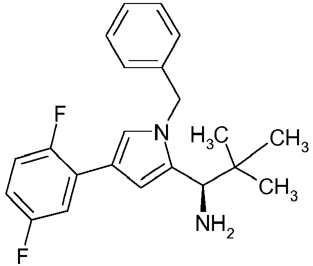

10.00 g (49.01 mmol) Methyl-4-brom-1H-pyrrol-2-carboxylat wurden in 100.0 mL DMF vorgelegt und mit 20.76 g (63.72 mmol) Cäsiumcarbonat und 9.22 g (53.91 mmol) Benzylbromid versetzt. Die Reaktionsmischung rührte über Nacht bei RT. Die Reaktionsmischung wurde zwischen Wasser und Ethylacetat verteilt und die wässrige Phase mit Ethylacetat extrahiert. Die vereinigten organischen Phasen wurden über Magnesiumsulfat getrocknet und das Lösemittel im Vakuum verdampft. Der Ansatz wurde mit 90.0 g Methyl-4-brom-1H-pyrrol-2-carboxylat wiederholt.

Die Reinigung der vereinigten beiden Ansätze erfolgte mittles präp. RP-HPLC (Säule: Daiso 300x100; 10µ, Fluss: 250 mL/min, MeCN/Wasser). Die Lösemittel wurden im Vakuum verdampft und der Rückstand im Hochvakuum getrocknet. Man erhielt 125.15 g (87 % d. Th.) der Verbindung Methyl-1-benzyl-4-brom-1H-pyrrol-2-carboxylat.
LC-MS (Methode 1): Rₜ = 1.18 min; MS (ESIpos): m/z = 295 [M+H]⁺.

Unter Argon wurden 4.80 g (16.32 mmol) Methyl-1-benzyl-4-brom-1H-pyrrol-2-carboxylat in DMF vorgelegt und mit 3.61 g (22.85 mmol) (2,5-Difluorphenyl)boronsäure und 19.20 mL ges. Natriumcarbonat-Lösung und 1.33 g (1.63 mmol) [1,1'-Bis-(diphenylphosphino)-ferrocen]-dichlorpalladium(II):Dichlormethan versetzt. Die Reaktionsmischung wurde über Nacht bei 85 °C gerührt. Die Reaktionsmischung wurde über Celite filtriert und der Filterkuchen mit Ethylacetat gewaschen. Die organische Phase wurde mit Wasser extahiert und dann mit ges. NaCl-Lösung gewaschen. Die organische Phase wurde über Magnesiumsulfat getrocknet und das Lösemittel im Vakuum verdampft. Der Rückstand wurde mittels Kieselgel (Laufmittel: Cyclohexan/Ethylacetat 100:3) gereinigt. Die Lösemittel wurden im Vakuum verdampft und der Rückstand im Hochvakuum getrocknet. Man erhielt 3.60 g (67 % d. Th.) der Verbindung Methyl-1-benzyl-4-(2,5-difluorphenyl)-1H-pyrrol-2-carboxylat.
LC-MS (Methode 7): Rₜ = 1.59 min; MS (ESIpos): m/z = 328 [M+H]⁺.

3.60 g (11.00 mmol) Methyl-1-benzyl-4-(2,5-difluorphenyl)-1H-pyrrol-2-carboxylat wurden in 90.0 mL THF vorgelegt und bei 0 °C mit 1.04 g (27.50 mmol) Lithiumaluminiumhydrid (2.4 M in THF) versetzt. Die Reaktionsmischung wurde 30 Minuten bei 0 °C gerührt. Es wurde bei 0 °C ges. Kaliumnatriumtartrat-Lösung zugegeben und die Reaktionsmischung mit Ethylacetat versetzt. Die organische Phase wurde dreimal mit ges. Kaliumnatriumtartrat-Lösung extrahiert. Die organische Phase wurde einmal mit ges. NaCl-Lösung gewaschen und über Magnesiumsulfat getrocknet. Das Lösemittel wurde im Vakuum verdampft und der Rückstand in 30.0 mL Dichlormethan gelöst. Es wurden 3.38 g (32.99 mmol) Mangan(IV)oxid zugegeben und 48 h bei RT gerührt. Es wurden nochmals 2.20 g (21.47 mmol) Mangan(IV)oxid zugegeben und über Nacht bei RT gerührt. Die Reaktionsmischung wurde über Celite filtriert und der Filterkuchen mit Dichlormethan gewaschen. Das Lösemittel wurde im Vakuum verdampft und der Rückstand 2.80 g (1-Benzyl-4-(2,5-difluorphenyl)-1H-pyrrol-2-carbaldehyd) wurde ohne weitere Reinigung in der nächsten Synthesestufe eingesetzt.
LC-MS (Methode 7): Rₜ = 1.48 min; MS (ESIpos): m/z = 298 [M+H]⁺.

28.21 g (94.88 mmol) 1-Benzyl-4-(2,5-difluorphenyl)-1H-pyrrol-2-carbaldehyd wurden zusammen mit 23.00 g (189.77 mmol) (R)-2-Methylpropan-2-sulfinamid in 403.0 mL absol. THF vorgelegt und mit 67.42 g (237.21 mmol) Titan(IV)isopropylat versetzt und über Nacht bei RT gerührt. Es wurden 500.0 mL ges. NaCl-Lösung und 1000.0 mL Ethylacetat zugegeben und 1 h bei RT gerührt. Es wurde über Kieselgur filtriert und das Filtrat zweimal mit ges. NaCl-Lösung gewaschen. Die org. Phase wurde über Magnesiumsulfat getrocknet und das Lösemittel wurde im Vakuum verdampft und der Rückstand wurde mittels Biotage Isolera gereinigt (Kieselgel, Säule 1500+340 g SNAP, Fluss 200 mL/min, Ethylacetat/Cyclohexan 1:10).
LC-MS (Methode 7): Rₜ = 1.63 min; MS (ESIpos): m/z = 401 [M+H]⁺.

25.00 g (62.42 mmol) (R)-N-{(E/Z)-[1-Benzyl-4-(2,5-difluorphenyl)-1H-pyrrol-2-yl]methylen}-2-methylpropan-2-sulfinamid wurden in absol. THF unter Argon vorgelegt und auf -78 °C abgekühlt. Dann wurden 12.00 g (187.27 mmol) tert.-Butyllithium (1.7 M Lösung in Pentan) bei -78 °C zugegeben und 3 h bei dieser Temperatur gerührt. Dann wurden bei -78 °C 71.4 mL Methanol und 214.3 mL ges. Ammoniumchlorid-Lösung nacheinander zugegeben und die Reaktionsmischung wurde auf RT kommen lassen und 1 h bei RT gerührt. Es wurde mit Ethylacetat verdünnt und mit Wasser gewaschen. Die organische Phase wurde über Magnesiumsulfat getrocknet und das Lösemittel wurde im Vakuum verdampft. Der Rückstand (R)-N-{(1R)-1-[1-Benzyl-4-(2,5-difluorphenyl)-1H-pyrrol-2-yl]-2,2-dimethylpropyl}-2-methylpropan-2-sulfinamid wurde ohne weitere Reinigung in der nächsten Synthesestufe eingesetzt.
LC-MS (Methode 6): Rₜ = 2.97 min; MS (ESIpos): m/z = 459 [M+H]⁺.

28.00 g (61.05 mmol) (R)-N-{(1R)-1-[1-Benzyl-4-(2,5-difluorphenyl)-1H-pyrrol-2-yl]-2,2-dimethylpropyl}-2-methylpropan-2-sulfinamid wurden in 186.7 mL 1,4-Dioxan vorgelegt und dann mit 45.8 mL HCl in 1,4-Dioxan-Lösung (4.0 M) versetzt. Die Reaktionsmischung wurde 2 h bei RT gerührt und das Lösemittel wurde im Vakuum verdampft. Der Rückstand wurde mittles präp. RP-HPLC gereinigt (Säule: Kinetix 100x30; Fluss: 60 mL/min, MeCN/Wasser). Das Acetonitril wurde im Vakuum verdampft und der wässrige Rückstand wurde mit Dichlormethan versetzt. Die organische Phase wurde mit Natriumhydrogencarbonat-Lösung gewaschen und über Magnesiumsulfat getrocknet. Das Lösemittel wurde im Vakuum verdampft und der Rückstand wurde im Hochvakuum getrocknet. Man erhielt 16.2 g (75 % d. Th.) der Titelverbindung.
LC-MS (Methode 6): Rₜ = 2.10 min; MS (ESIpos): m/z = 338 [M-NH₂]⁺, 709 [2M+H]⁺.
¹H-NMR (400 MHz, DMSO-d₆): δ [ppm] = 0.87 (s, 9H), 1.53 (s, 2H), 3.59 (s, 1H), 5.24 (d, 2H), 6.56 (s, 1H), 6.94 (m, 1H), 7.10 (d, 2H), 7.20 (m, 1H), 7.26 (m, 2H), 7.34 (m, 2H), 7.46 (m, 1H).

### Intermediat C53

### (2S)-4-[{(1R)-1-[1-Benzyl-4-(2,5-difluorphenyl)-1H-pyrrol-2-yl]-2,2-dimethylpropyl}(glycoloyl)amino]-2-{[(9H-fluoren-9-ylmethoxy)carbonyl]amino}butansäure

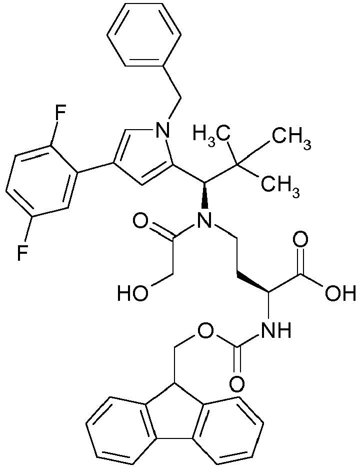

Zunächst wurde Intermediat C52 mit Benzyl-(2S)-2-{[(benzyloxy)carbonyl]amino}-4-oxobutanoat in Analogie zu Intermediat C2 reduktiv alkyliert. Anschließend wurde die sekundäre Aminogruppe mit 2-Chlor-2-oxoethylacetat wie in Intermediat C27 beschrieben acyliert und schließlich mit 2M Lithiumhydroxidlösung in Methanol die Hydrolyse der beiden Estergruppen durchgeführt. Das so erhaltene Intermediat wurde in Ethanol gelöst, mit Palladium auf Kohle (10%ig) versetzt und bei RT mit Wasserstoff unter Normaldruck 1 h hydriert. Die entschützte Verbindung wurde in Dioxan/Wasser 2:1 aufgenommen und mit 9H-Fluoren-9-ylmethylchlorocarbonat in Gegenwart von N,N-Diisopropylethylamin wurde im letzten Schritt die Fmoc-Schutzgruppe eingeführt.
LC-MS (Methode 1): Rₜ = 1.37 min; MS (ESIpos): m/z = 734 (M-H)⁻.

### Intermediat C54

### N-[(2S)-4-[{(1R)-1-[1-Benzyl-4-(2,5-difluorphenyl)-1H-pyrrol-2-yl]-2,2-dimethylpropyl} (glycoloyl)amino]-2-{[(9H-fluoren-9-ylmethoxy)carbonyl]amino}butanoyl]-beta-alanin

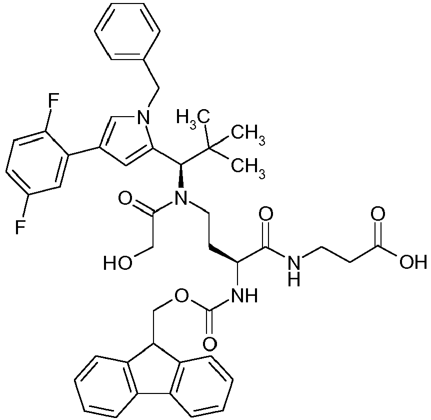

Zunächst wurde Intermediat C52 mit Benzyl-N-[(2S)-2-{[(benzyloxy)carbonyl]amino}-4-oxobutanoyl]-beta-alaninat in Analogie zu Intermediat C2 reduktiv alkyliert. Anschließend wurde die sekundäre Aminogruppe mit 2-Chlor-2-oxoethylacetat wie in Intermediat C27 beschrieben acyliert. Das so erhaltene Intermediat wurde in Methanol gelöst, mit Palladium auf Kohle (10%ig) versetzt und bei RT mit Wasserstoff unter Normaldruck 1 h hydriert. Anschließend wurde mit 2M Lithiumhydroxidlösung in Methanol die Hydrolyse der Estergruppe durchgeführt. Die entschützte Verbindung wurde in Dioxan/Wasser 2:1 aufgenommen und mit 9H-Fluoren-9-ylmethylchlorocarbonat in Gegenwart von N,N-Diisopropylethylamin wurde im letzten Schritt die Fmoc-Schutzgruppe eingeführt. Es wurden 48 mg der Titelverbindung erhalten.
LC-MS (Methode 1): Rₜ = 1.38 min; MS (ESIpos): m/z = 807 (M+H)⁺.

### Intermediat C55

### 2-[3-({(1R)-1-[4-Benzyl-1-(2,5-difluorphenyl)-1H-pyrazol-3-yl]-2,2-dimethylpropyl}amino)propyl]-1H-isoindol-1,3(2H)-dion

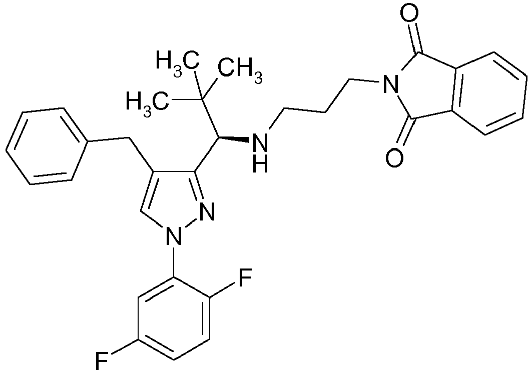

340 mg (0.96 mmol) (1R)-1-[4-benzyl-1-(2,5-difluorophenyl)-1H-pyrazol-3-yl]-2,2-dimethylpropan-1-amine wurden in 7 ml absolutem DCM gelöst und bei RT mit 69 mg (1.15 mmol, 60 µl) Essigsäure sowie 284 mg (1.34 mmol) Natriumtriacetoxyborhydrid versetzt. Es wurde 15 Min. gerührt und anschließend 233 mg (1.15 mmol) 3-(1,3-Dioxo-1,3-dihydro-2H-isoindol-2-yl)propanal addiert. Das Gemisch wurde 4.5 h bei RT gerührt. Es wurden erneut 233 mg (1.15 mmol) 3-(1,3-Dioxo-1,3-dihydro-2H-isoindol-2-yl)propanal, 69 mg (1.15 mmol, 60 µl) Essigsäure sowie 284 mg (1.34 mmol) Natriumtriacetoxyborhydrid zugegeben und 7 h bei RT gerührt. Zur Reaktionsmischung wurde Essigsäureethylester addiert und mit gesättigter Natriumcarbonat-Lösung gewaschen. Die organische Phase wurde eingeengt und der Rückstand zweimal mittels präparativer HPLC [1.) Eluent: ACN/Wasser + 0.1% TFA, Gradient; 2.) Eluent: ACN/Wasser + 1% TFA+1.0% NEt₃)] gereinigt. Es wurden 108 mg (21% d. Th.) der Zielverbindung erhalten.
LC-MS (Methode 1): Rₜ = 0.96 min; MS (ESIpos): m/z = 543 [M+H]⁺.

### Intermediat C56

### 2-({(1R)-1-[4-Benzyl-1-(2,5-difluorphenyl)-1H-pyrazol-3-yl]-2,2-dimethylpropyl}[3-(1,3-dioxo-1,3-dihydro-2H-isoindol-2-yl)propyl]amino)-2-oxoethylacetat

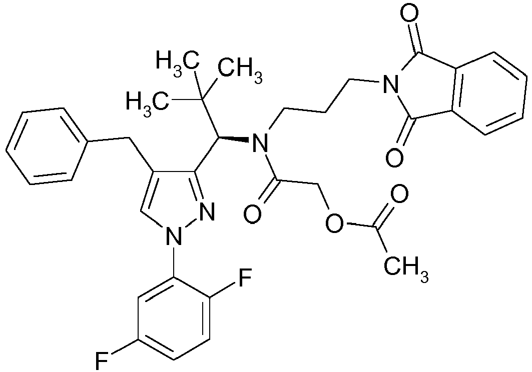

102 mg (0.19 mmol) 2-[3-({(1R)-1-[4-Benzyl-1-(2,5-difluorphenyl)-1H-pyrazol-3-yl]-2,2-dimethylpropyl}amino)propyl]-1H-isoindol-1,3(2H)-dion wurden in 2 ml absolutem DCM vorgelegt und bei RT mit 44 mg (0.43 mmol) Triethylamin versetzt. Bei 0°C wurden 31 mg (0.23 mmol) 2-Chlor-2-oxoethylacetat gelöst in 1 ml absolutem DCM addiert. Das Gemisch wurde 40 Min. bei RT gerührt. Es wurden erneut 26 mg) 2-Chlor-2-oxoethylacetat gelöst in 0.5 ml absolutem DCM sowie 19 mg (0.19 mmol) Triethylamin zugesetzt und 60 Min. bei RT gerührt.

Es wurde Wasser addiert, das Gemisch am Rotationsverdampfer eingeengt und der Rückstand mittels präparativer HPLC (Eluent: ACN/Wasser + 0.1% TFA, Gradient) gereinigt. Es wurden 106 mg (88% d. Th.) der Zielverbindung erhalten.
LC-MS (Methode 1): Rₜ = 1.37 min; MS (ESIpos): m/z = 643 [M+H]⁺.

### Intermediat C57

### Trifluoressigsäure-tert-butyl-{(2S)-1-[(2-aminoethyl)amino]-4-[{(1R)-1-[4-benzyl-1-(2,5-difluorphenyl)-1H-pyrazol-3-yl]-2,2-dimethylpropyl}(glycoloyl)amino]-1-oxobutan-2-yl}carbamat(1:1)

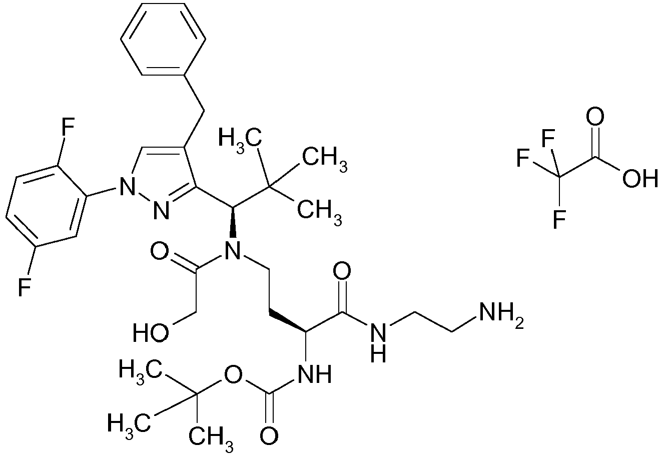

Die Titelverbindung wurde nach Standardmethoden durch Kupplung von Intermediat C49 mit 9H-Fluoren-9-ylmethyl-(2-aminoethyl)carbamat in Gegenwart von HATU und anschließender Abspaltung der Fmoc-Schutzgruppe mit Piperidin hergestellt. Es wurden 14 mg der Titelverbindung (40% d. Th. über 2 Stufen) erhalten.
LC-MS (Methode 1): Rₜ = 0.98 min; MS (ESIpos): m/z = 657 (M+H)⁺.

### Intermediat C58

### (2S)-4-[{(1R)-1-[1-Benzyl-4-(2,5-difluorphenyl)-1H-pyrrol-2-yl]-2,2-dimethylpropyl}(glycoloyl) amino]-2-({[2-(trimethylsilyl)ethoxy]carbonyl}amino)butansäure

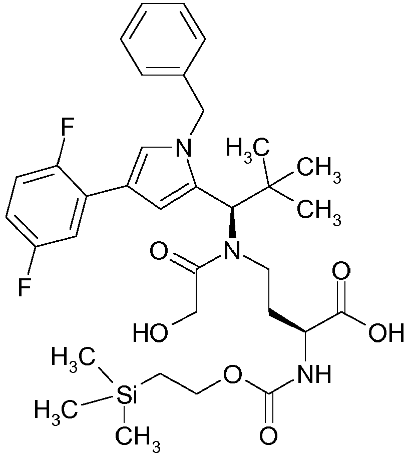

Zunächst wurde Intermediat C52 mit Benzyl-(2S)-2-{[(benzyloxy)carbonyl]amino}-4-oxobutanoat in Analogie zu Intermediat C2 reduktiv alkyliert. Anschließend wurde die sekundäre Aminogruppe mit 2-Chlor-2-oxoethylacetat wie in Intermediat C27 beschrieben acyliert und schließlich mit 2M Lithiumhydroxidlösung in Methanol die Hydrolyse der beiden Estergruppen durchgeführt. Das so erhaltene Intermediat wurde in Ethanol gelöst, mit Palladium auf Kohle (10%ig) versetzt und bei RT mit Wasserstoff unter Normaldruck 1 h hydriert.

500 mg (0.886 mmol) von diesem vollständig entschützten Intermediat wurden in 60 ml Dioxan aufgenommen und mit 253 mg (0.975 mmol) 1-({[2-(Trimethylsilyl)ethoxy]carbonyl}oxy)pyrrolidin-2,5-dion sowie 198 µL Triethylamin versetzt. Nach 24 h Rühren bei RT wurde der Ansatz eingeengt und der Rückstand durch präparative HPLC gereinigt. Nach Vereinigung der entsprechenden Fraktionen, Einengen im Vakuum und Trocknen im Hochvakuum wurden 312 mg (50% d.Th.) der Titelverbindung erhalten.
LC-MS (Methode 5): Rₜ = 4.61 min; MS (ESIpos): m/z = 658 (M+H)⁻.

### Intermediat C59

### (2S)-4-({(1R)-1-[1-Benzyl-4-(2,5-difluorphenyl)-1H-pyrrol-2-yl]-2,2-dimethylpropyl}[(2S)-2-methoxypropanoyl]amino)-2-{[(9H-fluoren-9-ylmethoxy)carbonyl]amino}butansäure

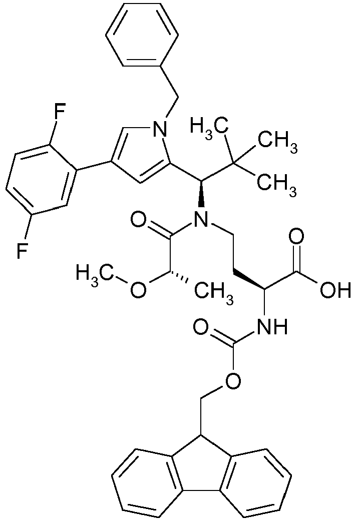

Zunächst wurde die sekundäre Aminogruppe von Benzyl-(2S)-4-({(1R)-1-[1-benzyl-4-(2,5-difluorphenyl)-1H-pyrrol-2-yl]-2,2-dimethylpropyl}amino)-2-{[(benzyloxy)carbonyl]amino}butanoat mit (2S)-2-Methoxypropanoylchlorid (Zwischenstufe aus Intermediat C53) in Gegenwart von Triethylamin wie in Intermediat C53 beschrieben acyliert. Das erhaltene Intermediat wurde in Ethanol aufgenommen, mit Palladium auf Kohle (10%ig) versetzt und bei RT mit Wasserstoff unter Normaldruck 1 h hydriert. Die entschützte Verbindung wurde in Dioxan/Wasser 2:1 aufgenommen und mit 9H-Fluoren-9-ylmethylchlorocarbonat in Gegenwart von N,N-Diisopropylethylamin wurde im letzten Schritt die Fmoc-Schutzgruppe eingeführt.
LC-MS (Methode 1): Rₜ = 1.39 min; MS (ESIpos): m/z = 764 (M-H)⁻.

### Intermediat C60

### (2S)-4-({(1R)-1-[1-Benzyl-4-(2,5-difluorphenyl)-1H-pyrrol-2-yl]-2,2-dimethylpropyl}[(2S)-2-methoxypropanoyl]amino)-2-{[(9H-fluoren-9-ylmethoxy)carbonyl]amino}butansäure

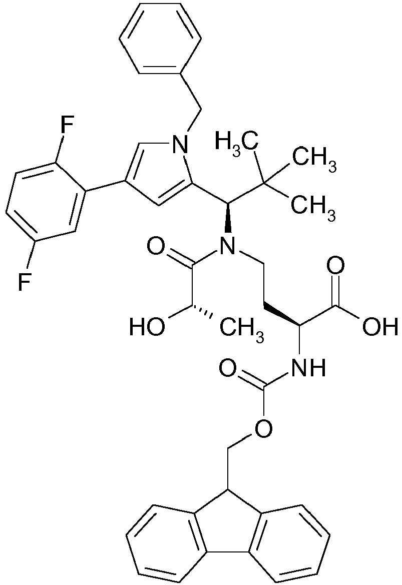

Die Synthese erfolgte in Analogie zu Intermediat C53.
LC-MS (Methode 1): Rₜ = 1.41 min; MS (ESIpos): m/z = 750 (M+H)⁺.

### Intermediat C61

### N-[(2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butanoyl]-beta-alanin

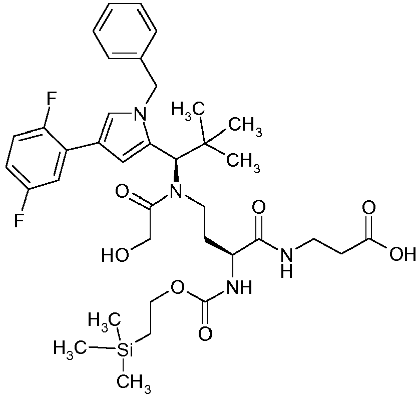

Die Titelverbindung wurde durch Kupplung von 60 mg (0.091 mmol) Intermediat C58 mit β-Alaninmethylester und anschließender Esterspaltung mit 2m Lithiumhydroxidlösung hergestellt. Es wurden 67mg (61% d.Th.) der Titelverbindung über 2 Stufen erhalten.
LC-MS (Methode 1): Rₜ = 1.29 min; MS (ESIpos): m/z = 729 (M+H)⁺.

### Intermediat C62

### N-[(2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butanoyl]-D-alanin

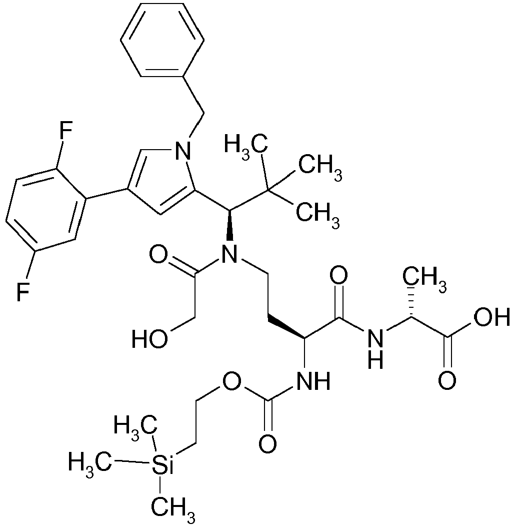

Die Titelverbindung wurde in Analogie zu Intermediat C61 aus Intermediat C58 und Methyl-D-alaninat hergestellt.
LC-MS (Methode 1): Rₜ = 1.32 min; MS (ESIpos): m/z = 729 (M+H)⁺.

### Intermediat C64

### Trifluoressigsäure --2-(trimethylsilyl)ethyl-{(2S)-1-[(2-aminoethyl)amino]-4-[{(1R)-1-[1-benzyl-4-(2,5-difluorphenyl)-1H-pyrrol-2-yl]-2,2-dimethylpropyl}(glycoloyl)amino]-1-oxobutan-2-yl}carbamat (1:1)

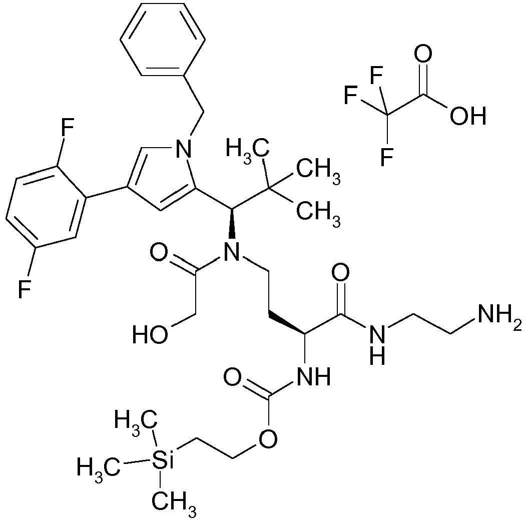

Die Titelverbindung wurde ausgehend von Intermediat C58 in Analogie zu Intermediat C63 hergestellt. HPLC (Methode 11): Rₜ = 2.4 min;
LC-MS (Methode 1): Rₜ = 1.01 min; MS (ESIpos): m/z = 700 (M+H)⁺.

### Intermediat C65

### (8S)-8-{2-[{(1R)-1-[1-Benzyl-4-(2,5-difluorphenyl)-1H-pyrrol-2-yl]-2,2-dimethylpropyl} (glycoloyl)amino]ethyl}-2,2-dimethyl-6,11-dioxo-5-oxa-7,10-diaza-2-silatetradecan-14-säure

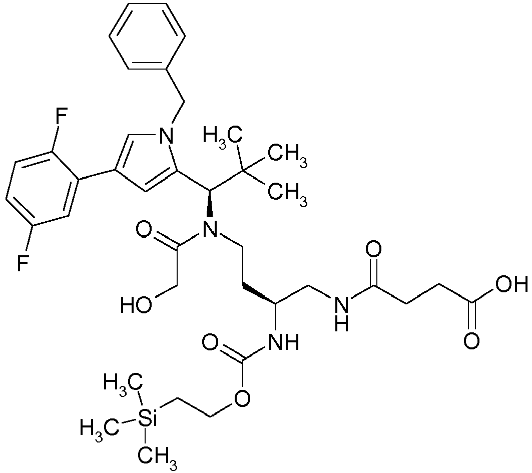

215 mg (0.59 mmol) von Intermediat L66 wurden in 25 mL Dichlormethan vorgelegt und mit 377 mg (0.89 mmol) Dess-Martin Periodinan und 144 µL (1.78 mmol) Pyridin versetzt. Man rührte 30 min bei RT. Anschließend wurde der Ansatz mit 300 ml Dichlormethan verdünnt und die organische Phase je zweimal mit 10%iger Na₂S₂O₃-Lösung, 10%iger Citronensäure-Lösung und ges. Natriumhydrogencarbonat-Lösung gewaschen. Die organische Phase wurde über Magnesiumsulfat getrocknet und das Lösemittel im Vakuum abgedampft. Man erhielt 305 mg des Aldehyds, der ohne weitere Reinigung umgesetzt wurde.

175 mg (0.49 mmol) von Intermediat C52 wurden in 50 ml Dichlormethan gelöst und mit 147mg (0.69 mmol) Natriumtriacetoxyborhydrid sowie 32.5 µL Essigsäure versetzt. Nach 5 min Rühren bei RT wurden 214 mg (0.593 mmol) vom oben beschriebenen Aldehyd hinzugegeben und der Ansatz über Nacht bei RT gerührt. Dabei ist anstelle erwarteten Produktes 2-(Trimethylsilyl)ethyl-[(2S)-4-({(1R)-1-[1-benzyl-4-(2,5-difluorphenyl)-1H-pyrrol-2-yl]-2,2-dimethylpropyl}amino)-1-(2,5-dioxopyrrolidin-1-yl)butan-2-yl]carbamat entstanden. Da auch dieses Imid sich weiter zur Titelverbindung umsetzen lassen sollte, wurde der Ansatz eingeengt und der Rückstand durch präparative HPLC gereinigt. Nach Vereinigung der entsprechenden Imid-enthaltenden Fraktionen wurde das Lösungsmittel im Vakuum abgedampft und der Rückstand im Hochvakuum getrocknet. So wurden 195 mg (58%) des oben benannten Imids erhalten.
LC-MS (Methode 5): Rₜ = 3.32 min; MS (ESIpos): m/z = 667 (M+H)⁺.

65 mg (97.5 µmol) dieses Imids wurden in 15 ml Dichlormethan aufgenommen und mit 367 µL (3.4 mmol) Acetoxyacetylchlorid sowie 595 µL *N,N*-Diisopropylethylamin versetzt. Nach 30 min Rühren bei RT wurde der Ansatz ohne Erwärmung im Vakuum eingeengt und der Rückstand durch präparative HPLC gereinigt. Die entsprechenden Fraktionen wurden vereinigt und nach Abdampfen der Lösungsmittel und Trocknung im Hochvakuum verblieben 28 mg (37% d. Th.) (8S)-11-{(1R)-1-[1-Benzyl-4-(2,5-difluorphenyl)-1H-pyrrol-2-yl]-2,2-dimethylpropyl}-8-[(2,5-dioxopyrrolidin-1-yl)methyl]-2,2-dimethyl-6,12-dioxo-5-oxa-7,11-diaza-2-silatridecan-13-yl-acetat.
LC-MS (Methode 1): Rₜ = 1.44 min; MS (ESIpos): m/z = 767 (M+H)⁺.

28 mg (37 µmol) von diesem Intermediat wurden in 3 mL Methanol gelöst und mit 548 µL einer 2M Lithiumhydroxidlösung versetzt. Nach 10 min Rühren bei RT wurde der Ansatz mit Trifluoressigsäure auf pH 4 gebracht und dann eingeengt. Der Rückstand wurde durch präparative HPLC gereinigt. Nach Vereinigung der entsprechenden Fraktionen, Abdampfen des Lösungsmittels und Trocknung des Rückstandes im Hochvakuum wurden 26 mg (96% d. Th.) der Titelverbindung als weißer Feststoff erhalten.
LC-MS (Methode 1): Rₜ = 1.33 min; MS (ESIpos): m/z = 743 (M+H)⁺.

### Intermediat C66

### 2-(Trimethylsilyl)ethyl-[(2S)-4-[{(1R)-1-[1-benzyl-4-(2,5-difluorphenyl)-1H-pyrrol-2-yl]-2,2-dimethylpropyl}(glycoloyl)amino]-1-{[2-(glycylamino)ethyl]amino}-1-oxobutan-2-yl]carbamat

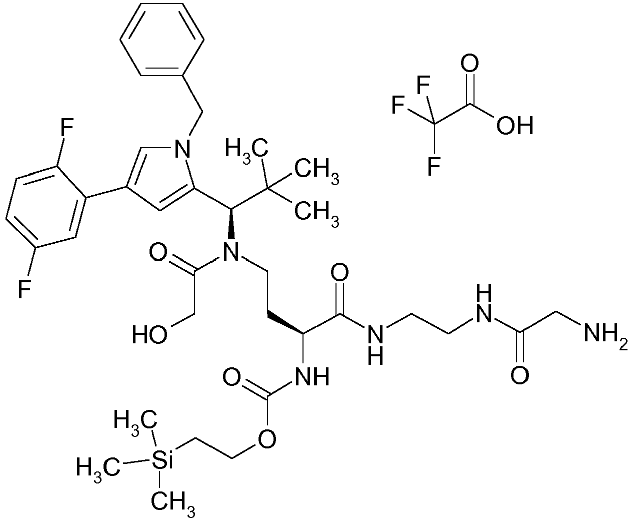

Zuächst wurde aus N-[(benzyloxy)carbonyl]glycin und tert-Butyl (2-aminoethyl)carbamat nach klassischen Methoden der Peptidchemie (HATU-Kupplung und Boc-Spaltung) Trifluoressigsäure - benzyl-{2-[(2-aminoethyl)amino]-2-oxoethyl}carbamat (1:1) hergestellt.

13 mg (0.036 mmol) von diesem Intermediat sowie 25 mg (0.033 mmol) von Intermediat C58 wurden in 3 mL DMF aufgenommen und mit 19 mg (0.05 mmol) HATU und 17 µl N,N-Diisopropylethylamin versetzt. Nach 10 min Rühren bei RT wurde eingeengt und der Rückstand durch präparative HPLC gereinigt. Es wurden 17.8 mg (60% d. Th.) der Zwischenstufe erhalten.
LC-MS (Methode 1): Rₜ = 1.36 min; MS (ESIpos): m/z = 891 (M+H)⁺.

17 mg (0.019 mmol) dieser Zwischenstufe wurden in 10 ml Ethanol gelöst, mit Palladium auf Kohle (10%ig) versetzt und bei RT mit Wasserstoff unter Normaldruck 2 h hydriert. Der Katalysator wurde abfiltriert, die Lösemittel im Vakuum verdampft und der Rückstand im Hochvakuum getrocknet. Es wurden 9 mg (62% d. Th.) der Titelverbindung erhalten.
LC-MS (Methode 1): Rₜ = 1.03 min; MS (ESIpos): m/z = 757 (M+H)⁺.

### Intermediat C67

### 9H-Fluoren-9-ylmethyl-[3-({(1R)-1-[1-benzyl-4-(2,5-difluorphenyl)-1H-pyrrol-2-yl]-2,2-dimethylpropyl}amino)propyl]carbamat

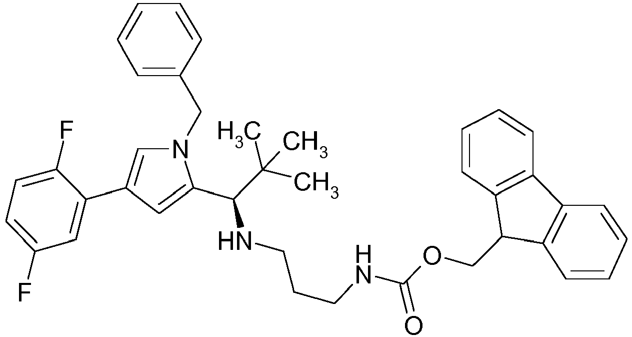

605.3 mg (1.71 mmol) (1R)-1-[1-Benzyl-4-(2,5-difluorphenyl)-1H-pyrrol-2-yl]-2,2-dimethylpropan-1-amin (Intermediat C52) wurden in 10.0 mL Dichlormethan vorgelegt und mit 506.7 mg (2.39 mmol) Natriumtriacetoxyborhydrid und 117.9 mg (1.96 mmol) Essigsäure versetzt und 5 min bei RT grührt. Es wurden 580.0 mg (1.96 mmol) 9H-Fluoren-9-ylmethyl-(3-oxopropyl)carbamat (Intermediat L70) gelöst in 10.0 mL Dichlormethan zugegeben und die Reaktionsmischung über Nacht bei RT gerührt. Die Reaktionsmischung wurde mit Ethylacetat verdünnt und die organische Phase je zweimal mit ges. Natriumcarbonat-Lösung und ges. NaCl-Lösung gewaschen. Die organische Phase wurde über Magnesiumsulfat getrocknet und das Lösemittel im Vakuum verdampft. Der Rückstand wurde mittels Kieselgel (Laufmittel: Cyclohexan/Ethylacetat 3:1) gereinigt. Die Lösemittel wurden im Vakuum verdampft und der Rückstand im Hochvakuum getrocknet. Man erhielt 514.7 mg (46 % d. Th.) der Titelverbindung.
LC-MS (Methode 1): Rₜ = 1.10 min; MS (ESIpos): m/z = 634 (M+H)⁺.

### Intermediat C68

### tert-Butyl-[3-({(1R)-1-[4-benzyl-1-(2,5-difluorphenyl)-1H-pyrazol-3-yl]-2,2-dimethylpropyl}amino)propyl]carbamat

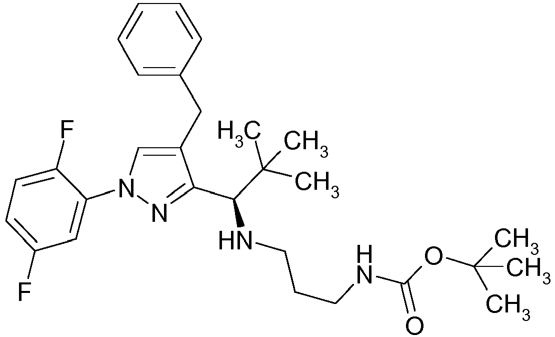

Die Synthese erfolgte in Analogie zur Synthese der Verbidnung Intermediat C67.

1000.0 mg (2.81 mmol) (1R)-1-[4-Benzyl-1-(2,5-difluorphenyl)-1H-pyrazol-3-yl]-2,2-dimethylpropan-1-amin (Intermediat C47)
835.0 mg (3.94 mmol) Natriumtriacetoxyborhydrid
194.0 mg (3.24 mmol) Essigsäure
560.0 mg (3.24 mmol) tert-Butyl-(3-oxopropyl)carbamat
Man erhielt 695.8 mg (48 % d. Th.) der Titelverbindung.
LC-MS (Methode 1): Rₜ = 1.02 min; MS (ESIpos): m/z = 513 (M+H)⁺.

### Intermediat C69

### 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure

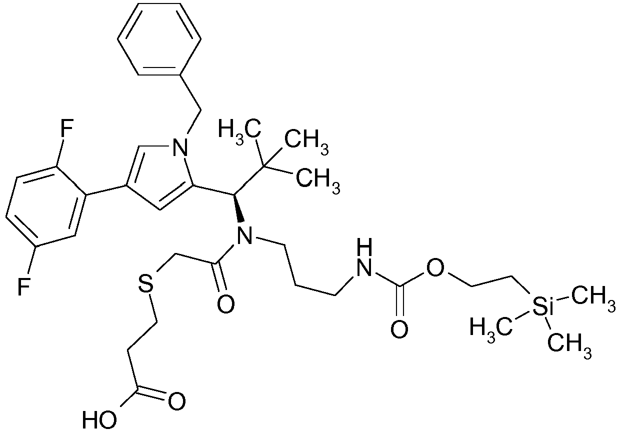

117.0 mg (0.19 mmol) (2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) und 21.6 mg (0.20 mmol) 3-Sulfanylpropansäure wurden in 3.0 mL Methanol vorgelegt und mit 89.5 mg (0.65 mmol) Kaliumcarbonat versetzt und 4 h bei 50 °C gerührt. Die Reaktionsmischung wurde mit Ethylacetat verdünnt und die organische Phase wurde mit Wasser und ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde ohne weitere Reinigung in der nächsten Synthesestufe eingesetzt. Man erhielt 106.1 mg (73 % d. Th.) der Titelverbindung.
LC-MS (Methode 1): Rₜ = 1.42 min; MS (ESIneg): m/z = 700 (M-H)⁻.

### Intermediat C70

### (2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat

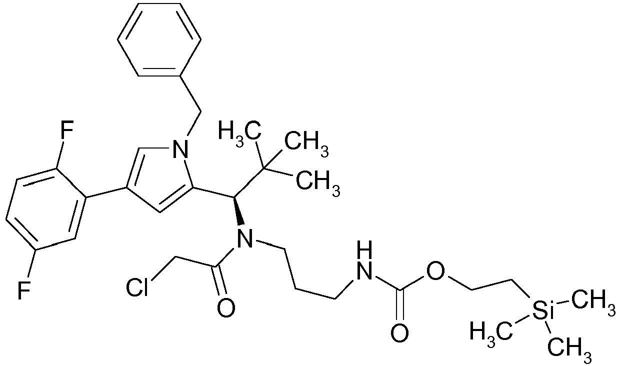

908.1 mg (1.63 mmol) 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat (siehe Synthese Intermediat C11) und 545.6 mg (5.39 mmol) Triethylamin wurden in 10.0 mL Dichlormethan vorgelegt und auf 0 °C abgekühlt. Bei dieser Temperatur wurden 590.5 mg (5.23 mmol) Chloracetylchlorid zugegeben und über Nacht bei RT gerührt. Die Reaktionsmischung wurde mit Ethylacetat verdünnt und die organische Phase wurde je dreimal mit ges. Natriumhydrogencarbonat-Lösung und ges. Ammoniumchlorid-Lösung gewaschen. Die organische Phase wurde mit ges. NaCl-Lösung gewaschen und über Magnesiumsulfat getrocknet. Der Rückstand wurde mittels präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 673.8 mg (65 % d. Th.) der Titelverbindung.
LC-MS (Methode 1): Rₜ = 1.53 min; MS (ESIneg): m/z = 676 (M+HCOO⁻)⁻.

### Intermediat C71

### S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1)

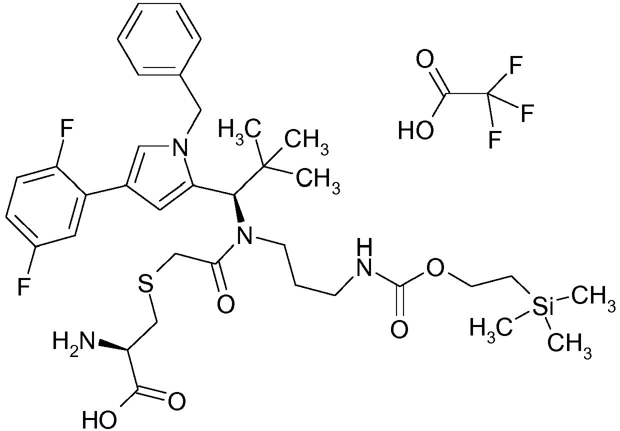

536.6 mg (4.43 mmol) L-Cystein wurden in 2.5 mL Wasser zusammen mit 531.5 mg (6.33 mmol) Natriumhydrogencarbonat suspendiert. Dazu wurden 400.0 mg (0.63 mmol) (2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) gelöst in 25.0 mL *iso*-Propanol und 1.16 g (7.59 mmol) 1,8-Diazabicyclo(5.4.0)undec-7-en gegeben. Die Reaktionsmischung wurde 1.5 h bei 50 °C gerührt. Die Reaktionsmischung wurde mit Ethylacetat versetzt und die org. Phase wurde mehrmals mit ges. Natriumhydrogencarbonat-Lösung und einmal mit ges. NaCl-Lösung gewaschen. Die organische Phase wurde über Magnesiumsulfat getrocknet und das Lösemittel im Vakuum verdampft und der Rückstand im Hochvakuum getrocknet. Der Rückstand wurde mittels präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 449.5 mg (86 % d. Th.) der Titelverbindung.
LC-MS (Methode 1): Rₜ = 1.20 min; MS (ESIpos): m/z = 717 (M+H)⁺.

### Intermediat C72

### (9S)-9-{[{(1R)-1-[1-Benzyl-4-(2,5-difluorphenyl)-1H-pyrrol-2-yl]-2,2-dimethylpropyl}(glycoloyl) amino]methyl}-2,2-dimethyl-6,11-dioxo-5-oxa-7,10-diaza-2-silatetradecan-14-säure

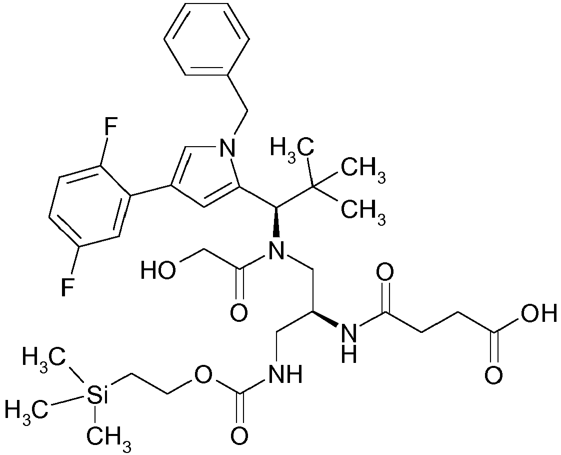

90 mg (0.212 mmol) von Intermediat L72 wurden in 6 mL Dichlormethan vorgelegt und mit 86 µL (1.06 mmol) Pyridin sowie 135 mg (0.318 mmol) Dess-Martin Periodinan versetzt. Man rührte 30 min bei RT. Anschließend wurde der Ansatz mit 30 ml Dichlormethan verdünnt und die organische Phase zweimal mit 10%iger Na₂S₂O₃-Lösung und einmal mit 5%iger Citronensäure-Lösung gewaschen. Die organische Phase wurde über Magnesiumsulfat getrocknet und das Lösemittel im Vakuum abgedampft. Der so erhaltene Aldehyd wurde ohne weitere Reinigung umgesetzt.

63 mg (0.177 mmol) von Intermediat C52 wurden in 15 ml Dichlormethan gelöst und mit 52.4 mg (0.247 mmol) Natriumtriacetoxyborhydrid sowie 20.2 µL Essigsäure versetzt. Nach 5 min Rühren bei RT wurden 89.6 mg (0.212 mmol) vom oben beschriebenen Aldehyd hinzugegeben und der Ansatz 20 min bei RT gerührt. Der Ansatz wurde im Vakuum eingeengt und der Rückstand durch präparative HPLC gereinigt. Nach Vereinigung der entsprechenden Fraktionen wurde das Lösungsmittel im Vakuum abgedampft und der Rückstand aus Acetonitril/Wasser lyophilisiert. So wurden 71 mg (53% d. Th. Über 2 Stufen) Benzyl-(9R)-9-[({(1R)-1-[1-benzyl-4-(2,5-difluorphenyl)-1H-pyrrol-2-yl]-2,2-dimethylpropyl} amino)methyl]-2,2-dimethyl-6,11-dioxo-5-oxa-7,10-diaza-2-silatetradecan-14-oat erhalten.
LC-MS (Methode 1): Rₜ = 1.21 min; MS (ESIpos): m/z = 761 (M+H)⁺.

70 mg (92 µmol) dieses Intermediats wurden in 15 ml Dichlormethan aufgenommen, auf 10°C abgekühlt und mit 54 µL Triethylamin sowie mit 25.5 µL (0.23 mmol) Acetoxyacetylchlorid versetzt. Nach 1 h Rühren bei RT wurden nochmals gleiche Mengen an Säurechlorid und Triethylamin zugegeben und nach einer weiteren Stunde Rühren bei RT erneut. Der Ansatz wurde anschließend für weitere 30 min bei RT gerührt und dann im Vakuum eingeengt und der Rückstand durch präparative HPLC gereinigt. Die entsprechenden Fraktionen wurden vereinigt und nach Abdampfen der Lösungsmittel und Lyophilisation des Rückstandes aus Acetonitril/Wasser verblieben 46.5 mg (59% d. Th.) des acylierten Intermediats.
LC-MS (Methode 1): Rₜ = 1.53 min; MS (ESIpos): m/z = 861 (M+H)⁺.

46 mg (53 µmol) von diesem Intermediat wurden in 5 mL Methanol gelöst und mit 2.7 mL einer 2M Lithiumhydroxidlösung versetzt. Nach 10 min Rühren bei RT wurde der Ansatz mit Essigsäure auf pH 3-4 gebracht und dann mit 15 ml Wasser verdünnt. Die wäßrige Phase wurde mit Ethylacetat extrahiert und die organische Phase über Magnesiumsulfat getrocknet und eingeengt. Der Rückstand wurde aus Acetronitril/Wasser lyophilisiert und nach Trocknung des Rückstandes im Hochvakuum wurden 37 mg (90% d. Th.) der Titelverbindung als weißer Feststoff erhalten.
LC-MS (Methode 1): Rₜ = 1.32 min; MS (ESIpos): m/z = 729 (M+H)⁺.

### Intermediat C73

### S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[3-(trimethylsilyl)propanoyl]-L-cystein

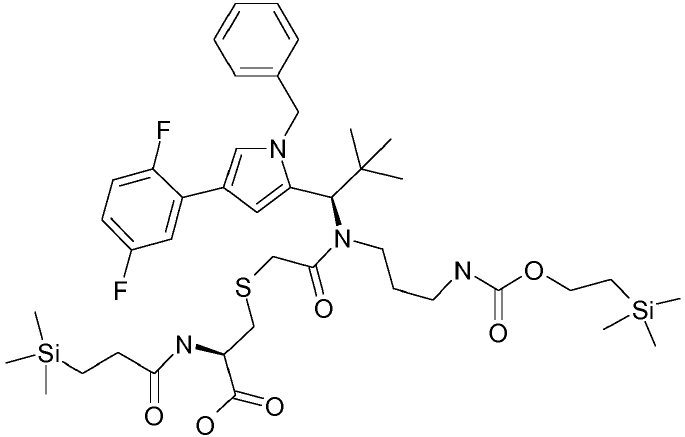

619 mg (0.86 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) wurden in 8.8 ml Dichlormethan vorgelegt und mit 87 mg (0.86 mmol) Triethylamin und 224 mg (0.86 mmol) N-[2-(Trimethylsilyl)-ethoxycarbonyloxy]-pyrrolidin-2,5-dion versetzt. Nach 1h wurden 45 mg (0.17 mmol) N-[2-(Trimethylsilyl)-ethoxycarbonyloxy]-pyrrolidin-2,5-dion zugegeben. Die Reaktionsmischung wurde 1 h bei RT gerührt. Das Gemisch wurde im Vakuum verdampft und der Rückstand in Dichlormethan aufgenommen und dann wurde die organische Phase zweimal mit Wasser und einer gesättigten Natriumhydrogenkarbonat-Lösung gewaschen. Die organische Phase wurde über Magnesiumsulfat getrocknet, am Rotationsverdampfer eingeengt und im Hochvakuum getrocknet. Der Rückstand wurde ohne weitere Reinigung weiter eingesetzt. Man erhielt 602 mg (71%, Reinheit 87%) der Titelverbindung.
LC-MS (Methode 1): Rₜ = 1.58 min; MS (ESIpos): m/z = 861 (M+H)⁺.

### Intermediat L1

### Trifluoressigsäure--N-(2-aminoethyl)-2-(2,5-dioxo-2,5-dihydro-1H-pyrrol-1-yl)acetamid(1:1)

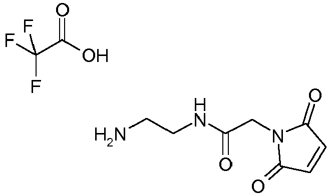

Die Titelverbindung wurde nach klassischen Methoden der Peptidchemie aus kommerziell erhältlicher (2,5-Dioxo-2,5-dihydro-1H-pyrrol-1-yl)essigsäure und tert-Butyl-(2-aminoethyl) carbamat hergestellt. HPLC (Methode 11): Rₜ = 0.19 min;
LC-MS (Methode 1): Rₜ = 0.17 min; MS (ESIpos): m/z = 198 (M+H)⁺.

### Intermediat L2

### Trifluoressigsäure--rel-(1R,2S)-2-amino-N-[2-(2,5-dioxo-2,5-dihydro-1H-pyrrol-1-yl)ethyl]cyclopentancarboxamid(1:1)

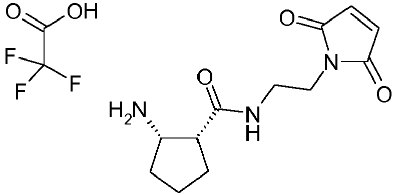

Die Titelverbindung wurde ausgehend von 50 mg (0.214 mmol) von kommerziell erhältlicher cis-2-[(tert-Butoxycarbonyl)amino]-1-cyclopentancarbonsäure mit 60 mg (0.235 mmol) von ebenso kommerziell erhältlichem Trifluoressigsäure--1-(2-aminoethyl)-1H-pyrrol-2,5-dion(1:1) durch Kupplung mit EDC/HOBT und anschließender Entschützung mit TFA hergestellt. Es wurden 36 mg (38% d.Th. über 2 Stufen) der Titelverbindung erhalten.
HPLC (Methode 11): Rₜ = 0.2 min;
LC-MS (Methode 1): Rₜ = 0.17 min; MS (ESIpos): m/z = 252 (M+H)⁺.

### Intermediat L3

### Trifluoressigsäure--(1S,2R)-2-amino-N-[2-(2,5-dioxo-2,5-dihydro-1H-pyrrol-1-yl)ethyl]cyclopentancarboxamid(1:1)

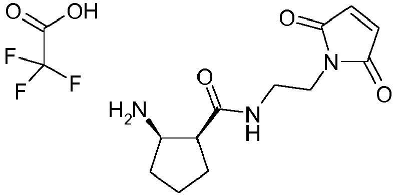

Die Titelverbindung wurde ausgehend von 50 mg (0.214 mmol) von kommerziell erhältlicher (1S,2R)-2-[(tert-Butoxycarbonyl)amino]cyclopentancarbonsäure mit 72 mg (0.283 mmol) von ebenso kommerziell erhältlichem Trifluoressigsäure--1-(2-aminoethyl)-1H-pyrrol-2,5-dion(1:1) durch Kupplung mit EDC/HOBT und anschließender Entschützung mit TFA hergestellt. Es wurden 13 mg (16% d.Th. über 2 Stufen) der Titelverbindung erhalten.
HPLC (Methode 11): Rₜ = 0.2 min;
LC-MS (Methode 1): Rₜ = 0.2 min; MS (ESIpos): m/z = 252 (M+H)⁺.

### Intermediat L4

### Trifluoressigsäure--N-(2-aminoethyl)-4-(2,5-dioxo-2,5-dihydro-1H-pyrrol-1-yl)cyclohexancarboxamid(1:1)

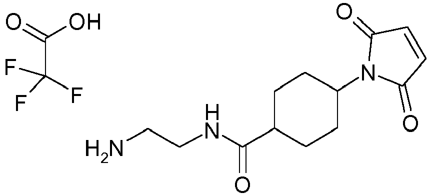

Die Titelverbindung wurde nach klassischen Methoden der Peptidchemie aus kommerziell erhältlichem 1-[(4-{[(2,5-Dioxopyrrolidin-1-yl)oxy]carbonyl}cyclohexyl)methyl]-1H-pyrrol-2,5-dion und tert-Butyl-(2-aminoethyl) carbamat hergestellt.
HPLC (Methode 11): Rₜ = 0.26 min;
LC-MS (Methode 1): Rₜ = 0.25 min; MS (ESIpos): m/z = 280 (M+H)⁺.

### Intermediat L5

### Trifluoressigsäure--N-[4-(2,5-dioxo-2,5-dihydro-1H-pyrrol-1-yl)phenyl]-beta-alaninamid(1:1)

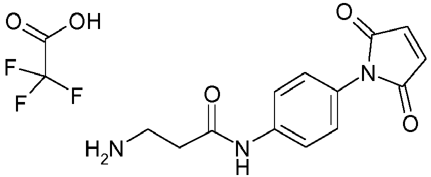

Die Titelverbindung wurde nach klassischen Methoden der Peptidchemie aus kommerziell erhältlichem 1-(4-Aminophenyl)-1H-pyrrol-2,5-dion und N-(tert-Butoxycarbonyl)-beta-alanin hergestellt.
HPLC (Methode 11): Rₜ = 0.22 min;
LC-MS (Methode 1): Rₜ = 0.22 min; MS (ESIpos): m/z = 260 (M+H)⁺.

### Intermediat L6

### Trifluoressigsäure--tert-butyl-N-[6-(2,5-dioxo-2,5-dihydro-1H-pyrrol-1-yl)hexanoyl]-L-valyl-L-alanyl-L-lysinat(1:1)

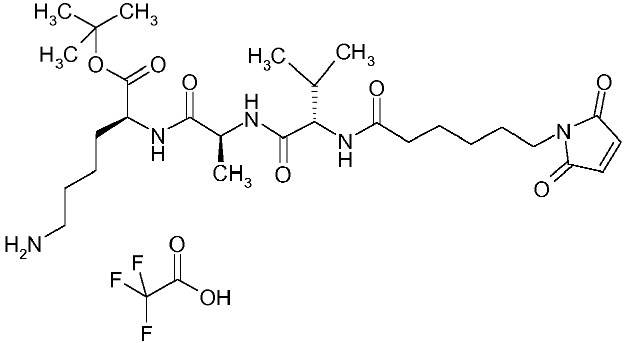

Die Titelverbindung wurde zunächst durch Kupplung von kommerziell erhältlicher 6-(2,5-Dioxo-2,5-dihydro-1H-pyrrol-1-yl)hexansäure mit dem nach klassischen Methoden der Peptidchemie hergestellten partiell geschützten Peptid tert-Butyl-L-valyl-L-alanyl-N⁶-(tert-butoxycarbonyl)-L-lysinat in Gegenwart von EDC/HOBT hergestellt. Anschließend erfolgte die Entschützung an der Aminogruppe unter schonenden Bedingungen durch Rühren in 5%iger Trifluoressigsäure in DCM bei RT, wobei in 37%iger Ausbeute die Titelverbindung erhalten wurde.
HPLC (Methode 11): Rₜ = 1.29 min;
LC-MS (Methode 1): Rₜ = 0.62 min; MS (ESIpos): m/z = 566 (M+H)⁺.

### Intermediat L7

### Trifluoressigsäure--beta-alanyl-L-valyl-N⁵-carbamoyl-N-[4-(2,5-dioxo-2,5-dihydro-1H-pyrrol-1-yl)phenyl]-L-ornithinamid(1:1)

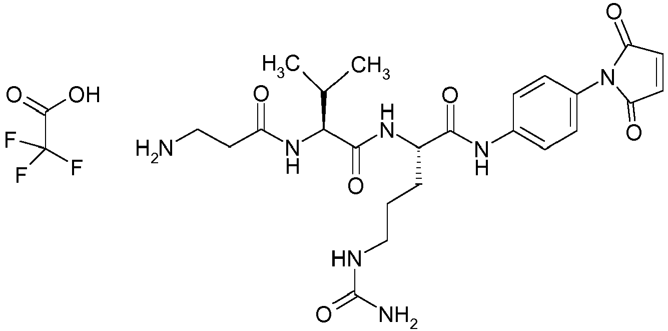

Die Titelverbindung wurde nach klassischen Methoden der Peptidchemie ausgehend von kommerziell erhältlichem 1-(4-Aminophenyl)-1H-pyrrol-2,5-dion durch sequentielle Kupplung mit N²-(tert-Butoxycarbonyl)-N⁵-carbamoyl-L-ornithin in Gegenwart von HATU, Entschützung mit TFA, Kupplung mit 2,5-Dioxopyrrolidin-1-yl-N-(tert-butoxycarbonyl)-L-valinat, Entschützung mit TFA, Kupplung mit 2,5-Dioxopyrrolidin-1-yl-N-(tert-butoxycarbonyl)-beta-alaninat und erneute Entschützung mit TFA hergestellt. Es wurden 32 mg der Titelverbindung erhalten.
HPLC (Methode 11): Rₜ = 0.31 min;
LC-MS (Methode 1): Rₜ = 0.47 min; MS (ESIpos): m/z = 516 (M+H)⁺.

### Intermediat L8

### Trifluoressigsäure--L-alanyl-N⁵-carbamoyl-N-[4-(2,5-dioxo-2,5-dihydro-1H-pyrrol-1-yl)phenyl]-L-ornithinamid(1:1)

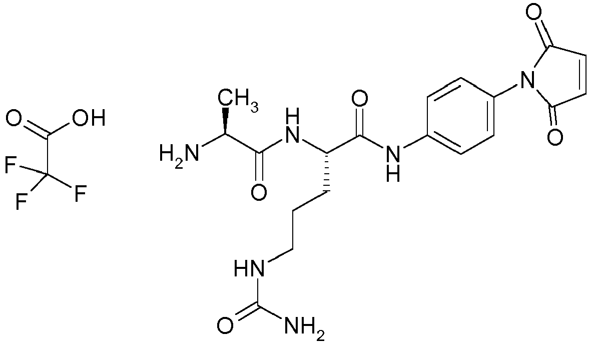

Die Titelverbindung wurde nach klassischen Methoden der Peptidchemie ausgehend von kommerziell erhältlichem 1-(4-Aminophenyl)-1H-pyrrol-2,5-dion durch sequentielle Kupplung mit N²-(tert-Butoxycarbonyl)-N⁵-carbamoyl-L-ornithin in Gegenwart von HATU, Entschützung mit TFA, Kupplung mit 2,5-Dioxopyrrolidin-1-yl-N-(tert-butoxycarbonyl)-L-alaninat und erneute Entschützung mit TFA hergestellt. Es wurden 171 mg der Titelverbindung erhalten.
HPLC (Methode 11): Rₜ = 0.23 min;
LC-MS (Methode 7): Rₜ = 0.3 min; MS (ESIpos): m/z = 417 (M+H)⁺.

### Intermediat L9

### Trifluoressigsäure--beta-alanyl-L-valyl-N⁵-carbamoyl-N-[4-(2-methoxy-2-oxoethyl)phenyl]-L-ornithinamid(1:1)

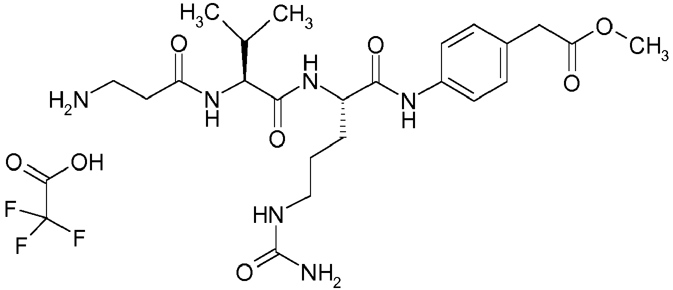

Die Titelverbindung in Analogie zu Intermediat L7 ausgehend von kommerziell erhältlichem Methyl-(4-aminophenyl)acetat hergestellt. Es wurden 320 mg der Titelverbindung erhalten.
HPLC (Methode 11): Rₜ = 0.45 min;
LC-MS (Methode 1): Rₜ = 0.48 min; MS (ESIpos): m/z = 493 (M+H)⁺.

### Intermediat L10

### N-[6-(2,5-Dioxo-2,5-dihydro-1H-pyrrol-1-yl)hexanoyl]-L-valyl-L-alanyl-rel-N⁶-{[(1R,2S)-2-aminocyclopentyl]carbonyl}-L-lysin-trifluoressigsäure(1:2)

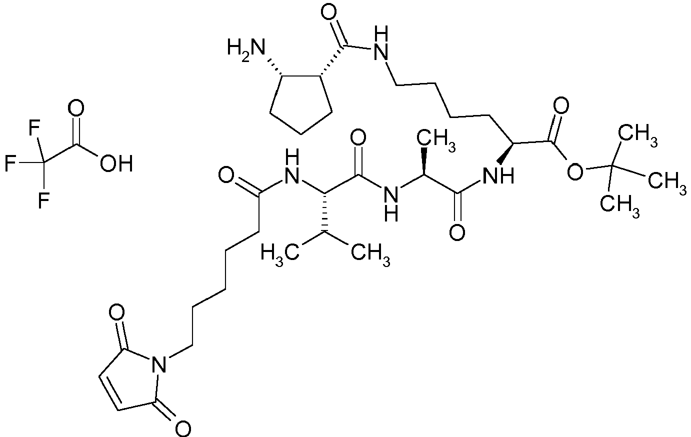

Die Titelverbindung wurde ausgehend von Intermediat L6 durch Kupplung mit cis-2-[(tert-Butoxycarbonyl)amino]-1-cyclopentancarbonsäure mit EDC/HOBT und anschließender Entschützung mit TFA hergestellt. Es wurden 12 mg (52% d.Th. über 2 Stufen) der Titelverbindung erhalten.
HPLC (Methode 11): Rₜ = 1.45 min;
LC-MS (Methode 1): Rₜ = 0.73 min; MS (ESIpos): m/z = 677 (M+H)⁺.

### Intermediat L11

### N-[6-(2,5-Dioxo-2,5-dihydro-1H-pyrrol-1-yl)hexanoyl]-L-valyl-L-alanyl-N⁶-{[(1S,2R)-2-aminocyclopentyl]carbonyl}-L-lysin-trifluoressigsäure(1:2)

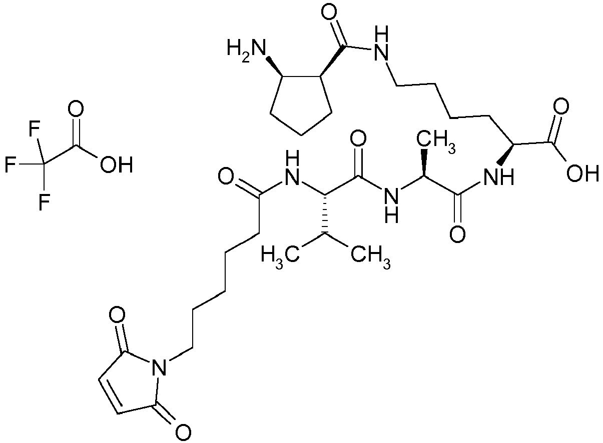

Die Titelverbindung wurde ausgehend von Intermediat L6 durch Kupplung mit (1S,2R)-2-[(tert-Butoxycarbonyl)amino]cyclopentancarbonsäure mit EDC/HOBT und anschließender Entschützung mit TFA hergestellt. Es wurden 11 mg (39% d.Th. über 2 Stufen) der Titelverbindung erhalten.
HPLC (Methode 11): Rₜ = 1.45 min;
LC-MS (Methode 1): Rₜ = 0.74 min; MS (ESIpos): m/z = 677 (M+H)⁺.

### Intermediat L12

### Trifluoressigsäure--1-[2-(2-aminoethoxy)ethyl]-1H-pyrrol-2,5-dion(1:1)

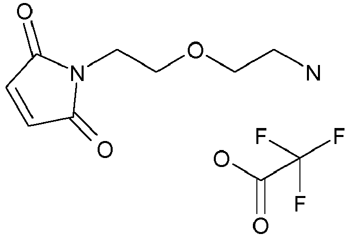

Zu 228 mg (1.12 mmol) tert-Butyl-[2-(2-aminoethoxy)ethyl]carbamat gelöst in 7 mL Dioxan/Wasser 1:1 gelöst wurden 381 mg (2.46 mmol) Methyl-2,5-dioxo-2,5-dihydro-1H-pyrrol-1-carboxylat gegeben. Dann wurden 1.2 mL einer gesättigten Natriumhydrogencarbonat Lösung zugesetzt und der Ansatz bei RT gerührt. Nach insgesamt 5-tägigem Rühren bei 2 weiteren Zugaben von gleichen Mengen der Natriumhydrogencarbonat Lösung wurde der Ansatz durch Ansäuern mit Trifluoressigsäure, Einrotieren und Reinigung des Rückstandes durch präparative HPLC aufgearbeitet. Die entsprechenden Fraktionen wurden vereinigt, das Lösungsmittel im Vakuum entfernt und der Rückstand aus Acetonitril/Wasser 1:1 lyophilisiert.

Der Rückstand wurde in 3 mL Dichlormethan aufgenommen und mit 1 mL Trifluoressigsäure versetzt. Nach 15 min Rühren bei RT wurde das Lösungsmittel im Vakuum entfernt und der Rückstand aus Acetonitril/Wasser 1:1 lyophilisiert. So wurden 70 mg (67% d.Th. über 2 Stufen) der Titelverbindung als harziger Rückstand erhalten.
HPLC (Methode 11): Rₜ = 0.2 min;
LC-MS (Methode 1): Rₜ = 0.18 min; MS (ESIpos): m/z = 185 (M+H)⁺.

### Intermediat L13

### Trifluoressigsäure--tert-butyl-N²-[(2,5-dioxo-2,5-dihydro-1H-pyrrol-1-yl)acetyl]-L-lysinat(1:1)

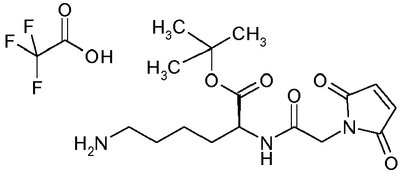

Die Titelverbindung wurde durch Kupplung von (2,5-Dioxo-2,5-dihydro-1H-pyrrol-1-yl)essigsäure mit tert-Butyl-N⁶-(tert-butoxycarbonyl)-L-lysinathydrochlorid(1:1) in Gegenwart von EDC/HOBT und anschließender schonender Abspaltung der tert-Butoxycarbonyl Schutzgruppe in Analogie zu Intermediat L6 hergestellt.
HPLC (Methode 11): Rₜ = 0.42 min;
LC-MS (Methode 1): Rₜ = 0.43 min; MS (ESIpos): m/z = 340 (M+H)⁺.

### Intermediat L14

### Trifluoressigsäure--1-[2-(4-aminopiperazin-1-yl)-2-oxoethyl]-1H-pyrrol-2,5-dion(1:1)

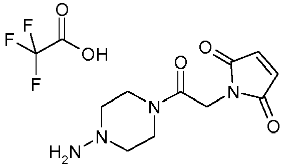

Die Titelverbindung wurde in Analogie zu Intermediat L2 über 2 Stufen aus tert-Butyl-piperazin-1-ylcarbamat und (2,5-dioxo-2,5-dihydro-1H-pyrrol-1-yl)essigsäure hergestellt.
HPLC (Methode 11): Rₜ = 0.2 min;
LC-MS (Methode 3): Rₜ = 0.25 min; MS (ESIpos): m/z = 239 (M+H)⁺.

### Intermediat L15

### Trifluoressigsäure--N-(2-aminoethyl)-3-(2-{2-[2-(2,5-dioxo-2,5-dihydro-1H-pyrrol-1-yl)ethoxy]ethoxy}ethoxy)propanamid(1:1)

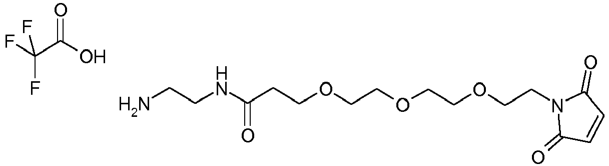

2.93 g (10.58 mmol) tert-Butyl-3-{2-[2-(2-aminoethoxy)ethoxy]ethoxy}propanoat wurden in 100 mL Dioxan/Wasser 1:1 gelöst und mit 3.28 g (21.15 mmol) Methyl-2,5-dioxo-2,5-dihydro-1H-pyrrol-1-carboxylat sowie einer gesättigten Natriumhydrogencarbonatlösung bis zum Erreichen eines pH-Wertes von 6-7 versetzt. Die Lösung wurde 30 min bei RT gerührt und anschließend wurde das 1,4-Dioxan im Vakuum abgedampft. Dann wurden 200 mL Wasser zugegeben und die Mischung dreimal mit jeweils 300 mL Ethylacetat ausgeschüttelt. Die organischen Extrakte wurden vereinigt, über Magnesiumsulfat getrocknet und filtriert. Nach Einengen wurde tert-Butyl-3-(2-{2-[2-(2,5-dioxo-2,5-dihydro-1H-pyrrol-1-yl)ethoxy]ethoxy}ethoxy)propanoat als braunes Öl erhalten, das anschließend im Hochvakuum getrocknet wurde.
HPLC (Methode 11): Rₜ = 1.5 min;
LC-MS (Methode 3): Rₜ = 0.88 min; MS (ESIpos): m/z = 375 (M+NH₄)⁺.

Dieses Intermediat wurde nach Standardverfahren (Entschützung mit TFA, Kupplung mit tert-Butyl-(2-aminoethyl)carbamat und erneute Entschützung mit TFA) in die Titelverbindung überführt.
HPLC (Methode 11): Rₜ = 0.2 min;
LC-MS (Methode 3): Rₜ = 0.25 min; MS (ESIpos): m/z = 344 (M+H)⁺.

### Intermediat L16

### N-[6-(2,5-Dioxo-2,5-dihydro-1H-pyrrol-1-yl)hexanoyl]-L-valyl-N⁵-carbamoyl-L-ornithin

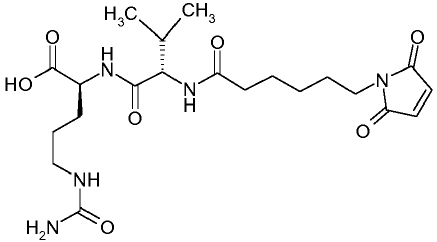

Zu einer Lösung von 266 mg (1.33 mmol) L-Valyl-N5-carbamoyl-L-ornithin in 24 mL DMF wurden 535 mg (1.73 mmol) von kommerziell erhältlichem 1-{6-[(2,5-Dioxopyrrolidin-1-yl)oxy]-6-oxohexyl}-1H-pyrrol-2,5-dion sowie 930 mL *N,N*-Diisopropylethylamin hinzugegeben. Der Ansatz wurde 24 h im Ultraschallbad behandelt und anschließend im Vakuum bis zur Trockene eingeengt. Der verbliebene Rückstand wurde mittels präparativer HPCL gereinigt und nach Einengen der entsprechenden Fraktionen und Trocknung des Rückstandes im Hochvakuum verblieben 337 mg (50% d.Th.) der Titelverbindung.
HPLC (Methode 11): Rₜ = 0.4 min;
LC-MS (Methode 3): Rₜ = 0.58 min; MS (ESIpos): m/z = 468 (M+H)⁺.

### Intermediat L17

### Trifluoressigsäure--tert-butyl-N-[6-(2,5-dioxo-2,5-dihydro-1H-pyrrol-1-yl)hexanoyl]-L-valyl-N⁵-carbamoyl-L-ornithyl-L-lysinat(1:1)

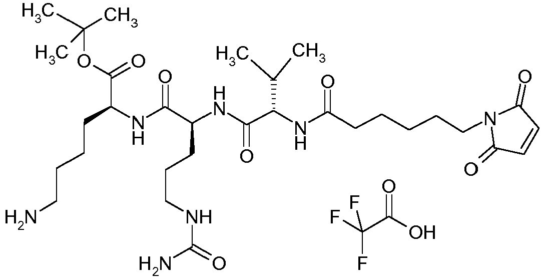

Die Titelverbindung wurde zunächst durch Kupplung von 172 mg (0.37 mmol) Intermediat L16 und 125 mg (0.37 mmol) tert-Butyl-N6-(tert-butoxycarbonyl)-L-lysinat hydrochlorid(1:1) in Gegenwart von EDC/HOBT und *N,N*-Diisopropylethylamin und anschließende Entschützung der Aminogruppe unter schonenden Bedingungen durch 2h Rühren in 10%iger Trifluoressigsäure in DCM bei RT hergestellt. Nach Gefriertrocknung aus Acetonitril/ Wasser wurden 194 mg (49% d.Th.) der Titelverbindung über 2 Stufen erhalten.
HPLC (Methode 11): Rₜ = 1.1 min;
LC-MS (Methode 1): Rₜ = 0.58 min; MS (ESIpos): m/z = 652 (M+H)⁺.

### Intermediat L18

### Trifluoressigsäure--beta-alanyl-L-alanyl-N⁵-carbamoyl-N-[4-(2-methoxy-2-oxoethyl)phenyl]-L-ornithinamid(1:1)

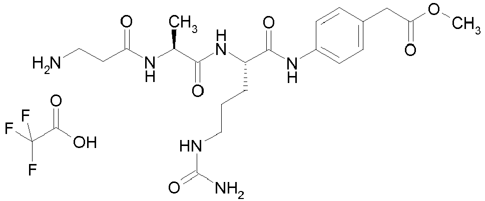

Die Titelverbindung wurde ausgehend von Methyl-(4-aminophenyl)acetat analog Intermediat L7 sequentiell nach klassischen Methoden der Peptidchemie durch Anknüpfung von N²-(tert-Butoxycarbonyl)-N⁵-carbamoyl-L-ornithin in Gegenwart von HATU, Entschützung mit TFA, Kupplung mit 2,5-Dioxopyrrolidin-1-yl-N-(tert-butoxycarbonyl)-L-alaninat, Entschützung mit TFA, Kupplung mit 2,5-Dioxopyrrolidin-1-yl-N-(tert-butoxycarbonyl)-beta-alaninat und erneute Entschützung mit TFA hergestellt. Es wurden 330 mg der Titelverbindung erhalten.
HPLC (Methode 11): Rₜ = 0.29 min;
LC-MS (Methode 1): Rₜ = 0.41 min; MS (ESIpos): m/z = 465 (M+H)⁺.

### Intermediat L19

### Trifluoressigsäure--L-alanyl-N5-carbamoyl-N-(4-{[(2,5-dioxo-2,5-dihydro-1H-pyrrol-1-yl)acetyl]amino}phenyl)-L-ornithinamid(1:1)

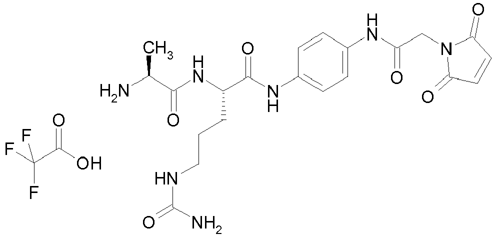

Die Titelverbindung wurde ausgehend von 1,4 Phenylendiamin sequentiell nach klassischen Methoden der Peptidchemie hergestellt. Im ersten Schritt wurden 942 mg (8.72 mmol) 1,4 Phenylendiamin mit 0.8 g (2.9 mmol) N²-(tert-Butoxycarbonyl)-N⁵-carbamoyl-L-ornithin in Gegenwart von HATU und *N,N-*Diisopropylethylamin monoacyliert. Im zweiten Schritt wurde in analoger Weise die zweite anilinische Aminogruppe mit (2,5-Dioxo-2,5-dihydro-1H-pyrrol-1-yl)essigsäure in Gegenwart von HATU und *N,N-*Diisopropylethylamin acyliert. Entschützung mit TFA, Kupplung mit 2,5-Dioxopyrrolidin-1-yl-N-(tert-butoxycarbonyl)-L-alaninat und erneute Entschützung mit TFA führten dann in 3 weiteren Syntheseschritten zur Titelverbindung, von der auf diesem Weg 148 mg erhalten wurden.
LC-MS (Methode 1): Rₜ = 0.21 min; MS (ESIpos): m/z = 474 (M+H)⁺.
LC-MS (Methode 4): Rₜ = 0.2 min; MS (ESIpos): m/z = 474 (M+H)⁺.

### Intermediat L20

### Trifluoressigsäure--L-valyl-N⁵-carbamoyl-N-[4-(2,5-dioxo-2,5-dihydro-1H-pyrrol-1-yl)phenyl]-L-ornithinamid(1:1)

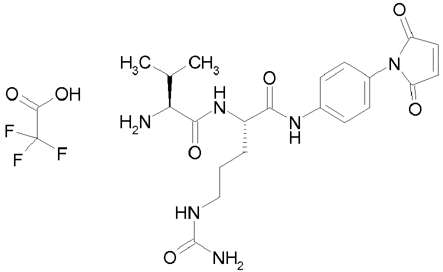

Die Titelverbindung wurde nach klassischen Methoden der Peptidchemie analog zu Intermediat L8 ausgehend von kommerziell erhältlichem 1-(4-Aminophenyl)-1H-pyrrol-2,5-dion durch sequentielle Kupplung mit N²-(tert-Butoxycarbonyl)-N⁵-carbamoyl-L-ornithin in Gegenwart von HATU, Entschützung mit TFA, Kupplung mit 2,5-Dioxopyrrolidin-1-yl-N-(tert-butoxycarbonyl)-L-valinat und erneute Entschützung mit TFA hergestellt. Es wurden 171 mg der Titelverbindung erhalten.
HPLC (Methode 11): Rₜ = 0.28 min;
LC-MS (Methode 1): Rₜ = 0.39 min; MS (ESIpos): m/z = 445 (M+H)⁺.

### Intermediat L21

### L-Valyl-N⁶-(tert-butoxycarbonyl)-N-[4-(2-methoxy-2-oxoethyl)phenyl]-L-lysinamid

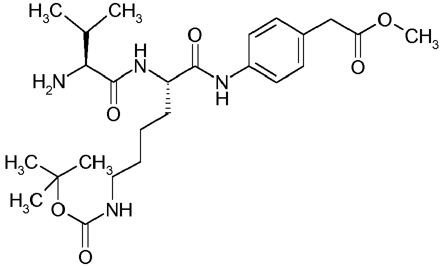

Die Titelverbindung wurde nach klassischen Methoden der Peptidchemie ausgehend von kommerziell erhältlichem 0.42 g (2.56 mmol) Methyl-(4-aminophenyl)acetat durch sequentielle Kupplung mit N6-(tert-Butoxycarbonyl)-N2-[(9H-fluoren-9-ylmethoxy)carbonyl]-L-lysin in Gegenwart von HATU, und *N,N*-Diisopropylethylamin, Entschützung mit Piperidin, Kupplung mit 2,5-Dioxopyrrolidin-1-yl-N-[(benzyloxy)carbonyl]-L-valinat in Gegenwart von *N,N*-Diisopropylethylamin und anschließende hydrogenolytische Abspaltung der Benzyloxycarbonylschutzgruppe über 10%-Palladium-Aktivkohle. Es wurden 360 mg (32% d.Th. über 4 Stufen) der Titelverbindung erhalten.
HPLC (Methode 11): Rₜ = 1.5 min;
LC-MS (Methode 1): Rₜ = 0.73 min; MS (ESIpos): m/z = 493 (M+H)⁺.

### Intermediat L22

### Trifluoressigsäure--N-[(9H-fluoren-9-ylmethoxy)carbonyl]-L-valyl-N-{4-[(2S)-2-amino-3-methoxy-3-oxopropyl]phenyl}-N⁵-carbamoyl-L-ornithinamid(1:1)

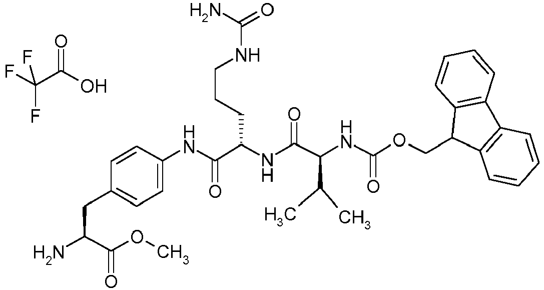

Die Titelverbindung wurde ausgehend von N-(tert-Butoxycarbonyl)-4-nitro-L-phenylalanin sequentiell nach klassischen Methoden der Peptidchemie hergestellt. 2.5 g (8.06 mmol) von diesem Edukt wurde im ersten Schritt zunächst in das Caesiumsalz und dann mit Iodmethan in DMF in den Methylester überführt.

Hydrogenolytisch wurde dann in Methanol über 10% Palladium-Aktivkohle die Nitrogruppe in eine Aminogruppe überführt.

Die so generierte Aminogruppe wurde dann mit N5-Carbamoyl-N2-[(9H-fluoren-9-ylmethoxy)carbonyl]-L-ornithin in DMF in Gegenwart von HATU und *N,N*-Diisopropylethylamin acyliert. Im nächsten Schritt wurde die Fmoc-Gruppe mit Piperidin in DMF abgespalten.

Anschließend erfolgte in DMF die Kupplung mit N-[(9H-Fluoren-9-ylmethoxy)carbonyl]-L-valin in Gegenwart von 1-(3-Dimethylaminopropyl)-3-ethylcarbodiimid-Hydrochlorid, 1-Hydroxy-1*H-*benzotriazol-Hydrat und *N,N*-Diisopropylethylamin und schließlich die Abspaltung der tert-Butoxycarbonylgruppe mit Trifluoressigsäure.
HPLC (Methode 11): Rₜ = 1.6 min;
LC-MS (Methode 1): Rₜ = 0.77 min; MS (ESIpos): m/z = 673 (M+H)⁺.

### Intermediat L23

### Trifluoressigsäure--N-[2-(2,5-dioxo-2,5-dihydro-1H-pyrrol-1-yl)ethyl]-beta-alaninamid(1:1)

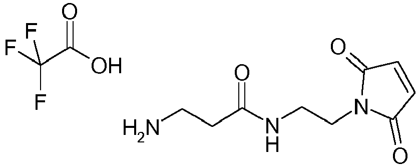

Die Titelverbindung wurde ausgehend von kommerziell erhältlichem Trifluoressigsäure--1-(2-aminoethyl)-1H-pyrrol-2,5-dion(1:1) durch Kupplung mit N-(tert-Butoxycarbonyl)-beta-alanin in Gegenwart von EDCI/HOBT und *N,N*-Diisopropylethylamin und anschließende Entschützung mit Trifluoressigsäure hergestellt.
HPLC (Methode 11): Rₜ = 0.19 min.

### Intermediat L24

### Trifluoressigsäure--1-amino-N-[2-(2,5-dioxo-2,5-dihydro-1H-pyrrol-1-yl)ethyl] cyclopropancarboxamid(1:1)

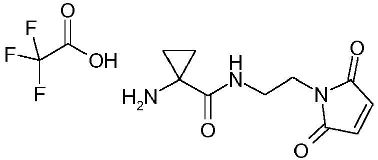

114 mg (0.67 mmol) von kommerziell erhältlicher 1-[(tert-Butoxycarbonyl)amino]cyclopropan carbonsäure wurden in 25 mL DCM gelöst und mit 110 mg (0.623 mmol) von kommerziell erhältlichem Trifluoressigsäure--1-(2-aminoethyl)-1H-pyrrol-2,5-dion(1:1) sowie mit 395 µl *N,N-*Diisopropylethylamin versetzt und auf -10°C abgekühlt. Dann wurden 217 mg (0.793 mmol) 2-Brom-1-ethylpyridiniumtetrafluoroborat zugegeben und der Ansatz 2 h bei RT gerührt. Anschließend wurde mit Ethylacetat verdünnt und nacheinander mit 10%-iger Zitronensäure, gesättigter Natriumhydrogencarbonatlösung un gesättigter Natriumchloridlösubng ausgeschüttelt, dann über Magnesiumsulfat getrocknet und eingeengt. Nach Trocknung im Hochvakuum wurden 152 mg des geschützten Intermediats erhalten.

Diese wurden anschließend in 10 mL DCM aufgenommen und mit 1 mL Trifluoressigsäure entschützt. Nach Lyophilisation aus Acetonitril/Wasser wurden 158 mg (71% d.Th. über 2 Stufen) der Titelverbindung erhalten.
HPLC (Methode 11): Rₜ = 0.19 min.
LC-MS (Methode 3): Rₜ = 0.98 min; MS (ESIpos): m/z = 224 (M+H)⁺.

### Intermediat L25

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-L-alanin

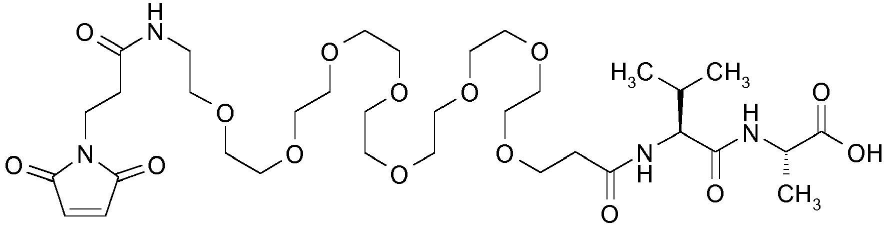

31.4 mg (0.17 mmol) Valyl-L-alanin wurden in 3.0 mL DMF gelöst und mit 115.0 mg (0.17 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid und mit 33.7 mg (0.0.33 mmol) Triethylamin versetzt. Es wurde über Nacht bei RT gerührt. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 74.1 mg (58 % d. Th.) der Titelverbindung.
LC-MS (Methode 1): Rₜ = 0.61 min; MS (ESIpos): m/z = 763 [M+H]⁺.

### Intermediat L26

### L-Valyl-N6-(tert-butoxycarbonyl)-L-lysin

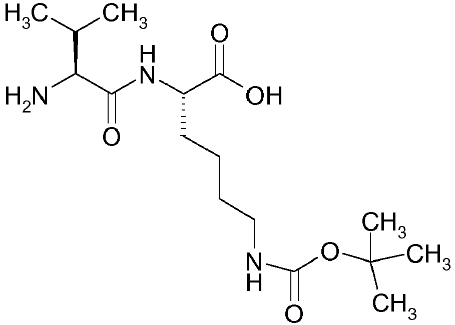

600.0 mg (1.58 mmol) N2-[(Benzyloxy)carbonyl]-N6-(tert-butoxycarbonyl)-L-lysin wurden in 25.0 mL Wasser/Ethanol/THF (1:1:0.5) suspendiert und mit Palladium auf Kohle (10%ig) versetzt und bei RT mit Wasserstoff unter Normaldruck 5 h hydriert. Der Katalysator wurde abfiltriert und die Lösemittel im Vakuum verdampft. Die erhaltene Verbindung wurde ohne weitere Reinigung in der nächsten Stufe eingesetzt.
LC-MS (Methode 1): Rₜ = 0.42 min; MS (ESIpos): m/z = 247 [M+H]⁺.

180 mg (0.73 mmol) N6-(tert-Butoxycarbonyl)-L-lysin wurden in 5.0 mL DMF gelöst und mit 74.0 mg (0.73 mmol) Triethylamin versetzt. Dann wurden 254.6 mg (0.73 mmol) 2,5-Dioxopyrrolidin-1-yl-N-[(benzyloxy)carbonyl]-L-valinat und 74.0 mg (0.73 mmol) Triethylamin zugegeben. Die Reaktionsmischung wurde 3.5 h bei RT gerührt. Die Reaktionslösung wurde direkt über die präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 294.1 mg (76 % d. Th.) N-[(Benzyloxy)carbonyl]-L-valyl-N6-(tert-butoxycarbonyl)-L-lysin.
LC-MS (Methode 1): Rₜ = 0.97 min; MS (ESIpos): m/z = 480 [M+H]⁺.

272.2 mg (0.57 mmol) N-[(Benzyloxy)carbonyl]-L-valyl-N6-(tert-butoxycarbonyl)-L-lysin wurden in 20.0 mL Ethylacetat/Ethanol/THF (1:1:1) vorgelegt und mit 27.2 mg Palladium auf Aktivkohle versetzt. Man hydrierte mit Wasserstoff bei RT unter Normaldruck 5 h. Es wurde mit Hilfe von Celite^{(R)} abfiltriert und der Filterkuchen mit Ethylacetat/Ethanol/THF (1:1:1) nachgewaschen. Die Lösemittel wurden im Vakuum verdampft und der Rückstand im Hochvakuum getrocknet. Die Titelverbindung (182 mg, 72 % d. Th.) wurde in der nächsten Reaktionsstufe ohne weitere Reinigung eingesetzt.
LC-MS (Methode 1): Rₜ = 0.53 min; MS (ESIpos): m/z = 346 [M+H]⁺.

### Intermediat L27

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N6-(tert-butoxycarbonyl)-L-lysin

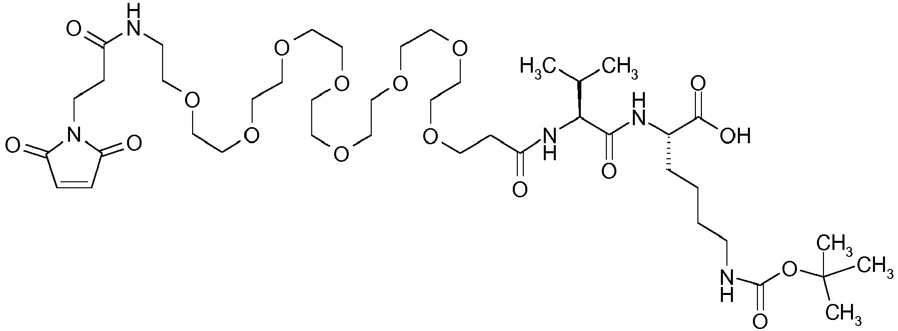

30 mg (0.07 mmol) L-Valyl-N6-(tert-butoxycarbonyl)-L-lysin (Intermediat L26)
und 46.1 mg (0.07 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid wurden in 1.5 mL DMF vorgelegt und mit 6.8 mg (0.07 mmol) 4-Methylmorpholin versetzt. Die Reaktionslösung rührte bei RT über Nacht. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 55.6 mg (90 % d. Th.) der Titelverbindung.
LC-MS (Methode 1): Rₜ = 0.77 min; MS (ESIpos): m/z = 920 [M+H]⁺.

### Intermediat L28

### tert-Butyl-3-formyl-4-({[2-(trimethylsilyl)ethoxy]carbonyl}amino)pyrrolidin-1-carboxylat

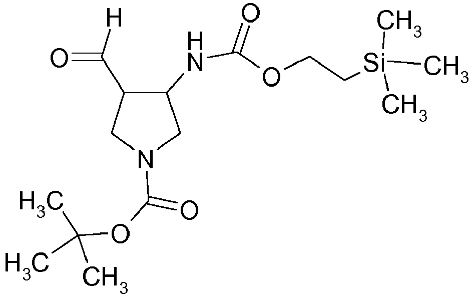

461.7 mg (1.15 mmol) 1-tert-Butyl-3-ethyl-4-({[2-(trimethylsilyl)ethoxy]carbonyl}amino)pyrrolidin-1,3-dicarboxylat (Diese Verbindung wurde gemäß Literaturvorschrift WO 2006/066896 hergestellt) wurden in 5.0 mL absol. Dichlormethan vorgelegt und auf -78 °C abgekühlt. Dann wurde langsam 326.2 mg (2.29 mmol) Diisobutylaluminiumhydrid-Lösung (1 M in THF) zugetropft und 2 h bei -78 °C gerührt (Dünnschichtchromatographische Kontrolle (Petrolether/Ethylacetat = 3:1). Es wurden 1.3 g (4.59 mmol) Kaliumnatriumtartrat gelöst in 60 mL Wasser zugetropft und die Reaktionsmischung auf RT kommen gelassen. Die Reaktionsmischung wurde mit Ethylacetat versetzt und die wässrige Phase dreimal mit Ethylacetat extrahiert. Die vereinigten organischen Phasen wurden einmal mit ges. NaCl-Lösung gewaschen und über Magnesiumsulfat getrocknet. Das Lösemittel wurde im Vakuum verdampft und der Rückstand im Hochvakuum getrocknet. Man erhielt 629.0 mg der Titelverbindung als Rohprodukt, welches ohne weitere Reinigung sofort in der nächsten Reaktionsstufe eingesetzt wurde.

### Intermediat L29

### tert-Butyl-3-formyl-4-[({[2-(trimethylsilyl)ethoxy]carbonyl}amino)methyl]pyrrolidin-1-carboxylat Mischung aus Diastereomeren.

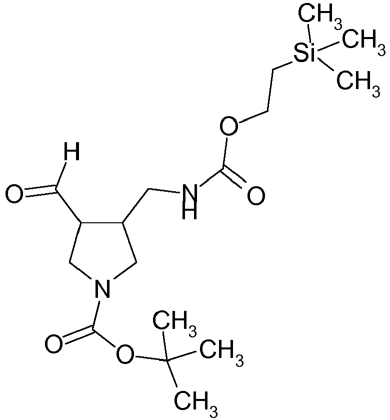

807.1 mg (2.34 mmol) tert-Butyl-3-({[tert-butyl(dimethyl)silyl]oxy}methyl)-4-(hydroxymethyl)pyrrolidin-1-carboxylat (Die Herstellung erfolgte gemäß der Literaturvorschrift WO 2006/100036) wurden in 8.0 mL Dichlormethan vorgelegt und 236.4 mg (2.34 mmol) Triethylamin zugegeben. Bei 0 °C wurden 267.6 mg (2.34 mmol) Methansulfonsäurechlorid zugetropft und die Reaktionsmischung über Nacht bei RT gerührt. Es erfolgte eine weitere Zugabe von 133.8 mg (1.17 mmol) Methansulfonsäurechlorid und 118.2 mg (1.17 mmol) Triethylamin. Die Reaktionsmischung wurde über Nacht bei RT gerührt. Es wurde mit Dichlormethan verdünnt und die organische Phase je einmal mit ges. Natriumhydrogencarbonat-Lösung, 5%iger Kaliumhydrogensulfat-Lösung und ges. NaCl-Lösung gewaschen. Nach der Trocknung über Magnesiumsulfat wurde das Lösemittel im Vakuum verdampft und der Rückstand wurde mittels Biotage Isolera gereinigt (Kieselgel, Säule 50 g SNAP, Fluss 66 mL/min, Cyclohexan/Ethylacetat). Die Lösemittel wurden im Vakuum verdampft und der Rückstand im Hochvakuum getrocknet. Man erhielt 402.0 mg (41 % d. Th.) der Verbindung tert-Butyl-3-({[tert-butyl(dimethyl)silyl]oxy}methyl)-4-{[(methylsulfonyl)oxy]methyl}pyrrolidin-1-carboxylat
LC-MS (Methode 1): Rₜ = 1.38 min; MS (ESIpos): m/z = 424 [M+H]⁺.

400.0 mg (0.94 mmol) tert-Butyl-3-({[tert-butyl(dimethyl)silyl]oxy}methyl)-4-{[(methylsulfonyl)oxy]methyl}pyrrolidin-1-carboxylat wurden in 5.0 mL DMF vorgelegt und mit 98.2 mg (1.51 mmol) Natriumazid versetzt. Die Reaktionsmischung wurde 10 h bei 40 °C gerührt. Dann wurden nochmals 30.7 mg (0.47 mmol) Natriumazid zugegeben und weitere 10 h bei 40 °C gerührt. Es wurde Ethylacetat zugegeben und die organsiche Phase mehrmals mit Wasser gewaschen. Nach der Trocknung der organischen Phase über Magnesiumsulfat wurde das Lösemittel im Vakuum verdampft und der Rückstand im Hochvakuum getrocknet. Man erhielt 309.5 mg (89 % d. Th.) der Verbindung tert-Butyl-3-(azidomethyl)-4-({[tert-butyl(dimethyl)silyl]oxy}methyl)pyrrolidin-1 -carboxylat. Die Verbindung wurde ohne weitere Reinigung in der nächsten Synthesestufe eingesetzt.
LC-MS (Methode 1): Rₜ = 1.50 min; MS (ESIpos): m/z = 371 [M+H]⁺.

250 mg (0.68 mmol) tert-Butyl-3-(azidomethyl)-4-({[tert-butyl(dimethyl)silyl]oxy}methyl)pyrrolidin-1-carboxylat wurden in 10.0 mL Ethylacetat/Ethanol (1:1) gelöst und mit 25.0 mg Palladium auf Aktivkohle (10%) versetzt. Man hydrierte mit Wasserstoff bei RT unter Normaldruck 8 h. Die Reaktion wurde über Celite^{(R)} filtiert und der Filterkuchen intensiv mit Ethylacetat gewaschen. Das Lösemittel wurde im Vakuum verdampft und der Rückstand im Hochvakuum getrocknet. Man erhielt 226.2 mg (82 % d. Th.) der Verbindung tert-Butyl-3-(aminomethyl)-4-({[tert-butyl(dimethyl)silyl]oxy}methyl)pyrrolidin-1-carboxylat. Die Verbindung wurde ohne weitere Reinigung in der nächsten Synthesestufe eingesetzt.
LC-MS (Methode 1): Rₜ = 0.89 min; MS (ESIpos): m/z = 345 [M+H]⁺.

715.0 mg (2.08 mmol) tert-Butyl-3-(aminomethyl)-4-({[tert-butyl(dimethyl)silyl]oxy}methyl)pyrrolidin-1-carboxylat wurden in 15.0 mL THF gelöst und mit 2.28 mL (2.28 mmol) TBAF-Lösung (1M in THF) versetzt. Die Reaktionsmischung wurde bei RT über Nacht gerührt. Das Lösemittel wurde im Vakuum verdampft und der Rückstand (1.54 g) ohne weitere Reinigung in der nächsten Synthesestufe eingesetzt.
LC-MS (Methode 1): Rₜ = 0.41 min; MS (ESIpos): m/z = 231 [M+H]⁺.

1.54 g (4.88 mmol) tert-Butyl-3-(aminomethyl)-4-(hydroxymethyl)pyrrolidin-1-carboxylat wurden in 1,4-Dioxan vorgelegt mit 541.8 mg (4.88 mmol) Calciumchlorid (wasserfrei) und 488.6 mg (4.88 mmol) Calciumcarbonat versetzt und kräftig gerührt. Dann wurden 592.8 mg (5.86 mmol) Triethylamin und 1.52 g (5.86 mmol) 1-({[2-(Trimethylsilyl)ethoxy]carbonyl}oxy)pyrrolidin-2,5-dion zugegeben und die Reaktionsmischung über Nacht bei RT gerührt. Es wurden 644.9 mg (10.7 mmol) HOAc und Ethylacetat zugegeben. Die organische Phase wurde zweimal mit Wasser und einmal mit ges. NaCl-Lösung gewaschen. Nach der Trocknung über Magnesiumsulfat wurde das Lsöemittel im Vakuum verdampft und der Rückstand mittels Kieselgel (Laufmittel: Dichlormethan/Methanol = 100:1) gereinigt. Die Lösemittel wurden im Vakuum verdampft und der Rückstand im Hochvakuum getrocknet. Man erhielt 346.9 mg (19 % d. Th.) der Verbindung tert-Butyl-3-(hydroxymethyl)-4-[({[2-(trimethylsilyl)ethoxy]carbonyl}amino)methyl]pyrrolidin-1-carboxylat.
LC-MS (Methode 1): Rₜ = 1.08 min; MS (ESIpos): m/z = 375 [M+H]⁺.

804.0 mg (2.15 mmol) tert-Butyl-3-(hydroxymethyl)-4-[({[2-(trimethylsilyl)ethoxy]carbonyl}amino)methyl]pyrrolidin-1-carboxylat wurden in 20.0 mL Chloroform und 20.0 mL 0.05 N Kaliumcarbonat/0.05 N Natriumhydrogencarbonat-Lösung (1:1) vorgelegt. Dann wurden 59.7 mg (0.22 mmol) Tetra-n-butylammoniumchlorid, 429.9 mg (3.22 mmol) N-Chlorsuccinimid und 33.5 mg (0.22 mmol) TEMPO zugegeben und die Reaktionsmischung kräftig über Nacht bei RT gerührt. Die organische Phase wurde abgetrennt und im Vakuum vom Lösemittel befreit. Der Rückstand wurde mittels Kieselgel (Lausfmittel: Cyclohexan/Ethylacetat = 3:1) gereinigt. Man erhielt 517.0 mg (46 % d. Th.) der Titelverbindung.
LC-MS (Methode 1): Rₜ = 1.13 min; MS (ESIpos): m/z = 373 [M+H]⁺.

### Intermediat L30

### tert-Butyl-3-({[tert-butyl(dimethyl)silyl]oxy}methyl)-4-formylpyrrolidin-1-carboxylat

### Mischung aus Stereoisomeren

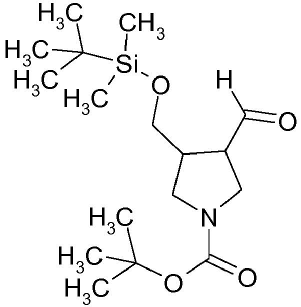

250.0 mg (0.72 mmol) tert-Butyl-3-({[tert-butyl(dimethyl)silyl]oxy}methyl)-4-(hydroxymethyl)pyrrolidin-1-carboxylat (Die Verbindung wurde gemäß der Literaturvorschrift WO2006/100036 hergestellt.) wurde in 12.5 mL Dichlormethan/DMSO (4:1) vorgelegt und mit 219.6 mg (2.17 mmol) Triethylamin versetzt. Bei 2 °C wurde 345.5 mg (2.17 mmol) Schwefeltrioxid-Pyridin-Komplex portionsweise zugegeben und 3 h bei 2 °C gerührt. Es wurden nochmals 345.5 mg (2.17 mmol) Schwefeltrioxid-Pyridin-Komplex portionsweise zugegeben und 17 h bei RT gerührt. Die Reaktionsmischung wurde zwischen Dichlormethan und Wasser verteilt. Die wässrige Phase wurde dreimal mit Dichlormethan extrahiert und die vereinigten organischen Phasen einmal mit Wasser gewaschen und über Magnesiumsulfat getrocknet. Das Lösemittel wurde im Vakuum verdampft und der Rückstand im Hochvakuum getrocknet. Die Rückstand wurde ohne weitere Reinigung in der nächsten Synthesestufe eingesetzt (Dünnschichtchromatographie: Petrolether/Ethylacetat 7:3).

### Intermediat L31

### Di-tert-butyl-{[(tert-butoxycarbonyl)amino]methyl}malonat

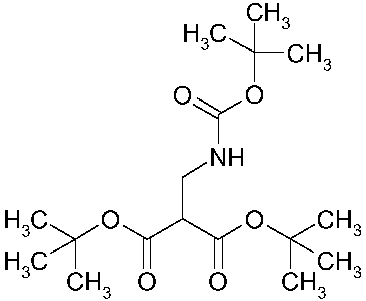

Zu 600 mL Wasser wurden 57.2 g (488.27 mmol) tert-Butylcarbamat, 51.2 mL (683.57 mmol) einer 37%-igen Lösung von Formaldehyd in Wasser und 25.9 g (244.13 mmol) Natriumcarbonat addiert. Das Gemisch wurde erwärmt, bis eine Lösung entstand und dann bei RT für 16 h gerührt. Die entstandene Suspension wurde mit 500 mL Dichlormethan extrahiert, die organische Phase abgetrennt, mit gesättigter Natriumchlorid-Lösung gewaschen und über Natriumsulfat getrocknet. Es wurde am Rotationsverdampfer eingeengt und der Rückstand im Hochvakuum getrocknet, wobei ein kristalliner Feststoff anfällt. Der Rückstand wurde in 1000 mL absoluten THF aufgenommen und bei RT ein Gemisch aus 322 mL (3.414 mol) Essigsäureanhydrid und 138 mL (1.707 mol) Pyridin zugetropft. Die Reaktionsmischung wurde bei RT für 16 h gerührt und anschließend am Rotationsverdampfer eingenegt, wobei das Wasserbad Raumtemperatur hatte. Der Rückstand wurde in Diethylether aufgenommen und drei mal mit einer gesättigten Natriumhydrogencarbonat-Lösung sowie ein mal mit einer gesättigten Natriumchlorid-Lösung gewaschen. Die organische Phase wurde über Natriumsulfat getrocknet, am Rotationsverdampfer eingeengt und der Rückstand 2 d im Hochvakuum getrocknet. Der Rückstand wurde in 2000 mL absolutem THF aufgenommen und unter Eiskühlung mit 456 mL (456.52 mmol) einer 1 M Lösung von Kalium-tert-butanolat in THF versetzt. Es wurde 20 Min. bei 0°C gerührt und anschließend 100.8 g (456.52 mmol) Di-tert-butylmalonat gelöst in 200 mL absolutem THF zugetropft. Es wurde 48 h bei RT gerührt und anschließend Wasser addiert. Das Reaktionsgemisch wurde am Rotationsverdampfer eingeengt und in 500 mL Essigsäureethylester aufgenommen. Das Gemisch wurde mit 500 mL Wasser und 100 mL einer gesättigten Natriumchlorid-Lösung gewaschen und die organische Phase über Natriumsulfat getrocknet. Die organische Phase wurde am Rotationsverdampfer eingeengt und der Rückstand im Hochvakuum getrocknet. Der Rückstand wurde mittels Filtration über Kieselgel (Eluent: Cylohexan/Essigsäureethylester, Gradient = 30:1 → 5:1) gereinigt. Es wurden 37.07 g (22% d. Th.) der Zielverbindung erhalten.
LC-MS (Methode 6): Rₜ = 2.87 min; MS (ESIpos): m/z = 346 [M+H]⁺.

### Intermediat L32

### tert-Butyl-[3-hydroxy-2-(hydroxymethyl)propyl]carbamat

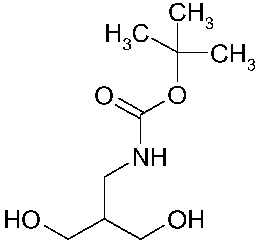

37.0 g (107.11 mmol) Di-tert-butyl-(acetoxymethyl)malonat wurden in 1000 mL absolutem THF gelöst und unter Eiskühlung mit 535.5 mL (1071.10 mmol) einer 2 M Lösung von Lithiumborhydrid in THF tropfenweise versetzt. Es wurden 19.3 mL (1071.10 mmol) Wasser zugetropft und 4.5 h bei RT gerührt. Das Reaktionsgemisch wurde am Rotationsverdampfer eingeengt und im Hochvakuum getrocknet. Der Rückstand wurde in 1500 mL Essigsäureethylester aufgenommen und mit 100 mL Wasser versetzt und 30 Min. unter Wasserkühlung gerührt (leicht exotherm). Die organische Phase wurde abgetrennt und die wässrige Phase zwei mal mit 500 mL Essigsäureethylester extrahiert. Die organische Phase wurde am Rotationsverdampfer eingeengt und der Rückstand im Hochvakuum getrocknet. Es wurden 20.7 g (94% d. Th.) der Zeilverbindung erhalten.
LC-MS (Methode 6): Rₜ = 1.49 min; MS (EIpos): m/z = 106 [M-C₅H₈O₂]⁺.

### Intermediat L33

### tert-Butyl-[3-{[tert-butyl(dimethyl)silyl]oxy}-2-(hydroxymethyl)propyl]carbamat

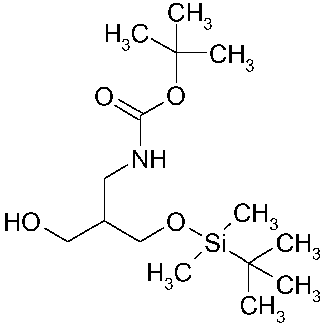

20.00 g (97.44 mmol) tert-Butyl-[3-hydroxy-2-(hydroxymethyl)propyl]carbamat wurden in 1000 mL absolutem Dichlormethan gelöst und bei RT mit 6.63 g (97.44 mmol) Imidazol sowie 16.16 g (107.18 mmol) tert-Butyl(chlor)dimethylsilan versetzt. Es wurde 16 h bei RT gerührt und das Reaktionsgemisch mit halbkonzentrierter Natriumchlorid-Lösung gewaschen. Die wässrige Phase wurde mit Essigsäureethylester extrahiert und die vereinigten oranischen Phasen über Natriumsulfat getrocknet, am Rotationsverdampfer eingeengt und im Hochvakuum getrocknet. Es wurden 28.50 g (92% d. Th.) der Zielverbindung erhalten.
¹H-NMR (400 MHz, DMSO-d₆): δ [ppm] = 0.02 (s, 6H), 0.86 (s, 9H), 1.37 (s, 9H), 1.58-1.73 (m, 1H), 2.91 (q, 2H), 3.33-3.36 [m, (2H, verdeckt)], 3.53-3.58 (m, 2H), 6.65-6.72 (m, 1H).

### Intermediat L34

### tert-Butyl-(3-{[tert-butyl(dimethyl)silyl]oxy}-2-formylpropyl)carbamat

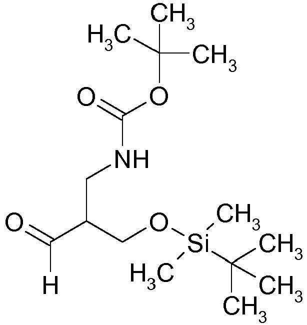

12.65 g (39.591 mmol) tert-Butyl-[3-{[tert-butyl(dimethyl)silyl]oxy}-2-(hydroxymethyl)propyl]carbamat wurden in 200 mL Dichlormethan gelöst und bei RT mit und mit 19.31 g (45.53 mmol) Dess-Martin-Periodinan gelöst in 150 mL Dichlormethan tropfenweise versetzt. Das Gemisch wurde 2 h bei Raumtemperatur gerührt und anschließend mit 250 mL einer halbkonzentrierten Natriumhydrogencarbonat-Lösung sowie mit 250 mL einer 10%-igen Natriumthiosulfat-Lösung versetzt, und für 20 Min. gerührt. Die organische Phase wurde abgetrennt und die wässrige Phase mit Essigsäureethylester extrahiert. Die vereinigten organischen Phasen wurden mit 300 mL Wasser gewaschen, über Natriumsulfat getrocknet, am Rotationsverdampfer eingeengt und im Hochvakuum getrocknet. Es wurden 11.35 g (90% d. Th.) der Zielverbindung erhalten.
¹H-NMR (400 MHz, DMSO-d₆): δ [ppm] = 0.02 (s, 6H), 0.84(s, 9H), 1.36 (s, 9H), 1.48-1.51 (m, 1H), 3.08-3.32 [m, (1H, verdeckt)], 3.50-3.58 (m, 2H), 3.81-3.91 (m, 1H), 6.71 (t, 1H), 9.60 (d, 1H).

### Intermediat L35

### tert-Butyl-(3-oxopropyl)carbamat

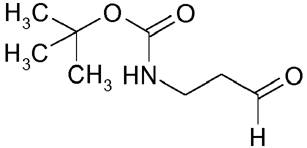

Die Titelverbindung wurde nach literaturbekannten Verfahren hergestellt (z.B. Jean Bastide et al. J. Med. Chem. 2003, 46(16), 3536-3545).

### Intermediat L36

### N-[(Benzyloxy)carbonyl]-L-valyl-N5-carbamoyl-L-ornithin

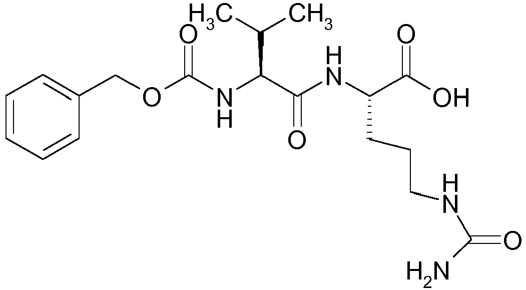

100 mg (0.57 mmol) N5-Carbamoyl-L-ornithin wurden in 4.0 mL DMF aufgenommen und mit 0.08 mL (0.57 mmol) Triethylamin versetzt. Dann wurden 199.0 mg (0.57 mmol) 2,5-Dioxopyrrolidin-1-yl-N-[(benzyloxy)carbonyl]-L-valin und 0.08 mL (0.57 mmol) Triethylamin zugesetzt. Es wurde 48 h bei RT gerührt. Die Reaktionsmischung wurde direkt über die präp. RP-HPLC gereinigt (Säule: Reprosil 250x30; 10µ, Fluss: 50 mL/min, MeCN/Wasser mit 0.1 % TFA). Die Lösemittel wurden im Vakuum verdampft und der Rückstand im Hochvakuum getrocknet. Man erhielt 75.7 mg (33 % d. Th.) der Titelverbindung.
LC-MS (Methode 1): Rₜ = 0.69 min; MS (ESIpos): m/z = 409 [M+H]⁺.

### Intermediat L37

### L-Valyl-N5-carbamoyl-L-ornithin

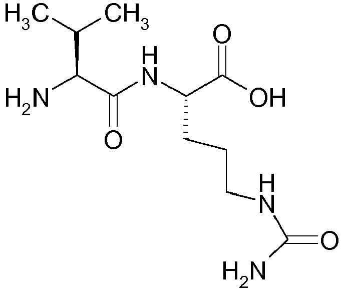

75.7 mg (0.19 mmol) von Intermediat L36 wurden in 25 mL Wasser/Ethanol/THF suspendiert und mit 7.5 mg Palladium auf Aktivkohle (10%ig) versetzt und bei RT 4.5 h lang mit Wasserstoff unter Normaldruck hydriert. Der Katalysator wurde abfiltriert und die Reaktionsmischung im Vakuum vom Lösemittel befreit und im Hochvakuum getrocknet. Der Rückstand wurde ohne weitere Reinigung in der nächsten Stufe eingesetzt. Man erhielt 64.9 mg (93 % d. Th.) der Titelverbindung.
LC-MS (Methode 6): Rₜ = 0.25 min; MS (ESIpos): m/z = 275 [M+H]⁺.

### Intermediat L38

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N5-carbamoyl-L-ornithin

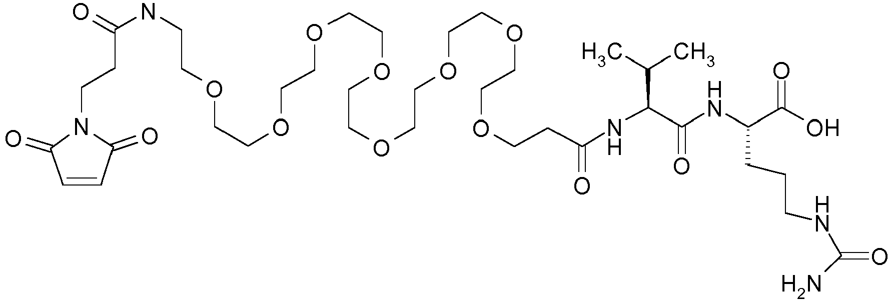

38.3 mg (0.14 mmol) von Intermediat L37 wurden in 3.0 mL DMF vorgelegt und mit 96.4 mg (0.14 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid und mit 39.0 µL (0.28 mmol) Triethylamin versetzt. Es wurde über Nacht bei RT gerührt. Die Reaktionsmischung wurde anschließend mit 16.0 µL (0.28 mmol) HOAc versetzt und direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 58.9 mg (45 % d. Th.) der Titelverbindung.
LC-MS (Methode 1): Rₜ = 0.61 min; MS (ESIpos): m/z = 849 [M+H]⁺.

### Intermediat L39

### 2-(Trimethylsilyl)ethyl-(2-sulfanylethyl)carbamat

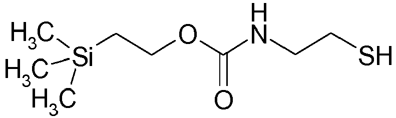

300 mg (2.64 mmol) 2-Aminoethanthiolhydrochlorid (1:1) wurden in 3.0 mL Dichlormethan vorgelegt und mit 668.0 mg (6.60 mmol)Triethylamin mit 719.1 mg (2.77 mmol) 1-({[2-(Trimethylsilyl)ethoxy]carbonyl}oxy)pyrrolidin-2,5-dion versetzt. Es wurde 2 Tage bei RT gerührt. (Dünnschichtchromatographische Kontrolle: Dichlormethan/Methanol = 100:1.5) Die Reaktionsmischung wurde mit Ethylacetat versetzt und dreimal mit Wasser gewaschen. Die organische Phase wurde zweimal mit ges. NaCl-Lösung gewaschen und über Magnesiumsulfat getrocknet. Das Lösemittel wurde im Vakuum verdampft und der Rückstand im Hochvakuum getrocknet. Einsatz der Verbindung ohne weitere Reinigung in der nächsten Synthesestufe.

### Intermediat L40

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N6-(tert-butoxycarbonyl)-L-lysin

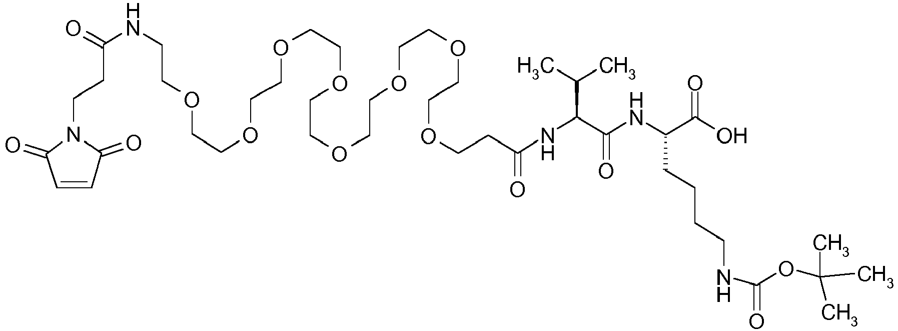

600 mg (1.58 mmol) N2-[(Benzyloxy)carbonyl]-N6-(tert-butoxycarbonyl)-L-lysin wurden in 25.0 mL Wasser/Ethanol/THF (1:1:0.5) mittels Palladium auf Kohle (10 %) bei RT unter Normaldruck mit Wasserstoff hydriert. Die Verbindung N6-(tert-Butoxycarbonyl)-L-lysin wird ohne weitere Reinigung in der nächsten Synthesestufe eingesetzt.
LC-MS (Methode 1): Rₜ = 0.99 min; MS (ESIpos): m/z = 247 [M+H]⁺.

180.0 (0.73 mmol) N6-(tert-Butoxycarbonyl)-L-lysin wurden in 5.0 mL DMF gelöst und mit 74.0 mg (0.73 mmol) Triethylamin versetzt. Es wurden 254.6 mg (0.73 mmol) 2,5-Dioxopyrrolidin-1-yl-N-[(benzyloxy)carbonyl]-L-valinat und 74.0 mg (0.73 mmol) Triethylamin zugegeben. Die Reaktionsmischung wurde 3.5 h bei RT gerührt. Die Reaktionsmischung wurde direkt mittles präp. RP-HPLC gereinigt (Säule: Reprosil 250x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 294.1 mg (76 % d. Th.) der Verbindung N-[(Benzyloxy)carbonyl]-L-valyl-N6-(tert-butoxycarbonyl)-L-lysin.
LC-MS (Methode 1): Rₜ = 0.97 min; MS (ESIpos): m/z = 480 [M+H]⁺.

272.2 mg (0.57 mmol) N-[(Benzyloxy)carbonyl]-L-valyl-N6-(tert-butoxycarbonyl)-L-lysin wurden in 20 mL Ethylacetat/Ethanol/THF (1:1:1) gelöst und mit 27.2 mg Palladium auf Aktivkohle versetzt und unter Normaldruck und bei RT mit Wasserstoff hydriert. Es wurde über Celite^{(R)} filtriert und der Filterkuchen intensiv mit Ethylacetat/Ethanol/THF (1:1:1) gewaschen. Die Lösemittel wurden im Vakuum verdampft und der Rückstand im Hochvakuum getrocknet. Man erhielt 182.0 mg (72 % d. Th.) der Verbindung L-Valyl-N6-(tert-butoxycarbonyl)-L-lysin.
LC-MS (Methode 1): Rₜ = 0.53 min; MS (ESIpos): m/z = 346 [M+H]⁺.

30.0 mg (0.07 mmol) L-Valyl-N6-(tert-butoxycarbonyl)-L-lysin und 46.1 mg (0.07 mmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid wurden in 1.5 mL DMF gelöst und mit 6.8 mg (0.07 mmol) 4-Methylmorpholin versetzt. Dir Reaktionsmischung wurde über Nacht bei RT gerührt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 55.6 mg (90 % d. Th.) der Titelverbindung.
LC-MS (Methode 1): Rₜ = 0.77 min; MS (ESIpos): m/z = 920 [M+H]⁺.

### Intermediat L41

### N-[19-(2,5-Dioxo-2,5-dihydro-1H-pyrrol-1-yl)-17-oxo-4,7,10,13-tetraoxa-16-azanonadecan-1-oyl]-L-valyl-N6-(tert-butoxycarbonyl)-L-lysin

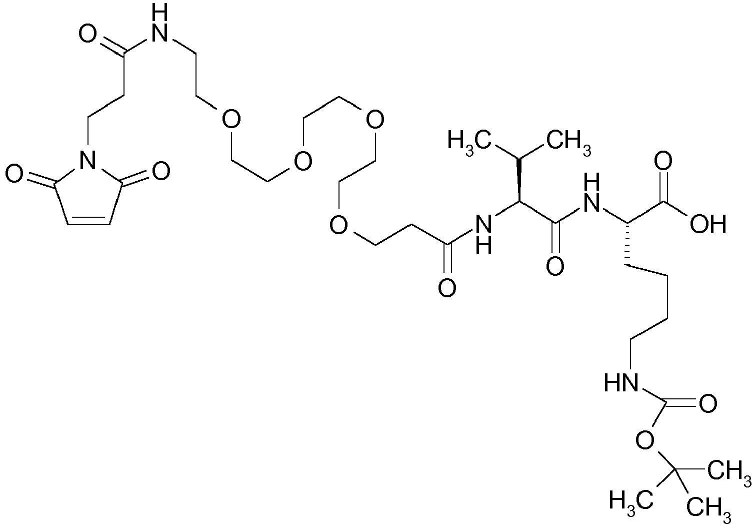

600 mg (1.58 mmol) N2-[(Benzyloxy)carbonyl]-N6-(tert-butoxycarbonyl)-L-lysin wurden in 25.0 mL Wasser/Ethanol/THF (1:1:0.5) mittels Palladium auf Kohle (10 %) bei RT unter Normaldruck mit Wasserstoff hydriert. Die Verbindung N6-(tert-Butoxycarbonyl)-L-lysin wird ohne weitere Reinigung in der nächsten Synthesestufe eingesetzt.
LC-MS (Methode 1): Rₜ = 0.99 min; MS (ESIpos): m/z = 247 [M+H]⁺.

180.0 (0.73 mmol) N6-(tert-Butoxycarbonyl)-L-lysin wurden in 5.0 mL DMF gelöst und mit 74.0 mg (0.73 mmol) Triethylamin versetzt. Es wurden 254.6 mg (0.73 mmol) 2,5-Dioxopyrrolidin-1-yl-N-[(benzyloxy)carbonyl]-L-valinat und 74.0 mg (0.73 mmol) Triethylamin zugegeben. Die Reaktionsmischung wurde 3.5 h bei RT gerührt. Die Reaktionsmischung wurde direkt mittles präp. RP-HPLC gereinigt (Säule: Reprosil 250x30; 10µ, Fluss: 50 mL/min, MeCN/Wasser, 0.1 % TFA). Die Lösemittel wurden dann im Vakuum verdampft und der Rückstand im Hochvakuum getrocknet. Man erhielt 294.1 mg (76 % d. Th.) der Verbindung N-[(Benzyloxy)carbonyl]-L-valyl-N6-(tert-butoxycarbonyl)-L-lysin.
LC-MS (Methode 1): Rₜ = 0.97 min; MS (ESIpos): m/z = 480 [M+H]⁺.

272.2 mg (0.57 mmol) N-[(Benzyloxy)carbonyl]-L-valyl-N6-(tert-butoxycarbonyl)-L-lysin wurden in 20.0 mL Ethylacetat/Ethanol/THF (1:1:1) gelöst und mit 27.2 mg Palladium auf Aktivkohle versetzt und unter Normaldruck und bei RT mit Wasserstoff hydriert. Es wurde über Celite^{(R)} filtriert und der Filterkuchen intensiv mit Ethylacetat/Ethanol/THF (1:1:1) gewaschen. Die Lösemittel wurden im Vakuum verdampft und der Rückstand im Hochvakuum getrocknet. Man erhielt 182.0 mg (72 % d. Th.) der Verbindung L-Valyl-N6-(tert-butoxycarbonyl)-L-lysin.
LC-MS (Methode 1): Rₜ = 0.53 min; MS (ESIpos): m/z = 346 [M+H]⁺.

30.0 mg (0.07 mmol) L-Valyl-N6-(tert-butoxycarbonyl)-L-lysin und 34.3 mg (0.07 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid wurden in 1.5 mL DMF gelöst und mit 6.8 mg (0.07 mmol) 4-Methylmorpholin versetzt. Dir Reaktionsmischung wurde über Nacht bei RT gerührt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 40.6 mg (82 % d. Th.) der Titelverbindung.
LC-MS (Methode 1): Rₜ = 0.73 min; MS (ESIpos): m/z = 744 [M+H]⁺.

### Intermediat L42

### N-[19-(2,5-Dioxo-2,5-dihydro-1H-pyrrol-1-yl)-17-oxo-4,7,10,13-tetraoxa-16-azanonadecan-1-oyl]-L-valyl-N5-carbamoyl-L-Ornithin

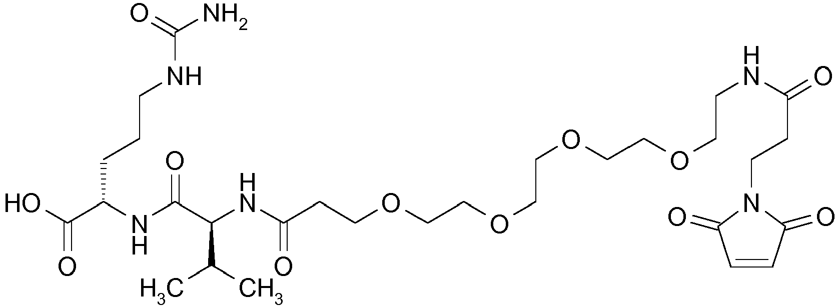

50.0 mg (0.18 mmol) L-Valyl-N5-carbamoyl-L-ornithin (Intermediat L37) wurden in DMF vorgelegt und mit 93.6 mg (0.18 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid und 36.9 mg (0.37 mmol) Triethylamin versetzt. Die Reaktionsmischung rührte bei RT über Nacht. Es wurden 21.9 mg (0.37 mmol) HOAc hinzugefügt und die Reaktionsmischung direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 20.6 mg (14 % d. Th.) der Titelverbindung.
LC-MS (Methode 1): Rₜ = 0.55 min; MS (ESIpos): m/z = 673 [M+H]⁺.

### Intermediat L43

### N-[67-(2,5-Dioxo-2,5-dihydro-1H-pyrrol-1-yl)-65-oxo-4,7,10,13,16,19,22,25,28,31,34,37,40,43,46,49,52,55,58,61-icosaoxa-64-azaheptahexacontan-1-oyl]-L-valyl-N5-carbamoyl-L-ornithin

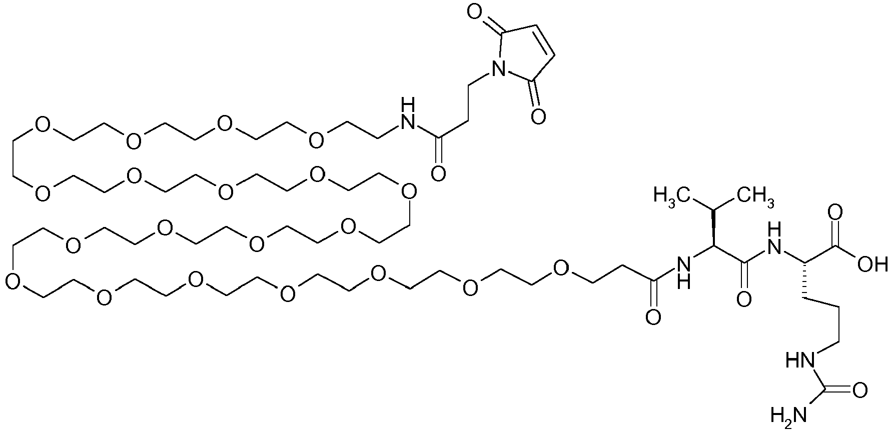

11.3 mg (0.04 mmol) L-Valyl-N5-carbamoyl-L-ornithin (Intermediat L37) wurden in DMF vorgelegt und mit 50.0 mg (0.04 mmol) 3-(2,5-Dioxo-2,5-dihydro-1H-pyrrol-1-yl)-N-{63-[(2,5-dioxopyrrolidin-1-yl)oxy]-63-oxo-3,6,9,12,15,18,21,24,27,30,33,36,39,42,45,48,51,54,57,60-icosaoxatrihexacont-1-yl}propanamid und 8.3 mg (0.08 mmol) Triethylamin versetzt. Die Reaktionsmischung rührte bei RT über Nacht. Es wurden 4.9 mg (0.08 mmol) HOAc hinzugefügt und die Reaktionsmischung direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 15.8 mg (20 % d. Th.) der Titelverbindung.
LC-MS (Methode 4): Rₜ = 0.94 min; MS (ESIpos): m/z = 1377 [M+H]⁺.

### Intermediat L44

### N-[19-(2,5-Dioxo-2,5-dihydro-1H-pyrrol-1-yl)-17-oxo-4,7,10,13-tetraoxa-16-azanonadecan-1-oyl]-L-valyl-L-alanin

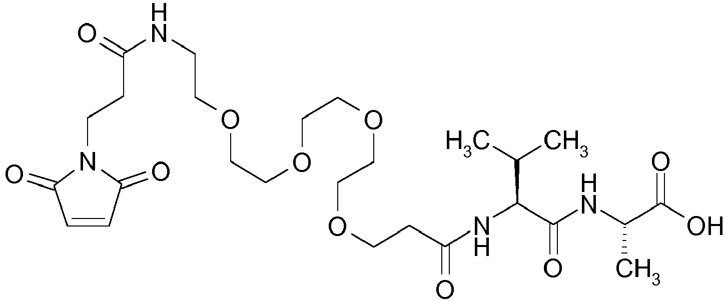

73.3 mg (0.39 mmol) L-Valyl-L-alanin wurden in 7.0 mL DMF gelöst und mit 200.0 mg (0.39 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid und 78.8 mg (0.78 mmol) Triethylamin versetzt. Die Reaktionsmischung wurde bei RT über Nacht gerührt. Die Reaktionsmischung wurde direkt über die präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 103.3 mg (45 % d. Th.) der Titelverbindung.
LC-MS (Methode 1): Rₜ = 0.58 min; MS (ESIpos): m/z = 587 [M+H]⁺.

### Intermediat L45

### tert-Butyl-(2S)-2-[(tert-butoxycarbonyl)amino]-4-oxobutanoat

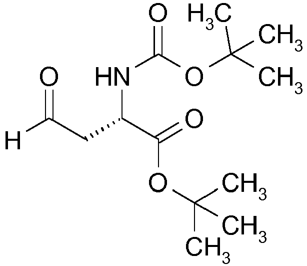

2.00 g (7.26 mmol) tert-Butyl-N-(tert-butoxycarbonyl)-L-homoserinat wurden in 90 ml Dichlormethan gelöst und dann mit 1.76 ml Pyridin sowie mit 4.62 g (10.90 mmol) 1,1,1-Triacetoxy-1lambda⁵,2-benziodoxol-3(1H)-on (Dess-Martin-Periodinan) versetzt. Der Ansatz wurde 2 h bei RT gerührt, anschließend mit 200 ml Dichlormethan verdünnt und zweimal mit 10%-iger Natriumtiosulfat-Lösung und dann nacheinander zweimal mit 5%-iger Zitronensäure und zweimal mit gesättigter Natriumhydrogencarbonat-Lösung ausgeschüttelt. Die organische Phase wurde abgetrennt, über Natriumsulfat getrocknet und anschließend im Vakuum eingeengt. Der Rückstand wurde mit 100 ml Diethylether und Cyclohecan (v/v=1:1) versetzt, etwas eingeengt, wobei ein weißer Niederschlag ausfiel. Dieser wurde abgesaugt. Das Filtrat wurde am Rotationsverdampfer eingeengt und im Hochvakuum getrocknet, wobei 1.74 g (88% d. Th.) der Zielverbindung als hellgelbes Öl erhalten wurden.
LC-MS (Methode 1): Rₜ = 0.85 min; MS (ESIpos): m/z = 274 [M+H]⁺.
¹H-NMR (400 MHz, DMSO-d₆): δ [ppm] = 1.38 (s, 18H), 2.64-2.81 (m, 2H), 4.31-4.36 (m, 1H), 7.23 (d, 1H), 9.59 (s, 1H).

### Intermediat L46

### Trifluoressigsäure--tert-butyl-N-[2-(2,5-dioxo-2,5-dihydro-1H-pyrrol-1-yl)ethyl]-L-glutaminat(1:1)

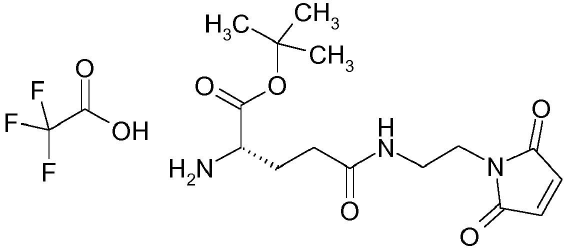

Die Titelverbindung wurde zunächst durch Kupplung von 200 mg (0.79 mmol) Trifluoressigsäure--1-(2-aminoethyl)-1H-pyrrol-2,5-dion(1:1) mit 263 mg (0.87 mmol) (4S)-5-tert-Butoxy-4-[(tert-butoxycarbonyl)amino]-5-oxopentansäure-trifluoressigsäure(1:1) in Gegenwart von EDC/HOBT und *N,N*-Diisopropylethylamin und anschließende Entschützung der Aminogruppe unter schonenden Bedingungen durch 1h Rühren in 10%iger Trifluoressigsäure in DCM bei RT hergestellt. Nach Gefriertrocknung aus Acetonitril/ Wasser wurden 85 mg (20% d.Th.) der Titelverbindung über 2 Stufen erhalten.
LC-MS (Methode 1): Rₜ = 0.37 min; MS (ESIpos): m/z = 326 [M+H]⁺.

### Intermediat L47

### Trifluoressigsäure--beta-alanyl-L-alanyl-N⁵-carbamoyl-N-[4-(2,5-dioxo-2,5-dihydro-1H-pyrrol-1-yl)phenyl]-L-omithinamid(1:1)

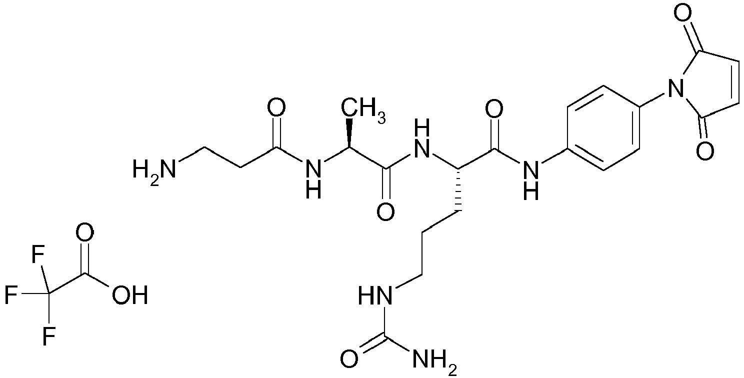

Die Titelverbindung wurde durch Kupplung von Intermediat L8 mit 2,5-Dioxopyrrolidin-1-yl-N-(tert-butoxycarbonyl)-beta-alaninat und anschließender Entschützung mit TFA hergestellt.
LC-MS (Methode 3): Rₜ = 1.36 min; MS (ESIpos): m/z = 488 (M+H)⁺.

### Intermediat L48

### Trifluoressigsäure--(1R,2S)-2-amino-N-[2-(2,5-dioxo-2,5-dihydro-1H-pyrrol-1-yl)ethyl]cyclopentancarboxamid(1:1)

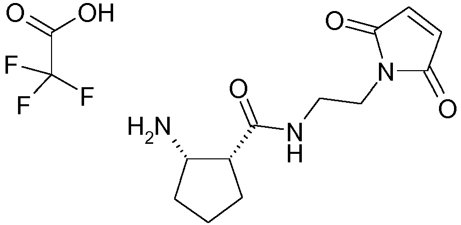

Die Titelverbindung wurde ausgehend von kommerziell erhältlicher (1R, 2S)-2-[(tert-Butoxycarbonyl)amino]cyclopentancarbonsäure in Analogie zu Intermediat L2 hergestellt.
LC-MS (Methode 3): Rₜ = 1.22 min; MS (ESIpos): m/z = 252 (M+H)⁺.

### Intermediat L49

### Trifluoressigsäure--tert-butyl-N-(bromacetyl)-L-valyl-L-alanyl-L-lysinat(1:1)

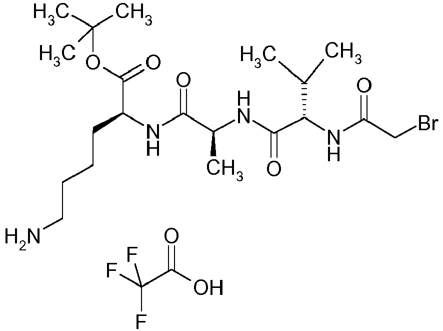

Die Titelverbindung wurde zunächst durch Kupplung von kommerziell erhältlichem Bromessigsäureanhydrid mit dem nach klassischen Methoden der Peptidchemie hergestellten partiell geschützten Peptid tert-Butyl-L-valyl-L-alanyl-N⁶-(tert-butoxycarbonyl)-L-lysinat in Gegenwart von *N,N*-Diisopropylethylamin in Dichlormethan hergestellt. Anschließend erfolgte die Entschützung an der Aminogruppe unter schonenden Bedingungen durch Rühren in 10%iger Trifluoressigsäure in DCM bei RT, wobei in 49%-iger Ausbeute über 2 Stufen die Titelverbindung erhalten wurde.
LC-MS (Methode 1): Rₜ = 1.09 min; MS (ESIpos): m/z = 593 und 595 (M+H)⁺.

### Intermediat L50

### Trifluoressigsäure--(1S,3R)-3-amino-N-[2-(2,5-dioxo-2,5-dihydro-1H-pyrrol-1-yl)ethyl]cyclopentancarboxamid(1:1)

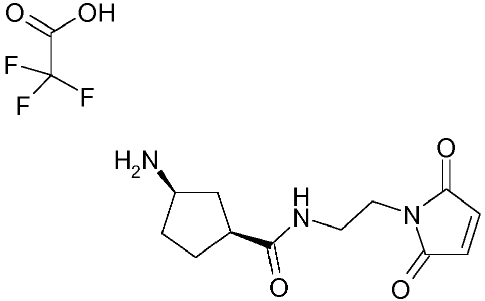

Die Titelverbindung wurde ausgehend von kommerziell erhältlicher (1S,3R)-3-[(tert-Butoxycarbonyl)amino]cyclopentancarbonsäure und ebenso kommerziell erhältlichem Trifluoressigsäure--1-(2-aminoethyl)-1H-pyrrol-2,5-dion(1:1) durch Kupplung mit HATU in Gegenwart von *N,N-*Diisopropylethylamin und anschließender Entschützung mit TFA hergestellt.
HPLC (Methode 11): Rₜ = 0.2 min;
LC-MS (Methode 3): Rₜ = 0.88 min; MS (ESIpos): m/z = 252 (M+H)⁺.

### Intermediat L51

### Trifluoressigsäure--(1R,3R)-3-amino-N-[2-(2,5-dioxo-2,5-dihydro-1H-pyrrol-1-yl)ethyl]cyclopentancarboxamid(1:1)

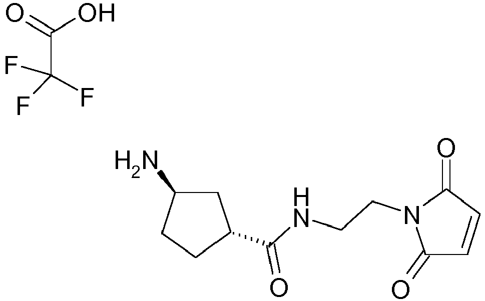

Die Titelverbindung wurde ausgehend von kommerziell erhältlicher (1R,3R)-3-[(tert-Butoxycarbonyl)amino]cyclopentancarbonsäure und ebenso kommerziell erhältlichem Trifluoressigsäure--1-(2-aminoethyl)-1H-pyrrol-2,5-dion(1:1) durch Kupplung mit HATU in Gegenwart von *N,N-*Diisopropylethylamin und anschließender Entschützung mit TFA hergestellt.
LC-MS (Methode 3): Rₜ = 0.98 min; MS (ESIpos): m/z = 250 (M-H)⁻.

### Intermediat L52

### Trifluoressigsäure --N-(2-aminoethyl)-2-bromacetamid (1:1)

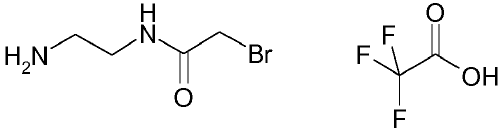

420 mg (2.62 mmol) tert-Butyl-(2-aminoethyl)carbamat wurden in 50 ml Dichlormethan aufgenommen und mit 817 mg (3.15 mmol) Bromessigsäureanhydrid sowie 913 µl (5.24 mmol) *N,N-*Diisopropylethylamin versetzt. Der Ansatz wurde 1 h bei RT gerührt und anschließend im Vakuum eingeengt. Der Rückstand wurde duch präparative HPLC gereinigt.

Es wurden 577 mg der geschützten Zwischenstufe erhalten, die anschließend in 50 ml Dichlormethan aufgenommen und mit 10 ml Trifluoressigsäure versetzt wurde. Nach 1 h Rühren bei RT wurde der Ansatz im Vakuum eingeengt und der Rückstand aus Acentonitril/Wasser lyophilisert. So wurden 705 mg (65% d. Th.) der Titelverbindung erhalten.
LC-MS (Methode 3): Rₜ = 0.34 min; MS (ESIpos): m/z = 181 und 183 (M+H)⁺.

### Intermediat L53

### Trifluoressigsäure--(1S,3S)-3-amino-N-[2-(2,5-dioxo-2,5-dihydro-1H-pyrrol-1-yl)ethyl]cyclopentancarboxamid(1:1)

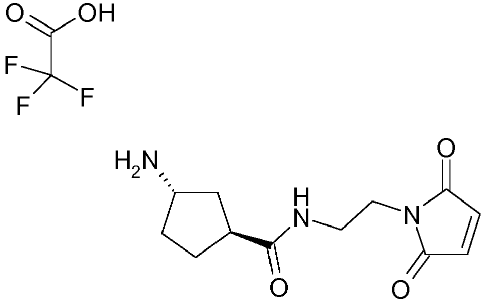

Die Titelverbindung wurde ausgehend von kommerziell erhältlicher (1S,3S)-3-[(tert-Butoxycarbonyl)amino]cyclopentancarbonsäure und ebenso kommerziell erhältlichem Trifluoressigsäure--1-(2-aminoethyl)-1H-pyrrol-2,5-dion(1:1) durch Kupplung mit HATU in Gegenwart von *N,N-*Diisopropylethylamin und anschließender Entschützung mit TFA hergestellt.
HPLC (Methode 11): Rₜ = 0.19 min;
LC-MS (Methode 3): Rₜ = 0.88 min; MS (ESIpos): m/z = 250 (M-H)⁻.

### Intermediat L54

### Trifluoressigsäure--(1R,3S)-3-amino-N-[2-(2,5-dioxo-2,5-dihydro-1H-pyrrol-1-yl)ethyl]cyclopentancarboxamid(1:1)

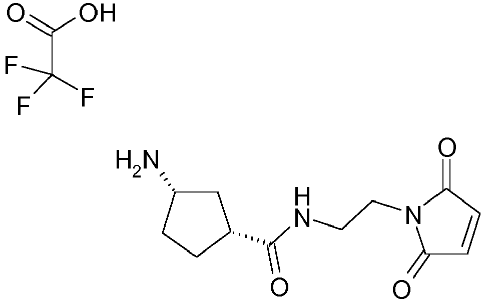

Die Titelverbindung wurde ausgehend von kommerziell erhältlicher (1R,3S)-3-[(tert-Butoxycarbonyl)amino]cyclopentancarbonsäure und ebenso kommerziell erhältlichem Trifluoressigsäure--1-(2-aminoethyl)-1H-pyrrol-2,5-dion(1:1) durch Kupplung mit HATU in Gegenwart von *N,N-*Diisopropylethylamin und anschließender Entschützung mit TFA hergestellt.
LC-MS (Methode 3): Rₜ = 0.89 min; MS (ESIpos): m/z = 252 (M+H)⁺.

### Intermediat L55

### Trifluoressigsäure--tert-butyl-N⁶-D-alanyl-N²-{N-[6-(2,5-dioxo-2,5-dihydro-1H-pyrrol-1-yl)hexanoyl]-L-valyl-L-alanyl}-L-lysinat (1:1)

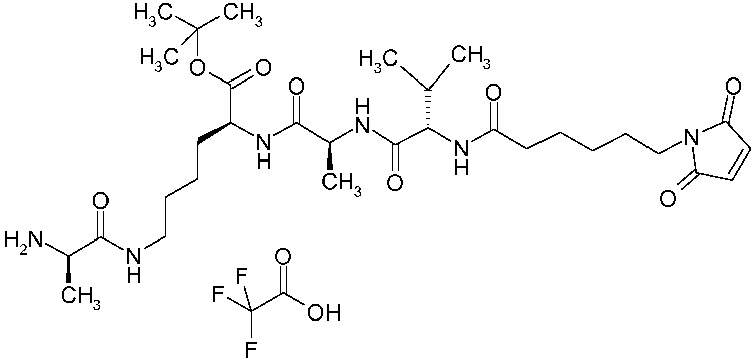

Die Titelverbindung wurde zunächst durch Kupplung von Intermediat L6 mit N-(tert-Butoxycarbonyl)-D-alanin in Gegenwart von HATU und anschließender Entschützung an der Aminogruppe unter schonenden Bedingungen durch 90 minütiges Rühren in 5%iger Trifluoressigsäure in DCM bei RT hergestellt.
HPLC (Methode 11): Rₜ = 1.35 min;
LC-MS (Methode 1): Rₜ = 0.67 min; MS (ESIpos): m/z = 637 (M+H)⁺.

### Intermediat L56

### Trifluoressigsäure --tert-butyl-N-[6-(2,5-dioxo-2,5-dihydro-1H-pyrrol-1-yl)hexanoyl]-L-valyl-L-alanyl-N⁶-{[(1R,3S)-3-aminocyclopentyl]carbonyl}-L-lysinat (1:1)

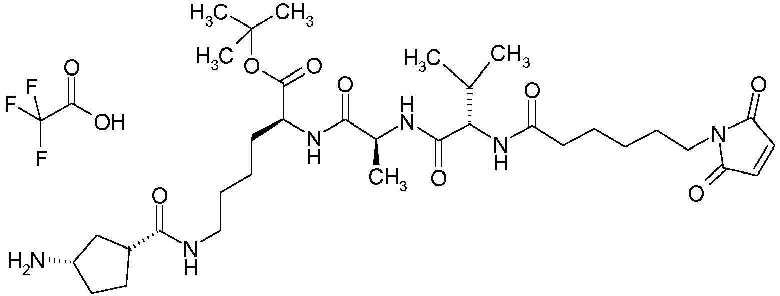

Die Titelverbindung wurde zunächst durch Kupplung von Intermediat L6 mit (1R,3S)-3-[(tert-Butoxycarbonyl)amino]cyclopentancarbonsäure in Gegenwart von HATU und anschließender Entschützung an der Aminogruppe unter schonenden Bedingungen durch 15 minütiges Rühren in 25%-iger Trifluoressigsäure in DCM bei RT hergestellt.
HPLC (Methode 11): Rₜ = 1.4 min;
LC-MS (Methode 1): Rₜ = 0.7 min; MS (ESIpos): m/z = 677 (M+H)⁺.

### Intermediat L57

### Methyl-(2S)-4-oxo-2-({[2-(trimethylsilyl)ethoxy]carbonyl} amino)butanoat

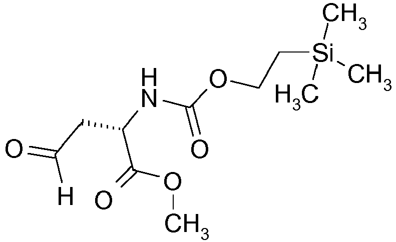

500.0 mg (2.72 mmol) L-Asparaginsäuremethylester Hydrochlorid und 706.3 mg (2.72 mmol) 2-(Trimethylsilyl)ethyl-2,5-dioxopyrrolidin-1-carboxylat wurden in 5.0 mL 1,4-Dioxan vorgelegt und mit 826.8 mg (8.17 mmol) Triethylamin versetzt. Die Reaktionsmischung wurde über Nacht bei RT gerührt. Die Reaktionsmischung wurde direkt mittles präp. RP-HPLC gereinigt (Säule: Reprosil 250x40; 10µ, Fluss: 50 mL/min, MeCN/Wasser, 0.1 % TFA). Die Lösemittel wurden dann im Vakuum verdampft und der Rückstand im Hochvakuum getrocknet. Man erhielt 583.9 mg (74 % d. Th.) der Verbindung (3S)-4-Methoxy-4-oxo-3-({[2-(trimethylsilyl)ethoxy]carbonyl}amino) butansäure.
LC-MS (Methode 1): Rₜ = 0.89 min; MS (ESIneg): m/z = 290 (M-H)⁻.

592.9 mg (3S)-4-Methoxy-4-oxo-3-({[2-(trimethylsilyl)ethoxy]carbonyl}amino)butansäure wurden in 10.0 mL 1,2-Dimethoxyethan vorgelegt und auf -15 °C abgekühlt und mit 205.8 mg (2.04 mmol) 4-Methylmorpholin und 277.9 mg (2.04 mmol) Isobutylchlorformiat versetzt. Der Niederschlag wird nach 15 min abgesaugt und zweimal mit je 10.0 mL 1,2-Dimethoxyethan gewaschen. Das Filtrat wird auf -10 °C abgekühlt und mit 115.5 mg (3.05 mmol) Natriumborhydrid gelöst in 10 mL Wasser unter starkem Rühren versetzt. Die Phasen werden getrennt und die organische Phase je einmal mit ges. Natriumhydrogencarbonat-Lösung und ges. NaCl-Lösung gewaschen. Die organische Phase wurde über Magnesiumsulfat getrocknet und das Lösemittel im Vakuum verdampft und der Rückstand im Hochvakuum getrocknet. Man erhielt 515.9 mg (91 % d. Th.) der Verbindung Methyl-N-{[2-(trimethylsilyl)ethoxy]carbonyl}-L-homoserinat.
LC-MS (Methode 1): Rₜ = 0.87 min; MS (ESIpos): m/z = 278 (M+H)⁺.

554.9 mg (2.00 mmol) Methyl-N-{[2-(trimethylsilyl)ethoxy]carbonyl}-L-homoserinat wurden in 30.0 mL Dichlormethan vorgelegt und mit 1.27 g (3.0 mmol) Dess-Martin periodinan und 474.7 mg (6.00 mmol) Pyridin versetzt. Man rührte über Nacht bei RT. Nach 4 h wurde der Ansatz mit Dichlormethan verdünnt und die organische Phase je dreimal mit 10%iger Na₂S₂O₃-Lösung, 10%iger Citronensäure-Lösung und ges. Natriumhydrogencarbonat-Lösung gewaschen. Die organische Phase wurde über Magnesiumsulfat getrocknet und das Lösemittel im Vakuum verdampft. Man erhielt 565.7 mg (97 % d. Th.) der Titelverbindung.
¹H-NMR (400 MHz, DMSO-d₆): δ [ppm] = 0.03 (s, 9H), 0.91 (m, 2H), 2.70-2.79 (m, 1H), 2.88 (dd, 1H), 3.63 (s, 3H), 4.04 (m, 2H), 4.55 (m, 1H), 7.54 (d, 1H), 9.60 (t, 1H).

### Intermediat L58

### 2-(Trimethylsilyl)ethyl-(3-oxopropyl)carbamat

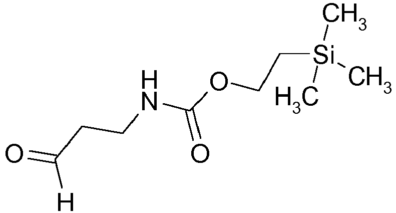

434.4 mg (5.78 mmol) 3-Amino-1-propanol und 1.50 g (5.78 mmol) 2-(Trimethylsilyl)ethyl-2,5-dioxopyrrolidin-1-carboxylat wurden in 10.0 mL Dichlormethan gelöst und mit 585.3 mg (5.78 mmol) Triethylamin versetzt und über Nacht bei RT gerührt. Die Reaktionsmischung wurde mit Dichlormethan verdünnt und die organische Phase mit Wasser und ges. Natriumhydrogencarbonat-Lösung gewaschen und anschließend über Magnesiumsulfat getrocknet. Das Lösemittel wurde im Vakuum verdampft. Der Rückstand 2-(Trimethylsilyl)ethyl-(3-hydroxypropyl)carbamat (996.4 mg, 79 % d. Th.) wurde im Hochvakuum getrocknet und ohne weitere Reinigung in der nächsten Synthesestufe eingesetzt.

807.0 mg (3.68 mmol) 2-(Trimethylsilyl)ethyl-(3-hydroxypropyl)carbamat wurden in 15.0 mL Chloroform und 15.0 mL 0.05 N Kaliumcarbonat/0.05 N Natriumhydrogencarbonat-Lösung (1:1) vorgelegt. Dann wurden 102.2 mg (0.37 mmol) Tetra-n-butylammoniumchlorid, 736.9 mg (5.52 mmol) N-Chlorsuccinimid und 57.5 mg (0.37 mmol) TEMPO zugegeben und die Reaktionsmischung kräftig über Nacht bei RT gerührt. Die Reaktionsmischung wurde mit Dichlormethan verdünnt und die organische Phase wurde mit Wasser und ges. NaCl-Lösung gewaschen. Die organische Phase wurde über Magnesiumsulfat getrocknet und das Lösemittel im Vakuum verdampft. Der Rückstand wurde im Hochvakuum getrocknet und ohne weitere Reinigung in der nächsten Synthesestufe eingesetzt (890.3 mg).

### Intermediat L59

### Trifluoressigsäure--1-{2-[2-(2-aminoethoxy)ethoxy]ethyl}-1H-pyrrol-2,5-dion(1:1)

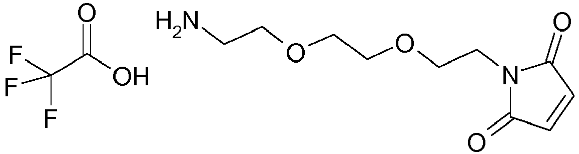

300.0 mg (0.91 mmol) tert-Butyl-(2-{2-[2-(2,5-dioxo-2,5-dihydro-1H-pyrrol-1-yl)ethoxy]ethoxy}ethyl)carbamat wurden in Dichlormethan vorgelegt und mit 4.2 g (36.54 mmol) TFA versetzt und 1 h bei RT gerührt (DC-Kontrolle: Dichlormethan/Methanol 10:1). Die flüchtigen Bestandteile wurden im Vakuum verdampft und der Rückstand viermal mit Dichlormethan kodestilliert. Der Rückstand wurde im Hochvakuum getrocknet und ohne weitere Reinigung in der nächsten Synthesestufe eingesetzt.
LC-MS (Methode 1): Rₜ = 0.19 min; MS (ESIpos): m/z = 229 (M+H)⁺.

### Intermediat L60

### 6-(2,5-Dioxo-2,5-dihydro-1H-pyrrol-1-yl)hexanoylchlorid

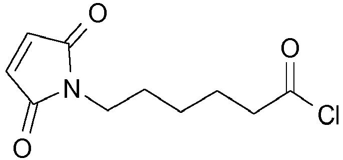

200.0 mg (0.95 mmol) 6-(2,5-Dioxo-2,5-dihydro-1H-pyrrol-1-yl)hexansäure wurden in 4.0 mL Dichlormethan gelöst und mit 338.0 mg (2.84 mmol) Thionylchlorid versetzt. Die Reaktionsmischung wurde 3 h bei RT gerührt und dann mit 1 Tropfen DMF vesetzt. Es wurde nochmal 1 h gerührt. Das Lösemittel wurde im Vakuum verdampft und es wurde dreimal mit Dichlormethan kodestilliert. Das Rohprodukt wurde ohne weitere Reinigung in der nächsten Synthesestufe eingesetzt.

### Intermediat L61

### Trifluoressigsäure --2-(trimethylsilyl)ethyl-N-[6-(2,5-dioxo-2,5-dihydro-1H-pyrrol-1-yl)hexanoyl]-L-valyl-L-alanyl-L-lysinat (1:1)

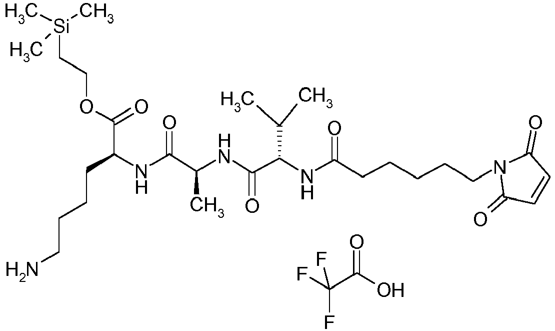

Zunächst wurde ausgehend von N2-[(Benzyloxy)carbonyl]-N6-(tert-butoxycarbonyl)-L-lysin nach klassischen Methoden der Peptidchemie (Veresterung mit 2-(Trimethylsilylethanol mittels EDCI/DMAP, Hydrogenolyse, Kupplung mit N-[(Benzyloxy)carbonyl]-L-valyl-L-alanin in Gegenwart von HATU und erneute Hydrogenolyse) das Tripeptidderivat 2-Trimethylsilyl)ethyl-L-valyl-L-alanyl-N6-(tert-butoxycarbonyl)-L-lysinat hergestellt. Die Titelverbindung wurde durch Kupplung dieses partiell geschützten Peptidderivats mit kommerziell erhältlicher 6-(2,5-Dioxo-2,5-dihydro-1H-pyrrol-1-yl)hexansäure in Gegenwart von HATU und *N,N*-Diisopropylethylamin hergestellt. Anschließend erfolgte die Entschützung an der Aminogruppe unter schonenden Bedingungen durch 2.5 stündiges Rühren in 5%iger Trifluoressigsäure in DCM bei RT unter Erhalt der Esterschutzgruppe. Nach Aufarbeitung und Reinigung durch präparative HPLC wurden 438 mg der Titelverbindung erhalten.
HPLC (Methode 11): Rₜ = 1.69 min;
LC-MS (Methode 1): Rₜ = 0.78 min; MS (ESIpos): m/z = 610 (M+H)⁺.

### Intermediat L62

### Trifluoressigsäure --2-(trimethylsilyl)ethyl-N-[6-(2,5-dioxo-2,5-dihydro-1H-pyrrol-1-yl)hexanoyl]-L-valyl-N⁵-carbamoyl-L-ornithyl-L-lysinat (1:1)

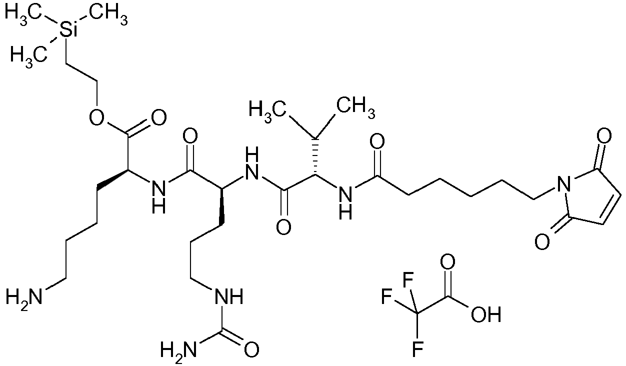

Zunächst wurde ausgehend von N2-[(Benzyloxy)carbonyl]-N6-(tert-butoxycarbonyl)-L-lysin nach klassischen Methoden der Peptidchemie 2-(Trimethylsilyl)ethyl-N6-(tert-butoxycarbonyl)-L-lysinat hergestellt. 148 mg (0.43 mmol) von diesem Intermediat wurden dann in Gegenwart von 195 mg (0.51 mmol) HATU und 149 µL *N,N*-Diisopropylethylamin mit 200 mg (0.43 mmol) von Intermediat L16 gekuppelt. Nach Einengen und Reinigung des Rückstandes mittels präparativer HPLC wurde das geschützte Intermediat in 20 mL DCM aufgenommen und durch Zugabe von 2 mL Trifluoressigsäure und 1 h Rühren bei RT die tert. Butoxycarbonyl-Schutzgruppe abgelöst. Nach Einengen und Lyophilisation des Rückstandes aus Acetonitril/Wasser wurden 254 mg (63% d. Th. über 2 Stufen) erhalten.
HPLC (Methode 11): Rₜ = 1.51 min;
LC-MS (Methode 1): Rₜ = 0.68 min; MS (ESIpos): m/z = 696 (M+H)⁺.

### Intermediat L63

### (4S)-4-{[(2S)-2-{[(2S)-2-{[6-(2,5-Dioxo-2,5-dihydro-1H-pyrrol-1-yl)hexanoyl]amino}-3-methylbutanoyl]amino}propanoyl]amino}-5-oxo-5-[2-(trimethylsilyl)ethoxy]pentansäure

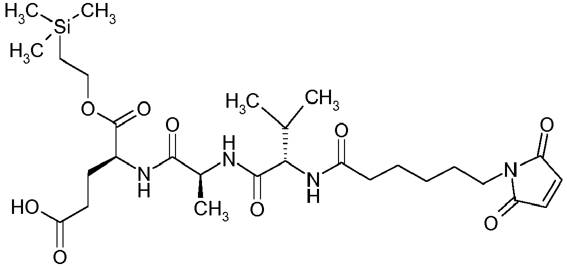

Zunächst wurde ausgehend von (2S)-5-(Benzyloxy)-2-[(tert-butoxycarbonyl)amino]-5-oxopentansäure nach klassischen Methoden der Peptidchemie (Veresterung mit 2-(Trimethylsilylethanol mittels EDCI/DMAP, Abspaltung der Boc-Schutzgruppe mit Trifluoressigsäure, Kupplung mit N-[(Benzyloxy)carbonyl]-L-valyl-L-alanin in Gegenwart von HATU und Hydrogenolyse in Methanol über 10%-igem Palladium auf Aktivkohle) das Tripeptidderivat (4S)-4-{[(2S)-2-{[(2S)-2-Amino-3-methylbutanoyl]amino}propanoyl]amino}-5-oxo-5-[2-(trimethylsilyl)ethoxy]pentansäure hergestellt. Die Titelverbindung wurde durch Kupplung dieses partiell geschützten Peptidderivats mit kommerziell erhältlichem 1-{6-[(2,5-Dioxopyrrolidin-1-yl)oxy]-6-oxohexyl}-1H-pyrrol-2,5-dion hergestellt. Nach Aufarbeitung und Reinigung durch präparative HPLC wurden 601 mg der Titelverbindung erhalten.
LC-MS (Methode 1): Rₜ = 0.96 min; MS (ESIpos): m/z = 611 (M+H)⁺.

### Intermediat L64

### (4S)-4-{[(2,5-Dioxo-2,5-dihydro-1H-pyrrol-1-yl)acetyl]amino}-5-oxo-5-[2-(trimethylsilyl)ethoxy] pentansäure

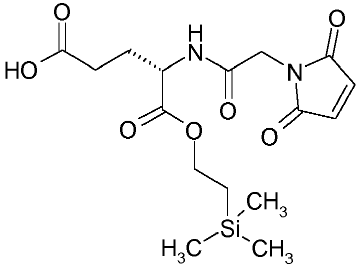

Die Titelverbindung wurde ausgehend von (2S)-5-(Benzyloxy)-2-[(tert-butoxycarbonyl)amino]-5-oxopentansäure nach klassischen Methoden der Peptidchemie (Veresterung mit 2-(Trimethylsilylethanol mittels EDCI/DMAP, Abspaltung der Boc-Schutzgruppe mit Trifluoressigsäure, hydrogenolytische Spaltung des Benzylesters in Methanol über 10%-igem Palladium auf Aktivkohle und Kupplung mit 1-{2-[(2,5-Dioxopyrrolidin-1-yl)oxy]-2-oxoethyl}-1H-pyrrol-2,5-dion in Gegenwart von *N,N-*Diisopropylethylamin) hergestellt.
LC-MS (Methode 1): Rₜ = 0.84 min; MS (ESIpos): m/z = 385 (M+H)⁺.

### Intermediat L65

### Trifluoressigsäure--2-(trimethylsilyl)ethyl-3-{[(benzyloxy)carbonyl]amino}-L-alaninat (1:1)

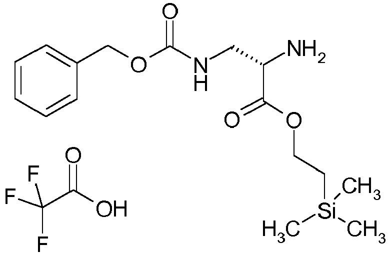

Die Titelverbindung wurde ausgehend von 3-{[(Benzyloxy)carbonyl]amino}-N-(tert-butoxycarbonyl)-L-alanin nach klassischen Methoden der Peptidchemie (Veresterung mit 2-(Trimethylsilylethanol mittels EDCI/DMAP und Abspaltung der Boc-Schutzgruppe mit Trifluoressigsäure hergestellt. Man erhielt 373 mg (79 % d. Th. über 2 Stufen) der Titelverbindung.
LC-MS (Methode 1): Rₜ = 0.72 min; MS (ESIpos): m/z = 339 (M+H)⁺.

### Intermediat L66

### Methyl-(8S)-8-(2-hydroxyethyl)-2,2-dimethyl-6,11-dioxo-5-oxa-7,10-diaza-2-silatetradecan-14-oat

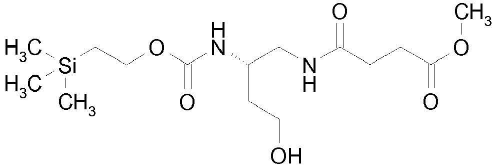

1000 mg (2.84 mmol) (3S)-3-{[(Benzyloxy)carbonyl]amino}-4-[(tert-butoxycarbonyl)amino] butansäure wurden in 10.0 mL 1,2-Dimethoxyethan vorgelegt und mit 344.4 mg (3.4 mmol) 4-Methylmorpholin und 504 mg (3.69 mmol) Isobutylchlorformiat versetzt. Nach 10min Rühren bei RT wurde der Ansatz auf 5°C abgekühlt und portionsweise mit 161 mg (4.26 mmol) Natriumborhydrid gelöst in 3 mL Wasser unter starkem Rühren versetzt. Nach 1 h erfolgte nochmals eine Zugabe der gleichen Menge an Natriumborhydrid und der Ansatz wurde anschließend langsam auf RT erwärmt. Es wurden 170 ml Wasser zugegeben und der Ansatz dann viermal mit jeweils 200 ml Ethylacetat extrahiert. Die Phasen wurden getrennt und die organische Phase einmal mit Citronensäure und anschließend mit gesättigter Natriumhydrogencarbonat-Lösung gewaschen. Die organische Phase wurde über Magnesiumsulfat getrocknet und das Lösemittel im Vakuum verdampft und der Rückstand im Hochvakuum getrocknet. Man erhielt 760 mg (78 % d. Th.) der Verbindung Benzyl-tert-butyl-[(2S)-4-hydroxybutan-1,2-diyl]biscarbamat.
LC-MS (Methode 1): Rₜ = 0.84 min; MS (ESIpos): m/z = 339 (M+H)⁺.

760 mg (2.16 mmol) von diesem Intermediat wurden in 13 ml Chlorwasserstoff/Dioxan gelöst 20 min bei RT gerührt. Anschließend wurde der Ansatz auf 5 ml aufkonzentriert und mit Diethylether versetzt. Der Niederschlag wurde abfiltriert und aus Acetonitril/Wasser 1:1 lyophilisiert.

Das so erhaltene Produkt wurde in 132 ml DMF gelöst und mit 345.5 mg (2.35 mmol) 4-Methoxy-4-oxobutansäure, 970 mg (2.55 mmol) HATU und 1025 µl *N,N*-Diisopropylethylamin versetzt. Die Mischung wurde 5 min bei RT gerührt. Das Lösungsmittel wurde im Vakuum entfernt und der verbliebene Rückstand mittels präparativer HPLC gereinigt. Die entsprechenden Fraktionen wurden vereinigt und das Acetonitril im Vakuum abgedampft. Die verbleibende wässrige Phase wurde zweimal mit Ethylacetat extrahiert und die organische Phase anschließend einggengt und im Hochvakuum getrocknet.

Das so erhaltene Zwischenprodukt wurde in Methanol aufgenommen und über 10%-igem Palladium auf Aktivkohle 1 h lang bei RT unter Wasserstoff-Normaldruck hydriert. Der Katalysator wurde danach abfiltriert und das Lösungsmittel im Vakuum entfernt.

247 mg von dieser entschützten Verbindung wurden in 20 ml DMF aufgenommen und mit 352 mg (1.36 mmol) 1-({[2-(Trimethylsilyl)ethoxy]carbonyl}oxy)pyrrolidin-2,5-dion sowie 592 µL *N,N-*Diisopropylethylamin versetzt. Die Reaktionsmischung wurde 1h bei RT gerührt, anschließend eingeengt und der Rückstand mittles präparativer HPLC gereinigt. Die Lösemittel wurden dann im Vakuum verdampft und der Rückstand im Hochvakuum getrocknet. Man erhielt über diese 5 Reaktionsschritte in einer 21%-igen Gesamtausbeute 218 mg der Titelverbindung.
LC-MS (Methode 1): Rₜ = 0.74 min; MS (ESIpos): m/z = 363 (M+H)⁺.

### Intermediat L67

### Trifluoressigsäure --2-(2,5-dioxo-2,5-dihydro-1H-pyrrol-1-yl)ethyl-beta-alaninat (1:1)

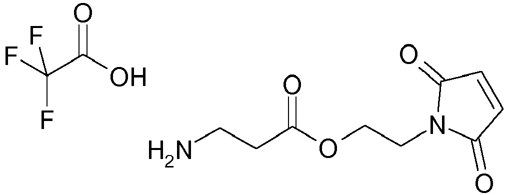

Die Titelverbindung wurde ausgehend von 50 mg (0.354 mmol) von kommerziell erhältlichem 1-(2-Hydroxyethyl)-1H-pyrrol-2,5-dion durch Kupplung mit 134 mg (0.71 mmol) N-(tert-Butoxycarbonyl)-beta-alanin in 10 ml Dichlormethan in Gegenwart von 1.5 Equivalenten EDCI und 0.1 Equivalent 4-N, *N*-Dimethylaminopyridin und anschließende Entschützung mit Trifluoressigsäure hergestellt.
Ausbeute: 56 mg (48% d. Th. über 2 Stufen)
LC-MS (Methode 3): Rₜ = 1.15 min; MS (ESIpos): m/z = 213 (M+H)⁺.

### Intermediat L68

### Trifluoressigsäure--N-(2-aminoethyl)-2-(2,5-dioxo-2,5-dihydro-1H-pyrrol-1-yl)propanamid(1:1)

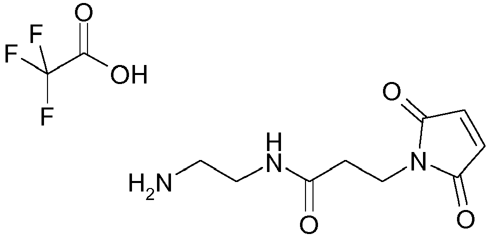

Die Titelverbindung wurde in Analogie zu Intermediat L1 nach klassischen Methoden der Peptidchemie aus kommerziell erhältlicher (2,5-Dioxo-2,5-dihydro-1H-pyrrol-1-yl)propansäure und tert-Butyl-(2-aminoethyl) carbamat hergestellt.
LC-MS (Methode 1): Rₜ = 0.17 min; MS (ESIpos): m/z = 212 (M+H)⁺.

### Intermediat L69

### Trifluoressigsäure--1-[(benzyloxy)carbonyl]piperidin-4-yl-L-valyl-N⁵-carbamoyl-L-omithinat (1:1)

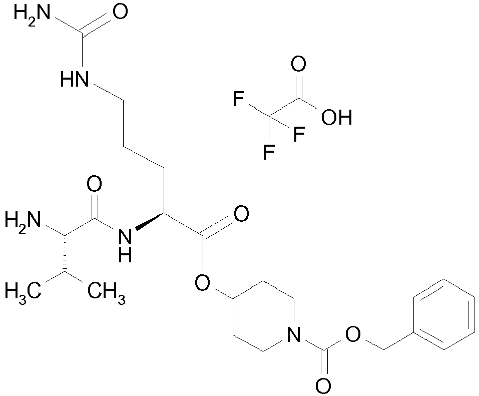

Die Titelverbindung wurde nach klassischen Methoden der Peptidchemie aus kommerziell erhältlichem Benzyl-4-hydroxypiperidin-1-carboxylat durch Veresterung mit N2-(tert-Butoxycarbonyl)-N5-carbamoyl-L-ornithin mittels EDCI/DMAP, anschließende Boc-Spaltung mit TFA, dann folgende Kupplung mit N-[(tert.-Butoxy)carbonyl]-L-valin in Gegenwart von HATU und *N,N-*Diisopropylethylamin und schließlich erneute Boc-Abspaltung mit TFA hergestellt.
LC-MS (Methode 1): Rₜ = 0.62 min; MS (ESIpos): m/z = 492 (M+H)⁺.

### Intermediat L70

### 9H-Fluoren-9-ylmethyl-(3-oxopropyl)carbamat

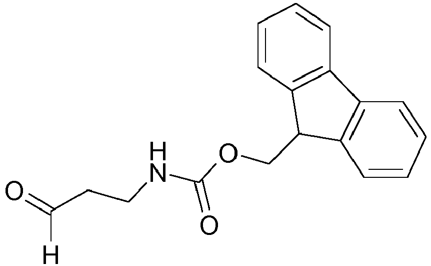

1000.0 mg (3.36 mmol) 9H-Fluoren-9-ylmethyl-(3-hydroxypropyl)carbamat wurden in 15.0 mL Chloroform und 15.0 mL 0.05 N Kaliumcarbonat/0.05 N Natriumhydrogencarbonat-Lösung (1:1) vorgelegt. Dann wurden 93.5 mg (0.34 mmol) Tetra-n-butylammoniumchlorid, 673.6 mg (5.04 mmol) N-Chlorsuccinimid und 52.5 mg (0.34 mmol) TEMPO zugegeben und die Reaktionsmischung kräftig über Nacht bei RT gerührt. Die Reaktionsmischung wurde mit Dichlormethan verdünnt und die organische Phase wurde mit Wasser und ges. NaCl-Lösung gewaschen. Die organische Phase wurde über Magnesiumsulfat getrocknet und das Lösemittel im Vakuum verdampft. Der Rückstand wurde im Hochvakuum getrocknet und mittels Kieselgel (Laufmittel: Cyclohexan/Ethylacetat 3:1-1:1) gereinigt. Die Lösemittel wurden im Vakuum verdampft und der Rückstand im Hochvakuum getrocknet. Man erhielt 589.4 mg (58 % d. Th.) der Titelverbindung.
LC-MS (Methode 6): Rₜ = 2.15 min; MS (ESIpos): m/z = 296 (M-H)⁺.

### Intermediat L71

### tert-Butyl-[4-(chlorcarbonyl)phenyl]carbamat

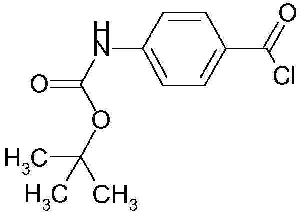

100.0 mg (0.42 mmol) 4-[(tert-Butoxycarbonyl)amino]benzoesäure wurden in 2.0 mL Dichlormethan vorgelegt und mit 64.2 mg (0.51 mmol) Oxalsäuredichlorid versetzt. Die Reaktionsmischung rührte bei RT 30 min (DC-Kontrolle: Dichlormethan/Methanol). Dann wurden nochmals 192.6 mg (1.53 mmol) Oxalsäuredichlorid und 1 Tropfen DMF zugegeben und 1 h bei RT gerührt. Das Lösemittel wurde im Vakuum verdampft und der Rückstand mehrmals mit Dichlormethan kodestilliert. Der Rückstand wurde ohne weitere Reinigung in der nächsten Synthesestufe eingesetzt.

### Intermediat L72

### Benzyl-(9S)-9-(hydroxymethyl)-2,2-dimethyl-6,11-dioxo-5-oxa-7,10-diaza-2-silatetradecan-14-oat

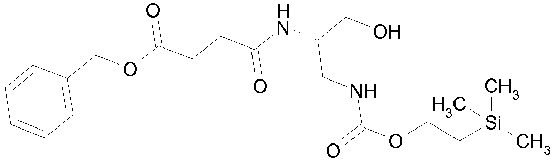

Die Titelverbindung wurde ausgehend von kommerziell erhältlichem Benzyl-tert-butyl-[(2S)-3-hydroxypropan-1,2-diyl]biscarbamat nach klassischen Methoden der Peptidchemie durch hydrogenolytische Abspaltung der Z-Schutzgruppe, anschließende Kupplung mit 4-(Benzyloxy)-4-oxobutansäure in Gegenwart von EDCI/HOBT, dann Abspaltung der Boc-Schutzgruppe mit TFA und schließlich durch Umsetzung mit 1-({[2-(Trimethylsilyl)ethoxy]carbonyl}oxy)pyrrolidin-2,5-dion in Gegenwart von Triethylamin hergestellt.
LC-MS (Methode 1): Rₜ = 0.94 min; MS (ESIpos): m/z = 425 [M+H]⁺.

### Intermediat L73

### N-(2-aminoethyl)-6-(2,5-dioxo-2,5-dihydro-1H-pyrrol-1-yl)hexanamide

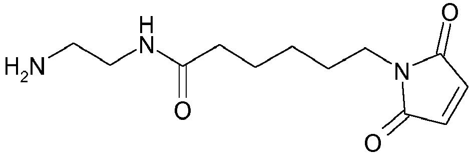

Zu einer Lösung von 300 mg (1.87 mmol) tert-butyl (2-aminoethyl)carbamate in 20 ml Dimethylformamid wurden 395.5 mg (1.87 mmol) 6-(2,5-Dioxo-2,5-dihydro-1H-pyrrol-1-yl)hexansäure, 1.21 g (9.36 mmol) N,N-Diisopropylethylamin und 854.3 mg (2.25 mmol) HATU zugegeben. Die Reaktionsmischung wurde 5 Minuten bei Rt gerührt. Nach Einengen der Mischung wurde der Rückstand in DCM aufgenommen und mit Wasser gewaschen. Die organische Phase wurde mit Brine gewaschen, über Magnesiumsulfat getrocknet, abfiltriert und eingeengt. Man erhielt 408 mg (33%, Reinheit 53%) der Titelverbindung, die ohne weitere Reinigung eingesetzt wurden.
LC-MS (Methode 1): Rₜ = 0.75 min; MS (ESIpos): m/z = 354 (M+H)⁺.

Zu einer Lösung von *tert*-butyl (2-{[6-(2,5-dioxo-2,5-dihydro-1H-pyrrol-1-yl)hexanoyl]amino}ethyl)carbamate (408 mg, 0.365 mmol) in 7 ml dichlormethan wurde 1 ml TFA zugegeben. Das Reaktionsgemisch wird 0.5 h bei RT gerührt. Die Reaktionsmischung wurde im Vakuum verdampft und der Rückstand wurde zweimal mit Dichlormethan kodestilliert. Der Rückstand wurde ohne weitere Reinigung weiter eingesetzt. Man erhielt 384 mg (94%, Reinheit 57%) der Titelverbindung.
LC-MS (Methode 1): Rₜ = 0.26 min; MS (ESIpos): m/z = 254 (M+H)⁺.

### Intermediat F82

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-{3-[{1-[1-benzyl-4-(2,5-difluorphenyl)-1H-pyrrol-2-yl]-2,2-dimethylpropyl} (glycoloyl)amino]propyl} -L-alaninamid

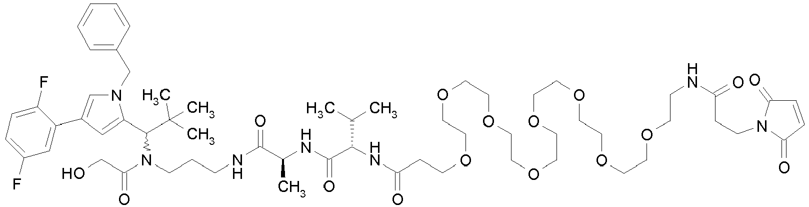

Die Synthese der Titelverbindung erfolgte in Analogie zur Synthese von Intermediat F83. Die dabei verwendeten racemischen Intermediate wurden in Analogie zu den entsprechenden R-Isomer Intermediaten erhalten.
LC-MS (Methode 2): Rₜ = 7.07 min; MS (EIpos): m/z = 1236 [M+Na]⁺.

### Intermediat F83

### N-[31-(2,5-Dioxo-2,5-dihydro-1H-pyrrol-1-yl)-29-oxo-4,7,10,13,16,19,22,25-octaoxa-28-azahentriacontan-1-oyl]-L-valyl-N-{3-[{(1R)-1-[1-benzyl-4-(2,5-difluorphenyl)-1H-pyrrol-2-yl]-2,2-dimethylpropyl} (glycoloyl)amino]propyl} -L-alaninamid

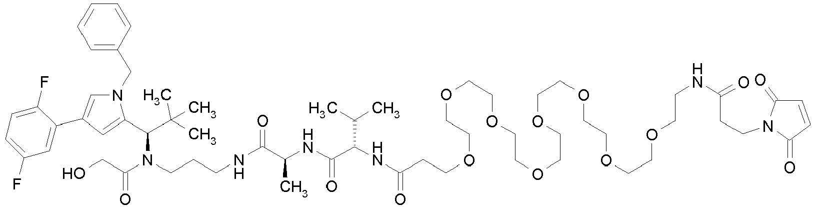

30.0 mg (0.06 mmol) N-(3-Aminopropyl)-N-{(1R)-1-[1-benzyl-4-(2,5-difluorphenyl)-1H-pyrrol-2-yl]-2,2-dimethylpropyl}-2-hydroxyacetamid (Beispiel 98) und 26.1 mg (0.06 mmol) 2,5-Dioxopyrrolidin-1-yl-N-[(9H-fluoren-9-ylmethoxy)carbonyl]-L-alaninat wurden in 2.0 mL DMF vorgelegt und mit 19.4 mg (0.19 mmol) 4-Methylmorpholin versetzt. Die Reaktionsmischung wurde über Nacht bei RT gerührt und mit 11.5 mg (0.19 mmol) HOAc versetzt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 41.9 mg (79 % d. Th.) der Verbindung 9H-Fluoren-9-ylmethyl-[(2S)-1-({3-[{(1R)-1-[1-benzyl-4-(2,5-difluorphenyl)-1H-pyrrol-2-yl]-2,2-dimethylpropyl}(glycoloyl)amino]propyl}amino)-1-oxopropan-2-yl]carbamat.
LC-MS (Methode 1): Rₜ = 1.44 min; MS (ESIpos): m/z = 763 [M+H]⁺.

37.2 mg (0.05 mmol) 9H-Fluoren-9-ylmethyl-[(2S)-1-({3-[{(1R)-1-[1-benzyl-4-(2,5-difluorphenyl)-1H-pyrrol-2-yl]-2,2-dimethylpropyl}(glycoloyl)amino]propyl}amino)-1-oxopropan-2-yl]carbamat wurden in 1.5 mL DMF gelöst und mit 124.6 mg (1.46 mmol) 2-Aminoethanol versetzt. Die Reaktionsmischung wurde über Nacht bei RT gerührt. Die Reaktionsmischung wurde zwischen Ethylacetat und Wasser verteilt und die organische Phase zweimal mit Wasser und einmal mit ges. NaCl-Lösung gewaschen. Nach der Trocknung über Magnesiumsulfat wurde das Lösemittel im Vakuum verdampft und der Rückstand mittels Kieselgel (Laufmittel: Dichlormethan/Methanol 10:1) gereinigt. Die Lösemittel wurden im Vakuum verdampft und der Rückstand im Hochvakuum getrocknet. Man erhielt 14.2 mg (50 % d. Th.) der Verbindung N-{3-[{(1R)-1-[1-Benzyl-4-(2,5-difluorphenyl)-1H-pyrrol-2-yl]-2,2-dimethylpropyl} (glycoloyl)amino]propyl} -L-alaninamid.
LC-MS (Methode 1): Rₜ = 0.92 min; MS (ESIpos): m/z = 541 [M+H]⁺.

14.1 mg (0.03 mmol) N-{3-[{(1R)-1-[1-Benzyl-4-(2,5-difluorphenyl)-1H-pyrrol-2-yl]-2,2-dimethylpropyl}(glycoloyl)amino]propyl}-L-alaninamid und 11.4 (0.03 mmol) 2,5-Dioxopyrrolidin-1-yl-N-[(9H-fluoren-9-ylmethoxy)carbonyl]-L-valinat wurden in 1.5 mL DMF gelöst und mit 7.9 mg (0.08 mmol) 4-Methylmorpholin versetzt. Die Reaktionsmischung wurde über Nacht bei RT gerührt und mit 4.7 mg (0.08 mmol) HOAc versetzt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 15.9 mg (71 % d. Th.) der Verbindung N-[(9H-Fluoren-9-ylmethoxy)carbonyl]-L-valyl-N-{3-[{(1R)-1-[1-benzyl-4-(2,5-difluorphenyl)-1H-pyrrol-2-yl]-2,2-dimethylpropyl}(glycoloyl)amino]propyl}-L-alaninamid.
LC-MS (Methode 1): Rₜ = 1.46 min; MS (ESIpos): m/z = 862 (M+H)⁺.

14.9 mg (0.02 mmol) N-[(9H-Fluoren-9-ylmethoxy)carbonyl]-L-valyl-N-{3-[{(1R)-1-[1-benzyl-4-(2,5-difluorphenyl)-1H-pyrrol-2-yl]-2,2-dimethylpropyl}(glycoloyl)amino]propyl}-L-alaninamid wurden in 1.5 mL DMF gelöst und mit 44.2 mg (0.52 mmol) 2-Aminoethanol versetzt. Die Reaktionsmischung wurde über Nacht bei RT gerührt. Die Reaktionsmischung wurde zwischen Ethylacetat und Wasser verteilt und die organische Phase zweimal mit Wasser und einmal mit ges. NaCl-Lösung gewaschen. Nach der Trocknung über Magnesiumsulfat wurde das Lösemittel im Vakuum verdampft und der Rückstand mittels Kieselgel (Laufmittel: Dichlormethan/Methanol 10:1) gereinigt. Die Lösemittel wurden im Vakuum verdampft und der Rückstand im Hochvakuum getrocknet. Man erhielt 5.7 mg (52 % d. Th.) der Verbindung L-Valyl-N-{3-[{(1R)-1-[1-benzyl-4-(2,5-difluorphenyl)-1H-pyrrol-2-yl]-2,2-dimethylpropyl} (glycoloyl)amino]propyl} -L-alaninamid.
LC-MS (Methode 1): Rₜ = 0.92 min; MS (ESIpos): m/z = 640 (M+H)⁺.

5.5 mg (8.6 µmol) L-Valyl-N-{3-[{(1R)-1-[1-benzyl-4-(2,5-difluorphenyl)-1H-pyrrol-2-yl]-2,2-dimethylpropyl}(glycoloyl)amino]propyl}-L-alaninamid und 6.5 mg (6.5 µmol) 3-(2,5-Dioxo-2,5-dihydro-1H-pyrrol-1-yl)-N-{27-[(2,5-dioxopyrrolidin-1-yl)oxy]-27-oxo-3,6,9,12,15,18,21,24-octaoxaheptacos-1-yl}propanamid wurden in 1.0 mL DMF gelöst und mit 0.9 mg (8.6 mmol) 4-Methylmorpholin versetzt. Die Reaktionsmischung wurde über Nacht bei RT gerührt und mit 0.8 mg (0.01 mmol) HOAc versetzt. Die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 7.7 mg (74 % d. Th.) der Titelverbindung.
LC-MS (Methode 1): Rₜ = 1.10 min; MS (ESIpos): m/z = 1214 (M+H)⁺.

### Intermediat F84

### Trifluoressigsäure-(2S)-2-amino-4-[{(1R)-1-[1-benzyl-4-(2,5-difluorphenyl)-1H-pyrrol-2-yl]-2,2-dimethylpropyl}(glycoloyl)amino]-N-(3-{[2-(2,5-dioxo-2,5-dihydro-1H-pyrrol-1-yl)ethyl]amino}-3-oxopropyl)butanamid(1:1)

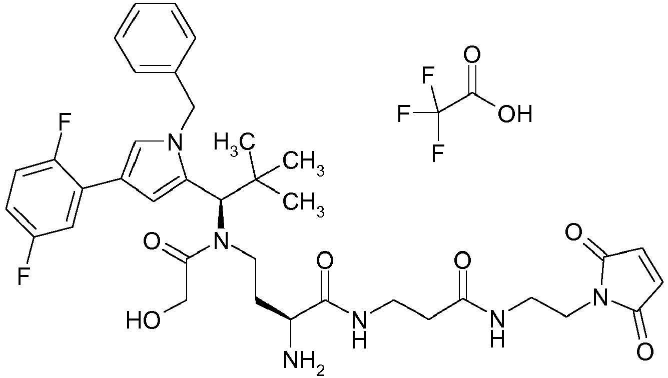

Zunächst wurden 16.5 mg (0.02 mmol) Intermediat C54 in 5 ml DMF aufgenommen und mit 10.4 mg (0.041 mmol) Trifluoressigsäure--1-(2-aminoethyl)-1H-pyrrol-2,5-dion(1:1) in Gegenwart von 11.7 mg (0.03 mmol) HATU sowie 18 µl N,N-Diisopropylethylamin umgesetzt. Nach 5 min Rühren bei RT wurde eingeengt und der Rückstand in Acetonitril/Wasser 1:1 aufgenommen. Mit Trifluoressigsäure wurde ein pH-Wert von 2 eingestellt und der Ansatz erneut eingeengt. Der verbliebene Rückstand durch präparative HPLC gereinigt. Es wurden 8 mg (42% d. Th.) des geschützten Intermediats erhalten.
LC-MS (Methode 1): Rₜ = 1.38 min; MS (EIpos): m/z = 929 [M+H]⁺.

7.6 mg (0.008 mmol) dieses Intermediats wurden in 3 ml DMF aufgenommen und mit 92 mg (0.82 mmol) 1,4-Diazabicyclo(2.2.2)octan versetzt. Der Ansatz wurde 1 h im Ultraschallbad behandelt. Anschließend wurden 31 µl Essigsäure zugegeben und der Ansatz im Hochvakuum eingeengt. Der Rückstand wurde durch präparative HPLC gereinigt. Es wurden 3 mg (45% d. Th.) der Titelverbindung erhalten.
LC-MS (Methode 1): Rₜ = 0.86 min; MS (EIpos): m/z = 707 [M+H]⁺.

¹H-NMR (500 MHz, DMSO-d₆): δ = 8.15 (t, 1H), 7.9-8.1 (m, 4H), 7.6 (m, 1H), 7.5 (s, 1H), 7.15-7.35 (m, 6H), 6.9-7.0 (m, 3H), 6.85 (s, 1H), 5.6 (s, 1H), 4.9 und 5.2 (2d, 2H), 4.05 und 4.2 (2d, 2H), 3.1-3.2 (m, 4H), 2.15 (m, 2H), 0.7 und 1.45 (2m, 2H), 0.8 (s, 9H).

### Intermediat F85

### Trifluoressigsäure-(2S)-2-amino-4-[{(1R)-1-[1-benzyl-4-(2,5-difluorphenyl)-1H-pyrrol-2-yl]-2,2-dimethylpropyl}(glycoloyl)amino]-N-[2-(2,5-dioxo-2,5-dihydro-1H-pyrrol-1-yl)ethyl]butanamid(1:1)

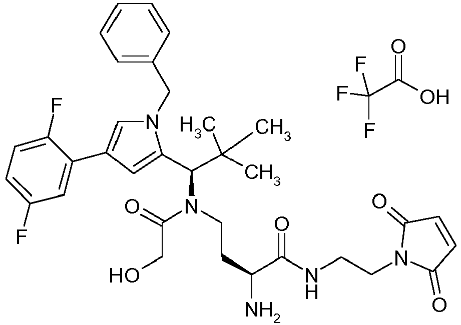

Zunächst wurden 10 mg (0.014 mmol) Intermediat C53 in 3.4 ml DMF aufgenommen und mit 7 mg (0.027 mmol) Trifluoressigsäure--1-(2-anünoethyl)-1H-pyrrol-2,5-dion(1:1) in Gegenwart von 7.8 mg ( 0.02 mmol) HATU sowie 12 µl N,N-Diisopropylethylamin umgesetzt. Nach 15 min Rühren bei RT wurde eingeengt und der Rückstand durch präparative HPLC gereinigt. Es wurden 6.6 mg (57% d. Th.) des geschützten Intermediats erhalten.
LC-MS (Methode 1): Rₜ = 1.4 min; MS (EIpos): m/z = 858 [M+H]⁺.

6.6 mg (0.008 mmol) dieses Intermediats wurden in 2 ml DMF aufgenommen und mit 86 mg (0.77 mmol) 1,4-Diazabicyclo(2.2.2)octan versetzt. Der Ansatz wurde 2 h im Ultraschallbad behandelt. Anschließend wurden 44 µl Essigsäure zugegeben und der Ansatz im Hochvakuum eingeengt. Der Rückstand wurde durch präparative HPLC gereinigt. Es wurden 3.3 mg (53% d. Th.) der Titelverbindung erhalten.
LC-MS (Methode 1): Rₜ = 0.88 min; MS (EIpos): m/z = 636 [M+H]⁺.

### Intermediat F86

### Trifluoressigsäure-(2S)-2-amino-4-[{(1R)-1-[4-benzyl-1-(2,5-difluorphenyl)-1H-pyrazol-3-yl]-2,2-dimethylpropyl}(glycoloyl)amino]-N-(3-{[2-(2,5-dioxo-2,5-dihydro-1H-pyrrol-1-yl)ethyl]amino}-3-oxopropyl)butanamid(1:1)

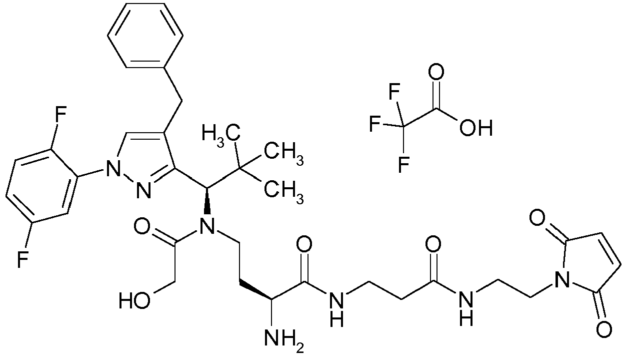

Die Titelverbindung wurde ausgehend von 8 mg (0.012 mmol) Intermediat C51 durch Umsetzung mit 4.5 mg (0.017 mmol) Trifluoressigsäure--1-(2-aminoethyl)-1H-pyrrol-2,5-dion(1:1) in Gegenwart von 5.8 mg (0.015 mmol) HATU sowie 10 µl N,N-Diisopropylethylamin und anschließender Entschützung mit Trifluoressigsäure hergestellt. Es wurden 7 mg (78% d. Th. über 2 Stufen) erhalten.
LC-MS (Methode 1): Rₜ = 0.83 min; MS (EIpos): m/z = 708 [M+H]⁺.

### Intermediat F87

### Trifluoressigsäure-(2S)-2-amino-4-[{(1R)-1-[4-benzyl-1-(2,5-difluorphenyl)-1H-pyrazol-3-yl]-2,2-dimethylpropyl}(glycoloyl)amino]-N-(3-{[2-(2,5-dioxo-2,5-dihydro-1H-pyrrol-1-yl)ethyl]amino}-3-oxopropyl)butanamid(1:1)

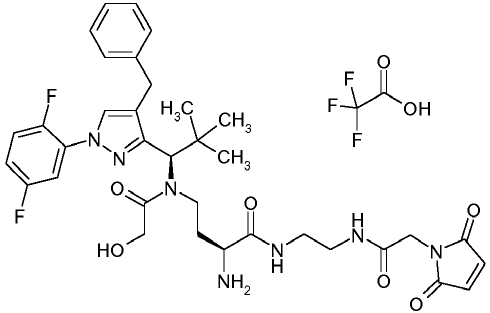

Die Titelverbindung wurde in Analogie zu Intermediat F2 ausgehend von 16 mg (0.025 mmol) Intermediat C49 durch Umsetzung mit 24 mg (0.076 mmol) von Intermediat L1 in Gegenwart von EDCI/HOBT und *N,N*-Diisopropylethylamin und anschließender Entschützung mit Trifluoressigsäure hergestellt. Es wurden 3 mg der Titelverbindung (14% d. Th. über 2 Stufen) erhalten.
LC-MS (Methode 1): Rₜ = 0.88 min; MS (EIpos): m/z = 694 [M+H]⁺.

### Intermediat F88

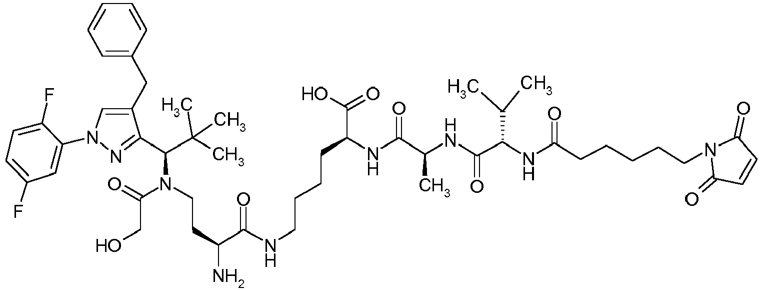

Die Verbindung wurde in Analogie zu Intermediat F8 hergestellt.
LC-MS (Methode 5): Rₜ = 2.97 min; MS (EIpos): m/z = 1006 [M+H]⁺.

### Intermediat F89

### Trifluoressigsäure--N-{(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}-beta-alanyl-L-alanyl-N⁵-carbamoyl-N-[4-(2,5-dioxo-2,5-dihydro-1H-pyrrol-1-yl)phenyl]-L-omithinamid(1:1)

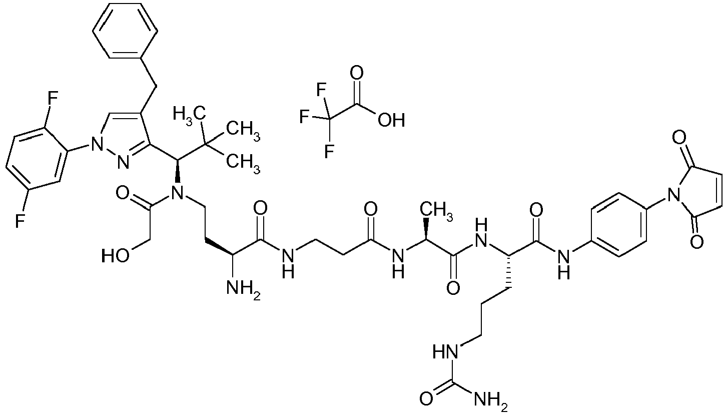

Die Titelverbindung wurde ausgehend von 8 mg (0.012 mmol) Intermediat C51 durch Umsetzung mit 7.4 mg (0.014 mmol) Intermediat L8 in Gegenwart von 5.8 mg (0.015 mmol) HATU sowie 10 µl N,N-Diisopropylethylamin und anschließender Entschützung mit Trifluoressigsäure hergestellt. Es wurden 10 mg (78% d. Th. über 2 Stufen) erhalten.
LC-MS (Methode 1): Rₜ = 0.87 min; MS (EIpos): m/z = 984 [M+H]⁺.

### Intermediat F90

### N-[6-(2,5-Dioxo-2,5-dihydro-1H-pyrrol-1-yl)hexanoyl]-L-valyl-N⁵-carbamoyl-L-ornithyl-N⁶-{(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}-L-lysin-trifluoressigsäure(1:1)

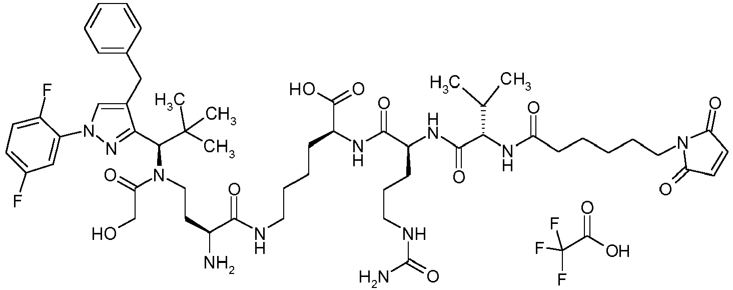

Die Titelverbindung wurde ausgehend von 11 mg (0.018 mmol) Intermediat C49 durch Umsetzung mit 13.7 mg (0.018 mmol) Intermediat L17 in Gegenwart von 34 mg (0.089 mmol) HATU sowie 19 µl N,N-Diisopropylethylamin und anschließender Entschützung mit Trifluoressigsäure hergestellt. Es wurden 7.5 mg (35% d. Th. über 2 Stufen) erhalten.
LC-MS (Methode 8): Rₜ = 6.78 min; MS (EIpos): m/z = 1092 [M+H]⁺.

### Intermediat F91

### Trifluoressigsäure--(2S)-2-amino-4-[{(1R)-1-[4-benzyl-1-(2,5-difluorphenyl)-1H-pyrazol-3-yl]-2,2-dimethylpropyl}(glycoloyl)amino]-N-[2-(2,5-dioxo-2,5-dihydro-1H-pyrrol-1-yl)ethyl]butanamid(1:1)

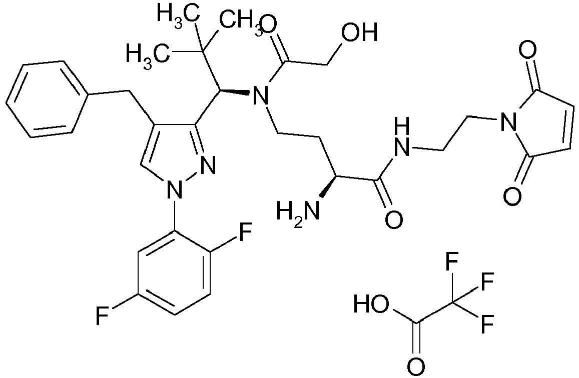

9.3 mg (0.01 mmol) tert-Butyl-[(2S)-4-[{(1R)-1-[4-benzyl-1-(2,5-difluorphenyl)-1H-pyrazol-3-yl]-2,2-dimethylpropyl}(glycoloyl)amino]-1-{[2-(2,5-dioxo-2,5-dihydro-1H-pyrrol-1-yl)ethyl]amino}-1-oxobutan-2-yl]carbamat wurden in 2 ml Dichlormethan gelöst und mit 740 mg (6.49 mmol, 0.50 ml) Trifluoressigsäure versetzt und 1.5 h bei RT gerührt. Anschließend wurde das Reaktionsgemisch eingeengt und der Rückstand in Acetonitril und Wasser aufgenommen und lyophilisiert. Es wurden 9.2 mg (96% d. Th.) der Zielverbindung erhalten.
LC-MS (Methode 1): Rₜ = 0.88 min; MS (EIpos): m/z = 637 [M+H]⁺.

### Intermediat F104

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-(2-{[(2,5-dioxo-2,5-dihydro-1H-pyrrol-1-yl)acetyl]amino}ethyl)butanamid(1:1)

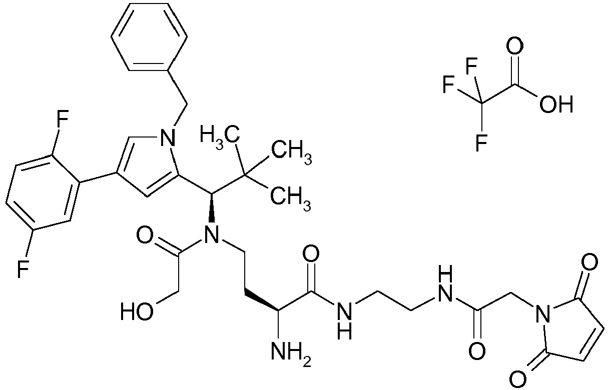

10 mg (0.014 mmol) von Intermediat C53 wurden in 3.3 mL DMF gelöst und mit 8.5 mg (0.027 mmol) von Intermediat L1 sowie mit 7.8 mg (0.02 mmol) HATU und 12 µl N,N-Diisopropylethylamin versetzt. Der Ansatz wurde 15 min bei RT gerührt und anschließend eingeengt. Der Rückstand wurde durch präparative HPLC gereinigt und nach Lyophilisation wurden 5.6 mg (38% d. Th.) der geschützten Zwischstufe erhalten.
LC-MS (Methode 1): Rₜ = 1.32 min; MS (ESIpos): m/z = 915 (M+H)⁺.

5.6 mg (0.006 mmol) dieses Intermediats wurden in 2 ml DMF aufgenommen und mit 69 mg (0.61 mmol) 1,4-Diazabicyclo(2.2.2)octan versetzt. Der Ansatz wurde 2 h im Ultraschallbad behandelt. Anschließend wurden 35 µl Essigsäure zugegeben und der Ansatz im Hochvakuum eingeengt. Der Rückstand wurde durch präparative HPLC gereinigt. Es wurden 2.4 mg (48% d. Th.) der Titelverbindung erhalten.
LC-MS (Methode 1): Rₜ = 0.84 min; MS (EIpos): m/z = 693 [M+H]⁺.
HPLC (Methode 11): Rₜ = 1.91 min;

Alternativ wurde die Titelverbindung auch ausgehend von Intermediat C58 hergestellt. 15 mg (0.023 mmol) von Intermediat C58 wurden zunächst mit 11 mg (0.036 mmol) Intermediat L1 in Gegenwart von 13 mg (0.034 mmol) HATU sowie 10 µl N,N-Diisopropylethylamin umgesetzt. Nach 60 min Rühren bei RT wurde eingeengt und der Rückstand durch präparative HPLC gereinigt. Es wurden 12.3 mg (63% d. Th.) des geschützten Intermediats erhalten.
LC-MS (Methode 1): Rₜ = 1.3 min; MS (EIpos): m/z = 837 [M+H]⁺.

Im zweiten Schritt wurde dieses Intermediat in 3 mL 2,2,2-Trifluorethanol gelöst. Es wurden 12 mg (0.088 mmol) Zinkchlorid zugegeben und der Ansatz 2 h bei 50°C gerührt. Anschließend wurden 26 mg (0.088 mmol) Ethylendiamin-N,N,N',N'-tetraessigsäure sowie 2 mL einer 0.1%-igen wässrigen Trifluoressigsäurelösung zugesetzt. Der Ansatz wurde mittels präparativer HPLC gereinigt. Nach Einengen der entsprechenden Fraktionen und Lyophilisation des Rückstands aus Acetonitril/Wasser wurden 8.1 mg (68% d.Th.) der Titelverbindung erhalten.
LC-MS (Methode 1): Rₜ = 0.89 min; MS (ESIpos): m/z = 693 (M+H)⁺.

### Intermediat F106

### Trifluoressigsäure--N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl-L-alanyl-N⁵-carbamoyl-N-[4-(2,5-dioxo-2,5-dihydro-1H-pyrrol-1-yl)phenyl]-L-omithinamid(1:1)

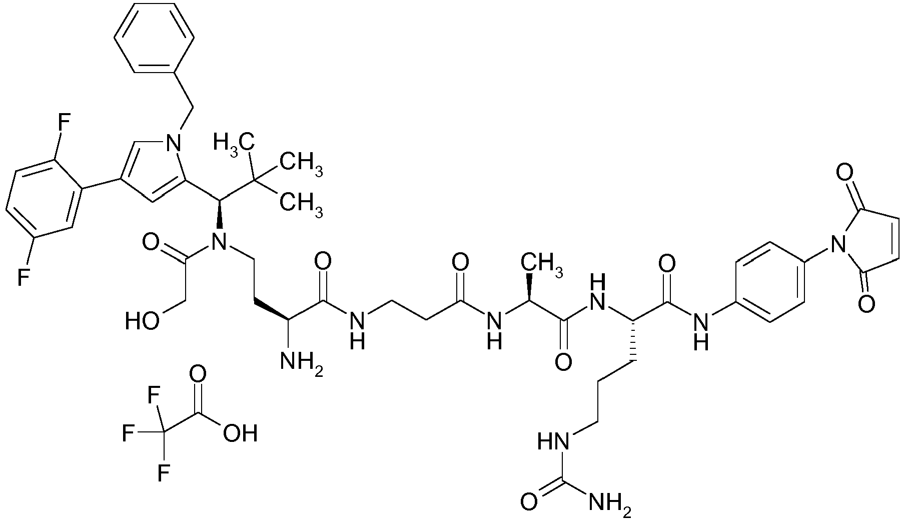

Die Titelverbindung wurde in Analogie zu Intermediat F104 ausgehend von Intermediat C53 und Intermediat L47 hergestellt.
HPLC (Methode 11): Rₜ = 1.85 min;
LC-MS (Methode 1): Rₜ = 0.86 min; MS (ESIpos): m/z = 983 (M+H)⁺.

### Intermediat F108

### Trifluoressigsäure--(1R,2S)-2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

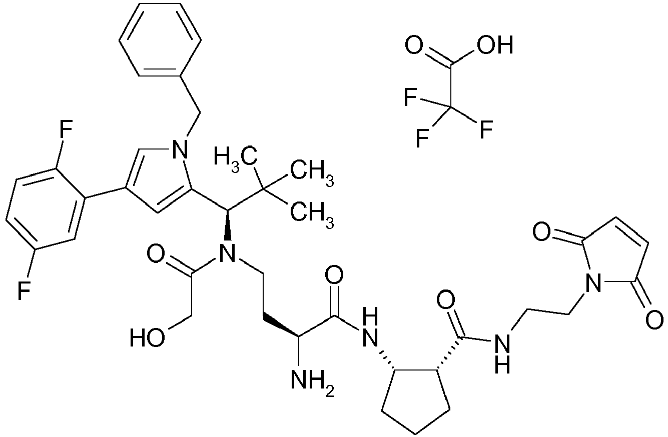

Die Titelverbindung wurde in Analogie zu Intermediat F104 aus 20 mg (0.027 mmol) Intermediat C53 und 24 mg (0.054 mmol) von Intermediat L48 hergestellt. Es wurden 3 mg (14% d. Th. über 2 Stufen) erhalten.
LC-MS (Methode 1): Rₜ = 0.93 min; MS (EIpos): m/z = 747 [M+H]⁺.

### Intermediat F109

### Trifluoressigsäure-(2S)-2-amino-4-[{(1R)-1-[4-benzyl-1-(2,5-difluorphenyl)-1H-pyrazol-3-yl]-2,2-dimethylpropyl}(glycoloyl)amino]-N-{2-[(bromacetyl)amino]ethyl}butanamid(1:1)

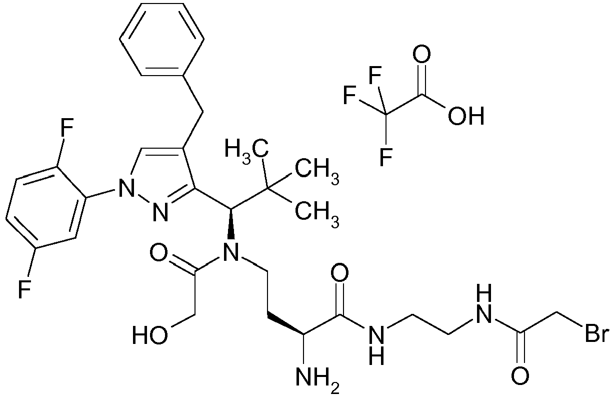

17 mg (0.026 mmol) von Intermediat C57 wurden in 3 mL DMF aufgenommen und mit 7 mg (0.027 mmol) von kommerziell erhältlichem 1-(2-Bromacetoxy)pyrrolidin-2,5-dion in Gegenwart von 14 µl *N,N*-Diisopropylethylamin umgesetzt. Nach 15min Rühren bei RT wurde eingeengt und der Rückstand durch präparative HPLC gereinigt. Es wurden 7 mg (33% d. Th.) dieser Zwischenstufe erhalten.
LC-MS (Methode 1): Rₜ = 1.29 min; MS (ESIpos): m/z = 777 und 779 (M+H)⁺.

Dieses Intermediat wurde in 1 mL Dichlormethan aufgenommen und mit 1 mL Trifluoressigsäure entschützt. Nach Einengen und Lyophilisation aus Acetonitril/Wasser wurden 6 mg (88% d.Th.) der Titelverbindung erhalten.
LC-MS (Methode 1): Rₜ = 0.86 min; MS (ESIpos): m/z = 677/679 (M+H)⁺.

### Intermediat F112

### Trifluoressigsäure--(1S,3R)-3-({(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

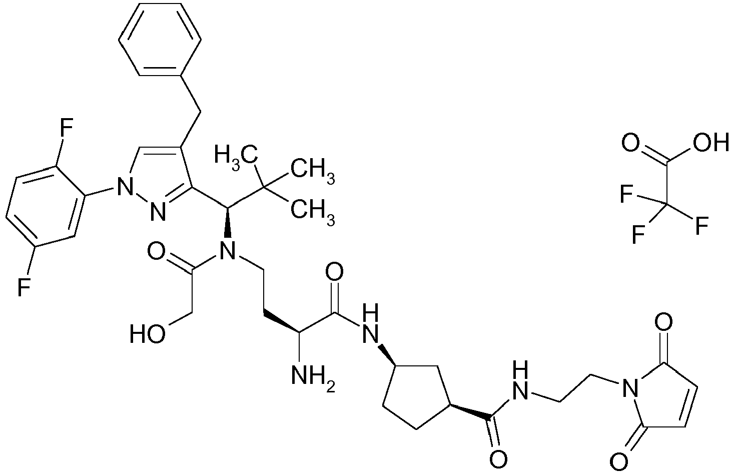

Die Titelverbindung wurde ausgehend von 15 mg (0.024 mmol) Intermediat C49 durch Umsetzung mit 18 mg (0.049 mmol) Intermediat L50 in Gegenwart von 14 mg (0.037 mmol) HATU sowie 21 µl N,N-Diisopropylethylamin und anschließender Entschützung mit Trifluoressigsäure hergestellt. Es wurden 12 mg (51% d. Th. über 2 Stufen) erhalten.
LC-MS (Methode 1): Rₜ = 0.89 min; MS (EIpos): m/z = 748 [M+H]⁺.

### Intermediat F113

### Trifluoressigsäure--(1S,3R)-3-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

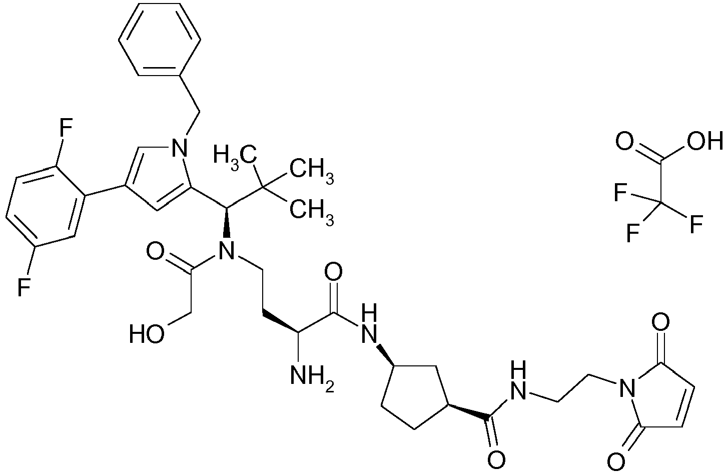

Die Titelverbindung wurde ausgehend von 15 mg (0.019 mmol) Intermediat C53 durch Umsetzung mit 14 mg (0.038 mmol) Intermediat L50 in Gegenwart von 11 mg (0.029 mmol) HATU sowie 17 µl N,N-Diisopropylethylamin und anschließender Entschützung mit 133 mg DABCO in 2 ml DMF hergestellt. Nach HPLC-Reinigung wurden 4 mg (24% d. Th. über 2 Stufen) erhalten.
LC-MS (Methode 5): Rₜ = 2.77 min; MS (EIpos): m/z = 747 [M+H]⁺.

### Intermediat F115

### Trifluoressigsäure--(1R,3R)-3-({(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

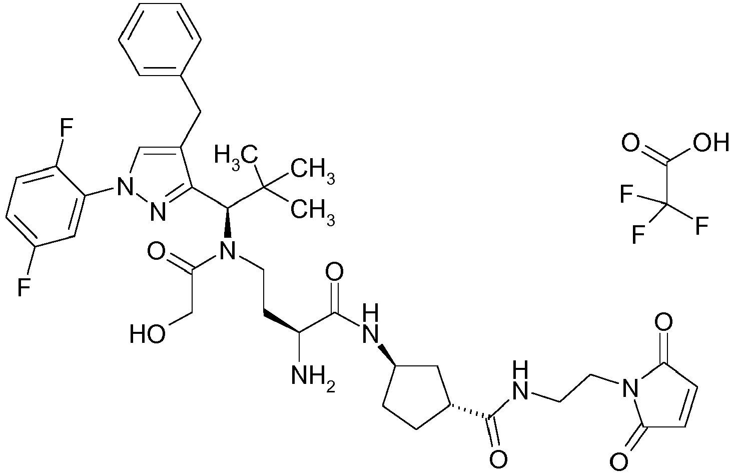

Die Titelverbindung wurde ausgehend von 15 mg (0.024 mmol) Intermediat C49 durch Umsetzung mit 18 mg (0.047 mmol) Intermediat L51 in Gegenwart von 13 mg (0.035 mmol) HATU sowie 21 µl N,N-Diisopropylethylamin und anschließender Entschützung mit Trifluoressigsäure hergestellt. Es wurden 12 mg (51% d. Th. über 2 Stufen) erhalten.
LC-MS (Methode 1): Rₜ = 0.87 min; MS (EIpos): m/z = 748 [M+H]⁺.

### Intermediat F116

### Trifluoressigsäure--(1R,3R)-3-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

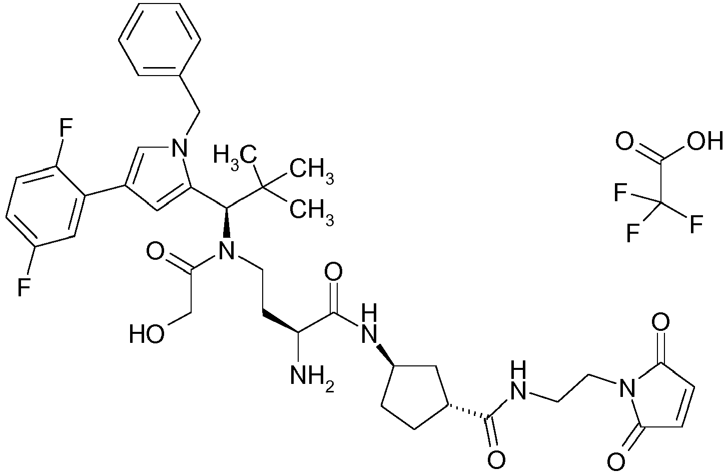

Die Titelverbindung wurde ausgehend von 11 mg (0.014 mmol) Intermediat C53 durch Umsetzung mit 11 mg (0.028 mmol) Intermediat L51 in Gegenwart von 8 mg (0.021 mmol) HATU sowie 12 µl N,N-Diisopropylethylamin und anschließender Entschützung mit 87 mg DABCO in 2 ml DMF hergestellt. Nach HPLC-Reinigung wurden 3.3 mg (28% d. Th. über 2 Stufen) erhalten.
LC-MS (Methode 1): Rₜ = 0.92 min; MS (EIpos): m/z = 747 [M+H]⁺.

### Intermediat F117

### Trifluoressigsäure--N-[(3S)-3-amino-4-{2-[6-(2,5-dioxo-2,5-dihydro-1H-pyrrol-1-yl)hexanoyl]hydrazino}-4-oxobutyl]-N-{(1R)-1-[4-benzyl-1-(2,5-difluorphenyl)-1H-pyrazol-3-yl]-2,2-dimethylpropyl}-2-hydroxyacetamid (1:1)

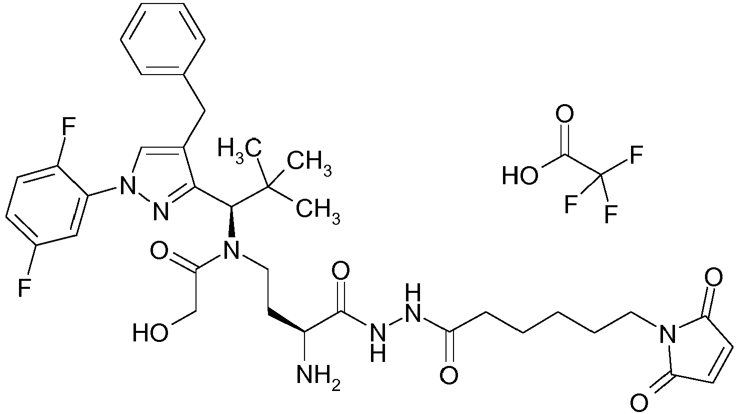

Die Titelverbindung wurde nach klassischen Methoden der Peptidchemie ausgehend von Intermediat C49 hergestellt. C49 wurde zunächst mit 9H-Fluoren-9-ylmethyl-hydrazincarboxylat in Gegenwart von HATU gekuppelt. Anschließend wurde die Fmoc-Schutzgruppe mit Piperidin in DMF entfernt und das erhaltene Hydrazid mit 6-(2,5-Dioxo-2,5-dihydro-1H-pyrrol-1-yl)hexansäure in Gegenwart von HATU gekuppelt. Im letzten Schritt wurde die Boc-Schutzgruppe mit TFA in Dichlormethan entfernt.
LC-MS (Methode 1): Rₜ = 0.93 min; MS (EIpos): m/z = 722 [M+H]⁺.

### Intermediat F118

### Trifluoressigsäure--N-[(3S)-3-amino-4-{2-[6-(2,5-dioxo-2,5-dihydro-1H-pyrrol-1-yl)hexanoyl]hydrazino}--4-oxobutyl]-N-{(1R)-1-[1-benzyl-4-(2,5-difluorphenyl)-1H-pyrrol-2-yl]-2,2-dimethylpropyl}-2-hydroxyacetamid (1:1)

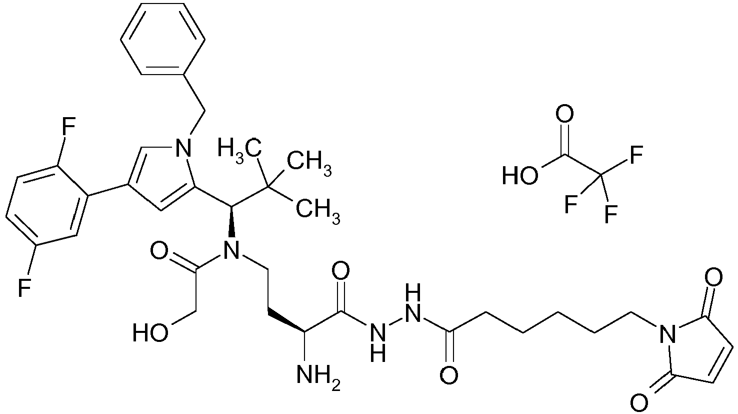

Die Titelverbindung wurde im ersten Schritt analog zu Intermediat F3 ausgehend von 15 mg (0.019 mmol) Intermediat C53 durch Kupplung mit kommerziell erhältlichem 6-(2,5-Dioxo-2,5-dihydro-1H-pyrrol-1-yl)hexanhydrazid in Gegenwart von HATU hergestellt. Anschließend erfolgte die Abspaltung der Fmoc-Schutzgrruppe mit 142 mg DABCO in DMF. Nach HPLC Reinigung wurden 3 mg (19 % d. Th.) der Titelverbindung erhalten.
LC-MS (Methode 1): Rₜ = 0.90 min; MS (EIpos): m/z = 721 [M+H]⁺.

### Intermediat F119

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-{2-[(bromacetyl)amino]ethyl}butanamid (1:1)

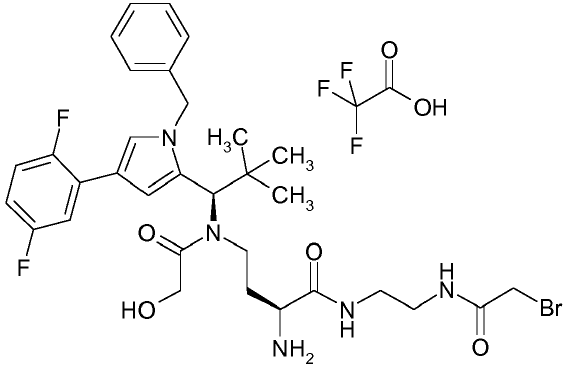

29 mg (0.044 mmol) von Intermediat C58 wurden in 3.4 mL DMF aufgenommen und mit 36 mg (0.087 mmol) von Intermediat L52 sowie mit 25 mg (0.065 mmol) HATU und 19 µl N,N-Diisopropylethylamin versetzt. Nach 60 min Rühren bei RT wurde eingeengt und der Rückstand durch präparative HPLC gereinigt. Es wurden 26.4 mg (73% d. Th.) der Zwischenstufe erhalten.
LC-MS (Methode 1): Rₜ = 1.34 min; MS (ESIpos): m/z = 820 und 822 (M+H)⁺.

Dieses Intermediat wurde in 3 mL 2,2,2-Trifluorethanol gelöst. Es wurden 6.5 mg (0.048 mmol) Zinkchlorid zugegeben und der Ansatz 4 h bei 50°C gerührt. Dann wurden 13.9 mg (0.048 mmol) Ethylendiamin-N,N,N',N'-tetraessigsäure sowie 2 mL einer 0.1%-igen wässrigen Trifluoressigsäurelösung zugesetzt. Der Ansatz wurde mittels präparativer HPLC gereinigt. Nach Einengen der entsprechenden Fraktionen und Lyophilisation des Rückstands aus Acetonitril/Wasser wurden 14.4 mg (58% d.Th.) der Titelverbindung erhalten.
LC-MS (Methode 1): Rₜ = 0.88 min; MS (ESIpos): m/z = 676 und 678 (M+H)⁺.

### Intermediat F121

### Trifluoressigsäure--(1S,3S)-3-({(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

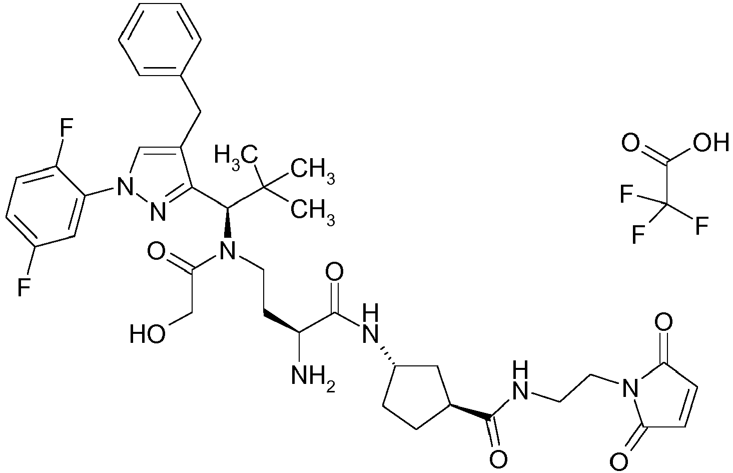

Die Titelverbindung wurde ausgehend von 10 mg (0.016 mmol) Intermediat C49 durch Umsetzung mit 11.5 mg (0.031 mmol) Intermediat L53 in Gegenwart von 9 mg (0.024 mmol) HATU sowie 14 µl N,N-Diisopropylethylamin und anschließender Entschützung mit Trifluoressigsäure hergestellt. Es wurden 9 mg (61% d. Th. über 2 Stufen) erhalten.
LC-MS (Methode 1): Rₜ = 0.84 min; MS (EIpos): m/z = 748 [M+H]⁺.

### Intermediat F122

### Trifluoressigsäure--(1S,3S)-3-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

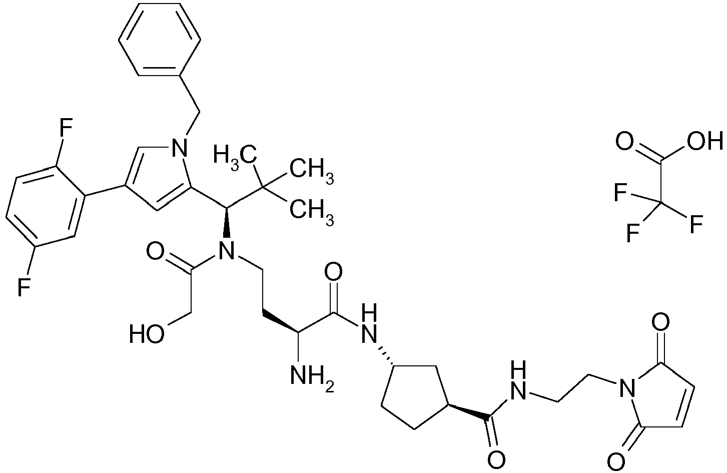

Die Titelverbindung wurde ausgehend von 15 mg (0.019 mmol) Intermediat C53 durch Umsetzung mit 14 mg (0.038 mmol) Intermediat L53 in Gegenwart von 11 mg (0.029 mmol) HATU sowie 17 µl N,N-Diisopropylethylamin und anschließender Entschützung mit 202 mg DABCO in 3 ml DMF hergestellt. Nach HPLC-Reinigung wurden 4 mg (24% d. Th. über 2 Stufen) erhalten.
LC-MS (Methode 1): Rₜ = 0.87 min; MS (EIpos): m/z = 747 [M+H]⁺.

### Intermediat F124

### Trifluoressigsäure--(1R,3S)-3-({(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

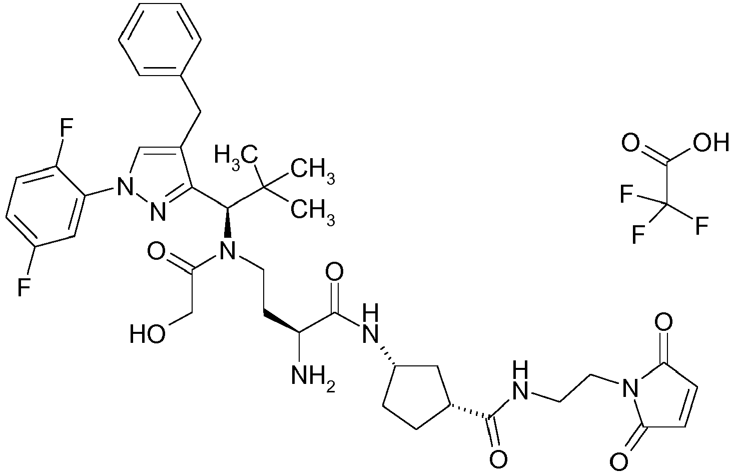

Die Titelverbindung wurde ausgehend von 10 mg (0.016 mmol) Intermediat C49 durch Umsetzung mit 11.5 mg (0.031 mmol) Intermediat L54 in Gegenwart von 9 mg (0.024 mmol) HATU sowie 14 µl N,N-Diisopropylethylamin und anschließender Entschützung mit Trifluoressigsäure hergestellt. Es wurden 9 mg (66% d. Th. über 2 Stufen) erhalten.
LC-MS (Methode 1): Rₜ = 0.84 min; MS (EIpos): m/z = 748 [M+H]⁺.

### Intermediat F125

### Trifluoressigsäure--(1R,3S)-3-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-[2-(2,5-dioxo-2,5-dihydro-1H-pyrrol-1-yl)ethyl]cyclopentancarboxamid(1:1)

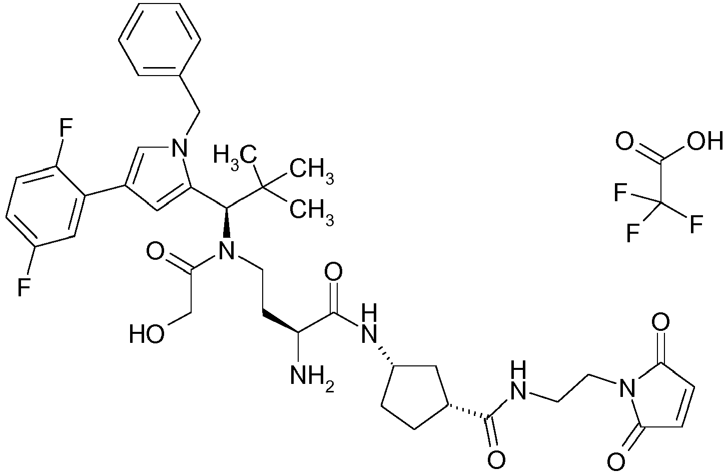

Die Titelverbindung wurde ausgehend von 15 mg (0.019 mmol) Intermediat C53 durch Umsetzung mit 14 mg (0.038 mmol) Intermediat L54 in Gegenwart von 11 mg (0.029 mmol) HATU sowie 17 µl N,N-Diisopropylethylamin und anschließender Entschützung mit 127 mg DABCO in 3 ml DMF hergestellt. Nach HPLC-Reinigung wurden 3 mg (17% d. Th. über 2 Stufen) erhalten.
LC-MS (Methode 4): Rₜ = 1.08 min; MS (EIpos): m/z = 769 [M+Na]⁺.

### Intermediat F126

### N-(Bromacetyl)-L-valyl-L-alanyl-N⁶-{(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}-L-lysin-trifluoressigsäure (1:1)

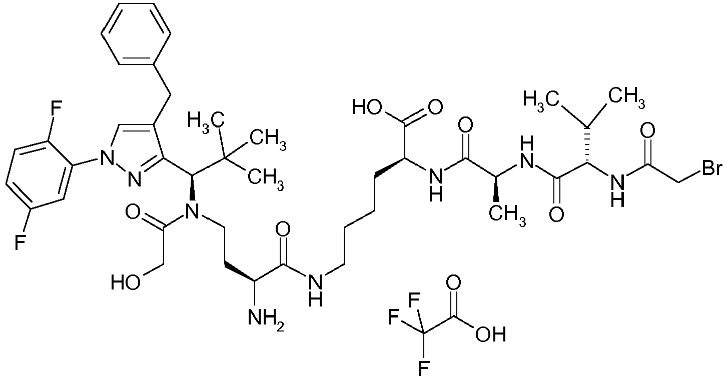

Die Titelverbindung wurde in Analogie zu Intermediat F110 aus 18 mg (0.027 mmol) von Intermediat C49 und 21 mg (0.027 mmol) von Intermediat L49 hergestellt. Es wurden 8.7 mg (30% d. Th. über 2 Stufen) der Titelverbindung erhalten.
HPLC (Methode 11): Rₜ = 1.94 min;
LC-MS (Methode 1): Rₜ = 0.86 min; MS (ESIpos): m/z = 933 und 935 (M+H)⁺.

### Intermediat F127

### Trifluoressigsäure--(2S)-2-amino-4-({(1R)-1-[1-benzyl-4-(2,5-difluorphenyl)-1H-pyrrol-2-yl]-2,2-dimethylpropyl}[(2S)-2-methoxypropanoyl]amino)-N-(2-{[(2,5-dioxo-2,5-dihydro-1H-pyrrol-1-yl)acetyl]amino}ethyl)butanamid (1:1)

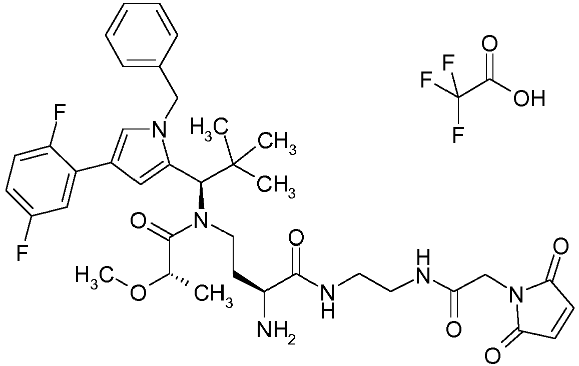

12 mg (0.015 mmol) von Intermediat C59 wurden in 2.4 mL DMF gelöst und mit 14.6 mg (0.046 mmol) von Intermediat L1 sowie mit 6 mg (0.031 mmol) 1-(3-Dimethylaminopropyl)-3-ethylcarbodiimid-Hydrochlorid, 5.9 mg (0.039 mmol) 1-Hydroxy-1*H*-benzotriazol-Hydrat und 8 µl *N,N-*Diisopropylethylamin versetzt. Nach 1 h Rühren bei RT wurde eingeengt und der Rückstand durch präparative HPLC gereinigt. 11 mg (70% d. Th.) dieser Zwischenstufe wurden erhalten.
LC-MS (Methode 1): Rₜ = 1.34 min; MS (ESIpos): m/z = 942 (M+H)⁺.

11 mg (0.011 mmol) dieses Intermediats wurden in 2 ml DMF aufgenommen und mit 123 mg (1.1 mmol) 1,4-Diazabicyclo(2.2.2)octan versetzt. Der Ansatz wurde 2 h im Ultraschallbad behandelt. Anschließend wurden 63 µl Essigsäure zugegeben und der Ansatz im Hochvakuum eingeengt. Der Rückstand wurde durch präparative HPLC gereinigt. Es wurden 2 mg (22% d. Th.) der Titelverbindung erhalten.
LC-MS (Methode 1): Rₜ = 0.89 min; MS (EIpos): m/z = 721 [M+H]⁺.
HPLC (Methode 11): Rₜ = 1.95 min;

### Intermediat F129

### N-[6-(2,5-Dioxo-2,5-dihydro-1H-pyrrol-1-yl)hexanoyl]-L-valyl-L-alanyl-N⁶-{[(1R,3S)-3-({(2S)-2-amino-4-[{(1R)-1-[4-benzyl-1-(2,5-difluorphenyl)-1H-pyrazol-3-yl]-2,2-dimethylpropyl} (glycoloyl)amino]butanoyl}amino)cyclopentyl]carbonyl}-L-lysin-trifluoressigsäure (1:1)

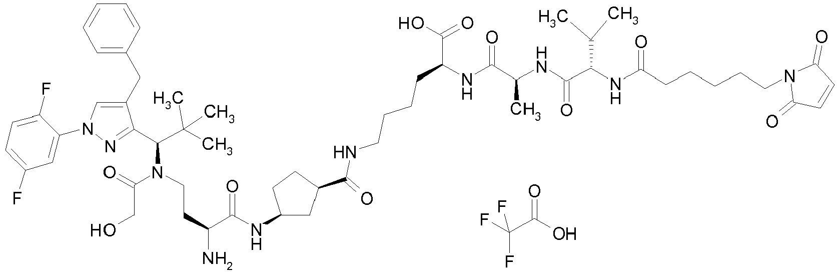

Die Titelverbindung wurde in Analogie zu Intermediat F128 ausgehend von 10 mg (0.016 mmol) Intermediat C49 durch Umsetzung mit 19 mg (0.024 mmol) Intermediat L56 in Gegenwart von 12 mg ( 0.031 mmol) HATU sowie 14 µl N,N-Diisopropylethylamin und anschließender Entschützung mit Trifluoressigsäure hergestellt. Es wurden 13.5 mg (70% d. Th. über 2 Stufen) erhalten.
LC-MS (Methode 1): Rₜ = 0.9 min; MS (EIpos): m/z = 1117 [M+H]⁺.

### Intermediat F142

### R/S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-homocystein-trifluoressigsäure(1:1)

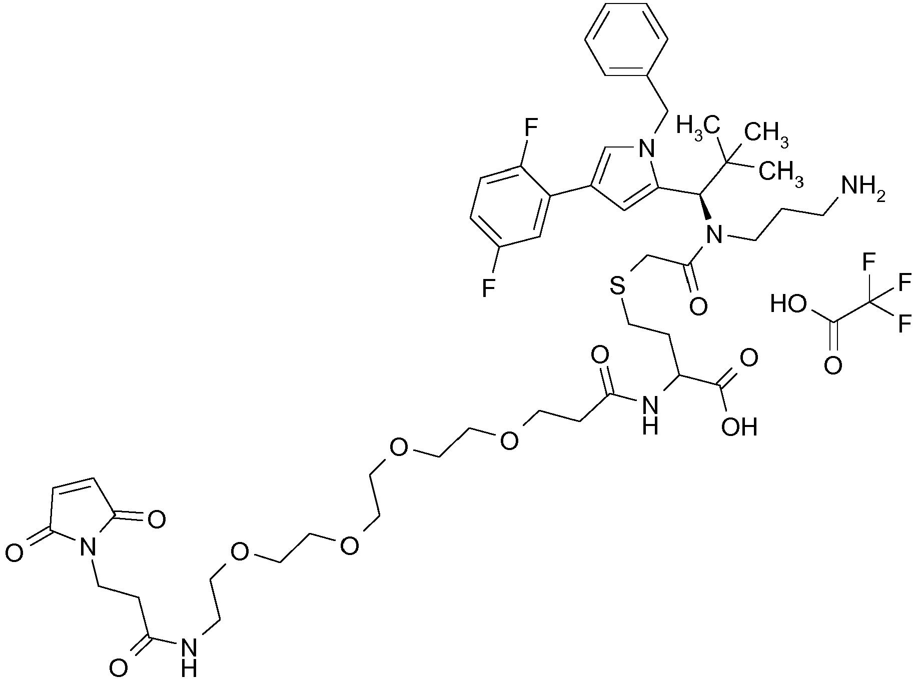

20.0 mg (23.7 µmol) R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-homocystein-trifluoressigsäure(1:1) und 13.4 mg (26.04 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid wurden in 1.0 mL DMF gelöst und mit 4.8 mg (47.34 µmol) 4-Methylmorpholin versetzt. Die Reaktionsmischung wurde über Nacht bei RT gerührt. Es wurden 3.6 mg (0.06 mmol) Essigsäure zugegeben und die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 12.4 mg (44 % d. Th.) der Verbindung R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-homocystein.
LC-MS (Methode 1): Rₜ = 1.30 min; MS (ESIpos): m/z = 1129 (M+H)⁺.

10.0 mg (8.85 µmol) R/S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-L-homocystein wurden in Trifluorethanol gelöst und mit 3.1 mg (22.71 µmol) Zinkdichlorid versetzt. Die Reaktionsmischung rührte über Nacht bei 50 °C. Die Reaktionsmischung wurde mit 3.9 mg (0.01 mmol) Ethylendiamin-N,N,N',N'-tetraessigsäure versetzt, kurz gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand mit etwas Wasser lyophilisiert. Man erhielt 7.6 mg (78 % d. Th.) der Titelverbindung.
LC-MS (Methode 1): Rₜ = 0.94 min; MS (ESIpos): m/z = 983 (M+H)⁺.
¹H-NMR (400 MHz, DMSO-d₆): δ [ppm] = 0.50 (m, 1H), 0.81 (s, 9H), 1.49 (m, 1H), 1.89 (m, 1H), 2.05 (m, 1H), 2.29-2.43 (m, 4H), 2.45-2.55 (m, 2H), 2.58-2.74 (m, 2H), 3.10-3.20 (m, 2H), 3.21-3.40 (m, 2H), 3.42-3.54 (m, 16H), 3.55-3.65 (m, 4H), 4.28 (m, 1H), 4.91 (dd, 1H), 5.18 (dd, 1H), 5.60 (s, 1H), 6.95 (m, 1H), 7.00 (s, 2H), 7.15-7.38 (m, 7H), 7.53 (s, 1H), 7.68 (m, 1H), 8.00 (m, 2H).

### Intermediat F143

### Trifluoressigsäure--6-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)-N-[2-(2,5-dioxo-2,5-dihydro-1H-pyrrol-1-yl)ethyl]hexanamid(1:1)

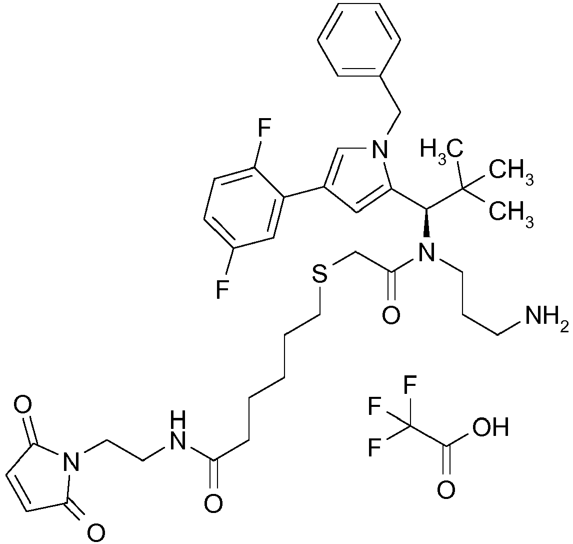

30.0 mg (0.05 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat und 13.5 mg (0.07 mmol) 6-(Acetylsulfanyl)hexansäure wurden in 2.0 mL Methanol mit einem Tropfen Wasser vorgelegt. 23.0 mg (0.17 mmol) Kaliumcarbonat wurden hinzugefügt. Die Reaktionsmischung wurde 4 h bei 50 °C gerührt. Die Reaktionsmischung wurde mit Ethylacetat versetzt. Die organische Phase wurde mit ges. NaCl-Lösung gewaschen und über Magnesiumsulfat getrocknet. Das Lösemittel wurde im Vakuum verdampft. Der Rückstand wurde mittles präp. RP-HPLC gereinigt (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 54.2 mg (90 % d. Th.) der Verbindung 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaicosan-20-säure.
LC-MS (Methode 1): Rₜ = 1.49 min; MS (ESIpos): m/z = 1106 (M+H)⁺.

54.0 mg (0.07 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaicosan-20-säure und 16.7 mg (0.09 mmol) 1-(2-Aminoethyl)-1H-pyrrol-2,5-dionhydrochlorid(1:1) wurden in 3.0 mL Acetonitril vorgelegt und mit 75.0 mg (0.58 mmol) N,N-Diisopropylethylamin versetzt. Es wurden 60.0 mg (0.09 mmol) T3P (50% in Acetonitril) zugegeben und über Nacht bei RT gerührt. Es wurde mit Wasser gequencht und die Reaktionsmischung direkt mittles präp. RP-HPLC gereinigt (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 42.8 mg (68 % d. Th.) der Verbindung 2-(Trimethylsilyl)ethyl-[3-({(1R)-1-[1-benzyl-4-(2,5-difluorphenyl)-1H-pyrrol-2-yl]-2,2-dimethylpropyl}{[(6-{[2-(2,5-dioxo-2,5-dihydro-1H-pyrrol-1-yl)ethyl]amino}-6-oxohexyl)sulfanyl]acetyl}amino)propyl]carbamat.
LC-MS (Methode 1): Rₜ = 1.48 min; MS (ESIpos): m/z = 866 (M+H)⁺.

20.0 mg (0.02 mmol) 2-(Trimethylsilyl)ethyl-[3-({(1R)-1-[1-benzyl-4-(2,5-difluorphenyl)-1H-pyrrol-2-yl]-2,2-dimethylpropyl}{[(6-{[2-(2,5-dioxo-2,5-dihydro-1H-pyrrol-1-yl)ethyl]amino}-6-oxohexyl)sulfanyl]acetyl}amino)propyl]carbamat wurden in 2.0 ml Trifluorethanol gelöst und mit 4.7 mg (0.04 mmol) Zinkdichlorid versetzt. Die Reaktionsmischung wurde über Nacht bei 50 °C gerührt und danach mit 10.1 mg (0.04 mmol) Ethylendiamin-N,N,N',N'-tetraessigsäure versetzt und 10 min gerührt. Wasser (0.1% TFA) wurde zugesetzt und die Reaktionsmischung mittles präp. RP-HPLC gereinigt (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 9.2 mg (48 % d. Th.) der Titelverbindung.
LC-MS (Methode 1): Rₜ = 0.98 min; MS (ESIpos): m/z = 722 (M+H)⁺.

### Intermediat F146

### R/S-[2-([(3S)-3-Amino-3-carboxypropyl]{(1R)-1-[1-benzyl-4-(2,5-difluorphenyl)-1H-pyrrol-2-yl]-2,2-dimethylpropyl}amino)-2-oxoethyl]-N-[19-(2,5-dioxo-2,5-dihydro-1H-pyrrol-1-yl)-17-oxo-4,7,10,13-tetraoxa-16-azanonadecan-1-oyl]homocystein-trifluoressigsäure(1:1)

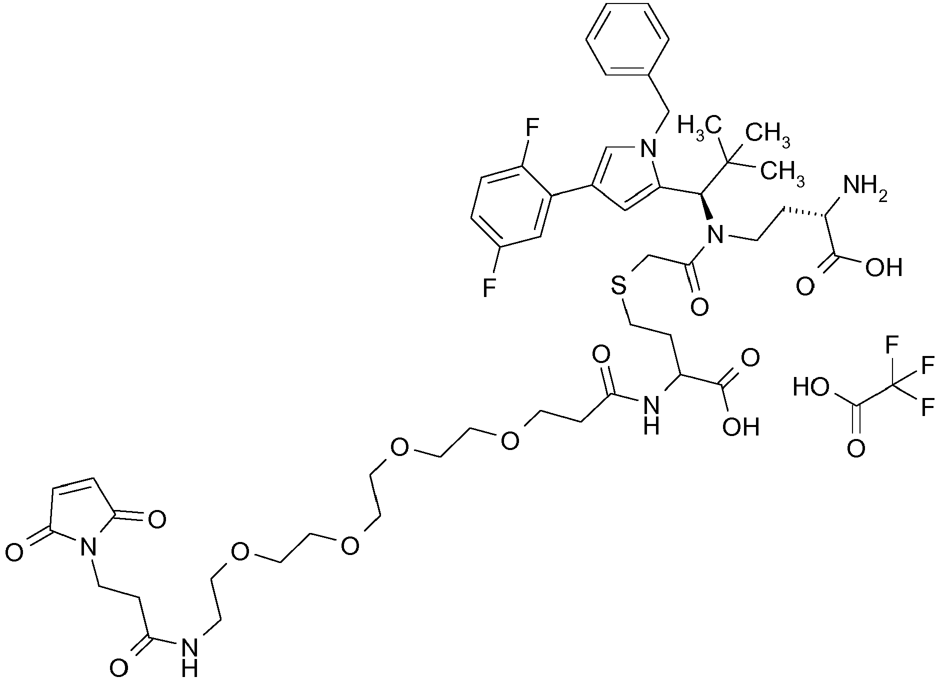

25.0 mg (28.12 µmol) R/S-[(8S)-11-{(1R)-1-[1-Benzyl-4-(2,5-difluorphenyl)-1H-pyrrol-2-yl]-2,2-dimethylpropyl}-8-carboxy-2,2-dimethyl-6,12-dioxo-5-oxa-7,11-diaza-2-silatridecan-13-yl]homocystein (Intermediat C12) und 15.9 mg (30.93 µmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid wurden in 2.0 mL DMF gelöst und mit 11.4 mg (112.48 µmol) 4-Methylmorpholin versetzt. Die Reaktionsmischung wurde über Nacht bei RT gerührt. Es wurden 7.6 mg (0.13 mmol) Essigsäure zugegeben und die Reaktionsmischung wurde direkt mittles präp. RP-HPLC gereinigt (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 23.9 mg (59 % d. Th.) der Verbindung R/S-[(8S)-11-{(1R)-1-[1-Benzyl-4-(2,5-difluorphenyl)-1H-pyrrol-2-yl]-2,2-dimethylpropyl}-8-carboxy-2,2-dimethyl-6,12-dioxo-5-oxa-7,11-diaza-2-silatridecan-13-yl]-N-[19-(2,5-dioxo-2,5-dihydro-1H-pyrrol-1-yl)-17-oxo-4,7,10,13-tetraoxa-16-azanonadecan-1-oyl]homocystein.
LC-MS (Methode 1): Rₜ = 1.26 min; MS (ESIpos): m/z = 1173 (M+H)⁺.

11.8 mg (8.23 µmol) R/S-[(8S)-11-{(1R)-1-[1-Benzyl-4-(2,5-difluorphenyl)-1H-pyrrol-2-yl]-2,2-dimethylpropyl}-8-carboxy-2,2-dimethyl-6,12-dioxo-5-oxa-7,11-diaza-2-silatridecan-13-yl]-N-[19-(2,5-dioxo-2,5-dihydro-1H-pyrrol-1-yl)-17-oxo-4,7,10,13-tetraoxa-16-azanonadecan-1-oyl]homocystein wurden in Trifluorethanol gelöst und mit 1.7 mg (12.35 µmol) Zinkdichlorid versetzt. Die Reaktionsmischung rührte über Nacht bei 50 °C. Die Reaktionsmischung wurde mit 3.6 mg (0.01 mmol) Ethylendiamin-N,N,N',N'-tetraessigsäure versetzt, kurz gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 5.8 mg (62 % d. Th.) der Titelverbindung.
LC-MS (Methode 4): Rₜ = 1.20 min; MS (ESIpos): m/z = 1029 (M+H)⁺.

### Intermediat F153

### Trifluoressigsäure --(2S)-2-amino-4-({(1R)-1-[1-benzyl-4-(2,5-difluorphenyl)-1H-pyrrol-2-yl]-2,2-dimethylpropyl}[(2S)-2-hydroxypropanoyl]amino)-N-(2-{[(2,5-dioxo-2,5-dihydro-1H-pyrrol-1-yl)acetyl]amino}ethyl)butanamid (1:1)

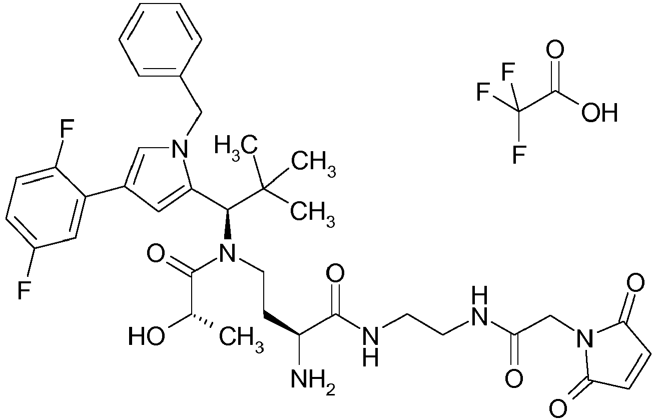

Die Synthese erfolgte in Analogie zu Intermediat F104 ausgehend von Intermediat C60.
LC-MS (Methode 1): Rₜ = 1.1 min; MS (ESIpos): m/z = 707 (M+H)⁺.

### Intermediat F155

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-N²-{N-[6-(2,5-dioxo-2,5-dihydro-1H-pyrrol-1-yl)hexanoyl]-L-valyl-L-alanyl}-L-lysin-trifluoressigsäure (1:1)

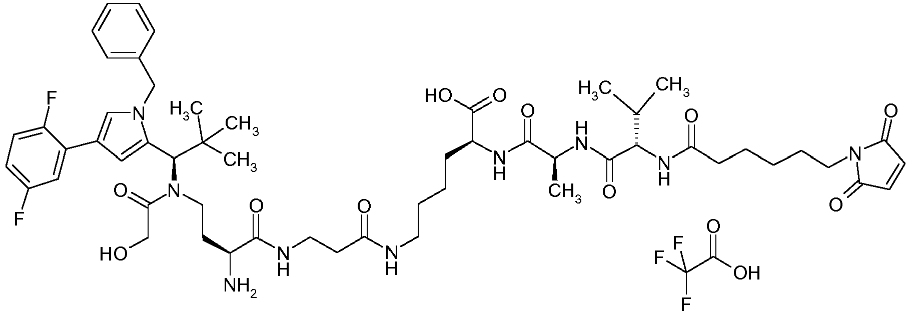

Die Titelverbindung wurde durch Kupplung von 14 mg (0.019 mmol) des Intermediats C61 mit 15 mg (0.021 mmol) von Intermediat L61 in Gegenwart von 8.7 mg (0.023 mmol) HATU sowie 17 µl N,N-Diisopropylethylamin und anschließender Entschützung mit Zinkchlorid in Trifluorethanol wie in Intermediat F119 beschrieben hergestellt. Nach Reinigung durch präparative HPLC wurden 13 mg (59% d. Th. über 2 Stufen) der Titelverbindung erhalten.
LC-MS (Methode 1): Rₜ = 0.86 min; MS (ESIpos): m/z = 1076 (M+H)⁺.

### Intermediat F156

### N-(Bromacetyl)-L-valyl-L-alanyl-N⁶-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-L-lysin-trifluoressigsäure (1:1)

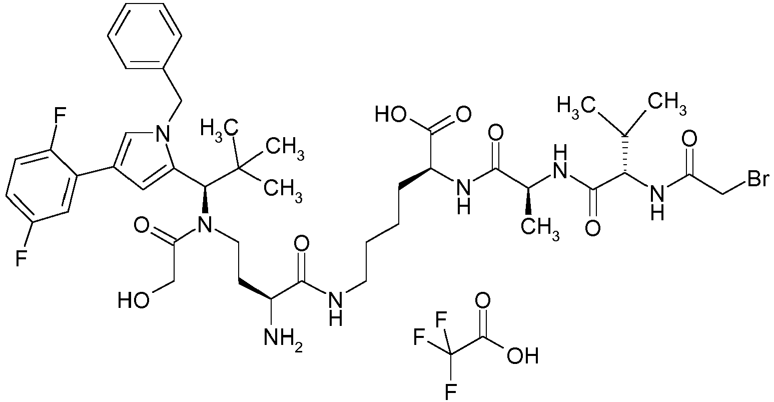

Zunächst wurde ausgehend von N2-[(Benzyloxy)carbonyl]-N6-(tert-butoxycarbonyl)-L-lysin nach klassischen Methoden der Peptidchemie (Veresterung mit 2-(Trimethylsilylethanol mittels EDCI/DMAP, Hydrogenolyse, Kupplung mit N-[(Benzyloxy)carbonyl]-L-valyl-L-alanin in Gegenwart von HATU und erneute Hydrogenolyse) das Tripeptidderivat 2-Trimethylsilyl)ethyl-L-valyl-L-alanyl-N6-(tert-butoxycarbonyl)-L-lysinat hergestellt.

84mg (0.163 mmol) von diesem Intermediat wurden in 2.5 ml DMF aufgenommen und mit 58 mg (0.244 mmol) 1-(2-Bromacetoxy)pyrrolidin-2,5-dion versetzt. Nach 10min Rühren bei RT wurde eingeengt, der Rückstand in Acetonitril/Wasser 1:1 aufgenommen und mit Trifluoressigsäure auf pH 2 gebracht und durch präparative HPLC gereinigt. Nach Einengen der entsprechenden Fraktionen wurde der Rückstand in 15 ml einer 5%-igen Trifluoressigsäurelösung in DCM aufgenommen und 2 h bei RT gerührt. Anschließend wurde bei leichter Kühlung eingeengt und der Rückstand aus Acetonitril/Wasser 1:1 lyophilisiert. Von diesem Intermediat wurden 53mg (50% d. Th.) über 2 Stufen erhalten.
LC-MS (Methode 1): Rₜ = 0.72 min; MS (ESIpos): m/z = 537 und 539 (M+H)⁺.

Zur Synthese der Titelverbindung wurden 18 mg (0.027 mmol) von diesem Intermediat in 4 ml DMF aufgenommen, mit 16 mg (0.025 mmol) Intermediat C61 sowie mit 19 mg HATU und 9 µL N,N-Diisopropylethylamin versetzt. Nach 5 min Rühren bei RT wurden einige Tropfen Trifluoressigsäure zugegeben und der Ansatz mittels präparativer HPLCC gereinigt. Nach Einengen der entsprechenden Fraktionen und Lyophilsation aus Acetonitril/Wasser 1:1 wurde das erhaltene Intermediat in 3 mL 2,2,2-Trifluorethanol gelöst. Nach Zugabe von 4.8 mg (0.035 mmol) Zinkchlorid wurde der Ansatz 2.5 h bei 50°C gerührt. Dann wurden 10 mg (0.035 mmol) Ethylendiamin-N,N,N',N'-tetraessigsäure hinzugegeben und der Ansatz mit Acetonitril/Wasser verdünnt und filtriert. Die Reinigung erfolgte mittels präparativer HPLC. Nach Einengen der entsprechenden Fraktionen und Lyophilisation des Rückstands aus Acetonitril/Wasser wurden 3.2 mg (13% d.Th.) der Titelverbindung über 2 Stufen erhalten.
HPLC (Methode 11): Rₜ = 1.94 min;
LC-MS (Methode 5): Rₜ = 2.79 min; MS (ESIpos): m/z = 932 und 934 (M+H)⁺.

### Intermediat F163

### N-[6-(2,5-Dioxo-2,5-dihydro-1H-pyrrol-1-yl)hexanoyl]-L-valyl-L-alanyl-N⁶-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-L-lysin-trifluoressigsäure (1:1)

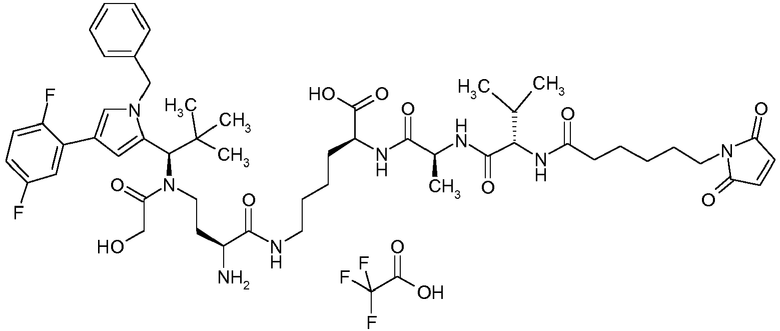

Die Titelverbindung wurde durch Kupplung von 37 mg (0.056 mmol) Intermediat C58 und 41 mg (0.056 mmol) Intermediat L61 in Gegenwart von HATU und anschließende Deblockierung mit Zinkchlorid wie in Intermediat F119 beschrieben, hergestellt. Es wurden 12 mg (19% d.Th. über 2 Stufen) der Titelverbindung erhalten.
HPLC (Methode 11): Rₜ = 1.49 min;
LC-MS (Methode 1): Rₜ = 0.89 min; MS (ESIpos): m/z = 1005 (M+H)⁺.

### Intermediat F164

### N-[6-(2,5-Dioxo-2,5-dihydro-1H-pyrrol-1-yl)hexanoyl]-L-valyl-N⁵-carbamoyl-L-ornithyl-N⁶-{(2S)-2-amino-4-[{(1R)-1-[1-benzyl-4-(2,5-difluorphenyl)-1H-pyrrol-2-yl]-2,2-dimethylpropyl} (glycoloyl)amino]butanoyl}-L-lysin -trifluoressigsäure (1:1)

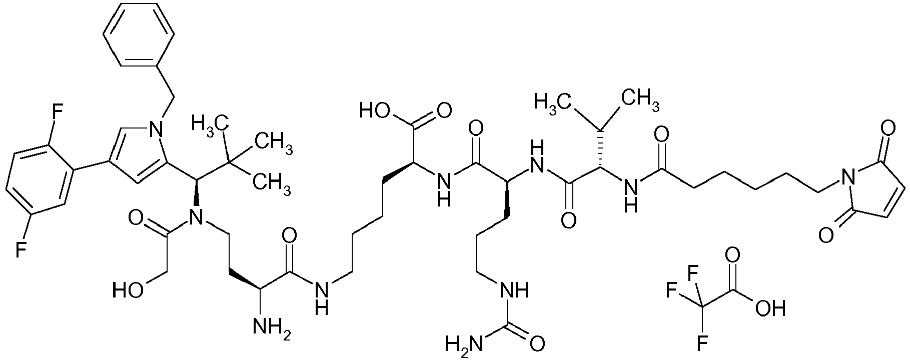

Die Titelverbindung wurde in Analogie zu Intermediat F155 durch Kupplung von 20 mg (0.030 mmol) Intermediat C58 mit 27 mg (0.033 mmol) von Intermediat L62 in Gegenwart von HATU und *N,N-*Diisopropylethylamin und anschließender Entschützung mittels Zinkchlorid in Trifluorethanol hergestellt.
HPLC (Methode 11): Rₜ = 1.92 min;
LC-MS (Methode 1): Rₜ = 0.87 min; MS (ESIpos): m/z = 1091 (M+H)⁺.

### Intermediat F165

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-N²-{N-[6-(2,5-dioxo-2,5-dihydro-1H-pyrrol-1-yl)hexanoyl]-L-valyl-N⁵-carbamoyl-L-ornithyl}-L-lysin-trifluoressigsäure (1:1)

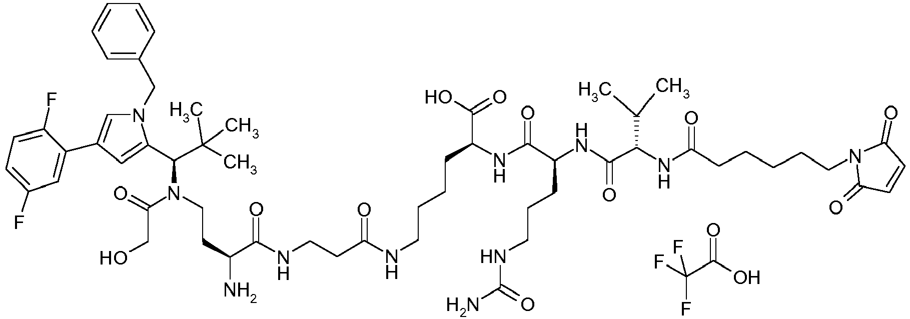

Die Titelverbindung wurde in Analogie zu Intermediat F155 durch Kupplung von 15 mg (0.021 mmol) Intermediat C61 mit 18 mg (0.023 mmol) Intermediat L62 in Gegenwart von HATU und anschließender Entschützung mit Zinkchlorid in Trifluorethanol hergestellt.
LC-MS (Methode 1): Rₜ = 0.88 min; MS (ESIpos): m/z = 1162 (M+H)⁺.

### Intermediat F166

### N-[6-(2,5-Dioxo-2,5-dihydro-1H-pyrrol-1-yl)hexanoyl]-L-valyl-N⁵-carbamoyl-L-ornithyl-N⁶-{[(1R,3S)-3-({(2S)-2-amino-4-[{(1R)-1-[1-benzyl-4-(2,5-difluorphenyl)-1H-pyrrol-2-yl]-2,2-dimethylpropyl} (glycoloyl)amino]butanoyl}amino)cyclopentyl]carbonyl} -L-lysin -trifluoressigsäure (1:1)

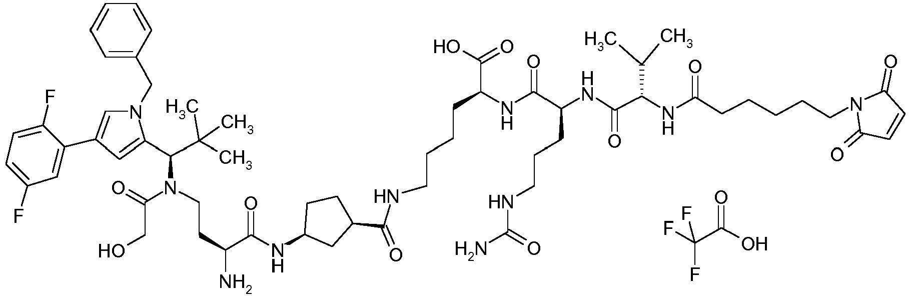

Zunächst wurde ausgehend von kommerziell erhältlicher (1R, 3S)-3-[(tert-Butoxycarbonyl) amino]cyclopentancarbonsäure nach klassischen Methoden der Peptidchemie durch Veresterung mit Benzylalkohol mittels EDCI/DMAP und anschließender Abspaltung der tert Butoxycarbonylschutzgruppe mit TFA in DCM Trifluoressigsäure-benzyl-(1R, 3S)-3-aminocyclopentancarboxylat (1:1) hergestellt.

51mg (0.076 mmol) von diesem Intermediat wurden in 6 ml DMF aufgenommen und mit 50 mg (0.076 mmol) von Intermediat C58 in Gegenwart von HATU und *N,N*-Diisopropylethylamin gekuppelt. Nach Reinigung durch präparative HPLC wurde das Intermediat in Methanol aufgenommen und über 10%-igem Palladium auf Aktivkohle 2 h lang bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Dioxan wurden 21 mg (34% d.Th. über 2 Stufen) von (1R,3S)-3-{[(2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl) ethoxy]carbonyl}amino)butanoyl]amino}cyclopentancarbonsäure erhalten.

Die Titelverbindung wurde dann in Analogie zu Intermediat F155 durch Kupplung von 10.5 mg (0.013 mmol) dieses Intermediats mit 11.4 mg (0.014 mmol) von Intermediat L62 in Gegenwart von HATU und anschließender Entschützung mit Zinkchlorid in Trifluorethanol hergestellt. Nach Reinigung durch präparative HPLC wurden 8.6 mg (48% d. Th. über 2 Stufen) der Titelverbindung erhalten.
LC-MS (Methode 1): Rₜ = 0.88 min; MS (ESIpos): m/z = 1203 (M+H)⁺.

### Intermediat F168

### N-[6-(2,5-Dioxo-2,5-dihydro-1H-pyrrol-1-yl)hexanoyl]-L-valyl-L-alanyl-N⁶-{[(1R,2S)-2-({(2S)-2-amino-4-[{(1R)-1-[1-benzyl-4-(2,5-difluorphenyl)-1H-pyrrol-2-yl]-2,2-dimethylpropyl} (glycoloyl)amino]butanoyl}amino)cyclopentyl]carbonyl}-L-lysin-trifluoressigsäure (1:1)

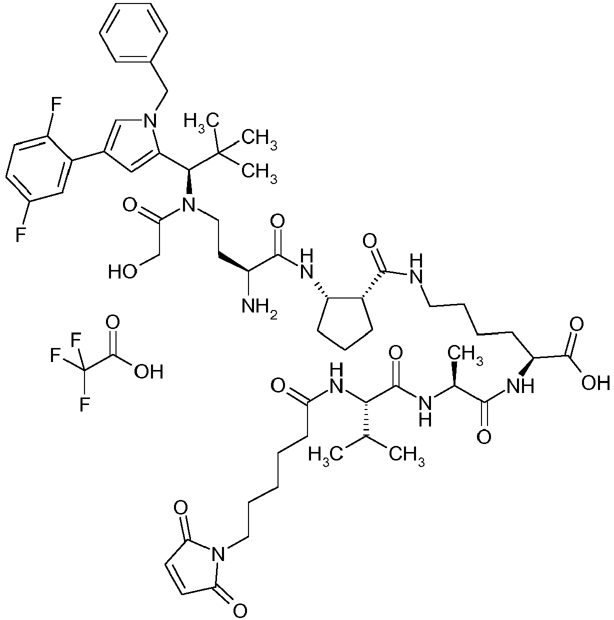

Zunächst wurde ausgehend von kommerziell erhältlicher (1R,2S)-2-[(tert-Butoxycarbonyl) amino]cyclopentancarbonsäure nach klassischen Methoden der Peptidchemie durch Veresterung mit Benzylalkohol mittels EDCI/DMAP und anschließender Abspaltung der tert Butoxycarbonylschutzgruppe mit TFA in DCM Trifluoressigsäure-benzyl-(1R,2S)-2-aminocyclopentancarboxylat (1:1) hergestellt.

102mg (0.305 mmol) von diesem Intermediat wurden in 12 ml DMF aufgenommen und mit 100 mg (0.152 mmol) von Intermediat C58 in Gegenwart von HATU und *N,N*-Diisopropylethylamin gekuppelt. Nach Reinigung durch präparative HPLC wurde das Intermediat in Methanol aufgenommen und über 10%-igem Palladium auf Aktivkohle 2 h lang bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser 1:1 wurden 70 mg (59% d.Th. über 2 Stufen) von (1R,2S)-2-{[(2S)-4-[{(1R)-1-[1-Benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butanoyl]amino}cyclopentancarbonsäure erhalten.

Die Titelverbindung wurde dann durch Kupplung von 20 mg (0.013 mmol) dieses Intermediats mit 16.6 mg (0.023 mmol) von Intermediat L61 in Gegenwart von 9.5 mg (0.025 mmol) HATU sowie 18 µl N,N-Diisopropylethylamin und anschließender Entschützung mit Zinkchlorid in Trifluorethanol wie in Intermediat F119 beschrieben hergestellt. Nach Reinigung durch präparative HPLC wurden 9.3 mg (30% d. Th. über 2 Stufen) der Titelverbindung erhalten.
LC-MS (Methode 1): Rₜ = 0.98 min; MS (ESIpos): m/z = 1116 (M+H)⁺.

### Intermediat F169

### N-[6-(2,5-Dioxo-2,5-dihydro-1H-pyrrol-1-yl)hexanoyl]-L-valyl-N⁵-carbamoyl-L-ornithyl-N⁶-{[(1R,2S)-2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)cyclopentyl]carbonyl}-L-lysin-trifluoressigsäure (1:1)

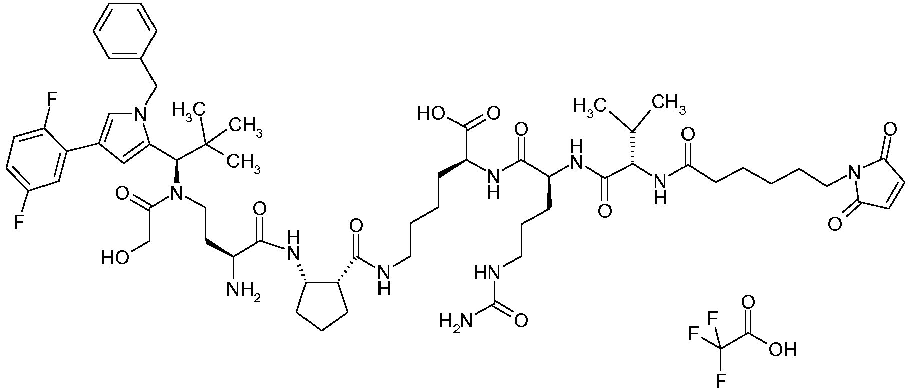

Die Synthese der Titelverbindung erfolgte in Analogie zu Intermediat F168 ausgehend von den Intermediaten C58 und L62.
LC-MS (Methode 1): Rₜ = 0.91 min; MS (ESIpos): m/z = 1202 (M+H)⁺.

### Intermediat F171

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-D-alanyl)-N²-{N-[6-(2,5-dioxo-2,5-dihydro-1H-pyrrol-1-yl)hexanoyl]-L-valyl-L-alanyl}-L-lysin-trifluoressigsäure (1:1)

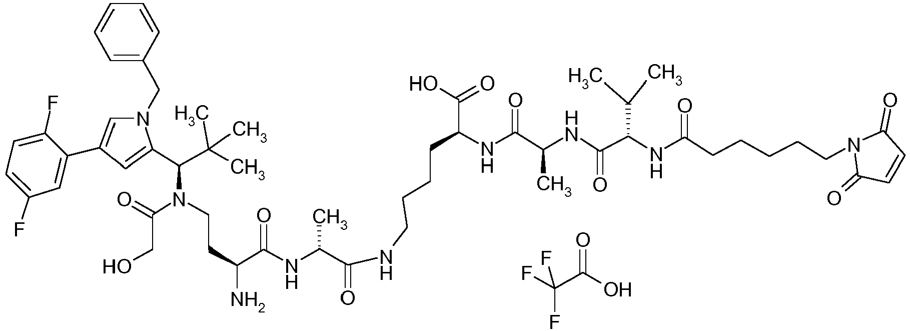

Die Synthese der Titelverbindung erfolgte in Analogie zu Intermediat F155 ausgehend von den Intermediaten C62 und L61.
LC-MS (Methode 1): Rₜ = 0.93 min; MS (ESIpos): m/z = 1076 (M+H)⁺.

### Intermediat F173

### N-[6-(2,5-Dioxo-2,5-dihydro-1H-pyrrol-1-yl)hexanoyl]-L-valyl-L-alanyl-N-[2-({(2S)-2-amino-4-[{(1R)-1-[1-benzyl-4-(2,5-difluorphenyl)-1H-pyrrol-2-yl]-2,2-dimethylpropyl}(glycoloyl)amino] butanoyl}amino)ethyl]-L-glutamin-trifluoressigsäure (1:1)

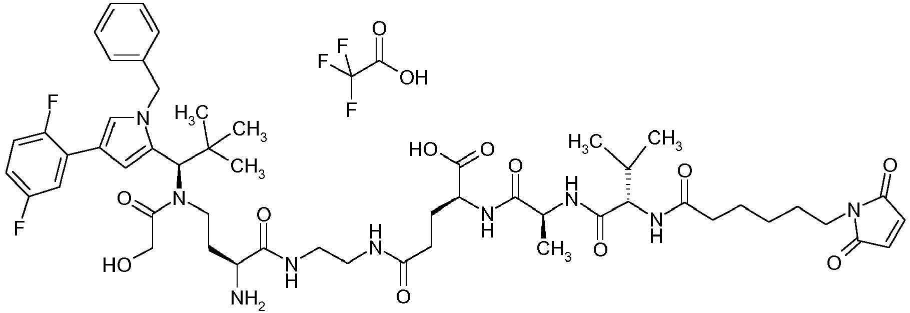

Die Titelverbindung wurde ausgehend von 15 mg (0.018 mmol) Intermediat C64 durch Kupplung mit 12 mg (0.02 mmol) von Intermediat L63 in Gegenwart von 7.7 mg (0.02 mmol) HATU sowie 16 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 12 mg (58% d. Th. über 2 Stufen) der Titelverbindung erhalten.
LC-MS (Methode 1): Rₜ = 0.91 min; MS (EIpos): m/z = 1048 [M+H]⁺.

### Intermediat F174

### N-[6-(2,5-Dioxo-2,5-dihydro-1H-pyrrol-1-yl)hexanoyl]-L-valyl-L-alanyl-N-[2-({(2S)-2-amino-4-[{(1R)-1-[4-benzyl-1-(2,5-difluorphenyl)-1H-pyrazol-3-yl]-2,2-dimethylpropyl}(glycoloyl)amino] butanoyl}amino)ethyl]-L-glutamin-trifluoressigsäure (1:1)

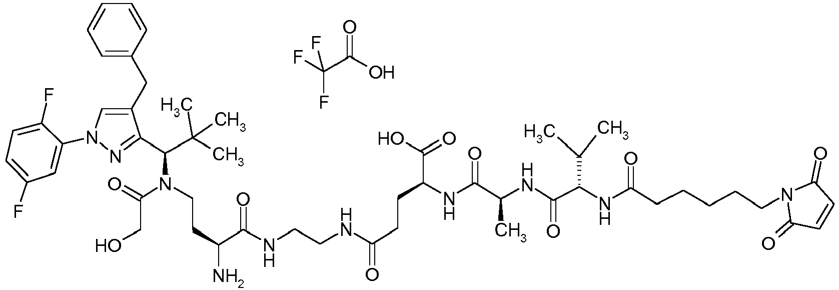

Die Titelverbindung wurde analog zu Intermediat F172 ausgehend Intermediat C57 und L63 hergestellt.
LC-MS (Methode 1): Rₜ = 0.9 min; MS (EIpos): m/z = 1049 [M+H]⁺.

### Intermediat F175

### Trifluoressigsäure --N-[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-6-(2,5-dioxo-2,5-dihydro-1H-pyrrol-1-yl)hexanamid (1:1)

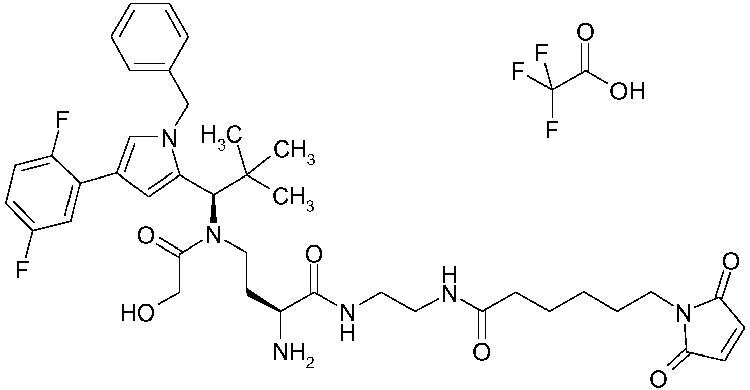

Die Titelverbindung wurde durch Kupplung von 11 mg (0.013 mmol) Intermediat C64 mit 3.4 mg (0.016 mmol) von 6-(2,5-Dioxo-2,5-dihydro-1H-pyrrol-1-yl)hexansäure in Gegenwart von 6.7 mg ( 0.018 mmol) HATU sowie 9 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 8 mg (69% d. Th. über 2 Stufen) der Titelverbindung erhalten.
LC-MS (Methode 1): Rₜ = 1.35 min; MS (EIpos): m/z = 893 [M+H]⁺.

### Intermediat F176

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-[1-(2,5-dioxo-2,5-dihydro-1H-pyrrol-1-yl)-12-oxo-3,6,9-trioxa-13-azapentadecan-15-yl]butanamid (1:1)

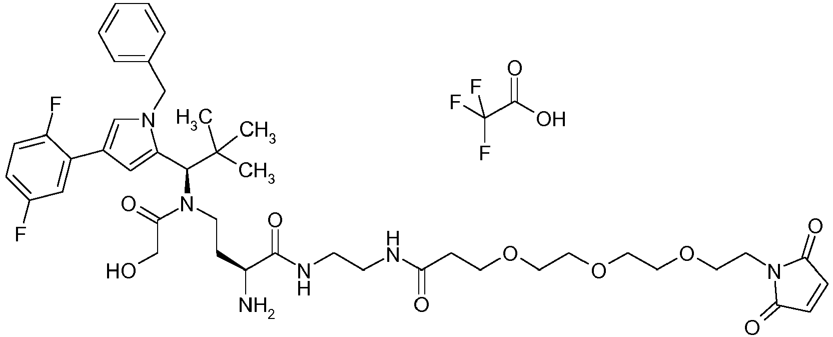

Die Titelverbindung wurde durch Kupplung von 5 mg (0.006 mmol) Intermediat C64 mit 2 mg (0.007 mmol) von 3-(2-{2-[2-(2,5-Dioxo-2,5-dihydro-1H-pyrrol-1-yl)ethoxy]ethoxy}ethoxy)propansäure, deren Herstellung unter Intermediat L15 beschrieben ist, in Gegenwart von 3.5 mg (0.009 mmol) HATU sowie 4 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 2 mg (35% d. Th. über 2 Stufen) der Titelverbindung erhalten.
LC-MS (Methode 1): Rₜ = 0.86 min; MS (EIpos): m/z = 839 [M+H]⁺.

### Intermediat F177

### Trifluoressigsäure--(1R,2S)-2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-(2-{[(2,5-dioxo-2,5-dihydro-1H-pyrrol-1-yl)acetyl]amino}ethyl)cyclopentancarboxamid (1:1)

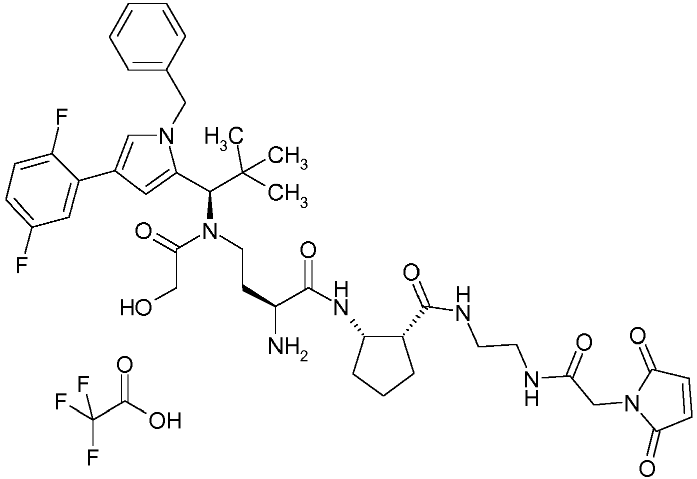

Die Titelverbindung wurde in Anlogie zu Intermediat F168 hergestellt, wobei an Stelle von Intermediat L61 das Intermediat L1 eingesetzt wurde.
LC-MS (Methode 1): Rₜ = 0.86 min; MS (EIpos): m/z = 804 [M+H]⁺.

### Intermediat F178

### Trifluoressigsäure--(1R,2S)-2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)-N-{2-[(bromacetyl)amino] ethyl}cyclopentancarboxamid (1:1)

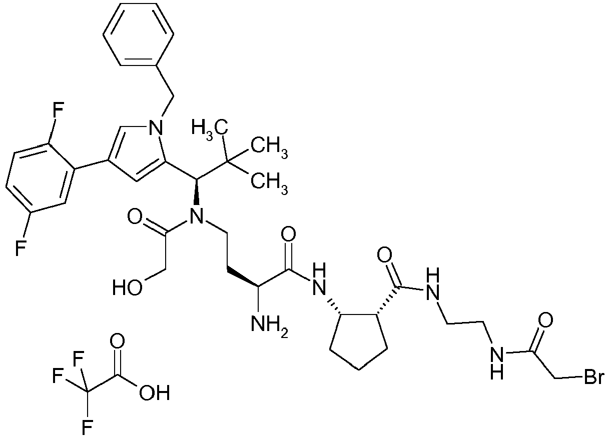

Die Titelverbindung wurde in Anlogie zu Intermediat F177 hergestellt, wobei an Stelle von Intermediat L1 das Intermediat L52 eingesetzt wurde.
LC-MS (Methode 1): Rₜ = 0.89 min; MS (EIpos): m/z = 787 und 789 [M+H]⁺.

### Intermediat F179

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-[6-(2,5-dioxo-2,5-dihydro-1H-pyrrol-1-yl)hexyl]butanamid (1:1)

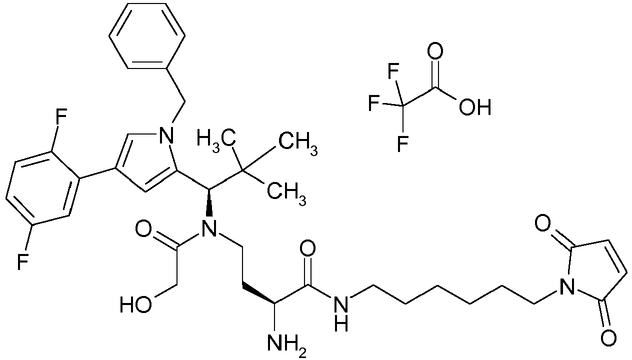

Die Titelverbindung wurde durch Kupplung von 15 mg (0.023 mmol) Intermediat C58 mit 6 mg (0.025 mmol) 1-(6-Aminohexyl)-1H-pyrrol-2,5-dion in Gegenwart von 13 mg (0.034 mmol) HATU sowie 16 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 8.5 mg (46% d. Th. über 2 Stufen) der Titelverbindung erhalten.
LC-MS (Methode 6): Rₜ = 2.22 min; MS (EIpos): m/z = 692 [M+H]⁺.

### Intermediat F180

### N-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-N²-[(2,5-dioxo-2,5-dihydro-1H-pyrrol-1-yl)acetyl]-L-glutamin-trifluoressigsäure (1:1)

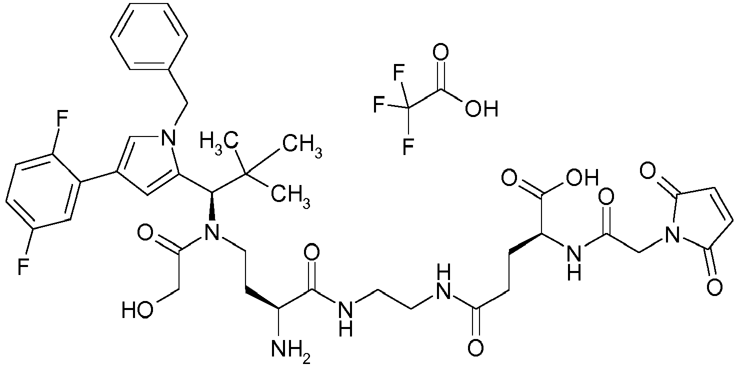

Die Titelverbindung wurde durch Kupplung von 9.6 mg (0.012 mmol) Intermediat C64 mit 5 mg (0.013 mmol) von Intermediat L64 in Gegenwart von 7 mg (0.018 mmol) HATU sowie 6 µl N,N-Diisopropylethylamin und anschließender Entschützung mittels Zinkchlorid in Trifluorethanol wie in Intermediat F119 beschrieben hergestellt. Nach Reinigung durch präparative HPLC wurden 3.1 mg (28% d. Th. über 2 Stufen) der Titelverbindung erhalten.
LC-MS (Methode 1): Rₜ = 0.85 min; MS (EIpos): m/z = 822 [M+H]⁺.

### Intermediat F192

### N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[(2,5-dioxo-2,5-dihydro-1H-pyrrol-1-yl)acetyl]amino}-L-alanin-trifluoressigsäure (1:1)

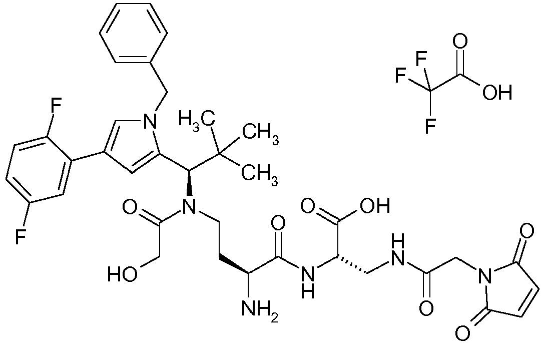

60mg (0.091 mmol) von Intermediat C58 wurden in 8 ml DMF aufgenommen und mit 45 mg (0.100 mmol) von Intermediat L65 in Gegenwart von 42 mg (0.11 mmol) HATU und 64 µL *N,N*-Diisopropyl-ethylamin gekuppelt. Nach Reinigung durch präparative HPLC wurde das Intermediat in 10 ml Ethanol aufgenommen und über 10%-igem Palladium auf Aktivkohle 45 min lang bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser 1:1 wurden 24.5 mg (31% d.Th. über 2 Stufen) von 2-(Trimethylsilyl)ethyl-3-amino-N-[(2S)-4-[{(1R)-1-[1-benzyl-4-(2,5-difluorphenyl)-1H-pyrrol-2-yl]-2,2-dimethylpropyl}(glycoloyl)amino]-2-({[2-(trimethylsilyl)ethoxy]carbonyl}amino)butanoyl]-L-alaninat erhalten.
LC-MS (Methode 1): Rₜ = 1.17 min; MS (EIpos): m/z = 844 [M+H]⁺.

Die Titelverbindung wurde dann durch Kupplung von 10 mg (0.012 mmol) dieses Intermediats mit 2 mg (0.013 mmol) von kommerziell erhältlicher (2,5-Dioxo-2,5-dihydro-1H-pyrrol-1-yl)essigsäure Intermediat in Gegenwart von 5.4 mg (0.014 mmol) HATU sowie 8 µl N,N-Diisopropylethylamin und anschließender Entschützung mit Zinkchlorid in Trifluorethanol wie in Intermediat F119 beschrieben hergestellt. Nach Reinigung durch präparative HPLC wurden 3.5 mg (33% d. Th. über 2 Stufen) der Titelverbindung erhalten.
LC-MS (Methode 1): Rₜ = 0.81 min; MS (ESIpos): m/z = 737 (M+H)⁺.

### Intermediat F193

### N- {(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-3-{[(2,5-dioxo-2,5-dihydro-1H-pyrrol-1-yl)acetyl]amino}-D-alanin-trifluoressigsäure (1:1)

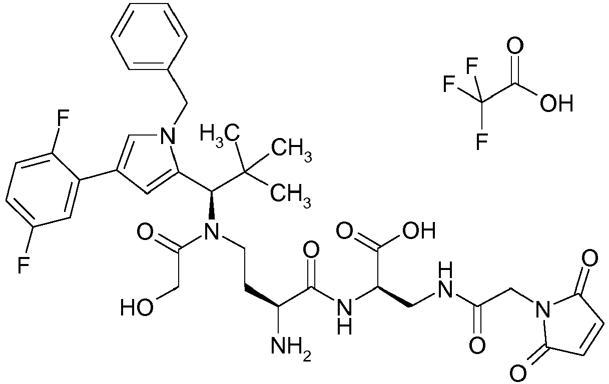

Die Synthese der Titelverbindung erfolgte in Analogie zu Intermediat F192 ausgehend von 3-{[(Benzyloxy)carbonyl]amino}-N-(tert-butoxycarbonyl)-D-alanin-N-cyclohexylcyclohexanamine (1:1).
LC-MS (Methode 1): Rₜ = 0.87 min; MS (ESIpos): m/z = 737 (M+H)⁺.

### Intermediat F194

### N-{5-[(2,5-Dioxopyrrolidin-1-yl)oxy]-5-oxopentanoyl}-L-valyl-N-{3-[{(1R)-1-[1-benzyl-4-(2,5-difluorphenyl)-1H-pyrrol-2-yl]-2,2-dimethylpropyl}(glycoloyl)amino]propyl}-L-alaninamid

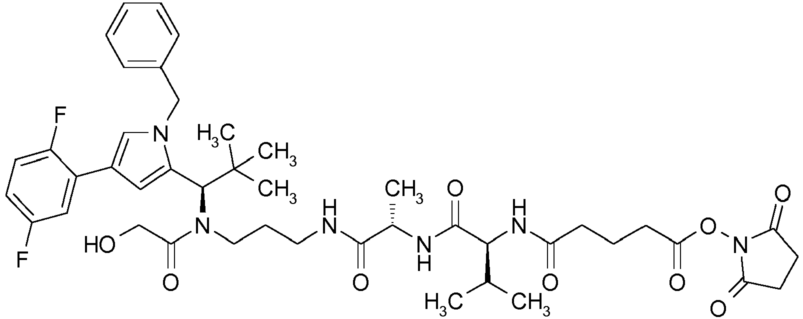

Die Titelverbindung wurde ausgehend von Beispiel 98 zunächst durch Kupplung mit N-[(Benzyloxy)carbonyl]-L-valyl-L-alanin in Gegenwart von HATU und N,N-Diisopropylethylamin hergestellt. Im nächsten Schritt wurde die Z-Schutzgruppe durch 1-stündige Hydrierung über 10%-igem Palladium auf Aktivkohle bei RT unter Wasserstoff-Normaldruck entfernt und das entschützte Intermediat anschließend wie für Intermediat F58 beschrieben durch Umsetzung mit 1,1'-[(1,5-Dioxopentan-1,5-diyl)bis(oxy)]dipyrrolidin-2,5-dion in die Titelverbindung überführt.
LC-MS (Methode 1): Rₜ = 1.19 min; MS (ESIpos): m/z = 851 [M+H]⁺.

### Intermediat F195

### Trifluoressigsäure--N-{(2S)-2-amino-4-[{(1R)-1-[1-benzyl-4-(2,5-difluorphenyl)-1H-pyrrol-2-yl]-2,2-dimethylpropyl}(glycoloyl)amino]butyl}-N'-[2-(2,5-dioxo-2,5-dihydro-1H-pyrrol-1-yl)ethyl]succinamid (1:1)

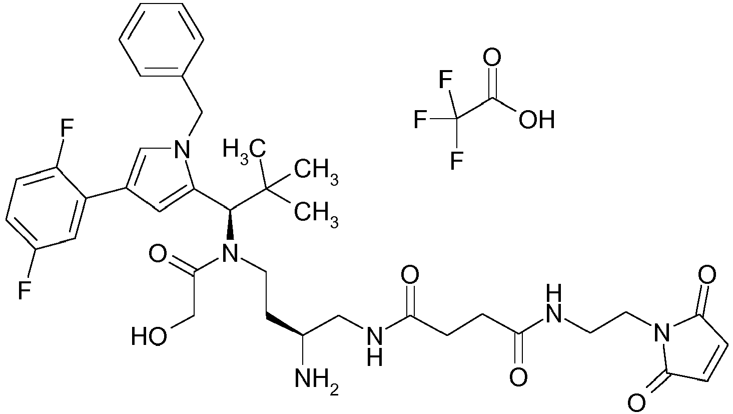

Die Titelverbindung wurde durch Kupplung von 26 mg (0.035 mmol) von Intermediat C65 mit 18 mg (0.07 mmol) von kommerziell erhältlichem Trifluoressigsäure--1-(2-aminoethyl)-1H-pyrrol-2,5-dion (1:1) in 8 ml DMF in Gegenwart von 40 mg (0.1054 mmol) HATU sowie 61 µl N,N-Diisopropylethylamin und anschließender Entschützung mit Zinkchlorid in Trifluorethanol wie in Intermediat F119 beschrieben hergestellt. Nach Reinigung durch präparative HPLC wurden 16 mg (43% d. Th. über 2 Stufen) der Titelverbindung erhalten.
LC-MS (Methode 1): Rₜ = 0.85 min; MS (ESIpos): m/z = 721 (M+H)⁺.
¹H-NMR (500 MHz, DMSO-d₆): δ = 7.99 (t, 1H), 7.95 (t, 1H), 7.6-7.75 (m, 4H), 7.5 (s, 1H) 7.2-7.4 (m, 6H), 6.8-7.0 (m, 4H), 5.63 (s, 1H), 4.9 und 5.2 (2d, 2H), 4.26 und 4.0 (2d, 2H), 3.3-3.6 (m, 4H), 3.15-3.25 (m, 3H), 2.85-3.0 (m, 2H), 2.2-2.3 (m, 4H), 0.64 und 1,49 (2m, 2H), 0.81 (s, 9H).

### Intermediat F196

### Trifluoressigsäure --2-(2,5-dioxo-2,5-dihydro-1H-pyrrol-1-yl)ethyl-N-{(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}-beta-alaninat (1:1)

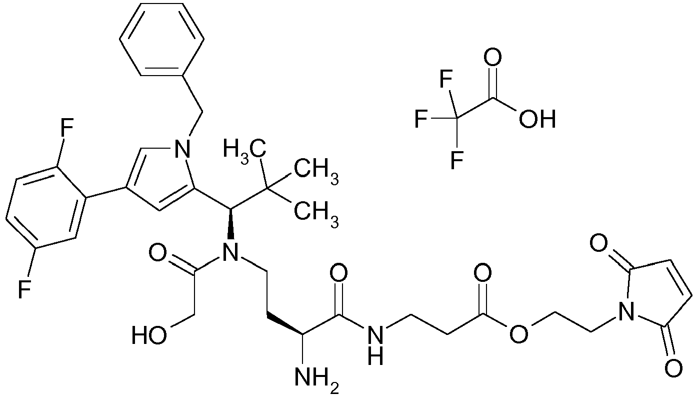

Zunächst wurden 15 mg (0.023 mmol) Intermediat C58 in 4 ml DMF aufgenommen und mit 8.2 mg (0.025 mmol) Intermediat L67 in Gegenwart von 13.0 mg (0.034 mmol) HATU sowie 16 µl N,N-Diisopropylethylamin umgesetzt. Nach 30 min Rühren bei RT wurde eingeengt und der Rückstand durch präparative HPLC gereinigt. Nach Vereinigung der entsprechenden Fraktionen und Einengen des Lösungsmittels wurde der Rückstand aus Acetonitril/Wasser 1:1 lyophilisiert. Es wurden 4.3 mg (20% d. Th.) des geschützten Intermediats erhalten.
LC-MS (Methode 1): Rₜ = 1.35 min; MS (EIpos): m/z = 852 [M+H]⁺.

4.3 mg (4.5 µmol) der Zwischenstufe wurden in 1 ml Trifluorethanol gelöst und wie für Intermediat F119 beschrieben mit 3.65 mg (27 µmol) Zinkchlorid entschützt. Nach Reinigung durch präparative HPLC wurde 1 mg (25% d. Th. über 2 Stufen) der Titelverbindung erhalten.
LC-MS (Methode 1): Rₜ = 0.88 min; MS (ESIpos): m/z = 708 (M+H)⁺

### Intermediat F204

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-(2-{[3-(2,5-dioxo-2,5-dihydro-1H-pyrrol-1-yl)propanoyl]amino}ethyl)butanamid (1:1)

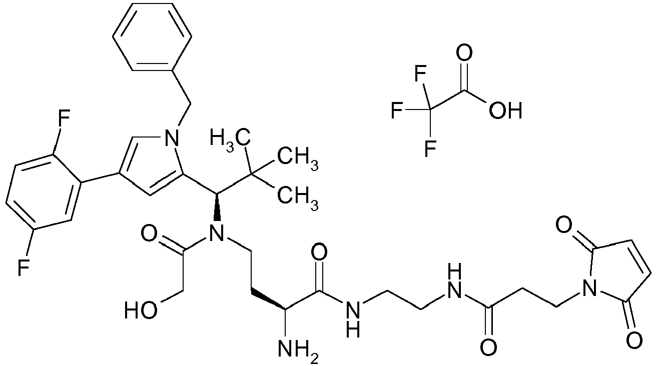

25 mg (0.038 mmol) von Intermediat C58 wurden zunächst mit 16.5 mg (75%-ig) (0.038 mmol) Intermediat L68 in Gegenwart von 17 mg (0.046 mmol) HATU sowie 20 µl N,N-Diisopropylethylamin umgesetzt. Nach 60 min Rühren bei RT wurde eingeengt und der Rückstand durch präparative HPLC gereinigt. Es wurden 18.3 mg (56% d. Th.) des geschützten Intermediats erhalten.
LC-MS (Methode 1): Rₜ = 1.32 min; MS (EIpos): m/z = 851 [M+H]⁺.

Im zweiten Schritt wurde dieses Intermediat in 3 mL 2,2,2-Trifluorethanol gelöst. Es wurden 12 mg (0.086 mmol) Zinkchlorid zugegeben und der Ansatz 2 h bei 50°C gerührt. Anschließend wurden 25 mg (0.086 mmol) Ethylendiamin-N,N,N',N'-tetraessigsäure sowie 2 mL einer 0.1%-igen wässrigen Trifluoressigsäurelösung zugesetzt. Der Ansatz wurde mittels präparativer HPLC gereinigt. Nach Einengen der entsprechenden Fraktionen und Lyophilisation des Rückstands aus Acetonitril/Wasser wurden 11 mg (62% d.Th.) der Titelverbindung erhalten.
LC-MS (Methode 1): Rₜ = 0.85 min; MS (ESIpos): m/z = 707 (M+H)⁺.

### Intermediat F205

### Trifluoressigsäure--1-[(2,5-dioxo-2,5-dihydro-1H-pyrrol-1-yl)acetyl]piperidin-4-yl-N-{(2S)-2-amino-4-[{(1R)-1-[1-benzyl-4-(2,5-difluorphenyl)-1H-pyrrol-2-yl]-2,2-dimethylpropyl} (glycoloyl)amino]butanoyl}-beta-alanyl-L-valyl-N⁵-carbamoyl-L-ornithinat (1:1)

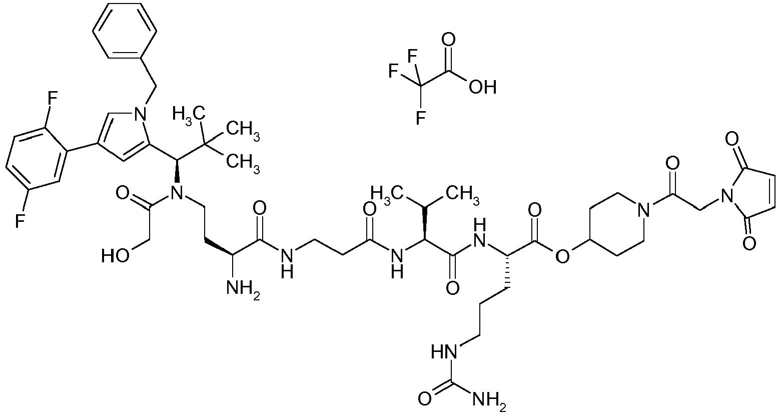

Die Synthese erfolgte durch Kupplung von 25 mg (0.034 mmol) Intermediat C61 und 29 mg (0.041 mmol) Intermediat L69 in Gegenwart von HATU und *N,N*-Diisopropylethylamin, anschließende Hydrierung mit Palladium auf Aktivkohle (10%ig) unter Normaldruck, dann folgende Kupplung mit (2,5-Dioxo-2,5-dihydro-1H-pyrrol-1-yl)essigsäure in Gegenwart von HATU und *N,N-*Diisopropylethylamin und abschließende Abspaltung der 2-(Trimethylsilyl)ethoxycarbonyl-Schutzgruppe mit Zinkchlorid. Nach HPLC Reinigung wurden 11mg (26 % d. Th. über 4 Stufen) erhalten.
LC-MS (Methode 1): Rₜ = 0.86 min; MS (ESIpos): m/z = 1061 (M+H)⁺.

### Intermediat F206

### Trifluoressigsäure--1-[(2,5-dioxo-2,5-dihydro-1H-pyrrol-1-yl)acetyl]piperidin-4-yl-N-{(2S)-2-amino-4-[{(1R)-1-[1-benzyl-4-(2,5-difluorphenyl)-1H-pyrrol-2-yl]-2,2-dimethylpropyl} (glycoloyl)amino]butanoyl}-beta-alanyl-L-valyl-L-alaninat (1:1)

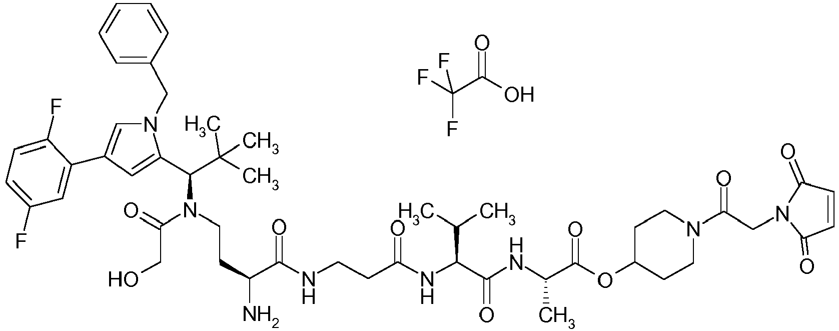

Die Synthese erfolgte in Analogie zu Intermediat F205.
LC-MS (Methode 1): Rₜ = 0.86 min; MS (ESIpos): m/z = 975 (M+H)⁺.

### Intermediat F207

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-N²-{N-[(2,5-dioxo-2,5-dihydro-1H-pyrrol-1 - yl)acetyl]-L-valyl-L-alanyl}-L-lysin -trifluoressigsäure (1:1)

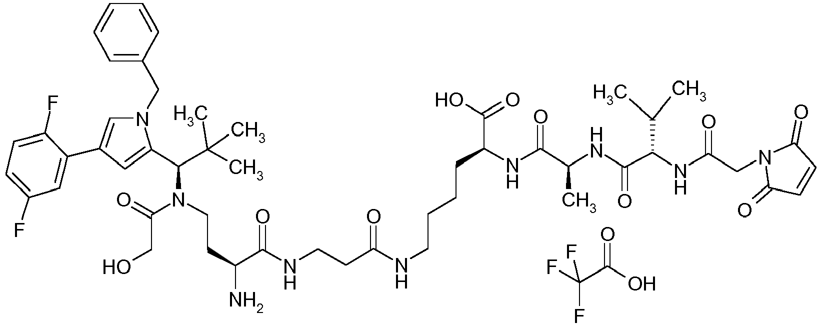

Die Titelverbindung wurde in Analogie zu Intermediat F155 hergestellt.
LC-MS (Methode 1): Rₜ = 0.81 min; MS (ESIpos): m/z = 1020 (M+H)⁺.

### Intermediat F209

### R-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-L-cystein-trifluoressigsäure (1:1)

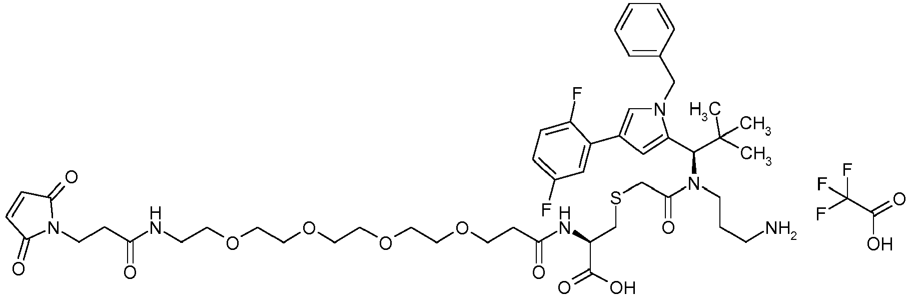

93.9 mg (0.78 mmol) L-Cystein wurden in einer Lösung aus 93.0 mg (1.11 mmol) Natriumhydrogencarbonat und 0.9 mL Wasser suspendiert. Es wurden 70.0 mg (0.11 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) gelöst in 6.0 mL *iso*-Propanol und 202.3 mg (1.33 mmol) 1,8-Diazabicyclo(5.4.0)undec-7-en zugegeben. Die Reaktionsmischung wurde 90 min bei 50 °C gerührt. Der Ansatz wurde mit Wasser (0.1% TFA) versetzt und mittles präp. RP-HPLC gereinigt (Säule: Reprosil 125x30; 10µ, Fluss: 50 mL/min, MeCN/Wasser; 0.1% TFA). Die Lösemittel wurden im Vakuum verdampft und der Rückstand im Hochvakuum getrocknet. Man erhielt 53.9 mg (59 % d. Th.) der Verbindung R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1).
LC-MS (Methode 1): Rₜ = 1.24 min; MS (ESIpos): m/z = 717 (M+H)⁺.

86.0 mg (0.1 mmol) R-(11-{(1R)-1-[1-Benzyl-4-(2,5-difluorphenyl)-1H-pyrrol-2-yl]-2,2 dimethylpropyl}-2,2-dimethyl-6,12-dioxo-5-oxa-7,11-diaza-2-silatridecan-13-yl)-L-cystein-trifluoressigsäure (1:1) und 58.5 mg (0.11 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid wurden in 4.0 mL DMF gelöst und mit 20.9 mg (0.21 mmol) 4-Methylmorpholin versetzt. Die Reaktionsmischung wurde über Nacht bei RT gerührt. Es wurden 15.5 mg (0.26 mmol) HOAc zugegeben und die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Man erhielt 68.6 mg (59 % d. Th.) der Verbindung R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-L-cystein.
LC-MS (Methode 6): Rₜ = 2.88 min; MS (ESIpos): m/z = 1115 (M+H)⁺.

46.4 mg (0.04 mmol) R-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-L-cystein wurden in 2.0 mL Trifluorethanol gelöst und mit 17.0 mg (0.13 mmol) Zinkdichlorid versetzt. Die Reaktionsmischung rührte über Nacht bei 50 °C. Es wurden nochmals 8.5 mg (0.07 mmol) Zinkdichlorid zugegeben und über Nacht bei 50 °C gerührt. Die Reaktionsmischung wurde mit 36.5 mg (0.13 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 19.4 mg (43 % d. Th.) der Titelverbindung.
LC-MS (Methode 1): Rₜ = 0.94 min; MS (ESIpos): m/z = 971 (M+H)⁺.

### Intermediat F210

### S-{2-[(3-Aminopropyl)-{(1R)-1-[1-benzyl-4-(2,5-difluorphenyl)-1H-pyrrol-2-yl]-2,2 dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-D-Cystein-trifluoressigsäure (1:1)

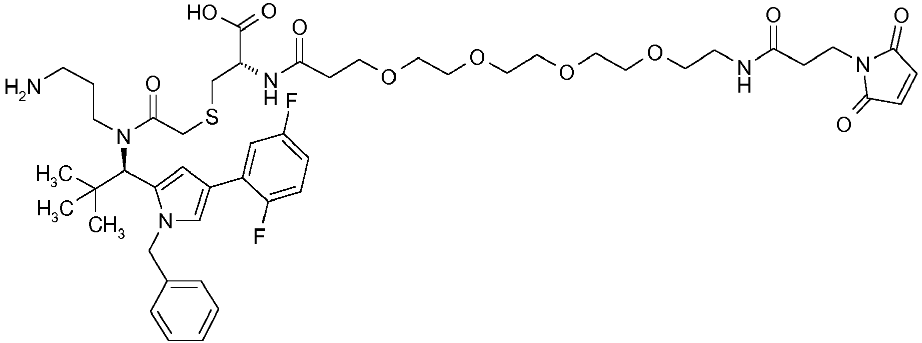

Die Titelverbindung wurde in Analogie zur Synthese des Intermediats F209 unter Verwendung von D-Cystein hergestellt.
LC-MS (Methode 1): Rₜ = 0.91 min; MS (ESIpos): m/z = 971 (M+H)⁺.

### Intermediat F211

### Trifluoressigsäure--3-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)-N-[2-(2,5-dioxo-2,5-dihydro-1H-pyrrol-1-yl)ethyl]propanamid (1:1)

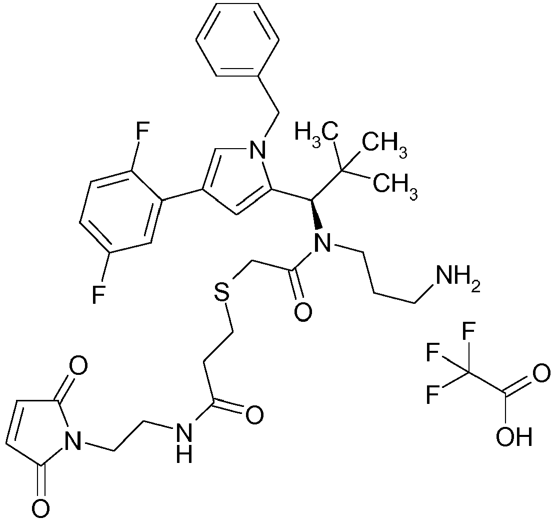

30.0 mg (0.05 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) wurden zusammen mit 5.5 mg (0.05 mmol) 3-Sulfanylpropansäure in 0.5 mL Methanol mit einem Tropfen Wasser vorgelegt. Dann wurden 23.0 mg (0.17 mmol) Kaliumcarbonat zugegeben und die Reaktionsmischung wurde 4 h bei 50 °C gerührt. Es wurde Ethylacetat zugesetzt und die org. Phase einmal mit Wasser und einmal mit ges. NaCl-Lösung gewaschen. Die org. Phase wurde über Magnesiumsulfat getrocknet und das Lösemittel im Vakuum verdampft. Der Rückstand wurde ohne weitere Aufreinigung in der nächsten Synthesestufe eingesetzt. Man erhielt 30.3 mg (86 % d. Th.) der Verbindung 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure
LC-MS (Methode 1): Rₜ = 1.39 min; MS (ESIpos): m/z = 702 (M+H)⁺.

30.0 mg (0.04 mol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure und 9.8 mg (0.06 mmol) 1-(2-Aminoethyl)-1H-pyrrol-2,5-dionhydrochlorid (1:1) wurden in 2.0 mL Acetonitril vorgelegt und mit 44.2 mg (0.34 mmol) N,N-Diisopropylethylamin versetzt. Es wurden
35.4 mg (0.06 mmol) T3P (50 % in Ethylacetat) zugegeben und die Reaktionsmischung wurde über Nacht bei RT gerührt. Es wurde Wasser zugegeben und die Reinigung erfolgte direkt mittles präp. RP-HPLC (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 22.0 mg (63 % d. Th.) der Verbindung 2-(Trimethylsilyl)ethyl-[3-({(1R)-1-[1-benzyl-4-(2,5-difluorphenyl)-1H-pyrrol-2-yl]-2,2-dimethylpropyl} {[(3-{[2-(2,5-dioxo-2,5-dihydro-1H-pyrrol-1-yl)ethyl]amino}-3-oxopropyl)sulfanyl]acetyl}amino)propyl]carbamat.
LC-MS (Methode 1): Rₜ = 1.41 min; MS (ESIpos): m/z = 824 (M+H)⁺.

22.0 mg (0.03 mol) 2-(Trimethylsilyl)ethyl-[3-({(1R)-1-[1-benzyl-4-(2,5-difluorphenyl)-1H-pyrrol-2-yl]-2,2-dimethylpropyl}{[(3-{[2-(2,5-dioxo-2,5-dihydro-1H-pyrrol-1-yl)ethyl]amino}-3-oxopropyl)sulfanyl]acetyl}amino)propyl]carbamat wurden in 1.0 mL Trifluorethanol gelöst und mit 9.1 mg (0.07 mmol) Zinkdichlorid versetzt. Die Reaktionsmischung rührte 5 h bei 50 °C. Die Reaktionsmischung wurde mit 19.5 mg (0.07 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 15.0 mg (71 % d. Th.) der Titelverbindung.
LC-MS (Methode 1): Rₜ = 0.88 min; MS (ESIpos): m/z = 680 (M+H)⁺.

### Intermediat F212

### Trifluoressigsäure--N-(3-aminopropyl)-N-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-10-oxo-3,6-dioxa-13-thia-9-azapentadecan-15-amid (1:1)

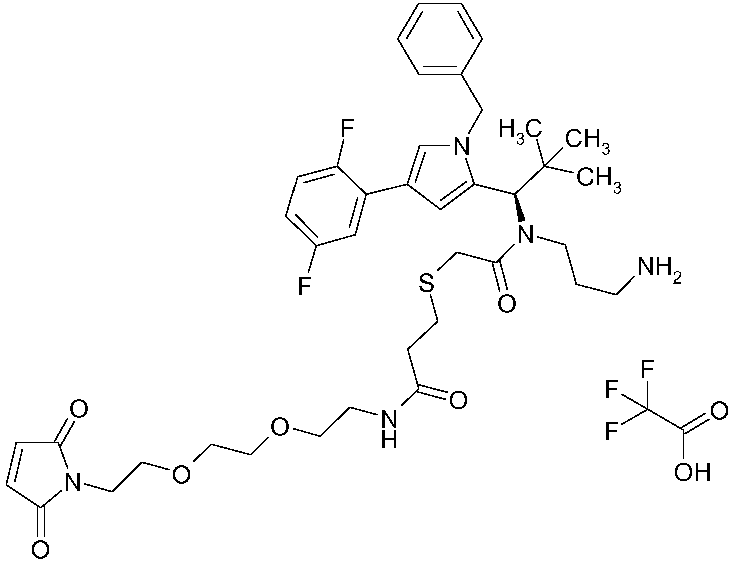

28.8 mg (0.04 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden zusammen mit 18.3 mg (0.05 mmol) Trifluoressigsäure--1-{2-[2-(2-aminoethoxy)ethoxy]ethyl}-1H-pyrrol-2,5-dion (1:1) (Intermediat L59) in 1.9 mL Acetonitril vorgelegt. Dann wurden 42.4 mg (0.33 mmol) N,N-Diisopropylethylamin zugegeben und 33.9 mg (0.05 mmol) T3P (50 % in Ethylacetat) zugetropft. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser) gereinigt. Die Lösemittel wurden im Vakuum verdampft und der Rückstand im Hochvakuum getrocknet. Man erhielt 10.7 mg (26 % d. Th.) der Verbindung 2-(Trimethylsilyl)ethyl-[16-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-10,15-dioxo-3,6-dioxa-13-thia-9,16-diazanonadecan-19-yl]carbamat.
LC-MS (Methode 1): Rₜ = 1.44 min; MS (ESIpos): m/z = 812 (M+H)⁺.

10.7 mg (0.01 mol) 2-(Trimethylsilyl)ethyl-[16-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-10,15-dioxo-3,6-dioxa-13-thia-9,16-diazanonadecan-19-yl]carbamat wurden in 0.8 mL Trifluorethanol gelöst und mit 8.0 mg (0.06 mmol) Zinkdichlorid versetzt. Die Reaktionsmischung rührte 5 h bei 50 °C. Die Reaktionsmischung wurde mit 17.1 mg (0.06 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet.
LC-MS (Methode 1): Rₜ = 1.03 min; MS (ESIpos): m/z = 768 (M+H)⁺.

### Intermediat F213

### Trifluoressigsäure--3-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)-N-(2-{[(2,5-dioxo-2,5-dihydro-1H-pyrrol-1 - yl)acetyl]amino}ethyl)propanamid (1:1)

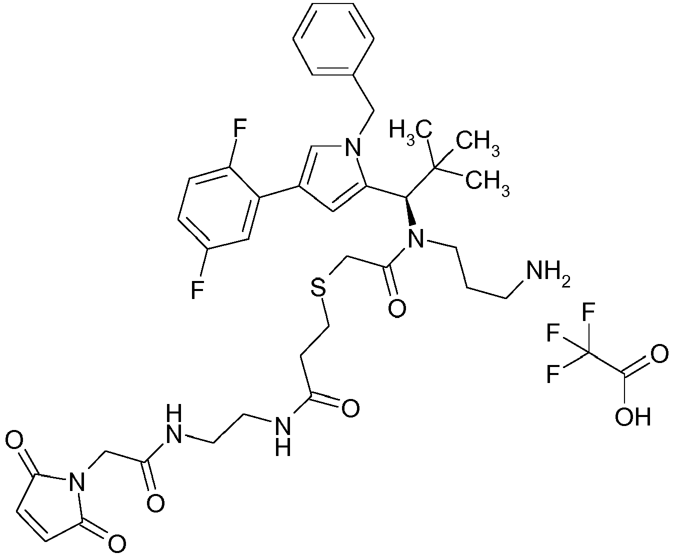

27.5 mg (0.04 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden zusammen mit 15.9 mg (0.05 mmol) Trifluoressigsäure --N-(2-aminoethyl)-2-(2,5-dioxo-2,5-dihydro-1H-pyrrol-1-yl)acetamid (1:1) (Intermediat L1) in 1.8 mL Acetonitril vorgelegt. Dann wurden 32.4 mg (0.31 mmol) N,N-Diisopropylethylamin zugegeben und 32.4 mg (0.05 mmol) T3P (50 % in Ethylacetat) zugetropft. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 11.9 mg (35 % d. Th.) der Verbindung 2-(Trimethylsilyl)ethyl-[13-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,7,12-trioxo-10-thia-3,6,13-triazahexadecan-16-yl]carbamat.
LC-MS (Methode 1): Rₜ = 1.39 min; MS (ESIpos): m/z = 881 (M+H)⁺.

11.9 mg (0.01 mol) 2-(Trimethylsilyl)ethyl-[13-{(1R)-1-[1-benzyl-4-(2,5-difluorphenyl)-1H-pyrrol-2-yl]-2,2-dimethylpropyl}-1-(2,5-dioxo-2,5-dihydro-1H-pyrrol-1-yl)-2,7,12-trioxo-10-thia-3,6,13-triazahexadecan-16-yl]carbamat wurden in 1.0 mL Trifluorethanol gelöst und mit 5.5 mg (0.04 mmol) Zinkdichlorid versetzt. Die Reaktionsmischung rührte über Nacht bei 50 °C. Die Reaktionsmischung wurde mit 11.8 mg (0.04 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 7.4 mg (60 % d. Th.) der Titelverbindung.
LC-MS (Methode 5): Rₜ = 2.75 min; MS (ESIpos): m/z = 737 (M+H)⁺.

### Intermediat F214

### N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein-trifluoressigsäure (1:1)

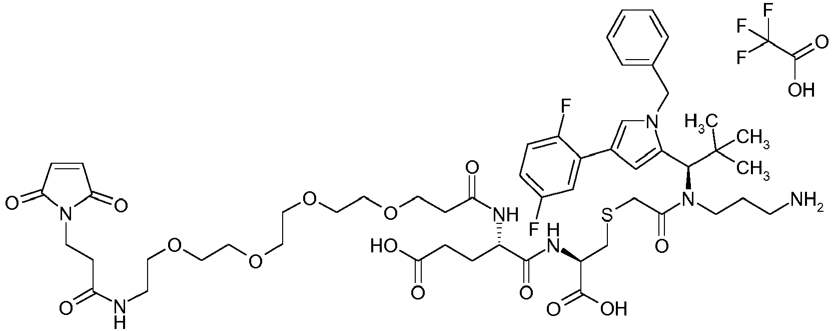

111.7 mg (0.30 mmol) (2S)-5-(Benzyloxy)-2-{[(benzyloxy)carbonyl]amino}-5-oxopentansäure wurden in 3.0 mL DMF vorgelegt und mit 46.1 (0.30 mmol) HOBt, 96.6 mg (0.30 mmol) TBTU und 38.9 mg (0.30 mmol) N,N-Diisopropylethylamin versetzt. Die Reaktionsmischung wurde 10 min bei RT gerührt. 250.0 mg (0.30 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) gelöst in 116.3 mg (0.9 mmol) N,N-Diisopropylethylamin und 3.0 mL DMF wurden dann zugegeben. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 257.0 mg (80 % d. Th.) der Verbindung (16R)-11-{(1R)-1-[1-Benzyl-4-(2,5-difluorphenyl)-1H-pyrrol-2-yl]-2,2-dimethylpropyl}-16-{[(2S)-5-(benzyloxy)-2-{[(benzyloxy)carbonyl]amino}-5-oxopentanoyl]amino}-2,2-dimethyl-6,12-dioxo-5-oxa-14-thia-7,11-diaza-2-silaheptadecan-17-säure.
LC-MS (Methode 1): Rₜ = 1.55 min; MS (ESIpos): m/z = 1071 (M+H)⁺.

Unter Argon wurden 24.6 mg (0.11 mmol) Palladium(II)acetat in 5.0 mL Dichlormethan vorgelegt und mit 33.2 mg (0.33 mmol) Triethylamin und 254.3 mg (2.19 mmol) Triethylsilan versetzt. Die Reaktionsmischung wurde 5 min bei RT gerührt und mit 234.1 mg (0.22 mmol) (16R)-11-{(1R)-1-[1-Benzyl-4-(2,5-difluorphenyl)-1H-pyrrol-2-yl]-2,2-dimethylpropyl}-16-{[(2S)-5-(benzyloxy)-2-{[(benzyloxy)carbonyl]amino}-5-oxopentanoyl]amino}-2,2-dimethyl-6,12-dioxo-5-oxa-14-thia-7,11-diaza-2-silaheptadecan-17-säure gelöst in 5.0 mL Dichlormethan versetzt. Die Reaktionsmischung wurde über Nacht bei RT gerührt. Die Reaktionsmischung wurde über einen Pappfilter filtriert und der Filterkuchen mit Dichlormethan nachgewaschen. Das Lösemittel wurde im Vakuum ohne Erwärmen verdampft. Der Rückstand wurde mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 177.5 mg (85 % d. Th.) der Verbindung L-alpha-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-S-oxa-7,11-diaza-2-silatridecan-13-yl)-L-cystein-trifluoressigsäure (1:1).
LC-MS (Methode 1): Rₜ = 1.07 min; MS (ESIpos): m/z = 846 (M+H)⁺.

20.0 mg (20.83 µmol) L-alpha-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) wurden zusammen mit 11.8 mg (22.91 µmol) 3-(2,5-Dioxo-2,5-dihydro-1H-pyrrol-1-yl)-N-{15-[(2,5-dioxopyrrolidin-1yl)oxy]-15-oxo-3,6,9,12-tetraoxapentadec-1-yl}propanamid in 1.5 mL DMF vorgelegt und mit 6.3 mg (62.49 µmol) 4-Methylmorpholin versetzt. Die Reaktionsmischung wurde über Nacht bei RT gerührt und dann mit 4.4 mg (0.07 mmol) Essigsäure versetzt. Die Reaktionsmischung wurde direkt mittles präp. RP-HPLC (Säule: Reprosil 250x30; 10µ, Fluss: 50 mL/min, MeCN/Wasser) gereinigt. Die Lösemittel wurden im Vakuum verdampft und der Rückstand im Hochvakuum getrocknet. Man erhielt 19.1 mg (74 % d. Th.) der Verbindung N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein.
LC-MS (Methode 1): Rₜ = 1.24 min; MS (ESIpos): m/z = 1244 (M+H)⁺.

17.5 mg (14.06 µmol) N-[19-(2,5-Dioxo-2,5-dihydro-1H-pyrrol-1-yl)-17-oxo-4,7,10,13-tetraoxa-16-azanonadecan-1-oyl]-L-alpha-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 1.5 mL Trifluorethanol gelöst und mit 11.5 mg (84.37 µmol) Zinkdichlorid versetzt. Die Reaktionsmischung rührte 4 h bei 50 °C. Die Reaktionsmischung wurde mit 24.7 mg (0.08 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 10.8 mg (63 % d. Th.) der Titelverbindung
LC-MS (Methode 1): Rₜ = 0.89 min; MS (ESIpos): m/z = 1100 (M+H)⁺.

### Intermediat F215

### N-[19-(2,5-Dioxo-2,5-dihydro-1H-pyrrol-1-yl)-17-oxo-4,7,10,13-tetraoxa-16-azanonadecan-1-oyl]-L-valyl-N-{3-[({[(2R)-2-acetamido-2-carboxyethyl]sulfanyl}acetyl){(1R)-1-[1-benzyl-4-(2,S-difluorphenyl)-1H-pyrrol-2-yl]-2,2-dimethylpropyl}amino]propyl}-L-alaninamid

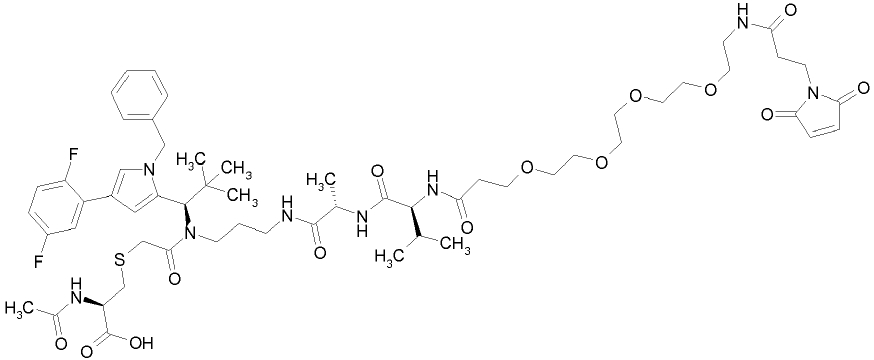

14.9 mg (0.02 mmol) N-Acetyl-S-[2-([3-(L-alanylamino)propyl]{(1R)-1-[1-benzyl-4-(2,5-difluorphenyl)-1H-pyrrol-2-yl]-2,2-dimethylpropyl}amino)-2-oxoethyl]-L-cystein-trifluoressigsäure (1:1) (Beispiel 229) und 7.1 mg (0.02 mmol) 2,5-Dioxopyrrolidin-1-yl-N-[(benzyloxy)carbonyl]-L-valinat wurden in 1.0 mL DMF vorgelegt und mit 5.7 mg (0.06 mmol) 4-Methylmorpholin versetzt. Die Reaktionsmischung wurde über Nacht bei RT gerührt und dann mit 4.5 mg (0.08 mmol) Essigsäure versetzt. Die Reaktionsmischung wurde direkt mittles präp. RP-HPLC (Säule: Reprosil 250x30; 10µ, Fluss: 50 mL/min, MeCN/Wasser). Die Lösemittel wurden im Vakuum verdampft und der Rückstand im Hochvakuum getrocknet. Man erhielt 13.3 mg (78 % d. Th.) der Verbindung N-[(Benzyloxy)carbonyl]-L-valyl-N-{3-[({[(2R)-2-acetamido-2-carboxyethyl]sulfanyl}acetyl){(1R)-1-[1-benzyl-4-(2,5-difluorphenyl)-1H-pyrrol-2-yl]-2,2-dimethylpropyl}amino]propyl}-L-alaninamid. LC-MS (Methode 1): Rₜ = 1.24 min; MS (ESIpos): m/z = 919 (M+H)⁺.

11.1 mg (0.01 mmol) N-[(Benzyloxy)carbonyl]-L-valyl-N-{3-[({[(2R)-2-acetamido-2-carboxyethyl]sulfanyl}acetyl){(1R)-1-[1-benzyl-4-(2,5-difluorphenyl)-1H-pyrrol-2-yl]-2,2-dimethylpropyl}amino]propyl}-L-alaninamid wurden in 5.0 mL Ethanol gelöst, mit 1.0 mg Palladium auf Aktivkohle (10 %) versetzt und bei RT und Normaldruck über Nacht hydriert. Die Reaktionsmischung wurde über Celite filtriert und der Filterkuchen wurde mit einem Ethanol/THF/Wasser Gemisch nachgewaschen. Die Lösemittel wurden im Vakuum verdampft. Der Rückstand wurde mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand wurde lyophilisiert. Man erhielt 7.5 mg (69 % d. Th.) der Verbindung L-Valyl-N-{3-[({[(2R)-2-acetamido-2-carboxyethyl]sulfanyl}acetyl){(1R)-1-[1-benzyl-4-(2,5-difluorphenyl)-1H-pyrrol-2-yl]-2,2-dimethylpropyl}amino]propyl}-L-alaninamid-trifluoressigsäure (1:1).
LC-MS (Methode 1): Rₜ = 0.86 min; MS (ESIpos): m/z = 785 (M+H)⁺.

7.3 mg (8.12 µmol) L-Valyl-N-{3-[({[(2R)-2-acetamido-2-carboxyethyl]sulfanyl}acetyl){(1R)-1-[1-benzyl-4-(2,5-difluorphenyl)-1H-pyrrol-2-yl]-2,2-dimethylpropyl}amino]propyl}-L-alaninamid - trifluoressigsäure (1:1) wurden zusammen mit 4.6 mg (8.93 µmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid in 0.5 mL DMF vorgelegt und mit 2.5 mg (24.36 µmol) 4-Methylmorpholin versetzt. Die Reaktionsmischung wurde über Nacht bei RT gerührt und dann mit 4.4 mg (0.03 mmol) Essigsäure versetzt. Die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 4.9 mg (50 % d. Th.) der Titelverbindung.
LC-MS (Methode 1): Rₜ = 1.07 min; MS (ESIpos): m/z = 1183 (M+H)⁺.

### Intermediat F216

### S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-L-cysteinyl-beta-alanin -trifluoressigsäure (1:1)

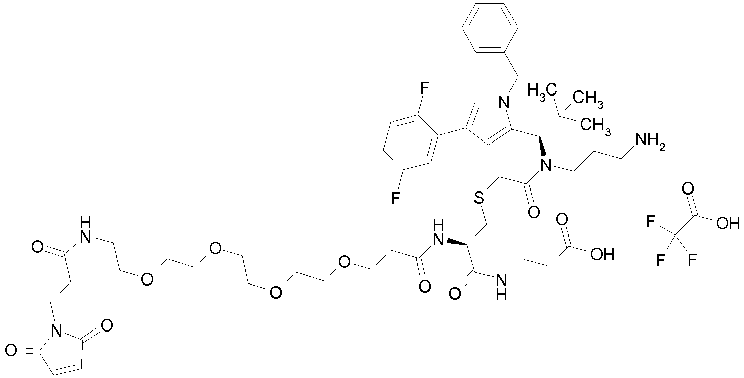

Unter Argon wurden 30.2 mg (0.06 mmol) N,N'-Bis[(benzyloxy)carbonyl]-L-cystin in 2.0 mL Wasser und 2.0 mL *iso*-Propanol vorgelegt und mit 56.7 mg (0.20 mmol) TCEP versetzt. Die Reaktionsmischung wurde 30 min bei RT gerührt. Es wurden dann 50.0 mg (0.08 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat(Intermediat C70) gelöst in 2.0 mL *iso*-Propanol und 122.2 mg (0.48 mmol) 1,8-Diazabicyclo(5.4.0)undec-7-en zugegeben und die Reaktionsmischung wurde 7 h bei 50 °C gerührt. Dann wurden nochmals 122.2 mg (0.48 mmol) 1,8-Diazabicyclo(5.4.0)undec-7-en zugegeben und die Reaktionsmischung wurde 1 h bei 50 °C gerührt. Es wurde mit Ethylacetat verdünnt und die organische Phase wurde mit Wasser und ges. Natriumhydrogencarbonat-Lsg. extrahiert und mit ges. NaCl-Lsg. gewaschen. Die organische Phase wurde über Magnesiumsulfat getrocknet und das Lösemittel im Vakuum verdampft. Der Rückstand wurde mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 43.1 mg (64 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(benzyloxy)carbonyl] -L-cystein.
LC-MS (Methode 1): Rₜ = 1.46 min; MS (ESIpos): m/z = 851 (M+H)⁺.

16.5 mg (0.05 mmol) 4-Methylbenzolsulfonsäure-benzyl-beta-alaninat (1:1) wurden zusammen mit 14.0 mg (0.11 mmol) N,N-Diisopropylethylamin in 1.5 mL Acetonitril vorgelegt. Die Reaktionsmischung wurde 3 min bei RT gerührt und dann wurden 30.8 mg (0.04 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(benzyloxy)carbonyl]-L-cystein gelöst in 1.5 mL Acetonitril, 23.4 mg (0.18 mmol) N,N-Diisopropylethylamin und 29.9 mg (0.05 mmol) T3P (50 % in Ethylacetat) zugegeben. Die Reaktionsmischung wurde über Nacht bei RT gerührt. Es wurde Wasser zugegeben und die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Benzyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(benzyloxy)carbonyl]-L-cysteinyl-beta-alaninat wurde als Verbindung erhalten.
LC-MS (Methode 1): Rₜ = 1.59 min; MS (ESIpos): m/z = 1012 (M+H)⁺.

43.8 mg (43.3 µmol) Benzyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(benzyloxy)carbonyl]-L-cysteinyl-beta-alaninat wurden in 8.0 mL Ethanol gelöst, mit 4.4 mg Palladium auf Aktivkohle (10%) versetzt und bei RT und Normaldruck über Nacht hydriert. Die Reaktionsmischung wurde über einen Pappfilter filtriert und der Filterkuchen wurde mit einem Ethanol nachgewaschen. Das Lösemittel wurden im Vakuum verdampft. Der Rückstand wurde noch zweimal wie soeben beschrieben behandelt. Der Rückstand wurde mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 14.5 mg (37 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteinyl-beta-alanin - trifluoressigsäure (1:1).
LC-MS (Methode 1): Rₜ = 1.08 min; MS (ESIpos): m/z = 788 (M+H)⁺.

14.5 mg (16.1 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteinyl-beta-alanin-trifluoressigsäure (1:1) wurden zusammen mit 9.1 mg (17.7 µmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid in 1.0 mL DMF vorgelegt und mit 4.9 mg (48.2 µmol) 4-Methylmorpholin versetzt. Die Reaktionsmischung wurde über Nacht bei RT gerührt und dann mit 3.4 mg (0.06 mmol) Essigsäure versetzt. Die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 4.9 mg (50 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cysteinyl-beta-alanin-trifluoressigsäure (1:1).
LC-MS (Methode 1): Rₜ = 1.28 min; MS (ESIpos): m/z = 1186 (M+H)⁺.

14.1 mg (11.9 µmol) S-(11-{(1R)-1-[1-Benzyl-4-(2,5-difluorphenyl)-1H-pyrrol-2-yl]-2,2 dimethylpropyl}-2,2-dimethyl-6,12-dioxo-5-oxa-7,11-diaza-2-silatridecan-13-yl)-N-[19-(2,5-dioxo-2,5-dihydro-1H-pyrrol-1-yl)-17-oxo-4,7,10,13-tetraoxa-16-azanonadecan-1-oyl]-L-cysteinyl-beta-alanin-trifluoressigsäure (1:1) wurden in 1.5 mL Trifluorethanol gelöst und mit 9.7 mg (71.3 µmol) Zinkdichlorid versetzt. Die Reaktionsmischung rührte 3 h bei 50 °C Es wurden nochmals 9.7 mg (71.3 µmol) Zinkdichlorid zugegeben und die Reaktionsmischung rührte 3 h bei 50 °C. Es wurden nochmals 9.7 mg (71.3 µmol) Zinkdichlorid zugegeben und die Reaktionsmischung rührte 4 h bei 70 °C. Die Reaktionsmischung wurde mit 20.8 mg (0.07 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand wurde lyophilisiert. Man erhielt 6.2 mg (44 % d. Th.) der Titelverbindung.
LC-MS (Methode 1): Rₜ = 0.82 min; MS (ESIpos): m/z = 1042 (M+H)⁺.

### Intermediat F217

### S-{2-[(3-Aminopropyl)-{(1R)-1-[1-benzyl-4-(2,5-difluorphenyl)-1H-pyrrol-2-yl]-2,2 dimethylpropyl}amino]-2-oxoethyl}-N-[(2,5-dioxo-2,5-dihydro-1H-pyrrol-1-yl)acetyl]-L-cystein-trifluoressigsäure (1:1)

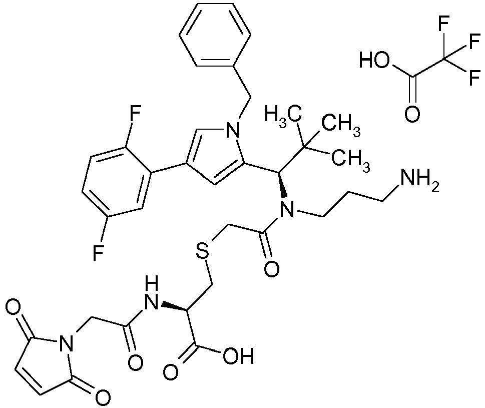

Unter Argon wurden 7.5 mg (0.05 mmol) (2,5-Dioxo-2,5-dihydro-1H-pyrrol-1-yl)essigsäure in 1.5 mL DMF vorgelegt und mit 7.5 mg (0.05 mmol) HOBt, 15.5 mg (0.05 mmol) TBTU und 6.2 mg (0.05 mmol) N,N-Diisopropylethylamin versetzt. Die Reaktionsmischung wurde 10 min bei RT gerührt. Es wurden dann 40.0 mg (0.05 mmol) S-(11-{(R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) (Intermediat C71) gelöst in 1.5 mL DMF und 18.7 mg (0.14 mmol) N,N-Diisopropylethylamin zugegeben und die Reaktionsmischung wurde über Nacht bei RT gerührt. Die Reaktionsmischung wurde direkt mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 11.2 mg (25 % d. Th.) der Verbindung S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(2,5-dioxo-2,5-dihydro-1H-pyrrol-1-yl)acetyl]-L-cystein.
LC-MS (Methode 1): Rₜ = 1.37 min; MS (ESIpos): m/z = 854 (M+H)⁺.

10.9 mg (12.8 µmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[(2,5-dioxo-2,5-dihydro-1H-pyrrol-1-yl)acetyl]-L-cystein wurden in 2.0 mL Trifluorethanol gelöst und mit 10.4 mg (76.6 µmol) Zinkdichlorid versetzt. Die Reaktionsmischung rührte 4 h bei 50 °C. Die Reaktionsmischung wurde mit 22.4 mg (0.08 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand wurde lyophilisiert. Man erhielt 7.5 mg (65 % d. Th.) der Titelverbindung.
LC-MS (Methode 1): Rₜ = 0.92 min; MS (ESIpos): m/z = 710 (M+H)⁺.

### Intermediat F218

### N-[19-(2,5-Dioxo-2,5-dihydro-1H-pyrrol-1-yl)-17-oxo-4,7,10,13-tetraoxa-16-azanonadecan-1-oyl]-L-gamma-glutamyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein-trifluoressigsäure (1:1)

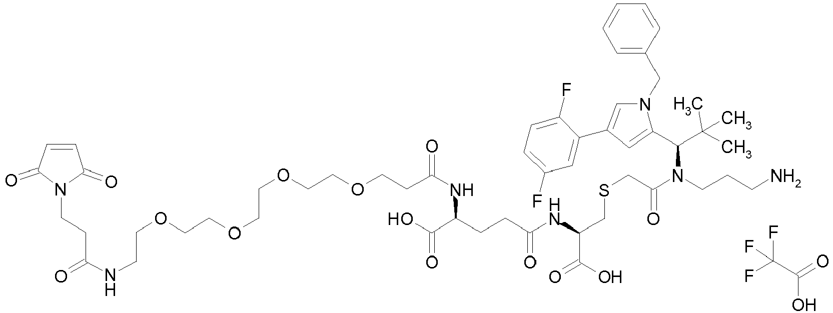

Unter Argon wurden 22.9 mg (0.06 mmol) (4S)-5-(Benzyloxy)-4-{[(benzyloxy)carbonyl]amino}-5-oxopentansäure in 2.0 mL DMF vorgelegt und mit 9.4 mg (0.05 mmol) HOBt, 19.8 mg (0.06 mmol) TBTU und 8.0 mg (0.06 mmol) N,N-Diisopropylethylamin versetzt. Die Reaktionsmischung wurde 10 min bei RT gerührt. Es wurden dann 51.2 mg (0.06 mmol) S-(11-{(R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein (Intermediat C71) gelöst in 1.0 mL DMF und 23.9 mg (0.19 mmol) N,N-Diisopropylethylamin zugegeben und die Reaktionsmischung wurde über Nacht bei RT gerührt. Die Reaktionsmischung wurde direkt mittles präp. RP-HPLC (Säule: Reprosil 250x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 11.2 mg (25 % d. Th.) der Verbindung (16R)-11-{(1R)-1-[1-Benzyl-4-(2,5-difluorphenyl)-1H-pyrrol-2-yl]-2,2-dimethylpropyl}-16-{[(4S)-5-(benzyloxy)-4-{[(benzyloxy)carbonyl]amino}-5-oxopentanoyl]amino}-2,2-dimethyl-6,12-dioxo-5-oxa-14-thia-7,11-diaza-2-silaheptadecan-17-säure.
LC-MS (Methode 1): Rₜ = 1.52 min; MS (ESIpos): m/z = 1070 (M+H)⁺.

Unter Argon wurden 3.9 mg (0.02 mmol) Palladium(II)acetat in 1.0 mL Dichlormethan vorgelegt und mit 5.3 mg (0.05 mmol) Triethylamin und 254.3 mg (2.19 mmol) Triethylsilan versetzt. Die Reaktionsmischung wurde 5 min bei RT gerührt und mit 18.6 mg (0.02 mmol) (16R)-11-{(1R)-1-[1-Benzyl-4-(2,5-difluorphenyl)-1H-pyrrol-2-yl]-2,2-dimethylpropyl}-16-{[(4S)-5-(benzyloxy)-4-{[(benzyloxy)carbonyl]amino}-5-oxopentanoyl]amino}-2,2-dimethyl-6,12-dioxo-5-oxa-14-thia-7,11-diaza-2-silaheptadecan-17-säure gelöst in 1.0 mL Dichlormethan versetzt. Das Lösemittel wurde im Vakuum ohne Erwärmen verdampft. Der Rückstand wurde in Aceonitril aufgenommen, durch einen Spritzenfilter filtriert und mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 11.0 mg (66 % d. Th.) der Verbindung L-gamma-Glutamyl-S-(11-{(1R)-1-[1-benzyl-4-(2,5-difluorphenyl)-1H-pyrrol-2-yl]-2,2-dimethylpropyl} -2,2-dimethyl-6,12-dioxo-5-oxa-7,11-diaza-2-silatridecan-13-yl)-L-cystein-trifluoressigsäure (1:1).
LC-MS (Methode 1): Rₜ = 1.14 min; MS (ESIpos): m/z = 846 (M+H)⁺.

15.0 mg (15.6 µmol) L-gamma-Glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein-trifluoressigsäure (1:1) wurden zusammen mit 8.8 mg (17.2 µmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid in 1.0 mL DMF vorgelegt und mit 4.7 mg (46.9 µmol) 4-Methylmorpholin versetzt. Die Reaktionsmischung wurde über Nacht bei RT gerührt und dann mit 3.3 mg (0.06 mmol) Essigsäure versetzt. Die Reaktionsmischung wurde direkt mittles präp. RP-HPLC (Säule: Reprosil 250x30; 10µ, Fluss: 50 mL/min, MeCN/Wasser) gereinigt. Die Lösemittel wurden im Vakuum verdampft und der Rückstand im Hochvakuum getrocknet. Man erhielt 14.2 mg (70 % d. Th.) der Verbindung N-[19-(2,5-Dioxo-2,5-dihydro-1H-pyrrol-1-yl)-17-oxo-4,7,10,13-tetraoxa-16-azanonadecan-1-oyl]-L-gamma-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein.
LC-MS (Methode 4) Rₜ = 1.24 min; MS (ESIpos): m/z = 1244 (M+H)⁺.

13.8 mg (11.1 µmol) N-[19-(2,5-Dioxo-2,5-dihydro-1H-pyrrol-1-yl)-17-oxo-4,7,10,13-tetraoxa-16-azanonadecan-1-oyl]-L-gamma-glutamyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 2.0 mL Trifluorethanol gelöst und mit 9.1 mg (66.5 µmol) Zinkdichlorid versetzt. Die Reaktionsmischung rührte 4 h bei 50 °C. Die Reaktionsmischung wurde mit 19.4 mg (0.07 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 6.9 mg (50 % d. Th.) der Titelverbindung.
LC-MS (Methode 1): Rₜ = 0.89 min; MS (ESIpos): m/z = 1100 (M+H)⁺.

### Intermediat F235

### Trifluoressigsäure--N-[19-(2,5-dioxo-2,5-dihydro-1H-pyrrol-1-yl)-17-oxo-4,7,10,13-tetraoxa-16-azanonadecan-1-oyl]-L-valyl-N-{4-[(3-aminopropyl){(1R)-1-[1-benzyl-4-(2,5-difluorphenyl)-1H-pyrrol-2-yl] -2,2-dimethylpropyl}carbamoyl]phenyl}-L-alaninamid (1:1)

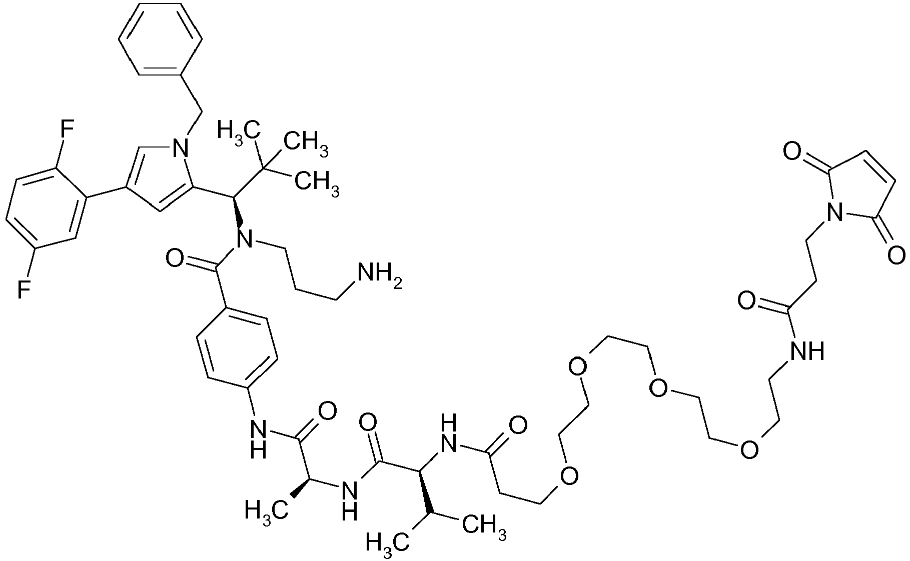

120.0 mg (0.22 mmol) 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat (siehe Synthese Intermediat C11) und 52.1 mg (0.28 mmol) 4-Nitrobenzoylchlorid wurden in 8.0 mL Dichlormethan gelöst und mit 28.4 mg (0.28 mmol) Triethlyamin versetzt. Die Reaktionsmischung rührte über Nacht bei RT. Das Lösemittel wurde im Vakuum verdampft und der Rückstand wurde mittles präp. RP-HPLC (Säule: Reprosil 125x30; 10µ, Fluss: 50 mL/min, MeCN/Wasser, 0.1% TFA) gereinigt. Man erhielt 97.7 mg (64 % d. Th.) der Verbindung 2-(Trimethylsilyl)ethyl-{3-[{(1R)-1-[1-benzyl-4-(2,5-difluorphenyl)-1H-pyrrol-2-yl]-2,2-dimethylpropyl}(4-nitrobenzoyl)amino]propyl}carbamat.
LC-MS (Methode 1): Rₜ = 1.54 min; MS (ESIpos): m/z = 705 (M+H)⁺.

97.0 mg (0.14 mmol) 2-(Trimethylsilyl)ethyl-{3-[{(1R)-1-[1-benzyl-4-(2,5-difluorphenyl)-1H-pyrrol-2-yl]-2,2-dimethylpropyl}(4-nitrobenzoyl)amino]propyl}carbamat wurden in 5.0 mL Ethanol gelöst, mit 9.7 mg Palladium auf Aktivkohle (10 %) versetzt und 5 h bei Normaldruck hydriert. Die Reaktionsmischung wurde über einen Papierfilter filtriert und der Filterkuchen wurde mit einem Ethanol nachgewaschen. Das Lösemittel wurde im Vakuum verdampft. Der Rückstand wurde ohne weitere Reinigung in der nächsten Synthesestufe eingesetzt. Man erhielt 87.4 mg (88 % d. Th.) der Verbindung 2-(Trimethylsilyl)ethyl-{3-[(4-aminobenzoyl){(1R)-1-[1-benzyl-4-(2,5-difluorphenyl)-1H-pyrrol-2-yl]-2,2-dimethylpropyl}amino]propyl}carbamat.
LC-MS (Methode 1): Rₜ = 1.47 min; MS (ESIpos): m/z = 675 (M+H)⁺.

59.3 mg (0.09 mmol) 2-(Trimethylsilyl)ethyl-{3-[(4-aminobenzoyl){(1R)-1-[1-benzyl-4-(2,5-difluorphenyl)-1H-pyrrol-2-yl]-2,2-dimethylpropyl}amino]propyl}carbamat und 25.5 mg (0.11 mmol) N-[(Benzyloxy)carbonyl]-L-alanin wurden zusammen mit 68.1 mg (0.53 mmol) N,N-Diisopropylethylamin in 5.0 mL Acetonitril vorgelegt. Es wurde 72.7 mg (0.11 mmol) T3P (50 % in Ethylacetat) langsam zugegeben. Die Reaktionsmischung wurde über Nacht bei RT gerührt. Die Reaktionsmischung wurde direkt mittles präp. RP-HPLC (Säule: Reprosil 125x30; 10µ, Fluss: 50 mL/min, MeCN/Wasser, 0.1 % TFA) gereinigt. Die Lösemittel wurden im Vakuum verdampft und der Rückstand im Hochvakuum getrocknet. Man erhielt 52.2 mg (68 % d. Th.) der Verbindung Benzyl-[(2S)-1-{[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]amino}-1-oxopropan-2-yl]carbamat. LC-MS (Methode 1): Rₜ = 1.48 min; MS (ESIpos): m/z = 880 (M+H)⁺.

23.9 mg (0.03 mmol) Benzyl-[(2S)-1-{[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]amino}-1-oxopropan-2-yl]carbamat wurden in 3.0 mL Ethylacetat gelöst, mit 2.4 mg Palladium auf Aktivkohle (10 %) versetzt und 2 h bei Normaldruck hydriert. Die Reaktionsmischung wurde über einen Papierfilter filtriert und der Filterkuchen wurde mit Ethylacetat nachgewaschen. Das Lösemittel wurde im Vakuum verdampft. Der Rückstand wurde ohne weitere Reinigung in der nächsten Synthesestufe eingesetzt. Man erhielt 20.1 mg (90 % d. Th.) der Verbindung 2-(Trimethylsilyl)ethyl-[3-([4-(L-alanylamino)benzoyl]{(1R)-1-[1-benzyl-4-(2,5-difluorphenyl)-1H-pyrrol-2-yl]-2,2-dimethylpropyl}amino)propyl]carbamat.
LC-MS (Methode 1): Rₜ = 1.13 min; MS (ESIpos): m/z = 746 (M+H)⁺.

20.0 mg (0.03 mmol) 2-(Trimethylsilyl)ethyl-[3-([4-(L-alanylamino)benzoyl]{(1R)-1-[1-benzyl-4-(2,5-difluorphenyl)-1H-pyrrol-2-yl]-2,2-dimethylpropyl}amino)propyl]carbamat wurden zusammen mit 14.9 mg (0.04 mmol) 2,5-Dioxopyrrolidin-1-yl-N-[(benzyloxy)carbonyl]-L-valinat in 2.0 mL DMF vorgelegt und mit 5.4 mg (0.05 mmol) 4-Methylmorpholin versetzt. Die Reaktionsmischung wurde direkt mittles präp. RP-HPLC (Säule: Reprosil 125x30; 10µ, Fluss: 50 mL/min, MeCN/Wasser) gereinigt. Die Lösemittel wurden im Vakuum verdampft und der Rückstand im Hochvakuum getrocknet. Man erhielt die Verbindung N-[(Benzyloxy)carbonyl]-L-valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid.
LC-MS (Methode 1): Rₜ = 1.49 min; MS (ESIpos): m/z = 979 (M+H)⁺.

17.0 mg (17.4 µmol) N-[(Benzyloxy)carbonyl]-L-valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid wurden in 2.5 mL Ethylacetat gelöst, mit 1.7 mg Palladium auf Aktivkohle (10 %) versetzt und über Nacht bei Normaldruck hydriert. Die Reaktionsmischung wurde über einen Papierfilter filtriert und der Filterkuchen wurde mit einem Ethylacetat nachgewaschen. Das Lösemittel wurde im Vakuum verdampft und der Rückstand im Hochvakuum getrocknet. Man erhielt 15.3 mg (60 % d. Th.) der Verbindung L-Valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid.
LC-MS (Methode 1): Rₜ = 1.15 min; MS (ESIpos): m/z = 845 (M+H)⁺.

15.3 mg (0.01 mmol) L-Valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid wurden zusammen mit 7.9 mg (0.02 mmol) 3-(2,5-Dioxo-2,5-dihydro-1H-pyrrol-1-yl)-N-{15-[(2,5-dioxopyrrolidin-1-yl)oxy]-15-oxo-3,6,9,12-tetraoxapentadec-1-yl}propanamid in 2.4 mL DMF vorgelegt und mit 1.9 mg (0.02 mmol) 4-Methylmorpholin versetzt. Die Reaktionsmischung wurde über Nacht bei RT gerührt und dann mit 1.4 mg (0.02 mmol) Essigsäure versetzt. Die Reaktionsmischung wurde direkt mittles präp. RP-HPLC (Säule: Reprosil 125x30; 10µ, Fluss: 50 mL/min, MeCN/Wasser) gereinigt. Die Lösemittel wurden im Vakuum verdampft und der Rückstand im Hochvakuum getrocknet. Man erhielt 11.7 mg (70 % d. Th.) der Verbindung N-[19-(2,5-Dioxo-2,5-dihydro-1H-pyrrol-1-yl)-17-oxo-4,7,10,13-tetraoxa-16-azanonadecan-1-oyl]-L-valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid.

11.7 mg (0.01 mmol) N-[19-(2,5-Dioxo-2,5-dihydro-1H-pyrrol-1-yl)-17-oxo-4,7,10,13-tetraoxa-16-azanonadecan-1-oyl]-L-gamma-glutamyl-S-(11-{(1R)-1-[1-N-[19-(2,5-Dioxo-2,5-dihydro-1H-pyrrol-1-yl)-17-oxo-4,7,10,13-tetraoxa-16-azanonadecan-1-oyl]-L-valyl-N-[4-({(1R)-1-[1-benzyl-4-(2,5-difluorphenyl)-1H-pyrrol-2-yl]-2,2-dimethylpropyl}[3-({[2-(trimethylsilyl)ethoxy]carbonyl}amino)propyl]carbamoyl)phenyl]-L-alaninamid wurden in 2.0 mL Trifluorethanol gelöst und mit 3.9 mg (0.03 mmol) Zinkdichlorid versetzt. Die Reaktionsmischung rührte über Nacht bei 50 °C. Die Reaktionsmischung wurde mit 8.3 mg (0.03 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 5.4 mg (47 % d. Th.) der Titelverbindung.
LC-MS (Methode 1): Rₜ = 0.94 min; MS (ESIpos): m/z = 1100 (M+H)⁺.

### Intermediat F236

### (2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-4-{[(2,5-dioxo-2,5-dihydro-1H-pyrrol-1-yl)acetyl]amino}butansäure -trifluoressigsäure (1:1)

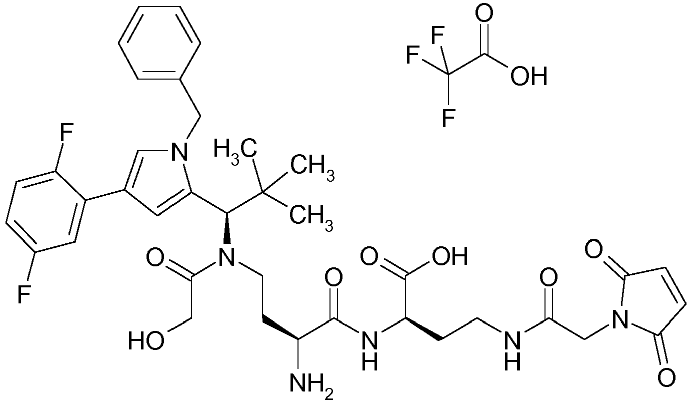

Die Synthese der Titelverbindung erfolgte in Analogie zu Intermediat F192 ausgehend von (2R)-4-{[(Benzyloxy)carbonyl]amino}-2-[(tert-butoxycarbonyl)amino]butansäure --N-cyclohexyl cyclohexanamin (1:1).
LC-MS (Methode 4): Rₜ = 1.1 min; MS (ESIpos): m/z = 751 (M+H)⁺.

### Intermediat F238

### Trifluoressigsäure --N-{(2S)-1-amino-3-[{(1R)-1-[1-benzyl-4-(2,S-difluorphenyl)-1H-pyrrol-2-yl]-2,2-dimethylpropyl}(glycoloyl)amino]propan-2-yl}-N'-[2-(2,5-dioxo-2,5-dihydro-1H-pyrrol-1-yl)ethyl]succinamid (1:1)

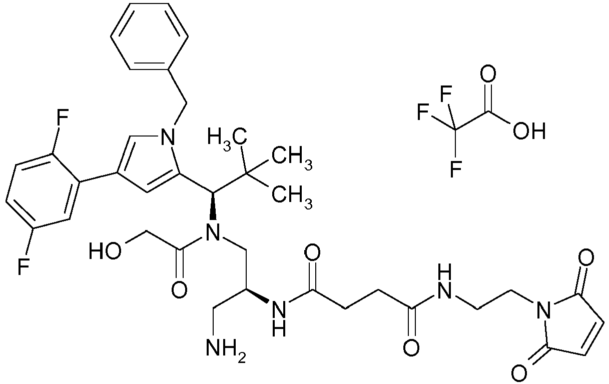

18 mg (0.025 mmol) von Intermediat C72 wurden in 6 ml DMF aufgenommen und mit 7.5 mg (0.03 mmol) von Trifluoressigsäure --1-(2-aminoethyl)-1H-pyrrol-2,5-dion (1:1) in Gegenwart von 11.3 mg (0.03 mmol) HATU und 22 µL *N,N*-Diisopropylethylamin gekuppelt. Nach 1 h Rühren bei RT wurde der Ansatz eingeengt und der Rückstand durch präparative HPLC gereinigt. Die entsprechenden Farktionen wurden eingeengt und der Rückstand aus Acetonitril/Wasser 1:1 lyophilisiert. Es wurden 15 mg (67% d. Th.) des Intermediats erhalten.
LC-MS (Methode 4): Rₜ = 1.71 min; MS (EIpos): m/z = 873 [M+Na]⁺.

Die Titelverbindung wurde anschließend aus diesem Intermediat durch Entschützung mit Zinkchlorid in 4 ml Trifluorethanol wie in Intermediat F119 beschrieben hergestellt. Nach Reinigung durch präparative HPLC wurden 8.5 mg (63% d. Th.) der Titelverbindung erhalten.
LC-MS (Methode 1): Rₜ = 0.86 min; MS (ESIpos): m/z = 707 (M+Na)⁺.

### Intermediat F241

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-(2-{[N-(bromacetyl)glycyl]amino}ethyl)butanamid (1:1)

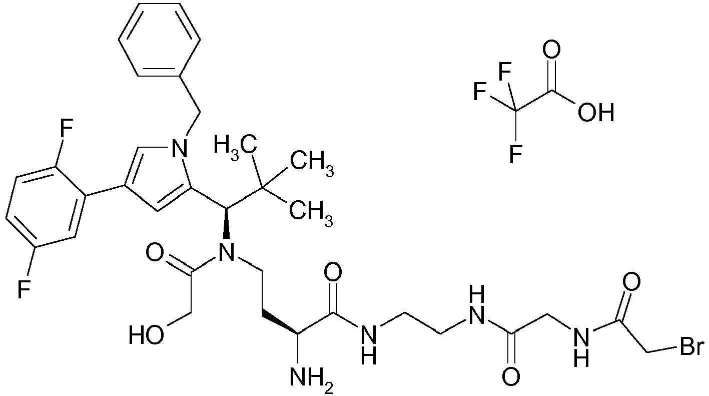

Die Titelverbindung wurde in Analogie aus Intermediat C66 durch Kupplung mit kommerziell erhältlichem 1-(2-Bromacetoxy)pyrrolidin-2,5-dion und anschließender Deblockierung mit Zinkclorid hergestellt.
LC-MS (Methode 1): Rₜ = 0.84 min; MS (EIpos): m/z = 733 und 735 [M+H]⁺.

### Intermediat F242

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-(3-{[(2,5-dioxo-2,5-dihydro-1H-pyrrol-1-yl)acetyl]amino}propyl)butanamid (1:1)

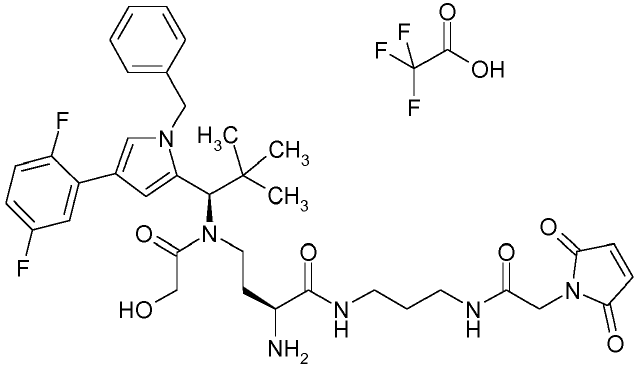

Die Synthese der Titelverbindung erfolgte in Analogie zu Intermediat F104.
LC-MS (Methode 1): Rₜ = 0.84 min; MS (ESIpos): m/z = 707 (M+H)⁺.

### Intermediat F243

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-[2-(2-{[(2,5-dioxo-2,5-dihydro-1H-pyrrol-1-yl)acetyl]amino}ethoxy)ethyl]butanamid (1:1)

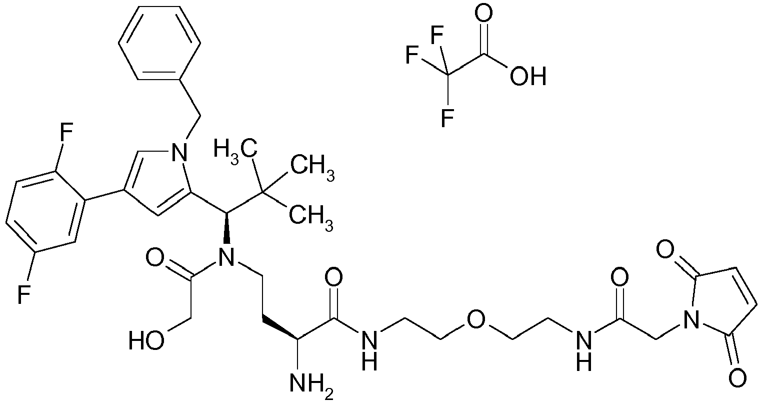

Die Synthese der Titelverbindung erfolgte in Analogie zu Intermediat F242.
LC-MS (Methode 1): Rₜ = 0.81 min; MS (ESIpos): m/z = 737 (M+H)⁺.

### Intermediat F244

### N-{2-[(S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-L-cysteinyl)amino]ethyl}-6-(2,5-dioxo-2,5-dihydro-1H-pyrrol-1-yl)hexanamid

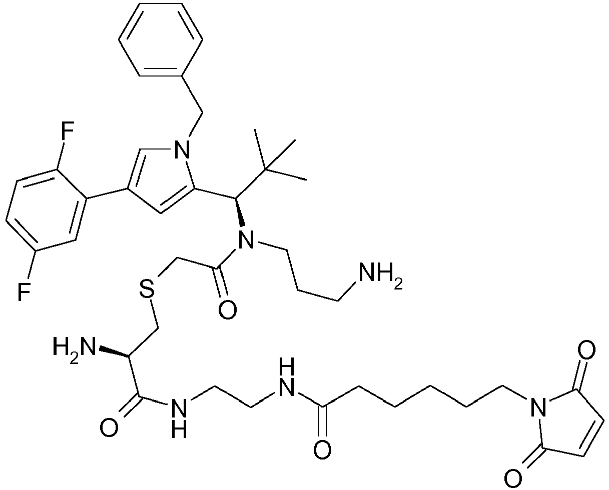

100 mg (ca. 0.101 mmol) S-(11-{(1R)-1-[1-Benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-N-[3-(trimethylsilyl)propanoyl]-L-cystein (Intermediat C 73) wurden in 88 ml Dimethylformamid vorgelegt und mit 107 mg (ca.0.15 mmol) N-(2-aminoethyl)-6-(2,5-dioxo-2,5-dihydro-1H-pyrrol-1-yl)hexanamide (Intermediat L73), 46 mg (0.12 mmol) HATU und 88 µl (0.50 mmol) versetzt. Die Reaktionsgemisch wurde 15 minuten bei RT gerührt. Das Gemisch wurde mit Wasser/Dichlormethan versetzt und dann die organische Phase wurde mit Wasser und Brine gewaschen, über Magnesiumsulfat getrocknet, am Rotationsverdampfer eingeengt und im Hochvakuum getrocknet. Der Rückstand wurde ohne weitere Reinigung weiter eingesetzt. Man erhielt 92 mg (59%, Reinheit 72%) der Titelverbindung.
LC-MS (Methode 1): Rₜ = 1.59 min; MS (ESIpos): m/z = 1096 (M+H)⁺.

Zu einer Lösung von 91 mg (ca. 0.06 mmol) 2-(Trimethylsilyl)ethyl-[(9R)-4-{(1R)-1-[1-benzyl-4-(2,5-difluorphenyl)-1H-pyrrol-2-yl]-2,2-dimethylpropyl}-20-(2,5-dioxo-2,5-dihydro-1H-pyrrol-1-yl)-5,10,15-trioxo-9-{[3-(trimethylsilyl)propanoyl]amino}-7-thia-4,11,14-triazaicos-1-yl]carbamat in 1.45 ml Trifluorethanol unter Argon wurden 40 mg (0.30 mmol) Zinkchlorid zugegeben. Das Reaktionsgemisch wurde 2h bei 50 °C gerührt. Danach wurden 30 mg (0.22 mmol) Zinkchlorid zugegeben und das Gemisch wurde 1 h bei RT weiter gerührt. Das Gemisch wurde mit 52 mg (0.18 mmol) EDTA versetzt und nach 10 Minuten Rühren bei RT wurde mit Wasser/Acetonitril etwas vedünnt und mittels präparativer HPLC (Eluent: ACN/Wasser +0.1 %TFA, Gradient) gereinigt. Man erhielt 17 mg (31%) der Titelverbindung.
LC-MS (Methode 1): Rₜ = 0.80 min; MS (ESIpos): m/z = 808 (M+H)⁺.

### Intermediat F245

### Trifluoressigsäure --N-{(2S)-2-amino-4-[{(1R)-1-[1-benzyl-4-(2,5-difluorphenyl)-1H-pyrrol-2-yl]-2,2-dimethylpropyl}(glycoloyl)amino]butyl}-N'-(2-{[(2,5-dioxo-2,5-dihydro-1H-pyrrol-1-yl)acetyl]amino}ethyl)succinamid (1:1)

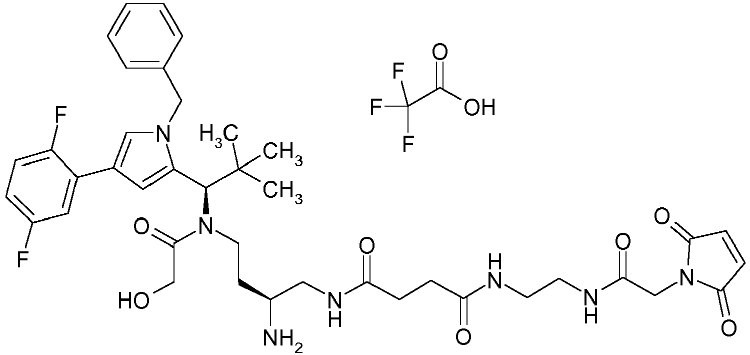

Die Titelverbindung wurde durch Kupplung von 10 mg (0.0135 mmol) von Intermediat C65 mit 8 mg (0.027 mmol) von Intermediat L1 in 8 ml DMF in Gegenwart von 15 mg (0.04 mmol) HATU sowie 9 µl N,N-Diisopropylethylamin und anschließender Entschützung mit Zinkchlorid in Trifluorethanol wie in Intermediat F119 beschrieben hergestellt. Nach Reinigung durch präparative HPLC wurden 8.8 mg (58% d. Th. über 2 Stufen) der Titelverbindung erhalten.
LC-MS (Methode 1): Rₜ = 0.84 min; MS (ESIpos): m/z = 778 (M+H)⁺.

### Intermediat F247

### Trifluoressigsäure -methyl-4-[(2-{[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-2-brom-4-oxobutanoat (1:1)

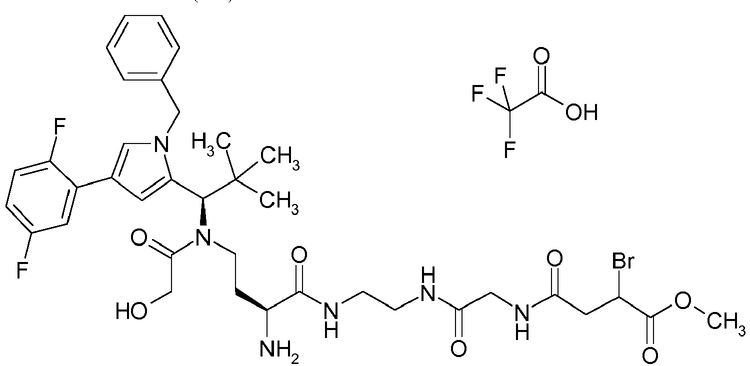

14 mg (0.018 mmol) von Intermediat C66 wurden in 14 mL DCM gelöst und mit 10.1 mg (0.037 mmol) 2-Brom-1-ethylpyridinium-Tetrafluoroborat (BEP) sowie portionsweise mit insgesamt 250 µl Pyridin versetzt, wobei der pH-Wert zwischen 5 und 6 gehalten wurde. Dann wurde mit Essigsäure ein pH-Wert von 4 eingestellt, der Ansatz eingeengt und der Rückstand durch präparative HPLC gereinigt. Nach Vereinigung der entsprechenden Fraktionen, Lyophilisation und Trocknung wurden 4 mg (21% d.Th.) der geschützten Zwischenstufe erhalten, die anschließend mit Zinkchlorid an der Aminofunktion entschützt wurde. Nach HPLC-Reinigung und Lyophilisation wurden 3 mg (72% d.Th.) der Titelverbindung als farbloser Schaum erhalten.
LC-MS (Methode 1): Rₜ = 0.88 min; MS (ESIpos): m/z = 805 und 807(M+H)⁺.

### Intermediat F248

### Trifluoressigsäure --(2S)-2-amino-4-[{(1R)-1-[1-benzyl-4-(2,5-difluorphenyl)-1H-pyrrol-2-yl]-2,2-dimethylpropyl}(glycoloyl)amino]-N-{2-[2-(2,5-dioxo-2,5-dihydro-1H-pyrrol-1-yl)ethoxy]ethyl}butanamid (1:1)

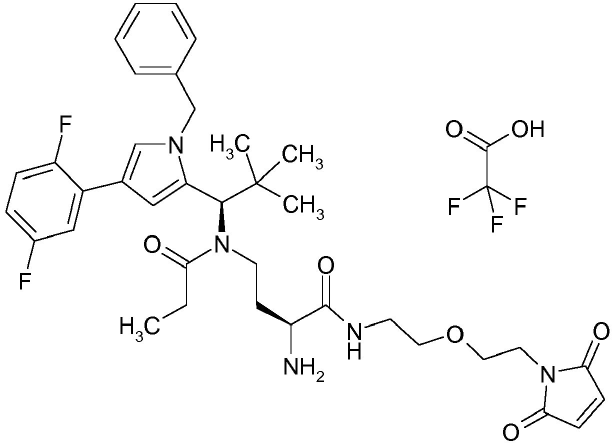

Die Titelverbindung wurde durch Kupplung von 10 mg (0.015 mmol) Intermediat C58 mit 5 mg (0.017 mmol) Intermediat L12 in Gegenwart von HATU und anschließende Entschützung mit Zinkchlorid hergestellt. Es wurden 6.5 mg (52% d.Th. über 2 Stufen) der Titelverbindung erhalten.
LC-MS (Methode 1): Rₜ = 0.91 min; MS (ESIpos): m/z = 680 (M+H)⁺.

### Intermediat F254

### Trifluoressigsäure-methyl-(3S)-4-[(2-{[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-brom-4-oxobutanoat (1:1)

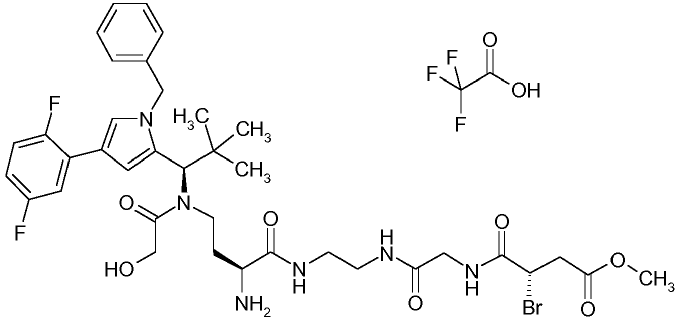

Die Titelverbindung wurde in Analogie zu Intermediat 247 durch Kupplung von 15 mg (0.02 mmol) Intermediat C66 mit 21 mg (0.099 mmol) (2S)-2-Brom-4-methoxy-4-oxobutansäure, die wie in (J.Org.Chem. 200, 65, 517-522) beschrieben aus (2S)-2-Amino-4-methoxy-4-oxobutansäurehydrochlorid (1:1) synthetisiert wurde, hergestellt.
LC-MS (Methode 1): Rₜ = 0.89 min; MS (ESIpos): m/z = 805 und 807(M+H)⁺.

### B: Herstellung von Antikörper-Wirkstoff-Konjugaten (ADC)

### B-1. Allgemeines Verfahren zur Generierung von anti-TWEAKR Antikörpern

Die anti-TWEAKR-Antikörper wurden beispielsweise durch das Screening einer Phagen Display Bibliothek auf rekombinantem humanem TWEAKR SEQ ID NO: 138 und murinem TWEAKR SEQ ID NO: 137 generiert. Die so gewonnenen Antikörper wurden ins humane IgG1 Format reformatiert und für die hier beschriebenen Ausführungsbeispiele verwendet. Darüber hinaus sind dem Fachmann Antikörper, die an TWEAKR binden bekannt, siehe z.B. WO2009/020933(A2) oder WO2009140177 (A2).

### B-2. Allgemeines Verfahren zur Expression von anti-TWEAKR Antikörpern in Säugerzellen

Die Antikörper, zum Beispiel TPP-2090 wurden in transienten Säugerzellkulturen produziert, wie von Tom et al., Kapitel 12 in Methods Express: Expression Systems herausgegeben von Micheal R. Dyson and Yves Durocher, Scion Publishing Ltd, 2007 (siehe AK-Beispiel 1) beschrieben.

### B-3. Allgemeines Verfahren zur Aufreinigung von Antikörpern aus Zellüberständen.

Die Antikörper, zum Beispiel TPP-2090 wurde aus den Zellkulturüberständen gewonnen. Die Zellüberstände wurden durch Zentrifugation von Zellen geklärt. Anschließend wurde der Zellüberstand durch Affinitätschromatographie auf einer MabSelect Sure (GE Healthcare) Chromatographiesäule gereinigt. Dazu wurde die Säule in DPBS pH 7.4 (Sigma/Aldrich) equillibriert, der Zellüberstand aufgtretragen und die Säule mit ca 10 Säulenvolumina DPBS pH 7.4 + 500 mM Natriumchlorid gewaschen. Die Antikörper wurden in 50 mM Natriumacetat pH 3.5 + 500 mM Natriumchlorid eluiert und anschliessend durch Gelfiltrationschromatographie auf einer Superdex 200 Säule (GE Helthcare) in DPBS pH 7.4 weiter gereinigt.

Der kommerziell erhältliche Antikörper Cetuximab (Handelsname Erbitux) wurde über standardmäßige chromatographische Methoden (Protein A, präparative SEC) aus dem Handelsprodukt aufgereinigt.

Der kommerziell erhältliche Antikörper Trastuzumab (Handelsname Herceptin) wurde über standardmäßige chromatographische Methoden (Protein A, präparative SEC) aus dem Handelsprodukt aufgereinigt.

Aus dem Handelsprodukt (Handelsname CIMAher) wurde der Antikörper Nimotuzumab über standardmäßige chromatographische Methoden (Protein A, präparative SEC) aufgereinigt.

Aus dem Handelsprodukt (Handelsname Vectibix) wurde der Antikörper Panitumumab über standardmäßige chromatographische Methoden (Protein A, präparative SEC) aufgereinigt

### B-4. Allgemeines Verfahren zur Kupplung an Cystein-Seitenketten

In die Kupplungsreaktionen wurden folgende Antikörper eingesetzt:
Cetuximab (Anti EGFR AK)
Anti-TWEAKR AK 1 (TPP-2090)
Trastuzumab (Anti-Her2 AK)
Nimotuzumab (Anti-EGFR AK)
Panitumumab (Anti-EGFR AK)

Zu einer Lösung des entsprechenden Antikörpers in PBS-Puffer im Konzentrationsbereich zwischen 1 mg/mL und 20 mg/mL, bevorzugt im Bereich von ungefähr 10mg/ml bis 15 mg/ml wurden zwischen 2 und 5 Äquivalenten Tris(2-carboxyethyl)phosphin-Hydrochlorid (TCEP), gelöst in PBS-Puffer, gegeben und 1h bei RT gerührt. Dazu kann die Lösung des jeweils eingesetzten Antikörpers in der in den Ausführungsbeispielen angegebenen Konzentration eingesetzt werden oder gegebenenfalls auch mit PBS Puffer bis etwa auf die Hälfte der angegebenen Startkonzentration verdünnt werden, um in den bevorzugten Konzentrationsbereich zu kommen. Anschließend wurden, je nach angestrebter Beladung, zwischen 2 und 12 Äquivalenten, bevorzugt etwa 5-10 Äquivalente der zu kuppelnden Maleinimid-Vorläufer-Verbindung oder Halogenid-Vorläufer-Verbindung als Lösung in DMSO hinzugefügt. Dabei sollte die Menge an DMSO 10% des Gesamtvolumens nicht überschreiten. Der Ansatz wurde bei Maleinimid-Precursern 60-240 min bei RT und bei Halogenid-Precursern zwischen 8 und 24 h bei RT gerührt und anschließend über in PBS equillibierte PD 10-Säulen (Sephadex® G-25, GE Healthcare) gegeben und mit PBS-Puffer eluiert. Üblicherweise wurden, wenn nicht anders angegeben, 5 mg des entsprechenden Antikörpers in PBS-Puffer zur Reduktion und der nachfolgenden Kupplung eingesetzt. Nach Aufreinigung über die PD10 Säule wurden so jeweils Lösungen des entsprechenden ADCs in 3.5 mL PBS-Puffer erhalten. Anschließend wurde eine Aufkonzentration mittels Ultrazentrifugation durchgeführt und die Probe ggf. mit PBS-Puffer zurückverdünnt. Falls notwendig wurde zur besseren Abtrennung niedermolekularer Bestandteile die Aufkonzentration mittels Ultrafiltration nach Rückverdünnung mit PBS Puffer wiederholt. Für biologische Testungen wurden je nach Bedarf in den finalen ADC Proben ggf. durch Rückverdünnung Konzentrationen im Bereich von 0.5-15 mg/mL eingestellt. Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

Wenn nicht anders angegeben wurden die in den Beispielen dargestellten Immunokonjugate nach diesem Verfahren hergestellt. Die in den Beispielen dargestellten ADCs können in Abhängigkeit vom Linker gegebenenfalls auch mehr oder weniger stark ausgeprägt in Form der hydrolysierten offenkettigen Bernsteinsäureamide mit den Antikörpern verknüpft vorliegen.

Insbesondere die KSP-I-ADCs, die über die Linker-Substruktur 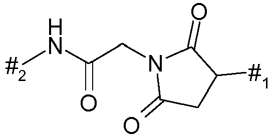 mit Thiolgruppen der Antikörper verknüpft sind, können auch ggf. durch Umpuffern im Anschluss an die Kupplung und ca. 20-stündiges Rühren bei pH 8 gemäß Schema 26 gezielt die über offenkettige Bersteinsäureamide verknüpften ADCs hergestellt werden.

#1 stellt die Schwefelbrücke zum Antikörper dar und #2die Verknüpfungsstelle zum modifizierten KSP-Inhibitor

Solche ADCs, bei denen der Linker über hydrolysierte offenkettige Bernsteinsäureamide mit den Antikörpern verknüpft vorliegt, können gegebenenfalls auch gezielt nach einer exemplarischen Vorschrift wie folgt dargestellt werden:
60 mg des betreffenden Antikörpers in 5 ml PBS-Puffer (c∼12 mg/mL) wurden unter Argon mit einer Lösung aus 0,344mg TCEP in 100µl PBS-Puffer versetzt. Der Ansatz wurde 30min bei RT gerührt und anschließend wurden 0.003 mmol einer Maleinimid-Vorläuferverbindung gelöst in 600µl DMSO zugegeben. Nach weiteren 1.5h-2h Rühren bei RT wurde der Ansatz mit 1075µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde mit PBS-Puffer pH8 auf ein Gesamtvolumen von 14 ml verdünnt. Diese Lösung wurde über Nacht bei RT unter Argon gerührt. Dann erfolgte ggf. wieder ein Umpuffern auf pH 7.2. Die ADC-Lösung wurde durch Ultrazentrifugation aufkonzentriert, rückverdünnt mit PBS-Puffer (pH7.2) und dann ggf. erneut bis auf eine Konzentration von etwa 10mg/mL aufkonzentriert.

Weitere potentiell hydrolyse-sensitive Thianylsuccinimid-Brücken zum Antikörper in den Ausführungsbeispielen enthalten folgende Linker-Substrukturen, wobei #1 die Thioetherverknüpfung zum Antikörper und #2 die Verknüpfungsstelle zum modifizierten KSP-Inhibitor darstellt:

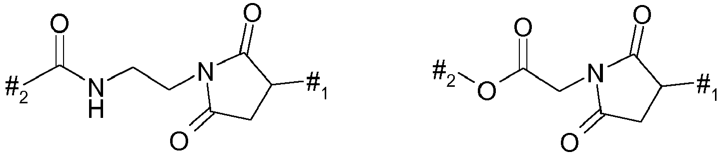

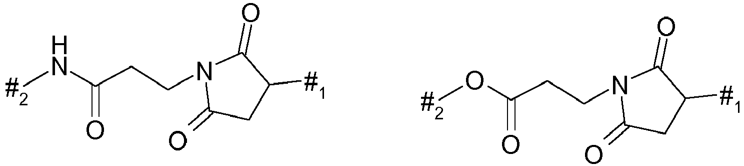

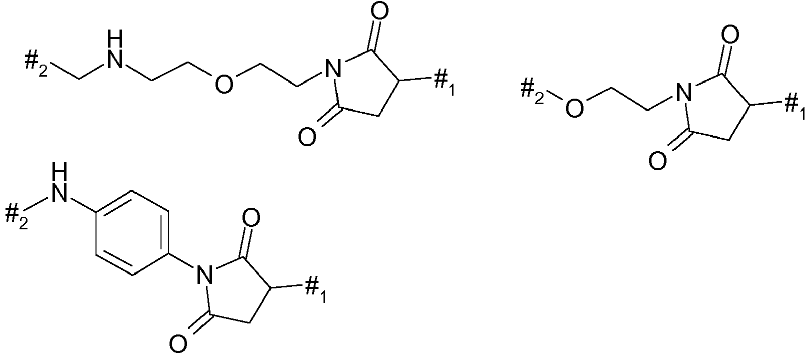

Diese Linker-Substrukturen stellen die Verknüpfungseinheit zum Antikörper dar und haben (neben der weiteren Linkerzusammensetzung) einen signifikanten Einfluß auf die Struktur und das Profil der in Tumorzellen gebildeten Metabolite.

In den dargestellten Strukturformeln hat dabei AK_{1A}, AK_{1B}, AK_{1E}, AK_{1I}, AK_{1H}, AK_{1K}, die Bedeutung
AK_{1A} = Cetuximab (partiell reduziert)- S§¹
AK_{1B} = anti-TWEAKR AK-1 (partiell reduziert)- S§¹
AK_{1E} = Trastuzumab (partiell reduziert)-S§¹
AK_{1I} = Nimotuzumab (partiell reduziert)-S§¹
AK_{1H} = Panitumumab (partiell reduziert)-S§¹
wobei
§¹ die Verknüpfung mit der Succinimid-Gruppe oder mit ggf. daraus entstandenen isomeren hydrolysierten offenkettigen Bernsteinsäureamiden bzw. des Alkylenrestes bedeutet,
   und
S für das Schwefelatom eines Cystein-Restes des partiell reduzierten Antikörpers steht.

### B-5. Allgemeines Verfahren zur Kupplung an Lysin-Seitenketten

In die Kupplungsreaktionen wurden folgende Antikörper eingesetzt:
Cetuximab (Anti EGFR AK)
Anti-TWEAKR AK 1 (TPP-2090)
Trastuzumab (Anti-Her2 AK)
Nimotuzumab (Anti-EGFR AK)
Panitumumab (Anti-EGFR AK)

Zu einer Lösung des entsprechenden Antikörpers in PBS-Puffer im Konzentrationsbereich zwischen 1 mg/mL und 20 mg/mL, bevorzugt etwa 10 mg/mL, wurden, je nach angestrebter Beladung, zwischen 2 und 8 Äquivalente der zu kuppelnden Vorläufer-Verbindung als Lösung in DMSO gegeben. Nach 30 min bis 6 h Rühren bei RT wurde nochmals die gleiche Menge an Vorläufer-Verbindung in DMSO hinzugefügt. Dabei sollte die Menge an DMSO 10% des Gesamtvolumens nicht überschreiten. Nach weiteren 30 min bis 6 h Rühren bei RT wurde der Ansatz über in PBS equillibierte PD 10-Säulen (Sephadex® G-25, GE Healthcare) gegeben und mit PBS-Puffer eluiert. Üblicherweise wurden, wenn nicht anders angegeben, 5 mg des entsprechenden Antikörpers in PBS-Puffer zur Reduktion und der nachfolgenden Kupplung eingesetzt. Nach Aufreinigung über die PD10 Säule wurden so jeweils Lösungen des entsprechenden ADCs in 3.5 mL PBS-Puffer erhalten. Anschließend wurde eine Aufkonzentration mittels Ultrazentrifugation durchgeführt und die Probe ggf. mit PBS-Puffer zurückverdünnt. Falls notwendig wurde zur besseren Abtrennung niedermolekularer Bestandteile die Aufkonzentration mittels Ultrafiltration nach Rückverdünnung mit PBS Puffer wiederholt. Für biologische Testungen wurden je nach Bedarf in den finalen ADC Proben ggf. durch Rückverdünnung Konzentrationen im Bereich von 0.5-15 mg/mL eingestellt.

Für die ADC Lösungen wurde die in den Ausführungsbeispielen jeweils angegebene Proteinkonzentration bestimmt. Weiterhin wurde die Beladung des Antikörpers (Drug/mAb Ratio) nach den unter B-7. beschriebenen Methoden ermittelt.

In den dargestellten Strukturformeln hat dabei AK_{2A}, AK_{2B}, AK_{2G}, AK_{2E}, AK_{2I}, AK_{2H}, AK_{2K}, die Bedeutung
AK_{2A} = Cetuximab (anti-EGFR AK)-NH§²
AK_{2B} = anti-TWEAKR AK-1- NH§²
AK_{2E} = Trastuzumab- NH§²
AK_{2I} = Nimotuzumab-NH§²
AK_{2H} = Panitumumab-NH§²
wobei
§² die Verknüpfung mit der Carbonylgruppe bedeutet,
und
NH für die Seitenketten-Aminogruppe eines Lysin-Restes des Antikörpers steht.
B-6a. Allgemeines Verfahren zur Herstellung von geschlossenen Succinimid-Cystein-Addukten:

In einer beispielhaften Ausführung wurden 10 µmol der oben beschriebenen Maleinimid-Vorläuferverbindungen wurden in 3-5 mL DMF aufgenommen und mit 2.1 mg (20 µmol) L-Cystein versetzt. Das Reaktionsgemisch wurde zwischen 2h und 24 h bei RT gerührt, anschließend im Vakuum eingeengt und dann mittels präparativer HPLC gereinigt.

### B-6aa. Allgemeines Verfahren zur Herstellung von isomeren geöffneten Bernsteinsäureamid-Cystein-Addukten:

In einer beispielhaften Ausführung wurden 68 µmol der oben beschriebenen Maleinimid-Vorläuferverbindungen in 15 mL DMF aufgenommen und mit 36 mg (136 µmol) N-{[2-(Trimethylsilyl)ethoxy]carbonyl}-L-cystein versetzt. Das Reaktionsgemisch wurde ∼20h bei RT gerührt, anschließend im Vakuum eingeengt und dann mittels präparativer HPLC gereinigt. Nach Vereinigung der entsprechenden Fraktionen und Abdampfen der Lösemittel im Vakuum wurde der Rückstand in 15 mL THF/Wasser 1:1 gelöst. Es wurden 131 µL einer 2M wässrigen Lithiumhydroxidlösung zugegeben und der Ansatz 1 h bei RT gerührt. Anschließend wurde der Ansatz mit einer 1M Salzsäure neutral gestellt, das Lösemittel im Vakuum abgedampft und der Rückstand durch präparative HPLC gereinigt. Es wurden ∼50% d. Th. der regioisomeren geschützten Intermediate als farbloser Schaum erhalten.

Im letzten Schritt wurden 0.023 mmol von diesen regiosisomeren Hydrolyseprodukten in 3 mL 2,2,2-Trifluorethanol gelöst. Es wurden 12.5 mg (0.092 mmol) Zinkchlorid zugegeben und der Ansatz 4 h bei 50°C gerührt. Anschließend wurden 27 mg (0.092 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden die hydrolysierten offenen Sulfanylbernsteinsäure-amide als Regioisomerengemisch erhalten.

### B-6b. Allgemeines Verfahren zur Herstellung von Lysin-Addukten:

In einer beispielhaften Ausführung wurden 10 µmol der oben beschriebenen Aktivester-Vorläuferverbindungen wurden in 3-5 mL DMF aufgenommen und in Gegenwart von 30 µmol *N,N-*Diisopropylethylamin mit α-Amino-geschütztem L-Lysin versetzt. Das Reaktionsgemisch wurde zwischen 2 h und 24 h bei RT gerührt, anschließend im Vakuum eingeengt und dann mittels präparativer HPLC gereinigt. Anschließend wurde die Schutzgruppe nach bekannten Methoden entfernt.

### Weitere Aufreinigung und Charakterisierung der erfindungsgemäßen Konjugate

Nach erfolgter Umsetzung wurde in einigen Fällen das Reaktionsgemisch beispielsweise durch Ultrafiltration aufkonzentriert und anschließend mittels Chromatographie, beispielsweise mittels einer Sephadex® G-25, entsalzt und gereinigt. Die Elution erfolgte beispielsweise mit Phosphat-gepufferter Salzlösung (PBS). Anschließend wurde die Lösung sterilfiltriert und eingefroren. Alternativ kann das Konjugat lyophylisiert werden.

### B-7. Bestimmung des Antikörpers, der Toxophorbeladung und des Anteils geöffneter Cystein-Addukte

Zur Protein-Identifizierung wurde neben der Molekulargewichtsbestimmung nach Deglykosilierung und/oder Denaturierung ein tryptischer Verdau durchgeführt, der nach Denaturierung, Reduktion und Derivatisierung die Identität des Proteins anhand der nachgewiesenen tryptischen Peptide bestätigt.

Von den erhaltenen Lösungen der in den Ausführungsbeispielen beschriebenen Konjugate in PBS Puffer wurde die Toxophorbeladung wie folgt bestimmt:
Die Bestimmung der Toxophor Beladung von Lysin-verknüpften ADCs erfolgte nach massenspektrometrischer Bestimmung der Molekulargewichte der einzelnen Konjugatspezies. Hierbei wurden vorab die Antikörperkonjugate mittels PNGaseF deglycosyliert, die Probe angesäuert und nach HPLC-Trennung/Entsalzung massenspektrometrisch mittels ESI-MicroTof_{Q} (Bruker Daltonik) analysiert. Alle Spektren über das Signal im TIC (Total Ion Chromatogramm) wurden addiert und das Molekulargewicht der verschiedenen Konjugatspezies auf Basis von MaxEnt Deconvolution kalkuliert. Nach Signal Integration der verschiedenen Spezies wurde dann die DAR (= Drug/Antibody Ratio) berechnet.

Die Bestimmung der Toxophorbeladung von Cystein-verknüpften Konjugaten wurde über Reversed-Phase-Chromatographie des reduzierten und denaturierten ADCs bestimmt. Zur ADC-Lösung (1mg/mL, 50µL) wurde Guanidinium-Hydrochlorid (GuHCl) (28.6 mg) und eine Lösung von DL-Dithiothreitol (DTT) (500mM, 3 µL) gegeben. Die Mischung wurde für eine Stunde bei 55 °C inkubiert und über HPLC analysiert.

Die HPLC-Analyse wurde auf einem Agilent 1260 HPLC-System mit Detektion bei 220 nm durchgeführt. Es wurde eine Polymer Laboratories PLRP-S Polymeric Reversed Phase Säule (Katalognummer PL1912-3802) (2.1 x150 mm, 8 µm particle size, 1000 Å) bei einer Flussrate von 1 mL/min mit folgendem Gradienten verwendet: 0 min, 25 %B; 3 min, 25 %B; 28 min, 50 %B. Laufmittel A bestand aus 0.05 % Trifluoressigsäure (TFA) in Wasser, Laufmittel B aus 0.05 % Trifluoressigsäure in Acetonitril.

Die detektierten Peaks wurden durch Retentionszeitvergleich mit der leichten Kette (L0) und der schweren Kette (H0) des nicht konjugierten Antikörpers zugeordnet. Peaks, die ausschließlich in der konjugierten Probe detektiert wurden, wurden der leichten Kette mit einem Toxophor (L1) und den schweren Ketten mit einem, zwei und drei Toxophoren (H1, H2, H3) zugeordnet.

Die durchschnittliche Beladung des Antikörpers mit Toxophoren wurde aus den durch Integration bestimmten Peakflächen als die zweifache Summe der Toxophor-Anzahl gewichteten Integrationsergebnisse aller Peaks geteilt durch die Summe der einfach gewichteten Integrationsergebnisse aller Peaks berechnet. In vereinzelten Fällen kann es vorkommen, dass die Toxophorbeladung aufgrund von Ko-Elutionen einiger Peaks nicht exakt möglich ist.

In den Fällen, in denen keine ausreichende HPLC-Trennung von leichter und schwerer Kette möglich war, erfolgte die Bestimmung der Toxophorbeladung von Cystein verknüpften Konjugaten mittels massenspektrometrischer Bestimmung der Molekulargewichte der einzelnen Konjugatspezies an leichter und schwerer Kette.

Zur ADC-Lösung (1mg/mL, 50µL) wurde Guanidinium-Hydrochlorid (GuHCl) (28.6 mg) und eine Lösung von DL-Dithiothreitol (DTT) (500mM, 3 µL) gegeben. Die Mischung wurde für eine Stunde bei 55 °C inkubiert und massenspektrometrisch nach online Entsaltzung mittels ESI-MicroTof_{Q} (Bruker Daltonik) analysiert.

Zur DAR-Bestimmung wurden alle Spektren über das Signal im TIC (Total Ion Chromatogramm) addiert und das Molekulargewicht der verschiedenen Konjugatspezies an leichter und schwerer Kette auf Basis von MaxEnt Deconvolution berechnet. Die durchschnittliche Beladung des Antikörpers mit Toxophoren wurde aus den durch Integration bestimmten Molekulargewichtsflächen als die zweifache Summe der Toxophor-Anzahl gewichteten Integrationsergebnisse aller Peaks geteilt durch die Summe der einfach gewichteten Integrationsergebnisse aller Peaks berechnet.

Zur Bestimmung des Anteils des geöffneten Cystein-Addukts wurde das Molekulargewichtsflächenverhältnis vom geschlossenen und offenen Cystein-Addukt (Molekulargewichtsdelta 18Dalton) aller einfach konjugierten leichten und schweren Kettenvarianten bestimmt. Der Mittelwert über alle Varianten ergab den Anteil des geöffneten Cystein-Addukts.

### B-8. Überprüfung der Antigen-Bindung des ADCs

Die Bindefähigkeit des Binders an das Zielmolekül wurde nach erfolgter Kopplung überprüft. Dazu sind dem Fachmann vielfältige Methoden bekannt, beispielsweise kann die Affinität des Konjugats mittels ELISA-Technologie oder Oberflächenplasmonresonanzanalyse (BIAcore™ Messungen) überprüft werden. Die Konjugatkonzentration kann der Fachmann mit gängigen Methoden messen, beispielsweise für Antikörper-Konjugate mittels Proteinbestimmung, (siehe auch Doronina et al.; Nature Biotechnol. 2003; 21:778-784 und Polson et al., Blood 2007; 1102:616-623).

### Ausführungsbeispiele ADCs

### Beispiel 82A

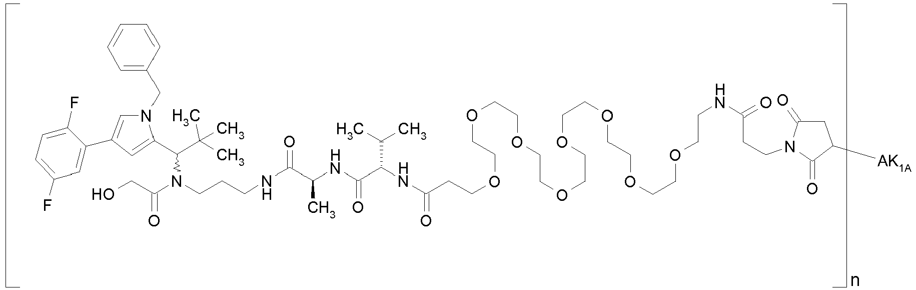

Eingesetzt wurden hier zur Kupplung mit Intermediat F82 5.0 mg Cetuximab in PBS (c = 5.90 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.59 mg/mL
Drug/mAb Ratio: 2.3

### Beispiel 82B

Eingesetzt wurden hier zur Kupplung mit Intermediat F82 5.0 mg anti-TWEAKR AK-1 Antikörper in PBS (c = 10.10 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.59 mg/mL
Drug/mAb Ratio: 1.8

### Beispiel 82E

Eingesetzt wurden hier zur Kupplung mit Intermediat F82 5.0 mg Trastuzumab Antikörper in PBS (c = 11.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 2.15 mg/mL
Drug/mAb Ratio: 2.7

### Beispiel 83A

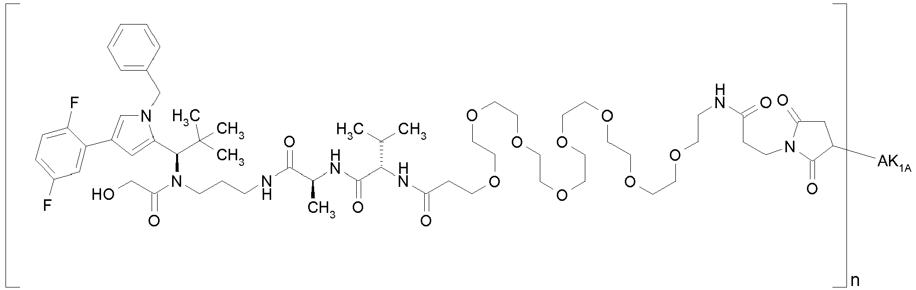

Eingesetzt wurden hier zur Kupplung mit Intermediat F83 5.0 mg Cetuximab in PBS (c = 5.90 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.77 mg/mL
Drug/mAb Ratio: 2.0

### Beispiel 83B

Eingesetzt wurden hier zur Kupplung mit Intermediat F83 5.0 mg anti-TWEAKR AK-1 Antikörper in PBS (c = 12.87 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.29 mg/mL
Drug/mAb Ratio: 1.3

### Beispiel 83E

Eingesetzt wurden hier zur Kupplung mit Intermediat F83 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.91 mg/mL
Drug/mAb Ratio: 2.1

### Beispiel 83H

Eingesetzt wurden hier zur Kupplung mit Intermediat F83 5.0 mg Panitumumab in PBS (c = 10 mg/mL). Die Reduktionszeit mit TCEP wurde auf 4 h erhöht und die Rührzeit bei der ADC Kupplung auf 20 h erhöht. Anschließend wurde der Ansatz nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.57 mg/mL
Drug/mAb Ratio: 0.9

### Beispiel 84A

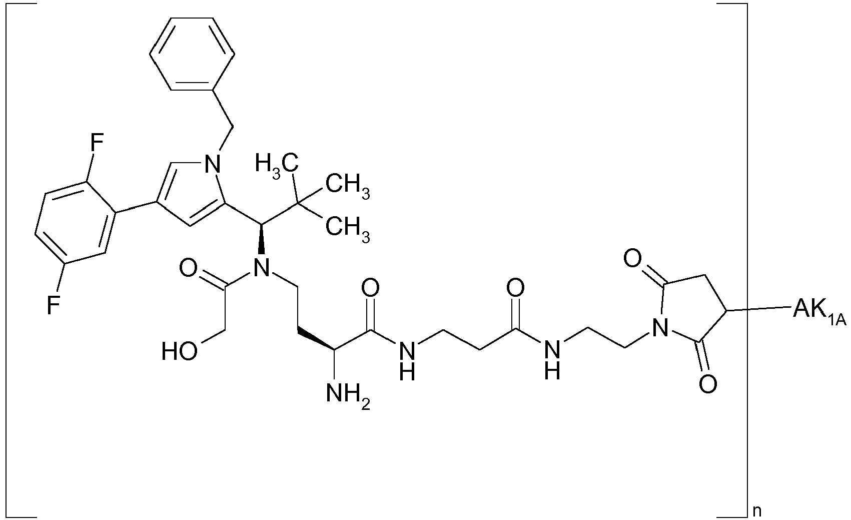

Eingesetzt wurden hier zur Kupplung mit Intermediat F84 5 mg Cetuximab in PBS (c=11.02 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.94 mg/mL
Drug/mAb Ratio: 2.9

### Beispiel 84B

Eingesetzt wurden hier zur Kupplung mit Intermediat F84 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.77 mg/mL
Drug/mAb Ratio: 3.0

### Beispiel 85A

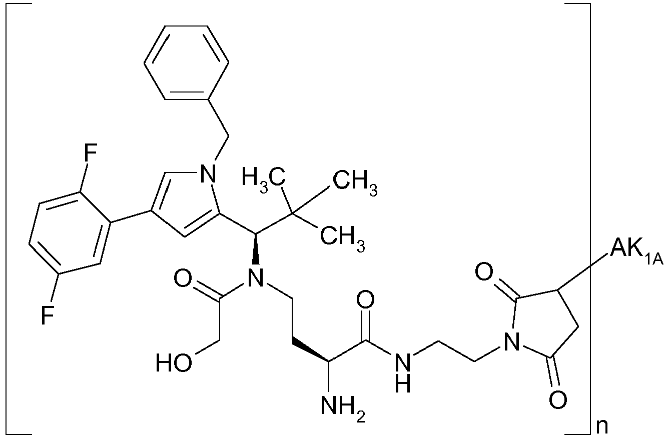

Eingesetzt wurden hier zur Kupplung mit Intermediat F85 5 mg Cetuximab in PBS (c=11.02 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.13 mg/mL
Drug/mAb Ratio: 3.4

### Beispiel 85B

Eingesetzt wurden hier zur Kupplung mit Intermediat F85 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.63 mg/mL
Drug/mAb Ratio: 3.2

### Beispiel 86A

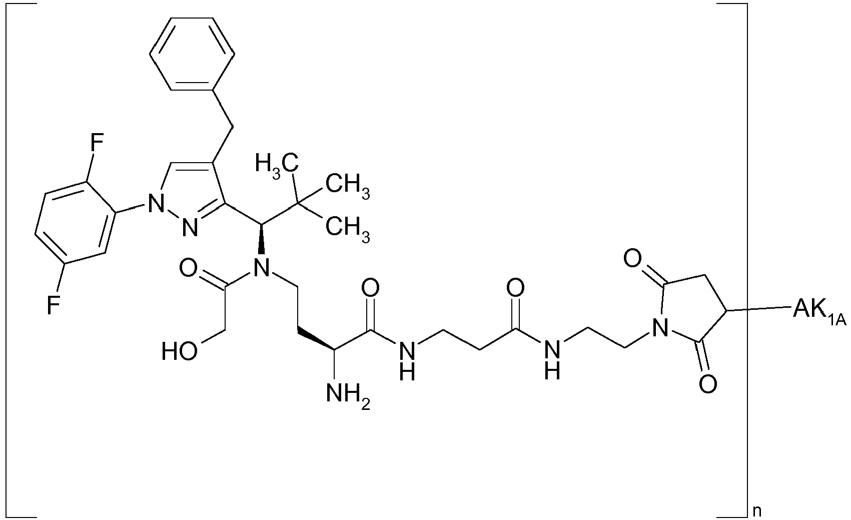

Eingesetzt wurden hier zur Kupplung mit Intermediat F86 5 mg Cetuximab in PBS (c=11.59 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.96 mg/mL
Drug/mAb Ratio: 3.2

### Beispiel 86B

Eingesetzt wurden hier zur Kupplung mit Intermediat F86 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.55 mg/mL
Drug/mAb Ratio: 2.8

### Beispiel 87A

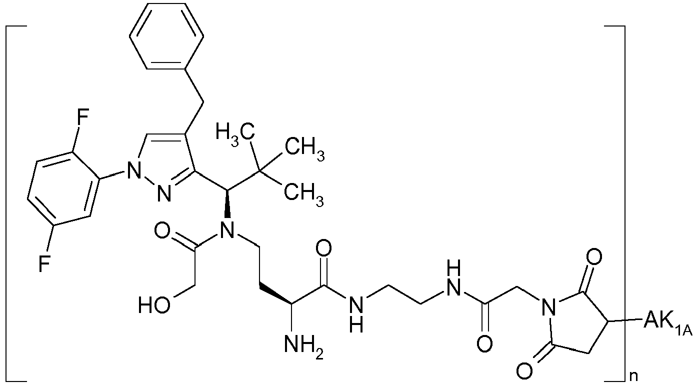

Eingesetzt wurden hier zur Kupplung mit Intermediat F87 5 mg Cetuximab in PBS (c=8.95 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.08 mg/mL
Drug/mAb Ratio: 3.5

### Beispiel 87B

Eingesetzt wurden hier zur Kupplung mit Intermediat F87 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.93 mg/mL
Drug/mAb Ratio: 2.2

### Beispiel 88A

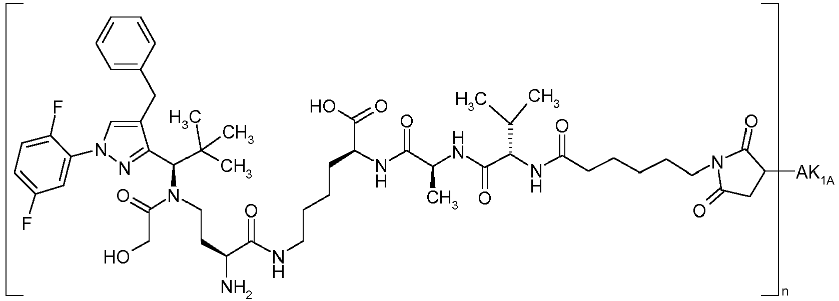

Eingesetzt wurden hier zur Kupplung mit Intermediat F88 5 mg Cetuximab in PBS (c=11.02 mg/mL) und der Ansatz wurde nach der Sephadex-Reinigung durch Ultrazentrifugation aufkonzentriert und rückverdünnt mit PBS.
Protein Konzentration: 2.03 mg/mL
Drug/mAb Ratio: 3.1

### Beispiel 88B

Eingesetzt wurden hier zur Kupplung mit Intermediat F88 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS.
Protein Konzentration: 1.9 mg/mL
Drug/mAb Ratio: 3.3

### Beispiel 89A

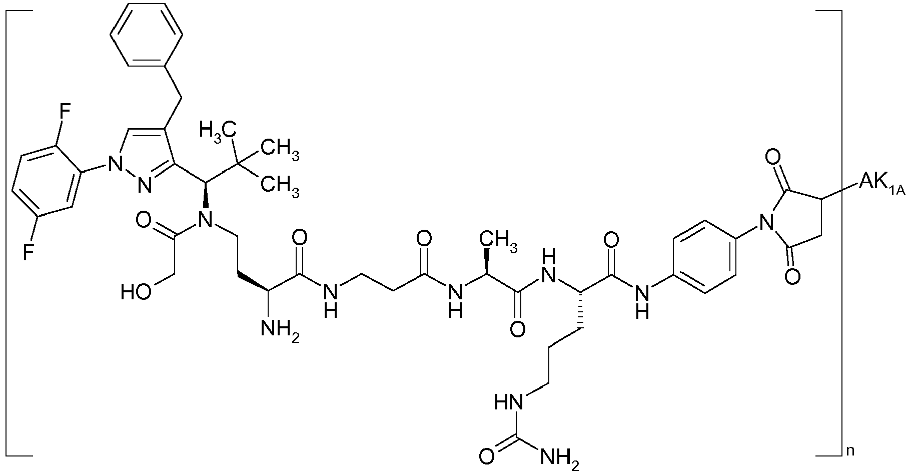

Eingesetzt wurden hier zur Kupplung mit Intermediat F89 5 mg Cetuximab in PBS (c=11.02 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.2 mg/mL
Drug/mAb Ratio: 3.3

### Beispiel 89B

Eingesetzt wurden hier zur Kupplung mit Intermediat F89 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.03 mg/mL
Drug/mAb Ratio: 3.4

### Beispiel 90A

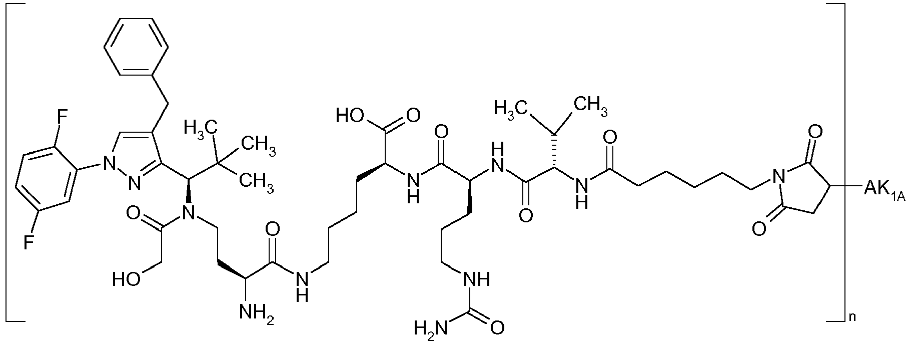

Eingesetzt wurden hier zur Kupplung mit Intermediat F90 5 mg Cetuximab in PBS (c=13.33 mg/mL) und der Ansatz wurde nach der Sephadex-Reinigung durch Ultrazentrifugation aufkonzentriert und rückverdünnt.
Protein Konzentration: 2.19 mg/mL
Drug/mAb Ratio: 3.0

### Beispiel 90B

Eingesetzt wurden hier zur Kupplung mit Intermediat F90 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS.
Protein Konzentration: 1.97 mg/mL
Drug/mAb Ratio: 2.9

### Beispiel 91A

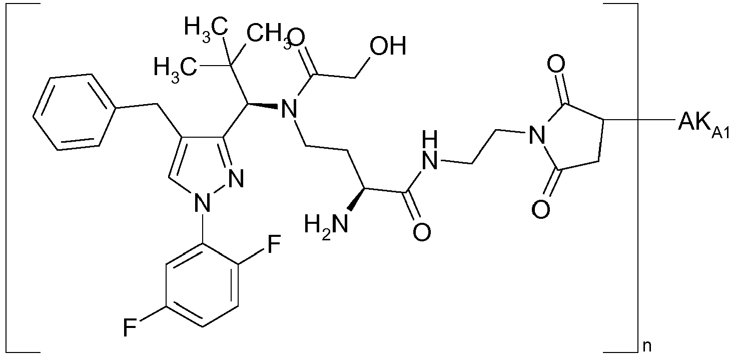

Eingesetzt wurden hier zur Kupplung mit Intermediat F91 80 mg Cetuximab in PBS (c=5.9 mg/ml) und der Ansatz wurde nach der Sephadex-Reinigung durch Ultrazentrifugation aufkonzentriert, rückverdünnt mit PBS und erneut aufkonzentriert. Das ADC kann auch teilweise in Form der hydrolysierten offenkettigen Bernsteinsäureamide mit dem Antikörper verknüpft vorliegen.
Protein Konzentration: 12.75 mg/ml
Drug/mAb Ratio: 3.7

### Beispiel 91B

Eingesetzt wurden hier zur Kupplung mit Intermediat F91 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 5.71 mg/mL
Drug/mAb Ratio: 4.0

### Beispiel 92

### S-(1-{2-[(N-{(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}-beta-alanyl)amino]ethyl}-2,5-dioxopyrrolidin-3-yl)-L-cystein-trifluoressigsäure(l: 1)

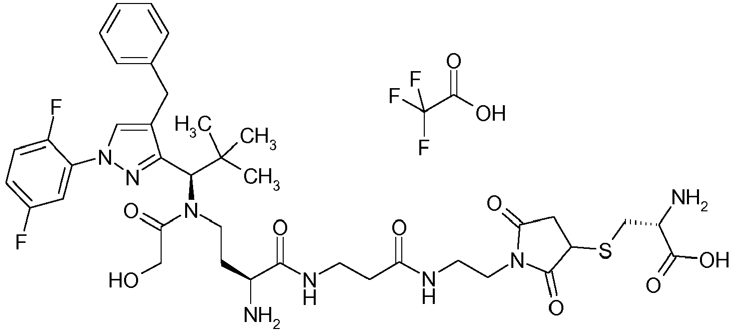

3 mg (4 µmol) von Intermediat F86 wurden in 3 ml DCM/Wasser 10:1 aufgenommen und mit 1.3 mg (11 µmol) L-Cystein versetzt. Das Reaktionsgemisch wurde 10 min bei RT gerührt, anschließend im Vakuum eingeengt und dann mittels präparativer HPLC gereinigt.
LC-MS (Methode 1): Rₜ = 0.77 min; MS (EIpos): m/z = 829 [M+H]⁺.

### Beispiel 96

### N-(3-Aminopropyl)-N-{(1R)-1-[4-benzyl-1-(2,5-difluorphenyl)-1H-pyrazol-3-yl]-2,2-dimethylpropyl}-2-hydroxyacetamid

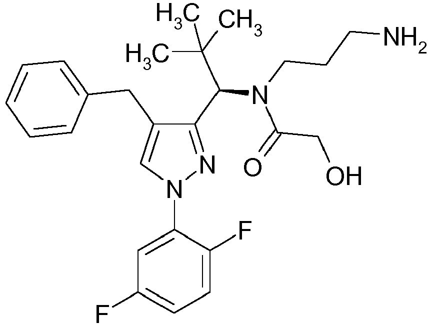

101 mg (0.16 mmol) 2-({(1R)-1-[4-Benzyl-1-(2,5-difluorphenyl)-1H-pyrazol-3-yl]-2,2-dimethylpropyl}[3-(1,3-dioxo-1,3-dihydro-2H-isoindol-2-yl)propyl]amino)-2-oxoethylacetat wurden in 2 ml absolutem Ethanol vorgelegt und mit 244 mg (3.14 mmol, 225 µl) einer 40%-igen Lösung von Methylamin in Wasser versetzt. Es wurde 1 h bei 50°C gerührt und anschließend erneut 244 mg (3.14 mmol, 225 µl) einer 40%-igen Lösung von Methylamin in Wasser zugesetzt und nach insgesamt 3.5 h direkt mittels präparativer HPLC (Eluent: ACN/Wasser + 1.0% NEt₃, Gradient) gereinigt. Es wurden 52 mg (70% d. Th.) der Zielverbindung erhalten.
LC-MS (Methode 3): Rₜ = 2.56 min; MS (EIpos): m/z = 471 [M+H]⁺.

### Beispiel 98

### N-(3-Aminopropyl)-N-{(1R)-1-[1-benzyl-4-(2,5-difluorphenyl)-1H-pyrrol-2-yl]-2,2-dimethylpropyl}-2-hydroxyacetamid

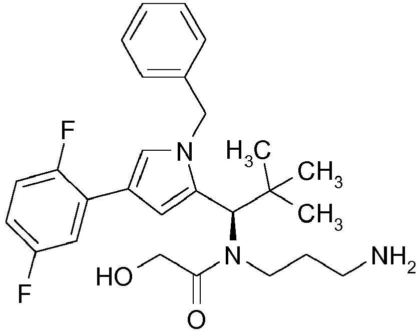

150.0 mg (0.42 mmol) (1R)-1-[1-Benzyl-4-(2,5-difluorphenyl)-1H-pyrrol-2-yl]-2,2-dimethylpropan-1-amin (Intermediat C52) wurden in 2.0 mL Dichlormethan vorgelegt und mit 29.2 mg (0.49 mmol) HOAc und 125.6 mg (0.59 mmol) Natriumtriacetoxyborhydrid versetzt und 5 min bei RT gerührt. Es wurden 98.9 mg (0.49 mmol) 3-(1,3-Dioxo-1,3-dihydro-2H-isoindol-2-yl)propanal zugegeben. Die Reaktionsmischung wurde über Nacht bei RT gerührt. Die Reaktionsmischung wurde mit Ethylacetat verdünnt und die organische Phase zweimal mit ges. Natriumcarbonat-Lösung und einmal mit ges. NaCl-Lösung gewaschen. Nach der Trocknung über Magnesiumsulfat wurde das Lösemittel im Vakuum verdampft und der Rückstand mittels Kieselgel (Laufmittel: Dichlormethan/Methanol 100:1) gereinigt. Die Lösemittel wurden im Vakuum verdampft und der Rückstand im Hochvakuum getrocknet. Man erhielt 188.6 mg (74 %) der Verbindung 2-[3-({(1R)-1-[1-Benzyl-4-(2,5-difluorphenyl)-1H-pyrrol-2-yl]-2,2-dimethylpropyl}amino)propyl]-1H-isoindol-1,3(2H)-dion.
LC-MS (Methode 1): Rₜ = 1.00 min; MS (ESIpos): m/z = 541 [M+H]⁺.

171.2 mg (0.32 mmol) 2-[3-({(1R)-1-[1-Benzyl-4-(2,5-difluorphenyl)-1H-pyrrol-2-yl]-2,2-dimethylpropyl}amino)propyl]-1H-isoindol-1,3(2H)-dion wurden in 5.0 mL Dichlormethan vorgelegt und mit 73.6 mg (0.73 mmol) Triethylamin versetzt. Bei 0 °C wurden 94.9 mg (0.70 mmol) Acetoxyessigsäurechlorid zugegeben und die Reaktionsmischung wurde über Nacht bei RT gerührt. Die Reaktionsmischung wurde mit Ethylacetat verdünnt und die organische Phase zweimal mit ges. Natriumhydrogencarbonat-Lösung und einmal mit ges. NaCl-Lösung gewaschen. Nach der Trocknung über Magnesiumsulfat wurde das Lösemittel im Vakuum verdampft und der Rückstand wurde mittels Biotage Isolera gereinigt (Kieselgel, Säule 10 g SNAP, Fluss 12 mL/min, Ethylacetat/Cyclohexan 1:3) gereinigt. Die Lösemittel wurden im Vakuum verdampft und der Rückstand im Hochvakuum getrocknet. Man erhielt 159.0 mg (77 %) der Verbindung 2-({(1R)-1-[1-Benzyl-4-(2,5-difluorphenyl)-1H-pyrrol-2-yl]-2,2-dimethylpropyl}[3-(1,3-dioxo-1,3-dihydro-2H-isoindol-2-yl)propyl]amino)-2-oxoethylacetat.
LC-MS (Methode 1): Rₜ = 1.35 min; MS (ESIpos): m/z = 642 [M+H]⁺.

147.2 mg (0.23 mmol) 2-({(1R)-1-[1-Benzyl-4-(2,5-difluorphenyl)-1H-pyrrol-2-yl]-2,2-dimethylpropyl}[3-(1,3-dioxo-1,3-dihydro-2H-isoindol-2-yl)propyl]amino)-2-oxoethylacetat wurden in 4.0 mL Ethanol vorgelegt und mit 356.2 mg (4.59 mmol) Methanamin (40 % in Wasser) versetzt. Die Reaktionsmischung wurde über Nacht bei 50 °C gerührt. Das Lösemittel wurde im Vakuum verdampft und der Rückstand dreimal mit Toluol kodestilliert. Der Rückstand wurde mittels Kieselgel (Laufmittel: Dichlormethan/Methanol 10:1) gereinigt. Die Lösemittel wurden im Vakuum verdampft und der Rückstand im Hochvakuum getrocknet. Man erhielt 67.4 mg (63 %) der Titelverbindung.
LC-MS (Methode 1): Rₜ = 0.91 min; MS (ESIpos): m/z = 470 [M+H]⁺.

### Beispiel 99

### Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-methylbutanamid(1:1)

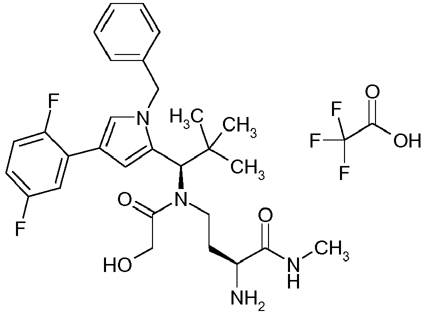

Zunächst wurde Intermediat C52 mit Benzyl-(2S)-2-{[(benzyloxy)carbonyl]amino}-4-oxobutanoat in Analogie zu Intermediat C2 reduktiv alkyliert. Anschließend wurde die sekundäre Aminogruppe mit 2-Chlor-2-oxoethylacetat wie in Intermediat C27 beschrieben acyliert.

190 mg (0.244 mmol) von dieser Zwischenstufe wurden in 7.5 ml Ethanol aufgenommen und mit 0.35 mL einer 40%-igen Methanaminlösung in Wasser versetzt. Der Ansatz wurde 3 h bei 50°C gerührt und dann wurde nochmals die gleiche Menge Methanamin zugegeben. Nach weiteren 5 h Rühren bei 50°C wurde der Ansatz eingeengt und der Rückstand durch präparative HPLC gereinigt. Es wurden 78 mg (48% d.Th.) dieser Zwischenstufe erhaltenen.
LC-MS (Methode 1): Rₜ = 1.32 min; MS (EIpos): m/z = 661 [M+H]⁺.

78 mg (0.118 mmol) dieser Zwischenstufe wurden in 8 mL Ethanol gelöst und nach Zugabe von 15 mg 10%-igem Palladium auf Aktivkohle 3 h lang bei RT unter Wasserstoff-Normaldruck hydriert. Der Katalysator wurde danach abfiltriert und das Lösungsmittel im Vakuum entfernt und das Produkt durch präparative HPLC gereinigt. Nach Lyophilisation aus Acetonitril/Wasser wurden 33 mg (44% d.Th.) der Titelverbindung erhalten.
LC-MS (Methode 1): Rₜ = 0.88 min; MS (ESIpos): m/z = 527 (M+H)⁺.
¹H-NMR (500 MHz, DMSO-d₆): δ = 8.1 (m, 1H), 8.0 (m, 3H), 7.9 (m, 1H), 7.65 (m, 1H), 7.5 (s, 1H), 7.15-7.35 (m, 5H) 7.0 (m, 1H), 6.85 (m, 1H), 5.6 (s, 1H), 4.9 und 5.2 (2d, 2H), 4.02 und 4.22 (2d, 2H), 3.2-3.5 (m, 6H), 0.7 und 1.46 (2m, 2H), 0.8 (s, 9H).

### Beispiel 102

### N-(3-Aminopropyl)-N-{(1S)-1-[1-benzyl-4-(2,5-difluorphenyl)-1H-pyrrol-2-yl]-2,2-dimethylpropyl}-2-hydroxyacetamid

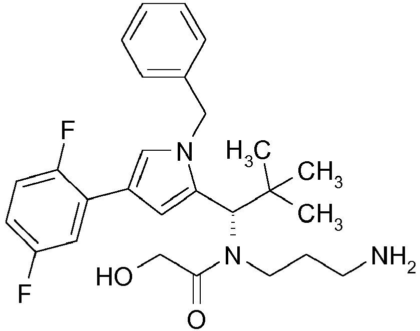

Die Synthese erfolgte in Analogie zur Synthese der Verbindung Beispiel 98 unter Verwendung der entsprechenden S-Isomer Intermediate.
LC-MS (Methode 1): Rₜ = 0.92 min; MS (ESIpos): m/z = 470 [M+H]⁺.

### Beispiel 104A

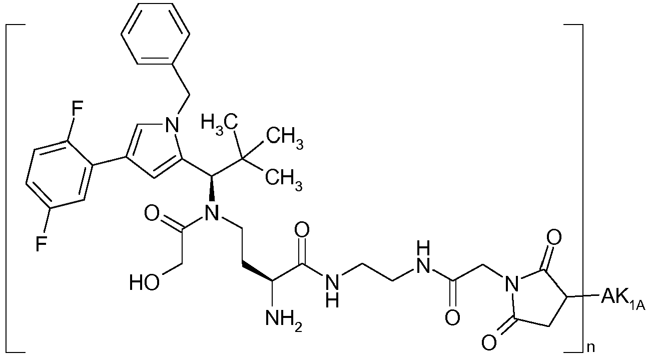

Eingesetzt wurden hier zur Kupplung mit Intermediat F104 5 mg Cetuximab in PBS (c=15.33 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.95 mg/mL
Drug/mAb Ratio: 3.7

### Beispiel 104B

Eingesetzt wurden hier zur Kupplung mit Intermediat F104 35 mg anti-TWEAKR AK-1 in PBS (c=12.9 mg/mL) und der Ansatz wurde nach der Sephadex-Reinigung durch Ultrazentrifugation aufkonzentriert, rückverdünnt mit PBS und erneut aufkonzentriert. Das ADC kann auch teilweise in Form der hydrolysierten offenkettigen Bernsteinsäureamide mit dem Antikörper verknüpft vorliegen.
Protein Konzentration: 10.93 mg/mL
Drug/mAb Ratio: 3.2

### Beispiel 104E

Eingesetzt wurden hier zur Kupplung mit Intermediat F104 5.0 mg Trastuzumab Antikörper in PBS (c = 8.23 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.11 mg/mL
Drug/mAb Ratio: 2.8

### Beispiel 104I

Eingesetzt wurden hier zur Kupplung mit Intermediat F104 5.0 mg Nimotuzumab in PBS (c = 13.1 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.89 mg/mL
Drug/mAb Ratio: 3.5

### Beispiel 104H

Eingesetzt wurden hier zur Kupplung mit Intermediat F104 5.0 mg Panitumumab in PBS (c = 12 mg/mL). Die Reduktionszeit mit TCEP wurde auf 4 h erhöht und die Rührzeit bei der ADC Kupplung auf 20 h erhöht. Anschließend wurde der Ansatz nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.79 mg/mL
Drug/mAb Ratio: 2.2

### Beispiel 106A

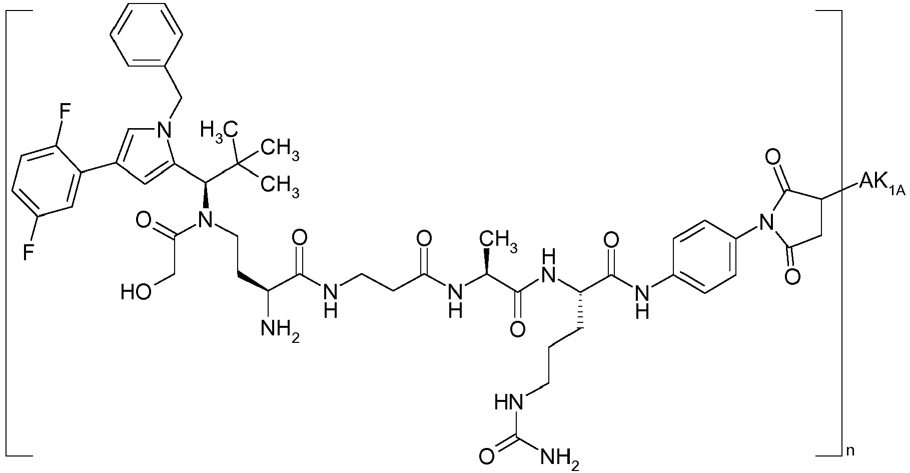

Eingesetzt wurden hier zur Kupplung mit Intermediat F106 5 mg Cetuximab in PBS (c=15.33 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.91 mg/mL
Drug/mAb Ratio: 3.3

### Beispiel 106B

Eingesetzt wurden hier zur Kupplung mit Intermediat F106 5 mg anti-TWEAKR AK-1 in PBS (c=12.87 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.76 mg/mL
Drug/mAb Ratio: 3.0

### Beispiel 106E

Eingesetzt wurden hier zur Kupplung mit Intermediat F106 5 mg Trastuzumab in PBS (c=8.23 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.5 mg/mL
Drug/mAb Ratio: 2.4

### Beispiel 107A

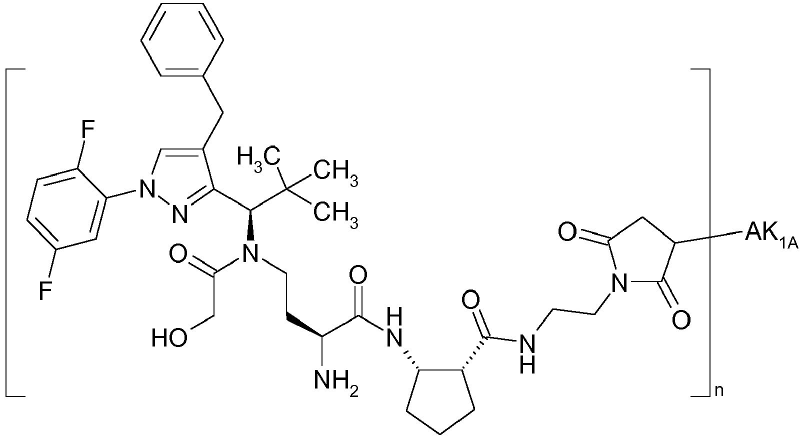

Eingesetzt wurden hier zur Kupplung mit Intermediat F107 5 mg Cetuximab in PBS (c=12.3 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.16 mg/mL
Drug/mAb Ratio: 3.3

### Beispiel 107B

Eingesetzt wurden hier zur Kupplung mit Intermediat F107 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.9 mg/mL
Drug/mAb Ratio: 3.1

### Beispiel 107E

Eingesetzt wurden hier zur Kupplung mit Intermediat F107 5 mg Trastuzumab in PBS (c=8.23 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.48 mg/mL
Drug/mAb Ratio: 2.9

### Beispiel 109A

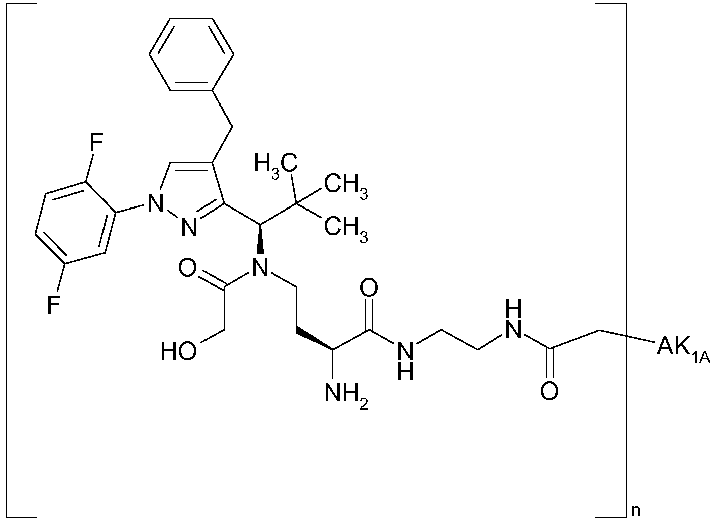

Eingesetzt wurden hier zur Kupplung mit Intermediat F109 5 mg Cetuximab in PBS (c=12.33 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 2.1 mg/mL
Drug/mAb Ratio: 3.1

### Beispiel 109B

Eingesetzt wurden hier zur Kupplung mit Intermediat F109 5 mg anti-TWEAKR AK-1 in PBS (c=34.4 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 1.63 mg/mL
Drug/mAb Ratio: 2.7

### Beispiel 112A

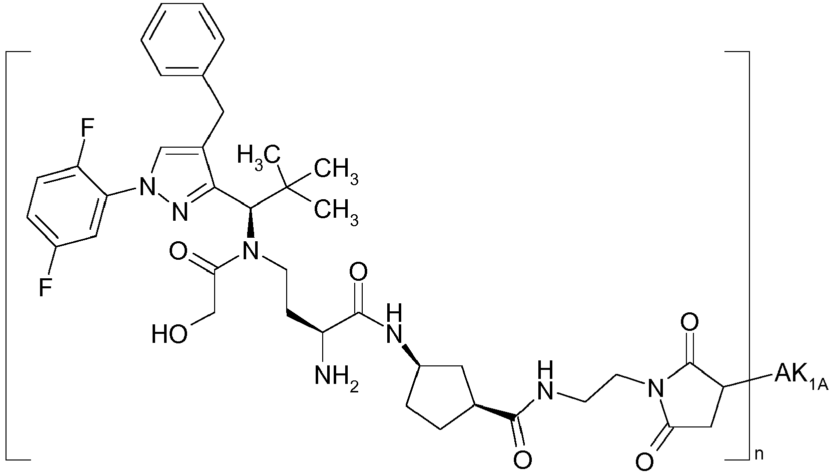

Eingesetzt wurden hier zur Kupplung mit Intermediat F112 5 mg Cetuximab in PBS (c=12.33 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.2 mg/mL
Drug/mAb Ratio: 3

### Beispiel 112B

Eingesetzt wurden hier zur Kupplung mit Intermediat F112 5 mg anti-TWEAKR AK-1 in PBS (c=34.4 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.39 mg/mL
Drug/mAb Ratio: 2.1

### Beispiel 113A

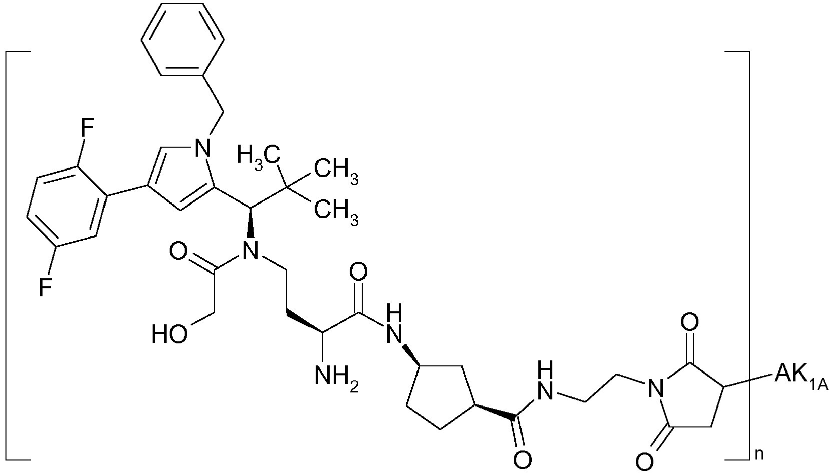

Eingesetzt wurden hier zur Kupplung mit Intermediat F113 5 mg Cetuximab in PBS (c=12.33 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.95 mg/mL
Drug/mAb Ratio: 2.2

### Beispiel 113B

Eingesetzt wurden hier zur Kupplung mit Intermediat F113 5 mg anti-TWEAKR AK-1 in PBS (c=34.4 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.8 mg/mL
Drug/mAb Ratio: 2.2

### Beispiel 115A

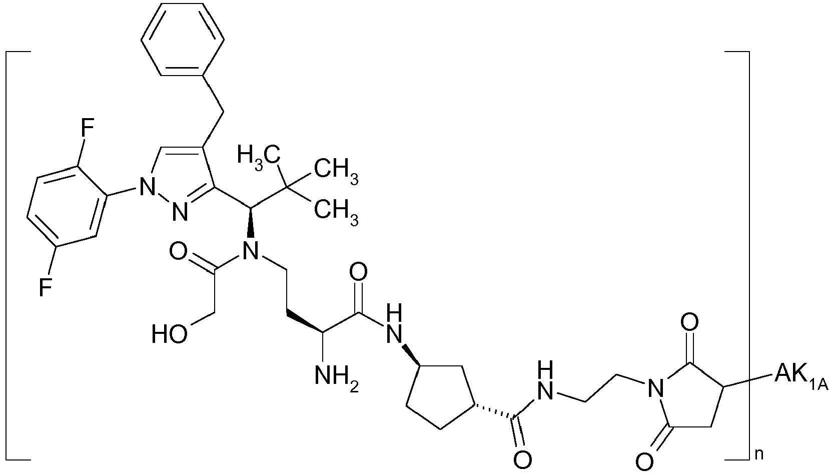

Eingesetzt wurden hier zur Kupplung mit Intermediat F115 5 mg Cetuximab in PBS (c=12.33 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.18 mg/mL
Drug/mAb Ratio: 3.7

### Beispiel 115B

Eingesetzt wurden hier zur Kupplung mit Intermediat F115 5 mg anti-TWEAKR AK-1 in PBS (c=34.4 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.0 mg/mL
Drug/mAb Ratio: 3.0

### Beispiel 116A

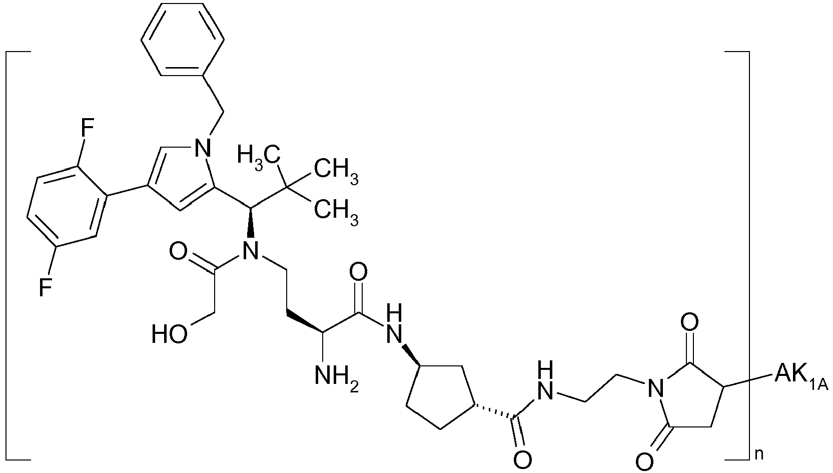

Eingesetzt wurden hier zur Kupplung mit Intermediat F116 5 mg Cetuximab in PBS (c=12.33 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.03 mg/mL
Drug/mAb Ratio: 4.4

### Beispiel 116B

Eingesetzt wurden hier zur Kupplung mit Intermediat F116 5 mg anti-TWEAKR AK-1 in PBS (c=34.4 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.96 mg/mL
Drug/mAb Ratio: 2.9

### Beispiel 117A

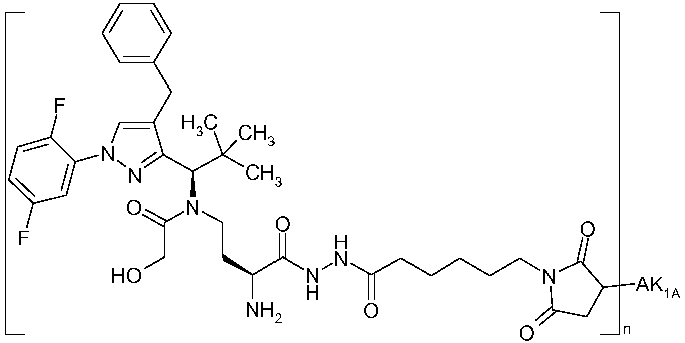

Eingesetzt wurden hier zur Kupplung mit Intermediat F117 5 mg Cetuximab in PBS (c=12.33 mg/mL) und der Ansatz wurde nach der Sephadex-Reinigung durch Ultrazentrifugation aufkonzentriert und rückverdünnt mit PBS.
Protein Konzentration: 2.02 mg/mL
Drug/mAb Ratio: 2.7

### Beispiel 117B

Eingesetzt wurden hier zur Kupplung mit Intermediat F117 5 mg anti-TWEAKR AK-1 in PBS (c=34.4 mg/mL) und der Ansatz wurde nach der Sephadex-Reinigung durch Ultrazentrifugation aufkonzentriert und rückverdünnt mit PBS.
Protein Konzentration: 1.77 mg/mL
Drug/mAb Ratio: 2.7

### Beispiel 118A

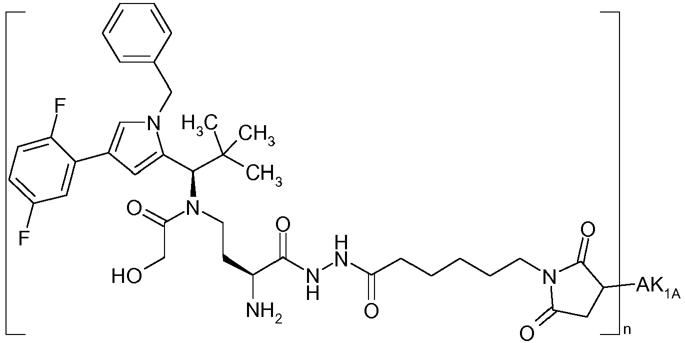

Eingesetzt wurden hier zur Kupplung mit Intermediat F118 5 mg Cetuximab in PBS (c=26.84 mg/mL) und der Ansatz wurde nach der Sephadex-Reinigung durch Ultrazentrifugation aufkonzentriert und rückverdünnt mit PBS.
Protein Konzentration: 2.38 mg/mL
Drug/mAb Ratio: 3.6

### Beispiel 118B

Eingesetzt wurden hier zur Kupplung mit Intermediat F118 5 mg anti-TWEAKR AK-1 in PBS (c=12.87 mg/mL) und der Ansatz wurde nach der Sephadex-Reinigung durch Ultrazentrifugation aufkonzentriert und rückverdünnt mit PBS.
Protein Konzentration: 1.14 mg/mL
Drug/mAb Ratio: 2.9

### Beispiel 118E

Eingesetzt wurden hier zur Kupplung mit Intermediat F118 5 mg Trastuzumab in PBS (c=8.23 mg/mL) und der Ansatz wurde nach der Sephadex-Reinigung durch Ultrazentrifugation aufkonzentriert und rückverdünnt mit PBS.
Protein Konzentration: 2.27 mg/mL
Drug/mAb Ratio: 3

### Beispiel 119A

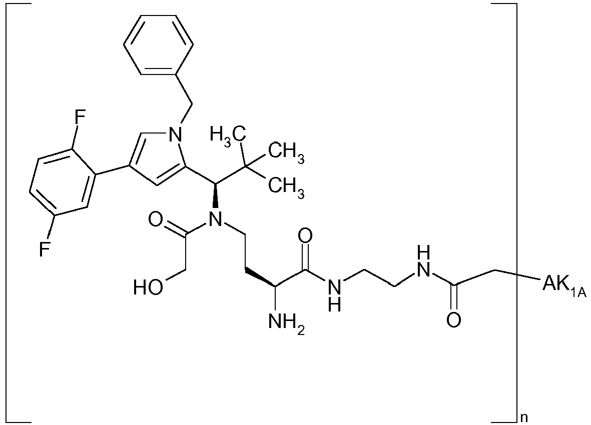

Eingesetzt wurden hier zur Kupplung mit Intermediat F119 5 mg Cetuximab in PBS (c=26.8 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 2.14 mg/mL
Drug/mAb Ratio: 3.9

### Beispiel 119B

Eingesetzt wurden hier zur Kupplung mit Intermediat F119 5 mg anti-TWEAKR AK-1 in PBS (c=12.87 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 0.91 mg/mL
Drug/mAb Ratio: 4.1

### Beispiel 119E

Eingesetzt wurden hier zur Kupplung mit Intermediat F119 5 mg Trastuzumab in PBS (c=13.5 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 1.69 mg/mL
Drug/mAb Ratio: 4.4

### Beispiel 121A

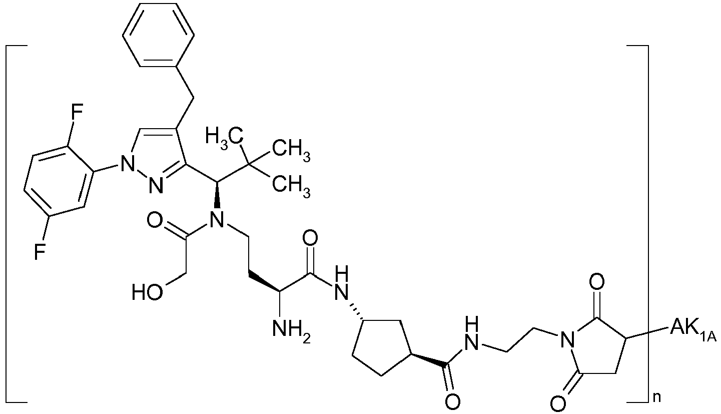

Eingesetzt wurden hier zur Kupplung mit Intermediat F121 5 mg Cetuximab in PBS (c=26.84 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.1 mg/mL
Drug/mAb Ratio: 3.2

### Beispiel 121B

Eingesetzt wurden hier zur Kupplung mit Intermediat F121 5 mg anti-TWEAKR AK-1 in PBS (c=12.87 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.88 mg/mL
Drug/mAb Ratio: 3.4

### Beispiel 122A

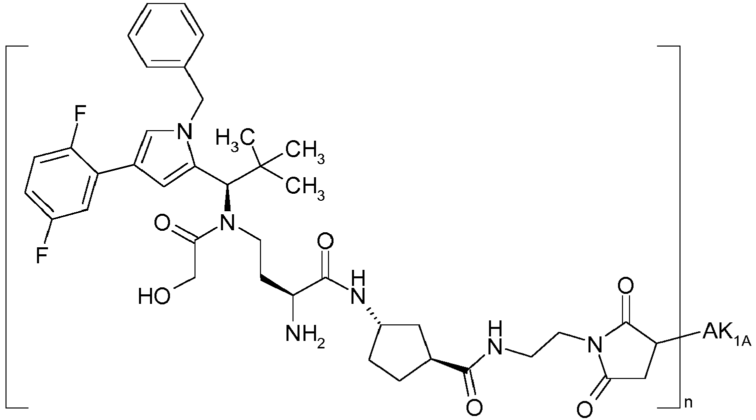

Eingesetzt wurden hier zur Kupplung mit Intermediat F122 5 mg Cetuximab in PBS (c=26.84 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.78 mg/mL
Drug/mAb Ratio: 3.2

### Beispiel 122B

Eingesetzt wurden hier zur Kupplung mit Intermediat F122 5 mg anti-TWEAKR AK-1 in PBS (c=12.87 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.64 mg/mL
Drug/mAb Ratio: 3.4

### Beispiel 124A

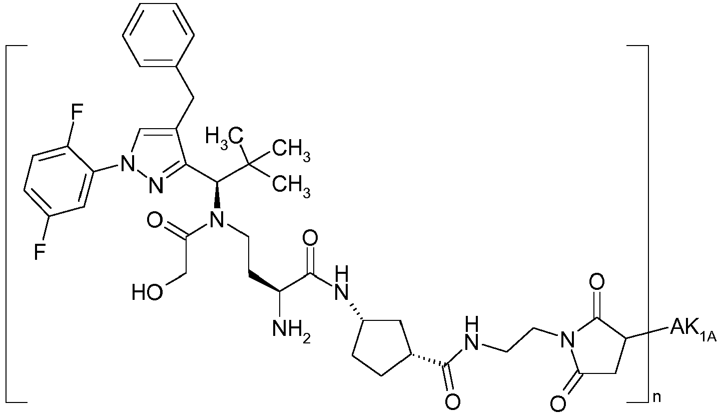

Eingesetzt wurden hier zur Kupplung mit Intermediat F124 5 mg Cetuximab in PBS (c=26.84 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.93 mg/mL
Drug/mAb Ratio: 2.8

### Beispiel 124B

Eingesetzt wurden hier zur Kupplung mit Intermediat F124 5 mg anti-TWEAKR AK-1 in PBS (c=12.87 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.84 mg/mL
Drug/mAb Ratio: 3.0

### Beispiel 125A

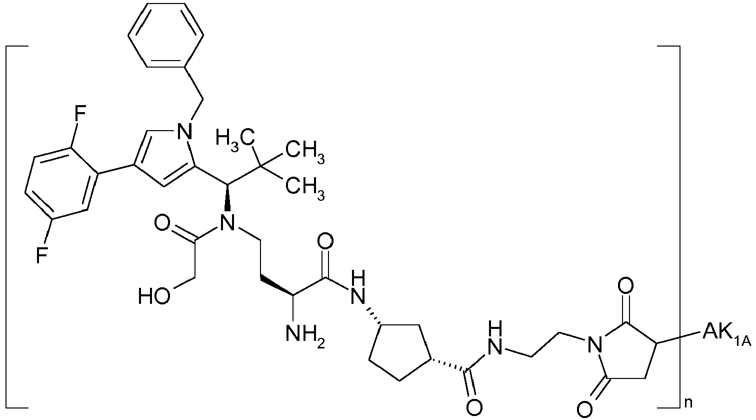

Eingesetzt wurden hier zur Kupplung mit Intermediat F125 5 mg Cetuximab in PBS (c=26.84 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.14 mg/mL
Drug/mAb Ratio: 2.9

### Beispiel 125B

Eingesetzt wurden hier zur Kupplung mit Intermediat F125 5 mg anti-TWEAKR AK-1 in PBS (c=12.87 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.83 mg/mL
Drug/mAb Ratio: 2.3

### Beispiel 126A

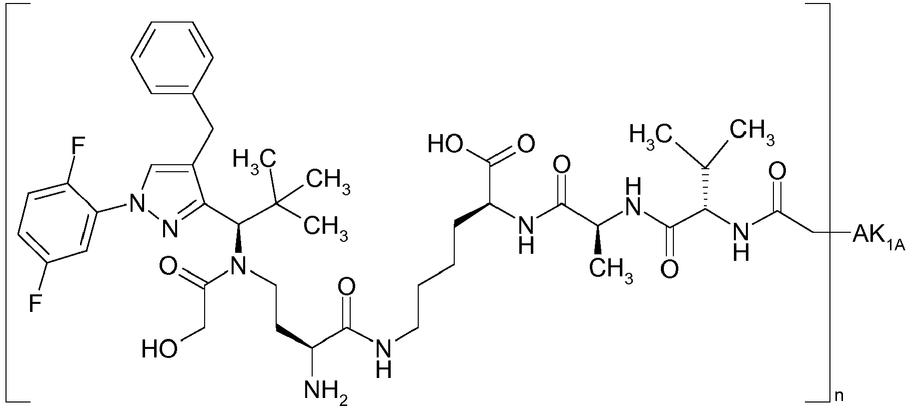

Eingesetzt wurden hier zur Kupplung mit Intermediat F126 5 mg Cetuximab in PBS (c=26.84 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 1.89 mg/mL
Drug/mAb Ratio: 2.5

### Beispiel 126B

Eingesetzt wurden hier zur Kupplung mit Intermediat F126 5 mg anti-TWEAKR AK-1 in PBS (c=12.9 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 1.62 mg/mL
Drug/mAb Ratio: 2.8

### Beispiel 126E

Eingesetzt wurden hier zur Kupplung mit Intermediat F126 5 mg Trastuzumab in PBS (c=8.23 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 1.93 mg/mL
Drug/mAb Ratio: 1.9

### Beispiel 127A

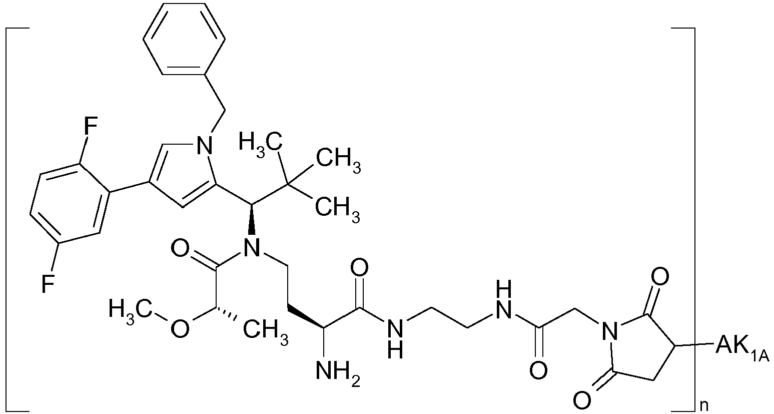

Eingesetzt wurden hier zur Kupplung mit Intermediat F127 5 mg Cetuximab in PBS (c=26.84 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.54 mg/mL
Drug/mAb Ratio: 3.3

### Beispiel 127B

Eingesetzt wurden hier zur Kupplung mit Intermediat F127 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.62 mg/mL
Drug/mAb Ratio: 3.3

### Beispiel 127E

Eingesetzt wurden hier zur Kupplung mit Intermediat F127 5.0 mg Trastuzumab Antikörper in PBS (c = 13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.07 mg/mL
Drug/mAb Ratio: 3.6

### Beispiel 129A

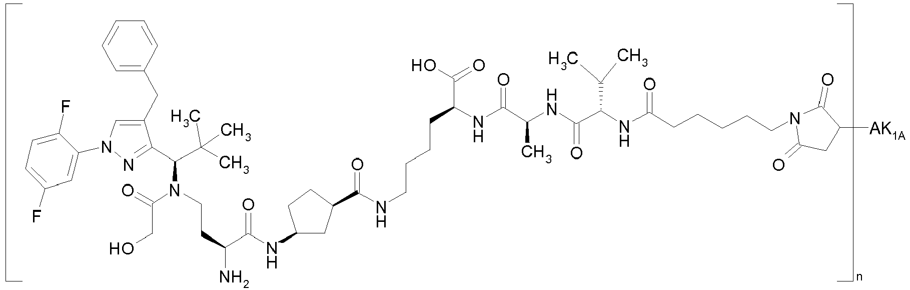

Eingesetzt wurden hier zur Kupplung mit Intermediat F129 5 mg Cetuximab in PBS (c=26.84 mg/mL) und der Ansatz wurde nach der Sephadex-Reinigung durch Ultrazentrifugation aufkonzentriert und rückverdünnt mit PBS.
Protein Konzentration: 2.28 mg/mL
Drug/mAb Ratio: 2.9

### Beispiel 129B

Eingesetzt wurden hier zur Kupplung mit Intermediat F129 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS.
Protein Konzentration: 2.06 mg/mL
Drug/mAb Ratio: 3.2

### Beispiel 129E

Eingesetzt wurden hier zur Kupplung mit Intermediat F129 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 2.0 mg/mL
Drug/mAb Ratio: 3.4

### Beispiel 131

### S-[1-(2-{[2-({(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}amino)ethyl]amino}-2-oxoethyl)-2,5-dioxopyrrolidin-3-yl]-L-cystein -trifluoressigsäure (1:1)

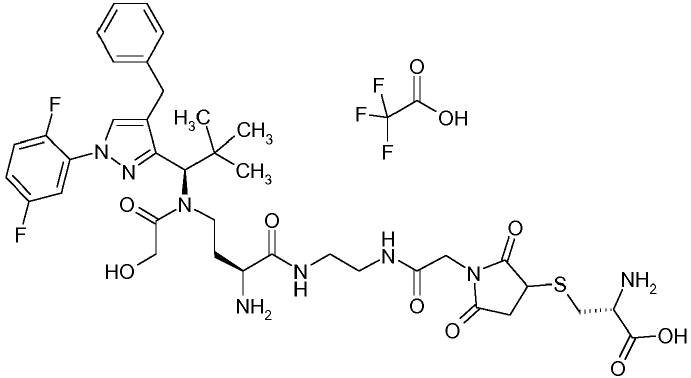

5 mg (6 µmol) von Intermediat F87 wurden in 1 ml DMF aufgenommen und mit 7.5 mg (62 µmol) L-Cystein versetzt. Das Reaktionsgemisch wurde 20 h bei RT gerührt, anschließend im Vakuum eingeengt und dann mittels präparativer HPLC gereinigt.
LC-MS (Methode 1): Rₜ = 0.81 min; MS (EIpos): m/z = 815 [M+H]⁺.

### Beispiel 132

### N-{(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}-beta-alanyl-L-alanyl-N-[4-(3-{[(2R)-2-amino-2-carboxyethyl]sulfanyl}-2,5-dioxopyrrolidin-1-yl)phenyl]-N⁵-carbamoyl-L-ornithinamid -trifluoressigsäure (1:1)

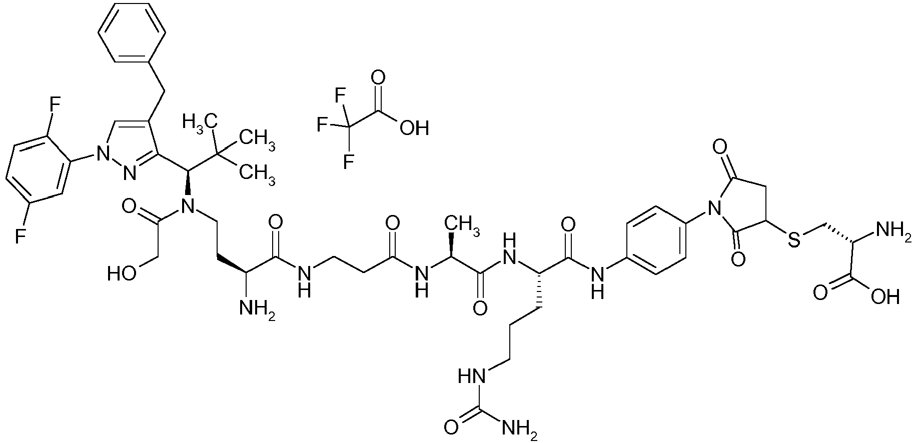

5 mg (5 µmol) von Intermediat F89 wurden in 2 ml DMF/Wasser 10:1 aufgenommen und mit 1.7 mg (14 µmol) L-Cystein versetzt. Das Reaktionsgemisch wurde 1 h bei RT gerührt und dann im Vakuum eingeengt. Der Rückstand wurde in Acetonitril/Wasser 1:1 aufgenommen, mit TFA auf pH 2 gebracht und anschließend im Vakuum eingeengt und dann mittels präparativer HPLC gereinigt. Die entsprechenden Fraktionen wurden eingeengt und nach Lyophilisation des Rückstands aus Acetonitril/Wasser verblieben 3 mg der Titelverbindung als weißer Schaum.
LC-MS (Methode 4): Rₜ = 0.93 min; MS (EIpos): m/z = 1105 [M+H]⁺.

### Beispiel 133

### S-(1-{2-[(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)amino]ethyl}-2,5-dioxopyrrolidin-3-yl)-L-cystein -trifluoressigsäure (1:1)

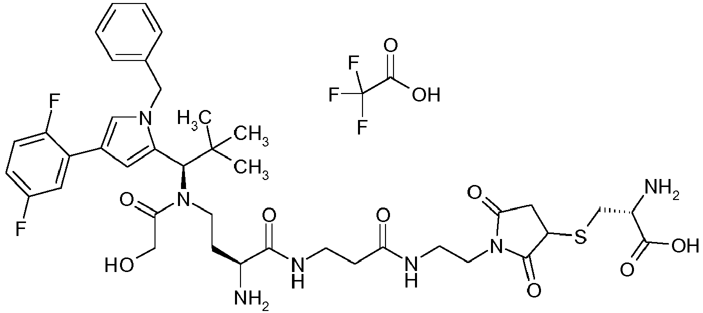

1.6 mg (2 µmol) von Intermediat F84 wurden in 1.5 ml DMF/Wasser 10:1 aufgenommen und mit 0.74 mg (6 µmol) L-Cystein versetzt. Das Reaktionsgemisch wurde 10 min bei RT gerührt und dann im Vakuum eingeengt. Der Rückstand wurde in Acetonitril/Wasser 1:1 aufgenommen, mit TFA auf pH 2 gebracht und anschließend im Vakuum eingeengt und dann mittels präparativer HPLC gereinigt. Die entsprechenden Fraktionen wurden eingeengt und nach Lyophilisation des Rückstands aus Acetonitril/Wasser verblieben 1.9 mg (89% d. Th.) der Titelverbindung als weißer Schaum.
LC-MS (Methode 1): Rₜ = 0.8 min; MS (EIpos): m/z = 828 [M+H]⁺.

### Beispiel 134

### N-[6-(3-{[(2R)-2-Amino-2-carboxyethyl]sulfanyl}-2,5-dioxopyrrolidin-1-yl)hexanoyl]-L-valyl-N⁵-carbamoyl-L-ornithyl-N⁶-{(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}-L-lysin-trifluoressigsäure (1:1)

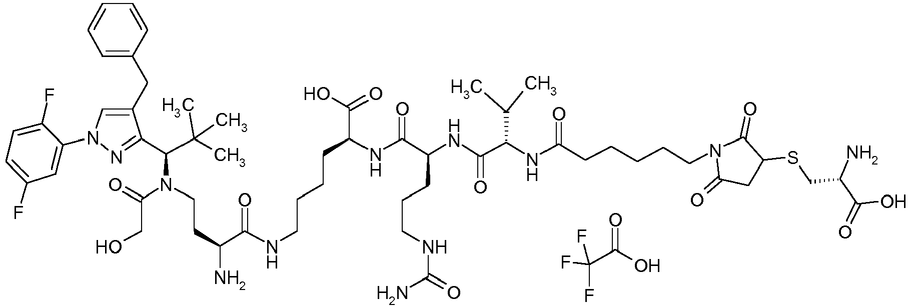

3.8 mg (3 µmol) von Intermediat F90 wurden in 1.5 ml DMF/Wasser 10:1 aufgenommen und mit 1.2 mg (9 µmol) L-Cystein versetzt. Das Reaktionsgemisch wurde 15 min bei RT gerührt und dann im Vakuum eingeengt. Der Rückstand wurde in Acetonitril/Wasser 1:1 aufgenommen, erneut eingeengt und dann mittels präparativer HPLC gereinigt. Die entsprechenden Fraktionen wurden eingeengt und nach Lyophilisation des Rückstands aus Acetonitril/Wasser verblieben 2.3 mg (56% d. Th.) der Titelverbindung als weißer Schaum.
LC-MS (Methode 4): Rₜ = 1.0 min; MS (EIpos): m/z = 1213 [M+H]⁺.

### Beispiel 135

### S-[1-(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)-2,5-dioxopyrrolidin-3-yl]-L-cystein -trifluoressigsäure (1:1)

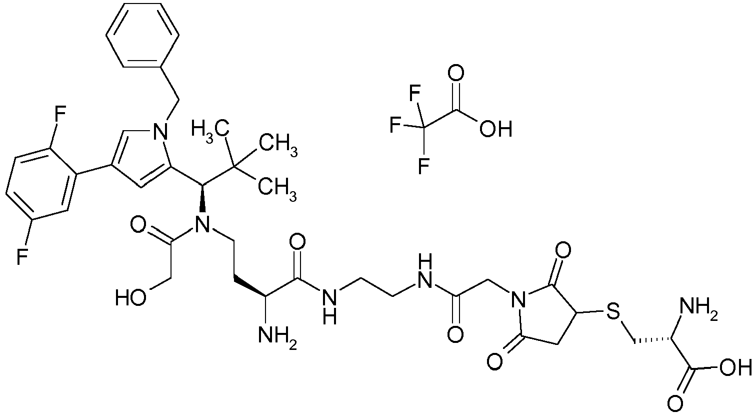

1.8 mg (2 µmol) von Intermediat F104 wurden in 1 ml DMF aufgenommen und mit 2.7 mg (22 µmol) L-Cystein versetzt. Das Reaktionsgemisch wurde 20 h bei RT gerührt, anschließend im Vakuum eingeengt und dann mittels präparativer HPLC gereinigt. Es verblieben 0.6 mg (26% d. Th.) der Titelverbindung als farbloser Schaum.
LC-MS (Methode 1): Rₜ = 0.80 min; MS (EIpos): m/z = 814 [M+H]⁺.

### Beispiel 136

### S-(2-{[2-({(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}amino)ethyl]amino}-2-oxoethyl)-L-cystein-trifluoressigsäure (1:1)

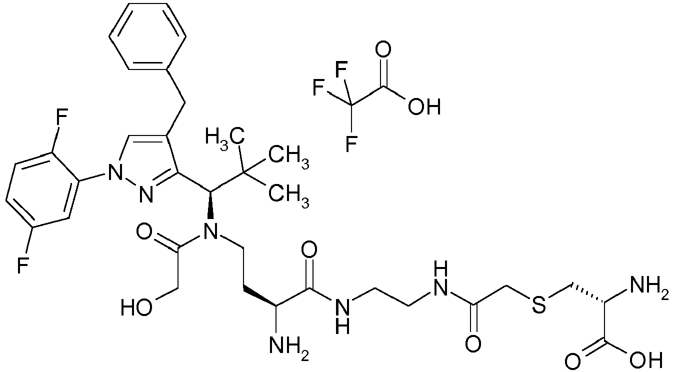

3.3 mg (4 µmol) von Intermediat F109 wurden in 2 ml DMF/Wasser 10:1 aufgenommen und mit 1.5 mg (13 µmol) L-Cystein versetzt. Das Reaktionsgemisch wurde 30 min bei RT gerührt und dann im Vakuum eingeengt. Der Rückstand wurde in Acetonitril/Wasser 1:1 aufgenommen, erneut eingeengt und dann mittels präparativer HPLC gereinigt. Die entsprechenden Fraktionen wurden eingeengt und nach Lyophilisation des Rückstands aus Acetonitril/Wasser verblieben 1.9 mg (55% d. Th.) der Titelverbindung als weißer Schaum.
LC-MS (Methode 1): Rₜ = 0.75 min; MS (EIpos): m/z = 718 [M+H]⁺.

### Beispiel 137

### S-{1-[6-(2-{(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}hydrazino)-6-oxohexyl]-2,5-dioxopyrrolidin-3-yl}-L-cystein -trifluoressigsäure (1:1)

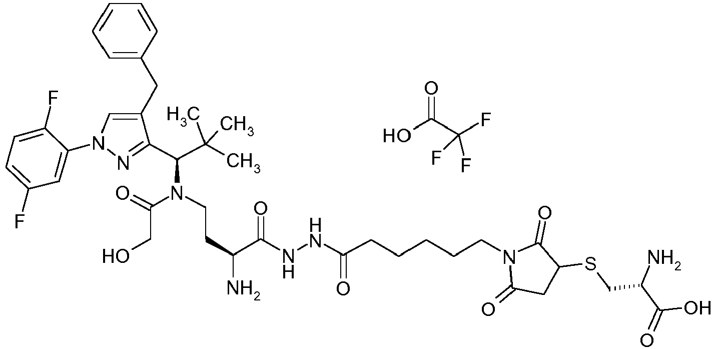

3.2 mg (4 µmol) von Intermediat F117 wurden in 2 ml DMF/Wasser 10:1 aufgenommen und mit 1.6 mg (13 µmol) L-Cystein versetzt. Das Reaktionsgemisch wurde 30 min bei RT gerührt und dann im Vakuum eingeengt. Der Rückstand wurde in Acetonitril/Wasser 1:1 aufgenommen, erneut eingeengt und dann mittels präparativer HPLC gereinigt. Die entsprechenden Fraktionen wurden eingeengt und nach Lyophilisation des Rückstands aus Acetonitril/Wasser verblieben 2 mg (47% d. Th.) der Titelverbindung als weißer Schaum.
LC-MS (Methode 1): Rₜ = 0.76 min; MS (EIpos): m/z = 843 [M+H]⁺.

### Beispiel 138

### N-[19-(3(R/S)-{[(2R)-2-Amino-2-carboxyethyl]sulfanyl}-2,5-dioxopyrrolidin-1-yl)-17-oxo-4,7,10,13-tetraoxa-16-azanonadecan-1-oyl]-R/S-{2-[(3-aminopropyl){(1R)-1-[1-benzyl-4-(2,5-difluorphenyl)-1H-pyrrol-2-yl]-2,2-dimethylpropyl}amino]-2-oxoethyl}homocystein-trifluoressigsäure(1:1)

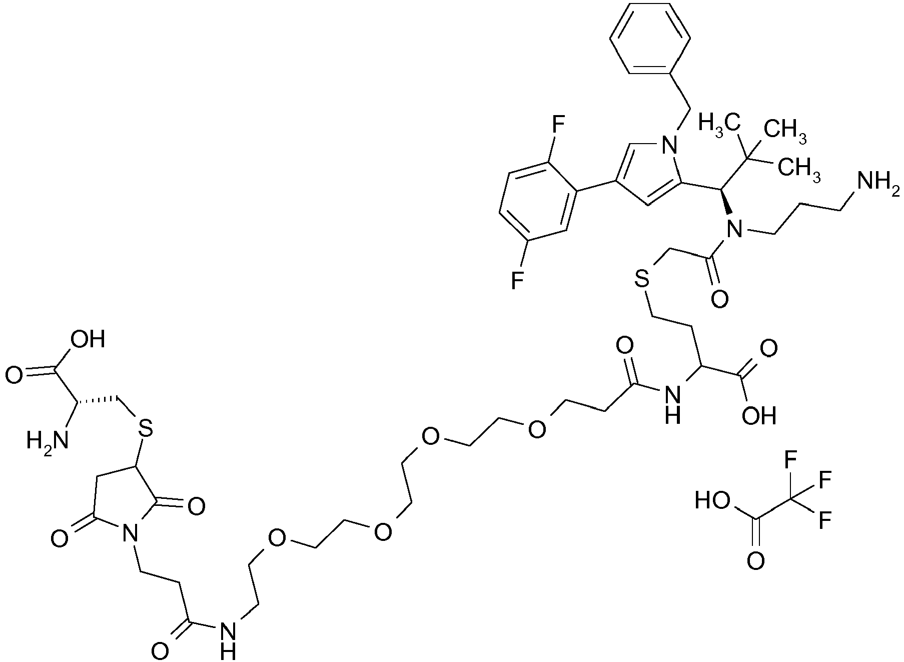

6.0 mg (0.01 mmol) R/S-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-homocystein-trifluoressigsäure (1:1) (Intermediat F146) wurden in 2.2 mL DMF/Wasser (10:1) vorgelegt und mit 2.0 mg (0.02 mmol) L-Cystein versetzt und 10 min bei RT gerührt. Die Reaktionsmischung wurde direkt mittles präp. RP-HPLC gereinigt (Säule: Reprosil 250x30; 10µ, Fluss: 50 mL/min, MeCN/Wasser, 0.1% TFA). Die Lösemittel wurden im Vakuum verdampft und der Rückstand im Hochvakuum getrocknet. Man erhielt 5.5 mg (76 % d. Th.) der Titelverbindung.
LC-MS (Methode 4): Rₜ = 1.07 min; MS (ESIpos): m/z = 1106 (M+H)⁺.

### Beispiel 139

### S-{(3R/S)-1-[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl}amino)ethyl]-2,5-dioxopyrrolidin-3-yl} -L-cystein-trifluoressigsäure(1:2)

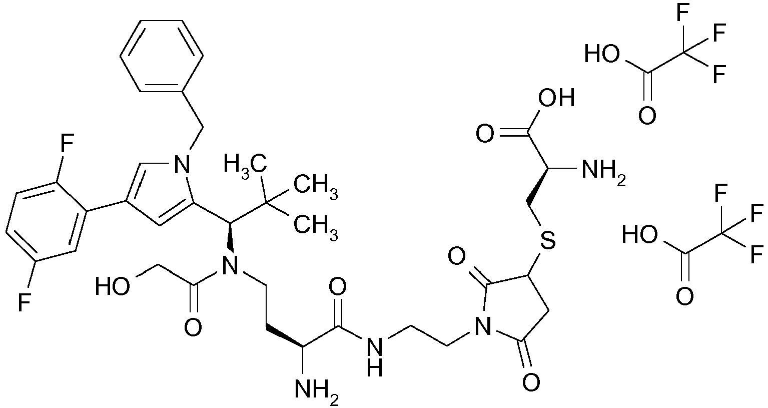

Die Synthese erfolgte in Analogie zur Synthese der Verbindung Beispiel 138.
6.0 mg (0.01 mmol) Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-[2-(2,5-dioxo-2,5-dihydro-1H-pyrrol-1-yl)ethyl]butanamid(1:1).
2.6 mg (0.02 mmol) L-Cystein.
Man erhielt 3.4 mg (43 % d. Th.) der Titelverbindung.
LC-MS (Methode 1): Rₜ = 0.82 min; MS (ESIpos): m/z = 757 (M+H)⁺.

### Beispiel 141

### S-[(3R/S)-1-(2-{[6-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)hexanoyl]amino}ethyl)-2,5-dioxopyrrolidin-3-yl]-L-cystein-trifluoressigsäure(1:2)

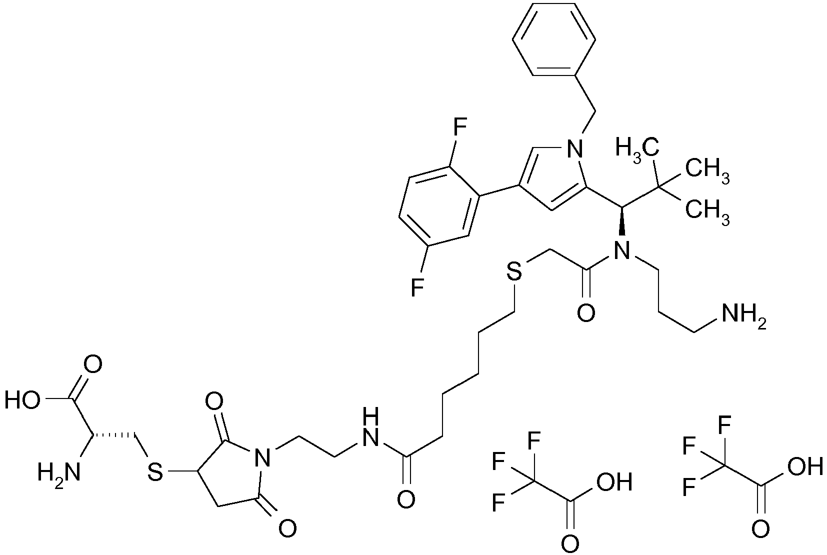

Die Synthese erfolgte in Analogie zur Synthese der Verbindung Beispiel 138.
10.07 mg (0.01 mmol) Trifluoressigsäure--6-({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)-N-[2-(2,5-dioxo-2,5-dihydro-1H-pyrrol-1-yl)ethyl]hexanamid(1:1) (Intermediat F143).
9.3 mg (0.08 mmol) L-Cystein.
Man erhielt 9.2 mg (67 % d. Th.) der Titelverbindung.
LC-MS (Methode 4): Rₜ = 1.05 min; MS (ESIpos): m/z = 843 (M+H)⁺.

### Beispiel 142A

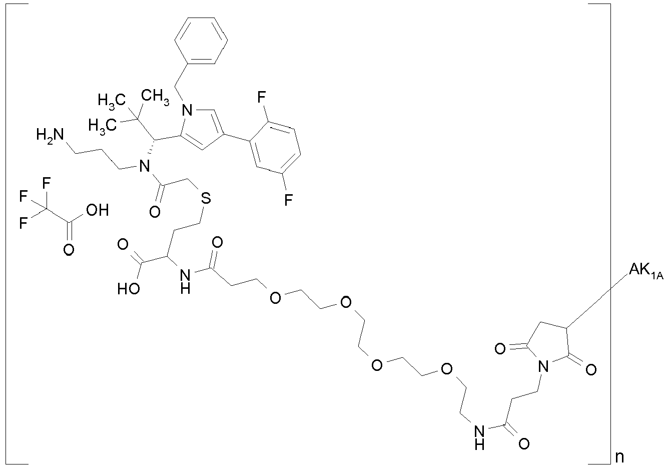

Eingesetzt wurden hier zur Kupplung mit Intermediat F142 5.0 mg Cetuximab in PBS (c = 12.33 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 2.08 mg/mL
Drug/mAb Ratio: 2.1

### Beispiel 142B

Eingesetzt wurden hier zur Kupplung mit Intermediat F1425.0 mg anti-TWEAKR AK-1 Antikörper in PBS (c = 34.42 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.84 mg/mL
Drug/mAb Ratio: 2.0

### Beispiel 142E

Eingesetzt wurden hier zur Kupplung mit Intermediat F142 5.0 mg TrastuzumabAntikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.92 mg/mL
Drug/mAb Ratio: 2.3

### Beispiel 1421

Eingesetzt wurden hier zur Kupplung mit Intermediat F142 5.0 mg Nimotuzumab in PBS (c = 13.8 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.92 mg/mL
Drug/mAb Ratio: 2.5

### Beispiel 142H

Eingesetzt wurden hier zur Kupplung mit Intermediat F142 5.0 mg Panitumumab in PBS (c = 2.1 mg/mL). Die Reduktionszeit mit TCEP betrug 1 h und die Rührzeit bei der ADC Kupplung betrug 1.5 h. Anschließend wurde der Ansatz nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.7 mg/mL
Drug/mAb Ratio: 2.2

### Beispiel 143A

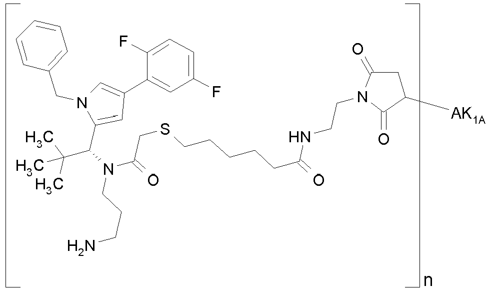

Eingesetzt wurden hier zur Kupplung mit Intermediat F143 5.0 mg Cetuximab in PBS (c = 12.33 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.84 mg/mL
Drug/mAb Ratio: 2.5

### Beispiel 143B

Eingesetzt wurden hier zur Kupplung mit Intermediat F143 5.0 mg anti-TWEAKR AK-1 Antikörper in PBS (c = 34.42 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.05 mg/mL
Drug/mAb Ratio: 1.6

### Beispiel 143E

Eingesetzt wurden hier zur Kupplung mit Intermediat F143 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.95 mg/mL
Drug/mAb Ratio: 2.3

### Beispiel 144B

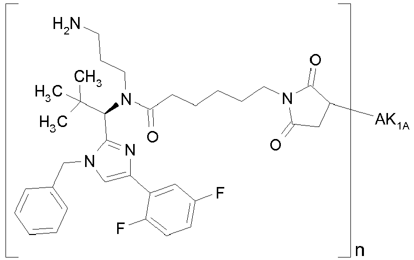

Eingesetzt wurden hier zur Kupplung mit Intermediat F144 5.0 mg anti-TWEAKR AK-1 Antikörper in PBS (c = 12.87 mg/mL) und der Ansatz wurde nach der Sephadex-Reinigung durch Ultrazentrifugation aufkonzentriert und rückverdünnt.
Protein Konzentration: 1.53 mg/mL
Drug/mAb Ratio: 2.0

### Beispiel 146A

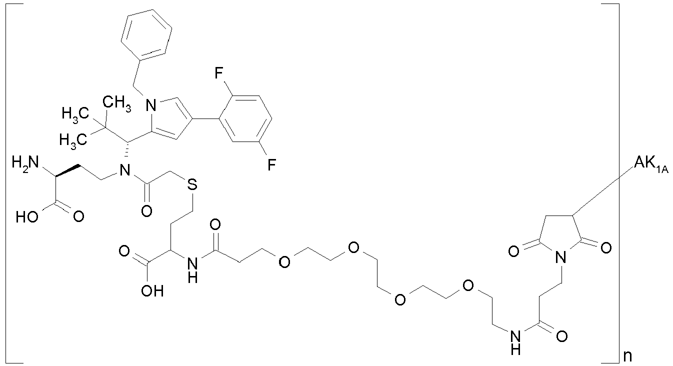

Eingesetzt wurden hier zur Kupplung mit Intermediat F146 5.0 mg Cetuximab in PBS (c = 12.33 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 2.02 mg/mL
Drug/mAb Ratio: 2.4

### Beispiel 146B

Eingesetzt wurden hier zur Kupplung mit Intermediat F146 5.0 mg anti-TWEAKR AK-1 Antikörper in PBS (c = 34.42 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.87 mg/mL
Drug/mAb Ratio: 2.4

### Beispiel 146E

Eingesetzt wurden hier zur Kupplung mit Intermediat F146 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.92 mg/mL
Drug/mAb Ratio: 2.5

### Beispiel 153A

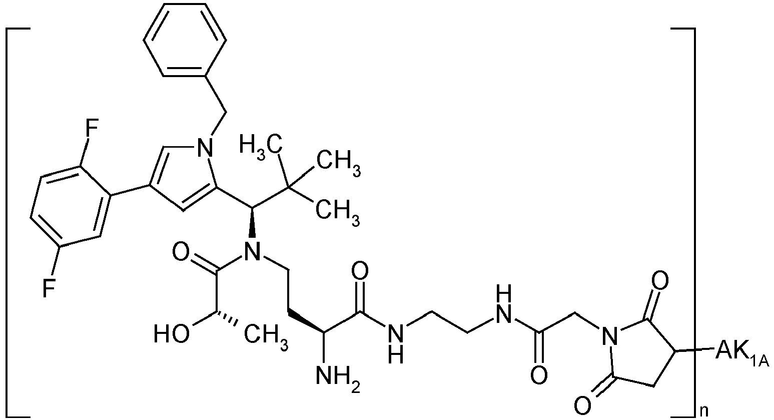

Eingesetzt wurden hier zur Kupplung mit Intermediat F153 5 mg Cetuximab in PBS (c=21.32 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.93 mg/mL
Drug/mAb Ratio: 3.2

### Beispiel 153B

Eingesetzt wurden hier zur Kupplung mit Intermediat F153 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.71 mg/mL
Drug/mAb Ratio: 2.8

### Beispiel 155A

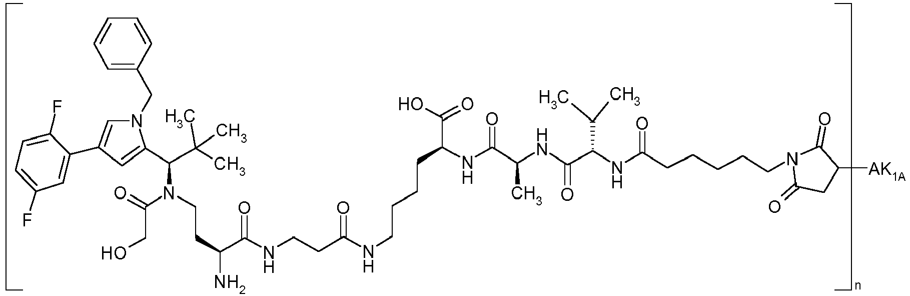

Eingesetzt wurden hier zur Kupplung mit Intermediat F155 50 mg Cetuximab in PBS (c=8.51 mg/mL) und der Ansatz wurde nach der Sephadex-Reinigung durch Ultrazentrifugation aufkonzentriert, rückverdünnt mit PBS und erneut aufkonzentriert.
Protein Konzentration: 14.85 mg/mL
Drug/mAb Ratio: 2.5

### Beispiel 155B

Eingesetzt wurden hier zur Kupplung mit Intermediat F155 40 mg anti-TWEAKR AK-1 in PBS (c=18.6 mg/mL) und der Ansatz wurde nach der Sephadex-Reinigung durch Ultrazentrifugation aufkonzentriert, rückverdünnt mit PBS und erneut aufkonzentriert.
Protein Konzentration: 11.25 mg/mL
Drug/mAb Ratio: 3.1

### Beispiel 156A

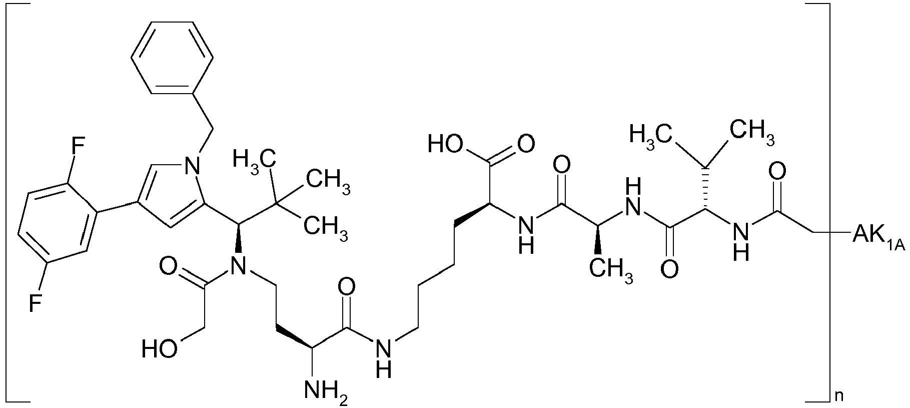

Eingesetzt wurden hier zur Kupplung mit Intermediat F156 5 mg Cetuximab in PBS (c=21.3 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 1.83 mg/mL
Drug/mAb Ratio: 3.6

### Beispiel 156B

Eingesetzt wurden hier zur Kupplung mit Intermediat F156 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS.
Protein Konzentration: 1.79 mg/mL
Drug/mAb Ratio: 3.9

### Beispiel 156E

Eingesetzt wurden hier zur Kupplung mit Intermediat F156 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 1.91 mg/mL
Drug/mAb Ratio: 4.2

### Beispiel 157

### S-{1-[2-({[(1R,3S)-3-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)cyclopentyl]carbonyl}amino)ethyl]-2,5-dioxopyrrolidin-3-yl}-L-cystein -trifluoressigsäure (1:1)

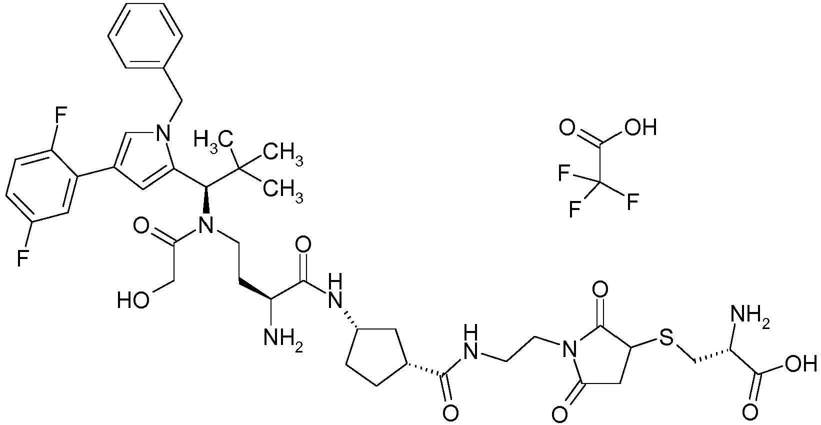

2 mg (2 µmol) von Intermediat F125 wurden in 2 ml DMF/Wasser 10:1 aufgenommen und mit 0.8 mg (6 µmol) L-Cystein versetzt. Das Reaktionsgemisch wurde 20 h bei RT gerührt, anschließend im Vakuum eingeengt und dann mittels präparativer HPLC gereinigt.

LC-MS (Methode 1): Rₜ = 0.81 min; MS (EIpos): m/z = 868 [M+H]⁺.

### Beispiel 158

### S-(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)-L-cystein -trifluoressigsäure (1:1)

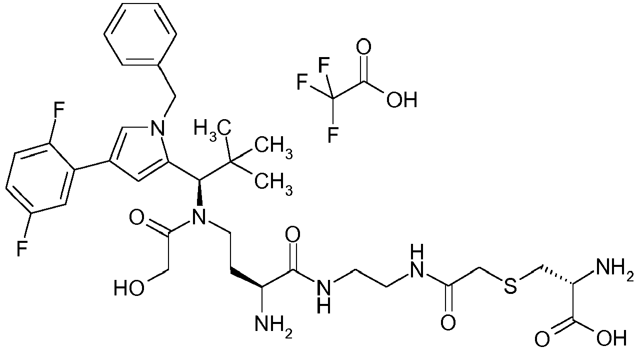

6 mg (8 µmol) von Intermediat F119 wurden in 3 mL DMF aufgenommen und mit 1.8 mg (15 µmol) L-Cystein versetzt. Das Reaktionsgemisch wurde 6 h bei RT gerührt und dann 3 Tage bei RT stehen gelassen. Anschließend wurde der Ansatz im Vakuum eingeengt und das Produkt mittels präparativer HPLC gereinigt.
LC-MS (Methode 1): Rₜ = 0.81 min; MS (ESIpos): m/z = 717 (M+H)⁺.

### Beispiel 160

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-N²-{N-[6-(3-{[(2R)-2-amino-2-carboxyethyl]sulfanyl}-2,5-dioxopyrrolidin-1-yl)hexanoyl]-L-valyl-L-alanyl}-L-lysin -trifluoressigsäure (1:1)

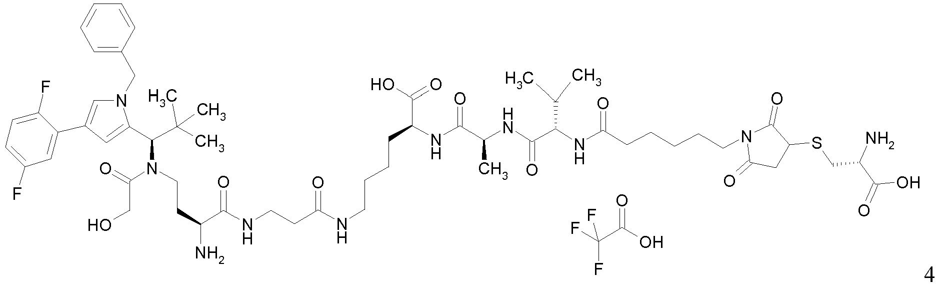

mg (3 µmol) von Intermediat F155 wurden in 2.5 ml DMF/Wasser 10:1 aufgenommen und mit 1.2 mg (10 µmol) L-Cystein versetzt. Das Reaktionsgemisch wurde 30 min bei RT gerührt, anschließend im Vakuum eingeengt und nach Aufnahme in Acetonitril/Wasser 1:1 mittels präparativer HPLC gereinigt.
LC-MS (Methode 1): Rₜ = 0.81 min; MS (EIpos): m/z = 1197 [M+H]⁺.

### Beispiel 161

### N-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-L-glutamin -trifluoressigsäure (1:1)

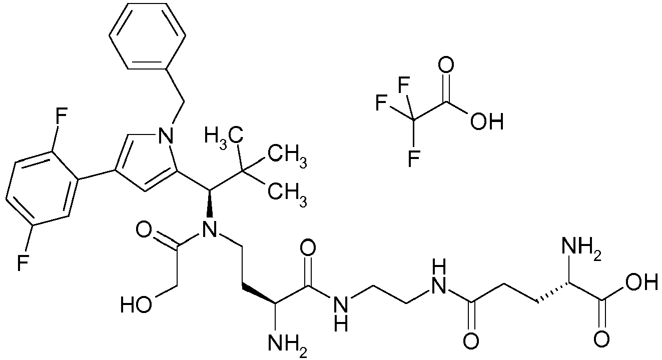

Zunächst wurde nach klassischen Methoden der Peptidchemie Trifluoressigsäure -benzyl-N-(2-aminoethyl)-N²-[(benzyloxy)carbonyl]-L-glutaminat (1:1) hergestellt. Diese Intermediat wurde dann in Gegenwart von HATU mit Intermediat C58 gekuppelt. Anschließend wurde zunächst durch hydrogenolytische Spaltung die Benzyloxycarbonyl-Schutzgruppe sowie der Benzylester entfernt und anschließend mit Zinkchlorid die 2-(Trimethylsilyl)ethoxycarbonyl-Schutzgrupe.
LC-MS (Methode 6): Rₜ = 1.91 min; MS (EIpos): m/z = 685 [M+H]⁺.

### Beispiel 162

### N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-L-lysin-trifluoressigsäure (1:1)

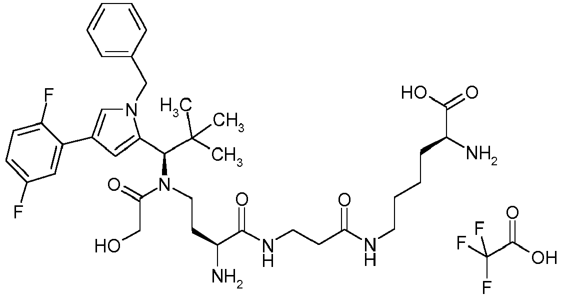

Zunächst wurde nach in der Peptidchemie bekannten klassischen Schutzgruppenoperationen Trifluoressigsäure --2-(trimethylsilyl)ethyl-N2-[(benzyloxy)carbonyl]-L-lysinat (1:1) hergestellt. Dieses Intermediat wurde dann in Gegenwart von HATU mit Intermediat C61 gekuppelt. Anschließend wurde zunächst mit Zinkchlorid die 2-(Trimethylsilyl)ethoxycarbonyl-Schutzgrupe sowie der 2-(Trimethylsilyl)ethylester gespalten. Schließlich wurde die Titelverbindung durch hydrogenolytische Spaltung der Benzyloxycarbonyl-Schutzgruppe und Reinigung durch präparative HPLC erhalten. HPLC (Methode 11): Rₜ = 1.65 min;
LC-MS (Methode 1): Rₜ = 0.76 min; MS (EIpos): m/z = 713 [M+H]⁺.

### Beispiel 163A

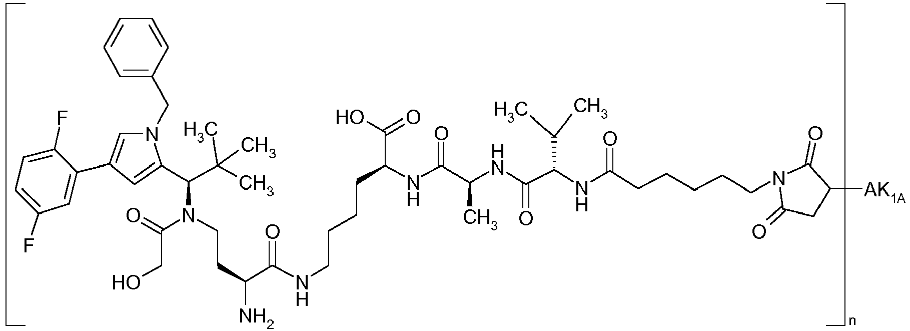

Eingesetzt wurden hier zur Kupplung mit Intermediat F163 5 mg Cetuximab in PBS (c=11.3 mg/mL) und der Ansatz wurde nach der Sephadex-Reinigung durch Ultrazentrifugation aufkonzentriert und rückverdünnt mit PBS.
Protein Konzentration: 2.02 mg/mL
Drug/mAb Ratio: 3.3

### Beispiel 163B

Eingesetzt wurden hier zur Kupplung mit Intermediat F163 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS.
Protein Konzentration: 1.76 mg/mL
Drug/mAb Ratio: 2.5

### Beispiel 163H

Eingesetzt wurden hier zur Kupplung mit Intermediat F163 5.0 mg Panitumumab in PBS (c = 10 mg/mL). Die Reduktionszeit mit TCEP betrug 30 min und die Rührzeit bei der ADC Kupplung betrug 2 h. Anschließend wurde der Ansatz nach der Sephadex-Reinigung durch Ultrazentrifugation aufkonzentriert und rückverdünnt mit PBS.
Protein Konzentration: 1.61mg/mL
Drug/mAb Ratio: 2.6

### Beispiel 164A

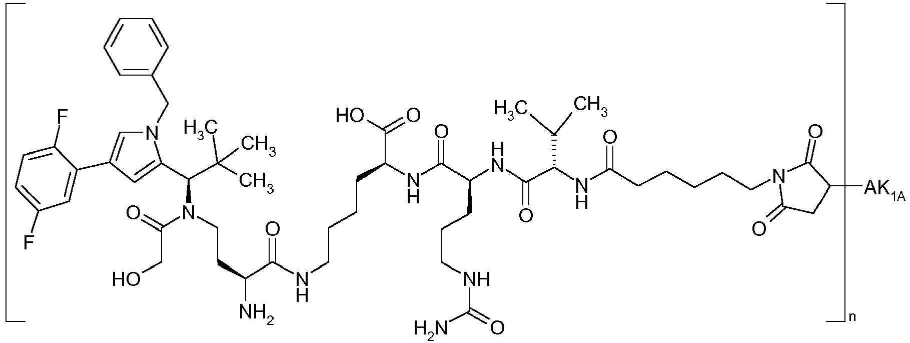

Eingesetzt wurden hier zur Kupplung mit Intermediat F164 5 mg Cetuximab in PBS (c=16.9 mg/mL) und der Ansatz wurde nach der Sephadex-Reinigung durch Ultrazentrifugation aufkonzentriert und rückverdünnt.
Protein Konzentration: 2.15 mg/mL
Drug/mAb Ratio: 3.5

### Beispiel 164B

Eingesetzt wurden hier zur Kupplung mit Intermediat F164 30 mg anti-TWEAKR AK-1 in PBS (c=18.6 mg/mL) und der Ansatz wurde nach der Sephadex-Reinigung durch Ultrazentrifugation aufkonzentriert, rückverdünnt mit PBS und erneut aufkonzentriert.
Protein Konzentration: 14.8 mg/mL
Drug/mAb Ratio: 2.8

### Beispiel 164E

Eingesetzt wurden hier zur Kupplung mit Intermediat F164 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 2.11 mg/mL
Drug/mAb Ratio: 3.8

### Beispiel 165A

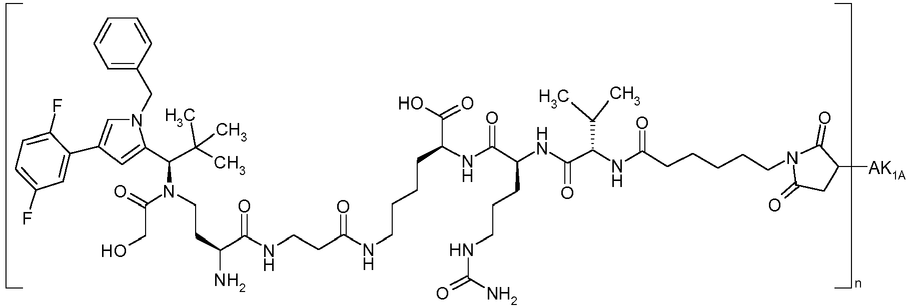

Eingesetzt wurden hier zur Kupplung mit Intermediat F165 5 mg Cetuximab in PBS (c=16.9 mg/mL) und der Ansatz wurde nach der Sephadex-Reinigung durch Ultrazentrifugation aufkonzentriert und rückverdünnt mit PBS.
Protein Konzentration: 1.93 mg/mL
Drug/mAb Ratio: 3.4

### Beispiel 165B

Eingesetzt wurden hier zur Kupplung mit Intermediat F165 40 mg anti-TWEAKR AK-1 in PBS (c=12.9 mg/mL) und der Ansatz wurde nach der Sephadex-Reinigung durch Ultrazentrifugation aufkonzentriert, rückverdünnt mit PBS und erneut aufkonzentriert.
Protein Konzentration: 12.02 mg/mL
Drug/mAb Ratio: 3.3

### Beispiel 165E

Eingesetzt wurden hier zur Kupplung mit Intermediat F165 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 1.94 mg/mL
Drug/mAb Ratio: 3.5

### Beispiel 166A

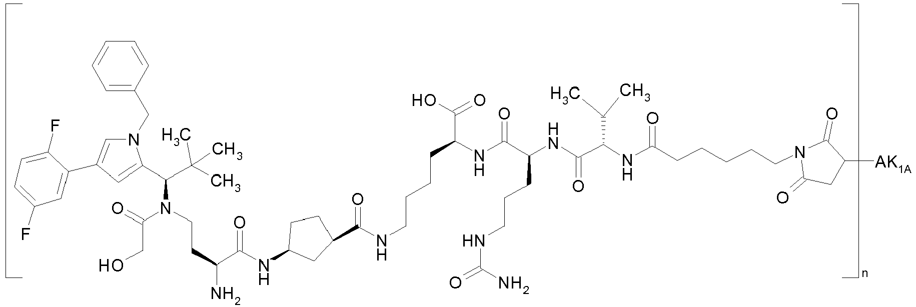

Eingesetzt wurden hier zur Kupplung mit Intermediat F166 5 mg Cetuximab in PBS (c=21.3 mg/mL) und der Ansatz wurde nach der Sephadex-Reinigung durch Ultrazentrifugation aufkonzentriert und rückverdünnt mit PBS.
Protein Konzentration: 2.0 mg/mL
Drug/mAb Ratio: 3.0

### Beispiel 166B

Eingesetzt wurden hier zur Kupplung mit Intermediat F166 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS.
Protein Konzentration: 1.76 mg/mL
Drug/mAb Ratio: 3.4

### Beispiel 166E

Eingesetzt wurden hier zur Kupplung mit Intermediat F166 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 2.01 mg/mL
Drug/mAb Ratio: 3.6

### Beispiel 168A

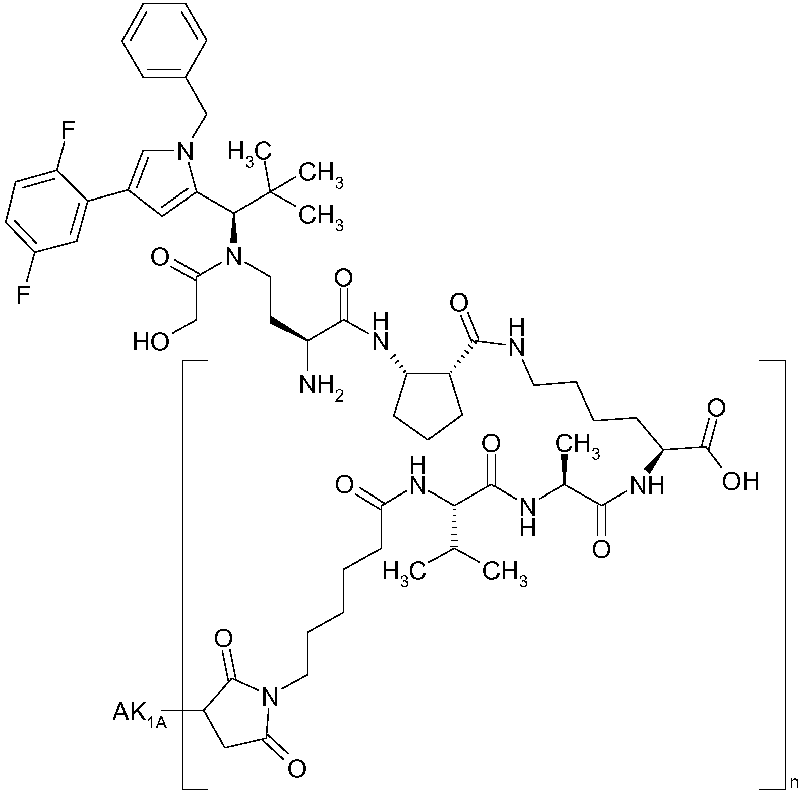

Eingesetzt wurden hier zur Kupplung mit Intermediat F168 5 mg Cetuximab in PBS (c=11.3 mg/mL) und der Ansatz wurde nach der Sephadex-Reinigung durch Ultrazentrifugation aufkonzentriert und rückverdünnt mit PBS.
Protein Konzentration: 1.94 mg/mL
Drug/mAb Ratio: 3.0

### Beispiel 168B

Eingesetzt wurden hier zur Kupplung mit Intermediat F168 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS.
Protein Konzentration: 1.33 mg/mL
Drug/mAb Ratio: 2.8

### Beispiel 168E

Eingesetzt wurden hier zur Kupplung mit Intermediat F168 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 1.89 mg/mL
Drug/mAb Ratio: 3.0

### Beispiel 168H

Eingesetzt wurden hier zur Kupplung mit Intermediat F168 5.0 mg Panitumumab in PBS (c = 10 mg/mL). Die Reduktionszeit mit TCEP betrug 4 h und die Rührzeit bei der ADC Kupplung betrug 20 h. Anschließend wurde der Ansatz nach der Sephadex-Reinigung durch Ultrazentrifugation aufkonzentriert und rückverdünnt mit PBS.
Protein Konzentration: 1.76mg/mL
Drug/mAb Ratio: 2.8

### Beispiel 169A

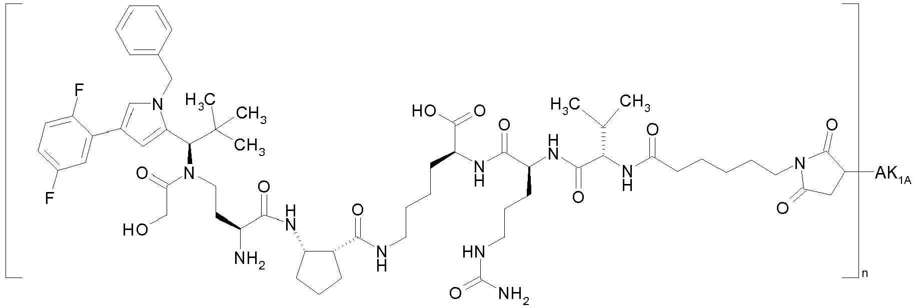

Eingesetzt wurden hier zur Kupplung mit Intermediat F169 5 mg Cetuximab in PBS (c=16.9 mg/mL) und der Ansatz wurde nach der Sephadex-Reinigung durch Ultrazentrifugation aufkonzentriert und rückverdünnt mit PBS.
Protein Konzentration: 1.98 mg/mL
Drug/mAb Ratio: 3.4

### Beispiel 169B

Eingesetzt wurden hier zur Kupplung mit Intermediat F169 40 mg anti-TWEAKR AK-1 in PBS (c=18.6 mg/mL) und der Ansatz wurde nach der Sephadex-Reinigung durch Ultrazentrifugation aufkonzentriert, rückverdünnt mit PBS und erneut aufkonzentriert.
Protein Konzentration: 11.2 mg/mL
Drug/mAb Ratio: 2.4

### Beispiel 169E

Eingesetzt wurden hier zur Kupplung mit Intermediat F169 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 1.95 mg/mL
Drug/mAb Ratio: 3.7

### Beispiel 171A

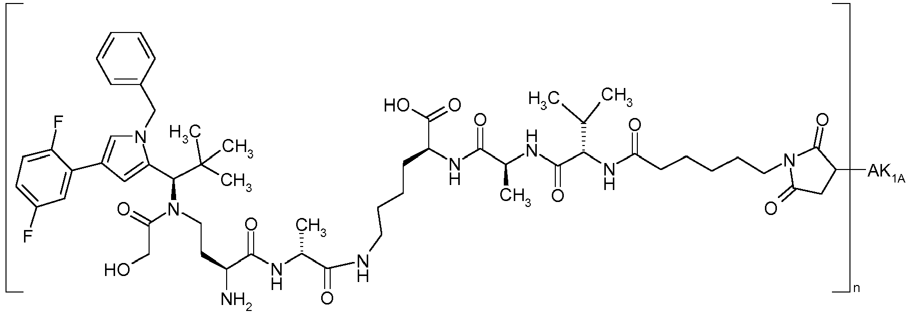

Eingesetzt wurden hier zur Kupplung mit Intermediat F171 5 mg Cetuximab in PBS (c=11.3 mg/mL) und der Ansatz wurde nach der Sephadex-Reinigung durch Ultrazentrifugation aufkonzentriert und rückverdünnt mit PBS.
Protein Konzentration: 1.94 mg/mL
Drug/mAb Ratio: 2.5

### Beispiel 171B

Eingesetzt wurden hier zur Kupplung mit Intermediat F171 5 mg anti-TWEAKR AK-1 in PBS (c=12.9 mg/mL) und der Ansatz wurde nach der Sephadex-Reinigung durch Ultrazentrifugation aufkonzentriert und rückverdünnt mit PBS.
Protein Konzentration: 1.58 mg/mL
Drug/mAb Ratio: 2.7

### Beispiel 173A

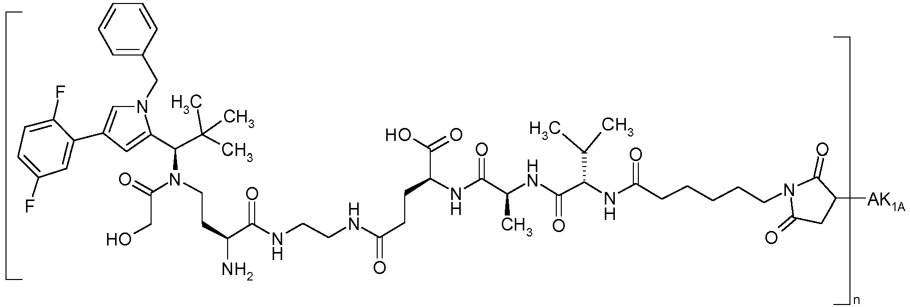

Eingesetzt wurden hier zur Kupplung mit Intermediat F173 5 mg Cetuximab in PBS (c=11.3 mg/mL) und der Ansatz wurde nach der Sephadex-Reinigung durch Ultrazentrifugation aufkonzentriert und rückverdünnt mit PBS.
Protein Konzentration: 2.1 mg/mL
Drug/mAb Ratio: 3.6

### Beispiel 173B

Eingesetzt wurden hier zur Kupplung mit Intermediat F173 5 mg anti-TWEAKR AK-1 in PBS (c=18.6 mg/mL) und der Ansatz wurde nach der Sephadex-Reinigung durch Ultrazentrifugation aufkonzentriert, rückverdünnt mit PBS und erneut aufkonzentriert.
Protein Konzentration: 12.26 mg/mL
Drug/mAb Ratio: 3.4

### Beispiel 173E

Eingesetzt wurden hier zur Kupplung mit Intermediat F173 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 2.33 mg/mL
Drug/mAb Ratio: 3.9

### Beispiel 174A

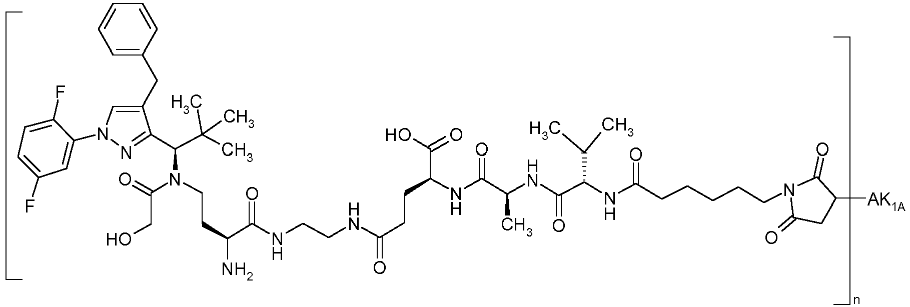

Eingesetzt wurden hier zur Kupplung mit Intermediat F174 5 mg Cetuximab in PBS (c=11.3 mg/mL) und der Ansatz wurde nach der Sephadex-Reinigung durch Ultrazentrifugation aufkonzentriert und rückverdünnt mit PBS.
Protein Konzentration: 2.18 mg/mL
Drug/mAb Ratio: 3.1

### Beispiel 174B

Eingesetzt wurden hier zur Kupplung mit Intermediat F174 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS.
Protein Konzentration: 1.99 mg/mL
Drug/mAb Ratio: 3.1

### Beispiel 174E

Eingesetzt wurden hier zur Kupplung mit Intermediat F174 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 2.03 mg/mL
Drug/mAb Ratio: 3.5

### Beispiel 175A

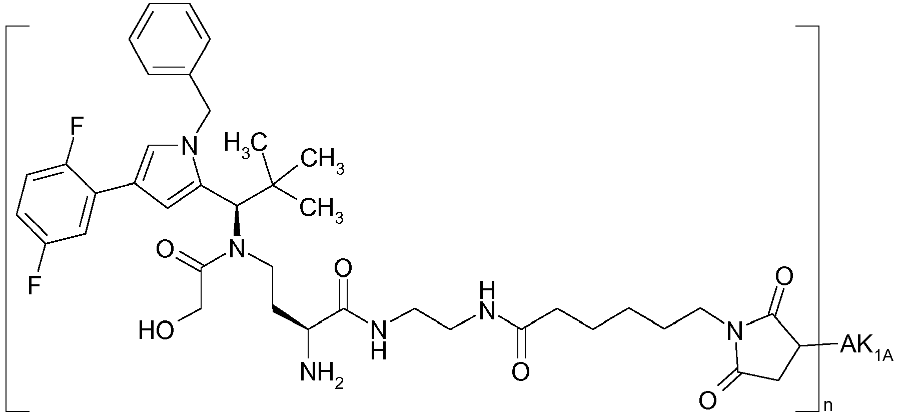

Eingesetzt wurden hier zur Kupplung mit Intermediat F175 5 mg Cetuximab in PBS (c=16.9 mg/mL) und der Ansatz wurde nach der Sephadex-Reinigung durch Ultrazentrifugation aufkonzentriert und rückverdünnt mit PBS.
Protein Konzentration: 1.98 mg/mL
Drug/mAb Ratio: 3.8

### Beispiel 175B

Eingesetzt wurden hier zur Kupplung mit Intermediat F175 40 mg anti-TWEAKR AK-1 in PBS (c=18.6 mg/mL) und der Ansatz wurde nach der Sephadex-Reinigung durch Ultrazentrifugation aufkonzentriert, rückverdünnt mit PBS und erneut aufkonzentriert.
Protein Konzentration: 9.8 mg/mL
Drug/mAb Ratio: 2.8

### Beispiel 175E

Eingesetzt wurden hier zur Kupplung mit Intermediat F175 5.0 mg Trastuzumab Antikörper in PBS (c = 13.5 mg/mL) und der Ansatz wurde nach der Sephadex-Reinigung durch Ultrazentrifugation aufkonzentriert und rückverdünnt mit PBS.
Protein Konzentration: 1.97 mg/mL
Drug/mAb Ratio: 4.2

### Beispiel 176A

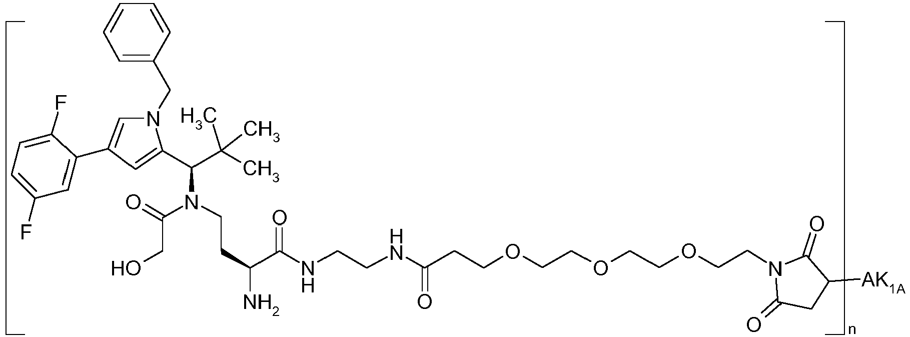

Eingesetzt wurden hier zur Kupplung mit Intermediat F176 5 mg Cetuximab in PBS (c=21.3 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.98 mg/mL
Drug/mAb Ratio: 2.6

### Beispiel 176B

Eingesetzt wurden hier zur Kupplung mit Intermediat F176 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.93 mg/mL
Drug/mAb Ratio: 2.8

### Beispiel 176E

Eingesetzt wurden hier zur Kupplung mit Intermediat F176 5.0 mg Trastuzumab Antikörper in PBS (c = 13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.85 mg/mL
Drug/mAb Ratio: 3.3

### Beispiel 177A

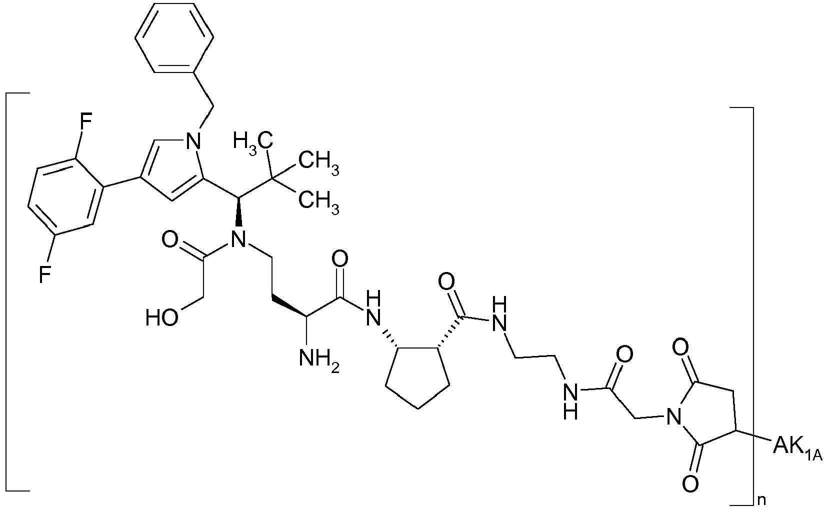

Eingesetzt wurden hier zur Kupplung mit Intermediat F177 5 mg Cetuximab in PBS (c=21.3 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.96 mg/mL
Drug/mAb Ratio: 2.8

### Beispiel 177B

Eingesetzt wurden hier zur Kupplung mit Intermediat F177 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.2 mg/mL
Drug/mAb Ratio: 2.3

### Beispiel 177E

Eingesetzt wurden hier zur Kupplung mit Intermediat F177 5 mg Trastuzumab in PBS (c=13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.83 mg/mL
Drug/mAb Ratio: 3.1

### Beispiel 178A

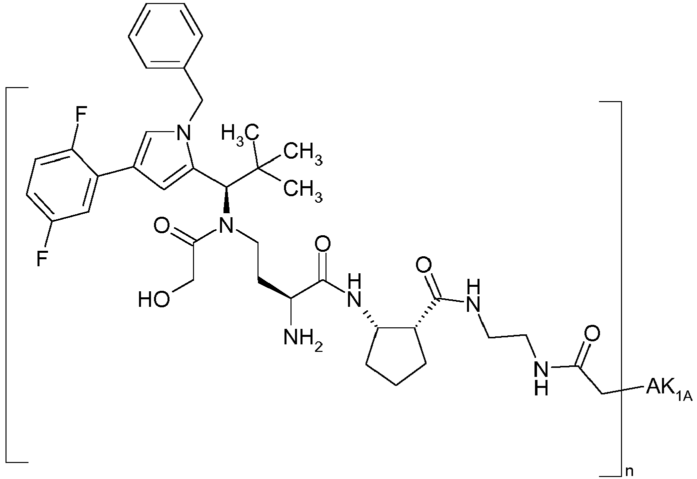

Eingesetzt wurden hier zur Kupplung mit Intermediat F178 5 mg Cetuximab in PBS (c=21.3 mg/mL) und der Ansatz wurde nach der Sephadex-Reinigung durch Ultrazentrifugation aufkonzentriert und rückverdünnt mit PBS.
Protein Konzentration: 1.8 mg/mL
Drug/mAb Ratio: 2.1

### Beispiel 178B

Eingesetzt wurden hier zur Kupplung mit Intermediat F178 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS.
Protein Konzentration: 1.45 mg/mL
Drug/mAb Ratio: 2.4

### Beispiel 178E

Eingesetzt wurden hier zur Kupplung mit Intermediat F178 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 1.8 mg/mL
Drug/mAb Ratio: 2.6

### Beispiel 179A

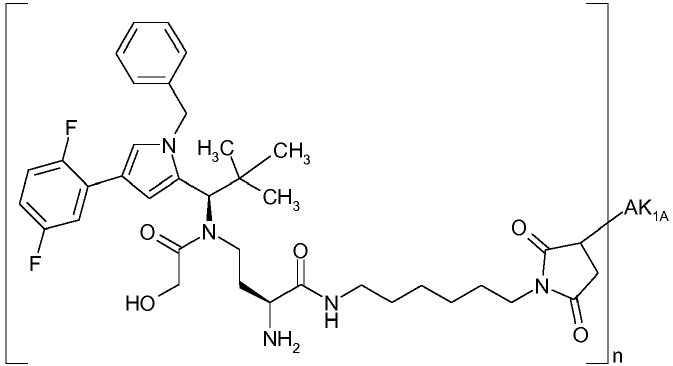

Eingesetzt wurden hier zur Kupplung mit Intermediat F179 5 mg Cetuximab in PBS (c=11.3 mg/mL) und der Ansatz wurde nach der Sephadex-Reinigung durch Ultrazentrifugation aufkonzentriert und rückverdünnt mit PBS.
Protein Konzentration: 2.04 mg/mL
Drug/mAb Ratio: 3.1

### Beispiel 179B

Eingesetzt wurden hier zur Kupplung mit Intermediat F179 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS.
Protein Konzentration: 1.65 mg/mL
Drug/mAb Ratio: 3.1

### Beispiel 179E

Eingesetzt wurden hier zur Kupplung mit Intermediat F179 5 mg Trastuzumab in PBS (c=13.5 mg/mL) und der Ansatz wurde nach der Sephadex-Reinigung durch Ultrazentrifugation aufkonzentriert und rückverdünnt mit PBS.
Protein Konzentration: 1.89 mg/mL
Drug/mAb Ratio: 3.3

### Beispiel 180A

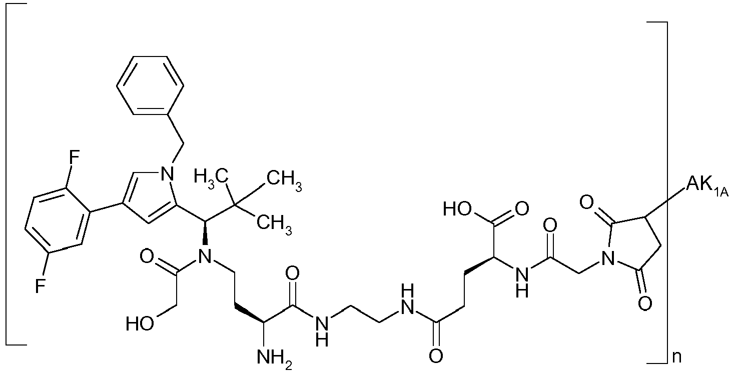

Eingesetzt wurden hier zur Kupplung mit Intermediat F180 5 mg Cetuximab in PBS (c=8.51 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.72 mg/mL
Drug/mAb Ratio: 3.5

### Beispiel 180B

Eingesetzt wurden hier zur Kupplung mit Intermediat F180 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.82 mg/mL
Drug/mAb Ratio: 3.4

### Beispiel 180E

Eingesetzt wurden hier zur Kupplung mit Intermediat F180 5 mg Trastuzumab in PBS (c=13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.01 mg/mL
Drug/mAb Ratio: 4.7

### Beispiel 181

### N-(3-Aminopropyl)-N-{(1R)-1-[4-benzyl-1-(2,5-difluorphenyl)-1H-pyrazol-3-yl]-2,2-dimethylpropyl} acetamid

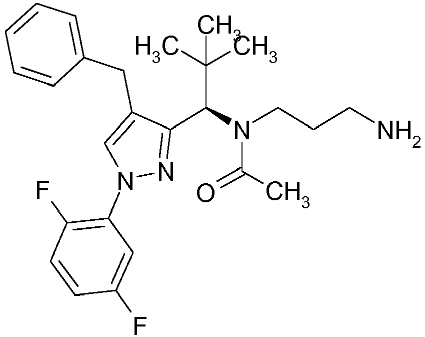

1.01 g (2.84 mmol) (1R)-1-[4-Benzyl-1-(2,5-difluorphenyl)-1H-pyrazol-3-yl]-2,2-dimethylpropan-1-amin wurden in 20 ml 1,2-Dichlormethan vorgelegt und mit 0.84 g (3.98 mmol) Natriumtriacetoxyborhydrid und 2.56 g (42.65 mmol) Essigsäure versetzt und 5 min bei RT gerührt. Danach wurde eine Lösung von 0.54 g (3.13 mmol) tert-Butyl (3-oxopropyl)carbamat in 5 ml 1,2-Dichlormethan zugegeben und die Reaktionsmischung über Nacht gerührt. Danach wurde vollständig eingedampft, der Rückstand in Essigsäureethylester aufgenommen, filtriert und das produkthaltige Filtrat vollständig eingedampft.

51.26 mg des Rückstandes wurden in 0.8 ml 1,2-Dichlormethan gelöst und zu 7.85 mg (0.1 mmol) Acetylchlorid auf einer 96er deep well Multititerplatte gegeben. Anschließend wurde mit 25.8 mg (0.2 mmol) N,N-Diisopropylethylamin versetzt und bei RT über Nacht geschüttelt. Dann wurde mittels Zentrifugaltrockner das Lösemittel vollständig entfernt und der Rückstand mit 0.4 ml 1,2-Dichlorethan und 0.4 ml Trifluoressigsäure versetzt und über Nacht geschüttelt. Dann wurde mittels Zentrifugaltrockner das Lösemittel vollständig entfernt und der Rückstand mit 0.8 ml DMF versetzt. Anschließend wurde filtriert und aus dem Filtrat die Zielverbindung per präparativer LC-MS (Methode 9):isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 12.2 mg (27% d. Th.; Reinheit 100%) der Titelverbindung erhalten.
LC-MS (Methode 10): Rₜ = 0.95 min; MS (ESIpos): m/z = 455 [M+H]⁺
In Analogie zu Beispiel 181 wurden die in Tabelle XA gezeigten Beispielverbindungen hergestellt.

**Tabelle XA mit Beispielen 182-185**

| **Beispiel** | **IUPAC-Name / Struktur** | **Analytische Daten** |
|---|---|---|
| | **(Ausbeute)** | |
| **182** | N-(3-Aminopropyl)-N-{(1R)-1-[4-benzyl-1-(2,5-difluorphenyl)-1H-pyrazol-3-yl]-2,2-dimethylpropyl}-2-methoxyacetamid | LC-MS (Methode 10): Rₜ = 0.95 min |
| | 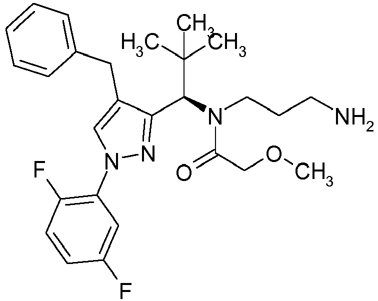 | MS (ESpos): m/z = 485 (M+H)⁺ |
| | (14% d. Th.; Reinheit 98%) | |
| **183** | N-(3-Aminopropyl)-N-{(1R)-1-[4-benzyl-1-(2,5-difluorphenyl)-1H-pyrazol-3-yl]-2,2-dimethylpropyl}-2,4-difluorobenzamid | LC-MS (Methode 10): Rₜ = 1.02 min |
| | 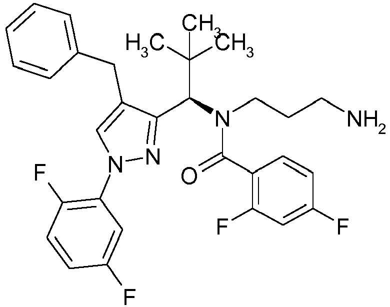 | MS (ESpos): m/z = 553 (M+H)⁺ |
| | (20% d. Th.; Reinheit 100%) | |
| **184** | N-(3-Aminopropyl)-N-{(1R)-1-[4-benzyl-1-(2,5-difluorphenyl)-1H-pyrazol-3-yl]-2,2-dimethylpropyl} -4-methylbenzamid | LC-MS (Methode 10): Rₜ = 1.04 min |
| | 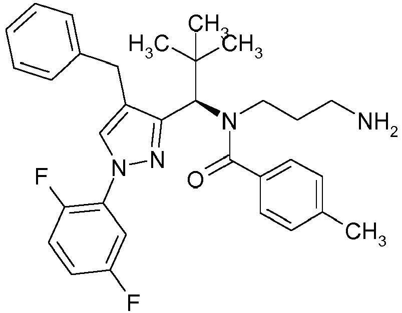 | MS (ESpos): m/z = 531 (M+H)⁺ |
| | (14% d. Th.; Reinheit 100%) | |
| **185** | N-(3-Aminopropyl)-N-{(1R)-1-[4-benzyl-1-(2,5-difluorphenyl)-1H-pyrazol-3-yl]-2,2-dimethylpropyl}-2-ethoxyacetamid | LC-MS (Methode 10): Rₜ = 0.98 min |
| | 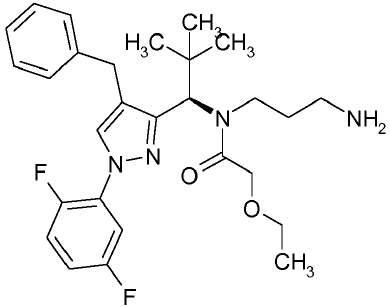 | MS (ESpos): m/z = 499 (M+H)⁺ |
| | (3% d. Th.; Reinheit 83%) | |

### Beispiel 186

### N-(3-Aminopropyl)-N-{(1R)-1-[1-benzyl-4-(2,5-difluorphenyl)-1H-pyrrol-2-yl]-2,2-dimethylpropyl}-3,3,3 -trifluorpropanamid

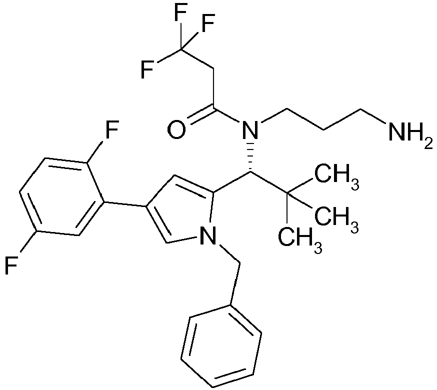

1.0 g (2.82 mmol) (1R)-1-[1-Benzyl-4-(2,5-difluorphenyl)-1H-pyrrol-2-yl]-2,2-dimethylpropan-1-amin wurden in 20 ml 1,2-Dichlormethan vorgelegt und mit 1.4 g (3.95 mmol) Natriumtriacetoxyborhydrid und 2.54 g (42.32 mmol) Essigsäure versetzt und 5 min bei RT gerührt. Danach wurde eine Lösung von 0.54 g (3.10 mmol) tert-Butyl (3-oxopropyl)carbamat in 5 ml 1,2-Dichlormethan zugegeben und die Reaktionsmischung über Nacht gerührt. Danach wurde vollständig eingedampft, der Rückstand in Essigsäureethylester aufgenommen, filtriert und das produkthaltige Filtrat vollständig eingedampft.

51.16 mg des Rückstandes wurden in 0.8 ml 1,2-Dichlormethan gelöst und zu 14.65 mg (0.1 mmol) 3,3,3-Trifluorpropanoylchlorid auf einer 96er deep well Multititerplatte gegeben. Anschließend wurde mit 25.8 mg (0.2 mmol) N,N-Diisopropylethylamin versetzt und bei RT über Nacht geschüttelt. Dann wurde mittels Zentrifugaltrockner das Lösemittel vollständig entfernt und der Rückstand mit 0.4 ml 1,2-Dichlorethan und 0.4 ml Trifluoressigsäure versetzt und über Nacht geschüttelt. Dann wurde mittels Zentrifugaltrockner das Lösemittel vollständig entfernt und der Rückstand mit 0.8 ml DMF versetzt. Anschließend wurde filtriert und aus dem Filtrat die Zielverbindung per präparativer LC-MS (Methode 9) isoliert. Die produkthaltigen Fraktionen wurden mittels Zentrifugaltrockner im Vakuum eingeengt. Der Rückstand der Produktfraktionen wurde in je 0.6 ml DMSO gelöst. Diese wurden vereint und abschließend im Zentrifugaltrockner vom Lösemittel befreit. Es wurden 1.0 mg (2% d. Th.; Reinheit 82%) der Titelverbindung erhalten.
LC-MS (Methode 10): Rₜ = 1.01 min MS (ESIpos): m/z = 522 [M+H]⁺

In Analogie zu Beispiel 186 wurden die in Tabelle XA1 gezeigten Beispielverbindungen hergestellt.

**Tabelle XA1 mit Beispielen 187-191**

| **Beispiel** | **IUPAC-Name / Struktur** | **Analytische Daten** |
|---|---|---|
| | **(Ausbeute)** | |
| **187** | N-(3-Aminopropyl)-N-{(1R)-1-[1-benzyl-4-(2,5-difluorphenyl)-1H-pyrrol-2-yl]-2,2-dimethylpropyl}-4-fluorbenzamid | LC-MS (Methode 10): Rₜ = 1.01 min |
| | 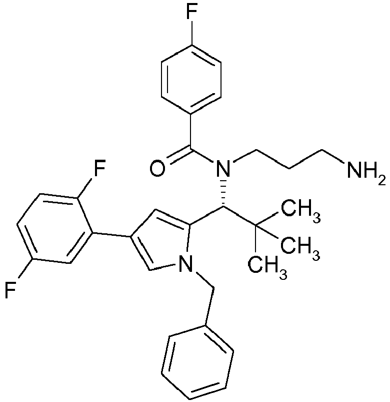 | MS (ESpos): m/z = 534 (M+H)⁺ |
| | (3% d. Th.;Reinheit 83%) | |
| **188** | N-(3-Aminopropyl)-N-{(1R)-1-[1-benzyl-4-(2,5-difluorphenyl)-1H-pyrrol-2-yl]-2,2-dimethylpropyl} acetamid | LC-MS (Methode 10): Rₜ = 0.94 min |
| | 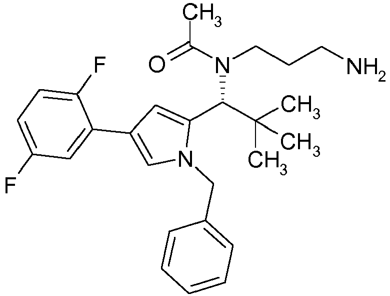 | MS (ESpos): m/z = 454 (M+H)⁺ |
| | (2% d. Th.; Reinheit 87%) | |
| **189** | N-(3-Aminopropyl)-N-{(1R)-1-[1-benzyl-4-(2,5-difluorphenyl)-1H-pyrrol-2-yl]-2,2-dimethylpropyl} -4-(trifluoromethyl) benzamid | LC-MS (Methode 10): Rₜ = 1.05 min |
| | 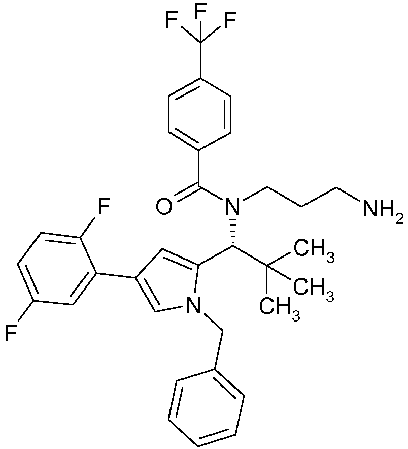 | MS (ESpos): m/z = 584 (M+H)⁺ |
| | (6% d. Th.; Reinheit 90%) | |
| **190** | N-(3-Aminopropyl)-N-{(1R)-1-[4-benzyl-1-(2,5-difluorphenyl)-1H-pyrazol-3-yl]-2,2-dimethylpropyl}-2-ethoxyacetamid | LC-MS (Methode 10): Rₜ = 0.98 min |
| | 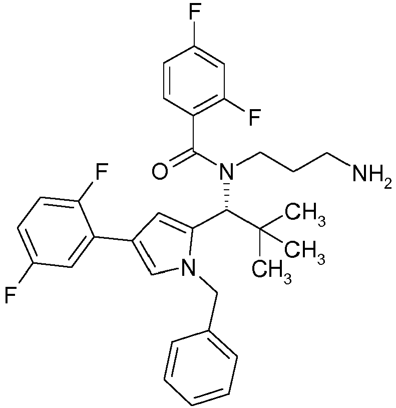 | MS (ESpos): m/z = 552 (M+H)⁺ |
| | (7% d. Th.; Reinheit 83%) | |
| **191** | N-(3-Aminopropyl)-N-{(1R)-1-[4-benzyl-1-(2,5-difluorphenyl)-1H-pyrazol-3-yl]-2,2-dimethylpropyl}-2-ethoxyacetamid | LC-MS (Methode 10): Rₜ = 1.02 min |
| | 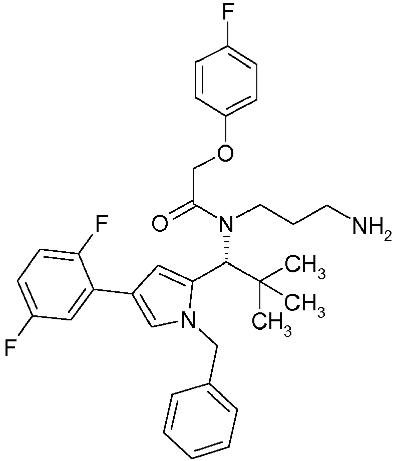 | MS (ESpos): m/z = 564 (M+H)⁺ |
| | (13% d. Th.; Reinheit 98%) | |

### Beispiel 192A

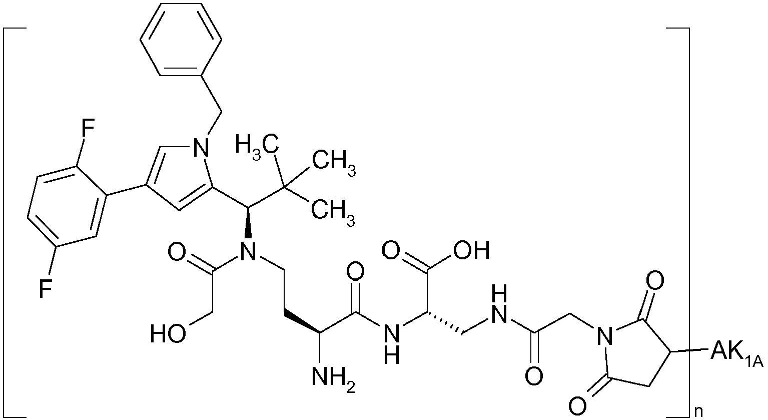

Eingesetzt wurden hier zur Kupplung mit Intermediat F192 5 mg Cetuximab in PBS (c=21.3 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.968 mg/mL
Drug/mAb Ratio: 2.9

### Beispiel 192B

Eingesetzt wurden hier zur Kupplung mit Intermediat F192 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.92 mg/mL
Drug/mAb Ratio: 2.6

### Beispiel 192E

Eingesetzt wurden hier zur Kupplung mit Intermediat F192 5 mg Trastuzumab Antikörper in PBS (c = 13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.04 mg/mL
Drug/mAb Ratio: 3.3

### Beispiel 193A

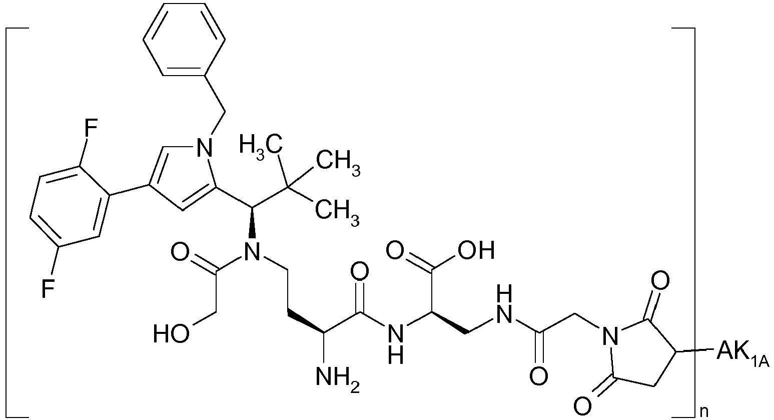

Eingesetzt wurden hier zur Kupplung mit Intermediat F193 5 mg Cetuximab in PBS (c=23.1 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.98 mg/mL
Drug/mAb Ratio: 2.9

### Beispiel 193B

Eingesetzt wurden hier zur Kupplung mit Intermediat F193 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.03 mg/mL
Drug/mAb Ratio: 3.2

### Beispiel 193E

Eingesetzt wurden hier zur Kupplung mit Intermediat F193 5 mg Trastuzumab Antikörper in PBS (c = 13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.61 mg/mL
Drug/mAb Ratio: 3.3

### Beispiel 194A

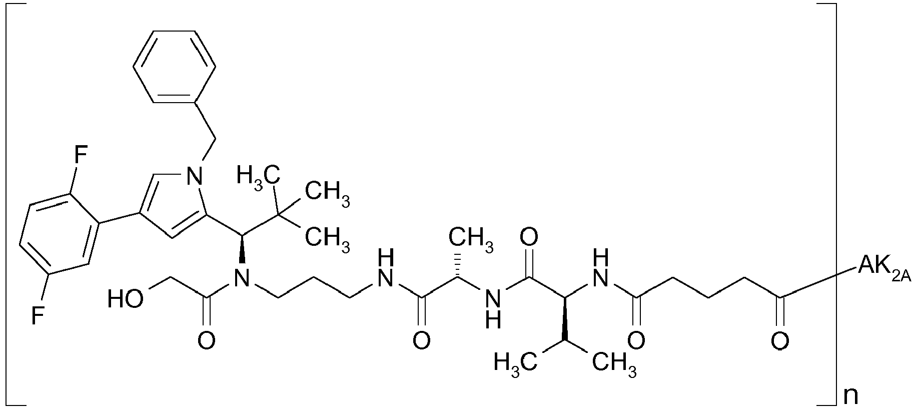

Eingesetzt wurden hier zur Kupplung mit Intermediat F194 5 mg Cetuximab in PBS (c = 23.1 mg/mL) und der Ansatz wurde nach der Sephadex-Reinigung durch Ultrazentrifugation aufkonzentriert und rückverdünnt mit PBS.
Protein Konzentration: 1.67 mg/mL
Drug/mAb Ratio: 1.9

### Beispiel 194B

Eingesetzt wurden hier zur Kupplung mit Intermediat F194 5.0 mg anti-TWEAKR AK-1 Antikörper in PBS (c = 18.6 mg/mL) und der Ansatz wurde nach der Sephadex-Reinigung durch Ultrazentrifugation aufkonzentriert und rückverdünnt mit PBS.
Protein Konzentration: 0.99 mg/mL
Drug/mAb Ratio: 3.8

### Beispiel 194E

Eingesetzt wurden hier zur Kupplung mit Intermediat F194 5.0 mg Trastuzumab Antikörper in PBS (c = 13.5 mg/mL) und der Ansatz wurde nach der Sephadex-Reinigung durch Ultrazentrifugation aufkonzentriert und rückverdünnt mit PBS.
Protein Konzentration: 1.39 mg/mL
Drug/mAb Ratio: 2.4

### Beispiel 195A

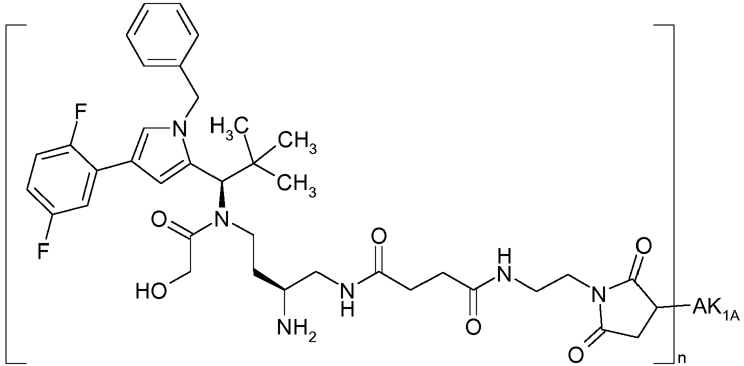

Eingesetzt wurden hier zur Kupplung mit Intermediat F195 5 mg Cetuximab in PBS (c=23.1 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.79 mg/mL
Drug/mAb Ratio: 3.2

### Beispiel 195B

Eingesetzt wurden hier zur Kupplung mit Intermediat F195 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.53 mg/mL
Drug/mAb Ratio: 3.3

### Beispiel 195E

Eingesetzt wurden hier zur Kupplung mit Intermediat F195 5.0 mg Trastuzumab Antikörper in PBS (c = 13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.91 mg/mL
Drug/mAb Ratio: 3.4

### Beispiel 196A

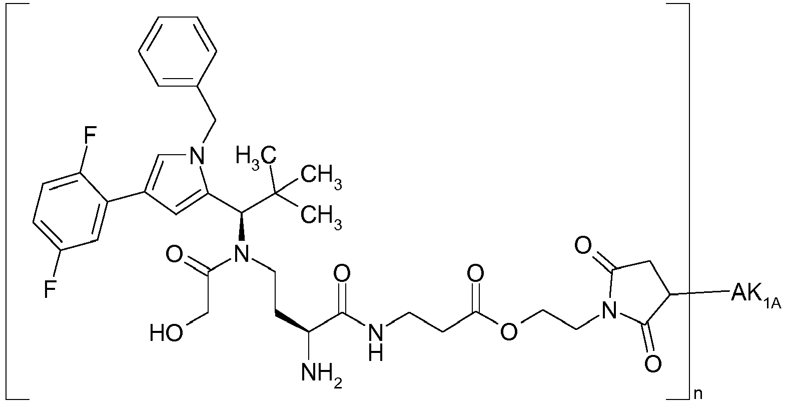

Eingesetzt wurden hier zur Kupplung mit Intermediat F196 5 mg Cetuximab in PBS (c=23.1 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.85 mg/mL
Drug/mAb Ratio: 3.0

### Beispiel 196B

Eingesetzt wurden hier zur Kupplung mit Intermediat F196 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.73 mg/mL
Drug/mAb Ratio: 3.1

### Beispiel 196E

Eingesetzt wurden hier zur Kupplung mit Intermediat F196 5.0 mg Trastuzumab Antikörper in PBS (c = 13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.86 mg/mL
Drug/mAb Ratio: 3.4

### Beispiel 197

### (2S)-2-Amino-4-[{(1R)-1-[1-benzyl-4-(2,5-difluorphenyl)-1H-pyrrol-2-yl]-2,2-dimethylpropyl} (glycoloyl)amino]butansäurehydrochlorid (1:1)

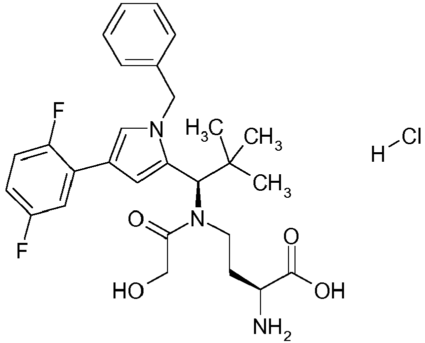

150 mg (0.2 mmol) von Intermediat C53 wurden in 15 mL DMF gelöst und mit 2.29 g (20.39 mmol) DABCO versetzt. Der Ansatz wurde 30 min im Ultraschallbad behandelt. Durch Zugabe von 1.17 ml Essigsäure wurde der Ansatz dann auf pH 3-4 gebracht und im Vakuum eingeengt. Der Rückstand wurde durch präparative HPLC gereinigt und die entsprechenden Fraktionen wurden bei RT im Vakuum eingeengt. Der Rückstand wurde in Acetonitril/Wasser 1:1 aufgenommen, mit 5 mL einer 4N Salzsäure versetzt und anschließend lyophilisiert. So wurden 81 mg (68% d. Th.) der Titelverbindung erhalten.
LC-MS (Methode 5): Rₜ = 2.69 min; MS (EIpos): m/z = 514 [M+H]⁺.

### Beispiel 198

### Trifluoressigsäure --(2S)-2-amino-N-(2-aminoethyl)-4-[{(1R)-1-[1-benzyl-4-(2,5-difluorphenyl)-1H-pyrrol-2-yl]-2,2-dimethylpropyl}(glycoloyl)amino]butanamid (1:1)

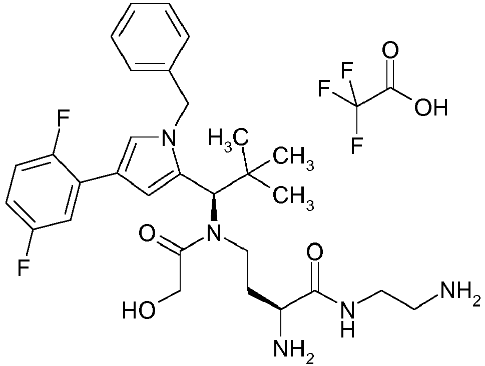

15 mg (0.018 mmol) von Intermediat C64 wurden in 4 mL 2,2,2-Trifluorethanol gelöst. Es wurden 15 mg (0.110 mmol) Zinkchlorid zugegeben und der Ansatz 2 h bei 50°C gerührt. Anschließend wurden 32 mg (0.110 mmol) Ethylendiamin-N,N,N',N'-tetraessigsäure zugesetzt und der Ansatz im Vakuum eingeengt. Der Rückstand wurde mittels präparativer HPLC gereinigt. Nach Einengen der entsprechenden Fraktionen und Lyophilisation des Rückstands aus Acetonitril/Wasser wurden 9.5 mg (77% d.Th.) der Titelverbindung erhalten.
LC-MS (Methode 1): Rₜ = 0.68 min; MS (ESIpos): m/z = 556 (M+H)⁺.

### Beispiel 199

### 4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure-trifluoressigsäure (1:1) und

### 4-[(2-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl}amino)ethyl]amino}-2-oxoethyl)amino] -2- {[(2R)-2-amino-2-carboxyethyl]sulfanyl}-4-oxobutansäure -trifluoressigsäure (1:1)

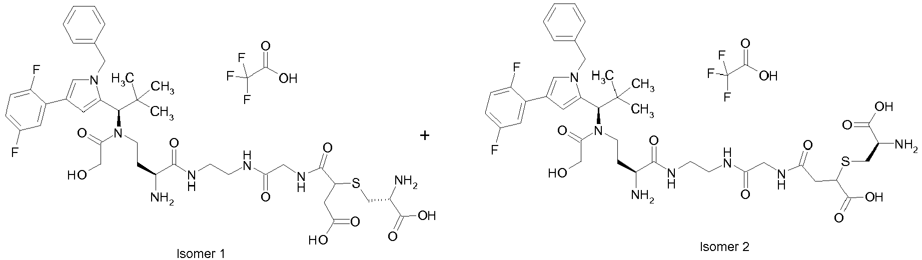

LC-MS (Methode 1): Rₜ = 0.80 min; MS (EIpos): m/z = 814 [M+H]⁺.

Zunächst wurde L-Cystein mit 1-({[2-(Trimethylsilyl)ethoxy]carbonyl}oxy)pyrrolidine-2,5-dion in DMF in Gegenwart von *N,N*-Diisopropylethylamin in N-{[2-(Trimethylsilyl)ethoxy]carbonyl}-L-cystein überführt.

406 mg (1.53 mmol) N-{[2-(Trimethylsilyl)ethoxy]carbonyl}-L-cystein wurden in 10 ml DMF gelöst und mit 157.5 mg (1.606 mmol) Maleinsäureanhydrid versetzt und der Ansatz 1 Stunde bei RT gerührt. 130 µL dieser Lösung wurden mit 7.5 mg (0.01 mmol) von Intermediat C66 versetzt und der Ansatz 5 min bei RT gerührt. Anschließend wurde im Vakuum eingeengt und der Rückstand durch präparative HPLC gereinigt. Das Lösemeittel wurde im Vakuum abgedampft und der Rückstand im Hochvakuum getrocknet. Es wurden 10 mg (89%) des geschützten Intermediats erhalten, wobei weder im HPLC noch im LC-MS die Regioisomere aufgetrennt werden konnten.
LC-MS (Methode 1): Rₜ = 1.38 min; MS (EIpos): m/z = 1120 [M+H]⁺.

Im letzten Schritt wurden die 10 mg von diesem Intermediat in 2 mL 2,2,2-Trifluorethanol gelöst. Es wurden 12 mg (0.088 mmol) Zinkchlorid zugegeben und der Ansatz 30 min bei 50°C gerührt. Anschließend wurden 26 mg (0.088 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 8.3 mg (99% d. Th.) der Titelverbindung als Regioisomerengemisch imVerhältnis 87:13 erhalten.
LC-MS (Methode 5): Rₜ = 2.3 min und 2.43 min; MS (ESIpos): m/z = 832 (M+H)⁺.
¹H-NMR Hauptregioisomer: (500 MHz, DMSO-d₆): δ = 8.7 (m, 1H), 8.5 (m, 2H), 8.1 (m, 1H), 7.6 (m, 1H), 7.5 (s, 1H) 7.4-7.15 (m, 6H), 6.9-7.0 (m, 1H), 6.85 (s, 1H), 5.61 (s, 1H), 4.9 und 5.2 (2d, 2H), 4.26 und 4.06 (2d, 2H), 3.5-3.8 (m, 5H), 3.0-3.4 (m, 5H), 2.75-3.0 (m, 3H), 2.58 und 2.57 (dd, 1H), 0.77 und 1,5 (2m, 2H), 0.81 (s, 9H).

Alternativ wurden die regioisomeren Titelverbindungen wie folgt hergestellt:
Zunächst wurde dazu L-Cystein mit 1-({[2-(Trimethylsilyl)ethoxy]carbonyl}oxy)pyrrolidine-2,5-dion in DMF in Gegenwart von *N,N*-Diisopropylethylamin in N-{[2-(Trimethylsilyl)ethoxy]carbonyl}-L-cystein überführt.

55 mg (0.068 mmol) von Intermediat F104 und 36 mg (0.136 mmol) N-{[2-(Trimethylsilyl) ethoxy]carbonyl}-L-cystein 15 ml DMF gelöst und der Ansatz 20 h bei RT gerührt. Anschließend wurde eingeengt und der Rückstand durch präparative HPLC gereinigt. Nach Vereinigung der entsprechenden Fraktionen und Abdampfen der Lösemittel im Vakuum wurde der Rückstand in 15 mL THF/Wasser 1:1 gelöst. Es wurden 131 µL einer 2M wässrigen Lithiumhydroxidlösung zugegeben und der Ansatz 1 h bei RT gerührt. Anschließend wurde der Ansatz mit einer 1M Salzsäure neutral gestellt, das Lösemittel im Vakuum abgedampft und der Rückstand durch präparative HPLC gereinigt. Es wurden 37 mg (50% d. Th.) der regioisomeren geschützten Intermediate als farbloser Schaum erhalten.
LC-MS (Methode 5): Rₜ = 3.33 min und 3.36 min; MS (ESIpos): m/z = 976 (M+H)⁺.

Im letzten Schritt wurden 25 mg (0.023 mmol) von diesem Intermediat in 3 mL 2,2,2-Trifluorethanol gelöst. Es wurden 12.5 mg (0.092 mmol) Zinkchlorid zugegeben und der Ansatz 4 h bei 50°C gerührt. Anschließend wurden 27 mg (0.092 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 18.5 mg (85% d. Th.) der Titelverbindung als Regioisomerengemisch imVerhältnis 21:79 erhalten.
LC-MS (Methode 5): Rₜ = 2.37 min und 3.44 min; MS (ESIpos): m/z = 832 (M+H)⁺.

Die gezielte Herstellung der einzelnen Regioisomere der Titelverbindungen erfolgte folgendermaßen:

### Beispiel 199-2

### 4-[(2-{[2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl}amino)ethyl]amino}-2-oxoethyl)amino] -2- {[(2R)-2-amino-2-carboxyethyl]sulfanyl} -4-oxobutansäure -trifluoressigsäure (1:1)

Zunächst wurde dazu Methyl-L-Cysteinat mit 1-({[2-(Trimethylsilyl)ethoxy]carbonyl}oxy)pyrrolidine-2,5-dion in DMF in Gegenwart von *N,N*-Diisopropylethylamin in Methyl-N-{[2-(trimethylsilyl)ethoxy]carbonyl}-L-cysteinat überführt.

53 mg (0.251 mmol) von kommerziell erhältlicher 3-Brom-4-methoxy-4-oxobutansäure und 70 mg (0.251 mmol) Methyl-N-{[2-(trimethylsilyl)ethoxy]carbonyl}-L-cysteinat wurden in 5 ml DMF gelöst und unter pH Kontrolle mit wässriger Natriumhydrogencarbonatlösung versetzt. Nach 15 min Rühren bei RT wurde mit Essigsäure ein pH Wert von 4.3 eingestellt und der Ansatz eingeengt. Der Rückstand durch präparative HPLC gereinigt. Nach Vereinigung der entsprechenden Fraktionen und Abdampfen der Lösemittel im Vakuum wurde 72 mg (70% d. Th.) 4-Methoxy-3-{[(2R)-3-methoxy-3-oxo-2-({[2-(trimethylsilyl)ethoxy]carbonyl} amino)propyl] sulfanyl} -4-oxobutansäure erhalten.
LC-MS (Methode 1): Rₜ = 0.93 min; MS (ESIpos): m/z = 410 (M+H)⁺.

Dieses Intermediat wurde in Gegenwart von HATU mit Intermediat C66 gekuppelt und anschließend zunächst mit Lithiumhydroxid in Methanol und anschließend mit Zinkchlorid wie oben beschrieben vollständig entschützt. Der Rückstand wurde mittels präparativer HPLC gereinigt. Nach Einengen der entsprechenden Fraktionen und Lyophilisation des Rückstands aus Acetonitril/Wasser wurden 2mg der Titelverbindung erhalten.
LC-MS (Methode 1): Rₜ = 0.78 min; MS (ESIpos): m/z = 832 (M+H)⁺.

In analoger Weise kann das Isomer 1 hergestellt werden.

### Beispiel 200

### N- {(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} -beta-alanin -trifluoressigsäure (1:1)

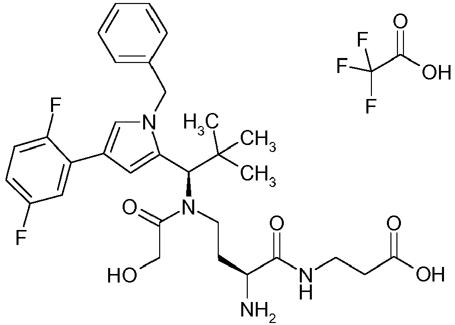

10 mg (0.014 mmol) Intermediat C61 in 3 mL 2,2,2-Trifluorethanol gelöst. Es wurden 11 mg (0.082 mmol) Zinkchlorid zugegeben und der Ansatz 30 min bei 50°C gerührt. Anschließend wurden 24 mg (0.082 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 4.2 mg (40% d. Th.) der Titelverbindung erhalten.
LC-MS (Methode 1): Rₜ = 0.79 min; MS (ESIpos): m/z = 585 (M+H)⁺.

### Beispiel 201

### N- {(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-L-serin -trifluoressigsäure (1:1)

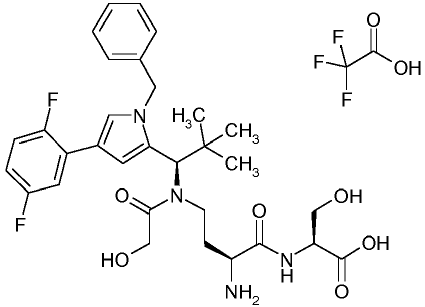

20 mg (0.03 mmol) von Intermediat C58 sowie 8.5 mg (0.037 mmol) Benzyl L-serinate hydrochloride (1:1) wurden in 5 mL DMF aufgenommen und mit 17 mg (0.046 mmol) HATU und 21 µl N,N-Diisopropylethylamin versetzt. Nach 10 min Rühren bei RT wurde eingeengt und der Rückstand durch präparative HPLC gereinigt. Es wurden 12.5 mg (49% d. Th.) der Zwischenstufe erhalten. LC-MS (Methode 1): Rₜ = 1.42 min; MS (ESIpos): m/z = 835 (M+H)⁺.

12.5 mg (0.015 mmol) dieser Zwischenstufe wurden in 10 ml Ethanol gelöst, mit Palladium auf Kohle (10%ig) versetzt und bei RT mit Wasserstoff unter Normaldruck 30 min hydriert. Der Katalysator wurde abfiltriert, die Lösemittel im Vakuum verdampft und nach Lyophilisation des Rückstandes aus Acetonitril/Wasser wurden 7.5 mg (67% d. Th.) der Zwischenstufe erhalten.
LC-MS (Methode 1): Rₜ = 1.28 min; MS (ESIpos): m/z = 745 (M+H)⁺.

7.5 mg (0.01 mmol) dieses Intermediats wurden in 3 mL 2,2,2-Trifluorethanol gelöst. Es wurden 8 mg (0.06 mmol) Zinkchlorid zugegeben und der Ansatz 4.5 h bei 50°C gerührt. Anschließend wurden 17.7 mg (0.06 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 4.2 mg (58% d. Th.) der Titelverbindung erhalten.
LC-MS (Methode 1): Rₜ = 0.75 min; MS (ESIpos): m/z = 601 (M+H)⁺.

### Beispiel 202

### N- {(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-L-alanin -trifluoressigsäure (1:1)

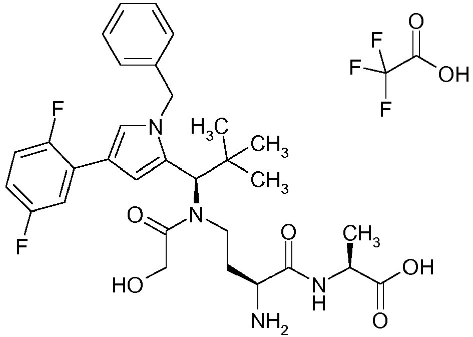

Die Herstellung der Titelverbindung erfolgte in Analogie zu Beispiel 201 ausgehend von Intermediat C58 und Benzyl L-alaninate hydrochloride (1:1).
LC-MS (Methode 1): Rₜ = 0.82 min; MS (ESIpos): m/z = 585 (M+H)⁺.

### Beispiel 203

### N- {(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}glycin -trifluoressigsäure (1:1)

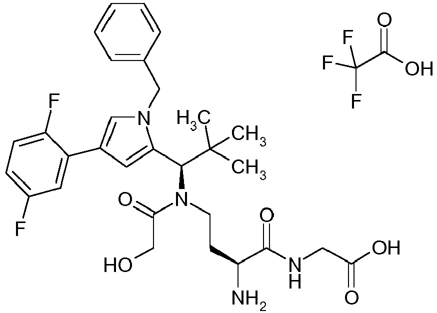

Die Herstellung der Titelverbindung erfolgte in Analogie zu Beispiel 201 ausgehend von Intermediat C58 und Benzylglycinathydrochlorid (1:1).
LC-MS (Methode 1): Rₜ = 0.82 min; MS (ESIpos): m/z = 571 (M+H)⁺.

### Beispiel 204A

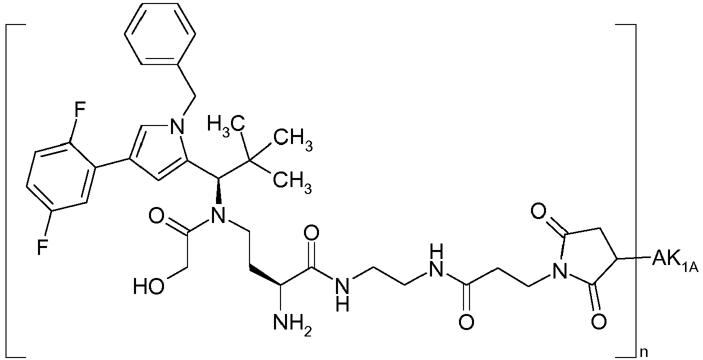

Eingesetzt wurden hier zur Kupplung mit Intermediat F204 5 mg Cetuximab in PBS (c=23.1 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.88 mg/mL
Drug/mAb Ratio: 2.6

### Beispiel 204B

Eingesetzt wurden hier zur Kupplung mit Intermediat F204 50 mg anti-TWEAKR AK-1 in PBS (c=18.6 mg/mL) und der Ansatz wurde nach der Sephadex-Reinigung durch Ultrazentrifugation aufkonzentriert, rückverdünnt mit PBS und erneut aufkonzentriert. Das ADC kann auch teilweise in Form der hydrolysierten offenkettigen Bernsteinsäureamide mit dem Antikörper verknüpft vorliegen.
Protein Konzentration: 12.66 mg/mL
Drug/mAb Ratio: 3.5

### Beispiel 204E

Eingesetzt wurden hier zur Kupplung mit Intermediat F204 5.0 mg Trastuzumab Antikörper in PBS (c = 13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.65 mg/mL
Drug/mAb Ratio: 3.5

### Beispiel 205A

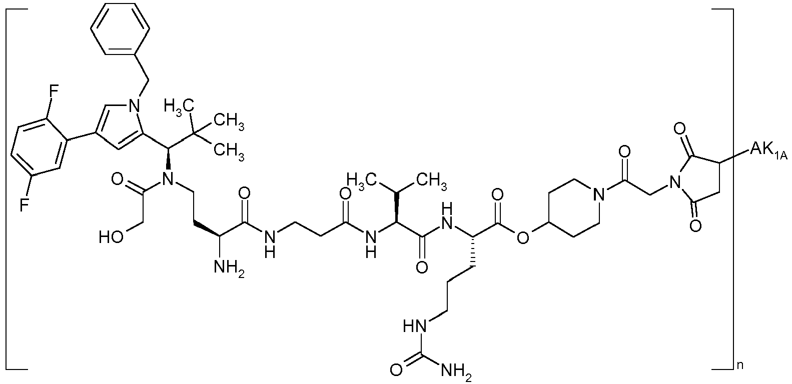

Eingesetzt wurden hier zur Kupplung mit Intermediat F205 5 mg Cetuximab in PBS (c=23.1 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.99 mg/mL
Drug/mAb Ratio: 3.2

### Beispiel 205B

Eingesetzt wurden hier zur Kupplung mit Intermediat F205 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 0.96 mg/mL
Drug/mAb Ratio: 2.6

### Beispiel 205E

Eingesetzt wurden hier zur Kupplung mit Intermediat F205 5 mg Trastuzumab in PBS (c=13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.94 mg/mL
Drug/mAb Ratio: 3.6

### Beispiel 206A

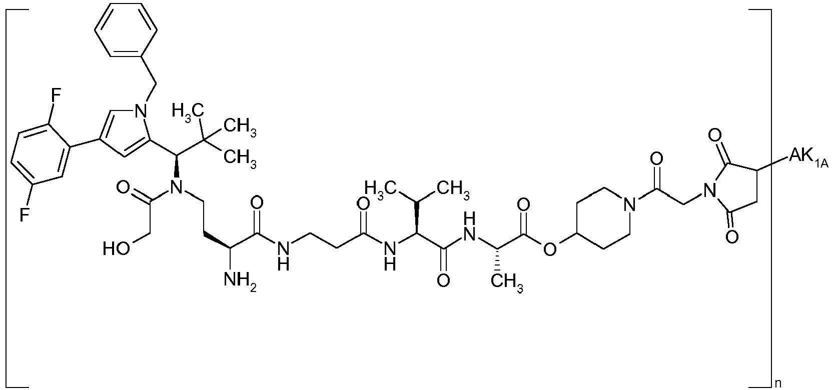

Eingesetzt wurden hier zur Kupplung mit Intermediat F206 5 mg Cetuximab in PBS (c=23.1 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.73 mg/mL
Drug/mAb Ratio: 2.6

### Beispiel 206B

Eingesetzt wurden hier zur Kupplung mit Intermediat F206 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.21 mg/mL
Drug/mAb Ratio: 2.0

### Beispiel 206E

Eingesetzt wurden hier zur Kupplung mit Intermediat F206 5 mg Trastuzumab in PBS (c=13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.84 mg/mL
Drug/mAb Ratio: 2.8

### Beispiel 207A

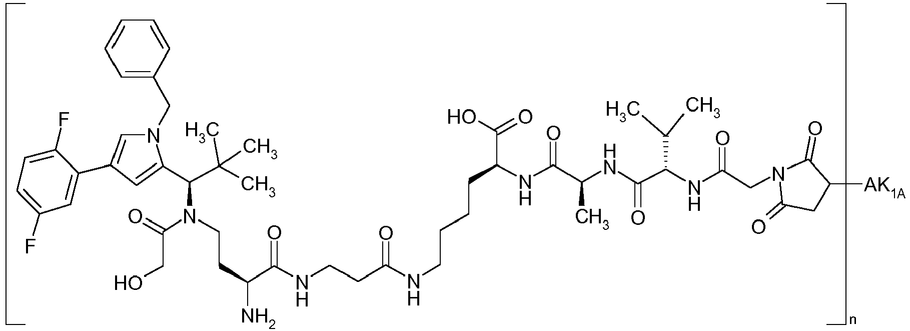

Eingesetzt wurden hier zur Kupplung mit Intermediat F207 5 mg Cetuximab in PBS (c=10 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 1.79 mg/mL
Drug/mAb Ratio: 3.4

### Beispiel 207B

Eingesetzt wurden hier zur Kupplung mit Intermediat F207 50 mg anti-TWEAKR AK-1 in PBS (c=18.6 mg/mL) und der Ansatz wurde nach der Sephadex-Reinigung durch Ultrazentrifugation aufkonzentriert, rückverdünnt mit PBS und erneut aufkonzentriert. Das ADC kann auch teilweise in Form der hydrolysierten offenkettigen Bernsteinsäureamide mit dem Antikörper verknüpft vorliegen. Bei dieser ADC-Präparation wurde unmittelbar nach der Synthese ein Anteil der ring-geöffneten Bernsteinsäureamid-Form von 19% ermittelt.
Protein Konzentration: 12.99 mg/mL
Drug/mAb Ratio: 4.3

### Beispiel 207E

Eingesetzt wurden hier zur Kupplung mit Intermediat F207 5 mg Trastuzumab in PBS (c=13.5 mg/mL) und der Ansatz wurde nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.39 mg/mL
Drug/mAb Ratio: 2.8

### Beispiel 2071

Eingesetzt wurden hier zur Kupplung mit Intermediat F207 5.0 mg Nimotuzumab in PBS (c = 13.1 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.92 mg/mL
Drug/mAb Ratio: 3.5

### Beispiel 207H

Eingesetzt wurden hier zur Kupplung mit Intermediat F207 5.0 mg Panitumumab in PBS (c = 13.6 mg/mL). Die Reduktionszeit mit TCEP betrug 4 h und die Rührzeit bei der ADC Kupplung betrug 20 h. Anschließend wurde der Ansatz nach der Sephadex-Reinigung durch Ultrazentrifugation aufkonzentriert und rückverdünnt mit PBS. Das ADC kann auch teilweise in Form der hydrolysierten offenkettigen Bernsteinsäureamide mit dem Antikörper verknüpft vorliegen.
Protein Konzentration: 2.0 mg/mL
Drug/mAb Ratio: 1.9

### Beispiel 208A

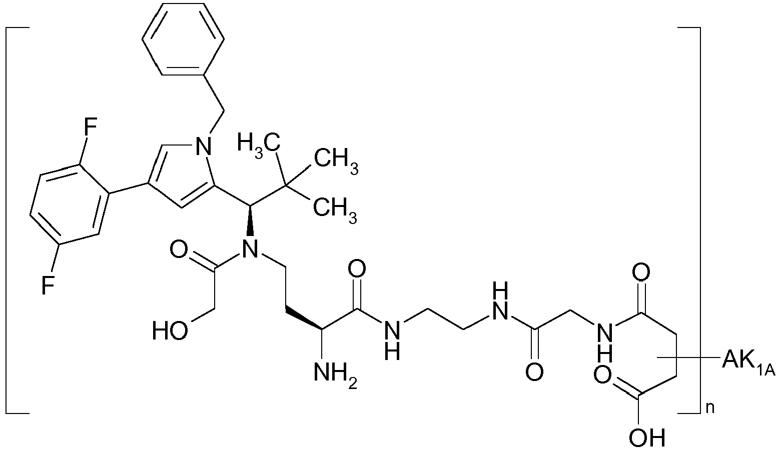

60 mg Cetuximab in 5494 µL ml PBS (c=10.92 mg/mL) wurden unter Argon mit einer Lösung aus 0.344 mg TCEP in 100µl PBS-Puffer versetzt. Der Ansatz wurde 30min bei RT gerührt und anschließend wurden 2.582 mg (0.003 mmol) von Intermediat F104 gelöst in 600µl DMSO zugegeben. Nach weiteren 120 min Rühren bei RT wurde der Ansatz mit 1306 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde mit PBS-Puffer pH8 auf ein Gesamtvolumen von 14 ml verdünnt. Diese Lösung wurde über Nacht bei RT unter Argon gerührt und anschließend durch Ultrazentrifugation aufkonzentriert, rückverdünnt mit PBS-Puffer (pH 7.2) und erneut aufkonzentriert. Der erhaltene ADC batch wurde folgendermaßen charakterisiert:
Protein Konzentration: 13.36 mg/mL
Drug/mAb Ratio: 1.8

Bei dieser ADC-Präparation wurde ein Anteil der ring-geöffneten Bernsteinsäureamid-Form von 94% ermittelt.

### Beispiel 208B

60 mg anti-TWEAKR AK-1 in 3225 µL ml PBS (c=18.6 mg/mL) wurden unter Argon mit 775 µL PBS-Puffer verdünnt und dann mit einer Lösung aus 0.344 mg TCEP in 100µl PBS-Puffer versetzt. Der Ansatz wurde 30min bei RT gerührt und anschließend wurden 2.582 mg (0.003 mmol) von Intermediat F104 gelöst in 600µl DMSO zugegeben. Nach weiteren 120 min Rühren bei RT wurde der Ansatz mit 300 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde mit PBS-Puffer pH8 auf ein Gesamtvolumen von 14 ml verdünnt. Diese Lösung wurde über Nacht bei RT unter Argon gerührt und anschließend durch Ultrazentrifugation aufkonzentriert, rückverdünnt mit PBS-Puffer (pH 7.2) und erneut aufkonzentriert. Der erhaltene ADC batch wurde folgendermaßen charakterisiert:
Protein Konzentration: 14.95 mg/mL
Drug/mAb Ratio: 3.2

### Beispiel 2081

60 mg Nimotuzumab in 4587 µL ml PBS (c=13.1 mg/mL) wurden unter Argon mit einer Lösung aus 0.344 mg TCEP in 100 µl PBS-Puffer versetzt. Der Ansatz wurde 30min bei RT gerührt und anschließend wurden 2.582 mg (0.003 mmol) von Intermediat F104 gelöst in 600µl DMSO zugegeben. Nach weiteren 120 min Rühren bei RT wurde der Ansatz mit 2213 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde mit PBS-Puffer pH8 auf ein Gesamtvolumen von 14 ml verdünnt. Diese Lösung wurde über Nacht bei RT unter Argon gerührt und anschließend durch Ultrazentrifugation aufkonzentriert, rückverdünnt mit PBS-Puffer (pH 7.2) und erneut aufkonzentriert. Der erhaltene ADC batch wurde folgendermaßen charakterisiert:
Protein Konzentration: 14.79 mg/mL
Drug/mAb Ratio: 3.1

Bei dieser ADC-Präparation wurde ein Anteil der ring-geöffneten Bernsteinsäureamid-Form von 91% ermittelt.

### Beispiel 208K

40 mg anti-TWEAKR AK-2 in 2759 µL ml PBS (c=14.5 mg/mL) wurden unter Argon mit einer Lösung aus 0.23 mg TCEP in 67µl PBS-Puffer versetzt. Der Ansatz wurde 30min bei RT gerührt und anschließend wurden 1.72 mg (0.002 mmol) von Intermediat F104 gelöst in 400 µl DMSO zugegeben. Nach weiteren 120 min Rühren bei RT wurde der Ansatz mit 1774 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde mit PBS-Puffer pH8 auf ein Gesamtvolumen von 14 ml verdünnt. Diese Lösung wurde über Nacht bei RT unter Argon gerührt und anschließend durch Ultrazentrifugation aufkonzentriert, rückverdünnt mit PBS-Puffer (pH 7.2) und erneut aufkonzentriert. Der erhaltene ADC batch wurde folgendermaßen charakterisiert:
Protein Konzentration: 11.66 mg/mL
Drug/mAb Ratio: 3.1

### Beispiel 209A

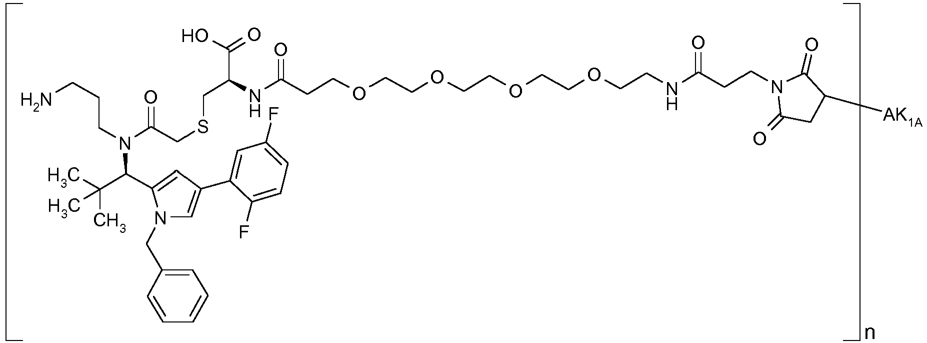

Eingesetzt wurden hier zur Kupplung mit Intermediat F209 5.0 mg Cetuximab in PBS (c = 21.32 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.75 mg/mL
Drug/mAb Ratio: 2.4

### Beispiel 209B

Eingesetzt wurden hier zur Kupplung mit Intermediat F209 5.0 mg anti-TWEAKR AK-1 Antikörper in PBS (c = 18.60 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.30 mg/mL
Drug/mAb Ratio: 2.1

### Beispiel 209E

Eingesetzt wurden hier zur Kupplung mit Intermediat F209 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 2.03 mg/mL
Drug/mAb Ratio: 2.3

### Beispiel 209H

Eingesetzt wurden hier zur Kupplung mit Intermediat F209 5.0 mg Panitumumab Antikörper in PBS (c = 70.5 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.91 mg/mL
Drug/mAb Ratio: 1.5

### Beispiel 2091

Eingesetzt wurden hier zur Kupplung mit Intermediat F209 5.0 mg Nimotuzumab Antikörper in PBS (c = 13.1 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.81 mg/mL
Drug/mAb Ratio: 2.5

### Beispiel 210A

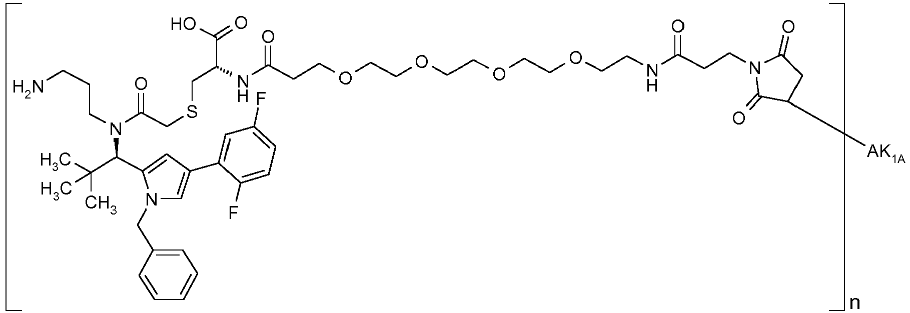

Eingesetzt wurden hier zur Kupplung mit Intermediat F210 5.0 mg Cetuximab in PBS (c = 21.32 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.92 mg/mL
Drug/mAb Ratio: 2.9

### Beispiel 210B

Eingesetzt wurden hier zur Kupplung mit Intermediat F210 5.0 mg anti-TWEAKR AK-1 Antikörper in PBS (c = 18.60 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.41 mg/mL
Drug/mAb Ratio: 2.5

### Beispiel 210E

Eingesetzt wurden hier zur Kupplung mit Intermediat F210 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.79 mg/mL
Drug/mAb Ratio: 2.8

### Beispiel 211A

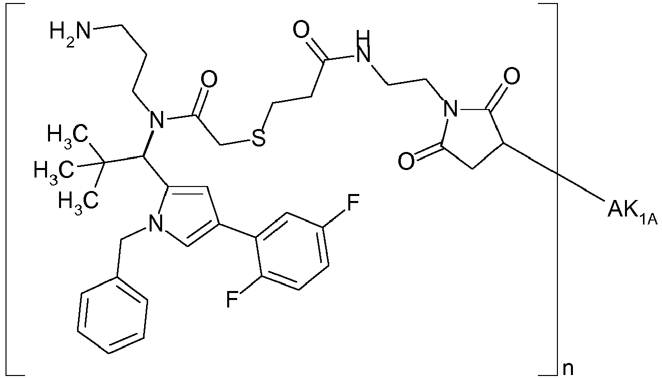

Eingesetzt wurden hier zur Kupplung mit Intermediat F211 5.0 mg Cetuximab in PBS (c = 12.33 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.82 mg/mL
Drug/mAb Ratio: 2.0

### Beispiel 211B

Eingesetzt wurden hier zur Kupplung mit Intermediat F211 5.0 mg anti-TWEAKR AK-1 Antikörper in PBS (c = 34.42 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.52 mg/mL
Drug/mAb Ratio: 2.4

### Beispiel 211E

Eingesetzt wurden hier zur Kupplung mit Intermediat F211 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.84 mg/mL
Drug/mAb Ratio: 2.4

### Beispiel 212A

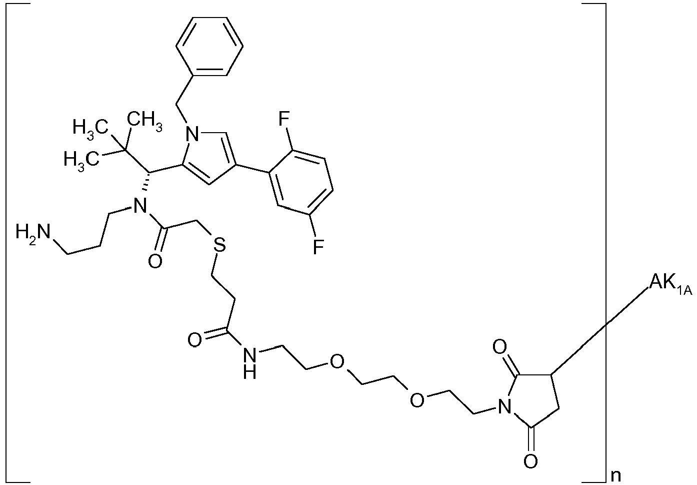

Eingesetzt wurden hier zur Kupplung mit Intermediat F212 5.0 mg Cetuximab in PBS (c = 11.3 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.94 mg/mL
Drug/mAb Ratio: 3.3

### Beispiel 212B

Eingesetzt wurden hier zur Kupplung mit Intermediat F212 5.0 mg anti-TWEAKR AK-1 Antikörper in PBS (c = 18.6 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 0.85 mg/mL
Drug/mAb Ratio: 2.0

### Beispiel 212E

Eingesetzt wurden hier zur Kupplung mit Intermediat F212 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.55 mg/mL
Drug/mAb Ratio: 2.0

### Beispiel 213A

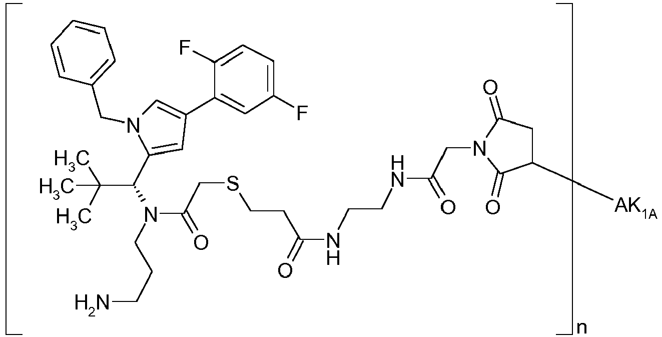

Eingesetzt wurden hier zur Kupplung mit Intermediat F213 5.0 mg Cetuximab in PBS (c = 21.32 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.83 mg/mL
Drug/mAb Ratio: 2.4

### Beispiel 213B

Eingesetzt wurden hier zur Kupplung mit Intermediat F213 5.0 mg anti-TWEAKR AK-1 Antikörper in PBS (c = 18.6 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.4 mg/mL
Drug/mAb Ratio: 2.3

### Beispiel 213E

Eingesetzt wurden hier zur Kupplung mit Intermediat F213 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.94 mg/mL
Drug/mAb Ratio: 2.5

### Beispiel 214A

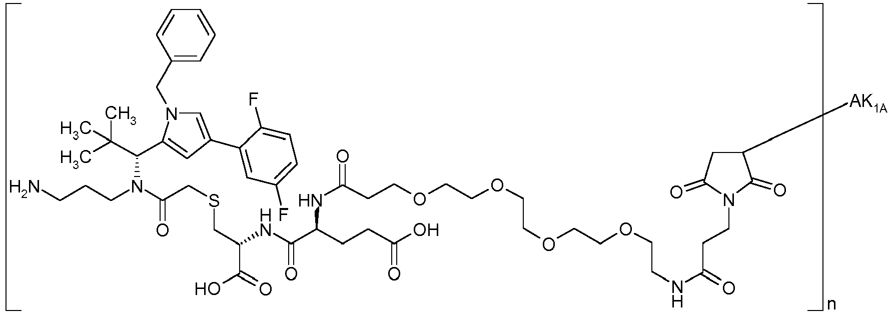

Eingesetzt wurden hier zur Kupplung mit Intermediat F214 5.0 mg Cetuximab in PBS (c = 15.21 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 2.00 mg/mL
Drug/mAb Ratio: 2.6

### Beispiel 214B

Eingesetzt wurden hier zur Kupplung mit Intermediat F214 5.0 mg anti-TWEAKR AK-1 Antikörper in PBS (c = 18.6 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 2.01 mg/mL
Drug/mAb Ratio: 2.6

### Beispiel 214E

Eingesetzt wurden hier zur Kupplung mit Intermediat F214 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.86 mg/mL
Drug/mAb Ratio: 2.7

### Beispiel 215A

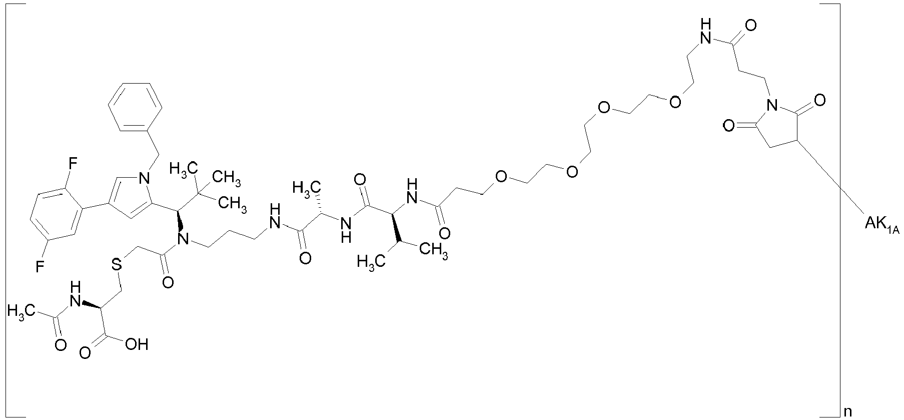

Eingesetzt wurden hier zur Kupplung mit Intermediat F215 5.0 mg Cetuximab in PBS (c = 15.21 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.99 mg/mL
Drug/mAb Ratio: 2.7

### Beispiel 215B

Eingesetzt wurden hier zur Kupplung mit Intermediat F215 5.0 mg anti-TWEAKR AK-1 Antikörper in PBS (c = 18.6 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.64 mg/mL
Drug/mAb Ratio: 2.8

### Beispiel 215E

Eingesetzt wurden hier zur Kupplung mit Intermediat F215 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.84 mg/mL
Drug/mAb Ratio: 2.8

### Beispiel 215H

Eingesetzt wurden hier zur Kupplung mit Intermediat F215 5.0 mg Panitumumab Antikörper in PBS (c = 70.5 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.86 mg/mL
Drug/mAb Ratio: 1.4

### Beispiel 2151

Eingesetzt wurden hier zur Kupplung mit Intermediat F215 5.0 mg Nimotuzumab in PBS (c = 13.1 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.83 mg/mL
Drug/mAb Ratio: 2.6

### Beispiel 216A

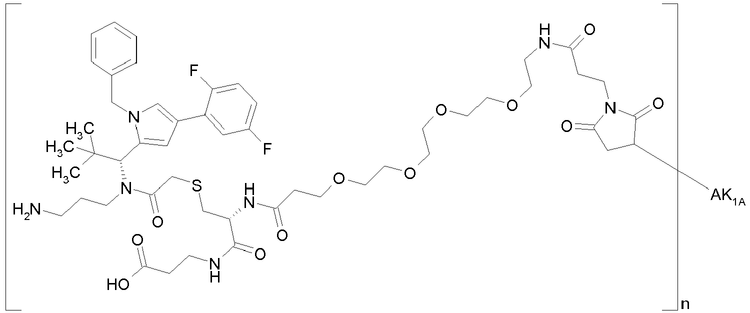

Eingesetzt wurden hier zur Kupplung mit Intermediat F216 5.0 mg Cetuximab in PBS (c = 15.21 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.97 mg/mL
Drug/mAb Ratio: 2.8

### Beispiel 216B

Eingesetzt wurden hier zur Kupplung mit Intermediat F216 5.0 mg anti-TWEAKR AK-1 Antikörper in PBS (c = 18.6 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.94 mg/mL
Drug/mAb Ratio: 2.5

### Beispiel 216E

Eingesetzt wurden hier zur Kupplung mit Intermediat F216 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.90 mg/mL
Drug/mAb Ratio: 1.7

### Beispiel 217A

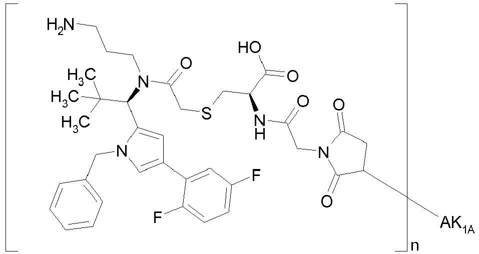

Eingesetzt wurden hier zur Kupplung mit Intermediat F217 5.0 mg Cetuximab in PBS (c = 15.21 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 2.05 mg/mL
Drug/mAb Ratio: 2.7

### Beispiel 217B

Eingesetzt wurden hier zur Kupplung mit Intermediat F217 5.0 mg anti-TWEAKR AK-1 Antikörper in PBS (c = 18.6 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.44 mg/mL
Drug/mAb Ratio: 2.4

### Beispiel 217E

Eingesetzt wurden hier zur Kupplung mit Intermediat F217 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.85 mg/mL
Drug/mAb Ratio: 2.8

### Beispiel 218A

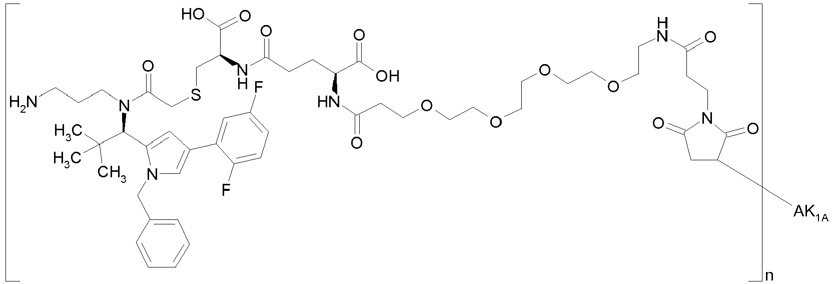

Eingesetzt wurden hier zur Kupplung mit Intermediat F218 5.0 mg Cetuximab in PBS (c = 15.21 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 2.05 mg/mL
Drug/mAb Ratio: 3.0

### Beispiel 218B

Eingesetzt wurden hier zur Kupplung mit Intermediat F218 5.0 mg anti-TWEAKR AK-1 Antikörper in PBS (c = 18.6 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.95 mg/mL
Drug/mAb Ratio: 2.9

### Beispiel 218E

Eingesetzt wurden hier zur Kupplung mit Intermediat F218 5.0 mg Trastuzumab Antikörper in PBS (c = 13.50 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.84 mg/mL
Drug/mAb Ratio: 2.7

### Beispiel 218H

Eingesetzt wurden hier zur Kupplung mit Intermediat F218 5.0 mg Panitumumab Antikörper in PBS (c = 20 mg/mL). Die Reduktionszeit mit TCEP wurde auf 4 h erhöht und die Rührzeit bei der ADC Kupplung auf 20 h erhöht. Der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.33 mg/mL
Drug/mAb Ratio: 0.8

### Beispiel 2181

Eingesetzt wurden hier zur Kupplung mit Intermediat F218 5.0 mg Nimotuzumab Antikörper in PBS (c = 13.8 mg/mL) und der Ansatz wurde nach der Sephadex-Reinigung durch Ultrazentrifugation aufkonzentriert und rückverdünnt. Das ADC kann auch teilweise in Form der hydrolysierten offenkettigen Bernsteinsäureamide mit dem Antikörper verknüpft vorliegen.
Protein Konzentration: 1.48 mg/mL
Drug/mAb Ratio: 3.0

### Beispiel 219

### Trifluoressigsäure--N-(3-aminopropyl)-N-{(1R)-1-[1-benzyl-4-(2,5-difluorphenyl)-1H-pyrrol-2-yl]-2,2-dimethylpropyl} -4-methylbenzamid (1:1)

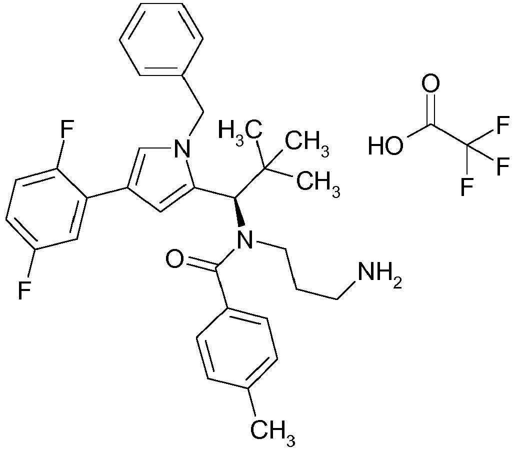

70.0 mg (0.09 mmol) 9H-Fluoren-9-ylmethyl-[3-({(1R)-1-[1-benzyl-4-(2,5-difluorphenyl)-1H-pyrrol-2-yl]-2,2-dimethylpropyl}amino)propyl]carbamat (Intermediat C67) wurden in 3.0 mL Dichlormethan vorgelegt mit 31.3 mg (0.31 mmol) Triethylamin und 31.8 mg (0.21 mmol) 4-Methylbenzoylchlorid versetzt. Die Reaktionsmischung wurde über Nacht bei RT gerührt. Das Lösemittel wurde im Vakuum verdampft und der Rückstand wurde direkt mittles präp. RP-HPLC gereinigt (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 33.4 mg (48 % d. Th.) der Verbindung 9H-Fluoren-9-ylmethyl-{3-[{(1R)-1-[1-benzyl-4-(2,5-difluorphenyl)-1H-pyrrol-2-yl]-2,2-dimethylpropyl}(4-methylbenzoyl)amino]propyl}carbamat.
LC-MS (Methode 2): Rₜ = 11.91 min; MS (ESIpos): m/z = 774 (M+Na)⁺.

33.0 mg (0.04 mmol) 9H-Fluoren-9-ylmethyl-{3-[{(1R)-1-[1-benzyl-4-(2,5-difluorphenyl)-1H-pyrrol-2-yl]-2,2-dimethylpropyl}(4-methylbenzoyl)amino]propyl}carbamat wurden über Nacht in 1.0 mL DMF mit 20.0 mg (0.23 mmol) Morpholin gerührt. Die Reaktionsmischung wurde direkt mittles präp. RP-HPLC gereinigt (Säule: Reprosil 125x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 9.6 mg (34 % d. Th.) der Titelverbindung.
LC-MS (Methode 1): Rₜ = 1.01 min; MS (ESIpos): m/z = 530 (M+H)⁺.

### Beispiel 220

### Trifluoressigsäure--N-(3-aminopropyl)-N-{(1R)-1-[1-benzyl-4-(2,5-difluorphenyl)-1H-pyrrol-2-yl]-2,2-dimethylpropyl} -4-(methylsulfanyl)benzamid (1:1)

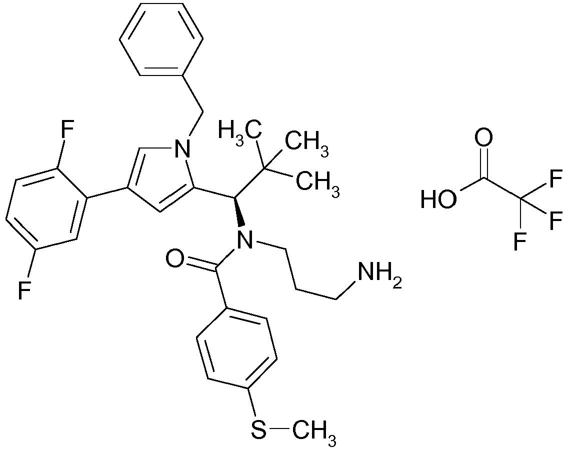

50.0 mg (0.07 mmol) 9H-Fluoren-9-ylmethyl-[3-({(1R)-1-[1-benzyl-4-(2,5-difluorphenyl)-1H-pyrrol-2-yl]-2,2-dimethylpropyl}amino)propyl]carbamat (Intermediat C67) wurden in 2.0 mL Dichlormethan vorgelegt mit 22.3 mg (0.22 mmol) Triethylamin und 27.5 mg (0.15 mmol) 4-(Methylsulfanyl)benzoylchlorid versetzt. Die Reaktionsmischung wurde 4 h bei 40 °C gerührt und nochmals mit 10.2 mg (0.10 mmol) Triethylamin und 27.5 mg (0.15 mmol) 4-(Methylsulfanyl)benzoylchlorid versetzt und über Nacht bei RT gerührt. Dann wurden nochmals 14.9 mg (0.15 mmol) Triethylamin und 27.5 mg (0.15 mmol) 4-(Methylsulfanyl)benzoylchlorid zugegeben und 2 h bei 40 °C gerührt. Es wurde mit Ethylacetat verdünnt und die organische Phase dreimal mit Wasser und einmal mit ges. NaCl-Lösung gewaschen. Das Lösemittel wurde über Magnesiumsulfat getrocknet und im Vakuum verdampft. Der Rückstand wurde mittles präp. RP-HPLC gereinigt (Säule: Reprosil 125x30; 10µ, Fluss: 50 mL/min, MeCN/Wasser) gereinigt. Die Lösemittel wurden im Vakuum verdampft und der Rückstand im Hochvakuum getrocknet. Man erhielt 39.8 mg (76 % d. Th.) der Verbindung 9H-Fluoren-9-ylmethyl-[3-({(1R)-1-[1-benzyl-4-(2,5-difluorphenyl)-1H-pyrrol-2-yl]-2,2-dimethylpropyl} [4-(methylsulfanyl)benzoyl]amino)propyl]carbamat.
LC-MS (Methode 1): Rₜ = 1.59 min; MS (ESIpos): m/z = 785 (M+H)⁺.

18.0 mg (0.02 mmol) 9H-Fluoren-9-ylmethyl-[3-({(1R)-1-[1-benzyl-4-(2,5-difluorphenyl)-1H-pyrrol-2-yl]-2,2-dimethylpropyl}[4-(methylsulfanyl)benzoyl]amino)propyl]carbamat wurden über Nacht in 1.0 mL DMF mit 10.0 mg (0.12 mmol) Morpholin gerührt. Die Reaktionsmischung wurde direkt mittles präp. RP-HPLC gereinigt (Säule: Reprosil 125x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 6.2 mg (40 % d. Th.) der Titelverbindung.
LC-MS (Methode 1): Rₜ = 0.99 min; MS (ESIpos): m/z = 562 (M+H)⁺.

### Beispiel 221

### Trifluoressigsäure--(2S)-N-(3-aminopropyl)-N-{(1R)-1-[1-benzyl-4-(2,5-difluorphenyl)-1H-pyrrol-2-yl]-2,2-dimethylpropyl}-2-hydroxypropanamid (1:1)

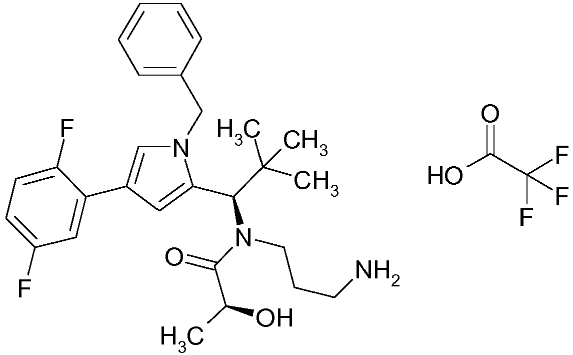

40.0 mg (0.06 mmol) 9H-Fluoren-9-ylmethyl-[3-({(1R)-1-[1-benzyl-4-(2,5-difluorphenyl)-1H-pyrrol-2-yl]-2,2-dimethylpropyl}amino)propyl]carbamat (Intermediat C67) wurden in 2.0 mL Dichlormethan vorgelegt mit 9.6 mg (0.10 mmol) Triethylamin und 14.3 mg (0.10 mmol) (2S)-1-Chlor-1-oxopropan-2-yl-acetat versetzt. Die Reaktionsmischung wurde über Nacht bei RT gerührt. Das Lösemittel wurde im Vakuum verdampft und der Rückstand wurde mittles präp. RP-HPLC gereinigt (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 39.7 mg (84 % d. Th.) der Verbindung (2S)-1-[{(1R)-1-[1-Benzyl-4-(2,5-difluorphenyl)-1H-pyrrol-2-yl]-2,2-dimethylpropyl}(3-{[(9H-fluoren-9-ylmethoxy)carbonyl]amino}propyl)amino]-1-oxopropan-2-yl-acetat.
LC-MS (Methode 1): Rₜ = 1.51 min; MS (ESIpos): m/z = 748 (M+H)⁺.

37.0 mg (0.05 mmol) (2S)-1-[{(1R)-1-[1-Benzyl-4-(2,5-difluorphenyl)-1H-pyrrol-2-yl]-2,2-dimethylpropyl}(3-{[(9H-fluoren-9-ylmethoxy)carbonyl]amino}propyl)amino]-1-oxopropan-2-yl-acetat wurden in 1.0 mL DMF mit 0.1 mL Morpholin und 3 Tropfen Wasser 10 h bei 50 °C gerührt. Es wurden nochmals 0.1 mL Morpholin und 0.1 mL Wasser zugegeben und 10 h bei 50 °C gerührt. Nach der Zugabe von 20.5 mg (0.15 mmol) Kaliumcarbonat und 72 h Rühren bei RT wurden 0.1 mL IN NaOH-Lösung zugegeben und über Nacht bei RT gerührt. Die Reaktionsmischung wurde direkt mittles präp. RP-HPLC gereinigt (Säule: Reprosil 125x30; 10µ, Fluss: 50 mL/min, MeCN/Wasser, 0.1 % TFA). Die Lösemittel wurden im Vakuum verdampft und der Rückstand im Hochvakuum getrocknet. Man erhielt 20.5 mg (69 % d. Th.) der Titelverbindung.
LC-MS (Methode 1): Rₜ = 0.89 min; MS (ESIpos): m/z = 484 (M+H)⁺.

### Beispiel 222

### Trifluoressigsäure--N-(3-aminopropyl)-N-{(1R)-1-[1-benzyl-4-(2,5-difluorphenyl)-1H-pyrrol-2-yl]-2,2-dimethylpropyl} -2-(methylsulfanyl)acetamid (1:1)

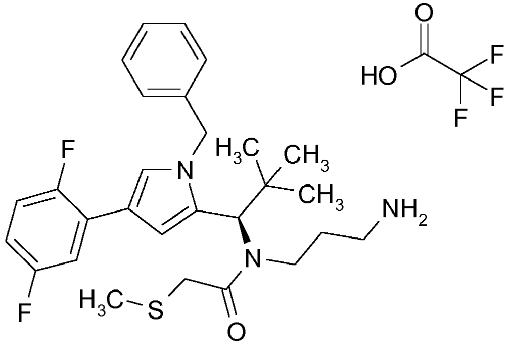

70.0 mg (0.11 mmol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C 70) wurden in 3.0 mL DMF vorgelegt. Es wurden 15.5 mg (0.22 mmol) Natriummethanthiolat
zugegeben und die Reaktionsmischung wurde 2 h bei 50 °C gerührt. Die Reaktionsmischung wurde direkt mittles präp. RP-HPLC gereinigt (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 60.0 mg (84 % d. Th.) der Verbindung 2-(Trimethylsilyl)ethyl-[3-({(1R)-1-[1-benzyl-4-(2,5-difluorphenyl)-1H-pyrrol-2-yl]-2,2-dimethylpropyl}[(methylsulfanyl)acetyl]amino)propyl]carbamat.
LC-MS (Methode 1): Rₜ = 1.50 min; MS (ESIpos): m/z = 644 (M+H)⁺.

40.0 mg (0.06 mol) 2-(Trimethylsilyl)ethyl-[3-({(1R)-1-[1-benzyl-4-(2,5-difluorphenyl)-1H-pyrrol-2-yl]-2,2-dimethylpropyl}[(methylsulfanyl)acetyl]amino)propyl]carbamat wurden in 2.0 mL Trifluorethanol gelöst und mit 21.2 mg (0.16 mmol) Zinkdichlorid versetzt. Die Reaktionsmischung rührte über Nacht bei 50 °C. Die Reaktionsmischung wurde mit 45.4 mg (0.01 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 34.6 mg (91 % d. Th.) der Titelverbindung.
LC-MS (Methode 1): Rₜ = 0.92 min; MS (ESIpos): m/z = 500 (M+H)⁺.

### Beispiel 223

### Trifluoressigsäure--(2S)-N-(3-aminopropyl)-N-{(1R)-1-[4-benzyl-1-(2,5-difluorphenyl)-1H-pyrazol-3-yl]-2,2-dimethylpropyl}-2-hydroxypropanamid (1:1)

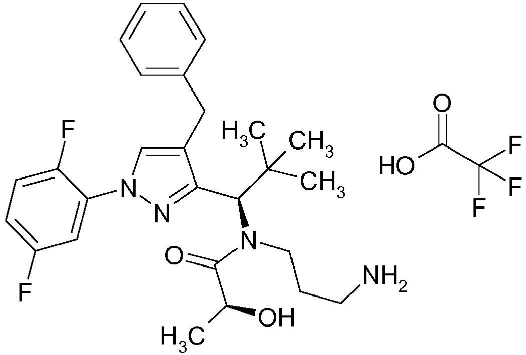

40.0 mg (0.08 mmol) tert-Butyl-[3-({(1R)-1-[4-benzyl-1-(2,5-difluorphenyl)-1H-pyrazol-3-yl]-2,2-dimethylpropyl}amino)propyl]carbamat wurden in 2.0 mL Dichlormethan gelöst und mit 19.7 mg (0.20 mmol) Triethylamin und 29.4 mg (0.20 mmol) (2S)-1-Chlor-1-oxopropan-2-yl-acetat versetzt. Die Reaktionsmischung wurde über Nacht gerührt. Das Lösemittel wurde im Vakuum verdampft und der Rückstand mittles präp. RP-HPLC gereinigt (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 21.2 mg (43 % d. Th.) der Verbindung (2S)-1-({(1R)-1-[4-Benzyl-1-(2,5-difluorphenyl)-1H-pyrazol-3-yl]-2,2-dimethylpropyl}{3-[(tert-butoxycarbonyl)amino]propyl}amino)-1-oxopropan-2-yl-acetat.
LC-MS (Methode 1): Rₜ = 1.46 min; MS (ESIpos): m/z = 627 (M+H)⁺.

21.2 mg (0.03 mmol) (2S)-1-({(1R)-1-[4-Benzyl-1-(2,5-difluorphenyl)-1H-pyrazol-3-yl]-2,2-dimethylpropyl} {3-[(tert-butoxycarbonyl)amino]propyl}amino)-1-oxopropan-2-yl-acetat wurden in 1.0 mL Dichlormethan gelöst, mit 77.1 mg (0.68 mmol) Trifluoressigsäure versetzt und 2 h bei RT gerührt. Es wurden noch jeweils zweimal 77.1 mg (0.68 mmol) Trifluoressigsäure zugegeben und jeweils über Nacht bei RT gerührt. Das Lösemittel wurde im Vakuum verdampft und der Rückstand mehrmals mit Dichlormethan kodestilliert und anschließend im Hochvakuum getrocknet. Der Rückstand mit der Substanz Trifluoressigsäure--(2S)-1-[(3-aminopropyl){(1R)-1-[4-benzyl-1-(2,5-difluorphenyl)-1H-pyrazol-3-yl]-2,2-dimethylpropyl}amino]-1-oxopropan-2-yl-acetat (1:1) wurde ohne weitere Reinigung weiter umgesetzt.
LC-MS (Methode 1): Rₜ = 0.92 min; MS (ESIpos): m/z = 527 (M+H)⁺.

26.5 mg (0.04 mmol) Trifluoressigsäure-(2S)-1-[(3-aminopropyl){(1R)-1-[4-benzyl-1-(2,5-difluorphenyl)-1H-pyrazol-3-yl]-2,2-dimethylpropyl}amino]-1-oxopropan-2-yl-acetat (1:1) wurden in THF/Methanol/Wasser (1.0 mL/1.0 mL/0.05 mL) gelöst und mit 17.2 mg Kaliumcarbonat versetzt. Die Reaktionsmischung wurde über Nacht bei RT gerührt. Die Reaktionsmischung wurde direkt mittles präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Man erhielt 17.3 mg (70 % d. Th.) der Titelverbindung.
LC-MS (Methode 1): Rₜ = 0.91 min; MS (ESIpos): m/z = 485 (M+H)⁺.

### Beispiel 224

### Trifluoressigsäure-methyl-4-[(3-aminopropyl){(1R)-1-[1-benzyl-4-(2,5-difluorphenyl)-1H-pyrrol-2-yl]-2,2-dimethylpropyl}amino]-4-oxobutanoat (1:1)

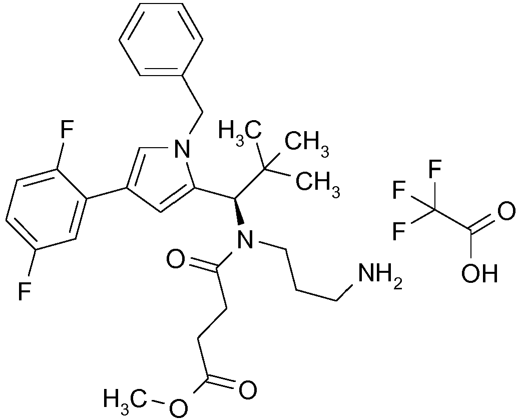

60.0 mg (0.11 mmol) 2-(Trimethylsilyl)ethyl-[3-({(1R)-1-[1-benzyl-4-(2,5-difluorphenyl)-1H-pyrrol-2-yl]-2,2-dimethylpropyl}amino)propyl]carbamat (siehe Synthese von Intermediat C11) wurden in 1.0 mL Dichlormethan gelöst und mit 19.6 mg (0.25 mmol) Pyridin und 35.8 mg (0.24 mmol) Methyl-4-chlor-4-oxobutanoat versetzt. Die Reaktionsmischung rührte über Nacht bei 40 °C. Es wurden nochmals 19.6 mg (0.25 mmol) Pyridin und 35.8 mg (0.24 mmol) Methyl-4-chlor-4-oxobutanoat zugegeben und über Nacht bei 40 °C gerührt. Das Lösemittel wurde im Vakuum verdampft und der Rückstand mittels präp. RP-HPLC gereinigt (Säule: Reprosil 125x30; 10µ, Fluss: 50 mL/min, MeCN/Wasser). Die Lösemittel wurden im Vakuum verdampft und der Rückstand im Hochvakuum getrocknet. Man erhielt 16.1 mg (22 % d. Th.) der Verbindung Methyl-11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-oat.
LC-MS (Methode 1): Rₜ = 1.52 min; MS (ESIpos): m/z = 670 (M+H)⁺.

16.1 mg (0.02 mmol) Methyl-11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silapentadecan-15-oat wurden in 1.0 mL Trifluorethanol gelöst und mit 16.4 mg (0.12 mmol) Zinkdichlorid versetzt. Die Reaktionsmischung rührte 5 h bei 50 °C. Die Reaktionsmischung wurde mit 35.1 mg (0.12 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Die Lösemittel wurden im Vakuum verdampft und der Rückstand im Hochvakuum getrocknet. Man erhielt 11.1 mg (72 % d. Th.) der Titelverbindung.
LC-MS (Methode 1): Rₜ = 0.93 min; MS (ESIpos): m/z = 526 (M+H)⁺.

### Beispiel 225

### 4-[(3-Aminopropyl) {(1R)-1-[1-benzyl-4-(2,5-difluorphenyl)-1H-pyrrol-2-yl]-2,2-dimethylpropyl}amino]-4-oxobutansäure-trifluoressigsäure (1:1)

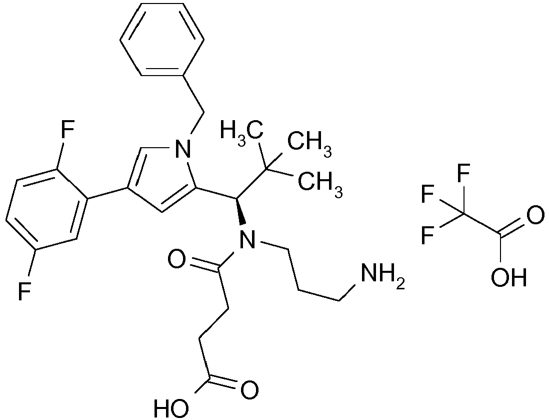

9.7 mg (0.02 mmol) Trifluoressigsäure-methyl-4-[(3-aminopropyl){(1R)-1-[1-benzyl-4-(2,5-difluorphenyl)-1H-pyrrol-2-yl]-2,2-dimethylpropyl}amino]-4-oxobutanoat (1:1) (Beispiel 224) wurden in THF/Methanol/Wasser (1.0 mL/0.2 mL/0.04 mL) vorgelegt und mit 1.3 mg (0.03 mmol) Lithiumhydroxid-Monohydrat versetzt. Die Reaktionsmischung rührte über Nacht bei RT. Es wurden nochmals 1.3 mg (0.03 mmol) Lithiumhydroxid-Monohydrat zugegeben und über Nacht bei RT gerührt. Es wurden 3.6 mg (0.06 mmol) HOAc zugegeben und die Reaktionsmischung wurde direkt mittles präp. RP-HPLC gereinigt (Säule: Reprosil 125x30; 10µ, Fluss: 50 mL/min, MeCN/Wasser; 0.1% TFA). Die Lösemittel wurden im Vakuum verdampft und der Rückstand im Hochvakuum getrocknet. Man erhielt 5.4 mg (57 % d. Th.) der Titelverbindung.
LC-MS (Methode 1): Rₜ = 0.90 min; MS (ESIpos): m/z = 512 (M+H)⁺.

### Beispiel 226

### (2R)-22-[(3R/S)-3-{[(2R)-2-Amino-2-carboxyethyl]sulfanyl}-2,5-dioxopyrrolidin-1-yl]-2-[({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)methyl]-4,20-dioxo-7,10,13,16-tetraoxa-3-säure-trifluoressigsäure (1:2)

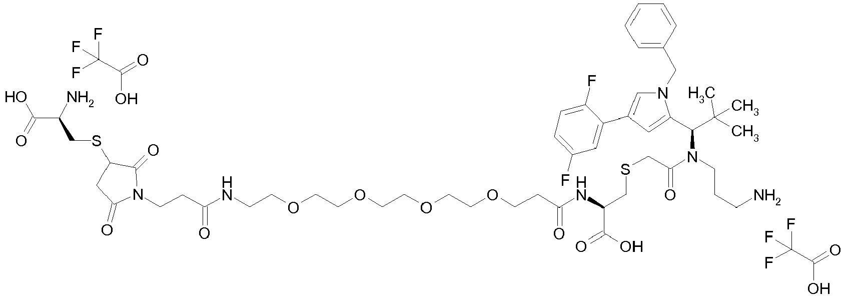

10.3 mg (mmol) R-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-L-cystein-trifluoressigsäure (1:1) (Intermediat F209) wurden in DMF/Wasser (2.0 mL/0.2 mL) mit L-Cystein versetzt und 10 min bei RT gerührt. Die Reaktionsmischung wurde mit Wasser versetzt und direkt mittles präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Man erhielt 10.3 mg (82 % d. Th.) der Titelverbindung.
LC-MS (Methode 1): Rₜ = 0.84 min; MS (ESIpos): m/z = 1092 (M+H)⁺.

### Beispiel 227

### Trifluoressigsäure--4-amino-N-(3-aminopropyl)-N-{(1R)-1-[4-benzyl-1-(2,5-difluorphenyl)-1H-pyrazol-3-yl]-2,2-dimethylpropyl}benzamid (2:1)

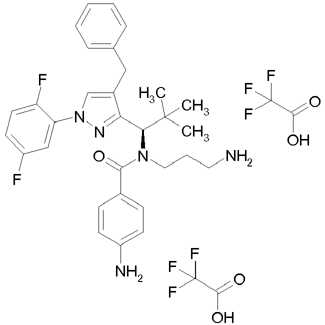

50.0 mg (0.10 mol) tert-Butyl-[3-({(1R)-1-[4-benzyl-1-(2,5-difluorphenyl)-1H-pyrazol-3-yl]-2,2-dimethylpropyl}amino)propyl]carbamat (Intermediat C68) wurden in Dichlormethan vorgelegt und mit 54.9 mg (0.22 mmol) tert-Butyl-[4-(chlorcarbonyl)phenyl]carbamat (Intermediat L71) sowie 22.7 mg (0.22 mmol) Triethylamin versetzt. Die Reaktionsmischung wurde über Nacht bei RT gerührt und nochmals mit 54.9 mg (0.22 mmol) tert-Butyl-[4-(chlorcarbonyl)phenyl]carbamat (Intermediat L71) sowie 22.7 mg (0.22 mmol) Triethylamin versetzt. Die Reaktionsmischung rührte über Nacht bei RT. Das Lösemittel wurde im Vakuum verdampft und der Rückstand mittles präp. RP-HPLC (Säule: Reprosil 250x40; 10µ, Fluss: 50 mL/min, MeCN/Wasser, 0.1% TFA). Die Lösemittel wurden im Vakuum verdampft und der Rückstand im Hochvakuum getrocknet. Man erhielt 26.2 mg (37 % d. Th.) der Verbindung.

tert-Butyl-[3-({(1R)-1-[4-benzyl-1-(2,5-difluorphenyl)-1H-pyrazol-3-yl]-2,2-dimethylpropyl}{4-[(tert-butoxycarbonyl)amino]benzoyl}amino)propyl]carbamat.
LC-MS (Methode 1): Rₜ = 5.34 min; MS (ESIpos): m/z = 732 (M+H)⁺.

26.2 mg (0.04 mmol) tert-Butyl-[3-({(1R)-1-[4-benzyl-1-(2,5-difluorphenyl)-1H-pyrazol-3-yl]-2,2-dimethylpropyl}{4-[(tert-butoxycarbonyl)amino]benzoyl}amino)propyl]carbamat wurden in 2.0 mL Dichlormethan gelöst und mit 204.1 mg (1.79 mmol) TFA versetzt. Die Reaktionsmischung rührte über Nacht bei RT. Das Lösemittel wurde im Vakuum verdampft und der Rückstand wurde mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 3.4 mg (13 % d. Th.) der Titelverbindung.
LC-MS (Methode 1): Rₜ = 0.92 min; MS (ESIpos): m/z = 532 (M+H)⁺.

### Beispiel 228

### N-Acetyl-S- {2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein-trifluoressigsäure (1:1)

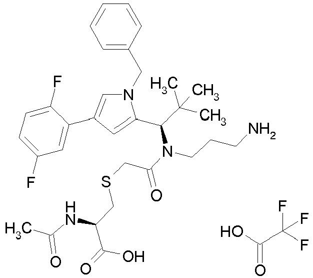

50.0 mg (0.08 mol) 2-(Trimethylsilyl)ethyl-{3-[{(1R)-1-[1-benzyl-4-(2,5-difluorphenyl)-1H-pyrrol-2-yl]-2,2-dimethylpropyl}(chloracetyl)amino]propyl}carbamat (Intermediat C70) wurden in 0.30 mL Wasser mit 66.43 mg Natriumhydrogencarbonat suspendiert. Die Suspension wurde mit einer Lösung von 144.47 (0.95 mmol) 1,8-Diazabicyclo(5.4.0)undec-7-en und 51.62 mg (0.32 mmol) N-Acetyl-L-cystein in 3.0 mL *iso*-Propanol versetzt. Die Reaktionsmischung wurde 2.5 h bei 50 °C gerührt. Die Reaktionsmischung wurde mit Wasser (0.1 % TFA) versetzt und direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 55.2 mg (92 % d. Th.) der Verbindung N-Acetyl-S-(11-{(1R)-1-[1-benzyl-4-(2,5-difluorphenyl)-1H-pyrrol-2-yl]-2,2-dimethylpropyl} -2,2-dimethyl-6,12-dioxo-5-oxa-7,11-diaza-2-silatridecan-13-yl)-L-cystein.
LC-MS (Methode 1): Rₜ = 1.41 min; MS (ESIpos): m/z = 759 (M+H)⁺.

53.1 mg (69.96 µmol) N-Acetyl-S-(11-{(1R)-1-[1-benzyl-4-(2,5-difluorphenyl)-1H-pyrrol-2-yl]-2,2-dimethylpropyl}-2,2-dimethyl-6,12-dioxo-5-oxa-7,11-diaza-2-silatridecan-13-yl)-L-cystein wurden in 5.0 mL Trifluorethanol gelöst und mit 57.2 mg (419.76 µmol) Zinkdichlorid versetzt. Die Reaktionsmischung rührte 4 h bei 50 °C. Die Reaktionsmischung wurde mit 122.67 mg (0.42 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Man erhielt 32.5 mg (64 % d. Th.) der Titelverbindung.
LC-MS (Methode 1): Rₜ = 0.90 min; MS (ESIpos): m/z = 615 (M+H)⁺.

### Beispiel 229

### N-Acetyl-S-[2-([3-(L-alanylamino)propyl]{(1R)-1-[1-benzyl-4-(2,5-difluorphenyl)-1H-pyrrol-2-yl]-2,2-dimethylpropyl}amino)-2-oxoethyl]-L-cystein-trifluoressigsäure (1:1)

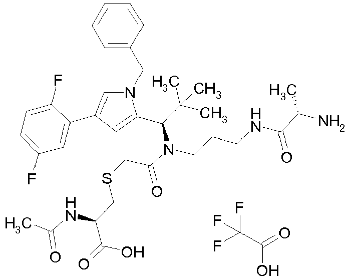

30.3 mg (41.58 µmol) N-Acetyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein-trifluoressigsäure (1:1) (Beispiel 228) wurden in 1.5 mL DMF gelöst und mit 8.4 mg (83.15 µmol) 4-Methylmorpholin und 14.65 mg (45.73 µmol) 2,5-Dioxopyrrolidin-1-yl-N-[(benzyloxy)carbonyl]-L-alaninat versetzt. Die Reaktionsmischung wurde über Nacht bei RT gerührt und dann wurden nochmals 8.4 mg (83.15 µmol) 4-Methylmorpholin zugegeben. Die Reaktionsmischung wurde nochmals über Nacht bei RT gerührt. Es wurden 10.0 mg (0.17 mmol) Essigsäure zugegeben und die Reaktionsmischung wurde direkt mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 31.2 mg (92 % d. Th.) der Verbindung N-Acetyl-S-[2-({(1R)-1-[1-benzyl-4-(2,5-difluorphenyl)-1H-pyrrol-2-yl]-2,2-dimethylpropyl}[3-({N-[(benzyloxy)carbonyl]-L-alanyl}amino)propyl]amino)-2-oxoethyl]-L-cystein.
LC-MS (Methode 1): Rₜ = 1.22 min; MS (ESIpos): m/z = 820 (M+H)⁺.

28.6 mg (0.04 mmol) N-Acetyl-S-[2-({(1R)-1-[1-benzyl-4-(2,5-difluorphenyl)-1H-pyrrol-2-yl]-2,2-dimethylpropyl}[3-({N-[(benzyloxy)carbonyl]-L-alanyl}amino)propyl]amino)-2-oxoethyl]-L-cystein wurden in 5.0 mL Ethanol gelöst und mit 2.9 mg Palladium auf Aktivkohle (10 %) versetzt. Die Reaktionsmischung wurde über Nacht bei Normaldruck und bei RT hydriert. Der Ansatz wurde über Celite filtriert und der Filterkuchen mit einem Gemisch aus Ethanol nachgewaschen. Die Lösemittel wurden im Vakuum verdampft und der Rückstand mittles präp. RP-HPLC (Säule: Reprosil 250x30; 10µ, Fluss: 50 mL/min, MeCN/Wasser, 0.1% TFA) gereinigt. Die Lösemittel wurden im Vakuum verdampft und der Rückstand wurde lyophilisert. Man erhielt 17.4 mg (62 % d. Th.) der Titelverbindung.
LC-MS (Methode 1): Rₜ = 0.85 min; MS (ESIpos): m/z = 686 (M+H)⁺.

### Beispiel 230

### Trifluoressigsäure--(2S)-N-(3-aminopropyl)-N-{(1R)-1-[1-benzyl-4-(2,5-difluorphenyl)-1H-pyrrol-2-yl]-2,2-dimethylpropyl}tetrahydrofuran-2-carboxamid (1:1)

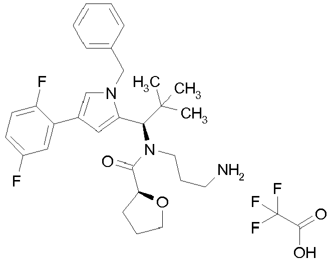

100.0 mg (0.16 mmol) 9H-Fluoren-9-ylmethyl-[3-({(1R)-1-[1-benzyl-4-(2,5-difluorphenyl)-1H-pyrrol-2-yl]-2,2-dimethylpropyl}amino)propyl]carbamat (Intermediat C67) wurden in 5.0 mL Dichlormethan mit 71.9 mg (0.71 mmol) Triethylamin gelöst und zu einer Lösung aus frisch hergestelltem (2S)-Tetrahydrofuran-2-carbonylchlorid (Herstellung: 54.7 mg (0.39 mmol) (2S)-Tetrahydrofuran-2-carbonsäure wurden in 0.7 mL Toluol vorgelegt und mit 0.04 mL Thionylchlorid versetzt und 1 h bei 90 °C gerührt. Die Reaktionslösung wurde nach dem Abkühlen roh weiter umgesetzt) getropft. Die Reaktionsmischung wurde über Nacht bei RT gerührt. Die Lösemittel wurden im Vakuum verdampft und der Rückstand wurde mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 44.3 mg (38 % d. Th.) der Verbindung 9H-Fluoren-9-ylmethyl-[3-({(1R)-1-[1-benzyl-4-(2,5-difluorphenyl)-1H-pyrrol-2-yl]-2,2-dimethylpropyl}[(2S)-tetrahydrofuran-2-ylcarbonyl]amino)propyl]carbamat.
LC-MS (Methode 1): Rₜ = 1.52 min; MS (ESIpos): m/z = 776 (M+HCOOH-H)⁻.

26.0 mg (0.04 mmol) 9H-Fluoren-9-ylmethyl-[3-({(1R)-1-[1-benzyl-4-(2,5-difluorphenyl)-1H-pyrrol-2-yl]-2,2-dimethylpropyl}[(2S)-tetrahydrofuran-2-ylcarbonyl]amino)propyl]carbamat wurden in 2.6 mL DMF gelöst und mit 0.26 mL Morpholin versetzt. Die Reaktionsmischung wurde 3 h bei RT gerührt. Die Reinigung erfolgte direkt mittles präp. RP-HPLC (Säule: Reprosil 125x30; 10µ, Fluss: 50 mL/min, MeCN/Wasser, 0.1% TFA). Man erhielt 11.7 mg (53 % d. Th.) der Titelverbindung.
LC-MS (Methode 1): Rₜ = 0.93 min; MS (ESIpos): m/z = 510 (M+H)⁺.

### Beispiel 231

### 3-({2-[(3-Aminopropyl){(1R)-1-[1-benzyl-4-(2,5-difluorphenyl)-1H-pyrrol-2-yl]-2,2-dimethylpropyl}amino]-2-oxoethyl}sulfanyl)propansäure-trifluoressigsäure (1:1)

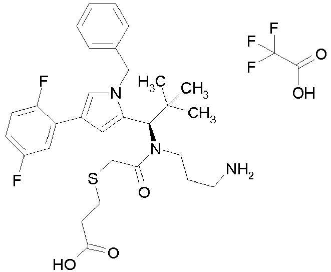

53.9 mg (0.08 mmol) 11-{(1R)-1-[1-Benzyl-4-(2,5-difluorphenyl)-1H-pyrrol-2-yl]-2,2-dimethylpropyl}-2,2-dimethyl-6,12-dioxo-5-oxa-14-thia-7,11-diaza-2-silaheptadecan-17-säure (Intermediat C69) wurden in 4.0 mL Trifluorethanol gelöst und mit 31.4 mg (0.23 mmol) Zinkdichlorid versetzt. Die Reaktionsmischung rührte über Nacht bei 50 °C. Die Reaktionsmischung wurde mit 67.3 mg (0.23 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Man erhielt 34.2 mg (66 % d. Th.) der Titelverbindung.
LC-MS (Methode 1): Rₜ = 0.91 min; MS (ESIpos): m/z = 558 (M+H)⁺.

### Beispiel 232

### S-{2-[(3-Aminopropyl){(1R)-1-[1-benzyl-4-(2,5-difluorphenyl)-1H-pyrrol-2-yl]-2,2-dimethylpropyl}amino]-2-oxoethyl}homocystein-trifluoressigsäure (1:2)

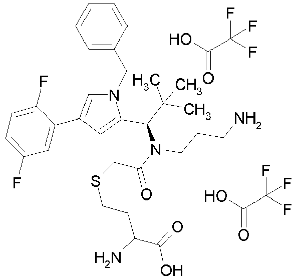

40.0 mg (47.3 µmol) S-(11-{(1R)-1-[1-Benzyl-4-(2,5-difluorphenyl)-1H-pyrrol-2-yl]-2,2-dimethylpropyl} -2,2-dimethyl-6,12-dioxo-5-oxa-7,11-diaza-2-silatridecan-13-yl)homocystein (Intermediat C11) wurden in 3.0 mL Trifluorethanol gelöst und mit 38.7 mg (0.28 mmol) Zinkdichlorid versetzt. Die Reaktionsmischung rührte 6 h bei 50 °C. Die Reaktionsmischung wurde mit 83 mg (0.28 mmol) Ethylendiamin-N,N,N',N'-tetraessigsäure versetzt, 10 min gerührt und dann mit Wasser (0.1% TFA) versetzt. Die Reinigung erfolgte direkt mittles präp. RP-HPLC (Säule: Reprosil 125x30; 10µ, Fluss: 50 mL/min, MeCN/Wasser, 0.1% TFA). Man erhielt 32.4 mg (84 % d. Th.) der Titelverbindung. LC-MS (Methode 1): Rₜ = 0.81 min; MS (ESIpos): m/z = 587 (M+H)⁺.

### Beispiel 233

### Trifluoressigsäure--4-amino-N-(3-aminopropyl)-N-{(1R)-1-[1-benzyl-4-(2,5-difluorphenyl)-1H-pyrrol-2-yl]-2,2-dimethylpropyl}benzamid (2:1)

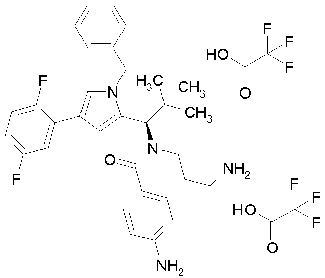

73.0 mg (0.12 mmol) 9H-Fluoren-9-ylmethyl-[3-({(1R)-1-[1-benzyl-4-(2,5-difluorphenyl)-1H-pyrrol-2-yl]-2,2-dimethylpropyl}amino)propyl]carbamat (Intermediat C67) und 27.8 mg (0.15 mmol) 4-Nitrobenzoylchlorid wurden in 2.0 mL Dichlormethan gelöst und mit 15.2 mg (0.15 mmol) Triethlyamin versetzt. Die Reaktionsmischung rührte über Nacht bei RT. Es wurden wurde nochmals die dieselbe Menge an 4-Nitrobenzoylchlorid und Triethylamin zugegeben und die Reaktionsmischung rührte über Nacht bei RT. Das Lösemittel wurde im Vakuum verdampft und der Rückstand wurde mittels präp. RP-HPLC (Säule: Reprosil 125x30; 10µ, Fluss: 50 mL/min, MeCN/Wasser, 0.1% TFA) gereinigt. Man erhielt 39.3 mg (44 % d. Th.) der Verbindung 9H-Fluoren-9-ylmethyl-{3-[{(1R)-1-[1-benzyl-4-(2,5-difluorphenyl)-1H-pyrrol-2-yl]-2,2-dimethylpropyl}(4-nitrobenzoyl)amino]propyl}carbamat.
LC-MS (Methode 1): Rₜ = 1.54 min; MS (ESIpos): m/z = 783 (M+H)⁺.

39.3 mg (0.05 mmol) Fluoren-9-ylmethyl-{3-[{(1R)-1-[1-benzyl-4-(2,5-difluorphenyl)-1H-pyrrol-2-yl]-2,2-dimethylpropyl}(4-nitrobenzoyl)amino]propyl}carbamat wurden in 2.0 mL Ethanol gelöst, mit 3.9 mg Palladiumhydroxid auf Aktivkohle (20 %) versetzt und über Nacht bei Normaldruck hydriert. Die Reaktionsmischung wurde über einen Papierfilter filtriert und der Filterkuchen wurde mit Ethanol nachgewaschen. Das Lösemittel wurde im Vakuum verdampft. Der Rückstand wurde mittles präp. RP-HPLC (Säule: Reprosil 125x30; 10µ, Fluss: 50 mL/min, MeCN/Wasser, 0.1% TFA) gereinigt. Die Lösemittel wurden im Vakuum verdampft und der Rückstand im Hochvakuum getrocknet. Man erhielt 32.9 mg (86 % d. Th.) der Titelverbindung. LC-MS (Methode 1): Rₜ = 0.93 min; MS (ESIpos): m/z = 531 (M+H)⁺.

### Beispiel 234A

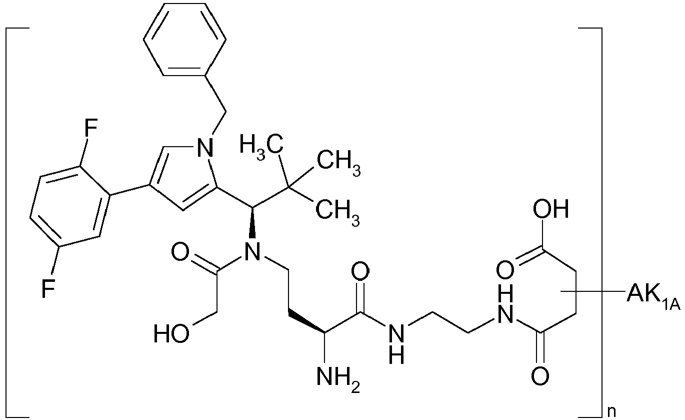

5 mg Cetuximab in 500 µL ml PBS (c= 10 mg/mL) wurden unter Argon mit einer Lösung aus 0,029 mg TCEP in 50µl PBS-Puffer versetzt. Der Ansatz wurde 30min bei RT gerührt und anschließend wurden 0.2 mg (0.00027 mmol) von Intermediat F85 gelöst in 50µl DMSO zugegeben. Nach weiteren 90 min Rühren bei RT wurde der Ansatz mit 1900 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.68 mg/mL
Drug/mAb Ratio: 3.8

### Beispiel 234B

5 mg anti-TWEAKR AK-1 in 269 µL ml PBS (c=18.6 mg/mL) wurden unter Argon mit einer Lösung aus 0.029 mg TCEP in 50µl PBS-Puffer versetzt. Der Ansatz wurde 30min bei RT gerührt und anschließend wurden 0.2 mg (0.00027 mmol) von Intermediat F85 gelöst in 50µl DMSO zugegeben. Nach weiteren 90 min Rühren bei RT wurde der Ansatz mit 2130 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.57 mg/mL
Drug/mAb Ratio: 3.7

### Beispiel 2341

5 mg Nimotuzumab in 500 µL ml PBS (c=10 mg/mL) wurden unter Argon mit einer Lösung aus 0.029 mg TCEP in 50µl PBS-Puffer versetzt. Der Ansatz wurde 30min bei RT gerührt und anschließend wurden 0.2 mg (0.00027 mmol) von Intermediat F85 gelöst in 50µl DMSO zugegeben. Nach weiteren 90 min Rühren bei RT wurde der Ansatz mit 1900 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.93 mg/mL
Drug/mAb Ratio: 3.6

### Beispiel 234H

5 mg Panitumumab in 500 µL ml PBS (c=10 mg/mL) wurden unter Argon mit einer Lösung aus 0.029 mg TCEP in 50µl PBS-Puffer versetzt. Der Ansatz wurde 4 h bei RT gerührt und anschließend wurden 0.2 mg (0.00027 mmol) von Intermediat F85 gelöst in 50µl DMSO zugegeben. Nach weiteren 120 min Rühren bei RT wurde der Ansatz mit 1900 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 2.12 mg/mL
Drug/mAb Ratio: 1.4

### Beispiel 235A

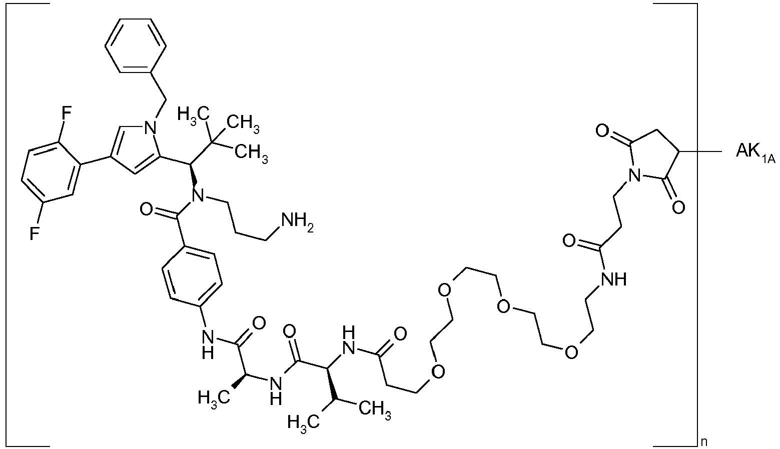

5 mg Cetuximab in 329 µL ml PBS (c= 15.2 mg/mL) wurden unter Argon mit einer Lösung aus 0,029 mg TCEP in 50µl PBS-Puffer versetzt und dann mit 2021 µL PBS Puffer, der zuvor auf pH 8 eingestellt wurde, verdünnt. Der Ansatz wurde 1 h bei RT gerührt und anschließend wurden 0.28 mg von Intermediat F235 gelöst in 50µl DMSO zugegeben. Nach weiteren 90 min Rühren bei RT wurde der Ansatz dann über mit PBS-Puffer pH8 equillibrierte PD 10-Säulen (Sephadex® G-25, GE Healthcare) gegeben und mit PBS-Puffer pH8 eluiert. Das Eluat wurde über Nacht bei RT unter Argon gerührt und anschließend durch Ultrazentrifugation aufkonzentriert und rückverdünnt mit PBS-Puffer (pH 7.2). Das so hergestellte ADC kann auch teilweise in Form der hydrolysierten offenkettigen Bernsteinsäureamide mit dem Antikörper verknüpft vorliegen. Der erhaltene ADC batch wurde folgendermaßen charakterisiert:
Protein Konzentration: 1.5 mg/mL
Drug/mAb Ratio: 2.4

### Beispiel 235B

5 mg anti-TWEAKR AK-1 in 269 µL ml PBS (c=18.6 mg/mL) wurden in Analogie zu Beispiel 235a mit PBS-Puffer pH8 auf eine Konzentration von 2 mg/ml verdünnt und mit Intermediat F235 gekuppelt. Das so hergestellte ADC kann auch teilweise in Form der hydrolysierten offenkettigen Bernsteinsäureamide mit dem Antikörper verknüpft vorliegen. Der erhaltene ADC batch wurde folgendermaßen charakterisiert:
Protein Konzentration: 0.56 mg/mL
Drug/mAb Ratio: 1.0

### Beispiel 235E

5 mg Trastuzumab in 370 µL ml PBS (c=14.5 mg/mL) wurden in Analogie zu Beispiel 235a mit PBS-Puffer pH8 auf eine Konzentration von 2 mg/ml verdünnt und mit Intermediat F235 gekuppelt. Das so hergestellte ADC kann auch teilweise in Form der hydrolysierten offenkettigen Bernsteinsäureamide mit dem Antikörper verknüpft vorliegen. Der erhaltene ADC batch wurde folgendermaßen charakterisiert:
Protein Konzentration: 1.67 mg/mL
Drug/mAb Ratio: 2.9

### Beispiel 2351

5 mg Nimotuzumab in 382 µL ml PBS (c=13.08 mg/mL) wurden in Analogie zu Beispiel 235a mit PBS-Puffer pH8 auf eine Konzentration von 2 mg/ml verdünnt und mit Intermediat F235 gekuppelt. Das so hergestellte ADC kann auch teilweise in Form der hydrolysierten offenkettigen Bernsteinsäureamide mit dem Antikörper verknüpft vorliegen. Der erhaltene ADC batch wurde folgendermaßen charakterisiert:
Protein Konzentration: 1.81 mg/mL
Drug/mAb Ratio: 2.8

### Beispiel 235H

5 mg Panitumumab in 74 µL ml PBS (c=67.4 mg/mL) wurden in Analogie zu Beispiel 235a mit PBS-Puffer pH8 auf eine Konzentration von 2 mg/ml verdünnt und mit Intermediat F235 gekuppelt. Das so hergestellte ADC kann auch teilweise in Form der hydrolysierten offenkettigen Bernsteinsäureamide mit dem Antikörper verknüpft vorliegen. Der erhaltene ADC batch wurde folgendermaßen charakterisiert:
Protein Konzentration: 1.14 mg/mL
Drug/mAb Ratio: 0.9

### Beispiel 236A

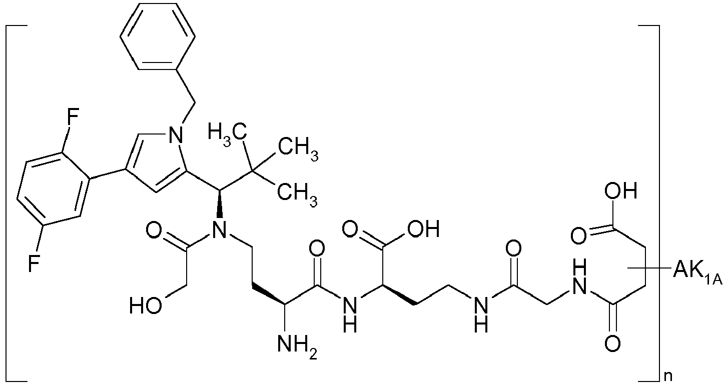

5 mg Cetuximab in 500 µL ml PBS (c= 10 mg/mL) wurden in Analogie zu Beispiel 234a mit Intermediat F236 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.85 mg/mL
Drug/mAb Ratio: 3.6

### Beispiel 236B

5 mg anti-TWEAKR AK-1 in 500 µL ml PBS (c=10 mg/mL) wurden in Analogie zu Beispiel 234a mit Intermediat F236 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.67 mg/mL
Drug/mAb Ratio: 3.5

### Beispiel 236E

5 mg Trastuzumab in 500 µL ml PBS (c=10 mg/mL) wurden in Analogie zu Beispiel 234a mit Intermediat F236 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.67 mg/mL
Drug/mAb Ratio: 3.9

### Beispiel 237A

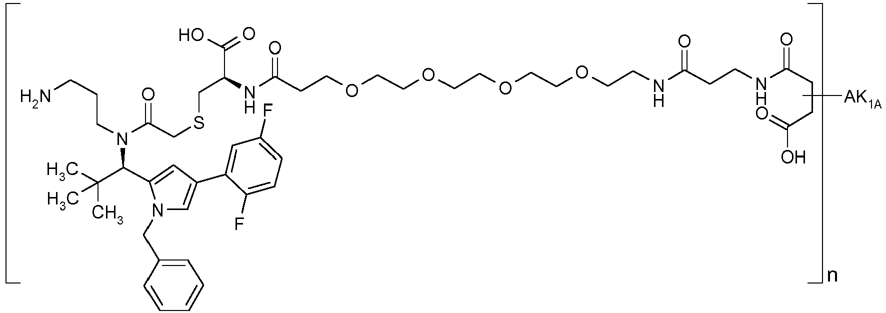

60 mg Cetuximab in 4000 µL ml PBS (c=15 mg/mL) wurden unter Argon mit einer Lösung aus 0.344 mg TCEP in 100µl PBS-Puffer versetzt. Der Ansatz wurde 1h bei RT gerührt und anschließend wurden 3.04 mg (0.003 mmol) von Intermediat F209 gelöst in 600µl DMSO zugegeben. Nach weiteren 90 min Rühren bei RT wurde der Ansatz mit PBS-Puffer, der zuvor auf pH 8 eingestellt wurde, auf 15 ml verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Diese Lösung wurde über Nacht bei RT unter Argon gerührt und anschließend durch Ultrazentrifugation aufkonzentriert, rückverdünnt mit PBS-Puffer (pH 7.2) und erneut aufkonzentriert. Ein Teil des ADCs kann unter diesen Bedingungen auch in der ring-geschlossenen Form vorliegen. Der erhaltene ADC batch wurde folgendermaßen charakterisiert:
Protein Konzentration: 13.78 mg/mL
Drug/mAb Ratio: 4.8

### Beispiel 237B

50 mg anti-TWEAKR AK-1 in 2688 µL ml PBS (c=18.6 mg/mL) wurden unter Argon mit einer Lösung aus 0.287 mg TCEP in 500µl PBS-Puffer versetzt und mit 8812 µL PBS Puffer, der zuvor auf pH 8 eingestelt wurde, verdünnt. Der Ansatz wurde 1h bei RT gerührt und anschließend wurden 2.894 mg (0.003 mmol) von Intermediat F209 gelöst in 500µl DMSO zugegeben. Nach weiteren 90 min Rühren bei RT wurde der Ansatz dann über mit PBS-Puffer pH8 equillibrierte PD 10-Säulen (Sephadex® G-25, GE Healthcare) gegeben und mit PBS-Puffer pH8 eluiert. Diese Lösung wurde über Nacht bei RT unter Argon gerührt und anschließend durch Ultrazentrifugation aufkonzentriert, rückverdünnt mit PBS-Puffer (pH 7.2) und erneut aufkonzentriert. Ein Teil des ADCs kann unter diesen Bedingungen auch in der ring-geschlossenen Form vorliegen. Der Anteil der ring-göffneten Form wurde bei diesem batch direkt nach der Synthese mit 23% bestimmt. Der erhaltene ADC batch wurde folgendermaßen charakterisiert:
Protein Konzentration: 11.1 mg/mL
Drug/mAb Ratio: 3.3

### Beispiel 2371

60 mg Nimotuzumab in 4587 µL ml PBS (c=13.08 mg/mL) wurden in Analogie zu Beispiel 237a mit Intermediat F209 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 15.88 mg/mL
Drug/mAb Ratio: 4.0

### Beispiel 238A

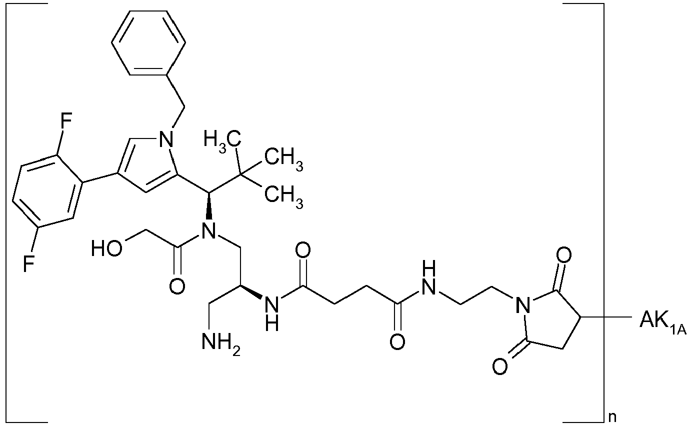

5 mg Cetuximab in 500 µL ml PBS (c= 10 mg/mL) wurden unter Argon mit einer Lösung aus 0,029 mg TCEP in 50µl PBS-Puffer versetzt. Der Ansatz wurde 30min bei RT gerührt und anschließend wurden 0.22 mg (0.00027 mmol) von Intermediat F238 gelöst in 50µl DMSO zugegeben. Nach weiteren 90 min Rühren bei RT wurde der Ansatz mit 1900 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde über Nacht bei RT unter Argon gerührt und anschließend durch Ultrazentrifugation aufkonzentriert und rückverdünnt mit PBS-Puffer (pH 7.2). Das so hergestellte ADC kann auch teilweise in Form der hydrolysierten offenkettigen Bernsteinsäureamide mit dem Antikörper verknüpft vorliegen. Der erhaltene ADC batch wurde folgendermaßen charakterisiert:
Protein Konzentration: 1.89 mg/mL
Drug/mAb Ratio: 2.9

### Beispiel 238B

5 mg anti-TWEAKR AK-1 in 500 µL ml PBS (c=10 mg/mL) wurden in Analogie zu Beispiel 238a mit Intermediat F238 gekuppelt. Das so hergestellte ADC kann auch teilweise in Form der hydrolysierten offenkettigen Bernsteinsäureamide mit dem Antikörper verknüpft vorliegen. Der erhaltene ADC batch wurde folgendermaßen charakterisiert:
Protein Konzentration: 1.66 mg/mL
Drug/mAb Ratio: 3.1

Unter diesen Kupplungsbedingungen lagen 26% des ADCs in Form der hydrolysierten offenkettigen Bernsteinsäureamide mit dem Antikörper verknüpft vor.

### Beispiel 238E

5 mg Trastuzumab in 500 µL ml PBS (c=10 mg/mL) wurden in Analogie zu Beispiel 238a mit Intermediat F238 gekuppelt. Das so hergestellte ADC kann auch teilweise in Form der hydrolysierten offenkettigen Bernsteinsäureamide mit dem Antikörper verknüpft vorliegen. Der erhaltene ADC batch wurde folgendermaßen charakterisiert:
Protein Konzentration: 1.84 mg/mL
Drug/mAb Ratio: 3.5

### Beispiel 239A

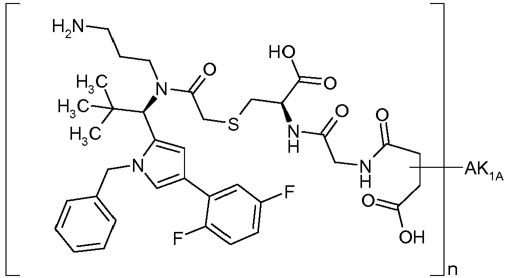

5 mg Cetuximab in 458 µL ml PBS (c=10.92 mg/mL) wurden unter Argon mit einer Lösung aus 0.029 mg TCEP in 50µl PBS-Puffer versetzt. Der Ansatz wurde mit 1892 µl PBS-Puffer, der zuvor auf pH 8 eingestellt wurde, verdünnt und 1 h bei RT gerührt. Anschließend wurden 0.19 mg (0.00027 mmol) von Intermediat F217 gelöst in 100µl DMSO zugegeben. Nach weiteren 90 min Rühren bei RT wurde der Ansatz über mit PBS-Puffer pH8 equillibrierte PD 10-Säulen (Sephadex® G-25, GE Healthcare) gegeben und mit PBS-Puffer pH8 eluiert. Das Eluat über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.86 mg/mL
Drug/mAb Ratio: 2.8

### Beispiel 239B

5 mg anti-TWEAKR AK-1 in 269 µL ml PBS (c=18.6 mg/mL) wurden unter Argon mit einer Lösung aus 0.029 mg TCEP in 50µl PBS-Puffer versetzt. Der Ansatz wurde mit 2081 µl PBS-Puffer, der zuvor auf pH 8 eingestellt wurde, verdünnt und 1 h bei RT gerührt. Anschließend wurden 0.19 mg (0.00027 mmol) von Intermediat F217 gelöst in 100µl DMSO zugegeben. Nach weiteren 90 min Rühren bei RT wurde der Ansatz über mit PBS-Puffer pH8 equillibrierte PD 10-Säulen (Sephadex® G-25, GE Healthcare) gegeben und mit PBS-Puffer pH8 eluiert. Das Eluat über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.28 mg/mL
Drug/mAb Ratio: 2.6

### Beispiel 2391

5 mg Nimotuzumab in 382 µL ml PBS (c=13.1 mg/mL) wurden unter Argon mit einer Lösung aus 0.029 mg TCEP in 50µl PBS-Puffer versetzt. Der Ansatz wurde mit 1968 µl PBS-Puffer, der zuvor auf pH 8 eingestellt wurde, verdünnt und 1 h bei RT gerührt. Anschließend wurden 0.19 mg (0.00027 mmol) von Intermediat F217 gelöst in 100µl DMSO zugegeben. Nach weiteren 90 min Rühren bei RT wurde der Ansatz über mit PBS-Puffer pH8 equillibrierte PD 10-Säulen (Sephadex® G-25, GE Healthcare) gegeben und mit PBS-Puffer pH8 eluiert. Das Eluat über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.32 mg/mL
Drug/mAb Ratio: 2.9

Bei dieser ADC-Präparation wurde ein Anteil der ring-geöffneten Bernsteinsäureamid-Form von 89% ermittelt.

### Beispiel 239H

5 mg Panitumumab in 74 µL ml PBS (c=67.5 mg/mL) wurden unter Argon mit einer Lösung aus 0.048 mg TCEP in 83µl PBS-Puffer versetzt. Der Ansatz wurde mit 2243 µl PBS-Puffer, der zuvor auf pH 8 eingestellt wurde, verdünnt und 4 h bei RT gerührt. Anschließend wurden 0.19 mg (0.00027 mmol) von Intermediat F217 gelöst in 100µl DMSO zugegeben. Der Ansatz wurde über Nacht bei RT gerührt. Die Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.33 mg/mL
Drug/mAb Ratio: 2.1

### Beispiel 240A

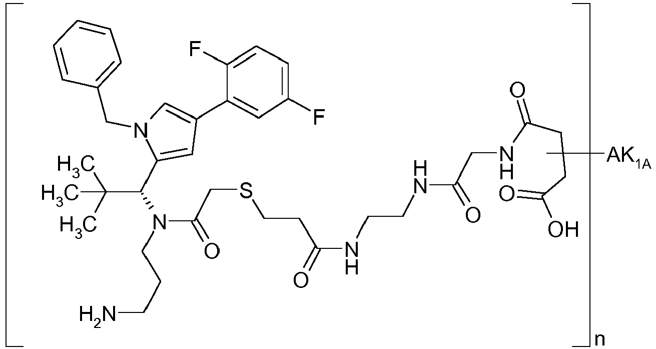

50 mg Cetuximab in 4579 µL ml PBS (c=10.92 mg/mL) wurden unter Argon mit einer Lösung aus 0.29 mg TCEP in 500µl PBS-Puffer versetzt. Der Ansatz wurde mit 7421 µl PBS-Puffer, der zuvor auf pH 8 eingestellt wurde, verdünnt und 1 h bei RT gerührt. Anschließend wurden 1.4 mg (0.0027 mmol) von Intermediat F213 gelöst in 500µl DMSO zugegeben. Nach weiteren 90 min Rühren bei RT wurde der Ansatz über mit PBS-Puffer pH8 equillibrierte PD 10-Säulen (Sephadex® G-25, GE Healthcare) gegeben und mit PBS-Puffer pH8 eluiert. Das Eluat über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 15.63 mg/mL
Drug/mAb Ratio: 2.7

### Beispiel 240B

5 mg anti-TWEAKR AK-1 in 500 µL ml PBS (c=10 mg/mL) wurden in Analogie zu Beispiel 239a mit Intermediat F213 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 0.9 mg/mL
Drug/mAb Ratio: 2.0

### Beispiel 240E

5 mg Trastuzumab in 500 µL ml PBS (c=10 mg/mL) wurden in Analogie zu Beispiel 239a mit Intermediat F213 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.4 mg/mL
Drug/mAb Ratio: 2.1

### Beispiel 241A

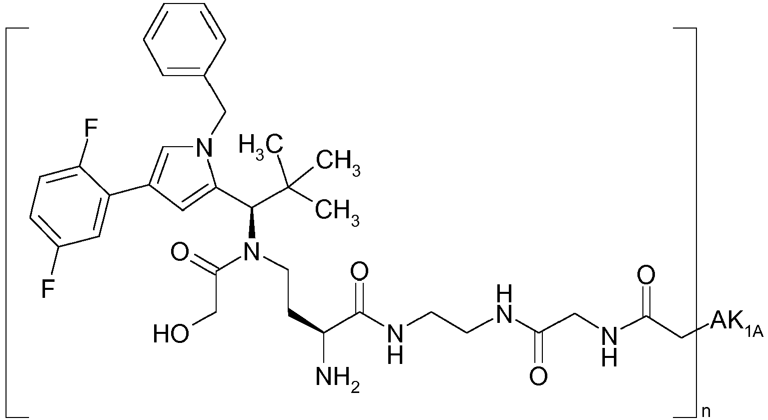

30 mg Cetuximab in 3 mL ml PBS (c=10 mg/mL) wurden unter Argon mit einer Lösung aus 0.172 mg TCEP in 300µl PBS-Puffer versetzt. Der Ansatz wurde 30min bei RT gerührt und anschließend wurden 1.36 mg (1.6 µmol) von Intermediat F241 gelöst in 330µl DMSO zugegeben. Nach 20h Rühren bei RT wurde der Ansatz mit 1.37 ml PBS Puffer verdünnt und über PD 10-Säulen (Sephadex® G-25, GE Healthcare) mit PBS-Puffer eluiert. Anschließend wurde durch Ultrazentrifugation aufkonzentriert, rückverdünnt mit PBS-Puffer (pH 7.2) und erneut aufkonzentriert. Der erhaltene ADC batch wurde folgendermaßen charakterisiert:
Protein Konzentration: 9.98 mg/mL
Drug/mAb Ratio: 3.3

### Beispiel 241B

Eingesetzt wurden hier zur Kupplung mit Intermediat F241 5 mg anti-TWEAKR AK-1 in PBS (c=10 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 1.39 mg/mL
Drug/mAb Ratio: 3.9

### Beispiel 241E

Eingesetzt wurden hier zur Kupplung mit Intermediat F241 5 mg Trastuzumab in PBS (c=10 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 1.83 mg/mL
Drug/mAb Ratio: 4.7

### Beispiel 2411

Eingesetzt wurden hier zur Kupplung mit Intermediat F241 5 mg Nimotuzumab in PBS (c=10 mg/mL). Die Kupplung mit dem Antikörper wurde nach der TCEP-Reduktion unter Rühren über Nacht durchgeführt bevor die weitere Aufarbeitung mittels Sephadex-Reinigung begann. Der Ansatz wurde nach der Sephadex-Reinigung durch Ultrazentrifugation aufkonzentriert und rückverdünnt mit PBS.
Protein Konzentration: 1.6 mg/mL
Drug/mAb Ratio: 4.0

### Beispiel 242A

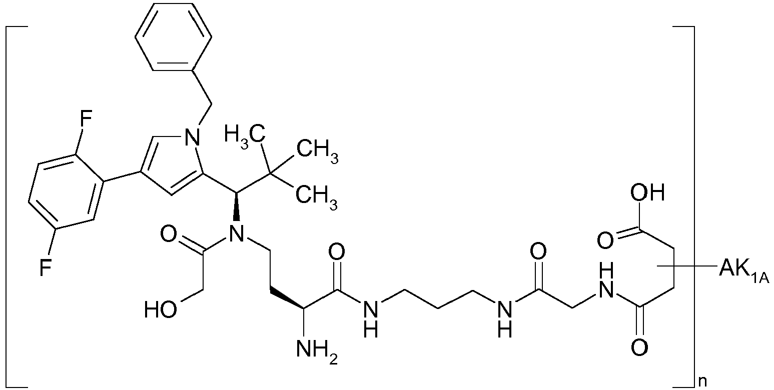

5 mg Cetuximab in 500 µL ml PBS (c= 10 mg/mL) wurden unter Argon mit einer Lösung aus 0,029 mg TCEP in 50µl PBS-Puffer versetzt. Der Ansatz wurde 30min bei RT gerührt und anschließend wurden 0.22 mg (0.00027 mmol) von Intermediat F242 gelöst in 50µl DMSO zugegeben. Nach weiteren 90 min Rühren bei RT wurde der Ansatz mit 1900 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.92 mg/mL
Drug/mAb Ratio: 2.7

### Beispiel 242B

5 mg anti-TWEAKR AK-1 in 500 µL PBS (c=10 mg/mL) wurden wie unter Beispiel 242A beschrieben mit 0.22 mg von Intermediat F242 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.54 mg/mL
Drug/mAb Ratio: 3.1

### Beispiel 242E

5 mg Trastuzumab in 500 µL PBS (c=10 mg/mL) wurden wie unter Beispiel 242A beschrieben mit 0.22 mg von Intermediat F242 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.76 mg/mL
Drug/mAb Ratio: 3.6

### Beispiel 243A

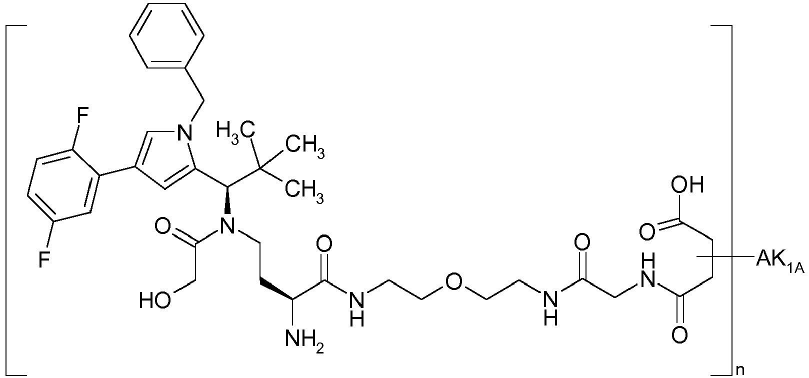

5 mg Cetuximab in 500 µL ml PBS (c= 10 mg/mL) wurden unter Argon mit einer Lösung aus 0,029 mg TCEP in 50µl PBS-Puffer versetzt. Der Ansatz wurde 30min bei RT gerührt und anschließend wurden 0.23 mg (0.00027 mmol) von Intermediat F243 gelöst in 50µl DMSO zugegeben. Nach weiteren 90 min Rühren bei RT wurde der Ansatz mit 1900 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.92 mg/mL
Drug/mAb Ratio: 2.9

### Beispiel 243B

5 mg anti-TWEAKR AK-1 in 500 µL PBS (c=10 mg/mL) wurden wie unter Beispiel 243A beschrieben mit 0.23 mg von Intermediat F243 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.63 mg/mL
Drug/mAb Ratio: 3.1

### Beispiel 243E

5 mg Trastuzumab in 500 µL PBS (c=10 mg/mL) wurden wie unter Beispiel 243A beschrieben mit 0.23 mg von Intermediat F243 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.8 mg/mL
Drug/mAb Ratio: 3.5

### Beispiel 2431

5 mg Nimotuzumab in 500 µL PBS (c=10 mg/mL) wurden wie unter Beispiel 243A beschrieben mit 0.23 mg von Intermediat F243 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.87 mg/mL
Drug/mAb Ratio: 3.1

### Beispiel 244A

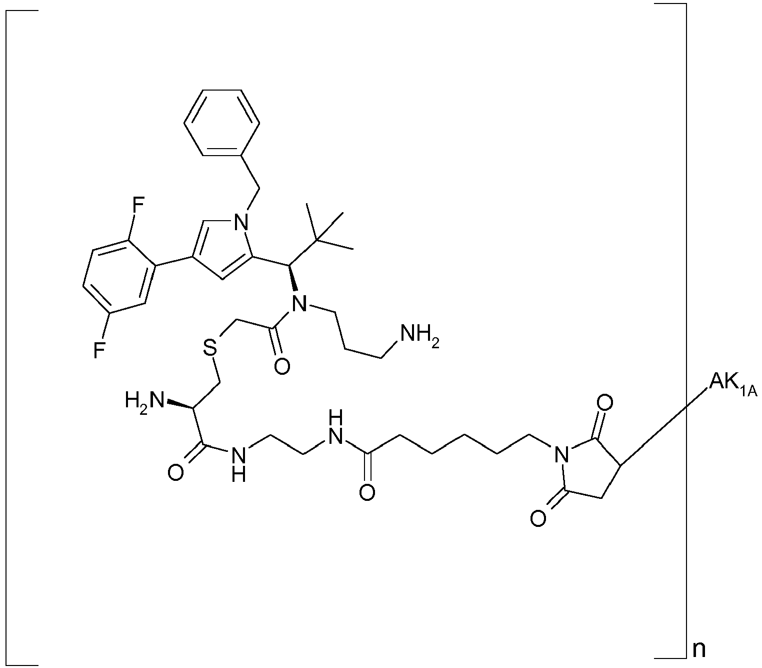

Eingesetzt wurden hier zur Kupplung mit Intermediat F244 5.0 mg Cetuximab PBS (c = 23.10 mg/mL) und der Ansatz wurde nach der Sephadex-Reinigung durch Ultrazentrifugation aufkonzentriert und rückverdünnt.
Protein Konzentration: 2.12 mg/mL
Drug/mAb Ratio: 3.5

### Beispiel 244B

Eingesetzt wurden hier zur Kupplung mit Intermediat F244 5.0 mg anti-TWEAK AK-1 PBS (c = 18.60 mg/mL) und der Ansatz wurde nach der Sephadex-Reinigung durch Ultrazentrifugation aufkonzentriert und rückverdünnt.
Protein Konzentration: 1.65 mg/mL
Drug/mAb Ratio: 3.5

### Beispiel 244E

Eingesetzt wurden hier zur Kupplung mit Intermediat F244 5.0 mg Trastuzumab in PBS (c = 13.50 mg/mL) und der Ansatz wurde nach der Sephadex-Reinigung durch Ultrazentrifugation aufkonzentriert und rückverdünnt.
Protein Konzentration: 1.91 mg/mL
Drug/mAb Ratio: 3.5

### Beispiel 2441

Eingesetzt wurden hier zur Kupplung mit Intermediat F244 5.0 mg Nimotuzumab in PBS (c = 13.08 mg/mL) und der Ansatz wurde nach der Sephadex-Reinigung durch Ultrazentrifugation aufkonzentriert und rückverdünnt.
Protein Konzentration: 1.91 mg/mL
Drug/mAb Ratio: 2.9

### Beispiel 245A

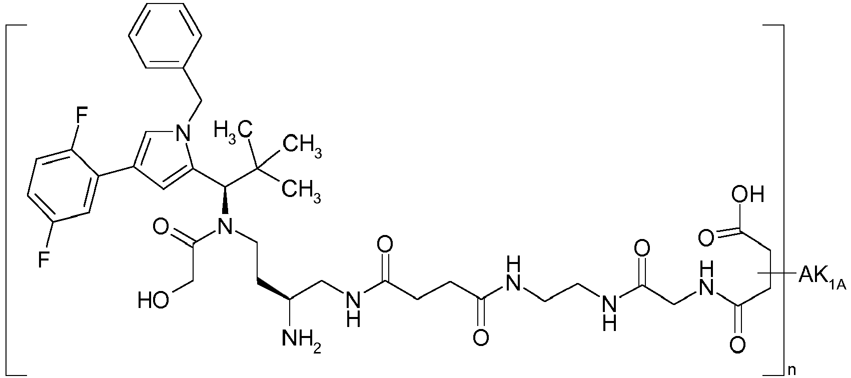

5 mg Cetuximab in 500 µL ml PBS (c= 10 mg/mL) wurden unter Argon mit einer Lösung aus 0,029 mg TCEP in 50µl PBS-Puffer versetzt. Der Ansatz wurde 30min bei RT gerührt und anschließend wurden 0.24 mg (0.00027 mmol) von Intermediat F245 gelöst in 50µl DMSO zugegeben. Nach weiteren 90 min Rühren bei RT wurde der Ansatz mit 1900 µl PBS-Puffer, der zuvor auf pH 8 eingestellt wurde, verdünnt.

Diese Lösung wurde dann über mit PBS-Puffer pH8 equillibrierte PD 10-Säulen (Sephadex® G-25, GE Healthcare) gegeben und mit PBS-Puffer pH8 eluiert. Das Eluat wurde über Nacht bei RT unter Argon gerührt und anschließend durch Ultrazentrifugation aufkonzentriert und rückverdünnt mit PBS-Puffer (pH 7.2). Ein Teil des ADCs kann unter diesen Bedingungen auch in der ring-geschlossenen Form vorliegen. Der erhaltene ADC batch wurde folgendermaßen charakterisiert:
Protein Konzentration: 1.69 mg/mL
Drug/mAb Ratio: 2.4

### Beispiel 245B

5 mg anti-TWEAKR AK-1 in 500 µL PBS (c=10 mg/mL) wurden wie unter Beispiel 245A beschrieben mit 0.24 mg von Intermediat F245 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.51 mg/mL
Drug/mAb Ratio: 2.5

### Beispiel 245E

5 mg Trastuzumab in 500 µL PBS (c=10 mg/mL) wurden wie unter Beispiel 245A beschrieben mit 0.24 mg von Intermediat F245 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.8 mg/mL
Drug/mAb Ratio: 3.5

### Beispiel 2451

5 mg Nimotuzumab in 500 µL PBS (c=10 mg/mL) wurden wie unter Beispiel 245A beschrieben mit 0.24 mg von Intermediat F245 gekuppelt. Ein Teil des ADCs kann unter diesen Bedingungen auch in der ring-geschlossenen Form vorliegen. Der erhaltene ADC batch wurde folgendermaßen charakterisiert:
Protein Konzentration: 1.65 mg/mL
Drug/mAb Ratio: 2.3

### Beispiel 246

### 4-[(2-{[(2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-2-carboxyethyl]amino}-2-oxoethyl)amino]-3-{[(2R)-2-amino-2-carboxyethyl]sulfanyl} -4-oxobutansäure -trifluoressigsäure (1:1) und 4-[(2-{[(2R)-2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)-2-carboxyethyl]amino}-2-oxoethyl)amino]-2-{[(2R)-2-amino-2-carboxyethyl]sulfanyl}-4-oxobutansäure-trifluoressigsäure (1:1)

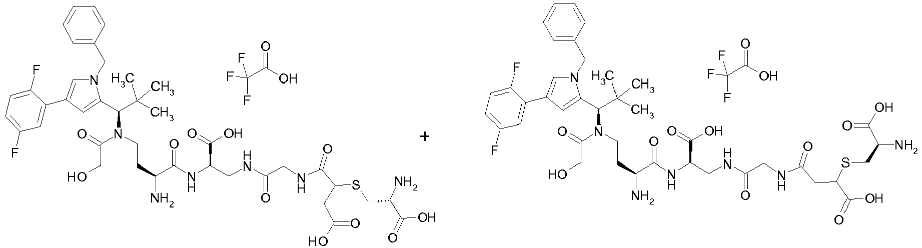

Zunächst wurde L-Cystein mit 1-({[2-(Trimethylsilyl)ethoxy]carbonyl}oxy)pyrrolidine-2,5-dion in DMF in Gegenwart von *N,N-*Diisopropylethylamin in N-{[2-(Trimethylsilyl)ethoxy]carbonyl}-L-cystein überführt.

11 mg (0.013 mmol) von Intermediat F193 und 8 mg (0.016 mmol) N-{[2-(Trimethylsilyl) ethoxy]carbonyl}-L-cystein wurden 3 ml DMF gelöst und der Ansatz 20 h bei RT gerührt. Anschließend wurde eingeengt und der Rückstand durch präparative HPLC gereinigt.

Nach Vereinigung der entsprechenden Fraktionen und Abdampfen der Lösemittel im Vakuum wurde der Rückstand in 2 mL THF/Wasser 1:1 gelöst. Es wurden 19 µL einer 2M wässrigen Lithiumhydroxidlösung zugegeben und der Ansatz 1 h bei RT gerührt. Dann wurden nochmals 19 µL der 2M wässrigen Lithiumhydroxidlösung zugegeben und der Ansatz über Nacht bei RT gerührt. Anschließend wurde mit einer 1M Salzsäure neutral gestellt, das Lösemittel im Vakuum abgedampft und der Rückstand durch präparative HPLC gereinigt. Es wurden 4.1 mg (38% d. Th.) der regioisomeren geschützten Intermediate als farbloser Schaum erhalten.
LC-MS (Methode 1): Rₜ = 1.03 min (breit); MS (ESIpos): m/z = 1020 (M+H)⁺.

Im letzten Schritt wurden 4.1 mg (0.004 mmol) von diesem Intermediat in 3 mL 2,2,2-Trifluorethanol gelöst. Es wurden 3 mg (0.022 mmol) Zinkchlorid zugegeben und der Ansatz 1 h bei 50°C gerührt. Anschließend wurden 6 mg (0.022 mmol) Ethylendiamin-N,N,N',N'-tetraessigsäure und 2 ml einer 0.1%-igen wässrigen Trifluoressigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 5 mg (quant.) der Titelverbindung als Regioisomerengemisch imVerhältnis 20:80 erhalten.
LC-MS (Methode 1): Rₜ = 0.78 min (breit); MS (ESIpos): m/z = 876 (M+H)⁺.
LC-MS (Methode 5): Rₜ = 2.36 min und 2.39 min; MS (ESIpos): m/z = 876 (M+H)⁺.

### Beispiel 247A

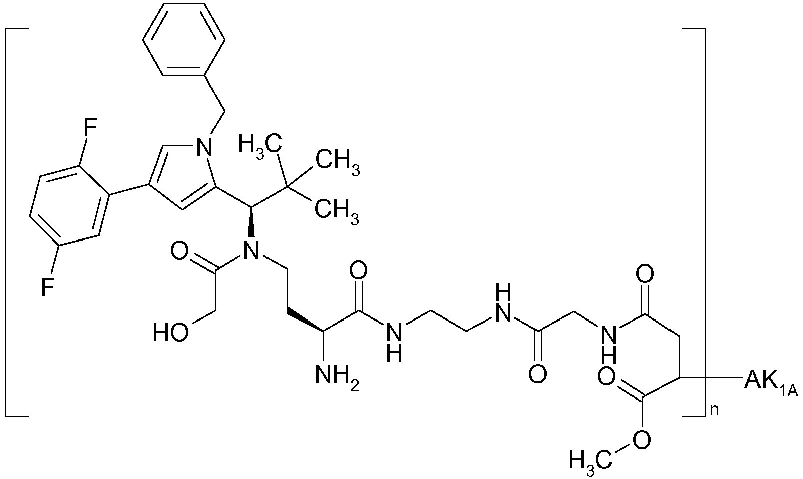

5 mg Cetuximab in 500 µL ml PBS (c=10 mg/mL) wurden unter Argon mit einer Lösung aus 0.029 mg TCEP in 50 µl PBS-Puffer versetzt. Der Ansatz wurde 30 min bei RT gerührt und anschließend wurden 0.264 mg (0.27 µmol) von Intermediat F247 gelöst in 50 µl DMSO zugegeben. Nach 20h Rühren bei RT wurde der Ansatz mit 1.9 ml PBS Puffer verdünnt und über PD 10-Säulen (Sephadex® G-25, GE Healthcare) mit PBS-Puffer eluiert. Anschließend wurde durch Ultrazentrifugation aufkonzentriert, rückverdünnt mit PBS-Puffer (pH 7.2) und erneut aufkonzentriert. Der erhaltene ADC batch wurde folgendermaßen charakterisiert:
Protein Konzentration: 1.66 mg/mL
Drug/mAb Ratio: 2.2

### Beispiel 247B

Eingesetzt wurden hier zur Kupplung mit Intermediat F247 5 mg anti-TWEAKR AK-1 in PBS (c=10 mg/mL) und die Kupplung und Aufarbeitung wurde wie in Beispiel 247A beschrieben durchgeführt.
Protein Konzentration: 1.49 mg/mL
Drug/mAb Ratio: 2.6

### Beispiel 247E

Eingesetzt wurden hier zur Kupplung mit Intermediat F247 5 mg Trastuzumab in PBS (c=10 mg/mL) und die Kupplung und Aufarbeitung wurde wie in Beispiel 247A beschrieben durchgeführt.
Protein Konzentration: 1.67 mg/mL
Drug/mAb Ratio: 2.3

### Beispiel 2471

Eingesetzt wurden hier zur Kupplung mit Intermediat F247 5 mg Nimotuzumab in PBS (c=10 mg/mL) und die Kupplung und Aufarbeitung wurde wie in Beispiel 247A beschrieben durchgeführt.
Protein Konzentration: 1.62 mg/mL
Drug/mAb Ratio: 2.4

### Beispiel 248A

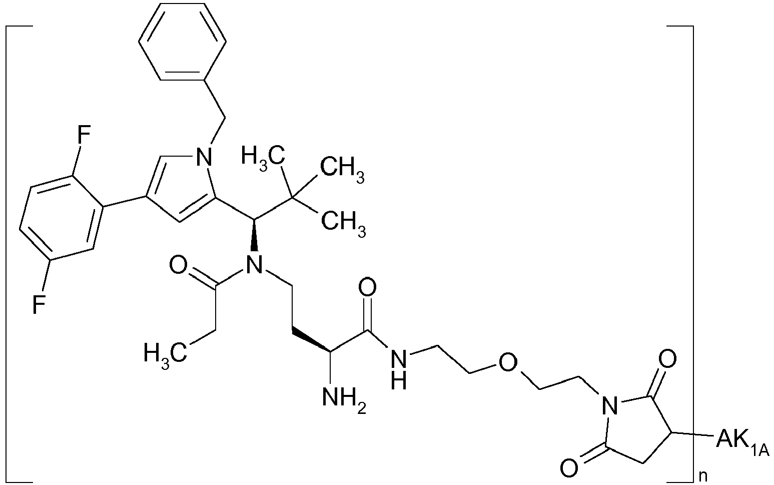

Eingesetzt wurden hier in Analogie zu Beispiel 5A zur Kupplung mit Intermediat F248 5 mg Cetuximab in PBS (c=10.92 mg/mL) und der Ansatz wurde nach der Sephadex-Reinigung durch Ultrazentrifugation aufkonzentriert und rückverdünnt mit PBS. Ein Teil des so hergestellten ADCs kann in Form der hydrolysierten offenkettigen Bernsteinsäureamide mit dem Antikörper verknüpft vorliegen.
Protein Konzentration: 2.06 mg/mL
Drug/mAb Ratio: 3.8

### Beispiel 248B

Eingesetzt wurden hier in Analogie zu Beispiel 5B zur Kupplung mit Intermediat F248 5 mg anti-TWEAKR AK-1 in PBS (c=18.6 mg/mL) und der Ansatz wurde nach der Sephadex-Reinigung durch Ultrazentrifugation aufkonzentriert und rückverdünnt mit PBS. Ein Teil des so hergestellten ADCs kann in Form der hydrolysierten offenkettigen Bernsteinsäureamide mit dem Antikörper verknüpft vorliegen.
Protein Konzentration: 1.84 mg/mL
Drug/mAb Ratio: 4.1

### Beispiel 249

### S-{1-[6-({(2S)-2-Amino-4-[-{(1R)-1-[1-benzyl-4-(2,5-difluorphenyl)-1H-pyrrol-2-yl]-2,2-dimethylpropyl}(glycoloyl)amino]butanoyl}amino)hexyl]-2,5-dioxopyrrolidin-3-yl}-L-cystein-trifluoressigsäure (1:1)

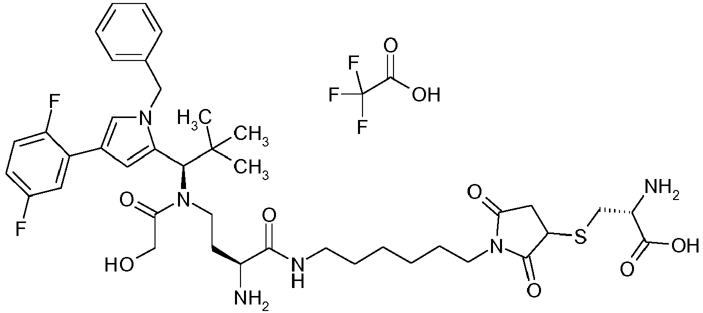

11 mg (14 µmol) von Intermediat F179 wurden in 2.2 ml DMF aufgenommen und mit 3.3 mg (27 µmol) L-Cystein versetzt. Das Reaktionsgemisch wurde 3 h bei RT gerührt und dann im Vakuum eingeengt. Der Rückstand wurde mittels präparativer HPLC gereinigt. Die entsprechenden Fraktionen wurden eingeengt und nach Lyophilisation des Rückstands aus Acetonitril/Wasser verblieben 7.3 mg (58% d. Th.) der Titelverbindung als farbloser Schaum.
LC-MS (Methode 4): Rₜ = 1.04 min; MS (EIpos): m/z = 813 [M+H]⁺.

### Beispiel 250

### 4-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-3-{[(2R)-2-amino-2-carboxyethyl]sulfanyl}-4-oxobutansäure-trifluoressigsäure (1:1) und 4-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-{[(2R)-2-amino-2-carboxyethyl]sulfanyl}-4-oxobutansäure-trifluoressigsäure (1:1)

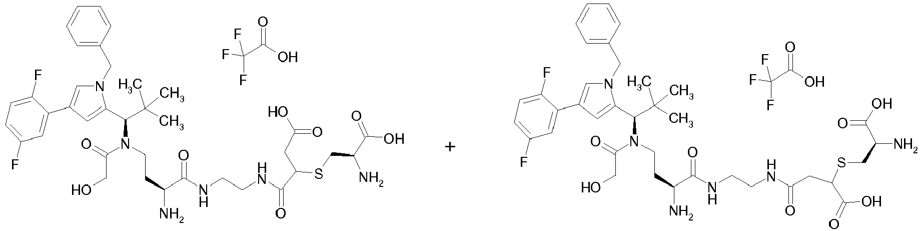

10 mg (0.013 mmol) von Intermediat F85 und 5.3 mg (0.02 mmol) N-{[2-(Trimethylsilyl) ethoxy]carbonyl}-L-cystein 3 ml DMF gelöst und der Ansatz 3 Tage bei RT gerührt. Anschließend wurde eingeengt und der Rückstand durch präparative HPLC gereinigt. Nach Vereinigung der entsprechenden Fraktionen und Abdampfen der Lösemittel im Vakuum wurde der Rückstand in 2 mL THF/Wasser 1:1 gelöst. Es wurden 9 µL einer 2M wässrigen Lithiumhydroxidlösung zugegeben und der Ansatz 1 h bei RT gerührt. Anschließend wurde der Ansatz mit einer 1M Salzsäure auf einen pH-Wert von ∼3 eingestellt, das Lösemittel im Vakuum abgedampft und der Rückstand durch präparative HPLC gereinigt. Es wurden 3 mg (24% d. Th. Über 2 Stufen) der regioisomeren geschützten Intermediate als farbloser Schaum erhalten.
LC-MS (Methode 5): Rₜ = 3.39 min und 3.43 min; MS (ESIpos): m/z = 919 (M+H)⁺.

Im letzten Schritt wurden 3 mg (0.0033 mmol) von diesem Intermediat in 3 mL 2,2,2-Trifluorethanol gelöst. Es wurden 2.2 mg (0.016 mmol) Zinkchlorid zugegeben und der Ansatz 3.5 h bei 50°C gerührt. Anschließend wurden 4.8 mg (0.016 mmol) Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 1 mg (33% d. Th.) der Titelverbindung als Regioisomerengemisch imVerhältnis 43:34 erhalten. Ein weiteres Isomer war mit 23% im Isomerengemisch (RT = 2.51) vertreten.
LC-MS (Methode 5): Rₜ = 2.57 min und 2.62 min; MS (ESIpos): m/z = 775 (M+H)⁺.

### Beispiel 251

### S-(1-{2-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethoxy]ethyl}-2,5-dioxopyrrolidin-3-yl)-L-cystein-trifluoressigsäure (1:1)

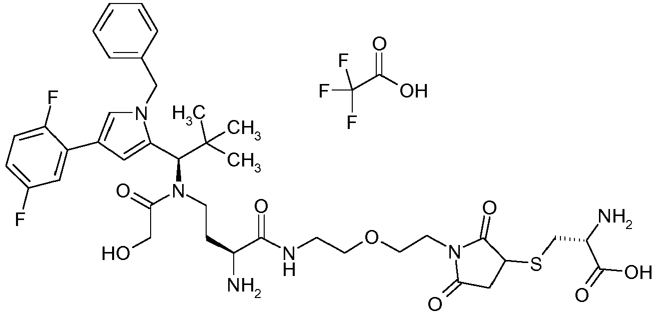

3 mg (4 µmol) von Intermediat F248 wurden in 2 ml DMF aufgenommen und mit 0.9 mg (8 µmol) L-Cystein versetzt. Das Reaktionsgemisch wurde 18 h bei RT gerührt und dann im Vakuum eingeengt. Der Rückstand wurde mittels präparativer HPLC gereinigt. Die entsprechenden Fraktionen wurden eingeengt und nach Lyophilisation des Rückstands aus Acetonitril/Wasser verblieben 1.1 mg (32% d. Th.) der Titelverbindung als weißer Feststoff.
LC-MS (Methode 1): Rₜ = 0.78 min; MS (EIpos): m/z = 801 [M+H]⁺.

### Beispiel 252

### (3R,7S)-7-Amino-17-{[(2R)-2-amino-2-carboxyethyl]sulfanyl}-3-[1-benzyl-4-(2,5-difluorphenyl)-1H-pyrrol-2-yl]-4-glycoloyl-2,2-dimethyl-8,16-dioxo-12-oxa-4,9,15-triazanonadecan-19-säure-trifluoressigsäure (1:1) und (3R,7S)-7-Amino-18-{[(2R)-2-amino-2-carboxyethyl]sulfanyl}-3-[1-benzyl-4-(2,5-difluorphenyl)-1H-pyrrol-2-yl]-4-glycoloyl-2,2-dimethyl-8,16-dioxo-12-oxa-4,9,15-triazanonadecan-19-säure-trifluoressigsäure (1:1)

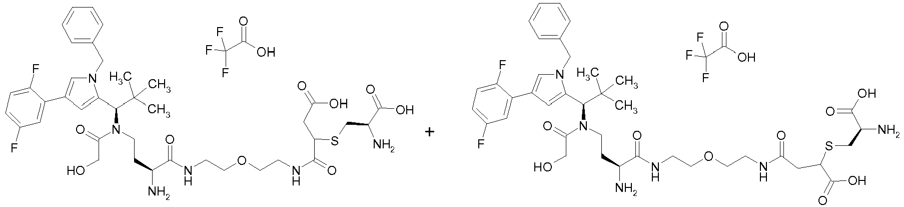

8 mg (0.010 mmol) von der geschützten Zwischenstufe von Intermediat F248 und 5.1 mg (0.02 mmol) N-{[2-(Trimethylsilyl) ethoxy]carbonyl}-L-cystein 3 ml DMF gelöst und der Ansatz 18 h bei RT gerührt und anschließend 2 h im Ultraschallbad behandelt. Anschließend wurde eingeengt und der Rückstand durch präparative HPLC gereinigt. Nach Vereinigung der entsprechenden Fraktionen und Abdampfen der Lösemittel im Vakuum wurde der Rückstand in 2 mL THF/Wasser 1:1 gelöst. Es wurden 15 µL einer 2M wässrigen Lithiumhydroxidlösung zugegeben und der Ansatz 15 min bei RT gerührt. Anschließend wurde der Ansatz mit einer 1M Salzsäure auf einen pH-Wert von ∼3 eingestellt, mit 20 ml Kochsalzlösung verdünnt und zweimal mit 20 ml Ethylacetat extrahiert. Die organische Phase wurde über Magnesiumsulfat getrocknet, eingeengt und der Rückstand aus Acetonitril/Wasser lyophilisiert. Es wurden 8.4 mg (78% d. Th. über 2 Stufen) der regioisomeren geschützten Intermediate als farbloser Schaum erhalten.
LC-MS (Methode 1): Rₜ = 1.44 min und 3.43 min; MS (ESIpos): m/z = 1107 (M+H)⁺.

Im letzten Schritt wurden 8 mg (0.007 mmol) von diesem Intermediat in 5 mL 2,2,2-Trifluorethanol gelöst. Es wurden 9.8 mg (0.072 mmol) Zinkchlorid zugegeben und der Ansatz 1.5 h bei 50°C gerührt. Anschließend wurde Ethylendiamin-N,N,N',N'-tetraessigsäure zugesetzt und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 4 mg (59% d. Th.) der Titelverbindung als Regioisomerengemisch imVerhältnis 31:67 erhalten.
LC-MS (Methode 1): Rₜ = 0.79 min und 0.81 min; MS (ESIpos): m/z = 819 (M+H)⁺.

### Beispiel 253

### 4-({2-[(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)amino]ethyl}amino)-3-{[(2R)-2-amino-2-carboxyethyl]sulfanyl}-4-oxobutansäure -trifluoressigsäure (1:1) und 4-({2-[(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)amino]ethyl}amino)-2-{[(2R)-2-amino-2-carboxyethyl]sulfanyl}-4-oxobutansäure -trifluoressigsäure (1:1)

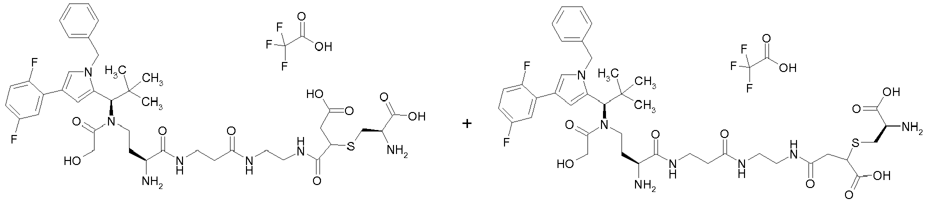

Die isomeren Titelverbindungen wurden in Analogie zu Beispiel 250 aus Intermediat F84 und N-{[2-(Trimethylsilyl)ethoxy]carbonyl}-L-cysteinhergestellt.

### Beispiel 254A

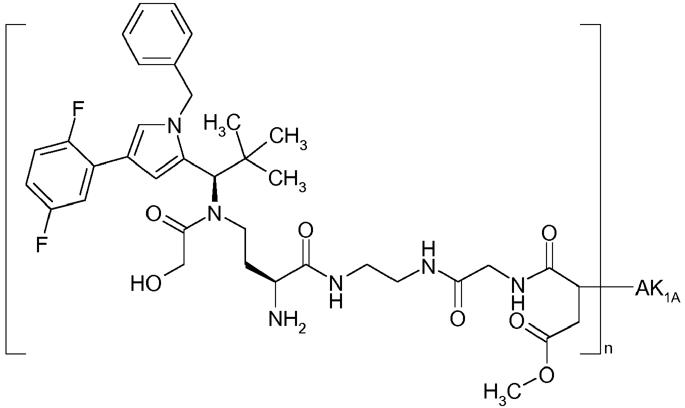

5 mg Cetuximab in 500 µL ml PBS (c=10 mg/mL) wurden unter Argon mit einer Lösung aus 0.029 mg TCEP in 50 µl PBS-Puffer versetzt. Der Ansatz wurde 30 min bei RT gerührt und anschließend wurden 0.264 mg (0.27 µmol) von Intermediat F254 gelöst in 50 µl DMSO zugegeben. Nach 20h Rühren bei RT wurde der Ansatz mit 1.9 ml PBS Puffer verdünnt und über PD 10-Säulen (Sephadex® G-25, GE Healthcare) mit PBS-Puffer eluiert. Anschließend wurde durch Ultrazentrifugation aufkonzentriert, rückverdünnt mit PBS-Puffer (pH 7.2) und erneut aufkonzentriert. Der erhaltene ADC batch wurde folgendermaßen charakterisiert:
Protein Konzentration: 1.74 mg/mL
Drug/mAb Ratio: 2.2

### Beispiel 254B

Eingesetzt wurden hier zur Kupplung mit Intermediat F254 5 mg anti-TWEAKR AK-1 in PBS (c=10 mg/mL) und die Kupplung und Aufarbeitung wurde wie in Beispiel 254A beschrieben durchgeführt.
Protein Konzentration: 1.8 mg/mL
Drug/mAb Ratio: 2.5

### Beispiel 254E

Eingesetzt wurden hier zur Kupplung mit Intermediat F254 5 mg Trastuzumab in PBS (c=10 mg/mL) und die Kupplung und Aufarbeitung wurde wie in Beispiel 254A beschrieben durchgeführt.
Protein Konzentration: 1.74 mg/mL
Drug/mAb Ratio: 2.4

### Beispiel 2541

Eingesetzt wurden hier zur Kupplung mit Intermediat F254 5 mg Nimotuzumab in PBS (c=10 mg/mL) und die Kupplung und Aufarbeitung wurde wie in Beispiel 254A beschrieben durchgeführt.
Protein Konzentration: 1.73 mg/mL
Drug/mAb Ratio: 2.0

### Beispiel 255

### (2R,28R)-28-Amino-2-[({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)methyl]-25-(carboxymethyl)-4,20,24-trioxo-7,10,13,16-tetraoxa-26-thia-3,19,23-triazanonacosan-1,29-disäure-trifluoressigsäure (1:2) und (1R,28R,34R)-1-Amino-33-(3-aminopropyl)-34-[1-benzyl-4-(2,5-difluorphenyl)-1H-pyrrol-2-yl]-35,35-dimethyl-6,10,26,32-tetraoxo-14,17,20,23-tetraoxa-3,30-dithia-7,11,27,33-tetraazahexatriacontan-1,4,28-tricarbonsäure -trifluoressigsäure (1:2)

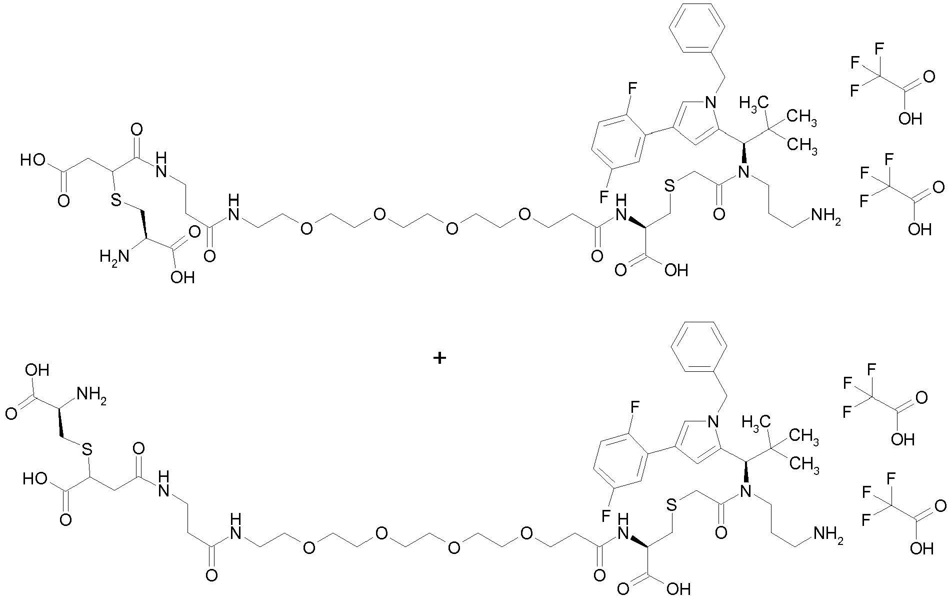

20 mg (0.018 mmol) R-{2-[(3-Aminopropyl){(1R)-1-[1-benzyl-4-(2,5-difluorphenyl)-1H-pyrrol-2-yl]-2,2-dimethylpropyl}amino]-2-oxoethyl}-N-[19-(2,5-dioxo-2,5-dihydro-1H-pyrrol-1-yl)-17-oxo-4,7,10,13-tetraoxa-16-azanonadecan-1-oyl]-L-cystein-trifluoressigsäure (1:1) (Intermediat F209) und 9.78 mg (0.036 mmol) N-{[2-(Trimethylsilyl) ethoxy]carbonyl}-L-cystein wurden in 2 ml DMF gelöst und der Ansatz 18 h bei RT gerührt. Die Reaktionsmischung wurde im Vakuum verdampft. Der Rückstand (47.7 mg) wurde in 3 mL THF/Wasser 1:1 gelöst. Es wurden 0.08 mL einer 2M wässrigen Lithiumhydroxidlösung zugegeben und der Ansatz 1 Stunde bei RT gerührt. Anschließend wurde der Ansatz mit 9.26 mg (0.15 mmol) Essigsäure auf einen pH-Wert von ∼7 eingestellt. Die Reaktionsmischung wurde direkt mittels präp. RP-HPLC gereinigt (Säule: Reprosil 125x30; 10µ, Fluss: 50 mL/min, MeCN/Wasser; 0.1 % TFA). Die Lösemittel wurden im Vakuum verdampft und der Rückstand im Hochvakuum getrocknet. Es wurden 15.3 mg (29% über 2 Stufen) der regioisomeren geschützten Intermediate erhalten.
LC-MS (Methode 6): Rₜ = 12.26 min und 12.30min; MS (ESIpos): m/z = 1254 (M+H)⁺.

Im letzten Schritt wurden 15.3 mg (0.01 mmol) von diesem Intermediat in 2 mL 2,2,2-Trifluorethanol gelöst. Es wurden 6.1 mg (0.05 mmol) Zinkchlorid zugegeben und der Ansatz 2 h bei 50°C gerührt. Anschließend wurde 13.1 mg (0.05 mmol) Ethylendiamin-N,N,N',N'-tetraessigsäure zugesetzt und das Lösemitte wurde mittels präparativer HPLC gereinigt. Nach Einengen der entsprechenden Fraktionen und Lyophilisation des Rückstands aus Acetonitril/Wasser wurden 11.9 mg (79.5%) der Titelverbindung als Regioisomerengemisch erhalten.
LC-MS (Methode 1): Rₜ = 0.85 min; MS (ESIpos): m/z = 1110 (M+H)⁺.

### Beispiel 256

### (3R)-6-{(11S,15R)-11-Amino-15-[1-benzyl-4-(2,5-difluorphenyl)-1H-pyrrol-2-yl]-14-glycoloyl-16,16-dimethyl-2,5,10-trioxo-3,6,9,14-tetraazaheptadec-1-yl}-5-oxothiomorpholin-3-carbonsäure-trifluoressigsäure (1:1)

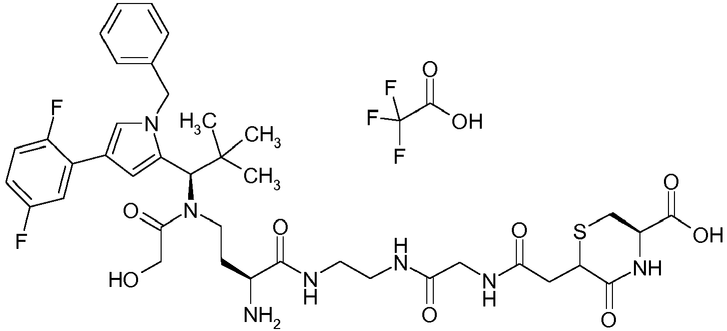

4 mg (0.004 mmol) der Verbindung aus Beispiel 135 wurden in 4 mL THF/Wasser gelöst und mit 48 µL einer 2molaren wässrigen Lithiumhydroxidlösung versetzt. Der Ansatz wurde 1 h bei RT gerührt und anschließend eingeengt und mittels präparativer HPLC gereinigt. Nach Vereinigung, Einengen und Lyophilisation aus Acetonitril/Wasser der entsprechenden Fraktionen wurden 2.4 mg (60% d. Th.) der Titelverbindung erhalten.
LC-MS (Methode 1): Rₜ = 0.86 min; MS (EIpos): m/z = 814 [M+H]⁺.

### C: Bewertung der biologischen Wirksamkeit

Die biologische Wirkung der erfindungsgemäßen Verbindungen kann durch die nachstehend beschriebenen Assays gezeigt werden:

### m. C-1a Bestimmung der cytotoxischen Wirkung der gegen TWEAKR gerichteten ADCs

Die Analyse der cytototoxischen Wirkung der anti-TWEAKR-ADCs erfolgt auf verschiedenen Zelllinien:
NCI-H292: humane mukoepidermoide Lungenkarzinomzellen, ATCC-CRL-1848, Standardmedium: RPMI 1640 (Biochrom; #FG1215, stab. Glutamin) + 10% FCS (Biochrom; #S0415), TWEAKR-positiv, EGFR-positiv.
BxPC3: humane Bauchspeicheldrüsenkrebszellen, ATCC-CRL-1687, Standardmedium: RPMI 1640 (Biochrom; #FG1215, stab. Glutamin) + 10% FCS (Biochrom; #S0415), TWEAKR-positiv.
KPL4: humane Brustkrebszellen, Standardmedium: RPMI 1640 + GlutaMAX I + 10% FBS, Zellbank, Bayer Pharma AG (Identität am 19.7.2012 bei der DSMZ überprüft und bestätigt), Berlin, ERBB2-positiv.

Kultivierung der Zellen erfolgt nach StandardMethode, wie bei der American Tissue Culture Collection (ATCC) für die jeweiligen Zelllinien angegeben.

### MTT-Assay

Zur Durchführung werden die Zellen mit einer Lösung von Accutase in PBS (Biochrom AG #L2143) abgelöst, pelletiert, in Kulturmedium resuspendiert, gezählt und in eine 96-Loch Kulturplatte mit weissem Boden (Costar #3610) ausgesät (1000-2000 Zellen in 100µl/ well abhängig von der verwendeten Zelle) und im Brutschrank bei 37°C und 5% Kohlendioxid inkubiert. Nach 48 h werden die Antikörper-Wirkstoffkonjugate in 10µl Kulturmedium in Konzentrationen von 10⁻⁵M bis 10⁻¹³M auf die Zellen gegeben (Triplikate) und im Brutschrank bei 37°C und 5% Kohlendioxid inkubiert. Nach 72 h erfolgt die Detektion der Proliferation mit Hilfe des MTT-Assays (ATCC, Manassas, Virginia, USA, Katalog-Nr. 30-1010K). Nach Ablauf der gewählten Inkubationszeit wird das MTT Reagens für 4h mit den Zellen inkubiert, bevor durch Zugabe des Detergenzes die Lyse der Zellen über Nacht erfolgt. Die Detektion des gebildeten Farbstoffs erfolgte bei 570nm. Die Proliferation ohne Testsubstanz, aber ansonsten identisch behandelten Zellen, wird als 100% Wert definiert.

### CTG-Assay

Die Kultivierung der Zellen erfolgte nach StandardMethode, mit den unter C-1 angegebenen Wachstumsmedien. Zur Durchführung wurden die Zellen mit einer Lösung von Trypsin (0.05%) und EDTA (0.02%) in PBS (Biochrom AG #L2143) abgelöst, pelletiert, in Kulturmedium resuspendiert, gezählt und in eine 96-Loch Kulturplatte mit weissem Boden (Costar #3610) ausgesät (in 75µl/Loch, folgende Zellzahlen je Loch: NCI-H292: 2500 Zellen/ Loch, BxPC3 2500 Zellen / Loch) und im Brutschrank bei 37°C und 5% Kohlendioxid inkubiert. Nach 24h wurden die Antikörper-Wirkstoffkonjugate in 25µl Kulturmedium (vierfach konzentriert) auf die Zellen gegeben, so dass finale Konzentrationen der Antikörper-Wirkstoffkonjugate von 3 x 10⁻⁷ M bis 3 x 10⁻¹¹ M auf den Zellen erreicht wurden (Triplikate). Anschließend wurden die Zellen im Brutschrank bei 37°C und 5% Kohlendioxid inkubiert. In einer parallelen Platte wurde die Zellvitalität zu Beginn der Wirkstoffbehandlung (Tag 0) mit dem Cell Titer Glow (CTG) Luminescent Cell Viability Assay (Promega #G7573 und #G7571) bestimmt. Dazu wurden pro Zellansatz 100µl des Substrats hinzugefügt, die Platten anschließend mit Alufolie abgedeckt, für 2 Minuten mit dem Plattenschüttler bei 180 rpm geschüttelt, für 8 Minuten auf der Laborbank stehen gelassen und dann mit einem Luminometer (Victor X2, Perkin Elmer) gemessen. Das Substrat detektiert den ATP-Gehalt in den lebenden Zellen, wobei ein Lumineszenz-Signal erzeugt wird, dessen Höhe direkt proportional zur Vitalität der Zellen ist. Nach 72h Inkubation mit den Antikörper-Wirkstoffkonjugaten wurde nun auch in diesen Zellen die Vitalität mit dem Cell Titer Glow Luminescent Cell Viability Assay wie oben beschrieben bestimmt. Aus den gemessenen Daten wurde die IC₅₀ der Wachstumshemmung im Vergleich zum Tag 0 unter Verwendung des DRC (Dose Response Curve) Analysis Spreadsheets anhand einer 4-Parameter Anpassung berechnet. Das DRC Analysis Spreadsheet ist ein von Bayer Pharma AG und Bayer Business Services auf der Plattform IDBS E-WorkBook Suite entwickeltes Biobook Spreadsheet (IDBS: ID Business Solutions Ltd., Guildford, UK).

In den folgenden Tabellen 1a, 1b und 1c sind die IC₅₀-Werte repräsentativer Ausführungsbeispiele mit dem anti-TWEAKR-Antikörper aus diesem Assay aufgeführt:

**Tabelle 1a**

| **Beispiel** | **BxPC3** | **NCI-H292** |
|---|---|---|
| | **IC₅₀ [M]** | **IC₅₀ [M]** |
| | **CTG** | **CTG** |
| 58b | 5.32E-09 | 5.76E-09 |
| 82b | 4.26E-09 | 6.00E-07 |
| 83b | 4.68E-09 | 1.97E-08 |
| 84b | 1.00E-09 | 8.58E-10 |
| 85b | 2.03E-09 | 8.84E-10 |
| 86b | 2.96E-09 | 8.68E-09 |
| 87b | 2.16E-09 | 1.33E-09 |
| 88b | 2.0E-08 | 8.34E-09 |
| 89b | 1.87E-09 | 6.00E-07 |
| 90b | 2.12E-08 | 8.49E-09 |
| 91b | 9.98E-09 | 3.07E-09 |

**Tabelle 1b**

| **Beispiel** | **BxPC3** | **NCI-H292** | **Beispiel** | **BxPC3** | **NCI-H292** |
|---|---|---|---|---|---|
| | **IC₅₀ [M]** | **IC₅₀ [M]** | | **IC₅₀ [M]** | **IC₅₀ [M]** |
| | **CTG** | **CTG** | | **CTG** | **CTG** |
| 104b | 5.88E-10 | 1.44E-10 | 126b | 6.00E-07 | 6.00E-07 |
| 106b | 2.43E-09 | 6.00E-07 | 127b | 3.31E-08 | 1.94E-09 |
| 107b | 4.19E-09 | 4.52E-09 | 129b | 6.00E-07 | 6.00E-07 |
| 109b | 9.26E-08 | 1.61E-07 | 142b | 1.41E-09 | 6.92E-10 |
| 112b | 6.00E-07 | 6.00E-07 | 143b | 1.04E-07 | 4.33E-09 |
| 113b | 2.48E-08 | 7.36E-08 | 144b | 1.17E-08 | 8.29E-08 |
| 115b | 2.43E-07 | 6.00E-07 | 146b | 4.85E-08 | 1.05E-08 |
| 116b | 1.01E-08 | 6.00E-07 | | | |
| 117b | 6.00E-07 | 6.00E-07 | | | |
| 118b | 6.00E-07 | 6.00E-07 | 153b | 1.44E-09 | 1.17E-08 |
| 119b | 8.90E-09 | 9.14E-10 | 155b | 9.44E-10 | 5.09E-10 |
| 121b | 6.00E-07 | 6.00E-07 | 156b | 1.39E-09 | 6.00E-07 |
| 122b | 6.00E-07 | 1.03E-08 | | | |
| 124b | 6.00E-07 | 6.00E-07 | | | |
| 125b | 2.99E-08 | 3.58E-09 | | | |

**Tabelle 1c**

| **Beispiel** | **BxPC3** | **NCI-H292** | **Beispiel** | **BxPC3** | **NCI-H292** |
|---|---|---|---|---|---|
| | **IC₅₀ [M]** | **IC₅₀ [M]** | | **IC₅₀ [M]** | **IC₅₀ [M]** |
| | **CTG** | **CTG** | | **CTG** | **CTG** |
| 163b | 3.80E-09 | 1.21E-08 | 211b | 5.94E-09 | 3.75E-09 |
| 164b | 8.54E-09 | 4.10E-09 | 212b | 3.65E-08 | 3.31E-08 |
| 165b | 9.14E-10 | 8.95E-10 | 213b | 6.02E-09 | 2.16E-09 |
| 166b | 3.72E-09 | 9.27E-08 | 214b | 3.86E-09 | 3.82E-09 |
| 168b | 9.12E-10 | 5.78E-09 | 216b | 7.15E-10 | 5.28E-10 |
| 169b | 2.04E-09 | 3.19E-09 | 217b | 5.60E-09 | 1.33E-08 |
| 171b | 6.00E-07 | 4.25E-09 | 234b | 8.17E-09 | 1.82E-09 |
| 173b | 1.39E-09 | 1.07E-09 | 236b | 1.93E-07 | 2.38E-07 |
| 174b | 5.27E-09 | 6.00E-07 | 237b | 1.05E-09 | 3.74E-10 |
| 175b | 9.58E-09 | 6.00E-07 | 238b | 6.00E-07 | 6.03E-08 |
| 176b | 3.84E-09 | 6.00E-07 | 239b | 2.52E-08 | 7.04E-09 |
| 177b | 2.00E-08 | 5.11E-08 | 240b | 1.13E-08 | 8.69E-09 |
| 178b | 1.78E-08 | 3.78E-08 | 241b | 1.10E-08 | 1.15E-09 |
| 179b | 2.29E-08 | 6.00E-07 | 242b | 8.54E-09 | 1.21E-09 |
| 180b | 4.39E-09 | 2.34E-09 | 243b | 9.99E-09 | 1.18E-09 |
| 192b | 6.29E-09 | 6.00E-07 | 244b | 4.63E-08 | 3.28E-08 |
| 193b | 1.85E-09 | 8.98E-09 | 245b | 1.05E-08 | 1.30E-09 |
| 194b | 3.96E-09 | 2.92E-09 | 247b | 6.19E-10 | 3.63E-10 |
| 195b | 4.07E-09 | 2.95E-09 | 248b | 1.53E-08 | 1.28E-09 |
| 196b | 5.73E-09 | 1.58E-09 | 254b | 3.46E-09 | 3.37E-10 |
| 204b | 6.42E-09 | 1.25E-09 | | | |
| 205b | 1.45E-09 | 5.17E-08 | | | |
| 206b | 3.89E-09 | 3.01E-08 | | | |
| 207b | 1.36E-09 | 3.33E-10 | | | |
| 208b | 2.55E-09 | 6.98E-10 | | | |
| 209b | 7.07E-10 | 1.42E-09 | | | |
| 210b | 8.53E-10 | 1.89E-08 | | | |

Die angegebenen Wirkdaten beziehen sich auf die im vorliegenden experimentellen Teil beschriebenen Ausführungsbeispiele mit den angegebenen Drug/mAB-Ratios. Die Werte können bei anderen Drug/mAB-Ratios gegebenenfalls abweichen. Bei den IC50-Werten handelt es sich um Mittelwerte aus mehreren unabhängigen Exprimenten oder um Einfachwerte. Die Wirkung der TWEAKR- Antikörper-Wirkstoffkonjugate war selektiv versus der jeweiligen Isotypkontrolle, die den jeweils entsprechenden Linker und Toxophor enthielt.

In der folgenden Tabelle 2 sind die IC50-Werte repräsentativer Ausführungsbeispiele mit dem Cetuximab-Antikörper aus dem MTT-Assay aufgeführt:

**Tabelle 2**

| **Beispiel** | **NCI-H292** |
|---|---|
| | **IC₅₀ [M]** |
| | **MTT** |
| 35a | 5.15E-09 |
| 36a | 1.65E-10 |
| 37a | 1.31E-10 |
| 82a | 4.37E-09 |
| 83a | 1.07E-09 |
| 84a | 9.43E-10 |
| 85a | 3.40E-10 |
| 86a | 1.05E-08 |
| 87a | 4.34E-09 |
| 88a | 1.32E-09 |
| 89a | 3.78E-09 |
| 90a | 2.35E-09 |
| 91a | 1.45E-10 |
| 96 | 2.09E-09 |
| 98 | 1.65E-09 |
| 99 | 8.51E-10 |
| 102 | 3.29E-08 |
| 104a | 1.85E-10 |
| 104h | 4.60E-10 |
| 104i | 1.47E-09 |
| 106a | 7.89E-11 |
| 107a | 4.02E-11 |
| 109a | 2.63E-10 |
| 112a | 1.80E-10 |
| 113a | 1,97E-10 |
| 115a | 2.39E-10 |
| 116a | 8.99E-11 |
| 117a | 1.09E-10 |
| 118a | 8.33E-09 |
| 119a | 8.39E-10 |
| 121a | 8.78E-11 |
| 122a | 2.01E-10 |
| 124a | 5.75E-11 |
| 125a | 9.11E-11 |
| 126a | 8.38E-09 |
| 127a | 5.97E-10 |
| 129a | 1.70E-09 |
| 131 | 5.00E-07 |
| 132 | 5.00E-07 |
| 133 | 1.55E-07 |
| 134 | 5.00E-07 |
| 135 | 5.00E-07 |
| 136 | 5.00E-07 |
| 137 | 5.00E-07 |
| 138 | 5.00E-07 |
| 139 | 1.34E-07 |
| 141 | 1.61E-08 |
| 142a | 4.95E-11 |
| 142h | 6.50E-10 |
| 142i | 3.62E-09 |
| 143a | 1.21E-10 |
| 146a | 6.44E-11 |
| 153a | 2.40E-12 |
| 155a | 7.34E-11 |
| 156a | 3.74E-11 |
| 157 | 5.00E-07 |
| 158 | 2.22E-08 |
| 160 | 5.00E-07 |
| 161 | 3.55E-08 |
| 162 | 1.21E-07 |
| 163a | 7.24E-10 |
| 163h | 6.03E-10 |
| 164a | 1.29E-10 |
| 165a | 3.26E-11 |
| 166a | 1.08E-10 |
| 168a | 1.23E-10 |
| 168h | 1.57E-10 |
| 169a | 1.86E-10 |
| 171a | 5.05E-10 |
| 173a | 3.16E-11 |
| 174a | 5.41E-10 |
| 175a | 4.72E-10 |
| 176a | 2.19E-10 |
| 177a | 1.87E-11 |
| 178a | 1.99E-10 |
| 179a | 1.51E-09 |
| 180a | 2.32E-10 |
| 181 | 7.72E-09 |
| 182 | 1.20E-09 |
| 183 | 3.29E-08 |
| 184 | 2.78E-08 |
| 185 | 1.20E-08 |
| 186 | 9.29E-09 |
| 187 | 1.98E-09 |
| 188 | 9.28E-10 |
| 189 | 8.76E-09 |
| 190 | 5.42E-09 |
| 191 | 9.36E-10 |
| 192a | 5,18E-10 |
| 193a | 3.10E-10 |
| 193h | 2.52E-10 |
| 194a | 6.17E-11 |
| 195a | 8.27E-10 |
| 196a | 3.67E-09 |
| 197 | 7.35E-09 |
| 198 | 1.95E-08 |
| 199 | 3.23E-07 |
| 199-2 | |
| 200 | 1,40E-07 |
| 201 | >5.00E-07 |
| 202 | 4.22E-07 |
| 203 | >5.00E-07 |
| 204a | 3.60E-09 |
| 205a | 6.64E-10 |
| 206a | 1.37E-09 |
| 207a | 1.05E-10 |
| 207h | 2.39E-09 |
| 207i | 6.87E-10 |
| 208a | 1.16E-08 |
| 208i | 8.22E-09 |
| 209a | 2.02E-11 |
| 209h | 1.35E-09 |
| 209i | 1.37E-09 |
| 210a | 1.30E-11 |
| 211a | 4.71E-10 |
| 212a | 4.43E-10 |
| 213a | 1.95E-11 |
| 214a | 6.83E-10 |
| 215a | 5.20E-10 |
| 215h | 5.18E-10 |
| 215i | 1.58E-08 |
| 216a | 1.12E-10 |
| 217a | 1.27E-09 |
| 218a | 3.44E-10 |
| 218h | 9.42E-10 |
| 218i | 1.37E-08 |
| 219 | 3.48E-09 |
| 220 | 1.97E-10 |
| 221 | 1.00E-10 |
| 222 | 3.30E-09 |
| 223 | 7.70E-12 |
| 224 | 3.37E-12 |
| 225 | 1.51E-09 |
| 226 | 2.22E-07 |
| 227 | 1.82E-09 |
| 228 | 5.36E-09 |
| 229 | 5.00E-07 |
| 230 | 1.21E-11 |
| 231 | 9.18E-10 |
| 232 | 1.55E-08 |
| 233 | 9.63E-10 |
| 234a | 2.10E-09 |
| 234h | 7.28E-09 |
| 234i | 6.97E-09 |
| 235a | 4.6E-08 |
| 235h | 3.04E-10 |
| 235i | 1.45E-08 |
| 236a | 5.98E-09 |
| 237a | 1.51E-09 |
| 237i | 5.94E-09 |
| 238a | 3.49E-10 |
| 239a | 7.78E-11 |
| 239h | 1.65E-09 |
| 239i | 6.48E-09 |
| 240a | 3.66E-11 |
| 241a | 5.91E-11 |
| 241i | 5.80E-10 |
| 242a | 8.26E-11 |
| 243a | 3.37E-10 |
| 243i | 3.22E-09 |
| 244a | 3.70E-10 |
| 244i | 4.50E-09 |
| 245a | 5.30E-11 |
| 245i | 2.50E-08 |
| 246 | 9.38E-09 |
| 247a | <1.00E-10 |
| 247i | 1.53E-10 |
| 248a | |
| 249 | 2.66E-08 |
| 252 | |
| 254a | |
| 254i | |

Die angegebenen Wirkdaten beziehen sich auf die im vorliegenden experimentellen Teil beschriebenen Ausführungsbeispiele mit den angegebenen Drug/mAB-Ratios. Die Werte können bei anderen Drug/mAB-Ratios gegebenenfalls abweichen. Bei den IC50-Werten handelt es sich um Mittelwerte aus mehreren unabhängigen Exprimenten oder um Einfachwerte. Die Wirkung der Cetuximab-Antikörper-Wirkstoffkonjugate war selektiv versus der jeweiligen Isotypkontrolle, die den jeweils entsprechenden Linker und Toxophor enthielt.

In der nachstehenden Tabelle 3 sind die IC50-Werte repräsentativer Ausführungsbeispiele mit dem Trastuzumab-Antikörper aus dem MTT-Assay aufgeführt:

**Tabelle 3**

| **Beispiel** | **KPL4** |
|---|---|
| | **IC₅₀ [M]** |
| | **MTT** |
| 82e | 9.53E-08 |
| 83e | 5.81E-09 |
| 104e | 2.09E-10 |
| 106e | 5.00E-05 |
| 118e | 5.0E-07 |
| 126e | 5.0E-07 |
| 127e | 1.22E-09 |
| 129e | 5.0E-07 |
| 142e | 1.53E-07 |
| 143e | 5.0E-07 |
| 146e | 5.0E-07 |
| 156e | 5.00E-07 |
| 164e | 5,85E-10 |
| 165e | 7.44E-11 |
| 166e | 5.00E-07 |
| 168e | 3.50E-10 |
| 169e | 6,27E-08 |
| 173e | 8.07E-10 |
| 175e | 5.00E-07 |
| 176e | 2,55E-08 |
| 179e | 1.21E-07 |
| 180e | 4.13E-10 |
| 192e | 5.00E-07 |
| 193e | 1.23E-09 |
| 194e | 1.55E-08 |
| 195e | 7.27E-10 |
| 196e | 5.49E-10 |
| 204e | 1.66E-10 |
| 205e | 3.24E-10 |
| 206e | 1.13E-08 |
| 207e | 1.55E-10 |
| 210e | 5.00E-07 |
| 211e | 1.06E-07 |
| 212e | 5.03E-09 |
| 213e | 5.00E-07 |
| 214e | 8.48E-09 |
| 215e | 1.89E-10 |
| 216e | 9.64E-11 |
| 217e | 1.47E-10 |
| 218e | 1.67E-09 |
| 235e | 8.0E-08 |
| 236e | 8.40E-08 |
| 238e | 2.30E-08 |
| 240e | 1.90E-11 |
| 241e | 1.90E-10 |
| 242e | 6.288E-11 |
| 243e | 2.92E-10 |
| 244e | 8.30E-09 |
| 245e | 4.48E-10 |
| 247e | 1.43E-10 |
| 254e | 1.09E-10 |

Die angegebenen Wirkdaten beziehen sich auf die im vorliegenden experimentellen Teil beschriebenen Ausführungsbeispiele mit den angegebenen Drug/mAB-Ratios. Die Werte können bei anderen Drug/mAB-Ratios gegebenenfalls abweichen. Bei den IC50-Werten handelt es sich um Mittelwerte aus mehreren unabhängigen Exprimenten oder um Einfachwerte. Die Wirkung der Trastuzumab-Antikörper-Wirkstoffkonjugate war selektiv versus der jeweiligen Isotypkontrolle, die den jeweils entsprechenden Linker und Toxophor enthielt.

### C-lb Bestimmung der Inhibition des Kinesinspindelproteins KSP/ Eg5 durch ausgewählte Beispiele

Die Motordomäne des humanen Kinesinspindelproteins KSP /Eg5 (Fa. tebu-bio/ Cytoskeleton Inc, No. 027EG01-XL) wird in einer Konzentration von 10nM mit 50 µg/ml Taxol (Fa. Sigma No. T7191-5MG) stabilisierten Microtubuli (bovine or porcine, Fa. tebu-bio/ Cytoskeleton Inc) für 5 min bei RT in 15mM PIPES, pH 6,8 (5mM MgCl₂ und 10mM DTT, Fa. Sigma) inkubiert. Die frisch hergestellte Mischung wird auf eine 384 MTP aliquotiert. Es folgt die Zugabeder zu untersuchenden Inhibitoren in Konzentration von 1.0 x10-6M bis 1.0x 10-13 M und ATP (finale Konzentration 500µM, Fa. Sigma). Die Inkubation erfolgt über 2h bei RT. Die ATPase-Aktivität wird durch den Nachweis des entstehenden anorganischen Phosphats mit Malachit-Grün detektiert (Fa. Biomol). Nach der Zugabe des Reagenz erfolgt eine 50 minütige Inkubation bei RT, bevor die Detektion der Absorption bei einer Wellenlänge von 620nM erfolgt. Als Positivkontrolle werden Monastrol und Ispinesib (Fa. Adooq A10486) verwendet. Die Einzeldaten der Dosis-Wirkungskurve stellen achtfach Bestimmungen dar. Bei den IC50-Werten handelt es sich um Mittelwerte aus drei unabhängigen Exprimenten. Als 100% Kontrolle dient die nicht mit Inhibitoren behandelte Probe.

In der folgenden Tabelle 4 sind die IC50-Werte repräsentativer Ausführungsbeispiele aus dem beschriebenen Assay zusammengefasst: Sie belegen beispielhaft die hohe Potenz der beschriebenen Toxophore und ADC-Metabolite am Target.

**Tabelle 4**

| **Beispiele** | **KSP-Assay** |
|---|---|
| | **IC₅₀ [M]** |
| 96 | 7.80E-09 |
| 98 | 1.80E-09 |
| 99 | n.d. |
| 102 | 4.70E-09 |
| 131 | 1.24E-09 |
| 132 | 1.65E-09 |
| 133 | 4.13E-09 |
| 134 | 4.35E-09 |
| 135 | 2.04E-09 |
| 136 | 3.28E-10 |
| 137 | 7.78E-10 |
| 138 | 1.01E-9 |
| 139 | 1.82E-09 |
| 141 | 9.02E-10 |
| 157 | 9.61E-09 |
| 158 | 6.20E-10 |
| 160 | 6.81E-10 |
| 161 | 5.17E-09 |
| 162 | 2.58E-09 |
| 181 | 7.90E-09 |
| 182 | 1.04E-08 |
| 183 | 6.21E-09 |
| 184 | 1.43E-08 |
| 185 | 1.80E-08 |
| 186 | 2.53E-08 |
| 187 | 1.41E-08 |
| 188 | 1.01E-08 |
| 189 | 1.12E-08 |
| 190 | 9.61E-09 |
| 191 | 1.26E-08 |
| 197 | 4.17E-09 |
| 198 | 1.07E-08 |
| 199 | 6.00E-09 |
| 199-2 | 9.09E-10 |
| 200 | 1.08E-10 |
| 201 | 1.79E-10 |
| 202 | 2.56E-10 |
| 203 | 2.30E-10 |
| 219 | 1.13E-09 |
| 220 | 1.45E-09 |
| 221 | 1.12E-10 |
| 222 | 1.58E-09 |
| 223 | 2.89E-10 |
| 224 | 1.96E-10 |
| 225 | 4.25E-10 |
| 226 | 1.49E-09 |
| 227 | 1.78E-09 |
| 228 | 3.07E-09 |
| 229 | 4.44E-09 |
| 230 | 3.41E-09 |
| 231 | 1.67E-09 |
| 232 | 2.20E-09 |
| 233 | 3.88E-09 |
| 246 | 2.69E-09 |
| 249 | 9.15E-10 |
| 250 | 3.26E-09 |
| 251 | 2,71E-10 |
| 252 | 4.57E-10 |
| 256 | 1.64E-08 |

Die angegebenen Wirkdaten beziehen sich auf die im vorliegenden experimentellen Teil beschriebenen Ausführungsbeispiele.

### C-2 Internalisierungsassay

Internalisierung ist der Schüsselprozess, um eine spezifische und effiziente Bereitstellung der zytotoxischen Payload in Antigen-exprimierenden Krebszellen durch Antikörper-Drug-Konjugate (ADC) zu ermöglichen. Dieser Prozess wird über Fluoreszenzmarkierung von spezifischen TWEAKR-Antikörpern und einen Isotyp-Kontrollantikörper (M014) verfolgt. Hierzu wird zunächst die Konjugation des Fluoreszenzfarbstoffes an Lysine des Antikörpers durchgeführt. Die Konjugation erfolgt mit zweifach molarem Überschuss von CypHer 5E mono NHS ester (Batch 357392, GE Healthcare) bei pH 8, 3. Nach erfolgter Kupplung wird die Reaktionsmischung über Nacht bei 4°C dialysiert (sSlide-A-Lyser Dialysis Cassettes MWCD 10kD, Fa. Pierce), um überschüssigen Farbstoff zu eliminieren sowie den pH-Wert zu adjustieren, bevor die Ankonzentrierung der Proteinlösung erfolgt (VIVASPIN 500, Fa Sartorius stedim biotec). Die Bestimmung der dye load des Antikörpers erfolgt mittels spektrophotometrischer Analyse (Fa. NanoDrop) und anschließender Berechnung (D: P = A_{dye} ε ₚᵣₒₜₑᵢₙ:(A₂₈₀-0,16A_{dye})ε_{dye}). Die dye load des hier untersuchten TWEAKR-Antikörpers sowie der Isotyp-Kontrolle lagen in vergleichbarer Größenordnung. In Zellbindungs-Assays wird getestet, dass die Konjugation zu keiner Affinitätsänderung des Antikörpers führt.

Die markierten Antikörper werden im Internalisierungs-Assays eingesetzt. Vor dem Behandlungsstart werden Zellen (2x10⁴/well) in 100µl Medium in einer 96-MTP ausgesät (fat, black, clear bottom No 4308776, Fa. Applied Biosystems). Nach 18h Inkubation bei 37°C/5%CO₂ wird das Medium gewechselt und markierte anti-TWEAKR-Antikörper in variierender Konzentration hinzugefügt (10, 5, 2.5, 1, 0.1µg/mL). Das gleiche Behandlungsschema erfolgt mit der markierten Isotyp-Kontrolle (negative control). Die gewählten Inkubationszeiten sind 0, 0,25h, 0,5h, 1h, 1,5h 2h, 3h, 6h and 24h. Die Fluoreszenz- Messung wird mit Hilfe des InCellAnalyzer 1000 (Fa. GE Healthcare) durchgeführt. Es erfolgt eine kinetische Evaluierung über die Messung der Parameter granule counts/cell und totale granule intensity/cell.

TWEAKR-Antikörper wurden nach Bindung an den TWEAKR auf ihre Internalisierungsfähigkeit hin untersucht. Hierzu wurden Zellen mit verschiedenen Expressionsleveln des TWEAKR gewählt. Es konnte Target-vermittelte spezifische Internalisierung mit den TWEAKR-Antikörpern beobachtet werden, wohingegen die Isotyp-Kontrolle keine Internalisierung zeigte.

### C-3 In vitro Tests zur Bestimmung der Zell-Permeabilität

Die Zell-Permeabilität einer Substanz kann mittels *in vitro*-Testung in einem Flux-Assay unter Verwendung von Caco-2-Zellen untersucht werden [M.D. Troutman und D.R. Thakker, Pharm. Res. 20 (8), 1210-1224 (2003)]. Hierzu wurden die Zellen auf 24-Loch-Filterplatten für 15-16 Tage kultiviert. Zur Bestimmung der Permeation wurde die jeweilige Testsubstanz in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC (Agilent 1200, Böblingen, Deutschland) unter Verwendung von reverse phase-Säulen aufgetrennt. Das HPLC-System war über ein Turbo Ion Spray-Interface an ein Triple Quadropol-Massenspektrometer API 4000 (AB SCIEX Deutschland GmbH, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als aktiv transportiert klassifiziert, wenn das Verhältnis von Pₐₚₚ (B-A) zu Pₐₚₚ (A-B) (efflux ratio) >2 oder <0.5 war.

Von entscheidender Bedeutung für Toxophore, die intrazellulär freigesetzt werden, sind die Permeabilität von B nach A [Pₐₚₚ (B-A)] und das Verhältnis von Pₐₚₚ (B-A) zu Pₐₚₚ (A-B) (efflux ratio): Je niedriger diese Permeabilität ist, desto langsamer sind die aktiven und passiven Transportvorgänge der Substanz durch die Monoschicht von Caco-2-Zellen. Gibt das efflux ratio zudem keine Hinweise auf aktiven Transport, kann die Substanz nach intrazellulärer Freisetzung länger in der Zelle verweilen. Damit steigt auch die Zeit, die für eine Interaktion mit dem biochemischen Target (hier: Kinesin-Spindelprotein, KSP / Eg5) zur Verfügung steht.

In der folgenden Tabelle 5 sind Permeabilitätsdaten repräsentativer Ausführungsbeispiele aus diesem Assay aufgeführt:

**Tabelle 5a**

| **Ausführungsbeispiel** | **Pₐₚₚ (B-A) [nm/s]** | **Efflux ratio** |
|---|---|---|
| 92 | 4 | 3 |
| 96 | 285 | 16 |
| 98 | 213 | 16 |
| 99 | 303 | 8.7 |
| 131 | 9 | 10 |
| 132 | 1.6 | 1 |
| 133 | 4.3 | 6 |
| 134 | 2.9 | 0.5 |
| 135 | 7.8 | 4 |
| 136 | 9.1 | 8.5 |
| 137 | 17 | 19 |
| 138 | 1.7 | 1.6 |
| 139 | 21 | 7.7 |
| 141 | 30 | 35 |
| 157 | 7.7 | 7.2 |
| 158 | 5.6 | 2.2 |
| 160 | 1.6 | 1 |
| 161 | 1.6 | 2 |
| 162 | 2 | 2.9 |
| 197 | 93 | 81 |
| 198 | 519 | 34 |
| 199 | 4.8 | 6.4 |
| 226 | 3 | 0.2 |
| 232 | 49 | 14 |
| 246 | 1.4 | 1.3 |
| 251 | 21 | 19 |
| 252 | 20 | 26 |

### C-4 In vitro Tests zur Bestimmung der Substrateigenschaften für P-Glycoprotein (P-gp)

Viele Tumorzellen exprimieren Transporterproteine für Wirkstoffe, was häufig mit einer Resistenzentwicklung gegenüber Cytostatika einhergeht. Substanzen, die keine Substrate von solchen Transporterproteinen wie beispielsweise P-Glycoprotein (P-gp) oder BCRP sind, könnten somit ein verbessertes Wirkprofil aufzeigen.

Die Substrateigenschaften einer Substanz für P-gp (ABCB1) wurden mittels eines Flux-Assays unter Verwendung von LLC-PK1-Zellen, die P-gp überexprimieren (L-MDR1-Zellen), bestimmt [A.H. Schinkel et al., J. Clin. Invest. 96, 1698-1705 (1995)]. Hierzu wurden die LLC-PK1- oder L-MDR1-Zellen auf 96-Loch-Filterplatten für 3-4 Tage kultiviert. Zur Bestimmung der Permeation wurde die jeweilige Testsubstanz allein oder in Gegenwart eines Inhibitors (wie z.B. Ivermectin oder Verapamil) in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC unter Verwendung von reverse phase-Säulen aufgetrennt. Das HPLC-System war über ein Turbo Ion Spray-Interface an ein Triple Quadropol-Massenspektrometer API 3000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als P-gp-Substrat klassifiziert, wenn das Efflux-Verhältnis Pₐₚₚ (B-A) zu Pₐₚₚ (A-B) >2 war.

Als weitere Kriterien zur Bewertung der P-gp-Substrateigenschaften können die Efflux-Verhältnisse in L-MDR1- und LLC-PK1-Zellen oder das Efflux-Verhältnis in An- oder Abwesenheit eines Inhibitors miteinander verglichen werden. Wenn sich diese Werte um mehr als einen Faktor 2 unterscheiden, so handelt es sich bei der betreffenden Substanz um ein P-gp-Substrat.

### C-5 Pharmakokinetik

### C5a: Identifizierung der ADC-Metabolite nach Internalisierung in vitro

### Methodenbeschreibung:

Internalisierungsuntersuchungen mit Immunkonjugaten werden durchgeführt, um intrazellulär entstandene Metaboliten zu analysieren. Hierzu werden humane Lungentumorzellen NCI H292 (3x10⁵/well) in 6-well Platten ausgesät und über Nacht inkubiert (37 °C, 5% CO₂). Es erfolgt eine Behandlung mit 10µg/mL des zu untersuchenden ADCs. Die Internalisierung wird bei 37°C und 5% CO₂ durchgeführt. Zu verschiedenen Zeitpunkten (0, 4, 24, 48, 72 h) werden Zellproben zur weiteren Analyse genommen. Zunächst werden die Überstände (ca. 5 mL) geerntet und nach erfolgter Zentrifugation (2 min, RT, 1000 rpm Heraeus Variofuge 3.0R) bei -80°C gelagert. Die Zellen werden mit PBS gewaschen, mit Accutase abgelöst und die Zellzahl bestimmt. Nach erneutem Waschen wird eine definierte Zellzahl (2x10⁵) mit 100 µL Lysis Puffer (Mammalian Cell Lysis Kit (Sigma MCL1) versetzt und unter ständigem Schütteln (Thermomixer, 15 min, 4 °C, 650 rpm) in Protein LoBind tubes (eppendorf Cat.No. 0030 108.116) inkubiert. Nach der Inkubation wird das Lysat zentrifugiert (10 min, 4 °C, 12000 g, eppendorf 5415R) und der Überstand geerntet. Der gewonnene Überstand wird bei -80 °C gelagert. Alle Proben werden anschließend wie folgt analysiert.

Die Messung der Verbindungen im Kulturüberstand bzw. Zellysat erfolgt nach Fällung der Proteine mit Methanol oder Acetonitril durch eine Hochdruck-Flüssigkeits-Chromatographie (HPLC) gekoppelt an ein Triple-Quadrupol-Massenspektrometer (MS).

Zur Aufarbeitung von 50 µL Kulturüberstand/Zelllysat werden diese mit 150 µL Fällungsreagenz (in der Regel Acetonitril) versetzt und für 10 Sekunden geschüttelt. Das Fällungsreagenz enthält einen internen Standard (ISTD) in geeigneter Konzentration (in der Regel im Bereich 20-100 ng/mL). Nach dem 3minütigen Zentrifugieren bei 16000 g wird der Überstand in ein Autosampler-Vial überführt, mit 500 µL eines auf das Laufmittel abgestimmten Puffers aufgefüllt und erneut geschüttelt.

Die Messung beider Matrixproben erfolgt schließlich an dem mit einer HPLC gekoppelten Triple-Quadrupol-Massenspektrometer API6500 der Firma AB SCIEX Deutschland GmbH.

Zur Kalibrierung werden Plasmaproben mit Konzentrationen von 0.5-2000 µg/L versetzt. Die Nachweisgrenze (LOQ) liegt bei ca. 2 µg/L. Der lineare Bereich erstreckt sich von 2 bis 1000 µg/L.

Zur Kalibrierung der Tumorproben wird der Überstand unbehandelter Tumore mit Konzentrationen von 0.5-200 µg/L versetzt. Die Nachweisgrenze liegt bei 4 µg/L. Der lineare Bereich erstreckt sich von 4 bis 200 µg/L.

Qualitätskontrollen zur Gültigkeitsprüfung enthalten 5 und 50 µg/L.

NCI-H292-Zellen wurden mit je 10 µg/mL der ADCs aus den Beispielen 104b, 119b, 155b, 165b und 173b inkubiert. Nach 72 h wurden die Zellen mit PBS gewaschen, lysiert und tiefgefroren (-80 °C). Nach der oben beschriebenen Methode wurden die Zelllysate und Zellkultur-Überstände aufgearbeitet und nach Extraktion folgende Metabolite identifiziert und quantifiziert:

| Inkubiertes ADC Beispiel | Isolierter Metabolit | Metabolit Konzentration im Zelllysat [µg/L] | Metabolit Konzentration im Überstand [µg/L] |
|---|---|---|---|
| 104b | 199 | 11.6 | < 1 |
| 104b | 198 | <2 | <2 |
| 104b | 135 | < 0.2 | < 0.2 |
| 104b | 197 | < 1 | < 1 |
| 104b | 256 | <0.2 | <0.2 |
| 119b | 158 | 14 | < 0.2 |
| 155b | 162 | 10 | < 0.2 |
| 165b | 162 | 8.8 | < 0.2 |
| 173b | 161 | 18 | < 0.2 |

NCI-H292-Zellen wurden mit je 10 µg/mL der ADCs aus den Beispielen 179a, 226a, 85b und 208b inkubiert. Nach 72 h wurden die Zellen mit PBS gewaschen, lysiert und tiefgefroren (-80 °C). Nach der oben beschriebenen Methode wurden die Zelllysate und Zellkultur-Überstände aufgearbeitet und nach Extraktion folgende Metabolite identifiziert und quantifiziert:

| Inkubiertes ADC Beispiel | Isolierter Metabolit | Metabolit Konzentration im Zelllysat [µg/L] | Metabolit Konzentration im Überstand [µg/L] |
|---|---|---|---|
| 179a | 249 | < 1 | 5.6 |
| 226a | 226 | <1 | 9.1 |
| 226a | 255 | <1 | 2.5 |
| 85b | 139 | 1.5 | 3.2 |
| 85b | 250 | 7.4 | 1.8 |
| 208b | 199 | 13.5 | 2.3 |
| 208b | 198 | <1 | <1 |

### C5b: Identifizierung der ADC-Metabolite in vivo

Nach i.v. Applikation von 3-30 mg/kg verschiedener ADCs können die Plasma- und Tumorkonzentrationen des ADCs sowie potentiell auftretender Metaboliten gemessen und die pharmakokinetischen Parameter wie Clearance (CL), Fläche unter der Kurve (AUC) und Halbwertszeit (t_{1/2}) berechnet werden.

### Analytik zur Quantifizierung der potentiell auftretenden Metabolite

Die Messung der Verbindungen in Plasma und Tumor erfolgt nach Fällung der Proteine mit Methanol oder Acetonitril durch eine Hochdruck-Flüssigkeits-Chromatographie (HPLC) gekoppelt an ein Triple-Quadrupol-Massenspektrometer (MS).

Zur Aufarbeitung von 50 µL Plasma werden diese mit 250 µL Fällungsreagenz (in der Regel Acetonitril) versetzt und für 10 Sekunden geschüttelt. Das Fällungsreagenz enthält einen internen Standard (ISTD) in geeigneter Konzentration (in der Regel im Bereich 20-100 ng/mL). Nach dem 3minütigen Zentrifugieren bei 16000 g wird der Überstand in ein Autosampler-Vial überführt, mit 500 µL eines auf das Laufmittel abgestimmten Puffers aufgefüllt und erneut geschüttelt.

Bei der Aufarbeitung eines Tumors wird dieser mit der 3fachen Menge an Extraktionspuffer versetzt. Der Extraktionspuffer enthält 50 mL Tissue Protein Extraction Reagent (Pierce, Rockford, IL), zwei Pellets Complete-Protease-Inhibitor-Cocktail (Roche Diagnostics GmbH, Mannheim, Deutschland) und Phenylmethylsulfonylfluorid (Sigma, St. Louis, MO) in einer finaler Konzentration von 1 mM. Im Tissuelyser II (Qiagen) wird die Probe zweimal 20 Minuten bei maximaler Schlagzahl homogenisiert. 50 µL des Homogenats wird in ein Autosampler-Vial überführt und mit 150 µL Methanol inklusive ISTD aufgefüllt. Nach 3minütigem Zentrifugieren bei 16000 g werden 10 µL des Überstands mit 180 µl eines auf das Laufmittel abgestimmten Puffers aufgefüllt und erneut geschüttelt. Danach ist die Tumorprobe messfertig.

Die Messung beider Matrixproben erfolgt schließlich an dem mit einer HPLC gekoppelten Triple-Quadrupol-Massenspektrometer API6500 der Firma AB SCIEX Deutschland GmbH.

Zur Kalibrierung werden Plasmaproben mit Konzentrationen von 0.5 - 2000 µg/L versetzt. Die Nachweisgrenze (LOQ) liegt bei ca. 2 µg/L. Der lineare Bereich erstreckt sich von 2 bis 1000 µg/L.

Zur Kalibrierung der Tumorproben wird der Überstand unbehandelter Tumore mit Konzentrationen von 0.5 - 200 µg/L versetzt. Die Nachweisgrenze liegt bei 5 µg/L. Der lineare Bereich erstreckt sich von 5 bis 200 µg/L.

Qualitätskontrollen zur Gültigkeitsprüfung enthalten 5 und 50 µg/L, in Plasma zusätzlich 500 µg/L.

Nach Applikation von 10mg/kg von den ADCs aus den Beispielen 119b und 104b in die Kontrollgruppen aus den Xenograftmodellen mit NCI-H292 wurden nach 24h die Mäuse abgetötet, Blut entnommen und die Tumore isoliert. Nach der oben beschriebenen Methode wurden die Plasma- und Tumorproben aufgearbeitet und nach Extraktion folgende Metabolite identifiziert und quantifiziert:

| Appliziertes ADC (Beispiel-Nummer) | Isolierter Metabolit (Beispiel-Nummer) | Metabolit Konzentration im Tumor [µg/L] | Metabolit Konzentration im Plasma [µg/L] |
|---|---|---|---|
| 119b | 158 | 155 | <5 |
| 104b | 199 | 147 | <5 |
| 104b | 198 | 27.4 | <5 |
| 104b | 135 | 5.23 | <5 |
| 104b | 197 | 7.75 | <5 |

### Analytik zur Quantifizierung der verwendeten Antikörper

Der Antikörperteil der ADCs wurde mittels Liganden-Bindungs-Assay (ELISA) als Gesamt-IgG-Konzentration in Plasmaproben und Tumorlysaten bestimmt. Dabei wurde das Sandwich-ELISA-Format verwendet. Dieser ELISA war qualifiziert und validiert für die Bestimmung in Plasma- und Tumorproben. Die ELISA-Platten wurden mit anti-human-IgG-Fc-Antikörpern der Ziege beschichtet. Nach Inkubation mit der Probe wurden die Platten gewaschen und mit einem Detektor-Konjugat aus anti-human-IgG(H+L)-Antikörper des Affen und Meerrettichperoxidase (HRP) inkubiert. Nach einem weiteren Waschschritt wurde das HRP-Substrat OPD hinzugegeben und die Farbentwicklung über die Absorption bei 490 nm verfolgt. Standardproben mit bekannter IgG-Konzentration wurden mittels 4-Parameter-Gleichung gefittet. Innerhalb der unteren (LLOQ) und oberen (ULOQ) Quantifizierungsgrenzen wurden die unbekannten Konzentrationen über Interpolation ermittelt.

### C-6 Wirksamkeitstest in vivo

Die Wirksamkeit der erfindungsgemäßen Konjugate wurde in vivo beispielsweise mittels XenograftModellen getestet. Der Fachmann kennt Methoden im Stand der Technik, anhand derer die Wirksamkeit der erfindungsgemäßen Verbindungen getestet werden kann (siehe z.B. WO 2005/081711; Polson et al., Cancer Res. 2009 Mar 15;69(6):2358-64). Beispielsweise wurde hierzu Nagern (z.B. Mäusen) eine Tumorzelllinie, welche das Zielmolekül des Binders exprimiert, implantiert. Anschließend wurde den Implantat-Tieren entweder ein erfindungsgemäßes Konjugat, ein Isotyp-Antikörper-Kontrollkonjugate oder ein Kontrollantikörper oder isotonische Salzlösung appliziert. Die Applikation erfolgte einmalig oder öfter. Nach einer Inkubationszeit von mehreren Tagen wurde die Tumorgröße im Vergleich von Konjugat-behandelten Tieren und der Kontrollgruppe bestimmt. Die Konjugat-behandelten Tiere zeigten eine geringere Tumorgröße.

### C-6a. Wachstumshemmung / Regression von experimentellen Tumoren in der Maus

Humane Tumorzellen, die das Antigen für das Antikörper-Wirkstoffkonjugat exprimieren, werden subkutan in die Flanke von immunsupprimierten Mäusen inokuliert, beispielsweise NMRi Nude- oder SCID-Mäuse. 1-10 Millionen Zellen werden aus der Zellkultur abgelöst, zentrifugiert und mit Medium oder Medium / Matrigel resuspendiert. Die Zellsuspension wird unter die Haut der Maus gespritzt.

Innerhalb von einigen Tagen wächst ein Tumor heran. Die Behandlung beginnt nach Etablierung des Tumors, ungefähr bei einer Tumorgröße von 40 mm². Um die Wirkung auf größere Tumoren zu untersuchen, kann die Behandlung auch erst bei einer Tumorgröße von 50-100 mm² begonnen werden.

Die Behandlung mit ADCs erfolgt über die intravenöse Route in die Schwanzvene der Maus. Das ADC wird mit einem Volumen von 5 mL / kg appliziert.

Das Behandlungsschema richtet sich nach der Pharmakokinetik des Antikörpers. Als Standard wird drei Mal in Folge jeden vierten Tag behandelt. Für eine zeitnahe Beurteilung kann sich auch ein Schema mit einer Einmalbehandlung eignen. Die Behandlung kann aber auch weiter fortgesetzt werden oder es kann sich zu einem späteren Zeitpunkt ein zweiter Zyklus mit drei Behandlungstagen anschließen. Standardmäßig werden 8 Tiere pro Behandlungsgruppe eingesetzt. Neben den Gruppen, die die Wirksubstanzen bekommen, wird eine Gruppe als Kontrollgruppe nur mit dem Puffer nach dem gleichen Schema behandelt.

Im Verlauf des Experiments wird die Tumorfläche regelmäßig mit einer Schieblehre in zwei Dimensionen (Länge / Breite) gemessen. Die Tumorfläche wird mittels Länge x Breite bestimmt. Der Vergleich der mittleren Tumorfläche der Behandlungsgruppe mit der Kontrollgruppe wird als T/C area angegeben.

Werden alle Gruppen des Experimentes nach Behandlungsende gleichzeitig beendet, können die Tumore entnommen und gewogen werden. Der Vergleich der mittleren Tumorgewichte der Behandlungsgruppe mit der Kontrollgruppe wird als T/C weight angegeben.

### C-6c. Wirksamkeit der anti-EGFR-Antikörper-Wirkstoffkonjugate im NCI-H292-Tumormodell bei Einmalbehandlung

1 Mio NCI-H292 Zellen werden subkutan in die Flanke von weiblichen NMRI-nude Mäusen (Janvier) inokuliert. Bei einer Tumorgröße von ∼37 mm² an Tag 9 wird einmalig intravenös mit einer Dosis von 3 mg/kg ADC bzw. 2.5 mg/kg Toxophor A (N-(3-Aminopropyl)-N-{(1R)-1-[1-benzyl-4-(2,5-difluorphenyl)-1H-imidazol-2-yl]-2,2-dimethylpropyl}-2-hydroxyacetamid) bzw. Toxophor B (N-[(3R)-3-Amino-4-fluorbutyl]-N-{(1R)-1-[1-benzyl-4-(2,5-difluorphenyl)-1H-imidazol-2-yl]-2,2-dimethylpropyl}-2-hydroxyacetamid) behandelt. Im Anschluss an die Behandlung wird das Tumorwachstum bis Tag 25 verfolgt.

Die Behandlung mit den Antikörper-Wirkstoffkonjugaten gemäß den Beispielen 35A und 02A führt zu einer deutlichen Wachstumshemmung der Tumore im Vergleich zur Kontrollgruppe. Bei der Behandlung mit 3 mg/kg kommt es zur Regression der meisten Tumore. Die Tabelle 7 gibt die T/C Werte ermittelt über die Tumorgewichte und Tumorfläche am Tag 25 an. Das Antikörper-Wirkstoffkonjugat 35A zeigt hier eine bessere Wirksamkeit als Cetuximab und als das Toxophor A oder das Toxophor B.

**Tabelle 7:**

| **Beispiel** | **Dosis** | **T/C weight** | **T/C area** |
|---|---|---|---|
| Toxophor A | 2,5 mg/kg | 0,82 | 0,87 |
| Toxophor B | 2,5 mg/kg | 0,40 | 0,57 |
| Cetuximab | 3 mg/kg | 0,47 | 0,54 |

### C-6d. Wirksamkeit der anti-EGFR- und anti-TWEAKR Antikörper-Wirkstoffkonjugate im NCI-H292-Tumormodell bei Einmalbehandlung

1 Mio NCI-H292 Zellen werden subkutan in die Flanke von weiblichen NMRI-nude Mäusen (Janvier) inokuliert. Bei einer Tumorgröße von ∼37 mm² an Tag 7 wird einmalig intravenös mit einer Dosis von 3 oder 10mg/kg Antikörper-Wirkstoffkonjugat behandelt. Im Anschluss an die Behandlung wird das Tumorwachstum bis Tag 24 verfolgt.

Die einmalige Behandlung mit dem anti-TWEAKR Antikörper-Wirkstoffkonjugat 02B führt zu einer deutlichen und lang anhaltenden Wachstumshemmung der Tumore im Vergleich zur Kontrollgruppe und dem unkonjugierten anti-TWEAKR Antikörper. Die Tabelle 8 gibt die T/C Werte an, ermittelt über die Tumorgewichte und Tumorfläche am Tag 24.

**Tabelle 8:**

| **Beispiel** | **Dosis** | **T/C weight** | **T/C area** |
|---|---|---|---|
| 91A | 3mg/kg | n.d. | 0,78 |
| Anti-TWEAKR Antikörper | 10mg/kg | 0,99 | 1,01 |
| Cetuximab | 3mg/kg | n.d. | 0,67 |
| Nimotuzumab | 3mg/kg | n.d. | 0.95 |
| 237i | 3mg/kg | n.d. | 0.47 |
| 208i | 3mg/kg | n.d. | 0.69 |

### C-6f. Wirksamkeit der anti-TWEAKR-Antikörper-Wirkstoffkonjugate im NCI-H292-Tumormodell bei Einmalbehandlung (2 unabhängige Versuche)

1 Mio NCI-H292 Zellen werden in beiden Versuchen subkutan in die Flanke von weiblichen NMRI-nude Mäusen (Janvier) inokuliert. Bei einer Tumorgröße von ∼45 mm² an Tag 10 (Versuch 1) bzw. Tag 8 (Versuch 2) wird einmalig intravenös mit einer Dosis von 10mg/kg Antikörper-Wirkstoffkojungat behandelt. Im Anschluss an die Behandlung wird das Tumorwachstum bis Tag 18 (Versuch 1) bzw. Tag 24 (Versuch 2) verfolgt.

Die einmalige Behandlung mit den anti-TWEAKR-Antikörper-Wirkstoffkonjugaten 02B, 07B und 08B führt im Versuch 1 zu einer deutlichen Wachstumshemmung der Tumore im Vergleich zur Kontrollgruppe und dem unkonjugierten anti-TWEAKR Antikörper. Die Tabelle 10 (Versuch 1) gibt die T/C Werte ermittelt über die Tumorgewichte und Tumorfläche am Tag 18 an. Die einmalige Behandlung mit den anti-TWEAKR-Antikörper-Wirkstoffkonjugaten 155B, 173B, 165B und 085B führt im Versuch 2 ebenfalls zu einer deutlichen Wachstumshemmung der Tumore im Vergleich zur Kontrollgruppe und den jeweiligen Isotypkontrollen (nicht aufgeführt). Die Tabelle 11 (Versuch 2) gibt die T/C Werte ermittelt über die Tumorfläche am Tag 24 an.

**Tabelle 10:**

| **Beispiel** | **Dosis** | **T/C weight** | **T/C area** |
|---|---|---|---|
| 91B | 10 mg/kg | 0,52 | 0,58 |
| Anti- TWEAKR Antikörper | 10 mg/kg | 0,68 | 0,84 |

**Tabelle 11:**

| **Beispiel** | **Dosis** | **T/C weight** | **T/C area** |
|---|---|---|---|
| 155B | 10 mg/kg | / | 0,32 |
| 173B | 10 mg/kg | / | 0,32 |
| 165B | 10 mg/kg | / | 0,30 |
| 085B | 10 mg/kg | / | 0,24 |

### C-6g. Wirksamkeit der anti-TWEAKR-Antikörper-Wirkstoffkonjugate im A375-Tumormodell bei Mehrfachbehandlung

5 Mio A375-(humane Melanom) Zellen werden subkutan in die Flanke von weiblichen NMRI-nude Mäusen (Janvier) inokuliert. Bei einer Tumorgröße von 41 mm² an Tag 10 beginnt die Behandlung intravenös mit einer Dosis von 10 mg/kg (Tag 10, 14, 18). Im Anschluss an die Behandlung wird das Tumorwachstum bis Tag 22 verfolgt.

Die Behandlung mit den Antikörper-Wirkstoffkonjugaten gemäß den Beispielen 38B und 104B führt zu einer deutlichen Wachstumshemmung der Tumore im Vergleich zur Kontrollgruppe. Die Tabelle 10 gibt die T/C Werte ermittelt über die Tumorgewichte und Tumorfläche am Tag 22 an. Eine Behandlung mit dem jeweiligen Kontrollkonjugat (Isotyp-Antikörper gegen irrelevantes Antigen) hat eine deutlich schwächere Tumor-wachstumshemmende-Wirkung. Die Behandlung mit den unkonjugierten Antikörpern führt ebenfalls zu einer teilweise schwächeren Wachstumshemmung der Tumore.

**Tabelle 10:**

| **Beispiel** | **Dosis** | **T/C weight** | **T/C area** |
|---|---|---|---|
| Anti-TWEAKR Antikörper | 10 mg/kg | 0,32 | 0,52 |
| Isotypkontrolle zu 104B | 10 mg/kg | 0,94 | 0,96 |
| Anti-TWEAKR Antikörper | 10 mg/kg | 0,32 | 0,52 |
| 104B | 10 mg/kg | 0,14 | 0,29 |

### C-6h. Wirksamkeit der anti-TWEAKR-Antikörper-Wirkstoffkonjugate im LoVo-Tumormodell bei Mehrfachbehandlung

5 Mio LoVo-(humane Kolonkarzinom) Zellen werden subkutan in die Flanke von weiblichen NMRI-nude Mäusen (Janvier) inokuliert. Bei einer Tumorgröße von 43 mm² an Tag 7 beginnt die Behandlung intravenös mit einer Dosis von 10 mg/kg (Tag 7, 11, 15). Im Anschluss an die Behandlung wird das Tumorwachstum bis Tag 45 verfolgt.

Die Behandlung mit den Antikörper-Wirkstoffkonjugaten gemäß der Beispiele 07B, 87B und 104B führt zu einer deutlichen Wachstumshemmung der Tumore im Vergleich zur Kontrollgruppe. Die Tabelle 11 gibt die T/C Werte ermittelt über die Tumorfläche am Tag 45 an. Eine Behandlung mit dem Kontrollkonjugat zur 07B (Isotyp-Antikörper gegen irrelevantes Antigen) hat eine deutlich schwächere Tumor-wachstumshemmende Wirkung.

**Tabelle 11:**

| **Beispiel** | **Dosis** | **T/C area** |
|---|---|---|
| 104B | 10 mg/kg | 0,43 |
| 87B | 10 mg/kg | 0,52 |

### D. Ausführungsbeispiele für pharmazeutische Zusammensetzungen

Die erfindungsgemäßen Verbindungen können folgendermaßen in pharmazeutische Zubereitungen überführt werden:

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, D-PBS, oder einer Formulierung mit Glycin und Natriumchlorid in Citratpuffer unter Zusatz von Polysorbat 80) gelöst. Die Lösung wird steril filtriert und in sterile und pyrogenfreie Injektionsbehältnisse abgefüllt.

### i.v.-Lösung:

Die erfindungsgemäßen Verbindungen können in die angeführten Applikationsformen überführt werden. Dies kann in an sich bekannter Weise durch "Mischen mit" bzw. "Lösen in" inerten, nichttoxischen, pharmazeutisch geeigneten Hilfsstoffen (z.B. Puffersubstanzen, Stabilisatoren, Lösungsvermittler, Konservierungsmittel) geschehen. Z.B können enthalten sein: Aminosäuren (Glycin, Histidin, Methionin, Arginin, Lysin, Leucin, Isoleucin, Threonin, Glutaminsäure, Phenylalanin und weitere), Zucker und verwandte Stoffe (Glucose, Saccharose, Mannitol, Trehalose, Sucrose, Mannose, Lactose, Sorbitol), Glycerin, Natrium-, Kalium, Ammonium-, und Calciumsalze (z.B. Natriumchlorid, Kaliumchlorid oder Dinatriumhydrogenphosphat und v.a. mehr), Acetat/Essigsäure - Puffersysteme, Phosphatpuffersysteme, Zitronensäure u. Citratpuffersysteme, Trometamol (TRIS und TRIS-Salze), Polysorbate (z.B. Polysorbat 80 und Polysorbat 20), Poloxamere (z.B. Poloxamer 188 und Poloxamer 171), Macrogole (PEG-Derivate, z.B. 3350), Triton X-100, EDTA-Salze, Glutathion, Albumine (z.B. human), Harnstoff, Benzylalkohol, Phenol, Chlorocresol, Metacresol, Benzalkoniumchlorid und viele andere mehr.

### Lyophilisat zur späteren Überführung in eine i.v., s.c. oder i.m.-Lösung:

Alternativ können die erfindungsgemäßen Verbindungen in ein stabiles Lyophilisat (e.v. durch Mithilfe von oben genannten Hilfstoffen) überführt werden und vor Applikation mit einem geeigneten Lösungsmittel (z.B. Wasser für Injektionszwecke, isoton. Kochsalzlösung) rekonstituiert und appliziert werden.

### Ausführungsbeispiele für anti-TWEAKR-Antikörper

Alle Beispiele wurden unter Verwendung von Standardverfahren ausgeführt, die dem Fachmann bekannt sind, sofern sie nicht hier im Detail beschrieben werden. Routeine-Molekularbiologie-Verfahren der folgenden Beispiele können wie in Standardlaborbüchern beschrieben ausgeführt werden, wie Sambrook et al., Molecular Cloning: ein Laboratory Manual, 2 Auflage; Cold Spring Harbor Laboratory Press, Cold Spring Harbor, N.Y., 1989.

### AK-BEISPIEL 1: Antikörper-Herstellung mittels einer Antikörperbibliothek

Eine vollständig humane Phagendisplaybibliothek (Hoet RM et al, Nat Biotechnol 2005;23(3):344-8) wurde verwendet, um TWEAKR-spezifische, humane monoklonale Antikörper der vorliegenden Erfindung durch Protein Panning zu isolieren (Hoogenboom H.R., Nat Biotechnol 2005;23(3):1105-16), wobei dimere Fc-fusionierte extrazelluläre Domänen von humanem und murinem TWEAKR als Target immobilisiert wurden.

**Tabelle AK-1: Liste rekombinanter Antigene, die für die Antikörper-Selektion verwendet wurden**

| **Nomenklatur** | **Beschreibung** | **SEQ ID NO** |
|---|---|---|
| TPP-599 | HUMAN-TNFRSF12Aaa28-80-hIgG1-Fc | 138 |
| TPP-601 | MURIN-TNFRSF12Aaa28-80-hIgG1-Fc | 137 |

Die Antigene wurden unter Verwendung eines ca. 2-fachen molaren Überschusses von Biotin-LC-NHS (Pierce; Kat.-Nr. 21347) gemäß den Angaben des Herstellers biotinyliert und unter Verwendung von Zeba Entsalzungssäulen (Pierce; Kat.-Nr. 89889) entsalzt. Gewaschene Magnet-Beads (DynaBeads) wurden über Nacht mit 200 nM biotinyliertem humanen Antigen bei 4°C inkubiert und für 1h bei 4°C mit Blockierungspuffer (PBS mit 3% BSA, 0.05% Tween-20) blockiert. Die blockierte Fab-Phagenbibliothek wurde zu den blockierten TWEAKR-Beads (DynaBeads Streptavidin-M280 - Invitrogen 112-06D) hinzugegeben und für 30 min bei Raumtemperatur inkubiert. Nach stringentem Waschen (3 x in Blockierungspuffer und 9 x in PBS (150 mM NaCl; 8 mM Na₂HPO₄; 1.5 mM KH₂PO₄; eingestellt auf pH = 7.4-7.6) mit 0.05% Tween-20) wurden Fab-Phagen, die spezifisch an biotinylierte TWEAKR-Beads binden (DynaBeads Streptavidin- M280 - Invitrogen 112-06D) in PBS resuspendiert und zur Amplifikation direkt für die Infektion von *Escherichia* coli-Stamm TG1 verwendet. In der zweiten Selektionsrunde wurden zwei murine TWEAKR (200nM) verwendet, um für kreuzreaktive Binder zu selektieren, und in der dritten Selektionsrunde wurde die Konzentration von humanem TWEAKR vermindert (100nM), um den Selektionsdruck für hochaffine Binder zu erhöhen.

11 verschiedene Fab-Phagen wurden identifiziert und die entsprechenden Antikörper wurden in einen Säuger-IgG Expressionsvektor umkloniert, der die fehlenden CH2-CH3 Domänen bereit stellt, die im löslichen Fab nicht vorhanden sind. Die daraus hervorgehenden IgGs wurden transient in Säugerzellen exprimiert, wie beschrieben in Tom et al., Kapitel 12 in Methods Express: Expression Systems herausgegeben von Micheal R. Dyson und Yves Durocher, Scion Publishing Ltd, 2007. Kurz gesagt wurde ein CMV-Promoter-basiertes Expressionsplasmid in HEK293-6E Zellen transfiziert und in Fernbach-Flaschen oder Wave-Bags inkubiert. Die Expression fand statt bei 37°C für 5 bis 6 Tage in F17-Medium (Invitrogen). 1% Ultra-Low IgG FCS (Invitrogen) und 0.5 mM Valproinsäure (Sigma) wurden 24 h nach der Transfektion ergänzend hinzugegeben. Die Antikörper wurden mittels Protein-A-Chromatographie gereinigt und mittels ihrer Bindungsaffinität für löslichen monomeren TWEAKR per ELISA- und BIAcore-Analyse weiter charakterisiert, wie in AK-Beispiel 2 beschrieben.

**Tabelle AK-2: Liste von rekombinantem Antigen, das für die Affinitätsmessung verwendet wurde**

| **Nomenklatur** | **Beschreibung** | **Origin** | **Kat.-Nr. (Fitzgerald Inc)** | **SEQ ID NO** |
|---|---|---|---|---|
| TPP-2305 | hTNFRSF12Aminosäurena28-80 | Human | 30R-AT080 | 168 |

Um die Zellbindungscharakteristika von anti-TWEAKR Antikörper zu bestimmen, wurde die Bindung an eine Reihe von Zelllinien (HT29, HS68, HS578) mittels Durchflusszytometrie untersucht. Die Zellen wurden in Verdünnungen der Antikörper (5µg/ml) in FACS-Puffer suspendiert, und für 1h auf Eis inkubiert. Anschließend wurde ein Zweit-Antikörper (PE Ziegen-anti-humanes IgG, Dianova #109-115-098) hinzugegeben. Nach der Inkubation für 1h auf Eis wurden die Zellen mittels Durchflusszytometrie unter Verwendung eines FACS-Array (BD Biosciences) analysiert.

NF-kappaB -Reportergenassays wurden durchgeführt, um die agonistische Aktivität aller 11 identifizierter Antikörper (humanes IgG1) zu beurteilen. HEK293 Zellen wurden transient mit einem NF-kappaB-Reporterkonstrukt (BioCat, Kat.-Nr. LR-0051-PA) unter Verwendung von 293fectin gemäß Herstellerangabe transfiziert. Weiße Poly-Lysin-beschichtete 384-Loch-Platten (BD) wurden mit transfizierten Zellen in F17-Medien (serum-free; Invitrogen) bei 37C, 5% CO₂ angesät. Am nächsten Tag wurden die Zellen mit gereinigten Antikörpern bei verschiedenen Konzentrationen für 6h stimuliert, und anschließend wurde ein Luciferase-Assay nach Standardverfahren durchgeführt.

Die Internalisierung wurde mittels Fluoreszenzmarkierung von anti-TWEAKR Antikörpern (CypHer 5E mono NHS ester; GE Healthcare) verfolgt. Vor der Behandlung wurden HT29-Zellen (2 x 10⁴/Loch) in 100 µl Medium in 96-MTP-Platten (dick, schwarz, durchsichtiger Boden, Nr. 4308776, Angewandten Biosystems) angesät. Nach 18h Inkubation bei 37°C / 5% CO₂ wurde das Medium gewechselt, und markierte anti-TWEAKR Antikörper wurden in verschiedenen Konzentrationen (10, 5, 2.5, 1, 0.1 µg/ml) hinzugegeben. Die gewählte Inkubationszeit betrug 0, 0,25, 0,5, 1, 1,5, 2, 3, 6 und 24h. Die Fluoreszenzmessung wurde mit einem InCell-Analyzer 1000 (GE Healthcare) durchgeführt.

Der Antikörper mit der stärksten in vitro-Wirksamkeit (TPP-883) wurde für weitere Wirksamkeits- und Affinitätsreifung ausgewählt.
**TPP-883** Aminosäurenequenzen der leichten (SEQ ID NO.71) und schweren (SEQ ID NO.72) Ketten von TPP-883; CDRs sowohl der schweren als auch der leichten Kette sind unterstrichen.

Die Reifung wurde in einer ersten Mutationssammelrunde vorgenommen, gefolgt von der Rekombination derjenigen Aminosäure-Veränderungen, die Affinität und Wirksamkeit am meisten erhöhten. Für das Sammeln von Mutationen NNK (N = AGCT, K = G oder T) wurde an den nachfolgenden einzelnen Aminosäurepositionen mittels ortsgerichteter Mutagenese unter Verwendung synthetischer Oligonucleotide einschließlich NNK-Codon-Diversifizierung (kontinuierliche Aminosäure Nomenklatur, vergleiche Figur 25) randomisiert: S35, S36, Y37 und N39 in CDR-L1; A51, S53, S54, Q56 und S57 in CDR-L2; S92, Y93, S94, S95, G97 und 198 in CDR-L3; P31, Y32, P33, M34 und M35 in CDR-H1; Y50, S52, P53, S54, G56, K57 und H59 in CDR-H2; G99, G100, D101, G102, Y103, F104, D105 und Y106 in CDR-H3. Die DNA aller einzelner NNK Sättigungs-Mutagenesebibliotheken wurde in einen Säuger-IgG Expressionsvektor für Wirksamkeitsreifung, bzw. in einen Phagemid-Vektor für die Affinitätsreifung umkloniert. Die Affinitätsreifung wurde mittels Phage Panning durchgeführt. Gewaschene Magnet-Beads (DynaBeads) wurden über Nacht mit 10 nM, 1 nM, 100 pM und 10 pM biotinyliertem humanen Antigen bei 4°C inkubiert und für 1h bei 4°C mit Blockierungspuffer (PBS mit 3% BSA, 0.05% Tween-20) blockiert. Die blockierte Fab-Phagenbibliothek wurde mit 10000-fachem, 1000-fachem und 100-fachem Überschuss, verglichen mit der theoretischen Bibliothekskomplexität zu den blockierten TWEAKR-DynaBeads hinzugegeben und für 30min bei Raumtemperatur inkubiert. Also wurden insgesamt 12 Strategien verfolgt (4 Antigen-Konzentrationen x 3 Fab-Phage-Titer). Nach stringentem Waschen (3 x in Blockierungspuffer und 9 x in PBS mit 0.05% Tween-20) wurden Fab-Phagen, die spezifisch an biotinylierte TWEAKR-DynaBeads (DynaBeads Streptavidin- M280 - Invitrogen 112-06D) binden, in PBS resuspendiert und direkt zum Zwecke der Amplifikation für die Infektion von *Escherichia coli*-Stamm TG1 verwendet. In Selektionsrunde zwei wurde die Konzentration von humanem TWEAKR-Fc vermindert (1 nM, 100 pM, 10 pM und 1 pM), und der gleiche Fab-Phagen-Titer wurde für alle 12 Strategien verwendet (4.4 x 10¹¹). Für die löslichen Fab-Expression wurde der Phagemid-Vektor mit MluI verdaut, um die Gen-III-Membrananker-Sequenz zu entfernen, die für die für den Fab-Display auf dem Phagen erforderlich ist, und wieder ligiert. 96 Varianten von jeder der 12 Selektions-Pools wurden als lösliche Fabs exprimiert und in einem ELISA-Format untersucht. Dafür wurden 2,5 nM biotinyliertes TWEAKR-Fc Antigenbeschichtet, und die Bindung von löslichen Fabs wurde mittels Anti-c-Myc Antikörpern (Abcam ab62928) nachgewiesen. 7 Einzelsubstitutions-Varianten (fortlaufende Aminosäure Nomenklatur, vergleiche Figur 25) mit verbesserter Bindung an TWEAKR-Fc (Seq ID No 138) wurden nachgewiesen: S36G von CDR-L1, A51Q und S57K von CDR-L2, S94T und G97F von CDR-L3, M35I von CDR-H1 und G102T von CDR-H3. Für die Wirksamkeitsreifung wurden HEK293 Zellen mit einem NF-kappaB-Reporter (BioCat, Kat.-Nr. LR-0051-PA) transfiziert. Weiße Poly-Lysin-beschichtete 384-Loch-Platten (BD) wurden mit transfiziert Zellen in F17-Medien (serum-free; Invitrogen) angesät, und einzelne Varianten der NNK-diversifizierten Positions-Antikörper (humanes IgG1)bibliotheken wurden transient in Säugerzellen exprimiert. Am nächsten Tag wurden NF-kappaB-Reporterzellen mit den exprimierten Einzel-NNK-AntikörperVarianten für 6h stimuliert, und anschließend wurde ein Luciferase-Assay nach Standardverfahren durchgeführt. Es wurde 1 Einzelsubstitutionsvariante mit verbesserter agonistischer Aktivität nachgewiesen: G102T von CDR-H3. Diese Variante wurde ebenfalls durch Affinitätsreifung erhalten und zeigte auch dort die größte Affinitätsverstärkung. Nach Mutationssammlung mittels Affinitäts- und Wirksamkeits-Screening wurden alle 7 günstigen Einzelsubstitutionen (BibliotheksKomplexität: 128Varianten) in eine Rekombinationsbibliothek rekombiniert. Dafür wurden Oligonukleotide synthetisiert, um ausgewählte Mutationen oder die entsprechenden Wildtyp-Aminosäure bei jeder ausgewählten Position einzuführen. Die Bibliothek wurde unter Verwendung von aufeinanderfolgenden Runden von "overlap extension PCR" hergestellt. Das finale PCR Product wurde in einen bakteriellen löslichen Fab-Expressionsvektor ligiert, und 528 Varianten wurden zufällig ausgewählt (∼ 4-facher Überschuss der Probennahme) für eine Gleichgewichts-ELISA-Screen mit löslichen Fabs, wie vorher beschrieben. Letztlich wurden 7 Varianten ausgewählt, basierend auf vergrößerter Affinität verglichen mit der besten Einzelsubstitutionsvariante, G102T. Die entsprechende DNA derselben wurde in einen Säuger-IgG Expressionsvektor umkloniert und im vorhergenannten NF-kappaB-Reporter-Zellassay auf funktionelle Aktivität untersucht. Letzlich wurden die erhaltenen Sequenzen verglichen mit humanen Keimbahn-Sequenzen, und Abweichungen ohne bedeutsamen Einfluss auf die Affinität und Wirksamkeit wurden angepasst. Antikörper mit den nachfolgenden Sequenzen wurden mittels Antikörper Bibliothek-Screening und mittels Affinitäts- und/oder Wirksamkeitsreifung erhalten:
**TPP-2090** Aminosäurenequenzen der leichten (SEQ ID NO.1) und schweren (SEQ ID NO.2) Ketten von TPP-2090; CDRs sowohl der schweren als auch der leichten Kette sind unterstrichen.
**TPP-2149** Aminosäurenequenzen der leichten (SEQ ID NO.11) und schweren (SEQ ID NO.12) Ketten von TPP-2149; CDRs sowohl der schweren als auch der leichten Kette sind unterstrichen.
**TPP-2093** Aminosäurenequenzen der leichten (SEQ ID NO.21) und schweren (SEQ ID NO.22) Ketten von TPP-2093; CDRs sowohl der schweren als auch der leichten Kette sind unterstrichen.
**TPP-2148** Aminosäurenequenzen der leichten (SEQ ID NO.31) und schweren (SEQ ID NO.32) Ketten von TPP-2148; CDRs sowohl der schweren als auch der leichten Kette sind unterstrichen.
**TPP-2084** Aminosäurenequenzen der leichten (SEQ ID NO.41) und schweren (SEQ ID NO.42) Ketten von TPP-2084; CDRs sowohl der schweren als auch der leichten Kette sind unterstrichen.
**TPP-2077** Aminosäurenequenzen der leichten (SEQ ID NO.51) und schweren (SEQ ID NO.52) Ketten von TPP-2077; CDRs sowohl der schweren als auch der leichten Kette sind unterstrichen.
**TPP-1538** Aminosäurenequenzen der leichten (SEQ ID NO.61) und schweren (SEQ ID NO.62) Ketten von TPP-1538; CDRs sowohl der schweren als auch der leichten Kette sind unterstrichen.
**TPP-1854** Aminosäurenequenzen der leichten (SEQ ID NO.81) und schweren (SEQ ID NO.82) Ketten von TPP-1854; CDRs sowohl der schweren als auch der leichten Kette sind unterstrichen.
**TPP-1853** Aminosäurenequenzen der leichten (SEQ ID NO.91) und schweren (SEQ ID NO.92) Ketten von TPP-1853; CDRs sowohl der schweren als auch der leichten Kette sind unterstrichen.
**TPP-1857** Aminosäurenequenzen der leichten (SEQ ID NO.101) und schweren (SEQ ID NO.102) Ketten von TPP-1857; CDRs sowohl der schweren als auch der leichten Kette sind unterstrichen.
**TPP-1858** Aminosäurenequenzen der leichten (SEQ ID NO.111) und schweren (SEQ ID NO.112) Ketten von TPP-1858; CDRs sowohl der schweren als auch der leichten Kette sind unterstrichen.

### BEISPIEL 2: Biochemische Charakteristika der Antikörper

### Bestimmung von Bindungs-Affinitäten mittels Biacore-Analyse:

Bindungs-Affinitäten von anti-TWEAKR Antikörpern wurden mittels Oberflächenplasmonresonanz-Analyse auf einem Biacore T100-Gerät (GE Healthcare Biacore, Inc.) durchgeführt. Die Antikörper wurden auf einen CM5-Sensorchip über ein indirektes Erfassungsreagenz immobilisiert, anti-humanes IgG(Fc). Reagenzien des "Human Antibody Capture Kit" (BR-1008-39, GE Healthcare Biacore, Inc.) wurden wie vom Hersteller beschrieben verwendet. Anti-TWEAKR Antikörper wurden bei einer Konzentration von 10µg/ml bei 10µl/min für 10 sec injiziert.

**Tabelle AK-3: Liste von rekombinantem Antigen (TWEAKR) verwendet für Affinitätsmessung**

| **Nomenklatur** | **Beschreibung** | **Origin** | **Kat.-Nr. (Fitzgerald Inc)** | **SEQ ID NO** |
|---|---|---|---|---|
| TPP-2305 | hTNFRSF12Aminosäurena28-80 | Human | 30R-AT080 | 168 |

**Tabelle AK-4: Liste von Antikörpern, die für die Affinitätsmessung verwendet wurden**

| **Nomenklatur** | **Beschreibung** | **SEQ ID NO** | |
|---|---|---|---|
| | | **Leichte Kette** | **Schwere Kette** |
| P3G5(TPP-2195) | Murines IgG2a | 121 | 122 |
| P4A8(TPP-1324) | Humanes IgG1 | 125 | 126 |
| P2D3(TPP-2196) | Murines IgG2a | 131 | 132 |
| 136.1(TPP-2194) | Murines IgG2a | 123 | 124 |
| PDL-192(TPP-1104) | Humanes IgG1 | 127 | 128 |
| 18.3.3(TPP-2193) | Murines IgG2a | 129 | 130 |
| TPP-883 | Humanes IgG1 | 71 | 72 |
| TPP-1538 | Humanes IgG1 | 61 | 62 |
| TPP-2077 | Humanes IgG1 | 51 | 52 |
| TPP-2084 | Humanes IgG1 | 41 | 42 |
| TPP-2148 | Humanes IgG1 | 31 | 32 |
| TPP-2093 | Humanes IgG1 | 21 | 22 |
| TPP-2149 | Humanes IgG1 | 11 | 12 |
| TPP-2090 | Humanes IgG1 | 1 | 2 |

**Tabelle AK-5: Liste von gewerblich erhältlichen Antikörpern, die für die Affinitätsmessung verwendet wurden**

| **Nomenklatur** | **Beschreibung** | **Kat.-Nr. (Abcam)** |
|---|---|---|
| ITEM-1 | Murines IgG1 | ab21359 |
| ITEM-4 | Murines IgG1 | ab21127 |

Verschiedene Konzentrationen (200 nM, 100 nM, 50 nM, 25 nM, 12,5 nM, 6,25 nM, 3,12 nM, 1,56nM) von gereinigtem rekombinantem humanem TWEAKR-Protein (TPP-2305, SEQ ID NO:168) wurden in HEPES-EP-Puffer (GE Healthcare Biacore, Inc.) über immobilisierte anti-TWEAKR Antikörper bei ein Durchflussrate von 60 µl/min für 3 Minuten injiziert, und die Dissoziationszeit betrug 5 Minuten. Sensorgramme wurden nach In-Line-Referenzzellkorrektur erstellt, gefolgt von Subtraktion der Puffer-Probe. Die Dissoziationsgleichgewichtskontante (K_{D}) wurde berechnet basierend auf dem Verhältnis von Assoziations- (kₒₙ) und Dissoziations- (k_{off})-Konstanten, erhalten mittels Fitting-Sensorgrammen mit einem 1:1 Bindungsmodell der ersten Ordnung.

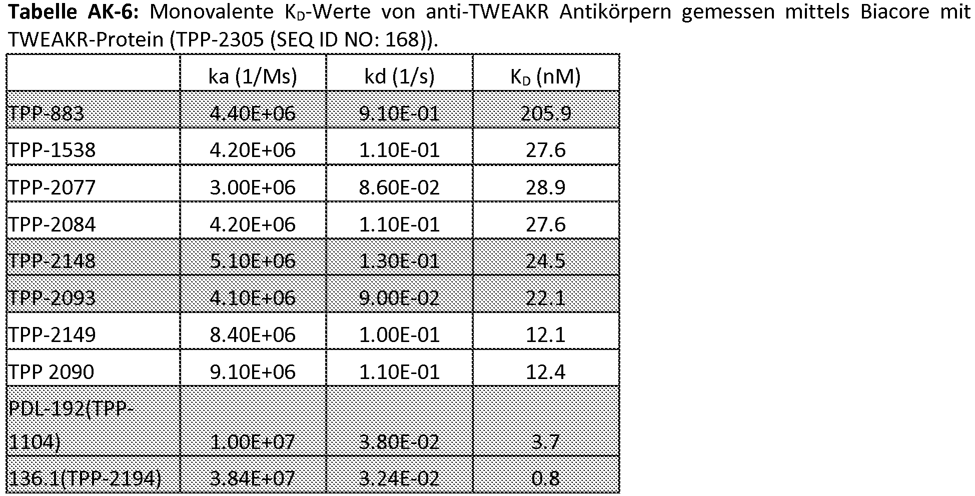

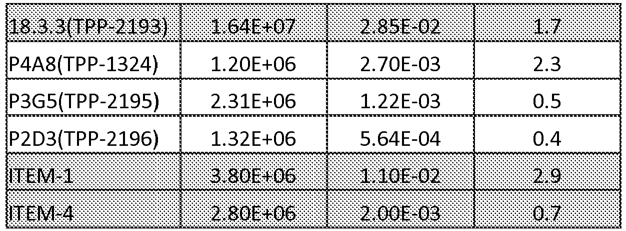

Es wurde bestimmt, dass die Antikörper der Erfindung TWEAKR mit moderater Affinität (K_{D} 10 - 200 nM) binden, während einige Antikörper Vergleichsantikörper (e.g. PDL-192(TPP-1104), 136.1(TPP-2194), 18.3.3(TPP-2193), P4A8(TPP-1324), P3G5(TPP-2195), P2D3(TPP-2196), ITEM-1, ITEM-4) eine hochaffine Bindung zeigen (0.7 - 3.7 nM). Die Sequenzen der variable Domänen der Antikörper PDL-192, 136.1, 18.3.3, P4A8, P3G5 und P2D3 wurden aus der Patentliteratur WO2009/020933 und WO2009/140177 erhalten, und die Sequenzen, die konstante Region von humanem IgG1 und murinem IgG2 kodieren, wurden hinzugegeben, woraus Volllängen-IgGs PDL-192(TPP-1104), 136.1(TPP-2194), 18.3.3(TPP-2193), P4A8(TPP-1324), P3G5(TPP-2195), P2D3(TPP-2196) hervorgingen. Der Bereich der Affinitäten, die in dieser Studie gemessen wurde, stimmt gut mit veröffentlichten Daten überein: für PDL-192, 18.3.3 und 136.1, wurden K_{D}-Werte von 5.5, 0.2 und 0.7 nM veröffentlicht (WO2009/020933); für P4A8 2.6 nM (WO2009/140177). Zum Vergleich: der native Liganden TWEAK bindet TWEAKR mit ein K_{D}-Wert von 0.8 - 2.4 nM (Immunity. 2001 Nov;15(5):837-46; Biochem J. 2006 Jul 15;397(2):297-304; Arterioscler Thromb Vasc Biol. 2003 Apr 1;23(4):594-600).

Als Ergebnis lässt sich festhalten, dass die Antikörper der Erfindung (TPP-883, TPP-1538, TPP-2077, TPP-2084, TPP-2148, TPP-2093, TPP-2149 und TPP-2090) TWEAKR mit moderater Affinität (K_{D} 10 - 200 nM) binden.

### Charakterisierung des Bindungsepitops von TPP-2090 mittels N- und C-terminal verkürzten Varianten der TWEAKR-Ektodomäne:

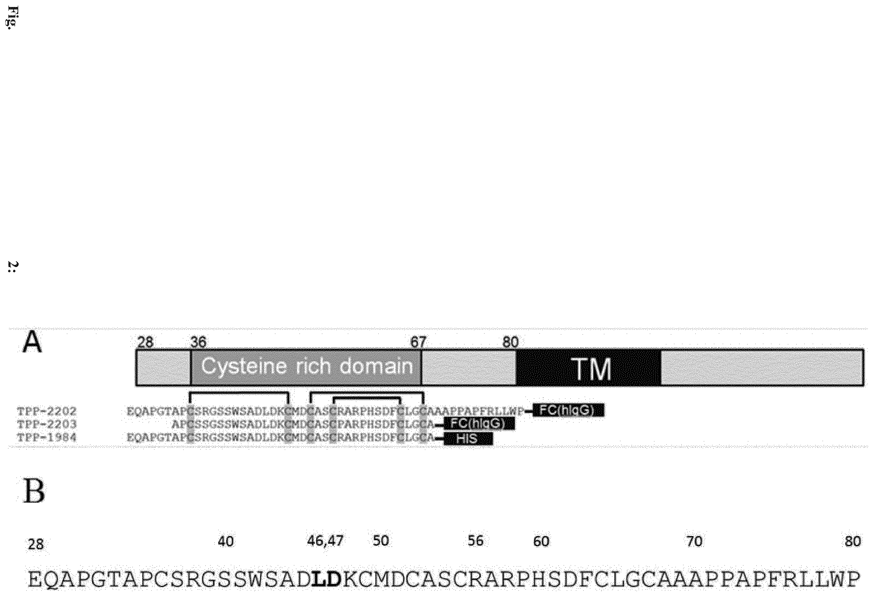

Das Alignment der Cysteinreichen Domäne von TWEAKR (Aminosäuren 34-68) von verschiedenen Spezies (Figur 1-Alignment) zeigt, dass er in allen 6 analysierten Spezies gut konserviert ist. PDL-192 bindet abhängig von R56 (WO2009/020933: Figur 2B) und bindet deshalb nicht an Ratten-, Schweine- und Maus-TWEAKR. TPP-2090 bindet abhängig von der konservierten Aminosäure D47, und deshalb bindet an alle dargestellten Spezies.

In einem ersten Ansatz, um das Bindungsepitop der vorher genannten Antikörper zu charakterisieren, wurde eine N- und C-terminal verkürzte Mutante der TWEAKR-Ektodomäne erzeugt und auf ihre Fähigkeit untersucht, die verschiedenen anti-TWEAKR Antikörper zu binden. N-terminal wurden Aminosäuren 28 bis 33 und C-terminal wurden Aminosäuren 69 bis 80 deletiert, so dass die cysteinreichen Domänen mit Disulfidbrücken zwischen Cys36-Cys49, Cys52-Cys67 und Cys55-Cys64 intakt blieben (vergleiche Figur 2). Beide Konstrukte, die volle Ektodomäne 28-80 einschließlich N- und C-Terminus und die verkürzte Ektodomäne 34-68, wurden als Fc-Fusionsproteine TPP-2202 bzw. TPP-2203 exprimiert und gereinigt.

Um die Bindung zu analysieren, wurde 1 µg/ml des entsprechenden dimeren TWEAKR-Fc-Konstrukts beschichtet, und 0,3 µg/ml und 0,08 µg/ml von biotinyliertem IgG wurden als löslicher Bindungspartner verwendet. Der Nachweis wurde mit Streptavidin-HRP und Amplex-Red Substrat vorgenommen. IgGs wurden unter Verwendung eines ca. 2-fachen molaren Überschusses von Biotin-LC-NHS (Pierce; Kat.-Nr. 21347) gemäß den Angaben des Herstellers biotinyliert und entsalzt unter Verwendung von Zeba Entsalzungssäulen (Pierce; Kat.-Nr. 89889). Bei allen angewandten Konzentrationen des löslichen Liganden zeigen die Antikörper der vorliegenden Erfindung eine gesättigte Bindung an beide Konstrukte, während Antikörper P4A8(TPP-1324), P3G5(TPP-2195) und Item-4 gesättigte Bindung lediglich an die Vollängen-Ektodomäne , aber eine verschlechterte Bindung an die N- und C-terminal verkürzten Konstrukte zeigen (Figur 3 & Figur 4). Dies zeigt an, dass sich das Bindungsepitop der Antikörper der vorliegenden Erfindung in der cysteinreichen Domäne zwischen Aminosäure 34-68 befindet. Um zu analysieren, ob der N-Terminus oder der C-Terminus der TWEAKR-Ektodomäne für P4A8(TPP-1324)- und P3G5(TPP-2195)-Bindung benötigt ist, wurde ein monomere Ektodomäne mit der C-terminalen Deletion von Aminosäuren 69 bis 80 erzeugt. Die Bindung von P4A8(TPP-1324) und P3G5(TPP-2195) an die C-terminal verkürzte TWEAKR - Ektodomäne ist ebenfalls verschlechtert, während die Antikörper der vorliegenden Erfindung eine gesättigte Bindung zeigen (Figur 5).

**Tabelle AK-9: Liste rekombinanter Antigene, die im ELISA-Analyse für Epitop-Profiling verwendet wurden**

| **Nomenklatur** | **Beschreibung** | **SEQ ID NO** |
|---|---|---|
| TPP-2202 | TWEAKR-ECD-28-80-hIgGFc-His | 139 |
| TPP-2203 | TWEAKR-ECD-34-68-hIgGFc-His | 140 |
| TPP-1984 | hTNFRSF12Aminosäurena28-68-CT-His | 141 |

**Tabelle AK-10: Liste der Antikörper, die im ELISA-Analyse für Epitop-Profiling verwendet wurden**

| **Nomenklatur** | **Beschreibung** | **SEQ ID NO** | |
|---|---|---|---|
| | | **Leichte Kette** | **Schwere Kette** |
| P3G5(TPP-2195) | Murines IgG2a | 121 | 122 |
| P4A8(TPP-1324) | Humanes IgG1 | 125 | 126 |
| 136.1(TPP-2194) | Mmurines IgG2a | 123 | 124 |
| PDL-192(TPP-1104) | Humanes IgG1 | 127 | 128 |
| TPP 2090 | Humanes IgG1 | 1 | 2 |
| TPP-2084 | Humanes IgG1 | 41 | 42 |

Also ist das Bindungsepitop von TPP-2090, TPP-2084, PDL-192(TPP-1104) und 136.1(TPP-2194) in der cysteinreichen Domäne und das Bindungsepitop von P4A8(TPP-1324) und P3G5(TPP-2195) mindestens teilweise außerhalb der der cysteinreichen Domäne lokalisiert.

### Wirkung von TWEAKR-Fc-Muteinen auf die Antikörper-Affinität:

Um die Bindungcharakteristika der Antikörper der Erfindung detaillierter zu untersuchen, wurden bestimmte-Muteine von TWEAKR, von denen vorgeschlagen worden ist, dass sie für die Wirkung bekannter agonistischer Antikörper relevant seien, wurden untersucht (WO2009/140177). Deshalb wurden die Vollängen-Ektodomäne (Aminosäuren 28-80) mit den nachfolgenden Einzel-Aminosäurenubstitutionen als Fc Fusions-Proteine exprimiert und gereinigt: T33Q; S40R; W42A; M50A; R56P; H60K; L65Q.

**Tabelle AK-11: Liste von rekombinanten Proteinen, die in der ELISA-Analyse für Muteinbindung verwendet wurden**

| **Nomenklatur** | **Beschreibung** | **SEQ ID NO** |
|---|---|---|
| TPP-1990 | hTNFRSF12Aminosäurena28-80-L65Q-hIgG1-Fc | 142 |
| TPP-1989 | hTNFRSF12Aminosäurena28-80-H60K-hIgG1-Fc | 143 |
| TPP-2683 | hTNFRSF12Aminosäurena28-80-R56P-hIgG1-Fc | 144 |
| TPP-1988 | hTNFRSF12Aminosäurena28-80-M50A-hIgG1-Fc | 145 |
| TPP-1985 | hTNFRSF12Aminosäurena28-80-W42A-hIgG1-Fc | 146 |
| TPP-1987 | hTNFRSF12Aminosäurena28-80-S40R-hIgG1-Fc | 147 |
| TPP-1986 | hTNFRSF12Aminosäurena28-80-T33Q-hIgG1-Fc | 148 |
| TPP-599 | hTNFRSF12Aminosäurena28-80-hIgG1-Fc | 138 |

Um Dosis-Reaktions-Daten zu erhalten, wurden die verschiedenen TWEAKR-Fc-Muteine mit einer niedrigen Konzentration (62 ng/ml) auf eine 384-Loch Maxisorb ELISA-Platte beschichtet, und eine serielle 2-fache Verdünnung von biotinyliertem IgG, beginnend mit einer Konzentration von 100 nM wurde als löslicher Bindungspartner verwendet. Der Nachweis wurde mit Streptavidin-HRP und Amplex Red vorgenommen. Die untersuchten IgGs waren TPP-2090 und TPP-2084 der vorliegenden Erfindung, PDL-192, 136.1 und 18.3.3 von WO2009/020933, P4A8 und P3G5 von WO2009/140177, und ITEM-1 und ITEM-4 von Nakayama et al [Biochem Biophys Res Com 306: 819-825].

**Tabelle AK-12: Liste der Antikörper, die in der ELISA-Analyse für Muteinbindung verwendet wurden**

| **Nomenklatur** | **Beschreibung** | **SEQ ID NO** | |
|---|---|---|---|
| | | **Leichte Kette** | **Schwere Kette** |
| P3G5(TPP-2195) | Murines IgG2a | 121 | 122 |
| P4A8(TPP-1324) | Humanes IgG1 | 125 | 126 |
| 136.1(TPP-2194) | Murines IgG2a | 123 | 124 |
| PDL-192(TPP-1104) | Humanes IgG1 | 127 | 128 |
| 18.3.3(TPP-2193) | Murines IgG2a | 129 | 130 |
| TPP 2090 | Humanes IgG1 | 1 | 2 |
| TPP-2084 | Humanes IgG1 | 41 | 42 |

**Tabelle AK-13: Liste von gewerblich erhältlichen Antikörpern, die im ELISA für Muteinbindung verwendet wurden**

| **Nomenklatur** | **Beschreibung** | **Kat.-Nr. (Abcam)** |
|---|---|---|
| ITEM-1 | Murines IgG1 | ab21359 |
| ITEM-4 | Murines IgG1 | ab21127 |

IgGs wurden unter Verwendung von einem ca. 2-fachen molaren Überschuss von Biotin-LC-NHS (Pierce; Kat.-Nr. 21347) gemäß den Angaben des Herstellers biotinyliert und entsalzt unter Verwendung von Zeba Entsalzungssäulen (Pierce; Kat.-Nr. 89889). Die Dosis-Reaktions-Daten wurden gefittet und die IC50s bestimmt. Zur Veranschaulichung der Ergebnisse wurde eine Tabelle erstellt, "-" zeigt IC50s über 50 nM an, "+" zeigt IC50s im Bereich von 1 to 150 pM an.

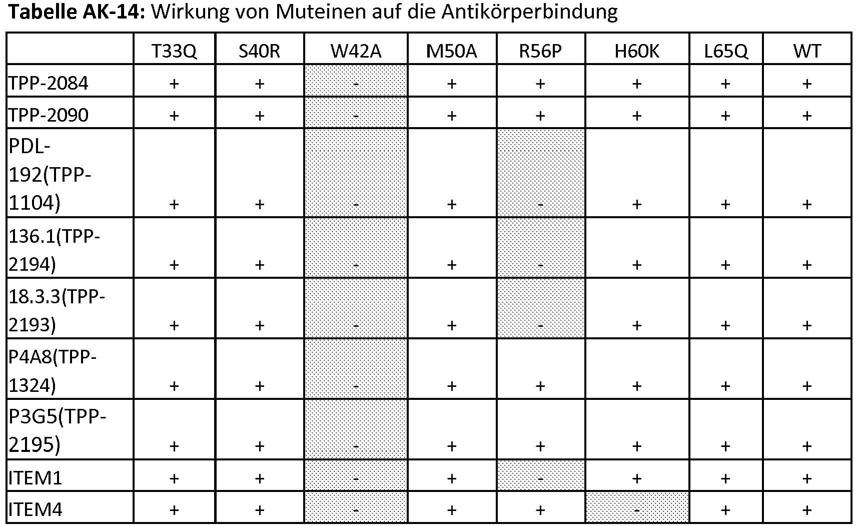

Wie bereits zuvor veröffentlicht, zeigt ITEM-4 verschlechterte Bindung an das H60K-Mutein [WO2009/140177: Figur 23F] und PDL-192 an das R56P-Mutein [WO2009/020933: Figur 22B]. Im Gegensatz zu veröffentlichten Daten, zeigt ITEM-1 eine verschlechterte Bindung an R56P, und alle Antikörper an W42A [WO2009/140177: Figur 23E, Figur 23F]. Diese Unterschiede können über die gewählten Methoden erklärt werden.

Im Gegensatz zu ITEM-1, ITEM-4, PDL-192, 136.1 und 18.3.3, binden die Antikörper der vorliegenden Erfindung unabhängig von allen Substitutionen außer W42A.

### Alanin-Scan der Cysteinreichen Domäne:

Um die Bindungsstelle der Antikörper der Erfindung zu bestimmen, wurde ein Alanin-Scan der cysteinreichen Domäne (Aminosäuren 34-68) durchgeführt. In Figur 6 wird gezeigt, dass N- und C-terminal verkürzte Varianten der Vollängen-Ektodomäne von TWEAKR die Bindung der Antikörper der Erfindung nicht verschlechtern. Deshalb ist das Bindungsepitop in der cysteinreichen Domäne lokalisiert. Die nachfolgenden Substitutionen wurden in die TWEAKR(34-68)-Fc-Konstrukt eingeführt: S37A, R38A, S40A, S41A, W42A, S43A, D45A, D47A, K48A, D51A, S54A, R56A, R58A, P59A, H60A, S61A, D62A, F63A und L65A.

**Tabelle AK-15: Liste von TWEAKR-Mutein Konstrukte für den Alanin-Scan der cysteinreichen Domäne**

| **Nomenklatur** | **Beschreibung** | **SEQ ID NO** |
|---|---|---|
| TPP-2203 | TweaKR-ECD-34-68-hIgGFc-His | 140 |
| TPP-2625 | TweakR-ECD-34-68-hIgGFc-His-L65A | 149 |
| TPP-2624 | TweakR-ECD-34-68-hIgGFc-His-F63A | 150 |
| TPP-2623 | TweakR-ECD-34-68-hIgGFc-His-D62A | 151 |
| TPP-2622 | TweakR-ECD-34-68-hIgGFc-His-S61A | 152 |
| TPP-2621 | TweakR-ECD-34-68-hIgGFc-His-H60A | 153 |
| TPP-2620 | TweakR-ECD-34-68-hIgGFc-His-P59A | 154 |
| TPP-2619 | TweakR-ECD-34-68-hIgGFc-His-R58A | 155 |
| TPP-2618 | TweakR-ECD-34-68-hIgGFc-His-R56A | 156 |
| TPP-2617 | TweakR-ECD-34-68-hIgGFc-His-S54A | 157 |
| TPP-2616 | TweakR-ECD-34-68-hIgGFc-His-D51A | 158 |
| TPP-2615 | TweakR-ECD-34-68-hIgGFc-His-K48A | 159 |
| TPP-2614 | TweakR-ECD-34-68-hIgGFc-His-D47A | 160 |
| TPP-2613 | TweakR-ECD-34-68-hIgGFc-His-D45A | 161 |
| TPP-2612 | TweakR-ECD-34-68-hIgGFc-His-S43A | 162 |
| TPP-2611 | TweakR-ECD-34-68-hIgGFc-His-W42A | 163 |
| TPP-2610 | TweakR-ECD-34-68-hIgGFc-His-S41A | 164 |
| TPP-2609 | TweakR-ECD-34-68-hIgGFc-His-S40A | 165 |
| TPP-2608 | TweakR-ECD-34-68-hIgGFc-His-R38A | 166 |
| TPP-2607 | TweakR-ECD-34-68-hIgGFc-His-S37A | 167 |

Diese TWEAKR(34-68)-Fc-Muteine wurden in HEK293 Zellen exprimiert. Um Dosis-Reaktions-Daten zu erhalten, wurden IgGs bei einer Konzentration von 1 µg/ml in eine 384-Loch Maxisorp ELISA-Platte beschichtet, und ein serielle 2-fache Verdünnung des TWEAKR-Mutein enthaltenden Überstands wurde als löslichen Bindungspartner verwendet. Der Nachweis wurde mit anti-HIS-HRP und Amplex Red vorgenommen. Die untersuchten IgGs waren TPP-2090 der vorliegenden Erfindung, PDL-192 von WO2009/020933 und P4A8 von WO2009/140177.

**Tabelle AK-16: Liste von Antikörpern, die für den Alaninscan der cysteinreichen Domäne verwendet wurden**

| **Nomenklatur** | **Beschreibung** | **SEQ ID NO** | |
|---|---|---|---|
| | | **Leichte Kette** | **Schwere Kette** |
| P4A8(TPP-1324) | Humanes IgG1 | 125 | 126 |
| PDL-192(TPP-1104) | Humanes IgG1 | 127 | 128 |
| TPP 2090 | Humanes IgG1 | 1 | 2 |

Um die Relevanz der TWEAKR-Mutein für Bindung an verschiedene IgGs zu beurteilen, wurde ein Korrelations-Blot bei einer bestimmten-Mutein Konzentration wurde vorbereitet. beispielhaft sind in Figur 6 die Korrelations-Blots für die 8-fach verdünnten Überstände der TWEAKR Expressionsbrühe gezeigt, mit PDL-192(TPP-1104) auf der X-Achse und TPP-2090 auf der Y-Achse. Die Blot zeigt, dass die Bindung von TPP-2090 verschlechtert mittels Substitution D47A, und die Bindung von PDL-192(TPP-1104) mittels Substitution R56A verschlechtert wurde. Für all Konstrukte konnte keine Bindung an P4A8(TPP-1324) nachgewiesen werden, was mit den vorher erhaltenen Ergebnissen (Figur 6) übereinstimmt. Also ist das P4A8-Epitop mindestens teilweise außerhalb der der cysteinreichen Domäne lokalisiert. Die identifizierten Abhängigkeiten von bestimmten TWEAKR-Aminosäuren für die Antikörperinteraktion korrelieren mit der agonistischen Aktivität, die für diese Antikörper bestimmt wurde. Der native Ligand TWEAK zeigt wirksame Aktivierung von TWEAKR und bindet abhängig von Leucin 46 an der cysteinreichen Domäne von TWEAKR (Pellegrini et al, FEBS 280:1818-1829). P4A8 zeigt sehr niedrige agonistische Aktivität und interagiert mindestens teilweise mit Domänen außerhalb der cysteinreichen Domäne von TWEAKR. PDL-192 zeigt moderate agonistische Aktivität und bindet abhängig von R56 an die cysteinreichen Domäne, aber gegenüber der TWEAK Ligandenstelle. TPP-2090 und TWEAK binden abhängig von D47 bzw. L46, und deshalb binden deshalb an eine ähnliche Bindungsstelle (Figur 7).

Es lässt sich schlussfolgern, dass die Antikörper der Erfindung (e.g. TPP-2090) an TWEAKR abhängig von D47 binden.

Die identifiziert Abhängigkeiten von bestimmten TWEAKR-Aminosäuren für die Antikörperinteraktion korrelieren mit der agonistischen Aktivität, die für diese Antikörper bestimmt worden ist. Der native Ligand TWEAK zeigt eine wirksame Aktivierung von TWEAKR und bindet abhängig von Leucin 46 an die cysteinreiche Domäne von TWEAKR (Pellegrini et al, FEBS 280:1818-1829). P4A8 zeigt sehr niedrige agonistische Aktivität und interagiert mindestens teilweise mit Domänen außerhalb der cysteinreichen Domäne von TWEAKR. PDL-192 zeigt moderate agonistische Aktivität und bindet abhängig von R56 an die cysteinreichen Domäne, aber gegenüber der TWEAK Ligandenstelle. Antikörper dieser Erfindung (Beispiel TPP-2090) binden abhängig von D47, und TWEAK bindet abhängig von L46, und bindet an eine ähnliche, aber unterscheidbare Bindungsstelle (Figur 7). Deshalb binden die Antikörper dieser Erfindung, die eine starke agonistische Aktivität zeigen, an ein neues Epitop (D47 abhängig) für Antikörper, das mit sehr starker agonistischer Aktivität verbunden ist. Interessanterweise gaben Michaelson et al (siehe Seite 369, linke Spalte in Michaelson JS et al, MAbs. 2011 Jul-Aug;3(4):362-75) eine Erklärung dafür, warum alle von ihnen untersuchten agonistischen Antikörper eine schwächere agonistische Aktivität verglichen mit dem natürlichen Liganden TWEAK haben. Ihre Schlussfolgerung ist, die verminderte Wirksamkeit könnte eine Funktion der dimeren Bindunginteraktion der Antikörper mit TWEAKR sein, wobei TWEAK wahrscheinlich eine eine trimere Interaktion eingeht. Deshalb ist es ein überraschendes Ergebnis, dass ein Antikörper der Erfindung, trotz einer dimeren Interaktion mit TWEAKR eine noch höhere agonistische Aktivität hat. Diese überraschende Wirkung ist an die spezifische Bindungseigenschaft der Antikörper der Erfindung geknüpft, also die spezifische Bindung an D47 von TWEAKR.

Weitere Ausführungsformen Der Schutzumfang wird lediglich durch die Ansprüche definiert.
1. Konjugat eines Binders oder Derivats hiervon mit einem oder mehreren Wirkstoffmolekülen, wobei das Wirkstoffmolekül ein Kinesin Spindel Protein-Inhibitor ist, das mit dem Binder über einen Linker L verbunden ist.
2. Konjugat nach Ziffer 1, wobei der Binder oder Derivat hiervon ein Binderpeptid oder -protein oder ein Derivat eines Binderpeptids oder -proteins ist.
3. Konjugat nach Ziffer 2, wobei jedes Wirkstoffmolekül an verschiedene Aminosäuren des Binderpeptids oder -proteins bzw. Derivats hiervon über den Linker gebunden ist.
4. Konjugat nach einem oder mehreren der vorhergehenden Ziffern, wobei das Konjugat im Durchschnitt 1,2 bis 20 Wirkstoffmoleküle pro Binder aufweist.
5. Konjugat nach einem oder mehreren der Ziffern 2 bis 4, wobei das Binderpeptid oder -protein einen Antikörper bzw. das Derivat des Binderpeptids oder -proteins 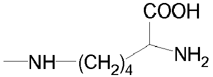 oder 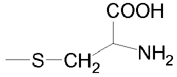 darstellt.
6. Konjugat nach einem oder mehreren der vorhergehenden Ziffern, wobei der Binder an ein Krebs-Zielmolekül bindet.
7. Konjugat nach Ziffer 6, wobei der Binder an ein extrazelluläres Zielmolekül bindet.
8. Konjugat nach Ziffer 7, wobei der Binder nach Bindung an das extrazelluläre Zielmolekül von der das Zielmolekül exprimierenden Zelle internalisiert und intrazellulär (vorzugsweise lysosomal) prozessiert wird.
9. Konjugat nach einem oder mehreren der Ziffern 2 bis 8, wobei das Binderpeptid oder -protein ein humaner, humanisierter oder chimärer monoklonaler Antikörper oder ein Antigen-bindendes Fragment hiervon ist.
10. Konjugat nach Ziffer 9, wobei das Binderpeptid oder -protein ein anti-HER2-Antikörper, eine anti-EGFR-Antikörper, ein anti-TWEAKR-Antikörper, oder ein Antigen-bindendes Fragment hiervon ist.
11. Konjugat nach Ziffer 10, wobei der anti-TWEAKR-Antikörper spezifisch an die Aminosäure D in Position 47 (D47) von TWEAKR (SEQ ID NO:169) bindet, bevorzugt der anti-TWEAKR-Antikörper TPP-2090.
12. Konjugat nach einem oder mehreren der vorhergehenden Ziffer, wobei der Kinesin Spindel Protein-Inhibitor die folgende Substruktur enthält wird: 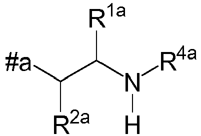 wobei
   #a eine Bindung zum Restmolekül darstellt;
   R^{1a} H oder -(CH₂)₀₋₃Z darstellt, wobei Z -H, -OY³, -SY³, -NHY³, Halogen, -CO-NY¹Y², oder - CO-OY³ darstellt,
   mit Y¹ und Y² unabhängig voneinander H, NH₂, -(CH₂CH₂O)₀₋₃-(CH₂)₀₋₃Z', oder oder -CH(CH₂W)Z' darstellen, und Y³ H oder -(CH₂)₀₋₃Z' darstellt, wobei Z' H, SO₃H, NH₂, COOH, -NH-CO-CH₂-CH₂-CH(NH₂)COOH oder -(CO-NH-CHY⁴)₁₋₃COOH darstellt, wobei W H oder OH darstellt, wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
   R^{2a} und R^{4a} unabhängig voneinander H, -CO-CHY⁴-NHY⁵ oder -(CH₂)₀₋₃Z darstellen, oder R^{2a} und R^{4a} gemeinsam (unter Bildung eines Pyrrolidinringes) -CH₂-CHR¹⁰- oder -CHR¹⁰-CH₂-darstellen, wobei R¹⁰ H, NH₂, COOH, SO₃H, SH, oder OH darstellt;
   wobei Z -H, -OY³, -SY³, -NHY³, -CO-NY¹Y², oder -CO-OY³ darstellt,
   wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt;
   wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
   wobei der Kinesin Spindel Protein-Inhibitor durch Substitution eines H-Atoms an R^{1a}, R^{2a}, R^{4a} oder an dem durch R^{2a} und R^{4a} gebildeten Pyrrolidinring mit dem Linker verbunden ist,
   sowie ihre Salze, Solvate und Salze der Solvate.
13. Konjugat nach einem oder mehreren der vorhergehenden Ziffern, wobei der Kinesin Spindel Protein-Inhibitor durch die allgemeine Formel (I) dargestellt wird: 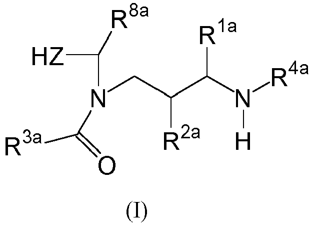 wobei
   R^{1a} H oder -(CH₂)₀₋₃Z darstellt, wobei Z -H, -OY³, -SY³, -NHY³, Halogen, -CO-NY¹Y², oder - CO-OY³ darstellt,
   mit Y¹ und Y² unabhängig voneinander H, NH₂, -(CH₂CH₂O)₀₋₃-(CH₂)₀₋₃Z', oder -CH(CH₂W)Z' darstellen, und Y³ H oder -(CH₂)₀₋₃Z' darstellt, wobei Z' H, SO₃H, NH₂, COOH, -NH-CO-CH₂-CH₂-CH(NH₂)COOH oder -(CO-NH-CHY⁴)₁₋₃COOH darstellt; wobei W H oder OH darstellt, wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
   R^{2a} und R^{4a} unabhängig voneinander H, -CO-CHY⁴-NHY⁵ oder -(CH₂)₀₋₃Z darstellen, oder R^{2a} und R^{4a} gemeinsam (unter Bildung eines Pyrrolidinringes) -CH₂-CHR¹⁰- oder -CHR¹⁰-CH₂-darstellen, wobei R¹⁰ H, SO₃H, NH₂, COOH, SH, oder OH darstellt;
   wobei Z -H, Halogen, -OY³, -SY³, -NHY³, -CO-NY¹Y², oder -CO-OY³ darstellt,
   wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt;
   wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
   R^{3a} eine gegebenenfalls substituierte Alkyl-, Aryl-, Heteroaryl- Heteroalkyl-, Heterocycloalkyl-Gruppe darstellt,
   bevorzugt eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe,
   die mit 1-3 -OH Gruppen, 1-3 Halogenatomen, 1-3 halogenierte Alkyl-Gruppen (die jeweils 1-3 Halogenatome aufweisen können), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 -N(Alkyl)₂ Gruppen, 1-3 -NH₂ Gruppen oder 1-3 - (CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
   substituiert sein kann
   (wobei "Alkyl" bevorzugt C₁₋₁₀-Alkyl bedeutet);
   R^{8a} C1-10-Alkyl darstellt;
   HZ ein mono- oder bicyclischer Heterozyklus ist, der mit einem oder mehreren Substituenten ausgewählt aus Halogen, C₁₋₁₀-Alkylgruppen, C₆₋₁₀-Arylgruppen oder C₆₋₁₀-Aralkylgruppen, die ggf. mit Halogen substituiert sein können, substituiert sein kann,
   wobei der Kinesin Spindel Protein-Inhibitor durch Substitution eines H-Atoms an R^{1a}, R^{2a}, R^{3a}, R^{4a}, R^{8a} oder an dem durch R^{2a} und R^{4a} gebildeten Pyrrolidinring mit dem Linker verbunden ist, sowie ihre Salze, Solvate und Salze der Solvate.
14. Konjugat nach einem oder mehreren der vorhergehenden Ziffern, wobei Wirkstoffmolekül-Linker durch die allgemeine Formel (II) dargestellt wird: 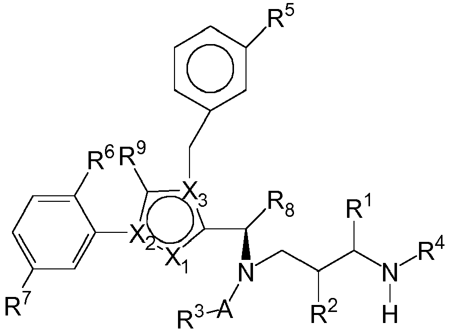 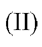 wobei
   X₁ N, X₂ N und X₃ C darstellt; oder
   X₁ CH, X₂ C und X₃ N darstellt; oder
   X₁ NH, X₂ C und X₃ C darstellt; oder
   X₁ CH, X₂ N und X₃ C darstellt;
   R¹ H, -L-#1 oder -(CH₂)₀₋₃Z darstellt, wobei Z -H, -NHY³, -OY³, -SY³, Halogen, -CO-NY¹Y², oder -CO-OY³ darstellt,
   wobei Y¹ und Y² unabhängig voneinander H, NH₂, -(CH₂CH₂O)₀₋₃-(CH₂)₀₋₃Z', oder oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt, wobei W H oder OH darstellt; wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
   R² und R⁴ unabhängig voneinander -L-#1, H, -CO-CHY⁴-NHY⁵ oder -(CH₂)₀₋₃Z darstellen, oder R² und R⁴ gemeinsam (unter Bildung eines Pyrrolidinringes) -CH₂-CHR¹⁰- oder -CHR¹⁰-CH₂-darstellen, wobei R¹⁰ L-#1, H, NH₂, SO₃H, COOH, SH, oder OH darstellt;
   wobei Z -H, OY³, -SY³, Halogen, NHY³, -CO-NY¹Y², oder -CO-OY³ darstellt,
   wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt;
   wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
   A CO, SO, SO₂, SO₂NH oder CNNH darstellt;
   R³ -L-#1 oder eine gegebenenfalls substituierte Alkyl-, Aryl-, Heteroaryl- Heteroalkyl-, Heterocycloalkyl- Gruppe darstellt,
   bevorzugt -L-#1 oder eine C₁₋₁₀-Alkyl-, C₆₋₁₀-Aryl-, C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe,
   die mit 1-3 -OH Gruppen, 1-3 Halogenatomen, 1-3 halogenierte Alkyl-Gruppen (die jeweils 1-3 Halogenatome aufweisen können), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 -N(Alkyl)₂ Gruppen, 1-3 -NH₂ Gruppen oder 1-3-(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
   substituiert sein kann (wobei "Alkyl" bevorzugt C₁₋₁₀-Alkyl bedeutet);
   R⁵ -L-#1, H, F, NH₂, NO₂, Halogen, SH oder -(CH₂)₀₋₃Z darstellt, wobei Z -H, OY³, -SY³, Halogen, NHY³, -CO-NY¹Y², oder -CO-OY³ darstellt,
   wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
   darstellt,
   wobei einer der Substituenten R¹, R², R³, R⁴ und R⁵ -L-#1 darstellt,
   L für den Linker steht und #1 für die Bindung zum Binder bzw. Derivat hiervon steht,
   R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy oder Halogen darstellen,
   R⁸ (ggf. fluoriertes) C₁₋₁₀-Alkyl, (ggf. fluoriertes) C₄₋₁₀-Cycloalkyl oder optional substituiertes Oxetan darstellt; und
   R⁹ H, F, CH₃, CF₃, CH₂F oder CHF₂ darstellt;
   sowie ihre Salze, Solvate und Salze der Solvate.
15. Konjugat nach Ziffer 14,
   wobei R¹ -L-#1, H, -(CH₂)₀₋₃Z darstellt, wobei Z -H, -CO-NY¹Y², NHY³, OY³, -SY³, oder-CO-OY³ darstellt,
   mit Y¹ und Y² unabhängig voneinander H, NH₂, -(CH₂CH₂O)₀₋₃-(CH₂)₀₋₃Z', oder oder-CH(CH₂W)Z' , und Y³ H oder -(CH₂)₀₋₃Z' darstellt, wobei Z' H, NH₂, COOH, -NH-CO-CH₂-CH₂-CH(NH₂)COOH oder -(CO-NH-CHY⁴)₁₋₃COOH darstellt; wobei W H oder OH darstellt, wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
   R² und R⁴ unabhängig voneinander -L-#1, -CO-CHY⁴-NHY⁵ oder H oder R² und R⁴ gemeinsam (unter Bildung eines Pyrrolidinringes) -CH₂-CHR¹⁰- darstellen, wobei R¹⁰ H, -L-#1, NH₂, COOH, SH, OH oder SO₃H darstellt;
   wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
   A CO darstellt,
   R³ -(CH₂)OH oder -L-#1 darstellt, und
   R⁵ -L-#1 oder H darstellt,
   wobei einer der Substituenten R¹, R², R³, R⁴ und R⁵ -L-#1 darstellt.
16. Konjugat nach Ziffer 14 oder 15,
   wobei R⁶ und R⁷ unabhängig voneinander H, C₁₋₃-Alkyl oder Halogen darstellen.
17. Konjugat nach einem oder mehreren der Ziffern 14 bis 16,
   wobei R⁸ C₁₋₄-Alkyl (vorzugsweise tert-butyl) darstellt.
18. Konjugat nach einem oder mehreren der Ziffern 14 bis 17,
   wobei R⁹ H darstellt.
19. Konjugat nach einem oder mehreren der Ziffern 14 bis 18, wobei R⁶ und R⁷ F darstellen.
20. Konjugat nach einem oder mehreren der vorhergehenden Ziffern, wobei der Linker -L- eine der folgenden Grundstrukturen (i) bis (iv) aufweist:
   (i) -(CO)ₘ-SG1-L1-L2-
   (ii) -(CO)ₘ-L1-SG-L1-L2-
   (iii)-(CO)ₘ-L1-L2-
   (iv)-(CO)ₘ-L1-SG-L2
   wobei m 0 oder 1 ist, SG und SG1 eine *in vivo* spaltbare Gruppe ist, L1 unabhängig voneinander für *in vivo* nicht spaltbare organische Gruppen stehen, und L2 für eine Kopplungsgruppe an den Binder steht.
21. Konjugat nach Ziffer 20, wobei die *in vivo* spaltbare Gruppe SG eine 2-8 Oligopeptidgruppe, bevorzugt eine Dipeptidgruppe oder ein Disulfid, ein Hydrazon, ein Acetal oder ein Aminal ist und SG1 eine 2-8 Oligopeptidgruppe, bevorzugt eine Dipeptidgruppe ist.
22. Konjugat nach einem oder mehreren der Ziffern 2 bis 21,
   wobei der Linker an eine Cystein-Seitenkette bzw. Cysteinrest gebunden ist und die folgende Formel aufweist:

   §-(CO)m-L1-L2-§ §

   wobei
   m 0 oder 1 ist;
   § die Bindung an das Wirkstoffmolekül darstellt und
   §§ die Bindung an das Binderpeptid oder -protein darstellt, und
   -L2- für 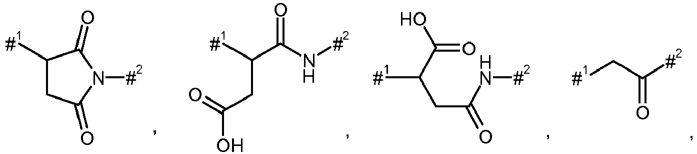 #¹-S-#², 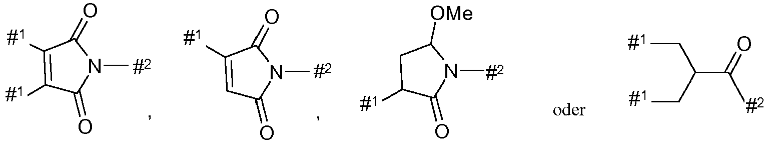 steht, wobei
   #¹ die Verknüpfungsstelle mit dem Schwefelatom des Binders kennzeichnet,
   #² die Verknüpfungsstelle mit der Gruppe L¹ kennzeichnet,
   L1-(NR¹⁰)n-(G1)o-G2- darstellt,
   wobei R¹⁰ H, NH2 oder C1-C3-Alkyl darstellt;
   G1 -NHCO- oder 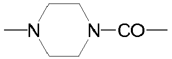 darstellt;
   n 0 oder 1 ist;
   o 0 oder 1 ist; und
   G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, SO₂, -NH-, -CO-, -Nme-, -NHNH-, -SO₂NHNH-,-NHCO-, -CONH-, -CONHNH- und einen 5 bis 10gliedriger, aromatischen oder nicht aromatischen Heterozyklus mit bis zu 4 Heteroatomen ausgewählt aus N, O und S, -SO-oder -SO2- unterbrochen sein kann (vorzugsweise 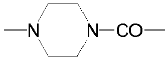 ), wobei die Seitenketten, sofern vorhanden, mit -NHCONH2, -COOH, -OH, -NH₂, NH-CNNH₂, Sulfonamid, Sulfon, Sulfoxid oder Sulfonsäure substituiert sein können,
   oder eine der folgenden Gruppen darstellt: 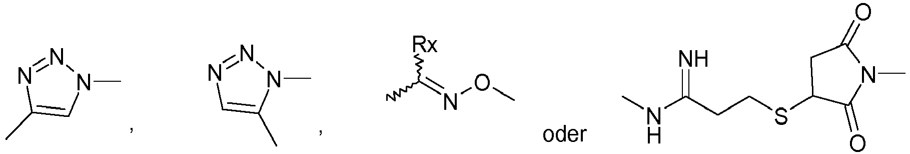 wobei Rx H, C₁-C₃-Alkyl oder Phenyl darstellt.
23. Konjugat nach Ziffer 22, wobei die Kohlenwasserstoffkette durch eine die folgenden Gruppen unterbrochen ist: 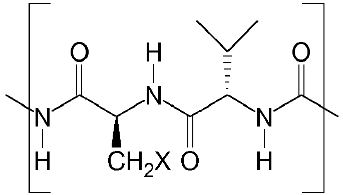 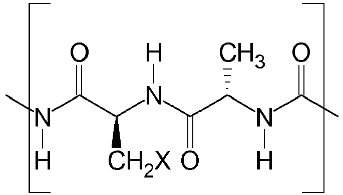 wobei X H, eine C₁₋₁₀-Alkylgruppe ist, die gegebenenfalls mit -NHCONH₂, -COOH, -OH, NH₂, -NH-CNNH₂, Sulfon, Sulfoxid oder Sulfonsäure substituiert sein kann.
24. Konjugat nach Ziffer 22, wobei der Linker die folgende Formel aufweist: 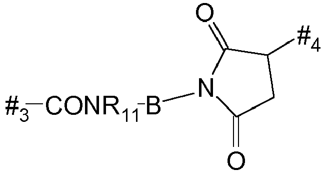 wobei
   #3 die Bindung an das Wirkstoffmolekül darstellt,
   #4 die Bindung an das Binderpeptid oder -protein darstellt,
   R¹¹ H oder NH₂ darstellt;
   B -[(CH₂)ₓ-(X⁴)_{y}]_{W}-(CH₂)_{z}- darstellt,
   w = 0 bis 20 ist;
   x = 0 bis 5 ist;
   x = 0 bis 5 ist;
   y = 0 oder 1 ist;
   z = 0 bis 5 ist; und
   X⁴ -O-, -CONH- oder -NHCO- 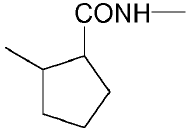 darstellt.
25. Konjugat nach Ziffer 24, wobei R¹ oder R⁴ -L-#1 darstellt.
26. Konjugat nach einem oder mehreren der Ziffern 21 bis 25, wobei das Konjugat eine der folgenden Formeln aufweist: 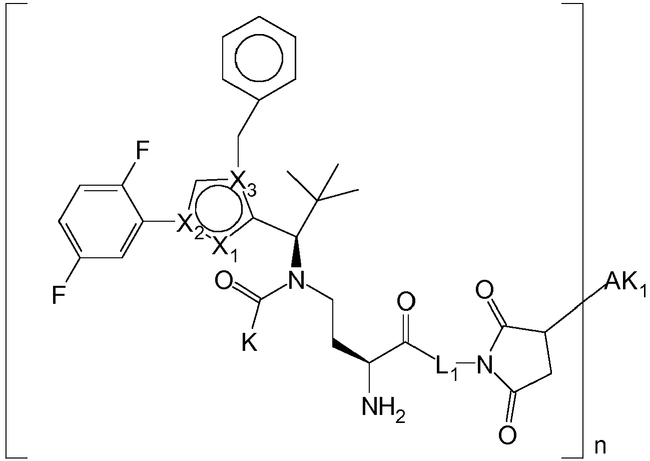 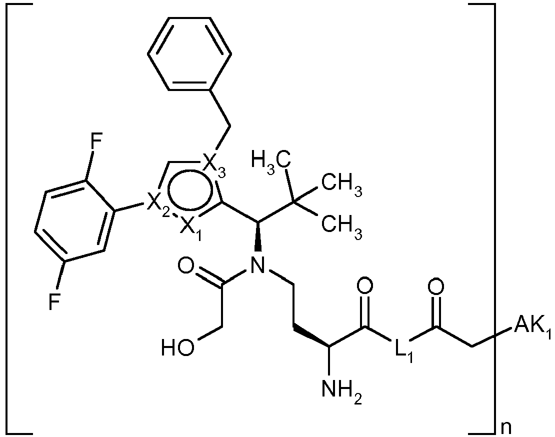 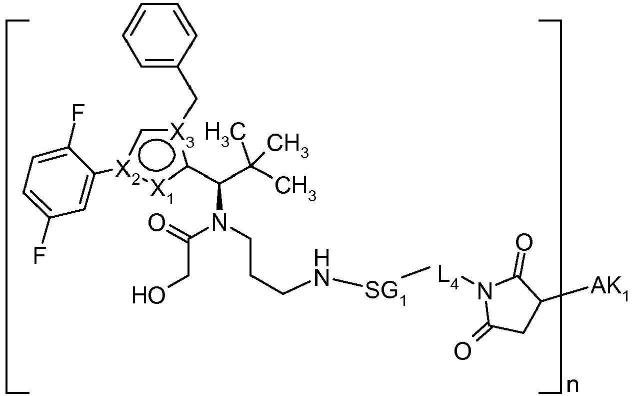 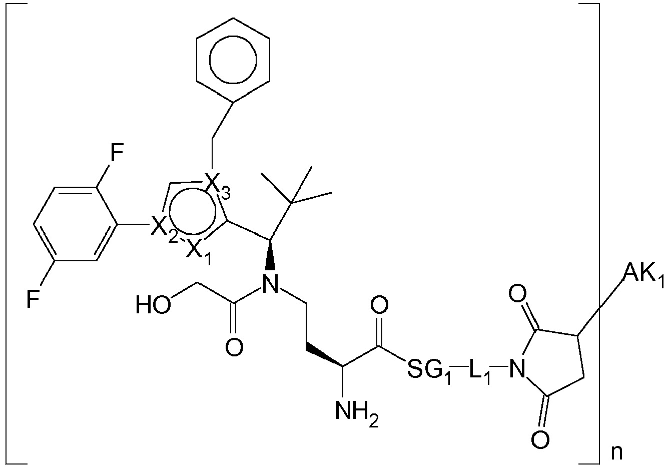 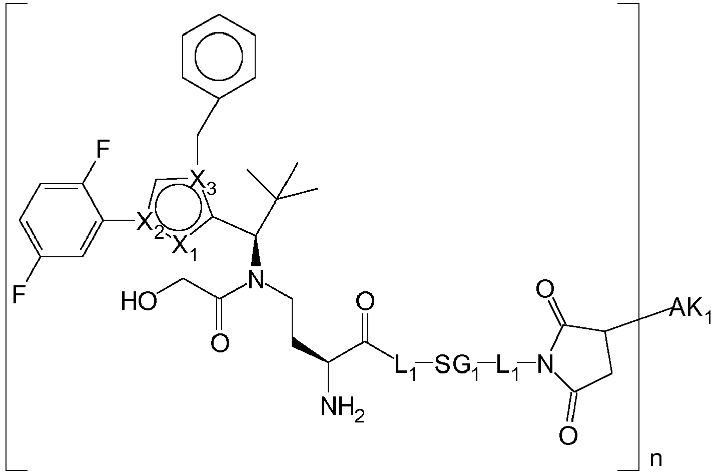 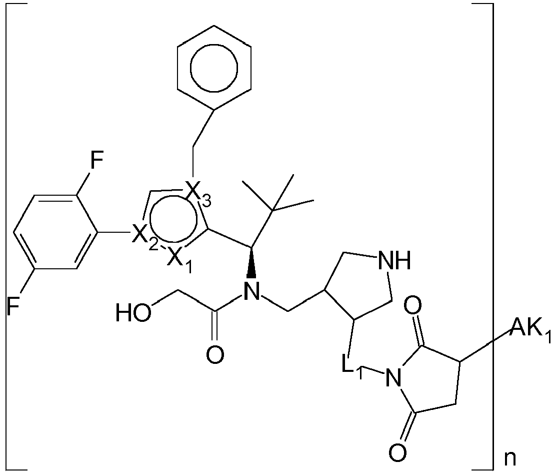 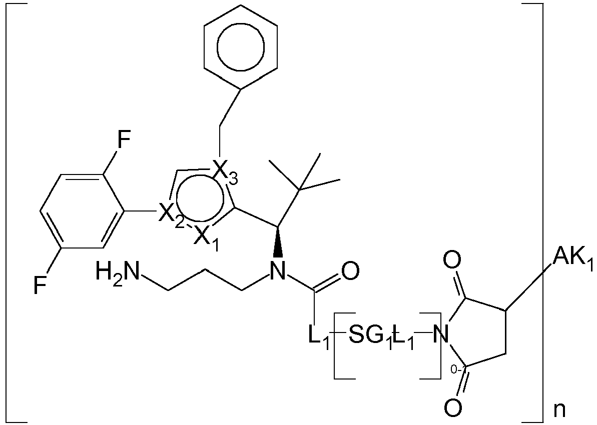 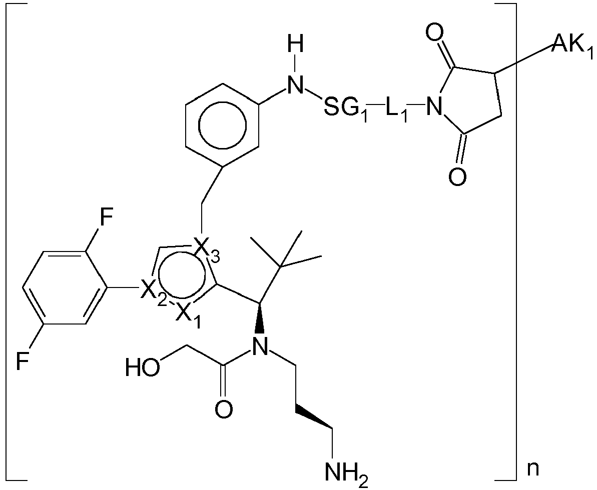 wobei
   X₁, X₂ und X₃ die gleiche Bedeutung aufweisen wie in Ziffer 14,
   AK₁ ein Binderpeptid oder-protein darstellt, das über ein Schwefelatom des Binders gebunden ist; n eine Zahl von 1 bis 20 darstellt; und L1 eine gegebenenfalls verzweigte Kohlenwasserstoffgruppe mit 1 bis 70 Kohlenstoffatomen ist, die eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, SO₂, -NH-, -CO-, -CONH-, -NHCO-, -Nme-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und S, -SO- oder -SO2- unterbrochen sein kann (vorzugsweise 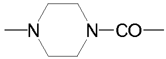 ), wobei die Seitenketten, sofern vorhanden, mit -NHCONH2, -COOH, -OH, -NH₂, NH-CNNH₂, Sulfonamid, Sulfon, Sulfoxid oder Sulfonsäure substituiert sein können, und SG1 ein 2-8 Oligopeptid, bevorzugt ein Dipeptid ist; L4 eine Einfachbindung oder eine Gruppe -(CO)_{y}-G4- darstellt, wobei y 0 oder 1 darstellt, und G4 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-,-SO-, SO₂, -NH-, -CO-, -NHCO-, -CONH-, -Nme-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und S, -SO-oder -SO2- unterbrochen sein kann (vorzugsweise 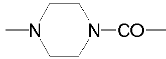 ), wobei die Seitenketten, sofern vorhanden, mit -NHCONH2, -COOH, -OH, -NH₂, NH-CNNH₂, Sulfonamid, Sulfon, Sulfoxid oder Sulfonsäure substituiert sein können.
27. Konjugat nach einem oder mehreren der Ziffern 1 bis 21, wobei der Linker -L- an eine Cystein-Seitenkette bzw. Cysteinrest gebunden ist und die folgende Formel aufweist: 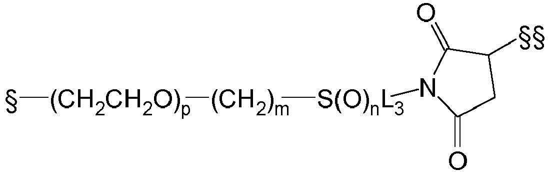 wobei
   § die Bindung an das Wirkstoffmolekül darstellt und
   §§ die Bindung an das Binderpeptid oder -protein darstellt,
   m 0, 1, 2, oder 3 ist;
   n 0, 1 oder 2 ist;
   p 0 bis 20 beträgt; und
   L3 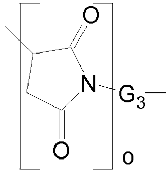 darstellt; wobei
      o 0 oder 1 ist;
      und
      G3 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -0-, -S-, - SO-, SO₂, -NH-, -CO-, -NHCO, -CONH- und einen 5 bis 10gliedriger, aromatischen oder nicht aromatischen Heterozyklus mit bis zu 4 Heteroatomen ausgewählt aus N, O und S, -, -Nme-,-NHNH-, -SO2NHNH-, -CONHNH- -SO-oder -SO2- unterbrochen sein kann (vorzugsweise 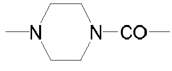 ), wobei die Seitenketten, sofern vorhanden, mit -NHCONH2, -COOH, -OH, Sulfon, Sulfoxid oder Sulfonsäure substituiert sein können.
28. Konjugat nach Ziffer 27, wobei der Linker -L- an eine Cystein-Seitenkette bzw. Cysteinrest gebunden ist und die folgende Formel aufweist: 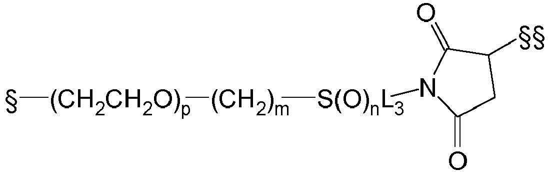 wobei
   § die Bindung an das Wirkstoffmolekül darstellt und
   §§ die Bindung an das Binderpeptid oder -protein darstellt,
   m 1 ist;
   p 0 ist;
   n 0 ist;
   und L3 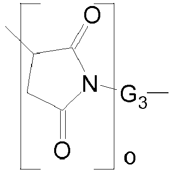 darstellt;
      wobei
      o 0 oder 1 ist; und
      G3-(CH2CH2O)ₛ(CH2)ₜ(CONH)ᵤ(CH2CH2O)ᵥ(CH2)_{w}- darstellt, wobei
      s, t, v und w jeweils unabhängig voneinander 0 bis 20 betragen, und u 0 oder 1 beträgt.
29. Konjugat nach Ziffer 27 oder 28, wobei R² oder R³ -L-#1 darstellt.
30. Konjugat nach Ziffer 29, wobei das Konjugat eine der folgenden Formeln aufweist: 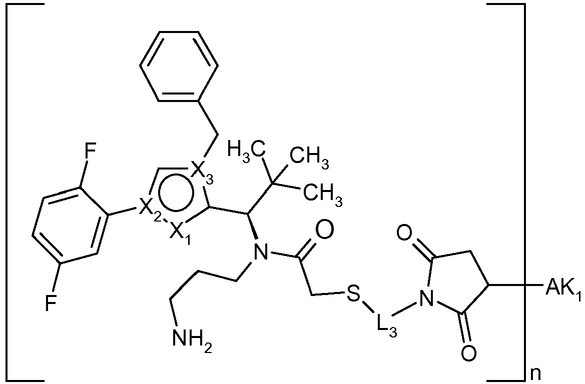 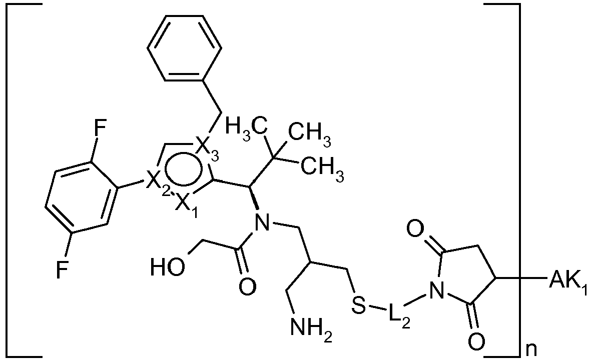 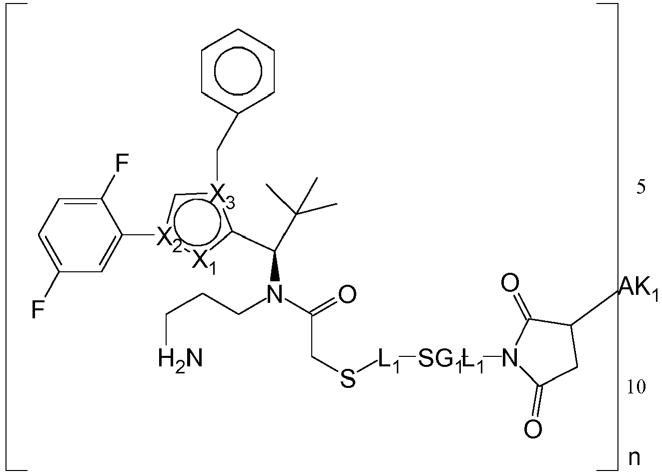 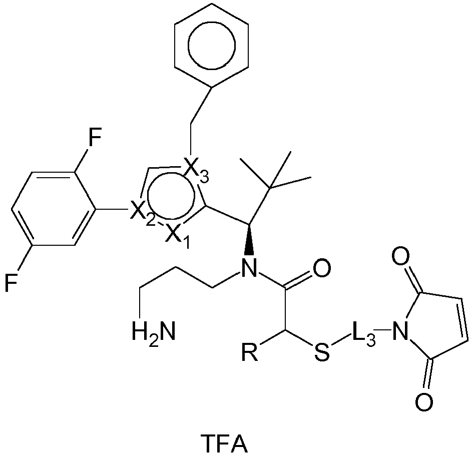 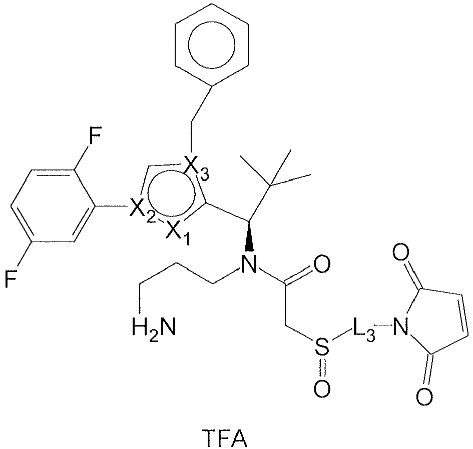 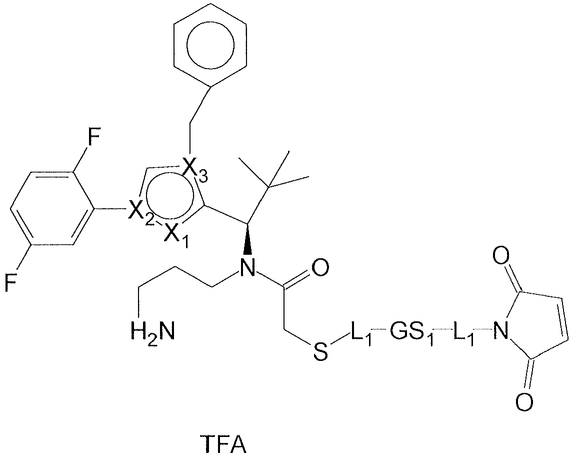 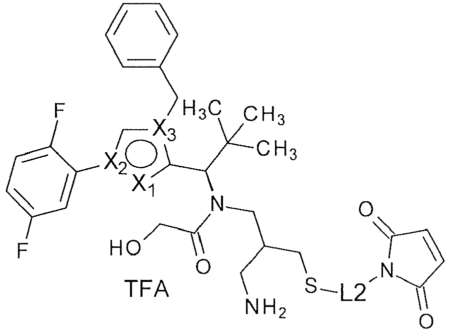 wobei
   X₁, X₂ und X₃ die gleiche Bedeutung aufweisen wie in Ziffer 14,
   AK₁ ein Binderpeptid oder-protein darstellt, das über ein Schwefelatom des Binders angebunden ist; n eine Zahl von 1 bis 20 darstellt; und L2 und L3 eine gegebenenfalls verzweigte geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, SO₂, -NH-, -CO-, -Nme-, -NHNH-, -SO₂NHNH-,-NHCO-, -CONH-, -CONHNH- und einen 5 bis 10gliedriger, aromatischen oder nicht aromatischen Heterozyklus mit bis zu 4 Heteroatomen ausgewählt aus N, O und S, -SO-oder -SO2- unterbrochen sein kann (vorzugsweise 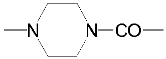 ), wobei die Seitenketten, sofern vorhanden, mit -NHCONH2, -COOH, -OH, -NH₂, NH-CNNH₂, Sulfonamid, Sulfon, Sulfoxid oder Sulfonsäure substituiert sein können.
31. Konjugat nach einem oder mehreren der Ziffern 1 bis 21, wobei der Linker -L- an eine Lysin-Seitenkette gebunden ist und die folgende Formel aufweist:

   -§-(SG)ₓ-L4-CO-§§,

   wobei
   § die Bindung an das Wirkstoffmolekül darstellt und
   §§ die Bindung an das Binderpeptid oder -protein darstellt,
   x 0 oder 1 darstellt,
   SG eine spaltbare Gruppe, vorzugsweise ein 2-8 Oligopeptid, besonders bevorzugt ein Dipeptid darstellt,
   und
   L4 eine Einfachbindung oder eine Gruppe -(CO)_{y}-G4- darstellt, wobei y 0 oder 1 darstellt, und G4 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-,-SO-, SO₂, -NH-, -CO-, -NHCO-, -CONH-, -Nme-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und S, oder -SO- unterbrochen sein kann (vorzugsweise 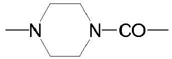 ), wobei die Seitenketten, sofern vorhanden, mit -NHCONH₂, -COOH, -OH, -NH₂, NH-CNNH₂, Sulfonamid, Sulfon, Sulfoxid oder Sulfonsäure substituiert sein können.
32. Konjugat eines Binderpeptids oder -proteins nach Ziffer 31, wobei das Konjugat eine der folgenden Formeln aufweist: 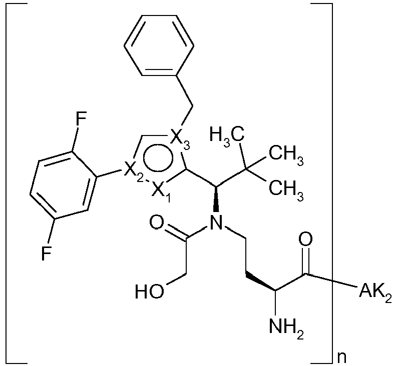 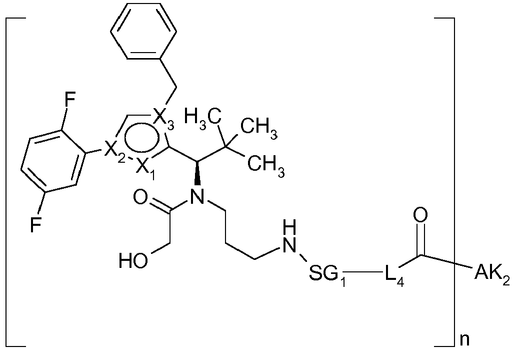 wobei
   X₁, X₂ und X₃ die gleiche Bedeutung aufweisen wie in Ziffer 14,
   AK₂ ein Binderpeptid oder-protein darstellt, das über ein Schwefelatom des Binders angebunden ist; n eine Zahl von 1 bis 20 darstellt; und L4 eine gegebenenfalls geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, SO₂, -NH-, -CO-, -Nme-,-NHNH-, -SO₂NHNH-,-NHCO-, -CONH-, -CONHNH- und einen 5 bis 10gliedriger, aromatischen oder nicht aromatischen Heterozyklus mit bis zu 4 Heteroatomen ausgewählt aus N, O und S, -SO- oder -SO2- unterbrochen sein kann (vorzugsweise 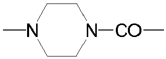 ), wobei die Seitenketten, sofern vorhanden, mit -NHCONH₂, -COOH, -OH, -NH₂, NH-CNNH₂, Sulfonamid, Sulfon, Sulfoxid oder Sulfonsäure substituiert sein können, und
   SG1 eine spaltbare Gruppe, vorzugsweise ein 2-8 Oligopeptid, besonders bevorzugt ein Dipeptid darstellt.
33. Konjugat nach einem oder mehreren der Ziffern 10 bis 32, wobei der anti-TWEAKR-Antikörper ein agonistischer Antikörper ist.
34. Konjugat einem oder mehreren der Ziffern 10 bis 33, der umfasst:
   eine variable schwere Kette umfassend:
      a. eine CDR1 der schweren Kette, die von einer Aminosäuresequenz umfassend die Formel PYPMX (SEQ ID NO: 171) kodiert wird, wobei X I oder M ist;
      b. eine CDR2 der schweren Kette, die von einer Aminosäuresequenz umfassend die Formel YISPSGGXTHYADSVKG (SEQ ID NO: 172) kodiert wird, wobei X S oder K ist; und
      c. eine CDR3 der schweren Kette, die von einer Aminosäuresequenz umfassend die Formel GGDTYFDYFDY (SEQ ID NO: 173) kodiert wird;
   und eine variable leichte Kette umfassend:
      d. eine CDR1 der leichten Kette, die von einer Aminosäuresequenz umfassend die Formel RASQSISXYLN (SEQ ID NO: 174) kodiert wird, wobei X G oder S ist;
      e. eine CDR2 der leichten Kette, die von einer Aminosäuresequenz umfassend die Formel XASSLQS (SEQ ID NO: 175) kodiert wird, wobei X Q, A oder N ist; und
      f. eine CDR3 der leichten Kette, die von einer Aminosäuresequenz umfassend die Formel QQSYXXPXIT (SEQ ID NO: 176) kodiert wird, wobei X an der Position 5 T oder S ist, X an der Position 6 T oder S ist und X an der Position 8 G oder F ist.
35. Konjugat einem oder mehreren der Ziffern 10 bis 34, umfassend:
   a. eine variable Sequenz der schweren Kette, wie dargestellt durch SEQ ID NO:10, sowie eine variable Sequenz der leichten Kette, wie dargestellt durch SEQ ID NO:9 oder
   b. eine variable Sequenz der schweren Kette, wie dargestellt durch SEQ ID NO:20, sowie eine variable Sequenz der leichten Kette, wie dargestellt durch SEQ ID NO:19 oder
   c. eine variable Sequenz der schweren Kette, wie dargestellt durch SEQ ID NO:30, sowie eine variable Sequenz der leichten Kette, wie dargestellt durch SEQ ID NO:29 oder
   d. eine variable Sequenz der schweren Kette, wie dargestellt durch SEQ ID NO:40, sowie eine variable Sequenz der leichten Kette, wie dargestellt durch SEQ ID NO:39 oder
   e. eine variable Sequenz der schweren Kette, wie dargestellt durch SEQ ID NO:50, sowie eine variable Sequenz der leichten Kette, wie dargestellt durch SEQ ID NO:49 oder
   f. eine variable Sequenz der schweren Kette, wie dargestellt durch SEQ ID NO:60, sowie eine variable Sequenz der leichten Kette, wie dargestellt durch SEQ ID NO:59 oder
   g. eine variable Sequenz der schweren Kette, wie dargestellt durch SEQ ID NO:70, sowie eine variable Sequenz der leichten Kette, wie dargestellt durch SEQ ID NO:69 oder
   h. eine variable Sequenz der schweren Kette, wie dargestellt durch SEQ ID NO:80, sowie eine variable Sequenz der leichten Kette, wie dargestellt durch SEQ ID NO:79 oder
   i. eine variable Sequenz der schweren Kette, wie dargestellt durch SEQ ID NO:90, sowie eine variable Sequenz der leichten Kette, wie dargestellt durch SEQ ID NO:89 oder
   j. eine variable Sequenz der schweren Kette, wie dargestellt durch SEQ ID NO:100, sowie eine variable Sequenz der leichten Kette, wie dargestellt durch SEQ ID NO:99 oder
   k. eine variable Sequenz der schweren Kette, wie dargestellt durch SEQ ID NO:110, sowie eine variable Sequenz der leichten Kette, wie dargestellt durch SEQ ID NO:109 oder
   l. eine variable Sequenz der schweren Kette, wie dargestellt durch SEQ ID NO:120, sowie eine variable Sequenz der leichten Kette, wie dargestellt durch SEQ ID NO:119.
36. Konjugat einem oder mehreren der Ziffern 10 bis 35, wobei der Antikörper ein IgG Antikörper ist.
37. Konjugat einem oder mehreren der Ziffern 10 bis 36, umfassend:
   a. eine Sequenz der schweren Kette, wie dargestellt durch SEQ ID NO:2, sowie eine Sequenz der leichten Kette, wie dargestellt durch SEQ ID NO:1 oder
   b. eine Sequenz der schweren Kette, wie dargestellt durch SEQ ID NO:12, sowie eine Sequenz der leichten Kette, wie dargestellt durch SEQ ID NO:11 oder
   c. eine Sequenz der schweren Kette, wie dargestellt durch SEQ ID NO:22, sowie eine Sequenz der leichten Kette, wie dargestellt durch SEQ ID NO:21 oder
   d. eine Sequenz der schweren Kette, wie dargestellt durch SEQ ID NO:32, sowie eine Sequenz der leichten Kette, wie dargestellt durch SEQ ID NO:31 oder
   e. eine Sequenz der schweren Kette, wie dargestellt durch SEQ ID NO:42, sowie eine Sequenz der leichten Kette, wie dargestellt durch SEQ ID NO:41 oder
   f. eine Sequenz der schweren Kette, wie dargestellt durch SEQ ID NO:52, sowie eine Sequenz der leichten Kette, wie dargestellt durch SEQ ID NO:51 oder
   g. eine Sequenz der schweren Kette, wie dargestellt durch SEQ ID NO:62, sowie eine Sequenz der leichten Kette, wie dargestellt durch SEQ ID NO:61 oder
   h. eine Sequenz der schweren Kette, wie dargestellt durch SEQ ID NO:72, sowie eine Sequenz der leichten Kette, wie dargestellt durch SEQ ID NO:71 oder
   i. eine Sequenz der schweren Kette, wie dargestellt durch SEQ ID NO:82, sowie eine Sequenz der leichten Kette, wie dargestellt durch SEQ ID NO:81 oder
   j. eine Sequenz der schweren Kette, wie dargestellt durch SEQ ID NO:92, sowie eine Sequenz der leichten Kette, wie dargestellt durch SEQ ID NO:91 oder
   k. eine Sequenz der schweren Kette, wie dargestellt durch SEQ ID NO:102, sowie eine Sequenz der leichten Kette, wie dargestellt durch SEQ ID NO:101 oder
   l. eine Sequenz der schweren Kette, wie dargestellt durch SEQ ID NO:112, sowie eine Sequenz der leichten Kette, wie dargestellt durch SEQ ID NO:111.
38. SpindelKonjugat nach einem oder mehreren der vorhergehenden Ziffern, wobei das Konjugat 1 bis 10, vorzugsweise 2 bis 8, Wirkstoffmoleküle pro Binderpeptid oder -protein aufweist.
39. Verfahren zur Herstellung des Konjugats nach Ziffer 26 oder 30, wobei eine Verbindung einer der folgenden Formeln, vorzugsweise in Form ihres Trifluoressigsäuresalzes, an einen Cysteinrest eines zuvor ggf. partiell reduzierten Binderpeptids oder -proteins gebunden wird, wobei die Verbindung vorzugsweise in 2 bis 12 fachem molaren Überschuss gegenüber dem Binderpeptid- oder protein verwendet wird: 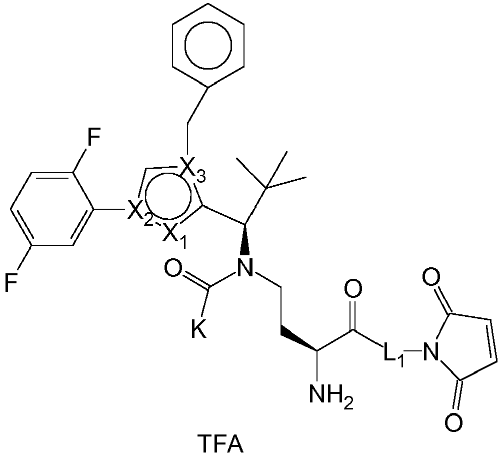 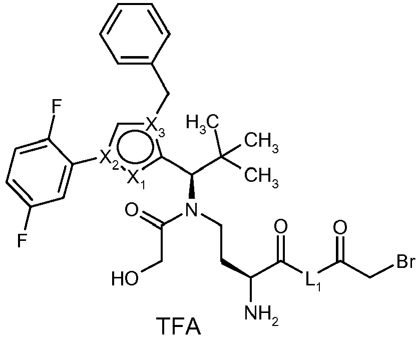 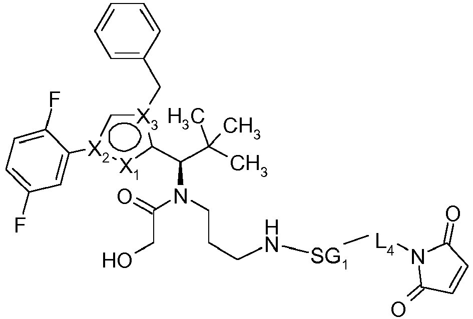 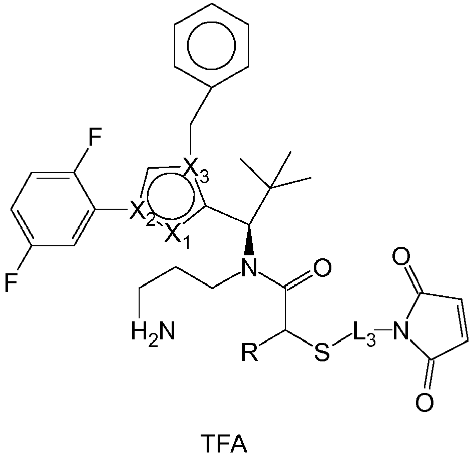 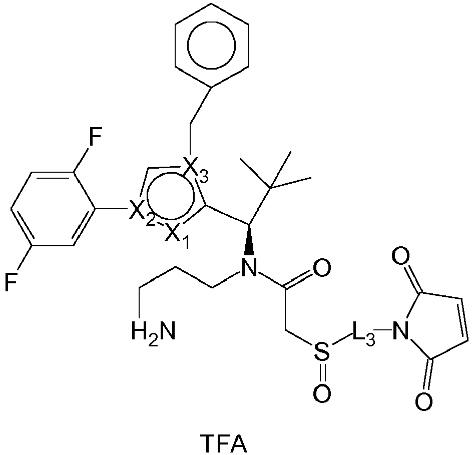 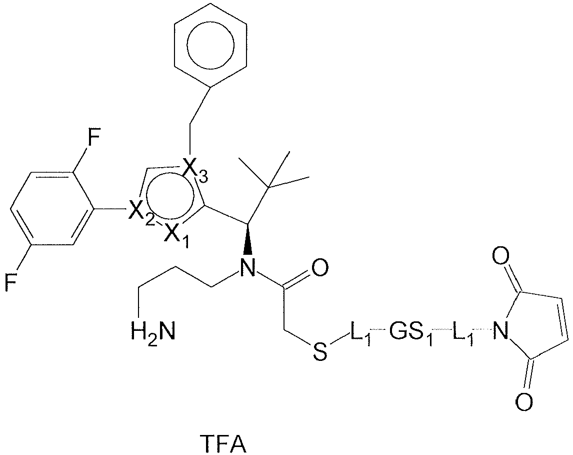 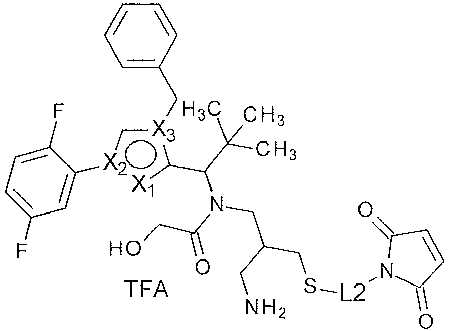 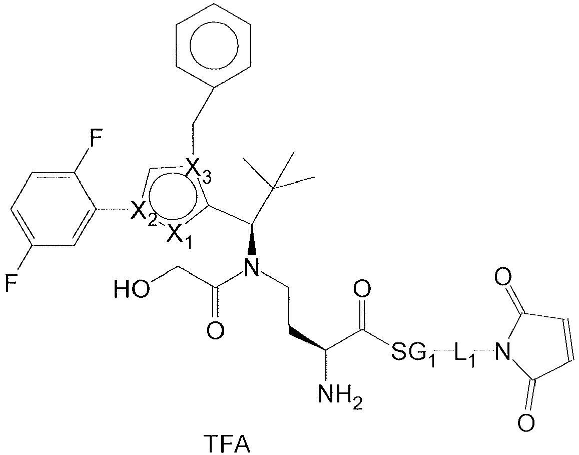 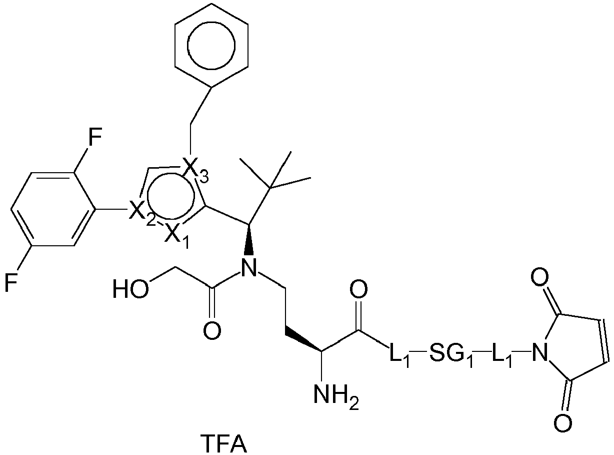 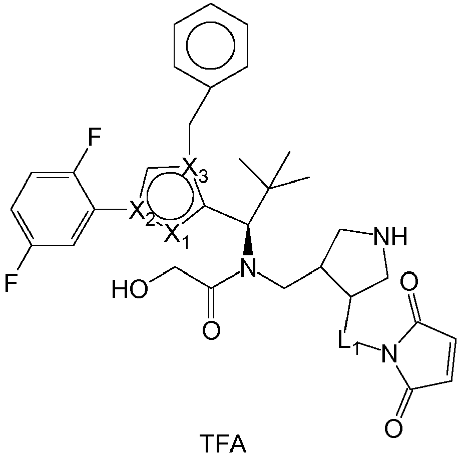 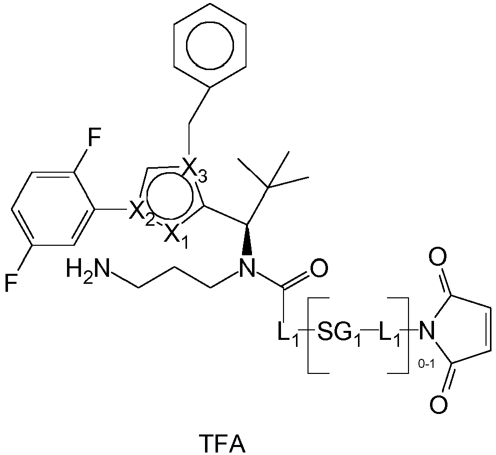 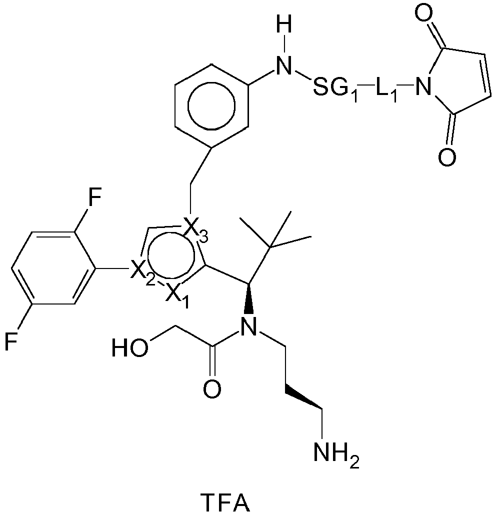
   wobei R -H oder -COOH darstellt,
   wobei K lineares oder verzweigtes gegebenenfalls substituiertes mit C₁-C₆ Alkoxy oder -OH C₁-C₆ Alkyl sarstellt, und
   wobei X₁, X₂, X₃, SG1, L1, L2, L3 und L4 die gleiche Bedeutung haben wie unter Ziffer 26 bzw. 30.
40. Verfahren zur Herstellung des Konjugats nach Ziffer 32, wobei eine Verbindung einer der folgenden Formeln, vorzugsweise in Form ihres Trifluoressigsäuresalzes, an einen Lysinrest eines Binderpeptids oder -proteins gebunden wird, wobei die Verbindung vorzugsweise in 2 bis 12 fachem molaren Überschuss gegenüber dem Binderpeptid- oder protein verwendet wird: 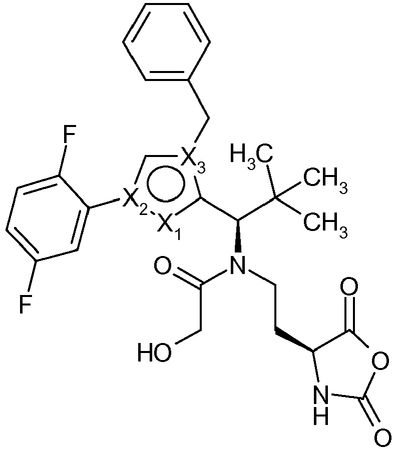 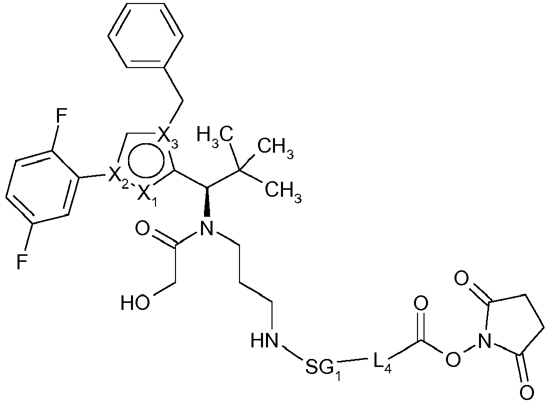 wobei X₁, X₂, X₃, SG1, und L4 die gleiche Bedeutung haben wie unter Ziffer 32.
41. Verbindung einer der folgenden Formeln: 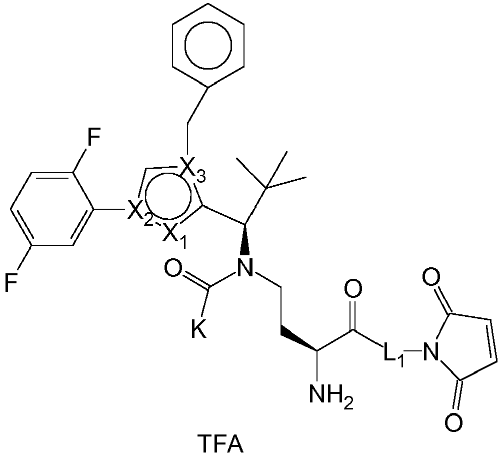 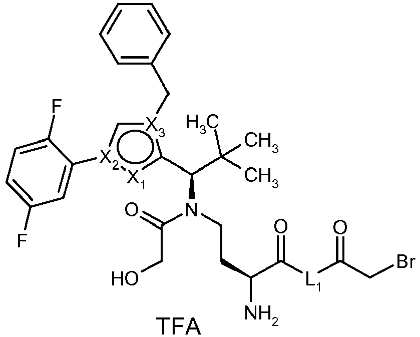 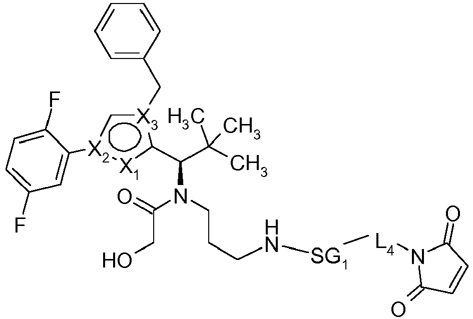 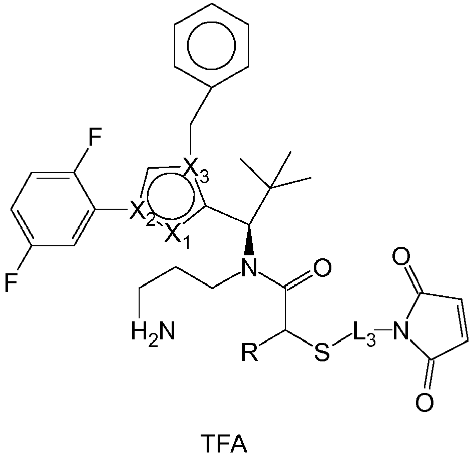 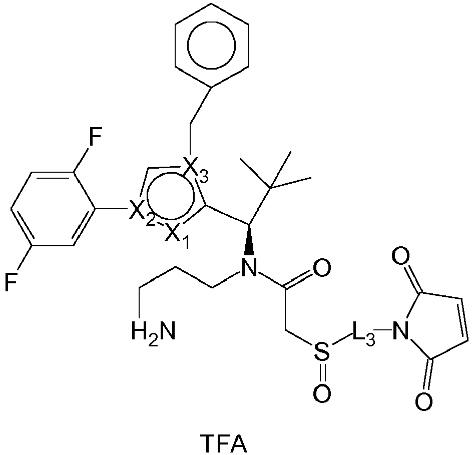 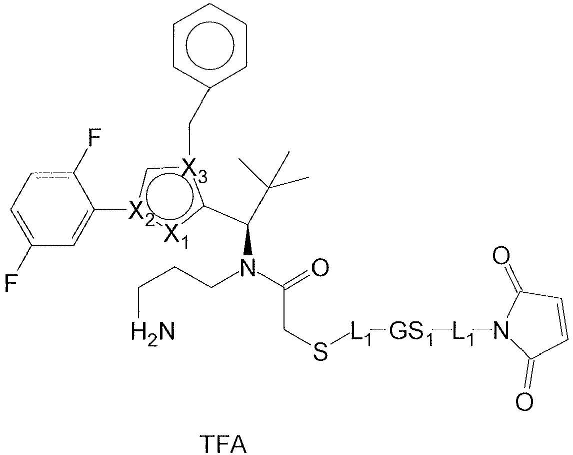 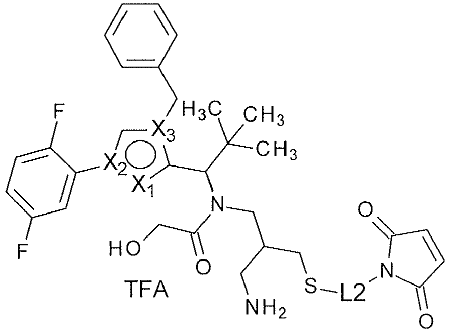 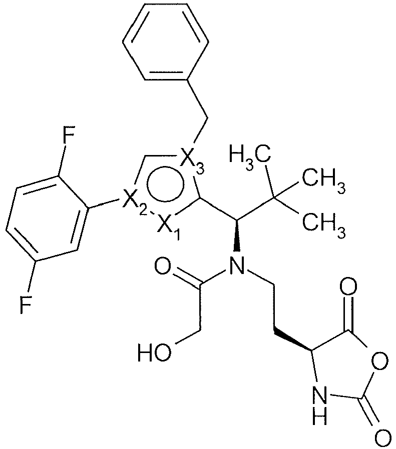 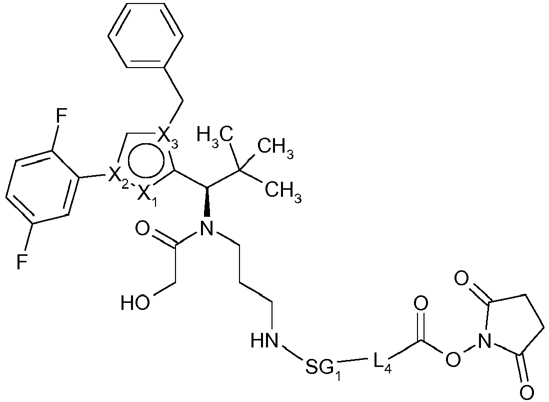 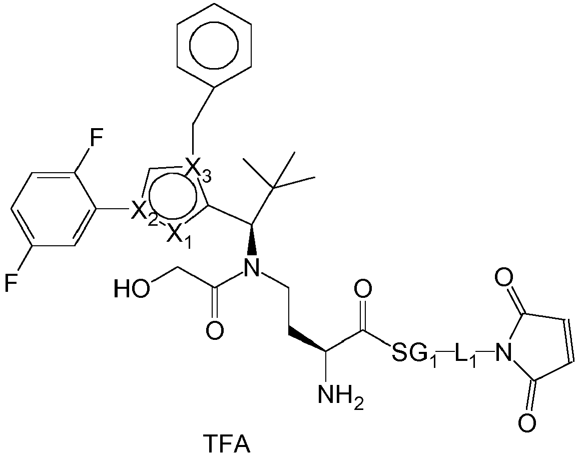 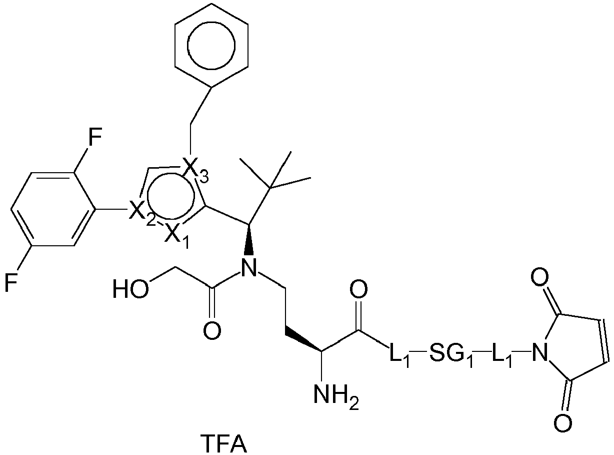 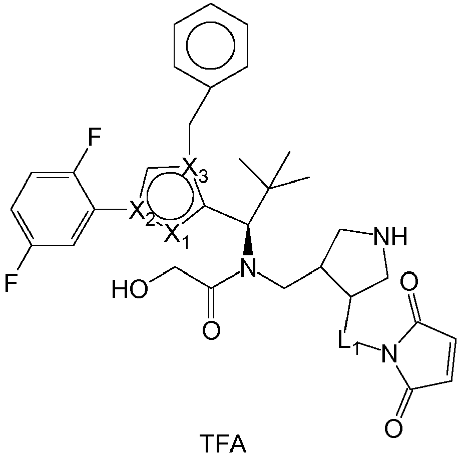 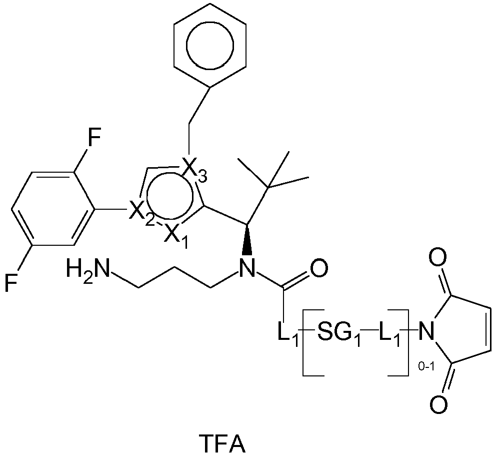 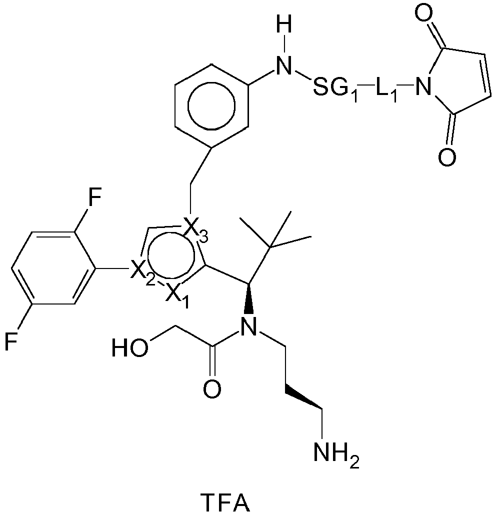
   wobei R -H oder -COOH darstellt,
   wobei K lineares oder verzweigtes gegebenenfalls substituiertes mit C₁-C₆ Alkoxy oder -OH C₁-C₆ Alkyl sarstellt, und
   wobei X₁, X₂, X₃, SG1, L1, L2, L3 und L4 die gleiche Bedeutung haben wie in Ziffer 26, 30 bzw. 32.
42. Verbindungen der allgemeinen Formel (III): 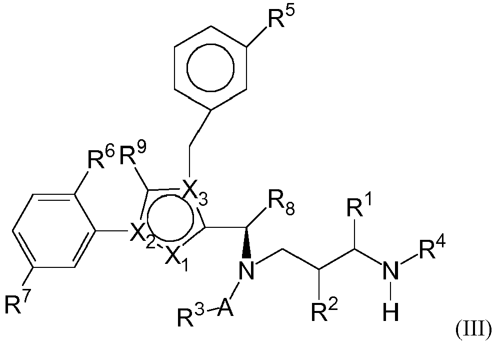 wobei
   X₁ N, X₂ N und X₃ C darstellt, oder X₁ CH, X₂ C und X₃ N darstellt oder X₁ NH, X₂ C und X₃ C darstellt, oder X₁ CH, X₂ N und X₃ C darstellt;
   R¹ -L-BINDER, H, oder -(CH₂)₀₋₃Z darstellt, wobei Z -H, -NHY³, -OY³, -SY³, Halogen, -CO-NY¹Y², oder -CO-OY³ darstellt,
   wobei Y¹ und Y² unabhängig voneinander H, NH2, -(CH₂CH₂O)₀₋₃-(CH₂)₀₋₃Z', oder-CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt, wobei W H oder OH darstellt;
   wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
   R² und R⁴ unabhängig voneinander -L-BINDER, H, -CO-CHY⁴-NHY⁵ oder -(CH₂)₀₋₃Z darstellen, oder R² und R⁴ gemeinsam (unter Bildung eines Pyrrolidinringes) -CH₂-CHR¹⁰- oder -CHR¹⁰-CH₂- darstellen, wobei R¹⁰ L-#1, H, NH₂, SO₃H, COOH, SH, oder OH darstellt; wobei Z -H, Halogen, -OY³, -SY³, NHY³, -CO-NY¹Y², oder -CO-OY³ darstellt,
   wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt;
   wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
   A CO, SO, SO₂, SO₂NH oder CNNH darstellt;
   R³ -L-BINDER oder eine gegebenenfalls substituierte Alkyl-, Aryl-, Heteroaryl- Heteroalkyl-, Heterocycloalkyl- Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3-NH-CO-NH-Alkyl Gruppen, 1-3 -S(O)ₙ-Alkyl Gruppen, 1-3 -SO₂-NH-Alkyl Gruppen, 1-3-NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
   substituiert sein kann (wobei "Alkyl" bevorzugt C₁₋₁₀-Alkyl bedeutet);
   R⁵ -L-BINDER, H, F, NH₂, NO₂, Halogen, SH oder -(CH₂)₀₋₃Z darstellt, wobei Z -H, Halogen, -OY³, -SY³, -NHY³, -CO-NY¹Y², oder -CO-OY³ darstellt,
   wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
   darstellt,
   wobei L für einen Linker steht und BINDER für Binder bzw. Derivat hiervon steht, wobei der Binder ggf. an mehrere Wirkstoffmoleküle gebunden sein kann,
   R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy oder Halogen darstellen,
   R⁸ (ggf. fluoriertes) C₁₋₁₀-Alkyl, (ggf. fluoriertes) C₄₋₁₀-Cycloalkyl oder optional substituiertes Oxetan darstellt; und
   R⁹ H, F, CH₃, CF₃, CH₂F oder CHF₂ darstellt;
   sowie ihre Salze, Solvate und Salze der Solvate.
43. Pharmazeutische Zusammensetzung umfassend ein Konjugat nach einem oder mehreren der Ziffern 1 bis38 und 41-42 in Kombination mit einem inerten, nicht-toxischen, pharmazeutisch geeigneten Hilfsstoff.
44. Konjugat nach einem oder mehreren der Ziffern 1 bis 38 und 41-42 zur Verwendung in einem Verfahren Behandlung und/oder Prophylaxe von Krankheiten.
45. Konjugat nach einem oder mehreren der Ziffern 1 bis 38 und 41-42 zur Verwendung in einem Verfahren zur Behandlung von hyperproliferativen und/oder angiogenen Erkrankungen.
46. Konjugat nach einem oder mehreren der Ziffern 1 bis 38 und 41-42, wobei R^{3a} bzw. R³ -L-BINDER oder eine substituierte Alkyl-, Aryl- oder Heteroaryl-Gruppe darstellt, bevorzugt -L-#1 oder eine C₁₋₁₀-Alkyl-, C₆₋₁₀-Aryl- oder C₆₋₁₀-Aralkyl-Gruppe oder C₅₋₁₀-Heteroalkyl-, die mit -OH, O-Alkyl, SH, S-Alkyl, O-CO-Alkyl, O-CO-NH-Alkyl, NH-CO-Alkyl, NH-CO-NH-Alkyl, S(O)ₙ-Alkyl, SO₂-NH-Alkyl, NH-Alkyl, N(Alkyl)₂, NH₂ oder-(CH₂)₀₋₃Z, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H oder -(CH₂)₀₋₃Z' darstellt, wobei Z' H, SO₃H, NH₂ oder COOH darstellt, substituiert sein kann (wobei "Alkyl" bevorzugt C₁₋₁₀-Alkyl bedeutet).
47. Verfahren zur Behandlung und/oder Prophylaxe von hyperproliferativen und/oder angiogenen Erkrankungen bei Menschen und Tieren unter Verwendung einer wirksamen Menge mindestens eines Konjugat nach einem oder mehreren der Ziffern 1 bis 38 und 41-42.

## Patentansprüche

1. Konjugat eines Binders oder Derivats hiervon mit einem oder mehreren Wirkstoffmolekülen,
wobei das Wirkstoffmolekül ein Kinesin Spindel Protein-Inhibitor ist, das mit dem Binder über einen Linker L verbunden ist, wobei der Kinesin Spindel Protein-Inhibitor die folgen Formel (IIa) aufweist: 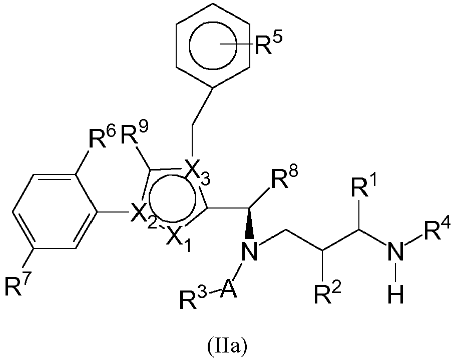 wobei
X₁ N, X₂ N und X₃ C darstellt; oder
X₁ CH oder CF, X₂ C und X₃ N darstellt; oder
X₁ NH, X₂ C und X₃ C darstellt; oder
X₁ CH, X₂ N und X₃ C darstellt;
(wobei X₁ CH, X₂ C und X₃ N bevorzugt ist);
R¹ H, -L-#1, -MOD oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander H, NH₂, -(CH₂CH₂O)₀₋₃-(CH₂)₀₋₃Z'(z.B. -(CH₂)₀₋₃Z'),
oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt,
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² -L-#1, H, -MOD, -CO-CHY⁴-NHY⁵ oder -(CH₂)₀₋₃Z darstellt, oder R² und R⁴ gemeinsam (unter Bildung eines Pyrrolidinringes) -CH₂-CHR¹⁰- oder -CHR¹⁰-CH₂- darstellen, wobei R¹⁰ L-#1, H, NH₂, SO₃H, COOH, SH, oder OH darstellt;
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
R⁴ -L-#1, H, -CO-CHY⁴-NHY⁵ oder -(CH₂)₀₋₃Z darstellt,
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
oder R² und R⁴ gemeinsam (unter Bildung eines Pyrrolidinringes) -CH₂-CHR¹⁰- oder -CHR¹⁰-CH₂- darstellen, wobei R¹⁰ L-#1, H, NH₂, SO₃H, COOH, SH, oder OH darstellt;
A CO, SO, SO₂, SO₂NH oder CNNH darstellt;
R³ -L-#1, -MOD, oder eine gegebenenfalls substituierte Alkyl-, Cycloalkyl-, Aryl-, Heteroaryl-Heteroalkyl-, Heterocycloalkyl- Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3-SO₂-NH-Alkyl Gruppen, 1-3 -NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder-CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet);
R⁵ -L-#1, H, NH₂, NO₂, Halogen (insbesondere F, Cl, Br), -CN, CF₃, -OCF₃, -CH₂F, -CH₂F, SH oder -(CH₂)₀₋₃Z darstellt, wobei Z -H, -OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
darstellt,
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy, NO₂, NH₂, COOH oder Halogen (insbesondere F, Cl, Br) darstellen,
R⁸ (ggf. fluoriertes) C₁₋₁₀-Alkyl, (ggf. fluoriertes) C₂₋₁₀-Alkenyl, (ggf. fluoriertes) C₂₋₁₀-Alkinyl, (ggf. fluoriertes) C₄₋₁₀-Cycloalkyl oder-(CH₂)₀₋₂-(HZ²) darstellt, wobei HZ² ein 4 bis 7-gliedriger Heterocyclus mit bis zu zwei Heteroatomen ausgewählt aus N, O und S darstellt, wobei jede dieser Gruppen mit -OH, CO₂H or NH₂ oder -L-#1 substituiert sein kann;
wobei einer oder keiner der Substituenten R¹, R², R³, R⁴ R⁵, R⁸ und R¹⁰ -L-#1 darstellt bzw. (im Falle von R⁸) aufweist,
L für den Linker steht und #1 für die Bindung zum Binder bzw. Derivat hiervon steht,
R⁹ H, F, CH₃, CF₃, CH₂F oder CHF₂ darstellt;
wobei -MOD -(NR¹⁰)ₙ-(G1)ₒ-G2-H darstellt, wobei
R¹⁰ H oder C₁-C₃-Alkyl darstellt;
G1 -NHCO- , -CONH- oder 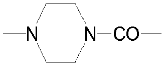 darstellt (wobei wenn G1 -NHCO- oder 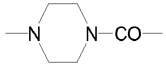 darstellt, R¹⁰ nicht NH₂ ist);
n 0 oder 1 ist;
o 0 oder 1 ist; und G2 eine geradkettige und/oder verzweigte Kohlenwasserstoffgruppe mit 1 bis 10 Kohlenstoffatomen ist, die einfach oder mehrfach durch eine oder mehrere der Gruppen -O-, -S-, -SO-, SO₂, -NRY-, -NRYCO-, CONRY-, -NRYNRY-, -SO2NR^{y}NR^{y}-, -CONR^{y}NR^{y}-, (wobei R^{y} H, Phenyl, C₁-C₁₀-Alkyl, C₂-C₁₀-Alkenyl, oder C₂-C₁₀-Alkinyl darstellt, die jeweils mit NHCONH₂, -COOH, -OH, -NH₂, NH-CNNH₂, Sulfonamid, Sulfon, Sulfoxid, oder Sulfonsäure substituiert sein können), -CO-, -CR^{x}=N-O- (wobei Rx H, C₁-C₃-Alkyl oder Phenyl darstellt) unterbrochen sein kann, wobei die Kohlenwasserstoffkette einschließlich der Seitenketten, sofern vorhanden, mit -NHCONH₂, -COOH, -OH, -NH₂, NH-CNNH₂, Sulfonamid, Sulfon, Sulfoxid, oder Sulfonsäure substituiert sein kann, wobei die Gruppe -MOD vorzugsweise zumindest eine Gruppe -COOH aufweist;
sowie ihre Salze, Solvate und Salze der Solvate.

2. Konjugat nach Anspruch 1, wobei X₁ CH, X₂ C und X₃ N ist.

3. Konjugat nach Anspruch 1 oder 2, wobei der Substituent R¹ -L-#1 darstellt.

4. Konjugat nach einem oder mehreren der vorhergehenden Ansprüche,wobei der Wirkstoffmolekül-Linker durch die allgemeine Formel (II) dargestellt wird: 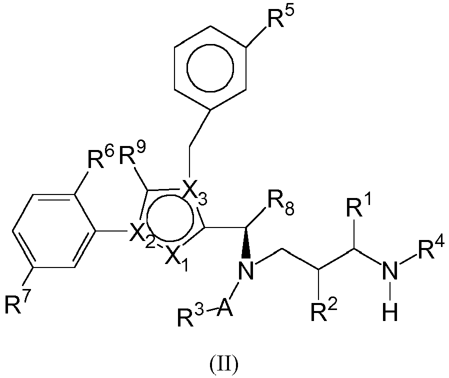 wobei
X₁ N, X₂ N und X₃ C darstellt; oder
X₁ CH, X₂ C und X₃ N darstellt; oder
X₁ NH, X₂ C und X₃ C darstellt; oder X₁ CH, X₂ N und X₃ C darstellt;
R¹ H, -L-#1 oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander H, NH₂, -(CH₂CH₂O)₀₋₃-(CH₂)₀₋₃Z', oder oder -CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt, wobei W H oder OH darstellt; wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
R² und R⁴ unabhängig voneinander -L-#1, H, -CO-CHY⁴-NHY⁵ oder -(CH₂)₀₋₃Z darstellen, oder R² und R⁴ gemeinsam (unter Bildung eines Pyrrolidinringes) -CH₂-CHR¹⁰- oder -CHR¹⁰-CH₂-darstellen, wobei R¹⁰ L-#1, H, NH₂, SO₃H, COOH, SH, oder OH darstellt;
wobei Z -H, OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
A CO, SO, SO₂, SO₂NH oder CNNH darstellt;
R³ -L-#1 oder eine gegebenenfalls substituierte Alkyl-, Aryl-, Heteroaryl- Heteroalkyl-, Heterocycloalkyl- Gruppe darstellt,
bevorzugt -L-#1 oder eine C₁₋₁₀-Alkyl-, C₆₋₁₀-Aryl-, C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe,
die mit 1-3 -OH Gruppen, 1-3 Halogenatomen, 1-3 halogenierte Alkyl-Gruppen (die jeweils 1-3 Halogenatome aufweisen können), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3 -NH-CO-NH-Alkyl Gruppen, 1-3 -S(O)ₙ-Alkyl Gruppen, 1-3 -SO₂-NH-Alkyl Gruppen, 1-3 -NH-Alkyl Gruppen, 1-3 -N(Alkyl)₂ Gruppen, 1-3 -NH₂ Gruppen oder 1-3-(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z%-(CH₂)₀₋₃-CH(NH₂)Z', oder -(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet);
R⁵ -L-#1, H, F, NH₂, NO₂, Halogen, SH oder -(CH₂)₀₋₃Z darstellt, wobei Z -H, OY³, -SY³, Halogen, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
darstellt,
wobei einer der Substituenten R¹, R², R³, R⁴ und R⁵ -L-#1 darstellt,
L für den Linker steht und #1 für die Bindung zum Binder bzw. Derivat hiervon steht,
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy oder Halogen darstellen,
R⁸ (ggf. fluoriertes) C₁₋₁₀-Alkyl, (ggf. fluoriertes) C₄₋₁₀-Cycloalkyl oder optional substituiertes Oxetan darstellt; und
R⁹ H, F, CH₃, CF₃, CH₂F oder CHF₂ darstellt;
sowie ihre Salze, Solvate und Salze der Solvate.

5. Konjugat nach Anspruch 4,
wobei R¹ -L-#1, H, -(CH₂)₀₋₃Z darstellt, wobei Z -H, -CO-NY¹Y², NHY³, OY³, -SY³, oder-CO-OY³ darstellt,
mit Y¹ und Y² unabhängig voneinander H, NH₂, -(CH₂CH₂O)₀₋₃-(CH₂)₀₋₃Z', oder oder-CH(CH₂W)Z' , und Y³ H oder -(CH₂)₀₋₃Z' darstellt, wobei Z' H, NH₂, COOH, -NH-CO-CH₂-CH₂-CH(NH₂)COOH oder -(CO-NH-CHY⁴)₁₋₃COOH darstellt; wobei W H oder OH darstellt, wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt;
R² und R⁴ unabhängig voneinander -L-#1, -CO-CHY⁴-NHY⁵ oder H oder R² und R⁴ gemeinsam (unter Bildung eines Pyrrolidinringes) -CH₂-CHR¹⁰- darstellen, wobei R¹⁰ H, -L-#1, NH₂, COOH, SH, OH oder SO₃H darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
A CO darstellt,
R³ -(CH₂)OH oder -L-#1 darstellt, und
R⁵ -L-#1 oder H darstellt,
wobei einer der Substituenten R¹, R², R³, R⁴ und R⁵ -L-#1 darstellt.

6. Konjugat nach Anspruch 4 oder 5, wobei R⁶ und R⁷ unabhängig voneinander H, C₁₋₃-Alkyl oder Halogen darstellen.

7. Konjugat nach einem oder mehreren der Ansprüche 4 bis 6, wobei R⁸ C₁₋₄-Alkyl (vorzugsweise tert-butyl) darstellt.

8. Konjugat nach einem oder mehreren der Ansprüche 4 bis 7, wobei R⁹ H darstellt.

9. Konjugat nach einem oder mehreren der Ansprüche 4 bis 8, wobei R⁶ und R⁷ F darstellen.

10. Konjugat nach einem oder mehreren der vorhergehenden Ansprüche, wobei der Binder oder Derivat hiervon ein Binderpeptid oder -protein oder ein Derivat eines Binderpeptids oder-proteins ist.

11. Konjugat nach Anspruch 10, wobei jedes Wirkstoffmolekül an verschiedene Aminosäuren des Binderpeptids oder -proteins bzw. Derivats hiervon über den Linker gebunden ist.

12. Konjugat nach einem oder mehreren der vorhergehenden Ansprüche, wobei das Konjugat im Durchschnitt 1,2 bis 20 Wirkstoffmoleküle pro Binder aufweist.

13. Konjugat nach einem oder mehreren der Ansprüche 10 bis 12, wobei das Binderpeptid oder - protein einen Antikörper bzw. das Derivat des Binderpeptids oder -proteins 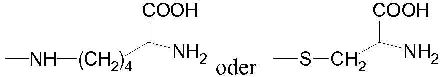 darstellt.

14. Konjugat nach einem oder mehreren der vorhergehenden Ansprüchen, wobei der Binder an ein Krebs-Zielmolekül bindet.

15. Konjugat nach Anspruch 14, wobei der Binder an ein extrazelluläres Zielmolekül bindet.

16. Konjugat nach Anspruch 15, wobei der Binder nach Bindung an das extrazelluläre Zielmolekül von der das Zielmolekül exprimierenden Zelle internalisiert und intrazellulär (vorzugsweise lysosomal) prozessiert wird.

17. Konjugat nach einem oder mehreren der Ansprüche 10 bis 16, wobei das Binderpeptid oder - protein ein humaner, humanisierter oder chimärer monoklonaler Antikörper oder ein Antigen-bindendes Fragment hiervon ist.

18. Konjugat nach Anspruch 17, wobei das Binderpeptid oder -protein ein anti-HER2-Antikörper, ein anti-EGFR-Antikörper, ein anti-TWEAKR-Antikörper, oder ein Antigen-bindendes Fragment hiervon ist.

19. Konjugat nach Anspruch 18, wobei der anti-TWEAKR-Antikörper spezifisch an die Aminosäure D in Position 47 (D47) von TWEAKR (SEQ ID NO:169) bindet, bevorzugt der anti-TWEAKR-Antikörper TPP-2090.

20. Konjugat nach Anspruch 18, wobei das Binderpeptid oder -protein ein anti-EGFR-Antikörper ist und R3 -L-#1 darstellt.

21. Konjugat nach einem oder mehreren der vorhergehenden Ansprüche, wobei der Linker -L- eine der folgenden Grundstrukturen (i) bis (iv) aufweist:
1. -(CO)ₘ-SG1-L1-L2-
(ii) -(CO)ₘ-L1-SG-L1-L2-
(iii) -(CO)ₘ-L1-L2-
(iv) -(CO)ₘ-L1-SG-L2
wobei m 0 oder 1 ist, SG und SG1 eine *in vivo* spaltbare Gruppe ist, L1 unabhängig voneinander für *in vivo* nicht spaltbare organische Gruppen stehen, und L2 für eine Kopplungsgruppe an den Binder steht.

22. Konjugat nach Anspruch 21, wobei die *in vivo* spaltbare Gruppe SG eine 2-8 Oligopeptidgruppe, bevorzugt eine Dipeptidgruppe oder ein Disulfid, ein Hydrazon, ein Acetal oder ein Aminal ist und SG1 eine 2-8 Oligopeptidgruppe, bevorzugt eine Dipeptidgruppe ist.

23. Konjugat nach einem oder mehreren der vorhergehenden Ansprüche,
wobei der Linker an eine Cystein-Seitenkette bzw. Cysteinrest gebunden ist und die folgende Formel aufweist:
§-(CO)m-L1-L2-§§
wobei
m 0 oder 1 ist;
§ die Bindung an das Wirkstoffmolekül darstellt und
§§ die Bindung an das Binderpeptid oder -protein darstellt, und
-L2- für 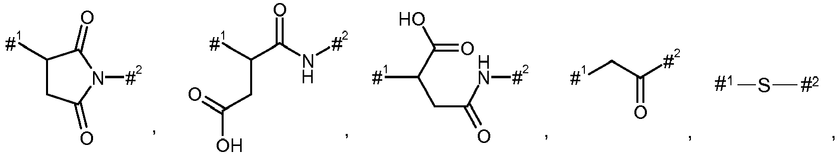 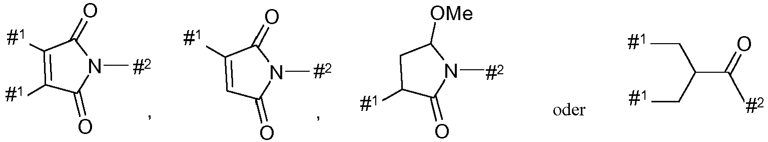
steht, wobei
#¹ die Verknüpfungsstelle mit dem Schwefelatom des Binders kennzeichnet,
#² die Verknüpfungsstelle mit der Gruppe L¹ kennzeichnet,
L1-(NR¹⁰)ₙ-(G1)ₒ-G2- darstellt,
wobei R¹⁰ H, NH2 oder C1-C3-Alkyl darstellt;
G1-NHCO- oder 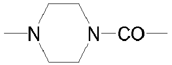 darstellt;
n 0 oder 1 ist;
o 0 oder 1 ist; und
G2 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, SO₂, -NH-, -CO-, -Nme-, -NHNH-, -SO₂NHNH-,-NHCO-, -CONH-, -CONHNH- und einen 5 bis 10gliedriger, aromatischen oder nicht aromatischen Heterozyklus mit bis zu 4 Heteroatomen ausgewählt aus N, O und S, -SO-oder -SO2- unterbrochen sein kann (vorzugsweise 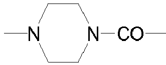 ), wobei die Seitenketten, sofern vorhanden, mit -NHCONH2, -COOH, -OH, -NH₂, NH-CNNH₂, Sulfonamid, Sulfon, Sulfoxid oder Sulfonsäure substituiert sein können,
oder eine der folgenden Gruppen darstellt: 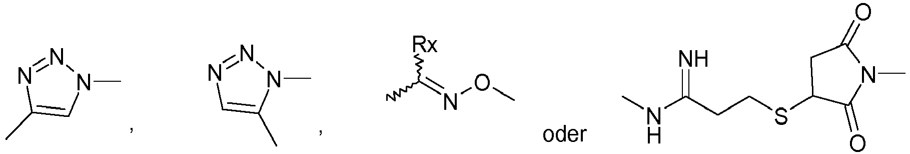 wobei Rx H, C₁-C₃-Alkyl oder Phenyl darstellt.

24. Konjugat nach Anspruch 23, wobei L2 dargestellt wird durch eine oder beide der folgenden Formeln: 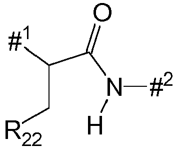 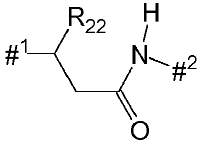 wobei #¹ die Verknüpfungsstelle mit dem Schwefelatom des Binders kennzeichnet, #² die Verknüpfungsstelle mit der Gruppe L¹ kennzeichnet, R²² COOH darstellt, und die Bindungen an das Schwefelatom des Binders zu über 80 %, (bezogen auf die Gesamtanzahl von Bindungen des Linkers an den Binder) in einer dieser beiden Strukturen vorliegen.

25. Konjugat nach Anspruch 23 oder 24, wobei die Kohlenwasserstoffkette durch eine die folgenden Gruppen unterbrochen ist: 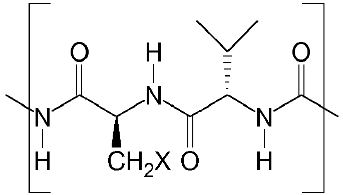 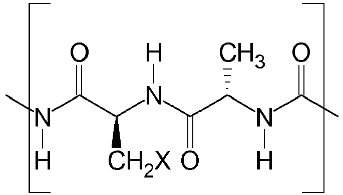 wobei X H, eine C₁₋₁₀-Alkylgruppe ist, die gegebenenfalls mit -NHCONH₂, -COOH, -OH, NH₂, -NH-CNNH₂, Sulfon, Sulfoxid oder Sulfonsäure substituiert sein kann.

26. Konjugat nach Anspruch 23, wobei der Linker die folgende Formel aufweist: 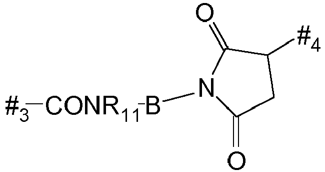 wobei
#3 die Bindung an das Wirkstoffmolekül darstellt,
#4 die Bindung an das Binderpeptid oder -protein darstellt,
R¹¹ H oder NH₂ darstellt;
B-[(CH₂)ₓ-(X⁴)_{y}]_{W}-(CH₂)_{z}- darstellt,
w = 0 bis 20 ist;
x = 0 bis 5 ist;
x = 0 bis 5 ist;
y = 0 oder 1 ist;
z = 0 bis 5 ist; und
X⁴ -O-, -CONH- oder -NHCO- 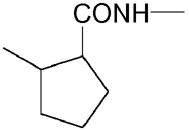 darstellt.

27. Konjugat nach Anspruch 23, wobei R¹ oder R⁴ -L-#1 darstellt.

28. Konjugat nach einem oder mehreren der Ansprüche 23 bis 27, wobei das Konjugat eine der folgenden Formeln aufweist: 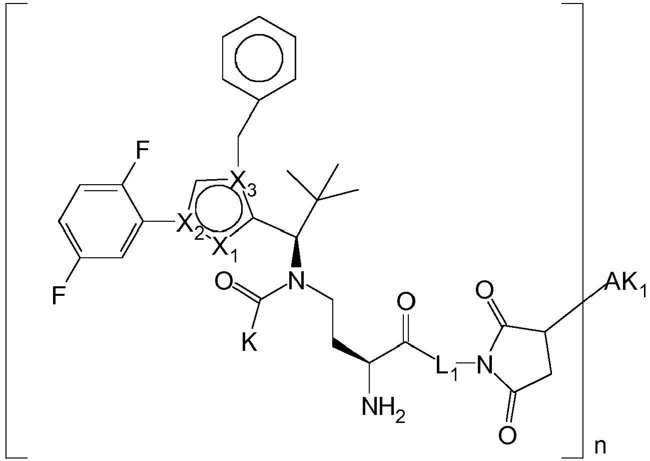 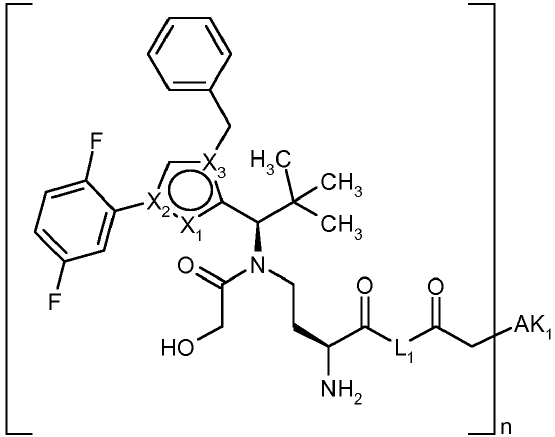 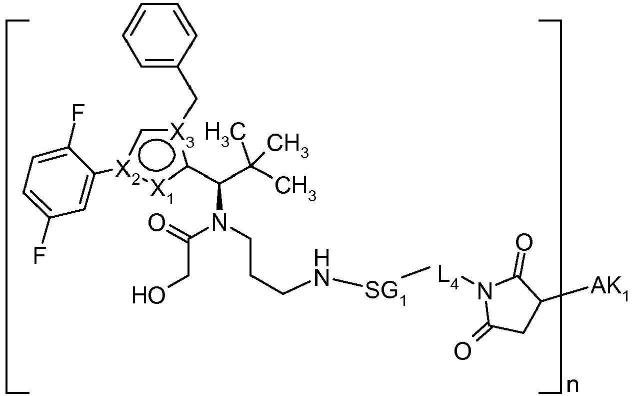 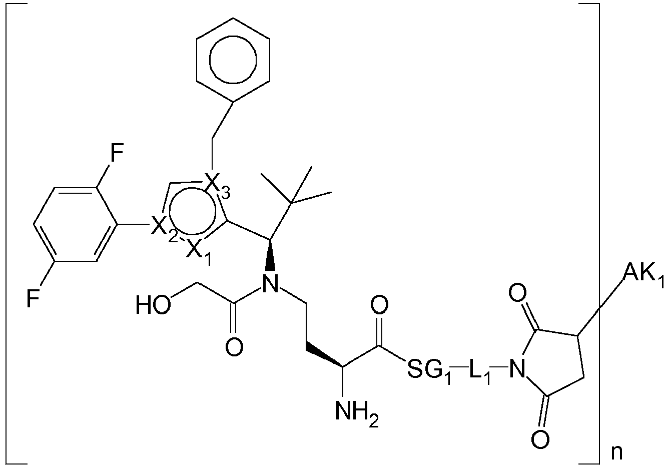 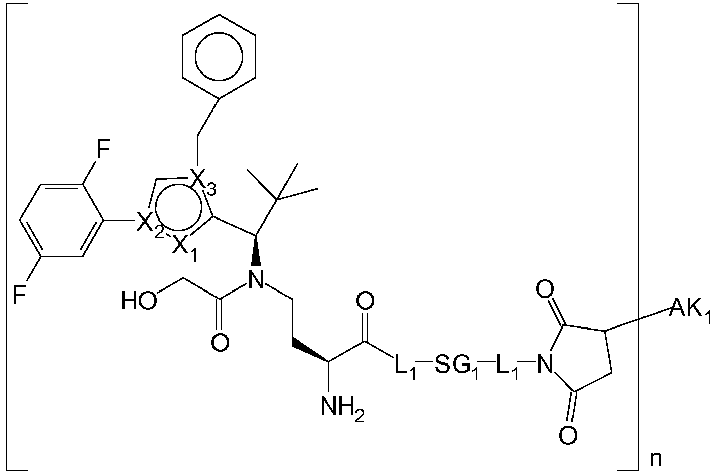 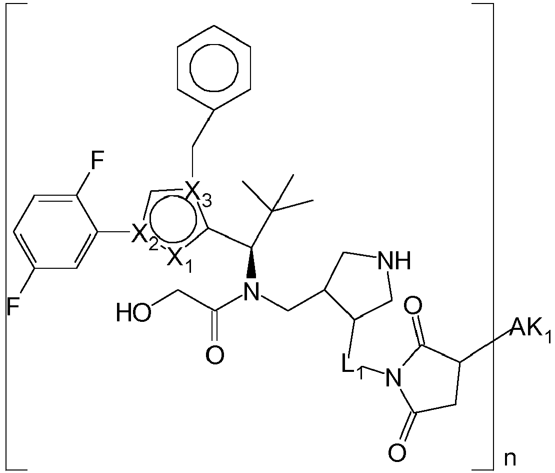 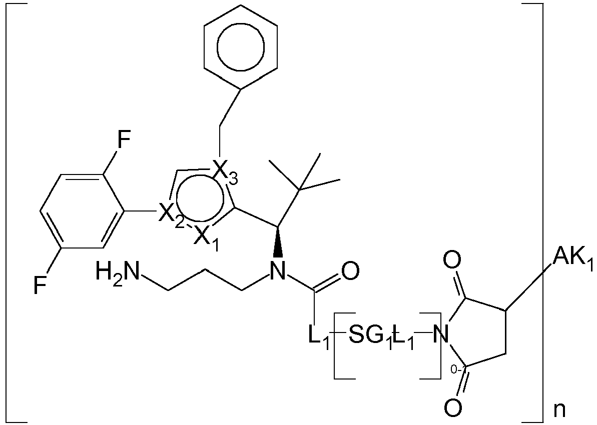 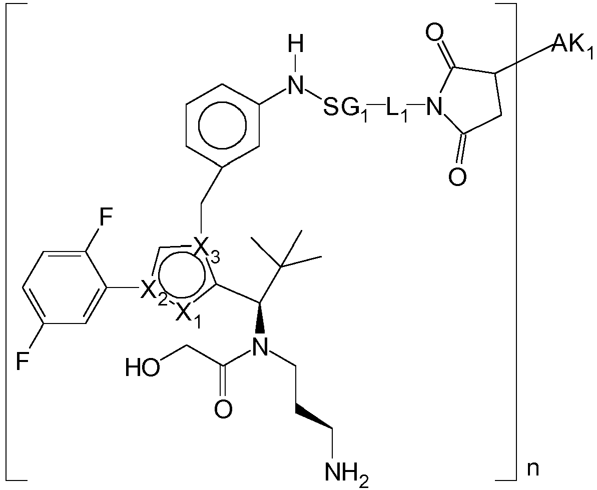 wobei
X₁, X₂ und X₃ die gleiche Bedeutung aufweisen wie in Anspruch 4,
AK₁ ein Binderpeptid oder-protein darstellt, das über ein Schwefelatom des Binders gebunden ist; n eine Zahl von 1 bis 20 darstellt; und L1 eine gegebenenfalls verzweigte Kohlenwasserstoffgruppe mit 1 bis 70 Kohlenstoffatomen ist, die eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, SO₂, -NH-, -CO-, -CONH-, -NHCO-, -Nme-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und S, -SO- oder -SO2- unterbrochen sein kann (vorzugsweise 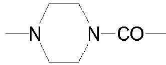 ), wobei die Seitenketten, sofern vorhanden, mit -NHCONH2, -COOH, -OH, -NH₂, NH-CNNH₂, Sulfonamid, Sulfon, Sulfoxid oder Sulfonsäure substituiert sein können,
und SG1 ein 2-8 Oligopeptid, bevorzugt ein Dipeptid ist;
L4 eine Einfachbindung oder eine Gruppe -(CO)_{y}-G4- darstellt, wobei y 0 oder 1 darstellt, und G4 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-,-SO-, SO₂, -NH-, -CO-, -NHCO-, -CONH-, -Nme-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und S, -SO-oder -SO2- unterbrochen sein kann (vorzugsweise 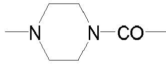 ), wobei die Seitenketten, sofern vorhanden, mit -NHCONH2, -COOH, -OH, -NH₂, NH-CNNH₂, Sulfonamid, Sulfon, Sulfoxid oder Sulfonsäure substituiert sein können.

29. Konjugat eines Binders oder Derivats hiervon mit einem oder mehreren Wirkstoffmolekülen, wobei das Konjugat eine oder beide der folgenden Formeln aufweist: 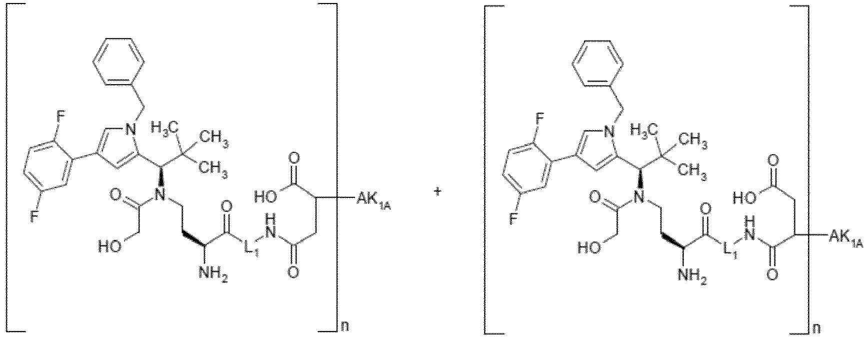 wobei
AK_{1A} ein Binderpeptid oder-protein darstellt, das über ein Schwefelatom des Binders gebunden ist; n eine Zahl von 1 bis 20 darstellt; und L1 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, SO₂, -NH-, -CO-, -CONH-, - NHCO-, -NMe-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und S, -SO- oder -SO2- unterbrochen sein kann (vorzugsweise 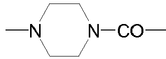 ), wobei die Seitenketten, sofern vorhanden, mit -NHCONH2, -COOH, -OH, -NH₂, NH-CNNH₂, Sulfonamid, Sulfon, Sulfoxid oder Sulfonsäure substituiert sein können.

30. Konjugat nach einem oder mehreren der vorhergenden Ansprüche, wobei der Linker -L- an eine Cystein-Seitenkette bzw. Cysteinrest gebunden ist und die folgende Formel aufweist: 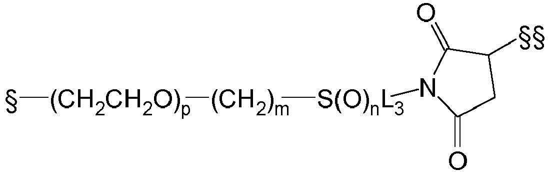 wobei
§ die Bindung an das Wirkstoffmolekül darstellt und
§§ die Bindung an das Binderpeptid oder -protein darstellt,
m 0, 1, 2, oder 3 ist;
n 0, 1 oder 2 ist;
p 0 bis 20 beträgt; und
L3 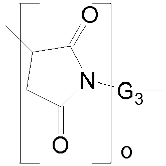 darstellt;
wobei
o 0 oder 1 ist;
und
G3 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -0-, -S-, - SO-, SO₂, -NH-, -CO-, -NHCO, -CONH- und einen 5 bis 10gliedriger, aromatischen oder nicht aromatischen Heterozyklus mit bis zu 4 Heteroatomen ausgewählt aus N, O und S, -, -Nme-,-NHNH-, -SO2NHNH-, -CONHNH- -SO-oder -SO2- unterbrochen sein kann (vorzugsweise 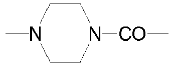 ), wobei die Seitenketten, sofern vorhanden, mit -NHCONH2, -COOH, -OH, Sulfon, Sulfoxid oder Sulfonsäure substituiert sein können.

31. Konjugat nach Anspruch 30, wobei der Linker -L- an eine Cystein-Seitenkette bzw. Cysteinrest gebunden ist und die folgende Formel aufweist: 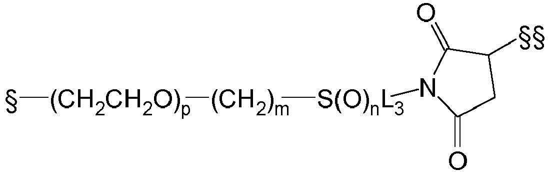 wobei
§ die Bindung an das Wirkstoffmolekül darstellt und
§ § die Bindung an das Binderpeptid oder -protein darstellt,
m 1 ist;
p 0 ist;
n 0 ist;
und L3 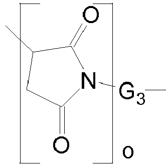 darstellt;
wobei
o 0 oder 1 ist; und
G3 -(CH2CH2O)ₛ(CH2)ₜ(CONH)ᵤ (CH2CH2O)ᵥ(CH2)_{w}- darstellt, wobei
s, t, v und w jeweils unabhängig voneinander 0 bis 20 betragen, und u 0 oder 1 beträgt.

32. Konjugat nach Anspruch 30 oder 31, wobei R² oder R³ -L-#1 darstellt.

33. Konjugat nach Anspruch 32, wobei das Konjugat eine der folgenden Formeln aufweist: 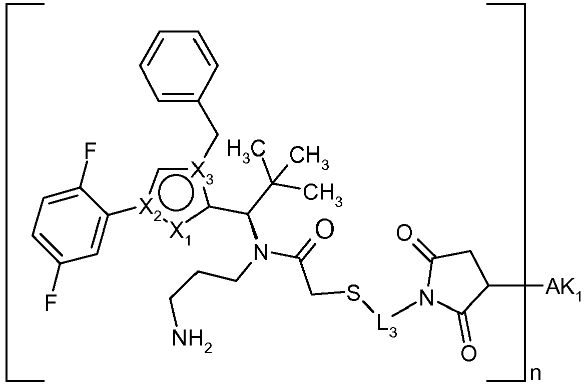 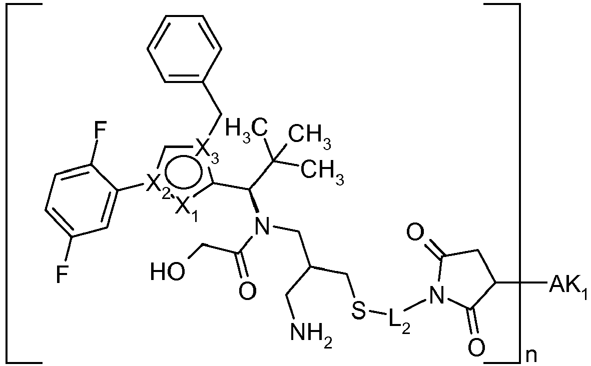 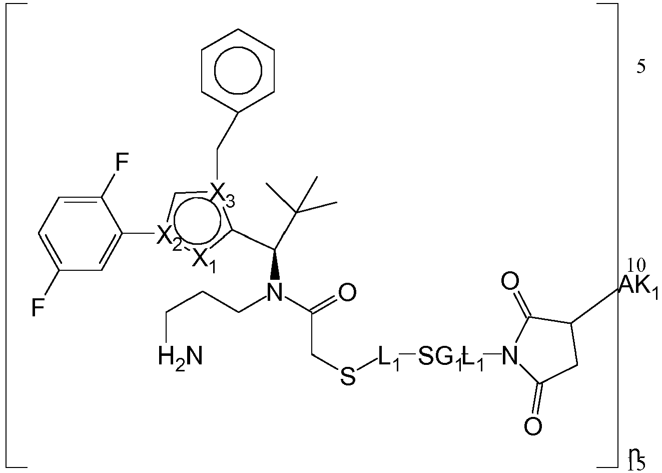 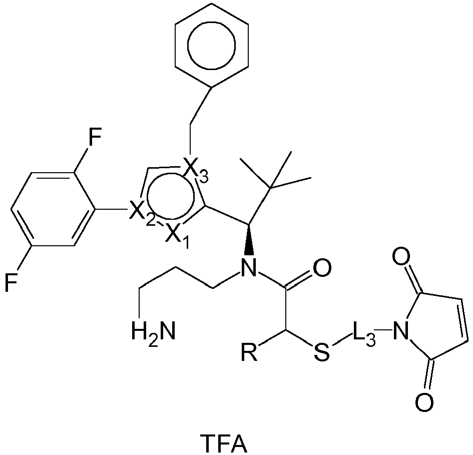 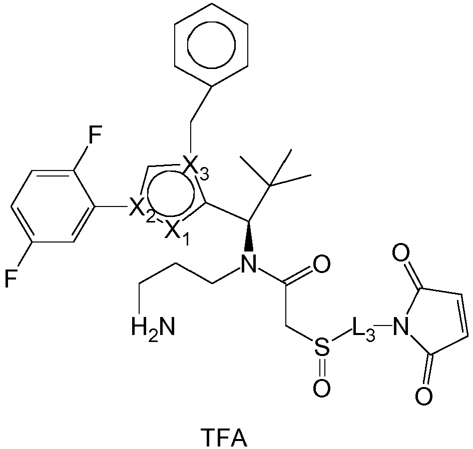 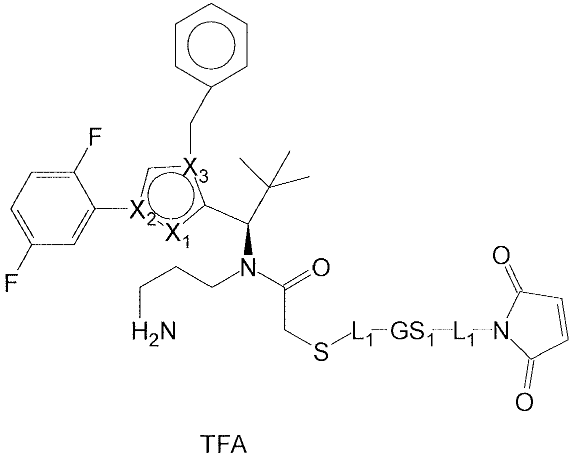 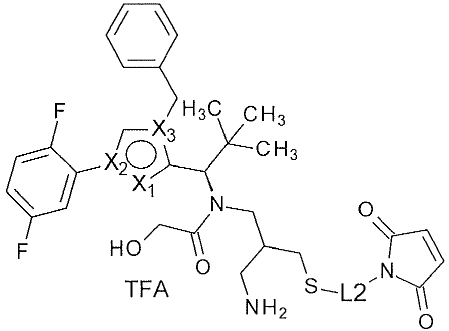 wobei
X₁, X₂ und X₃ die gleiche Bedeutung aufweisen wie in Anspruch 4,
AK₁ ein Binderpeptid oder-protein darstellt, das über ein Schwefelatom des Binders angebunden ist; n eine Zahl von 1 bis 20 darstellt; und L2 und L3 eine gegebenenfalls verzweigte geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, SO₂, -NH-, -CO-, -Nme-, -NHNH-, -SO₂NHNH-,-NHCO-, -CONH-, -CONHNH- und einen 5 bis 10gliedriger, aromatischen oder nicht aromatischen Heterozyklus mit bis zu 4 Heteroatomen ausgewählt aus N, O und S, -SO-oder -SO2- unterbrochen sein kann (vorzugsweise 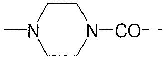 ), wobei die Seitenketten, sofern vorhanden, mit -NHCONH2, -COOH, -OH, -NH₂, NH-CNNH₂, Sulfonamid, Sulfon, Sulfoxid oder Sulfonsäure substituiert sein können.

34. Konjugat nach einem oder mehreren der Ansprüche 1 bis 29, wobei der Linker -L- an eine Lysin-Seitenkette gebunden ist und die folgende Formel aufweist:
-§-(SG)ₓ-L4-CO-§§,
wobei
§ die Bindung an das Wirkstoffmolekül darstellt und
§§ die Bindung an das Binderpeptid oder -protein darstellt,
x 0 oder 1 darstellt,
SG eine spaltbare Gruppe, vorzugsweise ein 2-8 Oligopeptid, besonders bevorzugt ein Dipeptid darstellt,
und
L4 eine Einfachbindung oder eine Gruppe -(CO)_{y}-G4- darstellt, wobei y 0 oder 1 darstellt, und G4 eine geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-,-SO-, SO₂, -NH-, -CO-, -NHCO-, -CONH-, -Nme-, -NHNH-, -SO2NHNH-, -CONHNH- und einen 5 bis 10gliedriger, aromatischen oder nicht aromatischen Heterozyklus mit bis zu 4 Heteroatomen ausgewählt aus N, O und S, oder -SO- unterbrochen sein kann (vorzugsweise 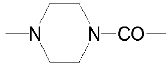 ), wobei die Seitenketten, sofern vorhanden, mit -NHCONH₂, -COOH, -OH, -NH₂, NH-CNNH₂, Sulfonamid, Sulfon, Sulfoxid oder Sulfonsäure substituiert sein können.

35. Konjugat eines Binderpeptids oder -proteins nach Anspruch 34, wobei das Konjugat eine der folgenden Formeln aufweist: 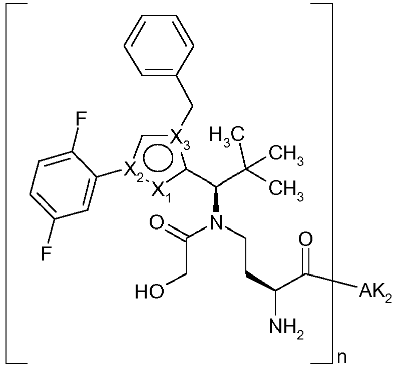 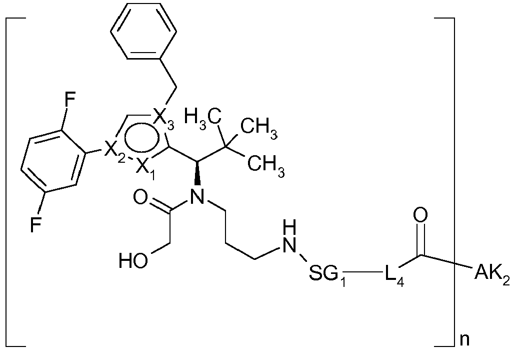 wobei
X₁, X₂ und X₃ die gleiche Bedeutung aufweisen wie in Anspruch 4,
AK₂ ein Binderpeptid oder-protein darstellt, das über ein Schwefelatom des Binders angebunden ist; n eine Zahl von 1 bis 20 darstellt; und L4 eine gegebenenfalls geradkettige oder verzweigte Kohlenwasserstoffkette mit 1 bis 100 Kohlenstoffatomen aus Arylengruppen und/oder geradkettigen und/oder verzweigten und/oder cyclischen Alkylengruppen ist, die einfach oder mehrfach durch eine oder mehrere der Gruppen -O-, -S-, , -SO-, SO₂, -NH-, -CO-, -Nme-,-NHNH-, -SO₂NHNH-,-NHCO-, -CONH-, -CONHNH- und einen 5 bis 10gliedriger, aromatischen oder nicht aromatischen Heterozyklus mit bis zu 4 Heteroatomen ausgewählt aus N, O und S, -SO- oder -SO2- unterbrochen sein kann (vorzugsweise 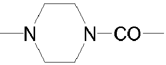 ), wobei die Seitenketten, sofern vorhanden, mit -NHCONH₂, -COOH, -OH, -NH₂, NH-CNNH₂, Sulfonamid, Sulfon, Sulfoxid oder Sulfonsäure substituiert sein können, und SG1 eine spaltbare Gruppe, vorzugsweise ein 2-8 Oligopeptid, besonders bevorzugt ein Dipeptid darstellt.

36. Konjugat nach einem oder mehreren der Ansprüche 18 bis 35, wobei der anti-TWEAKR-Antikörper ein agonistischer Antikörper ist.

37. Konjugat einem oder mehreren der Ansprüche 18 bis 36, der umfasst:
eine variable schwere Kette umfassend:
a. eine CDR1 der schweren Kette, die von einer Aminosäuresequenz umfassend die Formel PYPMX (SEQ ID NO: 171) kodiert wird, wobei X I oder M ist;
b. eine CDR2 der schweren Kette, die von einer Aminosäuresequenz umfassend die Formel YISPSGGXTHYADSVKG (SEQ ID NO: 172) kodiert wird, wobei X S oder K ist; und
c. eine CDR3 der schweren Kette, die von einer Aminosäuresequenz umfassend die Formel GGDTYFDYFDY (SEQ ID NO: 173) kodiert wird;
und eine variable leichte Kette umfassend:
d. eine CDR1 der leichten Kette, die von einer Aminosäuresequenz umfassend die Formel RASQSISXYLN (SEQ ID NO: 174) kodiert wird, wobei X G oder S ist;
e. eine CDR2 der leichten Kette, die von einer Aminosäuresequenz umfassend die Formel XASSLQS (SEQ ID NO: 175) kodiert wird, wobei X Q , A oder N ist; und
f. eine CDR3 der leichten Kette, die von einer Aminosäuresequenz umfassend die Formel QQSYXXPXIT (SEQ ID NO: 176) kodiert wird, wobei X an der Position 5 T oder S ist, X an der Position 6 T oder S ist und X an der Position 8 G oder F ist.

38. Konjugat einem oder mehreren der Ansprüche 18 bis 37, umfassend:
a. eine variable Sequenz der schweren Kette, wie dargestellt durch SEQ ID NO:10, sowie eine variable Sequenz der leichten Kette, wie dargestellt durch SEQ ID NO:9 oder
b. eine variable Sequenz der schweren Kette, wie dargestellt durch SEQ ID NO:20, sowie eine variable Sequenz der leichten Kette, wie dargestellt durch SEQ ID NO:19 oder
c. eine variable Sequenz der schweren Kette, wie dargestellt durch SEQ ID NO:30, sowie eine variable Sequenz der leichten Kette, wie dargestellt durch SEQ ID NO:29 oder
d. eine variable Sequenz der schweren Kette, wie dargestellt durch SEQ ID NO:40, sowie eine variable Sequenz der leichten Kette, wie dargestellt durch SEQ ID NO:39 oder
e. eine variable Sequenz der schweren Kette, wie dargestellt durch SEQ ID NO:50, sowie eine variable Sequenz der leichten Kette, wie dargestellt durch SEQ ID NO:49 oder
f. eine variable Sequenz der schweren Kette, wie dargestellt durch SEQ ID NO:60, sowie eine variable Sequenz der leichten Kette, wie dargestellt durch SEQ ID NO:59 oder
g. eine variable Sequenz der schweren Kette, wie dargestellt durch SEQ ID NO:70, sowie eine variable Sequenz der leichten Kette, wie dargestellt durch SEQ ID NO:69 oder
h. eine variable Sequenz der schweren Kette, wie dargestellt durch SEQ ID NO:80, sowie eine variable Sequenz der leichten Kette, wie dargestellt durch SEQ ID NO:79 oder
i. eine variable Sequenz der schweren Kette, wie dargestellt durch SEQ ID NO:90, sowie eine variable Sequenz der leichten Kette, wie dargestellt durch SEQ ID NO:89 oder
j. eine variable Sequenz der schweren Kette, wie dargestellt durch SEQ ID NO:100, sowie eine variable Sequenz der leichten Kette, wie dargestellt durch SEQ ID NO:99 oder
k. eine variable Sequenz der schweren Kette, wie dargestellt durch SEQ ID NO:110, sowie eine variable Sequenz der leichten Kette, wie dargestellt durch SEQ ID NO:109 oder
l. eine variable Sequenz der schweren Kette, wie dargestellt durch SEQ ID NO:120, sowie eine variable Sequenz der leichten Kette, wie dargestellt durch SEQ ID NO:119.

39. Konjugat einem oder mehreren der Ansprüche 18 bis 38, wobei der Antikörper ein IgG Antikörper ist.

40. Konjugat einem oder mehreren der Ansprüche 18 bis 39, umfassend:
a. eine Sequenz der schweren Kette, wie dargestellt durch SEQ ID NO:2, sowie eine Sequenz der leichten Kette, wie dargestellt durch SEQ ID NO:1 oder
b. eine Sequenz der schweren Kette, wie dargestellt durch SEQ ID NO:12, sowie eine Sequenz der leichten Kette, wie dargestellt durch SEQ ID NO:11 oder
c. eine Sequenz der schweren Kette, wie dargestellt durch SEQ ID NO:22, sowie eine Sequenz der leichten Kette, wie dargestellt durch SEQ ID NO:21 oder
d. eine Sequenz der schweren Kette, wie dargestellt durch SEQ ID NO:32, sowie eine Sequenz der leichten Kette, wie dargestellt durch SEQ ID NO:31 oder
e. eine Sequenz der schweren Kette, wie dargestellt durch SEQ ID NO:42, sowie eine Sequenz der leichten Kette, wie dargestellt durch SEQ ID NO:41 oder
f. eine Sequenz der schweren Kette, wie dargestellt durch SEQ ID NO:52, sowie eine Sequenz der leichten Kette, wie dargestellt durch SEQ ID NO:51 oder
g. eine Sequenz der schweren Kette, wie dargestellt durch SEQ ID NO:62, sowie eine Sequenz der leichten Kette, wie dargestellt durch SEQ ID NO:61 oder
h. eine Sequenz der schweren Kette, wie dargestellt durch SEQ ID NO:72, sowie eine Sequenz der leichten Kette, wie dargestellt durch SEQ ID NO:71 oder
i. eine Sequenz der schweren Kette, wie dargestellt durch SEQ ID NO:82, sowie eine Sequenz der leichten Kette, wie dargestellt durch SEQ ID NO:81 oder
j. eine Sequenz der schweren Kette, wie dargestellt durch SEQ ID NO:92, sowie eine Sequenz der leichten Kette, wie dargestellt durch SEQ ID NO:91 oder
k. eine Sequenz der schweren Kette, wie dargestellt durch SEQ ID NO:102, sowie eine Sequenz der leichten Kette, wie dargestellt durch SEQ ID NO:101 oder 1. eine Sequenz der schweren Kette, wie dargestellt durch SEQ ID NO:112, sowie eine Sequenz der leichten Kette, wie dargestellt durch SEQ ID NO:111.

41. Spindel-Konjugat nach einem oder mehreren der vorhergehenden Ansprüche, wobei das Konjugat 1 bis 10, vorzugsweise 2 bis 8, Wirkstoffmoleküle pro Binderpeptid oder -protein aufweist.

42. Verfahren zur Herstellung des Konjugats nach Anspruch 28 oder 32, wobei eine Verbindung einer der folgenden Formeln, vorzugsweise in Form ihres Trifluoressigsäuresalzes, an einen Cysteinrest eines zuvor ggf. partiell reduzierten Binderpeptids oder -proteins gebunden wird, wobei die Verbindung vorzugsweise in 2 bis 12 fachem molaren Überschuss gegenüber dem Binderpeptid- oder protein verwendet wird: 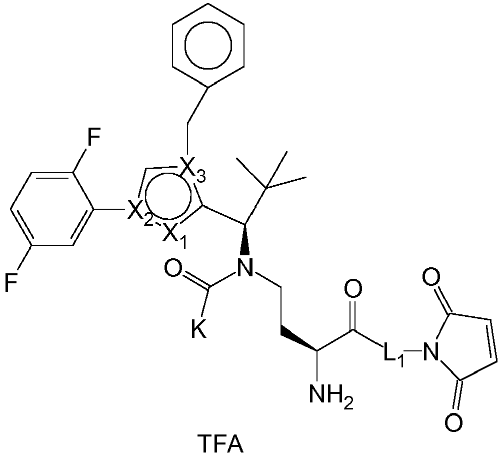 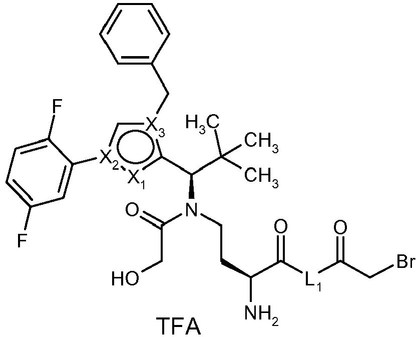 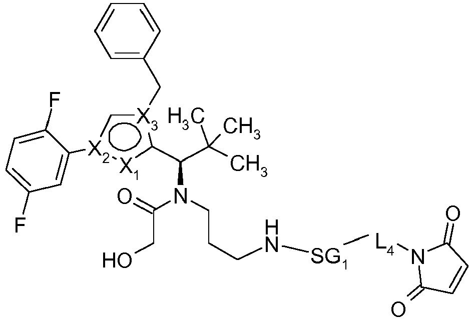 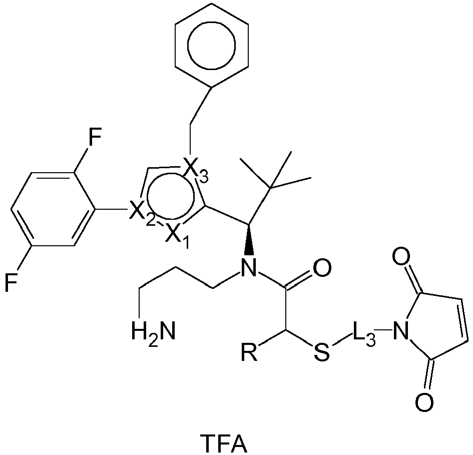 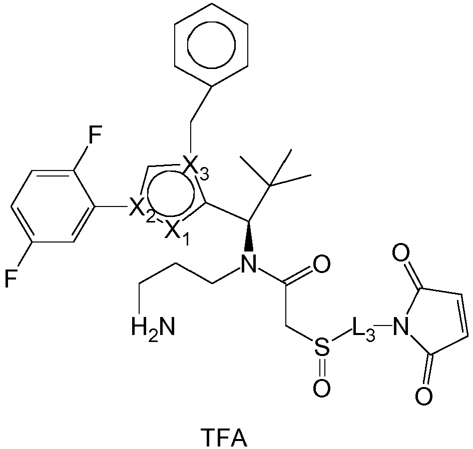 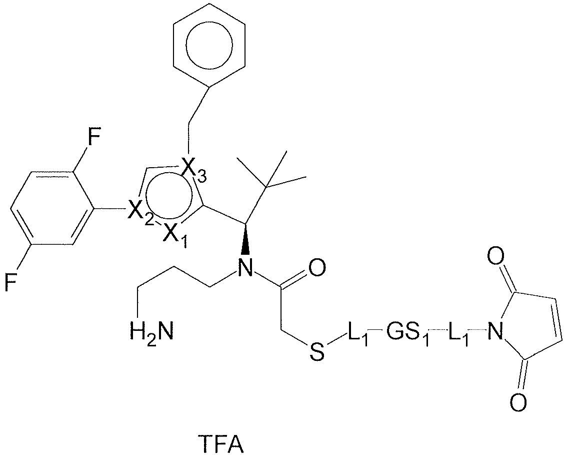 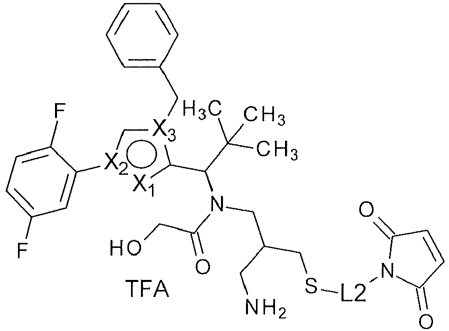 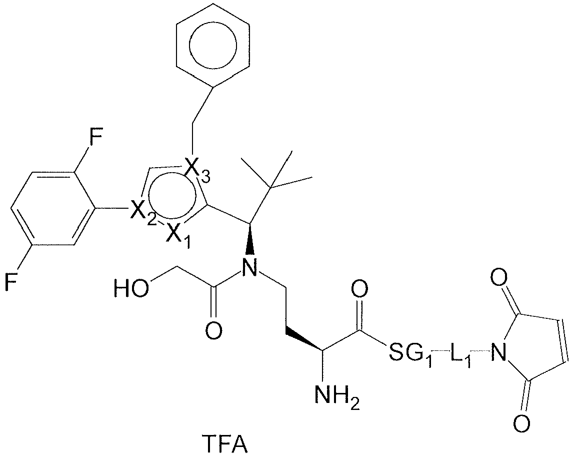 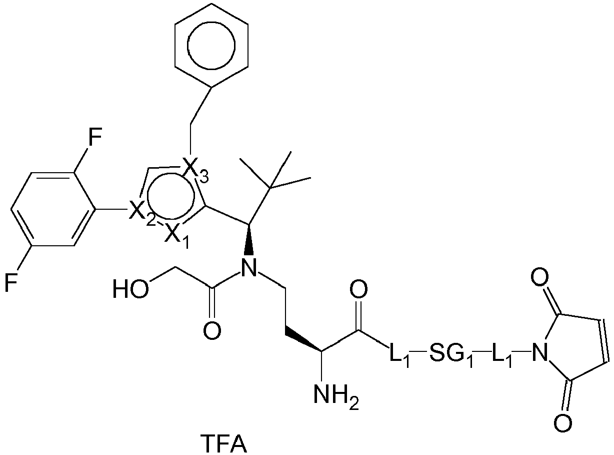 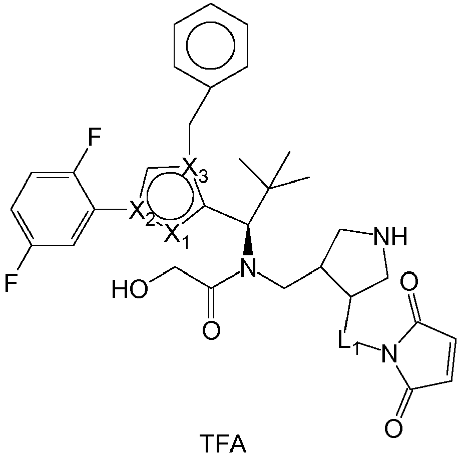 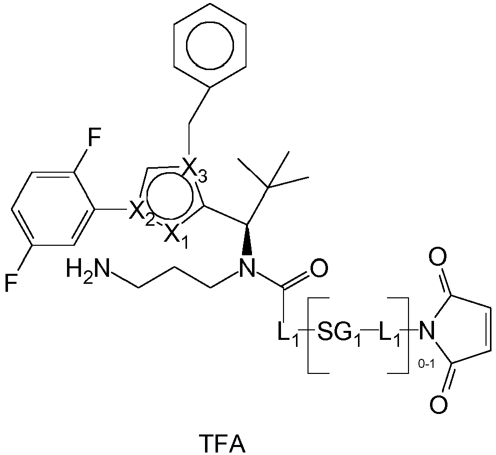 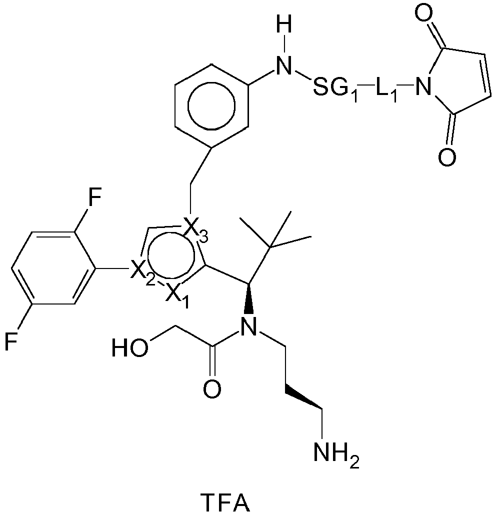
wobei R -H oder -COOH darstellt,
wobei K lineares oder verzweigtes gegebenenfalls substituiertes mit C₁-C₆ Alkoxy oder -OH C₁-C₆ Alkyl sarstellt, und
wobei X₁, X₂, X₃, SG1, L1, L2, L3 und L4 die gleiche Bedeutung haben wie in Anspruch 28, bzw.32 .

43. Verfahren zur Herstellung des Konjugats nach Anspruch 42, wobei eine Verbindung einer der folgenden Formeln, vorzugsweise in Form ihres Trifluoressigsäuresalzes, an einen Lysinrest eines Binderpeptids oder -proteins gebunden wird, wobei die Verbindung vorzugsweise in 2 bis 12 fachem molaren Überschuss gegenüber dem Binderpeptid- oder protein verwendet wird: 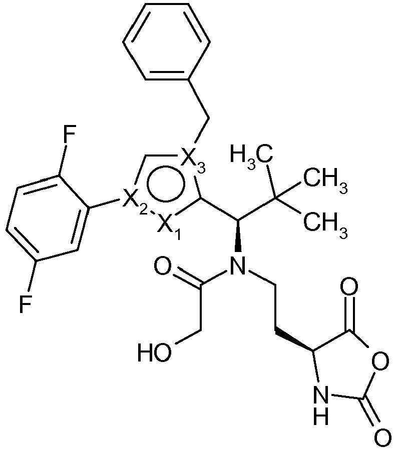 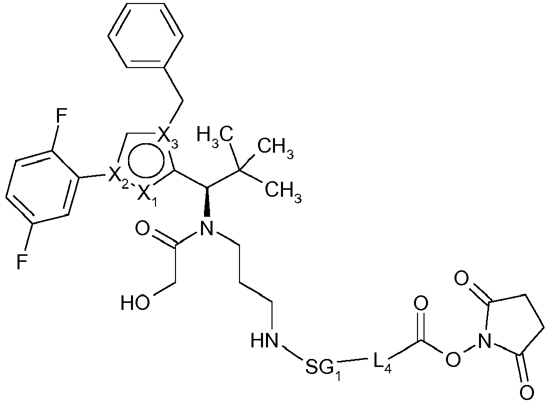 wobei X₁, X₂, X₃, SG1, und L4 die gleiche Bedeutung haben wie in Anspruch 34.

44. Verbindung einer der folgenden Formeln: 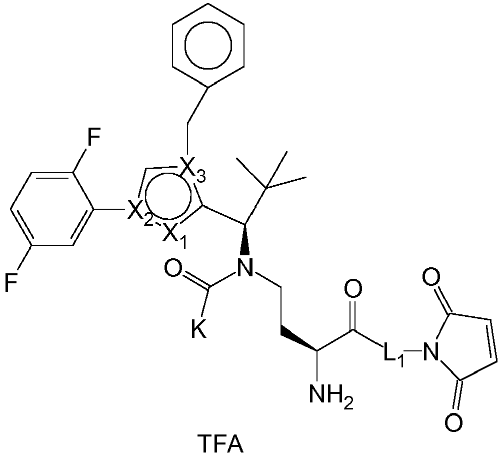 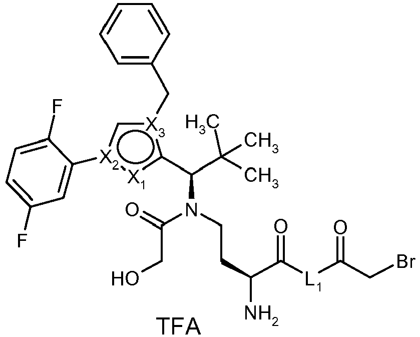 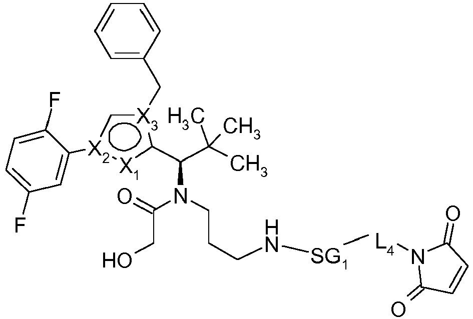 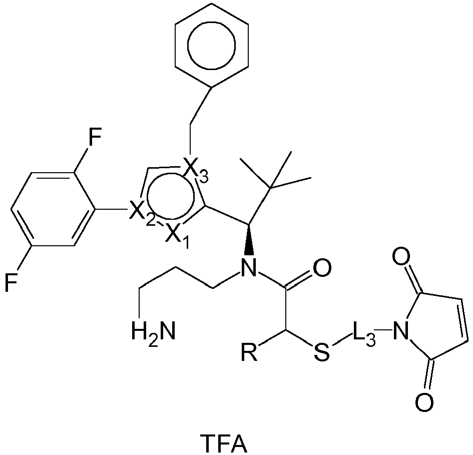 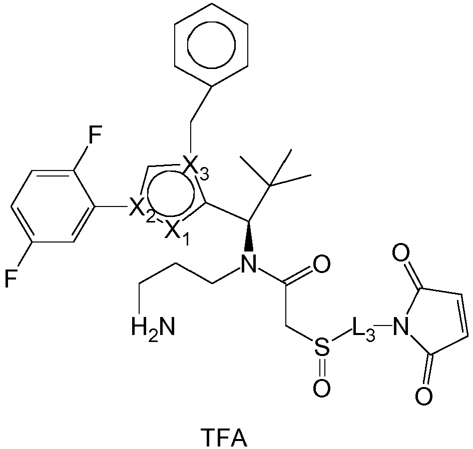 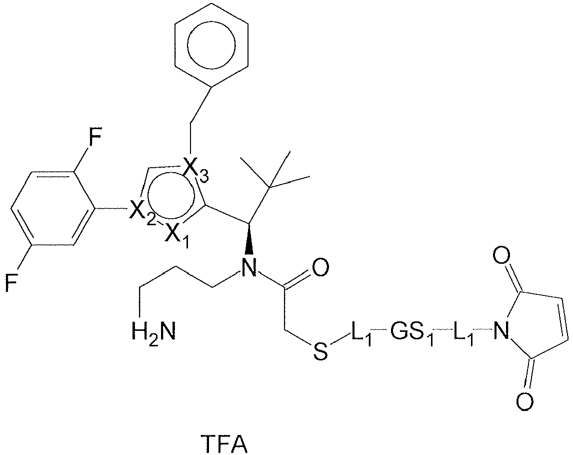 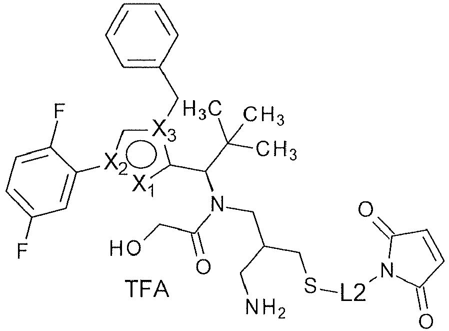 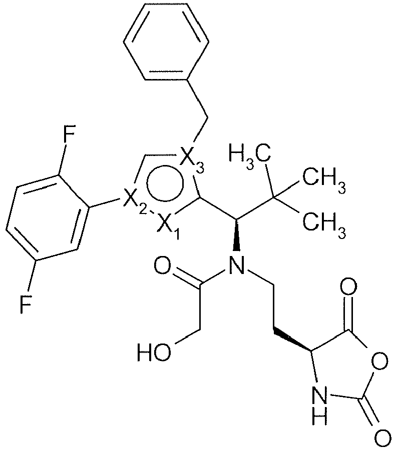 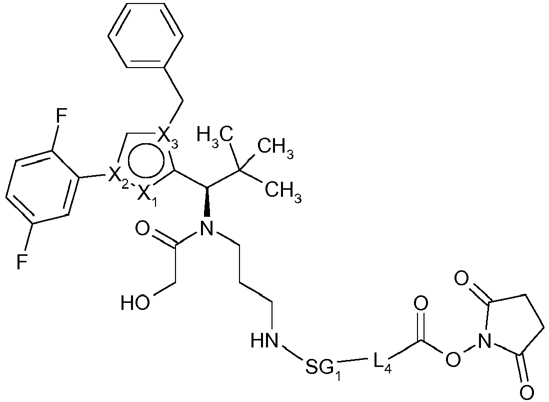 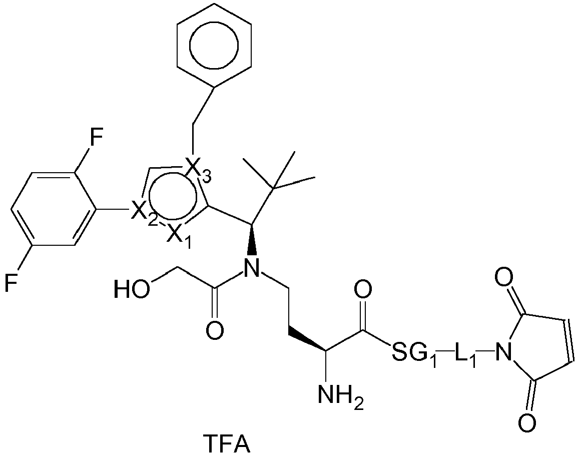 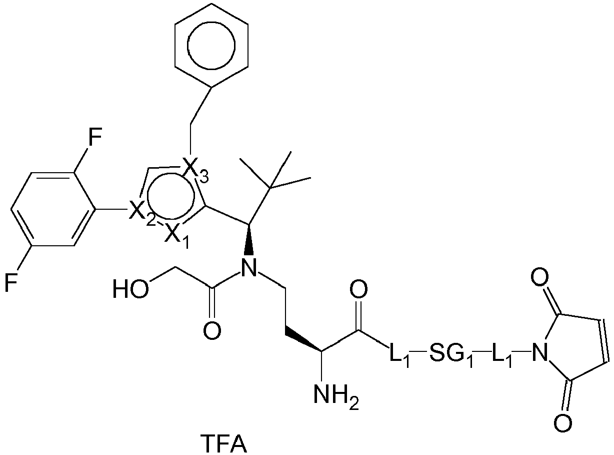 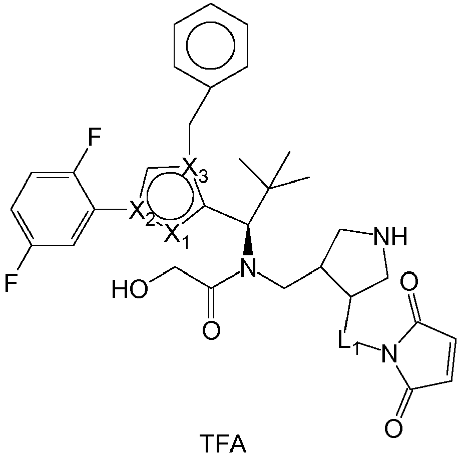 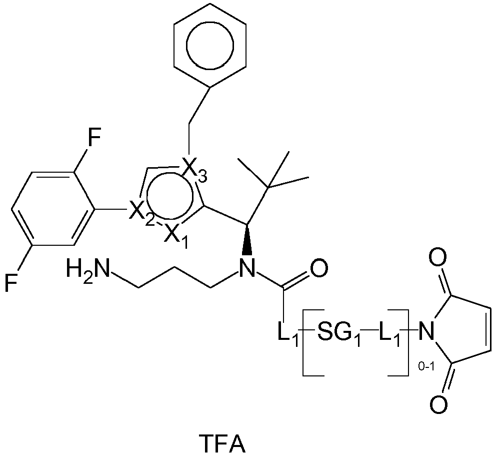 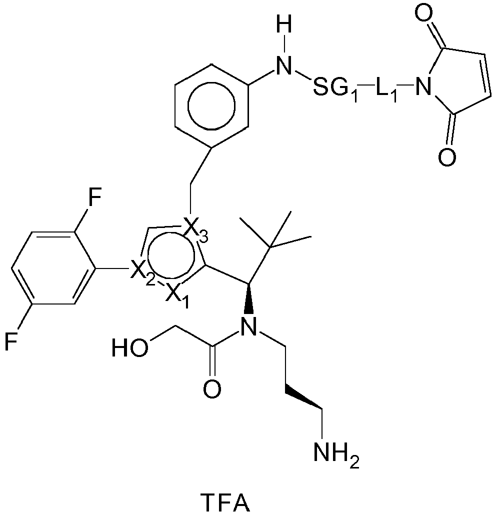
wobei R -H oder -COOH darstellt,
wobei K lineares oder verzweigtes gegebenenfalls substituiertes mit C₁-C₆ Alkoxy oder -OH C₁-C₆ Alkyl sarstellt, und
wobei X₁, X₂, X₃, SG1, L1, L2, L3 und L4 die gleiche Bedeutung haben wie in Anspruch 24, 28 bzw. 30.

45. Verbindungen der allgemeinen Formel (III): 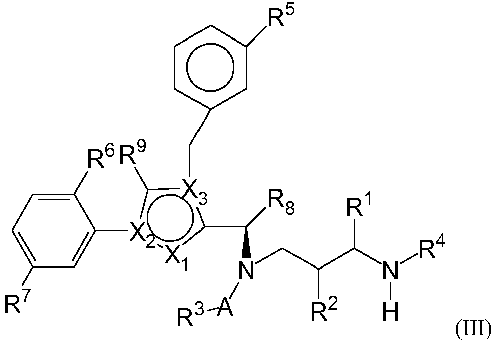 wobei
X₁ N, X₂ N und X₃ C darstellt, oder X₁ CH, X₂ C und X₃ N darstellt oder X₁ NH, X₂ C und X₃ C darstellt, oder X₁ CH, X₂ N und X₃ C darstellt;
R¹ -L-BINDER, H, oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander H, NH2, -(CH₂CH₂O)₀₋₃-(CH₂)₀₋₃Z', oder-CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt, wobei W H oder OH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt;
R² und R⁴ unabhängig voneinander -L-BINDER, H, -CO-CHY⁴-NHY⁵ oder -(CH₂)₀₋₃Z darstellen, oder R² und R⁴ gemeinsam (unter Bildung eines Pyrrolidinringes) -CH₂-CHR¹⁰- oder -CHR¹⁰-CH₂- darstellen, wobei R¹⁰ L-#1, H, NH₂, SO₃H, COOH, SH, oder OH darstellt;
wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
A CO, SO, SO₂, SO₂NH oder CNNH darstellt;
R³ -L-BINDER oder eine gegebenenfalls substituierte Alkyl-, Aryl-, Heteroaryl- Heteroalkyl-, Heterocycloalkyl- Gruppe darstellt, bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe, die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3-NH-CO-NH-Alkyl Gruppen, 1-3 -S(O)ₙ-Alkyl Gruppen, 1-3 -SO₂-NH-Alkyl Gruppen, 1-3-NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H, -(CH₂)₀₋₃-CH(NHCOCH₃)Z',-(CH₂)₀₋₃-CH(NH₂)Z', oder -(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet);
R⁵ -L-BINDER, H, F, NH₂, NO₂, Halogen, SH oder -(CH₂)₀₋₃Z darstellt, wobei Z -H, Halogen, -OY³, -SY³, -NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder - (CH₂)₀₋₃Z' darstellt, wobei Z' H, SO₃H, NH₂ oder COOH darstellt;
darstellt,
wobei L für einen Linker steht und BINDER für Binder bzw. Derivat hiervon steht, wobei der Binder ggf. an mehrere Wirkstoffmoleküle gebunden sein kann,
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy oder Halogen darstellen,
R⁸ (ggf. fluoriertes) C₁₋₁₀-Alkyl, (ggf. fluoriertes) C₄₋₁₀-Cycloalkyl oder optional substituiertes Oxetan darstellt; und
R⁹ H, F, CH₃, CF₃, CH₂F oder CHF₂ darstellt;
sowie ihre Salze, Solvate und Salze der Solvate.

46. Verbindungen der allgemeinen Formel (IV): 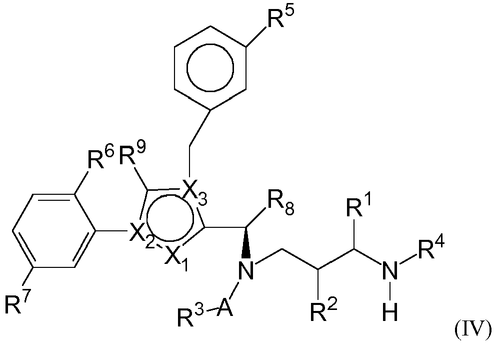 wobei
X₁ CH, X₂ C und X₃ N darstellt;
R¹ -L-BINDER, H, oder -(CH₂)₀₋₃Z darstellt, wobei Z -H, -NHY³, -OY³, -SY³, Halogen, -CO-NY¹Y², oder -CO-OY³ darstellt,
wobei Y¹ und Y² unabhängig voneinander H, NH2, -(CH₂CH₂O)₀₋₃-(CH₂)₀₋₃Z', oder-CH(CH₂W)Z' darstellen, und Y³ H oder -(CH₂)₀₋₃Z' darstellt, wobei Z' H, NH₂, SO₃H, COOH, -NH-CO-CH₂-CH₂-CH(NH₂)COOH oder -(CO-NH-CHY⁴)₁₋₃COOH darstellt; wobei W H oder OH darstellt;
wobei Y⁴ unabhängig voneinander lineares oder verzweigtes, gegebenenfalls mit -NHCONH₂ substituiertes, C₁₋₆Alkyl oder gegebenenfalls mit -NH₂ substituiertes Aryl oder Benzyl darstellt;
R² und R⁴ unabhängig voneinander -L-BINDER, H, -CO-CHY⁴-NHY⁵ oder -(CH₂)₀₋₃Z darstellen, oder R² und R⁴ gemeinsam (unter Bildung eines Pyrrolidinringes) -CH₂-CHR¹⁰- oder -CHR¹⁰-CH₂- darstellen, wobei R¹⁰ L-#1, H, NH₂, SO₃H, COOH, SH, oder OH darstellt; wobei Z -H, Halogen, -OY³, -SY³, NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
wobei Y⁴ unabhängig voneinander lineares oder verzweigtes, gegebenenfalls mit-NHCONH₂ substituiertes, C₁₋₆ Alkyl oder gegebenenfalls mit -NH₂ substituiertes Aryl oder Benzyl darstellt und Y⁵ H oder -CO-CHY⁶-NH₂ darstellt, wobei Y⁶ lineares oder verzweigtes C₁₋₆ Alkyl darstellt;
A CO, SO, SO₂, SO₂NH oder CNNH darstellt;
R³ -L-BINDER oder eine gegebenenfalls substituierte Alkyl-, Aryl-, Heteroaryl- Heteroalkyl-, Heterocycloalkyl- Gruppe darstellt,
bevorzugt -L-#1 oder eine C₁₋₁₀-Alkyl-, C₆₋₁₀-Aryl- oder C₆₋₁₀-Aralkyl-, C₅₋₁₀-Heteroalkyl-, C₁₋₁₀-Alkyl-O-C₆₋₁₀-Aryl- oder C₅₋₁₀-Heterocycloalkyl-Gruppe,
die mit 1-3 -OH Gruppen, 1-3 Halogenatomen, 1-3 halogenierten Alkylgruppen (die jeweils 1-3 Halogenatome aufweisen), 1-3 O-Alkyl Gruppen, 1-3 -SH Gruppen, 1-3 -S-Alkyl Gruppen, 1-3 -O-CO-Alkyl Gruppen, 1-3 -O-CO-NH-Alkyl Gruppen, 1-3 -NH-CO-Alkyl Gruppen, 1-3-NH-CO-NH-Alkyl Gruppen, 1-3 -S(O)ₙ-Alkyl Gruppen, 1-3 -SO₂-NH-Alkyl Gruppen, 1-3-NH-Alkyl Gruppen, 1-3 -N(Alkyl)₂ Gruppen, 1-3 -NH₂ Gruppen oder 1-3 -(CH₂)₀₋₃Z Gruppen, wobei Z -H, Halogen, -OY³, -SY³, -NHY³, -CO-NY¹Y², oder -CO-OY³ darstellt, wobei Y¹ und Y² unabhängig voneinander H, NH₂, oder -(CH₂)₀₋₃Z' darstellen, und Y³ H, -(CH₂)₀₋₃-CH(NHCOCH₃)Z', -(CH₂)₀₋₃-CH(NH₂)Z', oder -(CH₂)₀₋₃Z' darstellt, wobei Z' H, SO₃H, NH₂ oder COOH darstellt,
substituiert sein kann
(wobei "Alkyl" bevorzugt C₁₋₁₀-Alkyl bedeutet);
R⁵ -L-BINDER, H, F, NH₂, NO₂, Halogen, SH oder -(CH₂)₀₋₃Z darstellt, wobei Z -H, Halogen, -OY³, -SY³, -NHY³, -CO-NY¹Y², oder -CO-OY³ darstellt,
wobei Y¹ und Y² unabhängig voneinander H, NH₂, oder -(CH₂)₀₋₃Z' darstellen, und Y³ H oder-(CH₂)₀₋₃Z' darstellt, wobei Z' H, SO₃H, NH₂ oder COOH darstellt;
darstellt,
wobei L für einen Linker steht und BINDER für Binder bzw. Derivat hiervon steht, wobei der Binder ggf. an mehrere Wirkstoffmoleküle gebunden sein kann,
R⁶ und R⁷ unabhängig voneinander H, Cyano, (ggf. fluoriertes) C₁₋₁₀-Alkyl, (ggf. fluoriertes) C₂₋₁₀-Alkenyl, (ggf. fluoriertes) C₂₋₁₀-Alkinyl, Hydroxy oder Halogen darstellen,
R⁸ (ggf. fluoriertes) C₁₋₁₀-Alkyl, (ggf. fluoriertes) C₄₋₁₀-Cycloalkyl oder optional substituiertes Oxetan darstellt; und
R⁹ H, F, CH₃, CF₃, CH₂F oder CHF₂ darstellt;
sowie ihre Salze, Solvate und Salze der Solvate.

47. Verbindung nach Anspruch 46, wobei Z Cl oder Br darstellt.

48. Verbindung nach Anspruch 46, wobei R¹ -(CH₂)₀₋₃Z darstellt, wobei Z -CO-NY¹Y² darstellt, wobei Y² -(CH₂CH₂O)₀₋₃-(CH2)₀₋₃Z' und Y¹ H, NH₂, oder -(CH₂CH₂O)₀₋₃-(CH₂)₀₋₃ Z' darstellt.

49. Verbindung nach einem oder mehreren der Ansprüche 46-48 wobei Y¹ H darstellt, Y²-(CH₂CH₂O)₃-CH₂CH₂Z' darstellt und Z' -COOH darstellt.

50. Verbindung nach einem oder mehreren der Ansprüche 46-48, wobei Y¹ H darstellt, Y²-CH₂CH₂Z' darstellt und Z' -(CONHCHY⁴)₂COOH stellt.

51. Verbindung nach dem Anspruch 46, wobei Y⁴ lineares oder verzweigtes, gegebenenfalls mit-NHCONH₂ substituiertes, C₁₋₆ Alkyl darstellt.

52. Verbindung nach dem Anspruch 46, wobei mindestens ein Vertreter von Y⁴ ausgewählt wird aus *i*-propyl or -CH₃.

53. Verbindung nach einem oder mehreren der Ansprüche 46 und 48, wobei Y¹ H darstellt, Y² - CH₂CH₂Z' darstellt, Z' -CONHCHY⁴COOH darstellt und Y⁴ gegebenenfalls mit -NH₂ substituiertes Aryl oder Benzyl darstellt.

54. Verbindung nach dem Anspruch 53, wobei Y⁴ Aminobenzyl darstellt.

55. Verbindung nach dem Anspruch 46, wobei R² -(CH₂)₀₋₃Z darstellt und Z -SY³ darstellt.

56. Verbindung nach dem Anspruch 46, wobei R⁴ -CO-CHY⁴-NHY⁵ darstellt und Y⁵ H darstellt.

57. Verbindung nach dem Anspruch 46 wobei R⁴ -CO-CHY⁴-NHY⁵ darstellt und Y⁵-CO-CHY⁶-NH₂ darstellt.

58. Verbindung nach einem oder mehreren der Ansprüche 56 und 57, wobei Y⁴ lineares oder verzweigtes, gegebenenfalls mit -NHCONH₂ substituiertes C₁₋₆ Alkyl darstellt.

59. Verbindung nach eine oder mehrere Ansprüche 46 bis 58, wobei die Verbindung eine der folgende Formeln aufweist:
N-(3-Aminopropyl)-N-{(1R)-1-[4-benzyl-1-(2,5-difluorphenyl)-1H-pyrazol-3-yl]-2,2-dimethylpropyl}-2-hydroxyacetamid;
N-(3-Aminopropyl)-N-{(1R)-1-[1-benzyl-4-(2,5-difluorphenyl)-1H-pyrrol-2-yl]-2,2-dimethylpropyl}-2-hydroxyacetamid;
(2S)-2-amino-4-[{(1R)-1-[1-benzyl-4-(2,5-difluorphenyl)-1H-pyrrol-2-yl]-2,2-dimethylpropyl}(glycoloyl)amino]-N-methylbutanamid(1:1);
N-(3-Aminopropyl)-N-{(1S)-1-[1-benzyl-4-(2,5-difluorphenyl)-1H-pyrrol-2yl]-2,2-dimethylpropyl} acetamid.
N-(3-Aminopropyl)-N-{(1S)-1-[1-benzyl-4-(2,5-difluorphenyl)-1H-pyrrol-2-yl]-2,2-dimethylpropyl}-2-hydroxyacetamid;
S-[1-(2-{[2-({(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}amino)ethyl]amino}-2-oxoethyl)-2,5-dioxopyrrolidin-3-yl]-L-cystein:
N-{(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}-beta-alanyl-L-alanyl-N-[4-(3-{[(2R)-2-amino-2-carboxyethyl]sulfanyl}-2,5-dioxopyrrolidin-1-yl)phenyl]-N⁵-carbamoyl-L-ornithinamid;
S-(1-{2-[(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)amino]ethyl}-2,5-dioxopyrrolidin-3-yl)-L-cystein;
S-[1-(2-{[2-({(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}amino)ethyl]amino}-2-oxoethyl)-2,5-dioxopyrrolidin-3-yl]-L-cystein;
N-{(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}-beta-alanyl-L-alanyl-N-[4-(3-{[(2R)-2-amino-2-carboxyethyl]sulfanyl}-2,5-dioxopyrrolidin-1-yl)phenyl]-N⁵-carbamoyl-L-ornithinamid;
S-(1-{2-[(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)amino]ethyl}-2,5-dioxopyrrolidin-3-yl)-L-cystein;
N-[6-(3-{[(2R)-2-Amino-2-carboxyethyl]sulfanyl}-2,5-dioxopyrrolidin-1-yl)hexanoyl]-L-valyl-N⁵-carbamoyl-L-ornithyl-N⁶-{(2S)-2-amino-4-[{(1R)-1-[4-benzyl-1-(2,5-difluorphenyl)-1H-pyrazol-3-yl]-2,2-dimethylpropyl}(glycoloyl)amino]butanoyl}-L-lysin;
S-[1-(2-{[2-({(2S)-2-Amino-4-[-{(1R)-1-[1-benzyl-4-(2,5-difluorphenyl)-1H-pyrrol-2-yl]-2,2 dimethylpropyl}(glycoloyl)amino]butanoyl}amino)ethyl]amino}-2-oxoethyl)-2,5-dioxopyrrolidin-3-yl]-L-cystein;
S-(2-{[2-({(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}amino)ethyl]amino}-2-oxoethyl)-L-cystein;
S-{1-[6-(2-{(2S)-2-Amino-4-[{(1R)-1-[4-benzyl-1-(2,5-difluorphenyl)-1H-pyrazol-3-yl]-2,2-dimethylpropyl}(glycoloyl)amino]butanoyl}hydrazino)-6-oxohexyl]-2,5-dioxopyrrolidin-3-yl}-L-cystein;
N-[19-(3(R/S)-{[(2R)-2-Amino-2-carboxyethyl]sulfanyl}-2,5-dioxopyrrolidin-1-yl)-17-oxo-4,7,10,13-tetraoxa-16-azanonadecan-1-oyl]-R/S-{2-[(3-aminopropyl){(1R)-1-[1-benzyl-4-(2,5-difluorphenyl)-1H-pyrrol-2-yl]-2,2-dimethylpropyl}amino]-2-oxoethyl}homocystein;
S-{(3R1S)-1-[2-({(2S)-2-Amino-4-[-{(1R)-1-[1-benzyl-4-(2,5-difluorphenyl)-1H-pyrrol-2-yl]-2,2 dimethylpropyl}(glycoloyl)amino]butanoyl}amino)ethyl]-2,5-dioxopyrrolidin-3-yl}-L-cystein;
N-[19-(3(R/S)-{[(2R)-2-Amino-2-carboxyethyl]sulfanyl}-2,5-dioxopyrrolidin-1-yl)-17-oxo-4,7,10,13-tetraoxa-16-azanonadecan-1-oyl]-R/S-{2-[(3-aminopropyl){(1R)-1-[1-benzyl-4-(2,5-difluorphenyl)-1H-imidazol-2-yl]-2,2-dimethylpropyl}amino]-2-oxoethyl}homocystein;
S-[(3R/S)-1-(2-{[6-({2-[(3-Aminopropyl)-{(1R)-1-[1-benzyl-4-(2,5-difluorphenyl)-1H-pyrrol-2-yl]-2,2 dimethylpropyl}amino]-2-oxoethyl}sulfanyl)hexanoyl]amino}ethyl)-2,5-dioxopyrrolidin-3-yl]-L-cystein;
S-{1-[2-({[(1R,3S)-3-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)cyclopentyl]carbonyl}amino)ethyl]-2,5-dioxopyrrolidin-3-yl} -L-cystein;
S-(2-{[2-({(2S)-2-Amino-4-[-{(1R)-1-[1-benzyl-4-(2,5-difluorphenyl)-1H-pyrrol-2-yl]-2,2 dimethylpropyl}(glycoloyl)amino]butanoyl}amino)ethyl]amino}-2-oxoethyl)-L-cystein;
N⁶-(N-{(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl}-beta-alanyl)-N²-{N-[6-(3-{[(2R)-2-amino-2-carboxyethyl]sulfanyl}-2,5-dioxopyrrolidin-1-yl)hexanoyl]-L-valyl-L-alanyl}-L-lysin;
N-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-L-glutamin;
N⁶-(N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanyl)-L-lysin;
N-(3-Aminopropyl)-N-{(1R)-1-[4-benzyl-1-(2,5-difluorphenyl)-1H-pyrazol-3-yl]-2,2-dimethylpropyl} acetamid;
N-(3-Aminopropyl)-N-{(1R)-1-[4-benzyl-1-(2,5-difluorphenyl)-1H-pyrazol-3-yl]-2,2-dimethylpropyl}-2-methoxyacetamid;
N-(3-Aminopropyl)-N-{(1R)-1-[4-benzyl-1-(2,5-difluorphenyl)-1H-pyrazol-3-yl]-2,2-dimethylpropyl}-2,4-difluorobenzamid;
N-(3-Aminopropyl)-N-{(1R)-1-[4-benzyl-1-(2,5-difluorphenyl)-1H-pyrazol-3-yl]-2,2-dimethylpropyl}-4-methylbenzamid;
N-(3-Aminopropyl)-N-{(1R)-1-[4-benzyl-1-(2,5-difluorphenyl)-1H-pyrazol-3-yl]-2,2-dimethylpropyl}-2-ethoxyacetamid;
N-(3-Aminopropyl)-N-{(1R)-1-[1-benzyl-4-(2,5-difluorphenyl)-1H-pyrrol-2-yl]-2,2-dimethylpropyl}-3,3,3-trifluorpropanamid;
N-(3-Aminopropyl)-N-{(1R)-1-[1-benzyl-4-(2,5-difluorphenyl)-1H-pyrrol-2-yl]-2,2-dimethylpropyl}-4-fluorbenzamid;
N-(3-Aminopropyl)-N-{(1R)-1-[1-benzyl-4-(2,5-difluorphenyl)-1H-pyrrol-2-yl]-2,2-dimethylpropyl}acetamid;
N-(3-Aminopropyl)-N-{(1R)-1-[1-benzyl-4-(2,5-difluorphenyl)-1H-pyrrol-2-yl]-2,2-dimethylpropyl}-4-(trifluoromethyl)benzamid;
N-(3-Aminopropyl)-N-{(1R)-1-[4-benzyl-1-(2,5-difluorphenyl)-1H-pyrazol-3-yl]-2,2-dimethylpropyl}-2-ethoxyacetamid;
N-(3-Aminopropyl)-N-{(1R)-1-[4-benzyl-1-(2,5-difluorphenyl)-1H-pyrazol-3-yl]-2,2-dimethylpropyl}-2-ethoxyacetamid
(2S)-2-Amino-4-[{(1R)-1-[1-benzyl-4-(2,5-difluorphenyl)-1H-pyrrol-2-yl]-2,2-dimethylpropyl} (glycoloyl)amino]butansäure;
(2S)-2-amino-N-(2-aminoethyl)-4-[{(1R)-1-[1-benzyl-4-(2,5-difluorphenyl)-1H-pyrrol-2-yl]-2,2-dimethylpropyl}(glycoloyl)amino]butanamid;
4-[(2-{[2-({(2S)-2-Amino-4-[{(1R)-1-[1-benzyl-4-(2,5-difluorphenyl)-1H-pyrrol-2-yl]-2,2 dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure;
4-[(2-{[2-({(2S)-2-Amino-4-[-{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-2-{[(2R)-2-amino-2-carboxyethyl]sulfanyl}-4-oxobutansäure;
N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-beta-alanin;
N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-L-serin;
N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}-L-alanin;
N-{(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}glycin;
N-(3-aminopropyl)-N-{(1R)-1-[1-benzyl-4-(2,5-difluorphenyl)-1H-pyrrol-2-yl]-2,2-dimethylpropyl}-4-methylbenzamid;
N-(3-aminopropyl)-N-{(1R)-1-[1-benzyl-4-(2,5-difluorphenyl)-1H-pyrrol-2-yl]-2,2-dimethylpropyl}-4-(methylsulfanyl)benzamid;
(2S)-N-(3-aminopropyl)-N-{(1R)-1-[1-benzyl-4-(2,5-difluorphenyl)-1H-pyrrol-2-yl]-2,2-dimethylpropyl}-2-hydroxypropanamid;
N-(3-aminopropyl)-N-{(1R)-1-[1-benzyl-4-(2,5-difluorphenyl)-1H-pyrrol-2-yl]-2,2-dimethylpropyl}-2-(methylsulfanyl)acetamid;
(2S)-N-(3-aminopropyl)-N-{(1R)-1-[4-benzyl-1-(2,5-difluorphenyl)-1H-pyrazol-3-yl]-2,2-dimethylpropyl} -2-hydroxypropanamid;
Methyl-4-[(3-aminopropyl){(1R)-1-[1-benzyl-4-(2,5-difluorphenyl)-1H-pyrrol-2-yl]-2,2-dimethylpropyl} amino] -4-oxobutanoat;
4-[(3-Aminopropyl){(1R)-1-[1-benzyl-4-(2,5-difluorphenyl)-1H-pyrrol-2-yl]-2,2-dimethylpropyl} amino] -4-oxobutansäure;
(2R)-22-[(3R/S)-3-{[(2R)-2-Amino-2-carboxyethyl]sulfanyl}-2,5-dioxopyrrolidin-1-yl]-2-[({2-[(3-aminopropyl){(1R)-1-[1-benzyl-4-(2,5-difluorphenyl)-1H-pyrrol-2-yl]-2,2-dimethylpropyl}amino]-2-oxoethyl}sulfanyl)methyl]-4,20-dioxo-7,10,13,16-tetraoxa-3,19-diazadocosan-1-säure;
4-amino-N-(3-aminopropyl)-N-{(1R)-1-[4-benzyl-1-(2,5-difluorphenyl)-1H-pyrazol-3-yl]-2,2-dimethylpropyl} benzamid;
N-Acetyl-S-{2-[(3-aminopropyl){(1R)-1-[1-benzyl-4-(2,5-difluorphenyl)-1H-pyrrol-2-yl]-2,2-dimethylpropyl}amino]-2-oxoethyl}-L-cystein;
N-Acetyl-S-[2-([3-(L-alanylamino)propyl]{(1R)-1-[1-benzyl-4-(2,5-difluorphenyl)-1H-pyrrol-2-yl]-2,2-dimethylpropyl} amino)-2-oxoethyl] -L-cystein;
(2S)-N-(3-aminopropyl)-N-{(1R)-1-[1-benzyl-4-(2,5-difluorphenyl)-1H-pyrrol-2-yl]-2,2-dimethylpropyl}tetrahydrofuran-2-carboxamid;
3-({2-[(3-Aminopropyl){(1R)-1-[1-benzyl-4-(2,5-difluorphenyl)-1H-pyrrol-2-yl]-2,2-dimethylpropyl} amino] -2-oxoethyl} sulfanyl)propansäure;
S-{2-[(3-Aminopropyl){(1R)-1-[1-benzyl-4-(2,5-difluorphenyl)-1H-pyrrol-2-yl]-2,2-dimethylpropyl}amino]-2-oxoethyl}homocystein;
4-amino-N-(3-aminopropyl)-N-{(1R)-1-[1-benzyl-4-(2,5-difluorphenyl)-1H-pyrrol-2-yl]-2,2-dimethylpropyl} benzamid;
4-[(2-{[(2R)-2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)-2-carboxyethyl] amino} -2-oxoethyl)amino]-3-{[(2R)-2-amino-2-carboxyethyl]sulfanyl}-4-oxobutansäure;
4-[(2-{[(2R)-2-({(2S)-2-Amino-4-[{(1R)-1-[1-benzyl-4-(2,5-difluorphenyl)-1H-pyrrol-2-yl]-2,2-dimethylpropyl} (glycoloyl)amino]butanoyl} amino)-2-carboxyethyl] amino} -2-oxoethyl)amino]-2-{[(2R)-2-amino-2-carboxyethyl]sulfanyl}-4-oxobutansäure.

60. Pharmazeutische Zusammensetzung umfassend ein Konjugat nach einem oder mehreren der Ansprüche 1 bis 41 oder eine Verbindung nach den Ansprüchen 46-59 in Kombination mit einem inerten, nicht-toxischen, pharmazeutisch geeigneten Hilfsstoff.

61. Konjugat nach einem oder mehreren der Ansprüche 1 bis 41 oder Verbindung nach den Ansprüche 46-59 zur Verwendung in einem Verfahren Behandlung und/oder Prophylaxe von Krankheiten.

62. Konjugat nach einem oder mehreren der Ansprüche 1 bis 41 oder Verbindung nach den Ansprüchen 46-59 zur Verwendung zur Verwendung in einem Verfahren zur Behandlung von hyperproliferativen und/oder angiogenen Erkrankungen.

63. Konjugat nach einem oder mehreren der Ansprüche 1 bis 41 oder Verbindung nach Ansprüchen 46-59, wobei R^{3a} bzw. R³ -L-BINDER oder eine substituierte Alkyl-, Aryl- oder Heteroaryl-Gruppe darstellt,
bevorzugt -L-#1 oder eine C₁₋₁₀-Alkyl-, C₆₋₁₀-Aryl- oder C₆₋₁₀-Aralkyl-Gruppe oder C₅₋₁₀-Heteroalkyl-, die mit -OH, O-Alkyl, SH, S-Alkyl, O-CO-Alkyl, O-CO-NH-Alkyl, NH-CO-Alkyl, NH-CO-NH-Alkyl, S(O)ₙ-Alkyl, SO₂-NH-Alkyl, NH-Alkyl, N(Alkyl)2, NH₂ oder-(CH₂)₀₋₃Z, wobei Z -H, Halogen, -OY³, -SY³, -NHY³, -CO-NY¹Y², oder -CO-OY³ darstellt, mit Y¹ und Y² unabhängig voneinander H, NH₂, oder -(CH₂)₀₋₃Z' darstellen, und Y³ H oder -(CH₂)₀₋₃Z' darstellt, wobei Z' H, SO₃H, NH₂ oder COOH darstellt,
substituiert sein kann (wobei "Alkyl" bevorzugt C₁₋₁₀-Alkyl bedeutet).

64. Konjugat nach einem oder mehrerer der Ansprüche 1 bis 41 oder Verbindung nach einem der Ansprüche 46 bis 59 zur Verwendung in einem Verfahren zur Behandlung und/oder Prophylaxe von hyperproliferativen und/oder angiogenen Erkrankungen bei Menschen und Tieren unter Verwendung einer wirksamen Menge.

65. Antikörper-Konjugat gemäß einer der folgenden Formeln, wobei n eine Zahl von 1 bis 20 ist und AK1A bzsw. AK2A einen Antikörper darstellen: 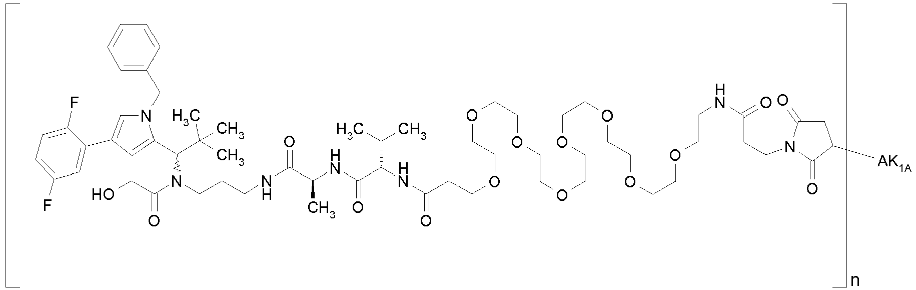 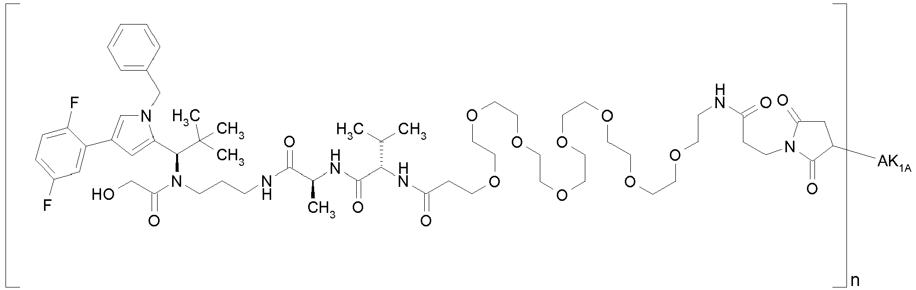 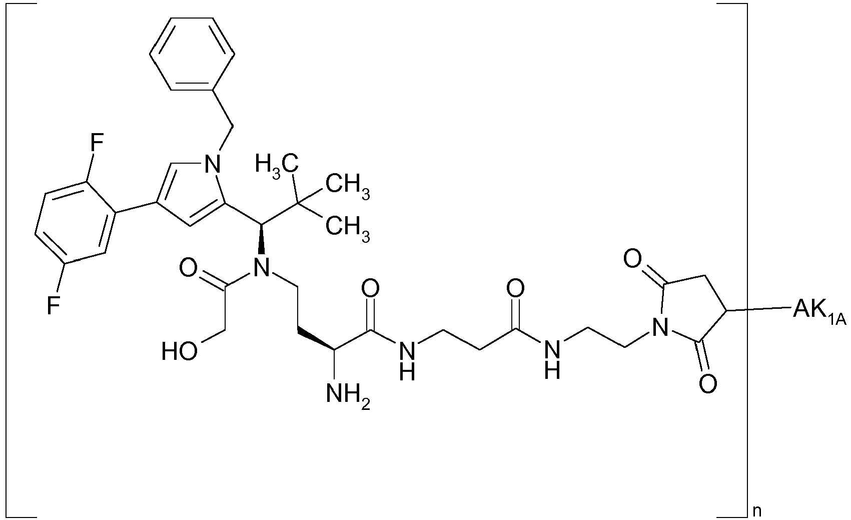 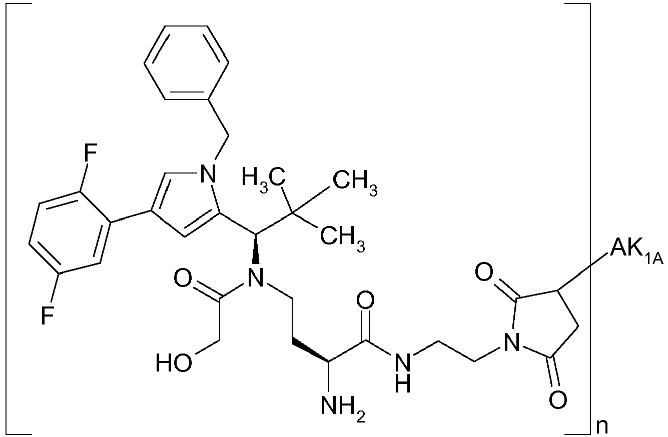 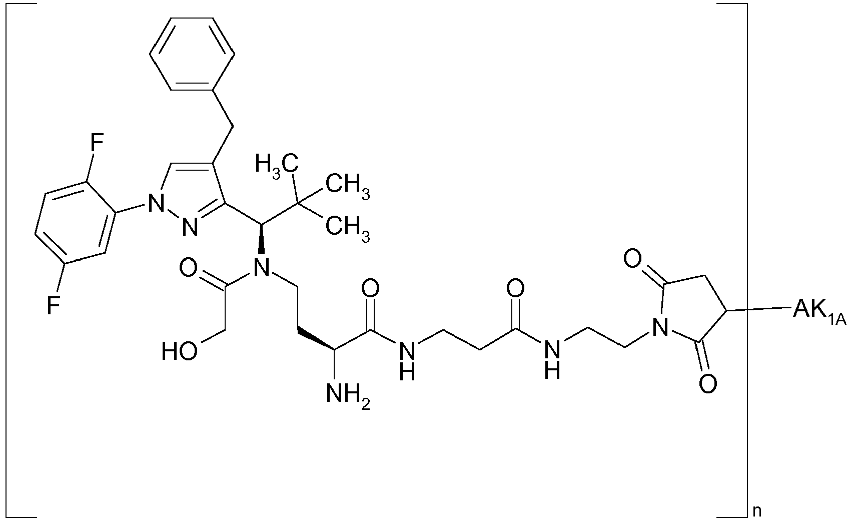 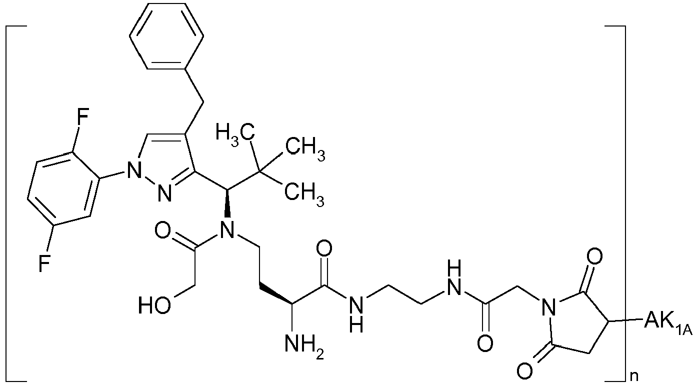 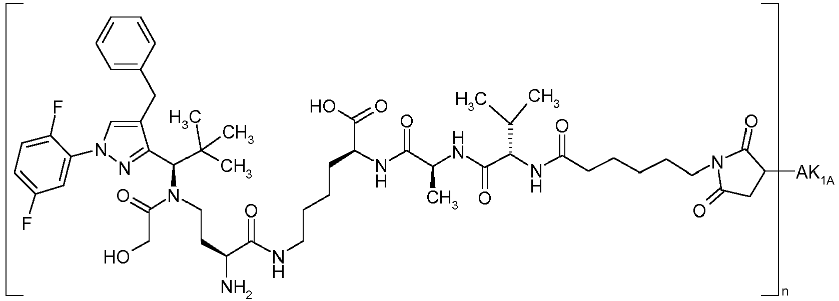 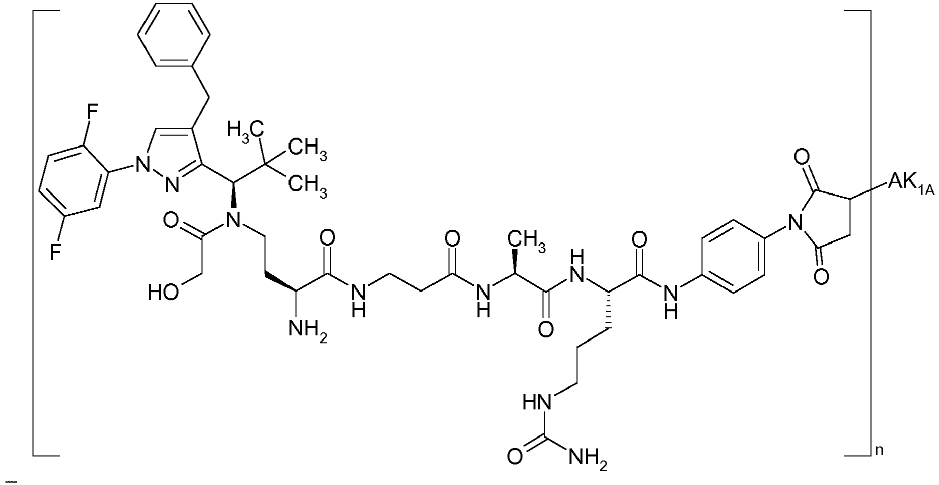 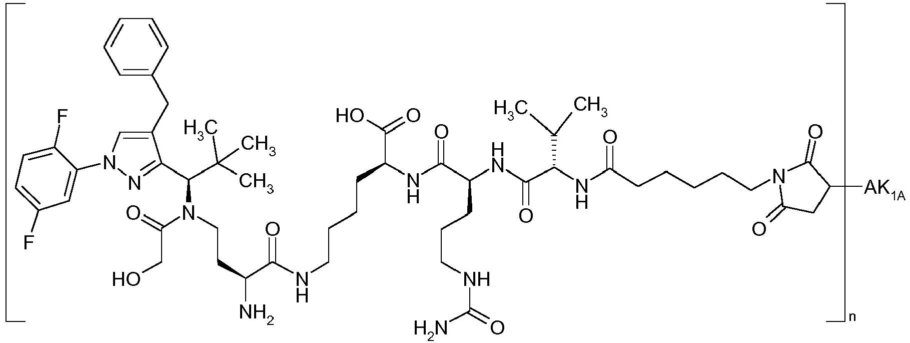 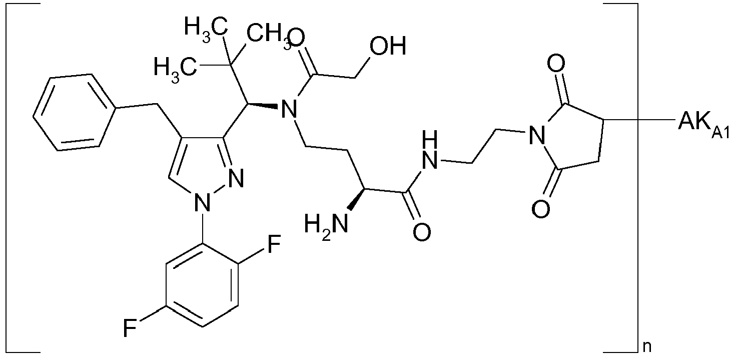 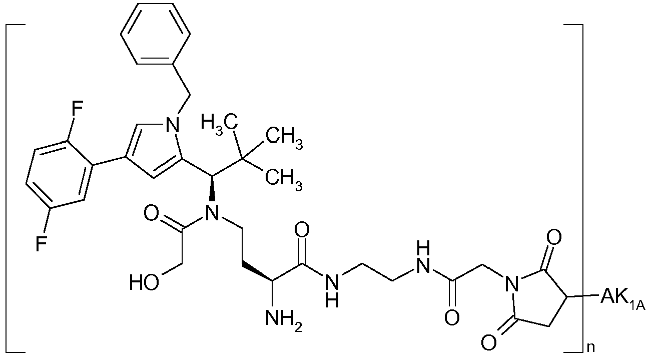 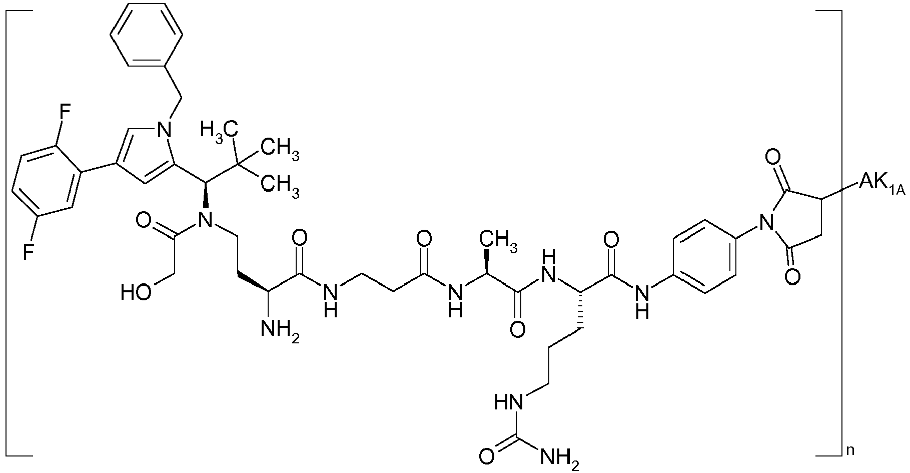 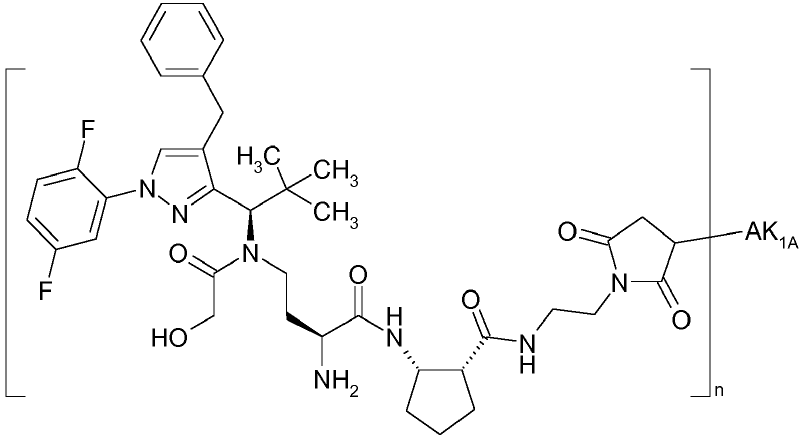 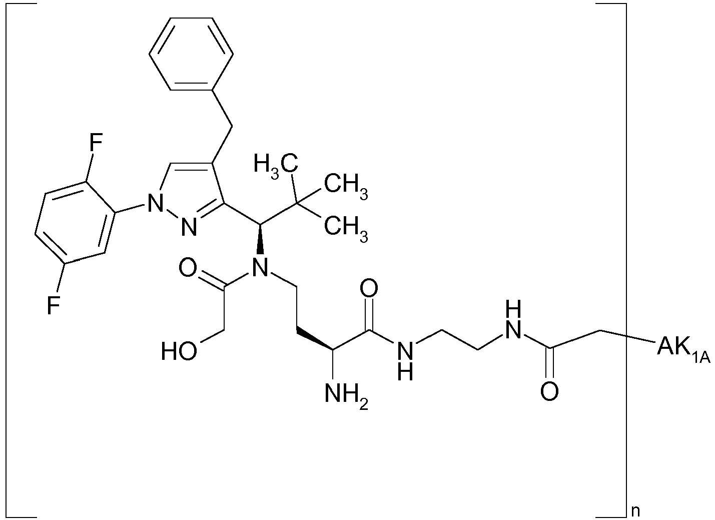 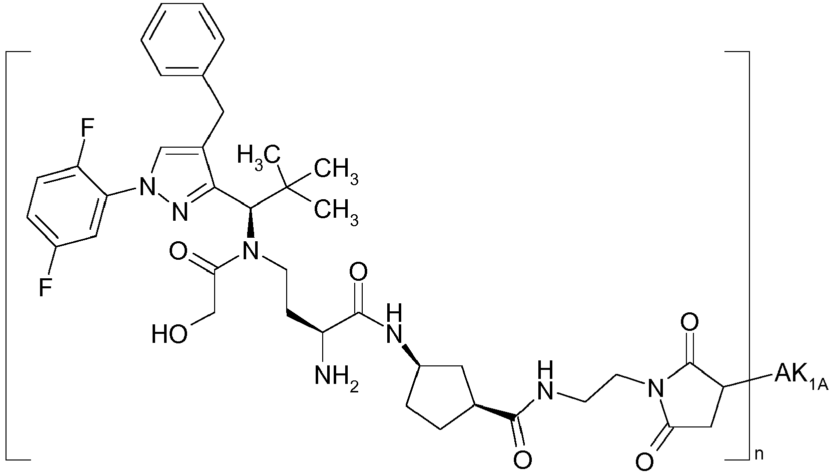 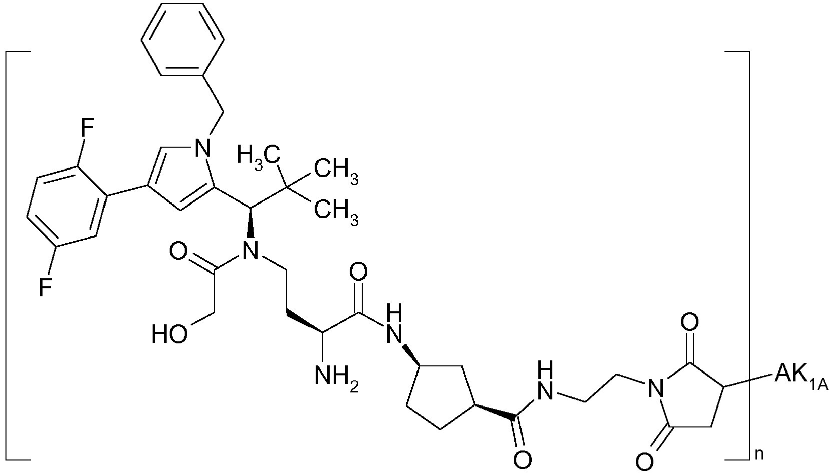 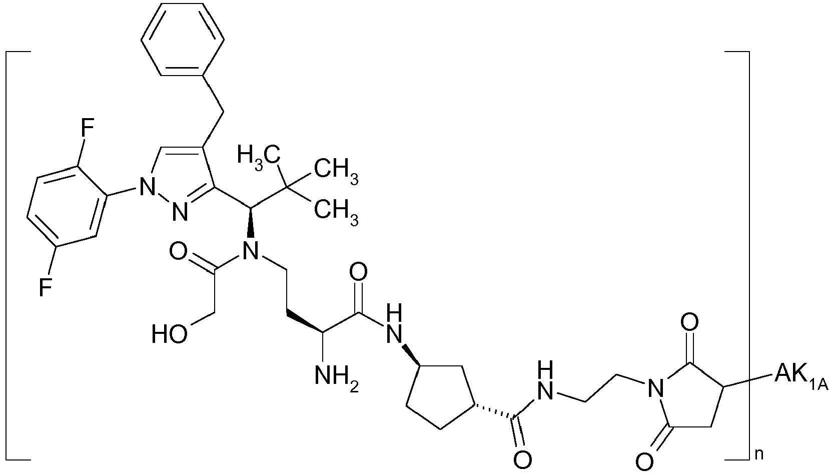 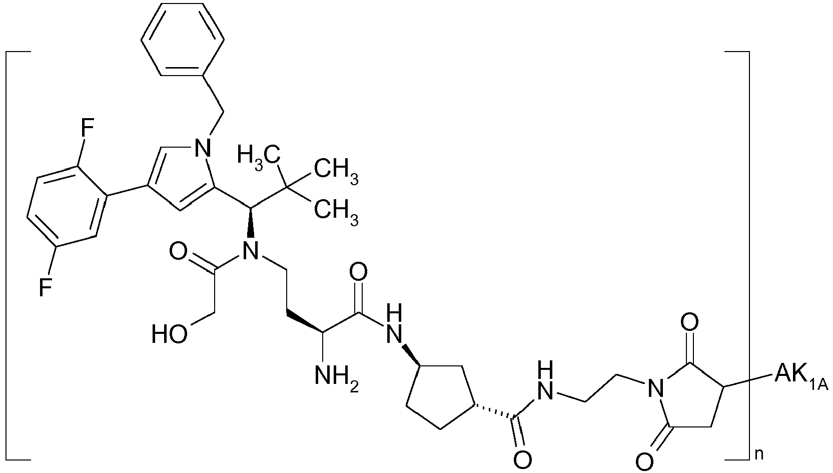 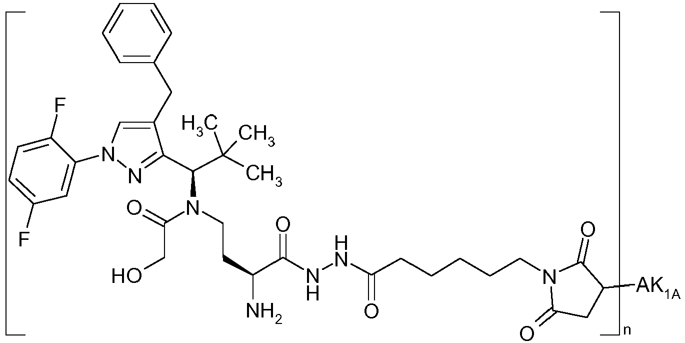 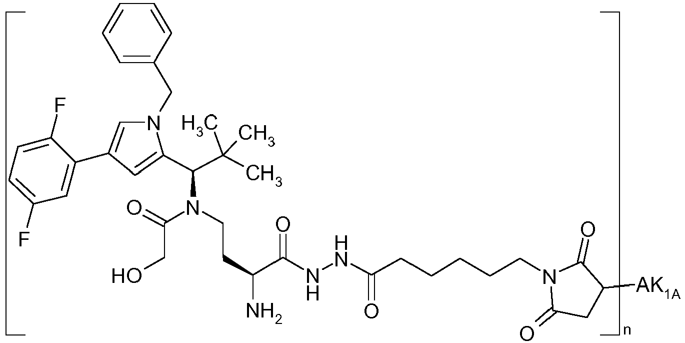 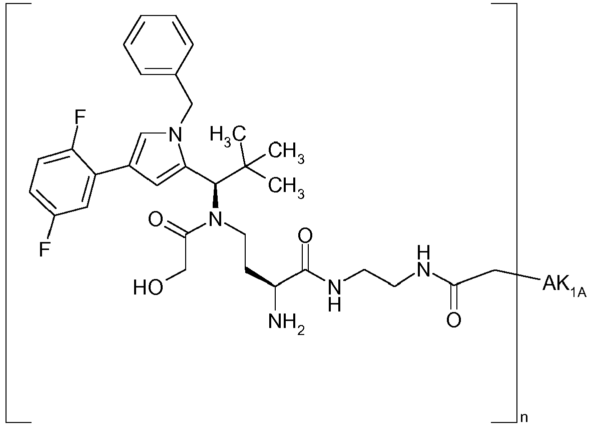 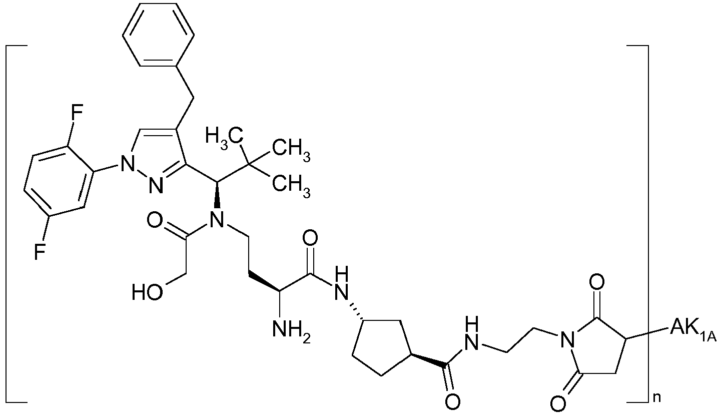 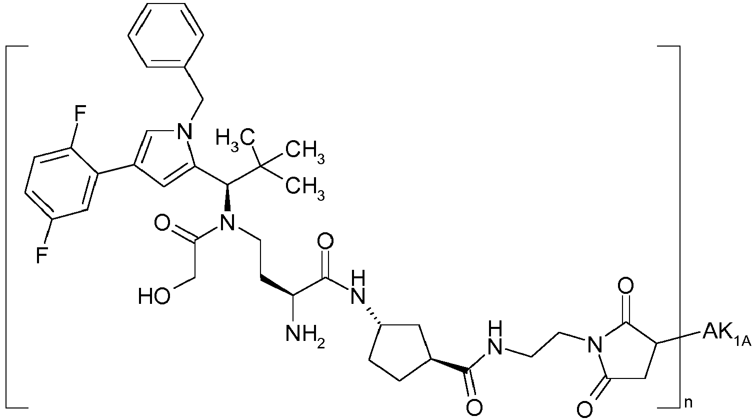 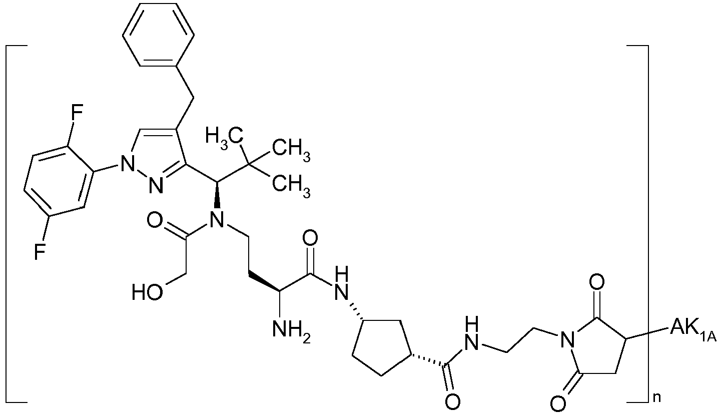 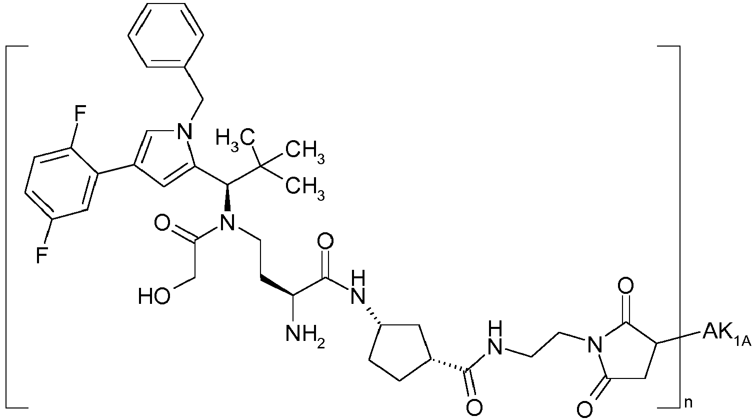 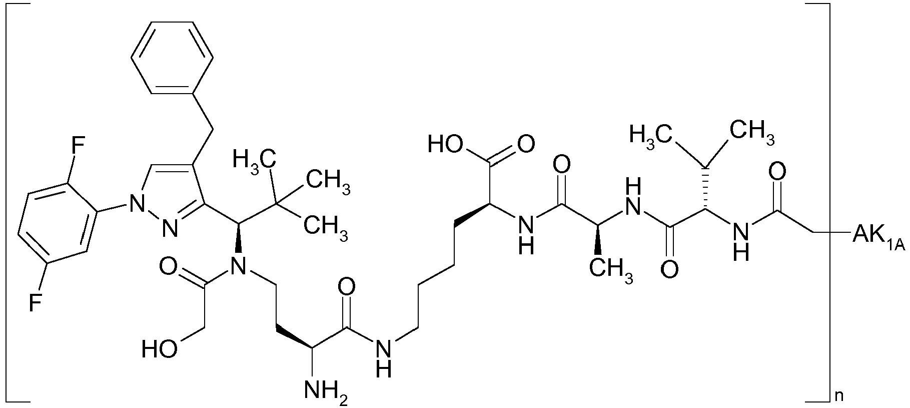 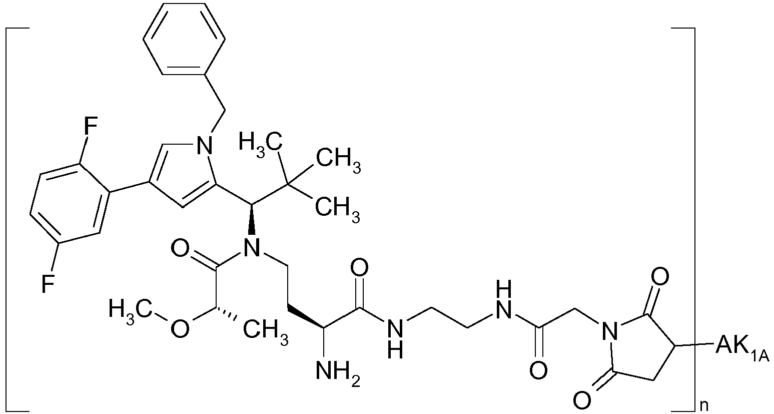 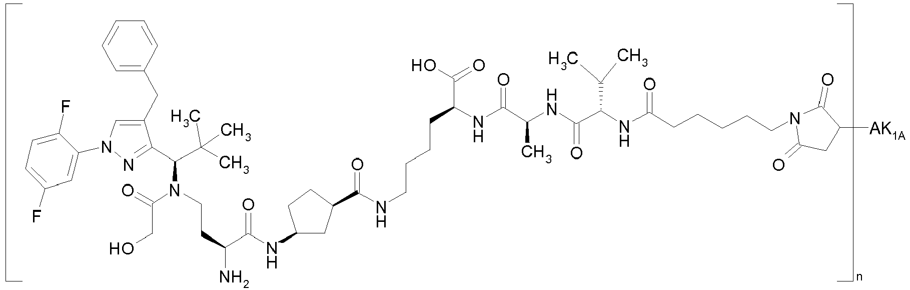 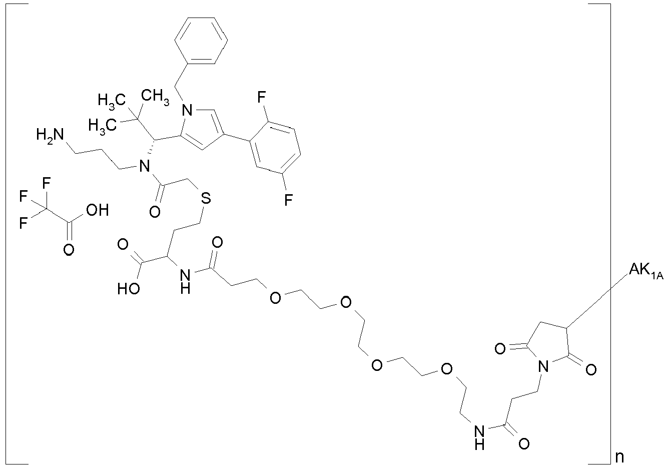 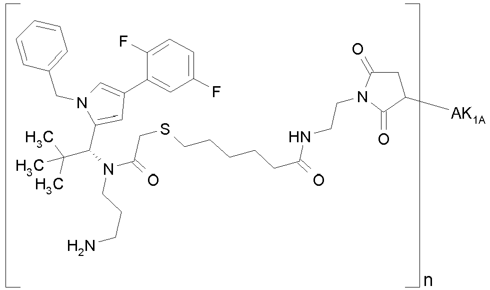 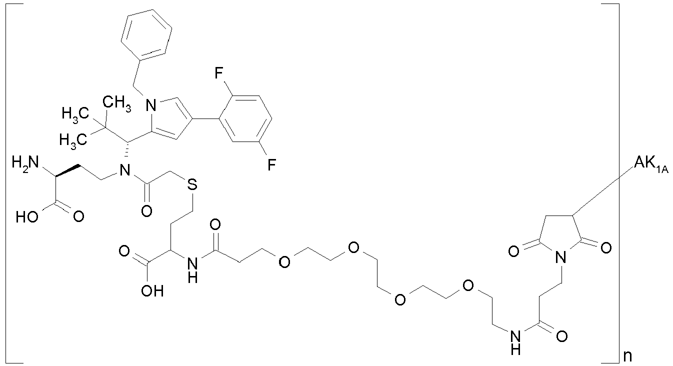 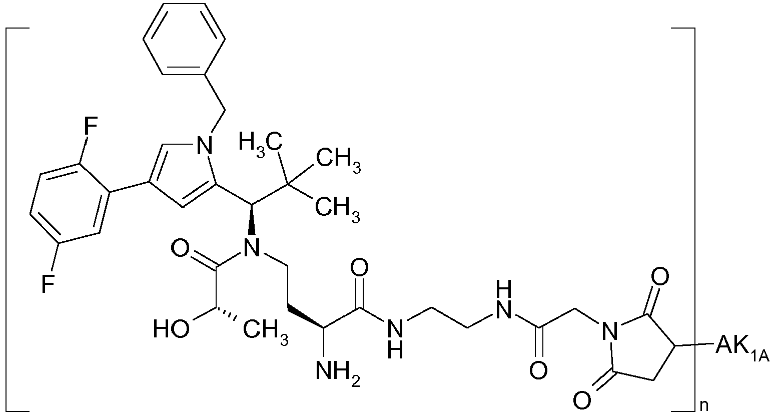 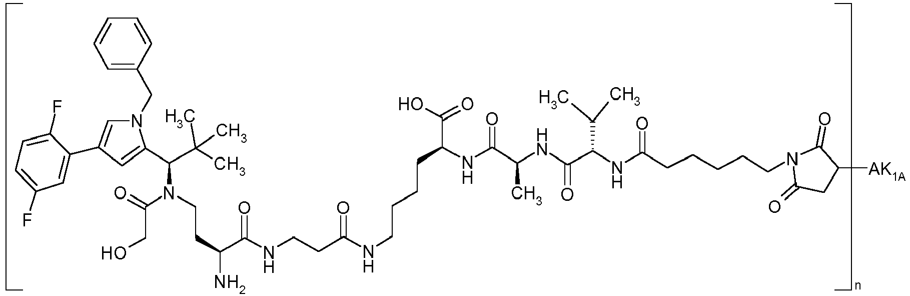 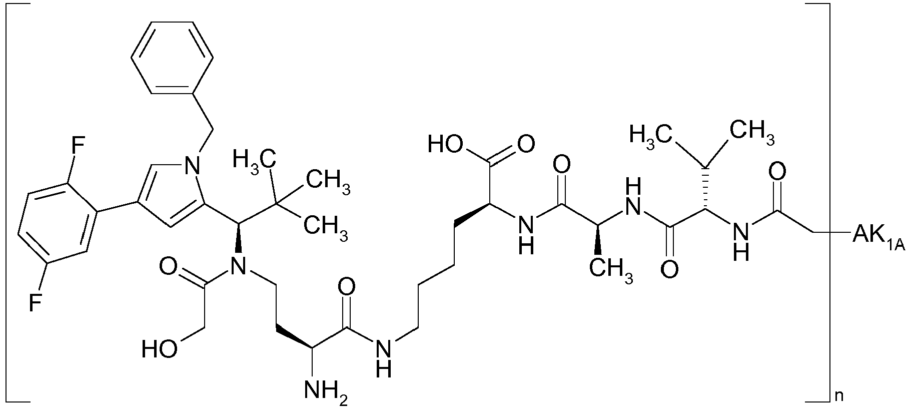 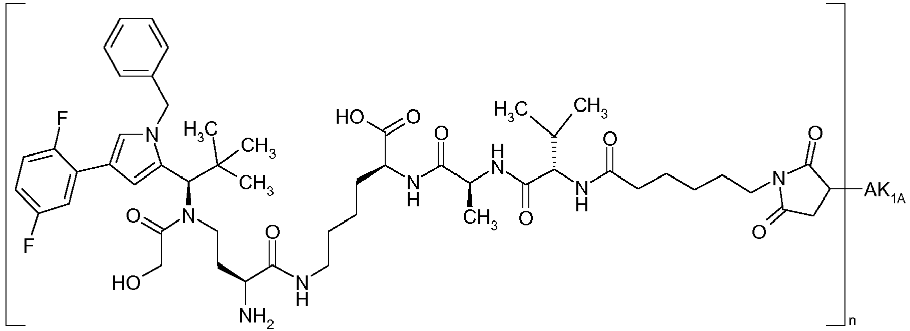 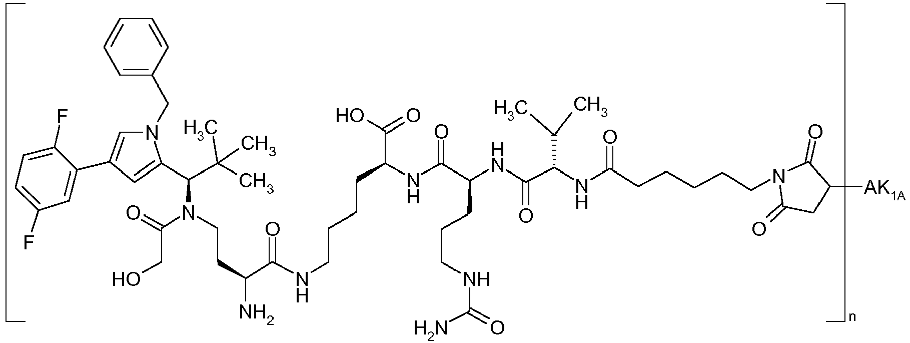 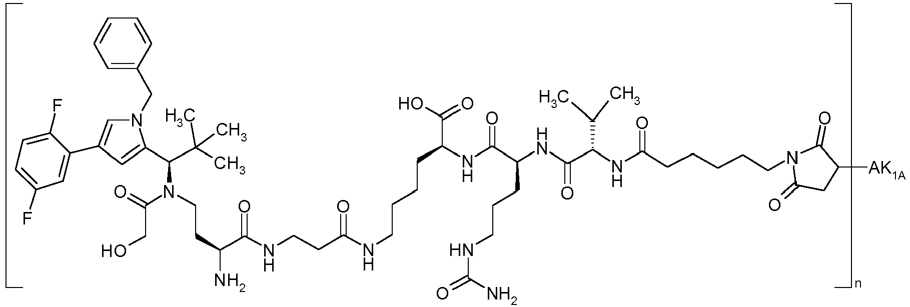 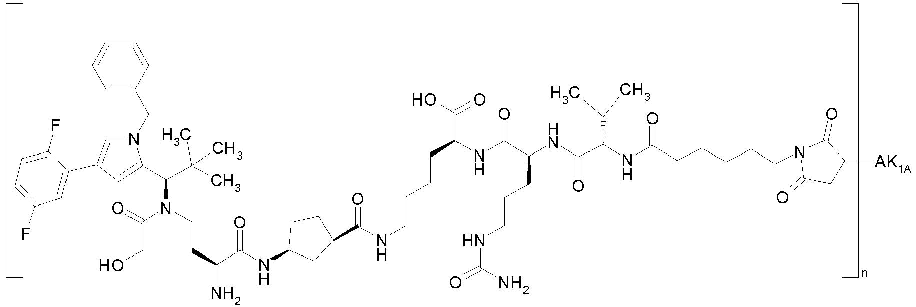 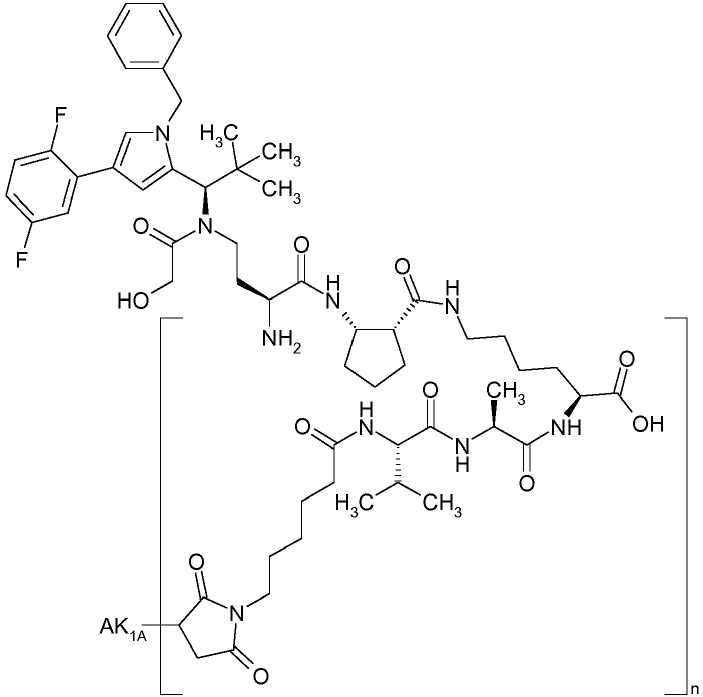 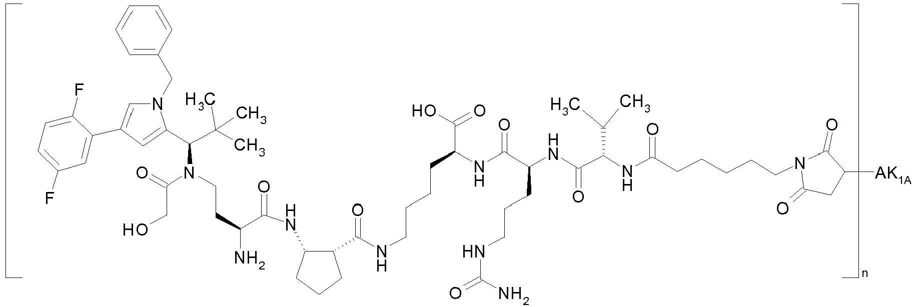 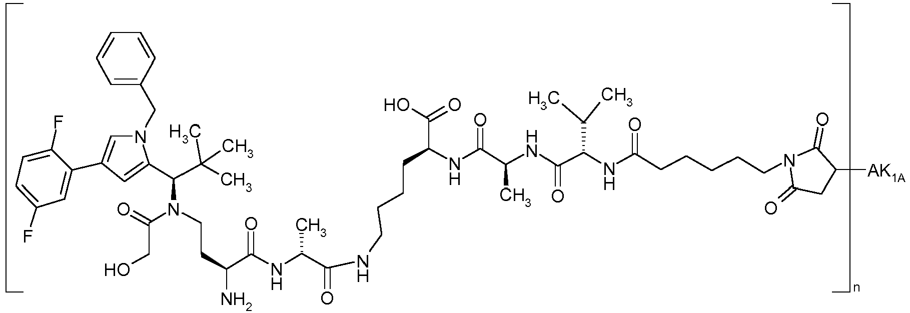 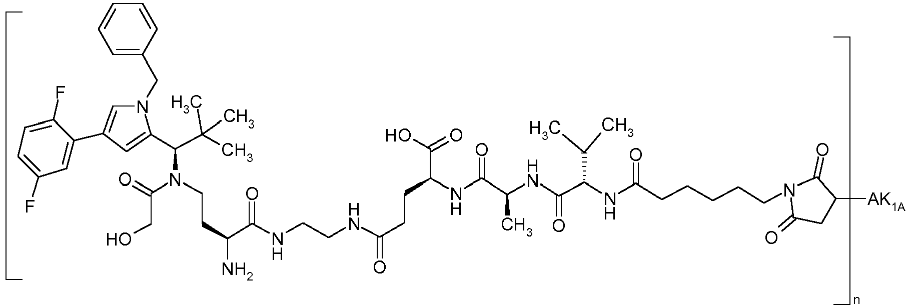 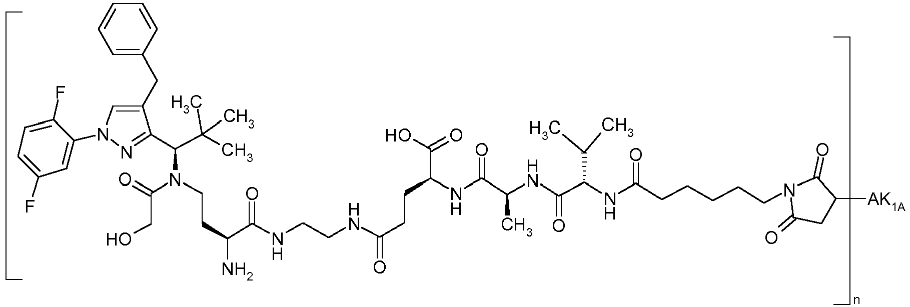 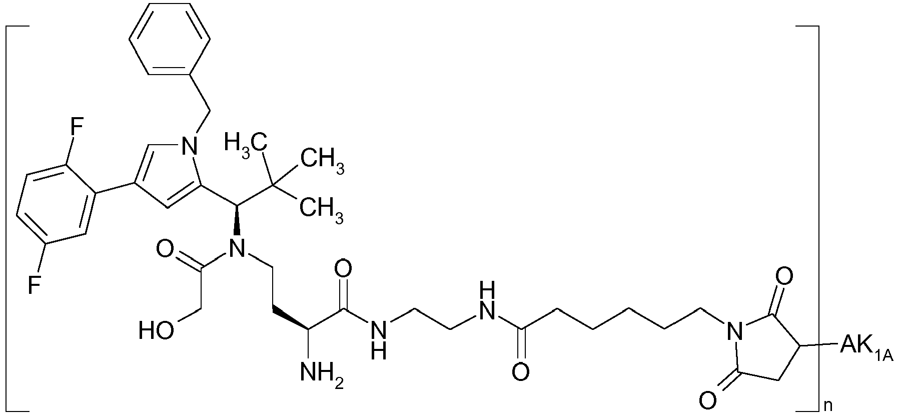 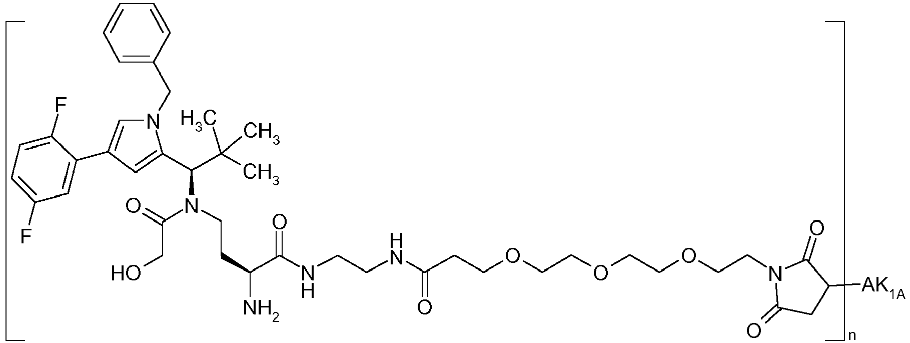 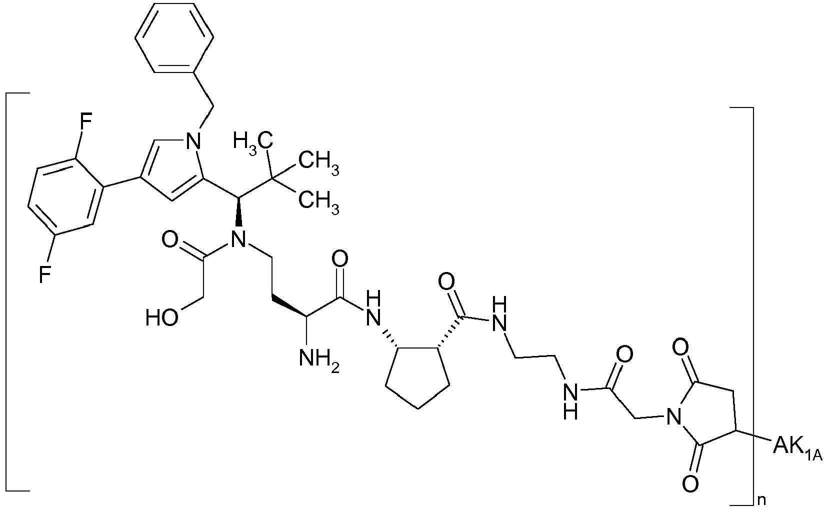 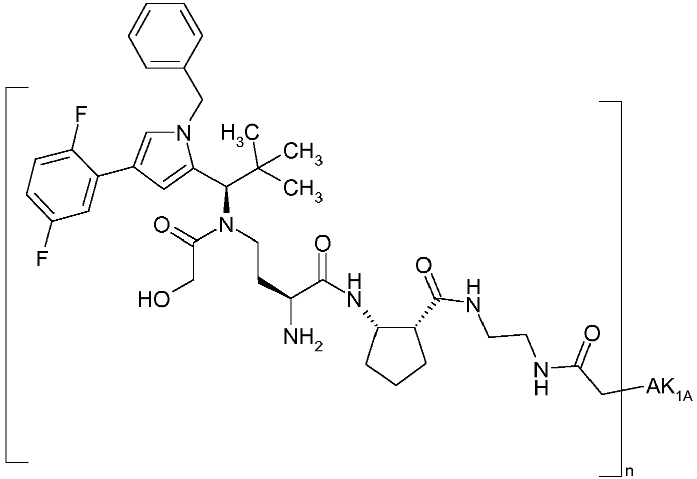 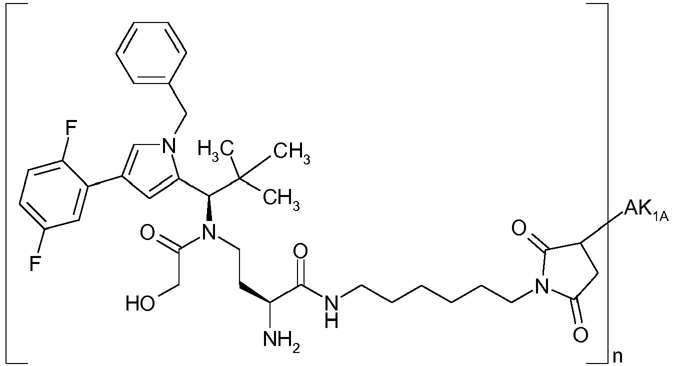 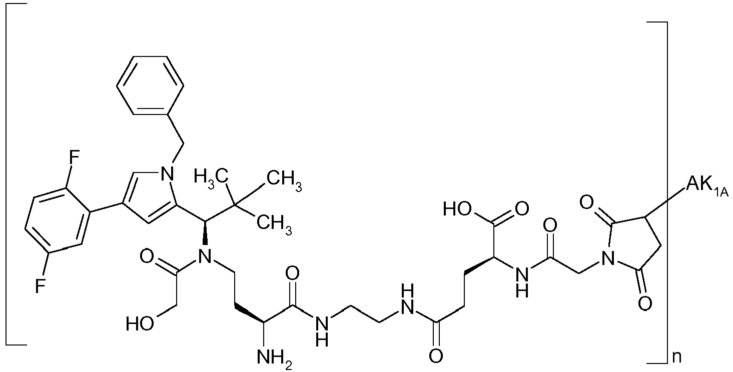 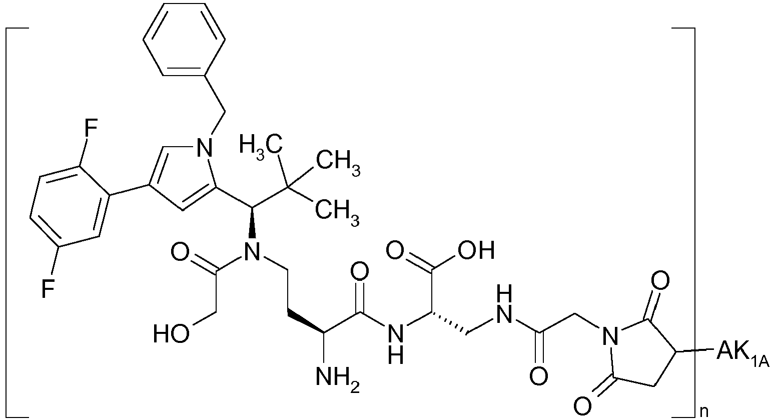 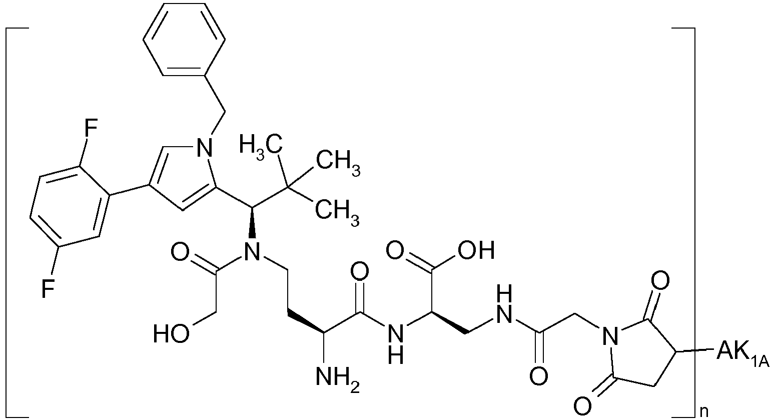 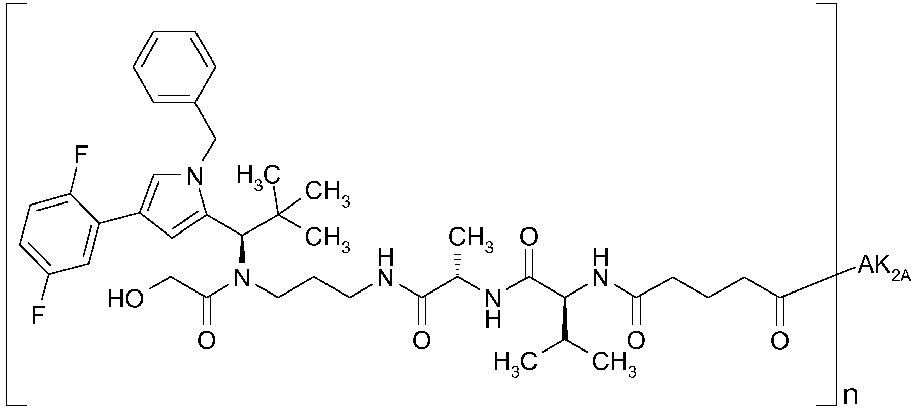 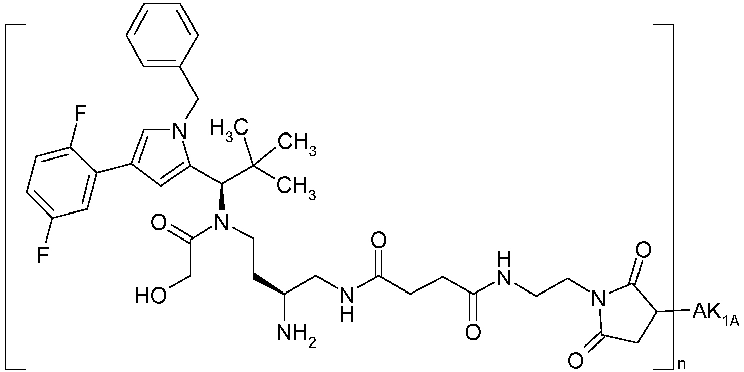 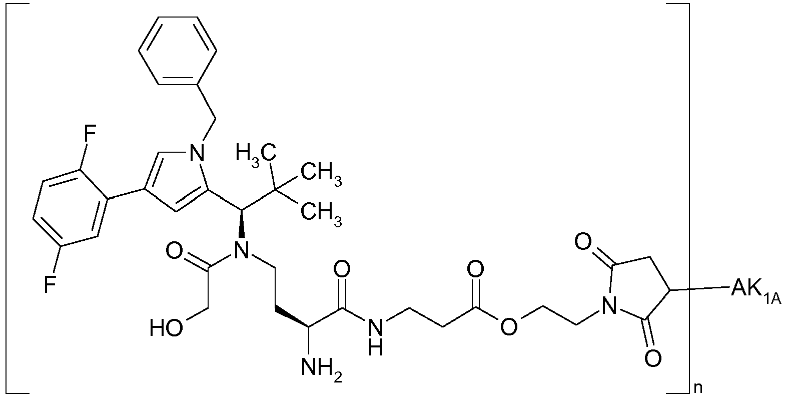 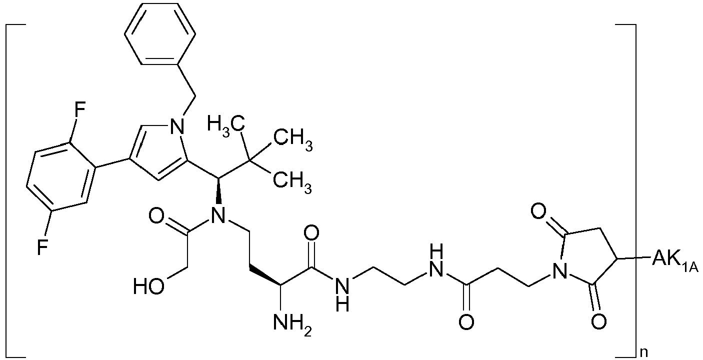 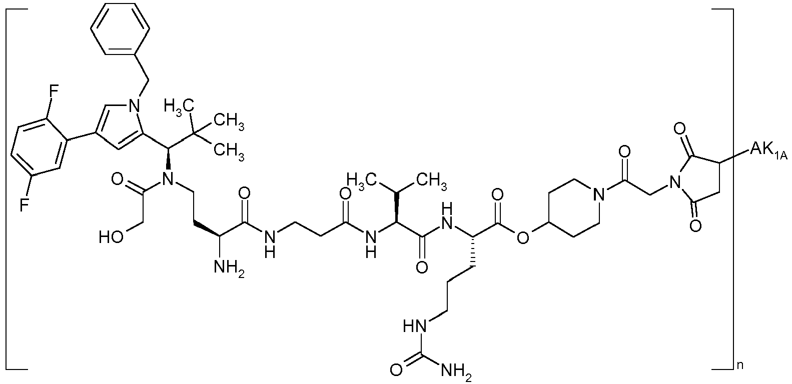 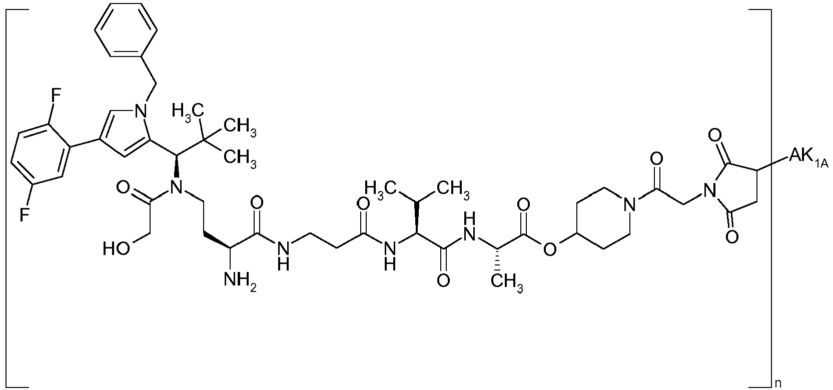 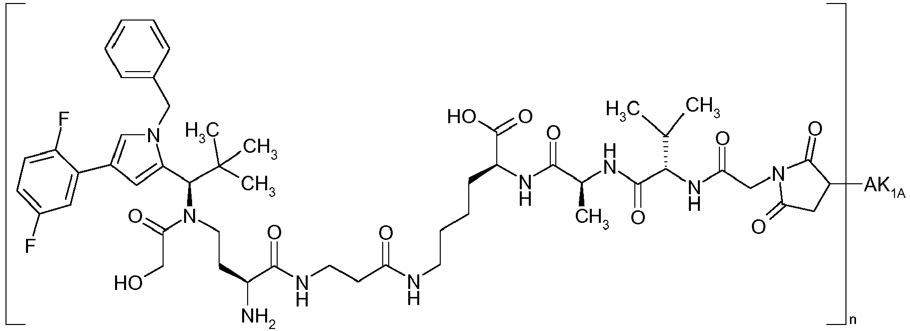 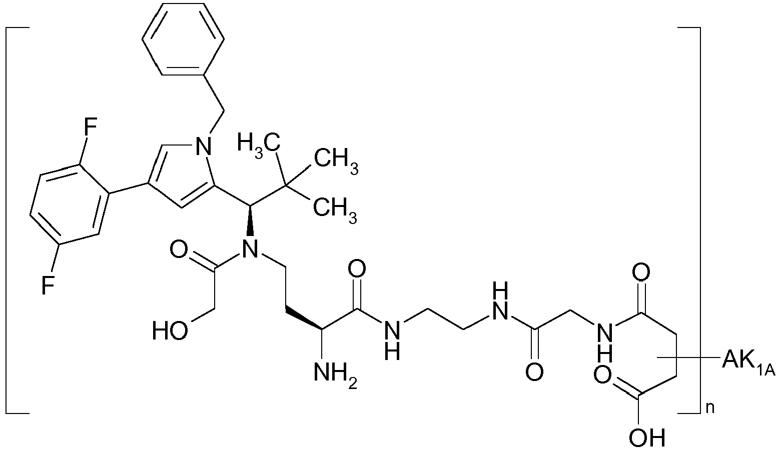 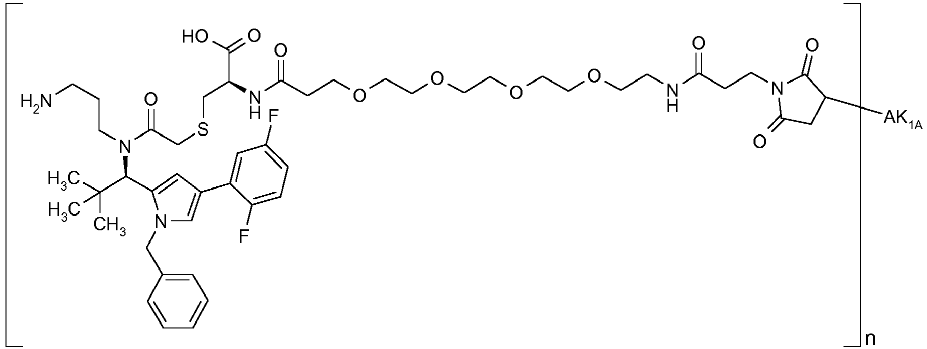 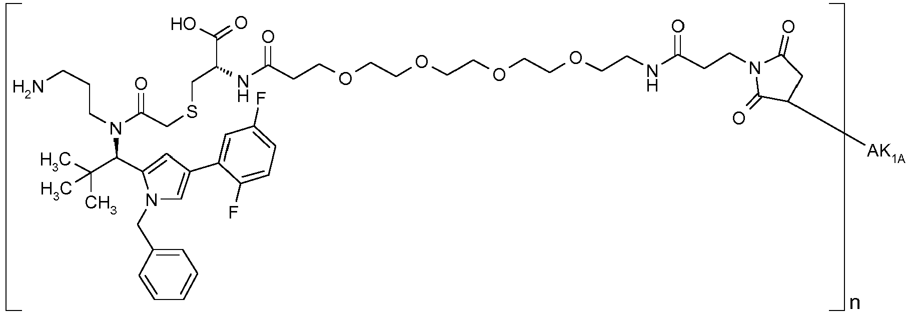 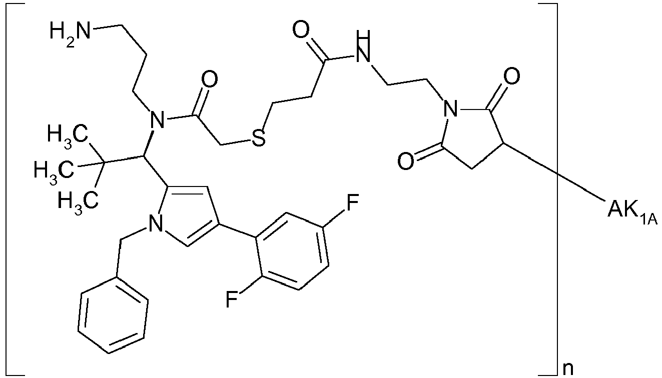 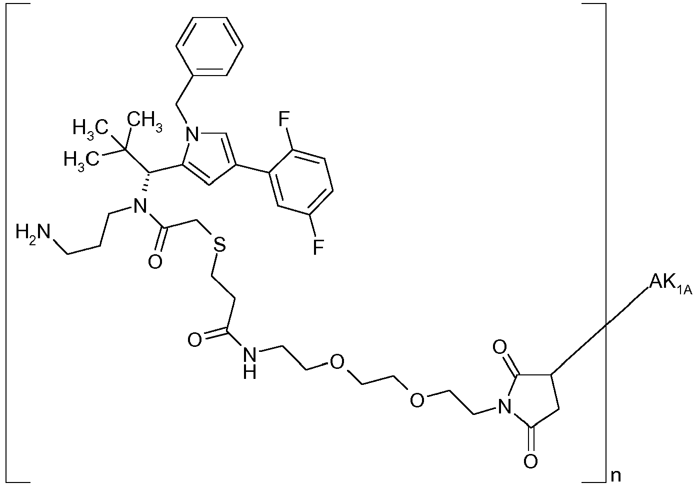 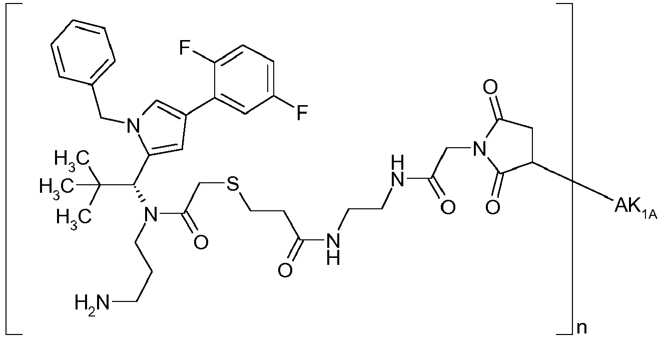 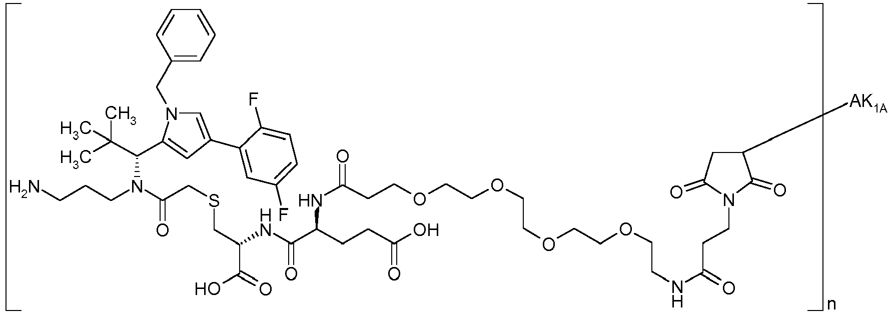 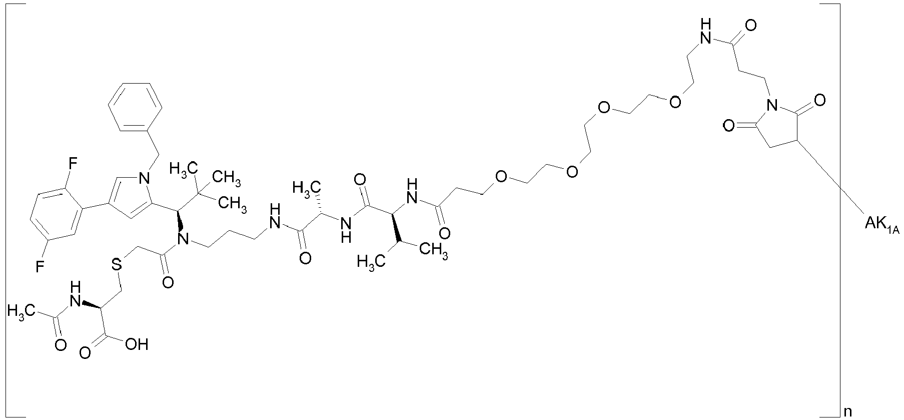 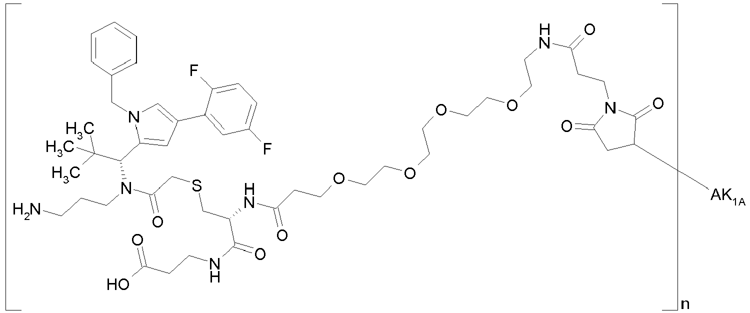 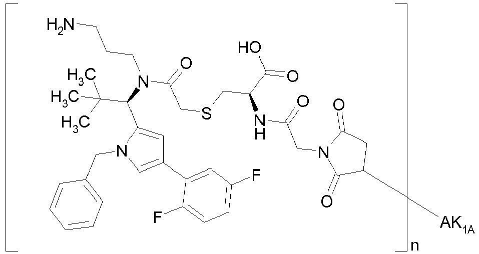 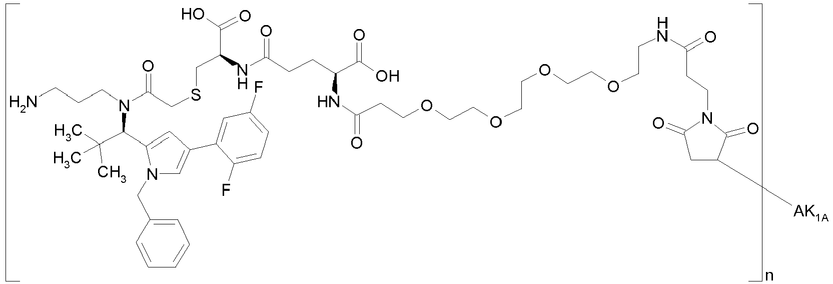 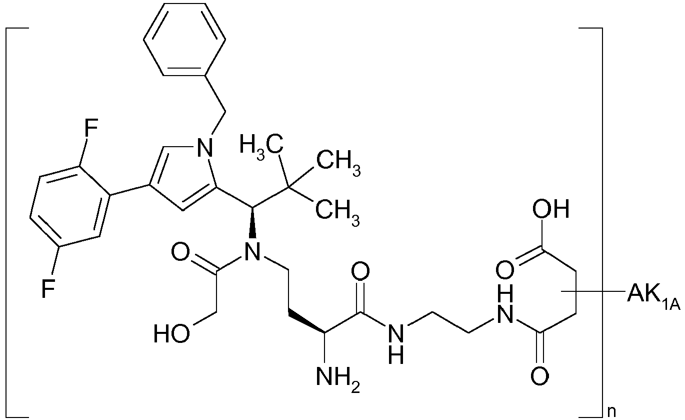 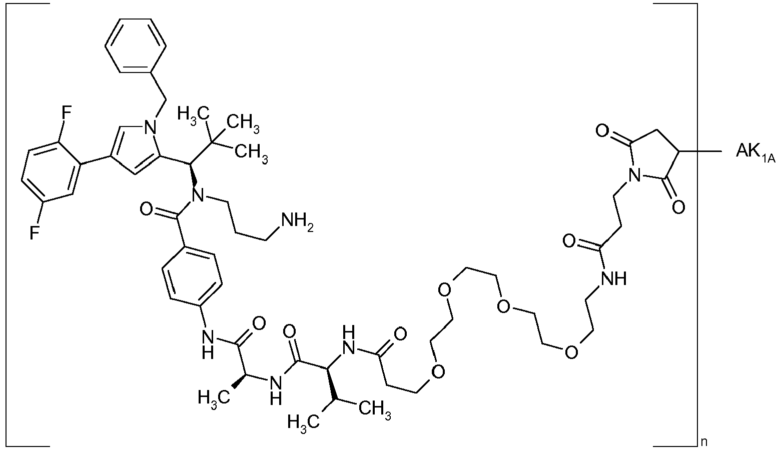 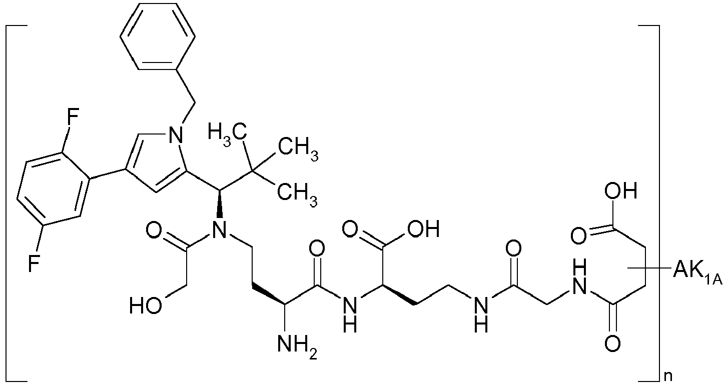 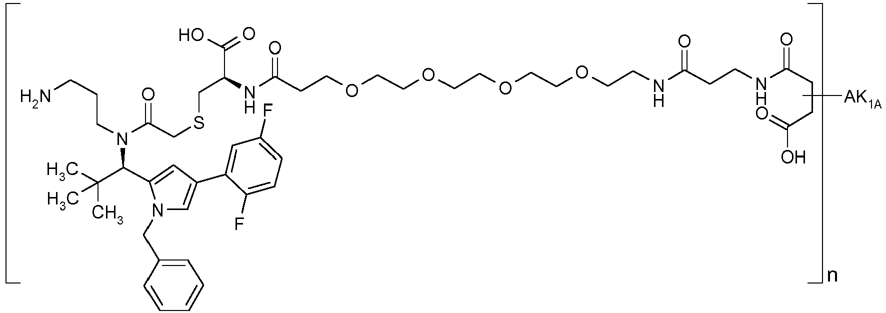 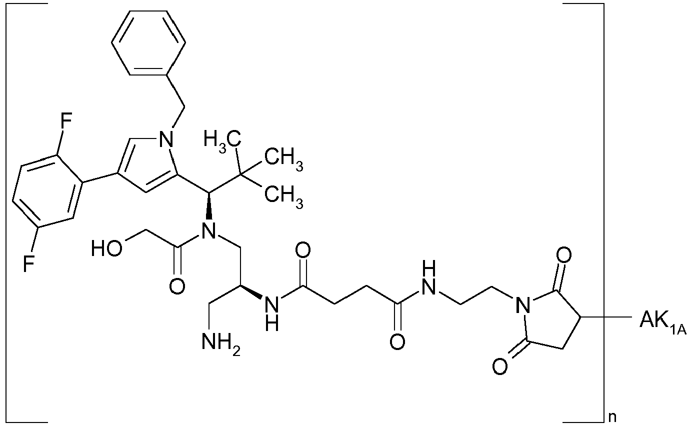 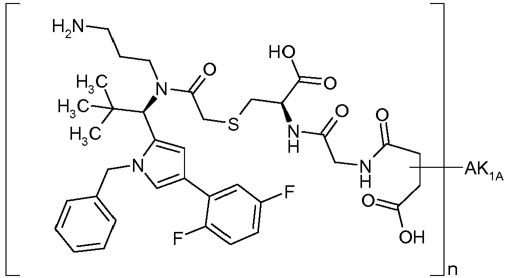 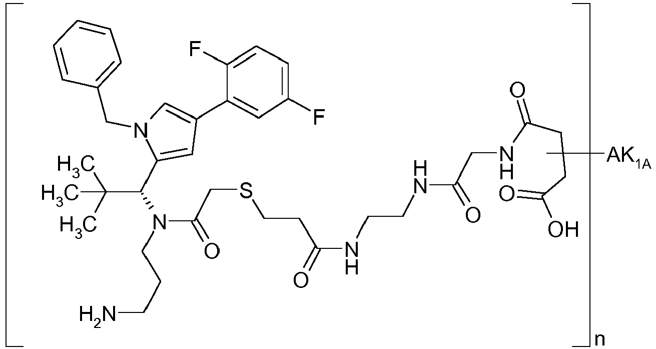 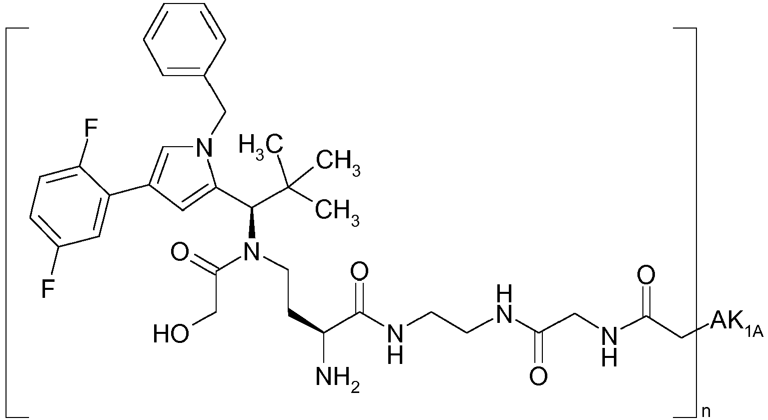 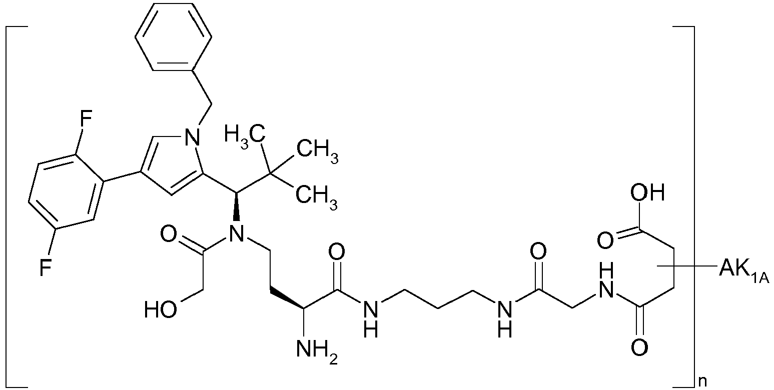 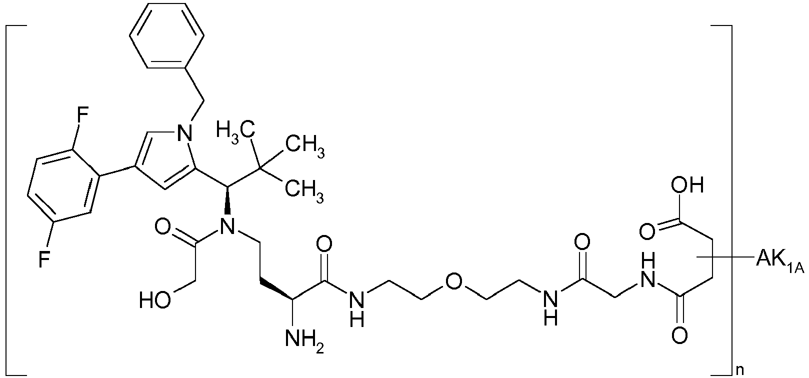 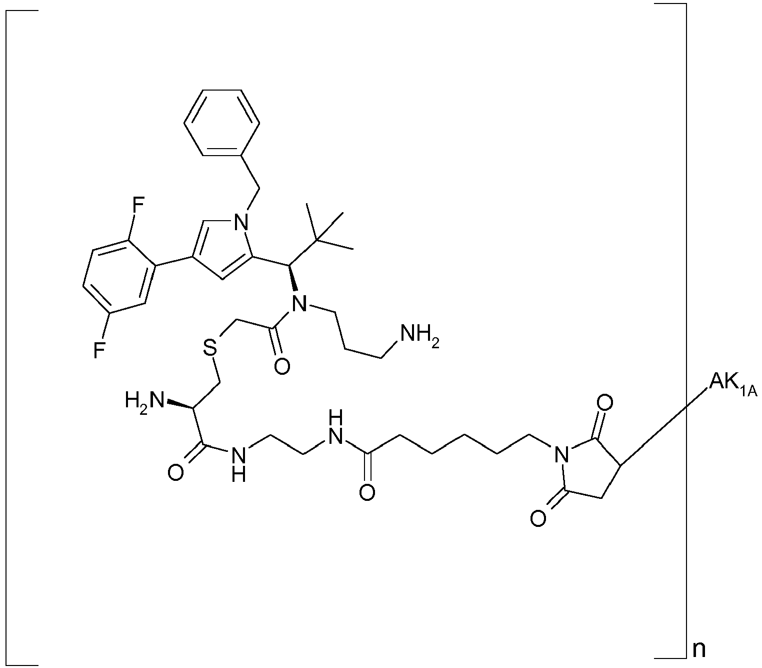 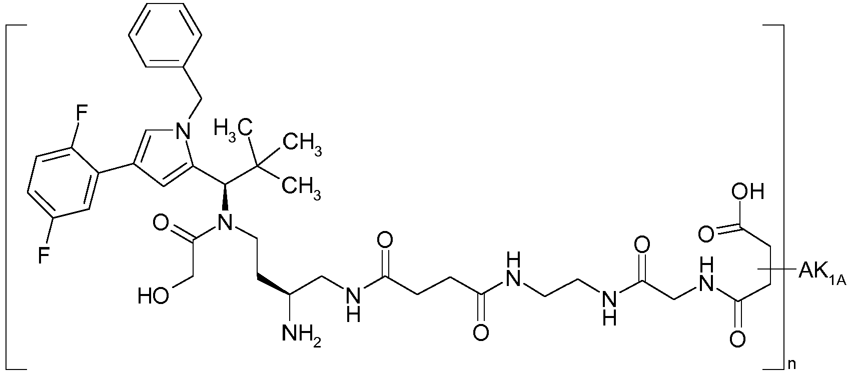 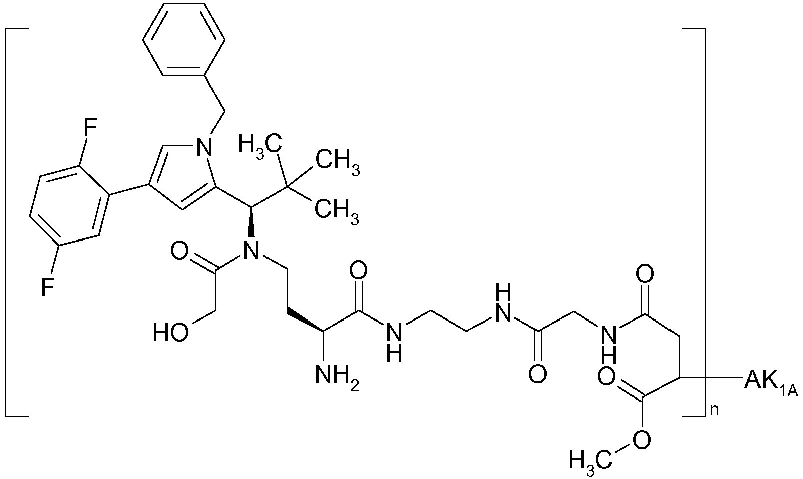 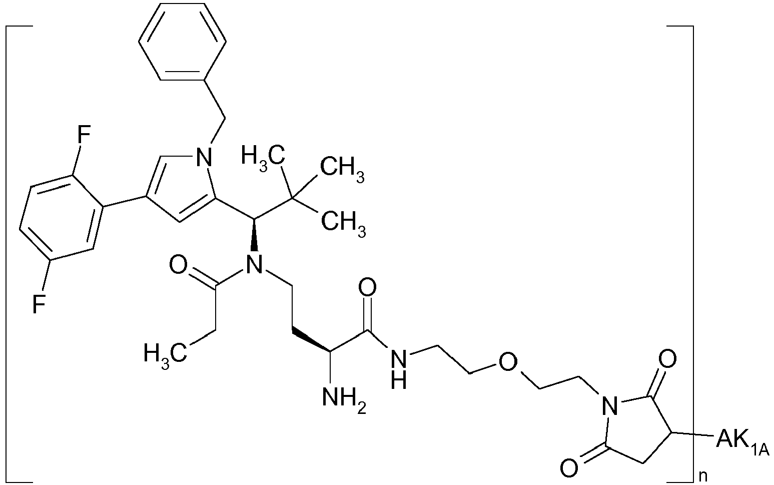 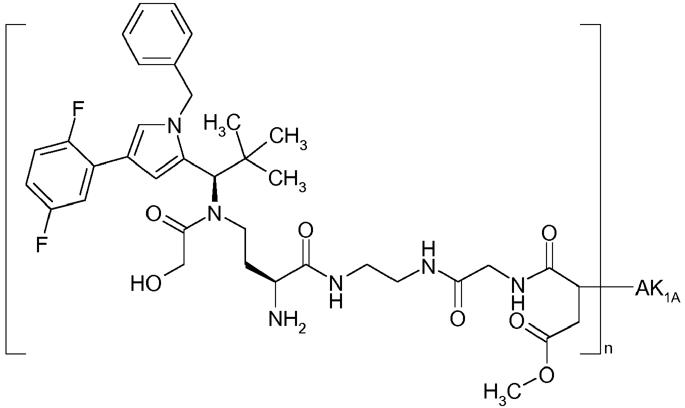 wobei das ADC, sofern es über eine Gruppe 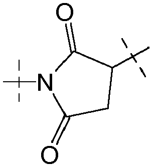 mit dem Antikörper verbunden ist, statt dieser Gruppe auch zumindest teilweise in Form der hydrolysierten offenkettigen Bernsteinsäureamide mit dem Antikörper verknüpft vorliegen kann.

66. Konjugat nach Anspruch 65, wobei der Antikörper ein humaner, humanisierter oder chimärer monoklonaler Antikörper oder ein Antigen-bindendes Fragment hiervon ist.

67. Konjugat nach Anspruch 66, wobei der Antikörper ein anti-HER2-Antikörper, ein anti-EGFR-Antikörper, ein anti-TWEAKR-Antikörper, oder ein Antigen-bindendes Fragment hiervon ist.

68. Konjugat nach Anspruch 67, wobei der anti-TWEAKR-Antikörper spezifisch an die Aminosäure D in Position 47 (D47) von TWEAKR (SEQ ID NO:169) bindet, bevorzugt der anti-TWEAKR-Antikörper TPP-2090.

## Claims

1. Conjugate of a binder or derivative thereof with one or more active compound molecules, the active compound molecule being a kinesin spindle protein inhibitor attached to the binder via a linker L, where the kinesin spindle protein inhibitor has the formula (IIa) below: 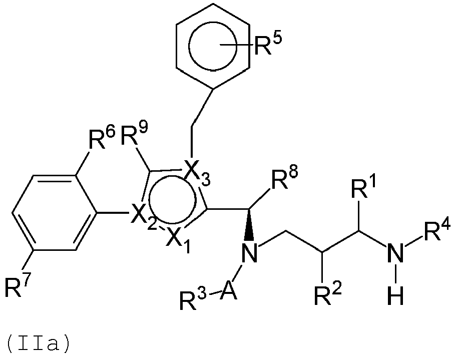 where
X₁ represents N, X₂ represents N and X₃ represents C; or
X₁ represents CH or CF, X₂ represents C and X₃ represents N; or
X₁ represents NH, X₂ represents C and X₃ represents C; or
X₁ represents CH, X₂ represents N and X₃ represents C;
(with X₁ representing CH, X₂ representing C and X₃ representing N being preferred);
R¹ represents H, -L-#1, -MOD or -(CH₂)₀₋₃Z, where Z represents -H, -NHY³, -OY³, -SY³, halogen, -CO-NY¹Y² or -CO-OY³,
where Y¹ and Y² independently of one another represent H, NH₂, - (CH₂CH₂O)₀₋₃-(CH₂)₀₋₃Z' (e.g.-(CH₂)₀₋₃Z') or -CH(CH₂W)Z', and Y³ represents H or-(CH₂)₀₋₃Z', where Z' represents H, NH₂, SO₃H, COOH, -NH-CO-CH₂-CH₂-CH(NH₂) COOH or -(CO-NH-CHY⁴)₁₋₃COOH;
where W represents H or OH,
where Y⁴ independently of one another represents straight-chain or branched C₁₋₆-alkyl which is optionally substituted by -NHCONH₂, or represents aryl or benzyl which are optionally substituted by -NH₂ ;
R² represents -L-#1, H, -MOD, -CO-CHY⁴-NHY⁵ or-(CH₂)₀₋₃Z, or R² and R⁴ together (with formation of a pyrrolidine ring) represent -CH₂-CHR¹⁰- or -CHR¹⁰-CH₂-, where R¹⁰ represents L-#1, H, NH₂, SO₃H, COOH, SH, or OH;
where Z represents -H, halogen, -OY³, -SY³, NHY³,-CO-NY¹Y² or -CO-OY³,
where Y¹ and Y² independently of one another represent H, NH₂ or -(CH₂)₀₋₃Z', and Y³ represents H or -(CH₂)₀₋₃Z', where Z' represents H, SO₃H, NH₂ or COOH;
where Y⁴ independently of one another represents straight-chain or branched C₁₋₆ alkyl which is optionally substituted by -NHCONH₂, or represents aryl or benzyl which are optionally substituted by -NH₂, and Y⁵ represents H or -CO-CHY⁶-NH₂, where Y⁶ represents straight-chain or branched C₁₋₆-alkyl;
R⁴ represents -L-#1, H, -CO-CHY⁴-NHY⁵ or -(CH₂)₀₋₃Z,
where Z represents -H, halogen, -OY³, -SY³, NHY³, - CO-NY¹Y² or -CO-OY³,
where Y¹ and Y² independently of one another represent H, NH₂ or -(CH₂)₀₋₃Z', and Y³ represents H or -(CH₂)₀₋₃Z', where Z' represents H, SO₃H, NH₂ or COOH;
where Y⁴ independently of one another represents straight-chain or branched C₁₋₆ alkyl which is optionally substituted by -NHCONH₂, or represents aryl or benzyl which are optionally substituted by -NH₂, and Y⁵ represents H or -CO-CHY⁶-NH₂, where Y⁶ represents straight-chain or branched C₁₋₆-alkyl;
or R² and R⁴ together (with formation of a pyrrolidine ring) represent -CH₂-CHR¹⁰- or -CHR¹⁰-CH₂-, where R¹⁰ represents L-#1, H, NH₂, SO₃H, COOH, SH or OH;
A represents CO, SO, SO₂, SO₂NH or CNNH;
R³ represents -L-#1, -MOD or an optionally substituted alkyl, cycloalkyl, aryl, heteroaryl, heteroalkyl, heterocycloalkyl group, preferably-L-#1 or a C₁₋₁₀-alkyl, C₆₋₁₀-aryl or C₆₋₁₀-aralkyl, C₅-₁₀-heteroalkyl, C₁₋₁₀-alkyl-O-C₆₋₁₀-aryl or C₅₋₁₀-heterocycloalkyl group which may be substituted by 1-3 -OH groups, 1-3 halogen atoms, 1-3 halogenated alkyl groups (each having 1-3 halogen atoms), 1-3 O-alkyl groups, 1-3 -SH groups, 1-3 -S-alkyl groups, 1-3-O-CO-alkyl groups, 1-3-O-CO-NH-alkyl groups, 1-3 -NH-CO-alkyl groups, 1-3 -NH-CO-NH-alkyl groups, 1-3-S(O)ₙ-alkyl groups, 1-3-SO₂-NH-alkyl groups, 1-3 -NH-alkyl groups, 1-3-N(alkyl)₂ groups, 1-3 -NH₂ groups or 1-3 -(CH₂)₀₋₃Z groups, where Z represents -H, halogen, -OY³, -SY³, -NHY³, -CO-NY¹Y² or -CO-OY³, where Y¹ and Y² independently of one another represent H, NH₂ or -(CH₂)₀₋₃Z' and Y³ represents H, - (CH₂)₀₋₃-CH (NHCOCH₃)Z', -(CH₂)₀₋₃-CH(NH₂) Z' or -(CH₂)₀₋₃Z', where Z' represents H, SO₃H, NH₂ or COOH
(where "alkyl" preferably represents C₁₋₁₀-alkyl);
R⁵ represents -L-#1, H, NH₂, NO₂, halogen (in particular F, Cl, Br), -CN, CF₃, -OCF₃, -CH₂F,-CH₂F, SH or -(CH₂)₀₋₃Z, where Z represents -H, -OY³, -SY³, halogen, NHY³, -CO-NY¹Y² or -CO-OY³,
where Y¹ and Y² independently of one another represent H, NH₂ or -(CH₂)₀₋₃Z', and Y³ represents H or -(CH₂)₀₋₃Z', where Z' represents H, SO₃H, NH₂ or COOH;
R⁶ and R⁷ independently of one another represent H, cyano, (optionally fluorinated) C₁₋₁₀-alkyl, (optionally fluorinated) C₂₋₁₀-alkenyl, (optionally fluorinated) C₂₋₁₀-alkynyl, hydroxy, NO₂, NH₂, COOH or halogen (in particular F, Cl, Br),
R⁸ represents (optionally fluorinated) C₁₋₁₀-alkyl, (optionally fluorinated) C₂₋₁₀-alkenyl, (optionally fluorinated) C₂₋₁₀-alkynyl, (optionally fluorinated) C₄₋₁₀-cycloalkyl or -(CH₂)₀₋₂-(HZ²), where HZ² represents a 4- to 7-membered heterocycle having up to two heteroatoms selected from the group consisting of N, O and S, where each of these groups may be substituted by -OH, CO₂H or NH₂ or-L-#1;
where one or none of the substituents R¹, R², R³, R⁴ R⁵ , R⁸ and R¹⁰ represents or (in the case of R⁸) contains -L-#1,
L represents the linker and #1 represents the bond to the binder or derivative thereof,
R⁹ represents H, F, CH₃, CF₃, CH₂F or CHF₂;
where -MOD represents -(NR¹⁰)ₙ-(G1)ₒ-G2-H, where
R¹⁰ represents H or C₁-C₃-alkyl;
G1 represents -NHCO- , -CONH- or 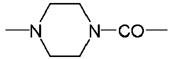 (where, if G1 represents -NHCO- or 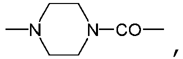 R¹⁰ does not represent NH₂) ;
n is 0 or 1;
o is 0 or 1; and
G2 represents a straight-chain and/or branched hydrocarbon group which has 1 to 10 carbon atoms and which may be interrupted once or more than once by one or more of the groups -O-, -S-, -SO-, SO₂, -NR^{y}-, -NR^{y}CO-, CONR^{y}-, -NR^{y}NR^{y}-,-SO₂NR^{y}NR^{y}-, -CONR^{y}NR^{y}- (where R^{y} represents H, phenyl, C₁-C₁₀-alkyl, C₂-C₁₀-alkenyl or C₂-C₁₀-alkynyl, each of which may be substituted by NHCONH₂, -COOH, -OH, -NH₂, NH-CNNH₂, sulphonamide, sulphone, sulphoxide or sulphonic acid), -CO-, -CR^{x}=N-O- (where Rx represents H, C₁-C₃-alkyl or phenyl), where the hydrocarbon chain including any side chains may be substituted by -NHCONH₂, -COOH, -OH, -NH₂, NH-CNNH₂, sulphonamide, sulphone, sulphoxide or sulphonic acid, where the group -MOD preferably has at least one group -COOH;
and the salts, solvates and salts of the solvates thereof.

2. Conjugate according to Claim 1 where X₁ represents CH, X₂ represents C and X₃ represents N.

3. Conjugate according to Claim 1 or 2 where the substituent R¹ represents -L-#1.

4. Conjugate according to one or more of the preceding claims where the active compound molecule linker is represented by general formula (II) : 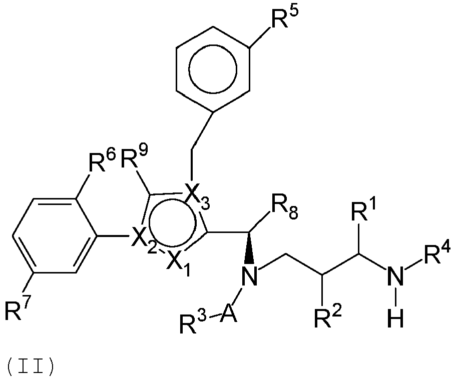 where
X₁ represents N, X₂ represents N and X₃ represents C; or
X₁ represents CH, X₂ represents C and X₃ represents N; or
X₁ represents NH, X₂ represents C and X₃ represents C; or
X₁ represents CH, X₂ represents N and X₃ represents C;
R¹ represents H, -L-#1 or - (CH₂)₀₋₃Z, where Z represents -H, -NHY³, -OY³, -SY³, halogen, -CO-NY¹Y² or -CO-OY³,
where Y¹ and Y² independently of one another represent H, NH₂, -(CH₂CH₂O)₀₋₃-(CH₂)₀₋₃Z' or-CH(CH₂W)Z', and Y³ represents H or -(CH₂)₀₋₃Z', where Z' represents H, NH₂, SO₃H, COOH, -NH-CO-CH₂-CH₂-CH (NH₂) COOH or -(CO-NH-CHY⁴)₁₋₃COOH, where W represents H or OH, where Y⁴ independently of one another represents straight-chain or branched C₁₋₆-alkyl which is optionally substituted by -NHCONH₂, or represents aryl or benzyl which are optionally substituted by -NH₂;
R² and R⁴ independently of one another represent-L-#1, H, -CO-CHY⁴-NHY⁵ or - (CH₂)₀₋₃Z, or R² and R⁴ together (with formation of a pyrrolidine ring) represent -CH₂-CHR¹⁰- or -CHR¹⁰-CH₂-, where R¹⁰ represents L-#1, H, NH₂, SO₃H, COOH, SH or OH,
where Z represents -H, OY³, -SY³, halogen, NHY³,-CO-NY¹Y² or -CO-OY³,
where Y¹ and Y² independently of one another represent H, NH₂ or -(CH₂)₀₋₃Z', and Y³ represents H or -(CH₂)₀₋₃Z', where Z' represents H, SO₃H, NH₂ or COOH;
where Y⁴ independently of one another represents straight-chain or branched C₁₋₆ alkyl which is optionally substituted by -NHCONH₂, or represents aryl or benzyl which are optionally substituted by -NH₂, and Y⁵ represents H or -CO-CHY⁶-NH₂, where Y⁶ represents straight-chain or branched C₁₋₆-alkyl;
A represents CO, SO, SO₂, SO₂NH or CNNH;
R³ represents -L-#1 or an optionally substituted alkyl, aryl, heteroaryl, heteroalkyl or heterocycloalkyl group,
preferably -L-#1 or a C₁₋₁₀-alkyl, C₆₋₁₀-aryl, C₆₋₁₀-aralkyl, C₅₋₁₀-heteroalkyl, C₁₋₁₀-alkyl-O-C₆₋₁₀-aryl-or C₅₋₁₀-heterocycloalkyl group,
which may be substituted by 1-3 -OH groups, 1-3 halogen atoms, 1-3 halogenated alkyl groups (which may each have 1-3 halogen atoms), 1-3 O-alkyl groups, 1-3 -SH groups, 1-3 -S-alkyl groups, 1-3-O-CO-alkyl groups, 1-3 -O-CO-NH-alkyl groups, 1-3 -NH-CO-alkyl groups, 1-3 -NH-CO-NH-alkyl groups, 1-3 -S(O)ₙ-alkyl groups, 1-3 -SO₂-NH-alkyl groups, 1-3 -NH-alkyl groups, 1-3 -N(alkyl)₂ groups, 1-3-NH₂ groups or 1-3 - (CH₂) ₀₋₃Z groups, where Z represents -H, halogen, -OY³, -SY³, -NHY³, -CO-NY¹Y² or -CO-OY³, where Y¹ and Y² independently of one another represent H, NH₂ or - (CH₂) ₀₋₃Z' and Y³ represents H, - (CH₂) ₀₋₃-CH(NHCOCH₃)Z', -(CH₂) ₀₋₃-CH(NH₂)Z' or -(CH₂)₀₋₃Z', where Z' represents H, SO₃H, NH₂ or COOH
(where "alkyl" preferably represents C₁₋₁₀-alkyl);
R⁵ represents -L-#1, H, F, NH₂, NO₂, halogen, SH or - (CH₂) ₀₋₃Z, where Z represents -H, OY³, -SY³, halogen, NHY³, -CO-NY¹Y² or -CO-OY³,
where Y¹ and Y² independently of one another represent H, NH₂ or -(CH₂)₀₋₃Z', and Y³ represents H or -(CH₂)₀₋₃Z', where Z' represents H, SO₃H, NH₂ or COOH;
where one of the substituents R¹, R², R³, R⁴ and R⁵ represents -L-#1,
L represents the linker and #1 represents the bond to the binder or derivative thereof,
R⁶ and R⁷ independently of one another represent H, cyano, (optionally fluorinated) C₁₋₁₀-alkyl, (optionally fluorinated) C₂₋₁₀-alkenyl, (optionally fluorinated) C₂₋₁₀-alkynyl, hydroxy or halogen,
R⁸ represents (optionally fluorinated) C₁₋₁₀-alkyl, (optionally fluorinated) C₄₋₁₀-cycloalkyl or optionally substituted oxetane; and
R⁹ represents H, F, CH₃, CF₃, CH₂F or CHF₂;
and the salts, solvates and salts of the solvates thereof.

5. Conjugate according to Claim 4,
where R¹ represents -L-#1, H, -(CH₂)₀₋₃Z, where Z represents -H, -CO-NY¹Y², NHY³, OY³, -SY³ or -CO-OY³,
where Y¹ and Y² independently of one another represent H, NH₂, - (CH₂CH₂O)₀₋₃-(CH₂)₀₋₃Z' or-CH(CH₂W)Z', and Y³ represents H or -(CH₂)₀₋₃Z', where Z' represents H, NH₂, COOH, -NH-CO-CH₂-CH₂-CH(NH₂)COOH or - (CO-NH-CHY⁴)₁₋₃COOH, where W represents H or OH, where Y⁴ independently of one another represents straight-chain or branched C₁₋₆-alkyl which is optionally substituted by -NHCONH₂, or represents aryl or benzyl which are optionally substituted by -NH₂;
R² and R⁴ independently of one another represent-L-#1, -CO-CHY⁴-NHY⁵ or H or R² and R⁴ together (with formation of a pyrrolidine ring) represent -CH₂-CHR¹⁰-, where R¹⁰ represents H, -L-#1, NH₂, COOH, SH, OH or SO₃H,
where Y⁴ independently of one another represents straight-chain or branched C₁₋₆ alkyl which is optionally substituted by -NHCONH₂, or represents aryl or benzyl which are optionally substituted by -NH₂, and Y⁵ represents H or -CO-CHY⁶-NH₂, where Y⁶ represents straight-chain or branched C₁₋₆-alkyl;
A represents CO,
R³ represents -(CH₂)OH or -L-#1, and
R⁵ represents -L-#1 or H,
where one of the substituents R¹, R², R³, R⁴ and R⁵ represents -L-#1.

6. Conjugate according to Claim 4 or 5 where R⁶ and R⁷ independently of one another represent H, C₁₋₃-alkyl or halogen.

7. Conjugate according to one or more of Claims 4 to 6 where R⁸ represents C₁₋₄-alkyl (preferably tert-butyl).

8. Conjugate according to one or more of Claims 4 to 7 where R⁹ represents H.

9. Conjugate according to one or more of Claims 4 to 8 where R⁶ and R⁷ represent F.

10. Conjugate according to one or more of the preceding claims where the binder or derivative thereof is a binder peptide or protein or a derivative of a binder peptide or protein.

11. Conjugate according to Claim 10 where each active compound molecule is attached to different amino acids of the binder peptide or protein or derivative thereof via the linker.

12. Conjugate according to one or more of the preceding claims where the conjugate has on average 1.2 to 20 active compound molecules per binder.

13. Conjugate according to one or more of Claims 10 to 12 where the binder peptide or protein represents an antibody or the derivative of the binder peptide or protein

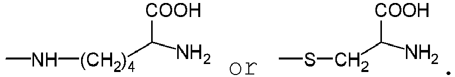

14. Conjugate according to one or more of the preceding claims where the binder binds to a cancer target molecule.

15. Conjugate according to Claim 14 where the binder binds to an extracellular target molecule.

16. Conjugate according to Claim 15 where the binder, after binding to the extracellular target molecule, is internalized and processed intracellularly (preferably lysosomally) by the cell expressing the target molecule.

17. Conjugate according to one or more of Claims 10 to 16 where the binder peptide or protein is a human, humanized or chimeric monoclonal antibody or an antigen-binding fragment thereof.

18. Conjugate according to Claim 17 where the binder peptide or protein is an anti-HER2 antibody, an anti-EGFR antibody, an anti-TWEAKR antibody or an antigen-binding fragment thereof.

19. Conjugate according to Claim 18 where the anti-TWEAKR antibody binds specifically to amino acid D in position 47 (D47) of TWEAKR (SEQ ID NO:169), preferably the anti-TWEAKR antibody TPP-2090.

20. Conjugate according to Claim 18 where the binder peptide or protein is an anti-EGFR antibody and R3 represents -L-#1.

21. Conjugate according to one or more of the preceding claims where the linker -L- has one of the basic structures (i) to (iv) below:
(i) -(CO)ₘ-SG1-L1-L2-
(ii) -(CO)ₘ-L1-SG-L1-L2-
(iii) -(CO)ₘ-L1-L2-
(iv) -(CO)ₘ-L1-SG-L2
where m is 0 or 1, SG and SG1 are *in vivo* cleavable groups, L1 independently of one another represent organic groups not cleavable *in vivo,* and L2 represents a coupling group to the binder.

22. Conjugate according to Claim 21 where the *in vivo* cleavable group SG is a 2-8 oligopeptide group, preferably a dipeptide group or a disulphide, a hydrazone, an acetal or an aminal and SG1 is a 2-8 oligopeptide group, preferably a dipeptide group.

23. Conjugate according to one or more of the preceding claims
where the linker is attached to a cysteine side chain or a cysteine residue and has the formula below:
§-(CO)m-L1-L2-§§
where
m is 0 or 1;
§ represents the bond to the active compound molecule and
§§ represents the bond to the binder peptide or protein, and
-L2- represents 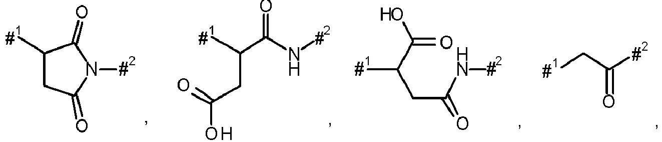 #¹-s-#² 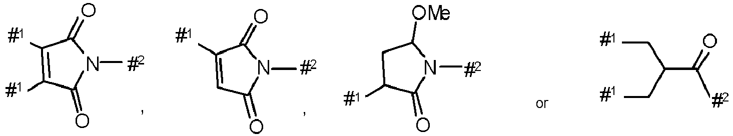
where
#¹ denotes the point of attachment to the sulphur atom of the binder,
#² denotes the point of attachment to group L¹,
L1 represents - (NR¹⁰)n-(G1)o-G2-,
where R¹⁰ represents H, NH₂ or C₁-C₃-alkyl;
G1 represents -NHCO- or 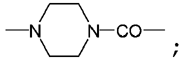
n is 0 or 1;
o is 0 or 1; and
G2 represents a straight-chain or branched hydrocarbon chain having 1 to 100 carbon atoms from arylene groups and/or straight-chain and/or branched and/or cyclic alkylene groups and which may be interrupted once or more than once by one or more of the groups -O-, -S-, -SO-, SO₂, -NH-,-CO-, -NMe-, -NHNH-, -SO₂NHNH-, -NHCO-, -CONH-,-CONHNH- and a 5- to 10-membered aromatic or non-aromatic heterocycle having up to 4 heteroatoms selected from the group consisting of N, O and S, -SO- or -SO₂- (preferably 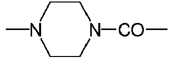 ), where the side chains, if present, may be substituted by-NHCONH₂, -COOH, -OH, -NH₂, NH-CNNH₂, sulphonamide, sulphone, sulphoxide or sulphonic acid,
or represents one of the groups below: 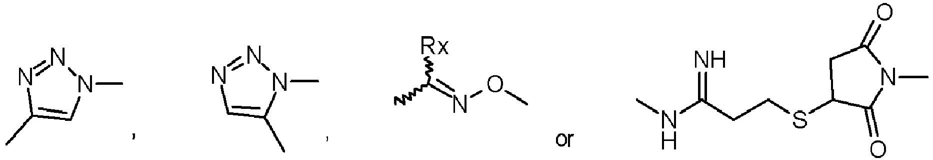 where Rx represents H, C₁-C₃-alkyl or phenyl.

24. Conjugate according to Claim 23 where L2 is represented by one or both of the formulae below: 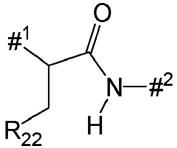 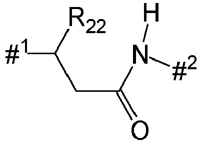 where #¹ denotes the point of attachment to the sulphur atom of the binder, #² denotes the point of attachment to group L¹, R²² represents COOH and more than 80% (based on the total number of bonds of the linker to the binder) of the bonds to the sulphur atom of the binder are present in one of these two structures.

25. Conjugate according to Claim 23 or 24 where the hydrocarbon chain is interrupted by one of the groups below: 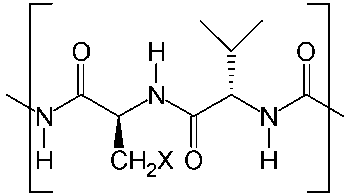 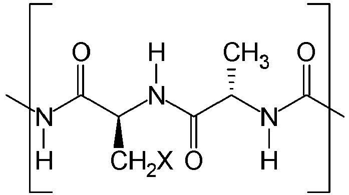 where X represents H or a C₁₋₁₀-alkyl group which may optionally be substituted by -NHCONH₂, -COOH,-OH, NH₂, -NH-CNNH₂, sulphone, sulphoxide or sulphonic acid.

26. Conjugate according to Claim 23 where the linker has the formula below: 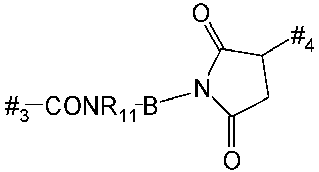 where
#3 represents the bond to the active compound molecule,
#4 represents the bond to the binder peptide or protein,
R¹¹ represents H or NH₂;
B represents - [(CH₂) ₓ-(X⁴)_{y}]w-(CH₂)_{z}-,
w = 0 to 20;
x = 0 to 5;
x = 0 to 5;
y = 0 or 1;
z = 0 to 5; and
X⁴ represents -O-, -CONH- or -NHCO-

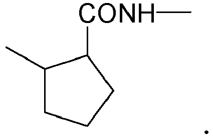

27. Conjugate according to Claim 23 where R¹ or R⁴ represents -L-#1.

28. Conjugate according to one or more of Claims 23 to 27 where the conjugate has one of the formulae below: 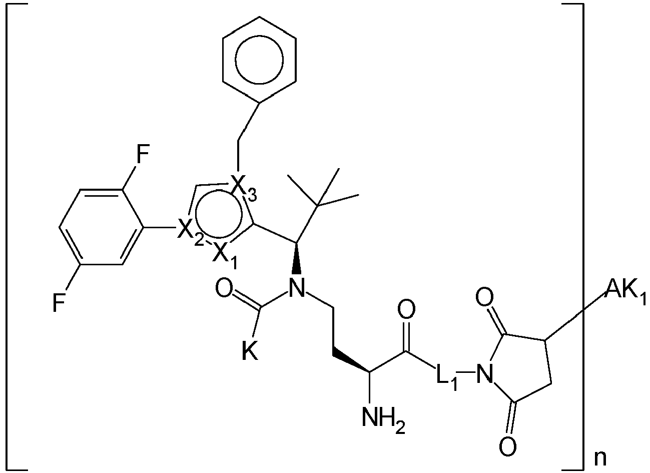 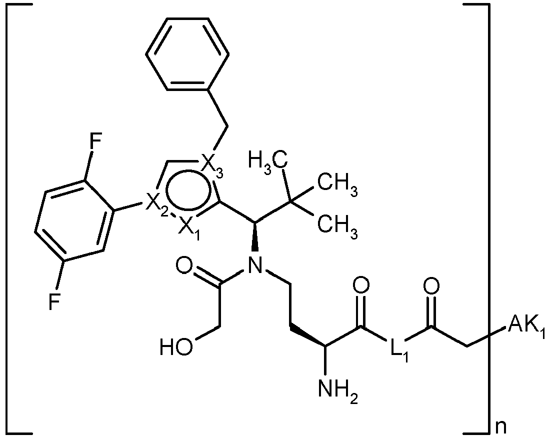 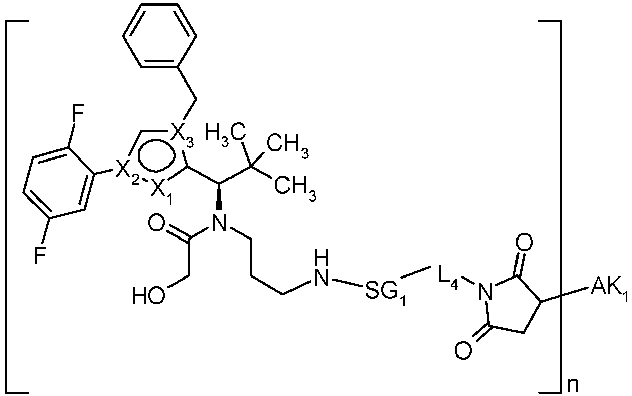 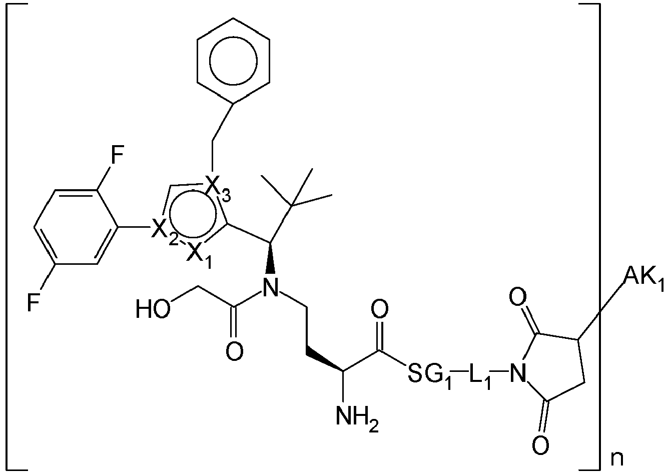 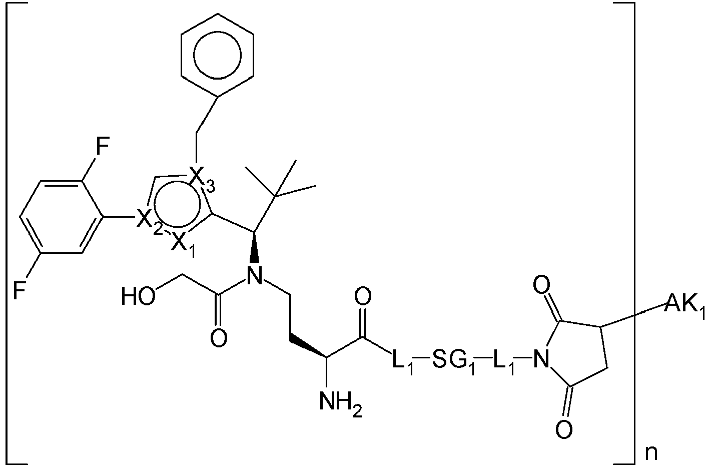 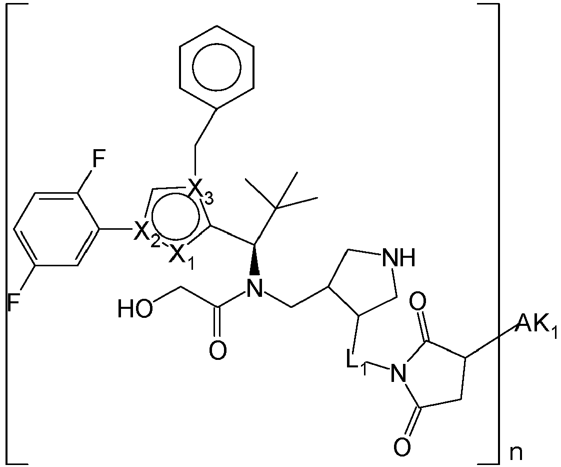 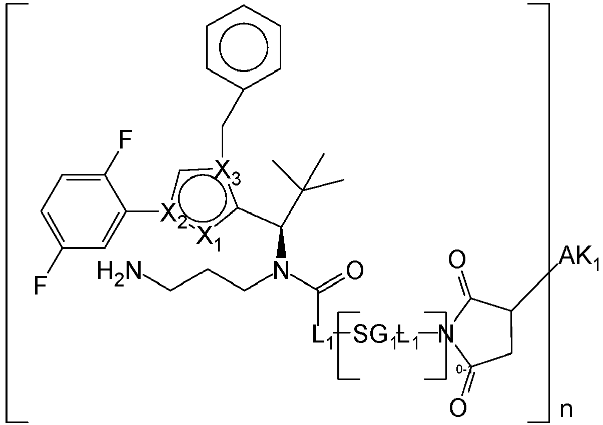 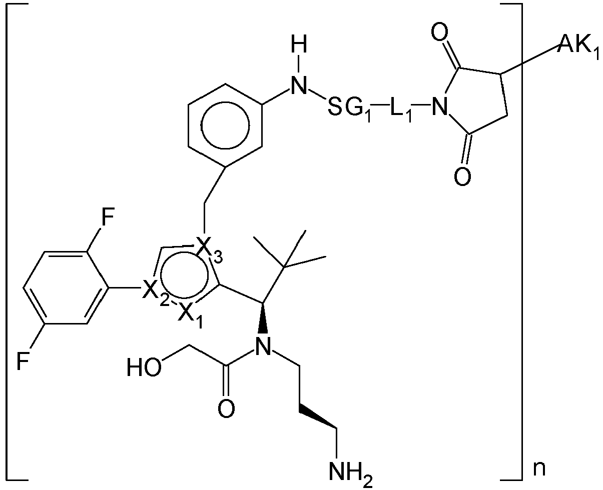 where
X₁, X₂ and X₃ have the same meaning as in Claim 4, AK₁ represents a binder peptide or protein which is attached via a sulphur atom of the binder; n represents a number from 1 to 20; and L1 represents an optionally branched hydrocarbon group having 1 to 70 carbon atoms, which represents a straight-chain or branched chain having 1 to 100 carbon atoms from arylene groups and/or straight-chain and/or branched and/or cyclic alkylene groups and which may be interrupted once or more than once by one or more of the groups -O-, -S-, -SO-, SO₂, -NH-, -CO-,-CONH-, -NHCO-, -NMe-, -NHNH-, -SO₂NHNH-, -CONHNH-and a 5- to 10-membered aromatic or non-aromatic heterocycle having up to 4 heteroatoms selected from the group consisting of N, O and S, or -SO- or -SO₂- (preferably 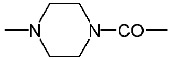 ), where the side chains, if present, may be substituted by -NHCONH₂, -COOH, -OH, -NH₂, NH-CNNH₂, sulphonamide, sulphone, sulphoxide or sulphonic acid,
and SG1 is a 2-8 oligopeptide, preferably a dipeptide;
L4 is a single bond or a group -(CO)_{y}-G4-, where y represents 0 or 1, and G4 represents a straight-chain or branched hydrocarbon chain having 1 to 100 carbon atoms from arylene groups and/or straight-chain and/or branched and/or cyclic alkylene groups and which may be interrupted once or more than once by one or more of the groups -O-, -S-, -SO-, SO₂, -NH-, -CO-, -NHCO-, -CONH-, -NMe-, -NHNH-, -SO₂NHNH-, -CONHNH- and a 5- to 10-membered aromatic or non-aromatic heterocycle having up to 4 heteroatoms selected from the group consisting of N, O and S, or -SO- or -SO₂-(preferably 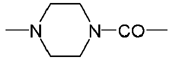 ), where the side chains, if present, may be substituted by -NHCONH₂, -COOH, -OH, -NH₂, NH-CNNH₂, sulphonamide, sulphone, sulphoxide or sulphonic acid.

29. Conjugate of a binder or derivative thereof with one or more active compound molecules where the conjugate has one or both of the formulae below: 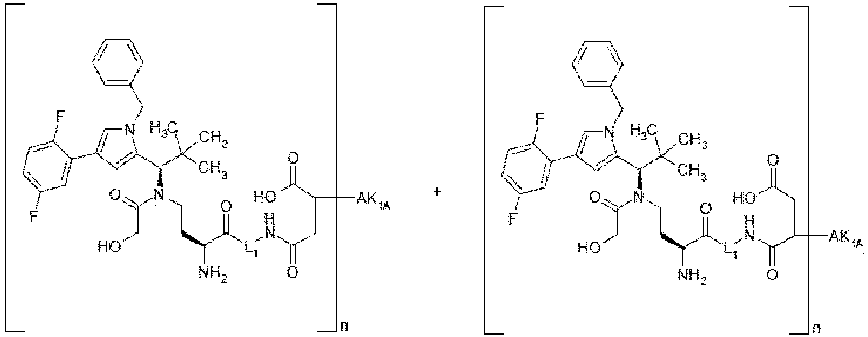 where
AK_{1A} represents a binder peptide or protein which is attached via a sulphur atom of the binder; n represents a number from 1 to 20; and L1 represents a straight-chain or branched hydrocarbon chain having 1 to 100 carbon atoms from arylene groups and/or straight-chain and/or branched and/or cyclic alkylene groups and which may be interrupted once or more than once by one or more of the groups -O-, -S-, -SO-, SO₂, -NH-,-CO-, -CONH-, -NHCO-, -NMe-, -NHNH-, -SO₂NHNH-,-CONHNH- and a 5- to 10-membered aromatic or non-aromatic heterocycle having up to 4 heteroatoms selected from the group consisting of N, O and S, -SO- or -SO₂- (preferably 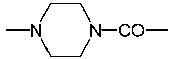 ), where the side chains, if present, may be substituted by-NHCONH₂, -COOH, -OH, -NH₂, NH-CNNH₂, sulphonamide, sulphone, sulphoxide or sulphonic acid.

30. Conjugate according to one or more of the preceding claims where the linker -L- is attached to a cysteine side chain or a cysteine residue and has the formula below: 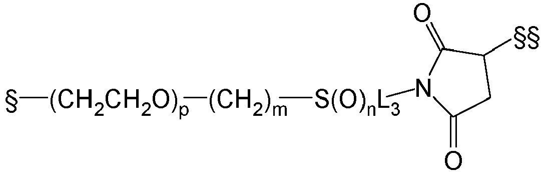 where
§ represents the bond to the active compound molecule and
§§ represents the bond to the binder peptide or protein,
m is 0, 1, 2 or 3;
n is 0, 1 or 2;
p is 0 to 20; and
L3 represents 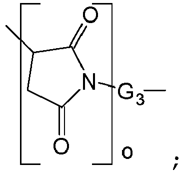
where
o is 0 or 1;
and
G3 represents a straight-chain or branched hydrocarbon chain having 1 to 100 carbon atoms from arylene groups and/or straight-chain and/or branched and/or cyclic alkylene groups and which may be interrupted once or more than once by one or more of the groups -O-, -S-, -SO-, SO₂, -NH-,-CO-, -NHCO-, -CONH-and a 5- to 10-membered aromatic or non-aromatic heterocycle having up to 4 heteroatoms selected from the group consisting of N, O and S, -NMe-, -NHNH-, -SO₂NHNH-, -CONHNH-, -SO- or -SO₂- (preferably 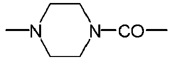 ), where the side chains, if present, may be substituted by-NHCONH₂, -COOH, -OH, sulphone, sulphoxide or sulphonic acid.

31. Conjugate according to Claim 30 where the linker-L- is attached to a cysteine side chain or a cysteine residue and has the formula below: 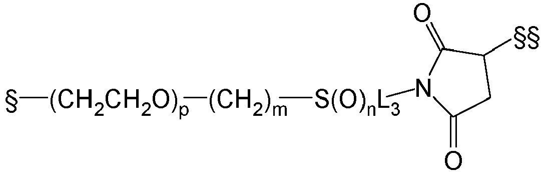 where
§ represents the bond to the active compound molecule and
§§ represents the bond to the binder peptide or protein,
m is 1;
p is 0;
n is 0;
and L3 represents 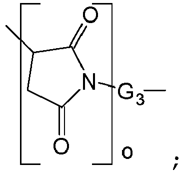
where
o is 0 or 1; and
G3 represents - (CH₂CH₂O)ₛ(CH₂)ₜ(CONH)ᵤ (CH₂CH₂O)ᵥ(CH₂)_{w}-, where
s, t, v and w each independently of one another are from 0 to 20 and u is 0 or 1.

32. Conjugate according to Claim 30 or 31 where R² or R³ represents -L-#1.

33. Conjugate according to Claim 32 where the conjugate has one of the formulae below: 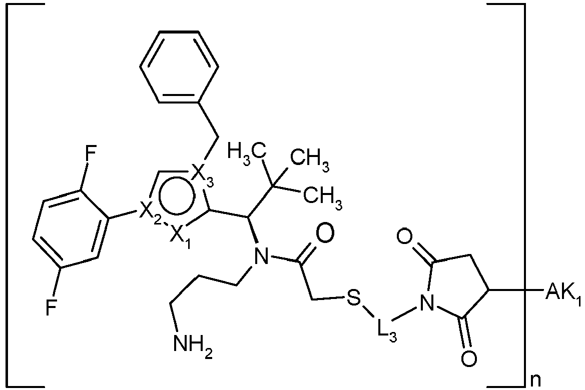 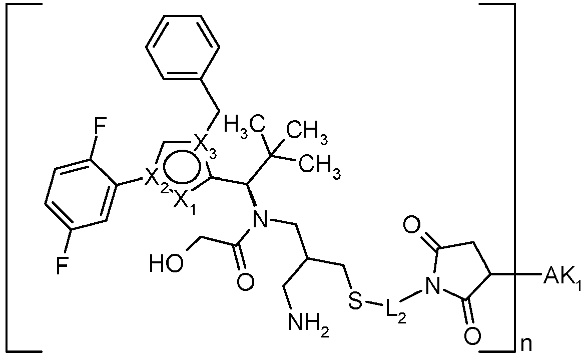 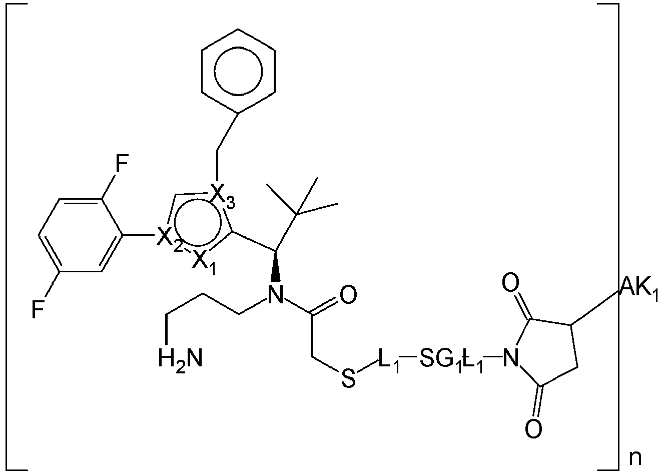 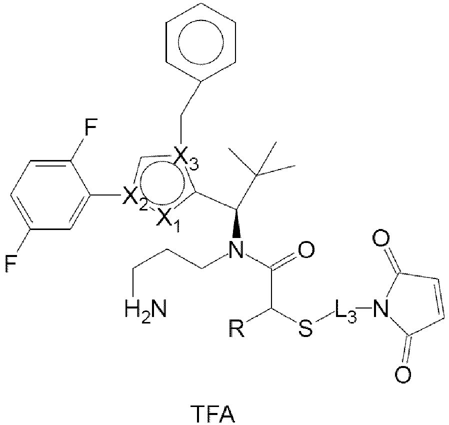 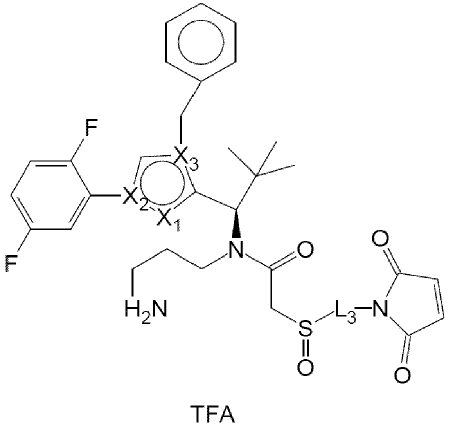 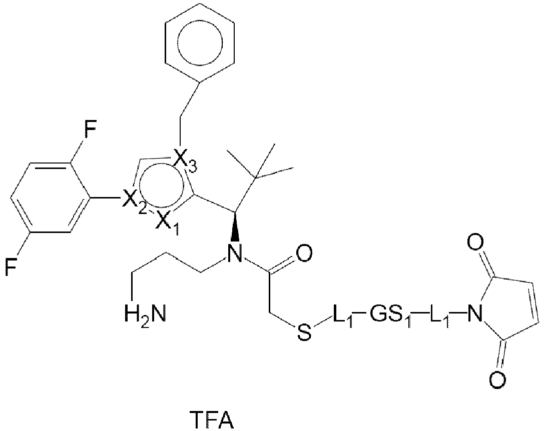 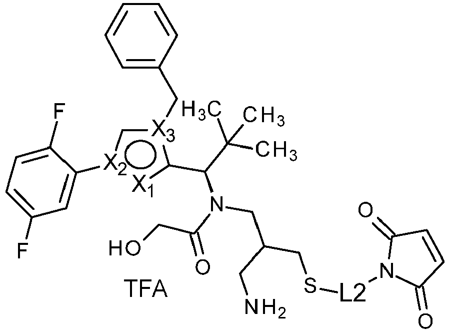 where
X₁, X₂ and X₃ have the same meaning as in Claim 4, AK₁ represents a binder peptide or protein which is attached via a sulphur atom of the binder; n represents a number from 1 to 20; and L2 and L3 represent a straight-chain or branched hydrocarbon chain having 1 to 100 carbon atoms from arylene groups and/or straight-chain and/or branched and/or cyclic alkylene groups and which may be interrupted once or more than once by one or more of the groups -O-, -S-, -SO-, SO₂, -NH-, -CO-,-NMe-, -NHNH-, -SO₂NHNH-, -NHCO-, -CONH-, -CONHNH-and a 5- to 10-membered aromatic or non-aromatic heterocycle having up to 4 heteroatoms selected from the group consisting of N, O and S, -SO- or-SO₂- (preferably 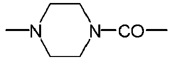 ), where the side chains, if present, may be substituted by -NHCONH₂, -COOH, -OH, -NH₂, NH-CNNH₂, sulphonamide, sulphone, sulphoxide or sulphonic acid.

34. Conjugate according to one or more of Claims 1 to 29 where the linker -L- is attached to a lysine side chain and has the formula below:
-§- (SG)ₓ-L4-CO-§§
where
§ represents the bond to the active compound molecule and
§§ represents the bond to the binder peptide or protein,
x represents 0 or 1,
SG represents a cleavable group, preferably a 2-8 oligopeptide, particularly preferably a dipeptide, and
L4 represents a single bond or a group -(CO)_{y}-G4-, where y represents 0 or 1, and
G4 represents a straight-chain or branched hydrocarbon chain having 1 to 100 carbon atoms from arylene groups and/or straight-chain and/or branched and/or cyclic alkylene groups and which may be interrupted once or more than once by one or more of the groups -O-, -S-, -SO-, SO₂, -NH-,-CO-, -NHCO-, -CONH-, -NMe-, -NHNH-, -SO₂NHNH-,-CONHNH- and a 5- to 10-membered aromatic or non-aromatic heterocycle having up to 4 heteroatoms selected from the group consisting of N, O and S, or -SO- (preferably 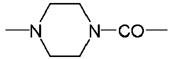 ), where the side chains, if present, may be substituted by -NHCONH₂, -COOH, -OH, -NH₂, NH-CNNH₂, sulphonamide, sulphone, sulphoxide or sulphonic acid.

35. Conjugate of a binder peptide or protein according to Claim 34 where the conjugate has one of the formulae below: 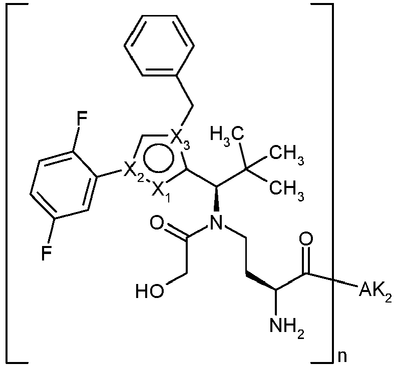 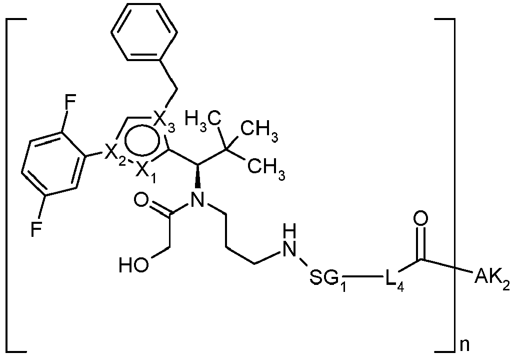 where
X₁, X₂ and X₃ have the same meaning as in Claim 4, AK₂ represents a binder peptide or protein which is attached via a sulphur atom of the binder; n represents a number from 1 to 20; and L4 represents an optionally straight-chain or branched hydrocarbon chain having 1 to 100 carbon atoms from arylene groups and/or straight-chain and/or branched and/or cyclic alkylene groups and which may be interrupted once or more than once by one or more of the groups -O-, -S-, -SO-, SO₂, -NH-, -CO-, -NMe-, -NHNH-, -SO₂NHNH-, -NHCO-, -CONH-,-CONHNH- and a 5- to 10-membered aromatic or non-aromatic heterocycle having up to 4 heteroatoms selected from the group consisting of N, O and S, -SO- or -SO₂- (preferably 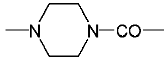 ), where the side chains, if present, may be substituted by-NHCONH₂, -COOH, -OH, -NH₂, NH-CNNH₂, sulphonamide, sulphone, sulphoxide or sulphonic acid, and
SG1 represents a cleavable group, preferably a 2-8 oligopeptide, particularly preferably a dipeptide.

36. Conjugate according to one or more of Claims 18 to 35 where the anti-TWEAKR antibody is an agonistic antibody.

37. Conjugate according to one or more of Claims 18 to 36 which comprises:
a variable heavy chain comprising:
a. a CDR1 of the heavy chain encoded by an amino acid sequence comprising the formula PYPMX (SEQ ID NO: 171), where X is I or M;
b. a CDR2 of the heavy chain encoded by an amino acid sequence comprising the formula YISPSGGXTHYADSVKG (SEQ ID NO: 172), where X is S or K; and
c. a CDR3 of the heavy chain encoded by an amino acid sequence comprising the formula GGDTYFDYFDY (SEQ ID NO: 173);
and a variable light chain comprising:
d. a CDR1 of the light chain encoded by an amino acid sequence comprising the formula RASQSISXYLN (SEQ ID NO: 174), where X is G or S;
e. a CDR2 of the light chain encoded by an amino acid sequence comprising the formula XASSLQS (SEQ ID NO: 175), where X is Q, A or N; and
f. a CDR3 of the light chain encoded by an amino acid sequence comprising the formula QQSYXXPXIT (SEQ ID NO: 176), where X at position 5 is T or S, X at position 6 is T or S and X at position 8 is G or F.

38. Conjugate according to one or more of Claims 18 to 37 which comprises:
a. a variable sequence of the heavy chain, as shown in SEQ ID NO:10, and also a variable sequence of the light chain, as shown in SEQ ID NO:9, or
b. a variable sequence of the heavy chain, as shown in SEQ ID NO:20, and also a variable sequence of the light chain, as shown in SEQ ID NO:19, or
c. a variable sequence of the heavy chain, as shown in SEQ ID NO:30, and also a variable sequence of the light chain, as shown in SEQ ID NO:29, or
d. a variable sequence of the heavy chain, as shown in SEQ ID NO:40, and also a variable sequence of the light chain, as shown in SEQ ID NO:39, or
e. a variable sequence of the heavy chain, as shown in SEQ ID NO:50, and also a variable sequence of the light chain, as shown in SEQ ID NO:49, or
f. a variable sequence of the heavy chain, as shown in SEQ ID NO:60, and also a variable sequence of the light chain, as shown in SEQ ID NO:59, or
g. a variable sequence of the heavy chain, as shown in SEQ ID NO:70, and also a variable sequence of the light chain, as shown in SEQ ID NO:69, or
h. a variable sequence of the heavy chain, as shown in SEQ ID NO:80, and also a variable sequence of the light chain, as shown in SEQ ID NO:79, or
i. a variable sequence of the heavy chain, as shown in SEQ ID NO:90, and also a variable sequence of the light chain, as shown in SEQ ID NO:89, or
j. a variable sequence of the heavy chain, as shown in SEQ ID NO: 100, and also a variable sequence of the light chain, as shown in SEQ ID NO:99, or
k. a variable sequence of the heavy chain, as shown in SEQ ID NO:110, and also a variable sequence of the light chain, as shown in SEQ ID NO:109, or
l. a variable sequence of the heavy chain, as shown in SEQ ID NO:120, and also a variable sequence of the light chain, as shown in SEQ ID NO:119.

39. Conjugate according to one or more of Claims 18 to 38 where the antibody is an IgG antibody.

40. Conjugate according to one or more of Claims 18 to 39 which comprises:
a. a sequence of the heavy chain, as shown in SEQ ID NO:2, and also a sequence of the light chain, as shown in SEQ ID NO:1, or
b. a sequence of the heavy chain, as shown in SEQ ID NO:12, and also a sequence of the light chain, as shown in SEQ ID NO:11, or
c. a sequence of the heavy chain, as shown in SEQ ID NO:22, and also a sequence of the light chain, as shown in SEQ ID NO:21, or
d. a sequence of the heavy chain, as shown in SEQ ID NO:32, and also a sequence of the light chain, as shown in SEQ ID NO:31, or
e. a sequence of the heavy chain, as shown in SEQ ID NO:42, and also a sequence of the light chain, as shown in SEQ ID NO:41, or
f. a sequence of the heavy chain, as shown in SEQ ID NO:52, and also a sequence of the light chain, as shown in SEQ ID NO:51, or
g. a sequence of the heavy chain, as shown in SEQ ID NO:62, and also a sequence of the light chain, as shown in SEQ ID NO:61, or
h. a sequence of the heavy chain, as shown in SEQ ID NO:72, and also a sequence of the light chain, as shown in SEQ ID NO:71, or
i. a sequence of the heavy chain, as shown in SEQ ID NO:82, and also a sequence of the light chain, as shown in SEQ ID NO:81, or
j. a sequence of the heavy chain, as shown in SEQ ID NO:92, and also a sequence of the light chain, as shown in SEQ ID NO:91, or
k. a sequence of the heavy chain, as shown in SEQ ID NO:102, and also a sequence of the light chain, as shown in SEQ ID NO:101, or
l. a sequence of the heavy chain, as shown in SEQ ID NO:112, and also a sequence of the light chain, as shown in SEQ ID NO:111.

41. Spindle conjugate according to one or more of the preceding claims where the conjugate has 1 to 10, preferably 2 to 8, active compound molecules per binder peptide or protein.

42. Process for preparing the conjugate according to Claim 28 or 32 where a compound of one of the formulae below, preferably in the form of its trifluoroacetic acid salt, is attached to a cysteine residue of a binder peptide or protein which is optionally partially reduced beforehand, where the compound is preferably employed in a 2-to 12-fold molar excess with respect to the binder peptide or protein: 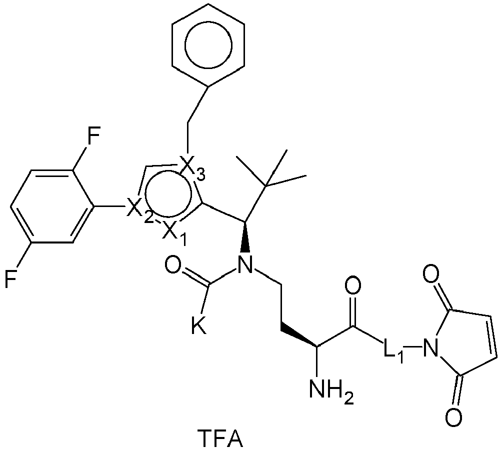 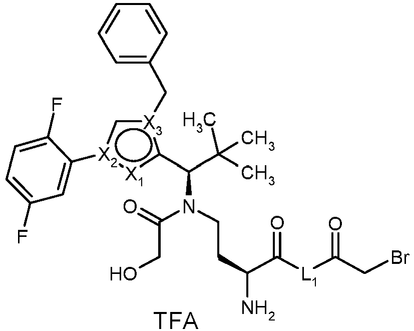 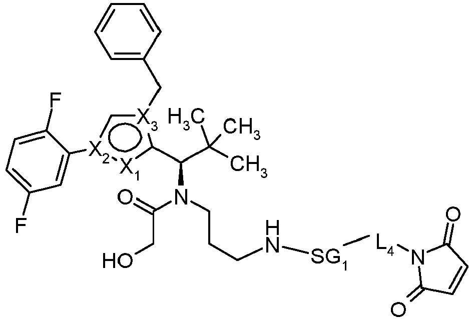 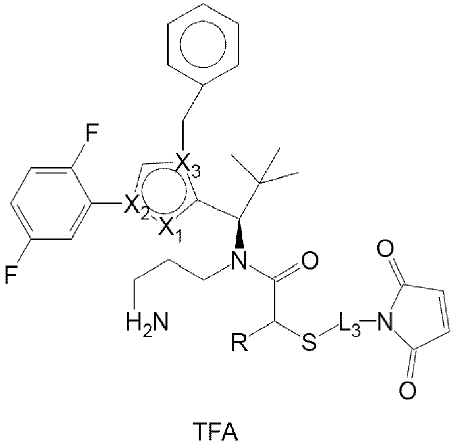 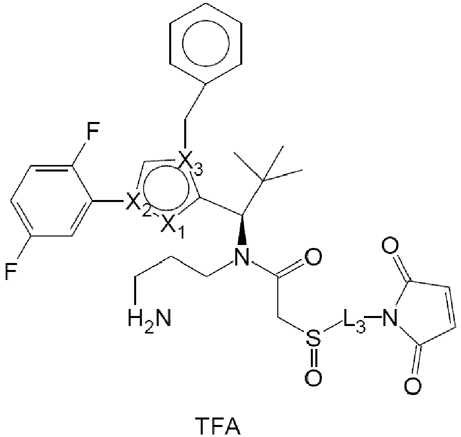 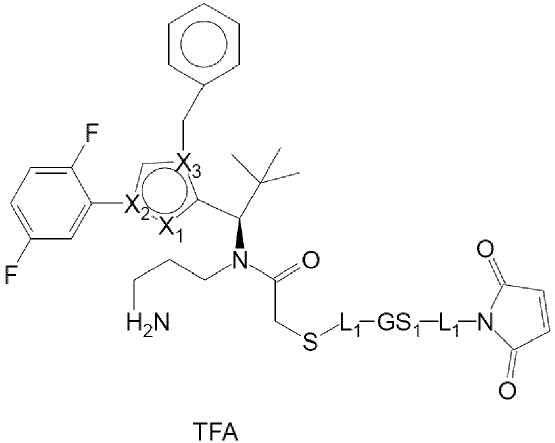 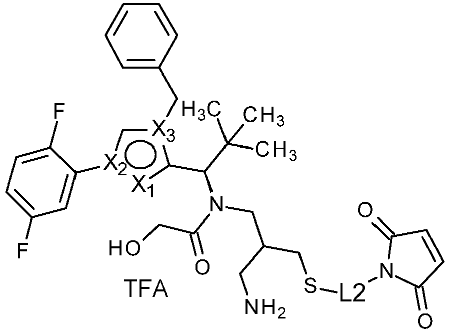 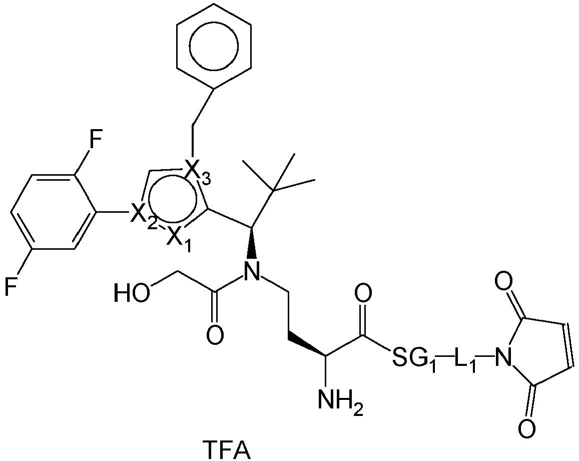 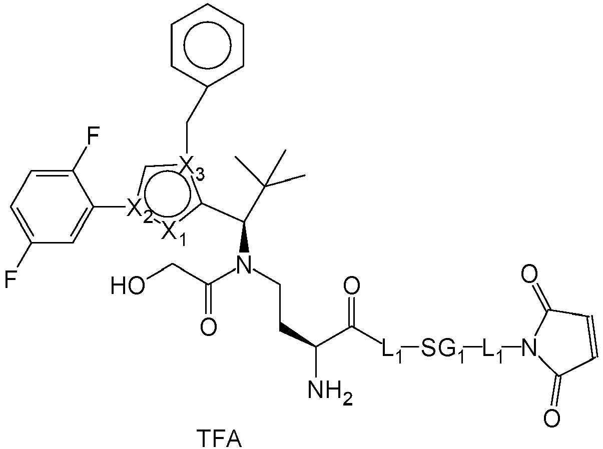 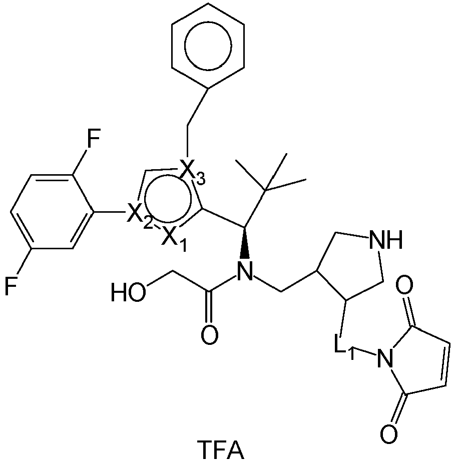 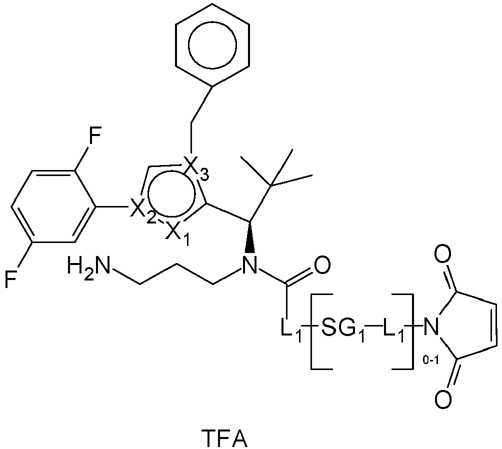 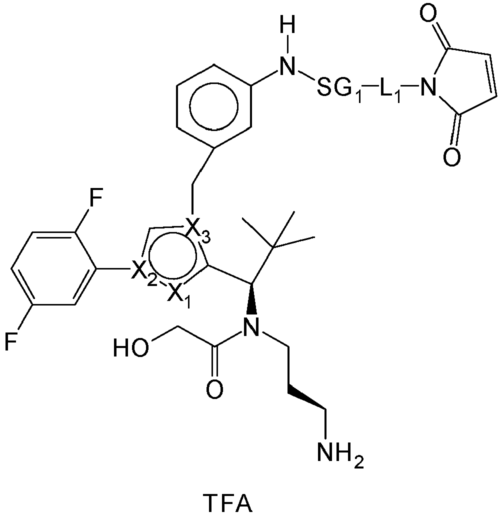
where R represents -H or -COOH,
where K represents straight-chain or branched optionally substituted by C₁-C₆-alkoxy or -OH-C₁-C₆-alkyl, and
where X₁, X₂, X₃, SG1, L1, L2, L3 and L4 have the same meaning as in Claim 28 or 32.

43. Process for preparing the conjugate according to Claim 42 where a compound of one of the formulae below, preferably in the form of its trifluoroacetic acid salt, is attached to a lysine residue of a binder peptide or protein, where the compound is preferably employed in a 2- to 12-fold molar excess with respect to the binder peptide or protein: 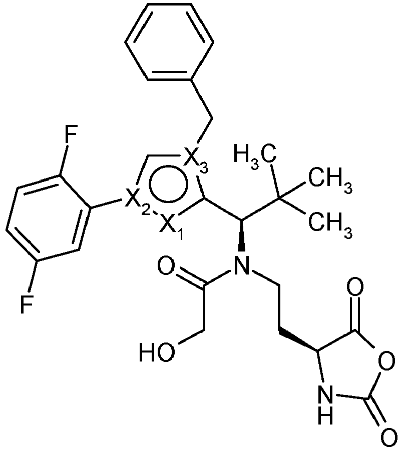 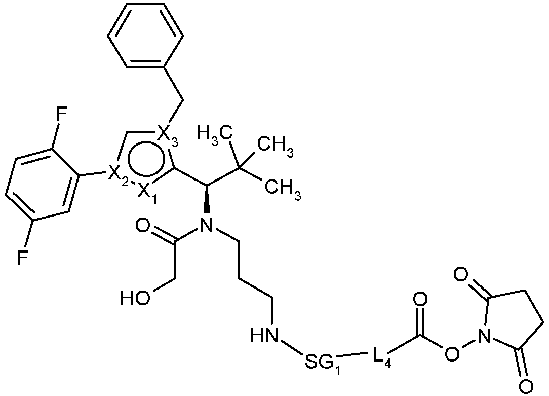 where X₁, X₂, X₃, SG1 and L4 have the same meaning as in Claim 34.

44. Compound of one of the formulae below: 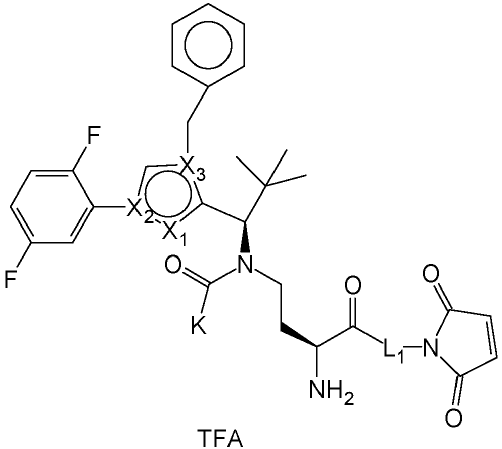 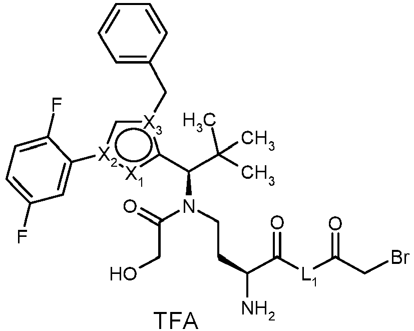 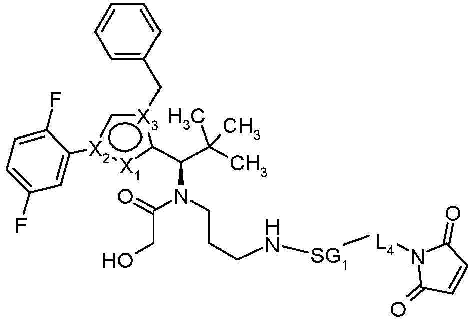 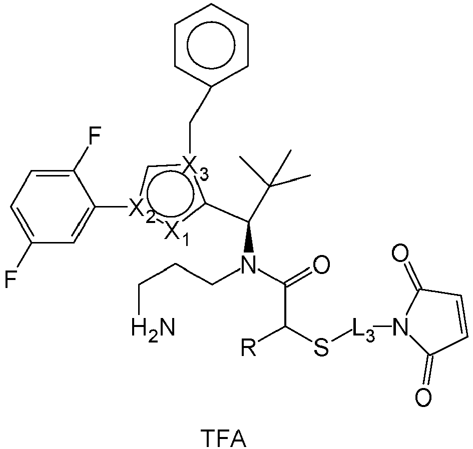 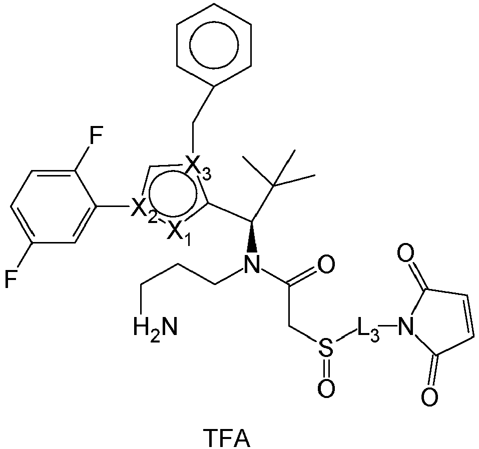 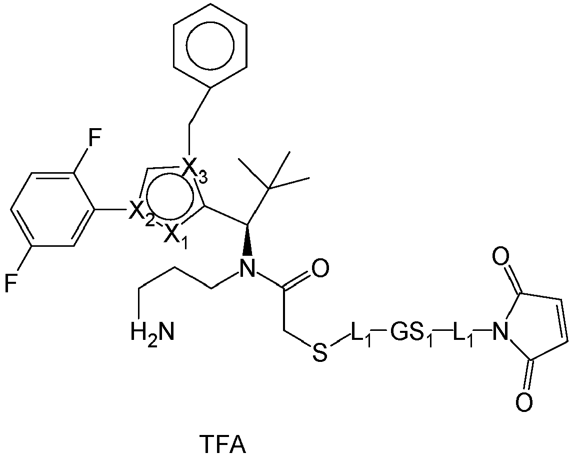 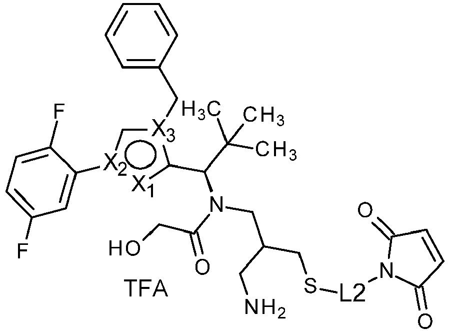 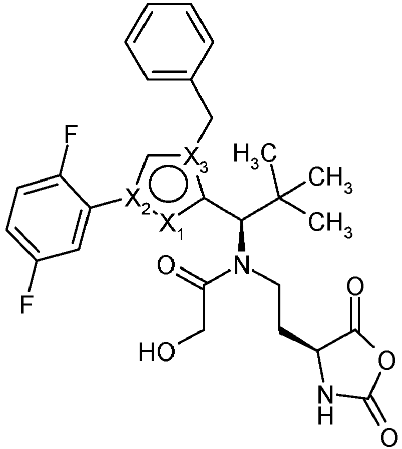 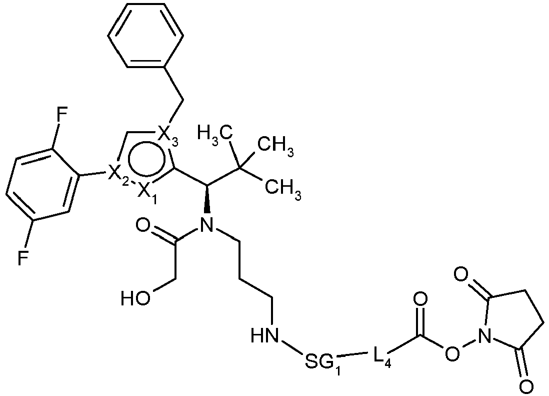 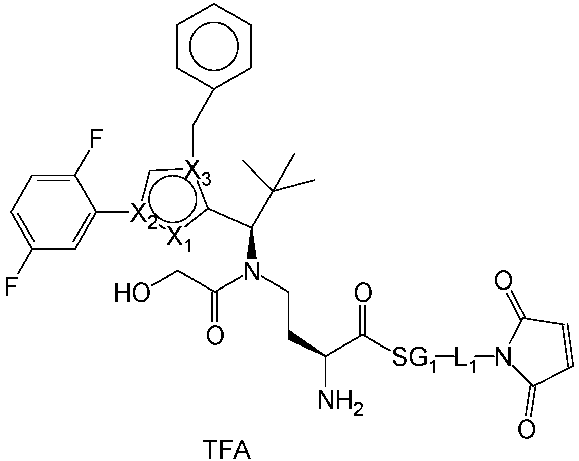 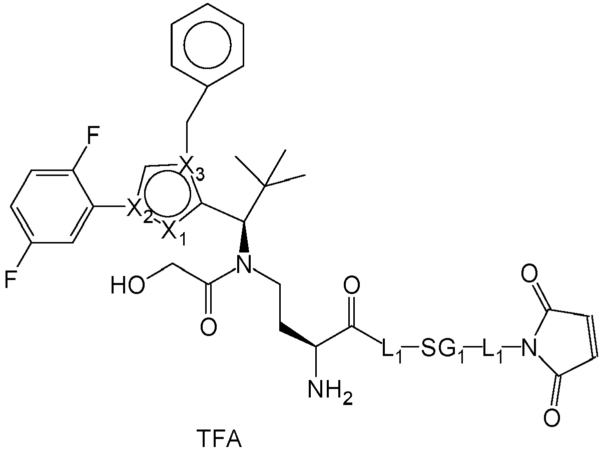 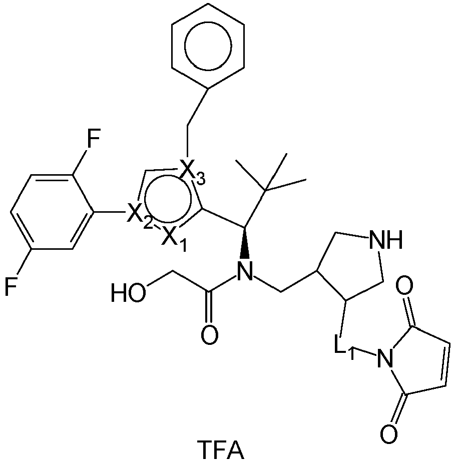 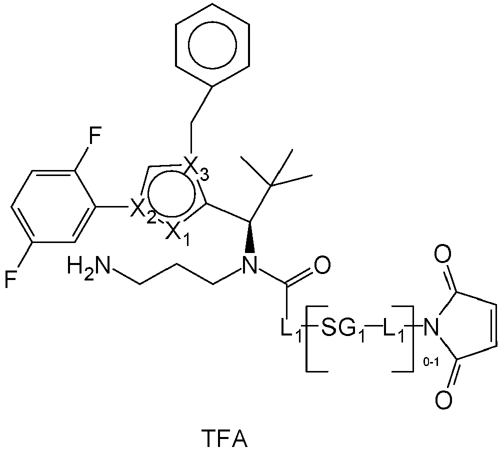 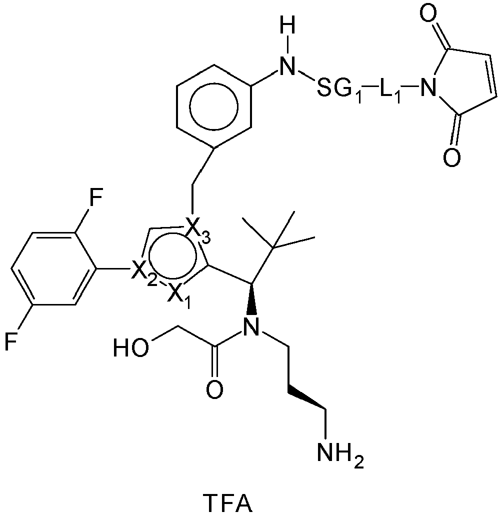
where R represents -H or -COOH,
where K represents straight-chain or branched optionally substituted by C₁-C₆-alkoxy or -OH C₁-C₆-alkyl, and
where X₁, X₂, X₃, SG1, L¹, L2, L3 and L4 have the same meaning as in Claim 24, 28 or 30.

45. Compounds of the general formula (III): 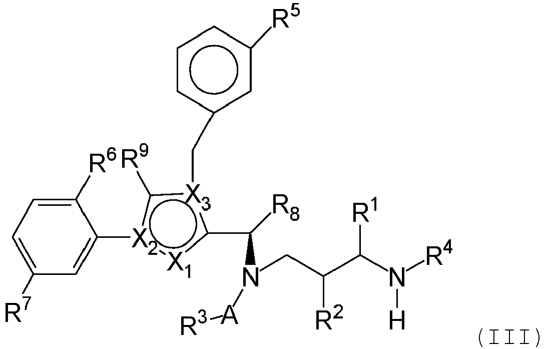 where
X₁ represents N, X₂ represents N and X₃ represents C, or X₁ represents CH, X₂ represents C and X₃ represents N or X₁ represents NH, X₂ represents C and X₃ represents C, or X₁ represents CH, X₂ represents N and X₃ represents C;
R¹ represents -L-BINDER, H or -(CH₂)₀₋₃Z, where Z represents -H, -NHY³, -OY³, -SY³, halogen, -CO-NY¹Y² or -CO-OY³,
where Y¹ and Y² independently of one another represent H, NH₂, - (CH₂CH₂O) ₀₋₃-(CH₂) ₀₋₃Z' or-CH(CH₂W)Z', and Y³ represents H or -(CH₂)₀₋₃Z', where Z' represents H, NH₂, SO₃H, COOH, -NH-CO-CH₂-CH₂-CH (NH₂)COOH or - (CO-NH-CHY⁴)₁₋₃COOH; where W represents H or OH;
where Y⁴ independently of one another represents straight-chain or branched C₁₋₆-alkyl which is optionally substituted by -NHCONH₂, or represents aryl or benzyl which are optionally substituted by -NH₂;
R² and R⁴ independently of one another represent-L-BINDER, H, -CO-CHY⁴-NHY⁵ or - (CH₂)₀₋₃Z, or R² and R⁴ together (with formation of a pyrrolidine ring) represent -CH₂-CHR¹⁰- or -CHR¹⁰-CH₂-, where R¹⁰ represents L-#1, H, NH₂, SO₃H, COOH, SH or OH, where Z represents -H, halogen, -OY³, -SY³, NHY³,-CO-NY¹Y² or -CO-OY³,
where Y¹ and Y² independently of one another represent H, NH₂ or - (CH₂) ₀-₃Z', and Y³ represents H or - (CH₂) ₀₋₃Z', where Z' represents H, SO₃H, NH₂ or COOH;
where Y⁴ independently of one another represents straight-chain or branched C₁₋₆ alkyl which is optionally substituted by -NHCONH₂, or represents aryl or benzyl which are optionally substituted by -NH₂, and Y⁵ represents H or -CO-CHY⁶-NH₂, where Y⁶ represents straight-chain or branched C₁₋₆-alkyl;
A represents CO, SO, SO₂, SO₂NH or CNNH;
R³ represents -L-BINDER or an optionally substituted alkyl, aryl, heteroaryl, heteroalkyl, heterocycloalkyl group, preferably -L-#1, or a C₁₋₁₀-alkyl, C₆₋₁₀-aryl or C₆₋₁₀-aralkyl, C₅₋₁₀-heteroalkyl, C₁₋₁₀-alkyl-O-C₆₋₁₀-aryl or C₅₋₁₀-heterocycloalkyl group which may be substituted by 1-3 -OH groups, 1-3 halogen atoms, 1-3 halogenated alkyl groups (each having 1-3 halogen atoms), 1-3 O-alkyl groups, 1-3 -SH groups, 1-3 -S-alkyl groups, 1-3 -O-CO-alkyl groups, 1-3 -O-CO-NH-alkyl groups, 1-3 -NH-CO-alkyl groups, 1-3 -NH-CO-NH-alkyl groups, 1-3 -S(O)ₙ-alkyl groups, 1-3 -SO₂-NH-alkyl groups, 1-3 -NH-alkyl groups, 1-3 -N(alkyl)₂ groups, 1-3 -NH₂ groups or 1-3 -(CH₂)₀₋₃Z groups, where Z represents -H, halogen, -OY³, -SY³, -NHY³, -CO-NY¹Y² or -CO-OY³, where Y¹ and Y² independently of one another represent H, NH₂ or -(CH₂)₀₋₃Z' and Y³ represents H, - (CH₂)₀₋₃-CH(NHCOCH₃)Z', - (CH₂)₀₋₃-CH(NH₂)Z' or - (CH₂)₀₋₃Z', where Z' represents H, SO₃H, NH₂ or COOH
(where "alkyl" preferably represents C₁₋₁₀-alkyl);
R⁵ represents -L-BINDER, H, F, NH₂, NO₂, halogen, SH or -(CH₂)₀₋₃Z, where Z represents -H, halogen,-OY³, -SY³, -NHY³, -CO-NY¹Y² or -CO-OY³,
where Y¹ and Y² independently of one another represent H, NH₂ or -(CH₂)₀₋₃Z', and Y³ represents H or -(CH₂)₀₋₃Z', where Z' represents H, SO₃H, NH₂ or COOH;
where L represents a linker and BINDER represents a binder or a derivative thereof, where the binder may optionally be attached to a plurality of active compound molecules,
R⁶ and R⁷ independently of one another represent H, cyano, (optionally fluorinated) C₁₋₁₀-alkyl, (optionally fluorinated) C₂₋₁₀-alkenyl, (optionally fluorinated) C₂₋₁₀-alkynyl, hydroxy or halogen,
R⁸ represents (optionally fluorinated) C₁₋₁₀-alkyl, (optionally fluorinated) C₄₋₁₀-cycloalkyl or optionally substituted oxetane; and
R⁹ represents H, F, CH₃, CF₃, CH₂F or CHF₂;
and the salts, solvates and salts of the solvates thereof.

46. Compounds of the general formula (IV): 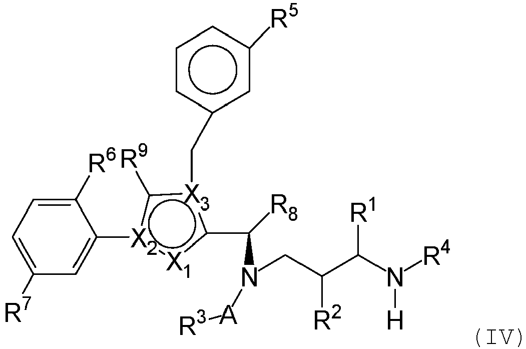 where
X₁ represents CH, X₂ represents C and X₃ represents N;
R¹ represents -L-BINDER, H or - (CH₂) ₀₋₃Z, where Z represents -H, -NHY³, -OY³, -SY³, halogen, -CO-NY¹Y² or -CO-OY³,
where Y¹ and Y² independently of one another represent H, NH₂, - (CH₂CH₂O) ₀₋₃-(CH₂) ₀₋₃Z' or-CH(CH₂W)Z', and Y³ represents H or -(CH₂)₀₋₃Z', where Z' represents H, NH₂, SO₃H, COOH, -NH-CO-CH₂-CH₂-CH(NH₂)COOH or - (CO-NH-CHY⁴)₁₋₃COOH; where W represents H or OH;
where Y⁴ independently of one another represents straight-chain or branched C₁₋₆-alkyl which is optionally substituted by -NHCONH₂, or represents aryl or benzyl which are optionally substituted by -NH₂;
R² and R⁴ independently of one another represent-L-BINDER, H, -CO-CHY⁴-NHY⁵ or - (CH₂) ₀₋₃Z, or R² and R⁴ together (with formation of a pyrrolidine ring) represent -CH₂-CHR¹⁰- or -CHR¹⁰-CH₂-, where R¹⁰ represents L-#1, H, NH₂, SO₃H, COOH, SH or OH, where Z represents -H, halogen, -OY³, -SY³, NHY³,-CO-NY¹Y² or -CO-OY³,
where Y¹ and Y² independently of one another represent H, NH₂ or - (CH₂) ₀₋₃Z', and Y³ represents H or - (CH₂) ₀₋₃Z', where Z' represents H, SO₃H, NH₂ or COOH;
where Y⁴ independently of one another represents straight-chain or branched C₁₋₆ alkyl which is optionally substituted by -NHCONH₂, or represents aryl or benzyl which are optionally substituted by -NH₂, and Y⁵ represents H or -CO-CHY⁶-NH₂, where Y⁶ represents straight-chain or branched C₁₋₆-alkyl;
A represents CO, SO, SO₂, SO₂NH or CNNH;
R³ represents -L-BINDER or an optionally substituted alkyl, aryl, heteroaryl, heteroalkyl or heterocycloalkyl group,
preferably -L-#1 or a C₁₋₁₀-alkyl, C₆₋₁₀-aryl, C₆₋₁₀-aralkyl, C₅₋₁₀-heteroalkyl, C₁₋₁₀-alkyl-O-C₆₋₁₀-aryl- or C₅₋₁₀-heterocycloalkyl group,
which may be substituted by 1-3 -OH groups, 1-3 halogen atoms, 1-3 halogenated alkyl groups (each having 1-3 halogen atoms), 1-3 O-alkyl groups, 1-3 -SH groups, 1-3 -S-alkyl groups, 1-3 -O-CO-alkyl groups, 1-3 -O-CO-NH-alkyl groups, 1-3 -NH-CO-alkyl groups, 1-3 -NH-CO-NH-alkyl groups, 1-3-S(O)ₙ-alkyl groups, 1-3 -SO₂-NH-alkyl groups, 1-3-NH-alkyl groups, 1-3 -N(alkyl)₂ groups, 1-3 -NH₂ groups or 1-3 -(CH₂)₀₋₃Z groups,
where Z represents -H, halogen, -OY³, -SY³, -NHY³, -CO-NY¹Y² or -CO-OY³, where Y¹ and Y² independently of one another represent H, NH₂ or -(CH₂)₀₋₃Z' and Y³ represents H, - (CH₂) ₀₋₃-CH(NHCOCH₃)Z', -(CH₂) ₀₋₃-CH(NH₂)Z' or -(CH₂)₀₋₃Z', where Z' represents H, SO₃H, NH₂ or COOH,
(where "alkyl" preferably represents C₁₋₁₀-alkyl);
R⁵ represents -L-BINDER, H, F, NH₂, NO₂, halogen, SH or -(CH₂)₀₋₃Z, where Z represents -H, halogen,-OY³, -SY³, -NHY³, -CO-NY¹Y² or -CO-OY³,
where Y¹ and Y² independently of one another represent H, NH₂ or -(CH₂)₀₋₃Z', and Y³ represents H or -(CH₂)₀₋₃Z', where Z' represents H, SO₃H, NH₂ or COOH;
where L represents a linker and BINDER represents a binder or a derivative thereof, where the binder may optionally be attached to a plurality of active compound molecules,
R⁶ and R⁷ independently of one another represent H, cyano, (optionally fluorinated) C₁₋₁₀-alkyl, (optionally fluorinated) C₂₋₁₀-alkenyl, (optionally fluorinated) C₂₋₁₀-alkynyl, hydroxy or halogen,
R⁸ represents (optionally fluorinated) C₁₋₁₀-alkyl, (optionally fluorinated) C₄₋₁₀-cycloalkyl or optionally substituted oxetane; and
R⁹ represents H, F, CH₃, CF₃, CH₂F or CHF₂;
and the salts, solvates and salts of the solvates thereof.

47. Compound according to Claim 46 where Z represents Cl or Br.

48. Compound according to Claim 46 where R¹ represents -(CH₂)₀₋₃Z, where Z represents -CO-NY¹Y², where Y² represents - (CH₂CH₂O)₀₋₃-(CH₂)₀₋₃Z' and Y¹ represents H, NH₂ or - (CH₂CH₂O)₀₋₃-(CH₂) ₀₋₃Z'.

49. Compound according to one or more of Claims 46-48 where Y¹ represents H, Y² represents - (CH₂CH₂O)₃-CH₂CH₂Z' and Z' represents -COOH.

50. Compound according to one or more of Claims 46-48, where Y¹ represents H, Y² represents -CH₂CH₂Z' and Z' represents -(CONHCHY⁴)₂COOH.

51. Compound according to Claim 46 where Y⁴ represents straight-chain or branched C₁₋₆-alkyl which is optionally substituted by -NHCONH₂.

52. Compound according to Claim 46 where at least one Y⁴ representative is selected from the group consisting of i-propyl and -CH₃.

53. Compound according to one or more of Claims 46 and 48 where Y¹ represents H, Y² represents -CH₂CH₂Z', Z' represents -CONHCHY⁴COOH and Y⁴ represents aryl or benzyl which are optionally substituted by -NH₂.

54. Compound according to Claim 53 where Y⁴ represents aminobenzyl.

55. Compound according to Claim 46 where R² represents -(CH₂)₀₋₃Z and Z represents -SY³.

56. Compound according to Claim 46 where R⁴ represents -CO-CHY⁴-NHY⁵ and Y⁵ represents H.

57. Compound according to Claim 46 where R⁴ represents -CO-CHY⁴-NHY⁵ and Y⁵ represents -CO-CHY⁶-NH₂.

58. Compound according to one or more of Claims 56 and 57 where Y⁴ represents straight-chain or branched C₁₋₆-alkyl which is optionally substituted by-NHCONH₂.

59. Compound according to one or more of Claims 46 to 58 where the compound has one of the formulae below:
N-(3-aminopropyl)-N-{(1R)-1-[4-benzyl-1-(2,5-difluorophenyl)-1H-pyrazol-3-yl]-2,2-dimethylpropyl}-2-hydroxyacetamide;
N-(3-aminopropyl)-N-{(1R)-1-[1-benzyl-4-(2,5-difluorophenyl)-1H-pyrrol-2-yl]-2,2-dimethylpropyl}-2-hydroxyacetamide;
(2S)-2-amino-4-[{(1R)-1-[1-benzyl-4-(2,5-difluorophenyl)-1H-pyrrol-2-yl]-2,2-dimethylpropyl} (glycoloyl)amino]-N-methylbutanamide (1:1) ;
N-(3-aminopropyl)-N-{(1S)-1-[1-benzyl-4-(2,5-difluorophenyl)-1H-pyrrol-2yl]-2,2-dimethylpropyl}acetamide.
N-(3-aminopropyl)-N-{(1S)-1-[1-benzyl-4-(2,5-difluorophenyl)-1H-pyrrol-2-yl]-2,2-dimethylpropyl}-2-hydroxyacetamide;
S-[1-(2-{[2-({(2S)-2-amino-4-[{(1R)-1-[4-benzyl-1-(2,5-difluorophenyl)-1H-pyrazol-3-yl]-2,2-dimethylpropyl}(glycoloyl)amino]butanoyl}amino)ethyl]am ino}-2-oxoethyl)-2,5-dioxopyrrolidin-3-yl]-L-cysteine;
N-{(2S)-2-amino-4-[{(1R)-1-[4-benzyl-1-(2,5-difluorophenyl)-1H-pyrazol-3-yl]-2,2-dimethylpropyl} (glycoloyl)amino]butanoyl}-beta-alanyl-L-alanyl-N-[4-(3-{[(2R)-2-amino-2-carboxyethyl]sulphanyl}-2,5-dioxopyrrolidin-1-yl)phenyl]-N⁵-carbamoyl-L-ornithinamide;
S-(1-{2-[(N-{(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}-beta-alanyl)amino]ethyl}-2,5-dioxopyrrolidin-3-yl)-L-cysteine;
S-[1-(2-{[2-({(2S)-2-amino-4-[{(1R)-1-[4-benzyl-1-(2,5-difluorophenyl)-1H-pyrazol-3-yl]-2,2-dimethylpropyl}(glycoloyl)amino]butanoyl}amino)ethyl]am ino}-2-oxoethyl)-2,5-dioxopyrrolidin-3-yl]-L-cysteine;
N-{(2S)-2-amino-4-[{(1R)-1-[4-benzyl-1-(2,5-difluorophenyl)-1H-pyrazol-3-yl]-2,2-dimethylpropyl} (glycoloyl)amino]butanoyl}-beta-alanyl-L-alanyl-N-[4-(3-{[(2R)-2-amino-2-carboxyethyl]sulphanyl}-2,5-dioxopyrrolidin-1-yl)phenyl]-N⁵-carbamoyl-L-ornithinamide;
S-(1-{2-[(N-{(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}-beta-alanyl)amino]ethyl}-2,5-dioxopyrrolidin-3-yl)-L-cysteine;
N-[6-(3-{[(2R)-2-amino-2-carboxyethyl]sulphanyl}-2,5-dioxopyrrolidin-1-yl)hexanoyl]-L-valyl-N⁵-carbamoyl-L-ornithyl-N⁶-{(2S)-2-amino-4-[{(1R)-1-[4-benzyl-1-(2,5-difluorophenyl)-1H-pyrazol-3-yl]-2,2-dimethylpropyl} (glycoloyl)amino]butanoyl}-L-lysine;
S-[1-(2-{[2-({(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}amino)ethyl]am ino}-2-oxoethyl)-2,5-dioxopyrrolidin-3-yl]-L-cysteine;
S-(2-{[2-({(2S)-2-amino-4-[{(1R)-1-[4-benzyl-1-(2,5-difluorophenyl)-1H-pyrazol-3-yl]-2,2-dimethylpropyl}(glycoloyl)amino]butanoyl}amino)ethyl]am ino}-2-oxoethyl)-L-cysteine;
S-{1-[6-(2-{(2S)-2-amino-4-[{(1R)-1-[4-benzyl-1-(2,5-difluorophenyl)-1H-pyrazol-3-yl]-2,2-dimethylpropyl} (glycoloyl)amino]butanoyl}hydrazino)-6-oxohexyl]-2,5-dioxopyrrolidin-3-yl}-L-cysteine;
N-[19-(3(R/S)-{[(2R)-2-amino-2-carboxyethyl]sulphanyl}-2,5-dioxopyrrolidin-1-yl)-17-oxo-4,7,10,13-tetraoxa-16-azanonadecan-1-oyl]-R/S-{2-[(3-aminopropyl){(1R)-1-[1-benzyl-4-(2,5-difluorophenyl)-1H-pyrrol-2-yl]-2,2-dimethylpropyl}amino]-2-oxoethyl}homocysteine;
S-{(3R/S)-1-[2-({(2S)-2-amino-4-[{(1R)-1-[1-benzyl-4-(2,5-difluorophenyl)-1H-pyrrol-2-yl]-2,2-dimethylpropyl} (glycoloyl)amino]butanoyl}amino)ethyl]-2,5-dioxopyrrolidin-3-yl}-L-cysteine;
N-[19-(3(R/S)-{[(2R)-2-amino-2-carboxyethyl]sulphanyl}-2,5-dioxopyrrolidin-1-yl)-17-oxo-4,7,10,13-tetraoxa-16-azanonadecan-1-oyl]-R/S-{2-[(3-aminopropyl){(1R)-1-[1-benzyl-4-(2,5-difluorophenyl)-1H-imidazol-2-yl]-2,2-dimethylpropyl}amino]-2-oxoethyl}homocysteine;
S-[(3R/S)-1-(2-{[6-({2-[(3-aminopropyl){(1R)-1-[1-benzyl-4-(2,5-difluorophenyl)-1H-pyrrol-2-yl]-2,2-dimethylpropyl}amino]-2-oxoethyl}sulphanyl)hexanoyl]amino}ethyl)-2,5-dioxopyrrolidin-3-yl]-L-cysteine;
S-{1-[2-({[(1R,3S)-3-({(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}amino)cyclopen tyl]carbonyl}amino)ethyl]-2,5-dioxopyrrolidin-3-yl}-L-cysteine;
S-(2-{[2-({(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}amino)ethyl]am ino}-2-oxoethyl)-L-cysteine;
N⁶-(N-1(2S)-2-amino-4-[1(1R)-1-[1-benzyl-4-(2,5-difluorophenyl)-1H-pyrrol-2-yl]-2,2-dimethylpropyl}(glycoloyl)amino]butanoyl}-beta-alanyl)-N²-{N-[6-(3-{[(2R)-2-amino-2-carboxyethyl]sulphanyl}-2,5-dioxopyrrolidin-1-yl)hexanoyl]-L-valyl-L-alanyl}-L-lysine;
N-[2-({(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}amino)ethyl]-L-glutamine;
N⁶-(N-{(2S)-2-amino-4-[{(1R)-1-[1-benzyl-4-(2,5-difluorophenyl)-1H-pyrrol-2-yl]-2,2-dimethylpropyl} (glycoloyl)amino]butanoyl}-beta-alanyl)-L-lysine;
N-(3-aminopropyl)-N-{(1R)-1-[4-benzyl-1-(2,5-difluorophenyl)-1H-pyrazol-3-yl]-2,2-dimethylpropyl}acetamide;
N-(3-aminopropyl)-N-{(1R)-1-[4-benzyl-1-(2,5-difluorophenyl)-1H-pyrazol-3-yl]-2,2-dimethylpropyl}-2-methoxyacetamide;
N-(3-aminopropyl)-N-{(1R)-1-[4-benzyl-1-(2,5-difluorophenyl)-1H-pyrazol-3-yl]-2,2-dimethylpropyl}-2,4-difluorobenzamide;
N-(3-aminopropyl)-N-{(1R)-1-[4-benzyl-1-(2,5-difluorophenyl)-1H-pyrazol-3-yl]-2,2-dimethylpropyl}-4-methylbenzamide;
N-(3-aminopropyl)-N-{(1R)-1-[4-benzyl-1-(2,5-difluorophenyl)-1H-pyrazol-3-yl]-2,2-dimethylpropyl}-2-ethoxyacetamide;
N-(3-aminopropyl)-N-{(1R)-1-[1-benzyl-4-(2,5-difluorophenyl)-1H-pyrrol-2-yl]-2,2-dimethylpropyl}-3,3,3-trifluoropropanamide;
N-(3-aminopropyl)-N-{(1R)-1-[1-benzyl-4-(2,5-difluorophenyl)-1H-pyrrol-2-yl]-2,2-dimethylpropyl}-4-fluorobenzamide;
N-(3-aminopropyl)-N-{(1R)-1-[1-benzyl-4-(2,5-difluorophenyl)-1H-pyrrol-2-yl]-2,2-dimethylpropyl}acetamide;
N-(3-aminopropyl)-N-{(1R)-1-[1-benzyl-4-(2,5-difluorophenyl)-1H-pyrrol-2-yl]-2,2-dimethylpropyl}-4-(trifluoromethyl)benzamide;
N-(3-aminopropyl)-N-{(1R)-1-[4-benzyl-1-(2,5-difluorophenyl)-1H-pyrazol-3-yl]-2,2-dimethylpropyl}-2-ethoxyacetamide;
N-(3-aminopropyl)-N-{(1R)-1-[4-benzyl-1-(2,5-difluorophenyl)-1H-pyrazol-3-yl]-2,2-dimethylpropyl}-2-ethoxyacetamide;
(2S)-2-amino-4-[{(1R)-1-[1-benzyl-4-(2,5-difluorophenyl)-1H-pyrrol-2-yl]-2,2-dimethylpropyl} (glycoloyl)amino]butanoic acid;
(2S)-2-amino-N-(2-aminoethyl)-4-[{(1R)-1-[1-benzyl-4-(2,5-difluorophenyl)-1H-pyrrol-2-yl]-2,2-dimethylpropyl} (glycoloyl)amino]butanamide;
4-[(2-{[2-({(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}amino)ethyl]am ino}-2-oxoethyl)amino]-3-{[(2R)-2-amino-2-carboxyethyl]sulphanyl}-4-oxobutanoic acid;
4-[(2-{[2-({(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}amino)ethyl]am ino}-2-oxoethyl)amino]-2-{[(2R)-2-amino-2-carboxyethyl]sulphanyl}-4-oxobutanoic acid;
N-{(2S)-2-amino-4-[{(1R)-1-[1-benzyl-4-(2,5-difluorophenyl)-1H-pyrrol-2-yl]-2,2-dimethylpropyl} (glycoloyl)amino]butanoyl}-beta-alanine;
N-{(2S)-2-amino-4-[{(1R)-1-[1-benzyl-4-(2,5-difluorophenyl)-1H-pyrrol-2-yl]-2,2-dimethylpropyl} (glycoloyl)amino]butanoyl}-L-serine;
N-{(2S)-2-amino-4-[{(1R)-1-[1-benzyl-4-(2,5-difluorophenyl)-1H-pyrrol-2-yl]-2,2-dimethylpropyl}(glycoloyl)amino]butanoyl}-L-alanine;
N-{(2S)-2-amino-4-[{(1R)-1-[1-benzyl-4-(2,5-difluorophenyl)-1H-pyrrol-2-yl]-2,2-dimethylpropyl} (glycoloyl)amino]butanoyl}glycine;
N-(3-aminopropyl)-N-{(1R)-1-[1-benzyl-4-(2,5-difluorophenyl)-1H-pyrrol-2-yl]-2,2-dimethylpropyl}-4-methylbenzamide;
N-(3-aminopropyl)-N-{(1R)-1-[1-benzyl-4-(2,5-difluorophenyl)-1H-pyrrol-2-yl]-2,2-dimethylpropyl}-4-(methylsulphanyl)benzamide;
(2S)-N-(3-aminopropyl)-N-{(1R)-1-[1-benzyl-4-(2,5-difluorophenyl)-1H-pyrrol-2-yl]-2,2-dimethylpropyl}-2-hydroxypropanamide;
N-(3-aminopropyl)-N-{(1R)-1-[1-benzyl-4-(2,5-difluorophenyl)-1H-pyrrol-2-yl]-2,2-dimethylpropyl}-2-(methylsulphanyl)acetamide;
(2S)-N-(3-aminopropyl)-N-{(1R)-1-[4-benzyl-1-(2,5-difluorophenyl)-1H-pyrazol-3-yl]-2,2-dimethylpropyl}-2-hydroxypropanamide;
methyl 4-[(3-aminopropyl)f(1R)-1-[1-benzyl-4-(2,5-difluorophenyl)-1H-pyrrol-2-yl]-2,2-dimethylpropyl}amino]-4-oxobutanoate;
4-[(3-aminopropyl){(1R)-1-[1-benzyl-4-(2,5-difluorophenyl)-1H-pyrrol-2-yl]-2,2-dimethylpropyl}amino]-4-oxobutanoic acid;
(2R)-22-[(3R/S)-3-{[(2R)-2-amino-2-carboxyethyl]sulphanyl}-2,5-dioxopyrrolidin-1-yl]-2-[({2-[(3-aminopropyl){(1R)-1-[1-benzyl-4-(2,5-difluorophenyl)-1H-pyrrol-2-yl]-2,2-dimethylpropyl}amino]-2-oxoethyl}sulphanyl)methyl]-4,20-dioxo-7,10,13,16-tetraoxa-3,19-diazadocosan-1-oic acid;
4-amino-N-(3-aminopropyl)-N-{(1R)-1-[4-benzyl-1-(2,5-difluorophenyl)-1H-pyrazol-3-yl]-2,2-dimethylpropyl}benzamide;
N-acetyl-S-{2-[(3-aminopropyl){(1R)-1-[1-benzyl-4-(2,5-difluorophenyl)-1H-pyrrol-2-yl]-2,2-dimethylpropyl}amino]-2-oxoethyl}-L-cysteine;
N-acetyl-S-[2-([3-(L-alanylamino)propyl]{(1R)-1-[1-benzyl-4-(2,5-difluorophenyl)-1H-pyrrol-2-yl]-2,2-dimethylpropyl}amino)-2-oxoethyl]-L-cysteine;
(2S)-N-(3-aminopropyl)-N-{(1R)-1-[1-benzyl-4-(2,5-difluorophenyl)-1H-pyrrol-2-yl]-2,2-dimethylpropyl}tetrahydrofuran-2-carboxamide;
3-({2-[(3-aminopropyl){(1R)-1-[1-benzyl-4-(2,5-difluorophenyl)-1H-pyrrol-2-yl]-2,2-dimethylpropyl}amino]-2-oxoethyl}sulphanyl)propanoic acid;
S-{2-[(3-aminopropyl){(1R)-1-[1-benzyl-4-(2,5-difluorophenyl)-1H-pyrrol-2-yl]-2,2-dimethylpropyl}amino]-2-oxoethyl}homocysteine;
4-amino-N-(3-aminopropyl)-N-{(1R)-1-[1-benzyl-4-(2,5-difluorophenyl)-1H-pyrrol-2-yl]-2,2-dimethylpropyl}benzamide;
4-[(2-{[(2R)-2-({(2S)-2-amino-4-[{(1R)-1-[1-benzyl-4-(2,5-difluorophenyl)-1H-pyrrol-2-yl]-2,2-dimethylpropyl} (glycoloyl)amino]butanoyl}amino)-2-carboxyethyl]amino}-2-oxoethyl)amino]-3-{[(2R)-2-amino-2-carboxyethyl]sulphanyl}-4-oxobutanoic acid;
4-[(2-{[2R)-2-({(2S)-2-amino-4-[{(1R)-1-[1-benzyl-4-(2,5-difluorophenyl)-1H-pyrrol-2-yl]-2,2-dimethylpropyl} (glycoloyl)amino]butanoyl}amino)-2-carboxyethyl]amino}-2-oxoethyl)amino]-2-{[(2R)-2-amino-2-carboxyethyl]sulphanyl}-4-oxobutanoic acid.

60. Pharmaceutical composition comprising a conjugate according to one or more of Claims 1 to 41 or a compound according to Claims 46-59 in combination with an inert non-toxic pharmaceutically suitable auxiliary.

61. Conjugate according to one or more of Claims 1 to 41 or compound according to Claims 46-59 for use in a method for the treatment and/or prophylaxis of diseases.

62. Conjugate according to one or more of Claims 1 to 41 or compound according to Claims 46-59 for use in a method for the treatment of hyperproliferative and/or angiogenic disorders.

63. Conjugate according to one or more of Claims 1 to 41 or compound according to Claims 46-59 where R^{3a} or R³ represents -L-BINDER or a substituted alkyl, aryl or heteroaryl group,
preferably -L-#1 or a C₁₋₁₀-alkyl,C₆₋₁₀-aryl or C₆₋₁₀-aralkyl group or C₅₋₁₀-heteroalkyl, which may be substituted by -OH, O-alkyl, SH, S-alkyl, O-CO-alkyl, O-CO-NH-alkyl, NH-CO-alkyl, NH-CO-NH-alkyl, S(O)ₙ-alkyl, SO₂-NH-alkyl, NH-alkyl, N(alkyl)₂, NH₂ or -(CH₂)₀₋₃Z, where Z represents -H, halogen, -OY³, -SY³, -NHY³, -CO-N¹Y² or -CO-OY³, where Y¹ and Y² independently of one another represent H, NH₂ or-(CH₂)₀₋₃Z' and Y³ represents H or -(CH₂)₀₋₃Z', where Z' represents H, SO₃H, NH₂ or COOH,
(where "alkyl" preferably represents C₁₋₁₀-alkyl).

64. Conjugate according to one or more of Claims 1 to 41 or compound according to one of Claims 46 to 59 for use in a method for the treatment and/or prophylaxis of hyperproliferative and/or angiogenic disorders in humans and animals using an effective amount.

65. Antibody conjugate according to one of the formulae below, where n is a number from 1 to 20 and AK_{1A} and AK_{2A} represent an antibody: 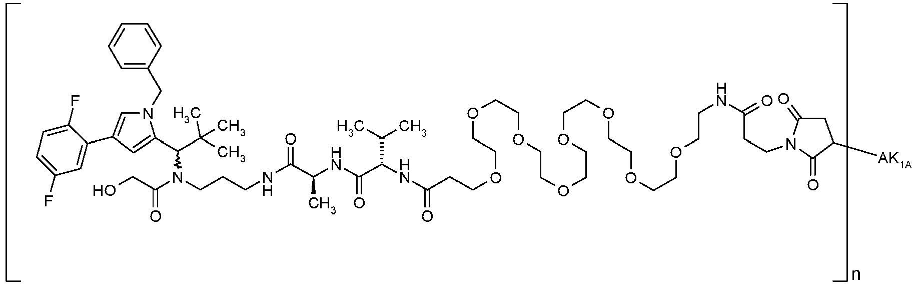 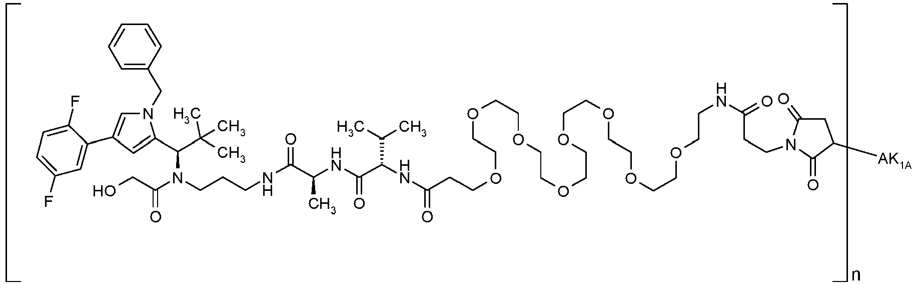 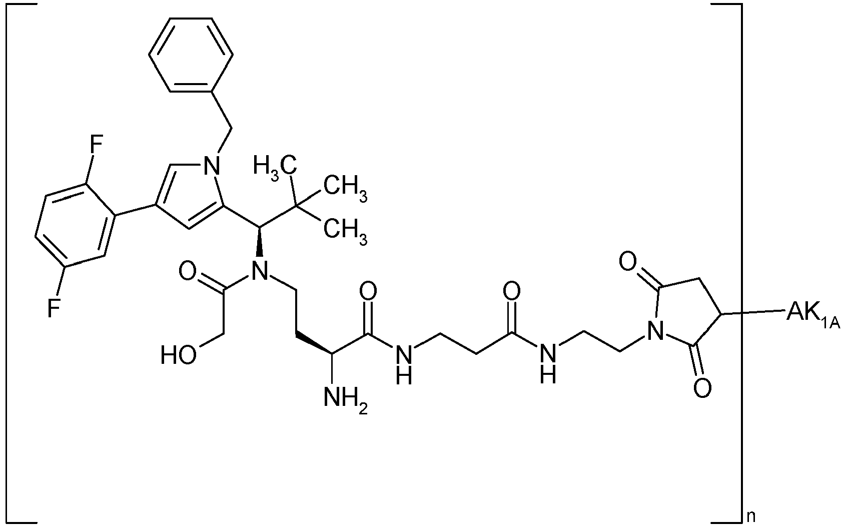 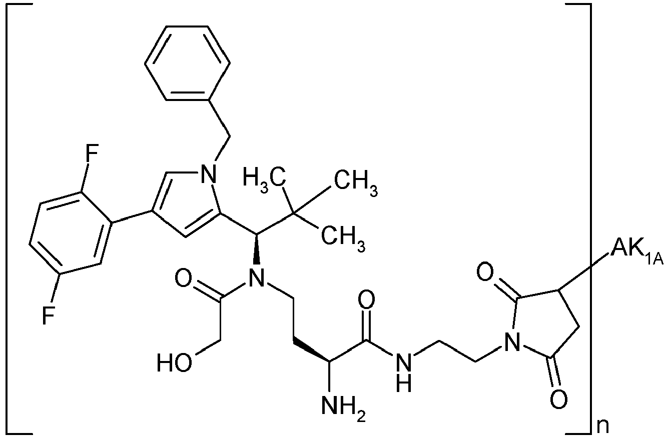 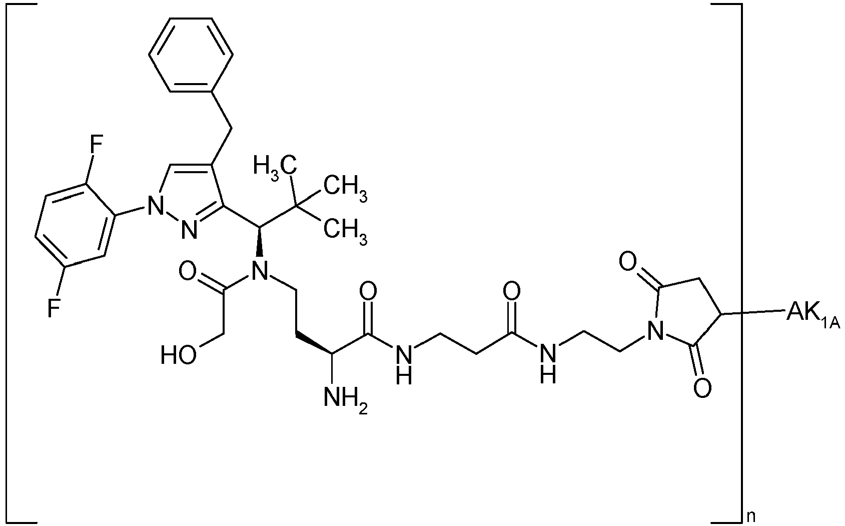 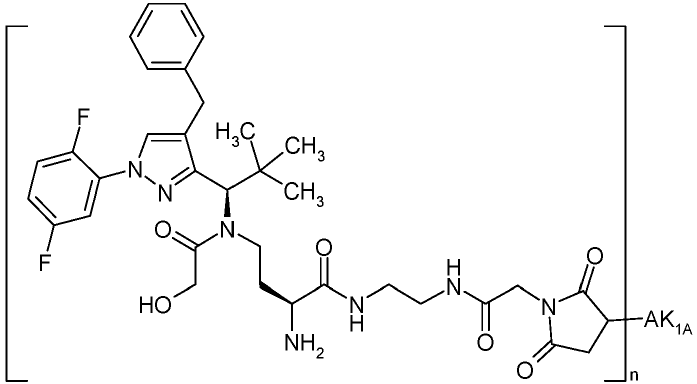 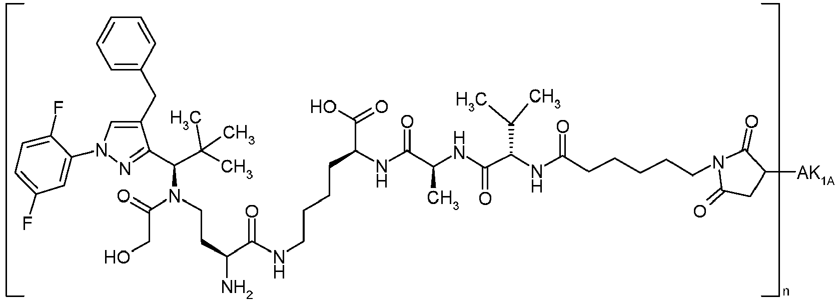 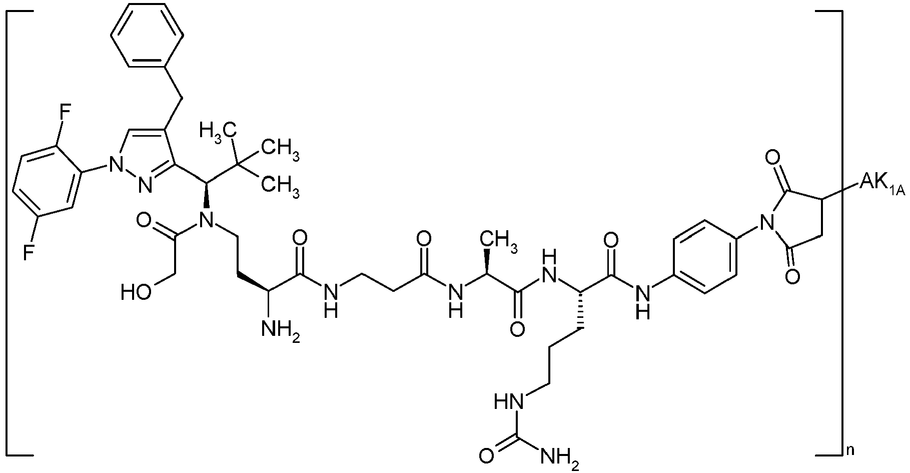 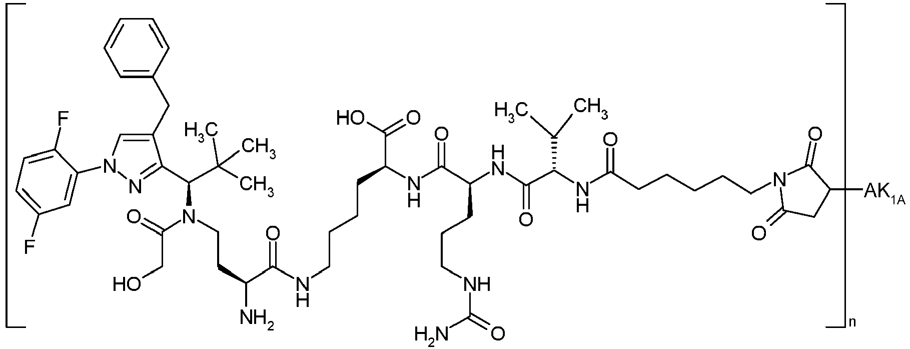 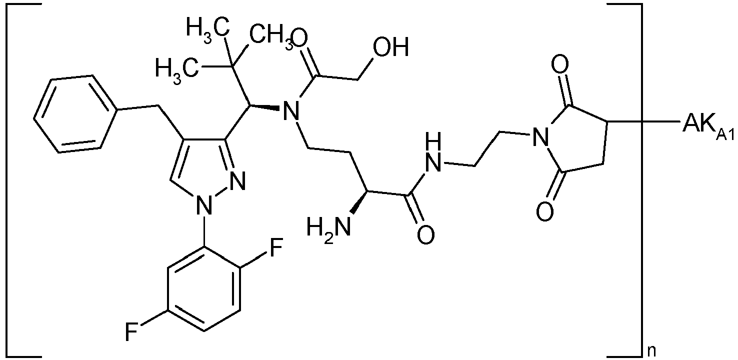 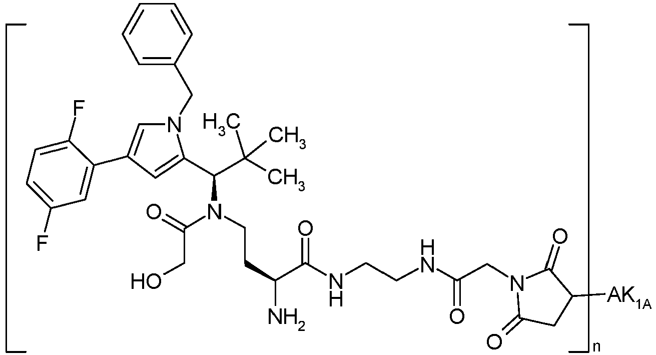 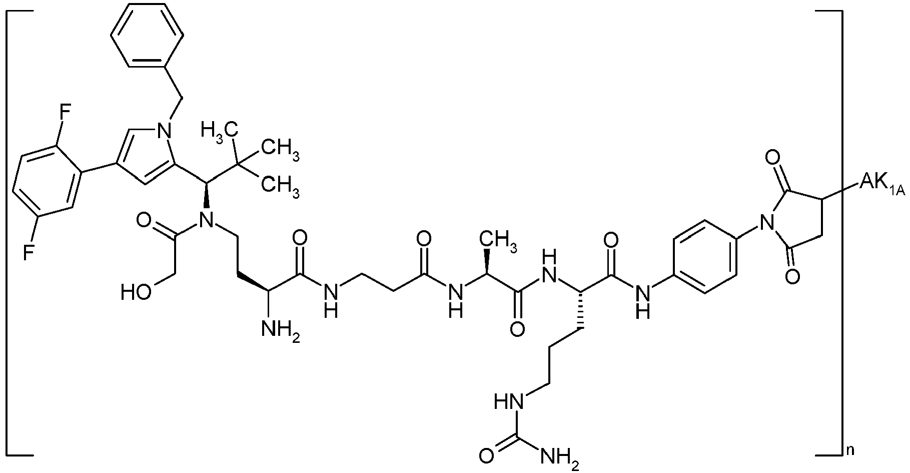 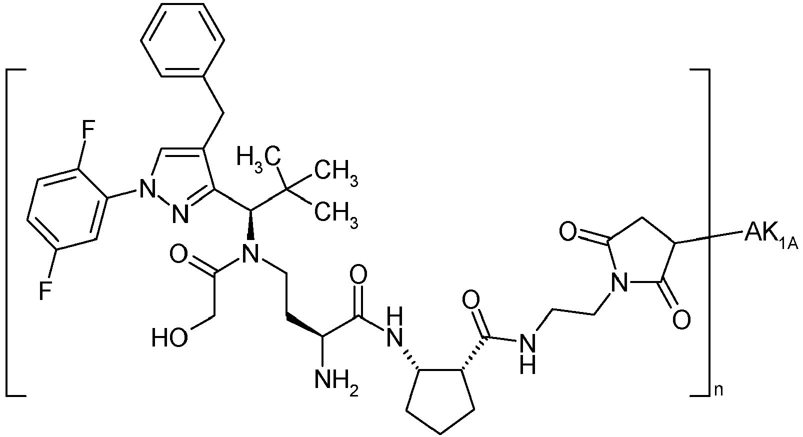 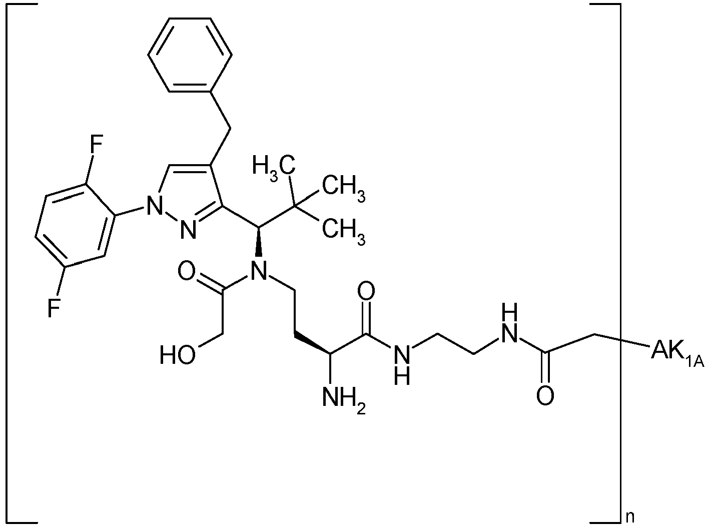 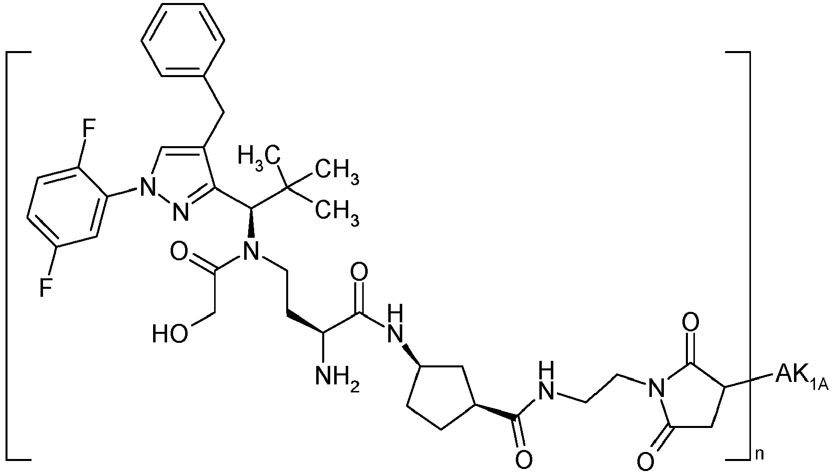 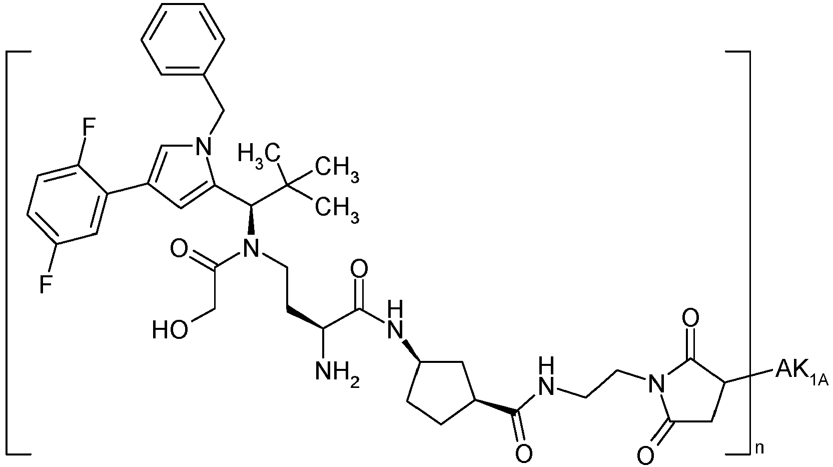 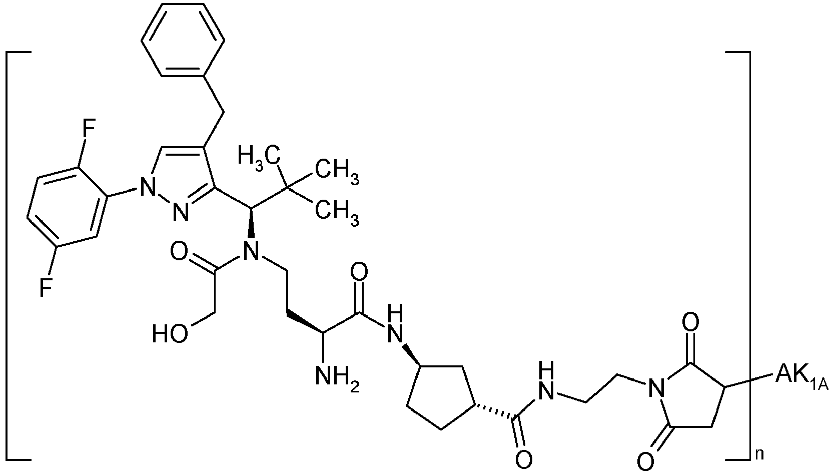 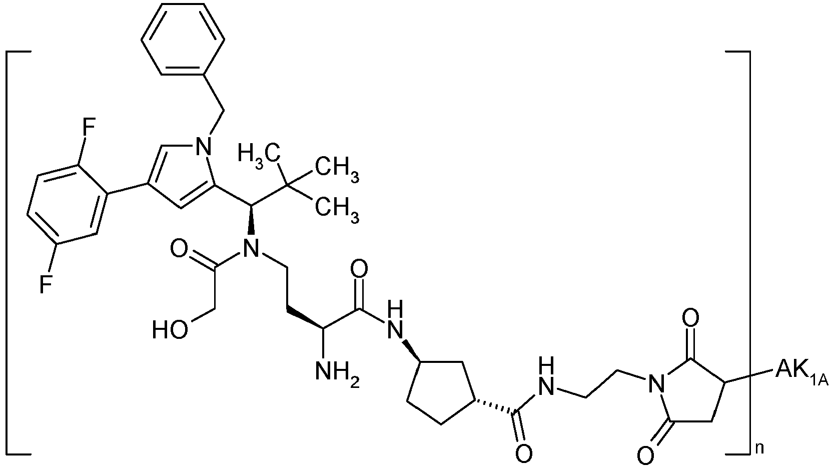 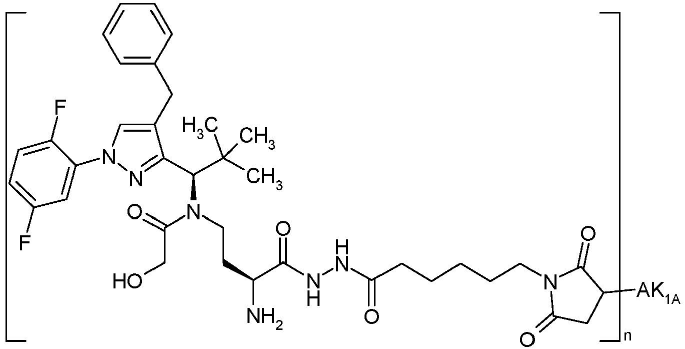 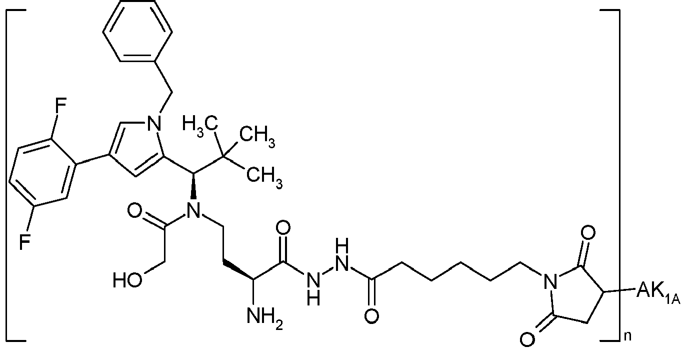 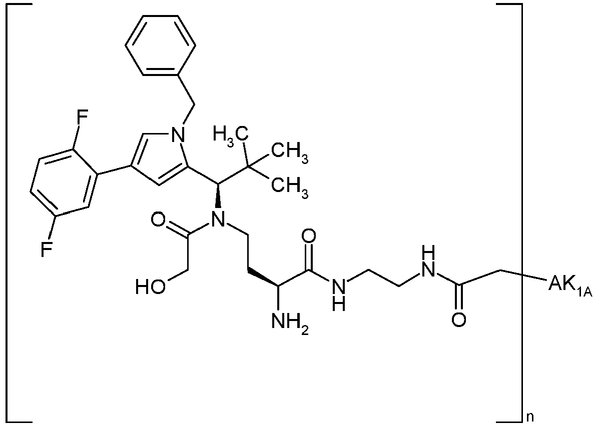 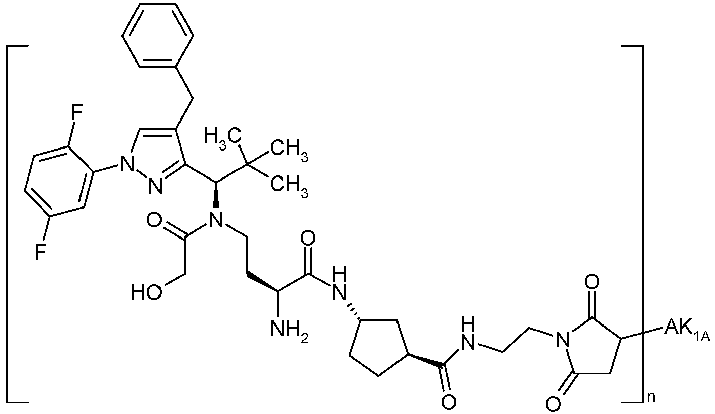 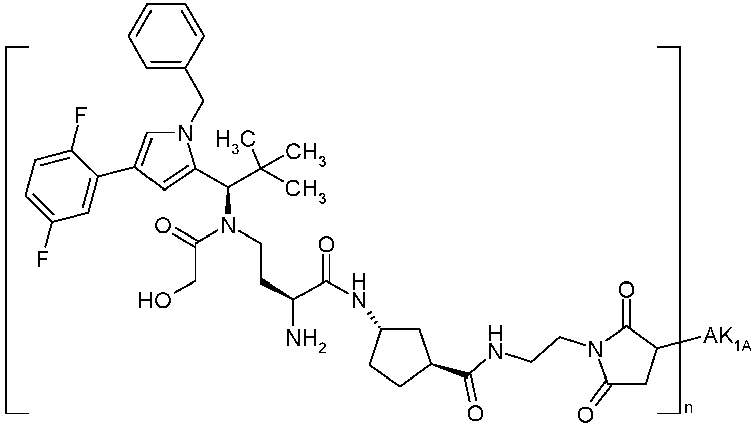 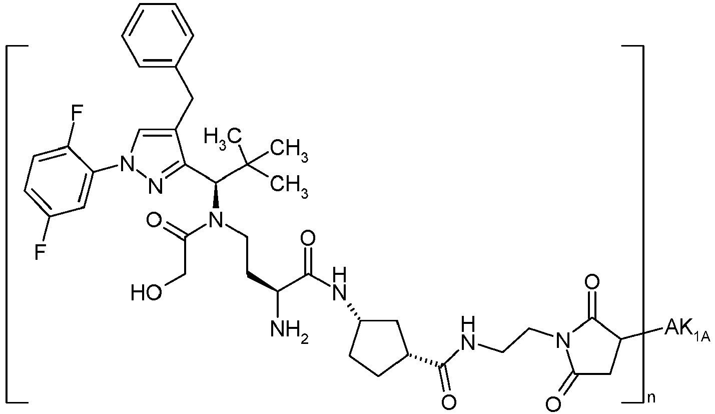 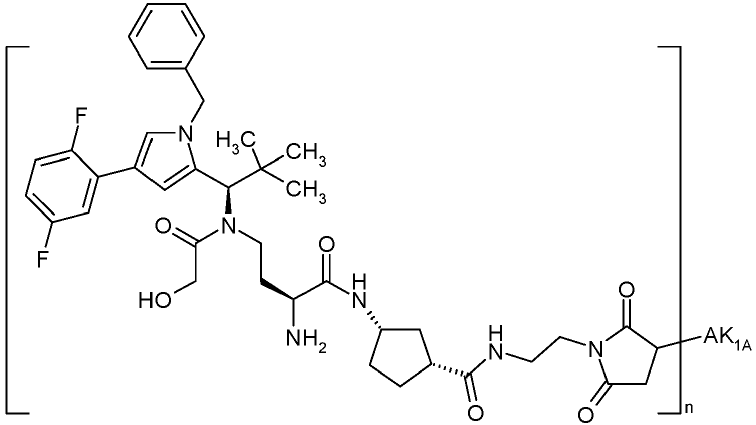 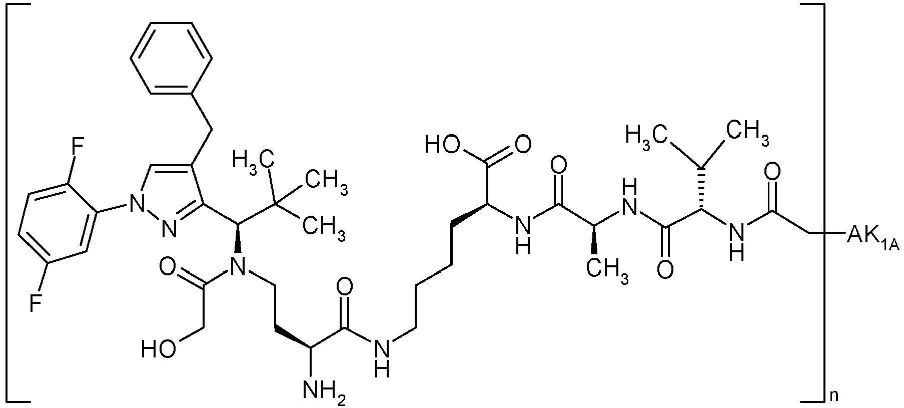 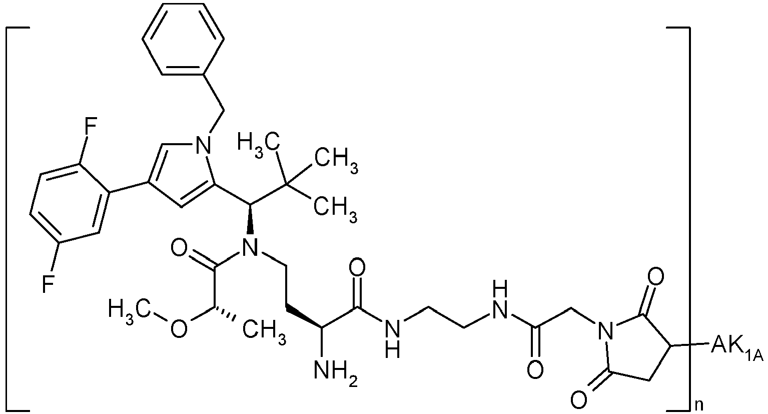 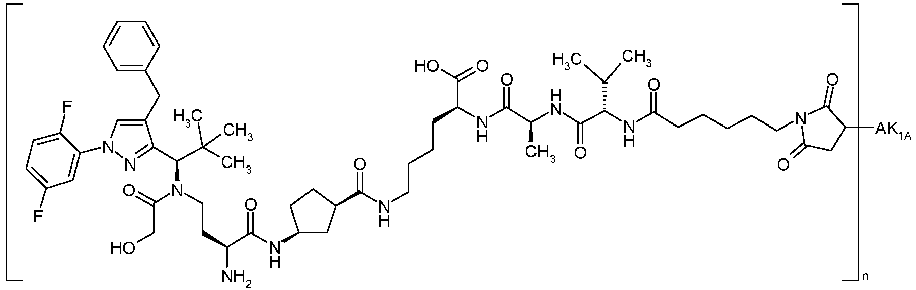 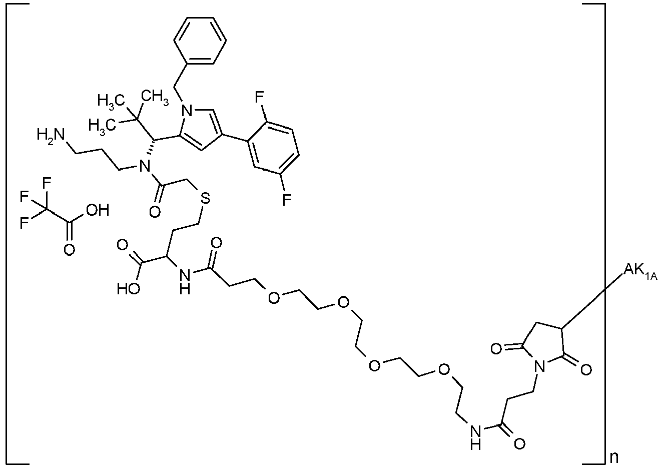 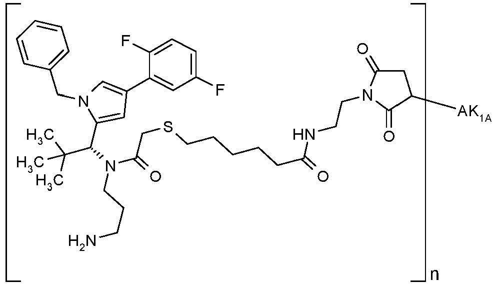 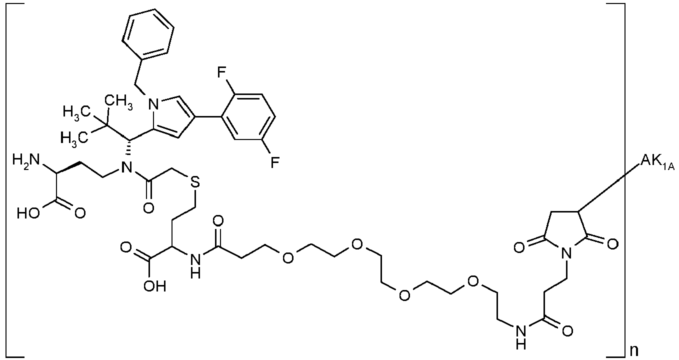 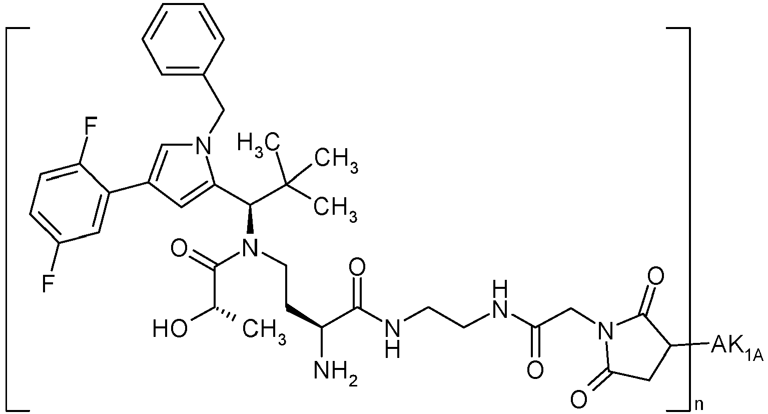 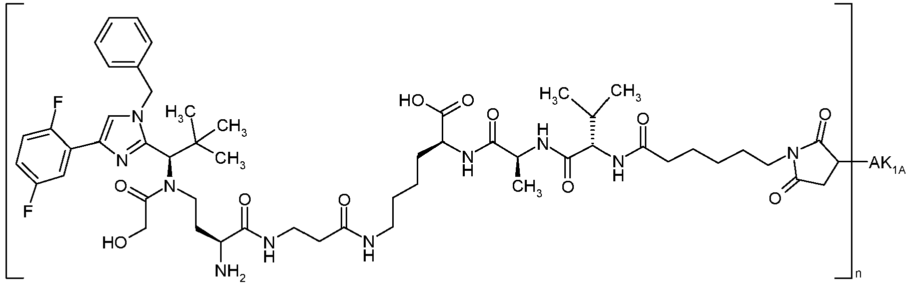 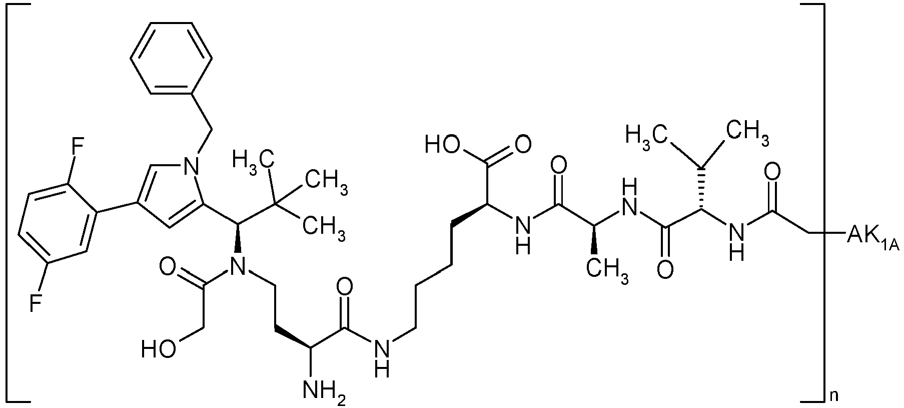 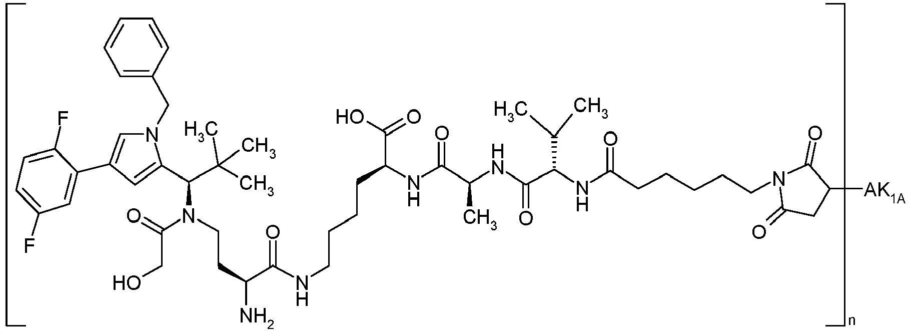 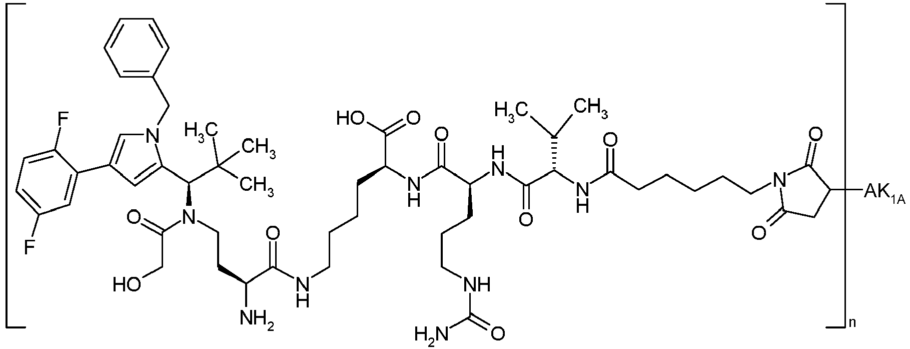 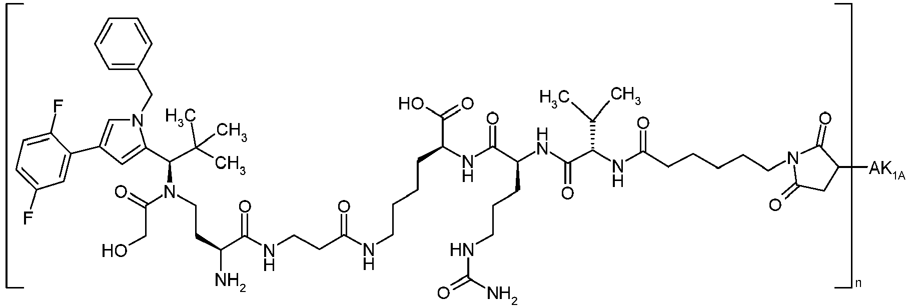 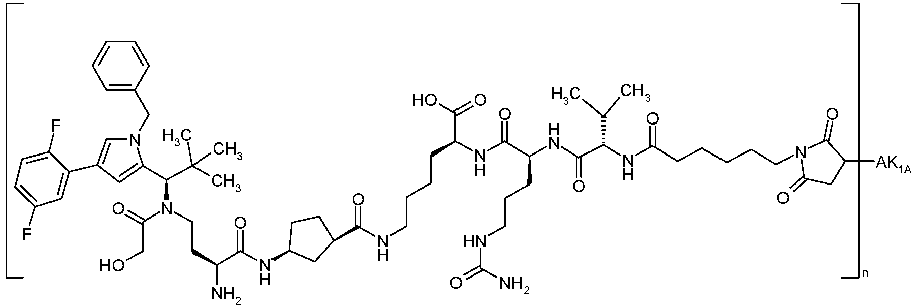 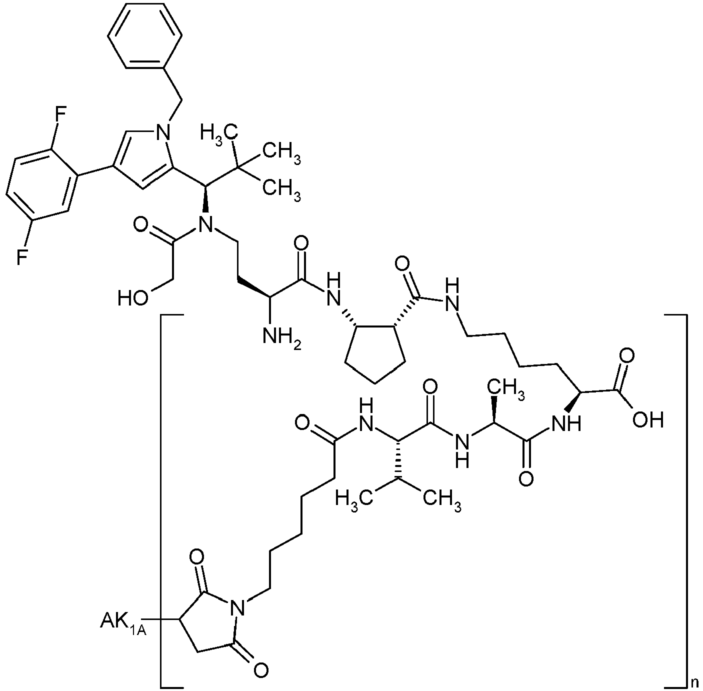 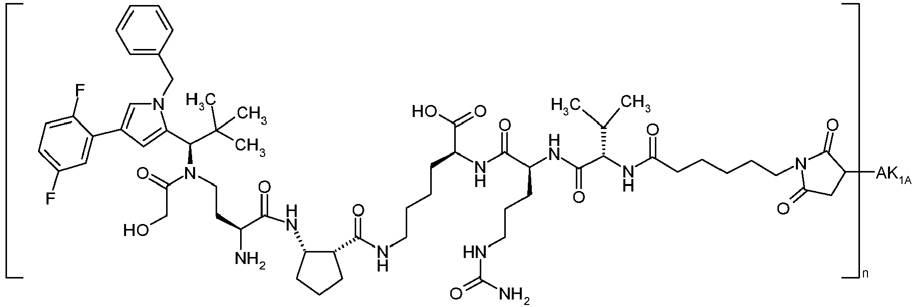 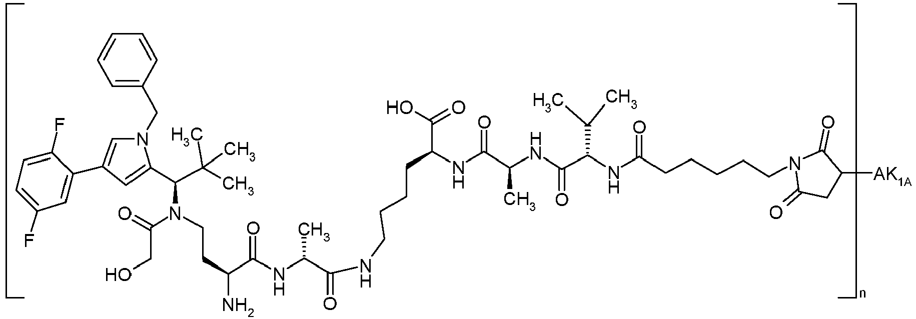 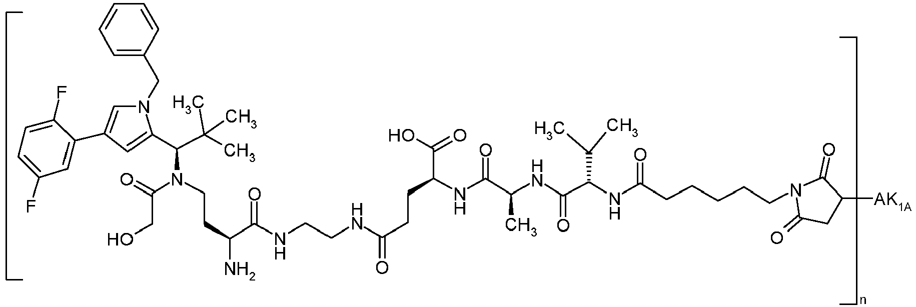 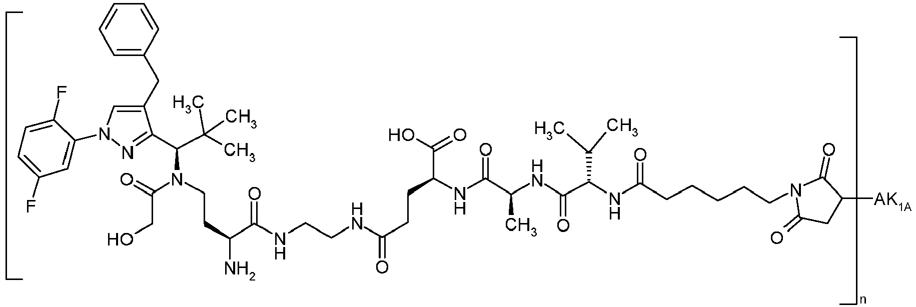 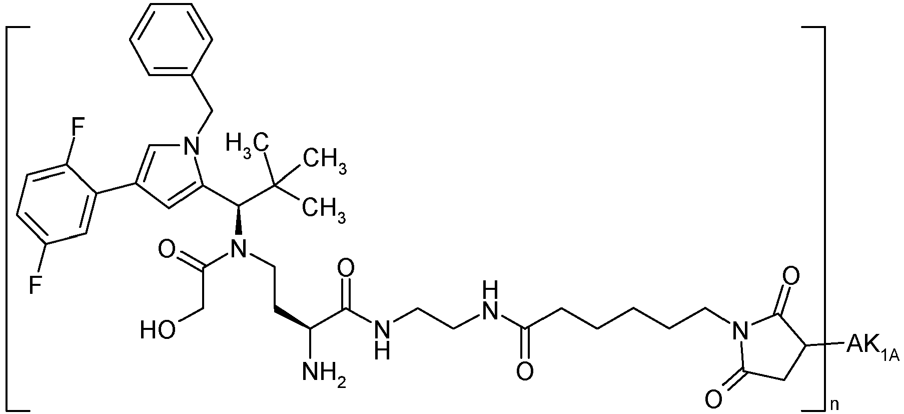 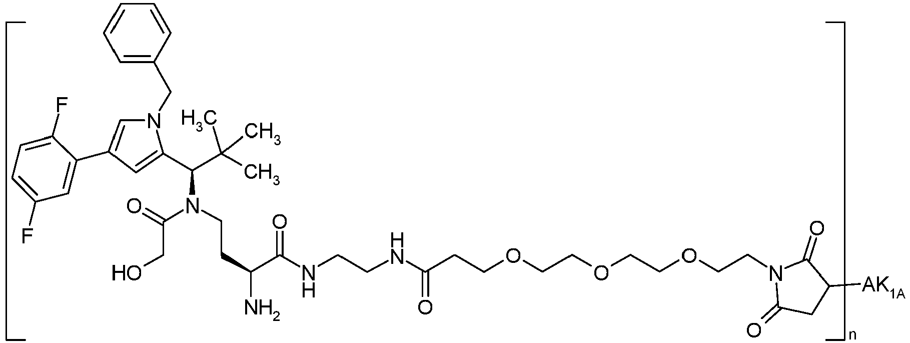 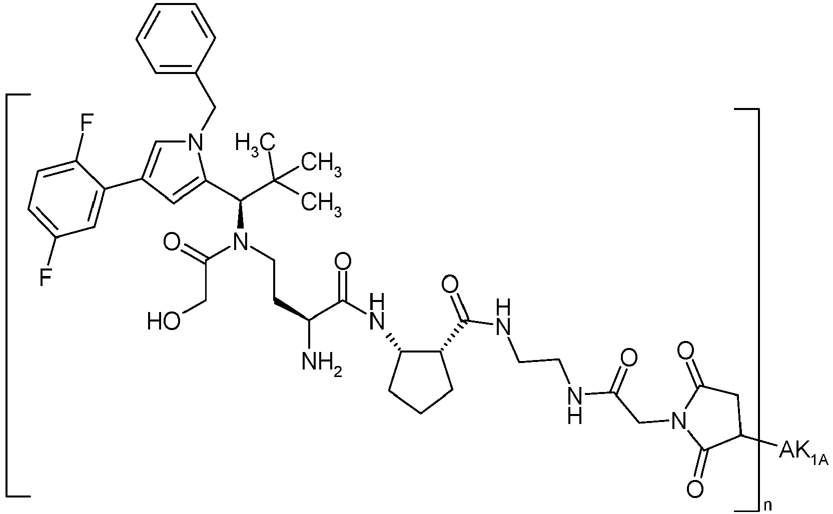 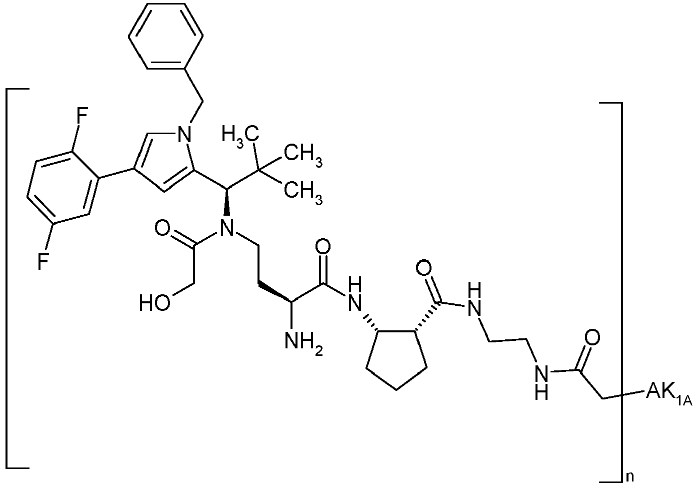 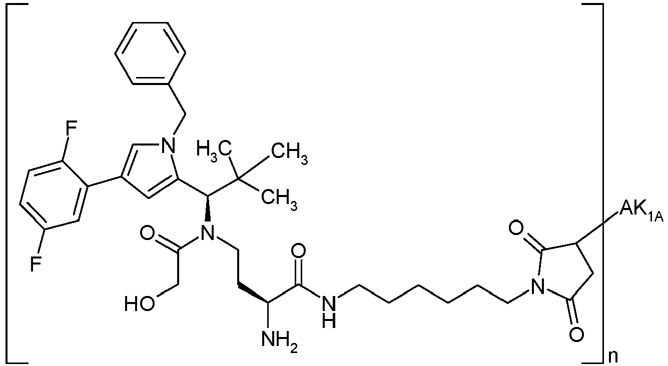 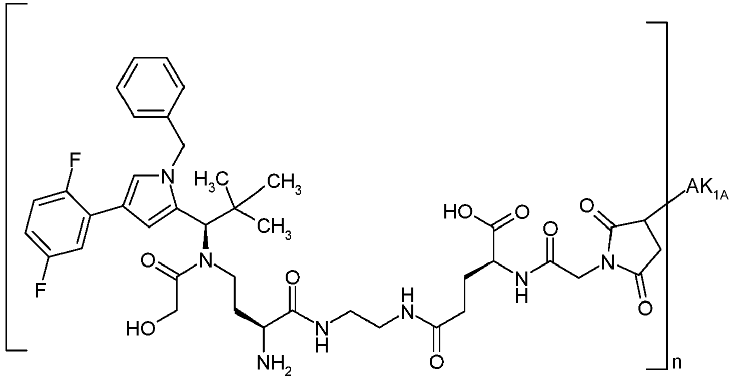 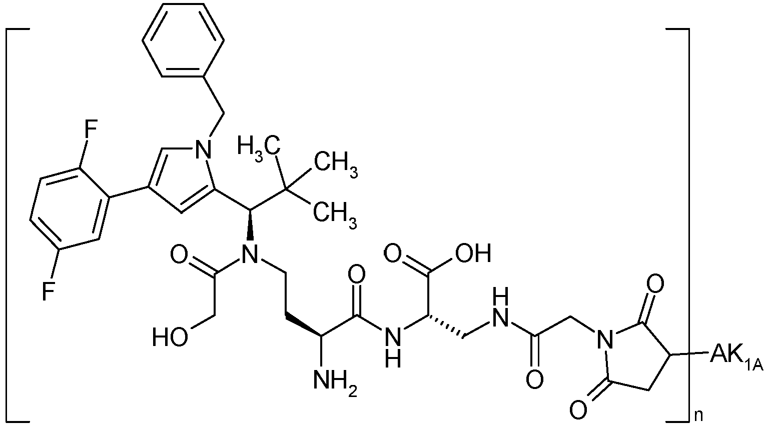 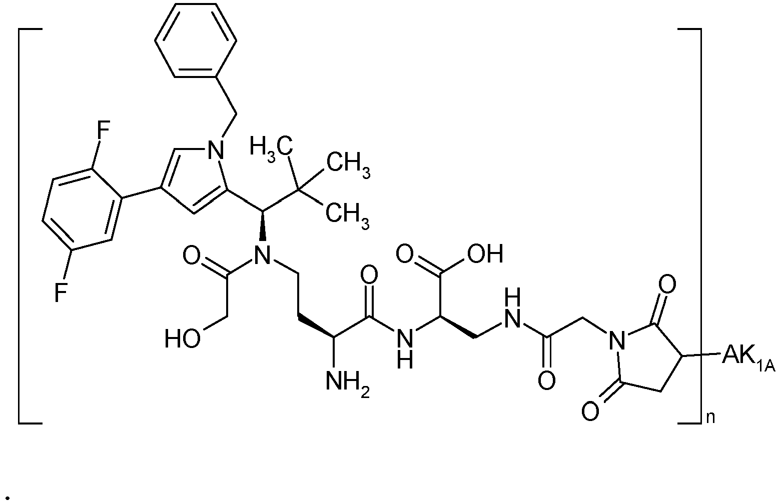 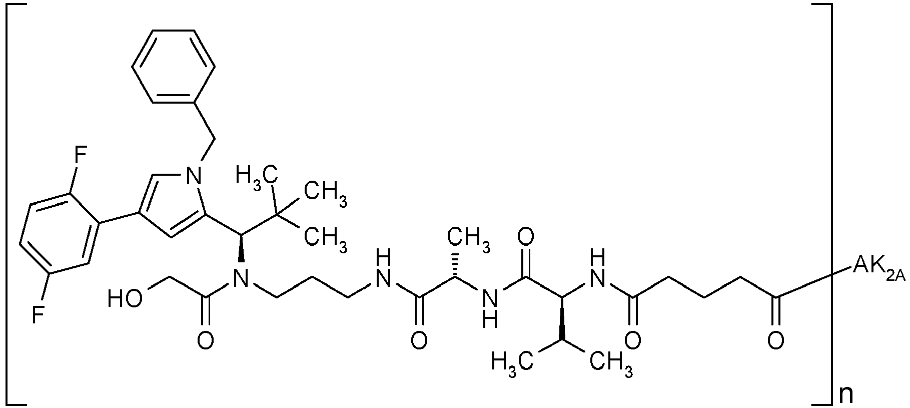 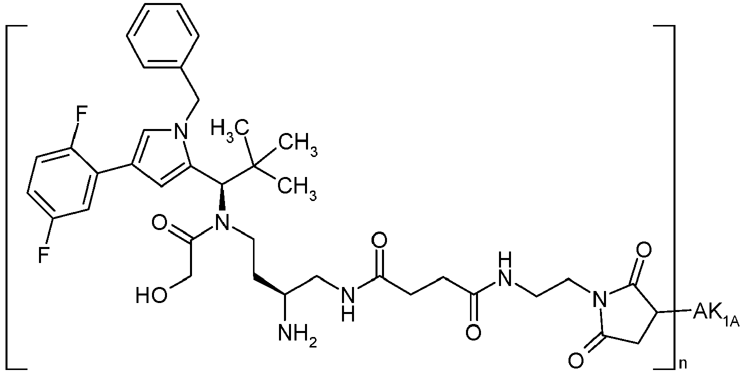 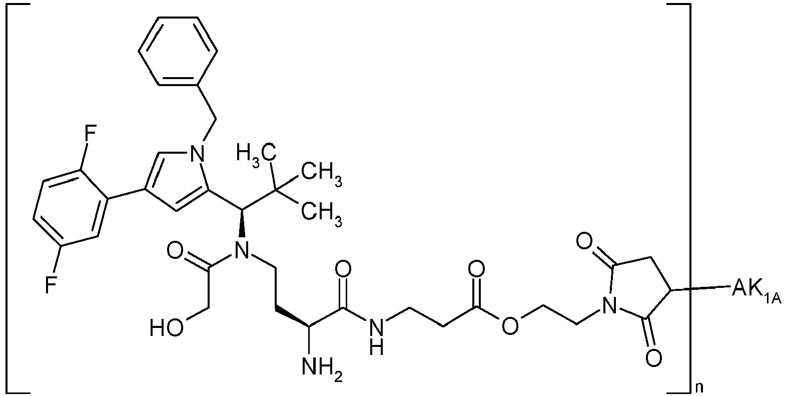 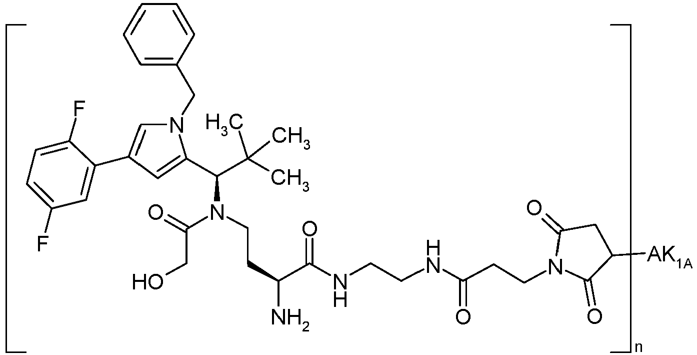 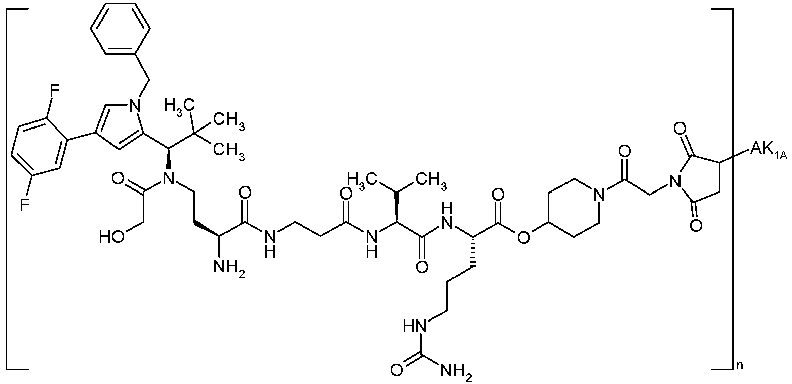 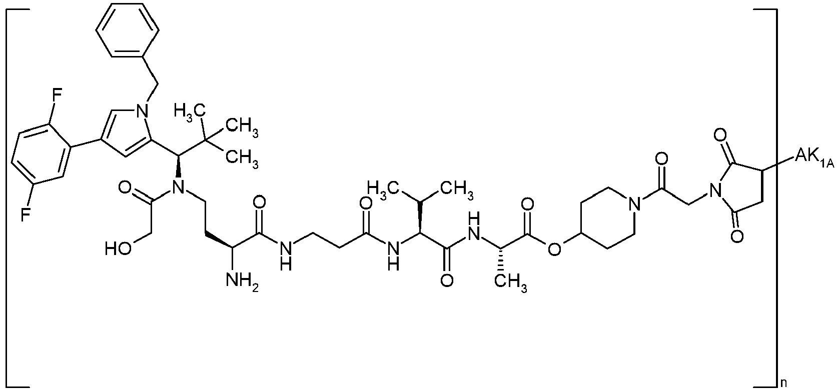 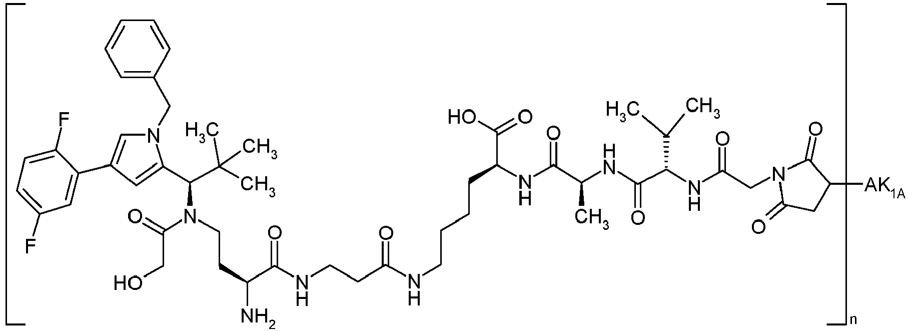 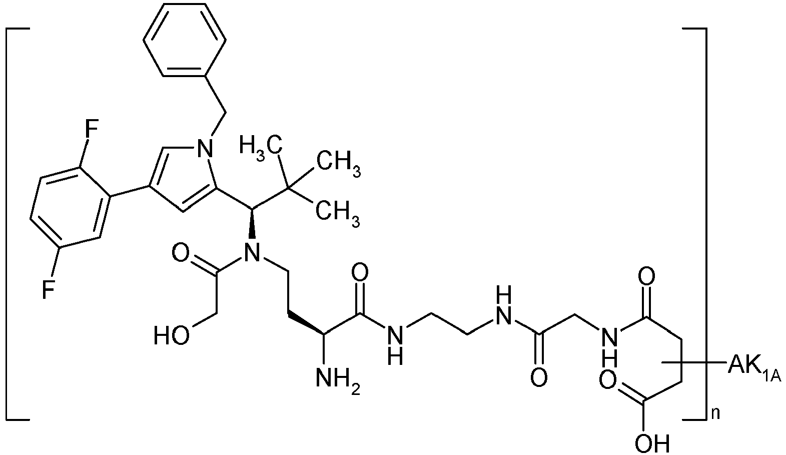 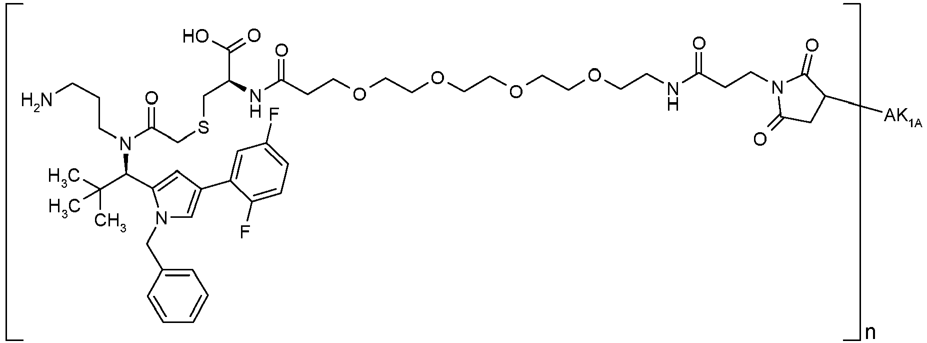 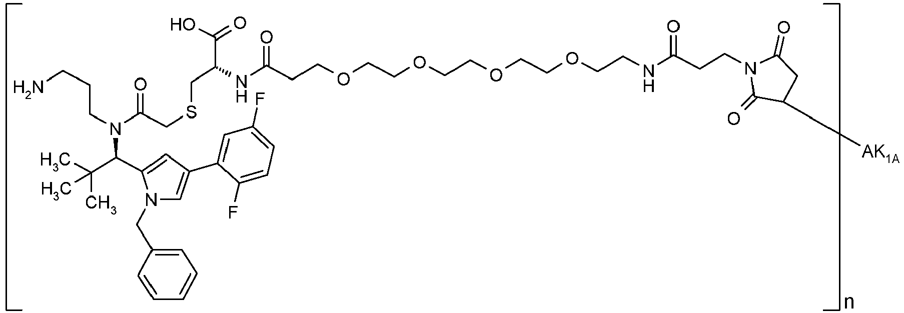 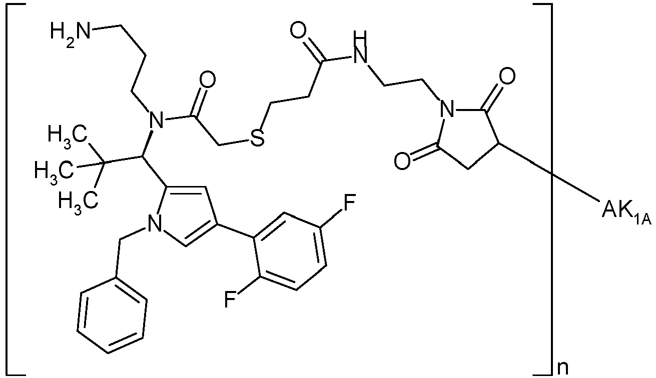 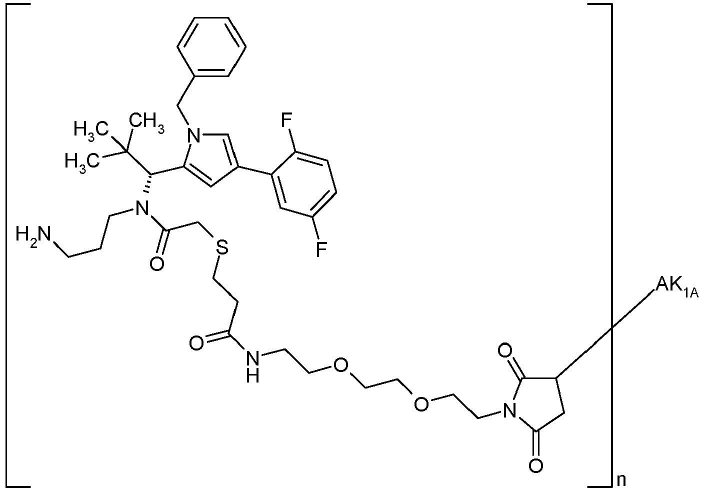 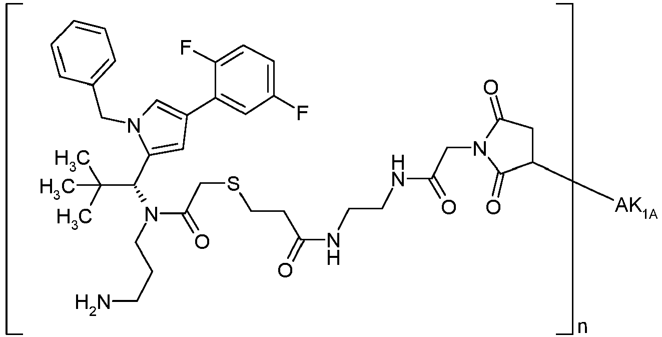 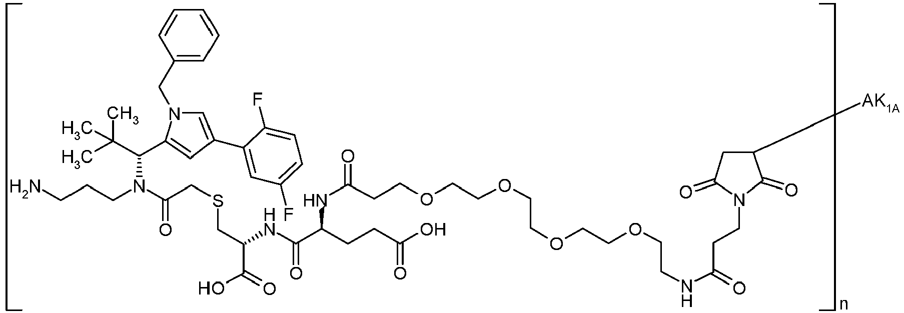 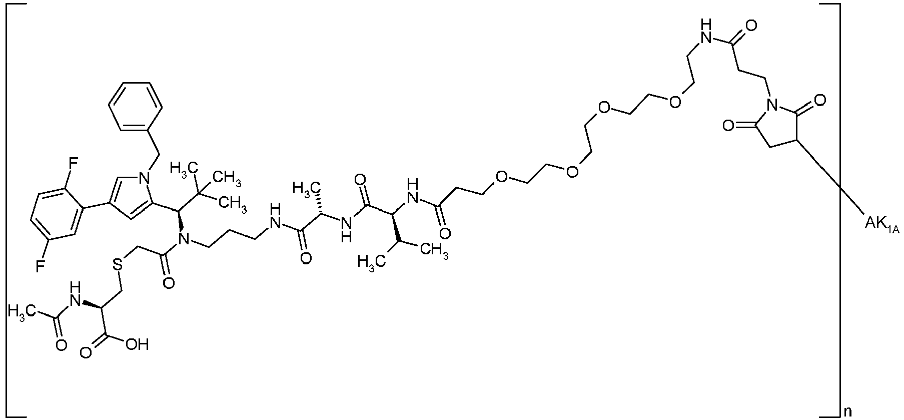 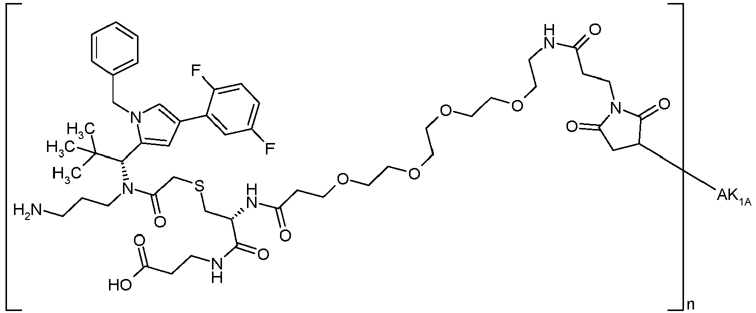 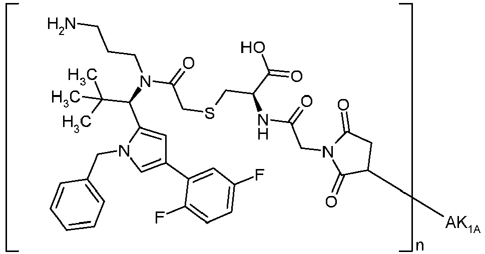 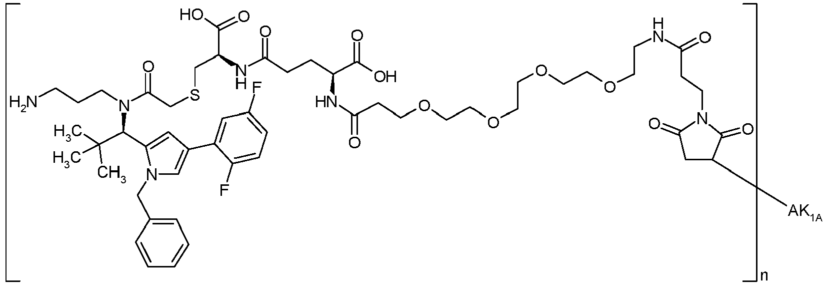 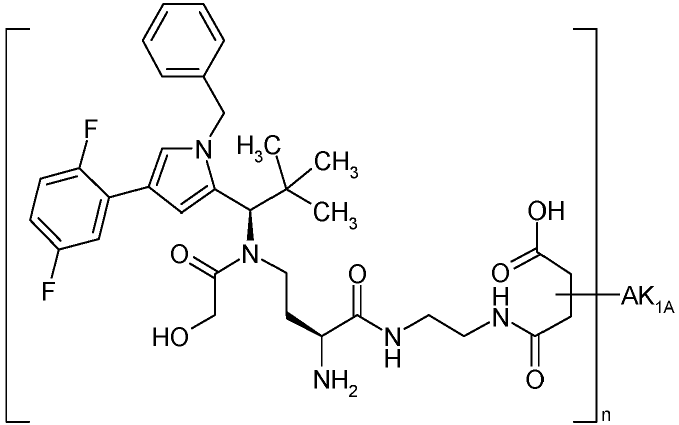 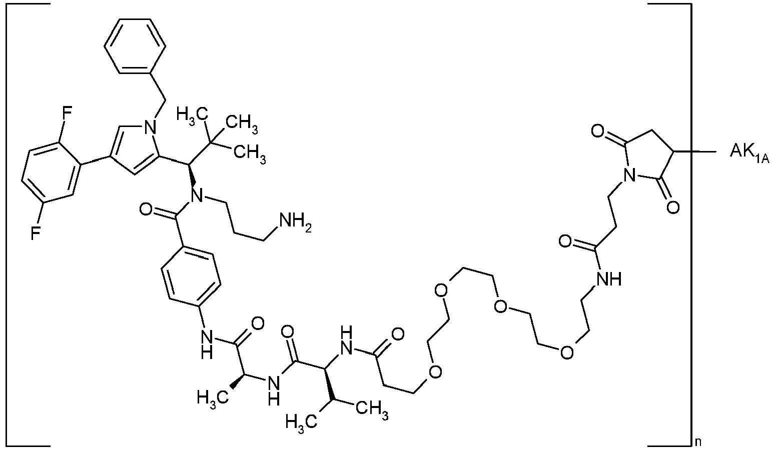 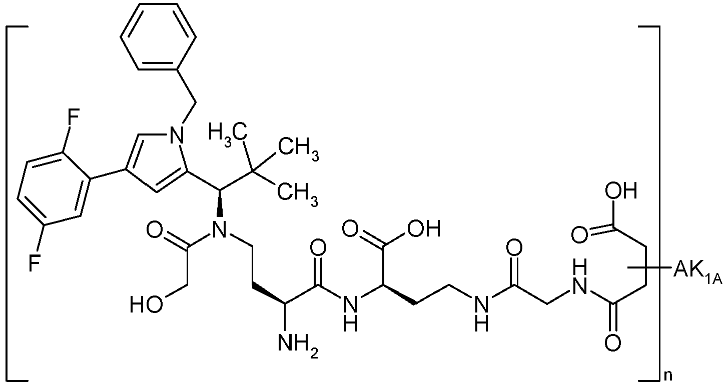 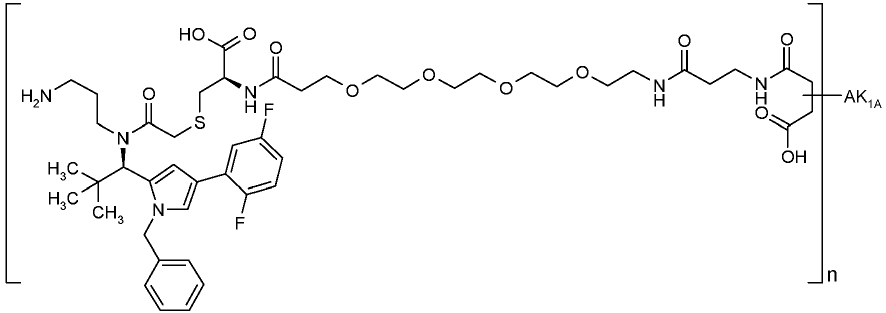 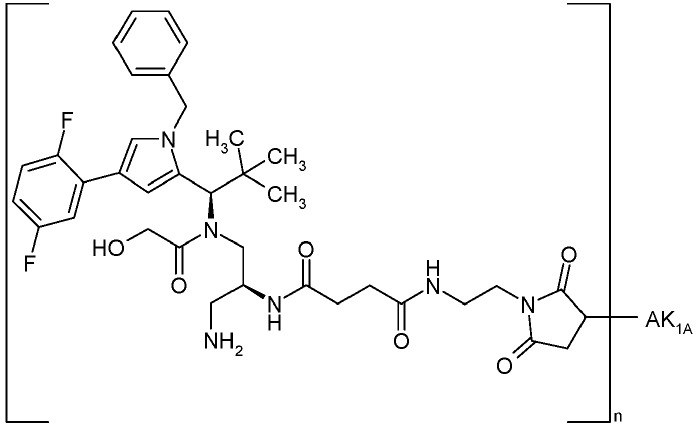 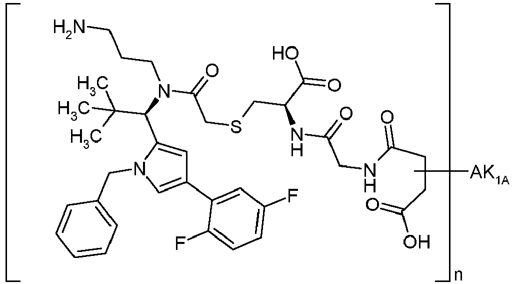 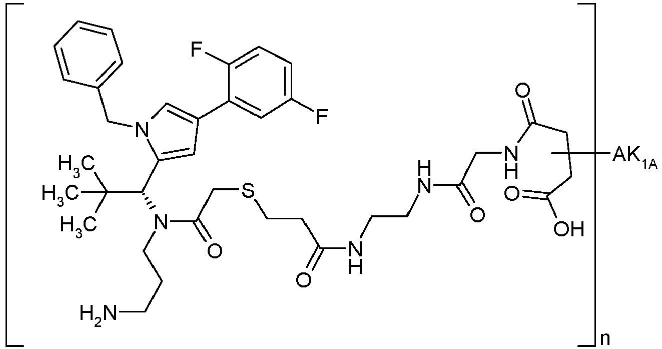 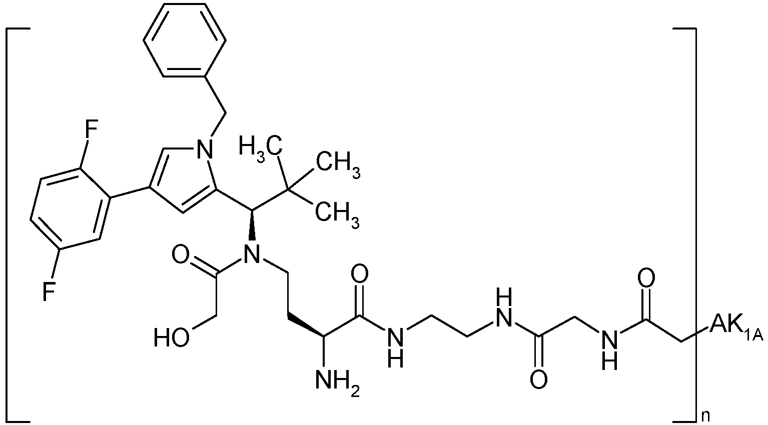 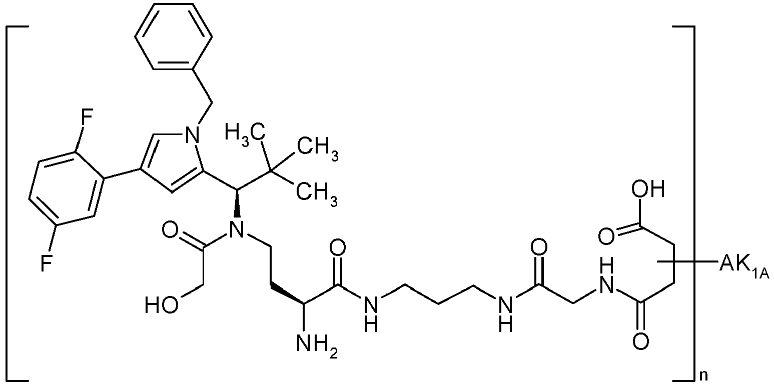 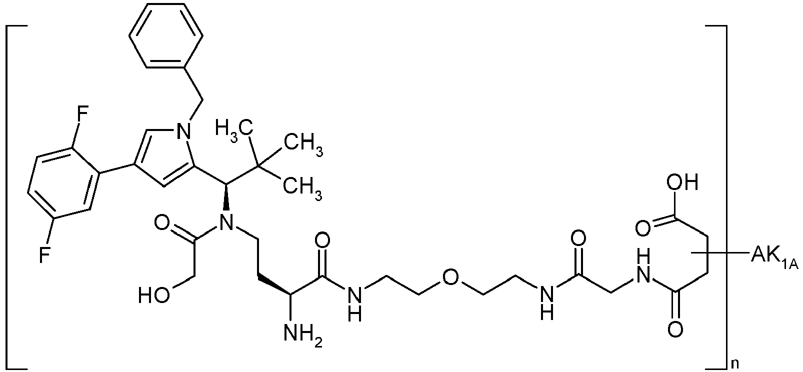 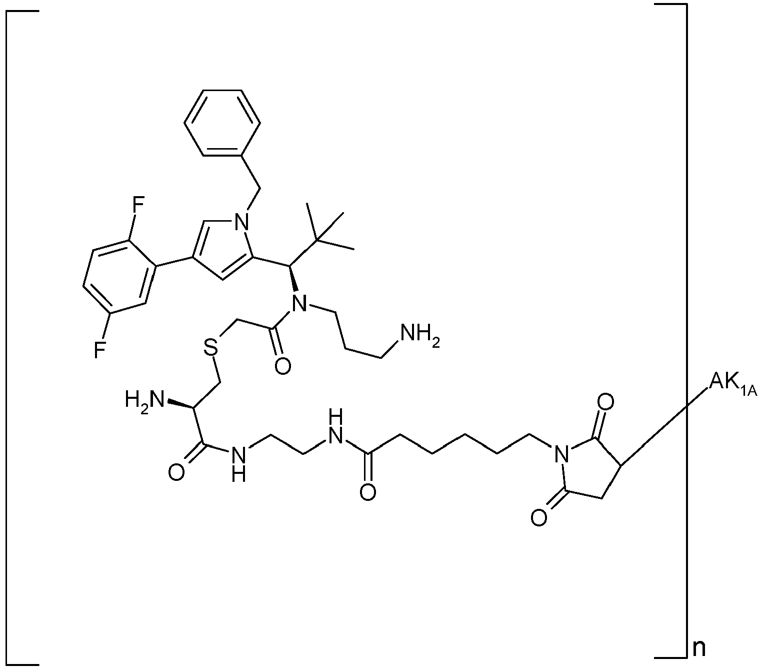 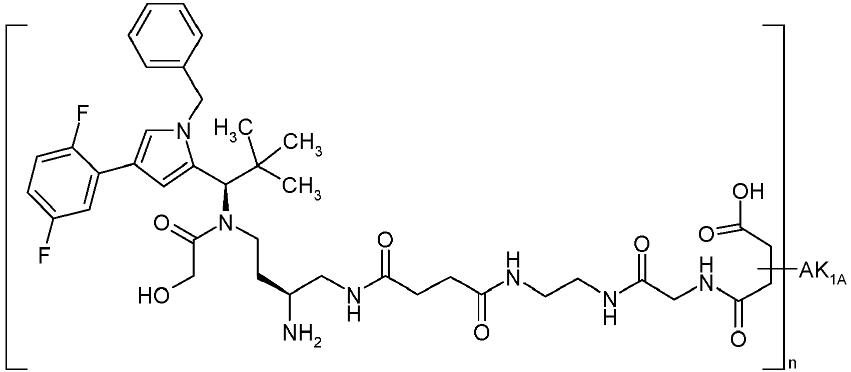 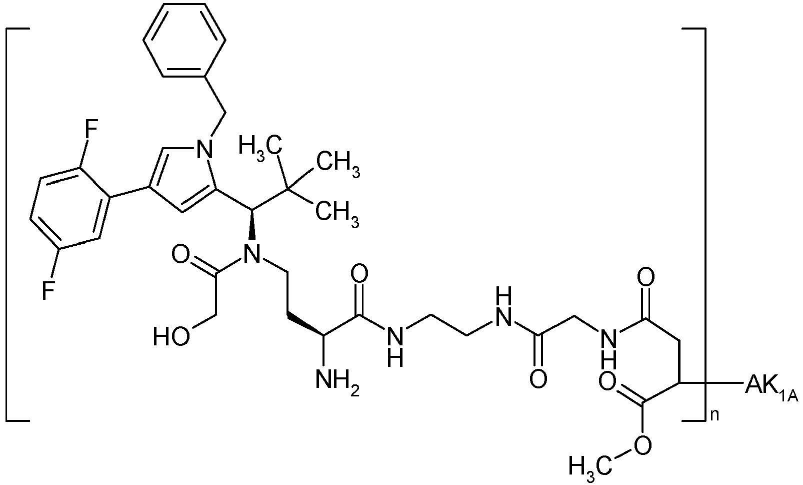 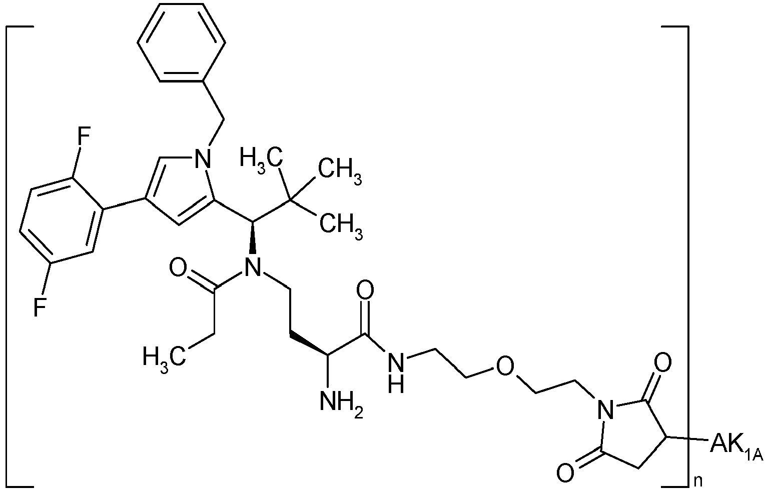 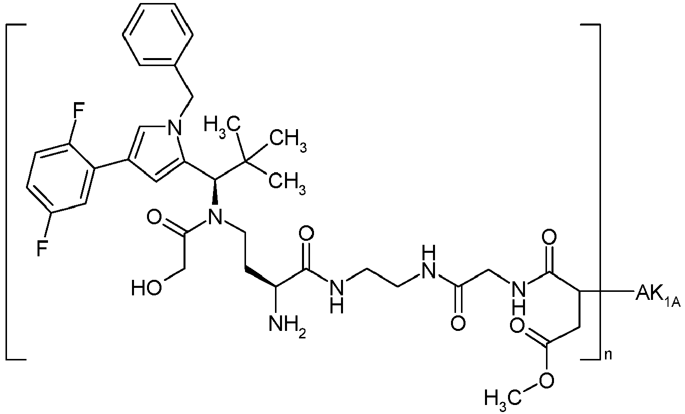 where the ADC, if attached via a group 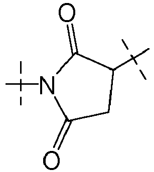 to the antibody, may also be present instead of this group at least partially in the form of the hydrolysed open-chain succinamides.

66. Conjugate according to Claim 65 where the antibody is a human, humanized or chimeric monoclonal antibody or an antigen-binding fragment thereof.

67. Conjugate according to Claim 66 where the antibody is an anti-HER2 antibody, and anti-EGFR antibody, an anti-TWEAKR antibody or an antigen-binding fragment thereof.

68. Conjugate according to Claim 67 where the anti-TWEAKR antibody binds specifically to amino acid D in position 47 (D47) of TWEAKR (SEQ ID NO:169), preferably the anti-TWEAKR antibody TPP-2090.

## Revendications

1. Conjugué d'un liant ou d'un dérivé de celui-ci avec une ou plusieurs molécules de principe actif, la molécule de principe actif étant un inhibiteur de protéine du fuseau kinésine, qui est reliée au liant via un lieur L, l'inhibiteur de protéine du fuseau kinésine présentant la formule suivante (IIa) : 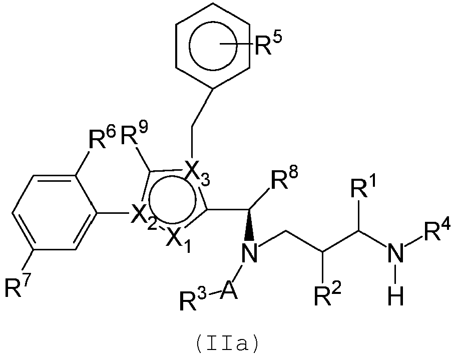
X₁ représentant N, X₂ représentant N et X₃ représentant C ; ou
X₁ représentant CH ou CF, X₂ représentant C et X₃ représentant N ; ou
X₁ représentant NH, X₂ représentant C et X₃ représentant C ; ou
X₁ représentant CH, X₂ représentant N et X₃ représentant C ;
(X₁ représentant CH, X₂ représentant C et X₃ représentant N étant préférés) ;
R¹ représentant H, -L-#1, -MOD ou -(CH₂)₀₋₃Z, Z représentant -H, -NHY³, -OY³, -SY³, halogène, -CO-NY¹Y² ou -CO-OY³,
Y¹ et Y² indépendamment l'un de l'autre représentant H, NH₂, -(CH₂CH₂O)₀₋₃-(CH₂)₀₋₃Z' (par exemple -(CH₂)₀₋₃Z') ou -CH(CH₂W)Z', et Y³ représentant H ou -(CH₂)₀₋₃Z', Z' représentant H, NH₂, SO₃H, COOH, -NH-CO-CH₂-CH₂-CH(NH₂)COOH ou -(CO-NH-CHY⁴)₁₋₃COOH ; W représentant H ou OH,
Y⁴ indépendamment les uns des autres représentant C₁₋₆-alkyle linéaire ou ramifié, éventuellement substitué par -NHCONH₂, ou aryle ou benzyle éventuellement substitué par -NH₂ ;
R² représentant -L-#1, H, -MOD, -CO-CHY⁴-NHY⁵ ou-(CH₂)₀₋₃Z, ou R² et R⁴ représentant ensemble -CH₂-CHR¹⁰- ou -CHR¹⁰-CH₂- (avec formation d'un cycle pyrrolidine), R¹⁰ représentant L-#1, H, NH₂, SO₃H, COOH, SH, ou OH ;
Z représentant -H, halogène, -OY³, -SY³, NHY³, -CO-NY¹Y², ou -CO-OY³,
Y¹ et Y² indépendamment l'un de l'autre représentant H, NH₂, ou -(CH₂)₀₋₃Z', et Y³ représentant H ou-(CH₂)₀₋₃Z', Z' représentant H, SO₃H, NH₂ ou COOH ;
Y⁴ indépendamment les uns des autres représentant C₁₋₆-alkyle linéaire ou ramifié, éventuellement substitué par -NHCONH₂, ou aryle ou benzyle éventuellement substitué par -NH₂ et Y⁵ représentant H ou -CO-CHY⁶-NH₂, Y⁶ représentant C₁₋₆-alkyle linéaire ou ramifié ;
R⁴ représentant -L-#1, H, -CO-CHY⁴-NHY⁵ ou -(CH₂)₀₋₃Z,
Z représentant -H, halogène, -OY³, -SY³, NHY³, -CO-NY¹Y², ou -CO-OY³,
Y¹ et Y² indépendamment l'un de l'autre représentant H, NH₂, ou -(CH₂)₀₋₃Z', et Y³ représentant H ou-(CH₂)₀₋₃Z', Z' représentant H, SO₃H, NH₂ ou COOH ;
Y⁴ indépendamment les uns des autres représentant C₁₋₆-alkyle linéaire ou ramifié, éventuellement substitué par -NHCONH₂, ou aryle ou benzyle éventuellement substitué par -NH₂ et Y⁵ représentant H ou -CO-CHY⁶-NH₂, Y⁶ représentant C₁₋₆-alkyle linéaire ou ramifié ;
ou R² et R⁴ représentant ensemble -CH₂-CHR¹⁰- ou-CHR¹⁰-CH₂- (avec formation d'un cycle pyrrolidine), R¹⁰ représentant L-#1, H, NH₂, SO₃H, COOH, SH, ou OH ;
A représentant CO, SO, SO₂, SO₂NH ou CNNH;
R³ représentant -L-#1, -MOD, ou un groupe alkyle, cycloalkyle, aryle, hétéroaryle, hétéroalkyle, hétérocycloalkyle éventuellement substitué, préférablement -L-#1 ou un groupe C₁₋₁₀-alkyle, C₆₋₁₀-aryle ou C₆₋₁₀-aralkyle, C₅₋₁₀-hétéroalkyle, C₁₋₁₀-alkyl-O-C₆₋₁₀-aryle ou C₅₋₁₀-hétérocycloalkyle, qui peut être substitué par 1 à 3 groupes-OH, 1 à 3 atomes d'halogène, 1 à 3 groupes alkyle halogénés (qui présentent à chaque fois 1 à 3 atomes d'halogène), 1 à 3 groupes O-alkyle, 1 à 3 groupes -SH, 1 à 3 groupes -S-alkyle, 1 à 3 groupes -O-CO-alkyle, 1 à 3 groupes -O-CO-NH-alkyle, 1 à 3 groupes -NH-CO-alkyle, 1 à 3 groupes -NH-CO-NH-alkyle, 1 à 3 groupes -S(O)ₙ-alkyle, 1 à 3 groupes -SO₂-NH-alkyle, 1 à 3 groupes -NH-alkyle, 1 à 3 groupes-N(alkyle)₂, 1 à 3 groupes -NH₂ ou 1 à 3 groupes-(CH₂)₀₋₃Z, Z représentant -H, halogène, -OY³, -SY³,-NHY³, -CO-NY¹Y², ou -CO-OY³, Y¹ et Y² indépendamment l'un de l'autre représentant H, NH₂, ou -(CH₂)₀₋₃Z', et Y³ représentant H, -(CH₂)₀₋₃-CH(NHCOCH₃)Z', -(CH₂)₀₋₃-CH(NH₂)Z', ou-(CH₂)₀₋₃Z', Z' représentant H, SO₃H, NH₂ ou COOH (« alkyle » signifiant préférablement C₁₋₁₀-alkyle) ;
R⁵ représentant -L-#1, H, NH₂, NO₂, halogène (en particulier F, Cl, Br), -CN, CF₃, -OCF₃, -CH₂F,-CH₂F, SH ou -(CH₂)₀₋₃Z, Z représentant -H, -OY³, -SY³, halogène, NHY³, -CO-NY¹Y², ou -CO-OY³,
Y¹ et Y² indépendamment l'un de l'autre représentant H, NH₂, ou -(CH₂)₀₋₃Z', et Y³ représentant H ou-(CH₂)₀₋₃Z', Z' représentant H, SO₃H, NH₂ ou COOH ;
R⁶ et R⁷ indépendamment l'un de l'autre représentant H, cyano, C₁₋₁₀-alkyle (éventuellement fluoré), C₂₋₁₀-alcényle (éventuellement fluoré), C₂₋₁₀-alcynyle (éventuellement fluoré), hydroxy, NO₂, NH₂, COOH ou halogène (en particulier F, Cl, Br),
R⁸ représentant C₁₋₁₀-alkyle (éventuellement fluoré), C₂₋₁₀-alcényle (éventuellement fluoré), C₂₋₁₀-alcynyle (éventuellement fluoré), C₄₋₁₀-cycloalkyle (éventuellement fluoré) ou -(CH₂)₀₋₂-(HZ²), HZ² représentant un hétérocycle à 4 à 7 chaînons comportant jusqu'à 2 hétéroatomes choisis parmi N, O et S, chacun de ces groupes pouvant être substitué par -OH, CO₂H ou NH₂ ou -L-#1 ;
un ou aucun des substituants R¹, R², R³, R⁴, R⁵, R⁸ et R¹⁰ représentant ou, selon le cas (dans le cas de R⁸), présentant -L-#1,
L représentant le lieur et #1 représentant la liaison au liant ou, selon le cas, à un dérivé de celui-ci,
R⁹ représentant H, F, CH₃, CF₃, CH₂F ou CHF₂ ;
-MOD représentant -(NR¹⁰)ₙ-(G1)ₒ-G2-H,
R¹⁰ représentant H ou C₁₋₃-alkyle ;
G1 représentant -NHCO- , -CONH- ou 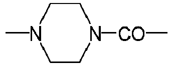 (où lorsque G1 représente -NHCO- ou 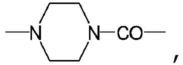
R¹⁰ n'est pas NH₂) ;
n étant 0 ou 1 ;
o étant 0 ou 1 ; et
G2 étant un groupe hydrocarboné linéaire et/ou ramifié comportant 1 à 10 atomes de carbone, qui peut être interrompu une fois ou plusieurs fois par un ou plusieurs des groupes -O-, -S-, -SO-, SO₂,-NR^{y}-, -NR^{y}CO-, CONR^{y}-, -NR^{y}NR^{y}-, -SO₂NR^{y}NR^{y}-, - CONR^{y}NR^{y}-, (R^{y} représentant H, phényle, C₁-C₁₀-alkyle, C₂-C₁₀-alcényle, ou C₂-C₁₀-alcynyle, qui peuvent être à chaque fois substitués par NHCONH₂, -COOH, -OH, -NH₂, NH-CNNH₂, sulfonamide, sulfone, sulfoxyde, ou acide sulfonique), -CO-, -CR^{x}=N-O- (Rx représentant H, C₁-C₃-alkyle ou phényle), la chaîne hydrocarbonée y compris les chaînes latérales, le cas échéant, pouvant être substituée par -NHCONH₂, -COOH, -OH, -NH₂, NH-CNNH₂, sulfonamide, sulfone, sulfoxyde, ou acide sulfonique, le groupe -MOD présentant de préférence au moins un groupe -COOH ; ainsi que leurs sels, solvates et sels des solvates.

2. Conjugué selon la revendication 1, X₁ étant CH, X₂ étant C et X₃ étant N.

3. Conjugué selon la revendication 1 ou 2, le substituant R¹ représentant -L-#1.

4. Conjugué selon l'une ou plusieurs des revendications précédentes, le lieur-principe actif étant représenté par la formule générale (II) : 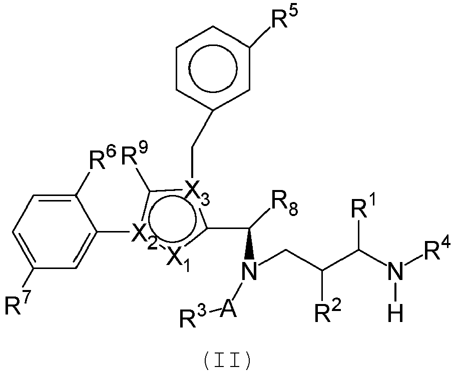
X₁ représentant N, X₂ représentant N et X₃ représentant C ; ou
X₁ représentant CH, X₂ représentant C et X₃ représentant N ; ou
X₁ représentant NH, X₂ représentant C et X₃ représentant C ; ou
X₁ représentant CH, X₂ représentant N et X₃ représentant C ;
R¹ représentant H, -L-#1 ou -(CH₂)₀₋₃Z, Z représentant -H, -NHY³, -OY³, -SY³, halogène, -CO-NY¹Y² ou -CO-OY³,
Y¹ et Y² indépendamment l'un de l'autre représentant H, NH₂, -(CH₂CH₂O)₀₋₃-(CH₂)₀₋₃Z', ou -CH(CH₂W)Z', et Y³ représentant H ou -(CH₂)₀₋₃Z', Z' représentant H, NH₂, SO₃H, COOH, -NH-CO-CH₂-CH₂-CH (NH₂)COOH ou -(CO-NH-CHY⁴)₁₋₃COOH, W représentant H ou OH ; Y⁴ indépendamment les uns des autres représentant C₁₋₆-alkyle linéaire ou ramifié, éventuellement substitué par -NHCONH₂, ou aryle ou benzyle éventuellement substitué par -NH₂ ;
R² et R⁴ indépendamment l'un de l'autre représentant -L-#1, H, -CO-CHY⁴-NHY⁵ ou -(CH₂)₀₋₃Z, ou R² et R⁴ représentant ensemble -CH₂-CHR¹⁰- ou -CHR¹⁰-CH₂- (avec formation d'un cycle pyrrolidine), R¹⁰ représentant L-#1, H, NH₂, SO₃H, COOH, SH, ou OH ;
Z représentant -H, OY³, -SY³, halogène, NHY³, -CO-NY¹Y², ou -CO-OY³,
Y¹ et Y² indépendamment l'un de l'autre représentant H, NH₂, ou -(CH₂)₀₋₃Z', et Y³ représentant H ou - (CH₂)₀₋₃Z' , Z' représentant H, SO₃H, NH₂ ou COOH ; Y⁴ indépendamment les uns des autres représentant C₁₋₆-alkyle linéaire ou ramifié, éventuellement substitué par -NHCONH₂, ou aryle ou benzyle éventuellement substitué par -NH₂ et Y⁵ représentant H ou -CO-CHY⁶-NH₂, Y⁶ représentant C₁₋₆-alkyle linéaire ou ramifié ;
A représentant CO, SO, SO₂, SO₂NH ou CNNH;
R³ représentant -L-#1, ou un groupe alkyle, aryle, hétéroaryle, hétéroalkyle, hétérocycloalkyle éventuellement substitué, préférablement -L-#1 ou un groupe C₁₋₁₀-alkyle, C₆₋₁₀-aryle ou C₆₋₁₀-aralkyle, C₅₋₁₀-hétéroalkyle, C₁₋₁₀-alkyl-O-C₆₋₁₀-aryle ou C₅₋₁₀-hétérocycloalkyle, qui peut être substitué par 1 à 3 groupes-OH, 1 à 3 atomes d'halogène, 1 à 3 groupes alkyle halogénés (qui peuvent présenter à chaque fois 1 à 3 atomes d'halogène), 1 à 3 groupes O-alkyle, 1 à 3 groupes -SH, 1 à 3 groupes -S-alkyle, 1 à 3 groupes -O-CO-alkyle, 1 à 3 groupes -O-CO-NH-alkyle, 1 à 3 groupes -NH-CO-alkyle, 1 à 3 groupes -NH-CO-NH-alkyle, 1 à 3 groupes -S(O)ₙ-alkyle, 1 à 3 groupes -SO₂-NH-alkyle, 1 à 3 groupes -NH-alkyle, 1 à 3 groupes -N(alkyle)₂, 1 à 3 groupes -NH₂ ou 1 à 3 groupes -(CH₂)₀₋₃Z, Z représentant -H, halogène,-OY³, -SY³, NHY³, -CO-NY¹Y², ou -CO-OY³, Y¹ et Y² indépendamment l'un de l'autre représentant H, NH₂, ou -(CH₂)₀₋₃Z', et Y³ représentant H, -(CH₂)₀₋₃-CH (NHCOCH₃)Z', -(CH₂)₀₋₃-CH(NH₂)Z', ou-(CH₂)₀₋₃Z', Z' représentant H, SO₃H, NH₂ ou COOH, (« alkyle » signifiant préférablement C₁₋₁₀-alkyle) ;
R⁵ représentant -L-#1, H, F, NH₂, NO₂, halogène, SH ou -(CH₂)₀₋₃Z, Z représentant -H, -OY³, -SY³, halogène, NHY³, -CO-NY¹Y², ou -CO-OY³,
Y¹ et Y² indépendamment l'un de l'autre représentant H, NH₂, ou -(CH₂)₀₋₃Z', et Y³ représentant H ou-(CH₂)₀₋₃Z', Z' représentant H, SO₃H, NH₂ ou COOH ;
un des substituants R¹, R², R³, R⁴ et R⁵ représentant -L-#1,
L représentant le lieur et #1 représentant la liaison au liant ou, selon le cas, à un dérivé de celui-ci,
R⁶ et R⁷ représentant indépendamment l'un de l'autre H, cyano, C₁₋₁₀-alkyle (éventuellement fluoré), C₂₋₁₀-alcényle (éventuellement fluoré), C₂₋₁₀-alcynyle (éventuellement fluoré), hydroxy ou halogène,
R⁸ représentant C₁₋₁₀-alkyle (éventuellement fluoré), C₄₋₁₀-cycloalkyle (éventuellement fluoré) ou oxétane éventuellement substitué ;
R⁹ représentant H, F, CH₃, CF₃, CH₂F ou CHF₂ ;
ainsi que leurs sels, solvates et sels des solvates.

5. Conjugué selon la revendication 4,
R¹ représentant -L-#1, H, - (CH₂)₀₋₃Z, Z représentant -H, -CO-NY¹Y², NHY³, OY³, -SY³, ou -CO-OY³,
Y¹ et Y² indépendamment l'un de l'autre représentant H, NH₂, -(CH₂CH₂O)₀₋₃-(CH₂)₀₋₃Z', ou -CH(CH₂W)Z', et Y³ représentant H ou - (CH₂)₀₋₃Z', Z' représentant H, NH₂, COOH, -NH-CO-CH₂-CH₂-CH (NH₂)COOH ou -(CO-NH-CHY⁴)₁₋₃COOH ; W représentant H ou OH, Y⁴ représentant indépendamment les uns des autres C₁₋₆ alkyle linéaire ou ramifié, éventuellement substitué par-NHCONH₂, ou aryle ou benzyle éventuellement substitué par -NH₂ ;
R² et R⁴ indépendamment l'un de l'autre représentant -L-#1, -CO-CHY⁴-NHY⁵ ou H ou R² et R⁴ représentant ensemble -CH₂-CHR¹⁰- (avec formation d'un cycle pyrrolidine), R¹⁰ représentant H, -L-#1, NH₂, COOH, SH, OH ou SO₃H ;
Y⁴ représentant indépendamment les uns des autres C₁₋₆ alkyle linéaire ou ramifié, éventuellement substitué par -NHCONH₂, ou aryle ou benzyle éventuellement substitué par -NH₂ et Y⁵ représentant H ou -CO-CHY⁶-NH₂, Y⁶ représentant C₁₋₆ alkyle linéaire ou ramifié ;
A représentant CO,
R³ représentant -(CH₂)OH ou -L-#1, et
R⁵ représentant -L-#1 ou H,
un des substituants R¹, R², R³, R⁴ et R⁵ représentant -L-#1.

6. Conjugué selon la revendication 4 ou 5, R⁶ et R⁷ représentant indépendamment l'un de l'autre H, C₁₋₃-alkyle ou halogène.

7. Conjugué selon l'une ou plusieurs des revendications 4 à 6, R⁸ représentant C₁₋₄-alkyle (de préférence tert-butyle) .

8. Conjugué selon l'une ou plusieurs des revendications 4 à 7, R⁹ représentant H.

9. Conjugué selon l'une ou plusieurs des revendications 4 à 8, R⁶ et R⁷ représentant F.

10. Conjugué selon l'une ou plusieurs des revendications précédentes, le liant ou le dérivé de celui-ci étant un peptide liant ou une protéine liante ou un dérivé d'un peptide liant ou d'une protéine liante.

11. Conjugué selon la revendication 10, chaque molécule de principe actif étant liée à des acides aminés divers du peptide liant ou de la protéine liante ou, selon le cas, d'un dérivé de celui-ci ou de celle-ci, par l'intermédiaire du lieur.

12. Conjugué selon l'une ou plusieurs des revendications précédentes, le conjugué présentant en moyenne 1,2 à 20 molécule(s) de principe actif par liant.

13. Conjugué selon l'une ou plusieurs des revendications 10 à 12, le peptide liant ou la protéine liante représentant un anticorps ou, selon le cas, le dérivé du peptide liant ou de la protéine liante représentant

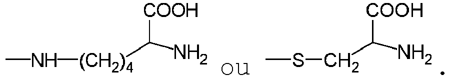

14. Conjugué selon l'une ou plusieurs des revendications précédentes, le liant se liant à une molécule cible du cancer.

15. Conjugué selon la revendication 14, le liant se liant à une molécule cible extracellulaire.

16. Conjugué selon la revendication 15, le liant, après liaison à la molécule cible extracellulaire, étant internalisé par la cellule exprimant la molécule cible et étant traité de manière intracellulaire (de préférence lysosomale).

17. Conjugué selon l'une ou plusieurs des revendications 10 à 16, le peptide liant ou la protéine liante étant un anticorps monoclonal humain, humanisé ou chimérique ou un fragment de liaison à un antigène de celui-ci.

18. Conjugué selon la revendication 17, le peptide liant ou la protéine liante étant un anticorps anti-HER2, un anticorps anti-EGFR, un anticorps anti-TWEAKR, ou un fragment de liaison à un antigène de celui-ci.

19. Conjugué selon la revendication 18, l'anticorps anti-TWEAKR se liant de manière spécifique à l'acide aminé D en position 47 (D47) de TWEAKR (SEQ ID NO : 169), préférablement l'anticorps anti-TWEAKR TPP-2090.

20. Conjugué selon la revendication 18, le peptide liant ou la protéine liante étant un anticorps anti-EGFR et R3 représentant -L-#1.

21. Conjugué selon l'une ou plusieurs des revendications précédentes, le lieur -L- présentant une des structures de base suivantes (i) à (iv) :
(i) -(CO)ₙ-SG1-L1-L2-
(ii) -(CO)ₘ-L1-SG-L1-L2-
(iii) -(CO)ₘ-L1-L2-
(iv) -(CO)ₘ-L1-SG-L2
m étant 0 ou 1, SG et SG1 étant un groupe clivable *in vivo,* L1 indépendamment les uns des autres représentant des groupes organiques non clivables *in vivo,* et L2 représentant un groupe de couplage au liant.

22. Conjugué selon la revendication 21, le groupe SG clivable *in vivo* étant un groupe oligopeptidique de 2 à 8, préférablement un groupe dipeptidique ou un disulfure, une hydrazone, un acétal ou un aminal et SG1 étant un groupe oligopeptidique de 2 à 8, préférablement un groupe dipeptidique.

23. Conjugué selon l'une ou plusieurs des revendications précédentes, le lieur étant lié au niveau d'une chaîne latérale de cystéine ou, selon le cas, d'un radical cystéine et présente la formule suivante :
§-(CO)m-L1-L2-§§
m étant 0 ou 1;
§ représentant la liaison à la molécule de principe actif et
§§ représentant la liaison au peptide liant ou à la protéine liante, et
-L2- représentant 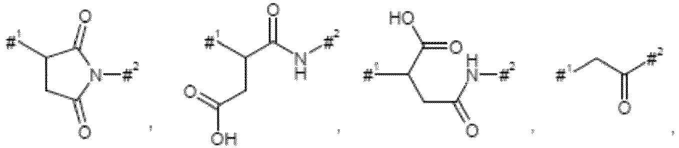 #¹-S-#² 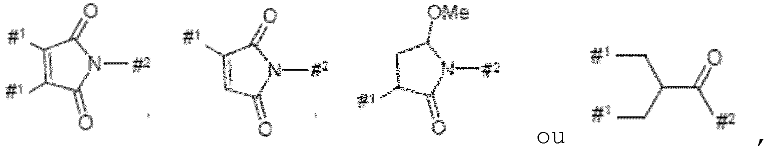
#¹ indiquant la position de liaison avec l'atome de soufre du liant,
#² indiquant la position de liaison avec le groupe L¹,
L1 représentant -(NR¹⁰)n-(G1)o-G2-,
R¹⁰ représentant H, NH₂ ou C₁-C₃-alkyle ;
G1 représentant -NHCO- ou 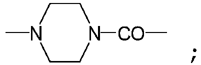
n étant 0 ou 1 ;
o étant 0 ou 1 ; et
G2 étant une chaîne hydrocarbonée linéaire ou ramifiée comportant 1 à 100 atome(s) de carbone de groupes arylène et/ou de groupes alkylène linéaires et/ou ramifiés et/ou cycliques, qui peut être interrompue une ou plusieurs fois par un ou plusieurs des groupes -O-, -S-, , -SO-, SO₂, -NH-, -CO-, -NMe-, -NHNH-, -SO₂NHNH-, -NHCO-, -CONH-,-CONHNH- et un hétérocycle aromatique ou non aromatique à 5 à 10 chaînons comportant jusqu'à 4 hétéroatomes choisis parmi N, O et S, -SO- et -SO₂-(de préférence 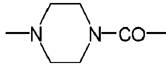 ), les chaînes latérales, le cas échéant, pouvant être substituées par-NHCONH₂, -COOH, -OH, -NH₂, NH-CNNH₂, sulfonamide, sulfone, sulfoxyde ou acide sulfonique,
ou représentant un des groupes suivants : 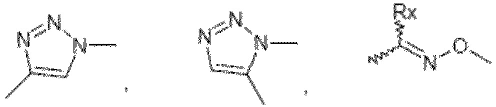 ou 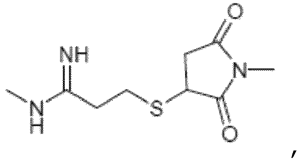
Rx représentant H, C₁-C₃-alkyle ou phényle.

24. Conjugué selon la revendication 23, L2 étant représenté par une des formules suivantes ou les deux : 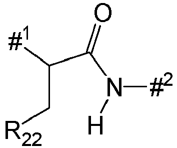 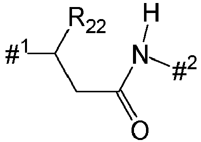 #¹ indiquant la position de liaison avec l'atome de soufre du liant, #² indiquant la position de liaison avec le groupe L¹, R²² représentant COOH, et les liaisons à l'atome de soufre du liant étant présentes à raison de plus de 80 % (par rapport au nombre total de liaisons du lieur au liant) dans une de ces deux structures .

25. Conjugué selon la revendication 23 ou 24, la chaîne hydrocarbonée étant interrompue par un des groupes suivants : 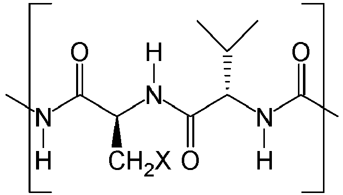 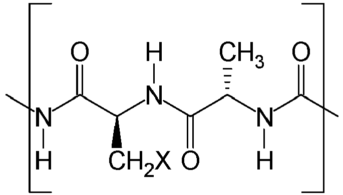 X étant H, un groupe C₁₋₁₀-alkyle, qui peut éventuellement être substitué par -NHCONH₂, -COOH, -OH, NH₂, -NH-CNNH₂, sulfone, sulfoxyde ou acide sulfonique.

26. Conjugué selon la revendication 23, le lieur présentant la formule suivante : 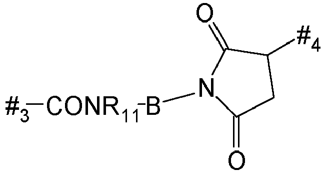
#3 représentant la liaison à la molécule de principe actif,
#4 représentant la liaison au peptide liant ou à la protéine liante,
R¹¹ représentant H ou NH₂ ;
B représentant -[(CH₂)ₓ-(X⁴)_{y}]_{w}-(CH₂)_{z}-,
w = 0 à 20 ;
x = 0 à 5 ;
x = 0 à 5 ;
y = 0 ou 1 ;
z = 0 à 5 ; et
X⁴ représentant -O-, -CONH- ou -NHCO- 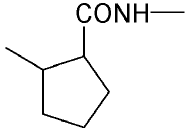 .

27. Conjugué selon la revendication 23, R¹ ou R⁴ représentant -L-#1.

28. Conjugué selon l'une ou plusieurs des revendications 23 à 27, le conjugué présentant une des formules suivantes : 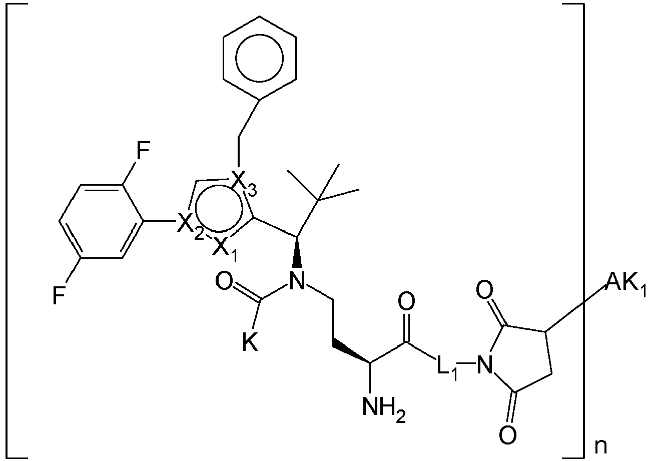 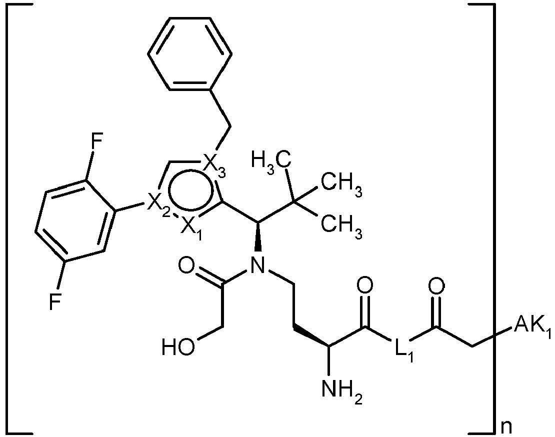 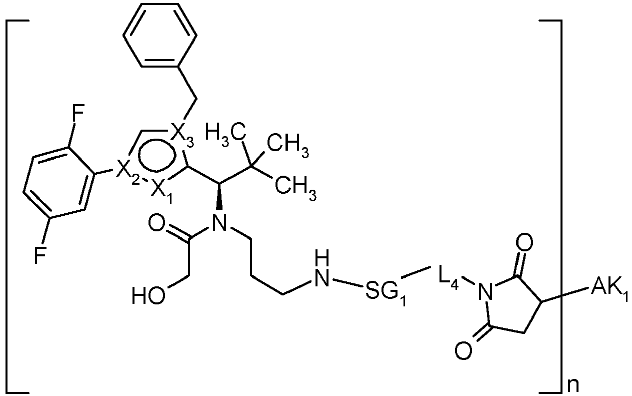 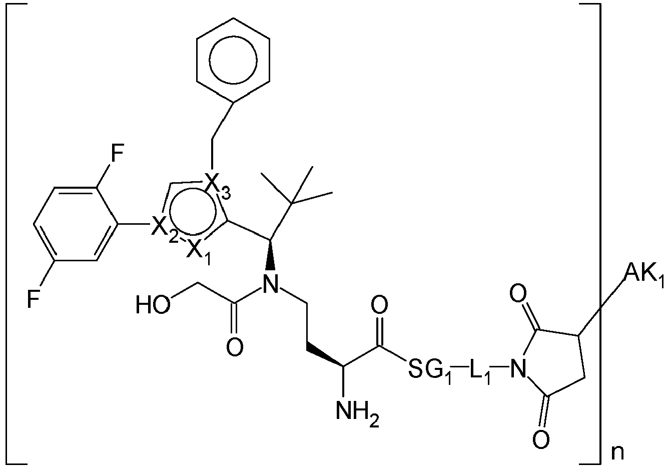 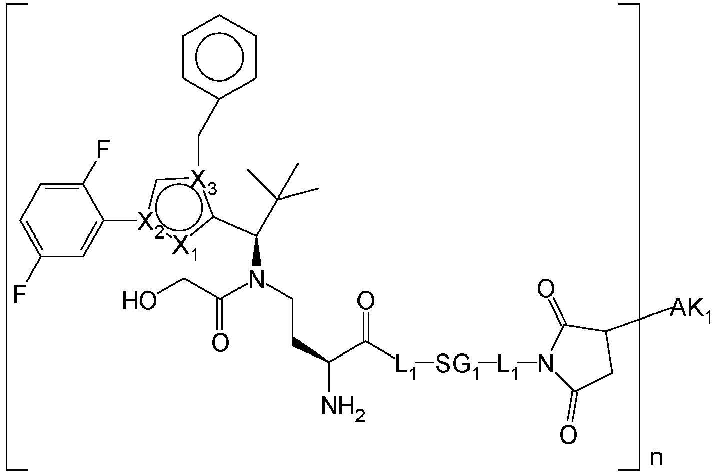 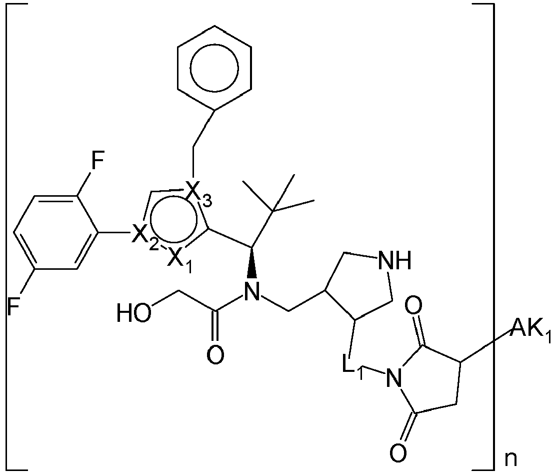 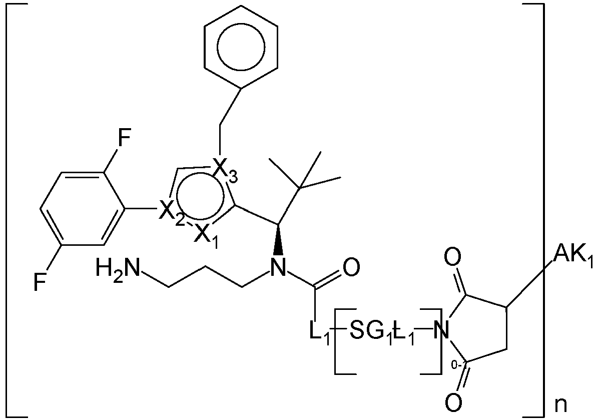 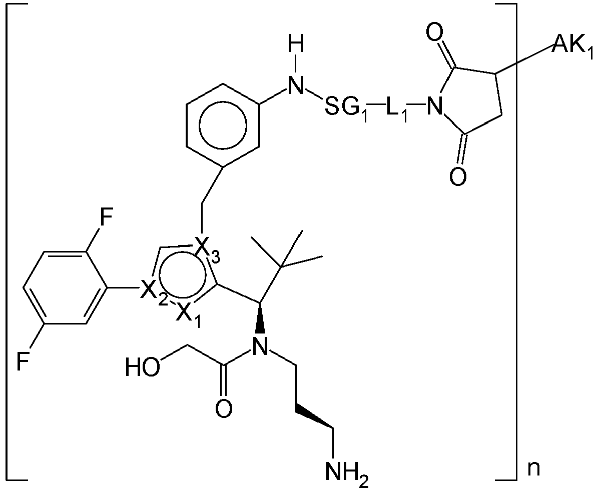
X₁, X₂ et X₃ présentant la même signification que dans la revendication 4,
AK₁ représentant un peptide liant ou une protéine liante, qui est lié par l'intermédiaire d'un atome de soufre du liant ; n représentant un nombre de 1 à 20 ; et L1 étant un groupe hydrocarboné éventuellement ramifié comportant 1 à 70 atome(s) de carbone, qui est une chaîne hydrocarbonée linéaire ou ramifiée comportant 1 à 100 atome(s) de carbone de groupes arylène et/ou de groupes alkylène linéaires et/ou ramifiés et/ou cycliques, qui peut être interrompue une ou plusieurs fois par un ou plusieurs des groupes -O-, -S-, -SO-, SO₂, -NH-, - CO-, -CONH-, -NHCO-, -NMe-, -NHNH-, -SO₂NHNH-, - CONHNH- et un hétérocycle aromatique ou non aromatique à 5 à 10 chaînons comportant jusqu'à 4 hétéroatomes choisis parmi N, O et S, -SO- et -SO₂-(de préférence 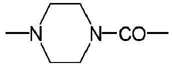 ), les chaînes latérales, le cas échéant, pouvant être substituées par-NHCONH₂, -COOH, -OH, -NH₂, NH-CNNH₂, sulfonamide, sulfone, sulfoxyde ou acide sulfonique,
et SG1 étant un oligopeptide de 2 à 8, préférablement un dipeptide ;
L4 représentant une simple liaison ou un groupe - (CO)_{y}-G4-, y représentant 0 ou 1, et G4 étant une chaîne hydrocarbonée linéaire ou ramifiée comportant 1 à 100 atome(s) de carbone de groupes arylène et/ou de groupes alkylène linéaires et/ou ramifiés et/ou cycliques, qui peut être interrompue une ou plusieurs fois par un ou plusieurs des groupes -O-, -S-, -SO-, SO₂, -NH-, -CO-, -CONH-, - NHCO-, -NMe-, -NHNH-, -SO₂NHNH-, -CONHNH- et un hétérocycle aromatique ou non aromatique à 5 à 10 chaînons comportant jusqu'à 4 hétéroatomes choisis parmi N, O et S, -SO- et -SO₂- (de préférence 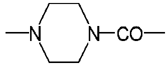 ), les chaînes latérales, le cas échéant, pouvant être substituées par -NHCONH₂, -COOH, -OH, -NH₂, NH-CNNH₂, sulfonamide, sulfone, sulfoxyde ou acide sulfonique.

29. Conjugué d'un liant ou d'un dérivé de celui-ci avec une ou plusieurs molécules de principe actif, le conjugué présentant une des formules suivantes ou les deux : 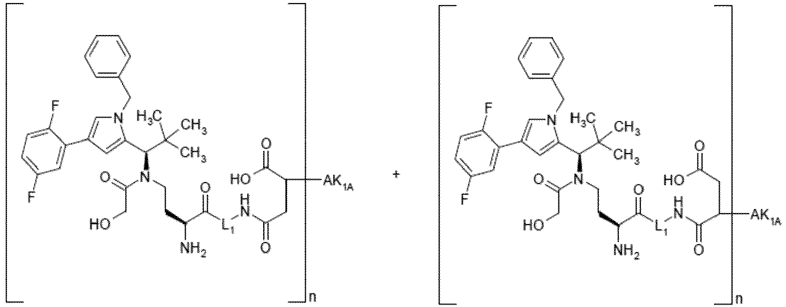 AK_{1A} représentant un peptide liant ou une protéine liante, qui est lié par l'intermédiaire d'un atome de soufre du liant ; n représentant un nombre de 1 à 20 ; et L1 étant une chaîne hydrocarbonée linéaire ou ramifiée comportant 1 à 100 atome(s) de carbone de groupes arylène et/ou de groupes alkylène linéaires et/ou ramifiés et/ou cycliques, qui peut être interrompue une ou plusieurs fois par un ou plusieurs des groupes -O-, -S-, -SO-, SO₂, -NH-, - CO-, -CONH-, -NHCO-, -NMe-, -NHNH-, -SO₂NHNH-, - CONHNH- et un hétérocycle aromatique ou non aromatique à 5 à 10 chaînons comportant jusqu'à 4 hétéroatomes choisis parmi N, O et S, -SO- et -SO₂-(de préférence 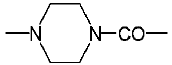 ), les chaînes latérales, le cas échéant, pouvant être substituées par - NHCONH₂, -COOH, -OH, -NH₂, NH-CNNH₂, sulfonamide, sulfone, sulfoxyde ou acide sulfonique.

30. Conjugué selon l'une ou plusieurs des revendications précédentes, le lieur -L- étant lié au niveau d'une chaîne latérale de cystéine ou, selon le cas, d'un radical cystéine et présentant la formule suivante 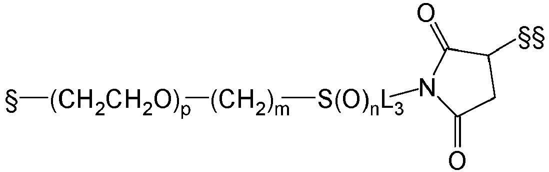
§ représentant la liaison à la molécule de principe actif et
§§ représentant la liaison au peptide liant ou à la protéine liante,
m étant 0, 1, 2, ou 3 ;
n étant 0, 1, ou 2 ;
p valant de 0 à 20 ; et
L3 représentant 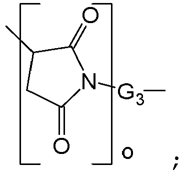
o étant 0 ou 1 ;
et
G3 étant une chaîne hydrocarbonée linéaire ou ramifiée comportant 1 à 100 atome(s) de carbone de groupes arylène et/ou de groupes alkylène linéaires et/ou ramifiés et/ou cycliques, qui peut être interrompue une ou plusieurs fois par un ou plusieurs des groupes -O-, -S-, -SO-, SO₂, -NH-, - CO-, -CONH-, -NHCO-, et un hétérocycle aromatique ou non aromatique à 5 à 10 chaînons comportant jusqu'à 4 hétéroatomes choisis parmi N, O et S -NMe-, -NHNH-, -SO₂NHNH-, -CONHNH-, -SO- et -SO₂-(de préférence 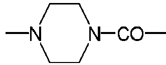 ), les chaînes latérales, le cas échéant, pouvant être substituées par - NHCONH₂, -COOH, -OH, sulfone, sulfoxyde ou acide sulfonique.

31. Conjugué selon la revendication 30, le lieur -L-étant lié au niveau d'une chaîne latérale de cystéine ou, selon le cas, d'un radical cystéine et présentant la formule suivante : 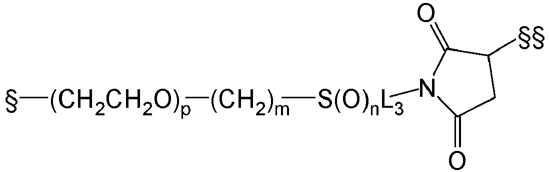
§ représentant la liaison à la molécule de principe actif et
§§ représentant la liaison au peptide liant ou à la protéine liante,
m étant 1 ;
p étant 0 ;
n étant 0 ;
et L3 représentant 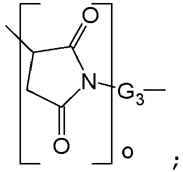
o étant 0 ou 1 ; et
G3 représentant -(CH2CH2O)ₛ(CH2)ₜ(CONH)ᵤ (CH2CH2O)ᵥ(CH2)_{w}-, s, t, v et w valant à chaque fois indépendamment les uns des autres 0 à 20, et u valant 0 ou 1.

32. Conjugué selon la revendication 30 ou 31, R² ou R³ représentant -L-#1 .

33. Conjugué selon la revendication 32, le conjugué présentant une des formules suivantes : 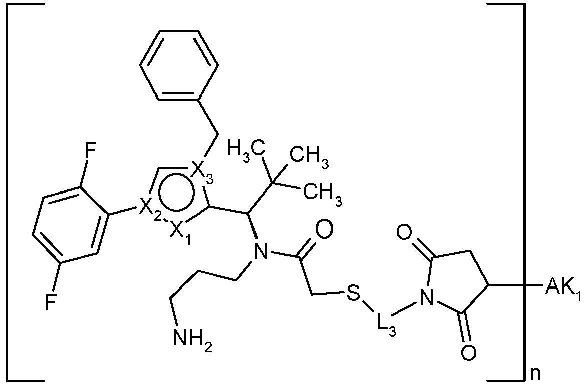 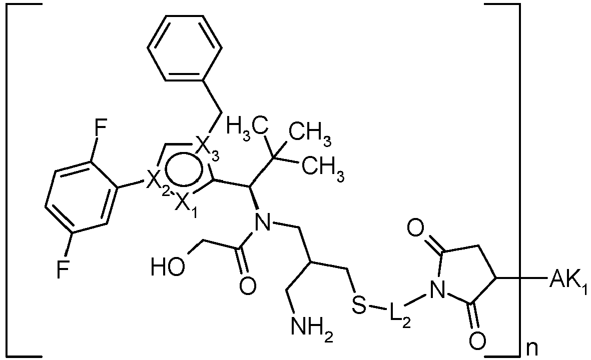 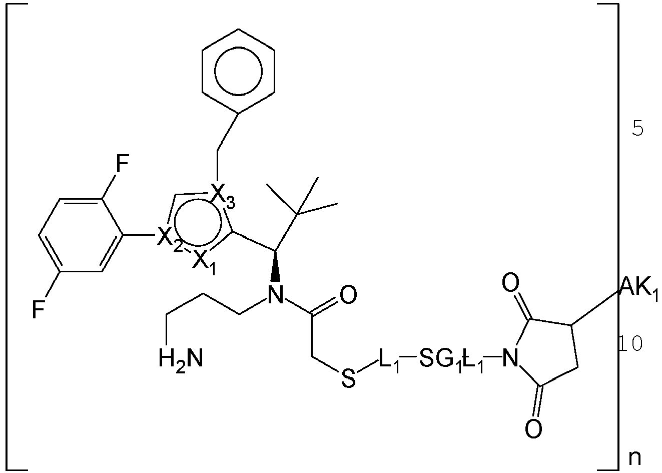 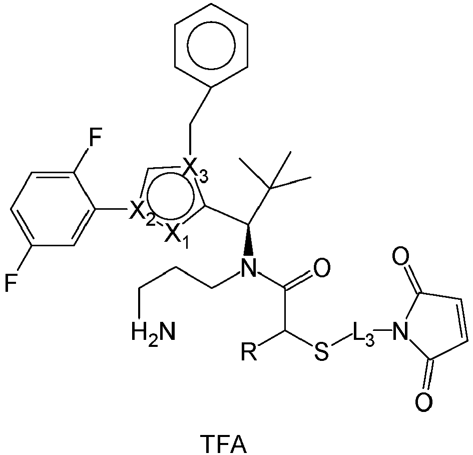 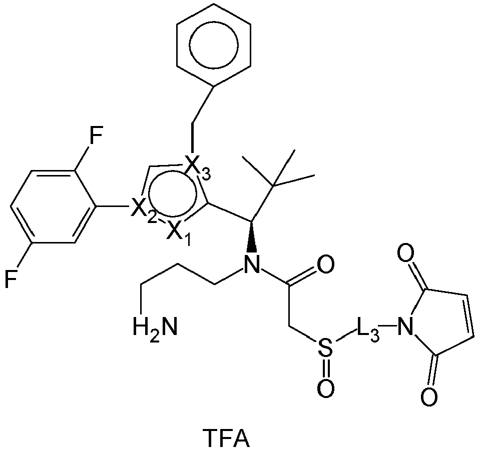 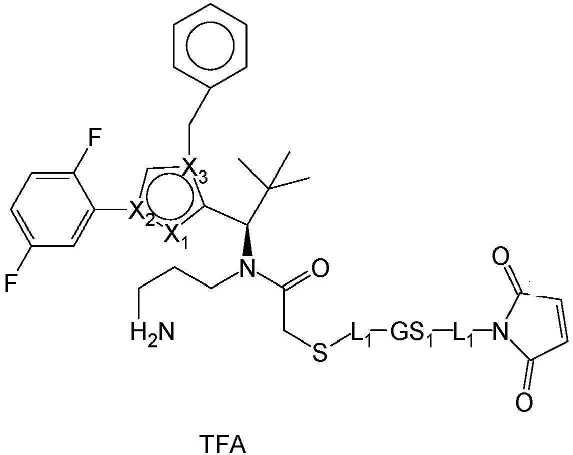 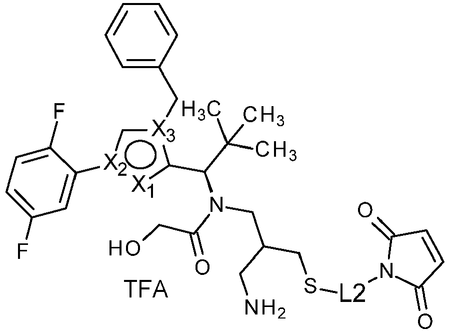
X₁, X₂ et X₃ présentant la même signification que dans la revendication 4,
AK₁ représentant un peptide liant ou une protéine liante, qui est lié par l'intermédiaire d'un atome de soufre du liant ; n représentant un nombre de 1 à 20 ; et L2 et L3 étant une chaîne hydrocarbonée linéaire ou ramifiée comportant 1 à 100 atome(s) de carbone de groupes arylène et/ou de groupes alkylène linéaires et/ou ramifiés et/ou cycliques, qui peut être interrompue une ou plusieurs fois par un ou plusieurs des groupes -O-, -S-, -SO-, SO₂, -NH-, - CO-, -NMe-, -NHNH-, -SO₂NHNH-, -NHCO-, -CONH-, - CONHNH- et un hétérocycle aromatique ou non aromatique à 5 à 10 chaînons comportant jusqu'à 4 hétéroatomes choisis parmi N, O et S, -SO- et -SO₂-(de préférence 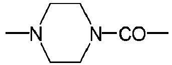 ), les chaînes latérales, le cas échéant, pouvant être substituées par-NHCONH₂, -COOH, -OH, -NH₂, NH-CNNH₂, sulfonamide, sulfone, sulfoxyde ou acide sulfonique.

34. Conjugué selon l'une ou plusieurs des revendications 1 à 29, le lieur -L- étant lié à une chaîne latérale de lysine et présentant la formule suivante :
-§-(SG)ₓ-L4-CO-§§,
§ représentant la liaison à la molécule de principe actif et
§§ représentant la liaison au peptide liant ou à la protéine liante,
x représentant 0 ou 1,
SG représentant un groupe clivable, de préférence un oligopeptide de 2 à 8, particulièrement préférablement un dipeptide,
et
L4 représentant une simple liaison ou un groupe - (CO)_{y}-G4-, y représentant 0 ou 1, et G4 étant une chaîne hydrocarbonée linéaire ou ramifiée comportant 1 à 100 atome(s) de carbone de groupes arylène et/ou de groupes alkylène linéaires et/ou ramifiés et/ou cycliques, qui peut être interrompue une ou plusieurs fois par un ou plusieurs des groupes -O-, -S-, -SO-, SO₂, -NH-, -CO-, -CONH-, - NHCO-, -NMe-, -NHNH-, -SO₂NHNH-, -CONHNH- et un hétérocycle aromatique ou non aromatique à 5 à 10 chaînons comportant jusqu'à 4 hétéroatomes choisis parmi N, O et S, -SO- et -SO₂- (de préférence 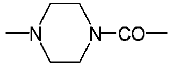 ), les chaînes latérales, le cas échéant, pouvant être substituées par -NHCONH₂, -COOH, -OH, -NH₂, NH-CNNH₂, sulfonamide, sulfone, sulfoxyde ou acide sulfonique.

35. Conjugué d'un peptide liant ou d'une protéine liante selon la revendication 34, le conjugué présentant une des formules suivantes : 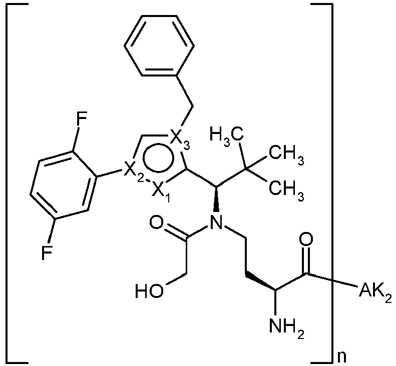 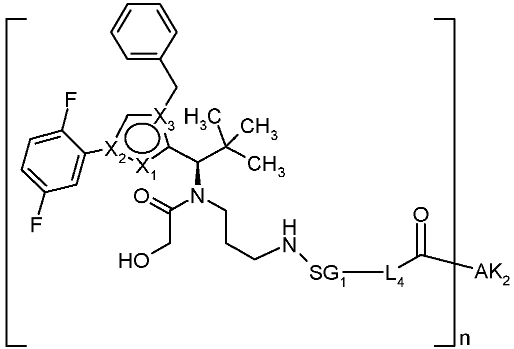
X₁, X₂ et X₃ présentant la même signification que dans la revendication 4,
AK₂ représentant un peptide liant ou une protéine liante, qui est lié par l'intermédiaire d'un atome de soufre du liant ; n représentant un nombre de 1 à 20 ; et L4 étant une chaîne hydrocarbonée eventuellement linéaire ou ramifiée comportant 1 à 100 atome(s) de carbone de groupes arylène et/ou de groupes alkylène linéaires et/ou ramifiés et/ou cycliques, qui peut être interrompue une ou plusieurs fois par un ou plusieurs des groupes -O-, -S-, -SO-, SO₂, -NH-, -CO-, -NMe-, -NHNH-, -SO₂NHNH-, -NHCO-, -CONH-, -CONHNH- et un hétérocycle aromatique ou non aromatique à 5 à 10 chaînons comportant jusqu'à 4 hétéroatomes choisis parmi N, O et S, -SO- et -SO₂- (de préférence 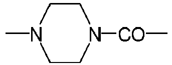 ), les chaînes latérales, le cas échéant, pouvant être substituées par -NHCONH₂, -COOH, -OH, -NH₂, NH-CNNH₂, sulfonamide, sulfone, sulfoxyde ou acide sulfonique, et
SG1 représentant un groupe clivable, de préférence un oligopeptide de 2 à 8, particulièrement préférablement un dipeptide.

36. Conjugué selon l'une ou plusieurs des revendications 18 à 35, l'anticorps anti-TWEAKR étant un anticorps agoniste.

37. Conjugué selon l'une ou plusieurs des revendications 18 à 36, qui comprend :
une chaîne lourde variable comprenant :
a. une CDR1 de la chaîne lourde, qui est codée par une séquence d'acides aminés comprenant la formule PYPMX (SEQ ID NO : 171), X étant I ou M ;
b. une CDR2 de la chaîne lourde, qui est codée par une séquence d'acides aminés comprenant la formule YISPSGGXTHYADSVKG (SEQ ID NO : 172), X étant S ou K ; et
c. une CDR3 de la chaîne lourde, qui est codée par une séquence d'acides aminés comprenant la formule GGDTYFDYFDY (SEQ ID NO : 173) ;
et une chaîne légère variable comprenant :
d. une CDR1 de la chaîne légère, qui est codée par une séquence d'acides aminés comprenant la formule RASQSISXYLN (SEQ ID NO : 174), X étant G ou S ;
e. une CDR2 de la chaîne légère, qui est codée par une séquence d'acides aminés comprenant la formule XASSLQS (SEQ ID NO : 175), X étant Q, A ou N ; et
f. une CDR3 de la chaîne légère, qui est codée par une séquence d'acides aminés comprenant la formule QQSYXXPXIT (SEQ ID NO : 176), X dans la position 5 étant T ou S, X dans la position 6 étant T ou S et X dans la position 8 étant G ou F.

38. Conjugué selon l'une ou plusieurs des revendications 18 à 37, comprenant :
a. une séquence variable de la chaîne lourde, telle que représentée par SEQ ID NO : 10, ainsi qu'une séquence variable de la chaîne légère, telle que représentée par SEQ ID NO : 9 ou
b. une séquence variable de la chaîne lourde, telle que représentée par SEQ ID NO : 20, ainsi qu'une séquence variable de la chaîne légère, telle que représentée par SEQ ID NO : 19 ou
c. une séquence variable de la chaîne lourde, telle que représentée par SEQ ID NO : 30, ainsi qu'une séquence variable de la chaîne légère, telle que représentée par SEQ ID NO : 29 ou
d. une séquence variable de la chaîne lourde, telle que représentée par SEQ ID NO : 40, ainsi qu'une séquence variable de la chaîne légère, telle que représentée par SEQ ID NO : 39 ou
e. une séquence variable de la chaîne lourde, telle que représentée par SEQ ID NO : 50, ainsi qu'une séquence variable de la chaîne légère, telle que représentée par SEQ ID NO : 49 ou
f. une séquence variable de la chaîne lourde, telle que représentée par SEQ ID NO : 60, ainsi qu'une séquence variable de la chaîne légère, telle que représentée par SEQ ID NO : 59 ou
g. une séquence variable de la chaîne lourde, telle que représentée par SEQ ID NO : 70, ainsi qu'une séquence variable de la chaîne légère, telle que représentée par SEQ ID NO : 69 ou
h. une séquence variable de la chaîne lourde, telle que représentée par SEQ ID NO : 80, ainsi qu'une séquence variable de la chaîne légère, telle que représentée par SEQ ID NO : 79 ou
i. une séquence variable de la chaîne lourde, telle que représentée par SEQ ID NO : 90, ainsi qu'une séquence variable de la chaîne légère, telle que représentée par SEQ ID NO : 89 ou
j. une séquence variable de la chaîne lourde, telle que représentée par SEQ ID NO : 100, ainsi qu'une séquence variable de la chaîne légère, telle que représentée par SEQ ID NO : 99 ou
k. une séquence variable de la chaîne lourde, telle que représentée par SEQ ID NO : 110, ainsi qu'une séquence variable de la chaîne légère, telle que représentée par SEQ ID NO : 109 ou
l. une séquence variable de la chaîne lourde, telle que représentée par SEQ ID NO : 120, ainsi qu'une séquence variable de la chaîne légère, telle que représentée par SEQ ID NO : 119.

39. Conjugué selon l'une ou plusieurs des revendications 18 à 38, l'anticorps étant un anticorps IgG.

40. Conjugué selon l'une ou plusieurs des revendications 18 à 39, comprenant :
a. une séquence de la chaîne lourde, telle que représentée par SEQ ID NO : 2, ainsi qu'une séquence de la chaîne légère, telle que représentée par SEQ ID NO : 1 ou
b. une séquence de la chaîne lourde, telle que représentée par SEQ ID NO : 12, ainsi qu'une séquence de la chaîne légère, telle que représentée par SEQ ID NO : 11 ou
c. une séquence de la chaîne lourde, telle que représentée par SEQ ID NO : 22, ainsi qu'une séquence de la chaîne légère, telle que représentée par SEQ ID NO : 21 ou
d. une séquence de la chaîne lourde, telle que représentée par SEQ ID NO : 32, ainsi qu'une séquence de la chaîne légère, telle que représentée par SEQ ID NO : 31 ou
e. une séquence de la chaîne lourde, telle que représentée par SEQ ID NO : 42, ainsi qu'une séquence de la chaîne légère, telle que représentée par SEQ ID NO : 41 ou
f. une séquence de la chaîne lourde, telle que représentée par SEQ ID NO : 52, ainsi qu'une séquence de la chaîne légère, telle que représentée par SEQ ID NO : 51 ou
g. une séquence de la chaîne lourde, telle que représentée par SEQ ID NO : 62, ainsi qu'une séquence de la chaîne légère, telle que représentée par SEQ ID NO : 61 ou
h. une séquence de la chaîne lourde, telle que représentée par SEQ ID NO : 72, ainsi qu'une séquence de la chaîne légère, telle que représentée par SEQ ID NO : 71 ou
i. une séquence de la chaîne lourde, telle que représentée par SEQ ID NO : 82, ainsi qu'une séquence de la chaîne légère, telle que représentée par SEQ ID NO : 81 ou
j. une séquence de la chaîne lourde, telle que représentée par SEQ ID NO : 92, ainsi qu'une séquence de la chaîne légère, telle que représentée par SEQ ID NO : 91 ou
k. une séquence de la chaîne lourde, telle que représentée par SEQ ID NO : 102, ainsi qu'une séquence de la chaîne légère, telle que représentée par SEQ ID NO : 101 ou
l. une séquence de la chaîne lourde, telle que représentée par SEQ ID NO : 112, ainsi qu'une séquence de la chaîne légère, telle que représentée par SEQ ID NO : 111.

41. Conjugué de fuseau selon l'une ou plusieurs des revendications précédentes, le conjugué présentant 1 à 10, de préférence 2 à 8, molécule(s) de principe actif par peptide liant ou par protéine liante.

42. Procédé pour la préparation du conjugué selon la revendication 28 ou 32, un composé d'une des formules suivantes, de préférence sous forme d'un sel avec l'acide trifluoroacétique, étant lié à un radical cystéine d'un peptide liant ou d'une protéine liante précédemment éventuellement partiellement réduit(e), le composé étant utilisé de préférence en un excès molaire de 2 à 12 par rapport au peptide liant ou à la protéine liante : 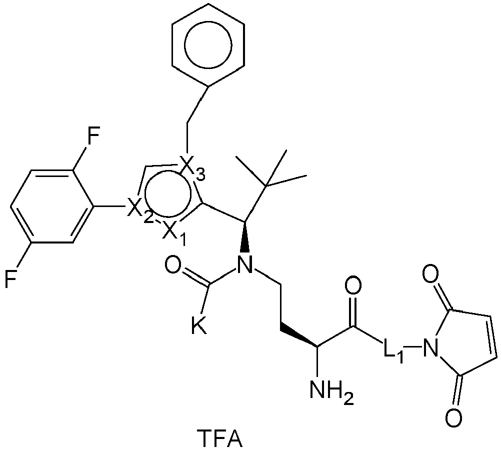 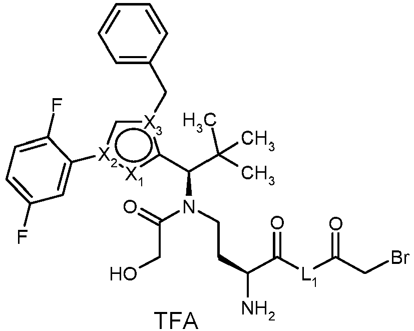 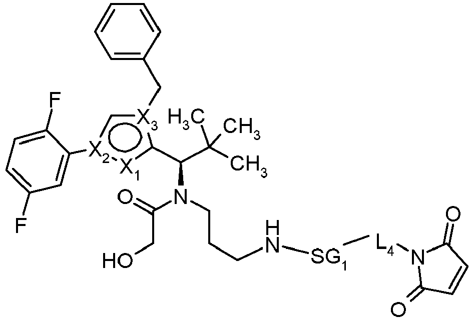 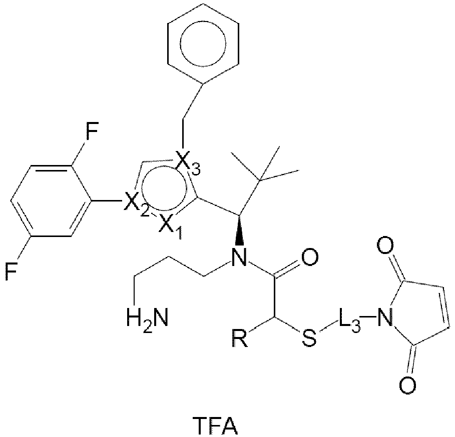 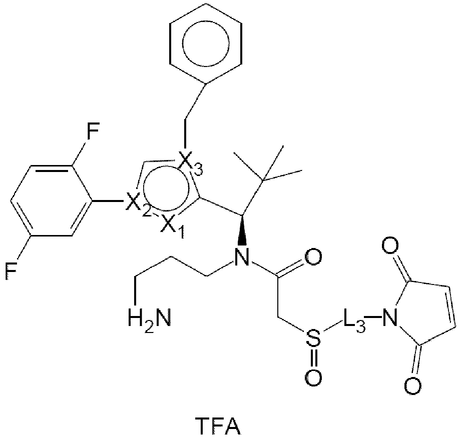 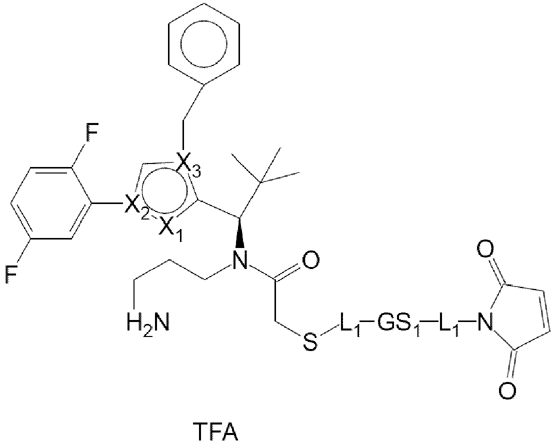 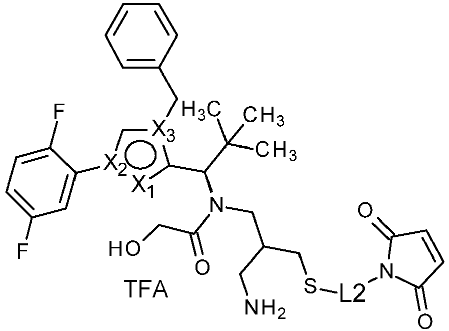 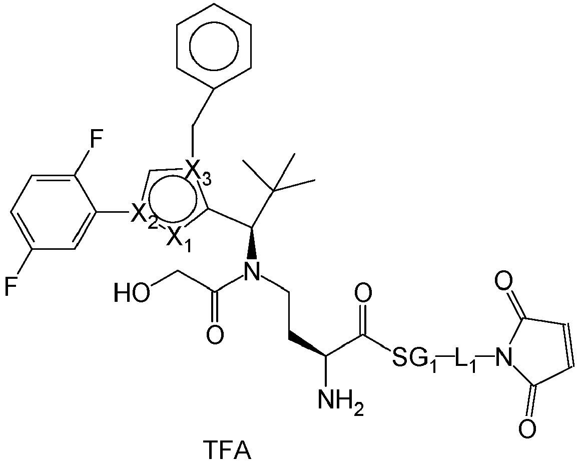 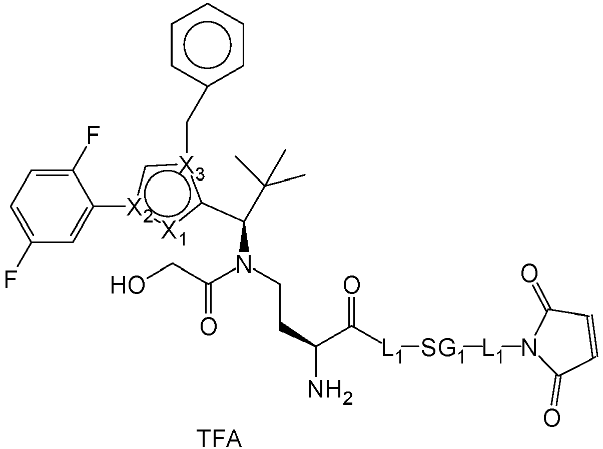 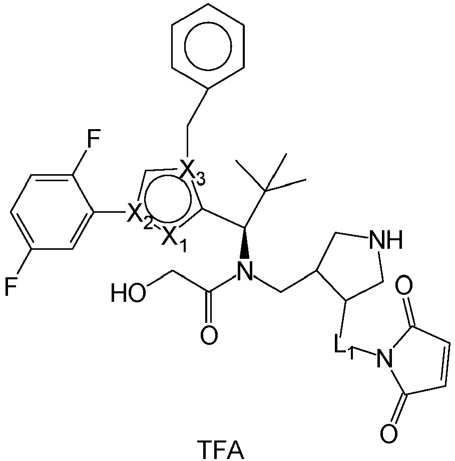 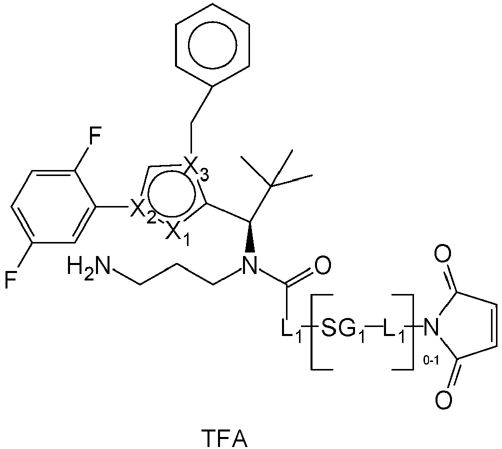 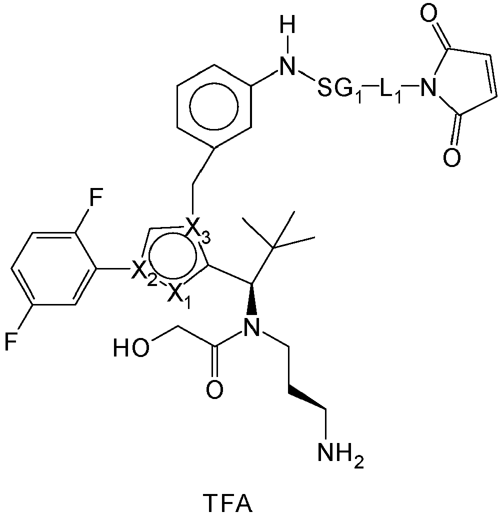
R représentant -H ou -COOH,
K représentant C₁-C₆-alkyle linéaire ou ramifié, éventuellement substitué par C₁-C₆-alcoxy ou -OH, et X₁, X₂, X₃, SG1, L1, L2, L3 et L4 possédant la même signification que dans la revendication 28, ou, selon le cas, 32.

43. Procédé pour la préparation du conjugué selon la revendication 42, un composé d'une des formules suivantes, de préférence sous forme d'un sel avec l'acide trifluoroacétique, étant lié à un radical lysine d'un peptide liant ou d'une protéine liante, le composé étant utilisé de préférence en un excès molaire de 2 à 12 par rapport au peptide liant ou à la protéine liante : 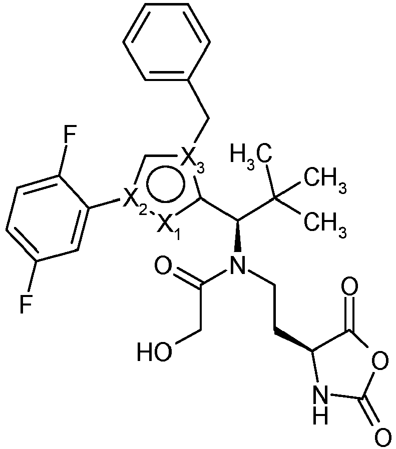 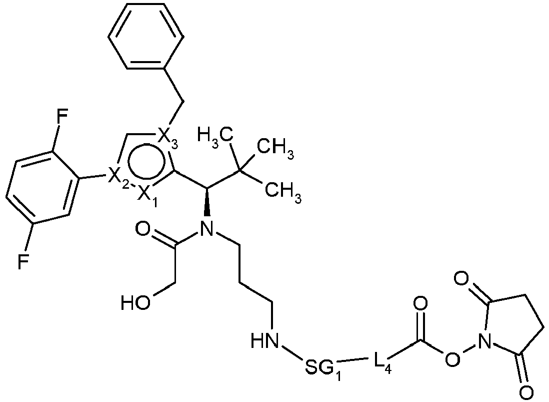 X₁, X₂, X₃, SG1, et L4 possédant la même signification que dans la revendication 34.

44. Composé d'une des formules suivantes : 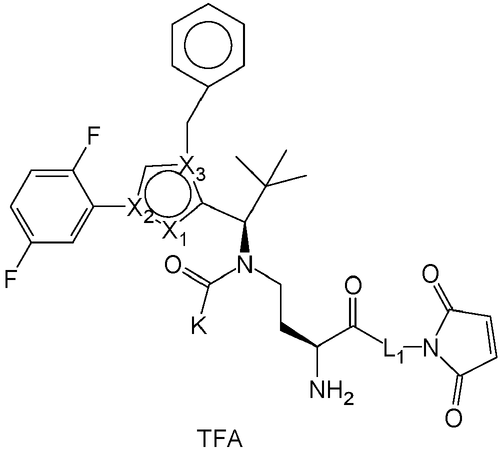 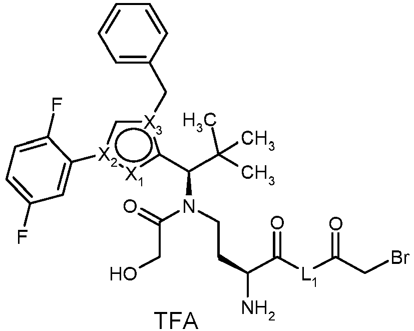 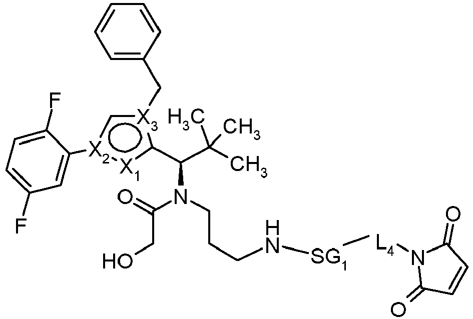 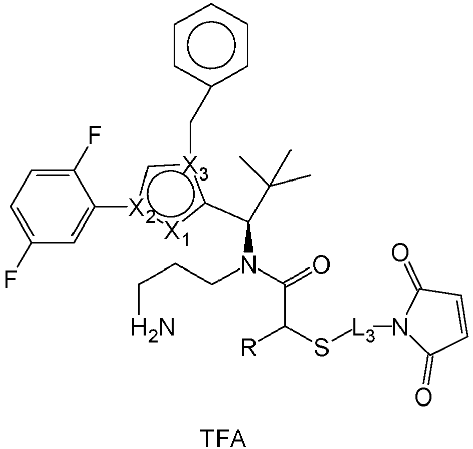 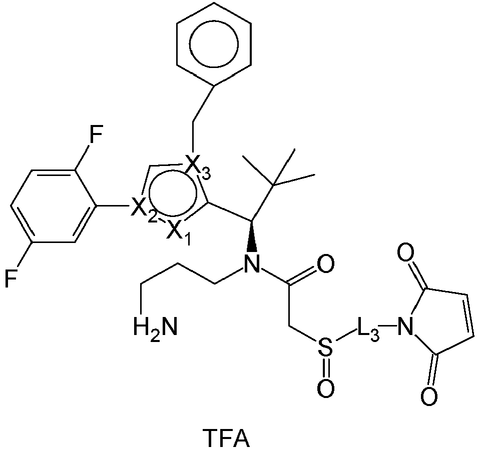 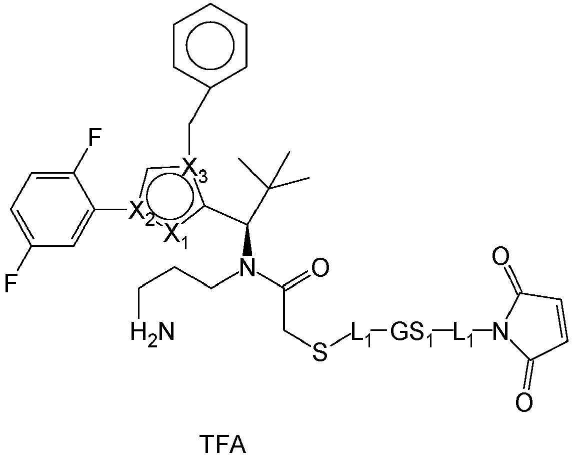 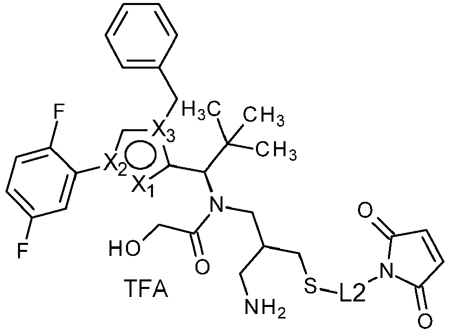 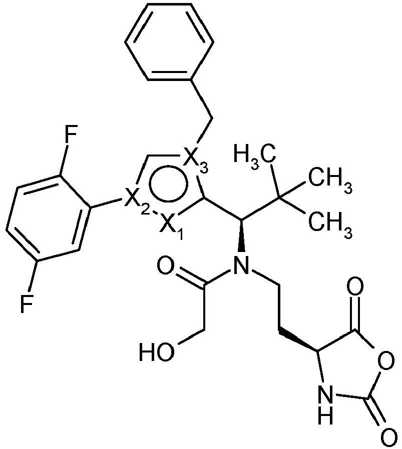 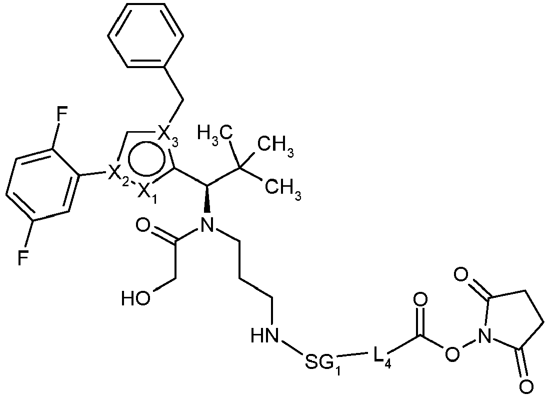 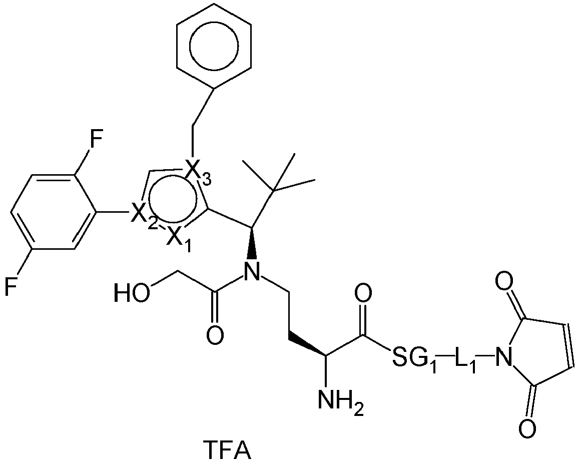 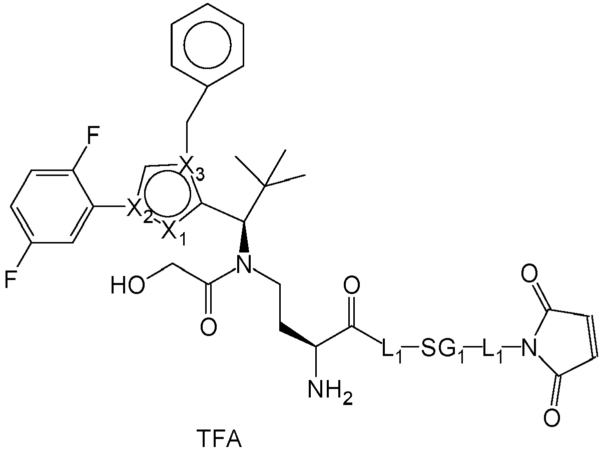 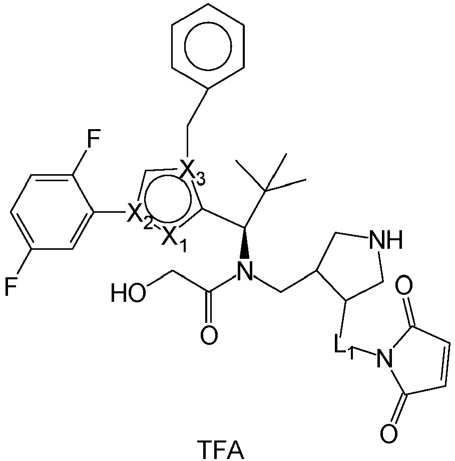 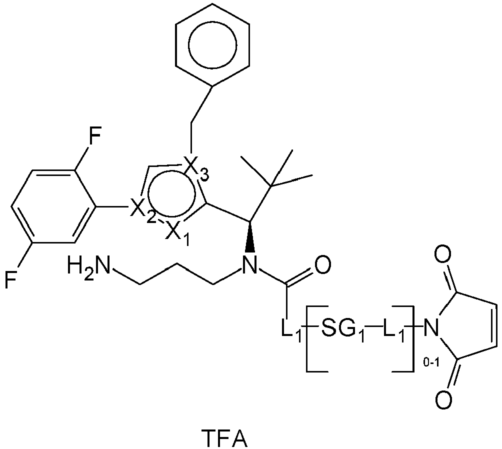 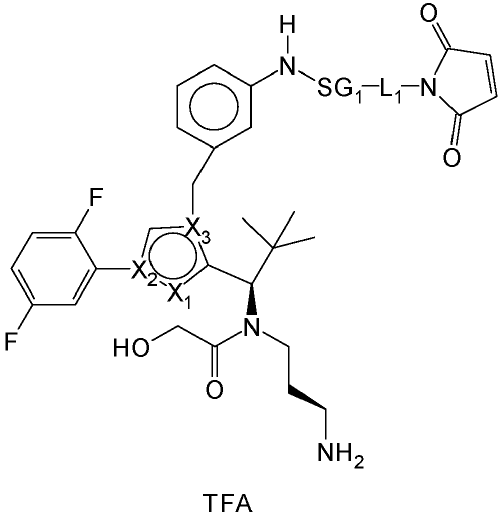
R représentant -H ou -COOH,
K représentant C₁-C₆-alkyle linéaire ou ramifié, éventuellement substitué par C₁-C₆-alcoxy ou -OH, et X₁, X₂, X₃, SG1, L1, L2, L3 et L4 possédant la même signification que dans la revendication 24, 28, ou, selon le cas, 30.

45. Composés de formule générale (III) : 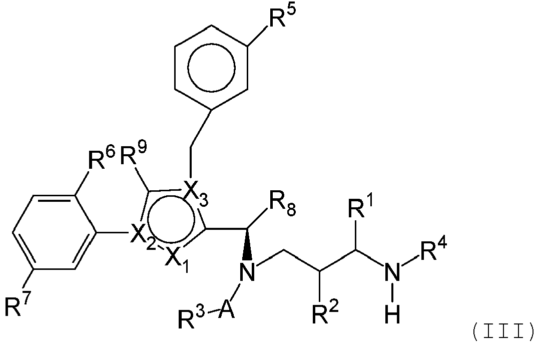
X₁ représentant N, X₂ représentant N et X₃ représentant C, ou X₁ représentant CH, X₂ représentant C et X₃ représentant N, ou X₁ représentant NH, X₂ représentant C et X₃ représentant C, ou X₁ représentant CH, X₂ représentant N et X₃ représentant C ;
R¹ représentant -L-LIANT, H, ou - (CH₂)₀₋₃Z, Z représentant -H, -NHY³, -OY³, -SY³, halogène, -CO-NY¹Y² ou -CO-OY³,
Y¹ et Y² indépendamment l'un de l'autre représentant H, NH₂, -(CH₂CH₂O)₀₋₃-(CH₂)₀₋₃Z' ou -CH(CH₂W)Z', et Y³ représentant H ou - (CH₂)₀₋₃Z', Z' représentant H, NH₂, SO₃H, COOH, -NH-CO-CH₂-CH₂-CH (NH₂)COOH ou - (CO-NH-CHY⁴)₁₋₃COOH ; W représentant H ou OH,
Y⁴ indépendamment les uns des autres représentant C₁₋₆-alkyle linéaire ou ramifié, éventuellement substitué par -NHCONH₂, ou aryle ou benzyle éventuellement substitué par -NH₂ ;
R² et R⁴ indépendamment l'un de l'autre représentant -L-LIANT, H, -CO-CHY⁴-NHY⁵ ou -(CH₂)₀₋₃Z, ou R² et R⁴ représentant ensemble -CH₂-CHR¹⁰- ou -CHR¹⁰-CH₂-(avec formation d'un cycle pyrrolidine), R¹⁰ représentant L-#1, H, NH₂, SO₃H, COOH, SH, ou OH ;
Z représentant -H, halogène, -OY³, -SY³, NHY³, -CO-NY¹Y², ou -CO-OY³,
Y¹ et Y² indépendamment l'un de l'autre représentant H, NH₂, ou -(CH₂)₀₋₃Z', et Y³ représentant H ou-(CH₂)₀₋₃Z', Z' représentant H, SO₃H, NH₂ ou COOH ;
Y⁴ indépendamment les uns des autres représentant C₁₋₆-alkyle linéaire ou ramifié, éventuellement substitué par -NHCONH₂, ou aryle ou benzyle éventuellement substitué par -NH₂ et Y⁵ représentant H ou -CO-CHY⁶-NH₂, Y⁶ représentant C₁₋₆-alkyle linéaire ou ramifié ;
A représentant CO, SO, SO₂, SO₂NH ou CNNH ;
R³ représentant -L-LIANT ou un groupe alkyle, aryle, hétéroaryle, hétéroalkyle, hétérocycloalkyle éventuellement substitué, préférablement -L-#1 ou un groupe C₁₋₁₀-alkyle, C₆₋₁₀-aryle ou C₆₋₁₀-aralkyle, C₅₋₁₀-hétéroalkyle, C₁₋₁₀-alkyl-O-C₆₋₁₀-aryle ou C₅₋₁₀-hétérocycloalkyle, qui peut être substitué par 1 à 3 groupes-OH, 1 à 3 atomes d'halogène, 1 à 3 groupes alkyle halogénés (qui présentent à chaque fois 1 à 3 atomes d'halogène), 1 à 3 groupes O-alkyle, 1 à 3 groupes -SH, 1 à 3 groupes -S-alkyle, 1 à 3 groupes -O-CO-alkyle, 1 à 3 groupes -O-CO-NH-alkyle, 1 à 3 groupes -NH-CO-alkyle, 1 à 3 groupes -NH-CO-NH-alkyle, 1 à 3 groupes -S(O)ₙ-alkyle, 1 à 3 groupes -SO₂-NH-alkyle, 1 à 3 groupes -NH-alkyle, 1 à 3 groupes -N(alkyle)₂, 1 à 3 groupes -NH₂ ou 1 à 3 groupes -(CH₂)₀₋₃Z, Z représentant -H, halogène,-OY³, -SY³, NHY³, -CO-NY¹Y², ou -CO-OY³, Y¹ et Y² indépendamment l'un de l'autre représentant H, NH₂, ou -(CH₂)₀₋₃Z', et Y³ représentant H, -(CH₂)₀₋₃-CH(NHCOCH₃)Z', -(CH₂)₀₋₃-CH(NH₂)Z', ou-(CH₂)₀₋₃Z', Z' représentant H, SO₃H, NH₂ ou COOH (« alkyle » signifiant préférablement C₁₋₁₀-alkyle) ;
R⁵ représentant -L-LIANT, H, F, NH₂, NO₂, halogène, SH ou -(CH₂)₀₋₃Z, Z représentant -H, halogène, -OY³, -SY³, NHY³, -CO-NY¹Y², ou -CO-OY³,
Y¹ et Y² indépendamment l'un de l'autre représentant H, NH₂, ou -(CH₂)₀₋₃Z', et Y³ représentant H ou-(CH₂)₀₋₃Z', Z' représentant H, SO₃H, NH₂ ou COOH ;
L représentant un lieur et LIANT représentant un liant ou, selon le cas, un dérivé de celui-ci, le liant pouvant éventuellement être lié à plusieurs molécules de principe actif,
R⁶ et R⁷ représentant indépendamment l'un de l'autre H, cyano, C₁₋₁₀-alkyle (éventuellement fluoré), C₂₋₁₀-alcényle (éventuellement fluoré), C₂₋₁₀-alcynyle (éventuellement fluoré), hydroxy ou halogène,
R⁸ représentant C₁₋₁₀-alkyle (éventuellement fluoré), C₄₋₁₀-cycloalkyle (éventuellement fluoré) ou oxétane éventuellement substitué ; et
R⁹ représentant H, F, CH₃, CF₃, CH₂F ou CHF₂ ;
ainsi que leurs sels, solvates et sels des solvates.

46. Composé de formule générale (IV) : 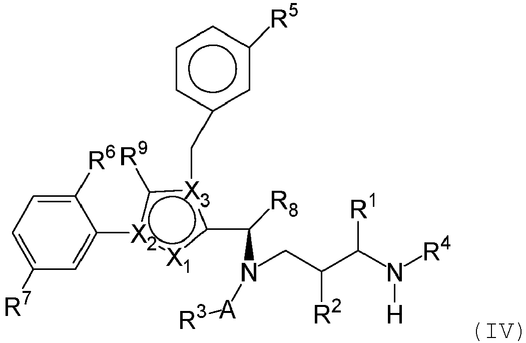
X₁ représentant CH, X₂ représentant C et X₃ représentant N ;
R¹ représentant -L-LIANT, H, ou -(CH₂)₀₋₃Z, Z représentant -H, -NHY³, -OY³, -SY³, halogène, -CO-NY¹Y² ou -CO-OY³,
Y¹ et Y² indépendamment l'un de l'autre représentant H, NH₂, -(CH₂CH₂O)₀₋₃-(CH₂)₀₋₃Z' ou -CH(CH₂W)Z', et Y³ représentant H ou -(CH₂)₀₋₃Z', Z' représentant H, NH₂, SO₃H, COOH, -NH-CO-CH₂-CH₂-CH (NH₂)COOH ou - (CO-NH-CHY⁴)₁₋₃COOH ; W représentant H ou OH,
Y⁴ indépendamment les uns des autres représentant C₁₋₆-alkyle linéaire ou ramifié, éventuellement substitué par -NHCONH₂, ou aryle ou benzyle éventuellement substitué par -NH₂ ;
R² et R⁴ indépendamment l'un de l'autre représentant -L-LIANT, H, -CO-CHY⁴-NHY⁵ ou -(CH₂)₀₋₃Z, ou R² et R⁴ représentant ensemble -CH₂-CHR¹⁰- ou -CHR¹⁰-CH₂-(avec formation d'un cycle pyrrolidine), R¹⁰ représentant L-#1, H, NH₂, SO₃H, COOH, SH, ou OH ;
Z représentant -H, halogène, -OY³, -SY³, NHY³, -CO-NY¹Y², ou -CO-OY³,
Y¹ et Y² indépendamment l'un de l'autre représentant H, NH₂, ou -(CH₂)₀₋₃Z', et Y³ représentant H ou-(CH₂)₀₋₃Z', Z' représentant H, SO₃H, NH₂ ou COOH ;
Y⁴ indépendamment les uns des autres représentant C₁₋₆-alkyle linéaire ou ramifié, éventuellement substitué par -NHCONH₂, ou aryle ou benzyle éventuellement substitué par -NH₂ et Y⁵ représentant H ou -CO-CHY⁶-NH₂, Y⁶ représentant C₁₋₆-alkyle linéaire ou ramifié ;
A représentant CO, SO, SO₂, SO₂NH ou CNNH ;
R³ représentant -L-LIANT ou un groupe alkyle, aryle, hétéroaryle, hétéroalkyle, hétérocycloalkyle éventuellement substitué, préférablement -L-#1 ou un groupe C₁₋₁₀-alkyle, C₆₋₁₀-aryle ou C₆₋₁₀-aralkyle, C₅₋₁₀-hétéroalkyle, C₁₋₁₀-alkyl-O-C₆₋₁₀-aryle ou C₅₋₁₀-hétérocycloalkyle, qui peut être substitué par 1 à 3 groupes -OH, 1 à 3 atomes d'halogène, 1 à 3 groupes alkyle halogénés (qui présentent à chaque fois 1 à 3 atomes d'halogène), 1 à 3 groupes O-alkyle, 1 à 3 groupes -SH, 1 à 3 groupes -S-alkyle, 1 à 3 groupes -O-CO-alkyle, 1 à 3 groupes -O-CO-NH-alkyle, 1 à 3 groupes -NH-CO-alkyle, 1 à 3 groupes -NH-CO-NH-alkyle, 1 à 3 groupes -S(O)ₙ-alkyle, 1 à 3 groupes -SO₂-NH-alkyle, 1 à 3 groupes -NH-alkyle, 1 à 3 groupes -N(alkyle)₂, 1 à 3 groupes -NH₂ ou 1 à 3 groupes -(CH₂)₀₋₃Z, Z représentant -H, halogène,-OY³, -SY³, NHY³, -CO-NY¹Y², ou -CO-OY³, Y¹ et Y² indépendamment l'un de l'autre représentant H, NH₂, ou - (CH₂)₀₋₃Z', et Y³ représentant H, -(CH₂)₀₋₃-CH(NHCOCH₃)Z', -(CH₂)₀₋₃-CH(NH₂)Z', ou-(CH₂)₀₋₃Z', Z' représentant H, SO₃H, NH₂ ou COOH (« alkyle » signifiant préférablement C₁₋₁₀-alkyle) ;
R⁵ représentant -L-LIANT, H, F, NH₂, NO₂, halogène, SH ou -(CH₂)₀₋₃Z, Z représentant -H, halogène, -OY³, -SY³, NHY³, -CO-NY¹Y², ou -CO-OY³,
Y¹ et Y² indépendamment l'un de l'autre représentant H, NH₂, ou -(CH₂)₀₋₃Z', et Y³ représentant H ou-(CH₂)₀₋₃Z', Z' représentant H, SO₃H, NH₂ ou COOH ;
L représentant un lieur et LIANT représentant un liant ou, selon le cas, un dérivé de celui-ci, le liant pouvant éventuellement être lié à plusieurs molécules de principe actif,
R⁶ et R⁷ représentant indépendamment l'un de l'autre H, cyano, C₁₋₁₀-alkyle (éventuellement fluoré), C₂₋₁₀-alcényle (éventuellement fluoré), C₂₋₁₀-alcynyle (éventuellement fluoré), hydroxy ou halogène,
R⁸ représentant C₁₋₁₀-alkyle (éventuellement fluoré), C₄₋₁₀-cycloalkyle (éventuellement fluoré) ou oxétane éventuellement substitué ; et
R⁹ représentant H, F, CH₃, CF₃, CH₂F ou CHF₂ ;
ainsi que leurs sels, solvates et sels des solvates.

47. Composé selon la revendication 46, Z représentant Cl ou Br.

48. Composé selon la revendication 46, R¹ représentant -(CH₂)₀₋₃Z, Z représentant -CO-NY¹Y², Y² représentant -(CH₂CH₂O)₀₋₃-(CH₂)₀₋₃Z' et Y¹ représentant H, NH₂, ou -(CH₂CH₂O)₀₋₃-(CH₂)₀₋₃Z'.

49. Composé selon l'une ou plusieurs des revendications 46 à 48, Y¹ représentant H, Y² représentant - (CH₂CH₂O)₃-CH₂CH₂Z' et Z' représentant -COOH.

50. Composé selon l'une ou plusieurs des revendications 46 à 48, Y¹ représentant H, Y² représentant -CH₂CH₂Z' et Z' représentant -(CONHCHY⁴)₂COOH.

51. Composé selon la revendication 46, Y⁴ représentant C₁₋₆-alkyle linéaire ou ramifié, éventuellement substitué par -NHCONH₂.

52. Composé selon la revendication 46, au moins un représentant de Y⁴ étant choisi parmi iso-propyle et -CH₃.

53. Composé selon l'une ou plusieurs des revendications 46 et 48, Y¹ représentant H, Y² représentant-CH₂CH₂Z', Z' représentant -CONHCHY⁴COOH et Y⁴ représentant aryle ou benzyle éventuellement substitué par -NH₂.

54. Composé selon la revendication 53, Y⁴ représentant aminobenzyle.

55. Composé selon la revendication 46, R² représentant -(CH₂)₀₋₃Z et Z représentant -SY³.

56. Composé selon la revendication 46, R⁴ représentant -CO-CHY⁴-NHY⁵ et Y⁵ représentant H.

57. Composé selon la revendication 46, R⁴ représentant -CO-CHY⁴-NHY⁵ et Y⁵ représentant -CO-CHY⁶-NH₂.

58. Composé selon l'une ou plusieurs des revendications 56 et 57, Y⁴ représentant C₁₋₆-alkyle linéaire ou ramifié, éventuellement substitué par -NHCONH₂.

59. Composé selon l'une ou plusieurs des revendications 46 à 58, le composé présentant l'une des formules suivantes :
N-(3-aminopropyl)-N-{(1R)-1-[4-benzyl-1-(2,5-difluorophényl)-1H-pyrazol-3-yl]-2,2-diméthylpropyl}-2-hydroxyacétamide ;
N-(3-aminopropyl)-N-{(1R)-1-[1-benzyl-4-(2,5-difluorophényl)-1H-pyrrol-2-yl]-2,2-diméthylpropyl}-2-hydroxyacétamide ;
(2S)-2-amino-4-[{(1R)-1-[1-benzyl-4-(2,5-difluorophényl)-1H-pyrrol-2-yl]-2,2-diméthylpropyl}(glycoloyl)amino]-N-méthylbutanamide (1:1);
N-(3-aminopropyl)-N-{(1S)-1-[1-benzyl-4-(2,5-difluorophényl)-1H-pyrrol-2yl]-2,2-diméthylpropyl}acétamide;
N-(3-aminopropyl)-N-{(1S)-1-[1-benzyl-4-(2,5-difluorophényl)-1H-pyrrol-2-yl]-2,2-diméthylpropyl}-2-hydroxyacétamide;
S-[1-(2-{[2-({(2S)-2-amino-4-[{(1R)-1-[4-benzyl-1-(2,5-difluorophényl)-1H-pyrazol-3-yl]-2,2-diméthylpropyl}(glycoloyl)amino]butanoyl}amino)éth yl]amino}-2-oxoéthyl)-2,5-dioxopyrrolidin-3-yl]-L-cystéine;
N-{(2S)-2-amino-4-[{(1R)-1-[4-benzyl-1-(2,5-difluorophényl)-1H-pyrazol-3-yl]-2,2-diméthylpropyl}(glycoloyl)amino]butanoyl}-bêta-alanyl-L-alanyl-N-[4-(3-{[(2R)-2-amino-2-carboxyéthyl]sulfanyl}-2,5-dioxopyrrolidin-1-yl)phényl]-N⁵-carbamoyl-L-ornithinamide;
S-(1-{2-[(N-{(2S)-2-amino-4-[{(1R)-1-[1-benzyl-4-(2,5-difluorophényl)-1H-pyrrol-2-yl]-2,2-diméthylpropyl}(glycoloyl)amino]butanoyl}-bêta-alanyl)amino]éthyl}-2,5-dioxopyrrolidin-3-yl)-L-cystéine;
S-[1-(2-{[2-({(2S)-2-amino-4-[{(1R)-1-[4-benzyl-1-(2,5-difluorophényl)-1H-pyrazol-3-yl]-2,2-diméthylpropyl}(glycoloyl)amino]butanoyl}amino)éth yl]amino}-2-oxoéthyl)-2,5-dioxopyrrolidin-3-yl]-L-cystéine;
N-{(2S)-2-amino-4-[{(1R)-1-[4-benzyl-1-(2,5-difluorophényl)-1H-pyrazol-3-yl]-2,2-diméthylpropyl}(glycoloyl)amino]butanoyl}-bêta-alanyl-L-alanyl-N-[4-(3-{[(2R)-2-amino-2-carboxyéthyl]sulfanyl}-2,5-dioxopyrrolidin-1-yl)phényl]-N⁵-carbamoyl-L-ornithinamide;
S-(1-{2-[(N-{(2S)-2-amino-4-[{(1R)-1-[1-benzyl-4-(2,5-difluorophényl)-1H-pyrrol-2-yl]-2,2-diméthylpropyl}(glycoloyl)amino]butanoyl}-bêta-alanyl)amino]éthyl}-2,5-dioxopyrrolidin-3-yl)-L-cystéine;
N-[6-(3-{[(2R)-2-amino-2-carboxyéthyl]sulfanyl}2,5-dioxopyrrolidin-1-yl)hexanoyl]-L-valyl-N⁵-carbamoyl-L-ornithyl-N⁶-{(2S)-2-amino-4-[{(1R)-1-[4-benzyl-1-(2,5-difluorophényl)-1H-pyrazol-3-yl]-2,2-diméthylpropyl}(glycoloyl)amino]butanoyl}-L-lysine ;
S-[1-(2-{[2-({(2S)-2-amino-4-[{(1R)-1-[1-benzyl-4-(2,5-difluorophényl)-1H-pyrrol-2-yl]-2,2-diméthylpropyl}(glycoloyl)amino]butanoyl}amino)éth yl]amino}-2-oxoéthyl)-2,5-dioxopyrrolidin-3-yl]-L-cystéine;
S-(2-{[2-({(2S)-2-amino-4-[{(1R)-1-[4-benzyl-1-(2,5-difluorophényl)-1H-pyrazol-3-yl]-2,2-diméthylpropyl}(glycoloyl)amino]butanoyl}amino)éth yl]amino}-2-oxoéthyl)-L-cystéine;
S-{1-[6-(2-{(2S)-2-amino-4-[{(1R)-1-[4-benzyl-1-(2,5-difluorophényl)-1H-pyrazol-3-yl]-2,2-diméthylpropyl}(glycoloyl)amino]butanoyl}hydrazino )-6-oxohexyl]-2,5-dioxopyrrolidin-3-yl}-L-cystéine ;
N-[19-(3(R/S)-{[(2R)-2-amino-2-carboxyéthyl]sulfanyl}-2,5-dioxopyrrolidin-1-yl)-17-oxo-4,7,10,13-tétraoxa-16-azanonadécan-1-oyl]-R/S-{2-[(3-aminopropyl){(1R)-1-[1-benzyl-4-(2,5-difluorophényl)-1H-pyrrol-2-yl]-2,2-diméthylpropyl}amino]-2-oxoéthyl}homocystéine;
S-{(3R/S)-1-[2-({(2S)-2-amino-4-[{(1R)-1-[1-benzyl-4-(2,5-difluorophényl)-1H-pyrrol-2-yl]-2,2-diméthylpropyl}(glycoloyl)amino]butanoyl}amino)éth yl]-2,5-dioxopyrrolidin-3-yl}-L-cystéine;
N-[19-(3(R/S)-{[(2R)-2-amino-2-carboxyéthyl]sulfanyl}-2,5-dioxopyrrolidin-1-yl)-17-oxo-4,7,10,13-tétraoxa-16-azanonadécan-1-oyl]-R/S-{2-[(3-aminopropyl){(1R)-1-[1-benzyl-4-(2,5-difluorophényl)-1H-imidazol-2-yl]-2,2-diméthylpropyl}amino]-2-oxoéthyl}homocystéine;
S-[(3R/S)-1-(2-{[6-({2-[(3-aminopropyl){(1R)-1-[1-benzyl-4-(2,5-difluorophényl)-1H-pyrrol-2-yl]-2,2-diméthylpropyl}amino]-2-oxoéthyl}sulfanyl)hexanoyl]amino}éthyl)-2,5-dioxopyrrolidin-3-yl]-L-cystéine;
S-{1-[2-({[(1R,3S)-3-({(2S)-2-amino-4-[{(1R)-1-[1-benzyl-4-(2,5-difluorophényl)-1H-pyrrol-2-yl]-2,2-diméthylpropyl}(glycoloyl)amino]butanoyl}amino)cyc lopentyl]carbonyl}amino)éthyl]-2,5-dioxopyrrolidin-3-yl}-L-cystéine;
S-(2-{[2-({(2S)-2-amino-4-[{(1R)-1-[1-benzyl-4-(2,5-difluorophényl)-1H-pyrrol-2-yl]-2,2-diméthylpropyl}(glycoloyl)amino]butanoyl}amino)éth yl]amino}-2-oxoéthyl)-L-cystéine;
N6-(N-{(2S)-2-amino-4-[{(1R)-1-[1-benzyl-4-(2,5-difluorophényl)-1H-pyrrol-2-yl]-2,2-diméthylpropyl}(glycoloyl)amino]butanoyl}-bêta-alanyl)-N²-{N-[6-(3-{[(2R)-2-amino-2-carboxyéthyl]sulfanyl}-2,5-dioxopyrrolidin-1-yl)hexanoyl]-L-valyl-L-alanyl}-L-lysine;
N-[2-({(2S)-2-amino-4-[{(1R)-1-[1-benzyl-4-(2,5-difluorophényl)-1H-pyrrol-2-yl]-2,2-diméthylpropyl}(glycoloyl)amino]butanoyl}amino)éth yl]-L-glutamine;
N6-(N-{(2S)-2-amino-4-[{(1R)-1-[1-benzyl-4-(2,5-difluorophényl)-1H-pyrrol-2-yl]-2,2-diméthylpropyl}(glycoloyl)amino]butanoyl}-bêta-alanyl)-L-lysine;
N-(3-aminopropyl)-N-{(1R)-1-[4-benzyl-1-(2,5-difluorophényl)-1H-pyrazol-3-yl]-2,2-diméthylpropyl}acétamide;
N-(3-aminopropyl)-N-{(1R)-1-[4-benzyl-1-(2,5-difluorophényl)-1H-pyrazol-3-yl]-2,2-diméthylpropyl}-2-méthoxyacétamide;
N-(3-aminopropyl)-N-{(1R)-1-[4-benzyl-1-(2,5-difluorophényl)-1H-pyrazol-3-yl]-2,2-diméthylpropyl}-2,4-difluorobenzamide;
N-(3-aminopropyl)-N-{(1R)-1-[4-benzyl-1-(2,5-difluorophényl)-1H-pyrazol-3-yl]-2,2-diméthylpropyl}-4-méthylbenzamide;
N-(3-aminopropyl)-N-{(1R)-1-[4-benzyl-1-(2,5-difluorophényl)-1H-pyrazol-3-yl]-2,2-diméthylpropyl}-2-éthoxyacétamide;
N-(3-aminopropyl)-N-{(1R)-1-[1-benzyl-4-(2,5-difluorophényl)-1H-pyrrol-2-yl]-2,2-diméthylpropyl}-3,3,3-trifluoropropanamide;
N-(3-aminopropyl)-N-{(1R)-1-[1-benzyl-4-(2,5-difluorophényl)-1H-pyrrol-2-yl]-2,2-diméthylpropyl}-4-fluorbenzamide;
N-(3-aminopropyl)-N-{(1R)-1-[1-benzyl-4-(2,5-difluorophényl)-1H-pyrrol-2-yl]-2,2-diméthylpropyl}acétamide;
N-(3-aminopropyl)-N-{(1R)-1-[1-benzyl-4-(2,5-difluorophényl)-1H-pyrrol-2-yl]-2,2-diméthylpropyl}-4-(trifluorométhyl)benzamide;
N-(3-aminopropyl)-N-{(1R)-1-[4-benzyl-1-(2,5-difluorophényl)-1H-pyrazol-3-yl]-2,2-diméthylpropyl}-2-éthoxyacétamide;
N-(3-aminopropyl)-N-{(1R)-1-[4-benzyl-1-(2,5-difluorophényl)-1H-pyrazol-3-yl]-2,2-diméthylpropyl}-2-éthoxyacétamide;
acide (2S)-2-amino-4-[{(1R)-1-[1-benzyl-4-(2,5-difluorophényl)-1H-pyrrol-2-yl]-2,2-diméthylpropyl}(glycoloyl)amino]butanoïque;
(2S)-2-amino-N-(2-aminoéthyl)-4-[{(1R)-1-[1-benzyl-4-(2,5-difluorophényl)-1H-pyrrol-2-yl]-2,2-diméthylpropyl}(glycoloyl)amino]butanamide;
acide 4-[(2-{[2-({(2S)-2-amino-4-[{(1R)-1-[1-benzyl-4-(2,5-difluorophényl)-1H-pyrrol-2-yl]-2,2-diméthylpropyl}(glycoloyl)amino]butanoyl}amino)éth yl]amino}-2-oxoéthyl)amino]-3-{[(2R)-2-amino-2-carboxyéthyl]sulfanyl}-4-oxobutanoïque;
acide 4-[(2-{[2-({(2S)-2-amino-4-[{(1R)-1-[1-benzyl-4-(2,5-difluorophényl)-1H-pyrrol-2-yl]-2,2-diméthylpropyl}(glycoloyl)amino]butanoyl}amino)éth yl]amino}-2-oxoéthyl)amino]-2-{[(2R)-2-amino-2-carboxyéthyl]sulfanyl}-4-oxobutanoïque;
N-{(2S)-2-amino-4-[{(1R)-1-[1-benzyl-4-(2,5-difluorophényl)-1H-pyrrol-2-yl]-2,2-diméthylpropyl}(glycoloyl)amino]butanoyl}-bêta-alanine;
N-{(2S)-2-amino-4-[{(1R)-1-[1-benzyl-4-(2,5-difluorophényl)-1H-pyrrol-2-yl]-2,2-diméthylpropyl}(glycoloyl)amino]butanoyl}-L-sérine;
N-{(2S)-2-amino-4-[{(1R)-1-[1-benzyl-4-(2,5-difluorophényl)-1H-pyrrol-2-yl]-2,2-diméthylpropyl}(glycoloyl)amino]butanoyl}-L-alanine;
N-{(2S)-2-amino-4-[{(1R)-1-[1-benzyl-4-(2,5-difluorophényl)-1H-pyrrol-2-yl]-2,2-diméthylpropyl}(glycoloyl)amino]butanoyl}glycine;
N-(3-aminopropyl)-N-{(1R)-1-[1-benzyl-4-(2,5-difluorophényl)-1H-pyrrol-2-yl]-2,2-diméthylpropyl}-4-méthylbenzamide;
N-(3-aminopropyl)-N-{(1R)-1-[1-benzyl-4-(2,5-difluorophényl)-1H-pyrrol-2-yl]-2,2-diméthylpropyl}-4-(méthylsulfanyl)benzamide;
(2S)-N-(3-aminopropyl)-N-{(1R)-1-[1-benzyl-4-(2,5-difluorophényl)-1H-pyrrol-2-yl]-2,2-diméthylpropyl}-2-hydroxypropanamide;
N-(3-aminopropyl)-N-{(1R)-1-[1-benzyl-4-(2,5-difluorophényl)-1H-pyrrol-2-yl]-2,2-diméthylpropyl}-2-(méthylsulfanyl)acétamide;
(2S)-N-(3-aminopropyl)-N-{(1R)-1-[4-benzyl-1-(2,5-difluorophényl)-1H-pyrazol-3-yl]-2,2-diméthylpropyl}-2-hydroxypropanamide;
4-[(3-aminopropyl){(1R)-1-[1-benzyl-4-(2,5-difluorophényl)-1H-pyrrol-2-yl]-2,2-diméthylpropyl}amino]-4-oxobutanoate de méthyle ;
acide 4-[(3-aminopropyl){(1R)-1-[1-benzyl-4-(2,5-difluorophényl)-1H-pyrrol-2-yl]-2,2-diméthylpropyl}amino]-4-oxobutanoïque;
acide (2R)-22-[(3R/S)-3-{[(2R)-2-amino-2-carboxyéthyl]sulfanyl}-2,5-dioxopyrrolidin-1-yl]-2-[({2-[(3-aminopropyl){(1R)-1-[1-benzyl-4-(2,5-difluorophényl)-1H-pyrrol-2-yl]-2,2-diméthylpropyl}amino]-2-oxoéthyl}sulfanyl)méthyl]-4,20-dioxo-7,10,13,16-tétraoxa-3,19-diazadocosan-1-oïque;
4-amino-N-(3-aminopropyl)-N-{(1R)-1-[4-benzyl-1-(2,5-difluorophényl)-1H-pyrazol-3-yl]-2,2-diméthylpropyl}benzamide;
N-acétyl-S-{2-[(3-aminopropyl){(1R)-1-[1-benzyl-4-(2,5-difluorophényl)-1H-pyrrol-2-yl]-2,2-diméthylpropyl}amino]-2-oxoéthyl}-L-cystéine;
N-acétyl-S-[2-([3-(L-alanylamino)propyl]{(1R)-1-[1-benzyl-4-(2,5-difluorophényl)-1H-pyrrol-2-yl]-2,2-diméthylpropyl}amino)-2-oxoéthyl]-L-cystéine;
(2S)-N-(3-aminopropyl)-N-{(1R)-1-[1-benzyl-4-(2,5-difluorophényl)-1H-pyrrol-2-yl]-2,2-diméthylpropyl}tétrahydrofuranne-2-carboxamide;
acide 3-({2-[(3-aminopropyl){(1R)-1-[1-benzyl-4-(2,5-difluorophényl)-1H-pyrrol-2-yl]-2,2-diméthylpropyl}amino]-2-oxoéthyl}sulfanyl)propanoïque;
S-{2-[(3-aminopropyl){(1R)-1-[1-benzyl-4-(2,5-difluorophényl)-1H-pyrrol-2-yl]-2,2-diméthylpropyl}amino]-2-oxoéthyl}homocystéine;
4-amino-N-(3-aminopropyl)-N-{(1R)-1-[1-benzyl-4-(2,5-difluorophényl)-1H-pyrrol-2-yl]-2,2-diméthylpropyl}benzamide;
acide 4-[(2-{[(2R)-2-({(2S)-2-amino-4-[{(1R)-1-[1-benzyl-4-(2,5-difluorophényl)-1H-pyrrol-2-yl]-2,2-diméthylpropyl}(glycoloyl)amino]butanoyl}amino)-2-carboxyéthyl]amino}-2-oxoéthyl)amino]-3-{[(2R)-2-amino-2-carboxyéthyl]sulfanyl}-4-oxobutanoïque;
acide 4-[(2-{[(2R)-2-({(2S)-2-amino-4-[{(1R)-1-[1-benzyl-4-(2,5-difluorophényl)-1H-pyrrol-2-yl]-2,2-diméthylpropyl}(glycoloyl)amino]butanoyl}amino)-2-carboxyéthyl]amino}-2-oxoéthyl)amino]-2-{[(2R)-2-amino-2-carboxyéthyl]sulfanyl}-4-oxobutanoïque.

60. Composition pharmaceutique comprenant un conjugué selon l'une ou plusieurs des revendications 1 à 41 ou un composé selon les revendications 46 à 59 en combinaison avec un excipient inerte, non toxique, pharmaceutiquement approprié.

61. Conjugué selon l'une ou plusieurs des revendications 1 à 41 ou composé selon les revendications 46 à 59 pour une utilisation dans un procédé de traitement et/ou de prophylaxie de maladies.

62. Conjugué selon l'une ou plusieurs des revendications 1 à 41 ou composé selon les revendications 46 à 59 pour une utilisation dans un procédé pour le traitement de maladies hyperprolifératives et/ou angiogéniques.

63. Conjugué selon l'une ou plusieurs des revendications 1 à 41 ou composés selon les revendications 46 à 59, R^{3a} ou, selon le cas, R³ représentant -L-LIANT ou un groupe alkyle, aryle ou hétéroaryle substitué, préférablement représentant -L-#1, ou un groupe C₁₋₁₀-alkyle, C₆₋₁₀-aryle ou C₆₋₁₀-aralkyle ou C₅₋₁₀-hétéroalkyle, qui peut être substitué par -OH, O-alkyle, SH, S-alkyle, O-CO-alkyle, O-CO-NH-alkyle, NH-CO-alkyle, NH-CO-NH-alkyle, S(O)ₙ-alkyle, SO₂-NH-alkyle, NH-alkyle, N(alkyle)₂, NH₂ ou (CH₂)₀₋₃Z, Z représentant -H, halogène, -OY³, -SY³, NHY³, -CO-NY¹Y², ou -CO-OY³, Y¹ et Y² indépendamment l'un de l'autre représentant H, NH₂, ou -(CH₂)₀₋₃Z', et Y³ représentant H ou -(CH₂)₀₋₃Z', Z' représentant H, SO₃H, NH₂ ou COOH (« alkyle » signifiant préférablement C₁₋₁₀-alkyle).

64. Conjugué selon l'une ou plusieurs des revendications 1 à 41 ou composé selon l'une des revendications 46 à 59 pour une utilisation dans un procédé pour le traitement et/ou la prophylaxie de maladies hyperprolifératives et/ou angiogéniques chez des humains et des animaux par l'application d'une quantité efficace.

65. Conjugué d'anticorps selon l'une des formules suivantes, n étant un entier de 1 à 20 et AK1A ou, selon le cas, AK2A représentant un anticorps : 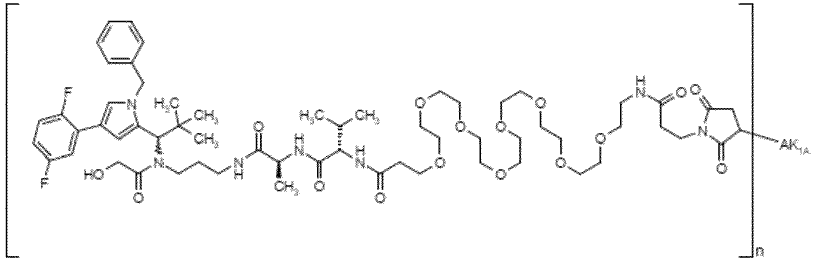 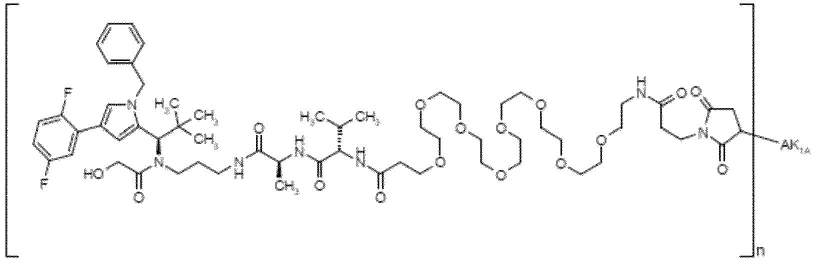 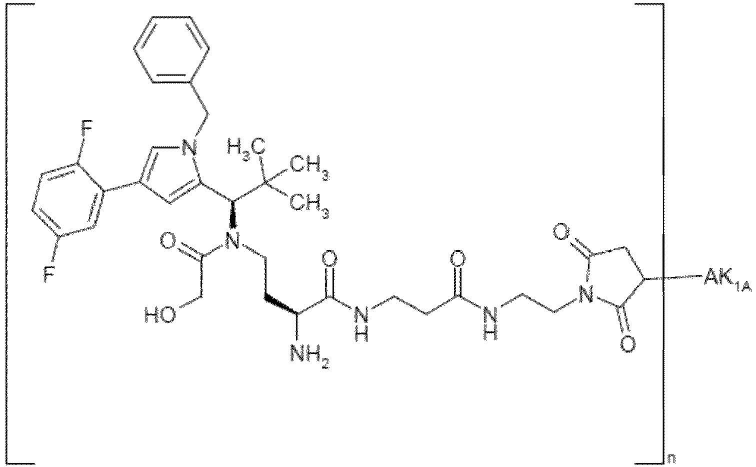 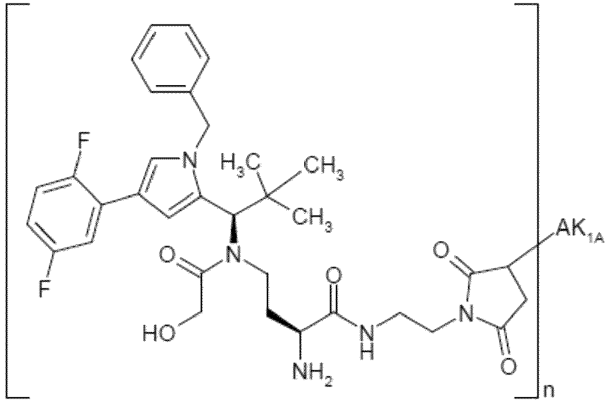 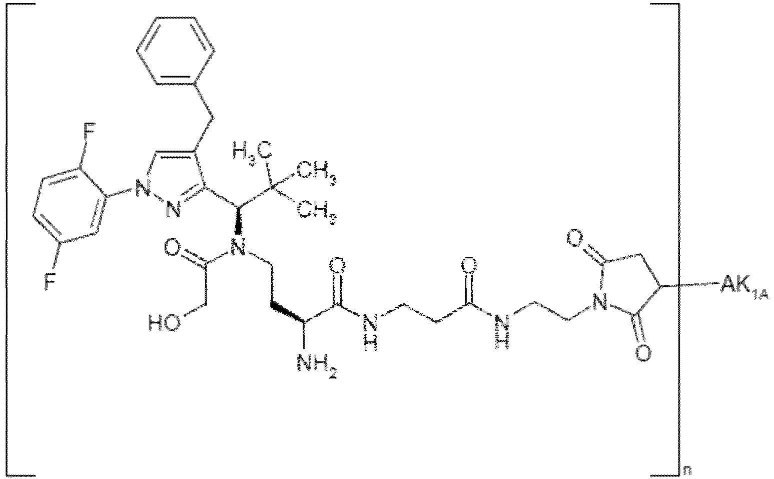 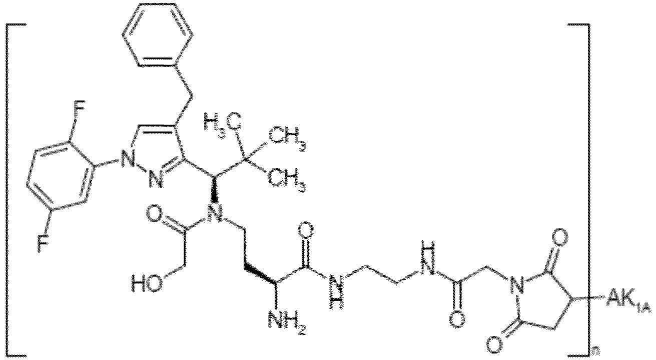 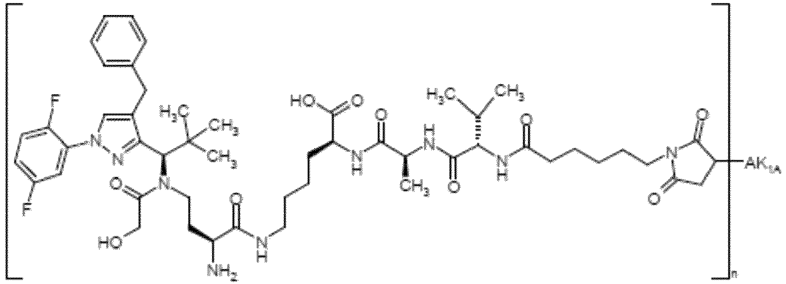 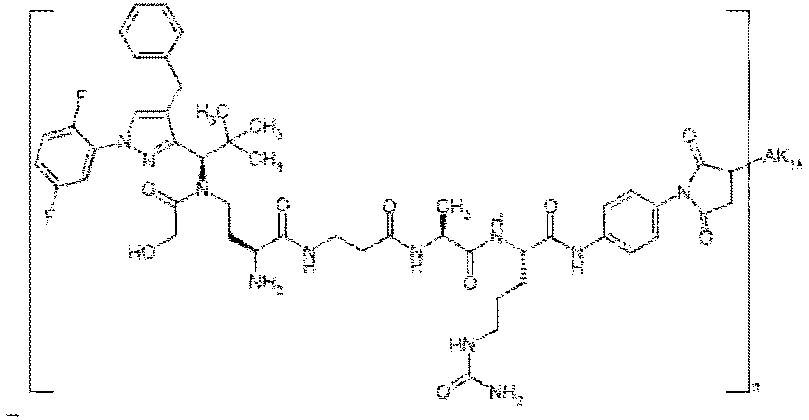 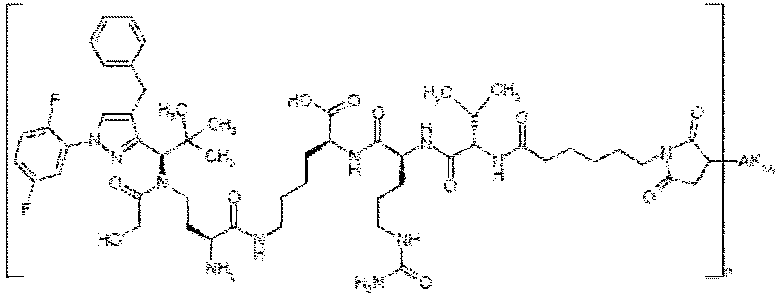 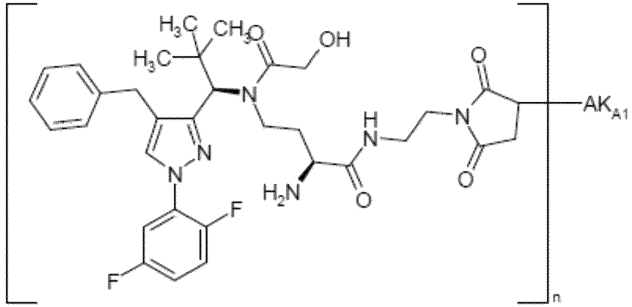 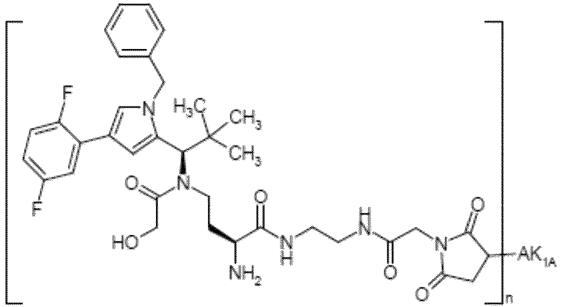 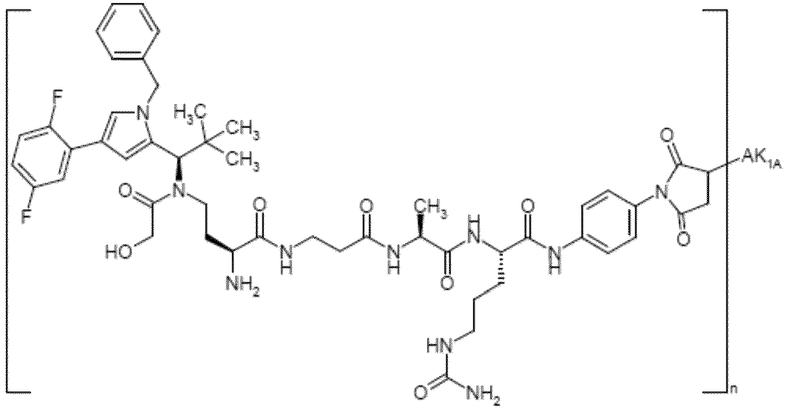 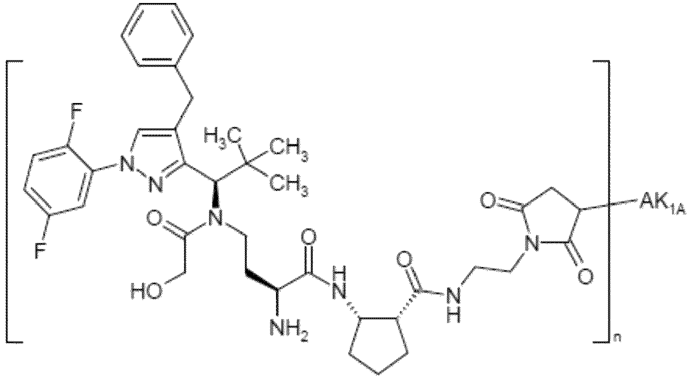 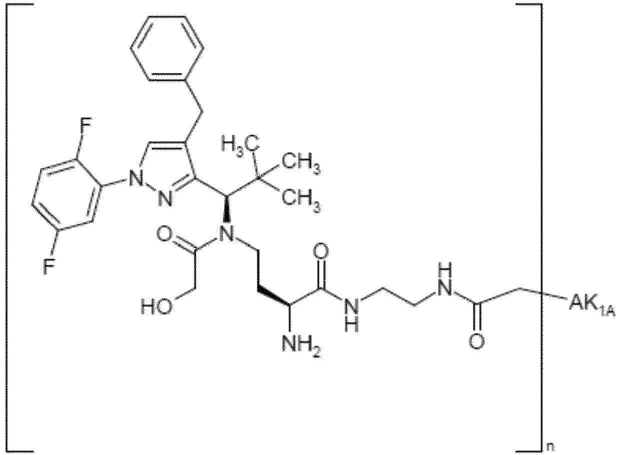 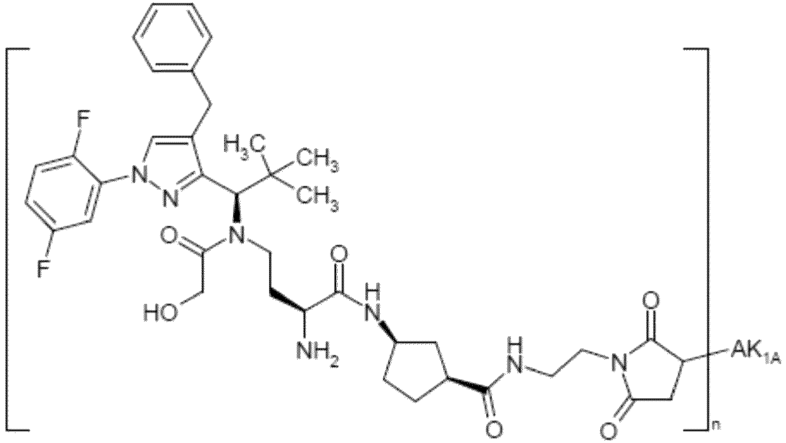 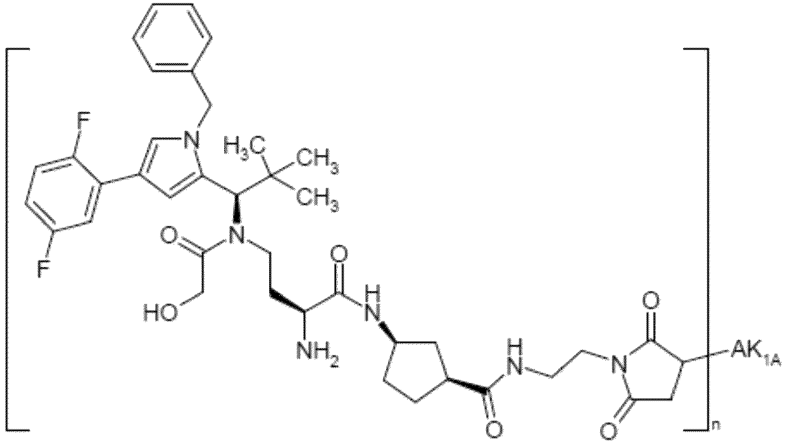 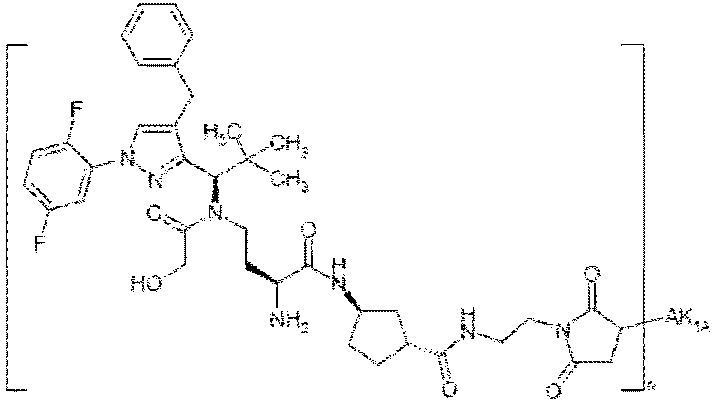 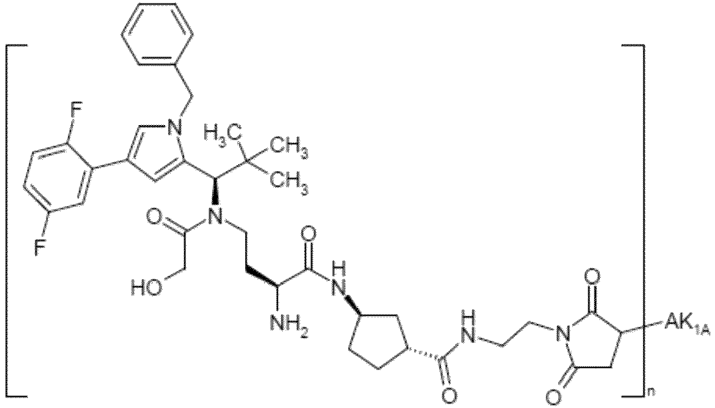 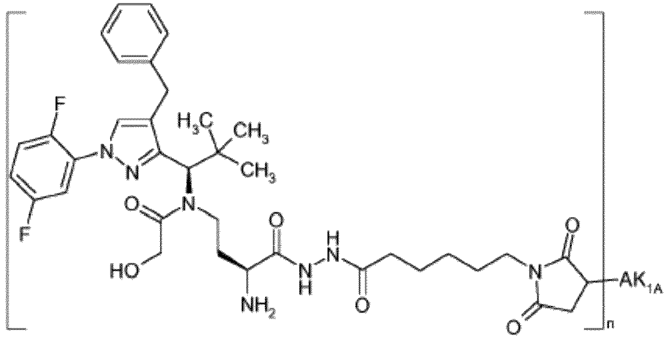 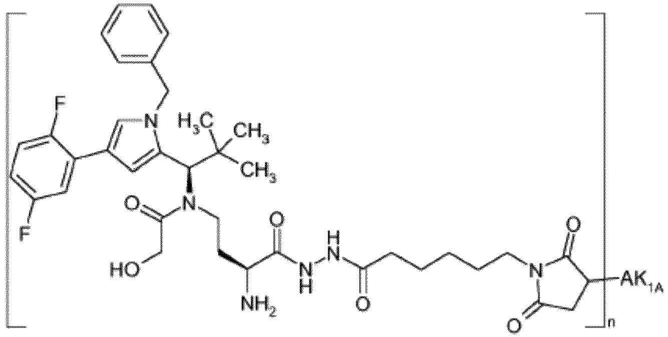 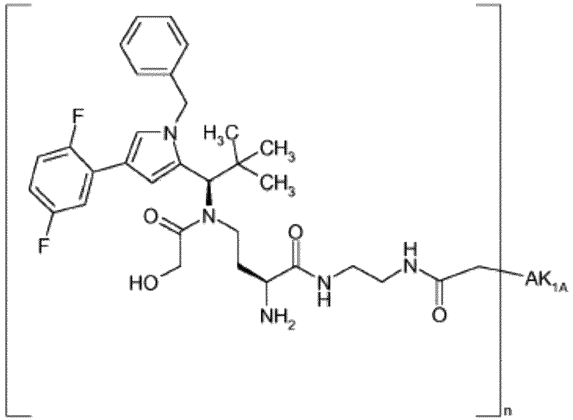 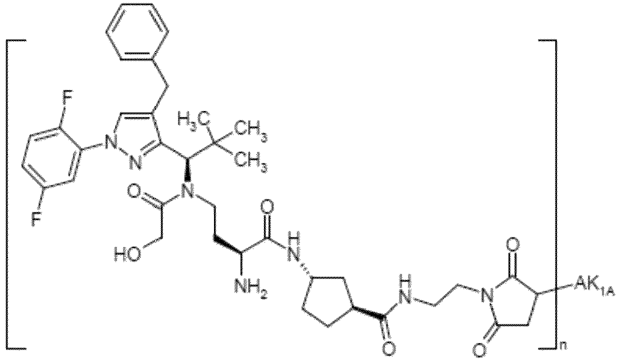 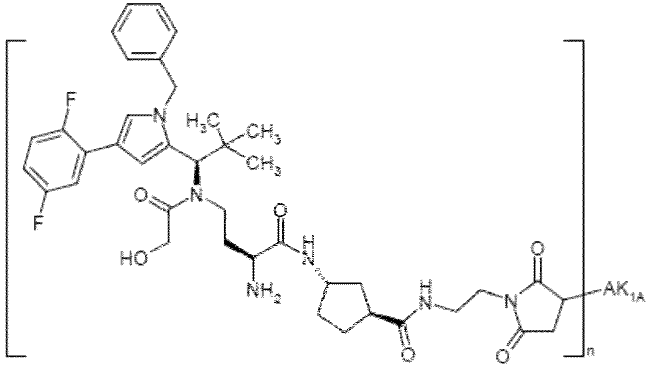 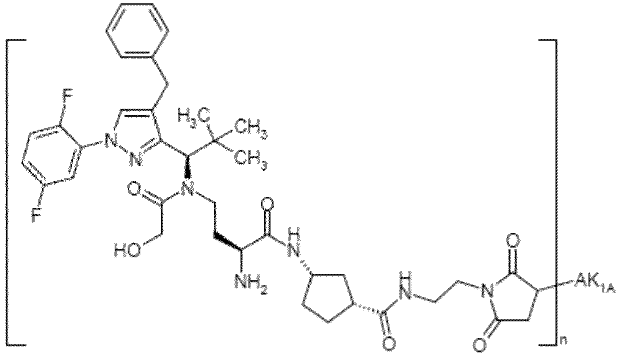 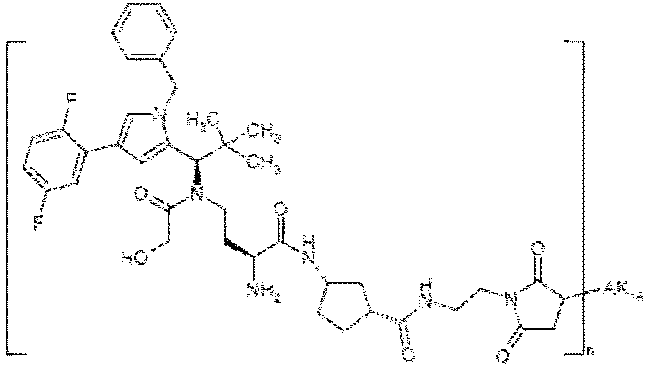 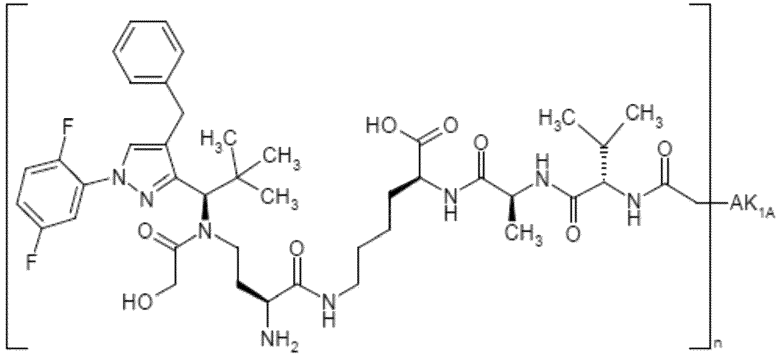 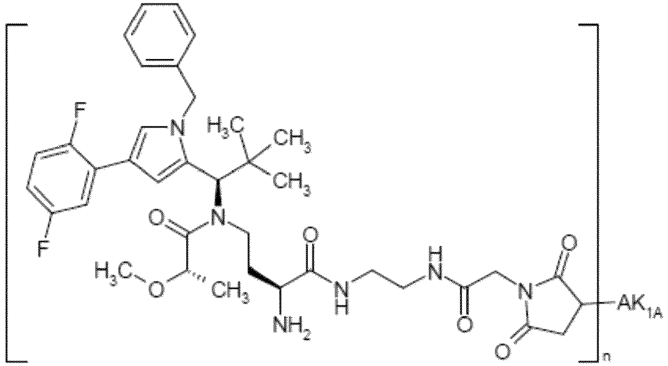 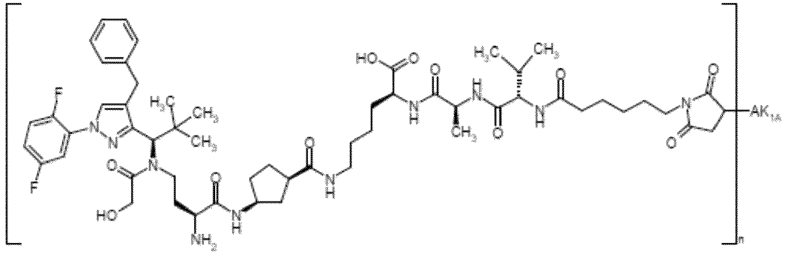 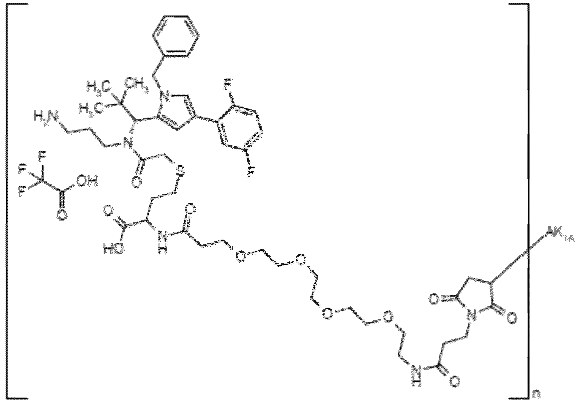 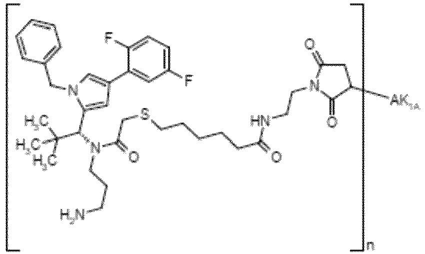 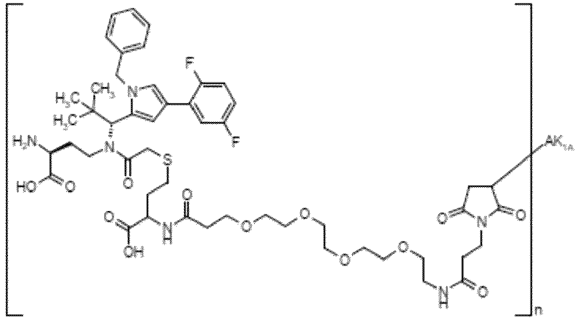 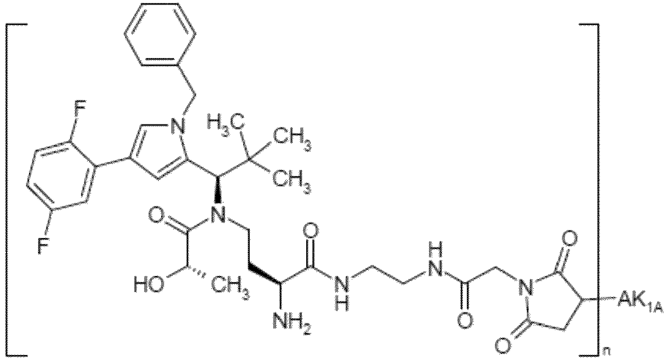 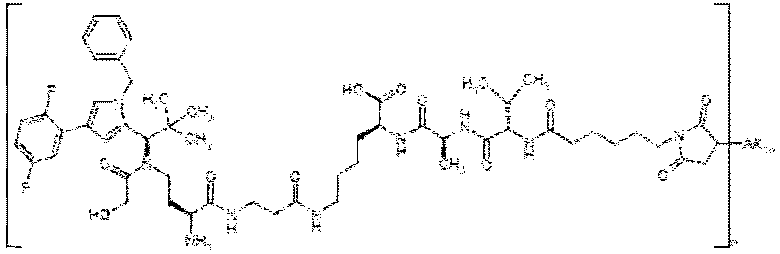 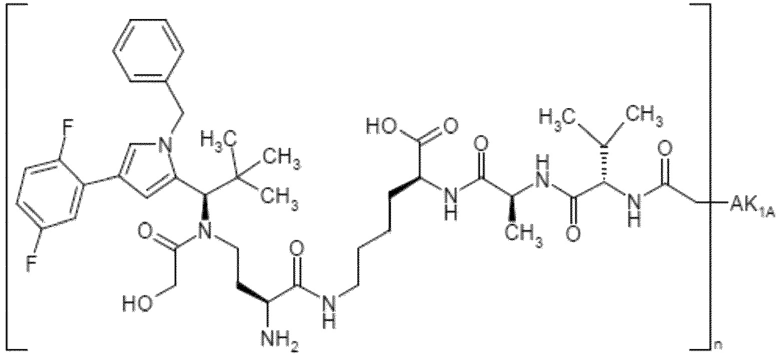 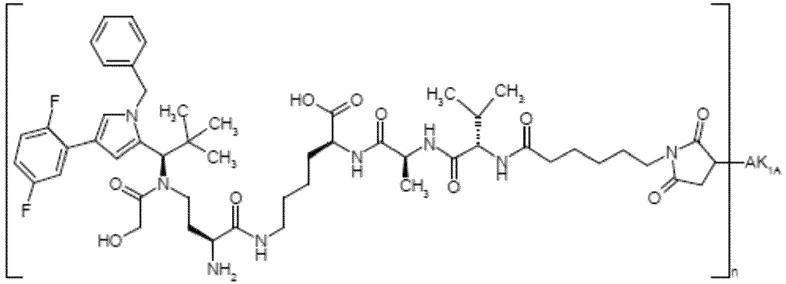 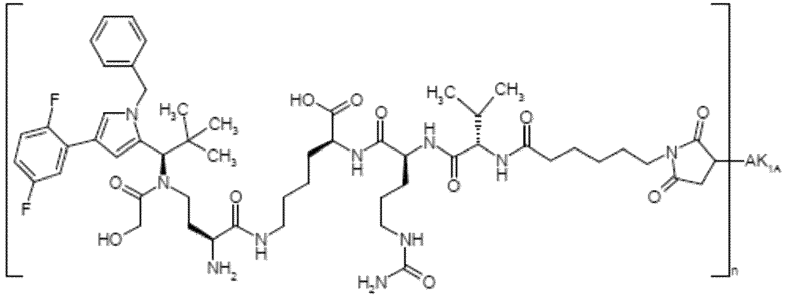 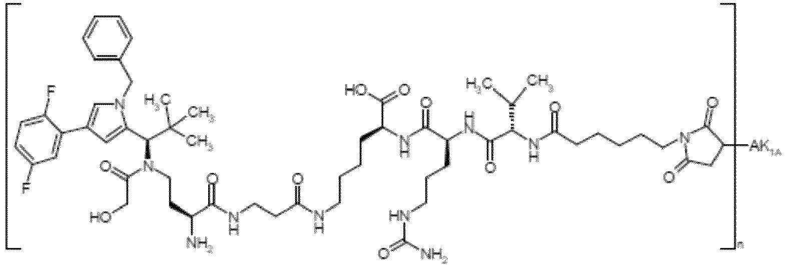 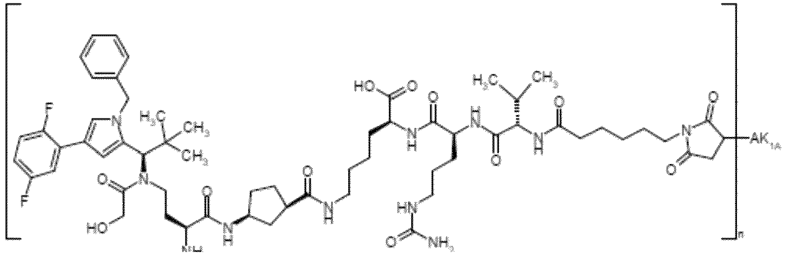 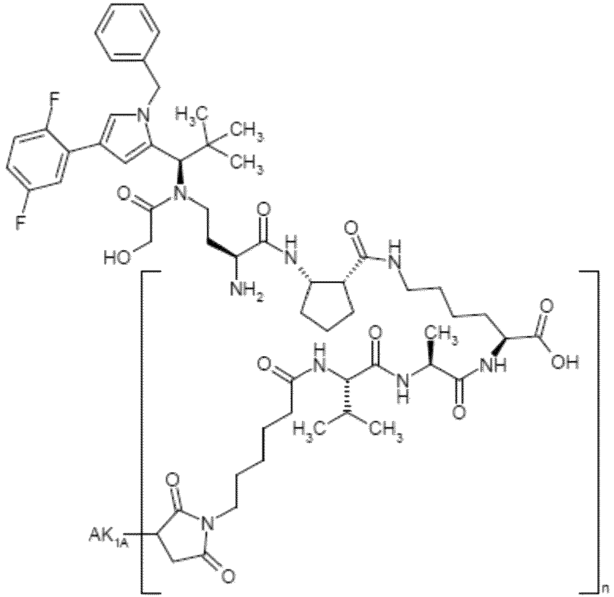 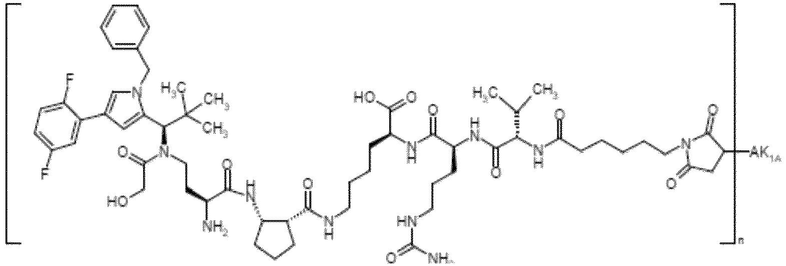 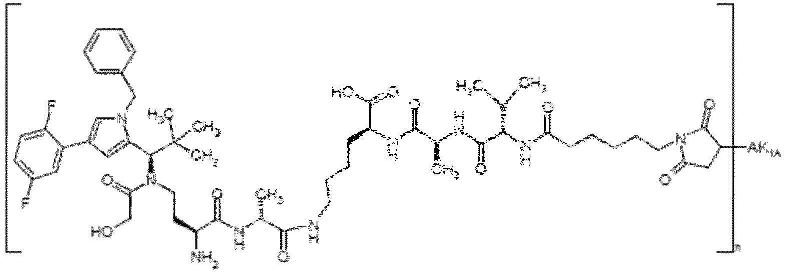 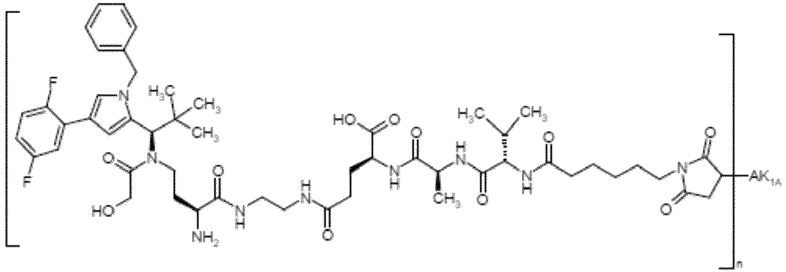 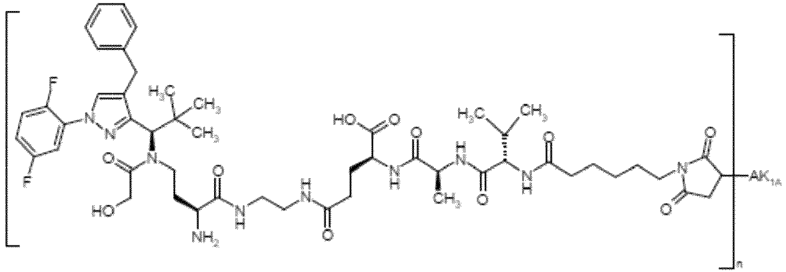 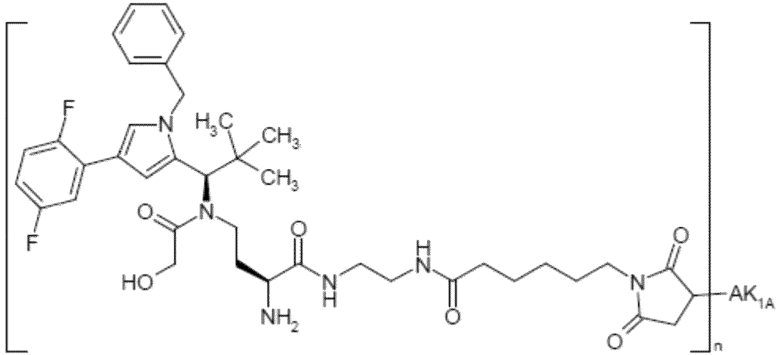 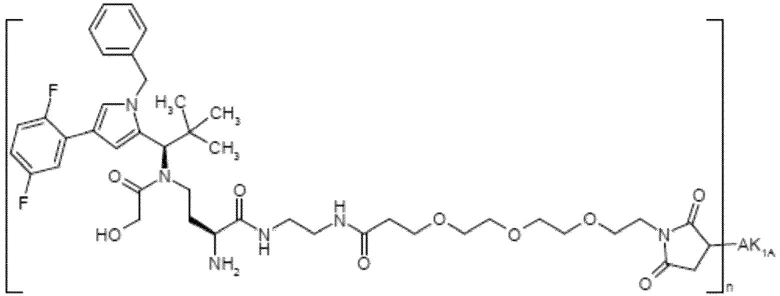 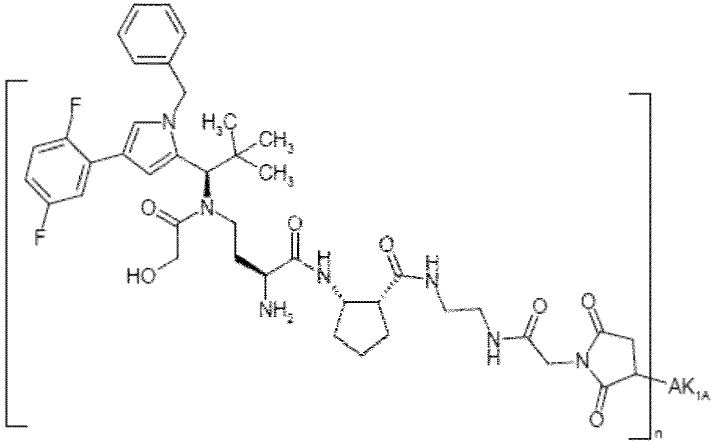 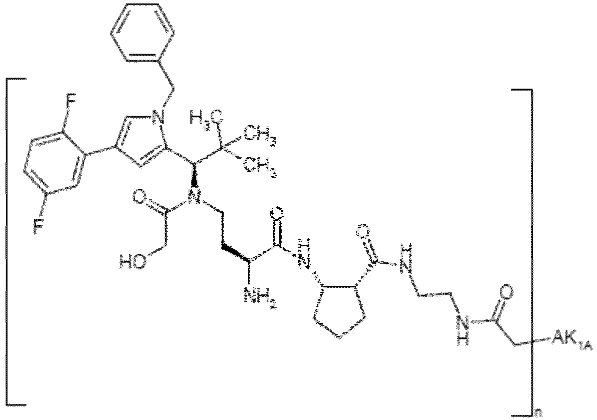 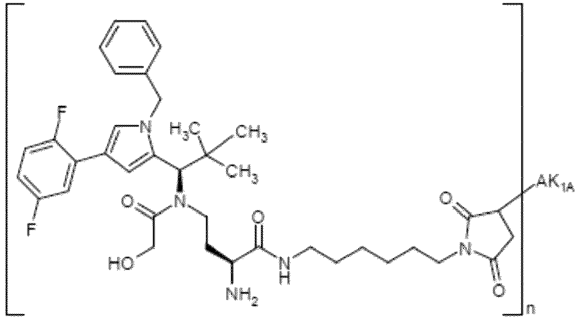 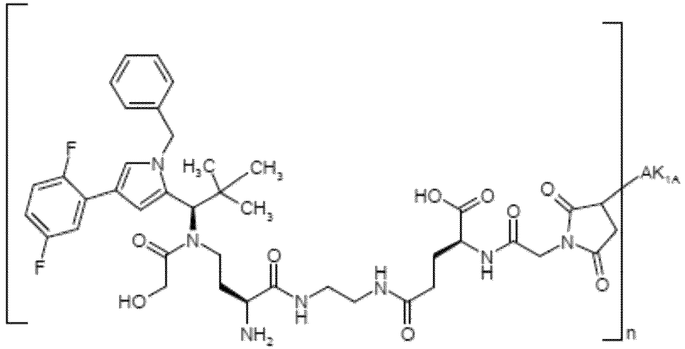 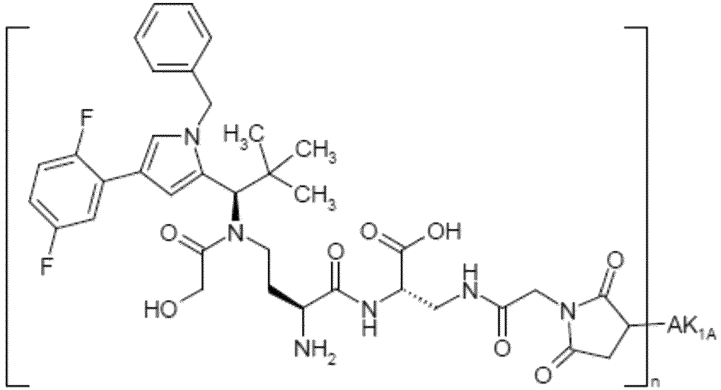 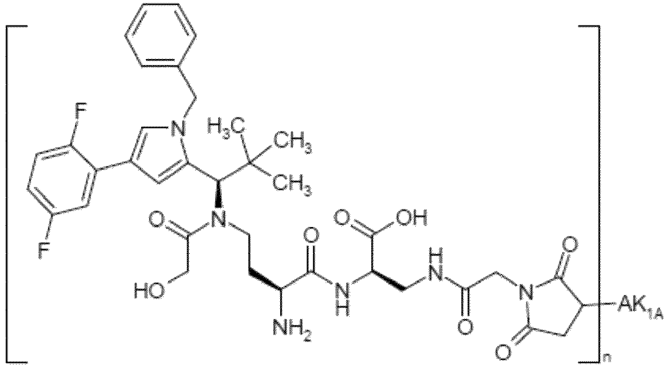 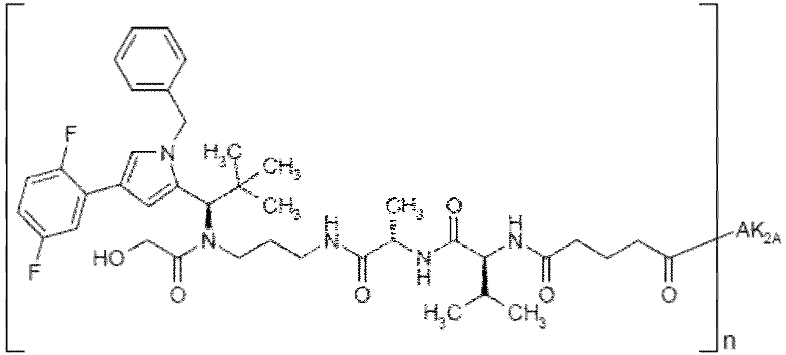 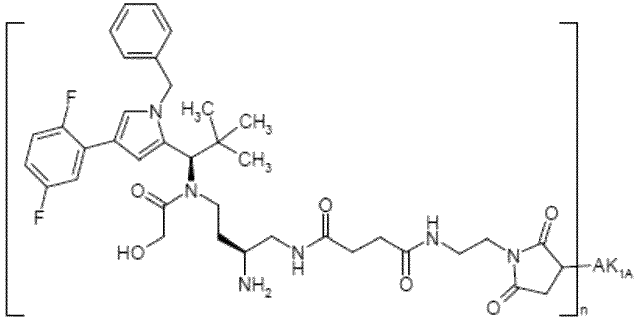 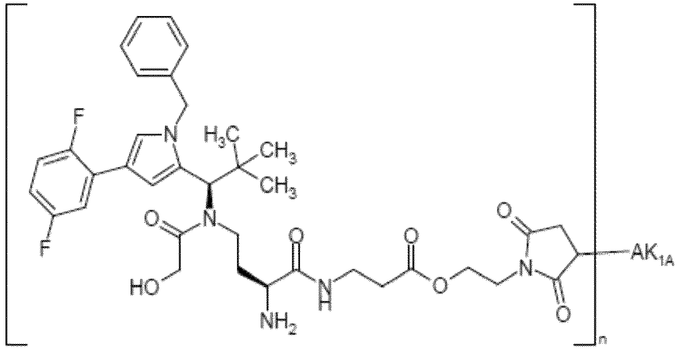 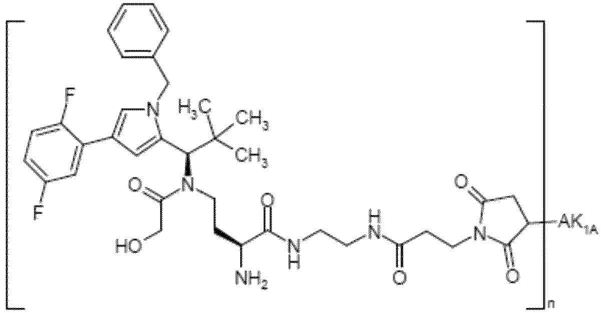 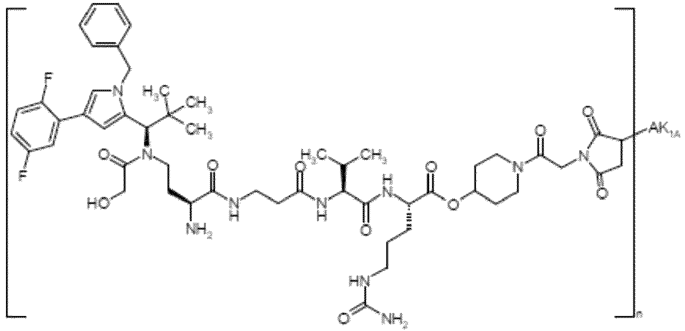 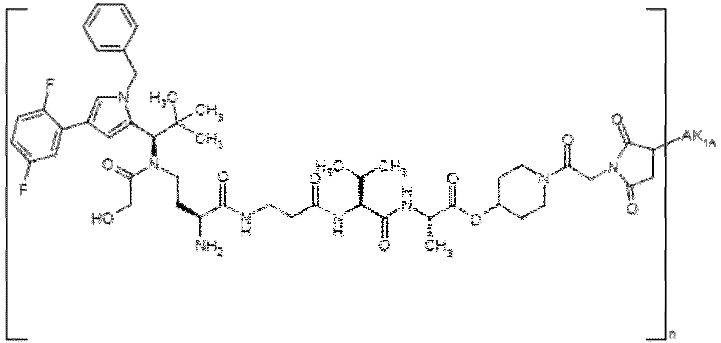 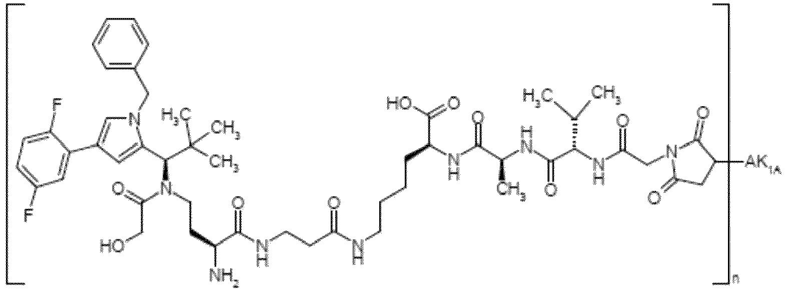 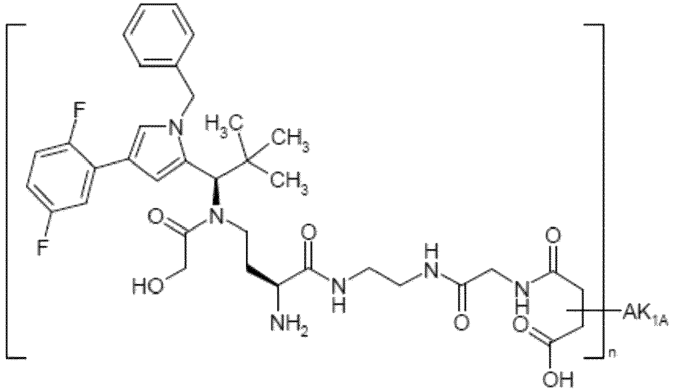 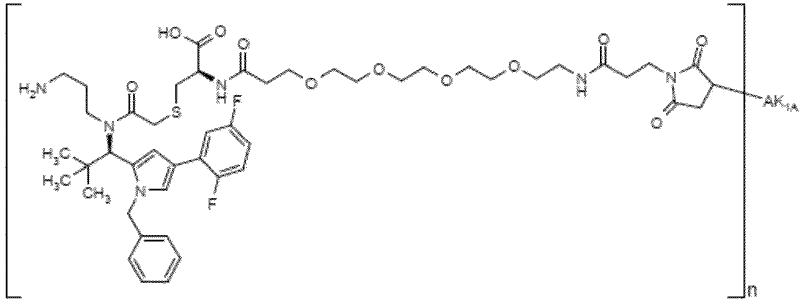 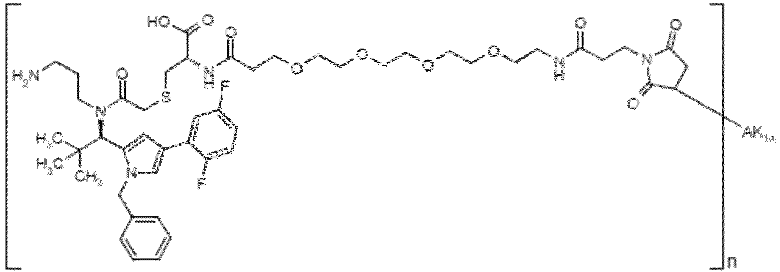 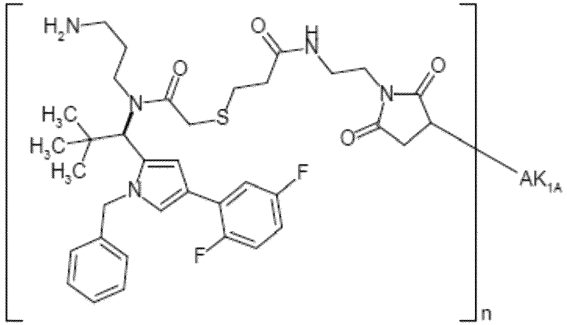 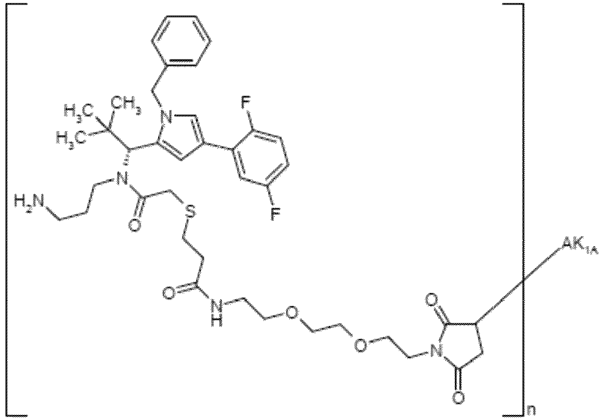 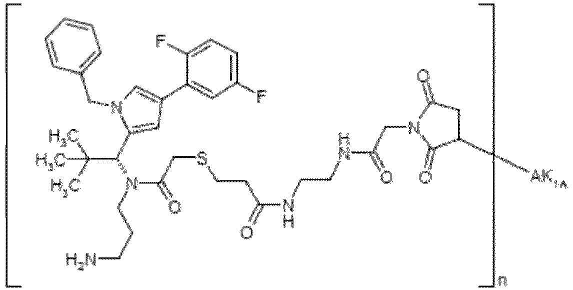 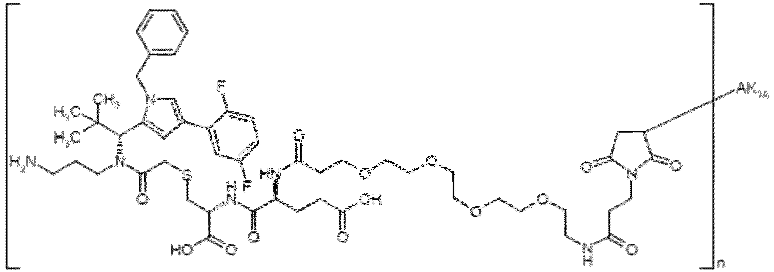 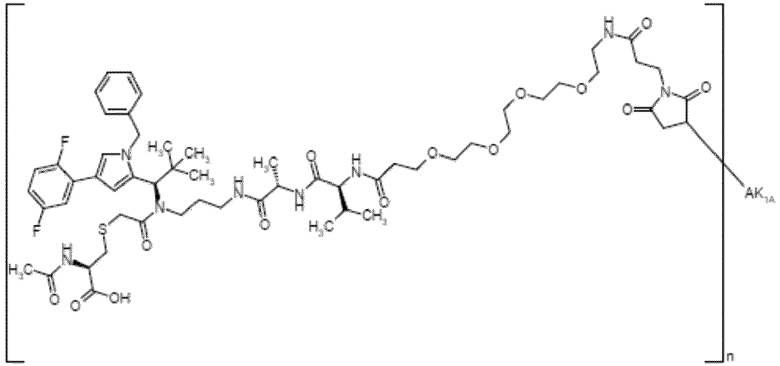 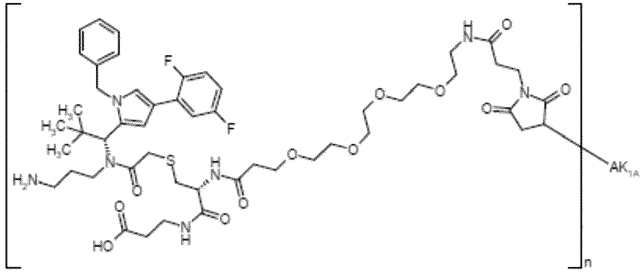 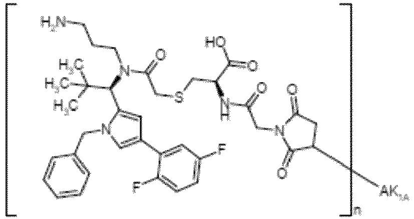 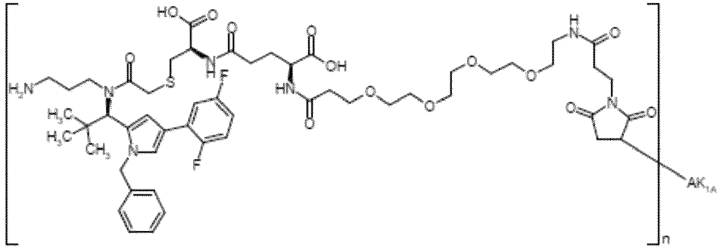 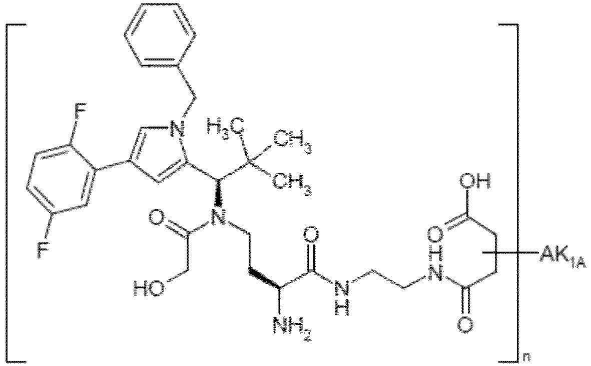 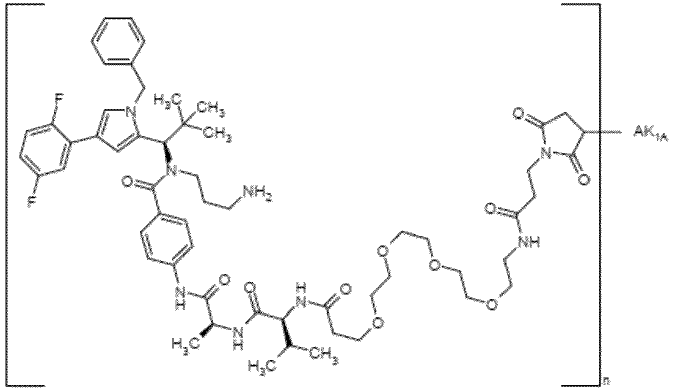 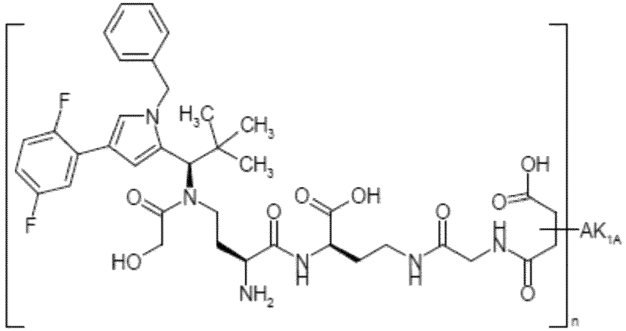 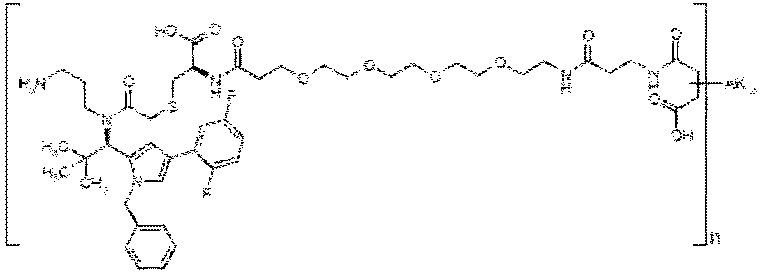 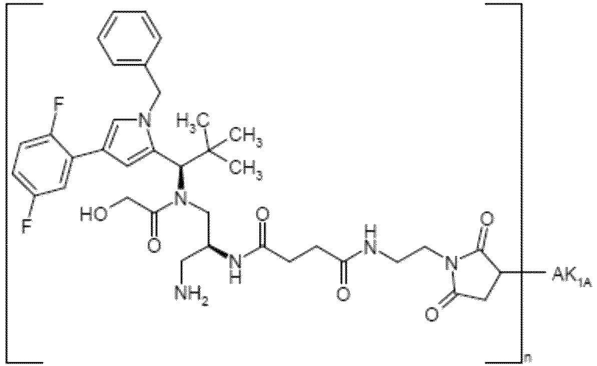 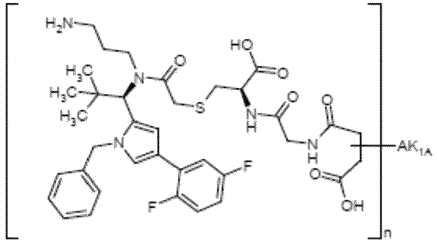 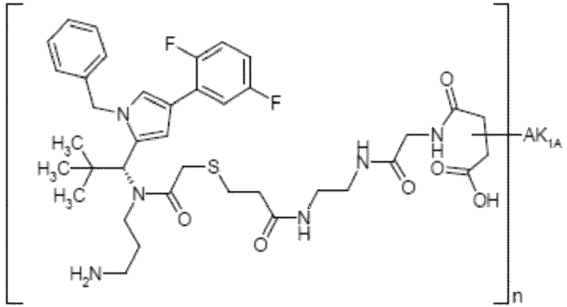 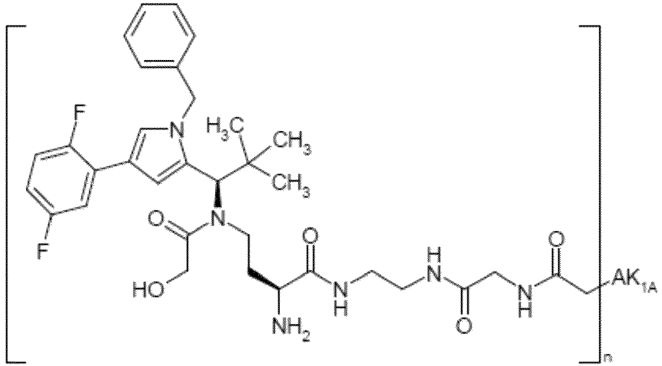 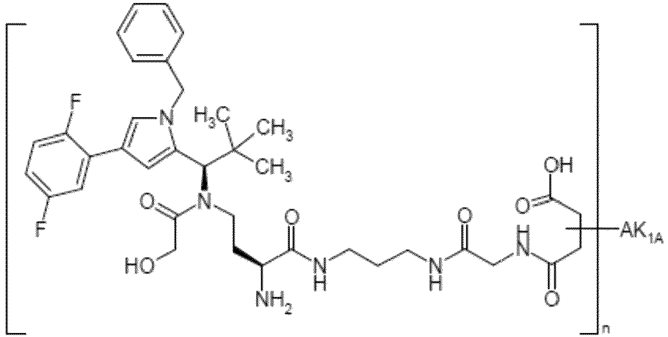 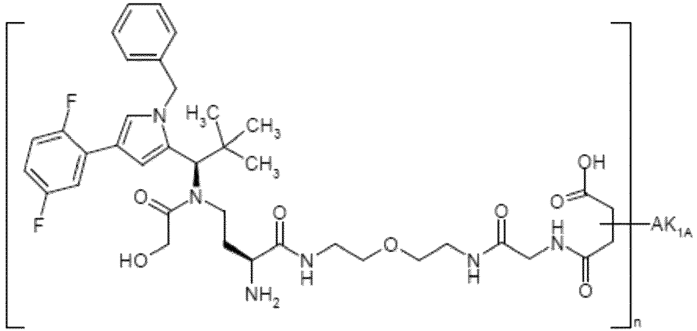 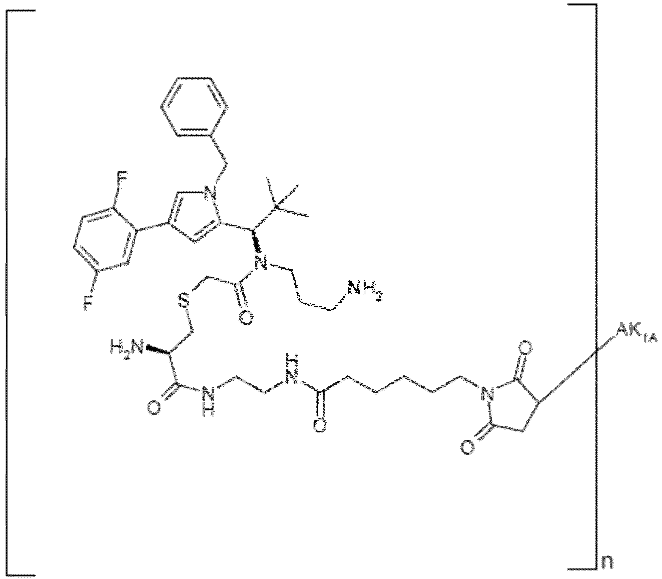 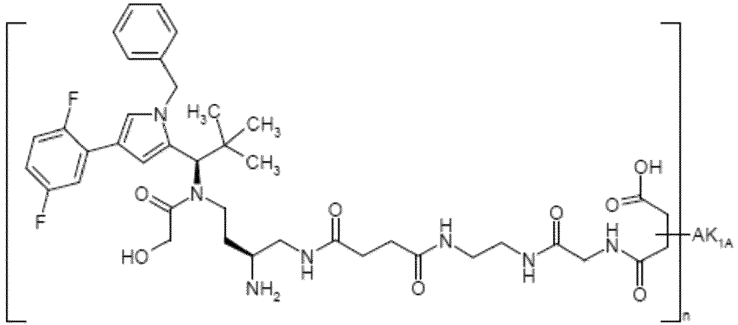 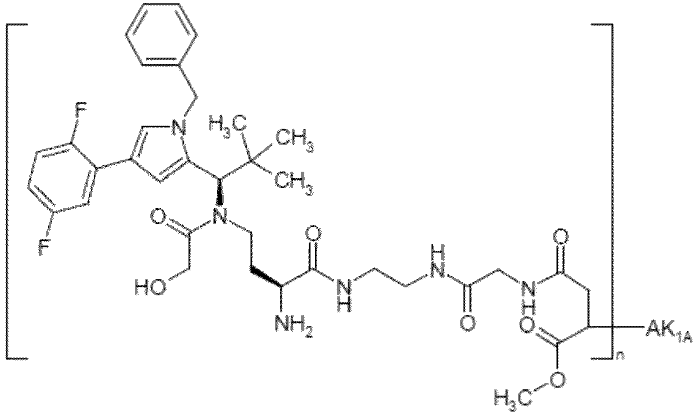 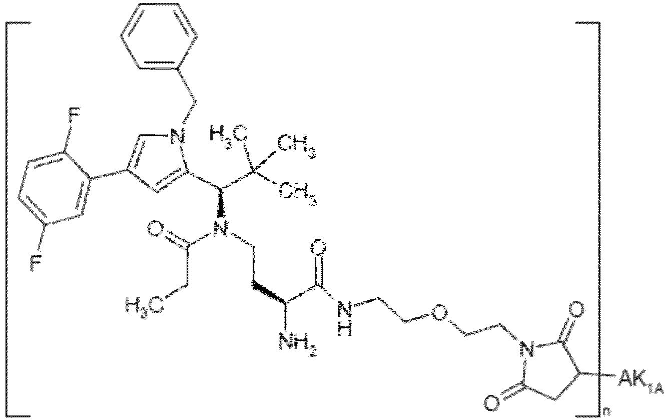 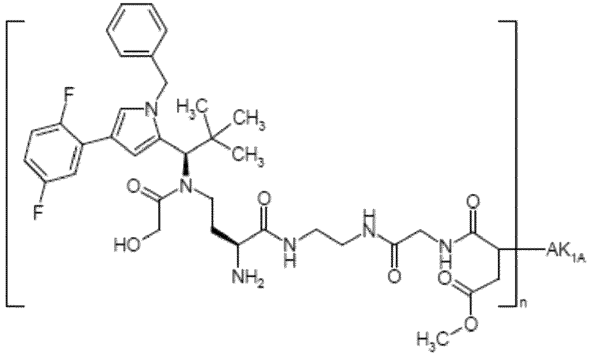 l'ADC (antibody-drug conjugate - conjugué anticorps-médicament), pour autant qu'il est relié à l'anticorps par l'intermédiaire d'un groupe 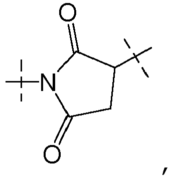 pouvant se trouver lié à l'anticorps aussi au moins partiellement sous forme de succinimides hydrolysés à chaîne ouverte, à la place de ce groupe.

66. Conjugué selon la revendication 65, l'anticorps étant un anticorps monoclonal humain, humanisé ou chimérique ou un fragment de liaison à l'antigène de celui-ci.

67. Conjugué selon la revendication 66, l'anticorps étant un anticorps anti-HER2, un anticorps anti-EGFR, un anticorps anti-TWEAKR, ou un fragment de liaison à un antigène de celui-ci.

68. Conjugué selon la revendication 67, l'anticorps anti-TWEAKR se liant de manière spécifique à l'amino acide D en position 47 (D47) de TWEAKR (SEQ ID NO : 169), préférablement l'anticorps anti-TWEAKR TPP-2090.


### SEQUENCE LISTING

<110> Bayer Pharma AG
<120> Binder-konjugate (ADCs) mit KSP-Inhibitoren
<130> 173 506
<160> 240
<170> BiSSAP 1.3
<210> 1
   <211> 215
   <212> PRT
   <213> Homo Sapiens
<400> 1
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Gly Tyr
               20                  25                  30
   Leu Asn Trp Tyr Gln Gln Lys Pro Gly Lys Ala Pro Lys Leu Leu Ile
           35                  40                  45
   Tyr Gln Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Thr Ser Pro Phe
                   85                  90                  95
   Ile Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 2
   <211> 449
   <212> PRT
   <213> Homo Sapiens
<400> 2
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Ile Trp Val Arg Gln Ala Pro Gly Lys Gly Leu Glu Trp Val
           35                  40                  45
   Ser Tyr Ile Ser Pro Ser Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
   85 90 95
   Ala Arg Gly Gly Asp Thr Tyr Phe Asp Tyr Phe Asp Tyr Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly
<210> 3
   <211> 11
   <212> PRT
   <213> Homo Sapiens
<400> 3
   Arg Ala Ser Gln Ser Ile Ser Gly Tyr Leu Asn
   1 5 10
<210> 4
   <211> 7
   <212> PRT
   <213> Homo Sapiens
<400> 4
   Gln Ala Ser Ser Leu Gln Ser
   1 5
<210> 5
   <211> 10
   <212> PRT
   <213> Homo Sapiens
<400> 5
   Gln Gln Ser Tyr Thr Ser Pro Phe Ile Thr
   1 5 10
<210> 6
   <211> 5
   <212> PRT
   <213> Homo Sapiens
<400> 6
   Pro Tyr Pro Met Ile
   1 5
<210> 7
   <211> 17
   <212> PRT
   <213> Homo Sapiens
<400> 7
   Tyr Ile Ser Pro Ser Gly Gly Ser Thr His Tyr Ala Asp Ser Val Lys
   1 5 10 15
   Gly
<210> 8
   <211> 11
   <212> PRT
   <213> Homo Sapiens
<400> 8
   Gly Gly Asp Thr Tyr Phe Asp Tyr Phe Asp Tyr
   1 5 10
<210> 9
   <211> 108
   <212> PRT
   <213> Homo Sapiens
<400> 9
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Gly Tyr
               20                  25                  30
   Leu Asn Trp Tyr Gln Gln Lys Pro Gly Lys Ala Pro Lys Leu Leu Ile
           35                  40                  45
   Tyr Gln Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Thr Ser Pro Phe
                   85                  90                  95
   Ile Thr Phe Gly Gln Gly Thr Lys Val Glu Ile Lys
               100                 105
<210> 10
   <211> 121
   <212> PRT
   <213> Homo Sapiens
<400> 10
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Ile Trp Val Arg Gln Ala Pro Gly Lys Gly Leu Glu Trp Val
           35                  40                  45
   Ser Tyr Ile Ser Pro Ser Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala
           115                 120
<210> 11
   <211> 215
   <212> PRT
   <213> Homo Sapiens
<400> 11
   Asp Ile Gln Met Thr Gln Ser Pro Ala Thr Leu Ser Ala Ser Val Gly
   1               5                   10                  15
   Asp Arg Val Thr Ile Thr Cys Arg Ala Ser Gln Ser Ile Ser Gly Tyr
               20                  25                  30
   Leu Asn Trp Tyr Gln Gln Lys Pro Gly Lys Ala Pro Lys Leu Leu Ile
           35                  40                  45
   Tyr Gln Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Thr Ser Pro Phe
                   85                  90                  95
   Ile Thr Phe Gly Pro Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Leu
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 12
   <211> 449
   <212> PRT
   <213> Homo Sapiens
<400> 12
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Pro Tyr
   20 25 30
   Pro Met Ile Trp Val Arg Gln Ala Pro Gly Lys Gly Leu Glu Trp Val
   35 40 45
   Ser Tyr Ile Ser Pro Ser Gly Gly Lys Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Gly Asp Thr Tyr Phe Asp Tyr Phe Asp Tyr Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly
<210> 13
   <211> 11
   <212> PRT
   <213> Homo Sapiens
<400> 13
   Arg Ala Ser Gln Ser Ile Ser Gly Tyr Leu Asn
   1               5                   10
<210> 14
   <211> 7
   <212> PRT
   <213> Homo Sapiens
<400> 14
   Gln Ala Ser Ser Leu Gln Ser
   1               5
<210> 15
   <211> 10
   <212> PRT
   <213> Homo Sapiens
<400> 15
   Gln Gln Ser Tyr Thr Ser Pro Phe Ile Thr
   1               5                   10
<210> 16
   <211> 5
   <212> PRT
   <213> Homo Sapiens
<400> 16
   Pro Tyr Pro Met Ile
   1               5
<210> 17
   <211> 17
   <212> PRT
   <213> Homo Sapiens
<400> 17
   Tyr Ile Ser Pro Ser Gly Gly Lys Thr His Tyr Ala Asp Ser Val Lys
   1               5                   10                  15
   Gly
<210> 18
   <211> 11
   <212> PRT
   <213> Homo Sapiens
<400> 18
   Gly Gly Asp Thr Tyr Phe Asp Tyr Phe Asp Tyr
   1               5                   10
<210> 19
   <211> 108
   <212> PRT
   <213> Homo Sapiens
<400> 19
   Asp Ile Gln Met Thr Gln Ser Pro Ala Thr Leu Ser Ala Ser Val Gly
   1               5                   10                  15
   Asp Arg Val Thr Ile Thr Cys Arg Ala Ser Gln Ser Ile Ser Gly Tyr
               20                  25                  30
   Leu Asn Trp Tyr Gln Gln Lys Pro Gly Lys Ala Pro Lys Leu Leu Ile
           35                  40                  45
   Tyr Gln Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Thr Ser Pro Phe
                   85                  90                  95
   Ile Thr Phe Gly Pro Gly Thr Lys Val Glu Ile Lys
               100                 105
<210> 20
   <211> 121
   <212> PRT
   <213> Homo Sapiens
<400> 20
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Ile Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala
           115                 120
<210> 21
   <211> 215
   <212> PRT
   <213> Homo Sapiens
<400> 21
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Gln Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Thr Ser Pro Phe
                   85                  90                  95
   Ile Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 22
   <211> 449
   <212> PRT
   <213> Homo Sapiens
<400> 22
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 23
   <211> 11
   <212> PRT
   <213> Homo Sapiens
<400> 23
   Arg Ala Ser Gln Ser Ile Ser Ser Tyr Leu Asn
   1 5 10
<210> 24
   <211> 7
   <212> PRT
   <213> Homo Sapiens
<400> 24
   Gln Ala Ser Ser Leu Gln Ser
   1 5
<210> 25
   <211> 10
   <212> PRT
   <213> Homo Sapiens
<400> 25
   Gln Gln Ser Tyr Thr Ser Pro Phe Ile Thr
   1 5 10
<210> 26
   <211> 5
   <212> PRT
   <213> Homo Sapiens
<400> 26
   Pro Tyr Pro Met Met
   1 5
<210> 27
   <211> 17
   <212> PRT
   <213> Homo Sapiens
<400> 27
   Tyr Ile Ser Pro Ser Gly Gly Ser Thr His Tyr Ala Asp Ser Val Lys
   1 5 10 15
   Gly
<210> 28
   <211> 11
   <212> PRT
   <213> Homo Sapiens
<400> 28
   Gly Gly Asp Thr Tyr Phe Asp Tyr Phe Asp Tyr
   1 5 10
<210> 29
   <211> 108
   <212> PRT
   <213> Homo Sapiens
<400> 29
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Gln Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Thr Ser Pro Phe
                   85                  90                  95
   Ile Thr Phe Gly Gln Gly Thr Lys Val Glu Ile Lys
               100                 105
<210> 30
   <211> 121
   <212> PRT
   <213> Homo Sapiens
<400> 30
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala
           115                 120
<210> 31
   <211> 215
   <212> PRT
   <213> Homo Sapiens
<400> 31
   Asp Ile Gln Met Thr Gln Ser Pro Ala Thr Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Gln Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Thr Ser Pro Phe
                   85                  90                  95
   Ile Thr Phe Gly Pro Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Leu
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 32
   <211> 449
   <212> PRT
   <213> Homo Sapiens
<400> 32
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 33
   <211> 11
   <212> PRT
   <213> Homo Sapiens
<400> 33
   Arg Ala Ser Gln Ser Ile Ser Ser Tyr Leu Asn
   1               5                   10
<210> 34
   <211> 7
   <212> PRT
   <213> Homo Sapiens
<400> 34
   Gln Ala Ser Ser Leu Gln Ser
   1               5
<210> 35
   <211> 10
   <212> PRT
   <213> Homo Sapiens
<400> 35
   Gln Gln Ser Tyr Thr Ser Pro Phe Ile Thr
   1               5                   10
<210> 36
   <211> 5
   <212> PRT
   <213> Homo Sapiens
<400> 36
   Pro Tyr Pro Met Met
   1               5
<210> 37
   <211> 17
   <212> PRT
   <213> Homo Sapiens
<400> 37
   Tyr Ile Ser Pro Ser Gly Gly Lys Thr His Tyr Ala Asp Ser Val Lys
   1               5                   10                  15
   Gly
<210> 38
   <211> 11
   <212> PRT
   <213> Homo Sapiens
<400> 38
   Gly Gly Asp Thr Tyr Phe Asp Tyr Phe Asp Tyr
   1               5                   10
<210> 39
   <211> 108
   <212> PRT
   <213> Homo Sapiens
<400> 39
   Asp Ile Gln Met Thr Gln Ser Pro Ala Thr Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Gln Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Thr Ser Pro Phe
                   85                  90                  95
   Ile Thr Phe Gly Pro Gly Thr Lys Val Glu Ile Lys
               100                 105
<210> 40
   <211> 121
   <212> PRT
   <213> Homo Sapiens
<400> 40
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala
           115                 120
<210> 41
   <211> 215
   <212> PRT
   <213> Homo Sapiens
<400> 41
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Thr Pro Gly
                   85                  90                  95
   Ile Thr Phe Gly Gln Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 42
   <211> 449
   <212> PRT
   <213> Homo Sapiens
<400> 42
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Ser Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 43
   <211> 11
   <212> PRT
   <213> Homo Sapiens
<400> 43
   Arg Ala Ser Gln Ser Ile Ser Ser Tyr Leu Asn
   1 5 10
<210> 44
   <211> 7
   <212> PRT
   <213> Homo Sapiens
<400> 44
   Ala Ala Ser Ser Leu Gln Ser
   1 5
<210> 45
   <211> 10
   <212> PRT
   <213> Homo Sapiens
<400> 45
   Gln Gln Ser Tyr Ser Thr Pro Gly Ile Thr
   1 5 10
<210> 46
   <211> 5
   <212> PRT
   <213> Homo Sapiens
<400> 46
   Pro Tyr Pro Met Met
   1 5
<210> 47
   <211> 17
   <212> PRT
   <213> Homo Sapiens
<400> 47
   Tyr Ile Ser Pro Ser Gly Gly Ser Thr His Tyr Ala Asp Ser Val Lys
   1 5 10 15
   Gly
<210> 48
   <211> 11
   <212> PRT
   <213> Homo Sapiens
<400> 4
<210> 49
   <211> 108
   <212> PRT
   <213> Homo Sapiens
<400> 49
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ser Ile Ser Ser Tyr
   20 25 30
   Leu Asn Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ser Tyr Ser Thr Pro Gly
   85 90 95
   Ile Thr Phe Gly Gln Gly Thr Lys Val Glu Ile Lys
   100 105
<210> 50
   <211> 121
   <212> PRT
   <213> Homo Sapiens
<400> 50
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Pro Tyr
   20 25 30
   Pro Met Met Trp Val Arg Gln Ala Pro Gly Lys Gly Leu Glu Trp Val
   35 40 45
   Ser Tyr Ile Ser Pro Ser Gly Gly Ser Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Gly Asp Thr Tyr Phe Asp Tyr Phe Asp Tyr Trp Gly Gln
   100 105 110
   Gly Thr Leu Val Thr Val Ser Ser Ala
   115 120
<210> 51
   <211> 215
   <212> PRT
   <213> Homo Sapiens
<400> 51
   Asp Ile Gln Met Thr Gln Ser Pro Ala Thr Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Ser Pro Gly
                   85                  90                  95
   Ile Thr Phe Gly Pro Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Leu
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 52
   <211> 449
   <212> PRT
   <213> Homo Sapiens
<400> 52
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 53
   <211> 11
   <212> PRT
   <213> Homo Sapiens
<400> 53
   Arg Ala Ser Gln Ser Ile Ser Ser Tyr Leu Asn
   1               5                   10
<210> 54
   <211> 7
   <212> PRT
   <213> Homo Sapiens
<400> 54
   Ala Ala Ser Ser Leu Gln Ser
   1               5
<210> 55
   <211> 10
   <212> PRT
   <213> Homo Sapiens
<400> 55
   Gln Gln Ser Tyr Ser Ser Pro Gly Ile Thr
   1 5 10
<210> 56
   <211> 5
   <212> PRT
   <213> Homo Sapiens
<400> 56
   Pro Tyr Pro Met Met
   1 5
<210> 57
   <211> 17
   <212> PRT
   <213> Homo Sapiens
<400> 57
   Tyr Ile Ser Pro Ser Gly Gly Lys Thr His Tyr Ala Asp Ser Val Lys
   1 5 10 15
   Gly
<210> 58
   <211> 11
   <212> PRT
   <213> Homo Sapiens
<400> 58
   Gly Gly Asp Thr Tyr Phe Asp Tyr Phe Asp Tyr
   1 5 10
<210> 59
   <211> 108
   <212> PRT
   <213> Homo Sapiens
<400> 59
   Asp Ile Gln Met Thr Gln Ser Pro Ala Thr Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Ser Ser Pro Gly
                   85                  90                  95
   Ile Thr Phe Gly Pro Gly Thr Lys Val Glu Ile Lys
               100                 105
<210> 60
   <211> 121
   <212> PRT
   <213> Homo Sapiens
<400> 60
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala
           115                 120
<210> 61
   <211> 217
   <212> PRT
   <213> Homo Sapiens
<400> 61
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Ser Tyr Leu Asn Trp Tyr Gln Gln Lys Pro Gly Lys Ala Pro Lys Leu
           35                  40                  45
   Leu Ile Tyr Ala Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Ser Ser
                   85                  90                  95
   Pro Gly Ile Thr Phe Gly Pro Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Leu Tyr Ala Cys Glu Val Thr His Gln Gly Leu Ser Ser Pro Val
           195                 200                 205
   Thr Lys Ser Phe Asn Arg Gly Glu Cys
       210                 215
<210> 62
   <211> 449
   <212> PRT
   <213> Homo Sapiens
<400> 62
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 63
   <211> 11
   <212> PRT
   <213> Homo Sapiens
<400> 63
   Arg Ala Ser Gln Ser Ile Ser Ser Tyr Leu Asn
   1               5                   10
<210> 64
   <211> 7
   <212> PRT
   <213> Homo Sapiens
<400> 64
   Ala Ala Ser Ser Leu Gln Ser
   1               5
<210> 65
   <211> 10
   <212> PRT
   <213> Homo Sapiens
<400> 65
   Gln Gln Ser Tyr Ser Ser Pro Gly Ile Thr
   1               5                   10
<210> 66
   <211> 5
   <212> PRT
   <213> Homo Sapiens
<400> 66
   Pro Tyr Pro Met Met
   1               5
<210> 67
   <211> 17
   <212> PRT
   <213> Homo Sapiens
<400> 67
   Tyr Ile Ser Pro Ser Gly Gly Lys Thr His Tyr Ala Asp Ser Val Lys
   1               5                   10                  15
   Gly
<210> 68
   <211> 11
   <212> PRT
   <213> Homo Sapiens
<400> 68
   Gly Gly Asp Thr Tyr Phe Asp Tyr Phe Asp Tyr
   1               5                   10
<210> 69
   <211> 110
   <212> PRT
   <213> Homo Sapiens
<400> 69
   Ala Gln Asp Ile Gln Met Thr Gln Ser Pro Ala Thr Leu Ser Ala Ser
   1 5 10 15
   Val Gly Asp Arg Val Thr Ile Thr Cys Arg Ala Ser Gln Ser Ile Ser
   20 25 30
   Ser Tyr Leu Asn Trp Tyr Gln Gln Lys Pro Gly Lys Ala Pro Lys Leu
   35 40 45
   Leu Ile Tyr Ala Ala Ser Ser Leu Gln Ser Gly Val Pro Ser Arg Phe
   50 55 60
   Ser Gly Ser Gly Ser Gly Thr Asp Phe Thr Leu Thr Ile Ser Ser Leu
   65 70 75 80
   Gln Pro Glu Asp Phe Ala Thr Tyr Tyr Cys Gln Gln Ser Tyr Ser Ser
   85 90 95
   Pro Gly Ile Thr Phe Gly Pro Gly Thr Lys Val Glu Ile Lys
   100 105 110
<210> 70
   <211> 121
   <212> PRT
   <213> Homo Sapiens
<400> 70
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Pro Tyr
   20 25 30
   Pro Met Met Trp Val Arg Gln Ala Pro Gly Lys Gly Leu Glu Trp Val
   35 40 45
   Ser Tyr Ile Ser Pro Ser Gly Gly Lys Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Gly Asp Thr Tyr Phe Asp Tyr Phe Asp Tyr Trp Gly Gln
   100 105 110
   Gly Thr Leu Val Thr Val Ser Ser Ala
   115 120
<210> 71
   <211> 217
   <212> PRT
   <213> Homo Sapiens
<400> 71
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Ser Tyr Leu Asn Trp Tyr Gln Gln Lys Pro Gly Lys Ala Pro Lys Leu
           35                  40                  45
   Leu Ile Tyr Ala Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Ser Ser
                   85                  90                  95
   Pro Gly Ile Thr Phe Gly Pro Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Leu Tyr Ala Cys Glu Val Thr His Gln Gly Leu Ser Ser Pro Val
           195                 200                 205
   Thr Lys Ser Phe Asn Arg Gly Glu Cys
       210                 215
<210> 72
   <211> 449
   <212> PRT
   <213> Homo Sapiens
<400> 72
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Gly Tyr Phe Asp Tyr Phe Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 73
   <211> 11
   <212> PRT
   <213> Homo Sapiens
<400> 73
   Arg Ala Ser Gln Ser Ile Ser Ser Tyr Leu Asn
   1 5 10
<210> 74
   <211> 7
   <212> PRT
   <213> Homo Sapiens
<400> 74
   Ala Ala Ser Ser Leu Gln Ser
   1 5
<210> 75
   <211> 10
   <212> PRT
   <213> Homo Sapiens
<400> 75
   Gln Gln Ser Tyr Ser Ser Pro Gly Ile Thr
   1 5 10
<210> 76
   <211> 5
   <212> PRT
   <213> Homo Sapiens
<400> 76
   Pro Tyr Pro Met Met
   1 5
<210> 77
   <211> 17
   <212> PRT
   <213> Homo Sapiens
<400> 77
   Tyr Ile Ser Pro Ser Gly Gly Lys Thr His Tyr Ala Asp Ser Val Lys
   1 5 10 15
   Gly
<210> 78
   <211> 11
   <212> PRT
   <213> Homo Sapiens
<400> 78
   Gly Gly Asp Gly Tyr Phe Asp Tyr Phe Asp Tyr
   1 5 10
<210> 79
   <211> 110
   <212> PRT
   <213> Homo Sapiens
<400> 79
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Ser Tyr Leu Asn Trp Tyr Gln Gln Lys Pro Gly Lys Ala Pro Lys Leu
           35                  40                  45
   Leu Ile Tyr Ala Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Ser Ser
                   85                  90                  95
   Pro Gly Ile Thr Phe Gly Pro Gly Thr Lys Val Glu Ile Lys
               100                 105                 110
<210> 80
   <211> 121
   <212> PRT
   <213> Homo Sapiens
<400> 80
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Gly Tyr Phe Asp Tyr Phe Asp Tyr Trp Gly Gln
               100                 105                 110
   Gly Thr Leu Val Thr Val Ser Ser Ala
           115                 120
<210> 81
   <211> 217
   <212> PRT
   <213> Homo Sapiens
<400> 81
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Gly Tyr Leu Asn Trp Tyr Gln Gln Lys Pro Gly Lys Ala Pro Lys Leu
           35                  40                  45
   Leu Ile Tyr Asn Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Thr Ser
                   85                  90                  95
   Pro Phe Ile Thr Phe Gly Pro Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Leu Tyr Ala Cys Glu Val Thr His Gln Gly Leu Ser Ser Pro Val
           195                 200                 205
   Thr Lys Ser Phe Asn Arg Gly Glu Cys
       210                 215
<210> 82
   <211> 449
   <212> PRT
   <213> Homo Sapiens
<400> 82
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Ile Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 83
   <211> 11
   <212> PRT
   <213> Homo Sapiens
<400> 83
   Arg Ala Ser Gln Ser Ile Ser Gly Tyr Leu Asn
   1               5                   10
<210> 84
   <211> 7
   <212> PRT
   <213> Homo Sapiens
<400> 84
   Asn Ala Ser Ser Leu Gln Ser
   1               5
<210> 85
   <211> 10
   <212> PRT
   <213> Homo Sapiens
<400> 85
   Gln Gln Ser Tyr Thr Ser Pro Phe Ile Thr
   1               5                   10
<210> 86
   <211> 5
   <212> PRT
   <213> Homo Sapiens
<400> 86
   Pro Tyr Pro Met Ile
   1               5
<210> 87
   <211> 17
   <212> PRT
   <213> Homo Sapiens
<400> 87
   Tyr Ile Ser Pro Ser Gly Gly Lys Thr His Tyr Ala Asp Ser Val Lys
   1               5                   10                  15
   Gly
<210> 88
   <211> 11
   <212> PRT
   <213> Homo Sapiens
<400> 88
   Gly Gly Asp Thr Tyr Phe Asp Tyr Phe Asp Tyr
   1               5                   10
<210> 89
   <211> 110
   <212> PRT
   <213> Homo Sapiens
<400> 89
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Gly Tyr Leu Asn Trp Tyr Gln Gln Lys Pro Gly Lys Ala Pro Lys Leu
           35                  40                  45
   Leu Ile Tyr Asn Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Thr Ser
                   85                  90                  95
   Pro Phe Ile Thr Phe Gly Pro Gly Thr Lys Val Glu Ile Lys
               100                 105                 110
<210> 90
   <211> 121
   <212> PRT
   <213> Homo Sapiens
<400> 90
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Ile Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala
           115                 120
<210> 91
   <211> 217
   <212> **PRT**
   <213> Homo Sapiens
<400> 91
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Ser Tyr Leu Asn Trp Tyr Gln Gln Lys Pro Gly Lys Ala Pro Lys Leu
           35                  40                  45
   Leu Ile Tyr Asn Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Thr Ser
                   85                  90                  95
   Pro Gly Ile Thr Phe Gly Pro Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Leu Tyr Ala Cys Glu Val Thr His Gln Gly Leu Ser Ser Pro Val
           195                 200                 205
   Thr Lys Ser Phe Asn Arg Gly Glu Cys
       210                 215
<210> 92
   <211> 449
   <212> PRT
   <213> Homo Sapiens
<400> 92
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Pro Tyr
   20 25 30
   Pro Met Met Trp Val Arg Gln Ala Pro Gly Lys Gly Leu Glu Trp Val
   35 40 45
   Ser Tyr Ile Ser Pro Ser Gly Gly Lys Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Gly Asp Thr Tyr Phe Asp Tyr Phe Asp Tyr Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly
<210> 93
   <211> 11
   <212> PRT
   <213> Homo Sapiens
<400> 93
   Arg Ala Ser Gln Ser Ile Ser Ser Tyr Leu Asn
   1               5                   10
<210> 94
   <211> 7
   <212> PRT
   <213> Homo Sapiens
<400> 94
   Asn Ala Ser Ser Leu Gln Ser
   1               5
<210> 95
   <211> 10
   <212> PRT
   <213> Homo Sapiens
<400> 95
   Gln Gln Ser Tyr Thr Ser Pro Gly Ile Thr
   1               5                   10
<210> 96
   <211> 5
   <212> PRT
   <213> Homo Sapiens
<400> 96
   Pro Tyr Pro Met Met
   1               5
<210> 97
   <211> 17
   <212> PRT
   <213> Homo Sapiens
<400> 97
   Tyr Ile Ser Pro Ser Gly Gly Lys Thr His Tyr Ala Asp Ser Val Lys
   1               5                   10                  15
   Gly
<210> 98
   <211> 11
   <212> PRT
   <213> Homo Sapiens
<400> 98
   Gly Gly Asp Thr Tyr Phe Asp Tyr Phe Asp Tyr
   1 5 10
<210> 99
   <211> 110
   <212> PRT
   <213> Homo Sapiens
<400> 99
   Ala Gln Asp Ile Gln Met Thr Gln Ser Pro Ala Thr Leu Ser Ala Ser
   1 5 10 15
   Val Gly Asp Arg Val Thr Ile Thr Cys Arg Ala Ser Gln Ser Ile Ser
   20 25 30
   Ser Tyr Leu Asn Trp Tyr Gln Gln Lys Pro Gly Lys Ala Pro Lys Leu
   35 40 45
   Leu Ile Tyr Asn Ala Ser Ser Leu Gln Ser Gly Val Pro Ser Arg Phe
   50 55 60
   Ser Gly Ser Gly Ser Gly Thr Asp Phe Thr Leu Thr Ile Ser Ser Leu
   65 70 75 80
   Gln Pro Glu Asp Phe Ala Thr Tyr Tyr Cys Gln Gln Ser Tyr Thr Ser
   85 90 95
   Pro Gly Ile Thr Phe Gly Pro Gly Thr Lys Val Glu Ile Lys
   100 105 110
<210> 100
   <211> 121
   <212> PRT
   <213> Homo Sapiens
<400> 100
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Pro Tyr
   20 25 30
   Pro Met Met Trp Val Arg Gln Ala Pro Gly Lys Gly Leu Glu Trp Val
   35 40 45
   Ser Tyr Ile Ser Pro Ser Gly Gly Lys Thr His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Gly Asp Thr Tyr Phe Asp Tyr Phe Asp Tyr Trp Gly Gln
   100 105 110
   Gly Thr Leu Val Thr Val Ser Ser Ala
   115 120
<210> 101
   <211> 217
   <212> PRT
   <213> Homo Sapiens
<400> 101
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Gly Tyr Leu Asn Trp Tyr Gln Gln Lys Pro Gly Lys Ala Pro Lys Leu
           35                  40                  45
   Leu Ile Tyr Asn Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Thr Ser
                   85                  90                  95
   Pro Gly Ile Thr Phe Gly Pro Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Leu Tyr Ala Cys Glu Val Thr His Gln Gly Leu Ser Ser Pro Val
           195                 200                 205
   Thr Lys Ser Phe Asn Arg Gly Glu Cys
       210                 215
<210> 102
   <211> 449
   <212> PRT
   <213> Homo Sapiens
<400> 102
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 103
   <211> 11
   <212> PRT
   <213> Homo Sapiens
<400> 103
   Arg Ala Ser Gln Ser Ile Ser Gly Tyr Leu Asn
   1               5                   10
<210> 104
   <211> 7
   <212> PRT
   <213> Homo Sapiens
<400> 104
   Asn Ala Ser Ser Leu Gln Ser
   1               5
<210> 105
   <211> 10
   <212> PRT
   <213> Homo Sapiens
<400> 105
   Gln Gln Ser Tyr Thr Ser Pro Gly Ile Thr
   1               5                   10
<210> 106
   <211> 5
   <212> PRT
   <213> Homo Sapiens
<400> 106
   Pro Tyr Pro Met Met
   1               5
<210> 107
   <211> 17
   <212> PRT
   <213> Homo Sapiens
<400> 107
   Tyr Ile Ser Pro Ser Gly Gly Lys Thr His Tyr Ala Asp Ser Val Lys
   1               5                   10                  15
   Gly
<210> 108
   <211> 11
   <212> PRT
   <213> Homo Sapiens
<400> 108
   Gly Gly Asp Thr Tyr Phe Asp Tyr Phe Asp Tyr
   1               5                   10
<210> 109
   <211> 110
   <212> PRT
   <213> Homo Sapiens
<400> 109
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Gly Tyr Leu Asn Trp Tyr Gln Gln Lys Pro Gly Lys Ala Pro Lys Leu
           35                  40                  45
   Leu Ile Tyr Asn Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Thr Ser
                   85                  90                  95
   Pro Gly Ile Thr Phe Gly Pro Gly Thr Lys Val Glu Ile Lys
               100                 105                 110
<210> 110
   <211> 121
   <212> PRT
   <213> Homo Sapiens
<400> 110
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala
           115                 120
<210> 111
   <211> 217
   <212> PRT
   <213> Homo Sapiens
<400> 111
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Ser Tyr Leu Asn Trp Tyr Gln Gln Lys Pro Gly Lys Ala Pro Lys Leu
           35                  40                  45
   Leu Ile Tyr Asn Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Thr Ser
                   85                  90                  95
   Pro Phe Ile Thr Phe Gly Pro Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Leu Tyr Ala Cys Glu Val Thr His Gln Gly Leu Ser Ser Pro Val
           195                 200                 205
   Thr Lys Ser Phe Asn Arg Gly Glu Cys
       210                 215
<210> 112
   <211> 449
   <212> PRT
   <213> Homo Sapiens
<400> 112
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 113
   <211> 11
   <212> PRT
   <213> Homo Sapiens
<400> 113
   Arg Ala Ser Gln Ser Ile Ser Ser Tyr Leu Asn
   1               5                   10
<210> 114
   <211> 7
   <212> PRT
   <213> Homo Sapiens
<400> 114
   Asn Ala Ser Ser Leu Gln Ser
   1 5
<210> 115
   <211> 10
   <212> PRT
   <213> Homo Sapiens
<400> 115
   Gln Gln Ser Tyr Thr Ser Pro Phe Ile Thr
   1 5 10
<210> 116
   <211> 5
   <212> PRT
   <213> Homo Sapiens
<400> 116
   Pro Tyr Pro Met Met
   1 5
<210> 117
   <211> 17
   <212> PRT
   <213> Homo Sapiens
<400> 117
   Tyr Ile Ser Pro Ser Gly Gly Lys Thr His Tyr Ala Asp Ser Val Lys
   1 5 10 15
   Gly
<210> 118
   <211> 11
   <212> PRT
   <213> Homo Sapiens
<400> 118
   Gly Gly Asp Thr Tyr Phe Asp Tyr Phe Asp Tyr
   1 5 10
<210> 119
   <211> 110
   <212> PRT
   <213> Homo Sapiens
<400> 119
   Ala Gln Asp Ile Gln Met Thr Gln Ser Pro Ala Thr Leu Ser Ala Ser
   1               5                   10                  15
   Val Gly Asp Arg Val Thr Ile Thr Cys Arg Ala Ser Gln Ser Ile Ser
               20                  25                  30
   Ser Tyr Leu Asn Trp Tyr Gln Gln Lys Pro Gly Lys Ala Pro Lys Leu
           35                  40                  45
   Leu Ile Tyr Asn Ala Ser Ser Leu Gln Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Phe Ala Thr Tyr Tyr Cys Gln Gln Ser Tyr Thr Ser
                   85                  90                  95
   Pro Phe Ile Thr Phe Gly Pro Gly Thr Lys Val Glu Ile Lys
               100                 105                 110
<210> 120
   <211> 121
   <212> **PRT**
   <213> Homo Sapiens
<400> 120
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Pro Tyr
               20                  25                  30
   Pro Met Met Trp Val Arg Gln Ala Pro Gly Lys Gly Leu Glu Trp Val
           35                  40                  45
   Ser Tyr Ile Ser Pro Ser Gly Gly Lys Thr His Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Thr Tyr Phe Asp Tyr Phe Asp Tyr Trp Gly Gln
               100                 105                 110
   Gly Thr Leu Val Thr Val Ser Ser Ala
           115                 120
<210> 121
   <211> 218
   <212> **PRT**
   <213> Mus Musculus
<400> 121
   Asp Ile Val Leu Thr Gln Ser Pro Ala Ser Leu Ala Val Ser Leu Gly
   1               5                   10                  15
   Gln Arg Ala Thr Ile Ser Cys Arg Ala Asn Lys Ser Val Ser Thr Ser
               20                  25                  30
   Ser Tyr Ser Tyr Met His Trp Tyr Gln Gln Lys Pro Gly Gln Pro Pro
           35                  40                  45
   Lys Leu Leu Ile Lys Tyr Ala Ser Asn Leu Glu Ser Gly Val Pro Ala
       50                  55                  60
   Arg Phe Ser Gly Ser Gly Ser Gly Thr Asp Phe Ile Leu Asn Ile His
   65                  70                  75                  80
   Pro Val Glu Glu Glu Asp Ala Ala Thr Tyr Tyr Cys Gln His Ser Arg
                   85                  90                  95
   Glu Leu Pro Phe Thr Phe Gly Ser Gly Thr Lys Leu Glu Ile Lys Arg
               100                 105                 110
   Ala Asp Ala Ala Pro Thr Val Ser Ile Phe Pro Pro Ser Ser Glu Gln
           115                 120                 125
   Leu Thr Ser Gly Gly Ala Ser Val Val Cys Phe Leu Asn Asn Phe Tyr
       130                 135                 140
   Pro Lys Asp Ile Asn Val Lys Trp Lys Ile Asp Gly Ser Glu Arg Gln
   145                 150                 155                 160
   Asn Gly Val Leu Asn Ser Trp Thr Asp Gln Asp Ser Lys Asp Ser Thr
                   165                 170                 175
   Tyr Ser Met Ser Ser Thr Leu Thr Leu Thr Lys Asp Glu Tyr Glu Arg
               180                 185                 190
   His Asn Ser Tyr Thr Cys Glu Ala Thr His Lys Thr Ser Thr Ser Pro
           195                 200                 205
   Ile Val Lys Ser Phe Asn Arg Asn Glu Cys
       210                 215
<210> 122
   <211> 450
   <212> PRT
   <213> Mus Musculus
<400> 122
   Gln Val Gln Leu Gln Gln Ser Gly Pro Glu Val Val Arg Pro Gly Val
   1               5                   10                  15
   Ser Val Lys Ile Ser Cys Lys Gly Ser Gly Tyr Thr Phe Thr Asp Tyr
               20                  25                  30
   Gly Ile His Trp Val Lys Gln Ser His Ala Lys Ser Leu Glu Trp Ile
           35                  40                  45
   Gly Val Ile Ser Thr Tyr Asn Gly Tyr Thr Asn Tyr Asn Gln Lys Phe
       50                  55                  60
   Lys Gly Lys Ala Thr Met Thr Val Asp Lys Ser Ser Ser Thr Ala Tyr
   65                  70                  75                  80
   Met Glu Leu Ala Arg Leu Thr Ser Glu Asp Ser Ala Ile Tyr Tyr Cys
                   85                  90                  95
   Ala Arg Ala Tyr Tyr Gly Asn Leu Tyr Tyr Ala Met Asp Tyr Trp Gly
               100                 105                 110
   Gln Gly Thr Ser Val Thr Val Ser Ser Ala Lys Thr Thr Ala Pro Ser
           115                 120                 125
   Val Tyr Pro Leu Ala Pro Val Cys Gly Asp Thr Thr Gly Ser Ser Val
       130                 135                 140
   Thr Leu Gly Cys Leu Val Lys Gly Tyr Phe Pro Glu Pro Val Thr Leu
   145                 150                 155                 160
   Thr Trp Asn Ser Gly Ser Leu Ser Ser Gly Val His Thr Phe Pro Ala
                   165                 170                 175
   Val Leu Gln Ser Asp Leu Tyr Thr Leu Ser Ser Ser Val Thr Val Thr
               180                 185                 190
   Ser Ser Thr Trp Pro Ser Gln Ser Ile Thr Cys Asn Val Ala His Pro
           195                 200                 205
   Ala Ser Ser Thr Lys Val Asp Lys Lys Ile Glu Pro Arg Gly Pro Thr
       210                 215                 220
   Ile Lys Pro Cys Pro Pro Cys Lys Cys Pro Ala Pro Asn Leu Leu Gly
   225                 230                 235                 240
   Gly Pro Ser Val Phe Ile Phe Pro Pro Lys Ile Lys Asp Val Leu Met
                   245                 250                 255
   Ile Ser Leu Ser Pro Ile Val Thr Cys Val Val Val Asp Val Ser Glu
               260                 265                 270
   Asp Asp Pro Asp Val Gln Ile Ser Trp Phe Val Asn Asn Val Glu Val
           275                 280                 285
   His Thr Ala Gln Thr Gln Thr His Arg Glu Asp Tyr Asn Ser Thr Leu
       290                 295                 300
   Arg Val Val Ser Ala Leu Pro Ile Gln His Gln Asp Trp Met Ser Gly
   305                 310                 315                 320
   Lys Glu Phe Lys Cys Lys Val Asn Asn Lys Asp Leu Pro Ala Pro Ile
                   325                 330                 335
   Glu Arg Thr Ile Ser Lys Pro Lys Gly Ser Val Arg Ala Pro Gln Val
               340                 345                 350
   Tyr Val Leu Pro Pro Pro Glu Glu Glu Met Thr Lys Lys Gln Val Thr
           355                 360                 365
   Leu Thr Cys Met Val Thr Asp Phe Met Pro Glu Asp Ile Tyr Val Glu
       370                 375                 380
   Trp Thr Asn Asn Gly Lys Thr Glu Leu Asn Tyr Lys Asn Thr Glu Pro
   385                 390                 395                 400
   Val Leu Asp Ser Asp Gly Ser Tyr Phe Met Tyr Ser Lys Leu Arg Val
                   405                 410                 415
   Glu Lys Lys Asn Trp Val Glu Arg Asn Ser Tyr Ser Cys Ser Val Val
               420                 425                 430
   His Glu Gly Leu His Asn His His Thr Thr Lys Ser Phe Ser Arg Thr
           435                 440                 445
   Pro Gly
       450
<210> 123
   <211> 218
   <212> PRT
   <213> Mus Musculus
<400> 123
   Asp Ile Val Leu Thr Gln Ser Pro Ala Ser Leu Thr Val Ser Leu Gly
   1               5                   10                  15
   Gln Arg Ala Thr Ile Ser Cys Arg Ala Ser Gln Ser Val Ser Thr Ser
               20                  25                  30
   Ser Tyr Ser Tyr Met Gln Trp Tyr Gln Gln Arg Pro Gly Gln Pro Pro
           35                  40                  45
   Lys Leu Leu Ile Lys Tyr Ala Thr Asn Leu Asp Ser Gly Val Pro Ala
       50                  55                  60
   Arg Phe Ser Gly Ser Gly Ser Gly Thr Asp Phe Thr Leu Asn Ile His
   65                  70                  75                  80
   Pro Val Glu Glu Glu Asp Ala Ala Thr Tyr Tyr Cys Gln His Ser Trp
                   85                  90                  95
   Glu Ile Pro Tyr Thr Phe Gly Gly Gly Thr Lys Leu Glu Ile Lys Arg
               100                 105                 110
   Ala Asp Ala Ala Pro Thr Val Ser Ile Phe Pro Pro Ser Ser Glu Gln
           115                 120                 125
   Leu Thr Ser Gly Gly Ala Ser Val Val Cys Phe Leu Asn Asn Phe Tyr
       130                 135                 140
   Pro Lys Asp Ile Asn Val Lys Trp Lys Ile Asp Gly Ser Glu Arg Gln
   145                 150                 155                 160
   Asn Gly Val Leu Asn Ser Trp Thr Asp Gln Asp Ser Lys Asp Ser Thr
                   165                 170                 175
   Tyr Ser Met Ser Ser Thr Leu Thr Leu Thr Lys Asp Glu Tyr Glu Arg
               180                 185                 190
   His Asn Ser Tyr Thr Cys Glu Ala Thr His Lys Thr Ser Thr Ser Pro
           195                 200                 205
   Ile Val Lys Ser Phe Asn Arg Asn Glu Cys
       210                 215
<210> 124
   <211> 448
   <212> PRT
   <213> Mus Musculus
<400> 124
   Glu Val Lys Leu Glu Glu Ser Gly Gly Gly Leu Val Gln Pro Gly Gly
   1               5                   10                  15
   Ser Met Lys Leu Ser Cys Val Ala Ser Gly Phe Thr Phe Asn Asn Tyr
               20                  25                  30
   Trp Met Ser Trp Val Arg Gln Ser Pro Glu Lys Gly Leu Glu Trp Leu
           35                  40                  45
   Ala Glu Ile Arg Leu Lys Ser Asp Asn Tyr Ala Thr His Tyr Ala Glu
       50                  55                  60
   Ser Val Lys Gly Lys Phe Thr Ile Ser Arg Asp Asp Ser Lys Ser Arg
   65                  70                  75                  80
   Leu Tyr Leu Gln Met Asn Asn Leu Arg Ala Glu Asn Thr Gly Ile Tyr
                   85                  90                  95
   Tyr Cys Thr Gly Gly Phe Ala Asp Tyr Phe Asp Tyr Trp Gly Gln Gly
               100                 105                 110
   Thr Thr Leu Thr Val Ser Ser Ala Lys Thr Thr Ala Pro Ser Val Tyr
           115                 120                 125
   Pro Leu Ala Pro Val Cys Gly Asp Thr Thr Gly Ser Ser Val Thr Leu
       130                 135                 140
   Gly Cys Leu Val Lys Gly Tyr Phe Pro Glu Pro Val Thr Leu Thr Trp
   145                 150                 155                 160
   Asn Ser Gly Ser Leu Ser Ser Gly Val His Thr Phe Pro Ala Val Leu
                   165                 170                 175
   Gln Ser Asp Leu Tyr Thr Leu Ser Ser Ser Val Thr Val Thr Ser Ser
               180                 185                 190
   Thr Trp Pro Ser Gln Ser Ile Thr Cys Asn Val Ala His Pro Ala Ser
           195                 200                 205
   Ser Thr Lys Val Asp Lys Lys Ile Glu Pro Arg Gly Pro Thr Ile Lys
       210                 215                 220
   Pro Cys Pro Pro Cys Lys Cys Pro Ala Pro Asn Leu Leu Gly Gly Pro
   225                 230                 235                 240
   Ser Val Phe Ile Phe Pro Pro Lys Ile Lys Asp Val Leu Met Ile Ser
                   245                 250                 255
   Leu Ser Pro Ile Val Thr Cys Val Val Val Asp Val Ser Glu Asp Asp
               260                 265                 270
   Pro Asp Val Gln Ile Ser Trp Phe Val Asn Asn Val Glu Val His Thr
           275                 280                 285
   Ala Gln Thr Gln Thr His Arg Glu Asp Tyr Asn Ser Thr Leu Arg Val
       290                 295                 300
   Val Ser Ala Leu Pro Ile Gln His Gln Asp Trp Met Ser Gly Lys Glu
   305                 310                 315                 320
   Phe Lys Cys Lys Val Asn Asn Lys Asp Leu Pro Ala Pro Ile Glu Arg
                   325                 330                 335
   Thr Ile Ser Lys Pro Lys Gly Ser Val Arg Ala Pro Gln Val Tyr Val
               340                 345                 350
   Leu Pro Pro Pro Glu Glu Glu Met Thr Lys Lys Gln Val Thr Leu Thr
           355                 360                 365
   Cys Met Val Thr Asp Phe Met Pro Glu Asp Ile Tyr Val Glu Trp Thr
       370                 375                 380
   Asn Asn Gly Lys Thr Glu Leu Asn Tyr Lys Asn Thr Glu Pro Val Leu
   385                 390                 395                 400
   Asp Ser Asp Gly Ser Tyr Phe Met Tyr Ser Lys Leu Arg Val Glu Lys
                   405                 410                 415
   Lys Asn Trp Val Glu Arg Asn Ser Tyr Ser Cys Ser Val Val His Glu
               420                 425                 430
   Gly Leu His Asn His His Thr Thr Lys Ser Phe Ser Arg Thr Pro Gly
           435                 440                 445
<210> 125
   <211> 218
   <212> PRT
   <213> Homo Sapiens
<400> 125
   Asp Ile Val Leu Thr Gln Ser Pro Ala Ser Leu Ala Val Ser Leu Gly
   1               5                   10                  15
   Gln Arg Ala Thr Ile Ser Cys Arg Ala Ser Lys Ser Val Ser Thr Ser
               20                  25                  30
   Ser Tyr Ser Tyr Met His Trp Tyr Gln Gln Lys Pro Gly Gln Pro Pro
           35                  40                  45
   Lys Leu Leu Ile Lys Tyr Ala Ser Asn Leu Glu Ser Gly Val Pro Ala
       50                  55                  60
   Arg Phe Ser Gly Ser Gly Ser Gly Thr Asp Phe Ser Leu Asn Ile His
   65                  70                  75                  80
   Pro Met Glu Glu Asp Asp Thr Ala Met Tyr Phe Cys Gln His Ser Arg
                   85                  90                  95
   Glu Leu Pro Phe Thr Phe Gly Gly Gly Thr Lys Leu Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 126
   <211> 450
   <212> PRT
   <213> Homo Sapiens
<400> 126
   Gln Val Gln Leu Val Gln Ser Gly Ala Glu Val Lys Lys Pro Gly Ala
   1               5                   10                  15
   Ser Val Lys Val Ser Cys Lys Gly Ser Gly Tyr Thr Phe Thr Asp Tyr
               20                  25                  30
   Gly Met His Trp Val Arg Gln Ala Pro Gly Gln Gly Leu Glu Trp Met
           35                  40                  45
   Gly Val Ile Ser Thr Tyr Asn Gly Tyr Thr Asn Tyr Asn Gln Lys Phe
       50                  55                  60
   Lys Gly Arg Val Thr Met Thr Val Asp Lys Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Ala Tyr Tyr Gly Asn Leu Tyr Tyr Ala Met Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly
       450
<210> 127
   <211> 218
   <212> PRT
   <213> Homo Sapiens
<400> 127
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Val Ser Thr Ser
               20                  25                  30
   Ser Tyr Ser Tyr Met His Trp Tyr Gln Gln Lys Pro Gly Lys Ala Pro
           35                  40                  45
   Lys Leu Leu Ile Lys Tyr Ala Ser Asn Leu Glu Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln His Ser Trp
                   85                  90                  95
   Glu Ile Pro Tyr Thr Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 128
   <211> 449
   <212> PRT
   <213> Homo Sapiens
<400> 128
   Gln Val Glu Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Trp Met Ser Trp Val Arg Gln Ala Pro Gly Lys Gly Leu Glu Trp Val
           35                  40                  45
   Ala Glu Ile Arg Leu Lys Ser Asp Asn Tyr Ala Thr His Tyr Ala Glu
       50                  55                  60
   Ser Val Lys Gly Arg Phe Thr Ile Ser Arg Asp Asp Ser Lys Asn Ser
   65                  70                  75                  80
   Leu Tyr Leu Gln Met Asn Ser Leu Arg Ala Glu Asp Thr Ala Val Tyr
                   85                  90                  95
   Tyr Cys Thr Gly Tyr Tyr Ala Asp Ala Met Asp Tyr Trp Gly Gln Gly
               100                 105                 110
   Thr Leu Val Thr Val Ser Ser Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Lys Val Glu Pro Lys Ser Cys Asp Lys
       210                 215                 220
   Thr His Thr Cys Pro Pro Cys Pro Ala Pro Glu Leu Leu Gly Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser His Glu Asp
               260                 265                 270
   Pro Glu Val Lys Phe Asn Trp Tyr Val Asp Gly Val Glu Val His Asn
           275                 280                 285
   Ala Lys Thr Lys Pro Arg Glu Glu Gln Tyr Asn Ser Thr Tyr Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Ala Leu Pro Ala Pro Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Arg Asp Glu Leu Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385                 390                 395                 400
   Asp Ser Asp Gly Ser Phe Phe Leu Tyr Ser Lys Leu Thr Val Asp Lys
                   405                 410                 415
   Ser Arg Trp Gln Gln Gly Asn Val Phe Ser Cys Ser Val Met His Glu
               420                 425                 430
   Ala Leu His Asn His Tyr Thr Gln Lys Ser Leu Ser Leu Ser Pro Gly
           435                 440                 445
   Lys
<210> 129
   <211> 218
   <212> PRT
   <213> Mus Musculus
<400> 129
   Asp Ile Val Leu Thr Gln Ser Pro Ala Ser Leu Ala Val Ser Leu Gly
   1               5                   10                  15
   Gln Arg Ala Thr Ile Ser Cys Lys Ala Ser Gln Ser Val Ser Thr Ser
               20                  25                  30
   Thr Tyr Ser Tyr Met Gln Trp Tyr Gln Gln Arg Pro Gly Gln Ser Pro
           35                  40                  45
   Lys Leu Leu Ile Lys Tyr Ala Ser Lys Leu Asp Ser Gly Val Pro Ala
       50                  55                  60
   Arg Phe Ser Gly Ser Gly Ser Gly Thr Asp Phe Thr Leu Asn Ile His
   65                  70                  75                  80
   Pro Val Glu Glu Glu Asp Thr Ala Thr Tyr Tyr Cys Gln His Ser Trp
                   85                  90                  95
   Glu Leu Pro Tyr Thr Phe Gly Gly Gly Thr Arg Leu Glu Ile Lys Arg
               100                 105                 110
   Ala Asp Ala Ala Pro Thr Val Ser Ile Phe Pro Pro Ser Ser Glu Gln
           115                 120                 125
   Leu Thr Ser Gly Gly Ala Ser Val Val Cys Phe Leu Asn Asn Phe Tyr
       130                 135                 140
   Pro Lys Asp Ile Asn Val Lys Trp Lys Ile Asp Gly Ser Glu Arg Gln
   145                 150                 155                 160
   Asn Gly Val Leu Asn Ser Trp Thr Asp Gln Asp Ser Lys Asp Ser Thr
                   165                 170                 175
   Tyr Ser Met Ser Ser Thr Leu Thr Leu Thr Lys Asp Glu Tyr Glu Arg
               180                 185                 190
   His Asn Ser Tyr Thr Cys Glu Ala Thr His Lys Thr Ser Thr Ser Pro
           195                 200                 205
   Ile Val Lys Ser Phe Asn Arg Asn Glu Cys
       210                 215
<210> 130
   <211> 448
   <212> PRT
   <213> Mus Musculus
<400> 130
   Glu Val Lys Leu Gly Glu Ser Gly Gly Gly Leu Val Gln Pro Gly Gly
   1               5                   10                  15
   Ser Met Lys Leu Ser Cys Val Ala Ser Gly Phe Pro Phe Thr Lys Tyr
               20                  25                  30
   Trp Met Asn Trp Val Arg Gln Ser Pro Glu Lys Gly Leu Glu Trp Val
           35                  40                  45
   Ala Glu Ile Arg Leu Lys Ser Asp Asn Tyr Ala Thr His Tyr Ala Glu
       50                  55                  60
   Ser Ala Lys Gly Arg Phe Thr Ile Ser Arg Asp Asp Ser Arg Ser Ser
   65                  70                  75                  80
   Val Tyr Leu Gln Met Asn Asn Leu Arg Ala Glu Asp Thr Ala Ile Tyr
                   85                  90                  95
   Tyr Cys Ser Pro Thr Tyr Ala Asp Thr Met Asp Tyr Trp Gly Gln Gly
               100                 105                 110
   Thr Ser Val Thr Val Ser Ser Ala Lys Thr Thr Ala Pro Ser Val Tyr
           115                 120                 125
   Pro Leu Ala Pro Val Cys Gly Asp Thr Thr Gly Ser Ser Val Thr Leu
       130                 135                 140
   Gly Cys Leu Val Lys Gly Tyr Phe Pro Glu Pro Val Thr Leu Thr Trp
   145                 150                 155                 160
   Asn Ser Gly Ser Leu Ser Ser Gly Val His Thr Phe Pro Ala Val Leu
                   165                 170                 175
   Gln Ser Asp Leu Tyr Thr Leu Ser Ser Ser Val Thr Val Thr Ser Ser
               180                 185                 190
   Thr Trp Pro Ser Gln Ser Ile Thr Cys Asn Val Ala His Pro Ala Ser
           195                 200                 205
   Ser Thr Lys Val Asp Lys Lys Ile Glu Pro Arg Gly Pro Thr Ile Lys
       210                 215                 220
   Pro Cys Pro Pro Cys Lys Cys Pro Ala Pro Asn Leu Leu Gly Gly Pro
   225                 230                 235                 240
   Ser Val Phe Ile Phe Pro Pro Lys Ile Lys Asp Val Leu Met Ile Ser
                   245                 250                 255
   Leu Ser Pro Ile Val Thr Cys Val Val Val Asp Val Ser Glu Asp Asp
               260                 265                 270
   Pro Asp Val Gln Ile Ser Trp Phe Val Asn Asn Val Glu Val His Thr
           275                 280                 285
   Ala Gln Thr Gln Thr His Arg Glu Asp Tyr Asn Ser Thr Leu Arg Val
       290                 295                 300
   Val Ser Ala Leu Pro Ile Gln His Gln Asp Trp Met Ser Gly Lys Glu
   305                 310                 315                 320
   Phe Lys Cys Lys Val Asn Asn Lys Asp Leu Pro Ala Pro Ile Glu Arg
                   325                 330                 335
   Thr Ile Ser Lys Pro Lys Gly Ser Val Arg Ala Pro Gln Val Tyr Val
               340                 345                 350
   Leu Pro Pro Pro Glu Glu Glu Met Thr Lys Lys Gln Val Thr Leu Thr
           355                 360                 365
   Cys Met Val Thr Asp Phe Met Pro Glu Asp Ile Tyr Val Glu Trp Thr
       370                 375                 380
   Asn Asn Gly Lys Thr Glu Leu Asn Tyr Lys Asn Thr Glu Pro Val Leu
   385                 390                 395                 400
   Asp Ser Asp Gly Ser Tyr Phe Met Tyr Ser Lys Leu Arg Val Glu Lys
                   405                 410                 415
   Lys Asn Trp Val Glu Arg Asn Ser Tyr Ser Cys Ser Val Val His Glu
               420                 425                 430
   Gly Leu His Asn His His Thr Thr Lys Ser Phe Ser Arg Thr Pro Gly
           435                 440                 445
<210> 131
   <211> 218
   <212> PRT
   <213> Mus Musculus
<400> 131
   Asp Ile Val Leu Thr Gln Ser Pro Ala Ser Leu Ala Val Ser Leu Gly
   1               5                   10                  15
   Gln Arg Ala Thr Ile Ser Cys Arg Ala Ser Lys Ser Val Ser Thr Ser
               20                  25                  30
   Ser Tyr Ser Tyr Met His Trp Tyr Gln Gln Lys Pro Gly Gln Pro Pro
           35                  40                  45
   Lys Leu Leu Ile Lys Tyr Thr Ser Asn Leu Glu Ser Gly Val Pro Ala
       50                  55                  60
   Arg Phe Ser Gly Ser Gly Ser Gly Thr Asp Phe Ile Leu Asn Ile His
   65                  70                  75                  80
   Pro Val Glu Glu Glu Asp Ala Ala Thr Tyr Tyr Cys Gln His Ser Arg
                   85                  90                  95
   Glu Leu Pro Trp Thr Phe Gly Gly Gly Thr Lys Leu Glu Ile Lys Arg
               100                 105                 110
   Ala Asp Ala Ala Pro Thr Val Ser Ile Phe Pro Pro Ser Ser Glu Gln
           115                 120                 125
   Leu Thr Ser Gly Gly Ala Ser Val Val Cys Phe Leu Asn Asn Phe Tyr
       130                 135                 140
   Pro Lys Asp Ile Asn Val Lys Trp Lys Ile Asp Gly Ser Glu Arg Gln
   145                 150                 155                 160
   Asn Gly Val Leu Asn Ser Trp Thr Asp Gln Asp Ser Lys Asp Ser Thr
                   165                 170                 175
   Tyr Ser Met Ser Ser Thr Leu Thr Leu Thr Lys Asp Glu Tyr Glu Arg
               180                 185                 190
   His Asn Ser Tyr Thr Cys Glu Ala Thr His Lys Thr Ser Thr Ser Pro
           195                 200                 205
   Ile Val Lys Ser Phe Asn Arg Asn Glu Cys
       210                 215
<210> 132
   <211> 451
   <212> PRT
   <213> Mus Musculus
<400> 132
   Gln Val Ser Leu Lys Glu Ser Gly Pro Gly Ile Leu Gln Pro Ser Gln
   1               5                   10                  15
   Thr Leu Ser Leu Thr Cys Ser Phe Ser Gly Phe Ser Leu Ser Thr Ser
               20                  25                  30
   Gly Met Gly Val Ser Trp Ile Arg Gln Pro Ser Gly Lys Gly Leu Glu
           35                  40                  45
   Trp Leu Ala His Ile Tyr Trp Asp Asp Asp Lys Arg Tyr Asn Pro Ser
       50                  55                  60
   Leu Lys Ser Arg Leu Thr Ile Ser Lys Asp Thr Ser Arg Asn Gln Val
   65 70 75 80
   Phe Leu Lys Ile Thr Ser Val Asp Thr Ala Asp Thr Ala Thr Tyr Tyr
   85 90 95
   Cys Ala Arg Arg Gly Pro Asp Tyr Tyr Gly Tyr Tyr Pro Met Asp Tyr
   100 105 110
   Trp Gly Gln Gly Thr Ser Val Thr Val Ser Ala Lys Thr Thr Ala Pro
   115 120 125
   Ser Val Tyr Pro Leu Ala Pro Val Cys Gly Asp Thr Thr Gly Ser Ser
   130 135 140
   Val Thr Leu Gly Cys Leu Val Lys Gly Tyr Phe Pro Glu Pro Val Thr
   145 150 155 160
   Leu Thr Trp Asn Ser Gly Ser Leu Ser Ser Gly Val His Thr Phe Pro
   165 170 175
   Ala Val Leu Gln Ser Asp Leu Tyr Thr Leu Ser Ser Ser Val Thr Val
   180 185 190
   Thr Ser Ser Thr Trp Pro Ser Gln Ser Ile Thr Cys Asn Val Ala His
   195 200 205
   Pro Ala Ser Ser Thr Lys Val Asp Lys Lys Ile Glu Pro Arg Gly Pro
   210 215 220
   Thr Ile Lys Pro Cys Pro Pro Cys Lys Cys Pro Ala Pro Asn Leu Leu
   225 230 235 240
   Gly Gly Pro Ser Val Phe Ile Phe Pro Pro Lys Ile Lys Asp Val Leu
   245 250 255
   Met Ile Ser Leu Ser Pro Ile Val Thr Cys Val Val Val Asp Val Ser
   260 265 270
   Glu Asp Asp Pro Asp Val Gln Ile Ser Trp Phe Val Asn Asn Val Glu
   275 280 285
   Val His Thr Ala Gln Thr Gln Thr His Arg Glu Asp Tyr Asn Ser Thr
   290 295 300
   Leu Arg Val Val Ser Ala Leu Pro Ile Gln His Gln Asp Trp Met Ser
   305 310 315 320
   Gly Lys Glu Phe Lys Cys Lys Val Asn Asn Lys Asp Leu Pro Ala Pro
   325 330 335
   Ile Glu Arg Thr Ile Ser Lys Pro Lys Gly Ser Val Arg Ala Pro Gln
   340 345 350
   Val Tyr Val Leu Pro Pro Pro Glu Glu Glu Met Thr Lys Lys Gln Val
   355 360 365
   Thr Leu Thr Cys Met Val Thr Asp Phe Met Pro Glu Asp Ile Tyr Val
   370 375 380
   Glu Trp Thr Asn Asn Gly Lys Thr Glu Leu Asn Tyr Lys Asn Thr Glu
   385 390 395 400
   Pro Val Leu Asp Ser Asp Gly Ser Tyr Phe Met Tyr Ser Lys Leu Arg
   405 410 415
   Val Glu Lys Lys Asn Trp Val Glu Arg Asn Ser Tyr Ser Cys Ser Val
   420 425 430
   Val His Glu Gly Leu His Asn His His Thr Thr Lys Ser Phe Ser Arg
   435 440 445
   Thr Pro Gly
   450
<210> 133
   <211> 282
   <212> PRT
   <213> Macaca fascicularis
<400> 133
   Glu Gln Ala Pro Gly Thr Ala Pro Cys Ser His Gly Ser Ser Trp Ser
   1 5 10 15
   Ala Asp Leu Asp Lys Cys Met Asp Cys Ala Ser Cys Arg Ala Arg Pro
   20 25 30
   His Ser Asp Phe Cys Leu Gly Cys Ser Ala Ala Pro Pro Ala Pro Phe
   35 40 45
   Arg Leu Leu Trp Pro Arg Ser Asp Lys Thr His Thr Cys Pro Pro Cys
   50 55 60
   Pro Ala Pro Glu Leu Leu Gly Gly Pro Ser Val Phe Leu Phe Pro Pro
   65 70 75 80
   Lys Pro Lys Asp Thr Leu Met Ile Ser Arg Thr Pro Glu Val Thr Cys
   85 90 95
   Val Val Val Asp Val Ser His Glu Asp Pro Glu Val Lys Phe Asn Trp
   100 105 110
   Tyr Val Asp Gly Val Glu Val His Asn Ala Lys Thr Lys Pro Arg Glu
   115 120 125
   Glu Gln Tyr Asn Ser Thr Tyr Arg Val Val Ser Val Leu Thr Val Leu
   130 135 140
   His Gln Asp Trp Leu Asn Gly Lys Glu Tyr Lys Cys Lys Val Ser Asn
   145 150 155 160
   Lys Ala Leu Pro Ala Pro Ile Glu Lys Thr Ile Ser Lys Ala Lys Gly
   165 170 175
   Gln Pro Arg Glu Pro Gln Val Tyr Thr Leu Pro Pro Ser Arg Glu Glu
   180 185 190
   Met Thr Lys Asn Gln Val Ser Leu Thr Cys Leu Val Lys Gly Phe Tyr
   195 200 205
   Pro Ser Asp Ile Ala Val Glu Trp Glu Ser Asn Gly Gln Pro Glu Asn
   210 215 220
   Asn Tyr Lys Thr Thr Pro Pro Val Leu Asp Ser Asp Gly Ser Phe Phe
   225 230 235 240
   Leu Tyr Ser Lys Leu Thr Val Asp Lys Ser Arg Trp Gln Gln Gly Asn
   245 250 255
   Val Phe Ser Cys Ser Val Met His Glu Ala Leu His Asn His Tyr Thr
   260 265 270
   Gln Lys Ser Leu Ser Leu Ser Pro Gly Lys
   275 280
<210> 134
   <211> 282
   <212> PRT
   <213> Rattus norvegicus
<400> 134
   Glu Gln Ala Pro Gly Asn Ala Pro Cys Ser Ser Gly Ser Ser Trp Ser
   1               5                   10                  15
   Ala Asp Leu Asp Lys Cys Met Asp Cys Ala Ser Cys Pro Ala Arg Pro
               20                  25                  30
   His Ser Asp Phe Cys Leu Gly Cys Ala Ala Ala Pro Pro Ala His Phe
           35                  40                  45
   Arg Met Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 135
   <211> 282
   <212> PRT
   <213> Sus scrofa
<400> 135
   Glu Arg Val Pro Gly Thr Thr Pro Cys Ser Arg Gly Ser Ser Trp Ser
   1               5                   10                  15
   Ala Asp Leu Asp Lys Cys Met Asp Cys Ala Ser Cys Pro Ala Arg Pro
               20                  25                  30
   His Ser Asp Phe Cys Leu Gly Cys Ala Ala Ala Pro Pro Ala Ser Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 136
   <211> 282
   <212> PRT
   <213> Canis lupus
<400> 136
   Glu Arg Val Pro Gly Thr Thr Pro Cys Pro Arg Gly Ser Ser Trp Ser
   1               5                   10                  15
   Ala Asp Leu Asp Lys Cys Met Asp Cys Ala Ser Cys Arg Ala Arg Pro
               20                  25                  30
   His Ser Asp Phe Cys Leu Gly Cys Thr Ala Ala Pro Pro Ala Pro Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 137
   <211> 282
   <212> PRT
   <213> Mus Musculus
<400> 137
   Glu Gln Ala Pro Gly Thr Ser Pro Cys Ser Ser Gly Ser Ser Trp Ser
   1               5                   10                  15
   Ala Asp Leu Asp Lys Cys Met Asp Cys Ala Ser Cys Pro Ala Arg Pro
               20                  25                  30
   His Ser Asp Phe Cys Leu Gly Cys Ala Ala Ala Pro Pro Ala His Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 138
   <211> 282
   <212> PRT
   <213> Homo Sapiens
<400> 138
   Glu Gln Ala Pro Gly Thr Ala Pro Cys Ser Arg Gly Ser Ser Trp Ser
   1               5                   10                  15
   Ala Asp Leu Asp Lys Cys Met Asp Cys Ala Ser Cys Arg Ala Arg Pro
               20                  25                  30
   His Ser Asp Phe Cys Leu Gly Cys Ala Ala Ala Pro Pro Ala Pro Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 139
   <211> 296
   <212> PRT
   <213> Homo Sapiens
<400> 139
   Glu Gln Ala Pro Gly Thr Ala Pro Cys Ser Arg Gly Ser Ser Trp Ser
   1               5                   10                  15
   Ala Asp Leu Asp Lys Cys Met Asp Cys Ala Ser Cys Arg Ala Arg Pro
               20                  25                  30
   His Ser Asp Phe Cys Leu Gly Cys Ala Ala Ala Pro Pro Ala Pro Phe
           35                  40                  45
   Arg Leu Leu Trp Pro Ile Glu Gly Arg Met Asp Pro Lys Ser Cys Asp
       50                  55                  60
   Lys Thr His Thr Cys Pro Pro Cys Pro Ala Pro Glu Leu Leu Gly Gly
   65                  70                  75                  80
   Pro Ser Val Phe Leu Phe Pro Pro Lys Pro Lys Asp Thr Leu Met Ile
                   85                  90                  95
   Ser Arg Thr Pro Glu Val Thr Cys Val Val Val Asp Val Ser His Glu
               100                 105                 110
   Asp Pro Glu Val Lys Phe Asn Trp Tyr Val Asp Gly Val Glu Val His
           115                 120                 125
   Asn Ala Lys Thr Lys Pro Arg Glu Glu Gln Tyr Asn Ser Thr Tyr Arg
       130                 135                 140
   Val Val Ser Val Leu Thr Val Leu His Gln Asp Trp Leu Asn Gly Lys
   145                 150                 155                 160
   Glu Tyr Lys Cys Lys Val Ser Asn Lys Ala Leu Pro Ala Pro Ile Glu
                   165                 170                 175
   Lys Thr Ile Ser Lys Ala Lys Gly Gln Pro Arg Glu Pro Gln Val Tyr
               180                 185                 190
   Thr Leu Pro Pro Ser Arg Glu Glu Met Thr Lys Asn Gln Val Ser Leu
           195                 200                 205
   Thr Cys Leu Val Lys Gly Phe Tyr Pro Ser Asp Ile Ala Val Glu Trp
       210                 215                 220
   Glu Ser Asn Gly Gln Pro Glu Asn Asn Tyr Lys Thr Thr Pro Pro Val
   225                 230                 235                 240
   Leu Asp Ser Asp Gly Ser Phe Phe Leu Tyr Ser Lys Leu Thr Val Asp
                   245                 250                 255
   Lys Ser Arg Trp Gln Gln Gly Asn Val Phe Ser Cys Ser Val Met His
               260                 265                 270
   Glu Ala Leu His Asn His Tyr Thr Gln Lys Ser Leu Ser Leu Ser Pro
           275                 280                 285
   Gly Lys His His His His His His
       290                 295
<210> 140
   <211> 278
   <212> PRT
   <213> Homo Sapiens
<400> 140
   Ala Pro Cys Ser Arg Gly Ser Ser Trp Ser Ala Asp Leu Asp Lys Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 141
   <211> 47
   <212> PRT
   <213> Homo Sapiens
<400> 141
   Glu Gln Ala Pro Gly Thr Ala Pro Cys Ser Arg Gly Ser Ser Trp Ser
   1               5                   10                  15
   Ala Asp Leu Asp Lys Cys Met Asp Cys Ala Ser Cys Arg Ala Arg Pro
               20                  25                  30
   His Ser Asp Phe Cys Leu Gly Cys Ala His His His His His His
           35                  40                  45
<210> 142
   <211> 282
   <212> PRT
   <213> Homo Sapiens
<400> 142
   Glu Gln Ala Pro Gly Thr Ala Pro Cys Ser Arg Gly Ser Ser Trp Ser
   1               5                   10                  15
   Ala Asp Leu Asp Lys Cys Met Asp Cys Ala Ser Cys Arg Ala Arg Pro
               20                  25                  30
   His Ser Asp Phe Cys Gln Gly Cys Ala Ala Ala Pro Pro Ala Pro Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 143
   <211> 282
   <212> PRT
   <213> Homo Sapiens
<400> 143
   Glu Gln Ala Pro Gly Thr Ala Pro Cys Ser Arg Gly Ser Ser Trp Ser
   1               5                   10                  15
   Ala Asp Leu Asp Lys Cys Met Asp Cys Ala Ser Cys Arg Ala Arg Pro
               20                  25                  30
   Lys Ser Asp Phe Cys Leu Gly Cys Ala Ala Ala Pro Pro Ala Pro Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 144
   <211> 282
   <212> PRT
   <213> Homo Sapiens
<400> 144
   Glu Gln Ala Pro Gly Thr Ala Pro Cys Ser Arg Gly Ser Ser Trp Ser
   1               5                   10                  15
   Ala Asp Leu Asp Lys Cys Met Asp Cys Ala Ser Cys Pro Ala Arg Pro
               20                  25                  30
   His Ser Asp Phe Cys Leu Gly Cys Ala Ala Ala Pro Pro Ala Pro Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 145
   <211> 282
   <212> PRT
   <213> Homo Sapiens
<400> 145
   Glu Gln Ala Pro Gly Thr Ala Pro Cys Ser Arg Gly Ser Ser Trp Ser
   1               5                   10                  15
   Ala Asp Leu Asp Lys Cys Ala Asp Cys Ala Ser Cys Arg Ala Arg Pro
               20                  25                  30
   His Ser Asp Phe Cys Leu Gly Cys Ala Ala Ala Pro Pro Ala Pro Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 146
   <211> 282
   <212> PRT
   <213> Homo Sapiens
<400> 146
   Glu Gln Ala Pro Gly Thr Ala Pro Cys Ser Arg Gly Ser Ser Ala Ser
   1               5                   10                  15
   Ala Asp Leu Asp Lys Cys Met Asp Cys Ala Ser Cys Arg Ala Arg Pro
               20                  25                  30
   His Ser Asp Phe Cys Leu Gly Cys Ala Ala Ala Pro Pro Ala Pro Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 147
   <211> 282
   <212> PRT
   <213> Homo Sapiens
<400> 147
   Glu Gln Ala Pro Gly Thr Ala Pro Cys Ser Arg Gly Arg Ser Trp Ser
   1               5                   10                  15
   Ala Asp Leu Asp Lys Cys Met Asp Cys Ala Ser Cys Arg Ala Arg Pro
               20                  25                  30
   His Ser Asp Phe Cys Leu Gly Cys Ala Ala Ala Pro Pro Ala Pro Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 148
   <211> 282
   <212> PRT
   <213> Homo Sapiens
<400> 148
   Glu Gln Ala Pro Gly Gln Ala Pro Cys Ser Arg Gly Ser Ser Trp Ser
   1               5                   10                  15
   Ala Asp Leu Asp Lys Cys Met Asp Cys Ala Ser Cys Arg Ala Arg Pro
               20                  25                  30
   His Ser Asp Phe Cys Leu Gly Cys Ala Ala Ala Pro Pro Ala Pro Phe
           35                  40                  45
   Arg Leu Leu Trp Pro Arg Ser Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Asn Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Glu Glu
               180                 185                 190
   Met Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Lys
           275                 280
<210> 149
   <211> 278
   <212> PRT
   <213> Homo Sapiens
<400> 149
   Ala Pro Cys Ser Arg Gly Ser Ser Trp Ser Ala Asp Leu Asp Lys Cys
   1               5                   10                  15
   Met Asp Cys Ala Ser Cys Arg Ala Arg Pro His Ser Asp Phe Cys Ala
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 150
   <211> 278
   <212> PRT
   <213> Homo Sapiens
<400> 150
   Ala Pro Cys Ser Arg Gly Ser Ser Trp Ser Ala Asp Leu Asp Lys Cys
   1               5                   10                  15
   Met Asp Cys Ala Ser Cys Arg Ala Arg Pro His Ser Asp Ala Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 151
   <211> 278
   <212> PRT
   <213> Homo Sapiens
<400> 151
   Ala Pro Cys Ser Arg Gly Ser Ser Trp Ser Ala Asp Leu Asp Lys Cys
   1               5                   10                  15
   Met Asp Cys Ala Ser Cys Arg Ala Arg Pro His Ser Ala Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 152
   <211> 278
   <212> PRT
   <213> Homo Sapiens
<400> 152
   Ala Pro Cys Ser Arg Gly Ser Ser Trp Ser Ala Asp Leu Asp Lys Cys
   1               5                   10                  15
   Met Asp Cys Ala Ser Cys Arg Ala Arg Pro His Ala Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 153
   <211> 278
   <212> PRT
   <213> Homo Sapiens
<400> 153
   Ala Pro Cys Ser Arg Gly Ser Ser Trp Ser Ala Asp Leu Asp Lys Cys
   1               5                   10                  15
   Met Asp Cys Ala Ser Cys Arg Ala Arg Pro Ala Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 154
   <211> 278
   <212> PRT
   <213> Homo Sapiens
<400> 154
   Ala Pro Cys Ser Arg Gly Ser Ser Trp Ser Ala Asp Leu Asp Lys Cys
   1               5                   10                  15
   Met Asp Cys Ala Ser Cys Arg Ala Arg Ala His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
                   100                 105                 110
   Lys Thr Lys Pro Arg Glu Glu Gln Tyr Asn Ser Thr Tyr Arg Val Val
               115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 155
   <211> 278
   <212> PRT
   <213> Homo Sapiens
<400> 155
   Ala Pro Cys Ser Arg Gly Ser Ser Trp Ser Ala Asp Leu Asp Lys Cys
   1               5                   10                  15
   Met Asp Cys Ala Ser Cys Arg Ala Ala Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
                   260                 265                 270
   His His His His His His
           275
<210> 156
   <211> 240
   <212> PRT
   <213> Homo Sapiens
<400> 156
   Ala Pro Cys Ser Arg Gly Ser Ser Trp Ser Ala Asp Leu Asp Lys Cys
   1               5                   10                  15
   Met Asp Cys Ala Ser Cys Ala Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
<210> 157
   <211> 278
   <212> PRT
   <213> Homo Sapiens
<400> 157
   Ala Pro Cys Ser Arg Gly Ser Ser Trp Ser Ala Asp Leu Asp Lys Cys
   1               5                   10                  15
   Met Asp Cys Ala Ala Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 158
   <211> 278
   <212> PRT
   <213> Homo Sapiens
<400> 158
   Ala Pro Cys Ser Arg Gly Ser Ser Trp Ser Ala Asp Leu Asp Lys Cys
   1               5                   10                  15
   Met Ala Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 159
   <211> 278
   <212> PRT
   <213> Homo Sapiens
<400> 159
   Ala Pro Cys Ser Arg Gly Ser Ser Trp Ser Ala Asp Leu Asp Ala Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 160
   <211> 278
   <212> PRT
   <213> Homo Sapiens
<400> 160
   Ala Pro Cys Ser Arg Gly Ser Ser Trp Ser Ala Asp Leu Ala Lys Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 161
   <211> 278
   <212> PRT
   <213> Homo Sapiens
<400> 161
   Ala Pro Cys Ser Arg Gly Ser Ser Trp Ser Ala Ala Leu Asp Lys Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 162
   <211> 278
   <212> PRT
   <213> Homo Sapiens
<400> 162
   Ala Pro Cys Ser Arg Gly Ser Ser Trp Ala Ala Asp Leu Asp Lys Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 163
   <211> 278
   <212> PRT
   <213> Homo Sapiens
<400> 163
   Ala Pro Cys Ser Arg Gly Ser Ser Ala Ser Ala Asp Leu Asp Lys Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 164
   <211> 278
   <212> PRT
   <213> Homo Sapiens
<400> 164
   Ala Pro Cys Ser Arg Gly Ser Ala Trp Ser Ala Asp Leu Asp Lys Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 165
   <211> 278
   <212> PRT
   <213> Homo Sapiens
<400> 165
   Ala Pro Cys Ser Arg Gly Ala Ser Trp Ser Ala Asp Leu Asp Lys Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 166
   <211> 278
   <212> PRT
   <213> Homo Sapiens
<400> 166
   Ala Pro Cys Ser Ala Gly Ser Ser Trp Ser Ala Asp Leu Asp Lys Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
               35                  40                  45
   His Thr Cys Pro Pro Cys Pro Ala Pro Glu Leu Leu Gly Gly Pro Ser
           50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 167
   <211> 278
   <212> PRT
   <213> Homo Sapiens
<400> 167
   Ala Pro Cys Ala Arg Gly Ser Ser Trp Ser Ala Asp Leu Asp Lys Cys
   1               5                   10                  15
   Met Asp Cys Ala Ser Cys Arg Ala Arg Pro His Ser Asp Phe Cys Leu
               20                  25                  30
   Gly Cys Ala Ile Glu Gly Arg Met Asp Pro Lys Ser Cys Asp Lys Thr
           35                  40                  45
   His Thr Cys Pro Pro Cys Pro Ala Pro Glu Leu Leu Gly Gly Pro Ser
       50                  55                  60
   Val Phe Leu Phe Pro Pro Lys Pro Lys Asp Thr Leu Met Ile Ser Arg
   65                  70                  75                  80
   Thr Pro Glu Val Thr Cys Val Val Val Asp Val Ser His Glu Asp Pro
                   85                  90                  95
   Glu Val Lys Phe Asn Trp Tyr Val Asp Gly Val Glu Val His Asn Ala
               100                 105                 110
   Lys Thr Lys Pro Arg Glu Glu Gln Tyr Asn Ser Thr Tyr Arg Val Val
           115                 120                 125
   Ser Val Leu Thr Val Leu His Gln Asp Trp Leu Asn Gly Lys Glu Tyr
       130                 135                 140
   Lys Cys Lys Val Ser Asn Lys Ala Leu Pro Ala Pro Ile Glu Lys Thr
   145                 150                 155                 160
   Ile Ser Lys Ala Lys Gly Gln Pro Arg Glu Pro Gln Val Tyr Thr Leu
                   165                 170                 175
   Pro Pro Ser Arg Glu Glu Met Thr Lys Asn Gln Val Ser Leu Thr Cys
               180                 185                 190
   Leu Val Lys Gly Phe Tyr Pro Ser Asp Ile Ala Val Glu Trp Glu Ser
           195                 200                 205
   Asn Gly Gln Pro Glu Asn Asn Tyr Lys Thr Thr Pro Pro Val Leu Asp
       210                 215                 220
   Ser Asp Gly Ser Phe Phe Leu Tyr Ser Lys Leu Thr Val Asp Lys Ser
   225                 230                 235                 240
   Arg Trp Gln Gln Gly Asn Val Phe Ser Cys Ser Val Met His Glu Ala
                   245                 250                 255
   Leu His Asn His Tyr Thr Gln Lys Ser Leu Ser Leu Ser Pro Gly Lys
               260                 265                 270
   His His His His His His
           275
<210> 168
   <211> 53
   <212> PRT
   <213> Homo Sapiens
<400> 168
   Glu Gln Ala Pro Gly Thr Ala Pro Cys Ser Arg Gly Ser Ser Trp Ser
   1               5                   10                  15
   Ala Asp Leu Asp Lys Cys Met Asp Cys Ala Ser Cys Arg Ala Arg Pro
               20                  25                  30
   His Ser Asp Phe Cys Leu Gly Cys Ala Ala Ala Pro Pro Ala Pro Phe
           35                  40                  45
   Arg Leu Leu Trp Pro
       50
<210> 169
   <211> 129
   <212> PRT
   <213> Homo Sapiens
<400> 169
   Met Ala Arg Gly Ser Leu Arg Arg Leu Leu Arg Leu Leu Val Leu Gly
   1               5                   10                  15
   Leu Trp Leu Ala Leu Leu Arg Ser Val Ala Gly Glu Gln Ala Pro Gly
               20                  25                  30
   Thr Ala Pro Cys Ser Arg Gly Ser Ser Trp Ser Ala Asp Leu Asp Lys
           35                  40                  45
   Cys Met Asp Cys Ala Ser Cys Arg Ala Arg Pro His Ser Asp Phe Cys
       50                  55                  60
   Leu Gly Cys Ala Ala Ala Pro Pro Ala Pro Phe Arg Leu Leu Trp Pro
   65                  70                  75                  80
   Ile Leu Gly Gly Ala Leu Ser Leu Thr Phe Val Leu Gly Leu Leu Ser
                   85                  90                  95
   Gly Phe Leu Val Trp Arg Arg Cys Arg Arg Arg Glu Lys Phe Thr Thr
               100                 105                 110
   Pro Ile Glu Glu Thr Gly Gly Glu Gly Cys Pro Ala Val Ala Leu Ile
           115                 120                 125
   Gln
<210> 170
   <211> 959
   <212> DNA
   <213> Homo Sapiens
<400> 170
<210> 171
   <211> 5
   <212> PRT
   <213> Homo Sapiens
<220>
   <221> SITE
   <222> 5..5
   <223> Xaa can be any naturally occurring amino acid
<400> 171
   Pro Tyr Pro Met Xaa
   1               5
<210> 172
   <211> 17
   <212> PRT
   <213> Homo Sapiens
<220>
   <221> SITE
   <222> 8..8
   <223> Xaa can be any naturally occurring amino acid
<400> 172
   Tyr Ile Ser Pro Ser Gly Gly Xaa Thr His Tyr Ala Asp Ser Val Lys
   1               5                   10                  15
   Gly
<210> 173
   <211> 11
   <212> PRT
   <213> Homo Sapiens
<400> 173
   Gly Gly Asp Thr Tyr Phe Asp Tyr Phe Asp Tyr
   1               5                   10
<210> 174
   <211> 11
   <212> PRT
   <213> Homo Sapiens
<220>
   <221> SITE
   <222> 8..8
   <223> Xaa can be any naturally occurring amino acid
<400> 174
   Arg Ala Ser Gln Ser Ile Ser Xaa Tyr Leu Asn
   1               5                   10
<210> 175
   <211> 7
   <212> PRT
   <213> Homo Sapiens
<220>
   <221> SITE
   <222> 1..1
   <223> Xaa can be any naturally occurring amino acid
<400> 175
   Xaa Ala Ser Ser Leu Gln Ser
   1               5
<210> 176
   <211> 10
   <212> PRT
   <213> Homo Sapiens
<220>
   <221> SITE
   <222> 5..6
   <223> Xaa can be any naturally occurring amino acid
<220>
   <221> SITE
   <222> 8..8
   <223> Xaa can be any naturally occurring amino acid
<400> 176
   Gln Gln Ser Tyr Xaa Xaa Pro Xaa Ile Thr
   1               5                   10
<210> 177
   <211> 645
   <212> DNA
   <213> Homo Sapiens
<400> 177
   gacatccaga tgacccagag ccccagcagc ctgagcgcct ccgtgggcga cagagtgacc      60
   atcacctgtc gggccagcca gagcatcagc ggctacctga actggtatca gcagaagccc     120
   ggcaaggccc ccaagctgct gatctaccag gccagctccc tgcagagcgg cgtgccaagc     180
   agattcagcg gcagcggctc cggcaccgac ttcaccctga ccatcagcag cctgcagccc 240
   gaggacttcg ccacctacta ctgccagcag agctacacca gccccttcat caccttcggc 300
   cagggcacca aggtggaaat caagcggacc gtggccgctc ccagcgtgtt catcttccca 360
   cccagcgacg agcagctgaa gtccggcaca gccagcgtgg tctgcctgct gaacaacttc 420
   tacccccgcg aggccaaggt gcagtggaag gtggacaacg ccctgcagtc cggcaactcc 480
   caggaaagcg tgaccgagca ggacagcaag gactccacct acagcctgag cagcaccctg 540
   accctgagca aggccgacta cgagaagcac aaggtgtacg cctgcgaagt gacccaccag 600
   ggcctgtcca gccccgtgac caagagcttc aaccggggcg agtgc 645
<210> 178
   <211> 1347
   <212> DNA
   <213> Homo Sapiens
<400> 178
   gaagttcaat tgttagagtc cggcggaggc ctggtgcagc ctggcggcag cctgagactg 60
   tcttgcgccg ccagcggctt cacattcagc ccctacccca tgatctgggt ccgccaggct 120
   ccaggcaagg gcctggaatg ggtgtcctac atcagcccca gcggcggcag cacccactac 180
   gccgatagcg tgaagggccg gttcaccatc agccgggaca acagcaagaa caccctgtac 240
   ctgcagatga acagcctgcg ggccgaggac accgccgtgt actattgcgc cagaggcggc 300
   gacacctact tcgattactt cgactactgg ggccagggca ccctggtgac agtgtccagc 360
   gcctccacca agggcccatc ggtcttcccg ctagcaccca gcagcaagag caccagcggc 420
   ggaacagccg ccctgggctg cctggtgaaa gactacttcc ccgagcccgt gaccgtgtcc 480
   tggaactctg gcgccctgac cagcggagtg cataccttcc ccgccgtgct gcagagcagc     540
   ggcctgtaca gcctgagcag cgtggtgaca gtgcccagca gcagcctggg aacccagacc 600
   tacatctgca acgtgaacca caagcccagc aacaccaagg tggacaagaa ggtggaaccc 660
   aagagctgcg acaagaccca cacctgtccc ccctgccctg cccctgaact gctgggcgga 720
   cccagcgtgt tcctgttccc cccaaagccc aaggacaccc tgatgatcag ccggaccccc 780
   gaagtgacct gcgtggtggt ggacgtgtcc cacgaggacc cagaagtgaa gtttaattgg 840
   tacgtggacg gcgtggaagt gcataacgcc aagaccaagc ccagagagga acagtacaac 900
   agcacctacc gggtggtgtc cgtgctgacc gtgctgcacc aggactggct gaacggcaaa 960
   gagtacaagt gcaaggtctc caacaaggcc ctgcctgccc ccatcgagaa aaccatcagc 1020
   aaggccaagg gccagccccg cgagcctcag gtgtacacac tgccccccag ccgggatgag 1080
   ctgaccaaga accaggtgtc cctgacctgt ctggtgaaag gcttctaccc cagcgatatc 1140
   gccgtggaat gggagagcaa cggccagccc gagaacaatt acaagaccac cccccctgtg 1200
   ctggacagcg acggctcatt cttcctgtac tccaagctga ccgtggacaa gagccggtgg 1260
   cagcagggca acgtgttcag ctgcagcgtg atgcacgagg ccctgcacaa tcactacacc 1320
   cagaagtccc tgagcctgag ccccggc 1347
<210> 179
   <211> 645
   <212> DNA
   <213> Homo Sapiens
<400> 179
   gacatccaga tgacccagtc tccagccacc ctgtctgcat ctgtaggaga cagagtcacc 60
   atcacttgcc gggcaagtca gagcattagc ggctatttaa attggtatca gcagaaacca 120
   gggaaagccc ctaagctcct gatctatcag gcatccagtt tgcaaagtgg ggtcccatca 180
   aggttcagtg gcagtggatc tgggacagat ttcactctca ccatcagcag tctgcaacct 240
   gaagattttg caacttacta ctgtcaacag agctacacta gtccattcat cactttcggc 300
   cctgggacca aggtggagat caaacgaact gtggctgcac catctgtctt catcttcccg 360
   ccatctgatg agcagttgaa atctggaact gcctctgttg tgtgcctgct gaataacttc 420
   tatcccagag aggccaaagt acagtggaag gtggataacg ccctccaatc gggtaactcc 480
   caggagagtg tcacagagca ggacagcaag gacagcacct acagcctcag cagcaccctg 540
   acgctgagca aagcagacta cgagaaacac aaactctacg cctgcgaagt cacccatcag 600
   ggcctgagct cgcccgtcac aaagagcttc aacaggggag agtgt 645
<210> 180
   <211> 1347
   <212> DNA
   <213> Homo Sapiens
<400> 180
   gaagttcaat tgttagagtc tggtggcggt cttgttcagc ctggtggttc tttacgtctt 60
   tcttgcgctg cttccggatt cactttctct ccttacccta tgatctgggt tcgccaagct 120
   cctggtaaag gtttggagtg ggtttcttat atctctcctt ctggtggcaa gactcattat 180
   gctgactccg ttaaaggtcg cttcactatc tctagagaca actctaagaa tactctctac 240
   ttgcagatga acagcttaag ggctgaggac acggccgtgt attactgtgc gagagggggt 300
   gatacttatt tcgactactt tgactactgg ggccagggaa ccctggtcac cgtctcaagc 360
   gcctccacca agggcccatc ggtcttcccg ctagcaccca gcagcaagag caccagcggc 420
   ggaacagccg ccctgggctg cctggtgaaa gactacttcc ccgagcccgt gaccgtgtcc 480
   tggaactctg gcgccctgac cagcggagtg cataccttcc ccgccgtgct gcagagcagc 540
   ggcctgtaca gcctgagcag cgtggtgaca gtgcccagca gcagcctggg aacccagacc 600
   tacatctgca acgtgaacca caagcccagc aacaccaagg tggacaagaa ggtggaaccc 660
   aagagctgcg acaagaccca cacctgtccc ccctgccctg cccctgaact gctgggcgga 720
   cccagcgtgt tcctgttccc cccaaagccc aaggacaccc tgatgatcag ccggaccccc 780
   gaagtgacct gcgtggtggt ggacgtgtcc cacgaggacc cagaagtgaa gtttaattgg 840
   tacgtggacg gcgtggaagt gcataacgcc aagaccaagc ccagagagga acagtacaac 900
   agcacctacc gggtggtgtc cgtgctgacc gtgctgcacc aggactggct gaacggcaaa 960
   gagtacaagt gcaaggtctc caacaaggcc ctgcctgccc ccatcgagaa aaccatcagc 1020
   aaggccaagg gccagccccg cgagcctcag gtgtacacac tgccccccag ccgggatgag 1080
   ctgaccaaga accaggtgtc cctgacctgt ctggtgaaag gcttctaccc cagcgatatc 1140
   gccgtggaat gggagagcaa cggccagccc gagaacaatt acaagaccac cccccctgtg 1200
   ctggacagcg acggctcatt cttcctgtac tccaagctga ccgtggacaa gagccggtgg 1260
   cagcagggca acgtgttcag ctgcagcgtg atgcacgagg ccctgcacaa tcactacacc 1320
   cagaagtccc tgagcctgag ccccggc 1347
<210> 181
   <211> 645
   <212> DNA
   <213> Homo Sapiens
<400> 181
   gacatccaga tgacccagag ccccagcagc ctgagcgcct ccgtgggcga cagagtgacc 60
   atcacctgtc gggccagcca gagcatcagc agctacctga actggtatca gcagaagccc 120
   ggcaaggccc ccaagctgct gatctaccag gccagctccc tgcagagcgg cgtgccaagc 180
   agattcagcg gcagcggctc cggcaccgac ttcaccctga ccatcagcag cctgcagccc 240
   gaggacttcg ccacctacta ctgccagcag agctacacca gccccttcat caccttcggc 300
   cagggcacca aggtggaaat caagcggacc gtggccgctc ccagcgtgtt catcttccca 360
   cccagcgacg agcagctgaa gtccggcaca gccagcgtgg tctgcctgct gaacaacttc 420
   tacccccgcg aggccaaggt gcagtggaag gtggacaacg ccctgcagtc cggcaactcc 480
   caggaaagcg tgaccgagca ggacagcaag gactccacct acagcctgag cagcaccctg 540
   accctgagca aggccgacta cgagaagcac aaggtgtacg cctgcgaagt gacccaccag 600
   ggcctgtcca gccccgtgac caagagcttc aaccggggcg agtgc 645
<210> 182
   <211> 1347
   <212> DNA
   <213> Homo Sapiens
<400> 182
   gaagttcaat tgttagagtc cggcggaggc ctggtgcagc ctggcggcag cctgagactg 60
   tcttgcgccg ccagcggctt cacattcagc ccctacccca tgatgtgggt ccgccaggct 120
   ccaggcaagg gcctggaatg ggtgtcctac atcagcccca gcggcggcag cacccactac 180
   gccgatagcg tgaagggccg gttcaccatc agccgggaca acagcaagaa caccctgtac 240
   ctgcagatga acagcctgcg ggccgaggac accgccgtgt actattgcgc cagaggcggc 300
   gacacctact tcgattactt cgactactgg ggccagggca ccctggtgac agtgtccagc 360
   gcctccacca agggcccatc ggtcttcccg ctagcaccca gcagcaagag caccagcggc 420
   ggaacagccg ccctgggctg cctggtgaaa gactacttcc ccgagcccgt gaccgtgtcc 480
   tggaactctg gcgccctgac cagcggagtg cataccttcc ccgccgtgct gcagagcagc 540
   ggcctgtaca gcctgagcag cgtggtgaca gtgcccagca gcagcctggg aacccagacc 600
   tacatctgca acgtgaacca caagcccagc aacaccaagg tggacaagaa ggtggaaccc 660
   aagagctgcg acaagaccca cacctgtccc ccctgccctg cccctgaact gctgggcgga 720
   cccagcgtgt tcctgttccc cccaaagccc aaggacaccc tgatgatcag ccggaccccc 780
   gaagtgacct gcgtggtggt ggacgtgtcc cacgaggacc cagaagtgaa gtttaattgg 840
   tacgtggacg gcgtggaagt gcataacgcc aagaccaagc ccagagagga acagtacaac 900
   agcacctacc gggtggtgtc cgtgctgacc gtgctgcacc aggactggct gaacggcaaa 960
   gagtacaagt gcaaggtctc caacaaggcc ctgcctgccc ccatcgagaa aaccatcagc 1020
   aaggccaagg gccagccccg cgagcctcag gtgtacacac tgccccccag ccgggatgag 1080
   ctgaccaaga accaggtgtc cctgacctgt ctggtgaaag gcttctaccc cagcgatatc 1140
   gccgtggaat gggagagcaa cggccagccc gagaacaatt acaagaccac cccccctgtg 1200
   ctggacagcg acggctcatt cttcctgtac tccaagctga ccgtggacaa gagccggtgg 1260
   cagcagggca acgtgttcag ctgcagcgtg atgcacgagg ccctgcacaa tcactacacc 1320
   cagaagtccc tgagcctgag ccccggc 1347
<210> 183
   <211> 645
   <212> DNA
   <213> Homo Sapiens
<400> 183
   gacatccaga tgacccagtc tccagccacc ctgtctgcat ctgtaggaga cagagtcacc 60
   atcacttgcc gggcaagtca gagcattagc agctatttaa attggtatca gcagaaacca 120
   gggaaagccc ctaagctcct gatctatcag gcatccagtt tgcaaagtgg ggtcccatca 180
   aggttcagtg gcagtggatc tgggacagat ttcactctca ccatcagcag tctgcaacct 240
   gaagattttg caacttacta ctgtcaacag agctacacta gtccattcat cactttcggc 300
   cctgggacca aggtggagat caaacgaact gtggctgcac catctgtctt catcttcccg 360
   ccatctgatg agcagttgaa atctggaact gcctctgttg tgtgcctgct gaataacttc 420
   tatcccagag aggccaaagt acagtggaag gtggataacg ccctccaatc gggtaactcc 480
   caggagagtg tcacagagca ggacagcaag gacagcacct acagcctcag cagcaccctg 540
   acgctgagca aagcagacta cgagaaacac aaactctacg cctgcgaagt cacccatcag 600
   ggcctgagct cgcccgtcac aaagagcttc aacaggggag agtgt 645
<210> 184
   <211> 1347
   <212> DNA
   <213> Homo Sapiens
<400> 184
   gaagttcaat tgttagagtc tggtggcggt cttgttcagc ctggtggttc tttacgtctt      60
   tcttgcgctg cttccggatt cactttctct ccttacccta tgatgtgggt tcgccaagct     120
   cctggtaaag gtttggagtg ggtttcttat atctctcctt ctggtggcaa gactcattat     180
   gctgactccg ttaaaggtcg cttcactatc tctagagaca actctaagaa tactctctac     240
   ttgcagatga acagcttaag ggctgaggac acggccgtgt attactgtgc gagagggggt     300
   gatacttatt tcgactactt tgactactgg ggccagggaa ccctggtcac cgtctcaagc     360
   gcctccacca agggcccatc ggtcttcccg ctagcaccca gcagcaagag caccagcggc     420
   ggaacagccg ccctgggctg cctggtgaaa gactacttcc ccgagcccgt gaccgtgtcc 480
   tggaactctg gcgccctgac cagcggagtg cataccttcc ccgccgtgct gcagagcagc 540
   ggcctgtaca gcctgagcag cgtggtgaca gtgcccagca gcagcctggg aacccagacc     600
   tacatctgca acgtgaacca caagcccagc aacaccaagg tggacaagaa ggtggaaccc     660
   aagagctgcg acaagaccca cacctgtccc ccctgccctg cccctgaact gctgggcgga     720
   cccagcgtgt tcctgttccc cccaaagccc aaggacaccc tgatgatcag ccggaccccc     780
   gaagtgacct gcgtggtggt ggacgtgtcc cacgaggacc cagaagtgaa gtttaattgg     840
   tacgtggacg gcgtggaagt gcataacgcc aagaccaagc ccagagagga acagtacaac     900
   agcacctacc gggtggtgtc cgtgctgacc gtgctgcacc aggactggct gaacggcaaa     960
   gagtacaagt gcaaggtctc caacaaggcc ctgcctgccc ccatcgagaa aaccatcagc    1020
   aaggccaagg gccagccccg cgagcctcag gtgtacacac tgccccccag ccgggatgag    1080
   ctgaccaaga accaggtgtc cctgacctgt ctggtgaaag gcttctaccc cagcgatatc    1140
   gccgtggaat gggagagcaa cggccagccc gagaacaatt acaagaccac cccccctgtg    1200
   ctggacagcg acggctcatt cttcctgtac tccaagctga ccgtggacaa gagccggtgg    1260
   cagcagggca acgtgttcag ctgcagcgtg atgcacgagg ccctgcacaa tcactacacc    1320
   cagaagtccc tgagcctgag ccccggc                                        1347
<210> 185
   <211> 645
   <212> DNA
   <213> Homo Sapiens
<400> 185
   gacatccaga tgacccagag ccccagcagc ctgtctgcca gcgtgggcga cagagtgacc 60
   atcacctgta gagccagcca gagcatcagc agctacctga actggtatca gcagaagccc 120
   ggcaaggccc ccaagctgct gatctatgcc gccagctctc tgcagagcgg agtgcccagc 180
   agattttctg gcagcggcag cggcaccgac ttcaccctga caatcagcag cctgcagccc 240
   gaggacttcg ccacctacta ctgccagcag agctacagca cccccggcat cacatttggc 300
   cagggcacca aggtggaaat caagcggaca gtggccgctc ccagcgtgtt catcttccca 360
   cctagcgacg agcagctgaa gtccggcaca gccagcgtcg tgtgcctgct gaacaacttc 420
   tacccccgcg aggccaaggt gcagtggaag gtggacaatg ccctgcagtc cggcaactcc 480
   caggaaagcg tcaccgagca ggacagcaag gactccacct acagcctgag cagcaccctg 540
   accctgagca aggccgacta cgagaagcac aaggtgtacg cctgcgaagt gacccaccag 600
   ggcctgtcta gccccgtgac caagagcttc aaccggggcg agtgt 645
<210> 186
   <211> 1347
   <212> DNA
   <213> Homo Sapiens
<400> 186
   gaagttcaat tgttagagtc cggcggaggc ctggtgcagc ctggcggatc tctgagactg 60
   agctgtgccg ccagcggctt caccttcagc ccctacccta tgatgtgggt ccgacaggcc 120
   cctggcaagg gactggaatg ggtgtcctac atctctccca gcggcggcag cacccactac 180
   gccgattctg tgaagggccg gttcaccatc agccgggaca acagcaagaa caccctgtac 240
   ctgcagatga acagcctgcg ggccgaggac accgccgtgt actattgtgc cagaggcggc 300
   gacacctact tcgattactt cgactactgg ggccagggca ccctggtcac cgtgtcatct 360
   gcctccacca agggcccatc ggtcttcccg ctagcaccca gcagcaagag caccagcggc 420
   ggaacagccg ccctgggctg cctggtgaaa gactacttcc ccgagcccgt gaccgtgtcc 480
   tggaactctg gcgccctgac cagcggagtg cataccttcc ccgccgtgct gcagagcagc 540
   ggcctgtaca gcctgagcag cgtggtgaca gtgcccagca gcagcctggg aacccagacc 600
   tacatctgca acgtgaacca caagcccagc aacaccaagg tggacaagaa ggtggaaccc 660
   aagagctgcg acaagaccca cacctgtccc ccctgccctg cccctgaact gctgggcgga 720
   cccagcgtgt tcctgttccc cccaaagccc aaggacaccc tgatgatcag ccggaccccc 780
   gaagtgacct gcgtggtggt ggacgtgtcc cacgaggacc cagaagtgaa gtttaattgg 840
   tacgtggacg gcgtggaagt gcataacgcc aagaccaagc ccagagagga acagtacaac 900
   agcacctacc gggtggtgtc cgtgctgacc gtgctgcacc aggactggct gaacggcaaa 960
   gagtacaagt gcaaggtctc caacaaggcc ctgcctgccc ccatcgagaa aaccatcagc 1020
   aaggccaagg gccagccccg cgagcctcag gtgtacacac tgccccccag ccgggatgag 1080
   ctgaccaaga accaggtgtc cctgacctgt ctggtgaaag gcttctaccc cagcgatatc 1140
   gccgtggaat gggagagcaa cggccagccc gagaacaatt acaagaccac cccccctgtg 1200
   ctggacagcg acggctcatt cttcctgtac tccaagctga ccgtggacaa gagccggtgg 1260
   cagcagggca acgtgttcag ctgcagcgtg atgcacgagg ccctgcacaa tcactacacc 1320
   cagaagtccc tgagcctgag ccccggc 1347
<210> 187
   <211> 645
   <212> DNA
   <213> Homo Sapiens
<400> 187
   gacatccaga tgacccagtc tccagccacc ctgtctgcat ctgtaggaga cagagtcacc 60
   atcacttgcc gggcaagtca gagcattagc agctatttaa attggtatca gcagaaacca 120
   gggaaagccc ctaagctcct gatctatgct gcatccagtt tgcaaagtgg ggtcccatca 180
   aggttcagtg gcagtggatc tgggacagat ttcactctca ccatcagcag tctgcaacct 240
   gaagattttg caacttacta ctgtcaacag agctactcta gtccagggat cactttcggc 300
   cctgggacca aggtggagat caaacgaact gtggctgcac catctgtctt catcttcccg 360
   ccatctgatg agcagttgaa atctggaact gcctctgttg tgtgcctgct gaataacttc 420
   tatcccagag aggccaaagt acagtggaag gtggataacg ccctccaatc gggtaactcc 480
   caggagagtg tcacagagca ggacagcaag gacagcacct acagcctcag cagcaccctg 540
   acgctgagca aagcagacta cgagaaacac aaactctacg cctgcgaagt cacccatcag 600
   ggcctgagct cgcccgtcac aaagagcttc aacaggggag agtgt 645
<210> 188
   <211> 1347
   <212> DNA
   <213> Homo Sapiens
<400> 188
   gaagttcaat tgttagagtc tggtggcggt cttgttcagc ctggtggttc tttacgtctt 60
   tcttgcgctg cttccggatt cactttctct ccttacccta tgatgtgggt tcgccaagct 120
   cctggtaaag gtttggagtg ggtttcttat atctctcctt ctggtggcaa gactcattat 180
   gctgactccg ttaaaggtcg cttcactatc tctagagaca actctaagaa tactctctac 240
   ttgcagatga acagcttaag ggctgaggac acggccgtgt attactgtgc gagagggggt 300
   gatacgtatt tcgactactt tgactactgg ggccagggaa ccctggtcac cgtctcaagc 360
   gcctccacca agggcccatc ggtcttcccg ctagcaccca gcagcaagag caccagcggc 420
   ggaacagccg ccctgggctg cctggtgaaa gactacttcc ccgagcccgt gaccgtgtcc 480
   tggaactctg gcgccctgac cagcggagtg cataccttcc ccgccgtgct gcagagcagc 540
   ggcctgtaca gcctgagcag cgtggtgaca gtgcccagca gcagcctggg aacccagacc 600
   tacatctgca acgtgaacca caagcccagc aacaccaagg tggacaagaa ggtggaaccc 660
   aagagctgcg acaagaccca cacctgtccc ccctgccctg cccctgaact gctgggcgga 720
   cccagcgtgt tcctgttccc cccaaagccc aaggacaccc tgatgatcag ccggaccccc 780
   gaagtgacct gcgtggtggt ggacgtgtcc cacgaggacc cagaagtgaa gtttaattgg 840
   tacgtggacg gcgtggaagt gcataacgcc aagaccaagc ccagagagga acagtacaac 900
   agcacctacc gggtggtgtc cgtgctgacc gtgctgcacc aggactggct gaacggcaaa 960
   gagtacaagt gcaaggtctc caacaaggcc ctgcctgccc ccatcgagaa aaccatcagc 1020
   aaggccaagg gccagccccg cgagcctcag gtgtacacac tgccccccag ccgggatgag 1080
   ctgaccaaga accaggtgtc cctgacctgt ctggtgaaag gcttctaccc cagcgatatc 1140
   gccgtggaat gggagagcaa cggccagccc gagaacaatt acaagaccac cccccctgtg 1200
   ctggacagcg acggctcatt cttcctgtac tccaagctga ccgtggacaa gagccggtgg 1260
   cagcagggca acgtgttcag ctgcagcgtg atgcacgagg ccctgcacaa tcactacacc 1320
   cagaagtccc tgagcctgag ccccggc 1347
<210> 189
   <211> 651
   <212> DNA
   <213> Homo Sapiens
<400> 189
   gcacaagaca tccagatgac ccagtctcca gccaccctgt ctgcatctgt aggagacaga 60
   gtcaccatca cttgccgggc aagtcagagc attagcagct atttaaattg gtatcagcag 120
   aaaccaggga aagcccctaa gctcctgatc tatgctgcat ccagtttgca aagtggggtc 180
   ccatcaaggt tcagtggcag tggatctggg acagatttca ctctcaccat cagcagtctg 240
   caacctgaag attttgcaac ttactactgt caacagagct actctagtcc agggatcact 300
   ttcggccctg ggaccaaggt ggagatcaaa cgaactgtgg ctgcaccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaac tctacgcctg cgaagtcacc 600
   catcagggcc tgagctcgcc cgtcacaaag agcttcaaca ggggagagtg t 651
<210> 190
   <211> 1347
   <212> DNA
   <213> Homo Sapiens
<400> 190
   gaagttcaat tgttagagtc tggtggcggt cttgttcagc ctggtggttc tttacgtctt 60
   tcttgcgctg cttccggatt cactttctct ccttacccta tgatgtgggt tcgccaagct 120
   cctggtaaag gtttggagtg ggtttcttat atctctcctt ctggtggcaa gactcattat 180
   gctgactccg ttaaaggtcg cttcactatc tctagagaca actctaagaa tactctctac 240
   ttgcagatga acagcttaag ggctgaggac acggccgtgt attactgtgc gagagggggt 300
   gatacgtatt tcgactactt tgactactgg ggccagggaa ccctggtcac cgtctcaagc 360
   gcctccacca agggcccatc ggtcttcccg ctagcaccca gcagcaagag caccagcggc 420
   ggaacagccg ccctgggctg cctggtgaaa gactacttcc ccgagcccgt gaccgtgtcc 480
   tggaactctg gcgccctgac cagcggagtg cataccttcc ccgccgtgct gcagagcagc 540
   ggcctgtaca gcctgagcag cgtggtgaca gtgcccagca gcagcctggg aacccagacc 600
   tacatctgca acgtgaacca caagcccagc aacaccaagg tggacaagcg ggtggaaccc 660
   aagagctgcg acaagaccca cacctgtccc ccctgccctg cccctgaact gctgggcgga 720
   cccagcgtgt tcctgttccc cccaaagccc aaggacaccc tgatgatcag ccggaccccc 780
   gaagtgacct gcgtggtggt ggacgtgtcc cacgaggacc cagaagtgaa gtttaattgg 840
   tacgtggacg gcgtggaagt gcataacgcc aagaccaagc ccagagagga acagtacaac 900
   agcacctacc gggtggtgtc cgtgctgacc gtgctgcacc aggactggct gaacggcaaa 960
   gagtacaagt gcaaggtctc caacaaggcc ctgcctgccc ccatcgagaa aaccatcagc 1020
   aaggccaagg gccagccccg cgagcctcag gtgtacacac tgccccccag ccgggaagag 1080
   atgaccaaga accaggtgtc cctgacctgt ctggtgaaag gcttctaccc cagcgatatc 1140
   gccgtggaat gggagagcaa cggccagccc gagaacaatt acaagaccac cccccctgtg 1200
   ctggacagcg acggctcatt cttcctgtac tccaagctga ccgtggacaa gagccggtgg 1260
   cagcagggca acgtgttcag ctgcagcgtg atgcacgagg ccctgcacaa tcactacacc 1320
   cagaagtccc tgagcctgag ccccggc 1347
<210> 191
   <211> 651
   <212> DNA
   <213> Homo Sapiens
<400> 191
   gcacaagaca tccagatgac ccagtctcca gccaccctgt ctgcatctgt aggagacaga      60
   gtcaccatca cttgccgggc aagtcagagc attagcagct atttaaattg gtatcagcag     120
   aaaccaggga aagcccctaa gctcctgatc tatgctgcat ccagtttgca aagtggggtc     180
   ccatcaaggt tcagtggcag tggatctggg acagatttca ctctcaccat cagcagtctg     240
   caacctgaag attttgcaac ttactactgt caacagagct actctagtcc agggatcact     300
   ttcggccctg ggaccaaggt ggagatcaaa cgaactgtgg ctgcaccatc tgtcttcatc     360
   ttcccgccat ctgatgagca gttgaaatct ggaactgcct ctgttgtgtg cctgctgaat     420
   aacttctatc ccagagaggc caaagtacag tggaaggtgg ataacgccct ccaatcgggt     480
   aactcccagg agagtgtcac agagcaggac agcaaggaca gcacctacag cctcagcagc     540
   accctgacgc tgagcaaagc agactacgag aaacacaaac tctacgcctg cgaagtcacc     600
   catcagggcc tgagctcgcc cgtcacaaag agcttcaaca ggggagagtg t              651
<210> 192
   <211> 1347
   <212> DNA
   <213> Homo Sapiens
<400> 192
   gaagttcaat tgttagagtc tggtggcggt cttgttcagc ctggtggttc tttacgtctt 60
   tcttgcgctg cttccggatt cactttctct ccttacccta tgatgtgggt tcgccaagct 120
   cctggtaaag gtttggagtg ggtttcttat atctctcctt ctggtggcaa gactcattat 180
   gctgactccg ttaaaggtcg cttcactatc tctagagaca actctaagaa tactctctac 240
   ttgcagatga acagcttaag ggctgaggac acggccgtgt attactgtgc gagagggggt 300
   gatggttatt tcgactactt tgactactgg ggccagggaa ccctggtcac cgtctcaagc 360
   gcctccacca agggcccatc ggtcttcccg ctagcaccca gcagcaagag caccagcggc 420
   ggaacagccg ccctgggctg cctggtgaaa gactacttcc ccgagcccgt gaccgtgtcc 480
   tggaactctg gcgccctgac cagcggagtg cataccttcc ccgccgtgct gcagagcagc 540
   ggcctgtaca gcctgagcag cgtggtgaca gtgcccagca gcagcctggg aacccagacc 600
   tacatctgca acgtgaacca caagcccagc aacaccaagg tggacaagcg ggtggaaccc 660
   aagagctgcg acaagaccca cacctgtccc ccctgccctg cccctgaact gctgggcgga 720
   cccagcgtgt tcctgttccc cccaaagccc aaggacaccc tgatgatcag ccggaccccc 780
   gaagtgacct gcgtggtggt ggacgtgtcc cacgaggacc cagaagtgaa gtttaattgg 840
   tacgtggacg gcgtggaagt gcataacgcc aagaccaagc ccagagagga acagtacaac 900
   agcacctacc gggtggtgtc cgtgctgacc gtgctgcacc aggactggct gaacggcaaa 960
   gagtacaagt gcaaggtctc caacaaggcc ctgcctgccc ccatcgagaa aaccatcagc 1020
   aaggccaagg gccagccccg cgagcctcag gtgtacacac tgccccccag ccgggaagag 1080
   atgaccaaga accaggtgtc cctgacctgt ctggtgaaag gcttctaccc cagcgatatc 1140
   gccgtggaat gggagagcaa cggccagccc gagaacaatt acaagaccac cccccctgtg 1200
   ctggacagcg acggctcatt cttcctgtac tccaagctga ccgtggacaa gagccggtgg 1260
   cagcagggca acgtgttcag ctgcagcgtg atgcacgagg ccctgcacaa tcactacacc 1320
   cagaagtccc tgagcctgag ccccggc 1347
<210> 193
   <211> 651
   <212> DNA
   <213> Homo Sapiens
<400> 193
   gcacaagaca tccagatgac ccagtctcca gccaccctgt ctgcatctgt aggagacaga 60
   gtcaccatca cttgccgggc aagtcagagc attagcggct atttaaattg gtatcagcag 120
   aaaccaggga aagcccctaa gctcctgatc tataacgcat ccagtttgca aagtggggtc 180
   ccatcaaggt tcagtggcag tggatctggg acagatttca ctctcaccat cagcagtctg 240
   caacctgaag attttgcaac ttactactgt caacagagct acactagtcc attcatcact 300
   ttcggccctg ggaccaaggt ggagatcaaa cgaactgtgg ctgcaccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaac tctacgcctg cgaagtcacc 600
   catcagggcc tgagctcgcc cgtcacaaag agcttcaaca ggggagagtg t 651
<210> 194
   <211> 1347
   <212> DNA
   <213> Homo Sapiens
<400> 194
   gaagttcaat tgttagagtc tggtggcggt cttgttcagc ctggtggttc tttacgtctt 60
   tcttgcgctg cttccggatt cactttctct ccttacccta tgatctgggt tcgccaagct 120
   cctggtaaag gtttggagtg ggtttcttat atctctcctt ctggtggcaa gactcattat 180
   gctgactccg ttaaaggtcg cttcactatc tctagagaca actctaagaa tactctctac 240
   ttgcagatga acagcttaag ggctgaggac acggccgtgt attactgtgc gagagggggt 300
   gatacttatt tcgactactt tgactactgg ggccagggaa ccctggtcac cgtctcaagc 360
   gcctccacca agggcccatc ggtcttcccg ctagcaccca gcagcaagag caccagcggc 420
   ggaacagccg ccctgggctg cctggtgaaa gactacttcc ccgagcccgt gaccgtgtcc      480
   tggaactctg gcgccctgac cagcggagtg cataccttcc ccgccgtgct gcagagcagc      540
   ggcctgtaca gcctgagcag cgtggtgaca gtgcccagca gcagcctggg aacccagacc 600
   tacatctgca acgtgaacca caagcccagc aacaccaagg tggacaagaa ggtggaaccc 660
   aagagctgcg acaagaccca cacctgtccc ccctgccctg cccctgaact gctgggcgga 720
   cccagcgtgt tcctgttccc cccaaagccc aaggacaccc tgatgatcag ccggaccccc 780
   gaagtgacct gcgtggtggt ggacgtgtcc cacgaggacc cagaagtgaa gtttaattgg 840
   tacgtggacg gcgtggaagt gcataacgcc aagaccaagc ccagagagga acagtacaac 900
   agcacctacc gggtggtgtc cgtgctgacc gtgctgcacc aggactggct gaacggcaaa 960
   gagtacaagt gcaaggtctc caacaaggcc ctgcctgccc ccatcgagaa aaccatcagc 1020
   aaggccaagg gccagccccg cgagcctcag gtgtacacac tgccccccag ccgggatgag 1080
   ctgaccaaga accaggtgtc cctgacctgt ctggtgaaag gcttctaccc cagcgatatc 1140
   gccgtggaat gggagagcaa cggccagccc gagaacaatt acaagaccac cccccctgtg 1200
   ctggacagcg acggctcatt cttcctgtac tccaagctga ccgtggacaa gagccggtgg 1260
   cagcagggca acgtgttcag ctgcagcgtg atgcacgagg ccctgcacaa tcactacacc 1320
   cagaagtccc tgagcctgag ccccggc 1347
<210> 195
   <211> 651
   <212> DNA
   <213> Homo Sapiens
<400> 195
   gcacaagaca tccagatgac ccagtctcca gccaccctgt ctgcatctgt aggagacaga 60
   gtcaccatca cttgccgggc aagtcagagc attagcagct atttaaattg gtatcagcag 120
   aaaccaggga aagcccctaa gctcctgatc tataacgcat ccagtttgca aagtggggtc 180
   ccatcaaggt tcagtggcag tggatctggg acagatttca ctctcaccat cagcagtctg 240
   caacctgaag attttgcaac ttactactgt caacagagct acactagtcc agggatcact 300
   ttcggccctg ggaccaaggt ggagatcaaa cgaactgtgg ctgcaccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaac tctacgcctg cgaagtcacc 600
   catcagggcc tgagctcgcc cgtcacaaag agcttcaaca ggggagagtg t 651
<210> 196
   <211> 1347
   <212> DNA
   <213> Homo Sapiens
<400> 196
   gaagttcaat tgttagagtc tggtggcggt cttgttcagc ctggtggttc tttacgtctt      60
   tcttgcgctg cttccggatt cactttctct ccttacccta tgatgtgggt tcgccaagct     120
   cctggtaaag gtttggagtg ggtttcttat atctctcctt ctggtggcaa gactcattat     180
   gctgactccg ttaaaggtcg cttcactatc tctagagaca actctaagaa tactctctac     240
   ttgcagatga acagcttaag ggctgaggac acggccgtgt attactgtgc gagagggggt     300
   gatacttatt tcgactactt tgactactgg ggccagggaa ccctggtcac cgtctcaagc     360
   gcctccacca agggcccatc ggtcttcccg ctagcaccca gcagcaagag caccagcggc     420
   ggaacagccg ccctgggctg cctggtgaaa gactacttcc ccgagcccgt gaccgtgtcc     480
   tggaactctg gcgccctgac cagcggagtg cataccttcc ccgccgtgct gcagagcagc     540
   ggcctgtaca gcctgagcag cgtggtgaca gtgcccagca gcagcctggg aacccagacc     600
   tacatctgca acgtgaacca caagcccagc aacaccaagg tggacaagaa ggtggaaccc     660
   aagagctgcg acaagaccca cacctgtccc ccctgccctg cccctgaact gctgggcgga     720
   cccagcgtgt tcctgttccc cccaaagccc aaggacaccc tgatgatcag ccggaccccc     780
   gaagtgacct gcgtggtggt ggacgtgtcc cacgaggacc cagaagtgaa gtttaattgg     840
   tacgtggacg gcgtggaagt gcataacgcc aagaccaagc ccagagagga acagtacaac     900
   agcacctacc gggtggtgtc cgtgctgacc gtgctgcacc aggactggct gaacggcaaa     960
   gagtacaagt gcaaggtctc caacaaggcc ctgcctgccc ccatcgagaa aaccatcagc    1020
   aaggccaagg gccagccccg cgagcctcag gtgtacacac tgccccccag ccgggatgag     1080
   ctgaccaaga accaggtgtc cctgacctgt ctggtgaaag gcttctaccc cagcgatatc    1140
   gccgtggaat gggagagcaa cggccagccc gagaacaatt acaagaccac cccccctgtg    1200
   ctggacagcg acggctcatt cttcctgtac tccaagctga ccgtggacaa gagccggtgg    1260
   cagcagggca acgtgttcag ctgcagcgtg atgcacgagg ccctgcacaa tcactacacc    1320
   cagaagtccc tgagcctgag ccccggc                                        1347
<210> 197
   <211> 651
   <212> DNA
   <213> Homo Sapiens
<400> 197
   gcacaagaca tccagatgac ccagtctcca gccaccctgt ctgcatctgt aggagacaga 60
   gtcaccatca cttgccgggc aagtcagagc attagcggct atttaaattg gtatcagcag 120
   aaaccaggga aagcccctaa gctcctgatc tataacgcat ccagtttgca aagtggggtc 180
   ccatcaaggt tcagtggcag tggatctggg acagatttca ctctcaccat cagcagtctg 240
   caacctgaag attttgcaac ttactactgt caacagagct acactagtcc agggatcact 300
   ttcggccctg ggaccaaggt ggagatcaaa cgaactgtgg ctgcaccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaac tctacgcctg cgaagtcacc 600
   catcagggcc tgagctcgcc cgtcacaaag agcttcaaca ggggagagtg t 651
<210> 198
   <211> 1347
   <212> DNA
   <213> Homo Sapiens
<400> 198
   gaagttcaat tgttagagtc tggtggcggt cttgttcagc ctggtggttc tttacgtctt      60
   tcttgcgctg cttccggatt cactttctct ccttacccta tgatgtgggt tcgccaagct     120
   cctggtaaag gtttggagtg ggtttcttat atctctcctt ctggtggcaa gactcattat     180
   gctgactccg ttaaaggtcg cttcactatc tctagagaca actctaagaa tactctctac     240
   ttgcagatga acagcttaag ggctgaggac acggccgtgt attactgtgc gagagggggt     300
   gatacttatt tcgactactt tgactactgg ggccagggaa ccctggtcac cgtctcaagc     360
   gcctccacca agggcccatc ggtcttcccg ctagcaccca gcagcaagag caccagcggc     420
   ggaacagccg ccctgggctg cctggtgaaa gactacttcc ccgagcccgt gaccgtgtcc     480
   tggaactctg gcgccctgac cagcggagtg cataccttcc ccgccgtgct gcagagcagc     540
   ggcctgtaca gcctgagcag cgtggtgaca gtgcccagca gcagcctggg aacccagacc     600
   tacatctgca acgtgaacca caagcccagc aacaccaagg tggacaagaa ggtggaaccc     660
   aagagctgcg acaagaccca cacctgtccc ccctgccctg cccctgaact gctgggcgga     720
   cccagcgtgt tcctgttccc cccaaagccc aaggacaccc tgatgatcag ccggaccccc     780
   gaagtgacct gcgtggtggt ggacgtgtcc cacgaggacc cagaagtgaa gtttaattgg     840
   tacgtggacg gcgtggaagt gcataacgcc aagaccaagc ccagagagga acagtacaac     900
   agcacctacc gggtggtgtc cgtgctgacc gtgctgcacc aggactggct gaacggcaaa     960
   gagtacaagt gcaaggtctc caacaaggcc ctgcctgccc ccatcgagaa aaccatcagc    1020
   aaggccaagg gccagccccg cgagcctcag gtgtacacac tgccccccag ccgggatgag     1080
   ctgaccaaga accaggtgtc cctgacctgt ctggtgaaag gcttctaccc cagcgatatc    1140
   gccgtggaat gggagagcaa cggccagccc gagaacaatt acaagaccac cccccctgtg    1200
   ctggacagcg acggctcatt cttcctgtac tccaagctga ccgtggacaa gagccggtgg    1260
   cagcagggca acgtgttcag ctgcagcgtg atgcacgagg ccctgcacaa tcactacacc    1320
   cagaagtccc tgagcctgag ccccggc                                        1347
<210> 199
   <211> 651
   <212> DNA
   <213> Homo Sapiens
<400> 199
   gcacaagaca tccagatgac ccagtctcca gccaccctgt ctgcatctgt aggagacaga      60
   gtcaccatca cttgccgggc aagtcagagc attagcagct atttaaattg gtatcagcag     120
   aaaccaggga aagcccctaa gctcctgatc tataacgcat ccagtttgca aagtggggtc     180
   ccatcaaggt tcagtggcag tggatctggg acagatttca ctctcaccat cagcagtctg     240
   caacctgaag attttgcaac ttactactgt caacagagct acactagtcc attcatcact     300
   ttcggccctg ggaccaaggt ggagatcaaa cgaactgtgg ctgcaccatc tgtcttcatc     360
   ttcccgccat ctgatgagca gttgaaatct ggaactgcct ctgttgtgtg cctgctgaat     420
   aacttctatc ccagagaggc caaagtacag tggaaggtgg ataacgccct ccaatcgggt     480
   aactcccagg agagtgtcac agagcaggac agcaaggaca gcacctacag cctcagcagc     540
   accctgacgc tgagcaaagc agactacgag aaacacaaac tctacgcctg cgaagtcacc     600
   catcagggcc tgagctcgcc cgtcacaaag agcttcaaca ggggagagtg t              651
<210> 200
   <211> 1347
   <212> DNA
   <213> Homo Sapiens
<400> 200
   gaagttcaat tgttagagtc tggtggcggt cttgttcagc ctggtggttc tttacgtctt      60
   tcttgcgctg cttccggatt cactttctct ccttacccta tgatgtgggt tcgccaagct     120
   cctggtaaag gtttggagtg ggtttcttat atctctcctt ctggtggcaa gactcattat     180
   gctgactccg ttaaaggtcg cttcactatc tctagagaca actctaagaa tactctctac     240
   ttgcagatga acagcttaag ggctgaggac acggccgtgt attactgtgc gagagggggt     300
   gatacttatt tcgactactt tgactactgg ggccagggaa ccctggtcac cgtctcaagc     360
   gcctccacca agggcccatc ggtcttcccg ctagcaccca gcagcaagag caccagcggc     420
   ggaacagccg ccctgggctg cctggtgaaa gactacttcc ccgagcccgt gaccgtgtcc     480
   tggaactctg gcgccctgac cagcggagtg cataccttcc ccgccgtgct gcagagcagc     540
   ggcctgtaca gcctgagcag cgtggtgaca gtgcccagca gcagcctggg aacccagacc     600
   tacatctgca acgtgaacca caagcccagc aacaccaagg tggacaagaa ggtggaaccc 660
   aagagctgcg acaagaccca cacctgtccc ccctgccctg cccctgaact gctgggcgga 720
   cccagcgtgt tcctgttccc cccaaagccc aaggacaccc tgatgatcag ccggaccccc 780
   gaagtgacct gcgtggtggt ggacgtgtcc cacgaggacc cagaagtgaa gtttaattgg 840
   tacgtggacg gcgtggaagt gcataacgcc aagaccaagc ccagagagga acagtacaac 900
   agcacctacc gggtggtgtc cgtgctgacc gtgctgcacc aggactggct gaacggcaaa 960
   gagtacaagt gcaaggtctc caacaaggcc ctgcctgccc ccatcgagaa aaccatcagc 1020
   aaggccaagg gccagccccg cgagcctcag gtgtacacac tgccccccag ccgggatgag 1080
   ctgaccaaga accaggtgtc cctgacctgt ctggtgaaag gcttctaccc cagcgatatc 1140
   gccgtggaat gggagagcaa cggccagccc gagaacaatt acaagaccac cccccctgtg 1200
   ctggacagcg acggctcatt cttcctgtac tccaagctga ccgtggacaa gagccggtgg 1260
   cagcagggca acgtgttcag ctgcagcgtg atgcacgagg ccctgcacaa tcactacacc 1320
   cagaagtccc tgagcctgag ccccggc 1347
<210> 201
   <211> 654
   <212> DNA
   <213> Mus Musculus
<400> 201
   gatatcgtgc tgacacagtc tcccgccagc ctggccgtgt ctctcggcca gagagccacc 60
   atcagctgcc gggccaacaa gagcgtgtcc accagcagct acagctacat gcactggtat 120
   cagcagaagc ccggccagcc ccccaagctg ctgattaagt acgccagcaa cctggaaagc 180
   ggcgtgcccg ccagattcag cggcagcggc tctggcaccg acttcatcct gaacatccac 240
   cccgtggaag aagaggacgc cgccacctac tactgccagc acagcagaga gctgcccttc 300
   accttcggca gcggcaccaa gctggaaatc aagcgggccg atgccgcccc taccgtgtcc 360
   atcttcccac ccagcagcga gcagctgacc agcggcggag ccagcgtcgt gtgcttcctg 420
   aacaacttct accccaagga catcaacgtg aagtggaaga tcgacggcag cgagcggcag 480
   aacggcgtgc tgaacagctg gaccgaccag gacagcaagg actccaccta cagcatgagc 540
   agcaccctga ccctgaccaa ggacgagtac gagcggcaca acagctacac atgcgaggcc 600
   acccacaaga ccagcaccag ccccatcgtg aagtccttca accggaacga gtgc 654
<210> 202
   <211> 1350
   <212> DNA
   <213> Mus Musculus
<400> 202
   caggtgcagc tgcagcagtc tggccctgaa gtcgtgcggc ctggcgtgtc cgtgaagatc 60
   agctgcaagg gcagcggcta caccttcacc gactacggca tccactgggt caagcagagc 120
   cacgccaaga gcctggaatg gatcggcgtg atcagcacct acaacggcta caccaactac 180
   aaccagaagt tcaagggcaa ggccaccatg accgtggaca agagcagcag caccgcctac 240
   atggaactgg cccggctgac cagcgaggac agcgccatct actactgcgc cagagcctac 300
   tacggcaacc tgtactacgc catggactac tggggccagg gcaccagcgt gaccgtgtcc 360
   tctgccaaga ccaccgcccc tagcgtgtac cctctggccc ctgtgtgtgg cgacaccacc 420
   ggcagctctg tgactctggg ctgcctggtc aagggctact tccccgagcc cgtgacactg 480
   acctggaaca gcggcagcct gagcagcggc gtgcacacct ttccagccgt gctgcagagc 540
   gacctgtaca ccctgagcag ctccgtgacc gtgacaagca gcacctggcc cagccagagc 600
   atcacctgta acgtggccca ccccgccagc agcaccaagg tggacaagaa gatcgagccc 660
   agaggcccca ccatcaagcc ctgcccccct tgcaagtgcc cagcccccaa tctgctgggc 720
   ggacccagcg tgttcatctt cccacccaag atcaaggacg tgctgatgat cagcctgagc 780
   cccatcgtga cctgcgtggt ggtggacgtg tccgaggacg accccgacgt gcagatcagt 840
   tggttcgtga acaacgtgga agtgcacacc gcccagaccc agacccacag agaggactac 900
   aacagcaccc tgcgggtggt gtccgccctg cccatccagc accaggactg gatgagcggc 960
   aaagaattca agtgcaaagt gaacaacaag gacctgcctg cccccatcga gcggaccatc 1020
   agcaagccca agggcagcgt gcgggctccc caggtgtacg tgctgccccc acccgaggaa 1080
   gagatgacca agaagcaggt cacactgacc tgcatggtca ccgacttcat gcccgaggac 1140
   atctacgtgg aatggaccaa caacggcaag accgagctga actacaagaa caccgagcct 1200
   gtgctggaca gcgacggcag ctacttcatg tacagcaagc tgcgggtgga aaagaaaaac 1260
   tgggtggaac ggaacagcta cagctgcagc gtggtgcacg agggcctgca caaccaccac 1320
   accaccaaga gcttcagccg gacccccggc 1350
<210> 203
   <211> 654
   <212> DNA
   <213> Mus Musculus
<400> 203
   gatatcgtgc tgacacagag ccccgccagc ctgaccgtgt ctctcggcca gagagccacc 60
   atcagctgcc gggccagcca gagcgtgtcc accagcagct acagctacat gcagtggtat 120
   cagcagcggc ctggccagcc ccccaagctg ctgattaagt acgccaccaa cctggacagc 180
   ggcgtgcccg ccagattttc tggcagcggc agcggcacag acttcaccct gaacatccac 240
   cccgtggaag aagaggacgc cgccacctac tactgccagc acagctggga gatcccttac 300
   accttcggcg gaggcaccaa gctggaaatc aagcgggccg atgccgcccc taccgtgtcc 360
   atcttcccac ccagcagcga gcagctgacc agcggcggag ccagcgtcgt gtgcttcctg 420
   aacaacttct accccaagga catcaacgtg aagtggaaga tcgacggcag cgagcggcag 480
   aacggcgtgc tgaacagctg gaccgaccag gacagcaagg actccaccta cagcatgagc 540
   agcaccctga ccctgaccaa ggacgagtac gagcggcaca acagctacac atgcgaggcc 600
   acccacaaga ccagcaccag ccccatcgtg aagtccttca accggaacga gtgc 654
<210> 204
   <211> 1344
   <212> DNA
   <213> Mus Musculus
<400> 204
   gaagtgaagc tggaagagtc tggcggcgga ctggtccagc ctggcggcag catgaagctg      60
   agctgcgtgg ccagcggctt caccttcaac aactactgga tgagctgggt ccgacagagc     120
   cccgagaagg gcctggaatg gctggccgag atccggctga agtccgacaa ctacgccacc     180
   cactacgccg agagcgtgaa gggcaagttc accatcagcc gggacgacag caagagccgg     240
   ctgtacctgc agatgaacaa cctgcgggcc gagaacaccg gcatctacta ctgcaccggc     300
   ggcttcgccg actacttcga ctactggggc cagggcacca ccctgaccgt gtcctctgcc     360
   aagaccaccg cccctagcgt gtaccctctg gcccctgtgt gtggcgacac caccggcagc     420
   tctgtgactc tgggctgcct ggtcaagggc tacttccccg agcccgtgac actgacctgg     480
   aacagcggca gcctgagcag cggcgtgcac acctttccag ccgtgctgca gagcgacctg     540
   tacaccctga gcagctccgt gaccgtgaca agcagcacct ggcccagcca gagcatcacc     600
   tgtaacgtgg cccaccccgc cagcagcacc aaggtggaca agaagatcga gcccagaggc     660
   cccaccatca agccctgccc cccttgcaag tgcccagccc ccaatctgct gggcggaccc     720
   agcgtgttca tcttcccacc caagatcaag gacgtgctga tgatcagcct gagccccatc     780
   gtgacctgcg tggtggtgga cgtgtccgag gacgaccccg acgtgcagat cagttggttc     840
   gtgaacaacg tggaagtgca caccgcccag acccagaccc acagagagga ctacaacagc     900
   accctgcggg tggtgtccgc cctgcccatc cagcaccagg actggatgag cggcaaagaa     960
   ttcaagtgca aagtgaacaa caaggacctg cctgccccca tcgagcggac catcagcaag    1020
   cccaagggca gcgtgcgggc tccccaggtg tacgtgctgc ccccacccga ggaagagatg    1080
   accaagaagc aggtcacact gacctgcatg gtcaccgact tcatgcccga ggacatctac    1140
   gtggaatgga ccaacaacgg caagaccgag ctgaactaca agaacaccga gcctgtgctg    1200
   gacagcgacg gcagctactt catgtacagc aagctgcggg tggaaaagaa aaactgggtg    1260
   gaacggaaca gctacagctg cagcgtggtg cacgagggcc tgcacaacca ccacaccacc    1320
   aagagcttca gccggacccc cggc                                           1344
<210> 205
   <211> 654
   <212> DNA
   <213> Homo Sapiens
<400> 205
   gacattgtgc tgacacagtc tcctgcttcc ctggctgtat ctctggggca gagggccacc      60
   atctcatgca gggccagcaa aagtgtcagt acatctagct atagttatat gcactggtac     120
   caacagaaac caggacagcc acccaaactc ctcatcaaat atgcatccaa cctagaatct     180
   ggggtccctg ccaggttcag tggcagtggg tctgggacag acttctccct caacatccat     240
   cccatggagg aggacgatac cgcaatgtat ttctgtcagc acagtaggga gcttccattc     300
   acgttcggcg gagggacaaa gttggaaata aaacgtacgg tggccgctcc cagcgtgttc     360
   atcttcccac ccagcgacga gcagctgaag tccggcaccg ccagcgtcgt gtgcctgctg     420
   aacaacttct acccccgcga ggccaaggtg cagtggaagg tggacaacgc cctgcagagc     480
   ggcaacagcc aggaaagcgt caccgagcag gacagcaagg actccaccta cagcctgtcc     540
   agcaccctga ccctgagcaa ggccgactac gagaagcaca aggtgtacgc ctgcgaagtg     600
   acccaccagg gcctgagcag ccccgtgacc aagagcttca accggggcga gtgc           654
<210> 206
   <211> 1350
   <212> DNA
   <213> Homo Sapiens
<400> 206
   caggtccagc tggtgcagtc tggggctgag gtgaagaagc ctggggcctc agtgaaggtt      60
   tcctgcaagg gttccggcta cacattcact gattatggca tgcactgggt gcggcaggcc     120
   cctggacaag ggctagagtg gatgggagtt attagtactt acaatggtta tacaaactac     180
   aaccagaagt ttaagggcag agtcacaatg actgtagaca aatccacgag cacagcctat     240
   atggaacttc ggagcttgag atctgacgat acggccgtgt attactgtgc aagagcctac     300
   tatggcaacc tttactatgc tatggactac tggggtcaag gaaccctggt caccgtctcc     360
   tcagctagca ccaaaggccc gagcgtgttt ccgctggccc cgagcagcaa gagcaccagc     420
   ggcggaacag ccgccctggg ctgcctggtg aaagactact tccccgaacc ggtgaccgtg     480
   tcctggaact ctggcgccct gaccagcgga gtgcatacct tccccgccgt gctgcagagc     540
   agcggcctgt acagcctgag cagcgtggtg acagtgccca gcagcagcct gggaacccag     600
   acctacatct gcaacgtgaa ccacaagccc agcaacacca aggtggacaa gaaggtggaa     660
   cccaagagct gcgacaagac ccacacctgt cccccctgcc ctgcccctga actgctgggc     720
   ggacccagcg tgttcctgtt ccccccaaag cccaaggaca ccctgatgat cagccggacc     780
   cccgaagtga cctgcgtggt ggtggacgtg tcccacgagg acccagaagt gaagtttaat 840
   tggtacgtgg acggcgtgga agtgcataac gccaagacca agcccagaga ggaacagtac 900
   aacagcacct accgggtggt gtccgtgctg accgtgctgc accaggactg gctgaacggc 960
   aaagagtaca agtgcaaggt ctccaacaag gccctgcctg cccccatcga gaaaaccatc 1020
   agcaaggcca agggccagcc ccgcgagcct caggtgtaca cactgccccc cagccgggat 1080
   gagctgacca agaaccaggt gtccctgacc tgtctggtga aaggcttcta ccccagcgat 1140
   atcgccgtgg aatgggagag caacggccag cccgagaaca attacaagac caccccccct 1200
   gtgctggaca gcgacggctc attcttcctg tactccaagc tgaccgtgga caagagccgg 1260
   tggcagcagg gcaacgtgtt cagctgcagc gtgatgcacg aggccctgca caatcactac 1320
   acccagaagt ccctgagcct gagccccggc 1350
<210> 207
   <211> 654
   <212> DNA
   <213> Homo Sapiens
<400> 207
   gatatccaga tgacccagag ccccagcagc ctgagcgcca gcgtgggcga cagagtgacc 60
   atcacctgtc gggccagcca gagcgtgtcc accagcagct acagctacat gcactggtat 120
   cagcagaagc ccggcaaggc ccccaagctg ctgattaagt acgccagcaa cctggaaagc 180
   ggcgtgccca gccggtttag cggctctggc agcggcaccg acttcaccct gaccatcagc 240
   agtctgcagc ccgaggactt cgccacctac tactgccagc acagctggga gatcccttac 300
   accttcggcg gaggcaccaa ggtggaaatc aagcgtacgg tggctgcacc atctgtcttc 360
   atcttcccgc catctgatga gcagttgaaa tctggaactg cctctgttgt gtgcctgctg 420
   aataacttct atcccagaga ggccaaagta cagtggaagg tggataacgc cctccaatcg 480
   ggtaactccc aggagagtgt cacagagcag gacagcaagg acagcaccta cagcctcagc 540
   agcaccctga cgctgtctaa agcagactac gagaaacaca aagtctacgc ctgcgaagtc 600
   acccatcagg gcctgagctc gcccgtcaca aagagcttca acaggggaga gtgt 654
<210> 208
   <211> 1347
   <212> DNA
   <213> Homo Sapiens
<400> 208
   caggtggaat tggtggaaag cggcggaggc ctggtgcagc ctggcggaag cctgagactg 60
   agctgtgccg ccagcggctt caccttcagc agctactgga tgagctgggt ccgacaggct 120
   ccaggcaagg gcctggaatg ggtggccgag atccggctga agtccgacaa ctacgccacc 180
   cactacgccg agagcgtgaa gggccggttc accatcagcc gggacgacag caagaacagc 240
   ctgtacctgc agatgaacag cctgcgggcc gaggacaccg ccgtgtacta ctgcaccggc 300
   tactacgccg acgccatgga ctactggggc cagggcaccc tggtcaccgt cagctcagcc 360
   tccaccaagg gtccatcggt cttccccctg gcaccctcct ccaagagcac ctctgggggc 420
   acagcggccc tgggctgcct ggtcaaggac tacttccccg aaccggtgac ggtgtcgtgg 480
   aactcaggcg ccctgaccag cggcgtgcac accttcccgg ctgtcctaca gtcctcagga 540
   ctctactccc tcagcagcgt ggtgaccgtg ccctccagca gcttgggcac ccagacctac 600
   atctgcaacg tgaatcacaa gcccagcaac accaaggtgg acaagcgggt tgagcccaaa 660
   tcttgtgaca aaactcacac atgcccaccg tgcccagcac ctgaactcct ggggggaccg 720
   tcagtcttcc tcttcccccc aaaacccaag gacaccctca tgatctcccg gacccctgag 780
   gtcacatgcg tggtggtgga cgtgagccac gaagaccctg aggtcaagtt caactggtac 840
   gtggacggcg tggaggtgca taatgccaag acaaagccgc gggaggagca gtacaacagc 900
   acgtaccggg tggtcagcgt cctcaccgtc ctgcaccagg actggctgaa tggcaaggag 960
   tacaagtgca aggtctccaa caaagccctc ccagccccca tcgagaaaac catctccaaa 1020
   gccaaagggc agccccgaga accacaggtg tacaccctgc ccccatcccg ggaagagatg 1080
   accaagaacc aggtcagcct gacctgcctg gtcaaaggct tctatcccag cgacatcgcc 1140
   gtggagtggg agagcaatgg gcagccggag aacaactaca agaccacgcc tcccgtgctg 1200
   gactccgacg gctccttctt cctctacagc aagctcaccg tggacaagag caggtggcag 1260
   caggggaacg tcttctcatg ctccgtgatg catgaggctc tgcacaacca ctacacgcag 1320
   aagagcctct ccctgtctcc gggtaaa 1347
<210> 209
   <211> 654
   <212> DNA
   <213> Mus Musculus
<400> 209
   gatatcgtgc tgacacagtc tcccgccagc ctggccgtgt ctctcggcca gagagccacc 60
   atcagctgca aggccagcca gagcgtgtcc accagcacct acagctacat gcagtggtat 120
   cagcagcggc ctggacagag ccccaagctg ctgattaagt acgccagcaa gctggacagc 180
   ggcgtgcccg ccagattttc tggcagcggc agcggcaccg acttcaccct gaacatccac 240
   cccgtggaag aagaggacac cgccacctac tactgccagc acagctggga gctgccctac 300
   accttcggcg gaggcacccg gctggaaatc aagagggccg atgccgcccc taccgtgtcc 360
   atcttcccac ccagcagcga gcagctgacc agcggcggag ccagcgtcgt gtgcttcctg 420
   aacaacttct accccaagga catcaacgtg aagtggaaga tcgacggcag cgagcggcag 480
   aacggcgtgc tgaacagctg gaccgaccag gacagcaagg actccaccta cagcatgagc 540
   agcaccctga ccctgaccaa ggacgagtac gagcggcaca acagctacac atgcgaggcc 600
   acccacaaga ccagcaccag ccccatcgtg aagtccttca accggaacga gtgc 654
<210> 210
   <211> 1344
   <212> DNA
   <213> Mus Musculus
<400> 210
   gaagtgaagc tgggagagtc tggcggcgga ctggtccagc ctggcggcag catgaagctg      60
   agctgcgtgg ccagcggctt cccattcacc aaatactgga tgaactgggt ccgacagagc     120
   cccgagaagg gcctggaatg ggtggccgag atccggctga agtccgacaa ctacgccacc     180
   cactacgccg agagcgccaa gggccggttc accatcagcc gggacgacag ccggtccagc     240
   gtgtacctgc agatgaacaa cctgcgggcc gaggacaccg ccatctacta ctgcagcccc     300
   acctatgccg acaccatgga ctactggggc cagggcacca gcgtgacagt gtccagcgcc     360
   aagaccaccg cccctagcgt gtaccctctg gcccctgtgt gtggcgacac caccggcagc     420
   tctgtgactc tgggctgcct ggtcaagggc tacttccccg agcccgtgac actgacctgg     480
   aacagcggca gcctgagcag cggcgtgcac acctttccag ccgtgctgca gagcgacctg     540
   tacaccctga gcagctccgt gaccgtgaca agcagcacct ggcccagcca gagcatcacc     600
   tgtaacgtgg cccaccccgc cagcagcacc aaggtggaca agaagatcga gcccagaggc     660
   cccaccatca agccctgccc cccttgcaag tgcccagccc ccaatctgct gggcggaccc     720
   agcgtgttca tcttcccacc caagatcaag gacgtgctga tgatcagcct gagccccatc     780
   gtgacctgcg tggtggtgga cgtgtccgag gacgaccccg acgtgcagat cagttggttc     840
   gtgaacaacg tggaagtgca caccgcccag acccagaccc acagagagga ctacaacagc     900
   accctgcggg tggtgtccgc cctgcccatc cagcaccagg actggatgag cggcaaagaa     960
   ttcaagtgca aagtgaacaa caaggacctg cctgccccca tcgagcggac catcagcaag    1020
   cccaagggca gcgtgcgggc tccccaggtg tacgtgctgc ccccacccga ggaagagatg    1080
   accaagaagc aggtcacact gacctgcatg gtcaccgact tcatgcccga ggacatctac    1140
   gtggaatgga ccaacaacgg caagaccgag ctgaactaca agaacaccga gcctgtgctg    1200
   gacagcgacg gcagctactt catgtacagc aagctgcggg tggaaaagaa aaactgggtg    1260
   gaacggaaca gctacagctg cagcgtggtg cacgagggcc tgcacaacca ccacaccacc    1320
   aagagcttca gccggacccc cggc                                           1344
<210> 211
   <211> 654
   <212> DNA
   <213> Mus Musculus
<400> 211
   gatatcgtgc tgacacagtc tcccgccagc ctggccgtgt ctctcggcca gagagccacc      60
   atcagctgcc gggccagcaa gagcgtgtcc accagcagct acagctacat gcactggtat     120
   cagcagaagc ccggccagcc ccccaagctg ctgatcaagt acaccagcaa cctggaaagc     180
   ggcgtgcccg ccagattcag cggaagcggc tccggcaccg acttcatcct gaacatccac     240
   cccgtggaag aagaggacgc cgccacctac tactgccagc acagcagaga gctgccctgg     300
   accttcggcg gaggcaccaa gctggaaatc aagcgggccg atgccgcccc taccgtgtcc     360
   atcttcccac ccagcagcga gcagctgacc agcggcggag ccagcgtcgt gtgcttcctg     420
   aacaacttct accccaagga catcaacgtg aagtggaaga tcgacggcag cgagcggcag     480
   aacggcgtgc tgaacagctg gaccgaccag gacagcaagg actccaccta cagcatgagc     540
   agcaccctga ccctgaccaa ggacgagtac gagcggcaca acagctacac atgcgaggcc     600
   acccacaaga ccagcaccag ccccatcgtg aagtccttca accggaacga gtgc           654
<210> 212
   <211> 1353
   <212> DNA
   <213> Mus Musculus
<400> 212
   caggtgtccc tgaaagagag cggccctggc atcctgcagc ctagccagac cctgagcctg      60
   acctgcagct tcagcggctt cagcctgagc accagcggca tgggcgtgtc ctggatcaga     120
   cagcccagcg gcaagggcct ggaatggctg gcccacatct actgggacga cgacaagcgg     180
   tacaacccca gcctgaagtc ccggctgacc atctccaagg acaccagccg gaatcaggtg     240
   ttcctgaaga tcaccagcgt ggacaccgcc gataccgcca cctactactg cgccagaaga     300
   ggccccgact actacggcta ctaccccatg gactattggg gccagggcac cagcgtgacc     360
   gtgtctgcca agaccaccgc ccctagcgtg taccctctgg cccctgtgtg tggcgacacc     420
   accggcagct ctgtgactct gggctgcctg gtcaagggct acttccccga gcccgtgaca     480
   ctgacctgga acagcggcag cctgagcagc ggcgtgcaca cctttccagc cgtgctgcag     540
   agcgacctgt acaccctgag cagctccgtg accgtgacaa gcagcacctg gcccagccag     600
   agcatcacct gtaacgtggc ccaccccgcc agcagcacca aggtggacaa gaagatcgag     660
   cccagaggcc ccaccatcaa gccctgcccc ccttgcaagt gcccagcccc caatctgctg     720
   ggcggaccca gcgtgttcat cttcccaccc aagatcaagg acgtgctgat gatcagcctg     780
   agccccatcg tgacctgcgt ggtggtggac gtgtccgagg acgaccccga cgtgcagatc     840
   agttggttcg tgaacaacgt ggaagtgcac accgcccaga cccagaccca cagagaggac     900
   tacaacagca ccctgcgggt ggtgtccgcc ctgcccatcc agcaccagga ctggatgagc     960
   ggcaaagaat tcaagtgcaa agtgaacaac aaggacctgc ctgcccccat cgagcggacc 1020
   atcagcaagc ccaagggcag cgtgcgggct ccccaggtgt acgtgctgcc cccacccgag 1080
   gaagagatga ccaagaagca ggtcacactg acctgcatgg tcaccgactt catgcccgag 1140
   gacatctacg tggaatggac caacaacggc aagaccgagc tgaactacaa gaacaccgag 1200
   cctgtgctgg acagcgacgg cagctacttc atgtacagca agctgcgggt ggaaaagaaa 1260
   aactgggtgg aacggaacag ctacagctgc agcgtggtgc acgagggcct gcacaaccac 1320
   cacaccacca agagcttcag ccggaccccc ggc 1353
<210> 213
   <211> 1210
   <212> PRT
   <213> Homo
<400> 213
<210> 214
   <211> 622
   <212> PRT
   <213> Homo sapiens
<400> 214
   Met Ala Leu Pro Thr Ala Arg Pro Leu Leu Gly Ser Cys Gly Thr Pro
   1               5                   10                  15
   Ala Leu Gly Ser Leu Leu Phe Leu Leu Phe Ser Leu Gly Trp Val Gln
               20                  25                  30
   Pro Ser Arg Thr Leu Ala Gly Glu Thr Gly Gln Glu Ala Ala Pro Leu
           35                  40                  45
   Asp Gly Val Leu Ala Asn Pro Pro Asn Ile Ser Ser Leu Ser Pro Arg
       50                  55                  60
   Gln Leu Leu Gly Phe Pro Cys Ala Glu Val Ser Gly Leu Ser Thr Glu
   65                  70                  75                  80
   Arg Val Arg Glu Leu Ala Val Ala Leu Ala Gln Lys Asn Val Lys Leu
                   85                  90                  95
   Ser Thr Glu Gln Leu Arg Cys Leu Ala His Arg Leu Ser Glu Pro Pro
               100                 105                 110
   Glu Asp Leu Asp Ala Leu Pro Leu Asp Leu Leu Leu Phe Leu Asn Pro
           115                 120                 125
   Asp Ala Phe Ser Gly Pro Gln Ala Cys Thr Arg Phe Phe Ser Arg Ile
       130                 135                 140
   Thr Lys Ala Asn Val Asp Leu Leu Pro Arg Gly Ala Pro Glu Arg Gln
   145                 150                 155                 160
   Arg Leu Leu Pro Ala Ala Leu Ala Cys Trp Gly Val Arg Gly Ser Leu
                   165                 170                 175
   Leu Ser Glu Ala Asp Val Arg Ala Leu Gly Gly Leu Ala Cys Asp Leu
               180                 185                 190
   Pro Gly Arg Phe Val Ala Glu Ser Ala Glu Val Leu Leu Pro Arg Leu
           195                 200                 205
   Val Ser Cys Pro Gly Pro Leu Asp Gln Asp Gln Gln Glu Ala Ala Arg
       210                 215                 220
   Ala Ala Leu Gln Gly Gly Gly Pro Pro Tyr Gly Pro Pro Ser Thr Trp
   225                 230                 235                 240
   Ser Val Ser Thr Met Asp Ala Leu Arg Gly Leu Leu Pro Val Leu Gly
                   245                 250                 255
   Gln Pro Ile Ile Arg Ser Ile Pro Gln Gly Ile Val Ala Ala Trp Arg
               260                 265                 270
   Gln Arg Ser Ser Arg Asp Pro Ser Trp Arg Gln Pro Glu Arg Thr Ile
           275                 280                 285
   Leu Arg Pro Arg Phe Arg Arg Glu Val Glu Lys Thr Ala Cys Pro Ser
       290                 295                 300
   Gly Lys Lys Ala Arg Glu Ile Asp Glu Ser Leu Ile Phe Tyr Lys Lys
   305                 310                 315                 320
   Trp Glu Leu Glu Ala Cys Val Asp Ala Ala Leu Leu Ala Thr Gln Met
                   325                 330                 335
   Asp Arg Val Asn Ala Ile Pro Phe Thr Tyr Glu Gln Leu Asp Val Leu
               340                 345                 350
   Lys His Lys Leu Asp Glu Leu Tyr Pro Gln Gly Tyr Pro Glu Ser Val
           355                 360                 365
   Ile Gln His Leu Gly Tyr Leu Phe Leu Lys Met Ser Pro Glu Asp Ile
       370                 375                 380
   Arg Lys Trp Asn Val Thr Ser Leu Glu Thr Leu Lys Ala Leu Leu Glu
   385                 390                 395                 400
   Val Asn Lys Gly His Glu Met Ser Pro Gln Val Ala Thr Leu Ile Asp
                   405                 410                 415
   Arg Phe Val Lys Gly Arg Gly Gln Leu Asp Lys Asp Thr Leu Asp Thr
               420                 425                 430
   Leu Thr Ala Phe Tyr Pro Gly Tyr Leu Cys Ser Leu Ser Pro Glu Glu
           435                 440                 445
   Leu Ser Ser Val Pro Pro Ser Ser Ile Trp Ala Val Arg Pro Gln Asp
       450                 455                 460
   Leu Asp Thr Cys Asp Pro Arg Gln Leu Asp Val Leu Tyr Pro Lys Ala
   465                 470                 475                 480
   Arg Leu Ala Phe Gln Asn Met Asn Gly Ser Glu Tyr Phe Val Lys Ile
                   485                 490                 495
   Gln Ser Phe Leu Gly Gly Ala Pro Thr Glu Asp Leu Lys Ala Leu Ser
               500                 505                 510
   Gln Gln Asn Val Ser Met Asp Leu Ala Thr Phe Met Lys Leu Arg Thr
           515                 520                 525
   Asp Ala Val Leu Pro Leu Thr Val Ala Glu Val Gln Lys Leu Leu Gly
       530                 535                 540
   Pro His Val Glu Gly Leu Lys Ala Glu Glu Arg His Arg Pro Val Arg
   545                 550                 555                 560
   Asp Trp Ile Leu Arg Gln Arg Gln Asp Asp Leu Asp Thr Leu Gly Leu
                   565                 570                 575
   Gly Leu Gln Gly Gly Ile Pro Asn Gly Tyr Leu Val Leu Asp Leu Ser
               580                 585                 590
   Met Gln Glu Ala Leu Ser Gly Thr Pro Cys Leu Leu Gly Pro Gly Pro
           595                 600                 605
   Val Leu Thr Val Leu Ala Leu Leu Leu Ala Ser Thr Leu Ala
       610                 615                 620
<210> 215
   <211> 459
   <212> PRT
   <213> Homo sapiens
<400> 215
   Met Ala Pro Leu Cys Pro Ser Pro Trp Leu Pro Leu Leu Ile Pro Ala
   1               5                   10                  15
   Pro Ala Pro Gly Leu Thr Val Gln Leu Leu Leu Ser Leu Leu Leu Leu
               20                  25                  30
   Val Pro Val His Pro Gln Arg Leu Pro Arg Met Gln Glu Asp Ser Pro
           35                  40                  45
   Leu Gly Gly Gly Ser Ser Gly Glu Asp Asp Pro Leu Gly Glu Glu Asp
       50                  55                  60
   Leu Pro Ser Glu Glu Asp Ser Pro Arg Glu Glu Asp Pro Pro Gly Glu
   65                  70                  75                  80
   Glu Asp Leu Pro Gly Glu Glu Asp Leu Pro Gly Glu Glu Asp Leu Pro
                   85                  90                  95
   Glu Val Lys Pro Lys Ser Glu Glu Glu Gly Ser Leu Lys Leu Glu Asp
               100                 105                 110
   Leu Pro Thr Val Glu Ala Pro Gly Asp Pro Gln Glu Pro Gln Asn Asn
           115                 120                 125
   Ala His Arg Asp Lys Glu Gly Asp Asp Gln Ser His Trp Arg Tyr Gly
       130                 135                 140
   Gly Asp Pro Pro Trp Pro Arg Val Ser Pro Ala Cys Ala Gly Arg Phe
   145                 150                 155                 160
   Gln Ser Pro Val Asp Ile Arg Pro Gln Leu Ala Ala Phe Cys Pro Ala
                   165                 170                 175
   Leu Arg Pro Leu Glu Leu Leu Gly Phe Gln Leu Pro Pro Leu Pro Glu
               180                 185                 190
   Leu Arg Leu Arg Asn Asn Gly His Ser Val Gln Leu Thr Leu Pro Pro
           195                 200                 205
   Gly Leu Glu Met Ala Leu Gly Pro Gly Arg Glu Tyr Arg Ala Leu Gln
       210                 215                 220
   Leu His Leu His Trp Gly Ala Ala Gly Arg Pro Gly Ser Glu His Thr
   225                 230                 235                 240
   Val Glu Gly His Arg Phe Pro Ala Glu Ile His Val Val His Leu Ser
                   245                 250                 255
   Thr Ala Phe Ala Arg Val Asp Glu Ala Leu Gly Arg Pro Gly Gly Leu
               260                 265                 270
   Ala Val Leu Ala Ala Phe Leu Glu Glu Gly Pro Glu Glu Asn Ser Ala
           275                 280                 285
   Tyr Glu Gln Leu Leu Ser Arg Leu Glu Glu Ile Ala Glu Glu Gly Ser
       290                 295                 300
   Glu Thr Gln Val Pro Gly Leu Asp Ile Ser Ala Leu Leu Pro Ser Asp
   305                 310                 315                 320
   Phe Ser Arg Tyr Phe Gln Tyr Glu Gly Ser Leu Thr Thr Pro Pro Cys
                   325                 330                 335
   Ala Gln Gly Val Ile Trp Thr Val Phe Asn Gln Thr Val Met Leu Ser
               340                 345                 350
   Ala Lys Gln Leu His Thr Leu Ser Asp Thr Leu Trp Gly Pro Gly Asp
           355                 360                 365
   Ser Arg Leu Gln Leu Asn Phe Arg Ala Thr Gln Pro Leu Asn Gly Arg
       370                 375                 380
   Val Ile Glu Ala Ser Phe Pro Ala Gly Val Asp Ser Ser Pro Arg Ala
   385                 390                 395                 400
   Ala Glu Pro Val Gln Leu Asn Ser Cys Leu Ala Ala Gly Asp Ile Leu
                   405                 410                 415
   Ala Leu Val Phe Gly Leu Leu Phe Ala Val Thr Ser Val Ala Phe Leu
               420                 425                 430
   Val Gln Met Arg Arg Gln His Arg Arg Gly Thr Lys Gly Gly Val Ser
           435                 440                 445
   Tyr Arg Pro Ala Glu Val Ala Glu Thr Gly Ala
       450                 455
<210> 216
   <211> 346
   <212> PRT
   <213> Homo sapiens
<400> 216
<210> 217
   <211> 61
   <212> PRT
   <213> Homo sapiens
<400> 217
   Met Lys Arg Phe Leu Phe Leu Leu Leu Thr Ile Ser Leu Leu Val Met
   1               5                   10                  15
   Val Gln Ile Gln Thr Gly Leu Ser Gly Gln Asn Asp Thr Ser Gln Thr
               20                  25                  30
   Ser Ser Pro Ser Ala Ser Ser Asn Ile Ser Gly Gly Ile Phe Leu Phe
           35                  40                  45
   Phe Val Ala Asn Ala Ile Ile His Leu Phe Cys Phe Ser
       50                  55                  60
<210> 218
   <211> 1255
   <212> PRT
   <213> Homo sapiens
<400> 218
<210> 219
   <211> 297
   <212> PRT
   <213> Homo sapiens
<400> 219
   Met Thr Thr Pro Arg Asn Ser Val Asn Gly Thr Phe Pro Ala Glu Pro
   1               5                   10                  15
   Met Lys Gly Pro Ile Ala Met Gln Ser Gly Pro Lys Pro Leu Phe Arg
               20                  25                  30
   Arg Met Ser Ser Leu Val Gly Pro Thr Gln Ser Phe Phe Met Arg Glu
           35                  40                  45
   Ser Lys Thr Leu Gly Ala Val Gln Ile Met Asn Gly Leu Phe His Ile
       50                  55                  60
   Ala Leu Gly Gly Leu Leu Met Ile Pro Ala Gly Ile Tyr Ala Pro Ile
   65                  70                  75                  80
   Cys Val Thr Val Trp Tyr Pro Leu Trp Gly Gly Ile Met Tyr Ile Ile
                   85                  90                  95
   Ser Gly Ser Leu Leu Ala Ala Thr Glu Lys Asn Ser Arg Lys Cys Leu
               100                 105                 110
   Val Lys Gly Lys Met Ile Met Asn Ser Leu Ser Leu Phe Ala Ala Ile
           115                 120                 125
   Ser Gly Met Ile Leu Ser Ile Met Asp Ile Leu Asn Ile Lys Ile Ser
       130                 135                 140
   His Phe Leu Lys Met Glu Ser Leu Asn Phe Ile Arg Ala His Thr Pro
   145                 150                 155                 160
   Tyr Ile Asn Ile Tyr Asn Cys Glu Pro Ala Asn Pro Ser Glu Lys Asn
                   165                 170                 175
   Ser Pro Ser Thr Gln Tyr Cys Tyr Ser Ile Gln Ser Leu Phe Leu Gly
               180                 185                 190
   Ile Leu Ser Val Met Leu Ile Phe Ala Phe Phe Gln Glu Leu Val Ile
           195                 200                 205
   Ala Gly Ile Val Glu Asn Glu Trp Lys Arg Thr Cys Ser Arg Pro Lys
       210                 215                 220
   Ser Asn Ile Val Leu Leu Ser Ala Glu Glu Lys Lys Glu Gln Thr Ile
   225                 230                 235                 240
   Glu Ile Lys Glu Glu Val Val Gly Leu Thr Glu Thr Ser Ser Gln Pro
                   245                 250                 255
   Lys Asn Glu Glu Asp Ile Glu Ile Ile Pro Ile Gln Glu Glu Glu Glu
               260                 265                 270
   Glu Glu Thr Glu Thr Asn Phe Pro Glu Pro Pro Gln Asp Gln Glu Ser
           275                 280                 285
   Ser Pro Ile Glu Asn Asp Ser Ser Pro
       290                 295
<210> 220
   <211> 595
   <212> PRT
   <213> Homo sapiens
<400> 220
   Met Arg Val Leu Leu Ala Ala Leu Gly Leu Leu Phe Leu Gly Ala Leu
   1               5                   10                  15
   Arg Ala Phe Pro Gln Asp Arg Pro Phe Glu Asp Thr Cys His Gly Asn
               20                  25                  30
   Pro Ser His Tyr Tyr Asp Lys Ala Val Arg Arg Cys Cys Tyr Arg Cys
           35                  40                  45
   Pro Met Gly Leu Phe Pro Thr Gln Gln Cys Pro Gln Arg Pro Thr Asp
       50                  55                  60
   Cys Arg Lys Gln Cys Glu Pro Asp Tyr Tyr Leu Asp Glu Ala Asp Arg
   65                  70                  75                  80
   Cys Thr Ala Cys Val Thr Cys Ser Arg Asp Asp Leu Val Glu Lys Thr
                   85                  90                  95
   Pro Cys Ala Trp Asn Ser Ser Arg Val Cys Glu Cys Arg Pro Gly Met
               100                 105                 110
   Phe Cys Ser Thr Ser Ala Val Asn Ser Cys Ala Arg Cys Phe Phe His
           115                 120                 125
   Ser Val Cys Pro Ala Gly Met Ile Val Lys Phe Pro Gly Thr Ala Gln
       130                 135                 140
   Lys Asn Thr Val Cys Glu Pro Ala Ser Pro Gly Val Ser Pro Ala Cys
   145                 150                 155                 160
   Ala Ser Pro Glu Asn Cys Lys Glu Pro Ser Ser Gly Thr Ile Pro Gln
                   165                 170                 175
   Ala Lys Pro Thr Pro Val Ser Pro Ala Thr Ser Ser Ala Ser Thr Met
               180                 185                 190
   Pro Val Arg Gly Gly Thr Arg Leu Ala Gln Glu Ala Ala Ser Lys Leu
           195                 200                 205
   Thr Arg Ala Pro Asp Ser Pro Ser Ser Val Gly Arg Pro Ser Ser Asp
       210                 215                 220
   Pro Gly Leu Ser Pro Thr Gln Pro Cys Pro Glu Gly Ser Gly Asp Cys
   225                 230                 235                 240
   Arg Lys Gln Cys Glu Pro Asp Tyr Tyr Leu Asp Glu Ala Gly Arg Cys
                   245                 250                 255
   Thr Ala Cys Val Ser Cys Ser Arg Asp Asp Leu Val Glu Lys Thr Pro
               260                 265                 270
   Cys Ala Trp Asn Ser Ser Arg Thr Cys Glu Cys Arg Pro Gly Met Ile
           275                 280                 285
   Cys Ala Thr Ser Ala Thr Asn Ser Arg Ala Arg Cys Val Pro Tyr Pro
       290                 295                 300
   Ile Cys Ala Ala Glu Thr Val Thr Lys Pro Gln Asp Met Ala Glu Lys
   305                 310                 315                 320
   Asp Thr Thr Phe Glu Ala Pro Pro Leu Gly Thr Gln Pro Asp Cys Asn
                   325                 330                 335
   Pro Thr Pro Glu Asn Gly Glu Ala Pro Ala Ser Thr Ser Pro Thr Gln
               340                 345                 350
   Ser Leu Leu Val Asp Ser Gln Ala Ser Lys Thr Leu Pro Ile Pro Thr
           355                 360                 365
   Ser Ala Pro Val Ala Leu Ser Ser Thr Gly Lys Pro Val Leu Asp Ala
       370                 375                 380
   Gly Pro Val Leu Phe Trp Val Ile Leu Val Leu Val Val Val Val Gly
   385                 390                 395                 400
   Ser Ser Ala Phe Leu Leu Cys His Arg Arg Ala Cys Arg Lys Arg Ile
                   405                 410                 415
   Arg Gln Lys Leu His Leu Cys Tyr Pro Val Gln Thr Ser Gln Pro Lys
               420                 425                 430
   Leu Glu Leu Val Asp Ser Arg Pro Arg Arg Ser Ser Thr Gln Leu Arg
           435                 440                 445
   Ser Gly Ala Ser Val Thr Glu Pro Val Ala Glu Glu Arg Gly Leu Met
       450                 455                 460
   Ser Gln Pro Leu Met Glu Thr Cys His Ser Val Gly Ala Ala Tyr Leu
   465                 470                 475                 480
   Glu Ser Leu Pro Leu Gln Asp Ala Ser Pro Ala Gly Gly Pro Ser Ser
                   485                 490                 495
   Pro Arg Asp Leu Pro Glu Pro Arg Val Ser Thr Glu His Thr Asn Asn
               500                 505                 510
   Lys Ile Glu Lys Ile Tyr Ile Met Lys Ala Asp Thr Val Ile Val Gly
           515                 520                 525
   Thr Val Lys Ala Glu Leu Pro Glu Gly Arg Gly Leu Ala Gly Pro Ala
       530                 535                 540
   Glu Pro Glu Leu Glu Glu Glu Leu Glu Ala Asp His Thr Pro His Tyr
   545                 550                 555                 560
   Pro Glu Gln Glu Thr Glu Pro Pro Leu Gly Ser Cys Ser Asp Val Met
                   565                 570                 575
   Leu Ser Val Glu Glu Glu Gly Lys Glu Asp Pro Leu Pro Thr Ala Ala
               580                 585                 590
   Ser Gly Lys
           595
<210> 221
   <211> 847
   <212> PRT
   <213> Homo sapiens
<400> 221
   Met His Leu Leu Gly Pro Trp Leu Leu Leu Leu Val Leu Glu Tyr Leu
   1               5                   10                  15
   Ala Phe Ser Asp Ser Ser Lys Trp Val Phe Glu His Pro Glu Thr Leu
               20                  25                  30
   Tyr Ala Trp Glu Gly Ala Cys Val Trp Ile Pro Cys Thr Tyr Arg Ala
           35                  40                  45
   Leu Asp Gly Asp Leu Glu Ser Phe Ile Leu Phe His Asn Pro Glu Tyr
       50                  55                  60
   Asn Lys Asn Thr Ser Lys Phe Asp Gly Thr Arg Leu Tyr Glu Ser Thr
   65                  70                  75                  80
   Lys Asp Gly Lys Val Pro Ser Glu Gln Lys Arg Val Gln Phe Leu Gly
                   85                  90                  95
   Asp Lys Asn Lys Asn Cys Thr Leu Ser Ile His Pro Val His Leu Asn
               100                 105                 110
   Asp Ser Gly Gln Leu Gly Leu Arg Met Glu Ser Lys Thr Glu Lys Trp
           115                 120                 125
   Met Glu Arg Ile His Leu Asn Val Ser Glu Arg Pro Phe Pro Pro His
       130                 135                 140
   Ile Gln Leu Pro Pro Glu Ile Gln Glu Ser Gln Glu Val Thr Leu Thr
   145                 150                 155                 160
   Cys Leu Leu Asn Phe Ser Cys Tyr Gly Tyr Pro Ile Gln Leu Gln Trp
                   165                 170                 175
   Leu Leu Glu Gly Val Pro Met Arg Gln Ala Ala Val Thr Ser Thr Ser
               180                 185                 190
   Leu Thr Ile Lys Ser Val Phe Thr Arg Ser Glu Leu Lys Phe Ser Pro
           195                 200                 205
   Gln Trp Ser His His Gly Lys Ile Val Thr Cys Gln Leu Gln Asp Ala
       210                 215                 220
   Asp Gly Lys Phe Leu Ser Asn Asp Thr Val Gln Leu Asn Val Lys His
   225                 230                 235                 240
   Thr Pro Lys Leu Glu Ile Lys Val Thr Pro Ser Asp Ala Ile Val Arg
                   245                 250                 255
   Glu Gly Asp Ser Val Thr Met Thr Cys Glu Val Ser Ser Ser Asn Pro
               260                 265                 270
   Glu Tyr Thr Thr Val Ser Trp Leu Lys Asp Gly Thr Ser Leu Lys Lys
           275                 280                 285
   Gln Asn Thr Phe Thr Leu Asn Leu Arg Glu Val Thr Lys Asp Gln Ser
       290                 295                 300
   Gly Lys Tyr Cys Cys Gln Val Ser Asn Asp Val Gly Pro Gly Arg Ser
   305                 310                 315                 320
   Glu Glu Val Phe Leu Gln Val Gln Tyr Ala Pro Glu Pro Ser Thr Val
                   325                 330                 335
   Gln Ile Leu His Ser Pro Ala Val Glu Gly Ser Gln Val Glu Phe Leu
               340                 345                 350
   Cys Met Ser Leu Ala Asn Pro Leu Pro Thr Asn Tyr Thr Trp Tyr His
           355                 360                 365
   Asn Gly Lys Glu Met Gln Gly Arg Thr Glu Glu Lys Val His Ile Pro
       370                 375                 380
   Lys Ile Leu Pro Trp His Ala Gly Thr Tyr Ser Cys Val Ala Glu Asn
   385                 390                 395                 400
   Ile Leu Gly Thr Gly Gln Arg Gly Pro Gly Ala Glu Leu Asp Val Gln
                   405                 410                 415
   Tyr Pro Pro Lys Lys Val Thr Thr Val Ile Gln Asn Pro Met Pro Ile
               420                 425                 430
   Arg Glu Gly Asp Thr Val Thr Leu Ser Cys Asn Tyr Asn Ser Ser Asn
           435                 440                 445
   Pro Ser Val Thr Arg Tyr Glu Trp Lys Pro His Gly Ala Trp Glu Glu
       450                 455                 460
   Pro Ser Leu Gly Val Leu Lys Ile Gln Asn Val Gly Trp Asp Asn Thr
   465                 470                 475                 480
   Thr Ile Ala Cys Ala Ala Cys Asn Ser Trp Cys Ser Trp Ala Ser Pro
                   485                 490                 495
   Val Ala Leu Asn Val Gln Tyr Ala Pro Arg Asp Val Arg Val Arg Lys
               500                 505                 510
   Ile Lys Pro Leu Ser Glu Ile His Ser Gly Asn Ser Val Ser Leu Gln
           515                 520                 525
   Cys Asp Phe Ser Ser Ser His Pro Lys Glu Val Gln Phe Phe Trp Glu
           530                 535                 540
   Lys Asn Gly Arg Leu Leu Gly Lys Glu Ser Gln Leu Asn Phe Asp Ser
   545                 550                 555                 560
   Ile Ser Pro Glu Asp Ala Gly Ser Tyr Ser Cys Trp Val Asn Asn Ser
                   565                 570                 575
   Ile Gly Gln Thr Ala Ser Lys Ala Trp Thr Leu Glu Val Leu Tyr Ala
               580                 585                 590
   Pro Arg Arg Leu Arg Val Ser Met Ser Pro Gly Asp Gln Val Met Glu
           595                 600                 605
   Gly Lys Ser Ala Thr Leu Thr Cys Glu Ser Asp Ala Asn Pro Pro Val
       610                 615                 620
   Ser His Tyr Thr Trp Phe Asp Trp Asn Asn Gln Ser Leu Pro Tyr His
   625                 630                 635                 640
   Ser Gln Lys Leu Arg Leu Glu Pro Val Lys Val Gln His Ser Gly Ala
                   645                 650                 655
   Tyr Trp Cys Gln Gly Thr Asn Ser Val Gly Lys Gly Arg Ser Pro Leu
               660                 665                 670
   Ser Thr Leu Thr Val Tyr Tyr Ser Pro Glu Thr Ile Gly Arg Arg Val
           675                 680                 685
   Ala Val Gly Leu Gly Ser Cys Leu Ala Ile Leu Ile Leu Ala Ile Cys
       690                 695                 700
   Gly Leu Lys Leu Gln Arg Arg Trp Lys Arg Thr Gln Ser Gln Gln Gly
   705                 710                 715                 720
   Leu Gln Glu Asn Ser Ser Gly Gln Ser Phe Phe Val Arg Asn Lys Lys
                   725                 730                 735
   Val Arg Arg Ala Pro Leu Ser Glu Gly Pro His Ser Leu Gly Cys Tyr
               740                 745                 750
   Asn Pro Met Met Glu Asp Gly Ile Ser Tyr Thr Thr Leu Arg Phe Pro
           755                 760                 765
   Glu Met Asn Ile Pro Arg Thr Gly Asp Ala Glu Ser Ser Glu Met Gln
       770                 775                 780
   Arg Pro Pro Pro Asp Cys Asp Asp Thr Val Thr Tyr Ser Ala Leu His
   785                 790                 795                 800
   Lys Arg Gln Val Gly Asp Tyr Glu Asn Val Ile Pro Asp Phe Pro Glu
                   805                 810                 815
   Asp Glu Gly Ile His Tyr Ser Glu Leu Ile Gln Phe Gly Val Gly Glu
               820                 825                 830
   Arg Pro Gln Ala Gln Glu Asn Val Asp Tyr Val Ile Leu Lys His
           835                 840                 845
<210> 222
   <211> 364
   <212> PRT
   <213> Homo sapiens
<400> 222
   Met Pro Leu Leu Leu Leu Leu Pro Leu Leu Trp Ala Gly Ala Leu Ala
   1               5                   10                  15
   Met Asp Pro Asn Phe Trp Leu Gln Val Gln Glu Ser Val Thr Val Gln
               20                  25                  30
   Glu Gly Leu Cys Val Leu Val Pro Cys Thr Phe Phe His Pro Ile Pro
           35                  40                  45
   Tyr Tyr Asp Lys Asn Ser Pro Val His Gly Tyr Trp Phe Arg Glu Gly
       50                  55                  60
   Ala Ile Ile Ser Arg Asp Ser Pro Val Ala Thr Asn Lys Leu Asp Gln
   65                  70                  75                  80
   Glu Val Gln Glu Glu Thr Gln Gly Arg Phe Arg Leu Leu Gly Asp Pro
                   85                  90                  95
   Ser Arg Asn Asn Cys Ser Leu Ser Ile Val Asp Ala Arg Arg Arg Asp
               100                 105                 110
   Asn Gly Ser Tyr Phe Phe Arg Met Glu Arg Gly Ser Thr Lys Tyr Ser
           115                 120                 125
   Tyr Lys Ser Pro Gln Leu Ser Val His Val Thr Asp Leu Thr His Arg
       130                 135                 140
   Pro Lys Ile Leu Ile Pro Gly Thr Leu Glu Pro Gly His Ser Lys Asn
   145                 150                 155                 160
   Leu Thr Cys Ser Val Ser Trp Ala Cys Glu Gln Gly Thr Pro Pro Ile
                   165                 170                 175
   Phe Ser Trp Leu Ser Ala Ala Pro Thr Ser Leu Gly Pro Arg Thr Thr
               180                 185                 190
   His Ser Ser Val Leu Ile Ile Thr Pro Arg Pro Gln Asp His Gly Thr
           195                 200                 205
   Asn Leu Thr Cys Gln Val Lys Phe Ala Gly Ala Gly Val Thr Thr Glu
       210                 215                 220
   Arg Thr Ile Gln Leu Asn Val Thr Tyr Val Pro Gln Asn Pro Thr Thr
   225                 230                 235                 240
   Gly Ile Phe Pro Gly Asp Gly Ser Gly Lys Gln Glu Thr Arg Ala Gly
                   245                 250                 255
   Val Val His Gly Ala Ile Gly Gly Ala Gly Val Thr Ala Leu Leu Ala
               260                 265                 270
   Leu Cys Leu Cys Leu Ile Phe Phe Ile Val Lys Thr His Arg Arg Lys
           275                 280                 285
   Ala Ala Arg Thr Ala Val Gly Arg Asn Asp Thr His Pro Thr Thr Gly
       290                 295                 300
   Ser Ala Ser Pro Lys His Gln Lys Lys Ser Lys Leu His Gly Pro Thr
   305                 310                 315                 320
   Glu Thr Ser Ser Cys Ser Gly Ala Ala Pro Thr Val Glu Met Asp Glu
                   325                 330                 335
   Glu Leu His Tyr Ala Ser Leu Asn Phe His Gly Met Asn Pro Ser Lys
               340                 345                 350
   Asp Thr Ser Thr Glu Tyr Ser Glu Val Arg Thr Gln
           355                 360
<210> 223
   <211> 572
   <212> PRT
   <213> Homo sapiens
<400> 223
   Met Glu Cys Leu Tyr Tyr Phe Leu Gly Phe Leu Leu Leu Ala Ala Arg
   1               5                   10                  15
   Leu Pro Leu Asp Ala Ala Lys Arg Phe His Asp Val Leu Gly Asn Glu
               20                  25                  30
   Arg Pro Ser Ala Tyr Met Arg Glu His Asn Gln Leu Asn Gly Trp Ser
           35                  40                  45
   Ser Asp Glu Asn Asp Trp Asn Glu Lys Leu Tyr Pro Val Trp Lys Arg
       50                  55                  60
   Gly Asp Met Arg Trp Lys Asn Ser Trp Lys Gly Gly Arg Val Gln Ala
   65                  70                  75                  80
   Val Leu Thr Ser Asp Ser Pro Ala Leu Val Gly Ser Asn Ile Thr Phe
                   85                  90                  95
   Ala Val Asn Leu Ile Phe Pro Arg Cys Gln Lys Glu Asp Ala Asn Gly
               100                 105                 110
   Asn Ile Val Tyr Glu Lys Asn Cys Arg Asn Glu Ala Gly Leu Ser Ala
           115                 120                 125
   Asp Pro Tyr Val Tyr Asn Trp Thr Ala Trp Ser Glu Asp Ser Asp Gly
       130                 135                 140
   Glu Asn Gly Thr Gly Gln Ser His His Asn Val Phe Pro Asp Gly Lys
   145                 150                 155                 160
   Pro Phe Pro His His Pro Gly Trp Arg Arg Trp Asn Phe Ile Tyr Val
                   165                 170                 175
   Phe His Thr Leu Gly Gln Tyr Phe Gln Lys Leu Gly Arg Cys Ser Val
               180                 185                 190
   Arg Val Ser Val Asn Thr Ala Asn Val Thr Leu Gly Pro Gln Leu Met
           195                 200                 205
   Glu Val Thr Val Tyr Arg Arg His Gly Arg Ala Tyr Val Pro Ile Ala
       210                 215                 220
   Gln Val Lys Asp Val Tyr Val Val Thr Asp Gln Ile Pro Val Phe Val
   225                 230                 235                 240
   Thr Met Phe Gln Lys Asn Asp Arg Asn Ser Ser Asp Glu Thr Phe Leu
                   245                 250                 255
   Lys Asp Leu Pro Ile Met Phe Asp Val Leu Ile His Asp Pro Ser His
               260                 265                 270
   Phe Leu Asn Tyr Ser Thr Ile Asn Tyr Lys Trp Ser Phe Gly Asp Asn
           275                 280                 285
   Thr Gly Leu Phe Val Ser Thr Asn His Thr Val Asn His Thr Tyr Val
       290                 295                 300
   Leu Asn Gly Thr Phe Ser Leu Asn Leu Thr Val Lys Ala Ala Ala Pro
   305                 310                 315                 320
   Gly Pro Cys Pro Pro Pro Pro Pro Pro Pro Arg Pro Ser Lys Pro Thr
                   325                 330                 335
   Pro Ser Leu Ala Thr Thr Leu Lys Ser Tyr Asp Ser Asn Thr Pro Gly
               340                 345                 350
   Pro Ala Gly Asp Asn Pro Leu Glu Leu Ser Arg Ile Pro Asp Glu Asn
           355                 360                 365
   Cys Gln Ile Asn Arg Tyr Gly His Phe Gln Ala Thr Ile Thr Ile Val
       370                 375                 380
   Glu Gly Ile Leu Glu Val Asn Ile Ile Gln Met Thr Asp Val Leu Met
   385                 390                 395                 400
   Pro Val Pro Trp Pro Glu Ser Ser Leu Ile Asp Phe Val Val Thr Cys
                   405                 410                 415
   Gln Gly Ser Ile Pro Thr Glu Val Cys Thr Ile Ile Ser Asp Pro Thr
               420                 425                 430
   Cys Glu Ile Thr Gln Asn Thr Val Cys Ser Pro Val Asp Val Asp Glu
           435                 440                 445
   Met Cys Leu Leu Thr Val Arg Arg Thr Phe Asn Gly Ser Gly Thr Tyr
       450                 455                 460
   Cys Val Asn Leu Thr Leu Gly Asp Asp Thr Ser Leu Ala Leu Thr Ser
   465                 470                 475                 480
   Thr Leu Ile Ser Val Pro Asp Arg Asp Pro Ala Ser Pro Leu Arg Met
                   485                 490                 495
   Ala Asn Ser Ala Leu Ile Ser Val Gly Cys Leu Ala Ile Phe Val Thr
               500                 505                 510
   Val Ile Ser Leu Leu Val Tyr Lys Lys His Lys Glu Tyr Asn Pro Ile
           515                 520                 525
   Glu Asn Ser Pro Gly Asn Val Val Arg Ser Lys Gly Leu Ser Val Phe
       530                 535                 540
   Leu Asn Arg Ala Lys Ala Val Phe Phe Pro Gly Asn Gln Glu Lys Asp
   545                 550                 555                 560
   Pro Leu Leu Lys Asn Gln Glu Phe Lys Gly Val Ser
                   565                 570
<210> 224
   <211> 848
   <212> PRT
   <213> Homo sapiens
<400> 224
   Met Leu Gln Thr Lys Asp Leu Ile Trp Thr Leu Phe Phe Leu Gly Thr
   1               5                   10                  15
   Ala Val Ser Leu Gln Val Asp Ile Val Pro Ser Gln Gly Glu Ile Ser
               20                  25                  30
   Val Gly Glu Ser Lys Phe Phe Leu Cys Gln Val Ala Gly Asp Ala Lys
           35                  40                  45
   Asp Lys Asp Ile Ser Trp Phe Ser Pro Asn Gly Glu Lys Leu Thr Pro
       50                  55                  60
   Asn Gln Gln Arg Ile Ser Val Val Trp Asn Asp Asp Ser Ser Ser Thr
   65                  70                  75                  80
   Leu Thr Ile Tyr Asn Ala Asn Ile Asp Asp Ala Gly Ile Tyr Lys Cys
                           85                  90                  95
   Val Val Thr Gly Glu Asp Gly Ser Glu Ser Glu Ala Thr Val Asn Val
                   100                 105                 110
   Lys Ile Phe Gln Lys Leu Met Phe Lys Asn Ala Pro Thr Pro Gln Glu
               115                 120                 125
   Phe Arg Glu Gly Glu Asp Ala Val Ile Val Cys Asp Val Val Ser Ser
       130                 135                 140
   Leu Pro Pro Thr Ile Ile Trp Lys His Lys Gly Arg Asp Val Ile Leu
   145                 150                 155                 160
   Lys Lys Asp Val Arg Phe Ile Val Leu Ser Asn Asn Tyr Leu Gln Ile
                   165                 170                 175
   Arg Gly Ile Lys Lys Thr Asp Glu Gly Thr Tyr Arg Cys Glu Gly Arg
               180                 185                 190
   Ile Leu Ala Arg Gly Glu Ile Asn Phe Lys Asp Ile Gln Val Ile Val
           195                 200                 205
   Asn Val Pro Pro Thr Ile Gln Ala Arg Gln Asn Ile Val Asn Ala Thr
       210                 215                 220
   Ala Asn Leu Gly Gln Ser Val Thr Leu Val Cys Asp Ala Glu Gly Phe
   225                 230                 235                 240
   Pro Glu Pro Thr Met Ser Trp Thr Lys Asp Gly Glu Gln Ile Glu Gln
                   245                 250                 255
   Glu Glu Asp Asp Glu Lys Tyr Ile Phe Ser Asp Asp Ser Ser Gln Leu
               260                 265                 270
   Thr Ile Lys Lys Val Asp Lys Asn Asp Glu Ala Glu Tyr Ile Cys Ile
           275                 280                 285
   Ala Glu Asn Lys Ala Gly Glu Gln Asp Ala Thr Ile His Leu Lys Val
       290                 295                 300
   Phe Ala Lys Pro Lys Ile Thr Tyr Val Glu Asn Gln Thr Ala Met Glu
   305                 310                 315                 320
   Leu Glu Glu Gln Val Thr Leu Thr Cys Glu Ala Ser Gly Asp Pro Ile
                   325                 330                 335
   Pro Ser Ile Thr Trp Arg Thr Ser Thr Arg Asn Ile Ser Ser Glu Glu
               340                 345                 350
   Lys Thr Leu Asp Gly His Met Val Val Arg Ser His Ala Arg Val Ser
           355                 360                 365
   Ser Leu Thr Leu Lys Ser Ile Gln Tyr Thr Asp Ala Gly Glu Tyr Ile
       370                 375                 380
   Cys Thr Ala Ser Asn Thr Ile Gly Gln Asp Ser Gln Ser Met Tyr Leu
   385                 390                 395                 400
   Glu Val Gln Tyr Ala Pro Lys Leu Gln Gly Pro Val Ala Val Tyr Thr
                   405                 410                 415
   Trp Glu Gly Asn Gln Val Asn Ile Thr Cys Glu Val Phe Ala Tyr Pro
               420                 425                 430
   Ser Ala Thr Ile Ser Trp Phe Arg Asp Gly Gln Leu Leu Pro Ser Ser
           435                 440                 445
   Asn Tyr Ser Asn Ile Lys Ile Tyr Asn Thr Pro Ser Ala Ser Tyr Leu
       450                 455                 460
   Glu Val Thr Pro Asp Ser Glu Asn Asp Phe Gly Asn Tyr Asn Cys Thr
   465                 470                 475                 480
   Ala Val Asn Arg Ile Gly Gln Glu Ser Leu Glu Phe Ile Leu Val Gln
                   485                 490                 495
   Ala Asp Thr Pro Ser Ser Pro Ser Ile Asp Gln Val Glu Pro Tyr Ser
               500                 505                 510
   Ser Thr Ala Gln Val Gln Phe Asp Glu Pro Glu Ala Thr Gly Gly Val
           515                 520                 525
   Pro Ile Leu Lys Tyr Lys Ala Glu Trp Arg Ala Val Gly Glu Glu Val
       530                 535                 540
   Trp His Ser Lys Trp Tyr Asp Ala Lys Glu Ala Ser Met Glu Gly Ile
   545                 550                 555                 560
   Val Thr Ile Val Gly Leu Lys Pro Glu Thr Thr Tyr Ala Val Arg Leu
                   565                 570                 575
   Ala Ala Leu Asn Gly Lys Gly Leu Gly Glu Ile Ser Ala Ala Ser Glu
               580                 585                 590
   Phe Lys Thr Gln Pro Val Gln Gly Glu Pro Ser Ala Pro Lys Leu Glu
           595                 600                 605
   Gly Gln Met Gly Glu Asp Gly Asn Ser Ile Lys Val Asn Leu Ile Lys
       610                 615                 620
   Gln Asp Asp Gly Gly Ser Pro Ile Arg His Tyr Leu Val Arg Tyr Arg
   625                 630                 635                 640
   Ala Leu Ser Ser Glu Trp Lys Pro Glu Ile Arg Leu Pro Ser Gly Ser
                   645                 650                 655
   Asp His Val Met Leu Lys Ser Leu Asp Trp Asn Ala Glu Tyr Glu Val
               660                 665                 670
   Tyr Val Val Ala Glu Asn Gln Gln Gly Lys Ser Lys Ala Ala His Phe
           675                 680                 685
   Val Phe Arg Thr Ser Ala Gln Pro Thr Ala Ile Pro Ala Asn Gly Ser
       690                 695                 700
   Pro Thr Ser Gly Leu Ser Thr Gly Ala Ile Val Gly Ile Leu Ile Val
   705                 710                 715                 720
   Ile Phe Val Leu Leu Leu Val Val Val Asp Ile Thr Cys Tyr Phe Leu
                   725                 730                 735
   Asn Lys Cys Gly Leu Phe Met Cys Ile Ala Val Asn Leu Cys Gly Lys
               740                 745                 750
   Ala Gly Pro Gly Ala Lys Gly Lys Asp Met Glu Glu Gly Lys Ala Ala
           755                 760                 765
   Phe Ser Lys Asp Glu Ser Lys Glu Pro Ile Val Glu Val Arg Thr Glu
       770                 775                 780
   Glu Glu Arg Thr Pro Asn His Asp Gly Gly Lys His Thr Glu Pro Asn
   785                 790                 795                 800
   Glu Thr Thr Pro Leu Thr Glu Pro Glu Lys Gly Pro Val Glu Ala Lys
                   805                 810                 815
   Pro Glu Cys Gln Glu Thr Glu Thr Lys Pro Ala Pro Ala Glu Val Lys
               820                 825                 830
   Thr Val Pro Asn Asp Ala Thr Gln Thr Lys Glu Asn Glu Ser Lys Ala
           835                 840                 845
<210> 225
   <211> 193
   <212> PRT
   <213> Homo sapiens
<400> 225
   Met Pro Glu Glu Gly Ser Gly Cys Ser Val Arg Arg Arg Pro Tyr Gly
   1               5                   10                  15
   Cys Val Leu Arg Ala Ala Leu Val Pro Leu Val Ala Gly Leu Val Ile
               20                  25                  30
   Cys Leu Val Val Cys Ile Gln Arg Phe Ala Gln Ala Gln Gln Gln Leu
           35                  40                  45
   Pro Leu Glu Ser Leu Gly Trp Asp Val Ala Glu Leu Gln Leu Asn His
       50                  55                  60
   Thr Gly Pro Gln Gln Asp Pro Arg Leu Tyr Trp Gln Gly Gly Pro Ala
   65                  70                  75                  80
   Leu Gly Arg Ser Phe Leu His Gly Pro Glu Leu Asp Lys Gly Gln Leu
                   85                  90                  95
   Arg Ile His Arg Asp Gly Ile Tyr Met Val His Ile Gln Val Thr Leu
               100                 105                 110
   Ala Ile Cys Ser Ser Thr Thr Ala Ser Arg His His Pro Thr Thr Leu
           115                 120                 125
   Ala Val Gly Ile Cys Ser Pro Ala Ser Arg Ser Ile Ser Leu Leu Arg
       130                 135                 140
   Leu Ser Phe His Gln Gly Cys Thr Ile Ala Ser Gln Arg Leu Thr Pro
   145                 150                 155                 160
   Leu Ala Arg Gly Asp Thr Leu Cys Thr Asn Leu Thr Gly Thr Leu Leu
                   165                 170                 175
   Pro Ser Arg Asn Thr Asp Glu Thr Phe Phe Gly Val Gln Trp Val Arg
               180                 185                 190
   Pro
<210> 226
   <211> 232
   <212> PRT
   <213> Homo sapiens
<400> 226
   Met His Arg Arg Arg Ser Arg Ser Cys Arg Glu Asp Gln Lys Pro Val
   1               5                   10                  15
   Met Asp Asp Gln Arg Asp Leu Ile Ser Asn Asn Glu Gln Leu Pro Met
               20                  25                  30
   Leu Gly Arg Arg Pro Gly Ala Pro Glu Ser Lys Cys Ser Arg Gly Ala
           35                  40                  45
   Leu Tyr Thr Gly Phe Ser Ile Leu Val Thr Leu Leu Leu Ala Gly Gln
       50                  55                  60
   Ala Thr Thr Ala Tyr Phe Leu Tyr Gln Gln Gln Gly Arg Leu Asp Lys
   65                  70                  75                  80
   Leu Thr Val Thr Ser Gln Asn Leu Gln Leu Glu Asn Leu Arg Met Lys
                   85                  90                  95
   Leu Pro Lys Pro Pro Lys Pro Val Ser Lys Met Arg Met Ala Thr Pro
               100                 105                 110
   Leu Leu Met Gln Ala Leu Pro Met Gly Ala Leu Pro Gln Gly Pro Met
           115                 120                 125
   Gln Asn Ala Thr Lys Tyr Gly Asn Met Thr Glu Asp His Val Met His
       130                 135                 140
   Leu Leu Gln Asn Ala Asp Pro Leu Lys Val Tyr Pro Pro Leu Lys Gly
   145                 150                 155                 160
   Ser Phe Pro Glu Asn Leu Arg His Leu Lys Asn Thr Met Glu Thr Ile
                   165                 170                 175
   Asp Trp Lys Val Phe Glu Ser Trp Met His His Trp Leu Leu Phe Glu
               180                 185                 190
   Met Ser Arg His Ser Leu Glu Gln Lys Pro Thr Asp Ala Pro Pro Lys
           195                 200                 205
   Glu Ser Leu Glu Leu Glu Asp Pro Ser Ser Gly Leu Gly Val Thr Lys
       210                 215                 220
   Gln Asp Leu Gly Pro Val Pro Met
   225                 230
<210> 227
   <211> 557
   <212> PRT
   <213> Homo sapiens
<400> 227
   Met Pro Pro Pro Arg Leu Leu Phe Phe Leu Leu Phe Leu Thr Pro Met
   1               5                   10                  15
   Glu Val Arg Pro Glu Glu Pro Leu Val Val Lys Val Glu Glu Gly Asp
               20                  25                  30
   Asn Ala Val Leu Gln Cys Leu Lys Gly Thr Ser Asp Gly Pro Thr Gln
           35                  40                  45
   Gln Leu Thr Trp Ser Arg Glu Ser Pro Leu Lys Pro Phe Leu Lys Leu
       50                  55                  60
   Ser Leu Gly Leu Pro Gly Leu Gly Ile His Met Arg Pro Leu Ala Ile
   65                  70                  75                  80
   Trp Leu Phe Ile Phe Asn Val Ser Gln Gln Met Gly Gly Phe Tyr Leu
                   85                  90                  95
   Cys Gln Pro Gly Pro Pro Ser Glu Lys Ala Trp Gln Pro Gly Trp Thr
               100                 105                 110
   Val Asn Val Glu Gly Ser Gly Glu Leu Phe Arg Trp Asn Val Ser Asp
           115                 120                 125
   Leu Gly Gly Leu Gly Cys Gly Leu Lys Asn Arg Ser Ser Glu Gly Pro
       130                 135                 140
   Ser Ser Pro Ser Gly Lys Leu Met Ser Pro Lys Leu Tyr Val Trp Ala
   145                 150                 155                 160
   Lys Asp Arg Pro Glu Ile Trp Glu Gly Glu Pro Pro Cys Leu Pro Pro
                   165                 170                 175
   Arg Asp Ser Leu Asn Gln Ser Leu Ser Gln Asp Leu Thr Met Ala Pro
               180                 185                 190
   Gly Ser Thr Leu Trp Leu Ser Cys Gly Val Pro Pro Asp Ser Val Ser
           195                 200                 205
   Arg Gly Pro Leu Ser Trp Thr His Val His Pro Lys Gly Pro Lys Ser
       210                 215                 220
   Leu Leu Ser Leu Glu Leu Lys Asp Asp Arg Pro Ala Arg Asp Met Trp
   225                 230                 235                 240
   Val Met Glu Thr Gly Leu Leu Leu Pro Arg Ala Thr Ala Gln Asp Ala
                   245                 250                 255
   Gly Lys Tyr Tyr Cys His Arg Gly Asn Leu Thr Met Ser Phe His Leu
               260                 265                 270
   Glu Ile Thr Ala Arg Pro Val Leu Trp His Trp Leu Leu Arg Thr Gly
           275                 280                 285
   Gly Trp Lys Val Ser Ala Val Thr Leu Ala Tyr Leu Ile Phe Cys Leu
       290                 295                 300
   Cys Ser Leu Val Gly Ile Leu His Leu Gln Arg Ala Leu Val Leu Arg
   305                 310                 315                 320
   Arg Lys Arg Lys Arg Met Thr Asp Pro Thr Arg Arg Phe Phe Lys Val
                   325                 330                 335
   Thr Pro Pro Pro Gly Ser Gly Pro Gln Asn Gln Tyr Gly Asn Val Leu
               340                 345                 350
   Ser Leu Pro Thr Pro Thr Ser Gly Leu Gly Arg Ala Gln Arg Trp Ala
           355                 360                 365
   Ala Gly Leu Gly Gly Thr Ala Pro Ser Tyr Gly Asn Pro Ser Ser Asp
       370                 375                 380
   Val Gln Ala Asp Gly Ala Leu Gly Ser Arg Ser Pro Pro Gly Val Gly
   385                 390                 395                 400
   Pro Glu Glu Glu Glu Gly Glu Gly Tyr Glu Glu Pro Asp Ser Glu Glu
                   405                 410                 415
   Asp Ser Glu Phe Tyr Glu Asn Asp Ser Asn Leu Gly Gln Asp Gln Leu
               420                 425                 430
   Ser Gln Asp Gly Ser Gly Tyr Glu Asn Pro Glu Asp Glu Pro Leu Gly
           435                 440                 445
   Pro Glu Asp Glu Asp Ser Phe Ser Asn Ala Glu Ser Tyr Glu Asn Glu
       450                 455                 460
   Asp Glu Glu Leu Thr Gln Pro Val Ala Arg Thr Met Asp Phe Leu Ser
   465                 470                 475                 480
   Pro His Gly Ser Ala Trp Asp Pro Ser Arg Glu Ala Thr Ser Leu Ala
                   485                 490                 495
   Gly Ser Gln Ser Tyr Glu Asp Met Arg Gly Ile Leu Tyr Ala Ala Pro
               500                 505                 510
   Gln Leu Arg Ser Ile Arg Gly Gln Pro Gly Pro Asn His Glu Glu Asp
           515                 520                 525
   Ala Asp Ser Tyr Glu Asn Met Asp Asn Pro Asp Gly Pro Asp Pro Ala
       530                 535                 540
   Trp Gly Gly Gly Gly Arg Met Gly Thr Trp Ser Thr Arg
   545                 550                 555
<210> 228
   <211> 273
   <212> PRT
   <213> Homo sapiens
<400> 228
   Met Thr Pro Gly Thr Gln Ser Pro Phe Phe Leu Leu Leu Leu Leu Thr
   1               5                   10                  15
   Val Leu Thr Val Val Thr Gly Ser Gly His Ala Ser Ser Thr Pro Gly
               20                  25                  30
   Gly Glu Lys Glu Thr Ser Ala Thr Gln Arg Ser Ser Val Pro Ser Ser
           35                  40                  45
   Thr Glu Lys Asn Ala Leu Ser Thr Gly Val Ser Phe Phe Phe Leu Ser
       50                  55                  60
   Phe His Ile Ser Asn Leu Gln Phe Asn Ser Ser Leu Glu Asp Pro Ser
   65                  70                  75                  80
   Thr Asp Tyr Tyr Gln Glu Leu Gln Arg Asp Ile Ser Glu Met Phe Leu
                   85                  90                  95
   Gln Ile Tyr Lys Gln Gly Gly Phe Leu Gly Leu Ser Asn Ile Lys Phe
               100                 105                 110
   Arg Pro Gly Ser Val Val Val Gln Leu Thr Leu Ala Phe Arg Glu Gly
           115                 120                 125
   Thr Ile Asn Val His Asp Val Glu Thr Gln Phe Asn Gln Tyr Lys Thr
       130                 135                 140
   Glu Ala Ala Ser Arg Tyr Asn Leu Thr Ile Ser Asp Val Ser Val Ser
   145                 150                 155                 160
   Asp Val Pro Phe Pro Phe Ser Ala Gln Ser Gly Ala Gly Val Pro Gly
                   165                 170                 175
   Trp Gly Ile Ala Leu Leu Val Leu Val Cys Val Leu Val Ala Leu Ala
               180                 185                 190
   Ile Val Tyr Leu Ile Ala Leu Ala Val Cys Gln Cys Arg Arg Lys Asn
           195                 200                 205
   Tyr Gly Gln Leu Asp Ile Phe Pro Ala Arg Asp Thr Tyr His Pro Met
       210                 215                 220
   Ser Glu Tyr Pro Thr Tyr His Thr His Gly Arg Tyr Val Pro Pro Ser
   225                 230                 235                 240
   Ser Thr Asp Arg Ser Pro Tyr Glu Lys Val Ser Ala Gly Asn Gly Gly
                   245                 250                 255
   Ser Ser Leu Ser Tyr Thr Asn Pro Ala Val Ala Ala Thr Ser Ala Asn
               260                 265                 270
   Leu
<210> 229
   <211> 310
   <212> PRT
   <213> Homo sapiens
<400> 229
   Met Arg Arg Ala Ala Leu Trp Leu Trp Leu Cys Ala Leu Ala Leu Ser
   1               5                   10                  15
   Leu Gln Pro Ala Leu Pro Gln Ile Val Ala Thr Asn Leu Pro Pro Glu
               20                  25                  30
   Asp Gln Asp Gly Ser Gly Asp Asp Ser Asp Asn Phe Ser Gly Ser Gly
           35                  40                  45
   Ala Gly Ala Leu Gln Asp Ile Thr Leu Ser Gln Gln Thr Pro Ser Thr
       50                  55                  60
   Trp Lys Asp Thr Gln Leu Leu Thr Ala Ile Pro Thr Ser Pro Glu Pro
   65                  70                  75                  80
   Thr Gly Leu Glu Ala Thr Ala Ala Ser Thr Ser Thr Leu Pro Ala Gly
                   85                  90                  95
   Glu Gly Pro Lys Glu Gly Glu Ala Val Val Leu Pro Glu Val Glu Pro
               100                 105                 110
   Gly Leu Thr Ala Arg Glu Gln Glu Ala Thr Pro Arg Pro Arg Glu Thr
           115                 120                 125
   Thr Gln Leu Pro Thr Thr His Gln Ala Ser Thr Thr Thr Ala Thr Thr
       130                 135                 140
   Ala Gln Glu Pro Ala Thr Ser His Pro His Arg Asp Met Gln Pro Gly
   145                 150                 155                 160
   His His Glu Thr Ser Thr Pro Ala Gly Pro Ser Gln Ala Asp Leu His
                   165                 170                 175
   Thr Pro His Thr Glu Asp Gly Gly Pro Ser Ala Thr Glu Arg Ala Ala
               180                 185                 190
   Glu Asp Gly Ala Ser Ser Gln Leu Pro Ala Ala Glu Gly Ser Gly Glu
           195                 200                 205
   Gln Asp Phe Thr Phe Glu Thr Ser Gly Glu Asn Thr Ala Val Val Ala
       210                 215                 220
   Val Glu Pro Asp Arg Arg Asn Gln Ser Pro Val Asp Gln Gly Ala Thr
   225                 230                 235                 240
   Gly Ala Ser Gln Gly Leu Leu Asp Arg Lys Glu Val Leu Gly Gly Val
                   245                 250                 255
   Ile Ala Gly Gly Leu Val Gly Leu Ile Phe Ala Val Cys Leu Val Gly
               260                 265                 270
   Phe Met Leu Tyr Arg Met Lys Lys Lys Asp Glu Gly Ser Tyr Ser Leu
           275                 280                 285
   Glu Glu Pro Lys Gln Ala Asn Gly Gly Ala Tyr Gln Lys Pro Thr Lys
       290                 295                 300
   Gln Glu Glu Phe Tyr Ala
   305                 310
<210> 230
   <211> 1048
   <212> PRT
   <213> Homo sapiens
<400> 230
   Met Ala Phe Pro Pro Arg Arg Arg Leu Arg Leu Gly Pro Arg Gly Leu
   1               5                   10                  15
   Pro Leu Leu Leu Ser Gly Leu Leu Leu Pro Leu Cys Arg Ala Phe Asn
               20                  25                  30
   Leu Asp Val Asp Ser Pro Ala Glu Tyr Ser Gly Pro Glu Gly Ser Tyr
           35                  40                  45
   Phe Gly Phe Ala Val Asp Phe Phe Val Pro Ser Ala Ser Ser Arg Met
       50                  55                  60
   Phe Leu Leu Val Gly Ala Pro Lys Ala Asn Thr Thr Gln Pro Gly Ile
   65                  70                  75                  80
   Val Glu Gly Gly Gln Val Leu Lys Cys Asp Trp Ser Ser Thr Arg Arg
                   85                  90                  95
   Cys Gln Pro Ile Glu Phe Asp Ala Thr Gly Asn Arg Asp Tyr Ala Lys
               100                 105                 110
   Asp Asp Pro Leu Glu Phe Lys Ser His Gln Trp Phe Gly Ala Ser Val
           115                 120                 125
   Arg Ser Lys Gln Asp Lys Ile Leu Ala Cys Ala Pro Leu Tyr His Trp
       130                 135                 140
   Arg Thr Glu Met Lys Gln Glu Arg Glu Pro Val Gly Thr Cys Phe Leu
   145                 150                 155                 160
   Gln Asp Gly Thr Lys Thr Val Glu Tyr Ala Pro Cys Arg Ser Gln Asp
                   165                 170                 175
   Ile Asp Ala Asp Gly Gln Gly Phe Cys Gln Gly Gly Phe Ser Ile Asp
               180                 185                 190
   Phe Thr Lys Ala Asp Arg Val Leu Leu Gly Gly Pro Gly Ser Phe Tyr
           195                 200                 205
   Trp Gln Gly Gln Leu Ile Ser Asp Gln Val Ala Glu Ile Val Ser Lys
       210                 215                 220
   Tyr Asp Pro Asn Val Tyr Ser Ile Lys Tyr Asn Asn Gln Leu Ala Thr
   225                 230                 235                 240
   Arg Thr Ala Gln Ala Ile Phe Asp Asp Ser Tyr Leu Gly Tyr Ser Val
                   245                 250                 255
   Ala Val Gly Asp Phe Asn Gly Asp Gly Ile Asp Asp Phe Val Ser Gly
               260                 265                 270
   Val Pro Arg Ala Ala Arg Thr Leu Gly Met Val Tyr Ile Tyr Asp Gly
           275                 280                 285
   Lys Asn Met Ser Ser Leu Tyr Asn Phe Thr Gly Glu Gln Met Ala Ala
           290                 295                 300
   Tyr Phe Gly Phe Ser Val Ala Ala Thr Asp Ile Asn Gly Asp Asp Tyr
   305                 310                 315                 320
   Ala Asp Val Phe Ile Gly Ala Pro Leu Phe Met Asp Arg Gly Ser Asp
                   325                 330                 335
   Gly Lys Leu Gln Glu Val Gly Gln Val Ser Val Ser Leu Gln Arg Ala
               340                 345                 350
   Ser Gly Asp Phe Gln Thr Thr Lys Leu Asn Gly Phe Glu Val Phe Ala
           355                 360                 365
   Arg Phe Gly Ser Ala Ile Ala Pro Leu Gly Asp Leu Asp Gln Asp Gly
       370                 375                 380
   Phe Asn Asp Ile Ala Ile Ala Ala Pro Tyr Gly Gly Glu Asp Lys Lys
   385                 390                 395                 400
   Gly Ile Val Tyr Ile Phe Asn Gly Arg Ser Thr Gly Leu Asn Ala Val
                   405                 410                 415
   Pro Ser Gln Ile Leu Glu Gly Gln Trp Ala Ala Arg Ser Met Pro Pro
               420                 425                 430
   Ser Phe Gly Tyr Ser Met Lys Gly Ala Thr Asp Ile Asp Lys Asn Gly
           435                 440                 445
   Tyr Pro Asp Leu Ile Val Gly Ala Phe Gly Val Asp Arg Ala Ile Leu
       450                 455                 460
   Tyr Arg Ala Arg Pro Val Ile Thr Val Asn Ala Gly Leu Glu Val Tyr
   465                 470                 475                 480
   Pro Ser Ile Leu Asn Gln Asp Asn Lys Thr Cys Ser Leu Pro Gly Thr
                   485                 490                 495
   Ala Leu Lys Val Ser Cys Phe Asn Val Arg Phe Cys Leu Lys Ala Asp
               500                 505                 510
   Gly Lys Gly Val Leu Pro Arg Lys Leu Asn Phe Gln Val Glu Leu Leu
           515                 520                 525
   Leu Asp Lys Leu Lys Gln Lys Gly Ala Ile Arg Arg Ala Leu Phe Leu
       530                 535                 540
   Tyr Ser Arg Ser Pro Ser His Ser Lys Asn Met Thr Ile Ser Arg Gly
   545                 550                 555                 560
   Gly Leu Met Gln Cys Glu Glu Leu Ile Ala Tyr Leu Arg Asp Glu Ser
                   565                 570                 575
   Glu Phe Arg Asp Lys Leu Thr Pro Ile Thr Ile Phe Met Glu Tyr Arg
               580                 585                 590
   Leu Asp Tyr Arg Thr Ala Ala Asp Thr Thr Gly Leu Gln Pro Ile Leu
           595                 600                 605
   Asn Gln Phe Thr Pro Ala Asn Ile Ser Arg Gln Ala His Ile Leu Leu
       610                 615                 620
   Asp Cys Gly Glu Asp Asn Val Cys Lys Pro Lys Leu Glu Val Ser Val
   625                 630                 635                 640
   Asp Ser Asp Gln Lys Lys Ile Tyr Ile Gly Asp Asp Asn Pro Leu Thr
                   645                 650                 655
   Leu Ile Val Lys Ala Gln Asn Gln Gly Glu Gly Ala Tyr Glu Ala Glu
               660                 665                 670
   Leu Ile Val Ser Ile Pro Leu Gln Ala Asp Phe Ile Gly Val Val Arg
           675                 680                 685
   Asn Asn Glu Ala Leu Ala Arg Leu Ser Cys Ala Phe Lys Thr Glu Asn
       690                 695                 700
   Gln Thr Arg Gln Val Val Cys Asp Leu Gly Asn Pro Met Lys Ala Gly
   705                 710                 715                 720
   Thr Gln Leu Leu Ala Gly Leu Arg Phe Ser Val His Gln Gln Ser Glu
                   725                 730                 735
   Met Asp Thr Ser Val Lys Phe Asp Leu Gln Ile Gln Ser Ser Asn Leu
               740                 745                 750
   Phe Asp Lys Val Ser Pro Val Val Ser His Lys Val Asp Leu Ala Val
           755                 760                 765
   Leu Ala Ala Val Glu Ile Arg Gly Val Ser Ser Pro Asp His Ile Phe
       770                 775                 780
   Leu Pro Ile Pro Asn Trp Glu His Lys Glu Asn Pro Glu Thr Glu Glu
   785                 790                 795                 800
   Asp Val Gly Pro Val Val Gln His Ile Tyr Glu Leu Arg Asn Asn Gly
                   805                 810                 815
   Pro Ser Ser Phe Ser Lys Ala Met Leu His Leu Gln Trp Pro Tyr Lys
               820                 825                 830
   Tyr Asn Asn Asn Thr Leu Leu Tyr Ile Leu His Tyr Asp Ile Asp Gly
           835                 840                 845
   Pro Met Asn Cys Thr Ser Asp Met Glu Ile Asn Pro Leu Arg Ile Lys
       850                 855                 860
   Ile Ser Ser Leu Gln Thr Thr Glu Lys Asn Asp Thr Val Ala Gly Gln
   865                 870                 875                 880
   Gly Glu Arg Asp His Leu Ile Thr Lys Arg Asp Leu Ala Leu Ser Glu
                   885                 890                 895
   Gly Asp Ile His Thr Leu Gly Cys Gly Val Ala Gln Cys Leu Lys Ile
               900                 905                 910
   Val Cys Gln Val Gly Arg Leu Asp Arg Gly Lys Ser Ala Ile Leu Tyr
           915                 920                 925
   Val Lys Ser Leu Leu Trp Thr Glu Thr Phe Met Asn Lys Glu Asn Gln
       930                 935                 940
   Asn His Ser Tyr Ser Leu Lys Ser Ser Ala Ser Phe Asn Val Ile Glu
   945                 950                 955                 960
   Phe Pro Tyr Lys Asn Leu Pro Ile Glu Asp Ile Thr Asn Ser Thr Leu
                   965                 970                 975
   Val Thr Thr Asn Val Thr Trp Gly Ile Gln Pro Ala Pro Met Pro Val
               980                 985                 990
   Pro Val Trp Val Ile Ile Leu Ala Val Leu Ala Gly Leu Leu Leu Leu
           995                 1000                1005
   Ala Val Leu Val Phe Val Met Tyr Arg Met Gly Phe Phe Lys Arg Val
       1010                1015                1020
   Arg Pro Pro Gln Glu Glu Gln Glu Arg Glu Gln Leu Gln Pro His Glu
   1025                1030                1035                1040
   Asn Gly Glu Gly Asn Ser Glu Thr
                   1045
<210> 231
   <211> 188
   <212> PRT
   <213> Homo sapiens
<400> 231
   Met Asp Cys Arg Lys Met Ala Arg Phe Ser Tyr Ser Val Ile Trp Ile
   1               5                   10                  15
   Met Ala Ile Ser Lys Val Phe Glu Leu Gly Leu Val Ala Gly Leu Gly
               20                  25                  30
   His Gln Glu Phe Ala Arg Pro Ser Arg Gly Tyr Leu Ala Phe Arg Asp
           35                  40                  45
   Asp Ser Ile Trp Pro Gln Glu Glu Pro Ala Ile Arg Pro Arg Ser Ser
       50                  55                  60
   Gln Arg Val Pro Pro Met Gly Ile Gln His Ser Lys Glu Leu Asn Arg
   65                  70                  75                  80
   Thr Cys Cys Leu Asn Gly Gly Thr Cys Met Leu Gly Ser Phe Cys Ala
                   85                  90                  95
   Cys Pro Pro Ser Phe Tyr Gly Arg Asn Cys Glu His Asp Val Arg Lys
               100                 105                 110
   Glu Asn Cys Gly Ser Val Pro His Asp Thr Trp Leu Pro Lys Lys Cys
           115                 120                 125
   Ser Leu Cys Lys Cys Trp His Gly Gln Leu Arg Cys Phe Pro Gln Ala
       130                 135                 140
   Phe Leu Pro Gly Cys Asp Gly Leu Val Met Asp Glu His Leu Val Ala
   145                 150                 155                 160
   Ser Arg Thr Pro Glu Leu Pro Pro Ser Ala Arg Thr Thr Thr Phe Met
                   165                 170                 175
   Leu Val Gly Ile Cys Leu Ser Ile Gln Ser Tyr Tyr
               180                 185
<210> 232
   <211> 750
   <212> PRT
   <213> Homo sapiens
<400> 232
   Met Trp Asn Leu Leu His Glu Thr Asp Ser Ala Val Ala Thr Ala Arg
   1               5                   10                  15
   Arg Pro Arg Trp Leu Cys Ala Gly Ala Leu Val Leu Ala Gly Gly Phe
               20                  25                  30
   Phe Leu Leu Gly Phe Leu Phe Gly Trp Phe Ile Lys Ser Ser Asn Glu
           35                  40                  45
   Ala Thr Asn Ile Thr Pro Lys His Asn Met Lys Ala Phe Leu Asp Glu
       50                  55                  60
   Leu Lys Ala Glu Asn Ile Lys Lys Phe Leu Tyr Asn Phe Thr Gln Ile
   65                  70                  75                  80
   Pro His Leu Ala Gly Thr Glu Gln Asn Phe Gln Leu Ala Lys Gln Ile
                   85                  90                  95
   Gln Ser Gln Trp Lys Glu Phe Gly Leu Asp Ser Val Glu Leu Ala His
               100                 105                 110
   Tyr Asp Val Leu Leu Ser Tyr Pro Asn Lys Thr His Pro Asn Tyr Ile
           115                 120                 125
   Ser Ile Ile Asn Glu Asp Gly Asn Glu Ile Phe Asn Thr Ser Leu Phe
       130                 135                 140
   Glu Pro Pro Pro Pro Gly Tyr Glu Asn Val Ser Asp Ile Val Pro Pro
   145                 150                 155                 160
   Phe Ser Ala Phe Ser Pro Gln Gly Met Pro Glu Gly Asp Leu Val Tyr
                   165                 170                 175
   Val Asn Tyr Ala Arg Thr Glu Asp Phe Phe Lys Leu Glu Arg Asp Met
               180                 185                 190
   Lys Ile Asn Cys Ser Gly Lys Ile Val Ile Ala Arg Tyr Gly Lys Val
           195                 200                 205
   Phe Arg Gly Asn Lys Val Lys Asn Ala Gln Leu Ala Gly Ala Lys Gly
       210                 215                 220
   Val Ile Leu Tyr Ser Asp Pro Ala Asp Tyr Phe Ala Pro Gly Val Lys
   225                 230                 235                 240
   Ser Tyr Pro Asp Gly Trp Asn Leu Pro Gly Gly Gly Val Gln Arg Gly
                   245                 250                 255
   Asn Ile Leu Asn Leu Asn Gly Ala Gly Asp Pro Leu Thr Pro Gly Tyr
               260                 265                 270
   Pro Ala Asn Glu Tyr Ala Tyr Arg Arg Gly Ile Ala Glu Ala Val Gly
           275                 280                 285
   Leu Pro Ser Ile Pro Val His Pro Ile Gly Tyr Tyr Asp Ala Gln Lys
       290                 295                 300
   Leu Leu Glu Lys Met Gly Gly Ser Ala Pro Pro Asp Ser Ser Trp Arg
   305                 310                 315                 320
   Gly Ser Leu Lys Val Pro Tyr Asn Val Gly Pro Gly Phe Thr Gly Asn
                   325                 330                 335
   Phe Ser Thr Gln Lys Val Lys Met His Ile His Ser Thr Asn Glu Val
               340                 345                 350
   Thr Arg Ile Tyr Asn Val Ile Gly Thr Leu Arg Gly Ala Val Glu Pro
           355                 360                 365
   Asp Arg Tyr Val Ile Leu Gly Gly His Arg Asp Ser Trp Val Phe Gly
       370                 375                 380
   Gly Ile Asp Pro Gln Ser Gly Ala Ala Val Val His Glu Ile Val Arg
   385                 390                 395                 400
   Ser Phe Gly Thr Leu Lys Lys Glu Gly Trp Arg Pro Arg Arg Thr Ile
                   405                 410                 415
   Leu Phe Ala Ser Trp Asp Ala Glu Glu Phe Gly Leu Leu Gly Ser Thr
               420                 425                 430
   Glu Trp Ala Glu Glu Asn Ser Arg Leu Leu Gln Glu Arg Gly Val Ala
           435                 440                 445
   Tyr Ile Asn Ala Asp Ser Ser Ile Glu Gly Asn Tyr Thr Leu Arg Val
   450 455 460
   Asp Cys Thr Pro Leu Met Tyr Ser Leu Val His Asn Leu Thr Lys Glu
   465 470 475 480
   Leu Lys Ser Pro Asp Glu Gly Phe Glu Gly Lys Ser Leu Tyr Glu Ser
   485 490 495
   Trp Thr Lys Lys Ser Pro Ser Pro Glu Phe Ser Gly Met Pro Arg Ile
   500 505 510
   Ser Lys Leu Gly Ser Gly Asn Asp Phe Glu Val Phe Phe Gln Arg Leu
   515 520 525
   Gly Ile Ala Ser Gly Arg Ala Arg Tyr Thr Lys Asn Trp Glu Thr Asn
   530 535 540
   Lys Phe Ser Gly Tyr Pro Leu Tyr His Ser Val Tyr Glu Thr Tyr Glu
   545 550 555 560
   Leu Val Glu Lys Phe Tyr Asp Pro Met Phe Lys Tyr His Leu Thr Val
   565 570 575
   Ala Gln Val Arg Gly Gly Met Val Phe Glu Leu Ala Asn Ser Ile Val
   580 585 590
   Leu Pro Phe Asp Cys Arg Asp Tyr Ala Val Val Leu Arg Lys Tyr Ala
   595 600 605
   Asp Lys Ile Tyr Ser Ile Ser Met Lys His Pro Gln Glu Met Lys Thr
   610 615 620
   Tyr Ser Val Ser Phe Asp Ser Leu Phe Ser Ala Val Lys Asn Phe Thr
   625 630 635 640
   Glu Ile Ala Ser Lys Phe Ser Glu Arg Leu Gln Asp Phe Asp Lys Ser
   645 650 655
   Asn Pro Ile Val Leu Arg Met Met Asn Asp Gln Leu Met Phe Leu Glu
   660 665 670
   Arg Ala Phe Ile Asp Pro Leu Gly Leu Pro Asp Arg Pro Phe Tyr Arg
   675 680 685
   His Val Ile Tyr Ala Pro Ser Ser His Asn Lys Tyr Ala Gly Glu Ser
   690 695 700
   Phe Pro Gly Ile Tyr Asp Ala Leu Phe Asp Ile Glu Ser Lys Val Asp
   705 710 715 720
   Pro Ser Lys Ala Trp Gly Glu Val Lys Arg Gln Ile Tyr Val Ala Ala
   725 730 735
   Phe Thr Val Gln Ala Ala Ala Glu Thr Leu Ser Glu Val Ala
   740 745 750
<210> 233
   <211> 976
   <212> PRT
   <213> Homo sapiens
<400> 233
<210> 234
   <211> 668
   <212> PRT
   <213> Homo sapiens
<400> 234
   Met Gly Gly Lys Gln Arg Asp Glu Asp Asp Glu Ala Tyr Gly Lys Pro
   1               5                   10                  15
   Val Lys Tyr Asp Pro Ser Phe Arg Gly Pro Ile Lys Asn Arg Ser Cys
               20                  25                  30
   Thr Asp Val Ile Cys Cys Val Leu Phe Leu Leu Phe Ile Leu Gly Tyr
           35                  40                  45
   Ile Val Val Gly Ile Val Ala Trp Leu Tyr Gly Asp Pro Arg Gln Val
       50                  55                  60
   Leu Tyr Pro Arg Asn Ser Thr Gly Ala Tyr Cys Gly Met Gly Glu Asn
   65                  70                  75                  80
   Lys Asp Lys Pro Tyr Leu Leu Tyr Phe Asn Ile Phe Ser Cys Ile Leu
                   85                  90                  95
   Ser Ser Asn Ile Ile Ser Val Ala Glu Asn Gly Leu Gln Cys Pro Thr
   100 105 110
   Pro Gln Thr Val Ile Thr Ser Leu Gln Gln Glu Leu Cys Pro Ser Phe
   115 120 125
   Leu Leu Pro Ser Ala Pro Ala Leu Gly Arg Cys Phe Pro Trp Thr Asn
   130 135 140
   Val Thr Pro Pro Ala Leu Pro Gly Ile Thr Asn Asp Thr Thr Ile Gln
   145 150 155 160
   Gln Gly Ile Ser Gly Leu Ile Asp Ser Leu Asn Ala Arg Asp Ile Ser
   165 170 175
   Val Lys Ile Phe Glu Asp Phe Ala Gln Ser Trp Tyr Trp Ile Leu Val
   180 185 190
   Ala Leu Gly Val Ala Leu Val Leu Ser Leu Leu Phe Ile Leu Leu Leu
   195 200 205
   Arg Leu Val Ala Gly Pro Leu Val Leu Val Leu Ile Leu Gly Val Leu
   210 215 220
   Gly Val Leu Ala Tyr Gly Ile Tyr Tyr Cys Trp Glu Glu Tyr Arg Val
   225 230 235 240
   Leu Arg Asp Lys Gly Ala Ser Ile Ser Gln Leu Gly Phe Thr Thr Asn
   245 250 255
   Leu Ser Ala Tyr Gln Ser Val Gln Glu Thr Trp Leu Ala Ala Leu Ile
   260 265 270
   Val Leu Ala Val Leu Glu Ala Ile Leu Leu Leu Met Leu Ile Phe Leu
   275 280 285
   Arg Gln Arg Ile Arg Ile Ala Ile Ala Leu Leu Lys Glu Ala Ser Lys
   290 295 300
   Ala Val Gly Gln Met Met Ser Thr Met Phe Tyr Pro Leu Val Thr Phe
   305 310 315 320
   Val Leu Leu Leu Ile Cys Ile Ala Tyr Trp Ala Met Thr Ala Leu Tyr
   325 330 335
   Leu Ala Thr Ser Gly Gln Pro Gln Tyr Val Leu Trp Ala Ser Asn Ile
   340 345 350
   Ser Ser Pro Gly Cys Glu Lys Val Pro Ile Asn Thr Ser Cys Asn Pro
   355 360 365
   Thr Ala His Leu Val Asn Ser Ser Cys Pro Gly Leu Met Cys Val Phe
   370 375 380
   Gln Gly Tyr Ser Ser Lys Gly Leu Ile Gln Arg Ser Val Phe Asn Leu
   385 390 395 400
   Gln Ile Tyr Gly Val Leu Gly Leu Phe Trp Thr Leu Asn Trp Val Leu
   405 410 415
   Ala Leu Gly Gln Cys Val Leu Ala Gly Ala Phe Ala Ser Phe Tyr Trp
   420 425 430
   Ala Phe His Lys Pro Gln Asp Ile Pro Thr Phe Pro Leu Ile Ser Ala
   435 440 445
   Phe Ile Arg Thr Leu Arg Tyr His Thr Gly Ser Leu Ala Phe Gly Ala
   450 455 460
   Leu Ile Leu Thr Leu Val Gln Ile Ala Arg Val Ile Leu Glu Tyr Ile
   465 470 475 480
   Asp His Lys Leu Arg Gly Val Gln Asn Pro Val Ala Arg Cys Ile Met
   485 490 495
   Cys Cys Phe Lys Cys Cys Leu Trp Cys Leu Glu Lys Phe Ile Lys Phe
   500 505 510
   Leu Asn Arg Asn Ala Tyr Ile Met Ile Ala Ile Tyr Gly Lys Asn Phe
   515 520 525
   Cys Val Ser Ala Lys Asn Ala Phe Met Leu Leu Met Arg Asn Ile Val
   530 535 540
   Arg Val Val Val Leu Asp Lys Val Thr Asp Leu Leu Leu Phe Phe Gly
   545 550 555 560
   Lys Leu Leu Val Val Gly Gly Val Gly Val Leu Ser Phe Phe Phe Phe
   565 570 575
   Ser Gly Arg Ile Pro Gly Leu Gly Lys Asp Phe Lys Ser Pro His Leu
   580 585 590
   Asn Tyr Tyr Trp Leu Pro Ile Met Thr Ser Ile Leu Gly Ala Tyr Val
   595 600 605
   Ile Ala Ser Gly Phe Phe Ser Val Phe Gly Met Cys Val Asp Thr Leu
   610 615 620
   Phe Leu Cys Phe Leu Glu Asp Leu Glu Arg Asn Asn Gly Ser Leu Asp
   625 630 635 640
   Arg Pro Tyr Tyr Met Ser Lys Ser Leu Leu Lys Ile Leu Gly Lys Lys
   645 650 655
   Asn Glu Ala Pro Pro Asp Asn Lys Lys Arg Lys Lys
   660 665
<210> 235
   <211> 214
   <212> PRT
   <213> Artificial Sequence
<220>
   <223> antibody constituent
<400> 235
   Asp Ile Leu Leu Thr Gln Ser Pro Val Ile Leu Ser Val Ser Pro Gly
   1 5 10 15
   Glu Arg Val Ser Phe Ser Cys Arg Ala Ser Gln Ser Ile Gly Thr Asn
   20 25 30
   Ile His Trp Tyr Gln Gln Arg Thr Asn Gly Ser Pro Arg Leu Leu Ile
   35 40 45
   Lys Tyr Ala Ser Glu Ser Ile Ser Gly Ile Pro Ser Arg Phe Ser Gly
   50 55 60
   Ser Gly Ser Gly Thr Asp Phe Thr Leu Ser Ile Asn Ser Val Glu Ser
   65 70 75 80
   Glu Asp Ile Ala Asp Tyr Tyr Cys Gln Gln Asn Asn Asn Trp Pro Thr
   85 90 95
   Thr Phe Gly Ala Gly Thr Lys Leu Glu Leu Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 236
   <211> 449
   <212> PRT
   <213> Artificial Sequence
<220>
   <223> antibody consituent
<400> 236
   Gln Val Gln Leu Lys Gln Ser Gly Pro Gly Leu Val Gln Pro Ser Gln
   1               5                   10                  15
   Ser Leu Ser Ile Thr Cys Thr Val Ser Gly Phe Ser Leu Thr Asn Tyr
               20                  25                  30
   Gly Val His Trp Val Arg Gln Ser Pro Gly Lys Gly Leu Glu Trp Leu
           35                  40                  45
   Gly Val Ile Trp Ser Gly Gly Asn Thr Asp Tyr Asn Thr Pro Phe Thr
       50                  55                  60
   Ser Arg Leu Ser Ile Asn Lys Asp Asn Ser Lys Ser Gln Val Phe Phe
   65                  70                  75                  80
   Lys Met Asn Ser Leu Gln Ser Asn Asp Thr Ala Ile Tyr Tyr Cys Ala
                   85                  90                  95
   Arg Ala Leu Thr Tyr Tyr Asp Tyr Glu Phe Ala Tyr Trp Gly Gln Gly
               100                 105                 110
   Thr Leu Val Thr Val Ser Ala Ala Ser Thr Lys Gly Pro Ser Val Phe
           115                 120                 125
   Pro Leu Ala Pro Ser Ser Lys Ser Thr Ser Gly Gly Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Ser Leu Gly Thr Gln Thr Tyr Ile Cys Asn Val Asn His Lys Pro
           195                 200                 205
   Ser Asn Thr Lys Val Asp Lys Arg Val Glu Pro Lys Ser Cys Asp Lys
       210                 215                 220
   Thr His Thr Cys Pro Pro Cys Pro Ala Pro Glu Leu Leu Gly Gly Pro
   225                 230                 235                 240
   Ser Val Phe Leu Phe Pro Pro Lys Pro Lys Asp Thr Leu Met Ile Ser
                   245                 250                 255
   Arg Thr Pro Glu Val Thr Cys Val Val Val Asp Val Ser His Glu Asp
               260                 265                 270
   Pro Glu Val Lys Phe Asn Trp Tyr Val Asp Gly Val Glu Val His Asn
           275                 280                 285
   Ala Lys Thr Lys Pro Arg Glu Glu Gln Tyr Asn Ser Thr Tyr Arg Val
       290                 295                 300
   Val Ser Val Leu Thr Val Leu His Gln Asp Trp Leu Asn Gly Lys Glu
   305                 310                 315                 320
   Tyr Lys Cys Lys Val Ser Asn Lys Ala Leu Pro Ala Pro Ile Glu Lys
                   325                 330                 335
   Thr Ile Ser Lys Ala Lys Gly Gln Pro Arg Glu Pro Gln Val Tyr Thr
               340                 345                 350
   Leu Pro Pro Ser Arg Glu Glu Met Thr Lys Asn Gln Val Ser Leu Thr
           355                 360                 365
   Cys Leu Val Lys Gly Phe Tyr Pro Ser Asp Ile Ala Val Glu Trp Glu
       370                 375                 380
   Ser Asn Gly Gln Pro Glu Asn Asn Tyr Lys Thr Thr Pro Pro Val Leu
   385                 390                 395                 400
   Asp Ser Asp Gly Ser Phe Phe Leu Tyr Ser Lys Leu Thr Val Asp Lys
                   405                 410                 415
   Ser Arg Trp Gln Gln Gly Asn Val Phe Ser Cys Ser Val Met His Glu
               420                 425                 430
   Ala Leu His Asn His Tyr Thr Gln Lys Ser Leu Ser Leu Ser Pro Gly
           435                 440                 445
   Lys
<210> 237
   <211> 214
   <212> PRT
   <213> Artificial Sequence
<220>
   <223> antibody constituent
<400> 237
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Gln Asp Ile Ser Asn Tyr
               20                  25                  30
   Leu Asn Trp Tyr Gln Gln Lys Pro Gly Lys Ala Pro Lys Leu Leu Ile
           35                  40                  45
   Tyr Asp Ala Ser Asn Leu Glu Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Phe Cys Gln His Phe Asp His Leu Pro Leu
                   85                  90                  95
   Ala Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 238
   <211> 444
   <212> PRT
   <213> Artificial Sequence
<220>
   <223> antibody constituent
<400> 238
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Gly Ser Val Ser Ser Gly
               20                  25                  30
   Asp Tyr Tyr Trp Thr Trp Ile Arg Gln Ser Pro Gly Lys Gly Leu Glu
           35                  40                  45
   Trp Ile Gly His Ile Tyr Tyr Ser Gly Asn Thr Asn Tyr Asn Pro Ser
       50                  55                  60
   Leu Lys Ser Arg Leu Thr Ile Ser Ile Asp Thr Ser Lys Thr Gln Phe
   65                  70                  75                  80
   Ser Leu Lys Leu Ser Ser Val Thr Ala Ala Asp Thr Ala Ile Tyr Tyr
                   85                  90                  95
   Cys Val Arg Asp Arg Val Thr Gly Ala Phe Asp Ile Trp Gly Gln Gly
               100                 105                 110
   Thr Met Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Asn Phe Gly Thr Gln Thr Tyr Thr Cys Asn Val Asp His Lys Pro
           195                 200                 205
   Ser Asn Thr Lys Val Asp Lys Thr Val Glu Arg Lys Cys Cys Val Glu
       210                 215                 220
   Cys Pro Pro Cys Pro Ala Pro Pro Val Ala Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser His Glu Asp Pro Glu Val Gln
               260                 265                 270
   Phe Asn Trp Tyr Val Asp Gly Val Glu Val His Asn Ala Lys Thr Lys
           275                 280                 285
   Pro Arg Glu Glu Gln Phe Asn Ser Thr Phe Arg Val Val Ser Val Leu
       290                 295                 300
   Thr Val Val His Gln Asp Trp Leu Asn Gly Lys Glu Tyr Lys Cys Lys
   305                 310                 315                 320
   Val Ser Asn Lys Gly Leu Pro Ala Pro Ile Glu Lys Thr Ile Ser Lys
                   325                 330                 335
   Thr Lys Gly Gln Pro Arg Glu Pro Gln Val Tyr Thr Leu Pro Pro Ser
               340                 345                 350
   Arg Glu Glu Met Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
       370                 375                 380
   Pro Glu Asn Asn Tyr Lys Thr Thr Pro Pro Met Leu Asp Ser Asp Gly
   385                 390                 395                 400
   Ser Phe Phe Leu Tyr Ser Lys Leu Thr Val Asp Lys Ser Arg Trp Gln
                   405                 410                 415
   Gln Gly Asn Val Phe Ser Cys Ser Val Met His Glu Ala Leu His Asn
               420                 425                 430
   His Tyr Thr Gln Lys Ser Leu Ser Leu Ser Pro Gly
           435                 440
<210> 239
   <211> 219
   <212> PRT
   <213> Artificial Sequence
<220>
   <223> antibody constituent
<400> 239
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Asn Ile Val His Ser
               20                  25                  30
   Asn Gly Asn Thr Tyr Leu Asp Trp Tyr Gln Gln Thr Pro Gly Lys Ala
           35                  40                  45
   Pro Lys Leu Leu Ile Tyr Lys Val Ser Asn Arg Phe Ser Gly Val Pro
       50                  55                  60
   Ser Arg Phe Ser Gly Ser Gly Ser Gly Thr Asp Phe Thr Phe Thr Ile
   65                  70                  75                  80
   Ser Ser Leu Gln Pro Glu Asp Ile Ala Thr Tyr Tyr Cys Phe Gln Tyr
                   85                  90                  95
   Ser His Val Pro Trp Thr Phe Gly Gln Gly Thr Lys Leu Gln Ile Thr
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 240
   <211> 453
   <212> PRT
   <213> Artificial Sequence
<220>
   <223> antibody constituent
<400> 240
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Asn Tyr
               20                  25                  30
   Tyr Ile Tyr Trp Val Arg Gln Ala Pro Gly Gln Gly Leu Glu Trp Ile
           35                  40                  45
   Gly Gly Ile Asn Pro Thr Ser Gly Gly Ser Asn Phe Asn Glu Lys Phe
       50                  55                  60
   Lys Thr Arg Val Thr Ile Thr Ala Asp Glu Ser Ser Thr Thr Ala Tyr
   65                  70                  75                  80
   Met Glu Leu Ser Ser Leu Arg Ser Glu Asp Thr Ala Phe Tyr Phe Cys
                   85                  90                  95
   Thr Arg Gln Gly Leu Trp Phe Asp Ser Asp Gly Arg Gly Phe Asp Phe
               100                 105                 110
   Trp Gly Gln Gly Thr Thr Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Lys Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Asn Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Asp Glu Leu Thr Lys Asn Gln
           355                 360                 365
   Val Ser Leu Thr Cys Leu Val Lys Gly Phe Tyr Pro Ser Asp Ile Ala
       370                 375                 380
   Val Glu Trp Glu Ser Asn Gly Gln Pro Glu Asn Asn Tyr Lys Thr Thr
   385                 390                 395                 400
   Pro Pro Val Leu Asp Ser Asp Gly Ser Phe Phe Leu Tyr Ser Lys Leu
                   405                 410                 415
   Thr Val Asp Lys Ser Arg Trp Gln Gln Gly Asn Val Phe Ser Cys Ser
               420                 425                 430
   Val Met His Glu Ala Leu His Asn His Tyr Thr Gln Lys Ser Leu Ser
           435                 440                 445
   Leu Ser Pro Gly Lys
       450